(19) Europäisches Patentamt
European Patent Office
Office européen des brevets

(11) **EP 4 682 145 A1**

(12) **EUROPEAN PATENT APPLICATION**
published in accordance with Art. 153(4) EPC


(43) Date of publication:
**21.01.2026 Bulletin 2026/04**

(21) Application number: **24770003.2**

(22) Date of filing: **14.03.2024**

(51) International Patent Classification (IPC):
***C07D 405/04*** (2006.01) ***C07D 405/14*** (2006.01)
***A61K 31/343*** (2006.01) ***A61K 31/4184*** (2006.01)
***A61K 31/404*** (2006.01) ***A61P 3/10*** (2006.01)

(52) Cooperative Patent Classification (CPC):
**A61K 31/343; A61K 31/404; A61K 31/4184; A61P 3/10; C07D 405/04; C07D 405/14**

(86) International application number:
**PCT/CN2024/081713**

(87) International publication number:
**WO 2024/188313 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.03.2023 CN 202310243275**
**07.03.2024 CN 202410263740**



(71) Applicant: **Cascade Pharmaceuticals, Inc.**
**Shanghai 201422 (CN)**

(72) Inventors:
- **SHI, Jingjing**
  **Shanghai 201422 (CN)**
- **GU, Yuefei**
  **Shanghai 201422 (CN)**
- **WU, Guohui**
  **Shanghai 201422 (CN)**
- **ZHANG, Zheng**
  **Shanghai 201422 (CN)**
- **GONG, Linpei**
  **Shanghai 201422 (CN)**
- **WANG, Peng**
  **Shanghai 201422 (CN)**
- **ZHU, Xin**
  **Shanghai 201422 (CN)**
- **DU, Xiaolong**
  **Shanghai 201422 (CN)**
- **YANG, Shengsheng**
  **Shanghai 201422 (CN)**
- **WANG, Gaihong**
  **Shanghai 201422 (CN)**
- **ZHAO, Guanguan**
  **Shanghai 201422 (CN)**
- **PAN, Hualing**
  **Shanghai 201422 (CN)**

(74) Representative: **Nederlandsch Octrooibureau**
**P.O. Box 29720**
**2502 LS The Hague (NL)**


(54) **BENZOHETEROCYCLIC COMPOUND, AND PREPARATION METHOD THEREFOR AND USE THEREOF**


(57) Disclosed in the present invention are a benzoheterocyclic compound, and a preparation method therefor and the use thereof. The compound has the general formula of formula I. The compound of the present invention has a good GPR40 agonistic activity and has good drug development prospects.


EP 4 682 145 A1

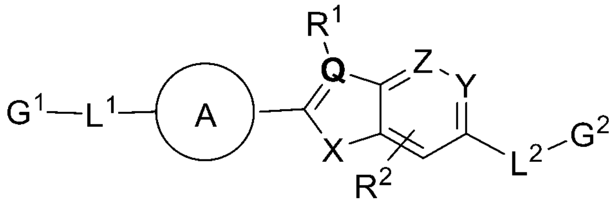

I

## Description

The present application claims the right of the priorities of Chinese patent application 2023102432757 filed on March 14, 2023 and Chinese patent application 2024102637408 filed on March 7, 2024. The contents of the above Chinese patent applications are incorporated herein by reference in their entireties.

TECHNICAL FIELD

The present disclosure relates to a benzoheterocyclic compound, a preparation method therefor, and a use thereof.

BACKGROUND

GPR40 is a member of the GPCR family, also known as the FFA1 receptor, and is a class A G protein-coupled receptor. It can be activated by endogenous medium- and long-chain fatty acids (such as capric acid, palmitic acid, oleic acid, and docosahexaenoic acid) *in vivo.* GPR40 is predominantly expressed at high levels in pancreatic β-cells, intestinal endocrine cells, and the brain, while also being expressed in tissues such as the gastrointestinal tract, liver, heart, skeletal muscle, and taste buds. When activated by its endogenous ligand, GPR40 induces insulin secretion only at higher blood glucose levels (activation of GPR40 promotes $Ca^{2+}$ influx in pancreatic β-cells, leading to insulin secretion), thereby eliminating the risk of hypoglycemia. Partial agonists of GPR40 activate the Gq/IP3 pathway to promote insulin secretion; full agonists additionally activate the Gs/cAMP pathway, stimulating the release of GLP-1 and GIP, thereby achieving a more potent glucose-lowering effect. These make GPR40 an important therapeutic target for diseases such as diabetes, obesity, cardiovascular disease, and dyslipidemia. The distribution of GPR40 in the brain may be related to pain regulation, neuroprotection, behavior regulation, and the like, and is a potential target for treating nervous system diseases.

Given the importance of GPR40, the development of drugs that can activate GPR40 is of great significance.

SUMMARY

The technical problem to be solved by the present disclosure is to overcome the limited types of drugs with GPR40 agonistic activity in the prior art. To this end, the present disclosure provides a benzoheterocyclic compound, a preparation method therefor, and a use thereof. The compounds of the present disclosure have good GPR40 agonistic activity, and further have the advantages of good pharmacokinetics and low toxicity.

The present disclosure solves the above technical problem through the following technical solutions.

The present disclosure provides a compound of formula I or a pharmaceutically acceptable salt thereof:

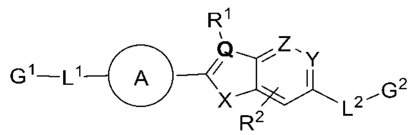

I

wherein X is O or $NR^3$, $R^3$ is H or $C_1$-$C_6$ alkyl; Q is C or N;

Z and Y are independently C or N;

$G^1$ is H, $C_1$-$C_6$ alkyl, $C_1$-$C_6$ alkyl substituted by one or more $G^{1-1}$, $C_6$-$C_{14}$ aryl, $C_6$-$C_{14}$ aryl substituted by one or more $G^{1-2}$, 5- to 10-membered heteroaryl, 5- to 10-membered heteroaryl substituted by one or more $G^{1-3}$, $C_1$-$C_6$ alkoxy, $C_1$-$C_6$ alkoxy substituted by one or more $G^{1-4}$, $C_2$-$C_6$ alkenyl, $C_2$-$C_6$ alkenyl substituted by one or more $G^{1-5}$, $C_2$-$C_6$ alkynyl, $C_2$-$C_6$ alkynyl substituted by one or more $G^{1-6}$, $C_3$-$C_8$ cycloalkyl, $C_3$-$C_8$ cycloalkyl substituted by one or more $G^{1-7}$, $C_3$-$C_8$ cycloalkenyl, $C_3$-$C_8$ cycloalkenyl substituted by one or more $G^{1-8}$, 3- to 8-membered heterocycloalkyl, 3- to 8-membered heterocycloalkyl substituted by one or more $G^{1-9}$, 3- to 8-membered heterocycloalkenyl, or 3- to 8-membered heterocycloalkenyl substituted by one or more $G^{1-10}$;

the 5- to 10-membered heteroaryl, the 5- to 10-membered heteroaryl substituted by one or more $G^{1-3}$, the 3- to 8-membered heterocycloalkyl, the 3- to 8-membered heterocycloalkyl substituted by one or more $G^{1-9}$, the 3- to 8-membered heterocycloalkenyl, and the 3- to 8-membered heterocycloalkenyl substituted by one or more $G^{1-10}$ have 1, 2, 3, or 4 heteroatoms selected from one or more types of N, S, and O;

each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$ is independently deuterium, halogen, cyano, $-NG^{1-1-1}G^{1-1-2}$, $-NC(=O)G^{1-1-3}G^{1-1-4}$, hydroxyl, $-S(=O)_2$-$C_1$-$C_6$ alkyl, $C_1$-$C_6$ alkyl, $C_1$-$C_6$ alkyl substituted by one or more $G^{1-1-5}$, $C_1$-$C_6$ alkoxy, $C_1$-$C_6$ alkoxy substituted by one or more

$G^{1-1-6}$, -S-$C_1$-$C_6$ alkyl, -S-$C_1$-$C_6$ alkyl substituted by one or more $G^{1-1-7}$, $C_3$-$C_8$ cycloalkyl, $C_3$-$C_8$ cycloalkyl substituted by one or more $G^{1-1-8}$, -O-$C_3$-$C_8$ cycloalkyl, -O-$C_3$-$C_8$ cycloalkyl substituted by one or more $G^{1-1-9}$, or -C(=O)N$G^{1-1-11}G^{1-1-12}$;

alternatively, any two adjacent $G^{1-2}$, together with the carbon atom to which they are attached, form a 3- to 8-membered heterocycloalkyl, a 3- to 8-membered heterocycloalkyl substituted by one or more $G^{1-1-9}$, a $C_3$-$C_8$ cycloalkyl, or a $C_3$-$C_8$ cycloalkyl substituted by one or more $G^{1-1-10}$;

$G^{1-1-1}$, $G^{1-1-2}$, $G^{1-1-3}$, $G^{1-1-4}$, $G^{1-1-11}$, and $G^{1-1-12}$ are independently H, $C_1$-$C_6$ alkyl, $C_3$-$C_8$ cycloalkyl, 5- to 10-membered heteroaryl, 5- to 10-membered heteroaryl substituted by one or more $0^{1-1-10-1}$, $C_6$-$C_{14}$ aryl, or $C_6$-$C_{14}$ aryl substituted by one or more $G^{1-1-10-2}$;

each $G^{1-1-10-1}$ and each $G^{1-1-10-2}$ is independently $C_1$-$C_6$ alkyl;

each $G^{1-1-5}$, each $G^{1-1-6}$, each $G^{1-1-7}$, each $G^{1-1-8}$, each $G^{1-1-9}$, and each $G^{1-1-10}$ is independently halogen, oxo, $C_1$-$C_6$ alkyl, $C_3$-$C_8$ cycloalkyl, 5- to 10-membered heteroaryl, or 5- to 10-membered heteroaryl substituted by one or more $C_1$-$C_6$ alkyl groups;

the 3- to 8-membered heterocycloalkyl, the 3- to 8-membered heterocycloalkyl substituted by one or more $G^{1-1-9}$, the 5- to 10-membered heteroaryl, the 5- to 10-membered heteroaryl substituted by one or more $G^{1-1-10-1}$, and the 5- to 10-membered heteroaryl substituted by one or more $C_1$-$C_6$ alkyl groups have 1, 2, 3, or 4 heteroatoms independently selected from one or more types of N, S, and O;

$L^1$ is a bond or $C_1$-$C_6$ alkylene;

ring A is $C_4$-$C_6$ cycloalkyl, $C_4$-$C_6$ cycloalkyl substituted by one or more $A^1$, $C_4$-$C_6$ cycloalkenyl, $C_4$-$C_6$ cycloalkenyl substituted by one or more $A^2$, 4- to 8-membered heterocycloalkyl, 4- to 8-membered heterocycloalkyl substituted by one or more $A^3$, 4-to 6-membered heterocycloalkenyl, or 4- to 6-membered heterocycloalkenyl substituted by one or more $A^4$;

the 4- to 8-membered heterocycloalkyl, the 4- to 8-membered heterocycloalkyl substituted by one or more $A^3$, the 4- to 6-membered heterocycloalkenyl, and the 4- to 6-membered heterocycloalkenyl substituted by one or more $A^4$ have 1 or 2 heteroatoms independently selected from one or more types of N, S, and O;

each $A^1$, each $A^2$, each $A^3$, and each $A^4$ is independently deuterium, halogen, cyano, -N$A^{1-1}A^{1-2}$, -NC(=O)$A^{1-3}A^{1-4}$, hydroxyl, $C_1$-$C_6$ alkyl, $C_1$-$C_6$ alkyl substituted by one or more $A^{1-5}$, $C_1$-$C_6$ alkoxy, or $C_1$-$C_6$ alkoxy substituted by one or more $A^{1-6}$;

$A^{1-1}$, $A^{1-2}$, $A^{1-3}$, and $A^{1-4}$ are independently deuterium, halogen, cyano, $C_1$-$C_6$ alkyl, or $C_1$-$C_6$ alkoxy;

each $A^{1-5}$ and each $A^{1-6}$ is independently hydrogen, deuterium, halogen, cyano, $C_1$-$C_6$ alkyl, or $C_1$-$C_6$ alkoxy;

$R^1$ is -C(=O)N$R^{1-1}R^{1-2}$, $C_1$-$C_6$ alkyl, $C_1$-$C_6$ alkyl substituted by one or more $R^{1-3}$ $C_2$-$C_6$ alkenyl, $C_2$-$C_6$ alkenyl substituted by one or more $R^{1-10}$, -C(=O)$R^{1-11}$, or ring B;

$R^{1-1}$, $R^{1-2}$, and $R^{1-11}$ are independently H, -S(=O)$_2C_1$-$C_{12}$ alkyl, $C_1$-$C_{12}$ alkyl, $C_1$-$C_{12}$ alkyl substituted by one or more $R^{1-1-1}$, $C_3$-$C_{12}$ cycloalkyl, $C_3$-$C_{12}$ cycloalkyl substituted by one or more $R^{1-1-2}$, $C_6$-$C_{14}$ aryl, $C_6$-$C_{14}$ aryl substituted by one or more $R^{1-1-3}$, 5- to 14-membered heteroaryl, or 5- to 14-membered heteroaryl substituted by one or more $R^{1-1-4}$, alternatively, $R^{1-1}$ and $R^{1-2}$, together with the N atom to which they are attached, form a 3- to 14-membered heterocycloalkyl or a 3- to 14-membered heterocycloalkyl substituted by one or more $R^{1-1-5}$ (wherein the heterocycloalkyl contains at least one N atom);

each $R^{1-1-1}$, each $R^{1-1-2}$, each $R^{1-1-3}$, $R^{1-1-4}$, and each $R^{1-1-5}$ is independently halogen, cyano, nitro, hydroxyl, amino, -NH($C_1$-$C_{12}$ alkyl), -N($C_1$-$C_{12}$ alkyl)$_2$, -C(=O)-$C_1$-$C_{12}$ alkyl, -NHC(=O)-$C_1$-$C_{12}$ alkyl, $C_1$-$C_{12}$ alkyl, $C_1$-$C_{12}$ alkyl substituted by one or more $R^{1-1-1-1}$, $C_1$-$C_{12}$ alkoxy, $C_1$-$C_{12}$ alkoxy substituted by one or more $R^{1-1-1-2}$, $C_3$-$C_{12}$ cycloalkyl, $C_3$-$C_{12}$ cycloalkyl substituted by one or more $R^{1-1-1-3}$, $C_6$-$C_{14}$ aryl, 3- to 12-membered heterocycloalkyl, or 5- to 14-membered heteroaryl;

each $R^{1-1-1-1}$, each $R^{1-1-1-2}$, and each $R^{1-1-1-3}$ is independently halogen, $C_1$-$C_{12}$ alkyl, $C_3$-$C_{12}$ cycloalkyl, or $C_1$-$C_{12}$ alkoxy;

each $R^{1-3}$ and each $R^{1-10}$ is independently deuterium, halogen, cyano, hydroxyl, - N$R^{1-3-1}R^{1-3-2}$, -C(=O)N$R^{1-3-3}R^{1-3-4}$, -C(=O)$R^{1-3-5}$, -S(=O)$_2$-$C_1$-$C_{12}$ alkyl, -S-$C_1$-$C_{12}$ alkyl, $C_1$-$C_{12}$ alkyl, $C_1$-$C_{12}$ alkyl substituted by one or more $R^{1-3-6}$, $C_1$-$C_{12}$ alkoxy, $C_1$-$C_{12}$ alkoxy substituted by one or more $R^{1-3-7}$, 5- to 14-membered heteroaryl, 5- to 14-membered heteroaryl substituted by one or more $R^{1-3-8}$, $C_3$-$C_{12}$ cycloalkyl, $C_3$-$C_{12}$ cycloalkyl substituted by one or more $R^{1-3-9}$, $C_2$-$C_6$ alkenyl, $C_2$-$C_6$ alkenyl substituted by one or more $R^{1-3-10}$, $C_6$-$C_{14}$ aryl, $C_6$-$C_{14}$ aryl substituted by one or more $R^{1-3-11}$, -O-$C_6$-$C_{14}$ aryl, -O-C(=O)$C_6$-$C_{14}$ aryl, -O-5- to 14-membered heteroaryl, $C_3$-$C_8$ cycloalkenyl, $C_3$-$C_8$ cycloalkenyl substituted by one or more $R^{1-3-12}$, 3- to 12-membered heterocycloalkyl, or 3- to 12-membered heterocycloalkyl substituted by one or more $R^{1-3-13}$;

$R^{1-3-1}$, $R^{1-3-2}$, $R^{1-3-3}$, and $R^{1-3-4}$ are independently H, hydroxyl, $C_1$-$C_6$ alkyl, -$C_1$-$C_6$ alkyl-$C_6$-$C_{14}$ aryl, $C_1$-$C_6$ alkoxy, -C(=O)$R^{1-3-1-1}$, $C_3$-$C_8$ cycloalkyl, $C_6$-$C_{14}$ aryl, or $C_6$-$C_{14}$ aryl substituted by one or more $R^{1-3-1-4}$

alternatively, $R^{1-3-1}$ and $R^{1-3-2}$, together with the N atom to which they are attached, form a 3- to 8-membered heterocycloalkyl or a 3- to 8-membered heterocycloalkyl substituted by one or more $R^{1-3-1-2,}$

alternatively, $R^{1-3-3}$ and $R^{1-3-4}$, together with the N atom to which they are attached, form a 3- to 8-membered

heterocycloalkyl or a 3- to 8-membered heterocycloalkyl substituted by one or more $R^{1-3-3-1}$,

each $R^{1-3-1-1}$, each $R^{1-3-1-2}$, each $R^{1-3-1-4}$, and each $R^{1-3-3-1}$ is independently halogen or $C_1$-$C_6$ alkyl;

$R^{1-3-5}$ is independently H, hydroxyl, $C_1$-$C_6$ alkyl, $C_1$-$C_6$ alkoxy, or $C_3$-$C_8$ cycloalkyl;

each $R^{1-3-6}$, each $R^{1-3-7}$, each $R^{1-3-9}$, each $R^{1-3-10}$, each $R^{1-3-12}$, and each $R^{1-3-13}$ is independently halogen, hydroxyl, carboxyl, cyano, -C(=O)-O-$C_1$-$C_6$ alkyl, -C(=O)-N($C_1$-$C_6$ alkyl)$_2$, -C(=O)-N($C_1$-$C_6$ alkyl)$_2$, $C_1$-$C_6$ alkyl, $C_1$-$C_6$ alkoxy, $C_3$-$C_8$ cycloalkyl, 3- to 8-membered heterocycloalkyl, $C_2$-$C_6$ alkenyl, $C_6$-$C_{14}$ aryl, $C_6$-$C_{14}$ aryl substituted by one or more $C_1$-$C_6$ alkyl groups, 5- to 10-membered heteroaryl, or 5- to 10-membered heteroaryl substituted by one or more $C_1$-$C_6$ alkyl groups;

each $R^{1-3-8}$ and each $R^{1-3-11}$ is independently halogen, hydroxyl, carboxyl, cyano, -C(=O)-O-$C_1$-$C_6$ alkyl, -C(=O)-N($C_1$-$C_6$ alkyl)$_2$, -C(=O)-N($C_1$-$C_6$ alkyl)$_2$, $C_1$-$C_6$ alkyl, $C_1$-$C_6$ alkoxy, $C_3$-$C_8$ cycloalkyl, 3- to 8-membered heterocycloalkyl, or $C_2$-$C_6$ alkenyl;

the 5- to 14-membered heteroaryl, the 5- to 14-membered heteroaryl substituted by one or more $R^{1-1-4}$, the 3- to 14-membered heterocycloalkyl, the 3- to 14-membered heterocycloalkyl substituted by one or more $R^{1-1-5}$, the 3- to 12-membered heterocycloalkyl, the 3- to 8-membered heterocycloalkyl, the 3- to 8-membered heterocycloalkyl substituted by one or more $R^{1-3-1-2}$, the 3- to 8-membered heterocycloalkyl substituted by one or more $R^{1-3-3-1}$, the 3- to 8-membered heterocycloalkyl substituted by one or more $R^{1-3-3-1}$, and the 5- to 10-membered heteroaryl substituted by one or more $C_1$-$C_6$ alkyl groups have 1, 2, 3, or 4 heteroatoms independently selected from one or more types of N, S, and O;

ring B is $C_3$-$C_{12}$ cycloalkyl, $C_3$-$C_{12}$ cycloalkyl substituted by one or more $R^{1-4}$, $C_3$-$C_{12}$ cycloalkenyl, $C_3$-$C_{12}$ cycloalkenyl substituted by one or more $R^{1-5}$, 3- to 12-membered heterocycloalkyl, 3- to 12-membered heterocycloalkyl substituted by one or more $R^{1-6}$, 3- to 12-membered heterocycloalkenyl, 3- to 12-membered heterocycloalkenyl substituted by one or more $R^{1-7}$, $C_6$-$C_{14}$ aryl, $C_6$-$C_{14}$ aryl substituted by one or more $R^{1-8}$ 5- to 14-membered heteroaryl, or 5- to 14-membered heteroaryl substituted by one or more $R^{1-9}$;

each $R^{1-4}$, each $R^{1-5}$, each $R^{1-6}$, each $R^{1-7}$, each $R^{1-8}$, and each $R^{1-9}$ is independently deuterium, halogen, cyano, hydroxyl, -$NR^{1-3-1a}R^{1-3-2a}$, -C(=O)$NR^{1-3-3a}R^{1-3-4a}$, -C(=O)$R^{1-3-5a}$, -S(=O)$_2$-$C_1$-$C_{12}$ alkyl, -S-$C_1$-$C_{12}$ alkyl, $C_1$-$C_{12}$ alkyl, $C_1$-$C_{12}$ alkyl substituted by one or more $R^{1-3-6a}$, $C_1$-$C_{12}$ alkoxy, $C_1$-$C_{12}$ alkoxy substituted by one or more $R^{1-3-7a}$, 5- to 14-membered heteroaryl, 5- to 14-membered heteroaryl substituted by one or more $R^{1-3-8a}$, $C_3$-$C_{12}$ cycloalkyl, $C_3$-$C_{12}$ cycloalkyl substituted by one or more $R^{1-3-9a}$, $C_2$-$C_6$ alkenyl, $C_2$-$C_6$ alkenyl substituted by one or more $R^{1-3-10a}$, $C_6$-$C_{14}$ aryl, $C_6$-$C_{14}$ aryl substituted by one or more $R^{1-3-11a}$, -O-$C_6$-$C_{14}$ aryl, -O-C(=O)$C_6$-$C_{14}$ aryl, -O-5- to 10-membered heteroaryl, $C_3$-$C_8$ cycloalkenyl, $C_3$-$C_8$ cycloalkenyl substituted by one or more $R^{1-3-12a}$, 3-to 12-membered heterocycloalkyl, or 3- to 12-membered heterocycloalkyl substituted by one or more $R^{1-3-13a}$;

alternatively, any two adjacent $R^{1-8}$, together with the carbon atom to which they are attached, form a 3- to 8-membered heterocycloalkyl, a 3- to 8-membered heterocycloalkyl substituted by one or more $R^{1-8-1}$, or a $C_3$-$C_{14}$ cycloalkyl;

$R^{1-3-1a}$, $R^{1-3-2a}$, $R^{1-3-3a}$, and $R^{1-3-4a}$ are independently H, hydroxyl, $C_1$-$C_6$ alkyl, $C_1$-$C_6$ alkoxy, -C(=O)$R^{1-3-1-1a}$, or $C_3$-$C_8$ cycloalkyl,

alternatively, $R^{1-3-1a}$ and $R^{1-3-2a}$, together with the N atom to which they are attached, form a 3- to 8-membered heterocycloalkyl or a 3- to 8-membered heterocycloalkyl substituted by one or more $R^{1-3-1-2a}$,

alternatively, $R^{1-3-3a}$ and $R^{1-3-4a}$, together with the N atom to which they are attached, form a 3- to 8-membered heterocycloalkyl or a 3- to 8-membered heterocycloalkyl substituted by one or more $R^{1-3-3-1a}$,

each $R^{1-3-1-1a}$, each $R^{1-3-1-2a}$, and each $R^{1-3-3-1a}$ is independently halogen or $C_1$-$C_6$ alkyl;

$R^{1-3-5a}$ is independently H, hydroxyl, $C_1$-$C_6$ alkyl, $C_1$-$C_6$ alkoxy, $C_3$-$C_8$ cycloalkyl, or 3- to 8-membered heterocycloalkyl;

each $R^{1-3-6a}$, each $R^{1-3-7a}$, each $R^{1-3-8a}$, each $R^{1-3-9a}$, each $R^{1-3-10a}$, each $R^{1-3-11a}$, each $R^{1-3-12a}$, each $R^{1-3-13a}$, and each $R^{1-8-1}$ is independently halogen, hydroxyl, carboxyl, cyano, -C(=O)-O-$C_1$-$C_6$ alkyl, -C(=O)-NH-$C_1$-$C_6$ alkyl, -C(=O)-N($C_1$-$C_6$ alkyl)$_2$, $C_1$-$C_6$ alkyl, $C_1$-$C_6$ alkoxy, $C_3$-$C_8$ cycloalkyl, 3- to 12-membered heterocycloalkyl, $C_2$-$C_6$ alkenyl, or - O-C(=O)-$C_1$-$C_6$ alkyl-3- to 12-membered heterocycloalkyl;

the 3- to 8-membered heterocycloalkyl, the 3- to 12-membered heterocycloalkyl, the 3- to 12-membered heterocycloalkenyl, the 5- to 10-membered heteroaryl, the 5- to 14-membered heteroaryl, the 3- to 12-membered heterocycloalkyl substituted by one or more $R^{1-6}$, the 3- to 12-membered heterocycloalkenyl substituted by one or more $R^{1-7}$, the 5- to 14-membered heteroaryl substituted by one or more $R^{1-9}$, the 5- to 14-membered heteroaryl substituted by one or more $R^{1-3-8a}$, the 3- to 12-membered heterocycloalkyl substituted by one or more $R^{1-3-13a}$, the 3- to 8-membered heterocycloalkyl substituted by one or more $R^{1-8-1}$, the 3- to 8-membered heterocycloalkyl substituted by one or more $R^{1-3-1-2a}$, the 3- to 8-membered heterocycloalkyl substituted by one or more $R^{1-3-3-1a}$, the 3-to 8-membered heterocycloalkyl substituted by one or more $R^{1-3-3-1a}$, the -O-5- to 10-membered heteroaryl, and the -O-C(=O)-$C_1$-$C_6$ alkyl-3- to 12-membered heterocycloalkyl have 1, 2, 3, or 4 heteroatoms independently selected from one or more types of N, S, and O;

$R^2$ is hydrogen, deuterium, halogen, cyano, $C_1$-$C_6$ alkyl, or $C_1$-$C_6$ alkoxy;

$L^2$ is a bond, $C_1$-$C_6$ alkylene, $C_1$-$C_6$ alkylene substituted by one or more $L^{2-1}$, $C_3$-$C_8$ cycloalkylene, $C_3$-$C_8$ cycloalkylene substituted by one or more $L^{2-2}$, -O-$C_1$-$C_6$ alkylene, -NH-$C_1$-$C_6$ alkylene, or -N($C_1$-$C_6$ alkyl)-$C_1$-$C_6$ alkylene;

each $L^{2-1}$ and each $L^{2-2}$ is independently halogen, $C_1$-$C_6$ alkyl, $C_1$-$C_6$ alkyl substituted by one or more $L^{2-1-1}$, $C_1$-$C_6$ alkoxy, $C_1$-$C_6$ alkoxy substituted by one or more $L^{2-1-2}$, $C_3$-$C_8$ cycloalkyl, $C_3$-$C_8$ cycloalkyl substituted by one or more $L^{2-1-3}$, $C_2$-$C_6$ alkynyl, or $C_2$-$C_6$ alkynyl substituted by one or more $L^{2-1-4}$;

each $L^{2-1-1}$, each $L^{2-1-2}$, each $L^{2-1-3}$, and each $L^{2-1-4}$ is independently $C_3$-$C_8$ cycloalkyl or $C_3$-$C_8$ cycloalkyl substituted by one or more $L^{2-1-1-1}$;

each $L^{2-1-1-1}$ is independently halogen or $C_1$-$C_6$ alkyl;

$G^2$ is H, -C(=O)$G^{2-1}$, -C(=O)N$G^{2-2}G^{2-3}$, 5- to 10-membered heteroaryl, 5- to 10-membered heteroaryl substituted by one or more $G^{2-4}$, -S(=O)$_2$-OH, -P(=O)-(OH)$_2$, - P(=O)-(O$C_1$-$C_6$ alkyl)(OH), 3- to 8-membered heterocycloalkenyl, or 3- to 8-membered heterocycloalkenyl substituted by one or more $G^{2-5}$;

$G^{2-1}$ is hydroxyl, $C_1$-$C_6$ alkyl, or -O-$NH_2$;

$G^{2-2}$ and $G^{2-3}$ are independently H, -S(=O)$_2$-$C_1$-$C_6$ alkyl, $C_3$-$C_8$ cycloalkyl, $C_1$-$C_6$ alkyl, $C_1$-$C_6$ alkyl substituted by one or more $G^{2-2-1}$, or -NH(=O)-5- to 10-membered heteroaryl;

each $G^{2-2-1}$ is independently carboxyl or -S(=O)$_2$OH;

each $G^{2-4}$ and each $G^{2-5}$ is independently hydroxyl or oxo;

the 5- to 10-membered heteroaryl, the 5- to 10-membered heteroaryl substituted by one or more $G^{2-4}$, the 3- to 8-membered heterocycloalkenyl, the 3- to 8-membered heterocycloalkenyl substituted by one or more $G^{2-5}$, and the -NH(=O)-5- to 10-membered heteroaryl have 1, 2, 3, or 4 heteroatoms independently selected from one or more types of N, S, and O.

the above heteroaryl, heterocycloalkenyl, and heterocycloalkyl may have 1, 2, 3, or 4 heteroatoms independently selected from one or more types of N, S, and O.

The definitions of some groups in the compound of formula I or the pharmaceutically acceptable salt thereof are as described below, while the definitions of the remaining groups are as described in any other embodiment.

In one embodiment, the compound of formula I or the pharmaceutically acceptable salt thereof:

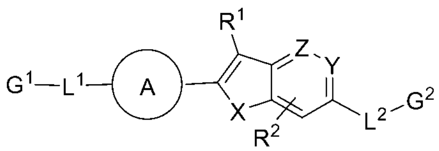

**Ia**

wherein X is O or $NR^3$, and $R^3$ is H or $C_1$-$C_6$ alkyl;

Z and Y are independently C or N;

$G^1$ is H, $C_1$-$C_6$ alkyl, $C_1$-$C_6$ alkyl substituted by one or more $G^{1-1}$, $C_6$-$C_{14}$ aryl, $C_6$-$C_{14}$ aryl substituted by one or more $G^{1-2}$, 5- to 10-membered heteroaryl, 5- to 10-membered heteroaryl substituted by one or more $G^{1-3}$, $C_1$-$C_6$ alkoxy, $C_1$-$C_6$ alkoxy substituted by one or more $G^{1-4}$, $C_2$-$C_6$ alkenyl, $C_2$-$C_6$ alkenyl substituted by one or more $G^{1-5}$, $C_2$-$C_6$ alkynyl, $C_2$-$C_6$ alkynyl substituted by one or more $G^{1-6}$, $C_3$-$C_8$ cycloalkyl, $C_3$-$C_8$ cycloalkyl substituted by one or more $G^{1-7}$, $C_3$-$C_8$ cycloalkenyl, $C_3$-$C_8$ cycloalkenyl substituted by one or more $G^{1-8}$, 3- to 8-membered heterocycloalkyl, 3- to 8-membered heterocycloalkyl substituted by one or more $G^{1-9}$, 3- to 8-membered heterocycloalkenyl, or 3- to 8-membered heterocycloalkenyl substituted by one or more $G^{1-10}$;

each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$ is independently deuterium, halogen, cyano, -N$G^{1-1-1}G^{1-1-2}$, -NC(=O)$G^{1-1-3}G^{1-1-4}$, hydroxyl, -S(=O)$_2$-$C_1$-$C_6$ alkyl, $C_1$-$C_6$ alkyl, $C_1$-$C_6$ alkyl substituted by one or more $G^{1-1-5}$, $C_1$-$C_6$ alkoxy, $C_1$-$C_6$ alkoxy substituted by one or more $G^{1-1-6}$, -S-$C_1$-$C_6$ alkyl, -S-$C_1$-$C_6$ alkyl substituted by one or more $G^{1-1-7}$, $C_3$-$C_8$ cycloalkyl, $C_3$-$C_8$ cycloalkyl substituted by one or more $G^{1-1-8}$, -O-$C_3$-$C_8$ cycloalkyl, -O-$C_3$-$C_8$ cycloalkyl substituted by one or more $G^{1-1-9}$, or -C(=O) N$G^{1-1-11}G^{1-1-12}$;

alternatively, any two adjacent $G^{1-2}$, together with the carbon atom to which they are attached, form a 3- to 8-membered heterocycloalkyl, a 3- to 8-membered heterocycloalkyl substituted by one or more $G^{1-1-9}$, a $C_3$-$C_8$ cycloalkyl, or a $C_3$-$C_8$ cycloalkyl substituted by one or more $G^{1-1-10}$;

$G^{1-1-1}$, $G^{1-1-2}$, $G^{1-1-3}$, $G^{1-1-4}$, $G^{1-1-11}$, and $G^{1-1-12}$ are independently H, $C_1$-$C_6$ alkyl, $C_3$-$C_8$ cycloalkyl, 5- to 10-membered heteroaryl, or 5- to 10-membered heteroaryl substituted by one or more $G^{1-1-10-1}$;

each $G^{1-1-10-1}$ is independently $C_1$-$C_6$ alkyl;

each $G^{1-1-5}$, each $G^{1-1-6}$, each $G^{1-1-7}$, each $G^{1-1-8}$, each $G^{1-1-9}$, and each $G^{1-1-10}$ is independently halogen, oxo, $C_1$-$C_6$ alkyl, or $C_3$-$C_8$ cycloalkyl;

$L^1$ is a bond or $C_1$-$C_6$ alkylene;

ring A is $C_4$-$C_6$ cycloalkyl, $C_4$-$C_6$ cycloalkyl substituted by one or more $A^1$, $C_4$-$C_6$ cycloalkenyl, $C_4$-$C_6$ cycloalkenyl substituted by one or more $A^2$, 4- to 6-membered heterocycloalkyl, 4- to 6-membered heterocycloalkyl substituted by one or more $A^1$, 4-to 6-membered heterocycloalkenyl, or 4- to 6-membered heterocycloalkenyl substituted by one or more $A^4$; the 4- to 6-membered heterocycloalkyl, the 4- to 6-membered heterocycloalkyl substituted by one or more $A^1$, the 4- to 6-membered heterocycloalkenyl, and the 4- to 6-membered heterocycloalkenyl substituted by one or more $A^4$ have 1 or 2 heteroatoms independently selected from one or more types of N, S, and O;

each $A^1$, each $A^2$, each $A^3$, and each $A^4$ is independently deuterium, halogen, cyano, $-NA^{1-1}A^{1-2}$, $-NC(=O)A^{1-3}A^{1-4}$, hydroxyl, $C_1$-$C_6$ alkyl, $C_1$-$C_6$ alkyl substituted by one or more $A^{1-5}$, $C_1$-$C_6$ alkoxy, or $C_1$-$C_6$ alkoxy substituted by one or more $A^{1-6}$;

$A^{1-1}$, $A^{1-2}$, $A^{1-3}$, and $A^{1-4}$ are independently deuterium, halogen, cyano, $C_1$-$C_6$ alkyl, or $C_1$-$C_6$ alkoxy;

each $A^{1-5}$ and each $A^{1-6}$ is independently hydrogen, deuterium, halogen, cyano, $C_1$-$C_6$ alkyl, or $C_1$-$C_6$ alkoxy;

$R^1$ is $-C(=O)NR^{1-1}R^{1-2}$, $C_1$-$C_6$ alkyl substituted by one or more $R^{1-3}$, or ring B;

$R^{1-1}$ and $R^{1-2}$ are independently H, $C_1$-$C_6$ alkyl, $C_1$-$C_6$ alkyl substituted by one or more $R^{1-1-1}$, $C_3$-$C_{10}$ cycloalkyl, $C_3$-$C_{10}$ cycloalkyl substituted by one or more $R^{1-1-2}$, $C_6$-$C_{14}$ aryl, $C_6$-$C_{14}$ aryl substituted by one or more $R^{1-1-3}$, 5- to 10-membered heteroaryl, or 5- to 10-membered heteroaryl substituted by one or more $R^{1-1-4}$;

each $R^{1-1-1}$, each $R^{1-1-2}$, each $R^{1-1-3}$, and $R^{1-1-4}$ is independently halogen, $C_1$-$C_6$ alkyl, or $C_3$-$C_8$ cycloalkyl;

ring B is $C_3$-$C_8$ cycloalkyl, $C_3$-$C_8$ cycloalkyl substituted by one or more $R^{1-4}$, $C_3$-$C_8$ cycloalkenyl, $C_3$-$C_8$ cycloalkenyl substituted by one or more $R^{1-5}$, 3- to 8-membered heterocycloalkyl, 3- to 8-membered heterocycloalkyl substituted by one or more $R^{1-6}$, 3-to 8-membered heterocycloalkenyl, 3- to 8-membered heterocycloalkenyl substituted by one or more $R^{1-7}$, $C_6$-$C_{14}$ aryl, $C_6$-$C_{14}$ aryl substituted by one or more $R^{1-8}$, 5- to 10-membered heteroaryl, or 5- to 10-membered heteroaryl substituted by one or more $R^{1-9}$;

each $R^{1-3}$, each $R^{1-4}$, each $R^{1-5}$, each $R^{1-6}$, each $R^{1-7}$, each $R^{1-8}$, and each $R^{1-9}$ is independently deuterium, halogen, cyano, hydroxyl, $-NR^{1-3-1}R^{1-3-2}$, $-C(=O)NR^{1-3-3}R^{1-3-4}$, $-C(=O)R^{1-3-5}$, $-S(-O)_2$-$C_1$-$C_6$ alkyl, -S-$C_1$-$C_6$ alkyl, $C_1$-$C_6$ alkyl, $C_1$-$C_6$ alkyl substituted by one or more $R^{1-3-6}$, $C_1$-$C_6$ alkoxy, $C_1$-$C_6$ alkoxy substituted by one or more $R^{1-3-7}$, 5- to 10-membered heteroaryl, 5- to 10-membered heteroaryl substituted by one or more $R^{1-3-8}$, $C_3$-$C_8$ cycloalkyl, $C_3$-$C_8$ cycloalkyl substituted by one or more $R^{1-3-9}$, $C_2$-$C_6$ alkenyl, or $C_2$-$C_6$ alkenyl substituted by one or more $R^{1-3-10}$;

alternatively, any two adjacent $R^{1-8}$, together with the carbon atom to which they are attached, form a 3- to 8-membered heterocycloalkyl or a 3- to 8-membered heterocycloalkyl substituted by one or more $R^{1-8-1}$;

$R^{1-3-1}$, $R^{1-3-2}$, $R^{1-3-3}$, and $R^{1-3-4}$ are independently H, hydroxyl, $C_1$-$C_6$ alkyl, $C_1$-$C_6$ alkoxy, $-C(=O)R^{1-3-1-1}$, or $C_3$-$C_8$ cycloalkyl,

alternatively, $R^{1-3-1}$ and $R^{1-3-2}$, together with the N atom to which they are attached, form a 3- to 8-membered heterocycloalkyl or a 3- to 8-membered heterocycloalkyl substituted by one or more $R^{1-3-1-2}$,

alternatively, $R^{1-3-3}$ and $R^{1-3-4}$, together with the N atom to which they are attached, form a 3- to 8-membered heterocycloalkyl or a 3- to 8-membered heterocycloalkyl substituted by one or more $R^{1-3-3-1}$,

$R^{1-3-1-1}$ and each $R^{1-3-1-1}$ is independently $C_1$-$C_6$ alkyl or 5- to 10-membered heteroaryl;

$R^{1-3-5}$ is independently H, hydroxyl, $C_1$-$C_6$ alkyl, $C_1$-$C_6$ alkoxy, or $C_3$-$C_8$ cycloalkyl;

each $R^{1-3-6}$, each $R^{1-3-7}$, each $R^{1-3-8}$, each $R^{1-3-9}$, and each $R^{1-3-10}$ is independently halogen, hydroxyl, carboxyl, -C(=O)-O-$C_1$-$C_6$ alkyl, -C(=O)-NH-$C_1$-$C_6$ alkyl, $C_1$-$C_6$ alkyl, $C_1$-$C_6$ alkoxy, $C_3$-$C_8$ cycloalkyl, or 3- to 8-membered heterocycloalkyl;

$R^2$ is hydrogen, deuterium, halogen, cyano, $C_1$-$C_6$ alkyl, or $C_1$-$C_6$ alkoxy;

$L^2$ is $C_1$-$C_6$ alkylene, $C_1$-$C_6$ alkylene substituted by one or more $L^{2-1}$, $C_3$-$C_8$ cycloalkylene, or $C_3$-$C_8$ cycloalkylene substituted by one or more $L^{2-2}$;

each $L^{2-1}$ and each $L^{2-2}$ is independently halogen, $C_1$-$C_6$ alkyl, $C_1$-$C_6$ alkyl substituted by one or more $L^{2-1-1}$, $C_1$-$C_6$ alkoxy, $C_1$-$C_6$ alkoxy substituted by one or more $L^{2-1-2}$, $C_3$-$C_8$ cycloalkyl, $C_3$-$C_8$ cycloalkyl substituted by one or more $L^{2-1-3}$, $C_2$-$C_6$ alkynyl, or $C_2$-$C_6$ alkynyl substituted by one or more $L^{2-1-4}$;

each $L^{2-1-1}$, each $L^{2-1-2}$, each $L^{2-1-3}$, and each $L^{2-1-4}$ is independently $C_3$-$C_8$ cycloalkyl or $C_3$-$C_8$ cycloalkyl substituted by one or more $L^{2-1-1-1}$;

$G^2$ is $-C(=O)G^{2-1}$, $-C(=O)NG^{2-2}G^{2-3}$, 5- to 10-membered heteroaryl, or 5- to 10-membered heteroaryl substituted by one or more $G^{2-4}$;

$G^{2-1}$ is hydroxyl, $C_1$-$C_6$ alkyl, or $-O-NH_2$;

$G^{2-2}$ and $G^{2-3}$ are independently H, $-S(=O)_2$-$C_1$-$C_6$ alkyl, $C_3$-$C_8$ cycloalkyl, $C_1$-$C_6$ alkyl, or $C_1$-$C_6$ alkyl substituted by one or more $G^{2-2-1}$;

each $G^{2-2-1}$ is independently carboxyl or $-S(=O)_2OH$;

each 5- to 10-membered heteroaryl, each 3- to 8-membered heterocycloalkenyl, and each 3- to 8-membered heterocycloalkyl has 1, 2, 3, or 4 heteroatoms independently selected from one or more types of N, S, and O.

In one embodiment, in $R^3$ and $G^1$, the "$C_1$-$C_6$ alkyl" in the $C_1$-$C_6$ alkyl and the $C_1$-$C_6$ alkyl substituted by one or more $G^{1-1}$ may independently be methyl, ethyl, n-propyl, isopropyl, n-butyl, isobutyl, sec-butyl, or *tert-butyl,* such as methyl.

In one embodiment, in $G^1$, the "$C_6$-$C_{14}$ aryl" in the $C_6$-$C_{14}$ aryl and the $C_6$-$C_{14}$ aryl substituted by one or more $G^{1-2}$ may independently be phenyl or naphthyl.

In one embodiment, in $G^1$, the "5- to 10-membered heteroaryl" in the 5- to 10-membered heteroaryl and the 5- to 10-membered heteroaryl substituted by one or more $G^{1-3}$ may independently be 5-, 6-, 9-, or 10-membered monocyclic or bicyclic heteroaryl with 1 or 2 heteroatoms independently selected from one or more types of N, S, and O, and may further be pyridyl (

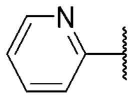 or 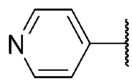

), thiazolyl (

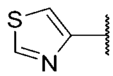 or 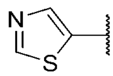

), furanophenyl (

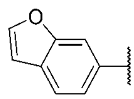

), or oxazolophenyl (

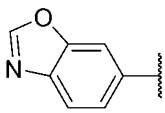

).

In one embodiment, in each $G^{1-1}$, each $G^{1-2}$, each $G^{1-3}$, each $G^{1-4}$, each $G^{1-5}$, each $G^{1-6}$, each $G^{1-7}$ , each $G^{1-8}$, each $G^{1-9}$, each $G^{1-10}$ , and each $G^{1-11}$, the halogen may independently be fluorine, chlorine, or bromine, such as fluorine or chlorine.

In one embodiment, in each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$, the "$C_1$-$C_6$ alkyl" in the -S(=O)$_2$-$C_1$-$C_6$ alkyl, the $C_1$-$C_6$ alkyl, the $C_1$-$C_6$ alkyl substituted by one or more $G^{1-1-5}$, the -S-$C_1$-$C_6$ alkyl, and the -S-$C_1$-$C_6$ alkyl substituted by one or more $G^{1-1-7}$ may independently be methyl, ethyl, n-propyl, isopropyl, *n*-butyl, isobutyl, *sec*-butyl, or *tert*-butyl, such as methyl or ethyl.

In one embodiment, in each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$, the "$C_1$-$C_6$ alkoxy" in the $C_1$-$C_6$ alkoxy and the $C_1$-$C_6$ alkoxy substituted by one or more $G^{1-1-6}$ may independently be methoxy, ethoxy, n-propoxy, isopropoxy, n-butoxy, isobutoxy, sec-butoxy, or tert-butoxy, such as methoxy or ethoxy.

In one embodiment, in each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$, the "$C_3$-$C_8$ cycloalkyl" in the $C_3$-$C_8$ cycloalkyl, the $C_3$-$C_8$ cycloalkyl substituted by one or more $G^{1-1-8}$, the -O-$C_3$-$C_8$ cycloalkyl, and the -O-$C_3$-$C_8$ cycloalkyl substituted by one or more $G^{1-9}$ may independently be cyclopropyl, cyclobutyl, cyclopentyl, or cyclohexyl, such as cyclopropyl.

In one embodiment, the "3- to 8-membered heterocycloalkyl" formed by any two adjacent $G^{1-2}$ together with the carbon atom to which they are attached and the "3- to 8-membered heterocycloalkyl" in the 3- to 8-membered heterocycloalkyl substituted by one or more $G^{1-1-9}$ may independently be 5- to 6-membered heterocycloalkyl with 1 or 2 heteroatoms independently being N and/or O, such as

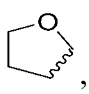, 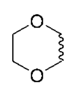, or 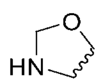,

where 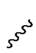 indicates that the group forms a fused ring with the $C_6$-$C_{14}$ aryl through this bond.

In one embodiment, the "$C_3$-$C_8$ cycloalkyl" formed by any two adjacent $G^{1-2}$ together with the carbon atom to which they are attached and the "$C_3$-$C_8$ cycloalkyl" in the $C_3$-$C_8$ cycloalkyl substituted by one or more $G^{1-1-10}$ may independently be $C_3$-$C_6$ cycloalkyl, such as

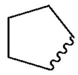,

where 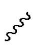 indicates that the group forms a fused ring with the $C_6$-$C_{14}$ aryl through this bond.

**In** one embodiment, in $G^{1-1-1}$, $G^{1-1-2}$, $G^{1-1-3}$, $G^{1-1-4}$, $G^{1-1-11}$, and $G^{1-1-12}$, the $C_1$-$C_6$ alkyl may independently be methyl, ethyl, n-propyl, isopropyl, n-butyl, isobutyl, *sec*-butyl, *tert-butyl,* or n-hexyl, such as methyl, *tert-butyl,* or n-hexyl.

**In** one embodiment, in $G^{1-1-1}$, $G^{1-1-2}$, $G^{1-1-3}$, $G^{1-1-4}$, $G^{1-1-11}$, and $G^{1-1-12}$, the $C_3$-$C_8$ cycloalkyl may independently be cyclopropyl, cyclobutyl, cyclopentyl, or cyclohexyl.

**In** one embodiment, in $G^{1-1-1}$, $G^{1-1-2}$, $G^{1-1-3}$, $G^{1-1-4}$, $G^{1-1-11}$, and $G^{1-1-12}$, the "5- to 10-membered heteroaryl" in the 5- to 10-membered heteroaryl and the 5- to 10-membered heteroaryl substituted by one or more $G^{1-1-10-1}$ may independently be 5- to 6-membered heteroaryl with 1 or 2 heteroatoms being N, such as pyridyl.

In one embodiment, in each $G^{1-1-10-1}$ and each $G^{1-1-10-2}$, the "$C_1$-$C_6$ alkyl" in the $C_1$-$C_6$ alkyl may independently be methyl, ethyl, n-propyl, isopropyl, n-butyl, isobutyl, sec-butyl, or *tert-butyl,* such as methyl.

In one embodiment, in each $G^{1-1-5}$, each $G^{1-1-6}$, each $G^{1-1-7}$, each $G^{1-1-8}$, each $G^{1-1-9}$, and each $G^{1-1-10}$, the halogen may independently be fluorine, chlorine, or bromine, such as fluorine.

In one embodiment, in each $G^{1-1-5}$, each $G^{1-1-6}$, each $G^{1-1-7}$, each $G^{1-1-8}$, each $G^{1-1-9}$, and each $G^{1-1-10}$, the $C_3$-$C_8$ cycloalkyl may independently be cyclopropyl, cyclobutyl, cyclopentyl, or cyclohexyl, such as cyclopropyl.

In one embodiment, in each $G^{1-1-5}$, each $G^{1-1-6}$, each $G^{1-1-7}$, each $G^{1-1-8}$, each $G^{1-1-9}$, and each $G^{1-1-10}$, the "5- to 10-membered heteroaryl" in the 5- to 10-membered heteroaryl and the 5- to 10-membered heteroaryl substituted by one or more $C_1$-$C_6$ alkyl groups may be 5-, 6-, 9-, or 10-membered monocyclic or bicyclic heteroaryl with 1, 2, or 3 heteroatoms being N, such as triazolyl (*e.g.*,

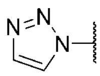

).

In one embodiment, in $G^1$, the "$C_1$-$C_6$ alkyl" in the $C_1$-$C_6$ alkyl substituted by one or more $G^{1-1}$ may be

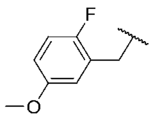, 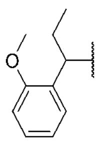, 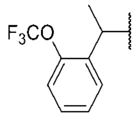, or 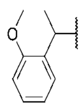.

In one embodiment, in $G^1$, the $C_6$-$C_{14}$ aryl substituted by one or more $G^{1-2}$ may be phenyl substituted by 1 or 2 $G^{1-2}$, or may be any one of the following groups:

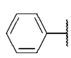 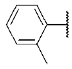 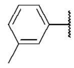 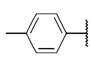 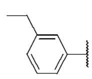 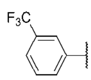 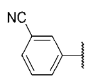 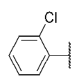 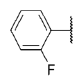 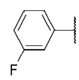 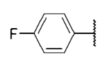 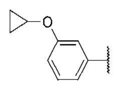

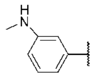 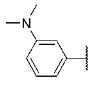 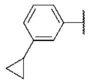 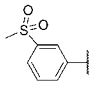 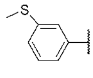 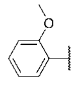 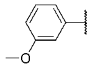 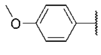 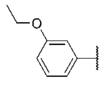 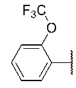 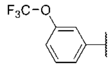 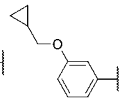

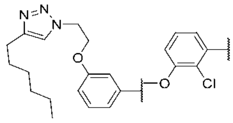 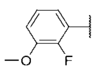 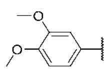 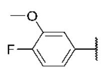 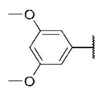 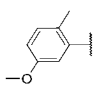 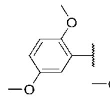 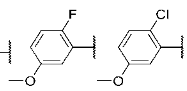 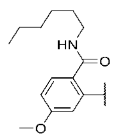

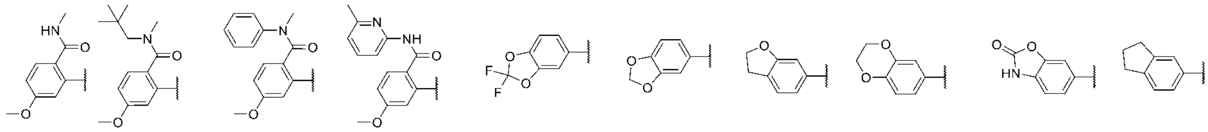

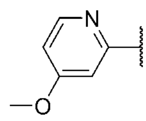 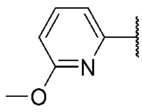 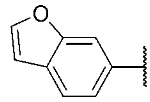 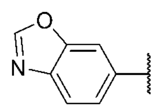 ;

such as

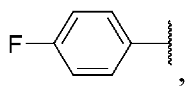 , 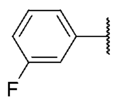 , 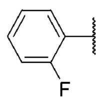 , 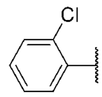 , 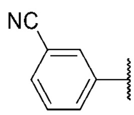 , 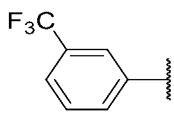 , 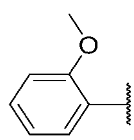 ,

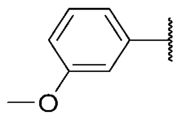 , 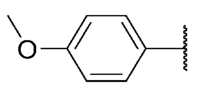 , 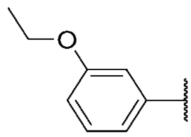 , 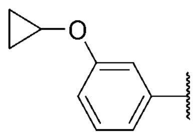 , 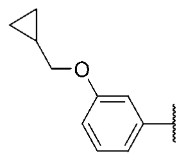 ,

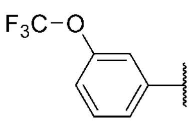 , 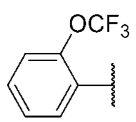 , 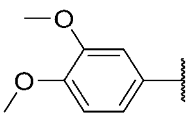 , 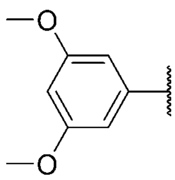 , 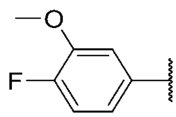 , 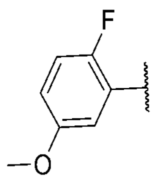 ,

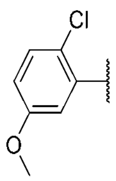 , 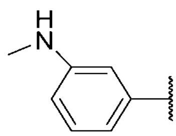 , 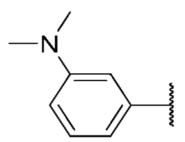 , 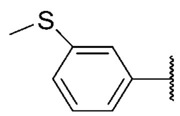 , 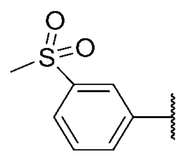 , 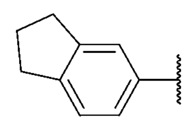 ,

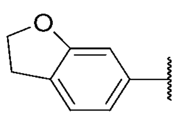 , 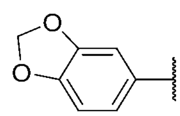 , 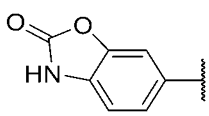 , 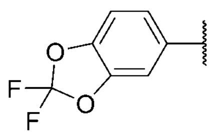 , 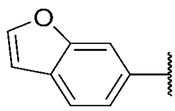 ,

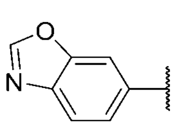 , 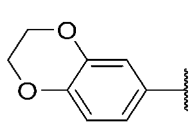 , 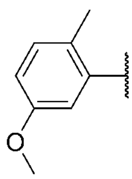 , 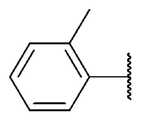 , 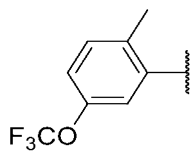 , 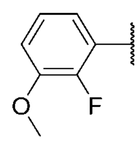 ,

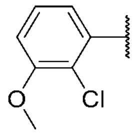, 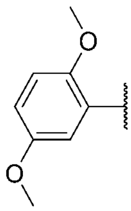, 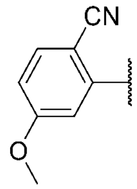, 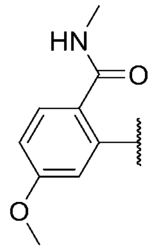, or 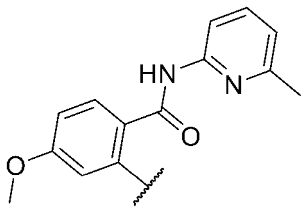.

In one embodiment, in $G^1$, the 5- to 10-membered heteroaryl substituted by one or more $G^{1-3}$ may be 5- to 6-membered monocyclic heterocycloalkyl substituted by 1 or 2 $G^{1-3}$, and may further be

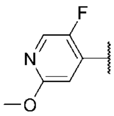, 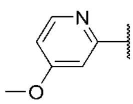, 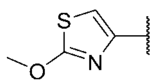, 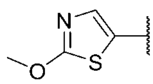, 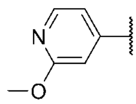,

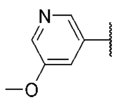, or 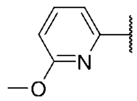.

In one embodiment, in $L^1$, the $C_1$-$C_6$ alkylene may be methylene, ethylene (

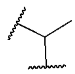

), or propylene (

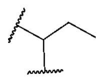

), such as methylene.

In one embodiment, in ring A, the "$C_4$-$C_6$ cycloalkyl" in the $C_4$-$C_6$ cycloalkyl and the $C_4$-$C_6$ cycloalkyl substituted by one or more $A^1$ is independently cyclopropyl, cyclobutyl, cyclopentyl, or cyclohexyl, such as cyclohexyl (

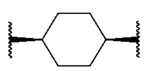, 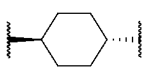,

where the left end is connected to $L^1$).

In one embodiment, in ring A, the "$C_4$-$C_6$ cycloalkenyl" in the $C_4$-$C_6$ cycloalkenyl and the $C_4$-$C_6$ cycloalkenyl substituted by one or more $A^2$ may be cyclohexenyl containing one double bond, such as

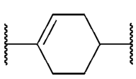,

where the left end is connected to L.

In one embodiment, in ring A, the "4- to 8-membered heterocycloalkyl" in the 4-to 8-membered heterocycloalkyl and the 4- to 8-membered heterocycloalkyl substituted by one or more $A^3$ may independently be 4- to 6-membered heterocycloalkyl (e.g., monocyclic) with 1 or 2 heteroatoms being N or 7- to 8-membered bridged heterocycloalkyl with 1 or 2 heteroatoms being N; the 4- to 6-membered heterocycloalkyl may be azetidinyl, pyrrolidinyl, or piperidinyl, such as

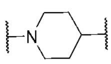, 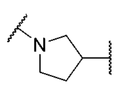,

or

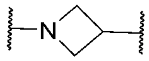

;

the 7- to 8-membered bridged heterocycloalkyl may be azabicyclo[3.2.1]octanyl, such as

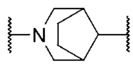

,

where the left end is connected to $L^1$ via N, and the right end is connected to

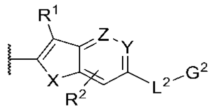

via C.

In one embodiment, in ring A, the "4- to 6-membered heterocycloalkenyl" in the 4- to 6-membered heterocycloalkenyl and the 4- to 6-membered heterocycloalkenyl substituted by one or more $A^4$ is independently 6-membered heterocycloalkenyl with 1 heteroatom being N, containing 1 double bond.

In one embodiment, in each $A^1$, each $A^2$, each $A^3$, and each $A^4$, the halogen may independently be fluorine, chlorine, or bromine, such as fluorine.

In one embodiment, in each $A^1$, each $A^2$, each $A^3$, and each $A^4$, the "$C_1$-$C_6$ alkyl" in the $C_1$-$C_6$ alkyl and the $C_1$-$C_6$ alkyl substituted by one or more $A^{1-5}$ may independently be methyl, ethyl, *n*-propyl, isopropyl, *n*-butyl, isobutyl, *sec*-butyl, or *tert-butyl,* such as methyl.

In one embodiment, in ring A, the 4- to 8-membered heterocycloalkyl substituted by one or more $A^3$ may be 4- to 6-membered heterocycloalkyl substituted by one or more $A^1$ or 7- to 8-membered bridged heterocycloalkyl substituted by one or more $A^1$; the 4-to 6-membered heterocycloalkyl substituted by one or more $A^1$ may be

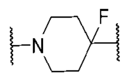

or

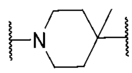

;

the 7- to 8-membered bridged heterocycloalkyl substituted by one or more $A^1$ or

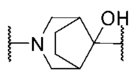

.

In one embodiment, in $R^1$, the "$C_1$-$C_6$ alkyl" in the $C_1$-$C_6$ alkyl and the $C_1$-$C_6$ alkyl substituted by one or more $R^{1-3}$ may be methyl, ethyl, n-propyl, isopropyl, n-butyl, isobutyl, sec-butyl, *tert-butyl,* n-pentyl,

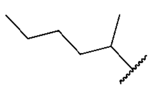

,

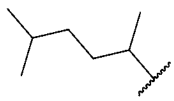

, or

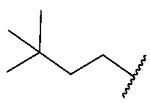

.

In one embodiment, in $R^1$, the "$C_2$-$C_6$ alkenyl" in the $C_2$-$C_6$ alkenyl and the $C_2$-$C_6$ alkenyl substituted by one or more $R^{1-10}$ may be vinyl, propenyl (

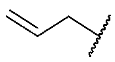

), pentenyl (

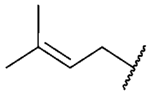 or 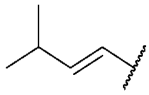

), or hexenyl (

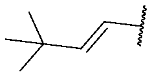 or 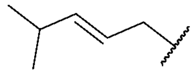

).

In one embodiment, in $R^{1-1}$, $R^{1-2}$, and $R^{1-11}$, the "$C_1$-$C_{12}$ alkyl" in the $C_1$-$C_{12}$ alkyl, the $C_1$-$C_{12}$ alkyl substituted by one or more $R^{1-1-1}$, and the -$S(=O)_2C_1$-$C_{12}$ alkyl may independently be $C_1$-$C_6$ alkyl or $C_7$-$C_{12}$ alkyl; the "$C_1$-$C_6$ alkyl" in the $C_1$-$C_6$ alkyl, the $C_1$-$C_6$ alkyl substituted by one or more $R^{1-1-1}$, and the -$S(=O)_2C_1$-$C_6$ alkyl may independently be methyl, ethyl, n-propyl, isopropyl, n-butyl, isobutyl, see-butyl, *tert*-butyl, or

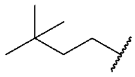,

such as methyl, ethyl, or isopropyl.

In one embodiment, in $R^{1-1}$, $R^{1-2}$, and $R^{1-11}$, the "$C_3$-$C_{12}$ cycloalkyl" in the $C_3$-$C_{12}$ cycloalkyl and the $C_3$-$C_{12}$ cycloalkyl substituted by one or more $R^{1-1-2}$ may independently be $C_3$-$C_{10}$ cycloalkyl or $C_{11}$-$C_{12}$ cycloalkyl; the "$C_3$-$C_{10}$ cycloalkyl" in the $C_3$-$C_{10}$ cycloalkyl and the $C_3$-$C_{10}$ cycloalkyl substituted by one or more $R^{1-1-2}$ may independently be $C_3$-$C_6$ monocyclic cycloalkyl, $C_5$-$C_7$ bridged cycloalkyl, or adamantyl, and may further be cyclopropyl, cyclobutyl, cyclopentyl, cyclohexyl, bicyclo[1.1.1]pentyl (

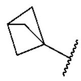

), bicyclo[2.2.1]heptyl (

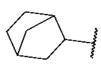

), or adamantyl (

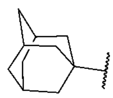

).

In one embodiment, $R^{1-1}$ and $R^{1-2}$, together with the N atom to which they are attached, form a 3- to 14-membered heterocycloalkyl, wherein the 3- to 14-membered heterocycloalkyl may be 4- to 6-membered monocyclic heterocycloalkyl with 1 or 2 heteroatoms being N or 6- to 14-membered bicyclic spirocycloalkyl with 1 or 2 heteroatoms being N, such as pyrrolidinyl or 2-azaspiro[3.3]heptyl.

In one embodiment, in $R^{1-1}$, $R^{1-2}$, and $R^{1-11}$, the "$C_6$-$C_{14}$ aryl" in the $C_6$-$C_{14}$ aryl and the $C_6$-$C_{14}$ aryl substituted by one or more $R^{1-1-3}$ may independently be phenyl or naphthyl.

In one embodiment, in $R^{1-1}$, $R^{1-2}$, and $R^{1-11}$, the "5- to 14-membered heteroaryl" in the 5- to 14-membered heteroaryl and the 5- to 14-membered heteroaryl substituted by one or more $G^{1-1-10-1}$ may independently be 5- to 10-membered heteroaryl or 11- to 14-membered heteroaryl, wherein the "5- to 10-membered heteroaryl" in the 5- to 10-membered heteroaryl and the 5- to 10-membered heteroaryl substituted by one or more $G^{1-1-10-1}$ may independently be 5- to 6-membered heteroaryl (monocyclic) or 8- to 10-membered bicyclic heteroaryl (*e.g.*, with 1 or 2 heteroatoms being N), and may further be thiazolyl (

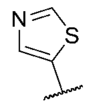

), oxazolyl (

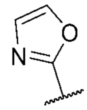

), imidazolyl (

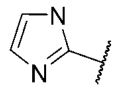

), pyrazolyl (

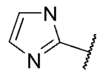

), thiadiazolyl (

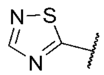

), triazolyl (

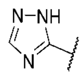

), tetrazolyl (

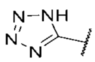

), pyridyl, benzo[d]isoxazolyl (

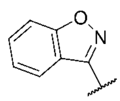

), or benzo[d]thiazolyl (

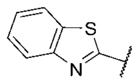

).

**In** one embodiment, in each $R^{1-1-1}$, each $R^{1-1-2}$, each $R^{1-1-3}$, $R^{1-1-4}$, and each $R^{1-1-5}$, the halogen may independently be fluorine, chlorine, or bromine, such as fluorine.

**In** one embodiment, in each $R^{1-1-1}$, each $R^{1-1-2}$, each $R^{1-1-3}$, $R^{1-1-4}$, and each $R^{1-1-5}$, the "$C_1$-$C_{12}$ alkyl" in the -NH($C_1$-$C_{12}$ alkyl), the -N($C_1$-$C_{12}$ alkyl)$_2$, the -C(=O)-$C_1$-$C_{12}$ alkyl, the -NHC(=O)-$C_1$-$C_{12}$ alkyl, the $C_1$-$C_{12}$ alkyl, and the $C_1$-$C_{12}$ alkyl substituted by one or more $R^{1-1-1-1}$ may independently be $C_1$-$C_6$ alkyl or $C_7$-$C_{12}$ alkyl; the $C_1$-$C_6$ alkyl may be methyl, ethyl, n-propyl, isopropyl, n-butyl, isobutyl, sec-butyl, or tert-butyl, such as methyl.

In one embodiment, in each $R^{1-1-1}$, each $R^{1-1-2}$, each $R^{1-1-3}$, $R^{1-1-4}$, and each $R^{1-1-5}$, the "$C_3$-$C_{12}$ cycloalkyl" in the

$C_3$-$C_{12}$ cycloalkyl and the $C_3$-$C_{12}$ cycloalkyl substituted by one or more $R^{1-1-1-3}$ may independently be $C_3$-$C_8$ cycloalkyl or $C_9$-$C_{10}$ cycloalkyl; the $C_3$-$C_8$ cycloalkyl may be cyclopropyl, cyclobutyl, cyclopentyl, or cyclohexyl, such as cyclopropyl.

In one embodiment, in each $R^{1-1-1}$, each $R^{1-1-2}$, each $R^{1-1-3}$, $R^{1-1-4}$, and each $R^{1-1-5}$, the $C_6$-$C_{14}$ aryl may be phenyl or naphthyl.

In one embodiment, in each $R^{1-1-1}$, each $R^{1-1-2}$, each $R^{1-1-3}$, $R^{1-1-4}$, and each $R^{1-1-5}$, the 3- to 12-membered heterocycloalkyl may be 3- to 6-membered heterocycloalkyl with 1 or 2 heteroatoms being N and/or O, and may further be piperidinyl (

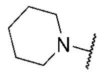

) or morpholinyl (

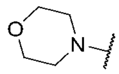

).

In one embodiment, in $R^{1-1}$, $R^{1-2}$, and $R^{1-11}$, the $C_1$-$C_{12}$ alkyl substituted by one or more $R^{1-1-1}$ may be $C_1$-$C_6$ alkyl substituted by one or more $R^{1-1-1}$, and may further be

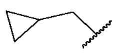 or 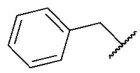,

such as

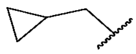.

In one embodiment, in $R^{1-1}$, $R^{1-2}$, and $R^{1-11}$, the $C_3$-$C_{12}$ cycloalkyl substituted by one or more $R^{1-1-2}$ may be $C_3$-$C_8$ cycloalkyl substituted by one or more (*e.g.,* 2 or 3) $R^{1-1-2}$, or may further be

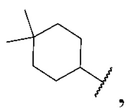, 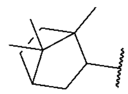, 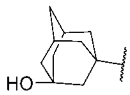, or 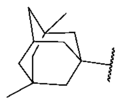.

In one embodiment, in $R^{1-1}$, $R^{1-2}$, and $R^{1-11}$, the $C_6$-$C_{14}$ aryl substituted by one or more $R^{1-1-3}$ may be phenyl substituted by 1, 2, or 3 $R^{1-1-3}$, and may further be

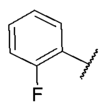,

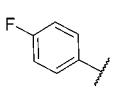, 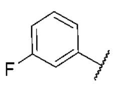, 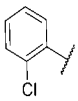, 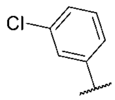, 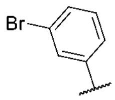, 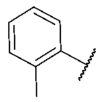, 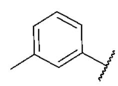, 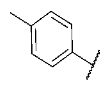, 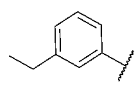,

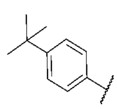, 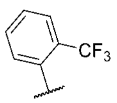, 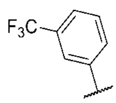, 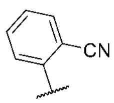, 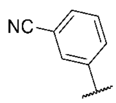, 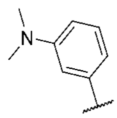, 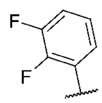, 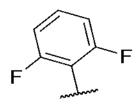,

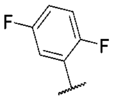, 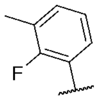, 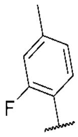, 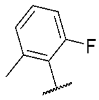, 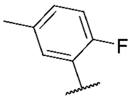, 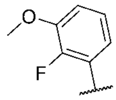, 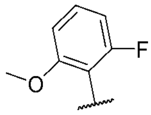, 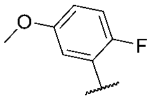,

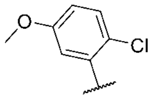, 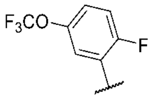, 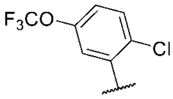, 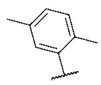, 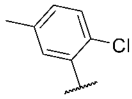, 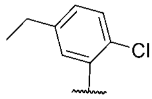, 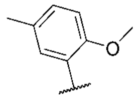,

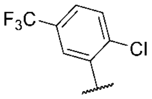, 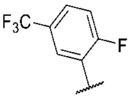, 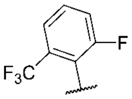, 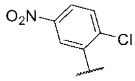, 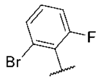, 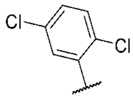, 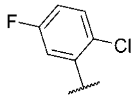,

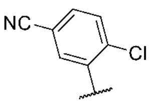, 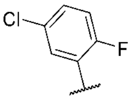, 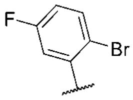, 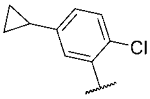, 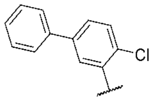, 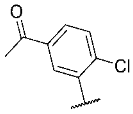, 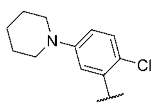,

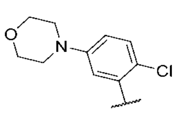, 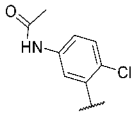, 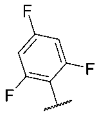, or 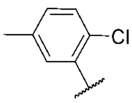,

such as

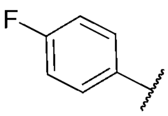, 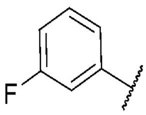,

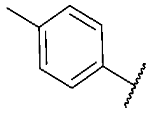, or 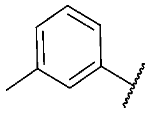.

In one embodiment, in $R^{1-1}$, $R^{1-2}$, and $R^{1-11}$, the 5- to 14-membered heteroaryl substituted by one or more $R^{1-1-4}$ may be 5- to 6-membered monocyclic heteroaryl substituted by 1 or 2 $R^{1-1-3}$ or 9- to 10-membered fused heteroaryl substituted by 1 or 2 $R^{1-1-3}$, and may further be

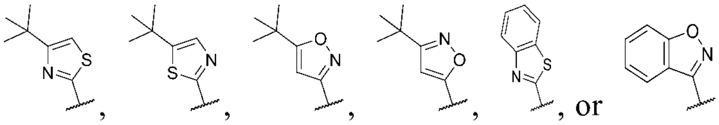, , , , , or .

In one embodiment, in each $R^{1-3}$ and each $R^{1-10}$, the halogen may independently be fluorine, chlorine, or bromine, such as fluorine or chlorine.

In one embodiment, in each $R^{1-3}$ and each $R^{1-10}$, the "$C_1$-$C_{12}$ alkyl" in the -S(=O)$_2$-$C_1$-$C_{12}$ alkyl, the -S-$C_1$-$C_{12}$ alkyl, the $C_1$-$C_{12}$ alkyl, and the $C_1$-$C_{12}$ alkyl substituted by one or more $R^{1-3-6}$ may independently be $C_1$-$C_6$ alkyl or $C_7$-$C_{12}$ alkyl;

the "$C_1$-$C_6$ alkyl" in the -S(=O)$_2$-$C_1$-$C_6$ alkyl, the -S-$C_1$-$C_6$ alkyl, the $C_1$-$C_6$ alkyl, and the $C_1$-$C_6$ alkyl substituted by one or more $R^{1-3-6}$ may independently be methyl, ethyl, *n*-propyl, isopropyl, *n*-butyl, isobutyl, *sec*-butyl, or *tert*-butyl, such as isopropyl; the $C_7$-$C_{12}$ alkyl may be pentyl, hexyl, or heptyl.

In one embodiment, in each $R^{1-3}$ and each $R^{1-10}$, the "$C_1$-$C_{12}$ alkoxy" in the $C_1$-$C_{12}$ alkoxy and the $C_1$-$C_{12}$ alkoxy substituted by one or more $R^{1-3-7}$ may independently be $C_1$-$C_6$ alkoxy; the "$C_1$-$C_6$ alkoxy" in the $C_1$-$C_6$ alkoxy and the

$C_1$-$C_6$ alkoxy substituted by one or more $R^{1-3-7}$ may independently be methoxy, ethoxy, *n*-propoxy, isopropoxy, *n*-butoxy, isobutoxy, *sec*-butoxy, or *tert*-butoxy.

In one embodiment, in each $R^{1-3}$ and each $R^{1-10}$, the "5- to 14-membered heteroaryl" in the 5- to 14-membered heteroaryl, the 5- to 14-membered heteroaryl substituted by one or more $R^{1-3-8}$, and the -O-5- to 14-membered heteroaryl may independently be 5- to 10-membered heteroaryl; the "5- to 10-membered heteroaryl" in the 5- to 10-membered heteroaryl and the 5- to 10-membered heteroaryl substituted by one or more $R^{1-3-8}$ may independently be 5- to 6-membered monocyclic heteroaryl with 1, 2, or 3 heteroatoms independently selected from one or two types of N, S, and O, such as 1*H*-pyrazolyl (

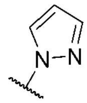

), pyridyl (

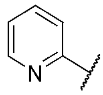

), or oxadiazolyl (

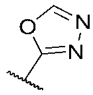

).

In one embodiment, in each $R^{1-3}$ and each $R^{1-10}$, the "$C_3$-$C_{12}$ cycloalkyl" in the $C_3$-$C_{12}$ cycloalkyl and the $C_3$-$C_{12}$ cycloalkyl substituted by one or more $R^{1-3-9}$ may independently be $C_3$-$C_8$ cycloalkyl or $C_9$-$C_{12}$ cycloalkyl; the "$C_3$-$C_8$ cycloalkyl" in the $C_3$-$C_8$ cycloalkyl and the $C_3$-$C_8$ cycloalkyl substituted by one or more $R^{1-3-9}$ may independently be cyclopropyl, cyclobutyl, cyclopentyl, or cyclohexyl, such as cyclopropyl or cyclobutyl; the $C_9$-$C_{12}$ cycloalkyl may be adamantyl.

In one embodiment, in each $R^{1-3}$ and each $R^{1-10}$, the "$C_2$-$C_6$ alkenyl" in the $C_2$-$C_6$ alkenyl and the $C_2$-$C_6$ alkenyl substituted by one or more $R^{1-3-10}$ may independently be vinyl or propenyl, such as

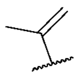

.

In one embodiment, in each $R^{1-3}$ and each $R^{1-10}$, the "$C_6$-$C_{14}$ aryl" in the $C_6$-$C_{14}$ aryl, the $C_6$-$C_{14}$ aryl substituted by one or more $R^{1-3-11}$, the -O-$C_6$-$C_{14}$ aryl, and the -O-C(=O)$C_6$-$C_{14}$ aryl is independently phenyl.

In one embodiment, in each $R^{1-3}$ and each $R^{1-10}$, the "$C_3$-$C_8$ cycloalkenyl" in the $C_3$-$C_8$ cycloalkenyl and the $C_3$-$C_8$ cycloalkenyl substituted by one or more $R^{1-3-12}$ may be cyclopentenyl containing one double bond or cyclohexenyl containing one double bond.

In one embodiment, in each $R^{1-3}$ and each $R^{1-10}$, the "3- to 12-membered heterocycloalkyl" in the 3- to 12-membered heterocycloalkyl and the 3- to 12-membered heterocycloalkyl substituted by one or more $R^{1-3-13}$ may be 3- to 6-membered monocyclic heterocycloalkyl with 1 or 2 heteroatoms being O, such as

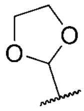

.

In one embodiment, in $R^{1-3-1}$, $R^{1-3-2}$, $R^{1-3-3}$, $R^{1-3-4}$, $R^{1-3-1a}$, $R^{1-3-2a}$ , $R^{1-3-3a}$, and $R^{1-3-4a}$, the $C_1$-$C_6$ alkyl may be methyl, ethyl, *n*-propyl, isopropyl, *n*-butyl, isobutyl, *sec*-butyl, or *tert*-butyl.

In one embodiment, in each $R^{1-3}$ and each $R^{1-10}$, the "$C_6$-$C_{14}$ aryl" in the $C_6$-$C_{14}$ aryl and the $C_6$-$C_{14}$ aryl substituted by one or more $R^{1-3-1-4}$ is independently phenyl.

In one embodiment, in each $R^{1-3-1-1}$, each $R^{1-3-1-2}$, each $R^{1-3-1-4}$, and each $R^{1-3-3-1}$, the $C_1$-$C_6$ alkyl may be methyl, ethyl, *n*-propyl, isopropyl, *n*-butyl, isobutyl, *sec*-butyl, or *tert*-butyl.

In one embodiment, in $R^{1-3-5}$, the $C_1$-$C_6$ alkyl may be methyl, ethyl, *n*-propyl, isopropyl, *n*-butyl, isobutyl, *sec*-butyl, or *tert*-butyl.

In one embodiment, in each $R^{1-3-6}$, each $R^{1-3-7}$, each $R^{1-3-8}$, each $R^{1-3-9}$, each $R^{1-3-10}$, each $R^{1-3-11}$, each $R^{1-3-12}$, and each $R^{1-3-13}$, the "$C_1$-$C_6$ alkyl" in the -C(=O)-O-$C_1$-$C_6$ alkyl, the -C(=O)-N($C_1$-$C_6$ alkyl$)_2$, the -C(=O)-N($C_1$-$C_6$ alkyl$)_2$, and the $C_1$-$C_6$ alkyl may independently be methyl, ethyl, *n*-propyl, isopropyl, *n*-butyl, isobutyl, *sec*-butyl, or *tert*-butyl.

In one embodiment, in each $R^{1-3-6}$, $R^{1-3-7}$, $R^{1-3-8}$, $R^{1-3-9}$, $R^{1-3-10}$, $R^{1-3-11}$, $R^{1-3-12}$, and $R^{1-3-13}$, the $C_3$-$C_8$ cycloalkyl may be cyclopropyl, cyclobutyl, cyclopentyl, or cyclohexyl.

In one embodiment, in each $R^{1-3-6}$, each $R^{1-3-7}$, each $R^{1-3-8}$, each $R^{1-3-9}$, each $R^{1-3-10}$, each $R^{1-3-11}$, each $R^{1-3-12}$, and each $R^{1-3-13}$, the "5- to 10-membered heteroaryl" in the 5- to 10-membered heteroaryl and the 5- to 10-membered heteroaryl substituted by one or more $C_1$-$C_6$ alkyl groups may be 5- to 6-membered heteroaryl with 1 or 2 heteroatoms selected from one or two types of N, S, and O, and may further be furanyl or thienyl.

In one embodiment, in $R^1$, in -C(=O)$NR^{1-1}R^{1-2}$, one of $R^{1-1}$ and $R^{1-2}$ may be H or $C_1$-$C_{12}$ alkyl, and the other may be -S(=O$)_2C_1$-$C_{12}$ alkyl, $C_1$-$C_{12}$ alkyl, $C_1$-$C_{12}$ alkyl substituted by one or more $R^{1-1-1}$, $C_3$-$C_{12}$ cycloalkyl, $C_3$-$C_{12}$ cycloalkyl substituted by one or more $R^{1-1-2}$, $C_6$-$C_{14}$ aryl, $C_6$-$C_{14}$ aryl substituted by one or more $R^{1-1-3}$, 5- to 14-membered heteroaryl, or 5- to 14-membered heteroaryl substituted by one or more $R^{1-1-4}$;

-C(=O)$NR^{1-1}R^{1-2}$ may further be the following group:

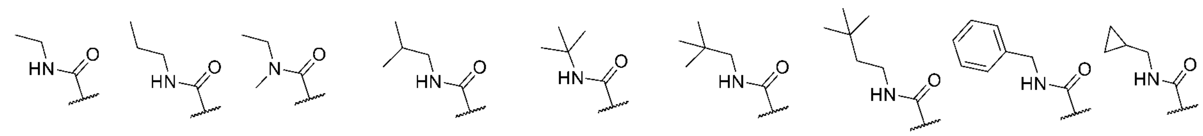

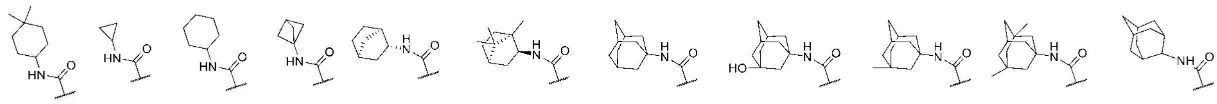

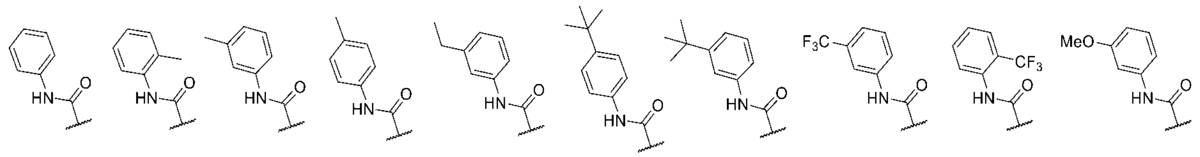

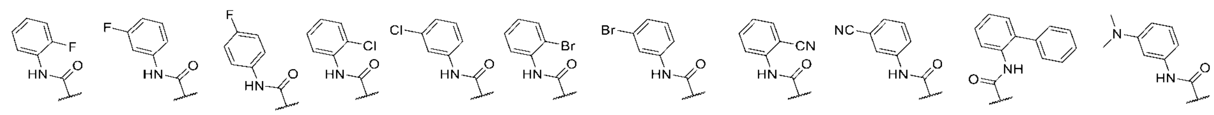

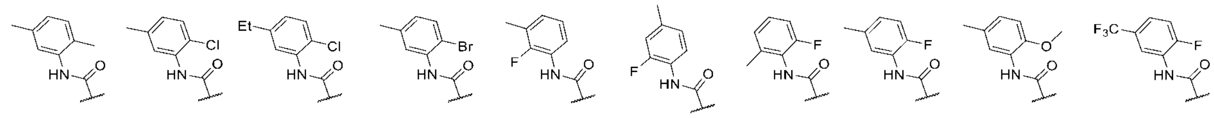

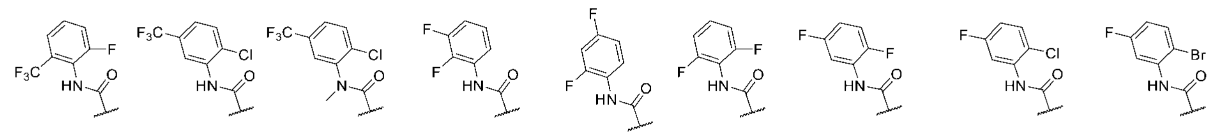

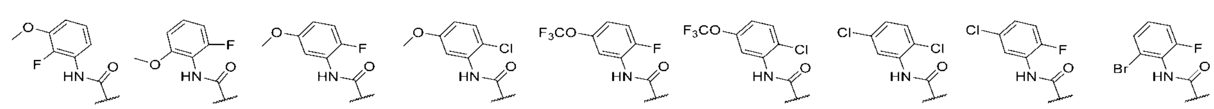

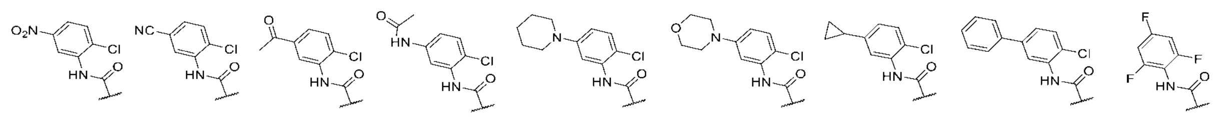

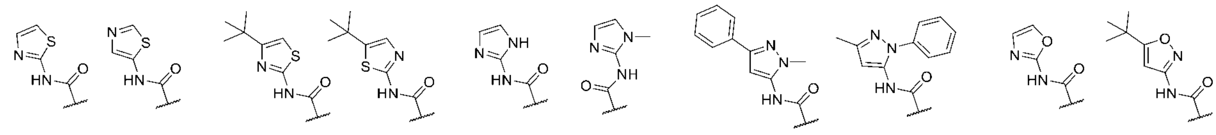

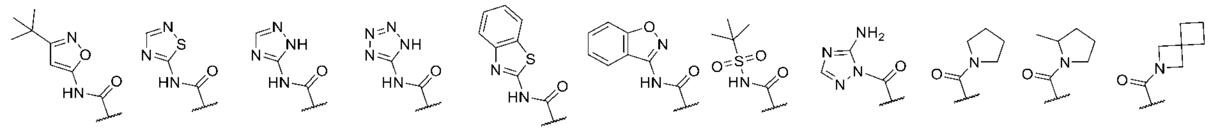

;

such as

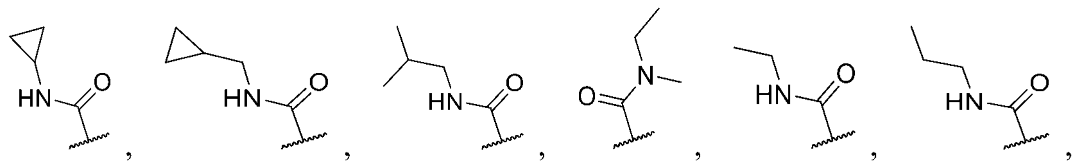

, , , , , ,

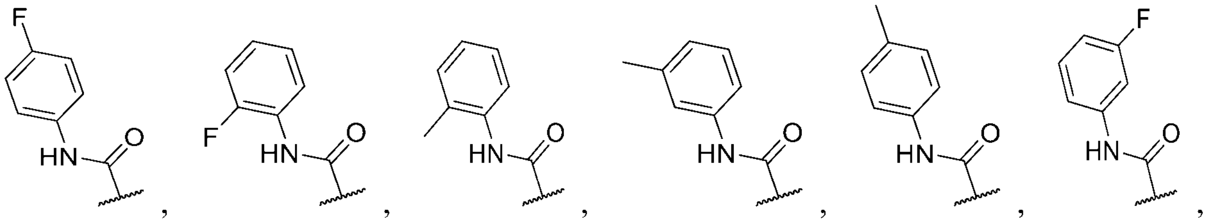

, , , , , ,

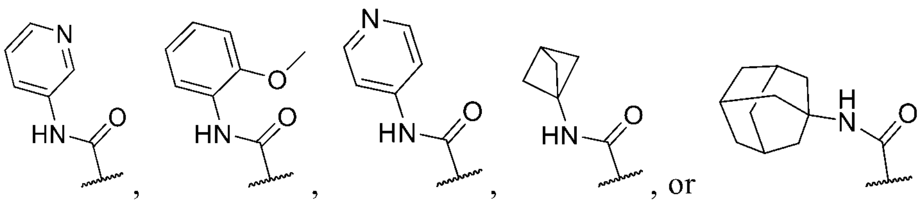

, , , , or .

In one embodiment, in $R^1$, the $C_1$-$C_6$ alkyl substituted by one or more $R^{1-3}$ may be any one of the following groups:

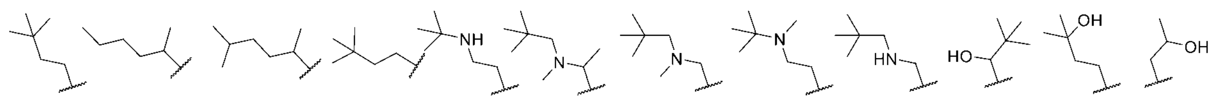

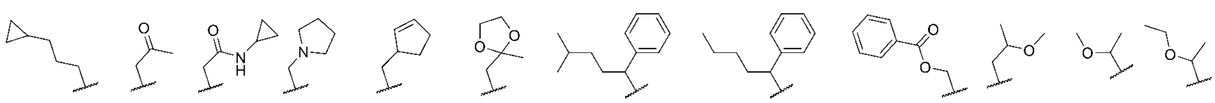

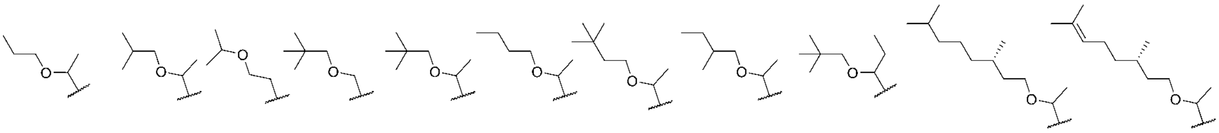

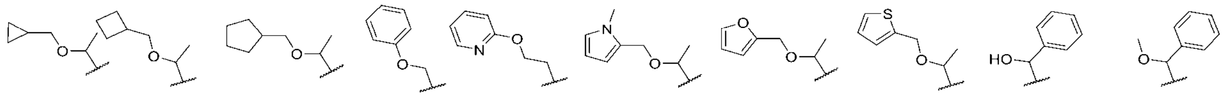

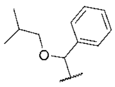 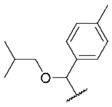 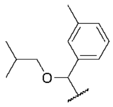 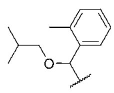 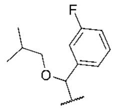 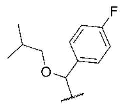 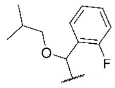 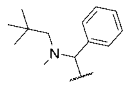 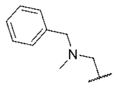

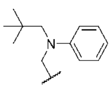 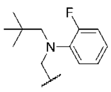 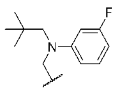 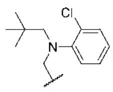 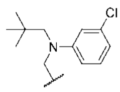 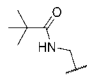 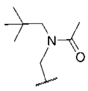 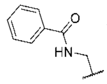 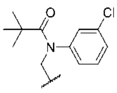 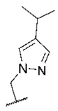 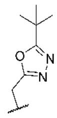

.

In one embodiment, in $R^1$, the $C_2$-$C_6$ alkenyl substituted by one or more $R^{1-10}$ may be

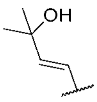, 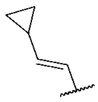, 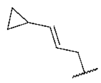, or 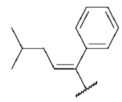 .

In one embodiment, in $R^1$, -C(=O)$R^{1-11}$ may be

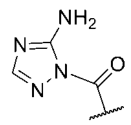.

In one embodiment, in ring B, the "$C_3$-$C_{12}$ cycloalkyl" in the $C_3$-$C_{12}$ cycloalkyl and the $C_3$-$C_{12}$ cycloalkyl substituted by one or more $R^{1-4}$ may independently be $C_3$-$C_8$ cycloalkyl or $C_9$-$C_{12}$ cycloalkyl; the "$C_3$-$C_8$ cycloalkyl" in the $C_3$-$C_8$ cycloalkyl and the $C_3$-$C_8$ cycloalkyl substituted by one or more $R^{1-4}$ may independently be cyclopropyl, cyclobutyl, cyclopentyl, or cyclohexyl, such as cyclohexyl.

In one embodiment, in ring B, the "$C_3$-$C_{12}$ cycloalkenyl" in the $C_3$-$C_{12}$ cycloalkenyl and the $C_3$-$C_{12}$ cycloalkenyl substituted by one or more $R^{1-5}$ may independently be $C_3$-$C_8$ cycloalkenyl or $C_9$-$C_{12}$ cycloalkenyl; the "$C_3$-$C_8$ cycloalkenyl" in the $C_3$-$C_8$ cycloalkenyl and the $C_3$-$C_8$ cycloalkenyl substituted by one or more $R^{1-5}$ may be cyclopropenyl containing one double bond, cyclobutenyl containing one double bond, cyclopentenyl containing one double bond, or cyclohexenyl containing one double bond, such as cyclopentenyl or cyclohexenyl.

In one embodiment, in ring B, the "3- to 12-membered heterocycloalkyl" in the 3- to 12-membered heterocycloalkyl and the 3- to 12-membered heterocycloalkyl substituted by one or more $R^{1-6}$ may independently be 3- to 8-membered heterocycloalkyl or 9- to 12-membered heterocycloalkyl; the "3- to 8-membered heterocycloalkyl" in the 3- to 8-membered heterocycloalkyl and the 3- to 8-membered heterocycloalkyl substituted by one or more $R^{1-6}$ may independently be 5- to 6-membered heterocycloalkyl with 1 or 2 heteroatoms independently being O and/or N, and the number is 1 or 2, such as piperidinyl, dioxolanyl, or dioxanyl.

In one embodiment, in ring B, the "3- to 12-membered heterocycloalkenyl" in the 3- to 12-membered heterocycloalkenyl and the 3- to 12-membered heterocycloalkenyl substituted by one or more $R^{1-7}$ may independently be 3- to 8-membered heterocycloalkenyl or 9- to 12-membered heterocycloalkenyl; the "3- to 8-membered heterocycloalkenyl" in the 3- to 8-membered heterocycloalkenyl and the 3- to 8-membered heterocycloalkenyl substituted by one or more $R^{1-7}$ may independently be 5-to 6-membered heterocycloalkenyl (*e.g.*, monocyclic) with 1 or 2 heteroatoms independently being N and containing one double bond, such as 1,2,3,6-tetrahydropyridyl.

In one embodiment, in ring B, the "$C_6$-$C_{14}$ aryl" in the $C_6$-$C_{14}$ aryl and the $C_6$-$C_{14}$ aryl substituted by one or more $R^{1-8}$ may independently be phenyl or naphthyl.

Preferably, when the "$C_6$-$C_{14}$ aryl" in the $C_6$-$C_{14}$ aryl substituted by one or more $R^{1-8}$ is phenyl, then the number of $R^{1-8}$ is 1, and the substitution position is at the ortho, meta, or para position of the phenyl, such as the para position.

In one embodiment, in ring B, the "5- to 14-membered heteroaryl" in the 5- to 14-membered heteroaryl and the 5- to 14-membered heteroaryl substituted by one or more $R^{1-9}$ may independently be 5- to 10-membered heteroaryl or 11- to 14-membered heteroaryl; the "5- to 10-membered heteroaryl" in the the 5- to 10-membered heteroaryl and the 5- to 10-membered heteroaryl substituted by one or more $R^{1-9}$ may independently be 5-, 6-, or 9-membered monocyclic or bicyclic heteroaryl with 1, 2, 3, or 4 heteroatoms selected from one or more types of N, S, and O, and may further be pyrrolyl, imidazolyl, 1*H*-pyrazolyl, oxazolyl, isoxazolyl, thiazolyl, 1,3,4-oxadiazolyl, 1,3,4-oxadiazolyl, 1,2,4-oxadiazolyl, thienyl, thiazolyl, pyridyl, pyrimidinyl, indolyl, indazolyl, thiazolophenyl, or triazolopyridyl; or may further be 1*H*-pyrazolyl, 1,3,4-oxadiazolyl, thienyl, thiazolyl, pyridyl, pyrimidinyl, indolyl, indazolyl, thiazolophenyl, or triazolopyridyl.

In one embodiment, in each $R^{1-4}$, each $R^{1-5}$, each $R^{1-6}$, each $R^{1-7}$ , each $R^{1-8}$, and each $R^{1-9}$, the halogen may independently be fluorine, chlorine, or bromine, such as fluorine or chlorine.

In one embodiment, in each $R^{1-4}$, each $R^{1-5}$, each $R^{1-6}$, each $R^{1-7}$, each $R^{1-8}$, and each $R^{1-9}$, the "$C_1$-$C_{12}$ alkyl" in the -S(=O)$_2$-$C_1$-$C_{12}$ alkyl, the -S-$C_1$-$C_{12}$ alkyl, the $C_1$-$C_{12}$ alkyl, and the $C_1$-$C_{12}$ alkyl substituted by one or more $R^{1-3-6a}$ may independently be $C_1$-$C_6$ alkyl or $C_7$-$C_{12}$ alkyl; the "$C_1$-$C_6$ alkyl" in the -S(=O)$_2$-$C_1$-$C_6$ alkyl, the -S-$C_1$-$C_6$ alkyl, the $C_1$-$C_6$ alkyl, and the $C_1$-$C_6$ alkyl substituted by one or more $R^{1-3-6a}$ may independently be methyl, ethyl, *n*-propyl, isopropyl, *n*-butyl, isobutyl, *sec*-butyl, or *tert*-butyl, such as isopropyl; the $C_7$-$C_{12}$ alkyl may be pentyl, hexyl, or heptyl.

In one embodiment, in each $R^{1-4}$, each $R^{1-5}$, each $R^{1-6}$, each $R^{1-7}$, each $R^{1-8}$, and each $R^{1-9}$, the "$C_1$-$C_{12}$ alkoxy" in the $C_1$-$C_{12}$ alkoxy and the $C_1$-$C_{12}$ alkoxy substituted by one or more $R^{1-3-7a}$ may independently be $C_1$-$C_6$ alkoxy; the "$C_1$-$C_6$ alkoxy" in the $C_1$-$C_6$ alkoxy and the $C_1$-$C_6$ alkoxy substituted by one or more $R^{1-3-7a}$ may independently be methoxy, ethoxy, *n*-propoxy, isopropoxy, *n*-butoxy, isobutoxy, *sec*-butoxy, or *tert*-butoxy.

In one embodiment, in each $R^{1-4}$, each $R^{1-5}$, each $R^{1-6}$, each $R^{1-7}$, each $R^{1-8}$, and each $R^{1-9}$, the "5- to 14-membered heteroaryl" in the 5- to 14-membered heteroaryl and the 5- to 14-membered heteroaryl substituted by one or more $R^{1-3-8a}$ may independently be 5- to 10-membered heteroaryl; the "5- to 10-membered heteroaryl" in the 5- to 10-membered heteroaryl and the 5- to 10-membered heteroaryl substituted by one or more $R^{1-3-8a}$ may independently be 5- to 6-membered monocyclic heteroaryl with 1 or 2 heteroatoms independently being N, and may further be 1*H*-pyrazolyl or pyridyl.

In one embodiment, in each $R^{1-4}$, each $R^{1-5}$, each $R^{1-6}$, each $R^{1-7}$, each $R^{1-8}$, and each $R^{1-9}$, the "$C_3$-$C_{12}$ cycloalkyl" in the $C_3$-$C_{12}$ cycloalkyl and the $C_3$-$C_{12}$ cycloalkyl substituted by one or more $R^{1-3-9a}$ may independently be $C_3$-$C_8$ cycloalkyl or $C_9$-$C_{12}$ cycloalkyl; the "$C_3$-$C_8$ cycloalkyl" in the $C_3$-$C_8$ cycloalkyl and the $C_3$-$C_8$ cycloalkyl substituted by one or more $R^{1-3-9a}$ may independently be cyclopropyl, cyclobutyl, cyclopentyl, or cyclohexyl, such as cyclopropyl or cyclobutyl; the $C_9$-$C_{12}$ cycloalkyl may be adamantyl.

In one embodiment, in each $R^{1-4}$, each $R^{1-5}$, each $R^{1-6}$, each $R^{1-7}$, each $R^{1-8}$, and each $R^{1-9}$, the "$C_2$-$C_6$ alkenyl" in the $C_2$-$C_6$ alkenyl and the $C_2$-$C_6$ alkenyl substituted by one or more $R^{1-3-10a}$ may independently be vinyl or propenyl, such as

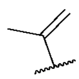.

In one embodiment, the "3- to 8-membered heterocycloalkyl" formed by any two adjacent $R^{1-8}$ together with the carbon atom to which they are attached and the "3- to 8-membered heterocycloalkyl" in the 3- to 8-membered heterocycloalkyl substituted by one or more $R^{1-8-1}$ may independently be 5- to 6-membered heterocycloalkyl with 1 or 2 heteroatoms independently being N and/or O, such as

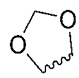 or 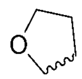,

where 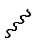 indicates that the group forms a fused ring with the $C_6$-$C_{14}$ aryl through this bond.

In one embodiment, the "$C_1$-$C_{14}$ cycloalkyl" formed by any two adjacent $R^{1-8}$ together with the carbon atom to which they are attached may be $C_{11}$-$C_{14}$ tricyclic cycloalkyl, such as

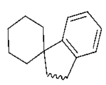,

where 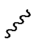 indicates that the group forms a fused ring with the $C_6$-$C_{14}$ aryl through this bond.

In one embodiment, in $R^{1-3-1a}$, $R^{1-3-2a}$, $R^{1-3-3a}$, $R^{1-3-4a}$, and $R^{1-3-5a}$, the $C_1$-$C_6$ alkyl may independently be methyl, ethyl, *n*-propyl, isopropyl, *n*-butyl, isobutyl, *sec*-butyl, *tert*-butyl,

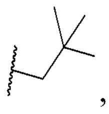, or 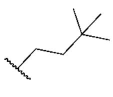.

In one embodiment, in $R^{1-3-1a}$, $R^{1-3-2a}$, $R^{1-3-3a}$, $R^{1-3-4a}$, and $R^{1-3-5a}$, the $C_1$-$C_6$ alkoxy may independently be

methoxy, ethoxy, *n*-propoxy, isopropoxy, *n*-butoxy, isobutoxy, *sec*-butoxy, or *tert*-butoxy.

In one embodiment, in $R^{1-3-1a}$, $R^{1-3-2a}$, $R^{1-3-3a}$, $R^{1-3-4a}$, and $R^{1-3-5a}$, the $C_3$-$C_8$ cycloalkyl may independently be cyclopropyl, cyclobutyl, cyclopentyl, or cyclohexyl, such as cyclopropyl or cyclopentyl.

In one embodiment, the "3- to 8-membered heterocycloalkyl" formed by $R^{1-3-1a}$ and $R^{1-3-2a}$ together with the N atom to which they are attached and the "3- to 8-membered heterocycloalkyl" in the 3- to 8-membered heterocycloalkyl substituted by one or more $R^{1-3-1-2a}$ may independently be 5- to 6-membered heterocycloalkyl with 1 or 2 heteroatoms independently being N, and may further be pyrrolidinyl.

In one embodiment, the "3- to 8-membered heterocycloalkyl" formed by $R^{1-3-3a}$ and $R^{1-3-4a}$ together with the N atom to which they are attached and the "3- to 8-membered heterocycloalkyl" in the 3- to 8-membered heterocycloalkyl substituted by one or more $R^{1-3-3-1a}$ may independently be 5- to 6-membered heterocycloalkyl with 1 or 2 heteroatoms independently being N or 6- to 7-membered dispiro heterocycloalkyl with 1 or 2 heteroatoms independently being N, and may further be pyrrolidinyl or 2-azaspiro[3.3]heptyl.

In one embodiment, in each $R^{1-3-1-1a}$, each $R^{1-3-1-2a}$, and each $R^{1-3-3-1a}$, the halogen may independently be fluorine, chlorine, or bromine, such as fluorine.

In one embodiment, in each $R^{1-3-1-1a}$, each $R^{1-3-1-2a}$, and each $R^{1-3-3-1a}$, the $C_1$-$C_6$ alkyl may independently be methyl, ethyl, *n*-propyl, isopropyl, *n*-butyl, isobutyl, *sec*-butyl, or *tert*-butyl, such as methyl.

In one embodiment, in each $R^{1-3-6a}$, each $R^{1-3-7a}$, each $R^{1-3-8a}$, each $R^{1-3-9a}$, each $R^{1-3-10a}$, each $R^{1-3-11a}$, each $R^{1-3-12a}$, and each $R^{1-8-1}$, the "$C_1$-$C_6$ alkyl" in the $C_1$-$C_6$ alkyl, the -C(=O)-O-$C_1$-$C_6$ alkyl, the -C(=O)-NH-$C_1$-$C_6$ alkyl, and the -C(=O)-N($C_1$-$C_6$ alkyl)$_2$ may independently be methyl, ethyl, *n*-propyl, isopropyl, *n*-butyl, isobutyl, *sec*-butyl, or *tert*-butyl, such as methyl or *tert*-butyl.

In one embodiment, in each $R^{1-3-6a}$, each $R^{1-3-7a}$, each $R^{1-3-8a}$, each $R^{1-3-9a}$, each $R^{1-3-10a}$, each $R^{1-3-11a}$, each $R^{1-3-12a}$, and each $R^{1-8-1}$, the "3- to 12-membered heterocycloalkyl" in the 3- to 12-membered heterocycloalkyl and the -O-C(=O)-$C_1$-$C_6$ alkyl-3- to 12-membered heterocycloalkyl may independently be 5- to 6-membered monocyclic heterocycloalkyl with 1 or 2 heteroatoms being N, such as pyrrolidinyl.

In one embodiment, in ring B, the $C_3$-$C_{12}$ cycloalkyl substituted by one or more $R^{1-4}$ may be $C_3$-$C_8$ cycloalkyl substituted by one or more $R^{1-4}$, and may further be

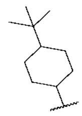.

In one embodiment, in ring B, the $C_3$-$C_{12}$ cycloalkenyl substituted by one or more $R^{1-5}$ may be $C_3$-$C_8$ cycloalkenyl substituted by one or more $R^{1-5}$, and may further be

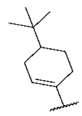.

In one embodiment, in ring B, the 3- to 12-membered heterocycloalkyl substituted by one or more $R^{1-6}$ may be 3- to 8-membered heterocycloalkyl substituted by one or more $R^{1-6}$, and may further be

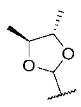, 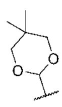, 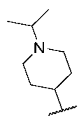, or 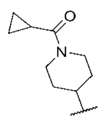.

In one embodiment, in ring B, the 3- to 12-membered heterocycloalkenyl substituted by one or more $R^{1-7}$ may be 3- to 8-membered heterocycloalkenyl substituted by one or more $R^{1-7}$, and may further be

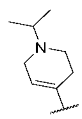.

In one embodiment, in ring B, the $C_6$-$C_{14}$ aryl substituted by one or more $R^{1-8}$ may be any one of the following groups:

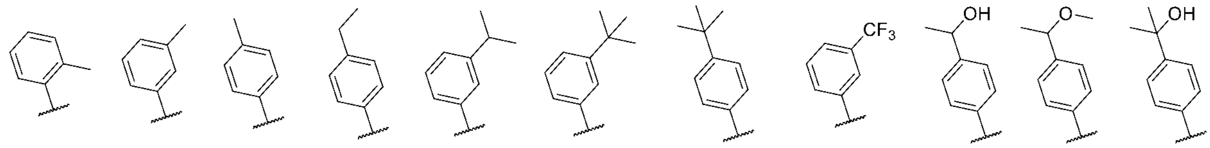

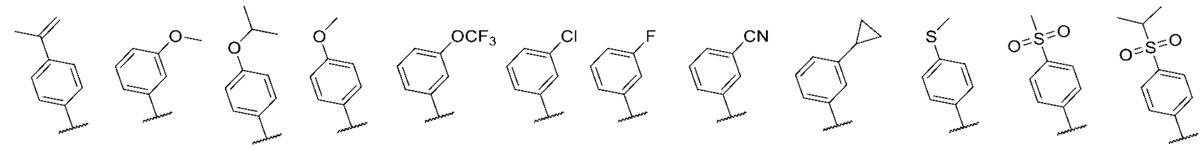

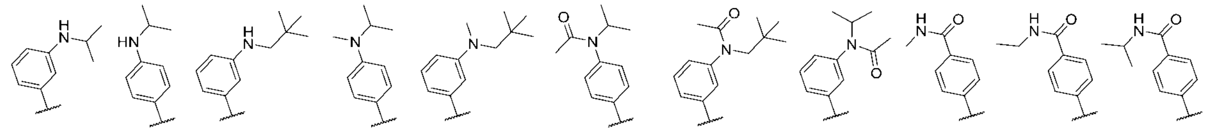

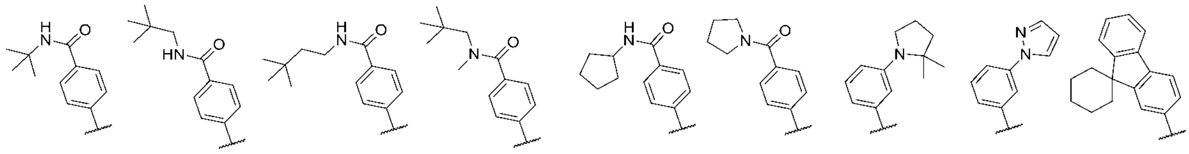,

such as

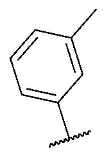, 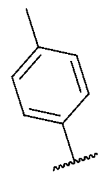, 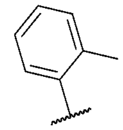, 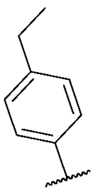, 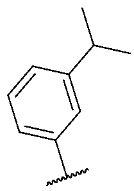, 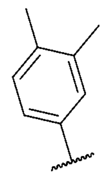, 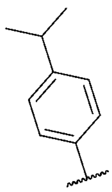, 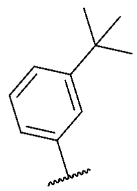,

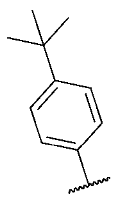, 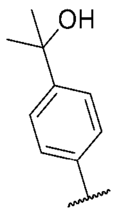, 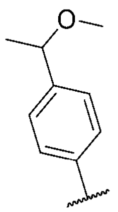, 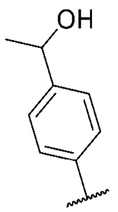, 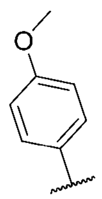, 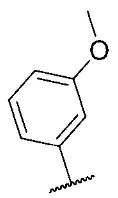, 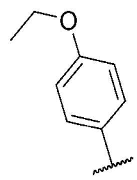, 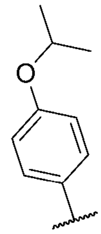,

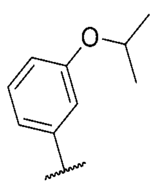, 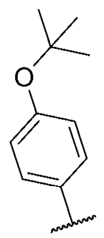, 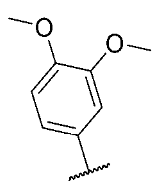, 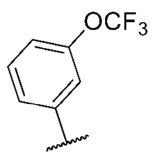, 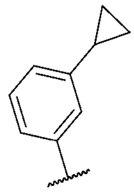, 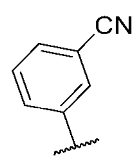, 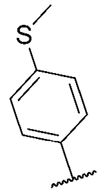,

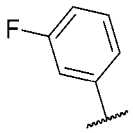, 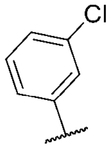, 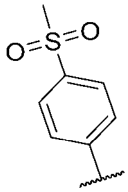, 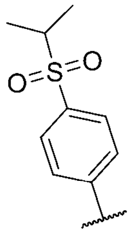, 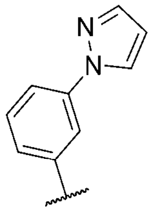, 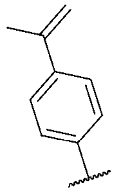, 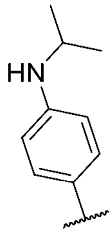,

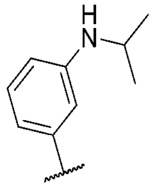, 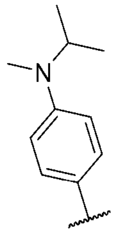, 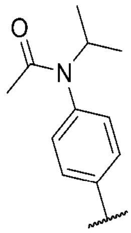, 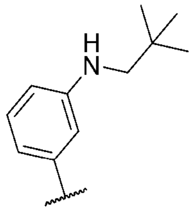, 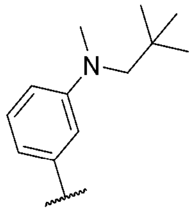, 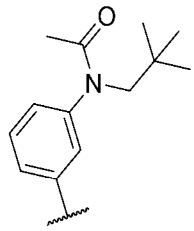,

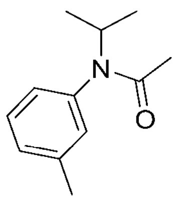, 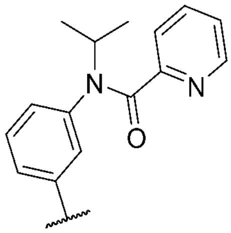, 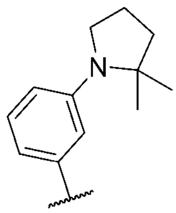, 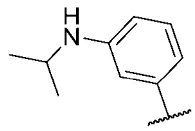, 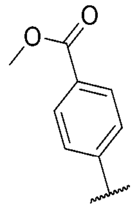, 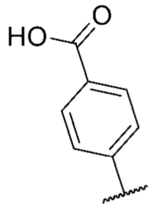,

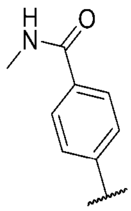, 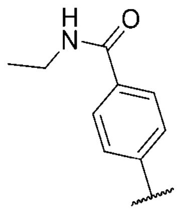, 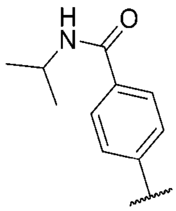, 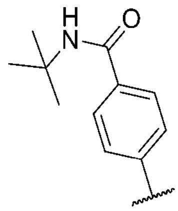, 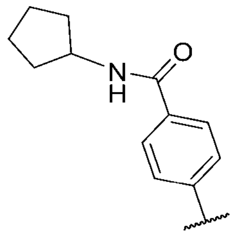,

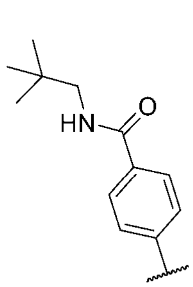, 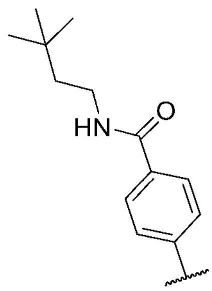, 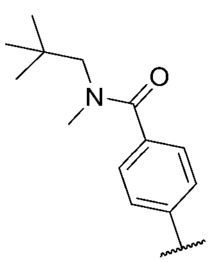, 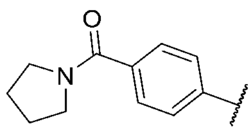, 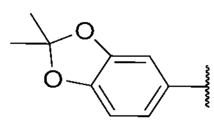,

or

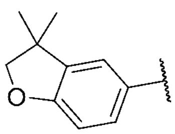.

In one embodiment, in ring B, the 5- to 10-membered heteroaryl substituted by one or more $R^{1-9}$ may be any one of the following groups:

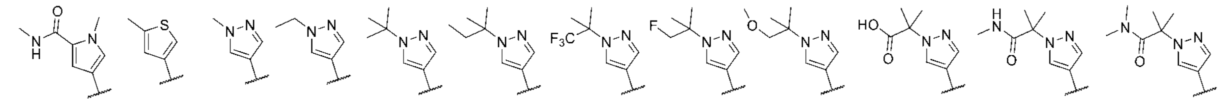

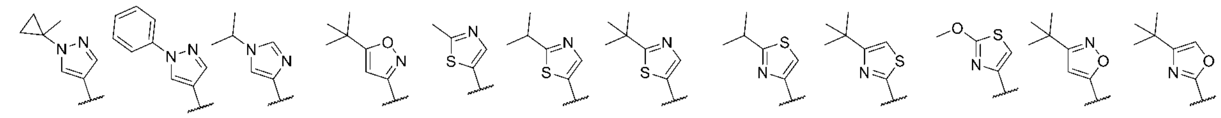

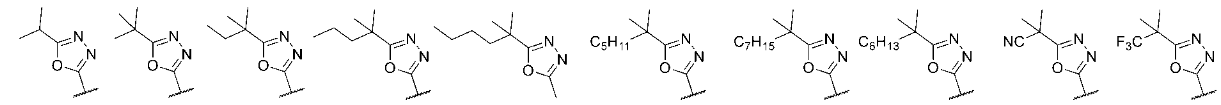

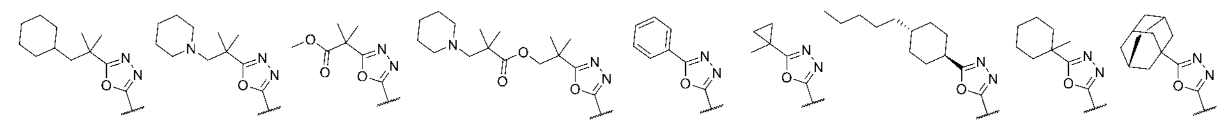

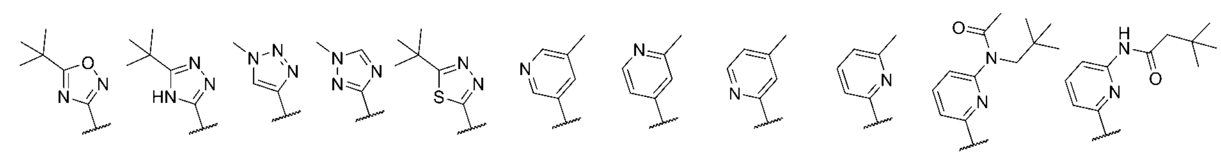

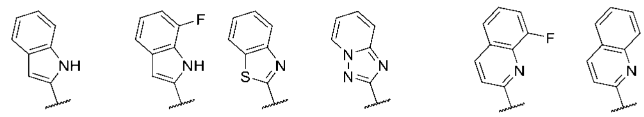,

such as

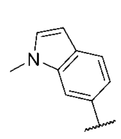, 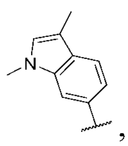, 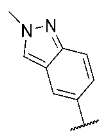, 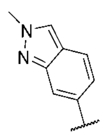, 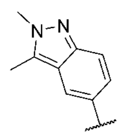, 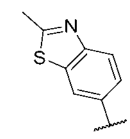, 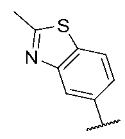, 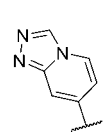,

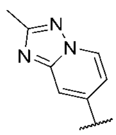, 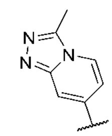, 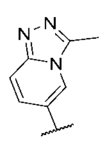, 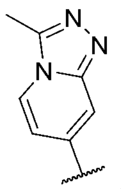, 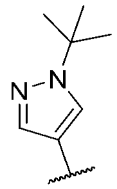, 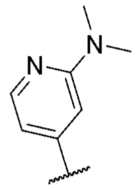, 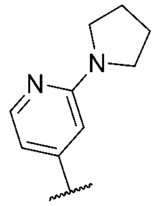, 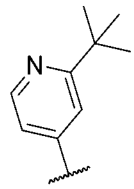,

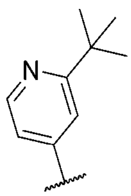, 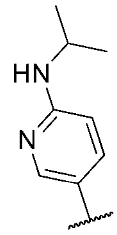, 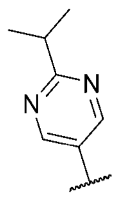, 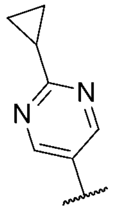, 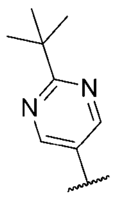, 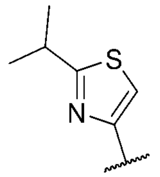, 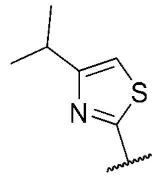,

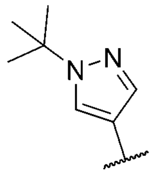, 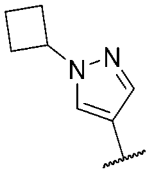, 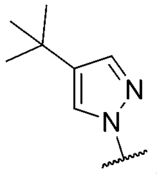, 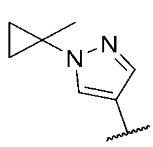, 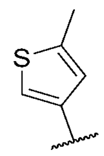, 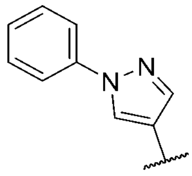, 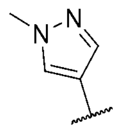,

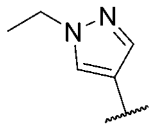, 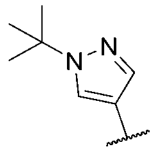, 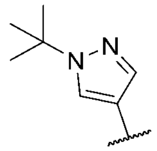, 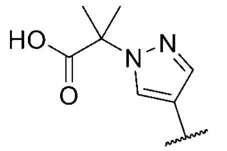, 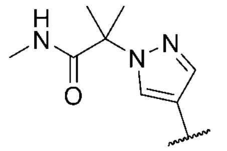,

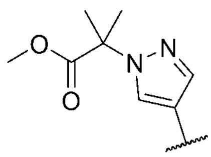, 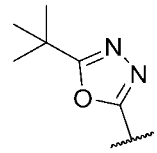, 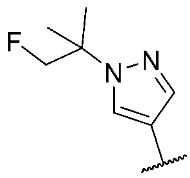, 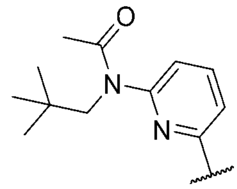, 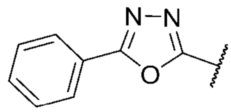,

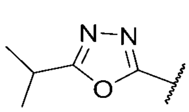, or 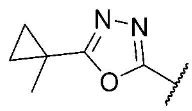.

In one embodiment, in $L^2$, the "$C_1$-$C_6$ alkylene" in the $C_1$-$C_6$ alkylene, the $C_1$-$C_6$ alkylene substituted by one or more $L^{2-1}$, the -O-$C_1$-$C_6$ alkylene, and the -N-$C_1$-$C_6$ alkylene may independently be methylene, ethylene, *n*-propylene, isopropylene, *n*-butylene, isobutylene, *sec*-butylene, or *tert*-butylene, such as methyl, ethyl, *n*-propyl, or isopropyl, for example, methylene or ethylene.

In one embodiment, when $L^2$ is $C_1$-$C_6$ alkylene, the C atom in the $C_1$-$C_6$ alkylene connected to

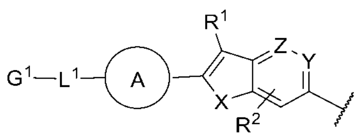

may be a non-chiral C, an *S*-configuration C, or an *R*-configuration C, preferably an *S*-configuration C.

In one embodiment, in $L^2$, the "$C_3$-$C_8$ cycloalkylene" in the $C_3$-$C_8$ cycloalkylene and the $C_3$-$C_8$ cycloalkylene substituted by one or more $L^{2-2}$ may independently be cyclopropylene, cyclobutylene, cyclopentylene, or cyclohexylene, such as cyclopropylene. In one embodiment, in each $L^{2-1}$ and each $L^{2-2}$, the halogen may independently be fluorine, chlorine, or bromine, such as fluorine.

In one embodiment, in each $L^{2-1}$ and each $L^{2-2}$, the "$C_1$-$C_6$ alkyl" in the $C_1$-$C_6$ alkyl and the $C_1$-$C_6$ alkyl substituted by one or more $L^{2-1-1}$ may independently be methyl, ethyl, *n*-propyl, isopropyl, *n*-butyl, isobutyl, *sec*-butyl, or *tert*-butyl, such as methyl, ethyl, *n*-propyl, or isopropyl.

In one embodiment, in each $L^{2-1}$ and each $L^{2-2}$, the "$C_1$-$C_6$ alkoxy" in the $C_1$-$C_6$ alkoxy and the $C_1$-$C_6$ alkoxy substituted by one or more $L^{2-1-2}$ may independently be methoxy, ethoxy, *n*-propoxy, isopropoxy, *n*-butoxy, isobutoxy, *sec*-butoxy, or *tert*-butoxy, such as methoxy or ethoxy.

In one embodiment, in each $L^{2-1}$ and each $L^{2-2}$, the "$C_3$-$C_8$ cycloalkyl" in the $C_3$-$C_8$ cycloalkyl and the $C_3$-$C_8$ cycloalkyl substituted by one or more $L^{2-1-3}$ may independently be cyclopropyl, cyclobutyl, cyclopentyl, or cyclohexyl, such as cyclopropyl or cyclobutyl.

In one embodiment, in each $L^{2-1}$ and each $L^{2-2}$, the "$C_2$-$C_6$ alkynyl" in the $C_2$-$C_6$ alkynyl and the $C_2$-$C_6$ alkynyl substituted by one or more $L^{2-1-4}$ may independently be ethynyl.

In one embodiment, in each $L^{2-1-1}$, each $L^{2-1-2}$, each $L^{2-1-3}$, and each $L^{2-1-4}$, the "$C_3$-$C_8$ cycloalkyl" in the $C_3$-$C_8$ cycloalkyl and the $C_3$-$C_8$ cycloalkyl substituted by one or more $L^{2-1-1-1}$ may independently be cyclopropyl, cyclobutyl, cyclopentyl, or cyclohexyl, such as cyclopropyl.

In one embodiment, in $G^2$, the "5- to 10-membered heteroaryl" in the 5- to 10-membered heteroaryl and the 5- to

10-membered heteroaryl substituted by one or more $G^{2-4}$ may independently be 5- to 6-membered heteroaryl with 2, 3, or 4 heteroatoms selected from one or more types of N, O, and S, and may further be 5- to 6-membered heteroaryl with 3 or 4 heteroatoms being N and/or O, such as tetrazolyl,

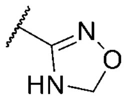,

oxazolyl (

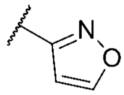

), or

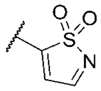.

In one embodiment, in $G^2$, the "3- to 8-membered heterocycloalkenyl" in the 3-to 8-membered heterocycloalkenyl and the 3- to 8-membered heterocycloalkenyl substituted by one or more $G^{2-5}$ may independently be 3- to 5-membered heterocycloalkenyl with 2 or 3 heteroatoms being N and/or S, such as

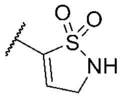.

In one embodiment, in $G^{2-1}$, $G^{2-2}$, and $G^{2-3}$, the "$C_1$-$C_6$ alkyl" in the -$S(=O)_2$-$C_1$-$C_6$ alkyl, $C_3$-$C_8$ cycloalkyl, the $C_1$-$C_6$ alkyl, and the $C_1$-$C_6$ alkyl substituted by one or more $G^{2-2-1}$ may independently be methyl, ethyl, *n*-propyl, isopropyl, *n*-butyl, isobutyl, *sec*-butyl, or *tert*-butyl, such as methyl or ethyl.

In one embodiment, in $G^{2-1}$, $G^{2-2}$, and $G^{2-3}$, the $C_3$-$C_8$ cycloalkyl may independently be cyclopropyl, cyclobutyl, cyclopentyl, or cyclohexyl, such as cyclopropyl.

In one embodiment, in $G^{2-2}$ and $G^{2-3}$, the "5- to 10-membered heteroaryl" in the -NH(=O)-5- to 10-membered heteroaryl is 5- to 6-membered heteroaryl with 1 or 2 heteroatoms being N, such as pyridyl.

In one embodiment, X may be O.

In one embodiment, Z and Y may be C.

In one embodiment, $R^2$ is hydrogen.

In one embodiment, $G^1$ may be $C_1$-$C_6$ alkyl, $C_6$-$C_{14}$ aryl, $C_6$-$C_{14}$ aryl substituted by one or more $G^{1-2}$, 5- to 10-membered heteroaryl, or 5- to 10-membered heteroaryl substituted by one or more $G^{1-3}$.

In one embodiment, each $G^{1-2}$ may independently be halogen, cyano, -$NG^{1-1-1}G^{1-1-2}$, -$S(=O)_2$-$C_1$-$C_6$ alkyl, $C_1$-$C_6$ alkyl, $C_1$-$C_6$ alkyl substituted by one or more $G^{1-1-5}$, $C_1$-$C_6$ alkoxy, $C_1$-$C_6$ alkoxy substituted by one or more $G^{1-1-6}$, -S-$C_1$-$C_6$ alkyl, $C_3$-$C_8$ cycloalkyl, $C_3$-$C_8$ cycloalkyl substituted by one or more $G^{1-1-8}$, or -O-$C_3$-$C_8$ cycloalkyl.

In one embodiment, each $G^{1-3}$ is independently halogen or $C_1$-$C_6$ alkoxy.

In one embodiment, ring A may be 4- to 6-membered heterocycloalkyl or 4- to 6-membered heterocycloalkyl substituted by one or more $A^1$; the "4- to 6-membered heterocycloalkyl" in the 4- to 6-membered heterocycloalkyl and the 4- to 6-membered heterocycloalkyl substituted by one or more $A^1$ has 1 heteroatom being N.

Preferably, when ring A is 4- to 6-membered heterocycloalkyl or 4- to 6-membered heterocycloalkyl substituted by one or more $A^3$, then $L^1$ is a bond, and $G^1$ is connected to ring A through a heteroatom.

In one embodiment, each $A^1$ is independently halogen or $C_1$-$C_6$ alkyl.

In one embodiment, $L^2$ may be $C_1$-$C_6$ alkylene or $C_1$-$C_6$ alkylene substituted by one or more $L^{2-1}$

In one embodiment,

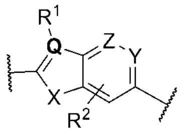

may be

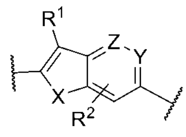 or 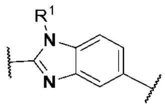,

and

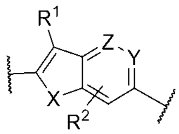

may be

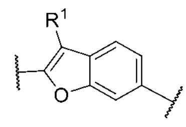 or 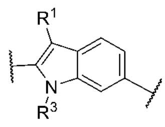.

In one embodiment,

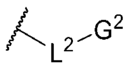

may be any one of the following groups:

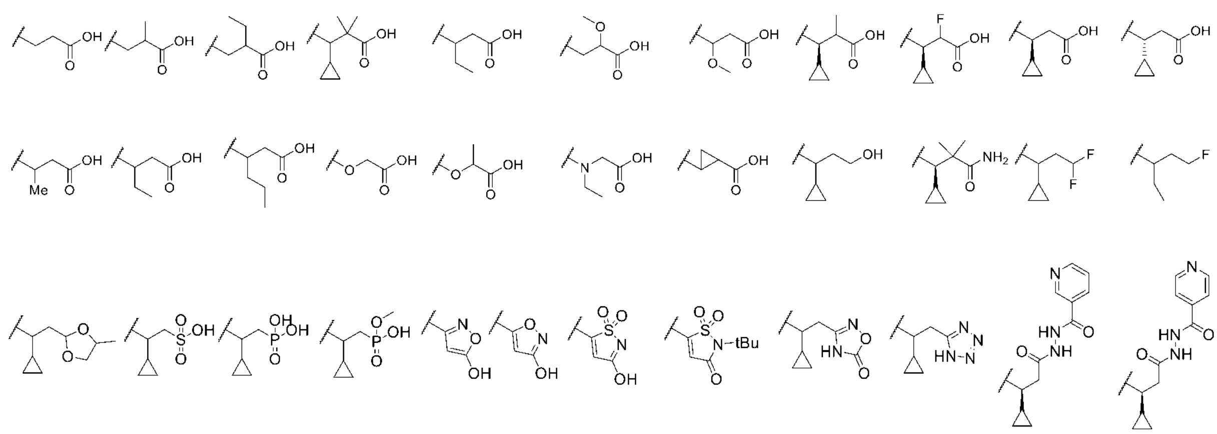

, such as

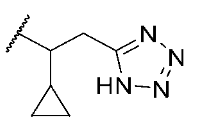, 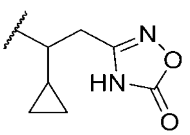, 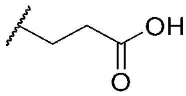, 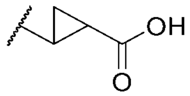,

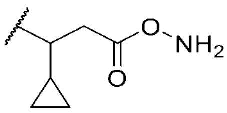, 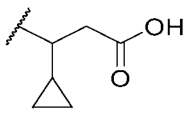, 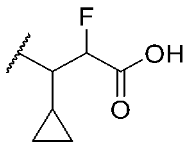, 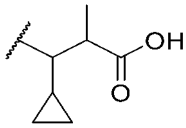, 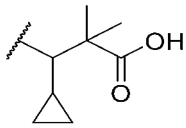,

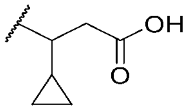

(such as

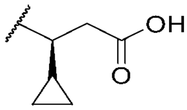

),

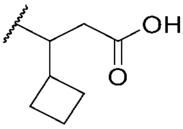, 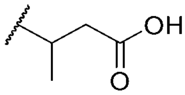, 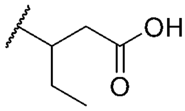,

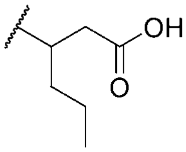, 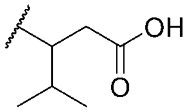, 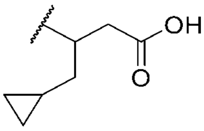, 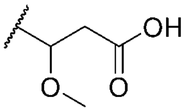, 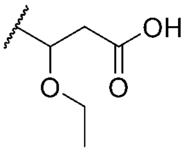,

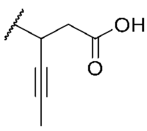, 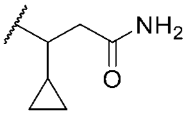, 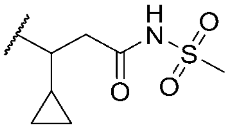, 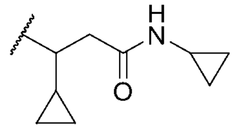, 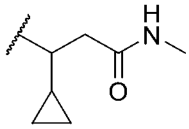,

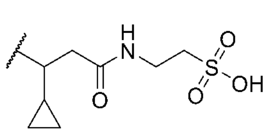, or 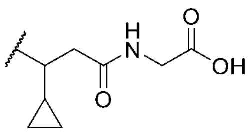.

In one embodiment, the compound of formula I may be the following general formula I-1:

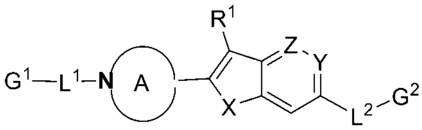

**I-1**

;

in formula I-1, the N in ring A represents a nitrogen atom, and all other definitions are as described above.

Preferably, ring A is azetidinyl, pyrrolidinyl, or piperidinyl.

More preferably, $L^2$ is $C_1$-$C_6$ alkylene substituted by one or more $L^{2-1}$, at least one $L^{2-1}$ is $C_3$-$C_8$ cycloalkyl, and $L^{2-1}$ is substituted at the terminal group of $L^2$.

In one embodiment, the compound of formula I may be the following general formulas I-2 to I-14:

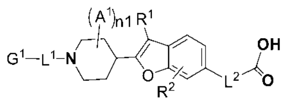
I-3

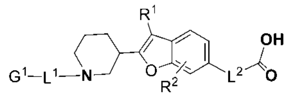
I-4

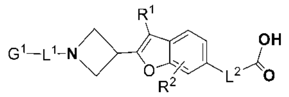
I-5

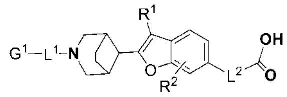
I-6

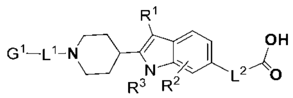
I-7

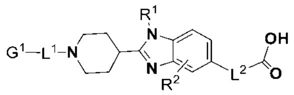
I-8

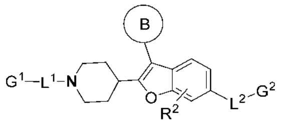
I-9

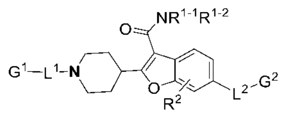
I-10

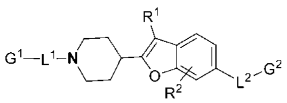
I-11

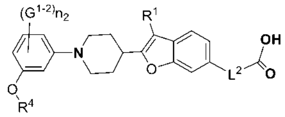
I-12

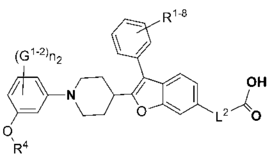
I-13

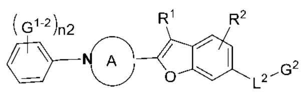
I-14

;

in formula I-3, n1 is 0, 1, or 2;

in formula I-11, $R^1$ is $C_1$-$C_6$ alkyl, $C_1$-$C_6$ alkyl substituted by one or more $R^{1-3}$, $C_2$-$C_6$ alkenyl, or $C_2$-$C_6$ alkenyl substituted by one or more $R^{1-10}$;

in formula I-12, n2 is 0, 1, or 2; $R^4$ is $C_1$-$C_6$ alkyl, or $R^4$ and $G^{1-2}$ together form - $(CH_2)n_3$-, wherein $n_3$ is 1, 2, or 3, and 1 or 2 of the $-(CH_2)n_3$- in $-(CH_2)n_3$- are optionally replaced by a group selected from: $-CHR^{4a}$-, $-CR^{4b}R^{4c}$-, -NH-, -O-, and -C(=O)-; $R^{4a}$, $R^{4b}$, and $R^{4c}$ are independently $C_1$-$C_6$ alkyl or halogen (*e.g.*,

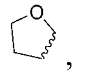 , 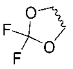 , 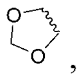 , 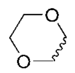 ,

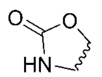

);

in formula I-13, n2 is 0, 1, or 2; (*e.g.,* $R^4$ is $C_1$-$C_6$ alkyl; $G^{1-2}$ is halogen; $R^4$ is $C_1$-$C_6$ alkyl);

in formula I-14, n2 is 0, 1, or 2 (*e.g.,* $R^2$ is hydrogen or halogen);

the definitions of other groups in formulas I-1 to I-14 are as described above (*e.g.*, the definitions of groups $G^{1-2}$, $A^1$, $G^1$, $G^2$, $L^1$, $L^2$, $R^1$, $R^2$, $R^3$, $R^{1-1}$, $R^{1-2}$, and ring B in I-1 to I-10 are as described above).

Preferably:

in formula I-9, ring B is $C_3$-$C_{12}$ cycloalkyl, $C_3$-$C_{12}$ cycloalkyl substituted by one or more $R^{1-4}$, $C_3$-$C_{12}$ cycloalkenyl, $C_3$-$C_{12}$ cycloalkenyl substituted by one or more $R^{1-5}$, 3- to 12-membered heterocycloalkyl, 3- to 12-membered heterocycloalkyl substituted by one or more $R^{1-6}$, 3- to 12-membered heterocycloalkenyl, 3- to 12-membered heterocycloalkenyl substituted by one or more $R^{1-7}$, $C_6$-$C_{14}$ aryl, $C_6$-$C_{14}$ aryl substituted by one or more $R^{1-8}$, 5- to 14-membered heteroaryl, or 5- to 14-membered heteroaryl substituted by one or more $R^{1-9}$;

each $R^{1-4}$, each $R^{1-5}$, each $R^{1-6}$, each $R^{1-7}$, each $R^{1-8}$, and each $R^{1-9}$ is independently halogen, cyano, hydroxyl, $-NR^{1-3-1a}R^{1-3-2a}$, $-C(=O)NR^{1-3-3a}R^{1-3-4a}$, $-C(=O)R^{1-3-5a}$, $-S(=O)_2$-$C_1$-$C_{12}$ alkyl, -S-$C_1$-$C_{12}$ alkyl, $C_1$-$C_{12}$ alkyl, $C_1$-$C_{12}$ alkyl substituted by one or more $R^{1-3-6a}$, $C_1$-$C_{12}$ alkoxy, $C_1$-$C_{12}$ alkoxy substituted by one or more $R^{1-3-7a}$, 5- to 14-membered heteroaryl, 5- to 14-membered heteroaryl substituted by one or more $R^{1-3-8a}$, $C_3$-$C_{12}$ cycloalkyl, $C_3$-$C_{12}$ cycloalkyl substituted by one or more $R^{1-3-9a}$, $C_2$-$C_6$ alkenyl, $C_2$-$C_6$ alkenyl substituted by one or more $R^{1-3-10a}$, $C_6$-$C_{14}$ aryl, $C_6$-$C_{14}$ aryl substituted by one or more $R^{1-3-11a}$, -O-$C_6$-$C_{14}$ aryl, -O-C(=O)$C_6$-$C_{14}$ aryl, -O-5- to 10-membered heteroaryl, 3-to 12-membered heterocycloalkyl, or 3- to 12-membered heterocycloalkyl substituted by one or more $R^{1-3-13a}$; alternatively, any two adjacent $R^{1-8}$, together with the carbon atom to which they are attached, form a $C_3$-$C_{14}$ cycloalkyl;

$R^{1-3-1a}$, $R^{1-3-2a}$, $R^{1-3-3a}$, and $R^{1-3-4a}$ are independently H, $C_1$-$C_6$ alkyl, $-C(=O)R^{1-3-1-1a}$, or $C_3$-$C_8$ cycloalkyl;

$R^{1-3-1-1a}$ is $C_1$-$C_6$ alkyl;

$R^{1-3-5a}$ is $C_3$-$C_8$ cycloalkyl or 3- to 8-membered heterocycloalkyl;

each $R^{1-3-6a}$ and each $R^{1-3-7a}$ is independently halogen, hydroxyl, carboxyl, cyano, $C_1$-$C_6$ alkoxy, $C_3$-$C_8$ cycloalkyl, 3- to 12-membered heterocycloalkyl, or -O-C(=O)-$C_1$-$C_6$ alkyl-3- to 12-membered heterocycloalkyl;
each $R^{1-3-8a}$, each $R^{1-3-9a}$, each $R^{1-3-10a}$, each $R^{1-3-11a}$, and each $R^{1-3-12a}$ is independently $C_1$-$C_6$ alkyl.

Preferably,

in formula I-10, $R^{1-1}$ and $R^{1-2}$ are independently H, -S(=O)$_2$$C_1$-$C_{12}$ alkyl, $C_1$-$C_{12}$ alkyl, $C_1$-$C_{12}$ alkyl substituted by one or more $R^{1-1-1}$, $C_3$-$C_{12}$ cycloalkyl, $C_3$-$C_{12}$ cycloalkyl substituted by one or more $R^{1-1-2}$, $C_6$-$C_{14}$ aryl, $C_6$-$C_{14}$ aryl substituted by one or more $R^{1-1-3}$, 5- to 14-membered heteroaryl, or 5- to 14-membered heteroaryl substituted by one or more $R^{1-1-4}$,
alternatively, $R^{1-1}$ and $R^{1-2}$, together with the N atom to which they are attached, form a 3- to 14-membered heterocycloalkyl or a 3- to 14-membered heterocycloalkyl substituted by one or more $R^{1-1-5}$;
each $R^{1-1-1}$ and each $R^{1-1-2}$ is independently halogen, cyano, nitro, hydroxyl, amino, -NH($C_1$-$C_{12}$ alkyl), -N($C_1$-$C_{12}$ alkyl)$_2$, $C_3$-$C_{12}$ cycloalkyl, $C_3$-$C_{12}$ cycloalkyl substituted by one or more $R^{1-1-1-3}$, $C_6$-$C_{14}$ aryl, 3- to 12-membered heterocycloalkyl, or 5- to 14-membered heteroaryl;
each $R^{1-1-3}$, $R^{1-1-4}$, and each $R^{1-1-5}$ is independently halogen, cyano, nitro, hydroxyl, amino, -NH($C_1$-$C_{12}$ alkyl), -N($C_1$-$C_{12}$ alkyl)$_2$, -C(=O)-$C_1$-$C_{12}$ alkyl, -NHC(=O)-$C_1$-$C_{12}$ alkyl, $C_1$-$C_{12}$ alkyl, $C_1$-$C_{12}$ alkyl substituted by one or more $R^{1-1-1-1}$, $C_1$-$C_{12}$ alkoxy, $C_1$-$C_{12}$ alkoxy substituted by one or more $R^{1-1-1-2}$, $C_3$-$C_{12}$ cycloalkyl, $C_3$-$C_{12}$ cycloalkyl substituted by one or more $R^{1-1-1-3}$, $C_6$-$C_{14}$ aryl, 3- to 12-membered heterocycloalkyl, or 5- to 14-membered heteroaryl;
each $R^{1-1-1-1}$, each $R^{1-1-1-2}$, and each $R^{1-1-1-3}$ is independently halogen, $C_1$-$C_{12}$ alkyl, or $C_3$-$C_{12}$ cycloalkyl.

Preferably,

in formula I-11, each $R^{1-3}$ and each $R^{1-10}$ is independently deuterium, halogen, cyano, hydroxyl, -$NR^{1-3-1}R^{1-3-2}$, -C(=O)$NR^{1-3-3}R^{1-3-4}$, -C(=O)$R^{1-3-5}$, -S(=O)$_2$-$C_1$-$C_{12}$ alkyl, -S-$C_1$-$C_{12}$ alkyl, $C_1$-$C_{12}$ alkyl, $C_1$-$C_{12}$ alkyl substituted by one or more $R^{1-3-6}$, $C_1$-$C_{12}$ alkoxy, $C_1$-$C_{12}$ alkoxy substituted by one or more $R^{1-3-7}$, 5- to 14-membered heteroaryl, 5- to 14-membered heteroaryl substituted by one or more $R^{1-3-8}$, $C_3$-$C_{12}$ cycloalkyl, $C_3$-$C_{12}$ cycloalkyl substituted by one or more $R^{1-3-9}$, $C_2$-$C_6$ alkenyl, $C_6$-$C_{14}$ aryl, $C_6$-$C_{14}$ aryl substituted by one or more $R^{1-3-11}$, -O-$C_6$-$C_{14}$ aryl, -O-C(=O) $C_6$-$C_{14}$ aryl, -O-5- to 14-membered heteroaryl, $C_3$-$C_8$ cycloalkenyl, $C_3$-$C_8$ cycloalkenyl substituted by one or more $R^{1-3-12}$, 3- to 12-membered heterocycloalkyl, or 3- to 12-membered heterocycloalkyl substituted by one or more $R^{1-3-13}$
$R^{1-3-1}$, $R^{1-3-2}$, $R^{1-3-3}$, and $R^{1-3-4}$ are independently H, $C_1$-$C_6$ alkyl, -$C_1$-$C_6$ alkyl-$C_6$-$C_{14}$ aryl, -C(=O)$R^{1-3-1-1}$, $C_6$-$C_{14}$ aryl, or $C_6$-$C_{14}$ aryl substituted by one or more $R^{1-3-1-4}$,
each $R^{1-3-6}$ and each $R^{1-3-7}$ is independently $C_3$-$C_8$ cycloalkyl, 3- to 8-membered heterocycloalkyl, $C_2$-$C_6$ alkenyl, 5- to 10-membered heteroaryl, or 5- to 10-membered heteroaryl substituted by one or more $C_1$-$C_6$ alkyl groups;
each $R^{1-3-8}$, each $R^{1-3-9}$, each $R^{1-3-10}$, each $R^{1-3-11}$, each $R^{1-3-12}$, and each $R^{1-3-13}$ is independently halogen, hydroxyl, carboxyl, cyano, $C_1$-$C_6$ alkyl, or $C_1$-$C_6$ alkoxy.

In one embodiment, the compound of formula I is preferably any one of the following compounds:

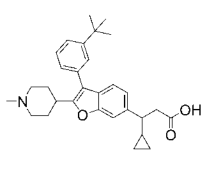 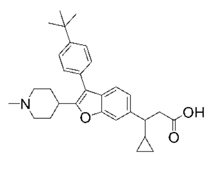 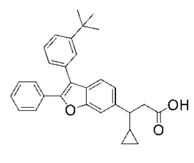 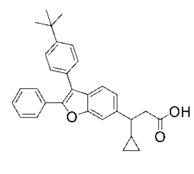

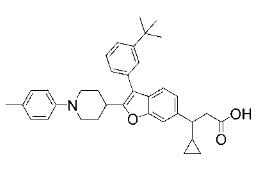 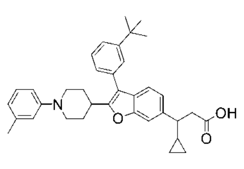 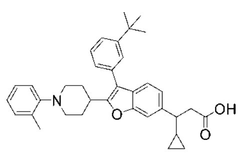 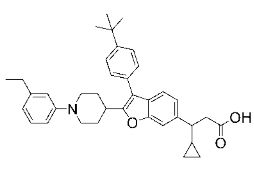

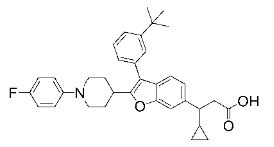
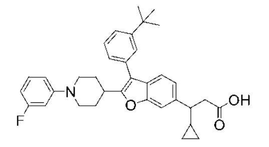
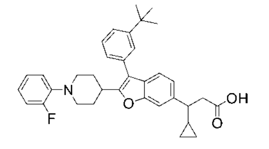
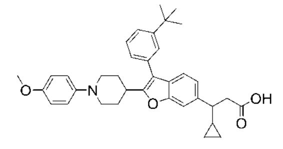

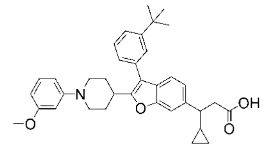
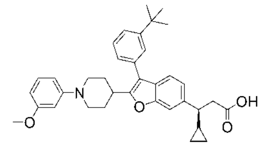
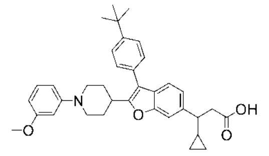
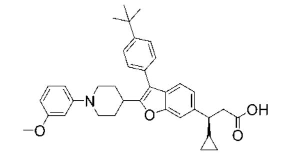

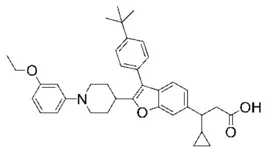
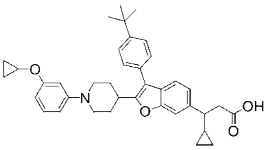
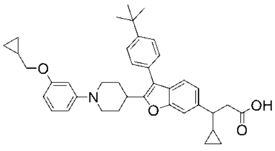
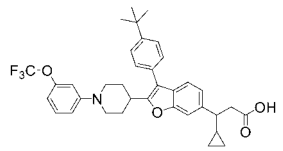

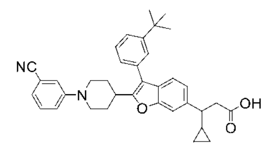
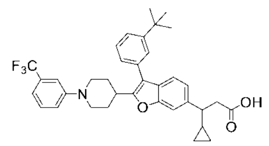
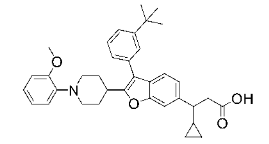
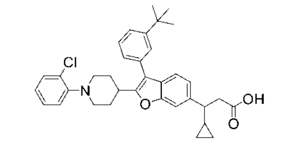

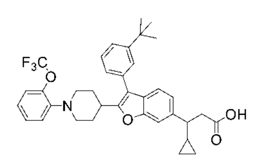
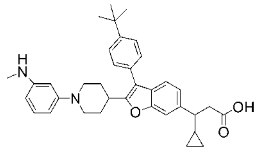
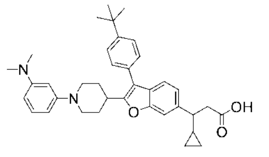
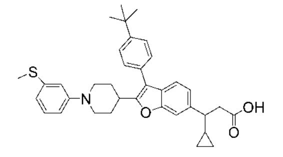

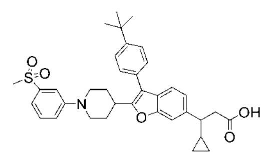
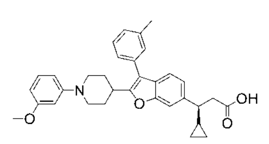
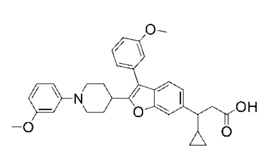
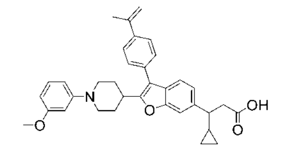

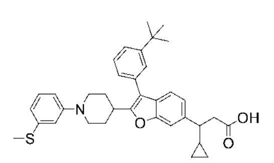
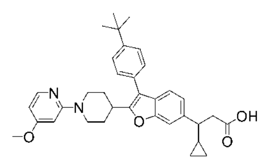
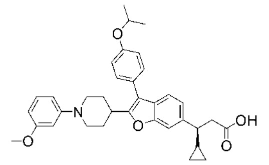
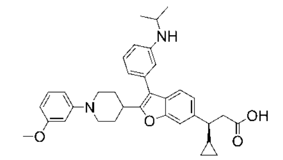

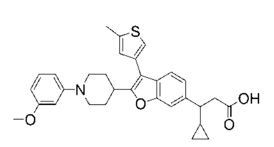
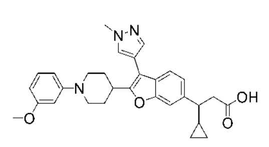
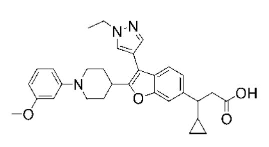
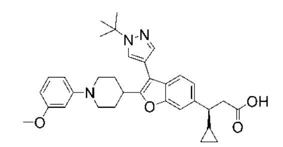

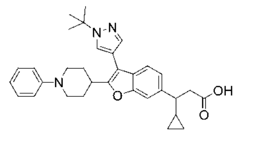
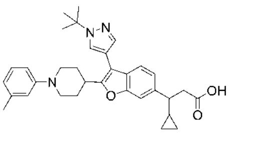
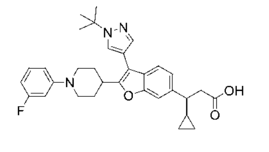
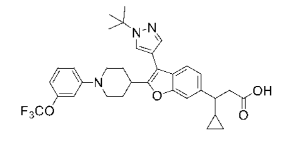

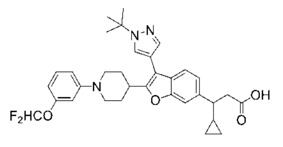
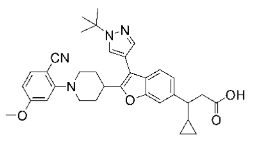
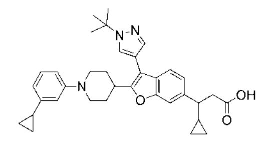
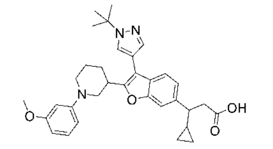

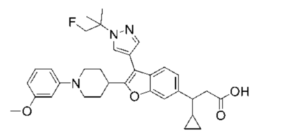
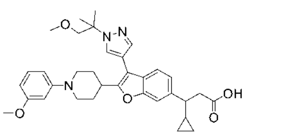
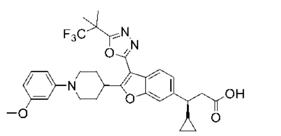
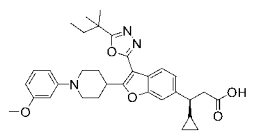

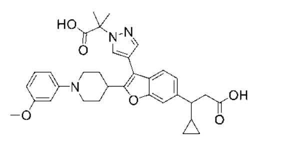
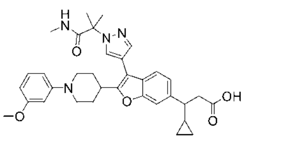
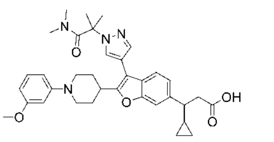
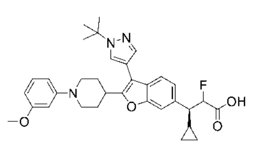

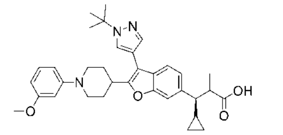
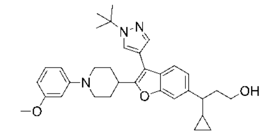
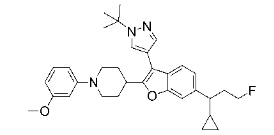
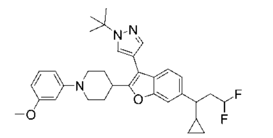

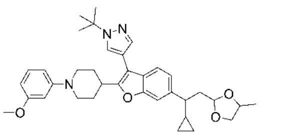
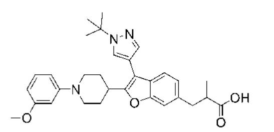
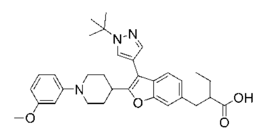
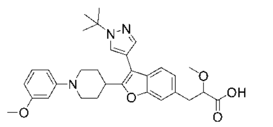

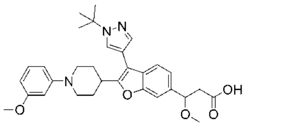
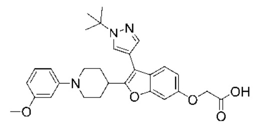
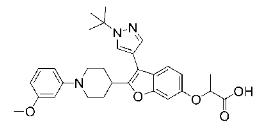
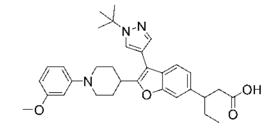

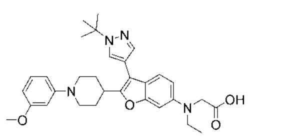
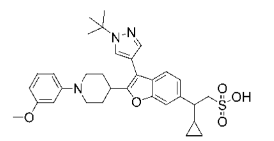
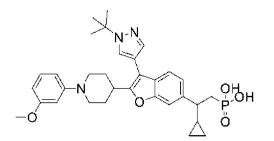
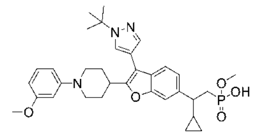

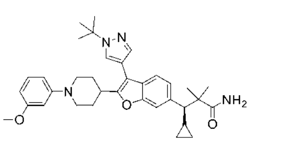
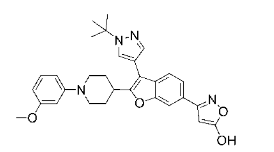
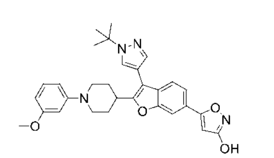
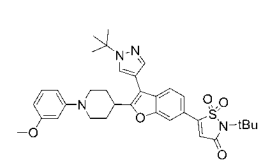

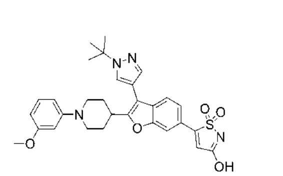
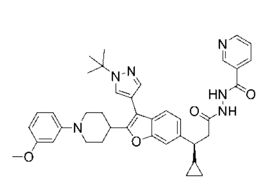
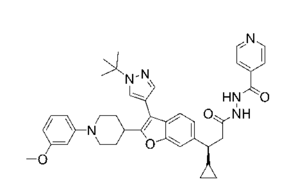
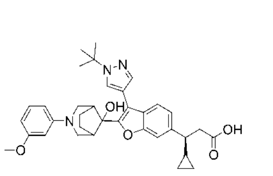

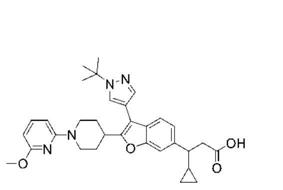
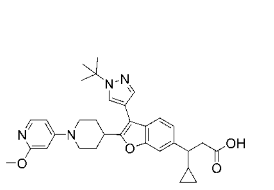
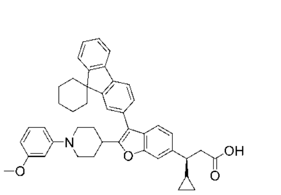
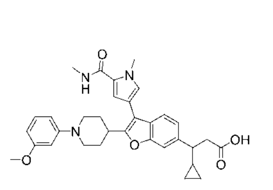

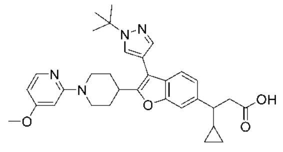
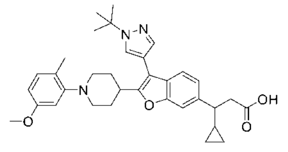
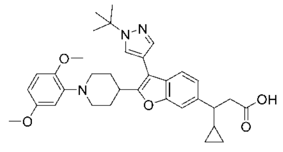
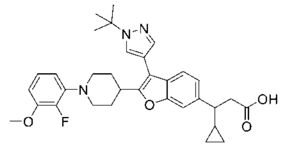

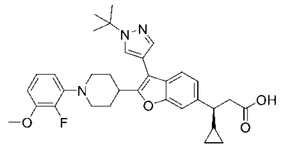
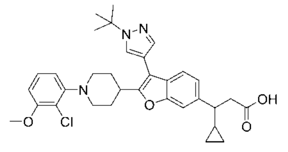
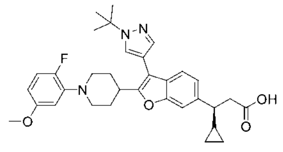
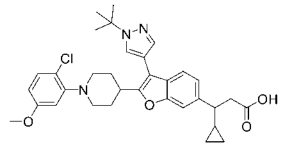

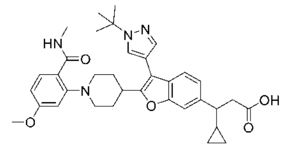
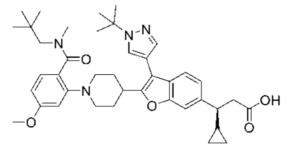
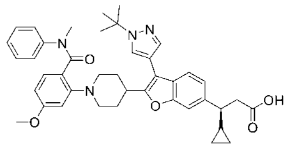
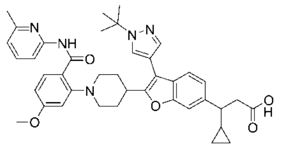

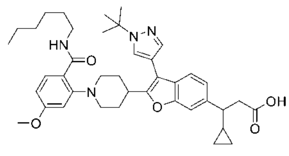
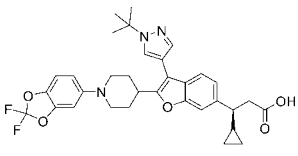
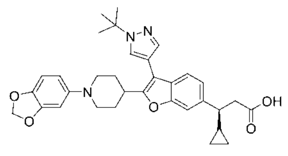
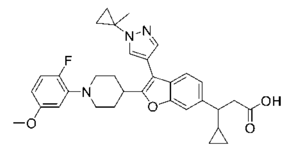

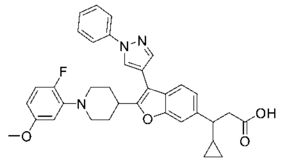
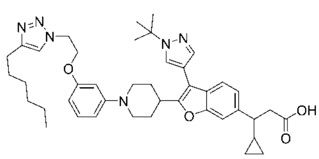
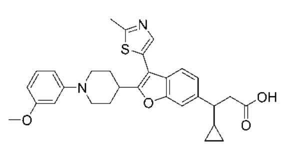
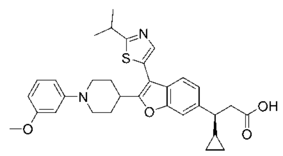

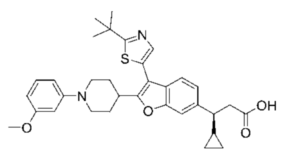
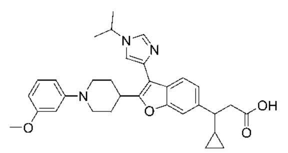
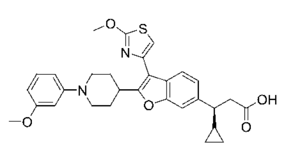
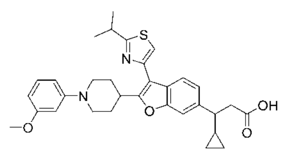

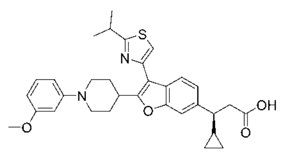
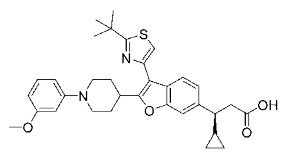
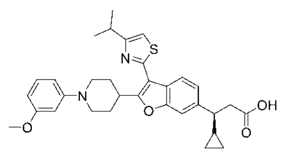
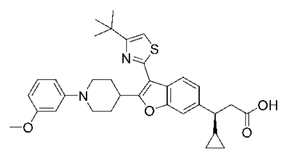

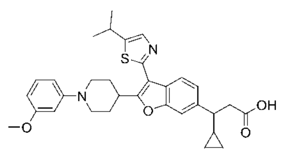
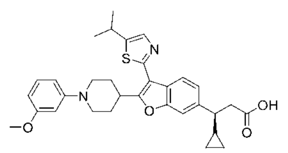
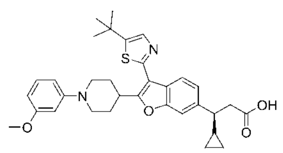
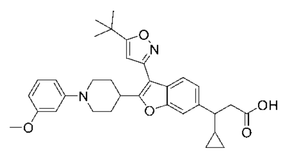

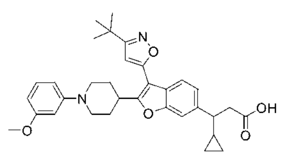
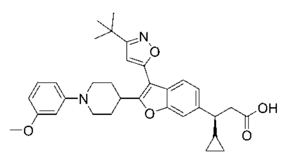
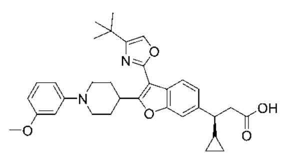
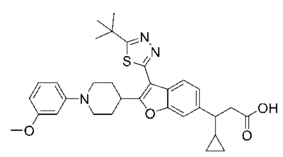

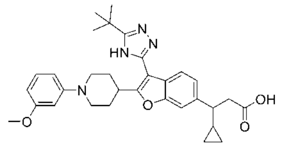
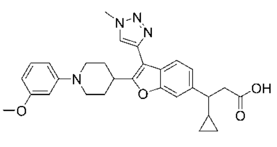
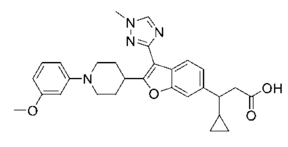
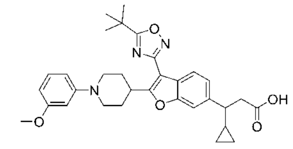

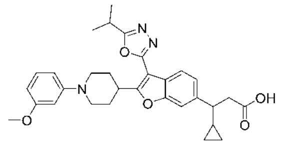
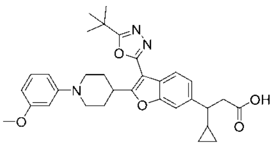
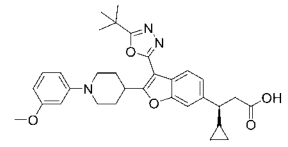
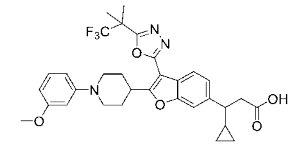

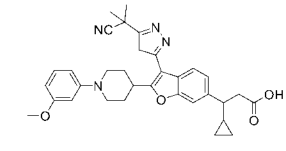
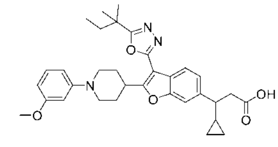
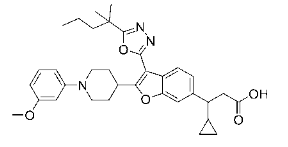
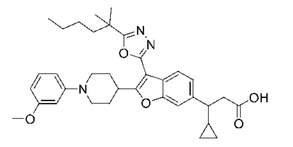

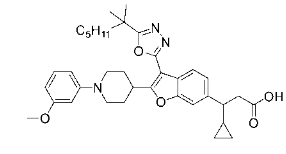
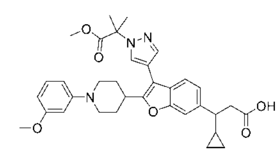
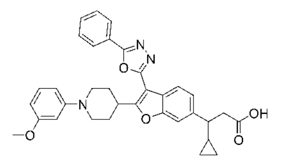
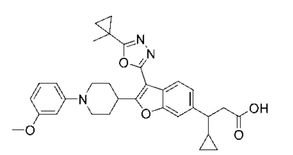

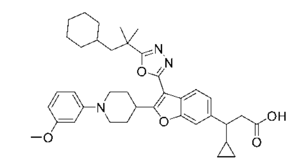
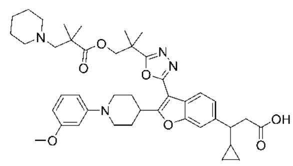
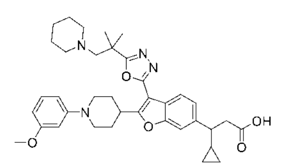
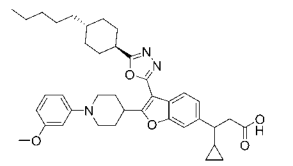

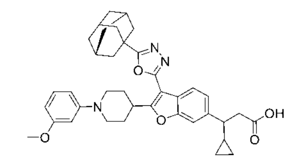
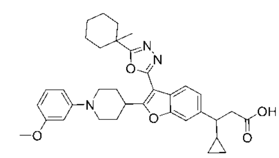
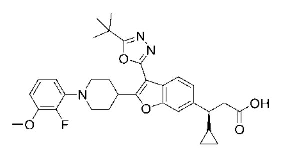
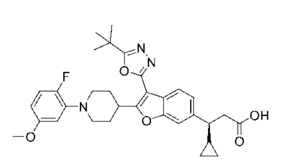

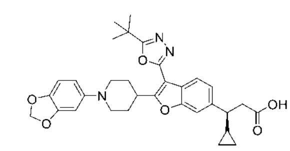
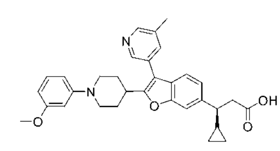
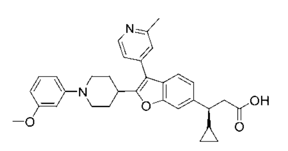
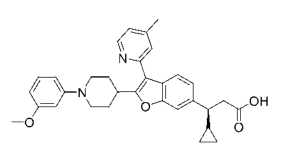

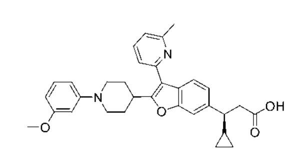
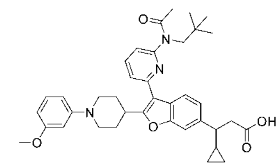
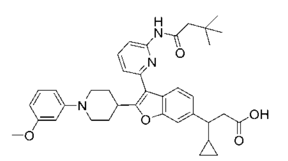
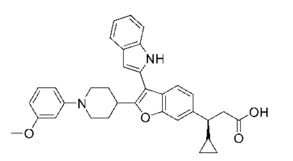

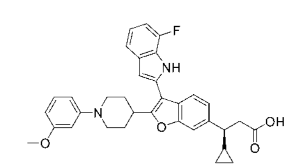
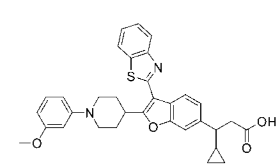
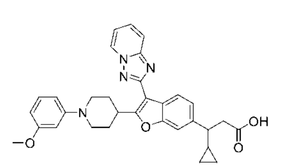
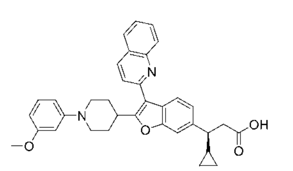

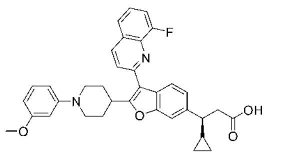 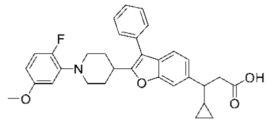 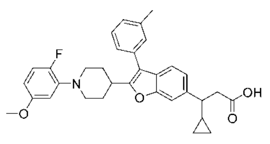 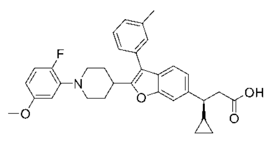

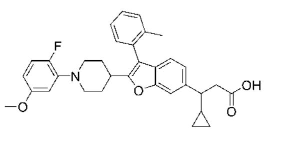 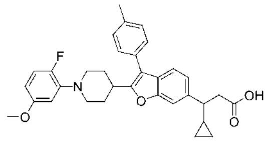 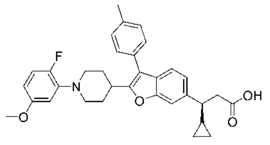 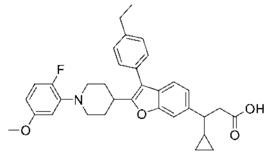

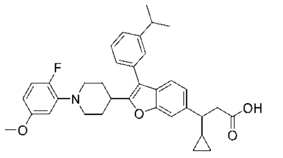 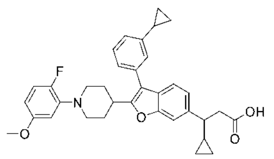 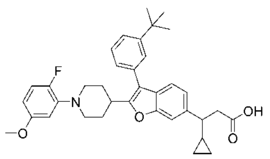 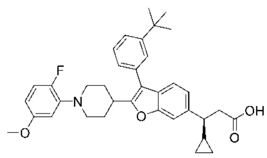

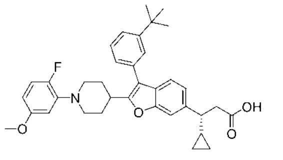 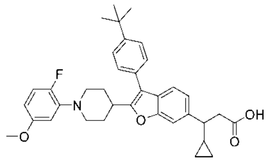 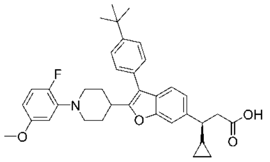 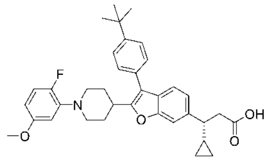

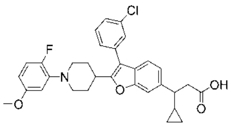 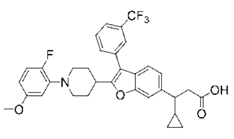 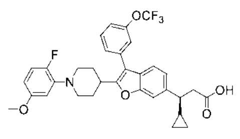 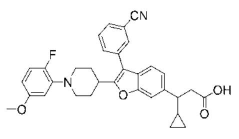

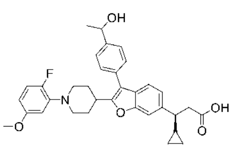 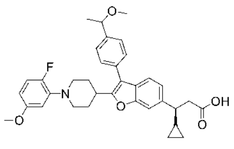 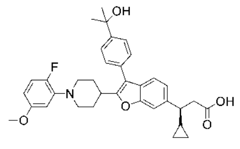 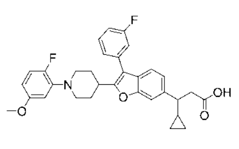

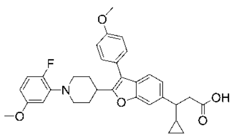 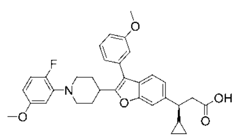 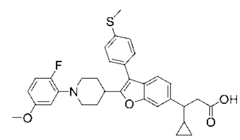 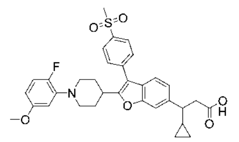

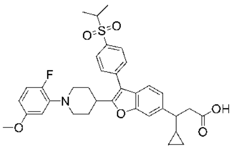 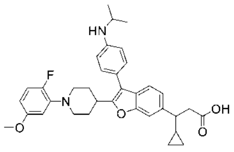 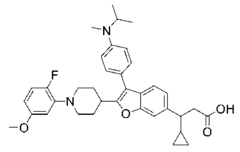 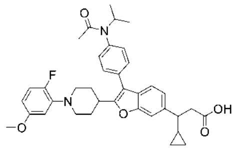

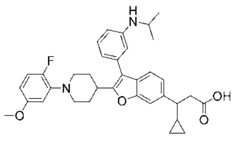 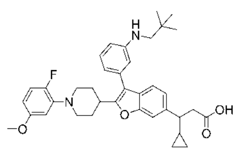 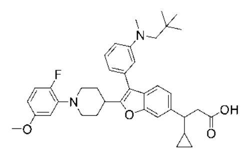 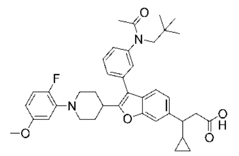

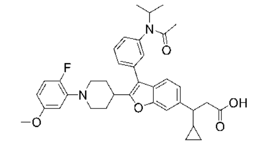
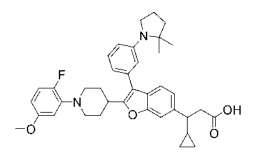
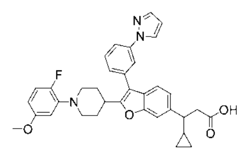
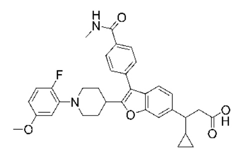
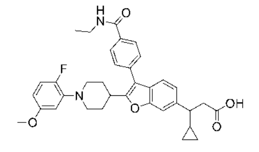
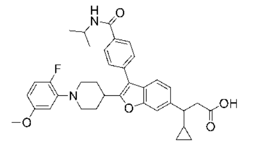
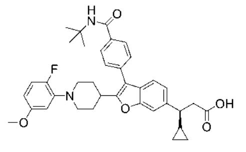
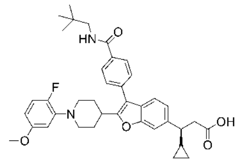
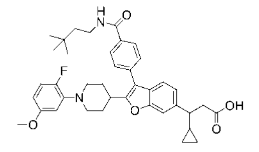
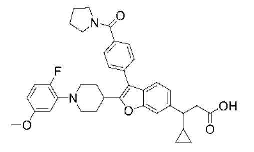
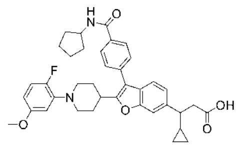
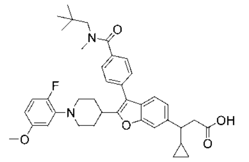
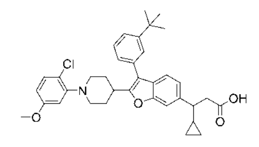
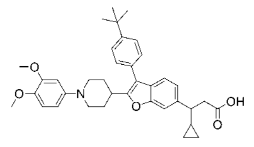
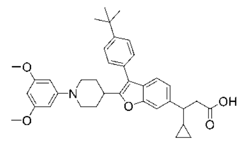
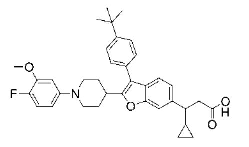
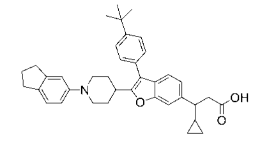
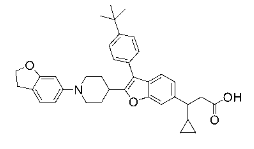
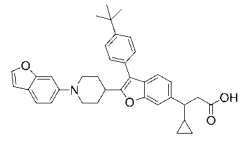
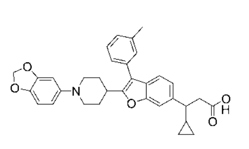
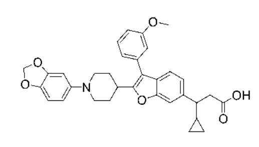
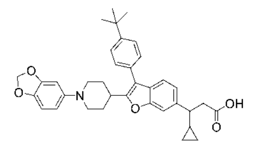
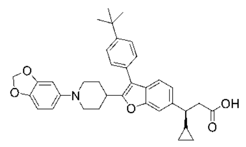
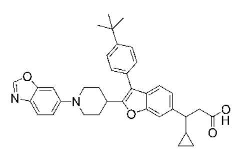
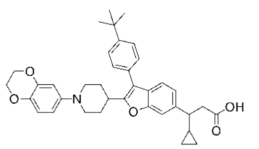
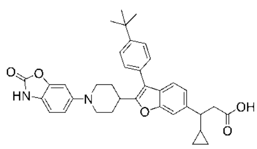
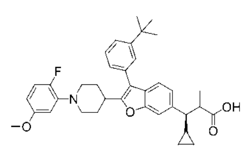
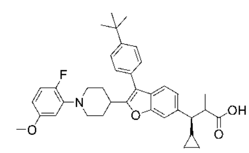
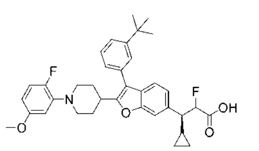
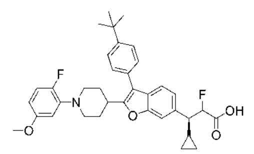
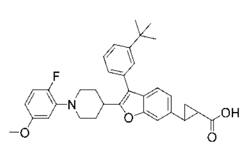
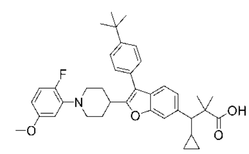
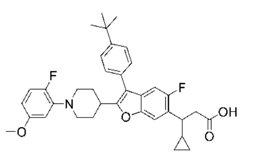
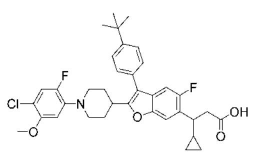
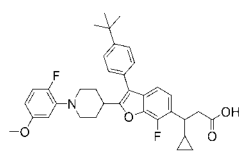
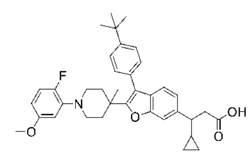

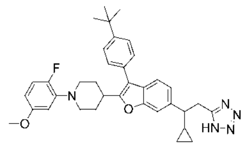
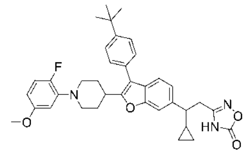
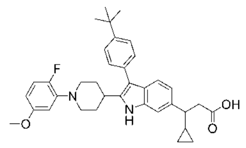
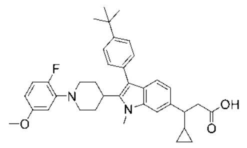
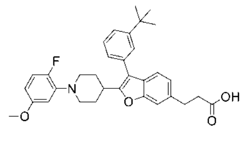
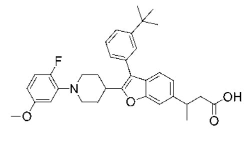
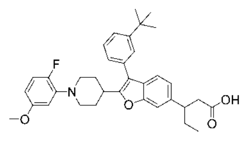
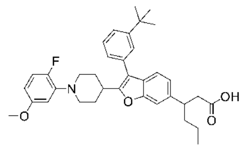
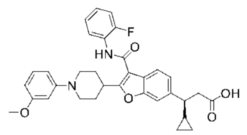
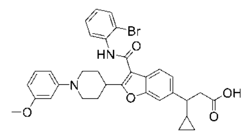
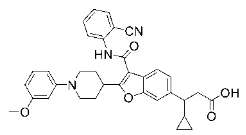
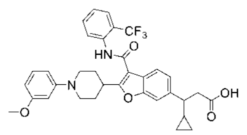
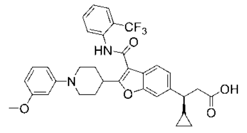
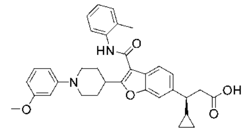
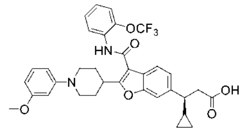
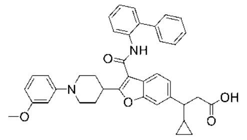
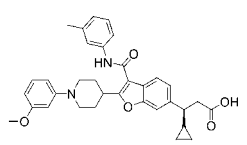
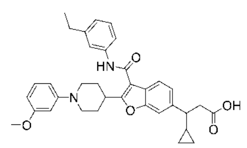
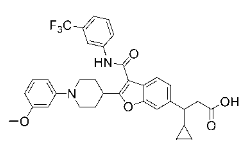
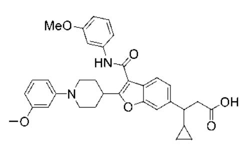
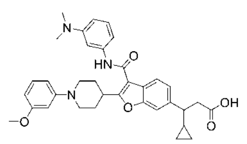
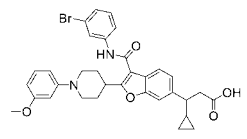
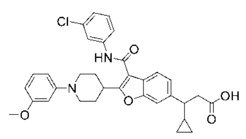
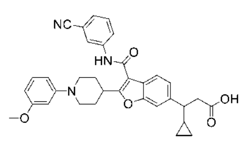
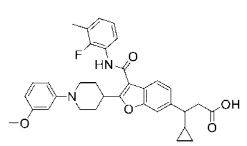
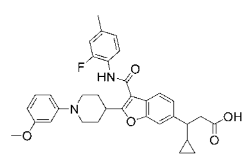
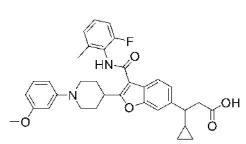
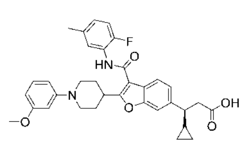
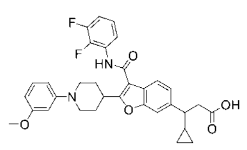
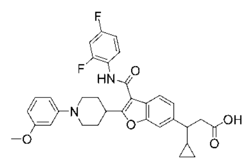
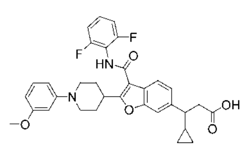
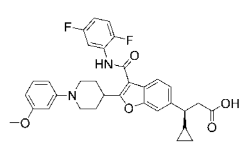
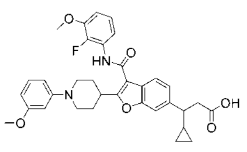
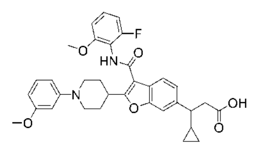
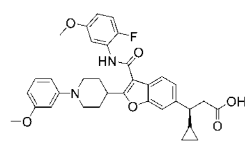
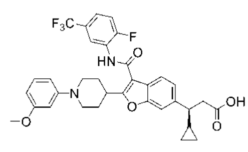

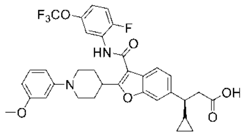
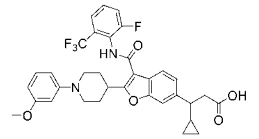
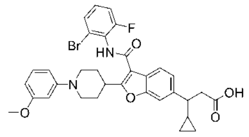
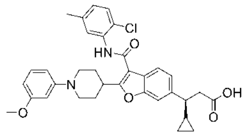
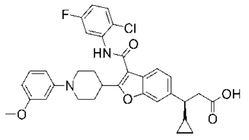
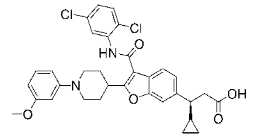
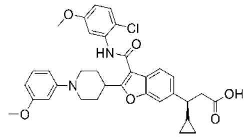
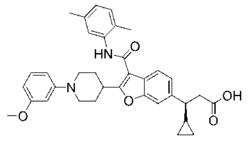
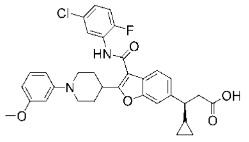
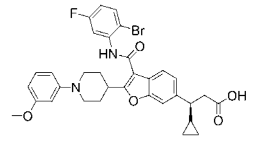
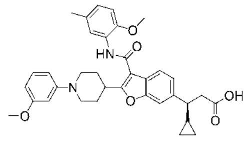
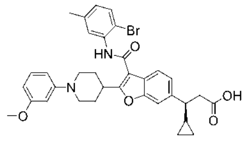
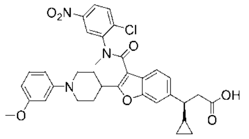
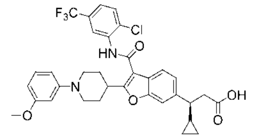
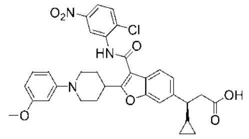
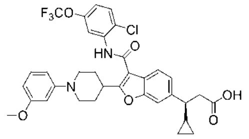
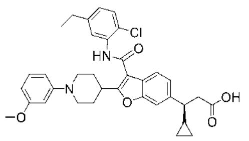
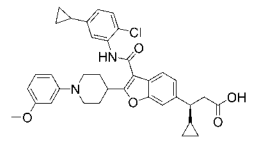
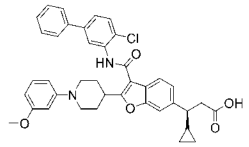
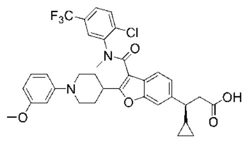
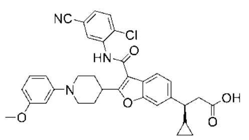
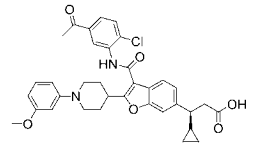
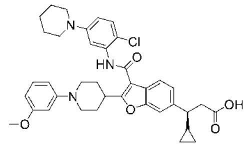
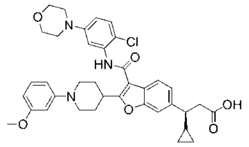
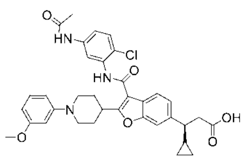
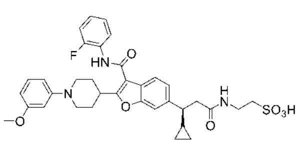
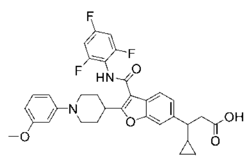
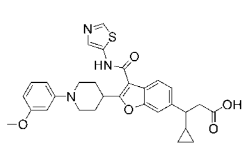
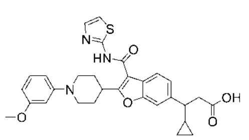
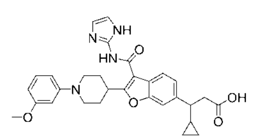
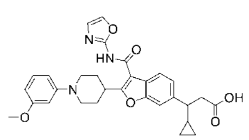
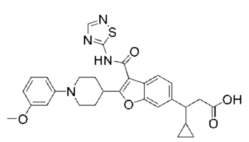
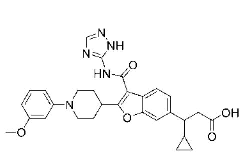
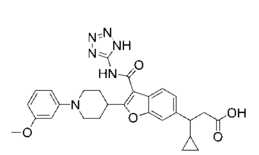
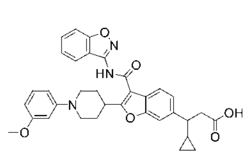
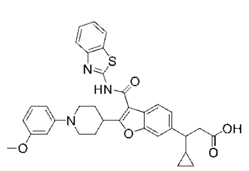

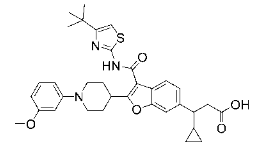
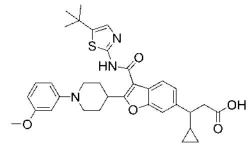
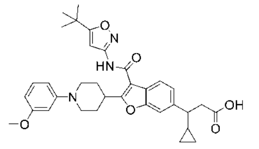
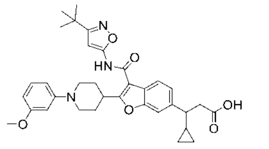

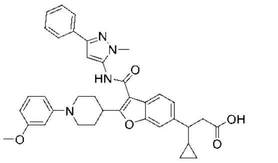
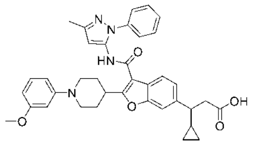
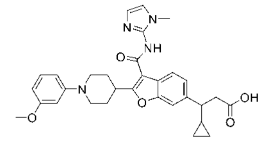
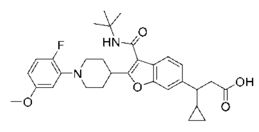

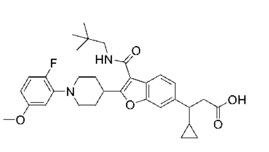
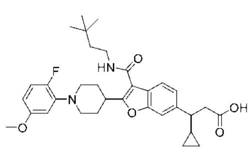
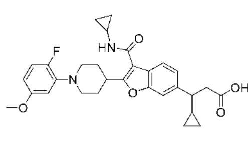
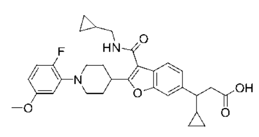

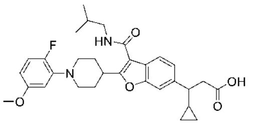
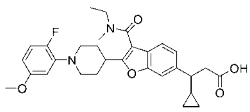
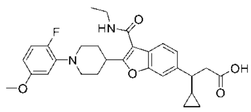
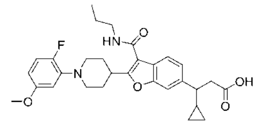

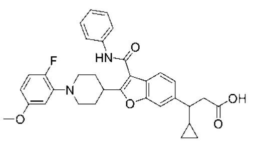
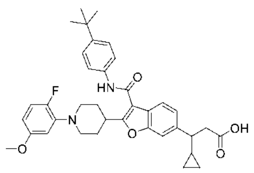
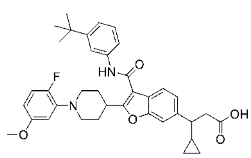
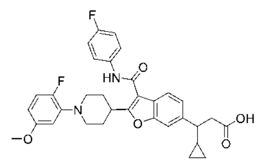

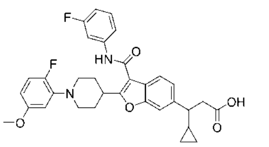
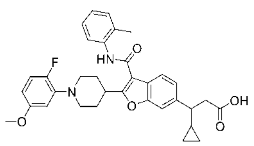
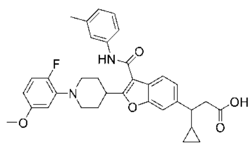
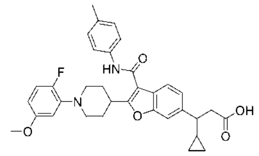

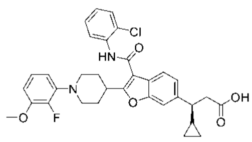
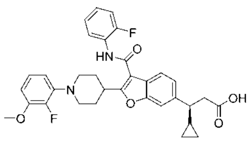
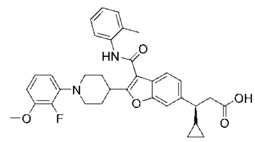
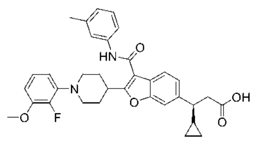

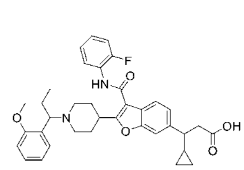
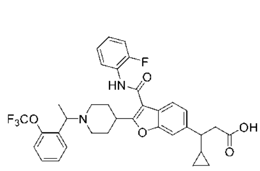
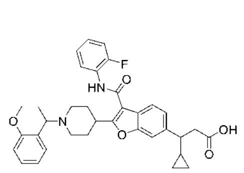
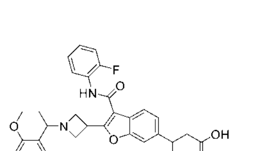

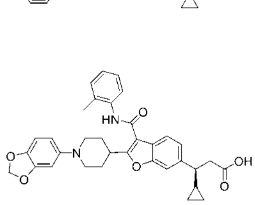
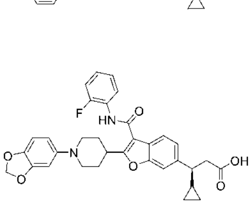
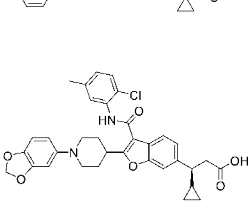
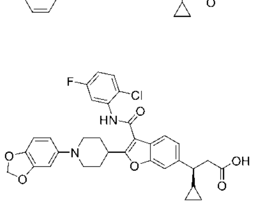

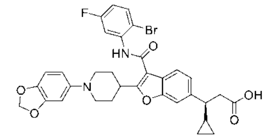
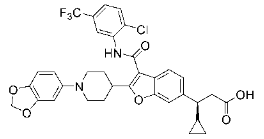
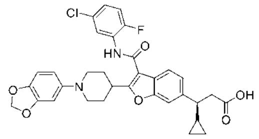
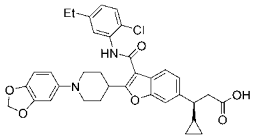

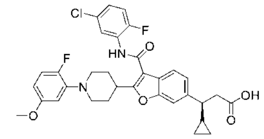
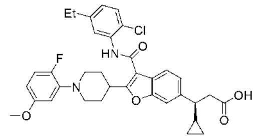
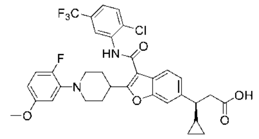
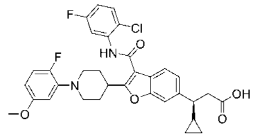

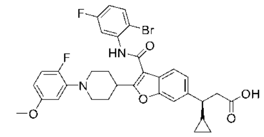
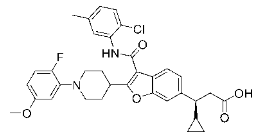
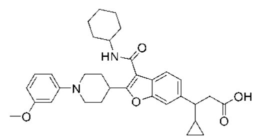
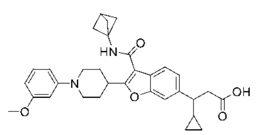

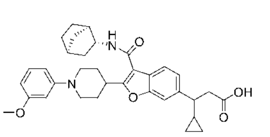
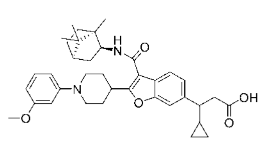
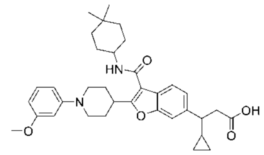
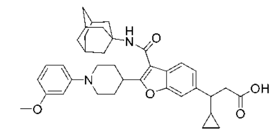

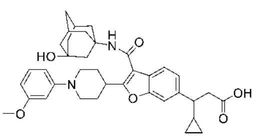
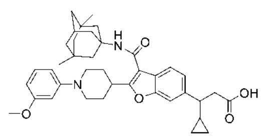
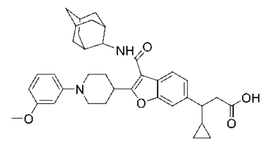
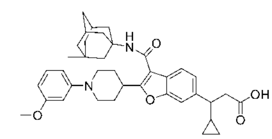

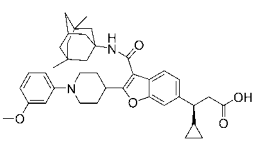
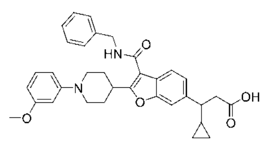
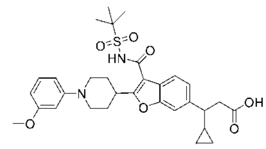
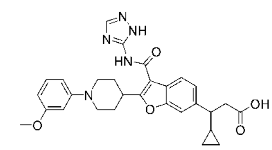

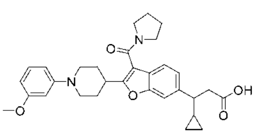
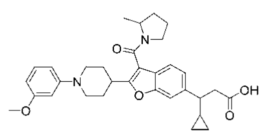
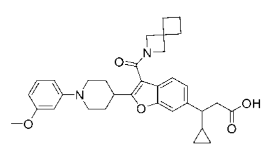
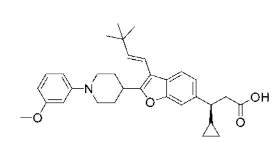

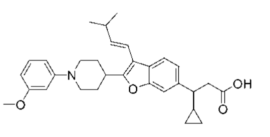
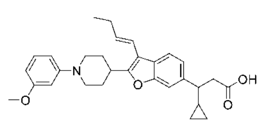
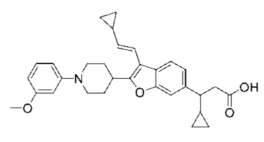
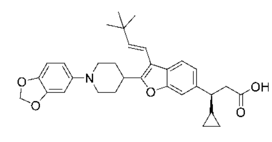

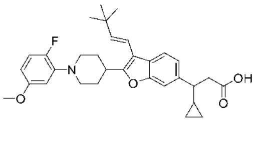
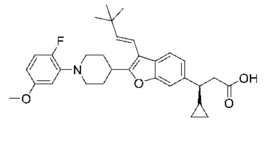
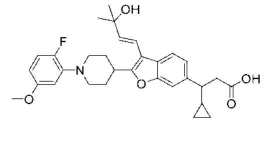
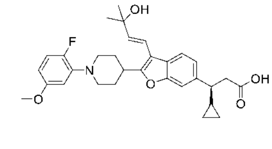

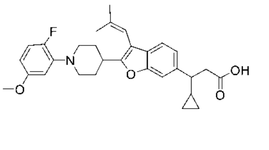
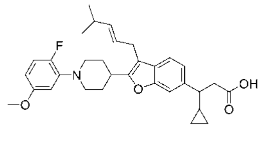
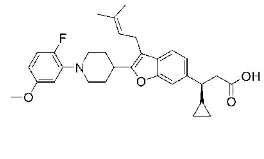
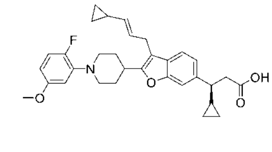

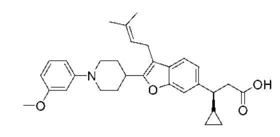
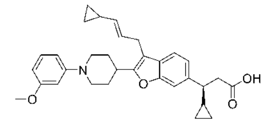
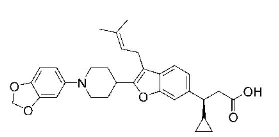
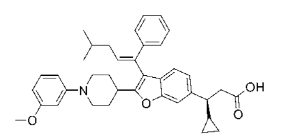

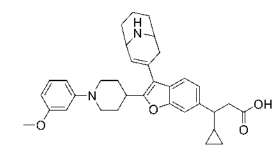
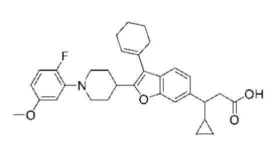
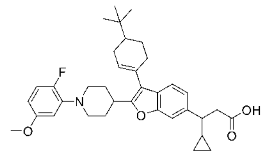
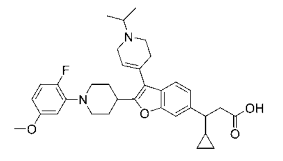

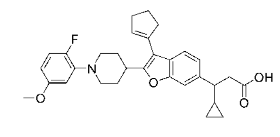
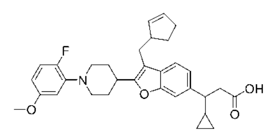
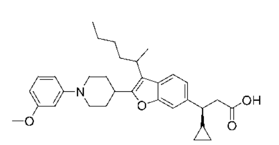
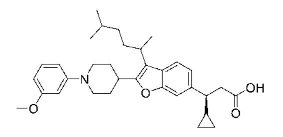

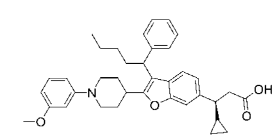
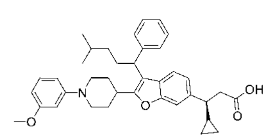
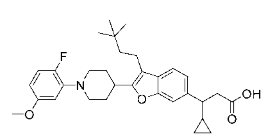
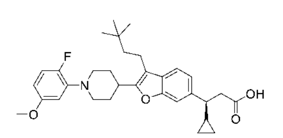

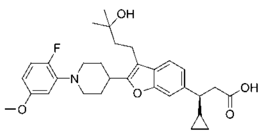
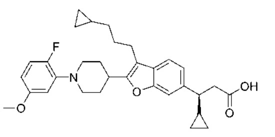
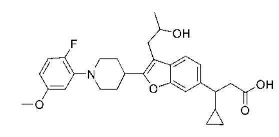
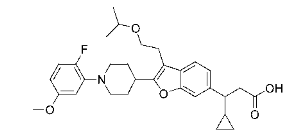

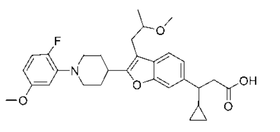
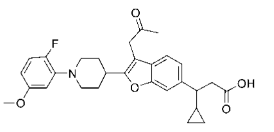
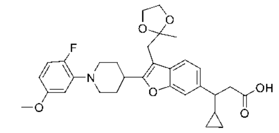
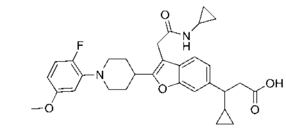

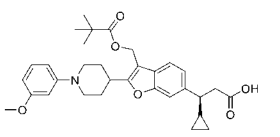
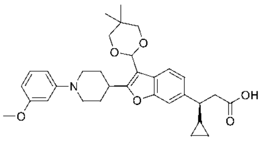
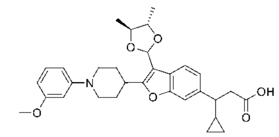
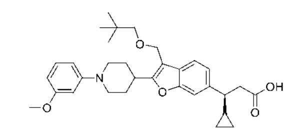

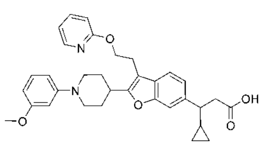
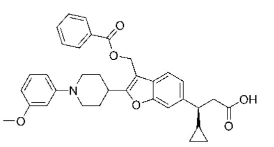
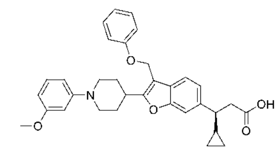
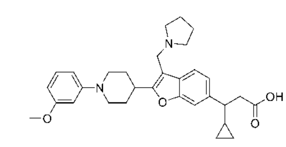

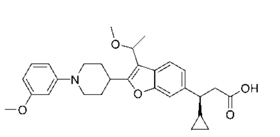
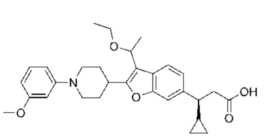
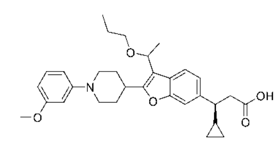
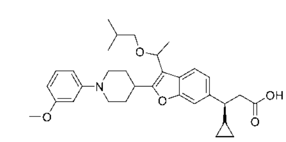

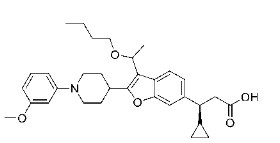
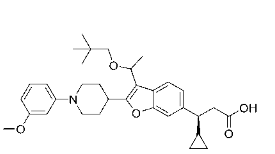
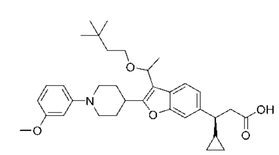
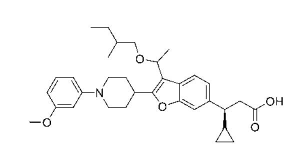

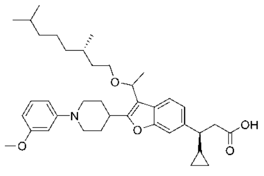
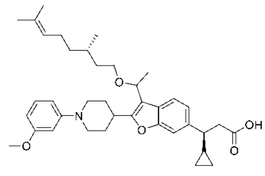
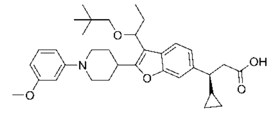
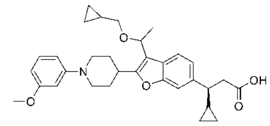
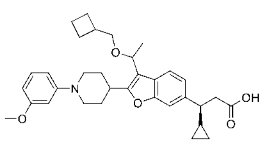
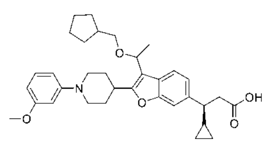
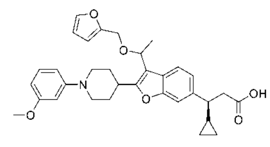
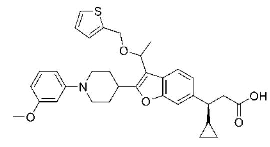
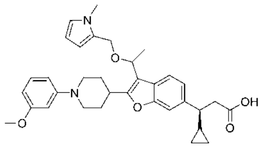
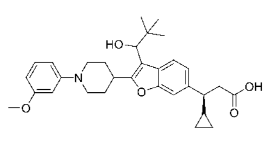
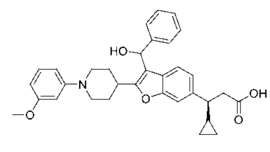
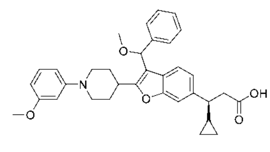
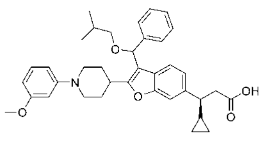
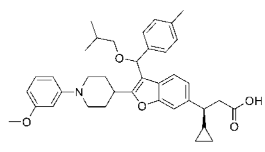
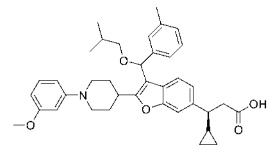
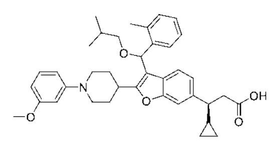
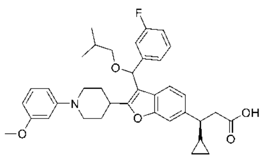
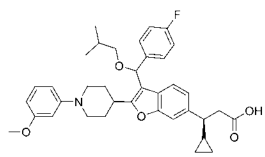
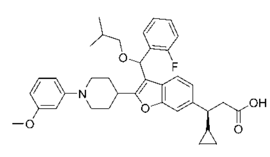
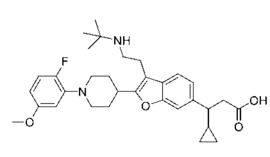
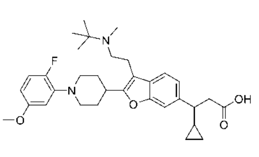
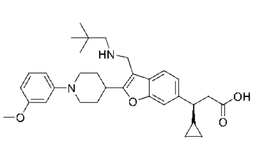
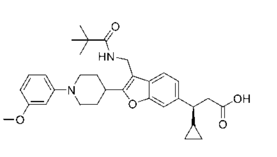
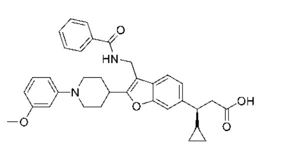
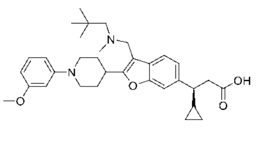
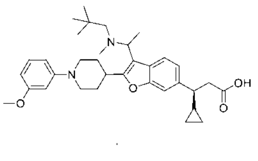
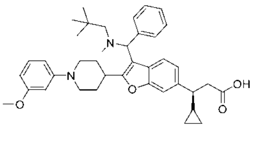
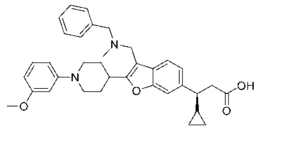
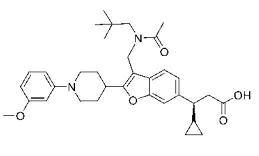
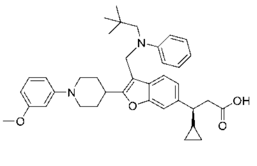
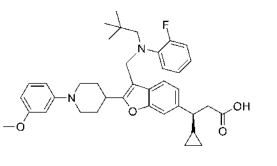
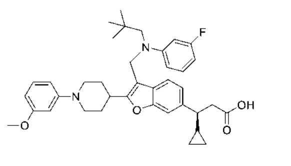
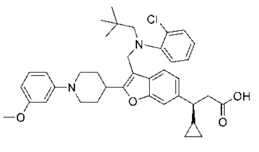
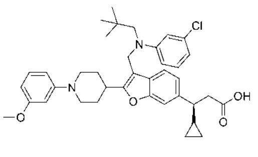
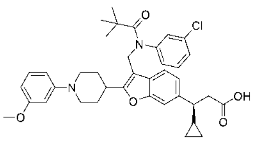
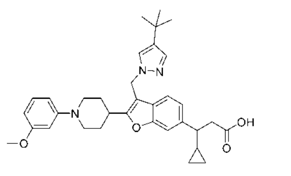

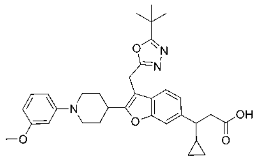 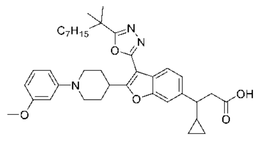 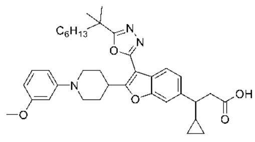 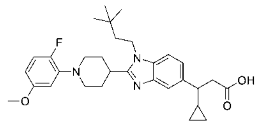 .

The present disclosure also provides a preparation method for the compound of formula I, wherein the method is method 1 or 2:

when $G^2$ is -C(=O)OH, the method is method 1; method 1 comprises the following step: subjecting compound II-1 to a hydrolysis reaction in a solvent in the presence of a base to obtain the compound of formula I;

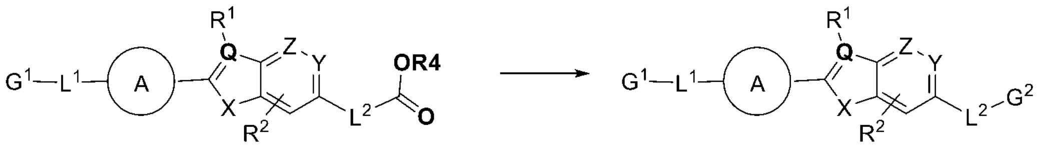

II-1 I ;

wherein $R^4$ is $C_1$-$C_6$ alkyl; the definitions of X, Y, Z, $R^1$, $R^2$, L, $G^1$, $G^2$, and ring A are as described above;
when $G^2$ is 5- to 10-membered heteroaryl, the method is method 2; method 2 comprises the following step: subjecting compound II-2 and trimethylsilyl azide to a cyclization reaction in a solvent in the presence of a catalyst to obtain the compound of formula I;
alternatively, subjecting compound II-2 and *N,N'*-carbonyldiimidazole to a cyclization reaction in a solvent in the presence of a catalyst to obtain the compound of formula I;

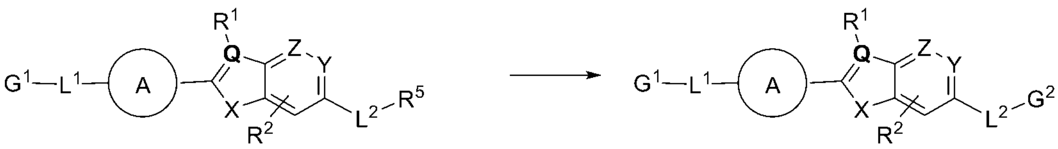

II-2 I ;

wherein $R^5$ is

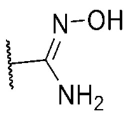

or cyano; $R^5$ is an amino protecting group or a hydroxyl protecting group; and the definitions of X, Y, Z, $R^1$, $R^2$, L, $G^1$, $G^2$, and ring A are as described above.

The present disclosure also provides a compound II-1, II-2, II-1a, or II-2a:

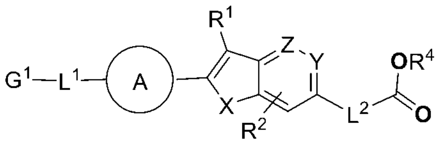 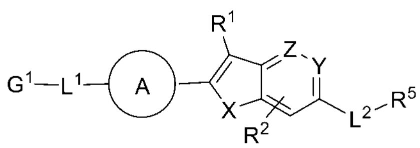

II-1a II-2a

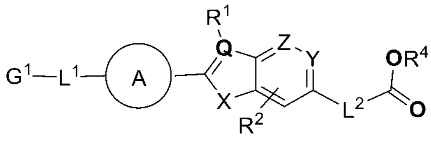

**II-1**

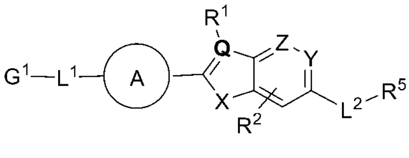

**II-2**

wherein the definitions of Q, X, Y, Z, $R^1$, $R^2$, $R^3$, $R^4$, $R^5$, $L^1$, $L^2$, $G^1$, and ring A are as described above.

The compound II is preferably any one of the following compounds:

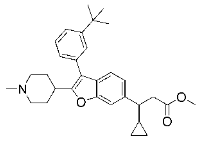
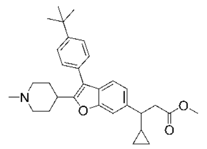
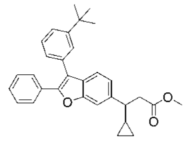
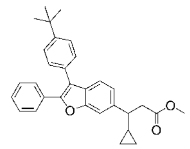
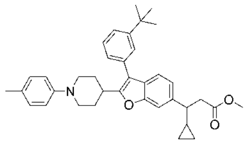
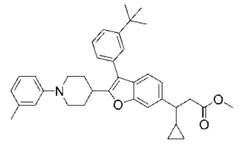
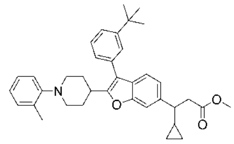
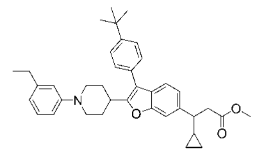
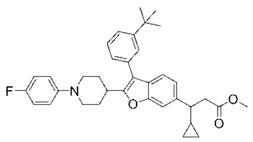
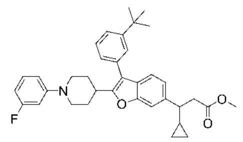
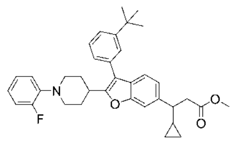
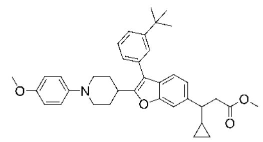
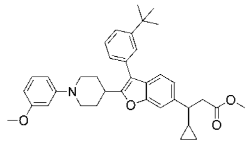
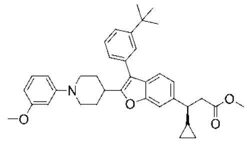
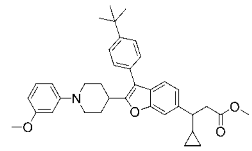
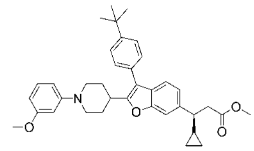
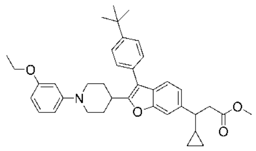
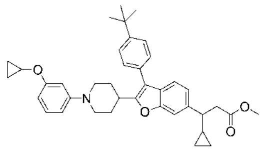
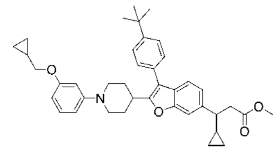
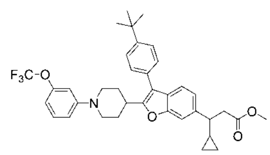
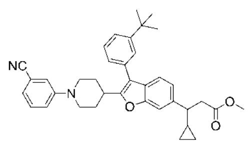
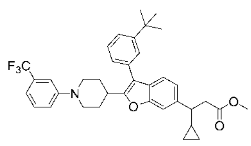
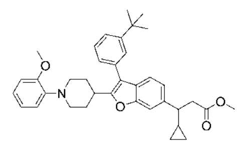
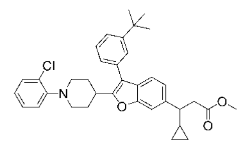
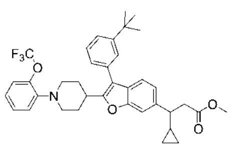
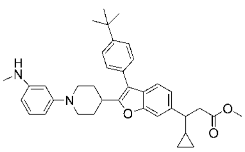
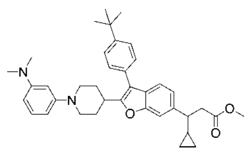
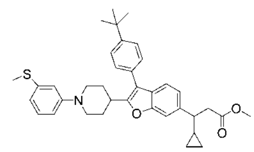

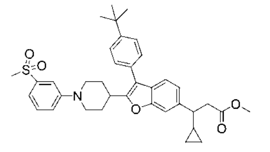
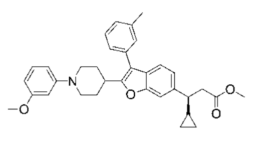
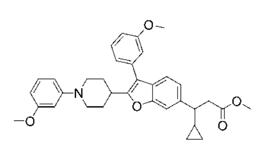
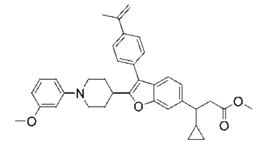

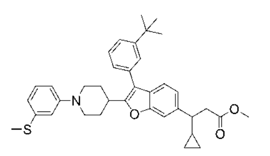
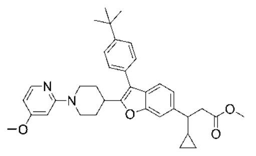
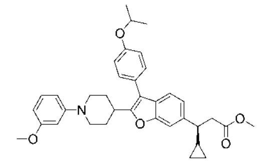
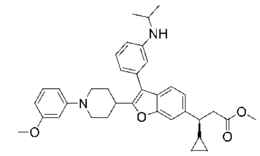

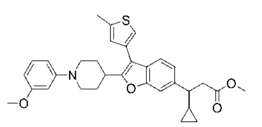
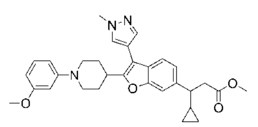
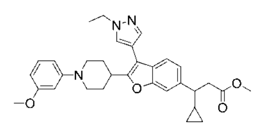
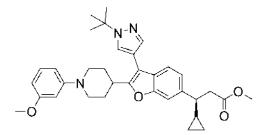

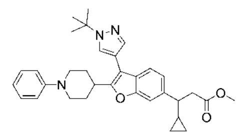
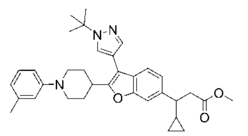
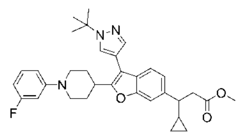
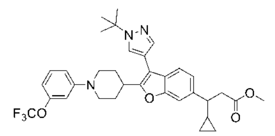

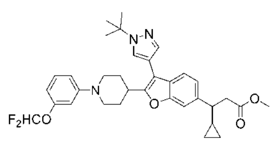
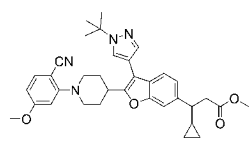
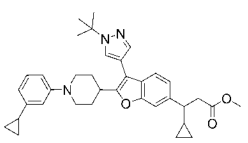
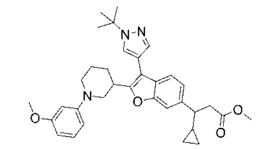

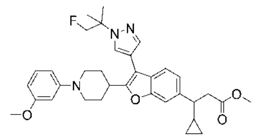
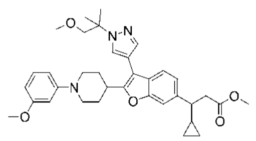
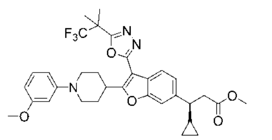
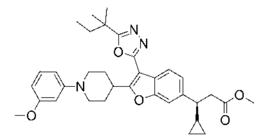

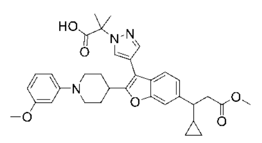
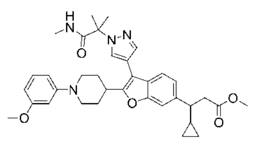
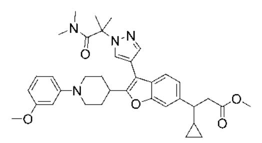
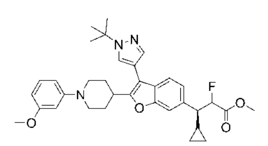

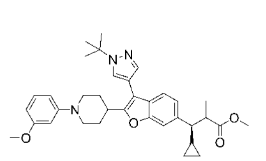
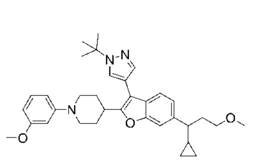
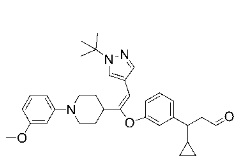

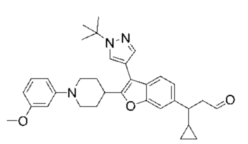
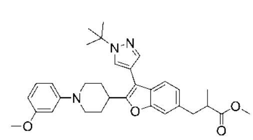
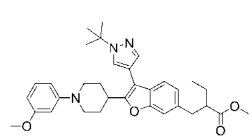
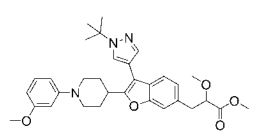

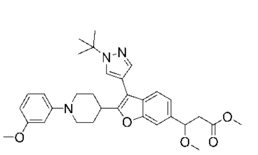
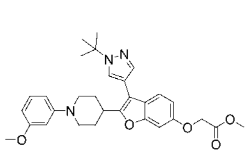
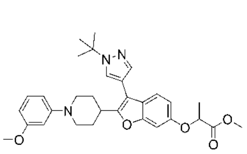
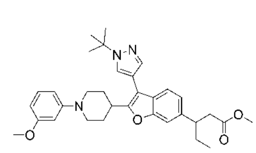

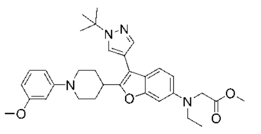
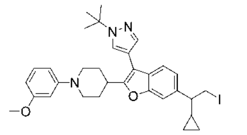
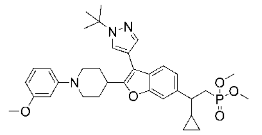
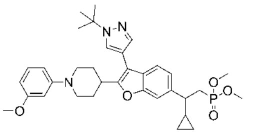
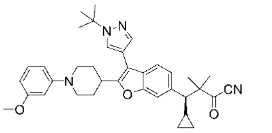
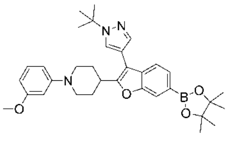
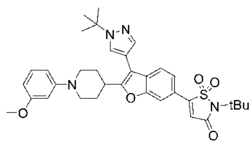
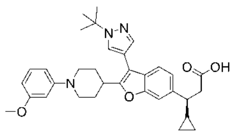
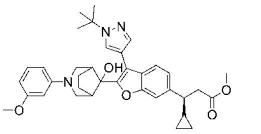
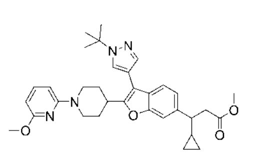
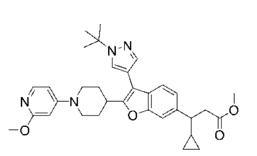
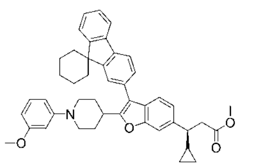
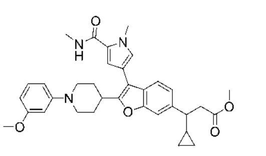
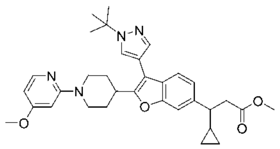
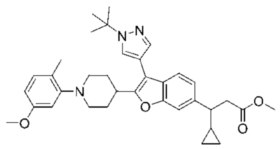
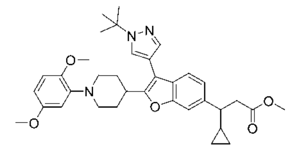
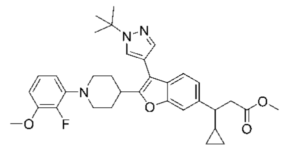
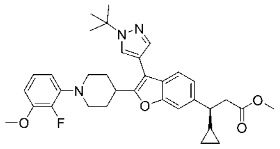
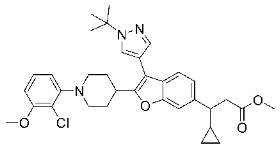
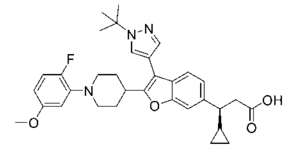
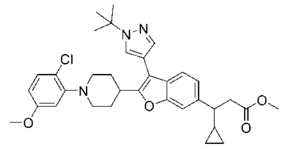
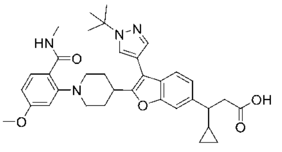
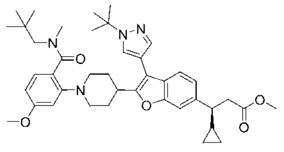
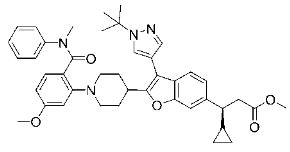
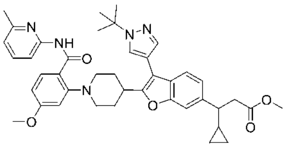
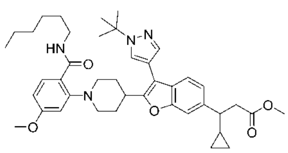
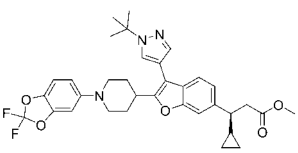
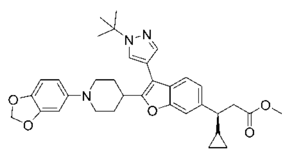
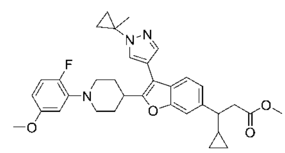
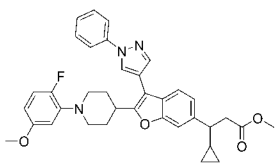
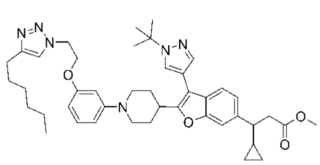
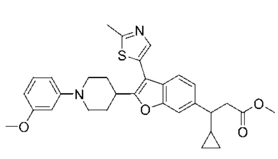
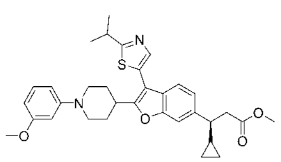

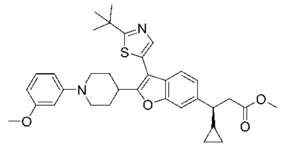
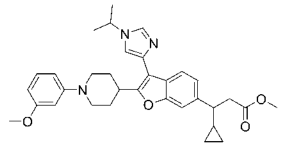
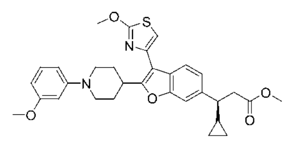
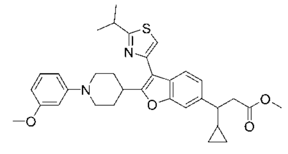
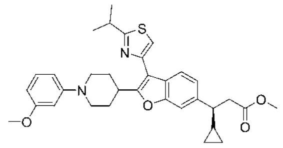
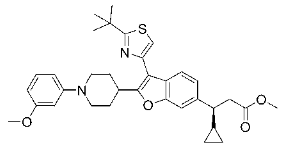
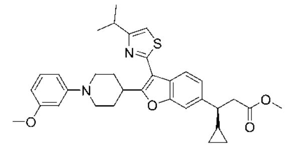
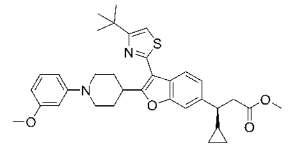
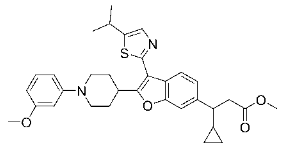
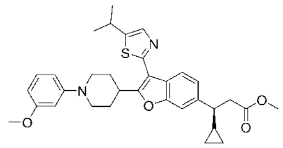
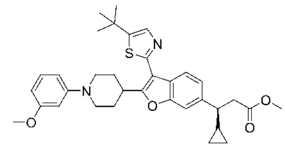
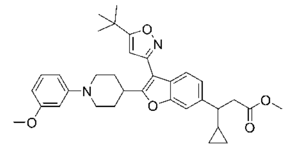
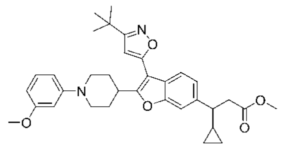
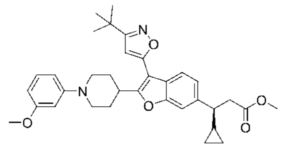
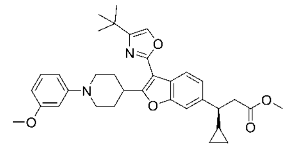
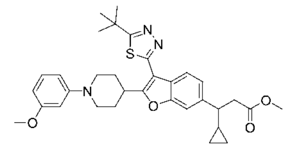
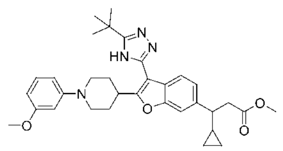
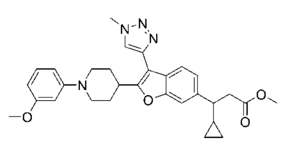
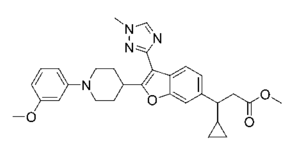
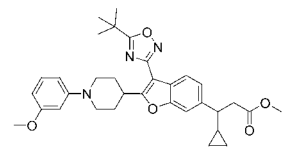
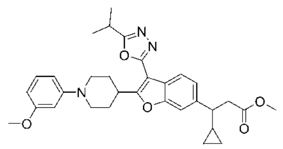
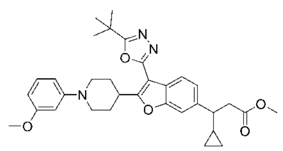
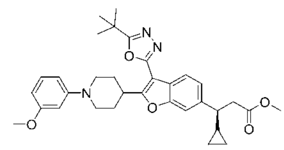
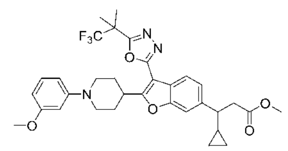
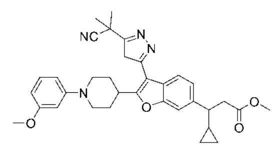
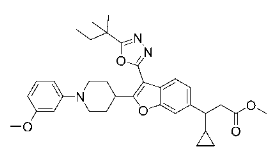
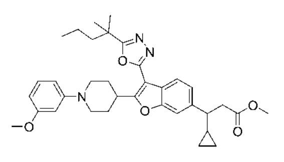
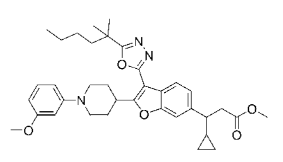
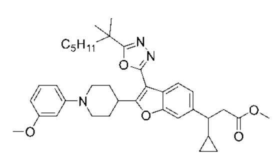
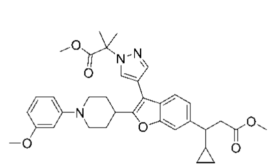
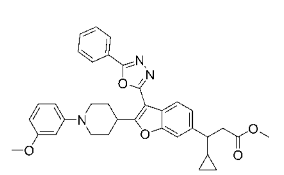
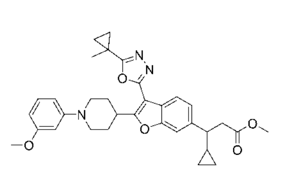
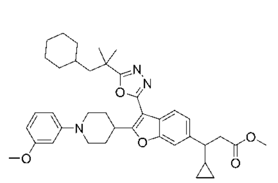
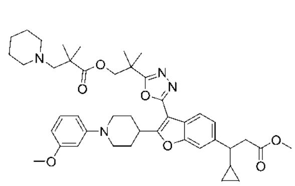
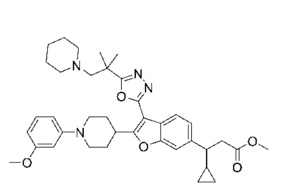
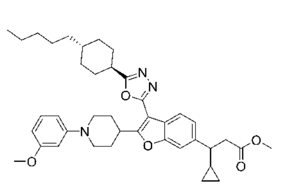

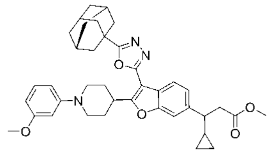
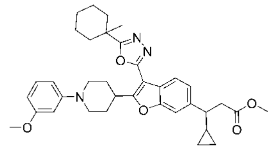
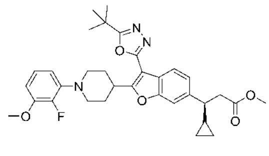
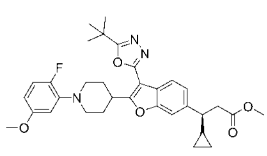
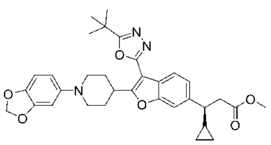
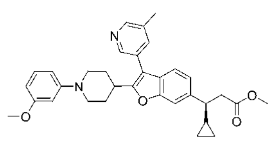
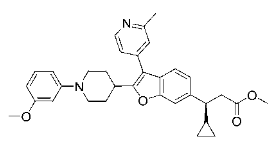
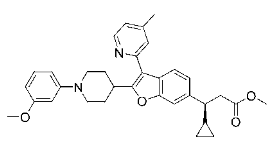
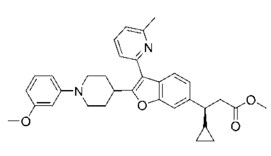
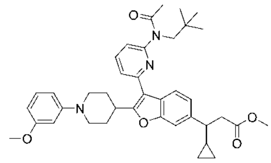
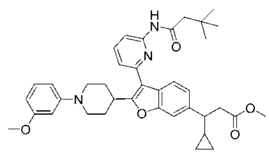
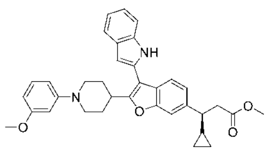
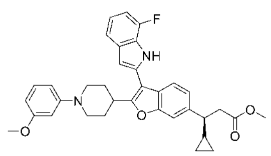
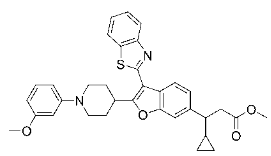
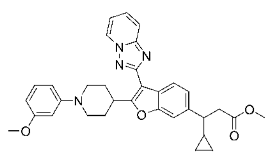
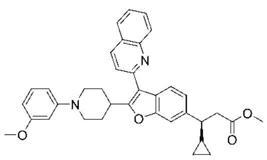
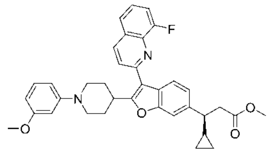
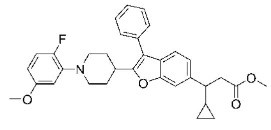
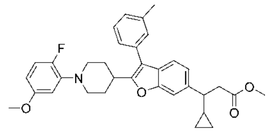
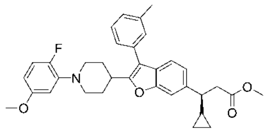
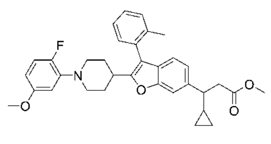
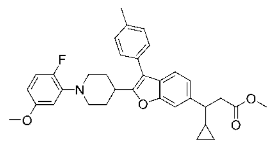
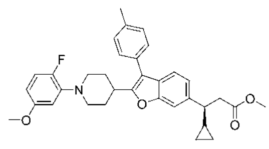
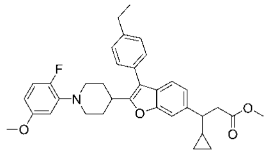
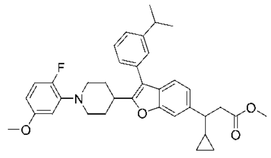
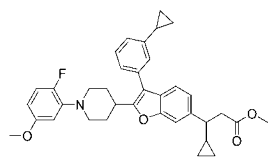
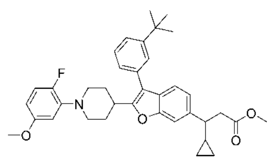
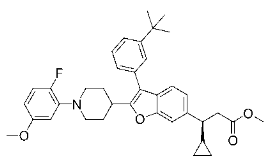
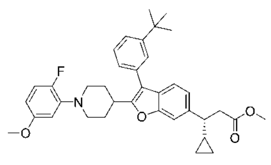
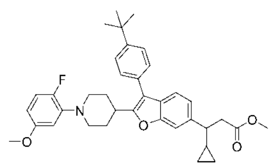
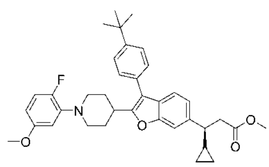
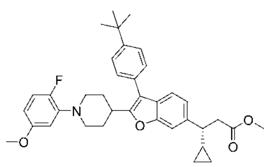
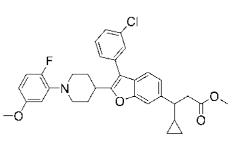
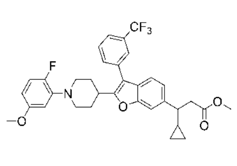
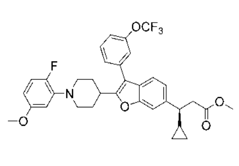
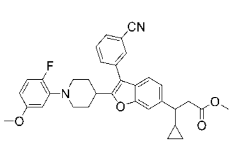

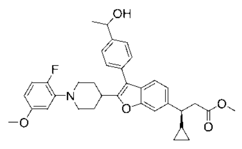
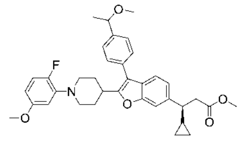
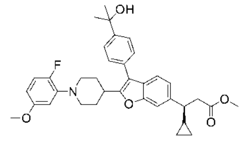
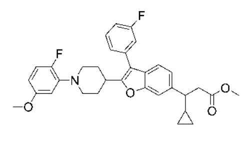

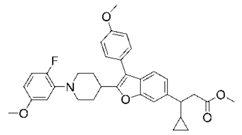
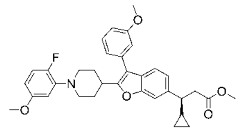
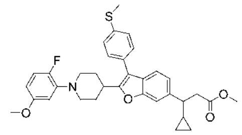
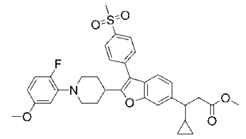

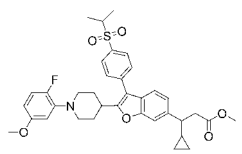
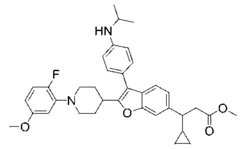
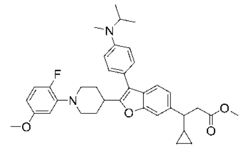
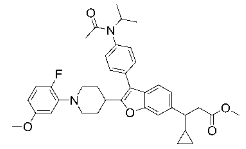

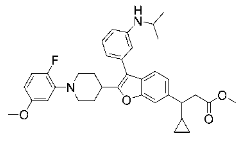
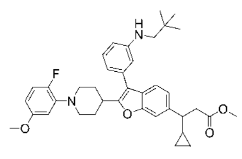
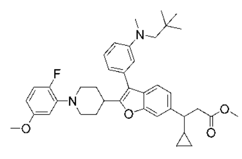
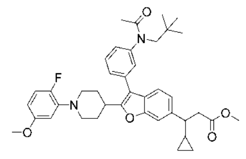

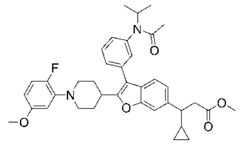
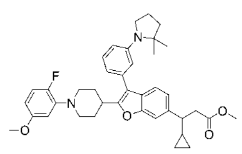
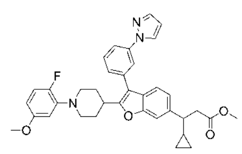
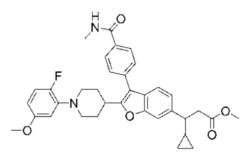

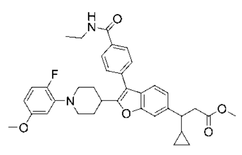
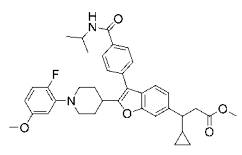
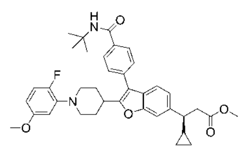
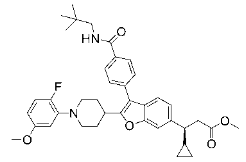

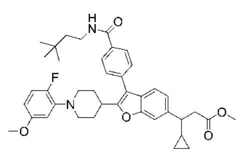
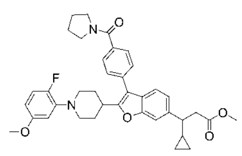
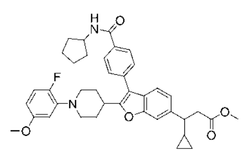
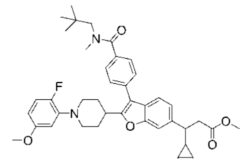

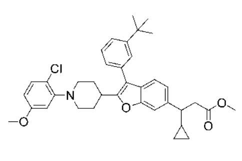
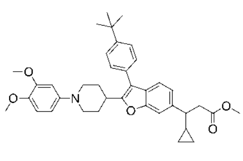
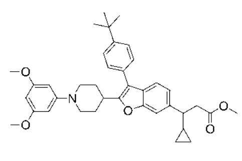
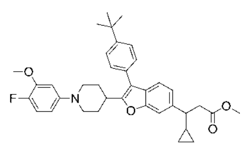

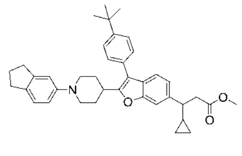
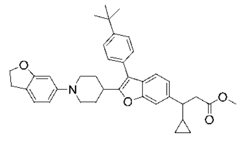
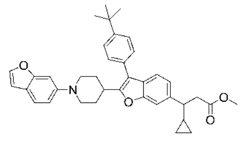
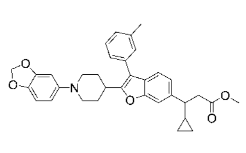

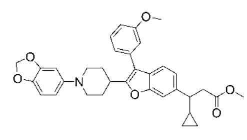
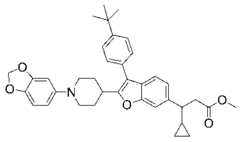
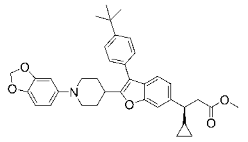
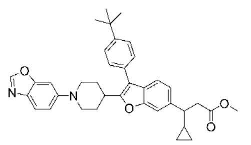
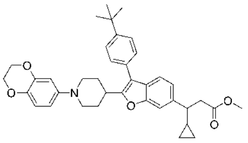
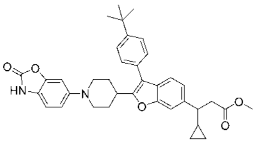
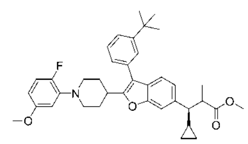
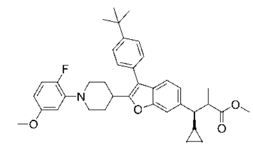
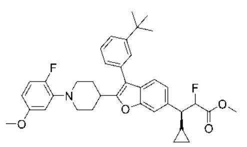
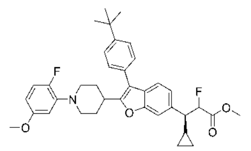
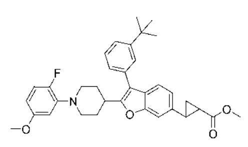
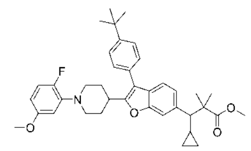
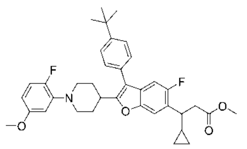
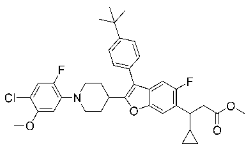
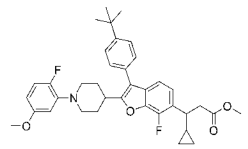
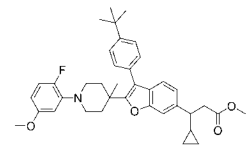
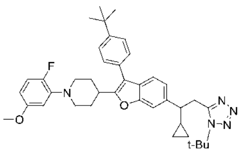
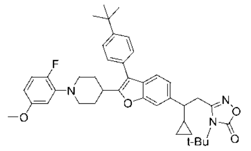
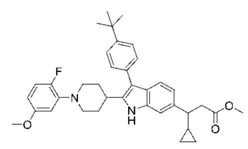
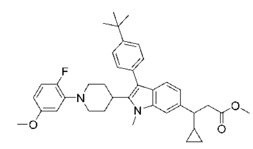
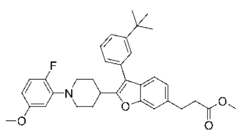
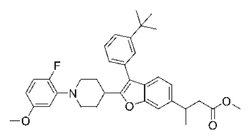
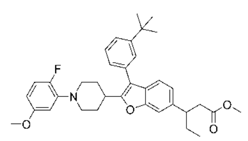
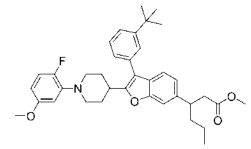
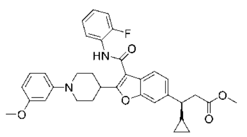
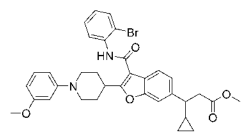
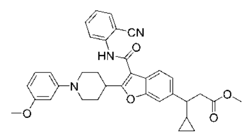
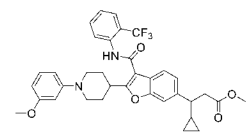
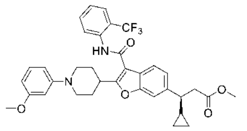
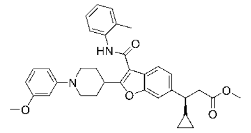
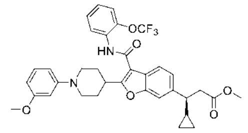
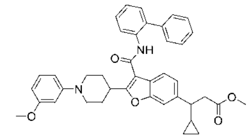
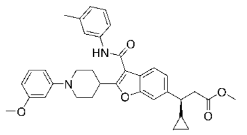
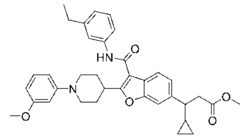
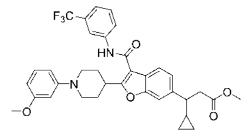
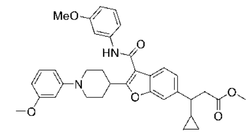

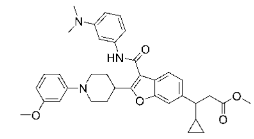
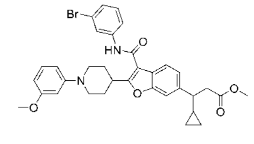
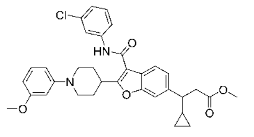
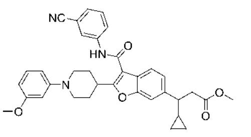
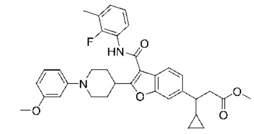
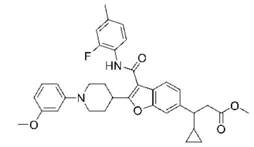
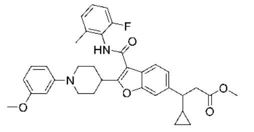
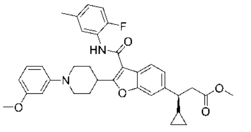
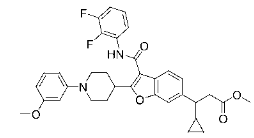
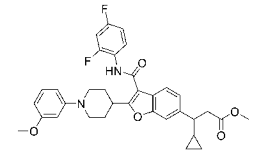
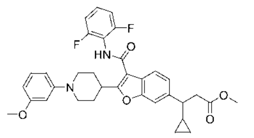
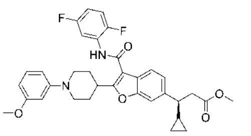
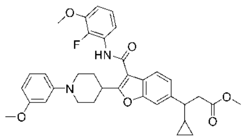
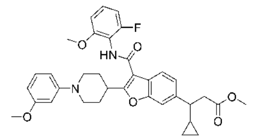
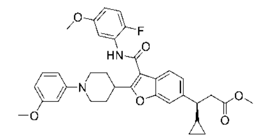
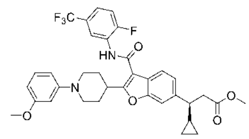
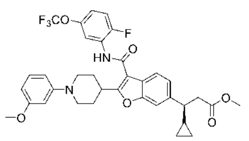
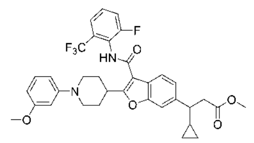
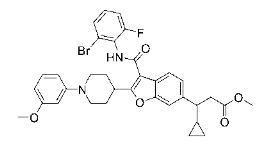
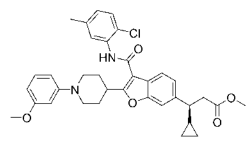
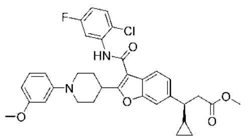
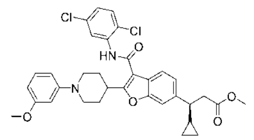
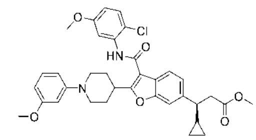
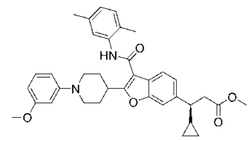
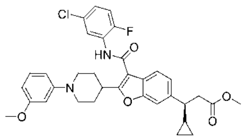
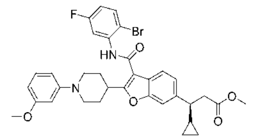
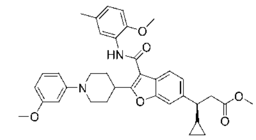
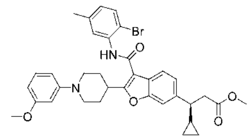
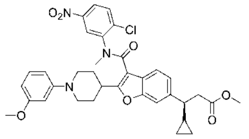
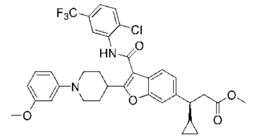
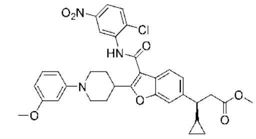
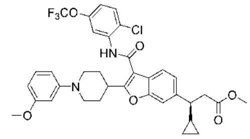
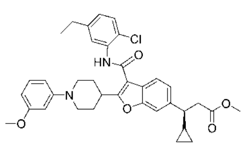
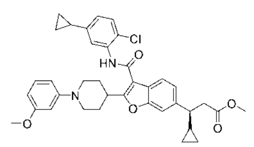
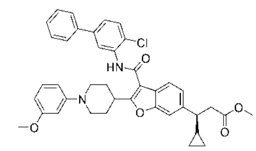
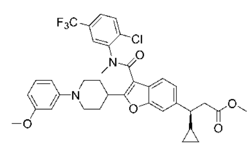

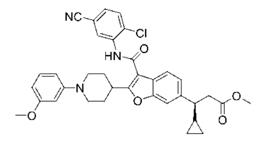 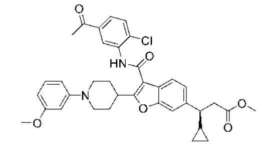 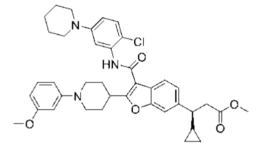 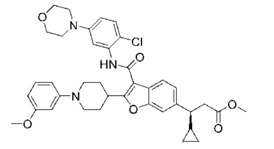

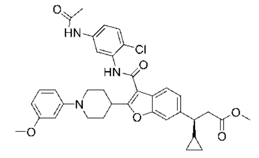 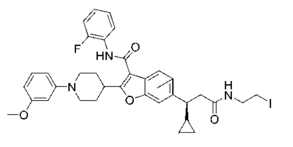 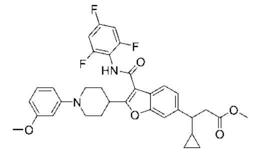 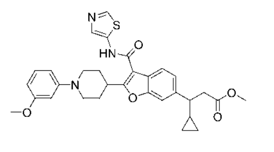

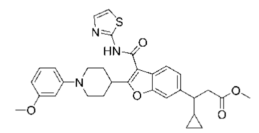 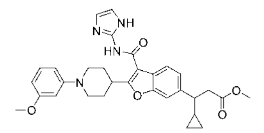 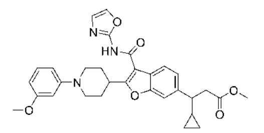 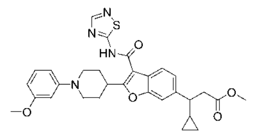

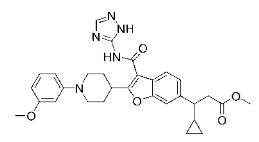 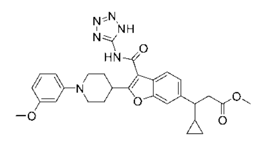 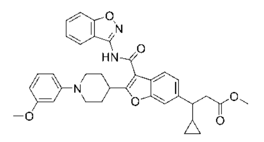 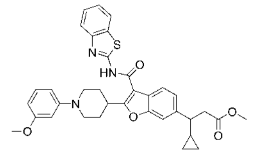

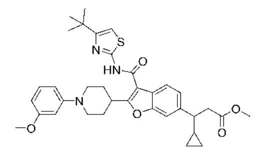 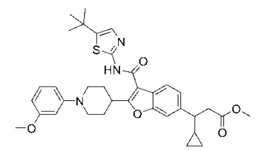 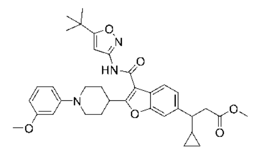 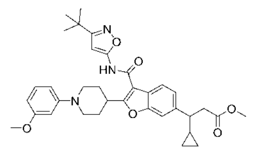

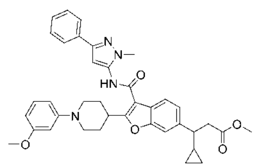 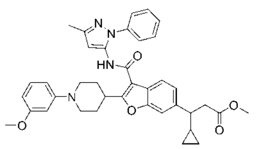 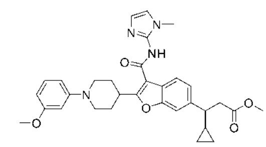 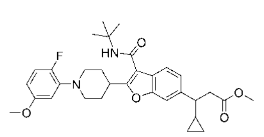

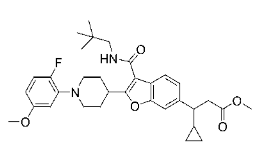 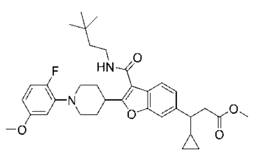 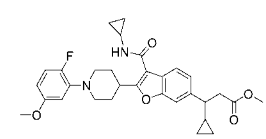 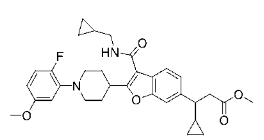

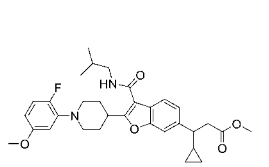 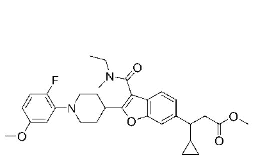 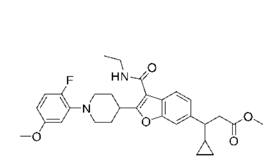 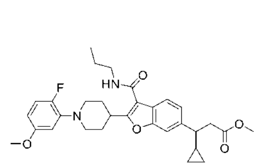

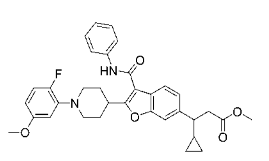 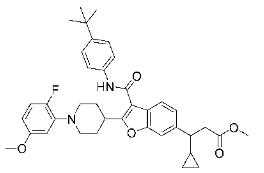 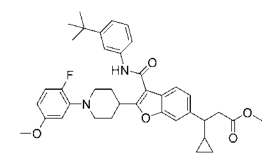 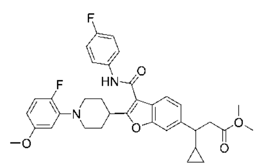

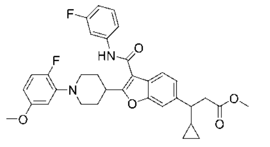 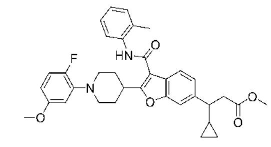 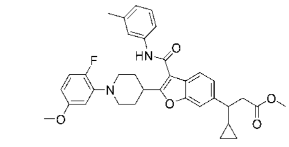 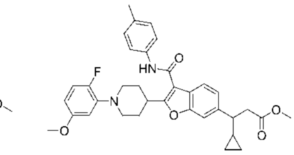

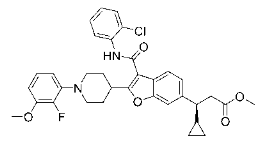 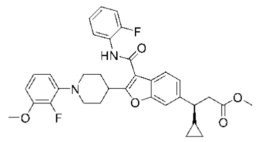 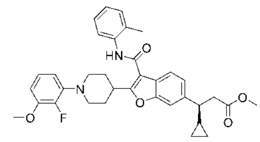 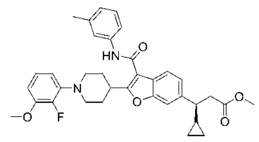

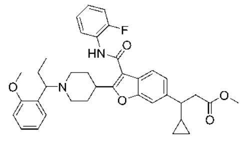 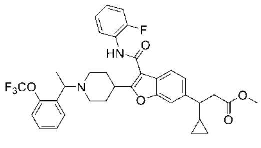 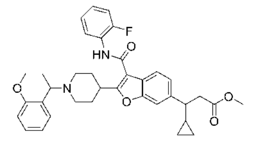 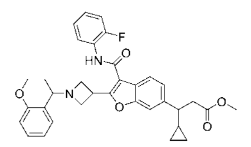

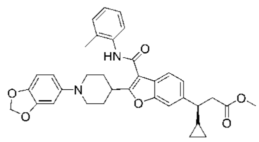 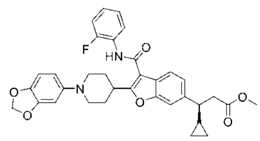 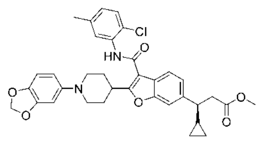 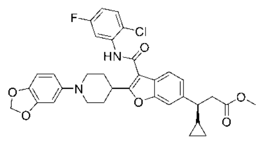

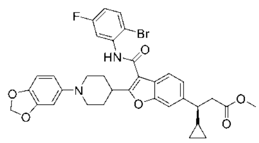 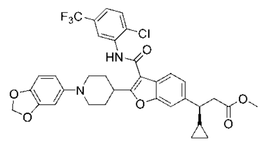 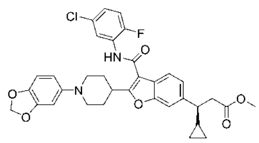 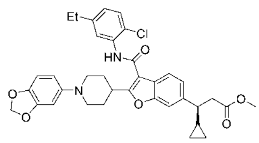

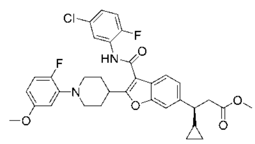 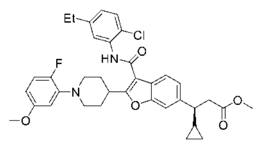 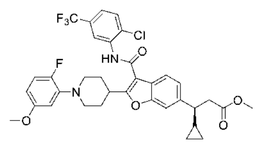 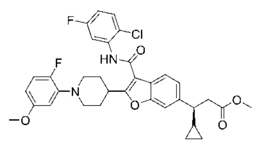

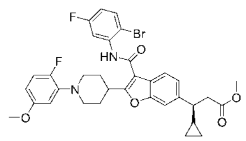 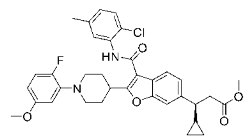 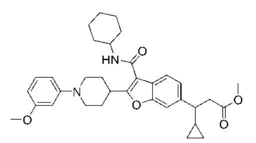 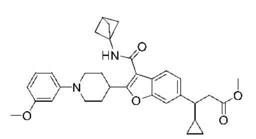

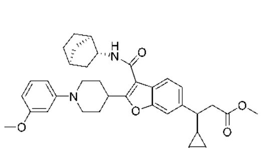 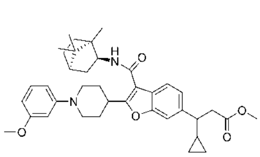 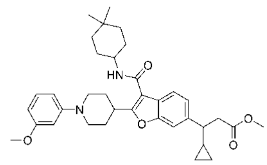 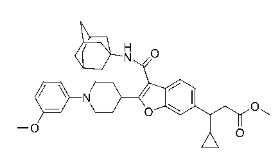

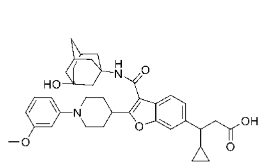 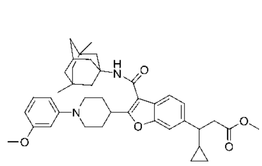 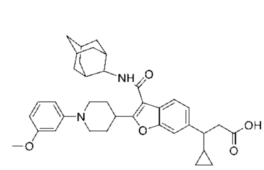 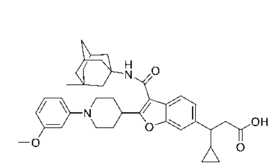

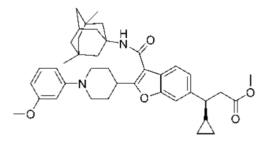 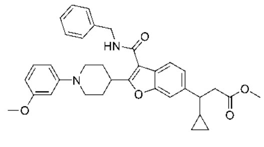 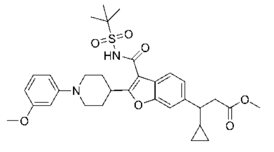 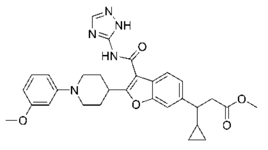

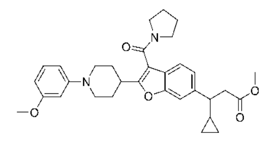 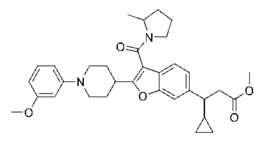 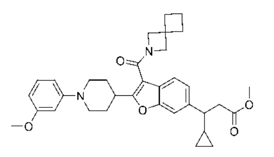 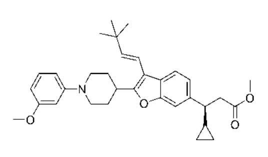

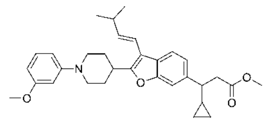 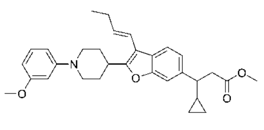 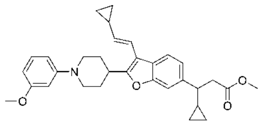 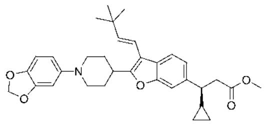

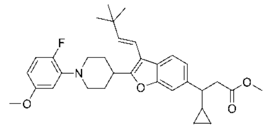 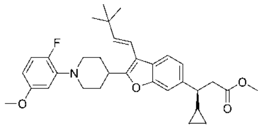 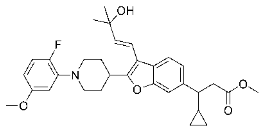 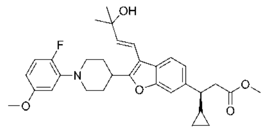

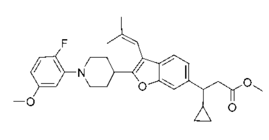 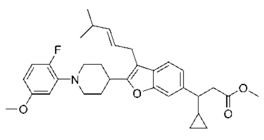 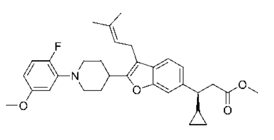 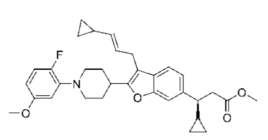

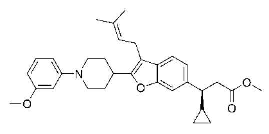 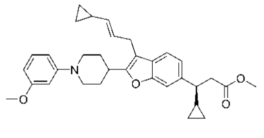 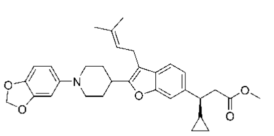 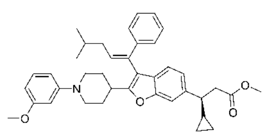

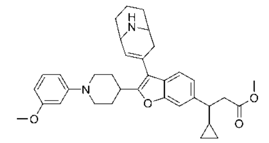 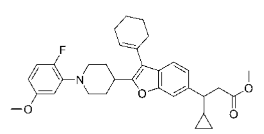 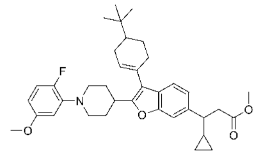 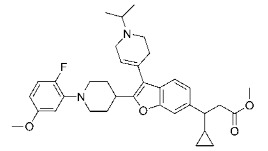

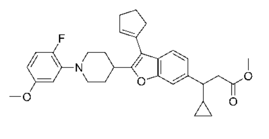 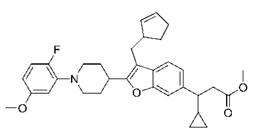 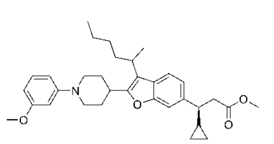 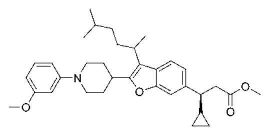

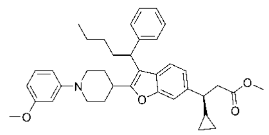 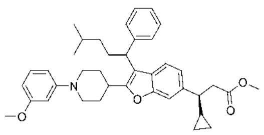 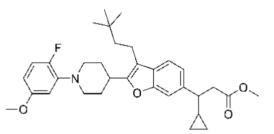 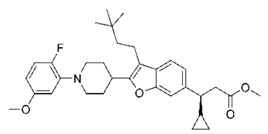

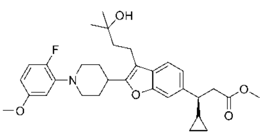 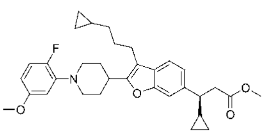 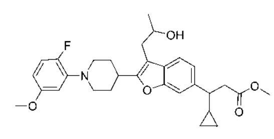 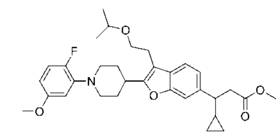

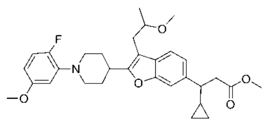
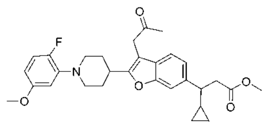
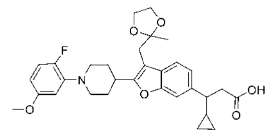
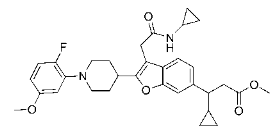

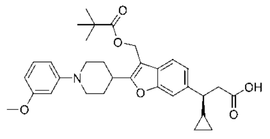
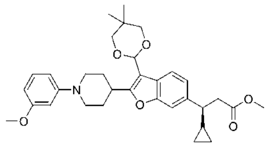
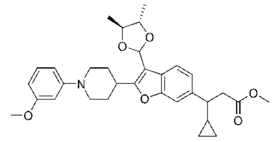
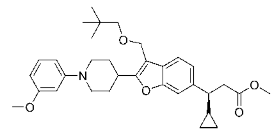

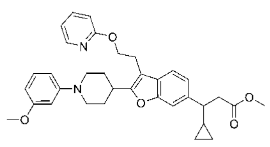
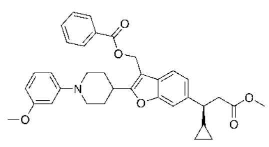
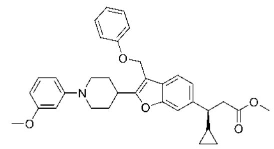
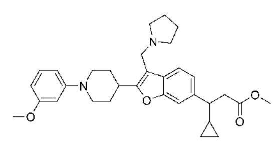

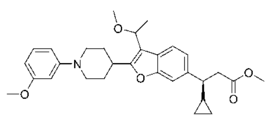
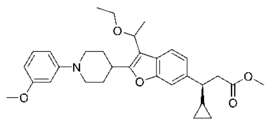
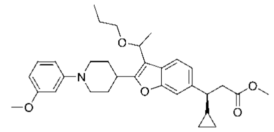
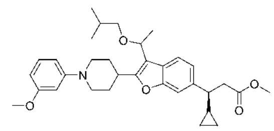

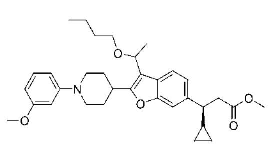
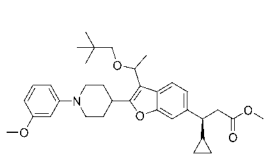
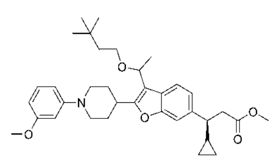
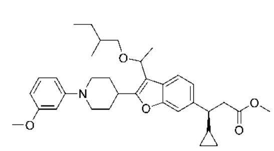

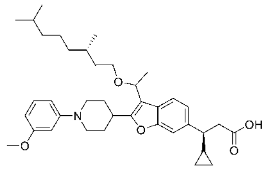
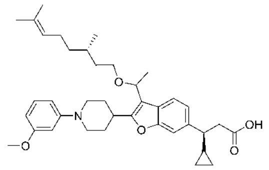
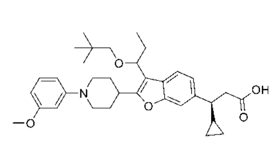
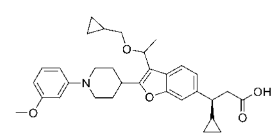

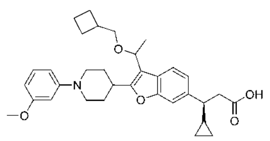
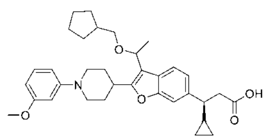
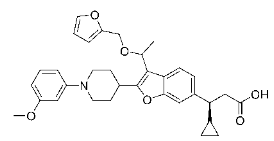
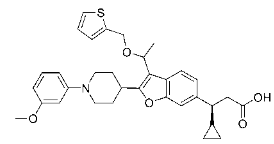

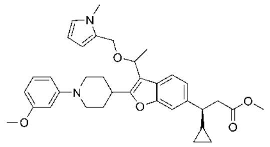
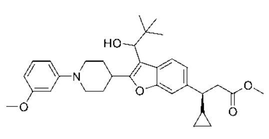
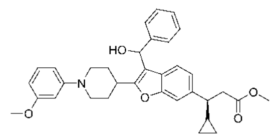
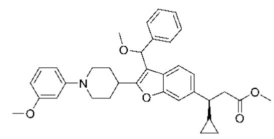

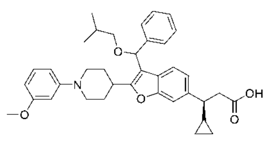
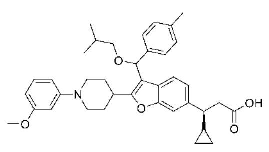
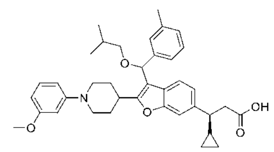
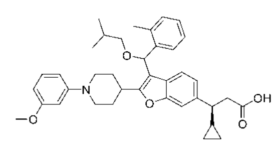

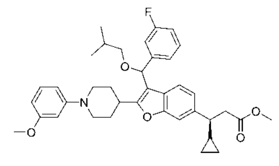 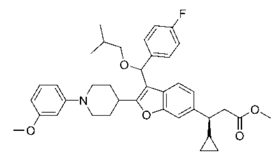 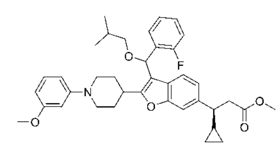 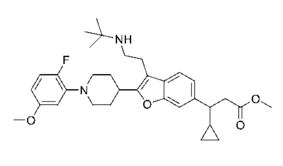

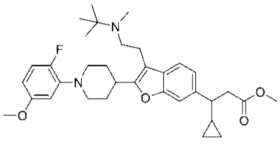 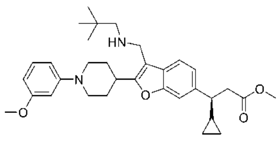 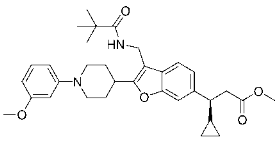 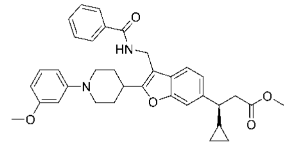

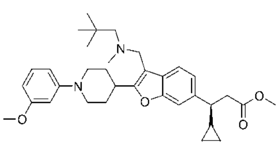 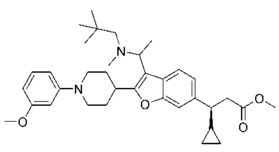 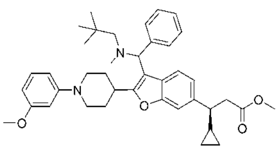 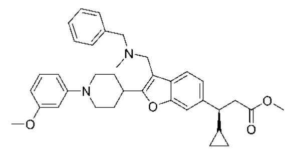

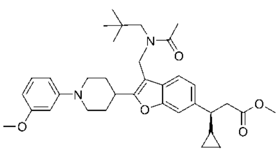 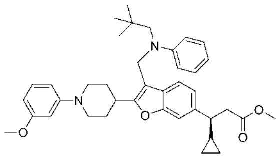 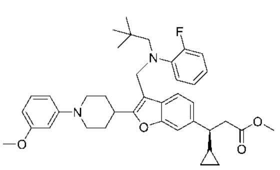 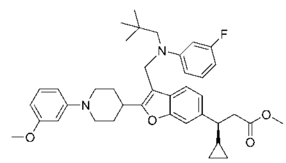

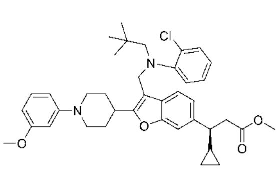 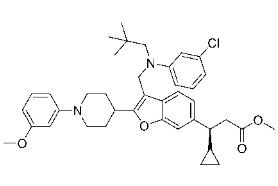 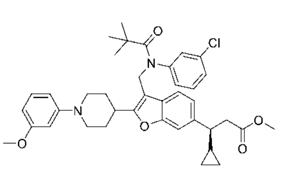 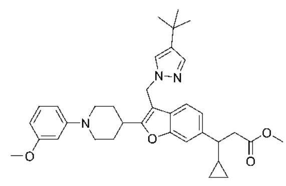

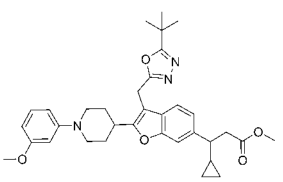 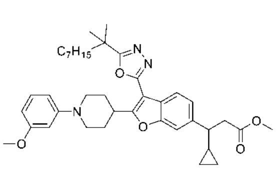 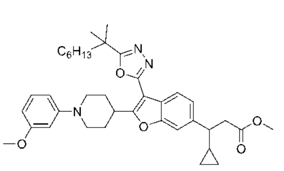 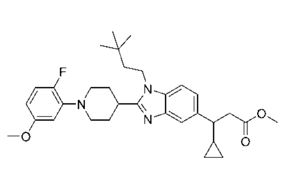

.

The compound II-2 is preferably

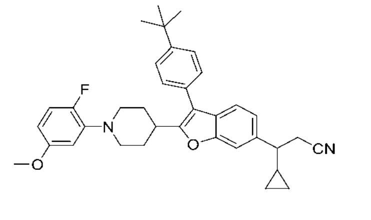

or

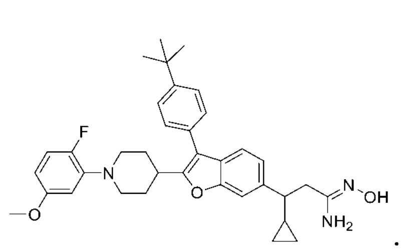

.

The present disclosure also provides a pharmaceutical composition comprising the compound of formula I or the pharmaceutically acceptable salt thereof, and a pharmaceutically acceptable excipient.

The present disclosure also provides a use of the compound of formula I or the pharmaceutically acceptable salt thereof in the manufacture of a GPR40 agonist (*in vivo* or *in vitro*).

The present disclosure also provides a use of the compound of formula I or the pharmaceutically acceptable salt thereof in the manufacture of a medicament for treating or preventing a GPR40-related disease.

In the use, the GPR40-related disease is preferably diabetes.

The present disclosure also provides a use of the compound of formula I or the pharmaceutically acceptable salt thereof in the manufacture of a medicament for treating or preventing diabetes, wherein the disease is diabetes.

The present disclosure also provides a method for treating a GPR40-related disease (preferably diabetes), comprising administering to a patient an effective amount of the compound of formula I or the pharmaceutically acceptable salt thereof.

The present disclosure also provides a method for treating diabetes, comprising administering to a patient an effective amount of the compound of formula I or the pharmaceutically acceptable salt thereof.

The more than one in the expression "group B substituted by one or more group A" refers to 2, 3, 4, or 5. The "group B substituted by one or more group A" means that 1, 2, 3, 4, or 5 hydrogen atoms in group B are independently substituted by group A. When more than one group A appears simultaneously, unless otherwise specified, their definitions are independent of and do not affect each other. For example, "$C_6$-$C_{10}$ aryl substituted by 3 halogens" refers to the $C_6$-$C_{10}$ aryl substituted by 3 halogens, where the definitions of the 3 halogens are independent of and do not affect each other, including but not limited to:

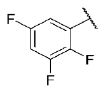, 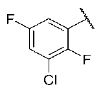,

*etc.*

The term "pharmaceutically acceptable salt" refers to a salt obtained by reacting a compound with a pharmaceutically acceptable acid or base. When the compound contains a relatively acidic functional group, a base addition salt can be obtained by contacting the compound with a sufficient amount of a pharmaceutically acceptable base in a suitable inert solvent. When the compound contains a relatively basic functional group, an acid addition salt can be obtained by contacting the compound with a sufficient amount of a pharmaceutically acceptable acid in a suitable inert solvent. For details, please refer to the "Handbook of Pharmaceutical Salts: Properties, Selection, and Use" (P. Heinrich Stahl, Camille G. Wermuth, 2011, 2nd Revised Edition).

The "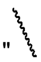" in a structural moiety means that the structural moiety is connected to other moieties in the molecule through this site. For example,

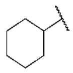

refers to cyclohexyl.

The "-" at the terminus of a group indicates that the group is connected to the rest of the molecule through this site. For example, $CH_3$-C(=O)- refers to acetyl.

The term "halogen" refers to fluorine, chlorine, bromine, or iodine.

The term "alkyl" refers to a straight or branched, saturated monovalent hydrocarbon group having a specified number of carbon atoms (*e.g.,* $C_1$-$C_{12}$ or $C_1$-$C_6$). The alkyl includes, but is not limited to, methyl, ethyl, *n*-propyl, isopropyl, *n*-butyl, isobutyl, *sec*-butyl, *tert*-butyl, *n*-pentyl, *n*-hexyl, *etc.*

The term "alkenyl" refers to a straight or branched, unsaturated monovalent hydrocarbon group having a specified number of carbon atoms (*e.g.,* $C_2$-$C_6$) with one or more (*e.g.*, 1, 2, or 3) carbon-carbon sp2 double bonds. The alkenyl includes, but is not limited to, vinyl,

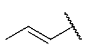,

*etc.*

The term "heterocycloalkenyl" refers to a cyclic, unsaturated monovalent hydrocarbon group having a specified number of ring atoms (*e.g.,* 5- to 14-membered or 5- to 10-membered), a specified number of heteroatoms (*e.g.*, 1, 2, or 3),

and a specified type of heteroatoms (one or more types of N, O, and S), which has one or more (*e.g.*, 1, 2, or 3) carbon-carbon sp2 double bonds and is not aromatic. (Monocyclic) heterocycloalkenyl is connected to the molecule via a carbon atom or a heteroatom.

The term "heterocycloalkyl" refers to a cyclic group having a specified number of ring atoms (*e.g.,* 5- to 14-membered, 5- to 10-membered, or 5- to 6-membered), a specified number of heteroatoms (*e.g.*, 1, 2, 3, or 4), and a specified type of heteroatoms (one or more types of N, O, and S), which is a monocyclic ring, a bridged ring, or a spiro ring (the bridged ring and the spiro ring may be a bicyclic ring or a tricyclic ring), and each ring is saturated. The heterocycloalkyl includes, but is not limited to, azetidinyl, tetrahydropyrrolyl, tetrahydrofuryl, morpholinyl, piperidinyl, *etc.*

The term "alkoxy" refers to the group $R^X$-O-, where the definition of $R^X$ is the same as that in the term "alkyl". The alkoxy includes, but is not limited to: methoxy, ethoxy, n-propoxy, isopropoxy, *etc.*

The term "alkylene" refers to a divalent group connected to the rest of the molecule via two single bonds, with the remaining definition being the same as the term "alkyl".

The term "cycloalkyl" refers to a cyclic, saturated monovalent hydrocarbon group having a specified number of carbon atoms (*e.g.*, $C_3$-$C_{12}$, $C_3$-$C_8$, or $C_3$-$C_6$), which is a monocyclic ring, a bridged ring, or a spiro ring (the bridged ring and the spiro ring may be a bicyclic ring or a tricyclic ring). The cycloalkyl includes, but is not limited to:

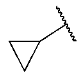, 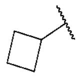, 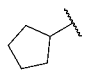, 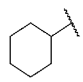,

*etc.*

The term "aryl" refers to a cyclic, unsaturated monovalent hydrocarbon group having a specified number of carbon atoms (*e.g.*, $C_6$-$C_{10}$), which is a monocyclic ring or a polycyclic ring (*e.g.,* 2 or 3 rings). When the aryl is a polycyclic ring, the monocyclic rings share two atoms and one bond, and each ring is aromatic. The aryl includes, but is not limited to, phenyl, naphthyl, *etc.*

The term "heterocycloalkyl" refers to a cyclic, saturated monovalent group having a specified number of ring atoms (*e.g.,* 5- to 14-membered, 5- to 10-membered, or 5- to 6-membered), a specified number of heteroatoms (*e.g.*, 1, 2, 3, or 4), and a specified type of heteroatoms (one or more types of P, N, O, and S), which is a monocyclic heterocycloalkyl, or a bicyclic or tricyclic fused, bridged, or spiro heterocycloalkyl. The heterocycloalkyl includes, but is not limited to:

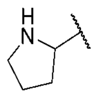,

*etc.*

The term "heteroaryl" refers to a cyclic, unsaturated monovalent group having a specified number of ring atoms (*e.g.,* 5- to 14-membered, 5- to 10-membered, or 5- to 6-membered), a specified number of heteroatoms (*e.g.*, 1, 2, or 3), and a specified type of heteroatoms (one or more types of P, N, O, and S), which is a monocyclic ring or a polycyclic ring (*e.g.,* 2 or 3 rings), where the monocyclic rings share two atoms and one bond, and each ring is aromatic. The heteroaryl is connected to the rest of the molecule via a carbon atom or a heteroatom; the heteroaryl is connected to the rest of the molecule through a ring with heteroatoms or a ring without heteroatoms. The heteroaryl includes, but is not limited to:

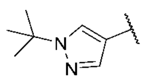, 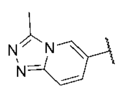, 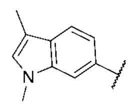,

*etc.*

The term "pharmaceutical excipient" refers to all substances contained in a pharmaceutical preparation other than the active pharmaceutical ingredient, generally classified into two categories: vehicles and additives. For details, please refer to the "Pharmacopoeia of the People's Republic of China (2020 Edition)" and the "Handbook of Pharmaceutical Excipients" (Paul J Sheskey, Bruno C Hancock, Gary P Moss, David J Goldfarb, 2020, 9th Edition).

On the basis of not violating the common sense in the field, the preferred conditions above can be arbitrarily combined to obtain the preferred examples of the present disclosure.

The reagents and raw materials used in the present disclosure are commercially available.

The positive and progressive effects of the present disclosure are that the compounds of the present disclosure have good GPR40 agonistic activity, and further have good pharmacokinetics and low toxicity.

DETAILED DESCRIPTION OF THE PREFERRED EMBODIMENT

The present disclosure is further described below by the way of examples, but the present disclosure is not thereby limited to the scope of the described examples. Experimental methods without specific conditions in the following examples are selected according to conventional methods and conditions, or according to the commercial specification.

**Synthesis of Intermediate M1**

Synthetic Route:

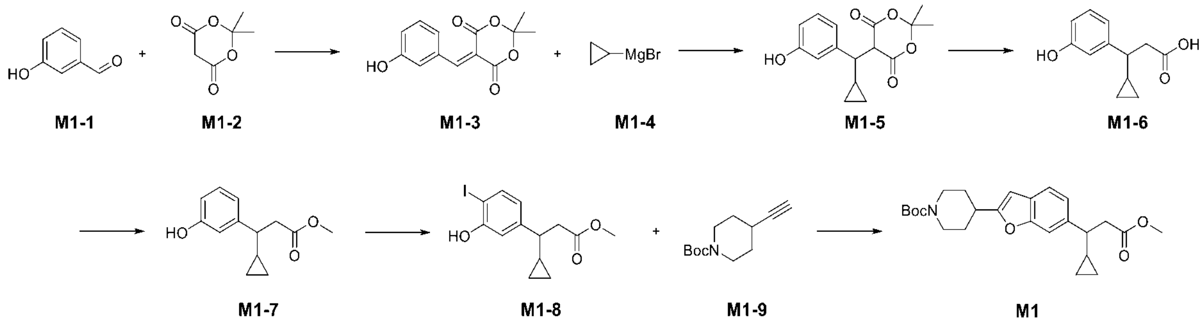

M1-1 M1-2 M1-3 M1-4 M1-5 M1-6

M1-7 M1-8 M1-9 M1

To a 500 mL three-necked flask, compound **M1-1** (17 g, 0.139 mol) and water (300 mL) were added separately and stirred to dissolve and carry out the reaction under an 80°C oil bath. Then, the pre-weighed compound **M1-2** (0.167 mol, 24 g) was slowly added to the reaction system. The reaction was continued at this temperature for 40 minutes, followed by hot filtration to obtain compound **M1-3** (31 g, yield: 90%). MS (ESI, m/z): 249.2 $[M+H]^+$.

In a 500 mL three-necked flask under a nitrogen atmosphere, compound **M1-3** (11 g, 44.31 mmol) and anhydrous tetrahydrofuran (20 mL) were added separately and stirred to dissolve. Cyclopropylmagnesium bromide **M1-4** (352 mL, 265.88 mmol) was slowly added dropwise under an ice bath. After the dropwise addition was completed, the reaction was gradually warmed to room temperature and continued for 2 hours, and then quenched with saturated ammonium chloride aqueous solution (20 mL). The reaction mixture was extracted with dichloromethane (20 mL × 3), and the combined organic phases were dried over anhydrous sodium sulfate and concentrated. The resulting crude product was purified by normal-phase column chromatography (ethyl acetate: petroleum ether = 0% to 100%) to obtain compound **M1-5** (8.7 g, yield: 68%) as a pale yellow solid. MS (ESI, m/z): 291.3 $[M+H]^+$.

In a 250 mL three-necked flask, compound **M1-5** (8.7 g, 29.97 mol) was added, and then *N,N*-dimethylformamide (50 mL) and water (5 mL) were added and stirred to dissolve. The reaction mixture was then placed in a 100°C oil bath and reacted overnight. The reaction was stopped after TLC monitoring confirmed the complete consumption of the starting material. Ethyl acetate (150 mL × 3) was added for extraction. The combined organic phases were dried over anhydrous sodium sulfate and concentrated to obtain crude compound **M1-6** (7.48 g) as a yellow solid, which was directly used in the next step. MS (ESI, m/z): 207.2 $[M+H]^+$.

At room temperature, in a 100 mL three-necked flask, compound **M1-6** (7.48 g, 36.27 mmol) and methanol (10 mL) were added and stirred to dissolve. Concentrated sulfuric acid (2 mL, 37.5 mmol) was then added, and the reaction was stirred for an additional 2 hours until TLC monitoring confirmed the complete consumption of the starting material. Ethyl acetate (20 mL × 3) and water (20 mL) were added to the reaction mixture for extraction. The combined organic phases were washed with saturated sodium bicarbonate aqueous solution (50 mL × 3), dried, and concentrated. The resulting crude product was purified by normal-phase column chromatography (ethyl acetate: petroleum ether = 0% to 100%) to obtain compound **M1-7** (4 g, yield: 50%) as a pale yellow solid. MS (ESI, m/z): 221.2 $[M+H]^+$.

Under an ice-water bath, in a 100 mL three-necked flask, compound **M1-7** (4 g, 18.16 mmol) and dichloromethane (50 mL) were added and stirred to dissolve. Then, N-iodosuccinimide (4.9 g, 21.79 mmol) was slowly added. After reacting for 1 hour, LCMS monitoring confirmed the complete consumption of the starting material. Dichloromethane (50 mL) and water (50 mL) were added for extraction. The organic phase was washed with saturated ammonium chloride (50 mL × 3), dried, and concentrated. The resulting crude product was purified by normal-phase column chromatography (ethyl acetate: petroleum ether = 0% to 100%) to obtain compound **M1-8** (2.87 g, yield: 46%) as a brown solid. MS (ESI, m/z): 347.2 $[M+H]^+$.

In a 25 mL three-necked flask under a nitrogen atmosphere, compound **M1-8** (1.47 g, 4.247 mmol), copper(I) iodide (0.08 g, 0.425 mmol), and bis(triphenylphosphine)palladium(II) chloride (0.17 g, 0.212 mmol) were added, followed by the addition of acetonitrile (20 mL) with stirring. Triethylamine (0.590 mL, 4.247 mmol) was then added, and the stirring

was continued. The reaction mixture was stirred at 80°C for 2 minutes, and then compound **M1-9** (0.89 g, 4.247 mmol) was added. After the addition was completed, the reaction was continued at 80°C for 90 minutes. After the reaction was completed, the reaction mixture was cooled to room temperature, and dichloromethane (50 mL) and water (50 mL) were added to the resulting suspension for extraction. The combined organic phases were dried and concentrated. The resulting crude product was purified by normal-phase column chromatography (ethyl acetate: petroleum ether = 0% to 100%) to obtain compound **M1** (1.2 g, yield: 66%) as a brown solid. MS (ESI, m/z): 428.3 $[M+H]^+$.

**Synthesis of Intermediate M2**

Synthetic Route:

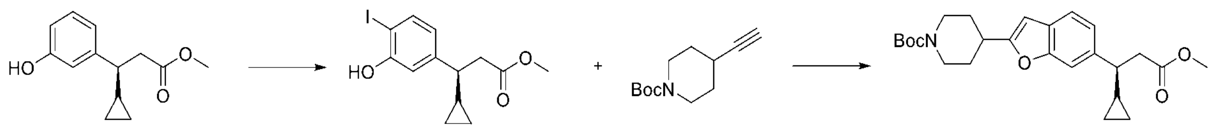

M2-1 M2-2 M1-9 M2

Referring to the synthetic route of intermediate **M1,** compound **M1-7** was replaced with commercially available starting material compound **M2-1:** methyl (*S*)-3-cyclopropyl-3-(3-hydroxyphenyl)propanoate to obtain compound **M2** (565 mg, yield: 93%) as a yellow oil. MS (ESI, m/z): 428.3 $[M+H]^+$.

Example **1:**

Synthetic Route:

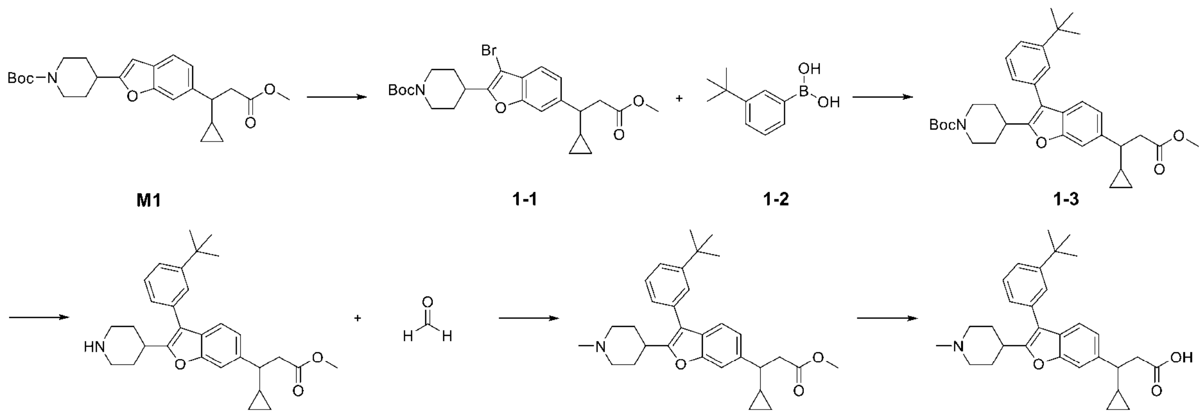

M1 1-1 1-2 1-3

1-4 1-5 1-6 1

To a solution of compound **M1** (1.69 g, 3.953 mmol) in anhydrous dichloromethane (20 mL), *N*-bromosuccinimide (0.845 g, 4.748 mmol) was slowly added under an ice-water bath. The reaction mixture was stirred in the ice-water bath for 2 hours. After the reaction was completed, water (50 mL) was added to the reaction system, followed by extraction with dichloromethane (30 mL × 3). The combined organic phases were dried and concentrated, then purified by normal-phase column chromatography (ethyl acetate: petroleum ether = 0% to 100%) to obtain compound **1-1** (1.48 g, yield: 74%) as a brownish-red solid. MS (ESI, m/z): 506.3 $[M+H]^+$.

At room temperature, in a 100 mL three-necked flask under a nitrogen atmosphere, compound **1-1** (200 mg, 0.396 mmol), potassium carbonate (120 mg, 0.871 mmol), bis(triphenylphosphine)palladium(II) chloride (57.4 mg, 0.081 mmol), and 3-*tert*-butylbenzeneboronic acid **1-2** (144.2 mg, 0.81 mmol) were added. Then, 1,4-dioxane (10 mL) and water (2 mL) were added and stirred. The reaction mixture was placed in a 100°C oil bath and reacted for 3 hours. LCMS monitoring confirmed the complete consumption of the starting material. After the reaction was stopped, the reaction mixture was cooled to room temperature and rotary evaporated to dryness. Water (10 mL) was added, and the mixture was extracted with ethyl acetate (10 mL × 3). The combined organic phases were dried, concentrated, and purified by normal-

phase column chromatography (ethyl acetate: petroleum ether = 0% to 10%) to obtain compound **1-3** (168 mg, yield: 74%) as a yellow oil. MS (ESI, m/z): 582.4 $[M+Na]^+$.

To a solution of compound **1-3** (168 mg, 0.3 mmol) and dichloromethane (2 mL), trifluoroacetic acid (137 mg, 1.2 mmol) was added at room temperature. The reaction was carried out at room temperature for 2 hours. LCMS monitoring confirmed the complete consumption of the starting material. The pH of the reaction system was adjusted to neutral with saturated sodium bicarbonate solution, followed by extraction with dichloromethane (5 mL $\times$ 3). The combined organic phases were dried and concentrated, then purified by normal-phase column chromatography (ethyl acetate: petroleum ether = 0% to 100%) to obtain compound **1-4** (150 mg, yield: 96%) as a yellow oil. MS (ESI, m/z): 460.3 $[M+H]^+$.

To a solution of compound **1-4** (100 mg, 0.22 mmol), anhydrous magnesium sulfate (637 mg, 5.30 mmol), dichloroethane (5 mL), methanol (5 mL), and 40% formaldehyde (5 mL), acetic acid (0.1 mL) was added at room temperature. The reaction was carried out at room temperature for 4 hours, followed by the addition of sodium triacetoxyborohydride (92 mg, 0.44 mmol). The reaction was continued at room temperature for 16 hours. LCMS monitoring confirmed the complete consumption of the starting material. The reaction was quenched with 5 M sodium hydroxide solution (20 mL) and extracted with ethyl acetate (20 mL $\times$ 3). The combined organic phases were dried and concentrated, then purified by normal-phase column chromatography (ethyl acetate: petroleum ether = 0% to 20%) to obtain compound **1-6** (70 mg, yield: 67%) as a yellow oil. MS (ESI, m/z): 474.4 $[M+H]^+$.

At room temperature, compound **1-6** (70 mg, 0.14 mmol) and methanol (5 mL) were stirred to dissolve. Lithium hydroxide (3 mg, 0.14 mmol) was then slowly added, and the reaction mixture was reacted at room temperature for 16 hours. LCMS monitoring confirmed the complete consumption of the starting material. The resulting crude product obtained after direct concentration was purified by reverse-phase column chromatography [acetonitrile/water (0.05% formic acid) = 0% to 100%] to obtain compound **1** (14 mg, yield: 22%). MS (ESI, m/z): 460.3 $[M+H]^+$. $^1H$ NMR (400 MHz, DMSO-$d_6$) $\delta$ 7.51 (s, 1H), 7.48 - 7.38 (m, 4H), 7.33 - 7.27 (m, 1H), 7.16 (d, $J$ = 8.0 Hz, 1H), 2.93 - 2.77 (m, 3H), 2.73 - 2.65 (m, 2H), 2.43 - 2.34 (m, 1H), 2.18 (s, 3H), 2.03 - 1.75 (m, 6H), 1.34 (s, 9H), 1.12 - 1.04 (m, 1H), 0.55 - 0.49 (m, 1H), 0.34 - 0.24 (m, 2H), 0.18 - 0.11(m, 1H).

Example **2:**

Synthetic Route:

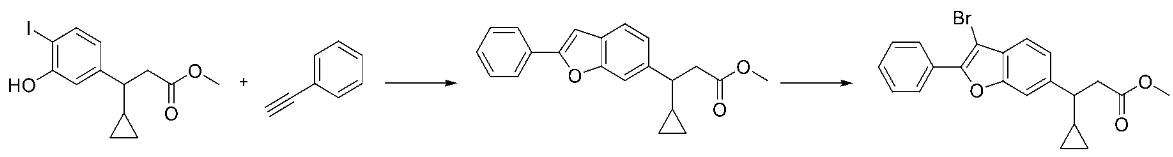

**M1-8** **2-1** **2-2** **2-3**

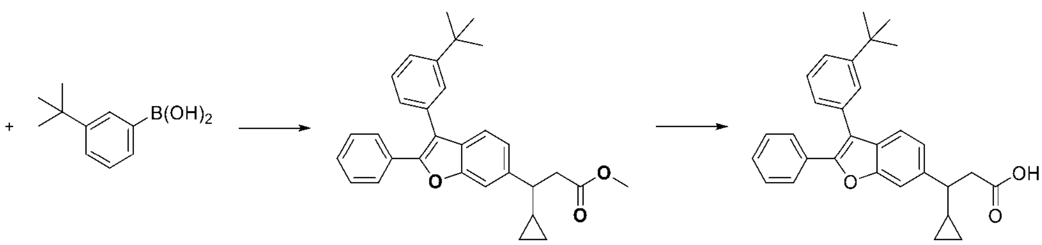

**1-2** **2-4** **2**

To a solution of compound **M1-8** (200 mg, 0.58 mmol) in triethylamine (5 mL), compound **2-1** (65 mg, 0.64 mmol), copper(I) iodide (5.5 mg, 0.03 mmol), and bis(triphenylphosphine)palladium(II) chloride (20 mg, 0.03 mmol) were added. The reaction was stirred at 90°C for 12 hours. LCMS monitoring confirmed the complete consumption of the starting material. The reaction mixture was directly concentrated, and the resulting crude product was purified by normal-phase column chromatography (ethyl acetate: petroleum ether = 0% to 100%) to obtain compound **2-2** (120 mg, yield: 65%) as a pale yellow oil. MS (ESI, m/z): 321.2 $[M+H]^+$.

To a solution of compound **2-2** (120 mg, 0.38 mmol) in dichloromethane (5 mL) at 0°C, N-bromosuccinimide (68 mg, 0.38 mmol) was added, and the reaction was stirred at room temperature for 4 hours. LCMS monitoring confirmed the complete consumption of the starting material. The reaction mixture was directly concentrated, and the resulting crude product was purified by normal-phase column chromatography (ethyl acetate: petroleum ether = 0% to 100%) to obtain compound **2-3** (148 mg, yield: 98%) as a pale yellow oil. MS (ESI, m/z): 421.0 $[M+Na]^+$.

To a mixture of compound **2-3** (148 mg, 0.37 mmol) in 1,4-dioxane (6 mL) and water (2 mL), compound **1-2** (66 mg, 0.37 mmol), potassium phosphate (235 mg, 1.11 mmol), and [1,1'-bis(diphenylphosphino)ferrocene]palladium(II) dichloride (29 mg, 0.04 mmol) were added. The reaction was stirred at 90°C for 5 hours. LCMS monitoring confirmed the complete consumption of the starting material. The reaction mixture was directly concentrated, and the resulting crude product was purified by normal-phase column chromatography (ethyl acetate: petroleum ether = 0% to 100%) to obtain compound **2-4** (137 mg, yield: 82%) as a pale yellow oil. MS (ESI, m/z): 453.2 $[M+H]^+$.

To a mixture of compound **2-4** (137 mg, 0.30 mmol) in tetrahydrofuran (2 mL), methanol (2 mL), and water (2 mL), lithium hydroxide (22 mg, 0.91 mmol) was added. The reaction was stirred at room temperature for 4 hours. LCMS monitoring confirmed the complete consumption of the starting material. The reaction mixture was directly concentrated, and the resulting crude product was purified by reverse-phase column chromatography [acetonitrile/water (0.05% formic acid) = 0% to 100%] to obtain compound **2** (46 mg, yield: 35%) as a white solid. MS (ESI, m/z): 439.2 $[M+H]^+$.

$^1H$ NMR (400 MHz, DMSO-$d_6$) $\delta$ 7.58 (dd, $J$ = 8.0, 1.6 Hz, 2H), 7.53 (s, 1H), 7.48 - 7.42 (m, 3H), 7.42 - 7.26 (m, 5H), 7.20 (d, $J$ = 8.0 Hz, 1H), 2.60 - 2.41 (m, 3H), 1.25 (s, 9H), 1.02 - 0.98 (m, 1H), 0.47 - 0.45 (m, 1H), 0.34 - 0.22 (m, 2H), 0.16 - 0.08 (m, 1H).

**Example 3:**

Synthetic Route:

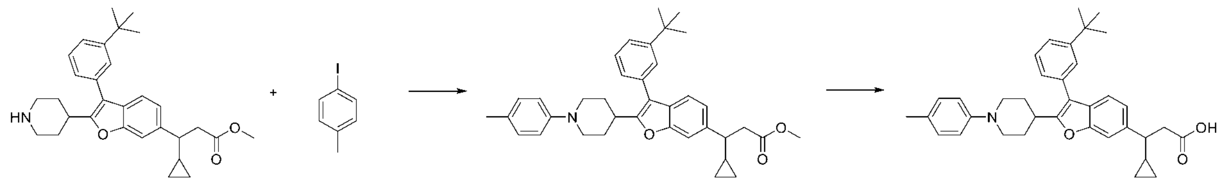

1-4 3-1 3-2 3

In a 25 mL single-necked flask, compound **1-4** (68.9 mg, 0.15 mmol), 2-dicyclohexylphosphino-2',6'-diisopropoxybiphenyl (3.1 mg, 0.006 mmol), tris(dibenzylideneacetone)dipalladium (3.2 mg, 0.003 mmol), compound **3-1** (65.4 mg, 0.3 mmol), and cesium carbonate (146.6 mg, 0.45 mmol) were added. Toluene (5 mL) was then added and stirred. The reaction mixture was placed at 100°C and reacted overnight. LCMS monitoring confirmed the complete consumption of the starting material. After the reaction was stopped, dichloromethane (20 mL) and water (20 mL) were added to the reaction mixture for extraction. The organic phase was dried and concentrated, and the resulting crude compound **3-2** (160 mg) was directly used in the next step. MS (ESI, m/z): 550.3 $[M+H]^+$.

Referring to the synthetic route of compound **1,** the synthesis was carried out, and the resulting crude product was purified by reverse-phase column chromatography [acetonitrile/water (0.05% formic acid) = 0% to 100%] to obtain compound **3** (17 mg, yield: 8%) as a white solid. MS (ESI, m/z): 536.3 $[M+H]^+$.

$^1H$ NMR (400 MHz, MeOD) $\delta$ 7.50 (s, 1H), 7.47 - 7.37 (m, 4H), 7.31 - 7.29 (m, 1H), 7.16 (d, $J$ = 8.4 Hz, 1H), 7.01 (d, $J$ = 9.2 Hz, 2H), 6.85 (d, $J$ = 9.2 Hz, 2H), 3.76 - 3.66 (m, 2H), 3.13 - 3.04 (m, 1H), 2.82 - 2.64 (m, 4H), 2.49 - 2.47 (m, 1H), 2.32 - 2.17 (m, 5H), 2.01 - 1.93 (m, 2H), 1.39 (s, 9H), 1.14 - 1.11 (m, 1H), 0.62 - 0.59 (m, 1H), 0.42 - 0.33 (m, 2H), 0.21 - 0.16 (m, 1H).

**Example 4:**

Synthetic Route:

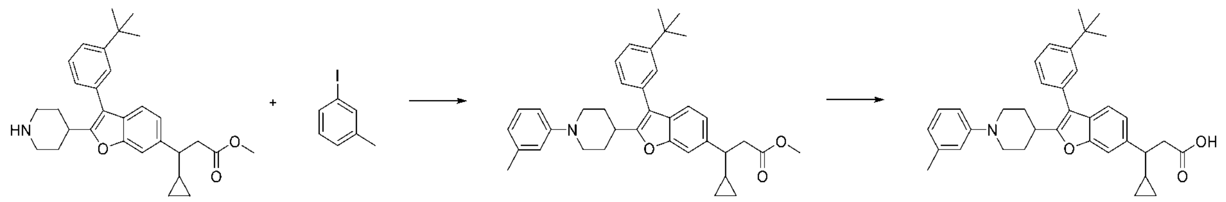

1-4 4-1 4-2 4

Referring to the synthetic route of compound **3,** compound **3-1** was replaced with compound **4-1** to carry out the synthesis. The resulting crude product was purified by reverse-phase column chromatography [acetonitrile/water (0.05% formic acid) = 0% to 100%] to obtain compound **4** (15 mg, yield: 7%) as a white solid. MS (ESI, m/z): 536.3 $[M+H]^+$.

$^1$H NMR (400 MHz, MeOD) δ 7.51 (s, 1H), 7.46 - 7.35 (m, 4H), 7.32 - 7.28 (m, 1H), 7.17 - 7.09 (m, 2H), 6.87 - 6.80 (m, 2H), 6.69 (d, *J* = 7.6 Hz, 1H), 3.82 - 3.73 (m, 2H), 3.13 - 3.04 (m, 1H), 2.87 - 2.67 (m, 4H), 2.51 - 2.42 (m, 1H), 2.31 - 2.17 (m, 5H), 2.01 - 1.91 (m, 2H), 1.39 (s, 9H), 1.17 - 1.07 (m, 1H), 0.65 - 0.56 (m, 1H), 0.46 - 0.30 (m, 2H), 0.22 - 0.13 (m, 1H).

**Example 5:**

Synthetic Route:

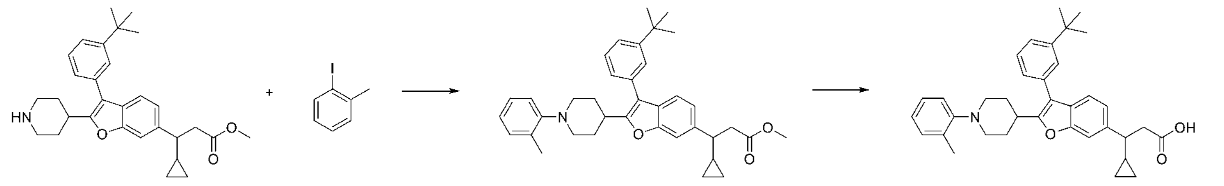

1-4 5-1 5-2 5

Referring to the synthetic route of compound **3,** compound **3-1** was replaced with compound **5-1** to carry out the synthesis. The resulting crude product was purified by reverse-phase column chromatography [acetonitrile/water (0.05% formic acid) = 0% to 100%] to obtain compound **5** (11 mg, yield: 19%) as a white solid. MS (ESI, m/z): 536.3 $[M+H]^+$.

$^1$H NMR (400 MHz, $CDCl_3$) δ 7.49 (s, 1H), 7.47-7.45 (m, 3H), 7.38 (d, *J* = 8.0 Hz, 1H), 7.33 (d, *J* = 6.8 Hz, 1H), 7.20-7.11 (m, 3H), 7.03 (d, *J* =7.6 Hz, 1H), 6.96-6.94 (m, 1H), 3.16-3.14 (m, 2H), 3.04-3.00 (m, 1H), 2.69-2.66 (m, 2H), 2.50-2.26 (m, 6H), 2.16-2.14 (m, 2H), 1.94-1.91 (m, 2H), 1.35 (s, 9H), 1.05-0.95 (s, 1H), 0.47-0.41 (m, 1H), 0.31-0.23 (m, 2H), 0.11-0.05 (m, 1H).

**Example 6:**

Synthetic Route:

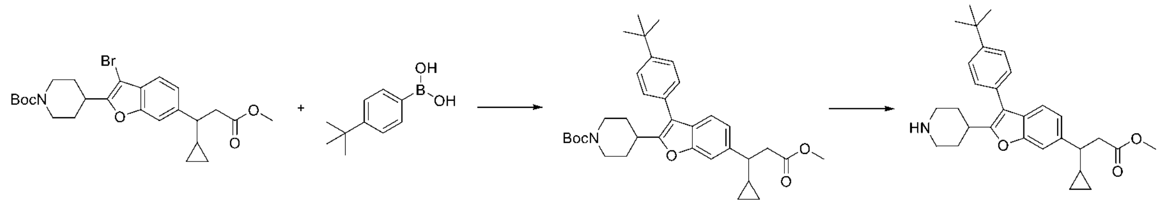

1-1 6-1 6-2 6-3

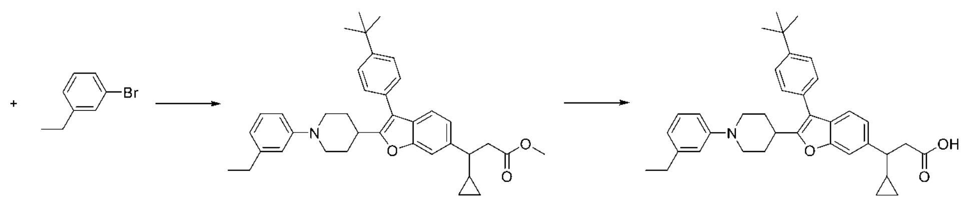

6-4 6-5 6

Referring to the synthetic route of compound **1,** compound **1-2** was replaced with compound **6-1** to carry out the synthesis and obtain compound **6-3.** Then, referring to the synthetic route of compound **3,** compound **3-1** was replaced with compound **6-4** to carry out the synthesis, and the resulting crude product was purified by reverse-phase column chromatography [acetonitrile/water (0.05% formic acid) = 0% to 100%] to obtain compound **6** (16 mg, yield: 50%) as a white solid. MS (ESI, m/z): 550.3 $[M+H]^+$.

$^1$H NMR (400 MHz, DMSO-$d_6$) δ 7.55 (d, *J* = 8.4 Hz, 2H), 7.46 - 7.43 (m, 3H), 7.38 (d, *J* = 8.0 Hz, 1H), 7.14 - 7.10

(m, 2H), 6.86 - 6.72 (m, 2H), 6.63 (d, *J* = 7.6 Hz, 1H), 3.85 - 3.74 (m, 2H), 3.12 - 3.05 (m, 1H), 2.81 - 2.75 (m, 2H), 2.58 - 2.35 (m, 5H), 2.31 - 2.03 (m, 2H), 1.89 - 1.86 (m, 2H), 1.35 (s, 9H), 1.21 - 1.14 (m, 3H), 1.06 - 0.92 (m, 1H), 0.47 - 0.43 (m, 1H), 0.31 - 0.20 (m, 2H), 0.14 - 0.03 (m, 1H).

**Example 7:**

Synthetic Route:

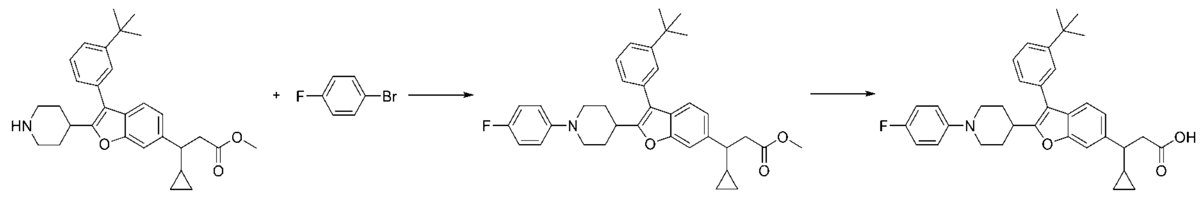

1-4 7-1 7-2 7

Referring to the synthetic route of compound **3,** compound **3-1** was replaced with compound **7-1** to carry out the synthesis. The resulting crude product was purified by reverse-phase column chromatography [acetonitrile/water (0.05% formic acid) = 0% to 100%] to obtain compound **57** (5 mg, yield: 32%) as a white solid. MS (ESI, m/z): 540.4 [M+H]$^+$.

$^1$H NMR (400 MHz, DMSO-*d*$_6$) $\delta$ 7.53 - 7.43 (m, 4H), 7.41 (d, *J* = 8.0 Hz, 1H), 7.37 - 7.33 (m, 1H), 7.20 - 7.15 (m, 1H), 7.12 - 6.96 (m, 4H), 3.80 - 3.71 (m, 2H), 3.12 - 3.02 (m, 1H), 2.80 - 2.70 (m, 2H), 2.56 - 2.38 (m, 3H), 2.16 - 2.04 (m, 2H), 1.98 - 1.88 (m, 2H), 1.37 (s, 9H), 1.06 - 0.98 (m, 1H), 0.52 - 0.44 (m, 1H), 0.32 - 0.24 (m, 2H), 0.15 - 0.07 (m, 1H).

**Example 8:**

Synthetic Route:

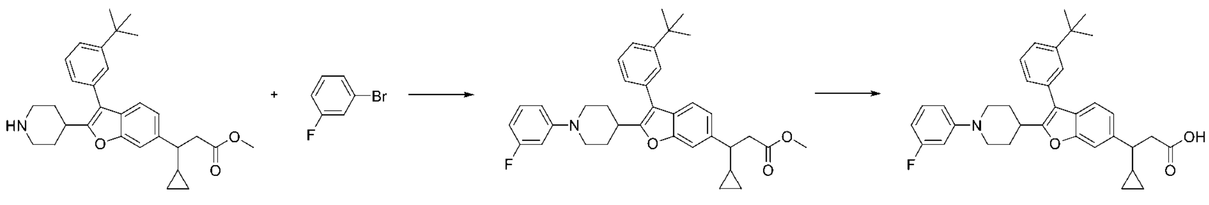

1-4 8-1 8-2 8

Referring to the synthetic route of compound **3,** compound **3-1** was replaced with compound **8-1** to carry out the synthesis. The resulting crude product was purified by reverse-phase column chromatography [acetonitrile/water (0.05% formic acid) = 0% to 100%] to obtain compound **8** (33 mg, yield: 51%) as a white solid. MS (ESI, m/z): 540.2 [M+H]$^+$.

$^1$H NMR (400 MHz, DMSO-*d*$_6$) $\delta$ 7.50 - 7.29 (m, 6H), 7.25 - 7.12 (m, 2H), 6.82 - 6.73 (m, 2H), 6.55 - 6.48 (m, 1H), 3.90 - 3.87 (m, 2H), 3.15 - 3.12 (m, 1H), 2.88 - 2.74 (m, 2H), 2.52 - 2.36 (m, 3H), 2.07 - 1.96 (m, 2H), 1.90 - 1.87 (m, 2H), 1.34 (s, 9H), 0.98 - 0.95 (m, 1H), 0.46 - 0.44 (m, 1H), 0.30 - 0.20 (m, 2H), 0.08 - 0.06 (m, 1H).

**Example 9:**

Synthetic Route:

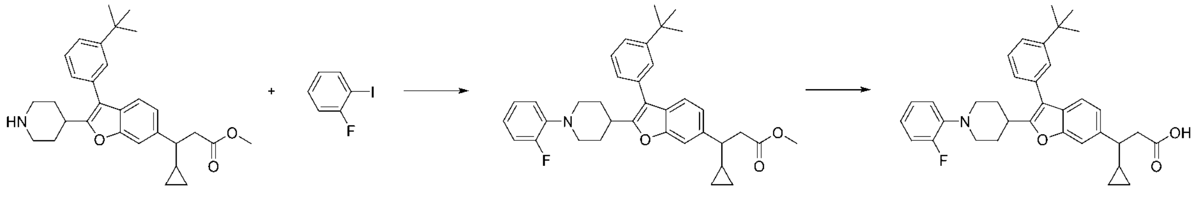

1-4 9-1 9-2 9

Referring to the synthetic route of compound **3,** compound **3-1** was replaced with compound **9-1** to carry out the synthesis. The resulting crude product was purified by reverse-phase column chromatography [acetonitrile/water (0.05% formic acid) = 0% to 100%] to obtain compound **9** (6 mg, yield: 29%) as a white solid. MS (ESI, m/z): 540.3 $[M+H]^+$.

$^1H$ NMR (400 MHz, MeOD) $\delta$ 7.51 (s, 1H), 7.45 - 7.39 (m, 3H), 7.32 - 7.29 (m, 1H), 7.16 (d, *J* = 9.2 Hz, 1H), 7.09 - 6.95 (m, 5H), 3.57 - 3.53 (m, 2H), 3.13 - 3.12 (m, 1H), 2.82 - 2.73 (m, 4H), 2.51 - 2.46 (m, 1H), 2.33 - 2.28 (m, 2H), 1.99 - 1.95 (m, 2H), 1.39 (s, 9H), 1.16 - 1.12 (m, 1H), 0.63 - 0.60 (m, 1H), 0.44 - 0.33 (m, 2H), 0.20 - 0.17 (m, 1H).

**Example 10:**

Synthetic Route:

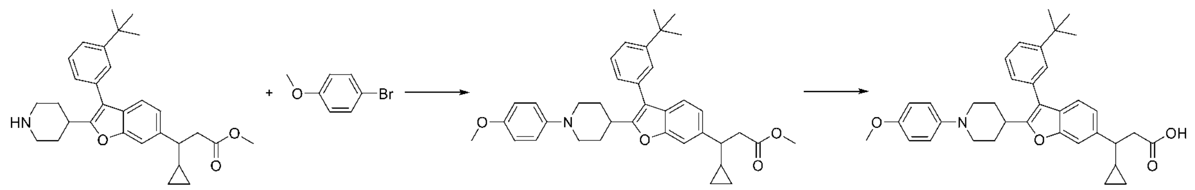

1-4 10-1 10-2 10

Referring to the synthetic route of compound **3,** compound **3-1** was replaced with compound **10-1** to carry out the synthesis. The resulting crude product was purified by reverse-phase column chromatography [acetonitrile/water (0.05% formic acid) = 0% to 100%] to obtain compound **10** (4 mg, yield: 12%) as a white solid. MS (ESI, m/z): 552.3 $[M+H]^+$.

$^1H$ NMR (400 MHz, MeOD) $\delta$ 7.50 (s, 1H), 7.47 - 7.37 (m, 4H), 7.31 - 7.29 (m, 1H), 7.16 (d, *J* = 8.4 Hz, 1H), 7.01 (d, *J* = 9.2 Hz, 2H), 6.85 (d, *J* = 9.2 Hz, 2H), 3.75 (s, 3H), 3.62 - 3.59 (m, 2H), 3.13 - 3.06 (m, 1H), 2.82 - 2.64 (m, 4H), 2.49 - 2.47 (m, 1H), 2.29 - 2.25 (m, 2H), 1.99 - 1.95 (m, 2H), 1.39 (s, 9H), 1.14 - 1.11 (m, 1H),0.62 - 0.59 (m, 1H), 0.42 - 0.33 (m, 2H), 0.21 - 0.16 (m, 1H).

**Example 11:**

Synthetic Route:

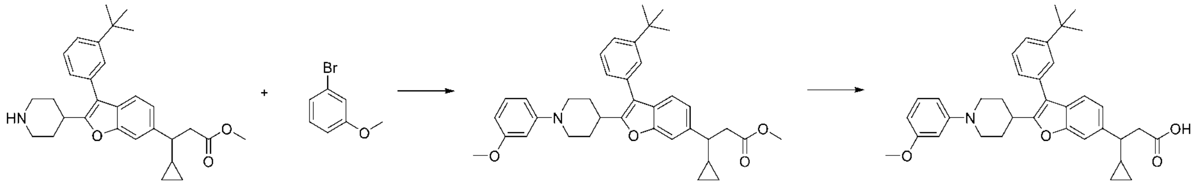

1-4 11-1 11-2 11

Referring to the synthetic route of compound **3,** compound **3-1** was replaced with compound **11-1** to carry out the synthesis. The resulting crude product was purified by reverse-phase column chromatography [acetonitrile/water (0.05% formic acid) = 0% to 100%] to obtain compound **11** (18 mg, yield: 32%) as a white solid. MS (ESI, m/z): 552.3 $[M+H]^+$.

$^1H$ NMR (400 MHz, MeOD) $\delta$ 7.50 (s, 1H), 7.45-7.39 (m, 3H), 7.38 (s, 1H), 7.32-7.28 (m, 1H), 7.18-7.10 (m, 2H), 6.61 (dd, *J* = 8.0, 2.0 Hz, 1H), 6.56-6.52 (m, 1H), 6.43 (dd, *J* = 8.0, 2.0 Hz, 1H), 3.84-3.74 (m, 5H), 3.15-3.05 (m, 1H), 2.85-2.69 (m, 4H), 2.51-2.42 (m, 1H), 2.30-2.15 (m, 2H), 2.00-1.90 (m, 2H), 1.38 (s, 9H), 1.18-1.06 (m, 1H), 0.65-0.56 (m,

1H), 0.46-0.28 (m, 2H), 0.22-0.11 (m, 1H).

**Example 12:**

Synthetic Route:

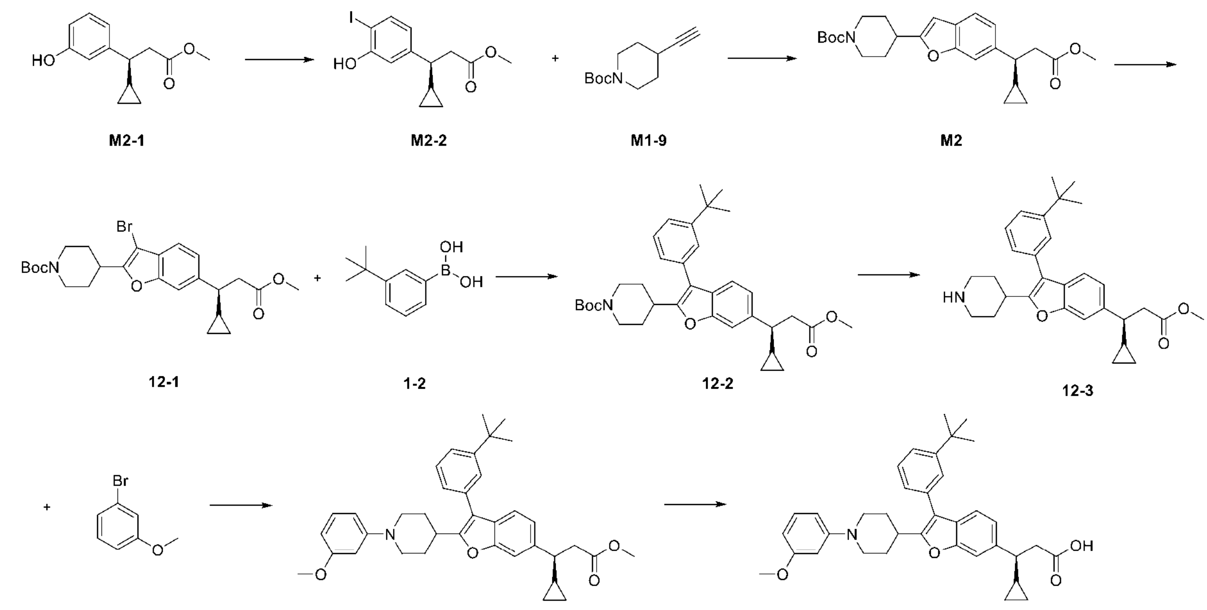

M2-1 M2-2 M1-9 M2

12-1 1-2 12-2 12-3

11-1 12-4 12

Referring to the synthetic route of intermediate **M1,** intermediate **M2** was obtained. Referring to the synthetic route of compound **11,** the synthesis was carried out, and the resulting crude product was purified by reverse-phase column chromatography [acetonitrile/water (0.05% formic acid) = 0% to 100%] to obtain compound **12** (13 mg, yield: 33%) as a white solid. MS (ESI, m/z): 552.3 $[M+H]^+$.

$^1H$ NMR (400 MHz, $CDCl_3$) δ 7.51 (d, *J* = 8.8 Hz, 1H), 7.44 (d, *J* = 6.4 Hz, 1H), 7.38 (s, 1H), 7.32 (d, *J* = 6.0 Hz, 1H), 7.20 (t, *J* = 8.0 Hz, 2H), 7.14 (d, *J* = 8.0 Hz, 2H), 6.62 (d, *J* = 8.0 Hz, 1H), 6.54 (s, 1H), 6.44 (d, *J* = 8.0 Hz, 1H), 3.84 - 3.81 (m, 5H), 3.18 - 3.05 (m, 1H), 2.93 - 2.84 (m, 2H), 2.84 - 2.73 (m, 2H), 2.57 - 2.44 (m, 1H), 2.36 - 2.21 (m, 2H), 1.96 (d, *J* = 13.2 Hz, 2H), 1.40 (s, 9H), 1.15 - 1.02 (m, 1H), 0.63 (s, 1H), 0.52 - 0.39 (m, 1H), 0.39 - 0.27 (m, 1H), 0.28 - 0.15 (m, 1H).

**Example 13:**

Synthetic Route:

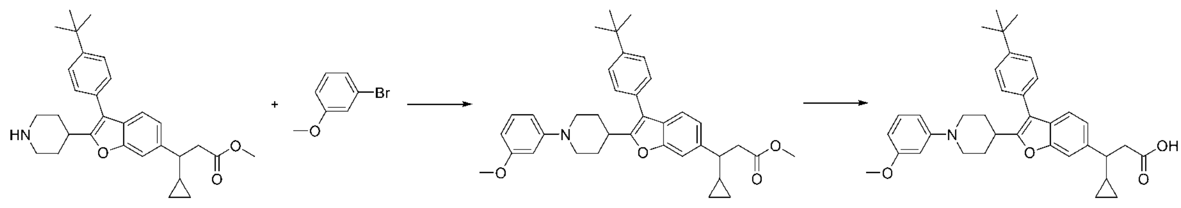

6-3 11-1 13-1 13

Referring to the synthetic route of compound **6,** compound **6-4** was replaced with compound **11-1** to carry out the synthesis. The resulting crude product was purified by reverse-phase column chromatography [acetonitrile/water (0.05% formic acid) = 0% to 100%] to obtain compound **13** (2 mg, yield: 8%) as a white solid. MS (ESI, m/z): 552.3 $[M+H]^+$.

$^1H$ NMR (400 MHz, DMSO-$d_6$) δ 7.55 (d, *J* = 8.4 Hz, 2H), 7.46 - 7.41 (m, 3H), 7.37 (d, *J* = 8.0 Hz, 1H), 7.13 - 7.09 (m, 2H), 6.59 - 6.54 (m, 1H), 6.50 - 6.47 (d, *J* = 2.4 Hz, 1H), 6.37 - 6.34 (m, 1H), 3.86 - 3.77 (m, 2H), 3.72 (s, 3H), 3.13 - 3.07

(m, 1H), 2.84 - 2.77 (m, 2H), 2.55 - 2.36 (m, 3H), 2.08 - 1.99 (m, 2H), 1.88 - 1.84 (m, 2H), 1.35 (s, 9H), 1.01 - 0.95 (m, 1H), 0.46 - 0.41 (m, 1H), 0.28 - 0.21 (m, 2H), 0.08 - 0.04 (m, 1H).

**Example 14:**

Synthetic Route:

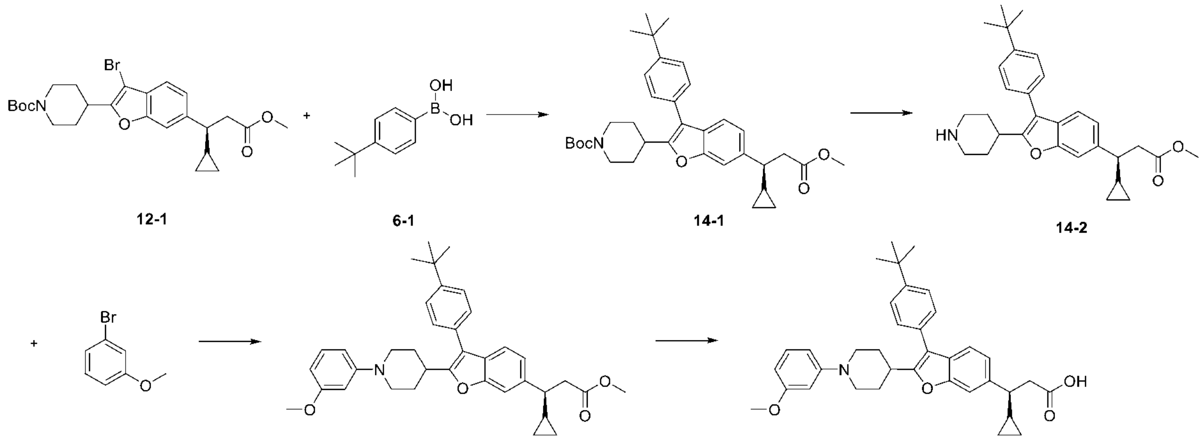

12-1 6-1 14-1 14-2

11-1 14-3 14

Referring to the synthetic route of compound **12,** compound **1-2** was replaced with compound **6-1** to carry out the synthesis. The resulting crude product was purified by reverse-phase column chromatography [acetonitrile/water (0.05% formic acid) = 0% to 100%] to obtain compound **14** (20 mg, yield: 36%) as a white solid. MS (ESI, m/z): 552.3 [M+H]$^+$.

$^1$H NMR (400 MHz, DMSO-$d_6$) $\delta$ 7.51 (t, $J$ = 8.0 Hz, 3H), 7.41 (d, $J$ = 7.6 Hz, 2H), 7.37 (s, 1H), 7.20 (t, $J$ = 8.0 Hz, 1H), 7.12 (d, $J$ = 8.0 Hz, 1H), 6.61 (d, $J$ = 8.0 Hz, 1H), 6.53 (s, 1H), 6.46 - 6.41 (m, 1H), 3.83 - 3.79 (m, 5H), 3.13 - 3.08 (m, 1H), 2.90 - 2.75 (m, 4H), 2.56 - 2.45 (m, 1H), 2.32 - 2.22 (m, 2H), 1.96 - 1.89 (m, 2H), 1.41 (s, 9H), 1.15 - 1.06 (m, 1H), 0.68 - 0.58 (m, 1H), 0.50 - 0.40 (m, 1H), 0.40 - 0.30 (m, 1H), 0.26 - 0.15 (m, 1H).

**Example 15:**

Synthetic Route:

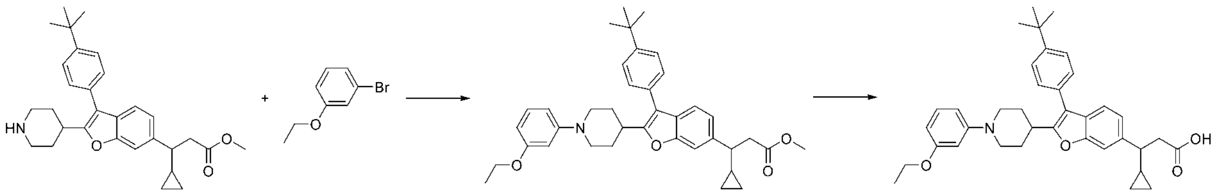

14-2 15-1 15-2 15

Referring to the synthetic route of compound **14,** compound **11-1** was replaced with compound **15-1** to carry out the synthesis. The resulting crude product was purified by reverse-phase column chromatography [acetonitrile/water (0.05% formic acid) = 0% to 100%] to obtain compound **15** (19 mg, yield: 39%) as a white solid. MS (ESI, m/z): 566.3 [M+H]$^+$.

$^1$H NMR (400 MHz, DMSO-$d_6$) $\delta$ 7. 55 (d, $J$ = 8.4 Hz, 2H), 7.47 - 7.41 (m, 3H), 7.38 (d, $J$ = 8.0 Hz, 1H), 7.14 - 7.07 (m, 2H), 6.56 - 6.53 (m, 1H), 6.48 - 6.44 (m, 1H), 6.35 - 6.32 (m, 1H), 3.99 (d, $J$ = 6.8 Hz, 2H), 3.82 - 3.79 (m, 2H), 3.13 - 3.07 (m, 1H), 2.84 - 2.77 (m, 2H), 2.68 - 2.38 (m, 3H), 2.06 - 1.97 (m, 2H), 1.85 - 1.82 (m, 2H), 1.35 (s, 9H), 1.31 (t, $J$ = 6.8 Hz, 3H), 1.03 - 0.98 (m, 1H), 0.47 - 0.42 (m, 1H), 0.30 - 0.23 (m, 2H), 0.09 - 0.05 (m, 1H).

**Example 16:**

Synthetic Route:

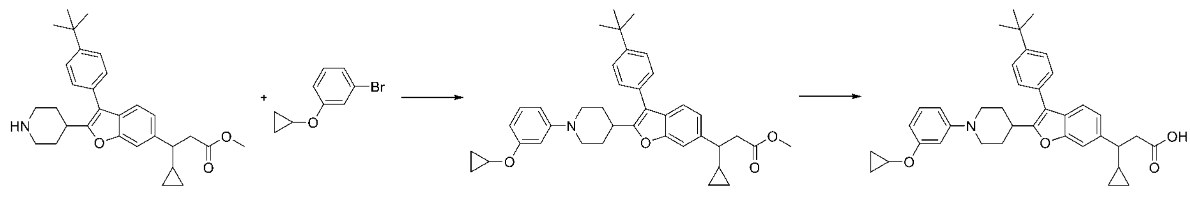

14-2 16-1 16-2 16

Referring to the synthetic route of compound **14,** compound **11-1** was replaced with compound **16-1** to carry out the synthesis. The resulting crude product was purified by reverse-phase column chromatography [acetonitrile/water (0.05% formic acid) = 0% to 100%] to obtain compound **16** (6 mg, yield: 30%) as a white solid. MS (ESI, m/z): 578.7 $[M+H]^+$.

$^1H$ NMR (400 MHz, DMSO-$d_6$) $\delta$ 7.54 (d, $J$ = 8.4 Hz, 2H), 7.43 (d, $J$ = 8.4 Hz, 2H), 7.39 (s, 1H), 7.35 (d, $J$ = 8.0 Hz, 1H), 7.14 - 7.07 (m, 2H), 6.61 - 6.55 (m, 2H), 6.51 - 6.46 (m, 1H), 3.85 - 3.74 (m, 3H), 3.13 - 3.04 (m, 1H), 2.85 - 2.73 (m, 2H), 2.45 - 2.30 (m, 3H), 2.08 - 1.94 (m, 2H), 1.90 - 1.79 (m, 2H), 1.33 (s, 9H), 1.01 - 0.90 (m, 1H), 0.76 - 0.69 (m, 2H), 0.65 - 0.57 (m, 2H), 0.45 - 0.38 (m, 1H), 0.28 - 0.17 (m, 2H), 0.08 - 0.03 (m, 1H).

**Example 17:**

Synthetic Route:

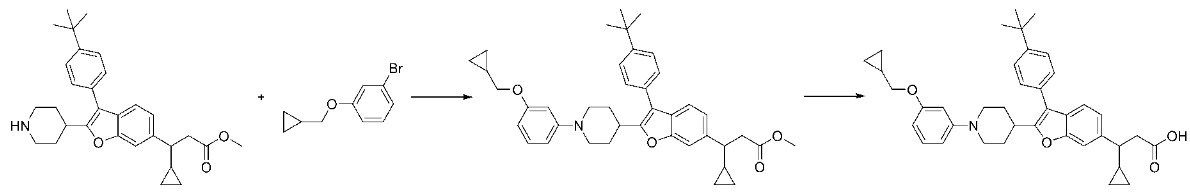

14-2 17-1 17-2 17

Referring to the synthetic route of compound **14,** compound **11-1** was replaced with compound **17-1** to carry out the synthesis. The resulting crude product was purified by reverse-phase column chromatography [acetonitrile/water (0.05% formic acid) = 0% to 100%] to obtain compound **17** (12 mg, yield: 38%) as a white solid. MS (ESI, m/z): 592.7 $[M+H]^+$.

$^1H$ NMR (400 MHz, DMSO-$d_6$) $\delta$ 7.53 (d, $J$ = 8.4 Hz, 2H), 7.41 (d, $J$ = 8.4 Hz, 2H), 7.37 (s, 1H), 7.34 (d, $J$ = 8.0 Hz, 1H), 7.12 - 7.02 (m, 2H), 6.51 (dd, $J$ = 8.4, 2.0 Hz, 1H), 6.44 (s, 1H), 6.30 (dd, $J$ = 8.0, 2.0 Hz, 1H), 3.83 - 3.71 (m, 4H), 3.12 - 3.03 (m, 1H), 2.81 - 2.72 (m, 2H), 2.45 - 2.31 (m, 3H), 2.09 - 1.92 (m, 2H), 1.87 - 1.78 (m, 2H), 1.32 (s, 9H), 1.21 - 1.12 (m, 1H), 1.01 - 0.89 (m, 1H), 0.57 - 0.49 (m, 2H), 0.45 - 0.36 (m, 1H), 0.31 - 0.19 (m, 4H), 0.07 - 0.00 (m, 1H).

**Example 18:**

Synthetic Route:

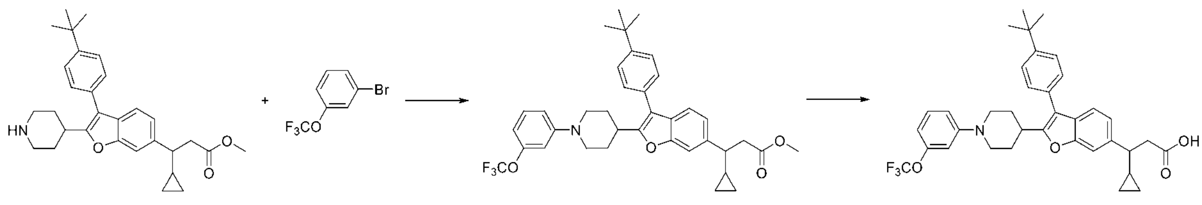

14-2 18-1 18-2 18

Referring to the synthetic route of compound **14,** compound **11-1** was replaced with compound **18-1** to carry out the synthesis. The resulting crude product was purified by reverse-phase column chromatography [acetonitrile/water (0.05% formic acid) = 0% to 100%] to obtain compound **18** (23 mg, yield: 46%) as a white solid. MS (ESI, m/z): 606.2 $[M+H]^+$.

$^1H$ NMR (400 MHz, $CDCl_3$) δ 7.52 - 7.47 (m, 3H), 7.41 -7.36 (m, 3H), 7.23 (d,*J* = 8.4 Hz, 1H), 7.13 - 7.09 (m, 1H), 6.86 (dd, *J* = 8.4, 2.4 Hz, 1H), 6.75 (s, 1H), 6.70 - 6.65 (m, 1H), 3.86 - 3.75 (m, 2H), 3.15 - 3.07 (m, 1H), 2.92 - 2.80 (m, 4H), 2.55 - 2.45 (m, 1H), 2.30 - 2.16 (m, 2H), 1.99 - 1.89 (m, 2H), 1.39 (s, 9H), 1.15 - 1.04 (m, 1H), 0.67 - 0.57 (m, 1H), 0.49 - 0.41 (m, 1H), 0.37 - 0.29 (m, 1H), 0.27 - 0.16 (m, 1H).

**Example 19:**

Synthetic Route:

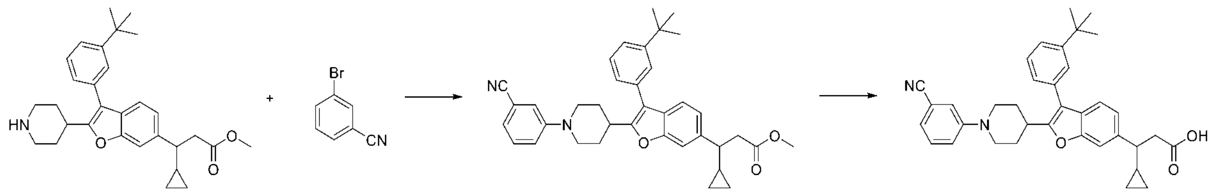

1-4 19-1 19-2 19

Referring to the synthetic route of compound **3,** compound **3-1** was replaced with compound **19-1** to carry out the synthesis. The resulting crude product was purified by reverse-phase column chromatography [acetonitrile/water (0.05% formic acid) = 0% to 100%] to obtain compound **19** (19 mg, yield: 39%) as a white solid. MS (ESI, m/z): 547.3 $[M+H]^+$.

$^1H$ NMR (400 MHz, $CDCl_3$) δ 7.50-7.28 (m, 9H), 7.17-7.11 (m, 2H), 3.96-3.94 (m, 2H), 3.18-3.10 (m, 1H), 2.89-2.86 (m, 2H), 2.50-2.35 (m, 3H), 2.03-1.99 (m, 2H), 1.91-1.88 (m,2H), 1.34 (s, 9H), 1.05-0.97 (m,1H), 0.51-0.40 (m, 1H), 0.30-0.21 (m, 2H), 0.13-0.05 (m, 1H).

**Example 20:**

Synthetic Route:

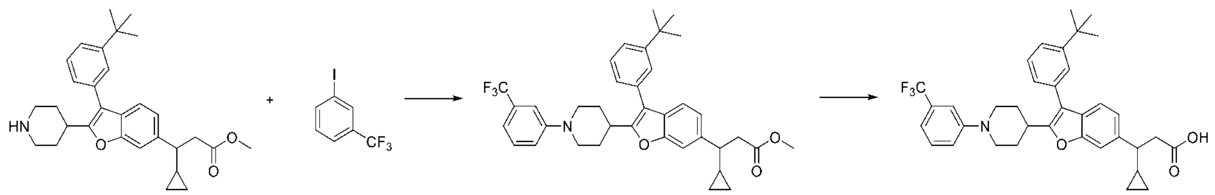

1-4 20-1 20-2 20

Referring to the synthetic route of compound **3,** compound **3-1** was replaced with compound **20-1** to carry out the synthesis. The resulting crude product was purified by reverse-phase column chromatography [acetonitrile/water (0.05% formic acid) = 0% to 100%] to obtain compound **20** (15 mg, yield: 39%) as a white solid. MS (ESI, m/z): 590.3 $[M+H]^+$.

$^1H$ NMR (400 MHz, DMSO-$d_6$) δ 7.50 - 7.36 (m, 6H), 7.35 - 7.31 (m, 1H), 7.29 - 7.24 (m, 1H), 7.23 - 7.22(m, 1H), 7.17 - 7.12 (m, 1H), 7.07 - 7.69 (m, 1H), 3.98 - 3.89 (m, 2H), 3.16 - 3.09 (m, 1H), 2.92 - 2.77 (m, 2H), 2.60 - 2.33 (m, 3H), 2.12 - 1.88 (m, 4H), 1.34 (s, 9H), 1.05 - 0.97 (m, 1H), 0.51 - 0.41 (m, 1H), 0.31 - 0.21 (m, 2H), 0.12 - 0.05 (m, 1H).

**Example 21:**

Synthetic Route:

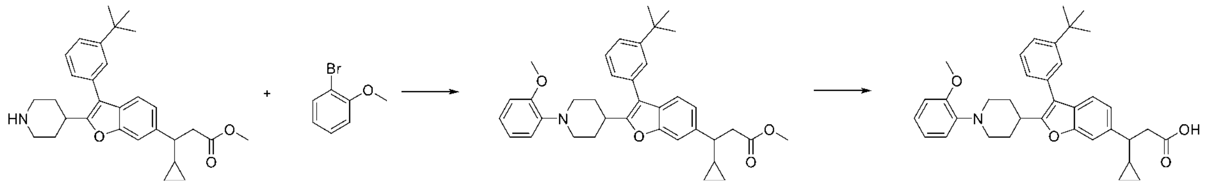

1-4 21-1 21-2 21

Referring to the synthetic route of compound **3,** compound **3-1** was replaced with compound **21-1** to carry out the synthesis. The resulting crude product was purified by reverse-phase column chromatography [acetonitrile/water (0.05% formic acid) = 0% to 100%] to obtain compound **71** (18 mg, yield: 32%) as a white solid. MS (ESI, m/z): 552.3 $[M+H]^+$.

$^1H$ NMR (400 MHz, DMSO-$d_6$) $\delta$ 7.50 - 7.41 (m, 4H), 7.38 - 7.34 (m, 1H), 7.33 - 7.31 (m, 1H), 7.16 (d, *J* = 8.4 Hz, 1H), 6.95 - 6.86 (m, 4H), 3.81 (s, 3H), 3.49 - 3.46 (m, 2H), 3.03 - 2.99 (m, 1H), 2.64 - 2.56 (m, 2H), 2.50 - 2.33 (m, 3H), 2.14 - 2.11(m, 2H), 1.91 - 1.88 (m, 2H), 1.35 (s, 9H), 1.05 - 0.95 (m, 1H), 0.44 - 0.42 (m, 1H), 0.32 - 0.21 (m, 2H), 0.09 - 0.08 (m, 1H).

**Example 22**:

Synthetic Route:

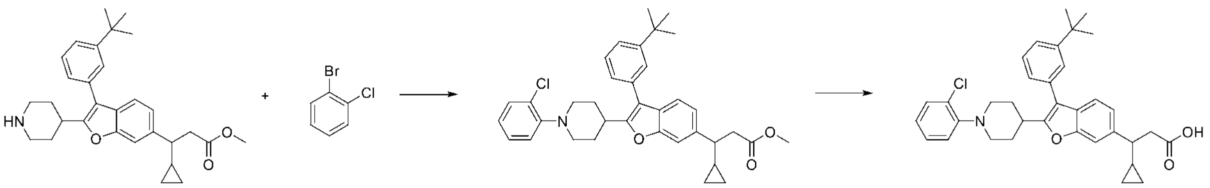

1-4 22-1 22-2 22

Referring to the synthetic route of compound **3**, compound **3-1** was replaced with compound **22-1** to carry out the synthesis. The resulting crude product was purified by reverse-phase column chromatography [acetonitrile/water (0.05% formic acid) = 0% to 100%] to obtain compound 22 (9 mg, yield: 59%) as a white solid. MS (ESI, m/z): 556.6 $[M+H]^+$.

$^1H$ NMR (400 MHz, DMSO-$d_6$) $\delta$ 7.52 - 7.37 (m, 6H), 7.35 - 7.26 (m, 2H), 7.21 - 7.14 (m, 2H), 7.07 - 7.00 (m, 1H), 3.42 - 3.30 (m, 2H), 3.11 - 3.00 (m, 1H), 2.80 - 2.69 (m, 2H), 2.57 - 2.38 (m, 3H), 2.24 - 2.09 (m, 2H), 2.01 - 1.89 (m, 2H), 1.35 (s, 9H), 1.10 - 0.97 (m, 1H), 0.53 - 0.44 (m, 1H), 0.34 - 0.23 (m, 2H), 0.16 - 0.07 (m, 1H).

**Example 23:**

Synthetic Route:

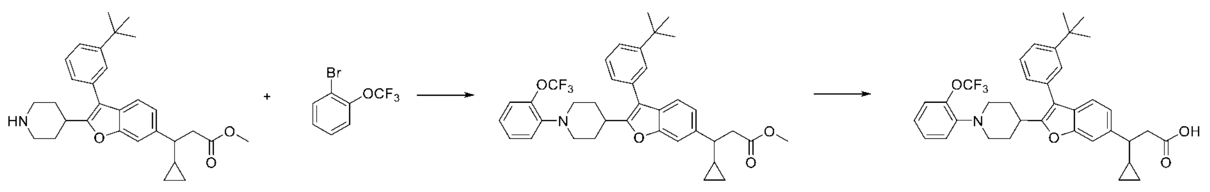

1-4 23-1 23-2 23

Referring to the synthetic route of compound **3**, compound **3-1** was replaced with compound **23-1** to carry out the synthesis. The resulting crude product was purified by reverse-phase column chromatography [acetonitrile/water (0.05% formic acid) = 0% to 100%] to obtain compound **23** (33 mg, yield: 55%) as a white solid. MS (ESI, m/z): 606.3 $[M+H]^+$.

$^1H$ NMR (400 MHz, MeOD) $\delta$ 7.51 (s, 1H), 7.47 - 7.41 (m, 3H), 7.40 - 7.37 (m, 1H), 7.31 - 7.13 (m, 5H), 7.05 - 7.00 (m, 1H), 3.52 - 3.46 (m, 2H), 3.14 - 3.04 (m, 1H), 2.79 - 2.65 (m, 4H), 2.55 - 2.45 (m, 1H), 2.34 - 2.20 (m, 2H), 1.99 - 1.89 (m, 2H), 1.39 (s, 9H), 1.18 - 1.04 (m, 1H), 0.64 - 0.55 (m, 1H), 0.45 - 0.33 (m, 2H), 0.21 - 0.12 (m, 1H).

**Example 24:**

Synthetic Route:

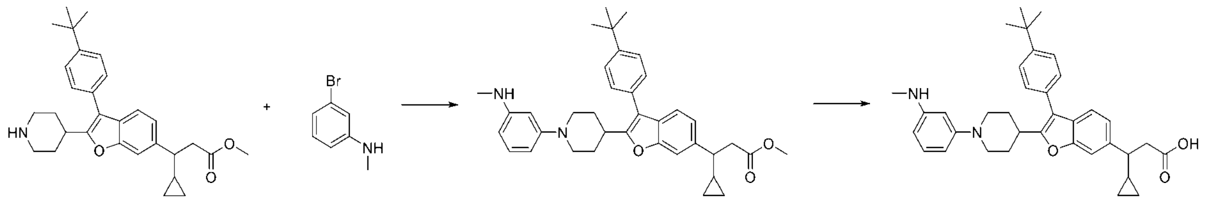

6-3 24-1 24-2 24

Referring to the synthetic route of compound **6**, compound **6-4** was replaced with compound **24-1** to carry out the synthesis. The resulting crude product was purified by reverse-phase column chromatography [acetonitrile/water (0.05% ammonia) = 0% to 100%] to obtain compound **24** (22 mg, yield: 41%) as a white solid. MS (ESI, m/z): 551.3 $[M+H]^+$.

$^1H$ NMR (400 MHz, MeOD) δ 7.54 (d, *J* = 8.4 Hz, 2H), 7.44 - 7.34 (m, 4H), 7.18 - 7.14 (m, 1H), 7.05 - 6.99 (m, 1H), 6.42 - 6.33 (m, 1H), 6.35 - 6.31 (m, 1H), 6.25 - 6.18 (m, 1H), 3.78 - 3.69 (m, 2H), 3.12 - 3.04 (m, 1H), 2.78 - 2.47 (m, 8H), 2.29 - 2.14 (m, 2H), 1.95 - 1.88 (m, 2H), 1.39 (s, 9H), 1.13 - 1.03 (m, 1H), 0.60 - 0.53 (m, 1H), 0.42 - 0.32 (m, 2H), 0.14 - 0.07 (m, 1H).

**Example 25:**

Synthetic Route:

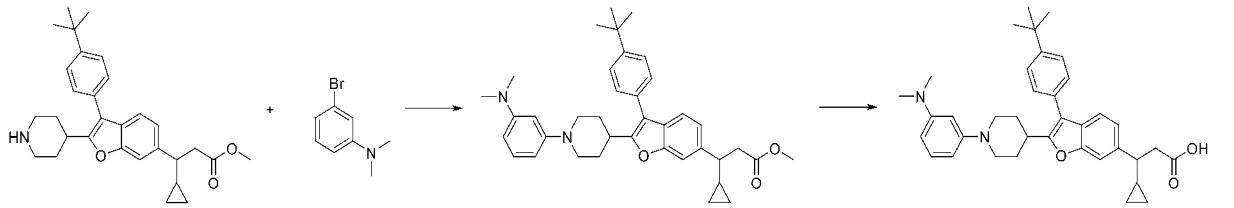

6-3 25-1 25-2 25

Referring to the synthetic route of compound **6**, compound **6-4** was replaced with compound **25-1** to carry out the synthesis. The resulting crude product was purified by reverse-phase column chromatography [acetonitrile/water (0.05% formic acid) = 0% to 100%] to obtain compound **25** (13 mg, yield: 61%) as a white solid. MS (ESI, m/z): 565.3 $[M+H]^+$.

$^1H$ NMR (400 MHz, DMSO-$d_6$) δ 7.60 - 7.51 (m, 2H), 7.47 - 7.42 (m, 2H), 7.40 (s, 1H), 7.37 - 7.32 (m, 1H), 7.12 (d, *J* = 8.0 Hz, 1H), 7.05 - 6.97 (m, 1H), 6.35 - 6.28 (m, 1H), 6.27 (s, 1H), 6.24 - 6.16 (m, 1H), 3.83 - 3.73 (m, 2H), 3.12 - 3.00 (m, 1H), 2.87 (s, 6H), 2.81 - 2.71 (m, 2H), 2.46 - 2.28 (m, 3H), 2.10 - 1.98 (m, 2H), 1.92 - 1.81 (m, 2H), 1.35 (s, 9H), 1.03 - 0.93 (m, 1H), 0.48 - 0.38 (m, 1H), 0.31 - 0.19 (m, 2H), 0.11 - 0.02 (m, 1H).

**Example 26:**

Synthetic Route:

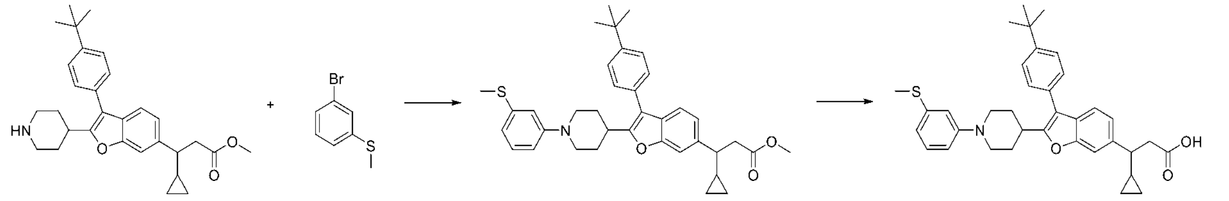

6-3 26-1 26-2 26

Referring to the synthetic route of compound **6**, compound **6-4** was replaced with compound **26-1** to carry out the synthesis. The resulting crude product was purified by reverse-phase column chromatography [acetonitrile/water (0.05% formic acid) = 0% to 100%] to obtain compound **26** (13 mg, yield: 61%) as a white solid. MS (ESI, m/z): 568.3 $[M+H]^+$.

$^1H$ NMR (400 MHz, $CDCl_3$) δ 7.55 - 7.43 (m, 3H), 7.44 - 7.34 (m, 3H), 7.22 - 7.17 (m, 1H), 7.10 (d, *J* = 8.0 Hz, 1H), 7.01 - 6.96 (m, 1H), 6.88 (s, 1H), 6.79 - 6.73 (m, 1H), 3.82 - 3.77 (m, 2H), 3.19 - 3.01 (m, 1H), 2.87 - 2.79 (m, 4H), 2.49 (s, 3H), 2.27 - 2.19 (m, 2H), 1.97 - 1.89 (m, 2H), 1.39 (s, 9H), 1.26 (s, 1H), 1.08 (s, 1H), 0.62 (s, 1H), 0.46 (s, 1H), 0.32 (s, 1H), 0.20 (s, 1H).

**Example 27:**

Synthetic Route:

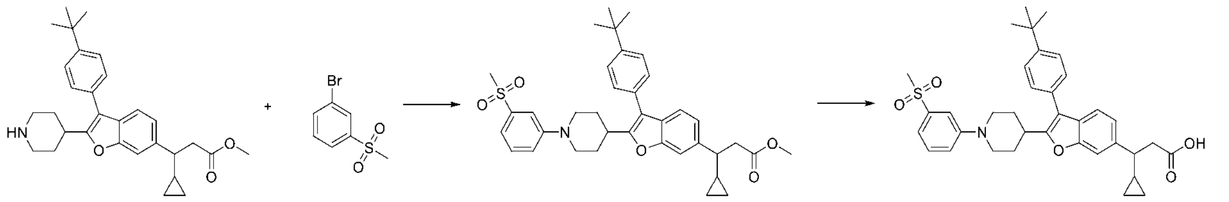

6-3 27-1 27-2 27

Referring to the synthetic route of compound **6**, compound **6-4** was replaced with compound **27-1** to carry out the synthesis. The resulting crude product was purified by reverse-phase column chromatography [acetonitrile/water (0.05% formic acid) = 0% to 100%] to obtain compound **27** (47 mg, yield: 47%) as a white solid. MS (ESI, m/z): 600.3 $[M+H]^+$.

$^1H$ NMR (400 MHz, $CDCl_3$) δ 7.53 - 7.45 (m, 4H), 7.43 - 7.34 (m, 5H), 7.20 (d, *J* = 8.0 Hz, 1H), 7.11 (d, *J* = 8.0 Hz, 1H), 3.92 - 3.87 (m, 2H), 3.19 - 3.15 (m, 1H), 3.05 (s, 3H), 2.97 - 2.91 (m, 2H), 2.97 - 2.91 (m, 2H), 2.52 - 2.46 (m, 1H), 2.26 - 2.21 (m, 2H), 2.00 - 1.95 (m, 2H), 1.39 (s, 9H), 1.09 (s, 1H), 0.61 (s, 1H), 0.44 (s, 1H), 0.36 - 0.29 (m, 1H), 0.23 - 0.15 (m, 1H).

**Example 28:**

Synthetic Route:

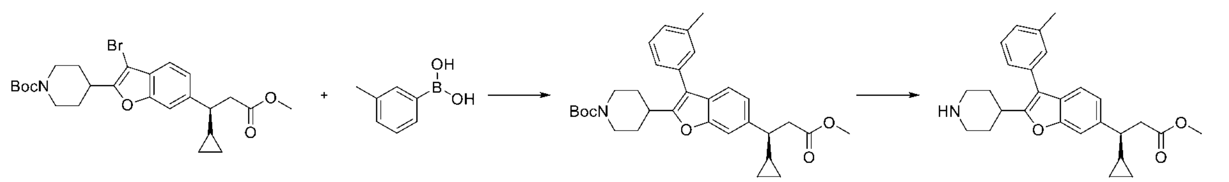

12-1 28-1 28-2 28-3

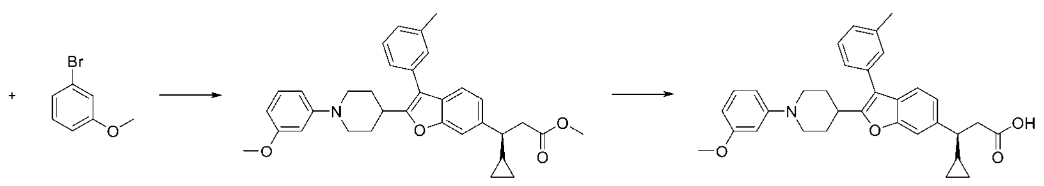

11-1 28-4 28

Referring to the synthetic route of compound **12**, compound **1-2** was replaced with compound **28-1** to carry out the synthesis. The resulting crude product was purified by reverse-phase column chromatography [acetonitrile/water (0.05% formic acid) = 0% to 100%] to obtain compound **28** (50 mg, yield: 71%) as a white solid. MS (ESI, m/z): 510.3 $[M+H]^+$.

$^1H$ NMR (400 MHz, $CDCl_3$) δ 7.47 (d, *J* = 8.0 Hz, 1H), 7.41 - 7.37 (m, 2H), 7.30 (s, 1H), 7.26 - 7.17 (m, 3H), 7.12 (d, *J* = 8.0 Hz, 1H), 6.65 - 6.58 (m, 1H), 6.53 (s, 1H), 6.46 - 6.42 (m, 1H), 3.83 - 3.79 (m, 5H), 3.14 - 3.04 (m, 1H), 2.89 - 2.78 (m, 4H), 2.54 - 2.47 (m, 1H), 2.45 (s, 3H), 2.28 - 2.17 (m, 2H), 1.95 - 1.89 (m, 2H), 1.16 - 1.06 (m, 1H), 0.69 - 0.57 (m, 1H), 0.50 - 0.41 (m, 1H), 0.39 - 0.31 (m, 1H), 0.25 - 0.16 (m, 1H).

**Example 29:**

Synthetic Route:

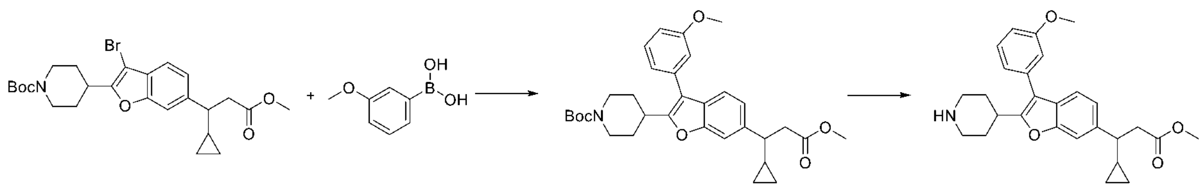

1-1 29-1 29-2 29-3

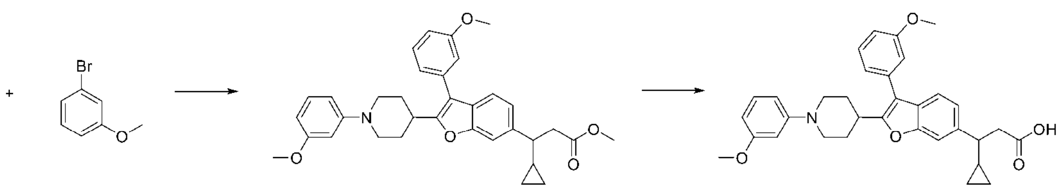

11-1 29-4 29

Referring to the synthetic route of compound **6**, compound **6-1** was replaced with compound **29-1** and compound **6-4** was replaced with compound **11-1** to carry out the synthesis. The resulting crude product was purified by reverse-phase column chromatography [acetonitrile/water (0.05% formic acid) = 0% to 100%] to obtain compound **29** (48.9 mg, yield: 13%) as a white solid. MS (ESI, m/z): 526.1 $[M+H]^+$.

$^1H$ NMR (400 MHz, MeOD) δ 7.44 - 7.36 (m, 3H), 7.18 - 7.10 (m, 2H), 7.05 (d, *J*= 7.6 Hz, 1H), 7.00 (s, 1H), 6.99 - 6.94 (m, 1H), 6.63 - 6.58 (m, 1H), 6.56 - 6.53 (m, 1H), 6.45 - 6.40 (m, 1H), 3.85 (s, 3H), 3.82 - 3.72 (m, 5H), 3.18 - 3.06 (m, 1H), 2.83 - 2.69 (m, 4H), 2.52 - 2.43 (m, 1H), 2.28 - 2.13 (m, 2H), 1.98-1.88 (m, 2H), 1.17 - 1.05 (m, 1H), 0.64 - 0.55 (m, 1H), 0.45 - 0.30 (m, 2H), 0.20 - 0.13 (m, 1H).

**Example 30:**

Synthetic Route:

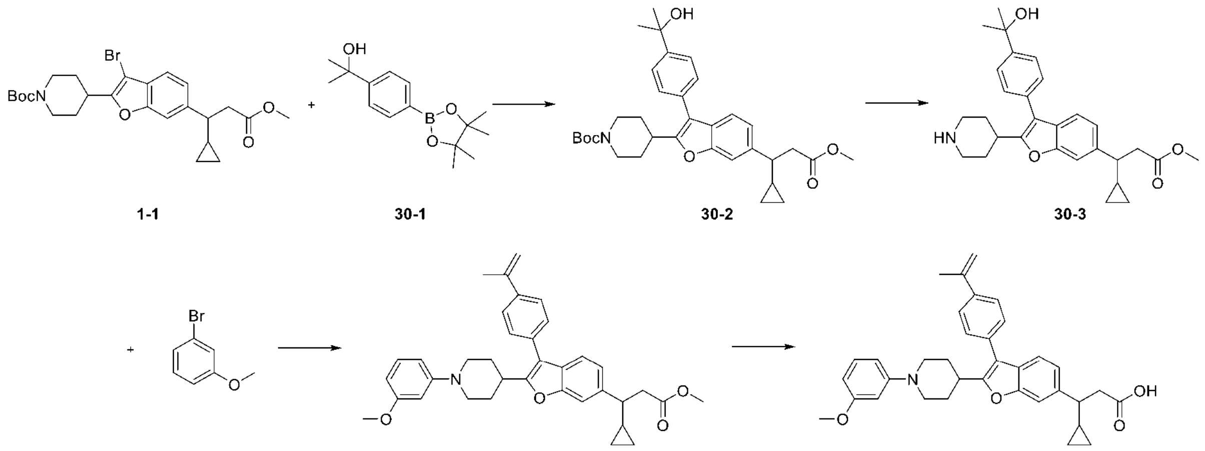

1-1 30-1 30-2 30-3

11-1 30-4 30

Referring to the synthetic route of compound **6**, compound **6-1** was replaced with compound **30-1** to carry out the synthesis. The resulting crude product was purified by reverse-phase column chromatography [acetonitrile/water (0.05% formic acid) = 0% to 100%] to obtain compound **30** (15 mg, yield: 19%) as a white solid. MS (ESI, m/z): 536.2 [M+H]$^+$.

$^1$H NMR (400 MHz, MeOD) $\delta$ 7.63 (d, $J$ = 8.4 Hz, 2H), 7.45 (d, $J$ = 8.4 Hz, 2H), 7.41-7.41 (m, 2H), 7.20 - 7.09 (m, 2H), 6.63 - 6.60 (m, 1H), 6.56 - 6.54 (m, 1H), 6.44 - 6.41 (m, 1H), 5.46 (s, 1H), 5.13 - 5.11 (m, 1H), 3.82 - 3.71 (s, 5H), 3.14 - 3.11 (m, 1H), 2.84 - 2.47 (m, 5H), 2.30 - 2.13 (m, 5H),1.95 - 1.92 (m, 2H), 1.09 - 1.06 (m, 1H), 0.58 - 0.54 (m, 1H), 0.39 - 0.35 (m, 2H), 0.15 - 0.12 (m, 1H).

**Example 31:**

Synthetic Route:

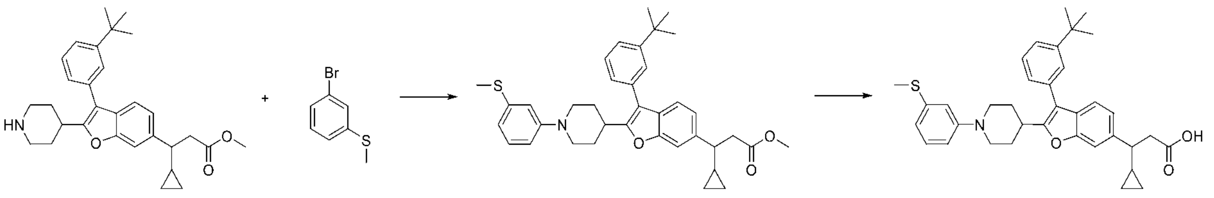

1-4 26-1 31-1 31

Referring to the synthetic route of compound **26**, compound **6-3** was replaced with compound **1-4** to carry out the synthesis. The resulting crude product was purified by reverse-phase column chromatography [acetonitrile/water (0.05% formic acid) = 0% to 100%] to obtain compound **31** (11 mg, yield: 47%) as a white solid. MS (ESI, m/z): 568.3 [M+H]$^+$.

$^1$H NMR (400 MHz, $CDCl_3$) $\delta$ 7.50 (s, 1H), 7.47 - 7.40 (m, 3H), 7.39 - 7.36 (m, 1H), 7.35 - 7.31 (m, 1H), 7.15 (d, $J$ = 8.0 Hz, 1H), 6.96 - 6.90 (m, 3H), 6.89 - 6.86 (m, 1H), 3.51 - 3.47 (m, 2H), 3.02 (s, 1H), 2.71 - 2.59 (m, 2H), 2.57 (s, 1H), 2.50 (s, 3H), 2.33 (s, 2H), 2.18 - 2.12 (m, 2H), 1.95 - 1.88 (m, 2H), 1.35 (s, 9H), 0.99 (s, 1H), 0.45 - 0.40 (m, 1H), 0.31 - 0.22 (m, 2H), 0.12 - 0.06 (m, 1H).

Example **32**:

Synthetic Route:

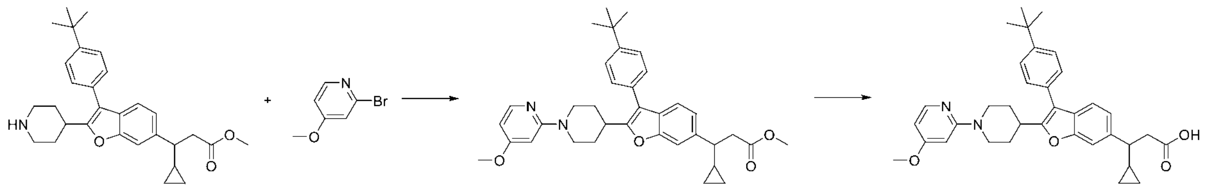

6-3 32-1 32-2 32

Referring to the synthetic route of compound **6**, compound **6-4** was replaced with compound **32-1** to carry out the synthesis. The resulting crude product was purified by reverse-phase column chromatography [acetonitrile/water (0.05% formic acid) = 0% to 100%] to obtain compound **32** (20 mg, yield: 42%) as a white solid. MS (ESI, m/z): 553.3 [M+H]$^+$.

$^1$H NMR (400 MHz, MeOD) δ 7.94 - 7.88 (m, 1H), 7.58 - 7.51 (m, 2H), 7.45 - 7.32 (m, 4H), 7.20 - 7.14 (m, 1H), 6.35 - 6.29 (m, 2H), 4.38 - 4.26 (m, 2H), 3.83 (s, 3H), 3.30 - 3.14 (m, 1H), 2.99 - 2.86 (m, 2H), 2.72 - 2.45 (m, 3H), 2.15 - 2.01 (m, 2H), 1.95 - 1.84 (m, 2H), 1.39 (s, 9H), 1.11 - 1.00 (m, 1H), 0.61 - 0.50 (m, 1H), 0.43 - 0.31 (m, 2H), 0.16 - 0.07 (m, 1H).

**Example 33:**

Synthetic Route:

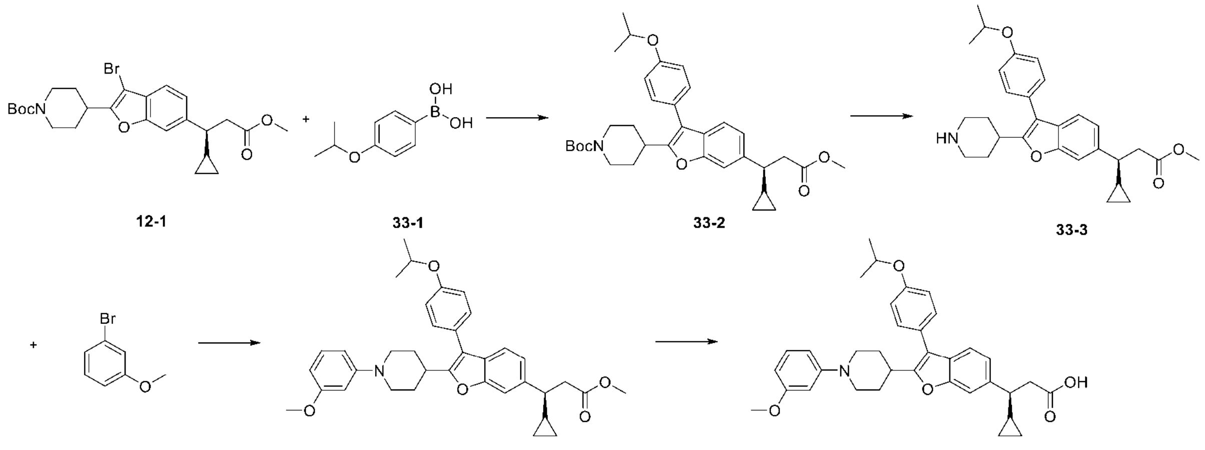

12-1 33-1 33-2 33-3

11-1 33-4 33

Referring to the synthetic route of compound **12**, compound **1-2** was replaced with compound **33-1** to carry out the synthesis. The resulting crude product was purified by reverse-phase column chromatography [acetonitrile/water (0.05% formic acid) = 0% to 100%] to obtain compound **33** (49 mg, yield: 79%) as a white solid. MS (ESI, m/z): 554.2 [M+H]$^+$.

$^1$H NMR (400 MHz, $CDCl_3$) δ 7.48 - 7.42 (m, 1H), 7.38 - 7.33 (m, 3H), 7.28 - 7.25 (m, 1H), 7.23 - 7.15 (m, 1H), 7.13 - 7.07 (m, 1H), 7.04 - 6.96 (m, 2H), 6.64 - 6.40 (m, 2H), 4.69 - 4.55 (m, 1H), 3.85 - 3.75 (m, 5H), 3.13 - 3.01 (m, 1H), 2.98 - 2.78 (m, 4H), 2.52 - 2.47 (m, 1H), 2.35 - 2.15 (m, 2H), 1.95 - 1.85 (m, 2H), 1.45 - 1.35 (m, 6H), 1.15 - 1.05 (m, 1H), 0.66 - 0.55 (m, 1H), 0.48 - 0.40 (m, 1H), 0.38 - 0.29 (m, 1H), 0.25 - 0.15 (m, 1H).

**Example 34:**

Synthetic Route:

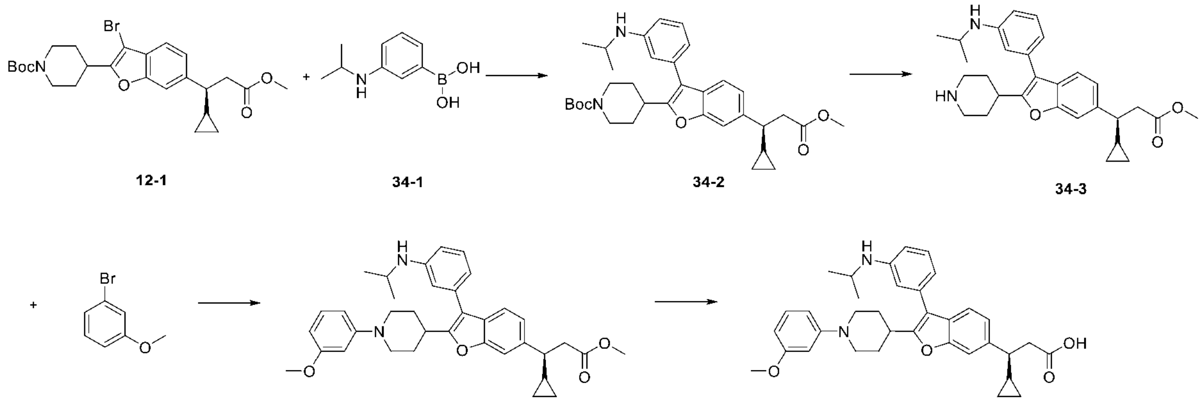

12-1 34-1 34-2 34-3

11-1 34-4 34

Referring to the synthetic route of compound **12**, compound **1-2** was replaced with compound **34-1** to carry out the synthesis. The resulting crude product was purified by reverse-phase column chromatography [acetonitrile/water (0.05% formic acid) = 0% to 100%] to obtain compound **34** (27 mg, yield: 39%) as a white solid. MS (ESI, m/z): 553.2 $[M+H]^+$.

$^1$H NMR (400 MHz, $CDCl_3$) δ 7.51 - 7.44 (m, 1H), 7.37 - 7.32 (m, 1H), 7.31 - 7.27 (m, 1H), 7.22 - 7.15 (m, 1H), 7.13 - 7.05 (m, 1H), 6.90 - 6.36 (m, 6H), 3.86 - 3.74 (m, 4H), 3.70 - 3.60 (m, 1H), 3.21 - 3.05 (m, 1H), 2.92 - 2.76 (m, 3H), 2.54 - 2.42 (m, 1H), 2.35 - 2.16 (m, 2H), 1.96 - 1.83 (m, 2H), 1.36 - 1.20 (m, 7H), 1.13 - 1.02 (m, 1H), 0.96 - 0.80 (m, 1H), 0.68 - 0.56 (m, 1H), 0.48 - 0.40 (m, 1H), 0.38 - 0.28 (m, 1H), 0.24 - 0.14 (m, 1H).

**Example 35**:

Synthetic Route:

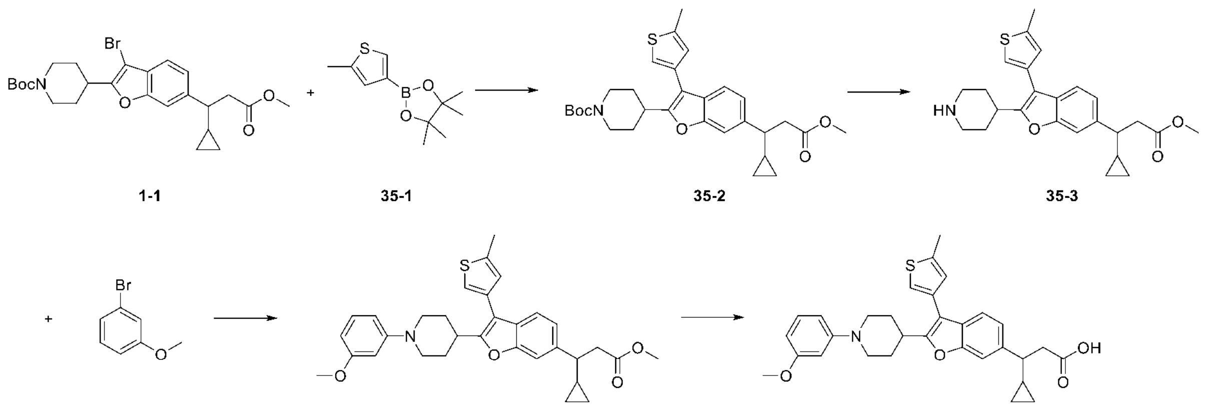

1-1 35-1 35-2 35-3

11-1 35-4 35

Referring to the synthetic route of compound **6**, compound **6-1** was replaced with compound **35-1** to carry out the synthesis. The resulting crude product was purified by reverse-phase column chromatography [acetonitrile/water (0.05% formic acid) = 0% to 100%] to obtain compound **35** (2 mg, yield: 12%) as a white solid. MS (ESI, m/z): 516.2 $[M+H]^+$.

$^1$H NMR (400 MHz, MeOD) δ 7.46 (d, *J* = 8.0 Hz, 1H), 7.35 (s, 1H), 7.20 - 7.19 (m, 1H), 7.17 - 7.11 (m, 2H), 6.98 (s, 1H), 6.65 - 6.60 (m, 1H), 6.57 - 6.54 (m, 1H), 6.46 - 6.41 (m, 1H), 3.83 - 3.74 (m, 5H), 3.22 - 3.13 (m, 1H), 2.87 - 2.69 (m, 4H), 2.56 (s, 3H), 2.50 - 2.41 (m, 1H), 2.25 - 2.12 (m, 2H), 1.98 - 1.90 (m, 2H), 1.17 - 1.05 (m, 1H), 0.64 - 0.55 (m, 1H), 0.44 - 0.27 (m, 2H), 0.20 - 0.13 (m, 1H).

**Example 36:**

Synthetic Route:

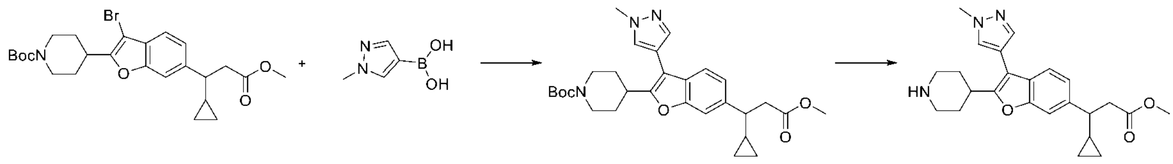

1-1 36-1 36-2 36-3

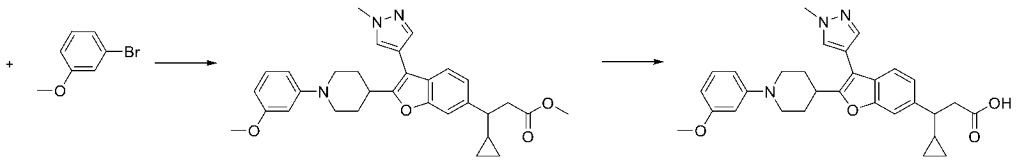

11-1 36-4 36

Referring to the synthetic route of compound 6, compound 6-1 was replaced with compound 36-1 to carry out the synthesis. The resulting crude product was purified by reverse-phase column chromatography [acetonitrile/water (0.05% formic acid) = 0% to 100%] to obtain compound 36 (8 mg, yield: 14%) as a white solid. MS (ESI, m/z): 500.2 $[M+H]^+$.

$^1$H NMR (400 MHz, DMSO-$d_6$) δ 8.07 (s, 1H), 7.73 (s, 1H), 7.52 - 7.44 (m, 1H), 7.38 (s, 1H), 7.15 - 7.09 (m, 2H), 6.61 - 6.54 (m, 1H), 6.51 - 6.47 (m, 1H), 6.38 - 6.32 (m, 1H), 3.93 (s, 3H), 3.86 - 3.78 (m, 2H), 3.73 (s, 3H), 3.23 - 3.13 (m, 1H), 2.93 - 2.80 (m, 2H), 2.47 - 2.31 (m, 3H), 2.05 - 1.80 (m, 4H), 1.02 - 0.94 (m, 1H), 0.47 - 0.40 (m, 1H), 0.29 - 0.19 (m, 2H), 0.10 - 0.03 (m, 1H).

**Example 37:**

Synthetic Route:

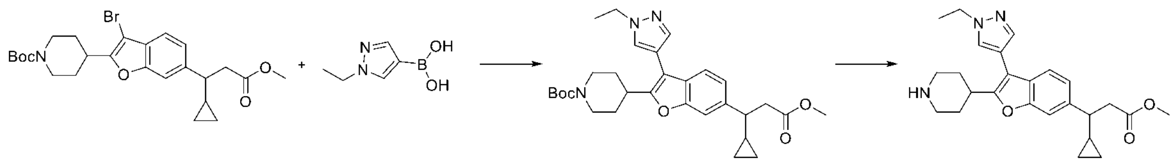

1-1 37-1 37-2 37-3

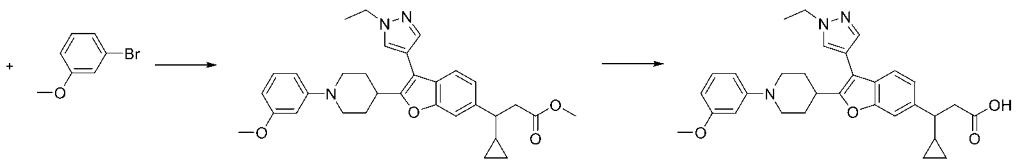

11-1 37-4 37

Referring to the synthetic route of compound **6**, compound **6-1** was replaced with compound **37-1** to carry out the synthesis. The resulting crude product was purified by reverse-phase column chromatography [acetonitrile/water (0.05% formic acid) = 0% to 100%] to obtain compound **37** (7 mg, yield: 16%) as a white solid. MS (ESI, m/z): 514.2 $[M+H]^+$.

$^1$H NMR (400 MHz, MeOD) δ 7.93 (s, 1H), 7.71 (s, 1H), 7.48 (d, *J* = 8.0 Hz, 1H), 7.35 (s, 1H), 7.18 - 7.11 (m, 2H), 6.65 - 6.60 (m, 1H), 6.58 - 6.54 (m, 1H), 6.48 - 6.39 (m, 1H), 4.35 - 4.25 (m, 2H), 3.84 - 3.74 (m, 5H), 3.19 - 3.10 (m, 1H), 2.90 - 2.69 (m, 4H), 2.50 - 2.40 (m, 1H), 2.26 - 2.11 (m, 2H), 1.99 - 1.89 (m, 2H), 1.56 - 1.37 (m, 3H), 1.16 - 1.07 (m, 1H), 0.65 - 0.56 (m, 1H), 0.45 - 0.28 (m, 2H), 0.22 - 0.12 (m, 1H).

**Example 38:**

Synthetic Route:

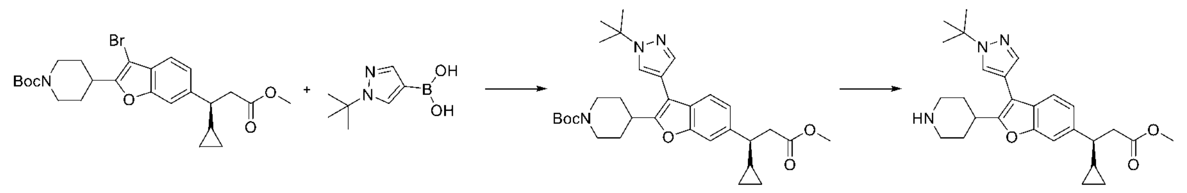

12-1 38-1 38-2 38-3

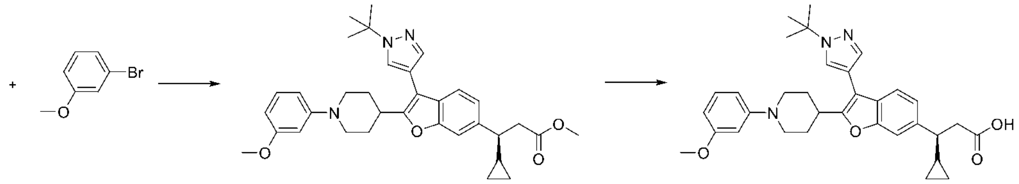

11-1 38-4 38

Referring to the synthetic route of compound **12**, compound **1-2** was replaced with compound **38-1** to carry out the synthesis. The resulting crude product was purified by reverse-phase column chromatography [acetonitrile/water (0.05% formic acid) = 0% to 100%] to obtain compound **38** (13 mg, yield: 33%) as a white solid. MS (ESI, m/z): 542.2 $[M+H]^+$.

$^1H$ NMR (400 MHz, $CDCl_3$) δ 7.70 (s, 2H), 7.48 (d, *J* = 8.0 Hz, 1H), 7.36 (s, 1H), 7.24 - 7.10 (m, 2H), 6.67 - 6.58 (m, 1H), 6.57 - 6.50 (m, 1H), 6.49 - 6.39 (m, 1H), 3.87 - 3.78 (m, 5H), 3.15 - 3.05 (m, 1H), 2.94 - 2.78 (m, 4H), 2.55 - 2.44 (m, 1H), 2.31 - 2.15 (m, 2H), 2.00 - 1.88 (m, 2H), 1.68 (s, 9H), 1.16 - 1.03 (m, 1H), 0.68 - 0.56 (m, 1H), 0.51 - 0.39 (m, 1H), 0.38 - 0.29 (m, 1H), 0.26 - 0.15 (m, 1H).

**Example 39:**

Synthetic Route:

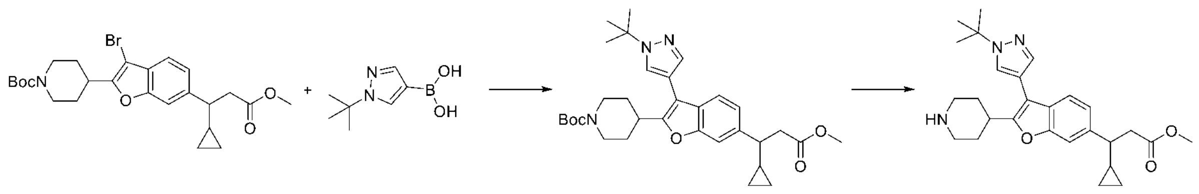

1-1 38-1 39-2 39-3

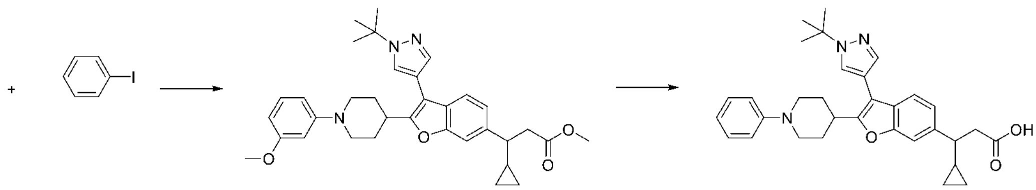

39-4 39-5 39

Referring to the synthetic route of compound **6**, compound **6-1** was replaced with compound **38-1** and compound **6-4** was replaced with compound **39-4** to carry out the synthesis. The resulting crude product was purified by reverse-phase column chromatography [acetonitrile/water (0.05% formic acid) = 0% to 100%] to obtain compound **39** (17 mg, yield: 40%) as a white solid. MS (ESI, m/z): 512.2 $[M+H]^+$.

$^1H$ NMR (400 MHz, $CDCl_3$) δ 7.72 - 7.68 (m, 2H), 7.48 (d, *J* = 8.0 Hz, 1H), 7.36 (s, 1H), 7.35 - 7.29 (m, 2H), 7.29 - 7.28 (m, 1H), 7.14 (d, *J* = 8.0 Hz, 1H), 7.05 - 7.00 (m, 1H), 6.96 - 6.85 (m, 1H), 3.89 - 3.79 (m, 2H), 3.19 - 3.02 (m, 1H), 2.93 - 2.82 (m, 4H), 2.56 - 2.46 (m, 1H), 2.37 - 2.17 (m, 2H), 1.98 - 1.90 (m, 2H), 1.68 (s, 9H), 1.16 - 1.05 (m, 1H), 0.67 - 0.58 (m, 1H), 0.49 - 0.42 (m, 1H), 0.39 - 0.31 (m, 1H), 0.26 - 0.15 (m, 1H).

**Example 40:**

Synthetic Route:

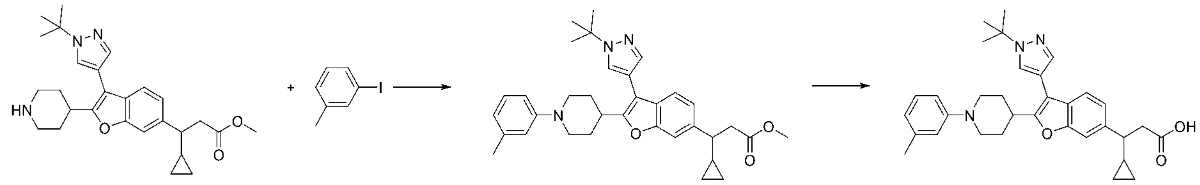

39-3 4-1 40-2 40

Referring to the synthetic route of compound **39**, compound **39-4** was replaced with compound **4-1** to carry out the synthesis. The resulting crude product was purified by reverse-phase column chromatography [acetonitrile/water (0.05% formic acid) = 0% to 100%] to obtain compound **40** (30 mg, yield: 61%) as a white solid. MS (ESI, m/z): 526.3 $[M+H]^+$.

$^1H$ NMR (400 MHz, $CDCl_3$) δ 7.72 - 7.69 (m, 2H), 7.48 (d, *J* = 8.0 Hz, 1H), 7.36 (s, 1H), 7.22 - 7.12 (m, 2H), 6.87 - 6.79 (m, 2H), 6.74 - 6.69 (m, 1H), 3.85 - 3.79 (m, 2H), 3.15 - 3.05 (m, 1H), 2.92 - 2.81 (m, 4H), 2.54 - 2.47 (m, 1H), 2.35 (s, 3H), 2.29 - 2.20 (m, 2H), 1.98 - 1.92 (m, 2H), 1.68 (s, 9H), 1.13 - 1.06 (m, 1H), 0.65 - 0.58 (m, 1H), 0.46 - 0.41 (m, 1H), 0.37 - 0.31 (m, 1H), 0.24 - 0.17 (m, 1H).

**Example 41:**

Synthetic Route:

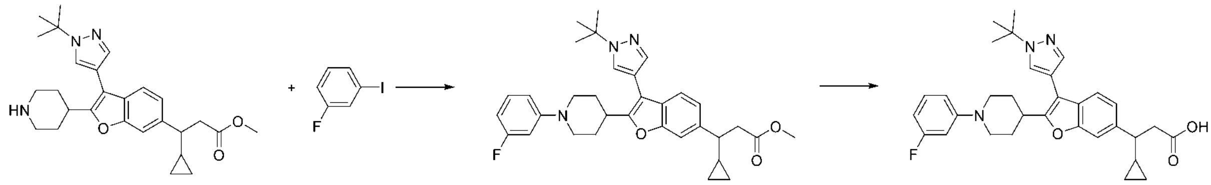

39-3 41-1 41-2 41

Referring to the synthetic route of compound **39**, compound **39-4** was replaced with compound **41-1** to carry out the synthesis. The resulting crude product was purified by reverse-phase column chromatography [acetonitrile/water (0.05% formic acid) = 50% to 70%] to obtain compound **41** (25 mg, yield: 71%) as a white solid. MS (ESI, m/z): 530.3 $[M+H]^+$.

$^1H$ NMR (400 MHz, $CDCl_3$) δ 7.72 - 7.68 (m, 2H), 7.48 (d, *J* = 8.0 Hz, 1H), 7.36 (s, 1H), 7.26 - 7.17 (m, 1H), 7.14 (d, *J* = 8.0 Hz, 1H), 6.79 - 6.72 (m, 1H), 6.71 - 6.63 (m, 1H), 6.59 - 6.51 (m, 1H), 3.90 - 3.78 (m, 2H), 3.20 - 3.09 (m, 1H), 2.95 - 2.80 (m, 4H), 2.54 - 2.44 (m, 1H), 2.28 - 2.15 (m, 2H), 2.00 - 1.91 (m, 2H), 1.68 (s, 9H), 1.19 - 1.04 (m, 1H), 0.67 - 0.58 (m, 1H), 0.49 - 0.40 (m, 1H), 0.38 - 0.30 (m, 1H), 0.24 - 0.15 (m, 1H).

**Example 42:**

Synthetic Route:

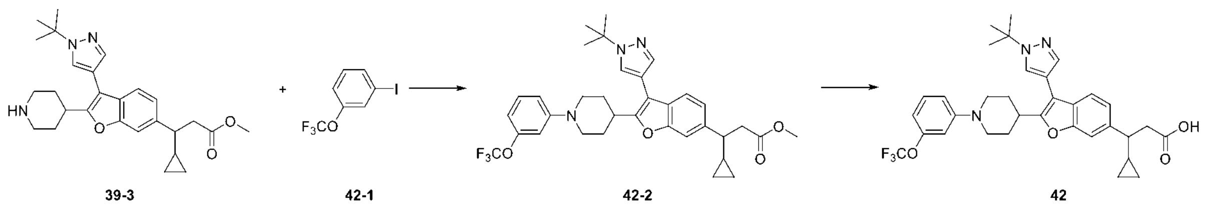

39-3 42-1 42-2 42

Referring to the synthetic route of compound **39**, compound **39-4** was replaced with compound **42-1** to carry out

the synthesis. The resulting crude product was purified by reverse-phase column chromatography [acetonitrile/water (0.05% formic acid) = 50% to 70%] to obtain compound **42** (36 mg, yield: 72%) as a white solid. MS (ESI, m/z): 596.3 $[M+H]^+$.

$^1$H NMR (400 MHz, $CDCl_3$) δ 7.72 - 7.67 (m, 2H), 7.48 (d, *J* = 8.0 Hz, 1H), 7.36 (s, 1H), 7.31 - 7.28 (m, 1H), 7.14 (d, *J* = 8.0 Hz, 1H), 6.99 - 6.88 (m, 1H), 6.87 - 6.78 (m, 1H), 6.78 - 6.68 (m, 1H), 3.90 - 3.79 (m, 2H), 3.20 - 3.08 (m, 1H), 2.99 - 2.84 (m, 4H), 2.55 - 2.46 (m, 1H), 2.33 - 2.16 (m, 2H), 2.02 - 1.93 (m, 2H), 1.68 (s, 9H), 1.15 - 1.05 (m, 1H), 0.70 - 0.60 (m, 1H), 0.49 - 0.40 (m, 1H), 0.37 - 0.30 (m, 1H), 0.23 - 0.17 (m, 1H).

**Example 43:**

Synthetic Route:

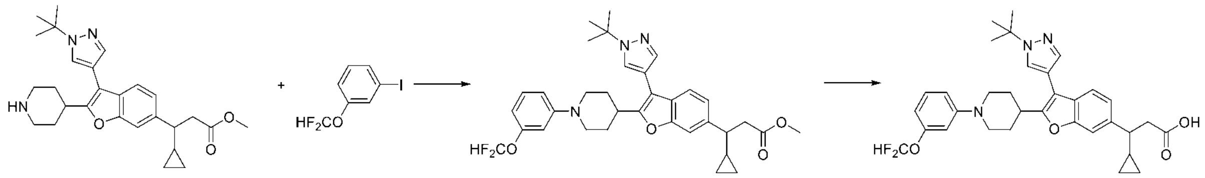

39-3 43-1 43-2 43

Referring to the synthetic route of compound **39**, compound **39-4** was replaced with compound **43-1** to carry out the synthesis. The resulting crude product was purified by reverse-phase column chromatography [acetonitrile/water (0.05% formic acid) = 50% to 70%] to obtain compound **4**3 (9.6 mg, yield: 47%) as a white solid. MS (ESI, m/z): 578.3 $[M+H]^+$.

$^1$H NMR (400 MHz, DMSO-$d_6$) δ 8.10 (s, 1H), 7.75 (s, 1H), 7.54 (d, *J* = 8.0 Hz, 1H), 7.41 (s, 1H), .27 - 7.21 (m, 1H), 7.23 (t, *J* = 74.6 Hz, 1H), 7.14 (d, *J* = 8.0 Hz, 1H), 6.88 - 6.82 (m, 1H), 6.75 - 6.71 (m, 1H), 6.56 - 6.51 (m, 1H), 3.94 - 3.82 (m, 2H), 3.25 - 3.16 (m, 1H), 2.99 - 2.88 (m, 2H), 2.64 - 2.50 (m, 2H), 2.44 - 2.37 (m, 1H), 2.07 - 1.77 (m, 4H), 1.60 (s, 9H), 1.07 - 0.95 (m, 1H), 0.52 - 0.41 (m, 1H), 0.33 - 0.19 (m, 2H), 0.12 - 0.08 (m, 1H).

**Example 44:**

Synthetic Route:

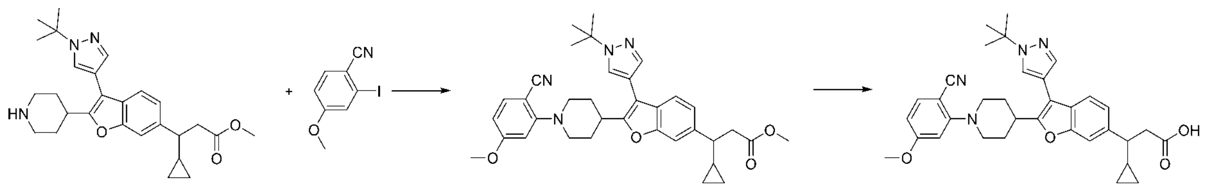

39-3 44-1 44-2 44

Referring to the synthetic route of compound **39**, compound **39-4** was replaced with compound **44-1** to carry out the synthesis. The resulting crude product was purified by reverse-phase column chromatography [acetonitrile/water (0.05% formic acid) = 50% to 70%] to obtain compound **44** (15 mg, yield: 47%) as a white solid. MS (ESI, m/z): 567.3 $[M+H]^+$.

$^1$H NMR (400 MHz, $CDCl_3$) δ 7.73 - 7.69 (m, 2H), 7.55 - 7.47 (m, 2H), 7.38 (s, 1H), 7.15 (d, *J* = 8.0 Hz, 1H), 6.57 - 6.51 (m, 2H), 3.86 (s, 3H), 3.79 - 3.72 (m, 2H), 3.17 - 3.08 (m, 1H), 2.95 - 2.86 (m, 4H), 2.57 - 2.49 (m, 1H), 2.42 - 2.31 (m, 2H), 2.01 - 1.96 (m, 2H), 1.69 (s, 9H), 1.15 - 1.09 (m, 1H), 0.70 - 0.60 (m, 1H), 0.50 - 0.43 (m, 1H), 0.39 - 0.32 (m, 1H), 0.27 - 0.19 (m, 1H).

**Example 45:**

Synthetic Route:

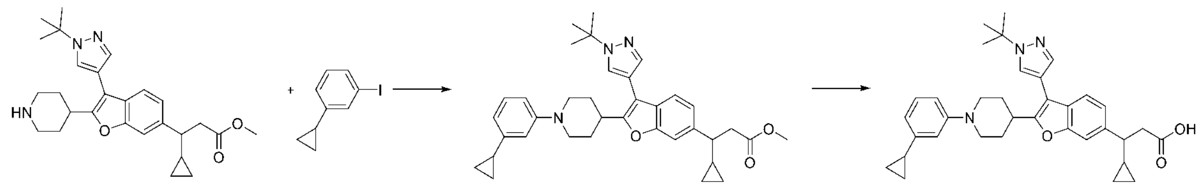

39-3 45-1 45-2 45

Referring to the synthetic route of compound **39**, compound **39-4** was replaced with compound **45-1** to carry out the synthesis. The resulting crude product was purified by reverse-phase column chromatography [acetonitrile/water (0.05% ammonia) = 0% to 100%] to obtain compound **40** (6 mg, yield: 30%) as a white solid. MS (ESI, m/z): 552.3 [M+H]$^+$.

$^1$H NMR (400 MHz, DMSO-*d*$_6$) *δ* 8.12 (s, 1H), 7.77 (s, 1H), 7.56 (d, *J* = 8.0 Hz, 1H), 7.44 (s, 1H), 7.18 (d, *J* = 8.0 Hz, 1H), 7.15 -7.08 (m, 1H), 6.85 - 6.75 (m, 1H), 6.74 (s, 1H), 6.53 - 6.45 (m, 1H), 3.90 - 3.80 (m, 2H), 3.24 - 3.15 (m, 1H), 2.93 - 2.82 (m, 2H), 2.64 - 2.56 (m, 2H), 2.49 - 2.40 (m, 1H), 2.08 - 1.98 (m, 2H), 1.96 - 1.85 (m, 3H), 1.63 (s, 9H), 1.10 - 1.00 (m, 1H), 0.96 - 0.90 (m, 2H), 0.72 - 0.65 (m, 2H), 0.55 - 0.45 (m, 1H), 0.33 - 0.26 (m, 2H), 0.18 - 0.08 (m, 1H).

**Example 46:**

Synthetic Route:

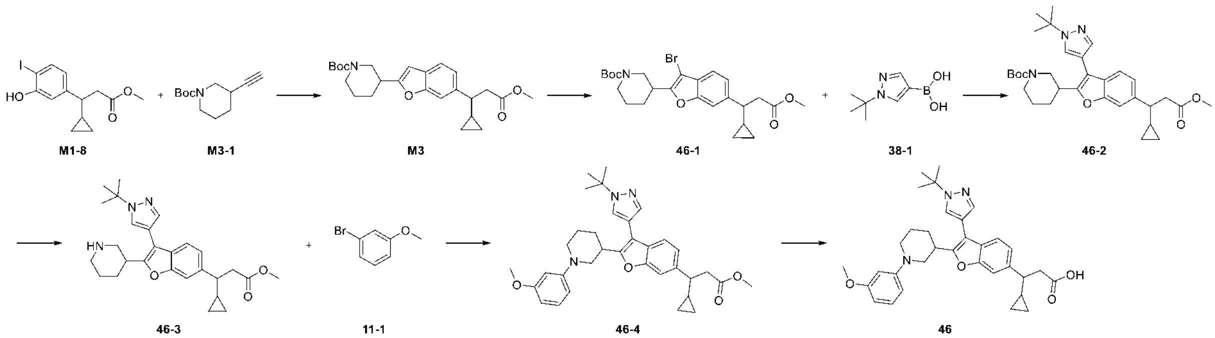

M1-8 M3-1 M3 46-1 38-1 46-2

46-3 11-1 46-4 46

The synthesis route of intermediate **M1** was referred to obtain intermediate M3. Then, referring to the synthetic route of compound **38**, compound **12-1** was replaced with compound 46-1 to carry out the synthesis. The resulting crude product was purified by reverse-phase column chromatography [acetonitrile/water (0.05% formic acid) = 0% to 100%] to obtain compound 46 (60 mg, yield: 42%) as a white solid. MS (ESI, m/z): 542.3 [M+H]$^+$.

$^1$H NMR (400 MHz, $CDCl_3$) δ 7.79 - 7.61 (m, 2H), 7.55 - 7.45 (m, 1H), 7.36 (s, 1H), 7.17 - 7.09 (m, 2H), 6.61 - 6.34 (m, 3H), 3.80 - 3.75 (m, 5H), 3.41 - 3.29 (m, 1H), 3.21 - 3.11 (m, 1H), 2.91 - 2.81 (m, 3H), 2.54 - 2.48 (m, 1H), 2.07 - 1.94 (m, 2H), 1.89 - 1.77 (m, 2H), 1.66 (s, 9H), 1.15 - 1.06 (m, 1H), 0.65 - 0.58 (m, 1H), 0.48 - 0.42 (m, 1H), 0.36 - 0.30 (m, 1H), 0.25 - 0.16 (m, 1H).

**Example 47:**

Synthetic Route:

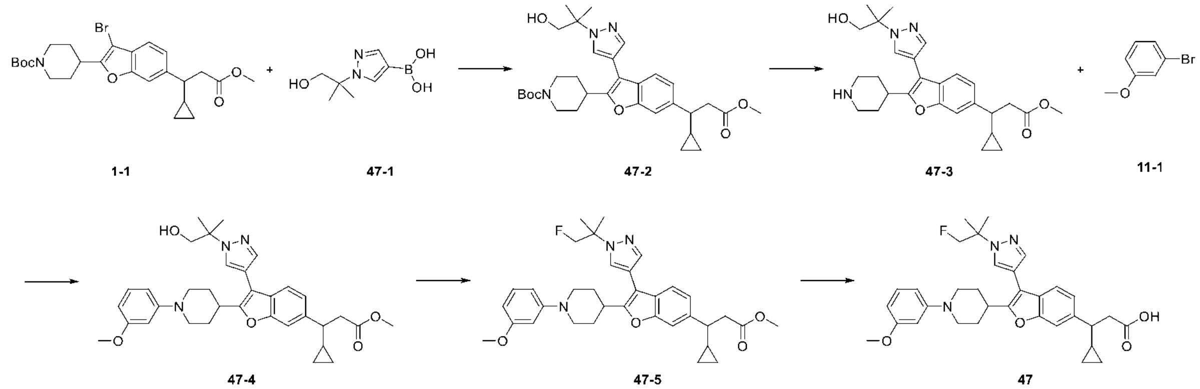

1-1 47-1 47-2 47-3 11-1

47-4 47-5 47

Referring to the synthetic route of compound **6**, compound **6-1** was replaced with compound **47-1** to synthesize compound **47-4** (29 mg, 0.0507 mmol). To compound **47-4** (29 mg, 0.0507 mmol), dichloromethane (2 mL) and diethylaminosulfur trifluoride (11 mg, 0.066) were added at 0°C, and the reaction was carried out at room temperature for 2 hours. Water (10 mL) was added, and the mixture was extracted with ethyl acetate (10 mL × 3). The organic phase was dried over anhydrous sodium sulfate, filtered, and rotary evaporated to dryness to remove the solvent. The resulting crude product was purified by normal-phase column chromatography (ethyl acetate: petroleum ether = 20%) to obtain compound **47-5** (15 mg, yield: 52%) as a white solid. MS (ESI, m/z): 574.3 $[M+H]^+$.

Then, referring to the synthetic route of compound 6, the synthesis was carried out, and the resulting crude product was purified by reverse-phase column chromatography [acetonitrile/water (0.05% formic acid) = 0% to 100%] to obtain compound **47** (3 mg, yield: 21%) as a white solid. MS (ESI, m/z): 560.3 $[M+H]^+$.

$^1$H NMR (400 MHz, $CDCl_3$) δ 7.73 - 7.65 (m, 2H), 7.48 (d, *J* = 8.0 Hz, 1H), 7.36 (s, 1H), 7.23 - 7.17 (m, 1H), 7.16 - 7.12 (m, 1H), 6.64 - 6.58 (m, 1H), 6.57 - 6.51 (m, 1H), 6.47 - 6.41 (m, 1H), 4.37 (d, *J* = 22.0 Hz, 2H), 3.88 - 3.77 (m, 5H), 3.17 - 3.00 (m, 1H), 2.92 - 2.79 (m, 4H), 2.56 - 2.46 (m, 1H), 2.28 - 2.17 (m, 2H), 1.97 - 1.90 (m, 2H), 1.42 (s, 3H), 1.37 (s, 3H), 1.13 - 1.07 (m, 1H), 0.67 - 0.60 (m, 1H), 0.51 - 0.42 (m, 1H), 0.39 - 0.31 (m, 1H), 0.25 - 0.17 (m, 1H).

**Example** 48:

Synthetic Route:

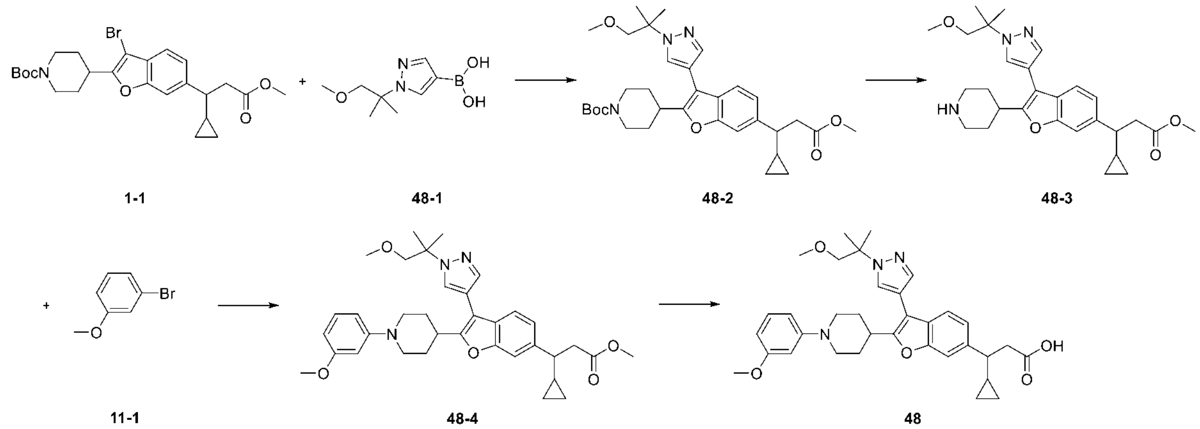

1-1 48-1 48-2 48-3

11-1 48-4 48

Referring to the synthetic route of compound 6, compound 6-1 was replaced with compound 48-1 to carry out the synthesis. The resulting crude product was purified by reverse-phase column chromatography [acetonitrile/water (0.05% formic acid) = 0% to 100%] to obtain compound 48 (27 mg, yield: 46%) as a white solid. MS (ESI, m/z): 572.3 $[M+H]^+$.

$^1$H NMR (400 MHz, $CDCl_3$) δ 7.79 - 7.64 (m, 2H), 7.49 (d, *J* = 8.0 Hz, 1H), 7.35 (s, 1H), 7.24 - 7.17 (m, 2H), 7.14 (d, *J* = 8.0 Hz, 1H), 6.67 - 6.58 (m, 1H), 6.57 - 6.51 (m, 1H), 6.50 - 6.40 (m, 1H), 3.97 - 3.83 (m, 2H), 3.82 (s, 3H), 3.70 - 3.59 (m, 2H), 3.31 (s, 3H), 3.16 - 3.04 (m, 1H), 2.93 - 2.76 (m, 4H), 2.55 - 2.45 (m, 1H), 2.28 - 2.19 (m, 2H), 1.99 - 1.86 (m, 2H), 1.67 (s, 6H), 1.17 - 1.02 (m, 1H), 0.70 - 0.56 (m, 1H), 0.51 - 0.39 (m, 1H), 0.39 - 0.28 (m, 1H), 0.22 -0.17 (m, 1H).

**Example 49:**

Synthetic Route:

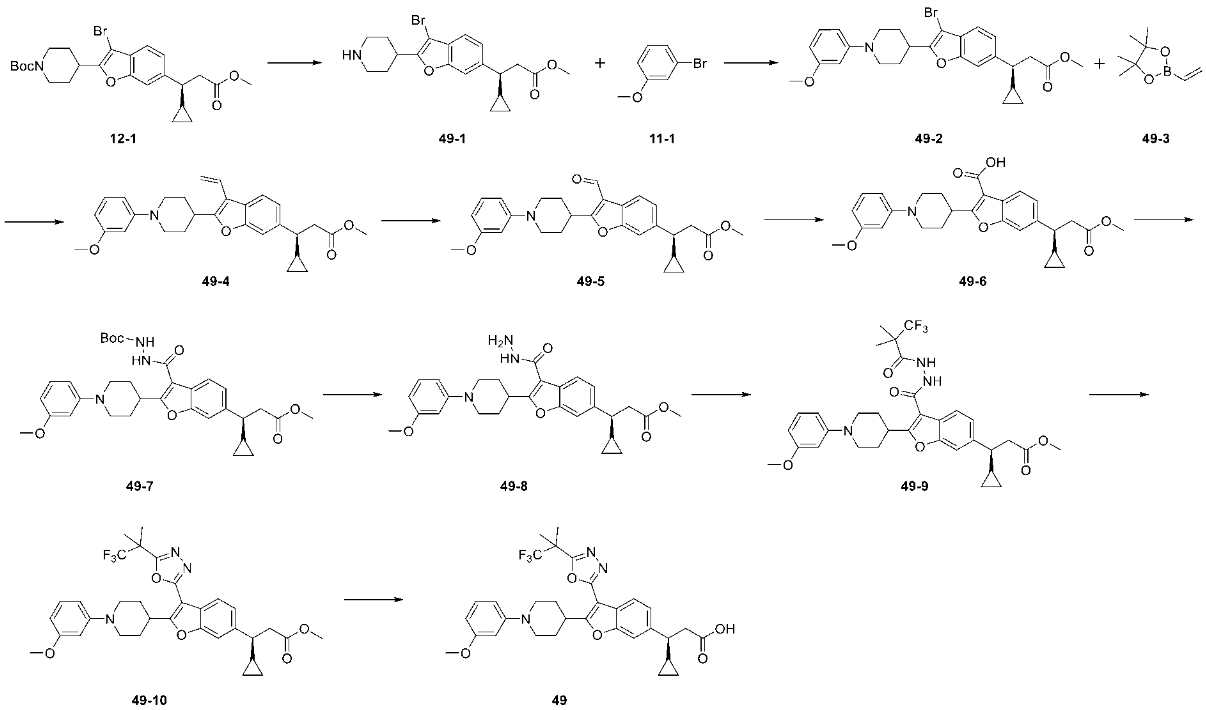

12-1 49-1 11-1 49-2 49-3

49-4 49-5 49-6

49-7 49-8 49-9

49-10 49

A mixture of compound **12-1** (7.6 g, 15.0 mmol), trifluoroacetic acid (5.13 g, 45.0 mmol), and dichloromethane (100 mL) was stirred at room temperature for 2 hours. After the reaction was completed, saturated sodium bicarbonate solution (100 mL) was added, followed by extraction with dichloromethane (200 mL × 3). The organic phase was concentrated, and the resulting crude product was purified by normal-phase column chromatography (ethyl acetate: petroleum ether = 50%) to obtain compound **49-1** (5.65 g, yield: 93%) as a yellow oil. MS (ESI, m/z): 406.2 $[M+H]^+$.

Compound **49-1** (5.65 g, 13.9 mmol), 2,2'-bis(diphenylphosphino)-1,1'-binaphthyl (1.21 g, 1.9 mmol), palladium acetate (400 mg, 1.9 mmol), compound **11-1** (4.28 g, 20.8 mmol), and cesium carbonate (18.12 g, 55.6 mol) were added to toluene (100 mL) and stirred at 100°C overnight. LCMS monitoring confirmed the complete consumption of the starting material. After the reaction was stopped, dichloromethane (200 mL) and water (200 mL) were added to the reaction mixture. The reaction mixture was extracted, and the organic phase was concentrated. The resulting crude product was purified by normal-phase column chromatography (ethyl acetate: petroleum ether = 50%) to obtain compound **49-2** (949 mg, yield: 13%) as a white solid. MS (ESI, m/z): 512.1 $[M+H]^+$.

Compound **49-2** (525 mg, 1.0 mmol), compound **49-3** (385 mg, 2.5 mmol), [1,1'-bis(diphenylphosphino) ferrocene]palladium(II) chloride dichloromethane complex (40 mg, 0.5 mmol), and cesium carbonate (975 mg, 3 mmol) were added to a mixture of 1,4-dioxane: water = 5:1 (25 mL). The reaction mixture was stirred at 80°C for 16 hours. LCMS monitoring confirmed the complete consumption of the starting material. The reaction mixture was extracted with ethyl acetate (50 mL) and concentrated. The resulting crude product was purified by normal-phase column chromatography (ethyl acetate: petroleum ether = 20%) to obtain compound **49-4** (390 mg, yield: 97%) as a yellow oil. MS (ESI, m/z): 460.2 $[M+H]^+$.

Compound **49-4** (390 mg, 0.85 mmol) and 2,6-dimethylpyridine (182 mg, 1.7 mmol) were added to a mixture of 1,4-dioxane: water = 3:1 (40 mL). Potassium osmate dihydrate (15.5 mg, 0.0425 mmol) and sodium periodate (910 mg, 4.25 mmol) were added at 0°C, and the reaction mixture was stirred at 0°C for 4 hours. LCMS monitoring confirmed the complete consumption of the starting material. Saturated sodium thiosulfate aqueous solution (25 mL) was added at 0°C, and the reaction mixture was extracted with ethyl acetate (50 mL) and concentrated. The resulting crude product was purified by normal-phase column chromatography (ethyl acetate: petroleum ether = 20%) to obtain compound **49-5** (195 mg, yield: 49%) as a yellow oil. MS (ESI, m/z): 462.2 $[M+H]^+$.

Compound **49-5** (195 mg, 0.425 mmol) and 2-methyl-2-butene (600 mg, 8.5 mmol) were added to a mixture of tetrahydrofuran: tert-butanol = 1:1 (20 mL). An aqueous solution (10 mL) containing sodium dihydrogen phosphate (700 mg, 5.1 mmol) and sodium chlorite (192 mg, 2.15 mmol) was added dropwise at 25°C. The reaction mixture was stirred at 25°C for 4 hours. LCMS monitoring confirmed the complete consumption of the starting material. The reaction mixture was

extracted with ethyl acetate (30 mL) and concentrated. The resulting crude product was purified by normal-phase column chromatography (ethyl acetate: petroleum ether = 50%) to obtain compound **49-6** (128 mg, yield: 63%) as a yellow oil. MS (ESI, m/z): 478.2 [M+H]$^+$.

Compound **49-6** (120 mg, 0.25 mmol), triethylamine (76 mg, 0.75 mmol), and 2-(7-azabenzotriazol-1-yl)-*N*,*N*,*N*',*N*'-tetramethyluronium hexafluorophosphate (191 mg, 0.50 mmol) were added to N,N-dimethylformamide (5 mL) and stirred. Then, tert-butyl carbazate (66 mg, 0.50 mmol) was added, and the reaction mixture was stirred at room temperature for 16 hours. After the reaction was completed, ethyl acetate (80 mL) was added for dilution, and the organic phase was washed with water (40 mL × 3), dried over anhydrous sodium sulfate, and filtered. The filtrate was concentrated, and the resulting crude product was purified by normal-phase column chromatography (ethyl acetate: petroleum ether = 50%) to obtain compound **49-7** (145 mg, yield: 97%) as a yellow oil. MS (ESI, m/z): 592.2 [M+H]$^+$.

Compound **49-7** (145 mg, 0.25 mmol) was added to 1,4-dioxane (2 mL) and stirred. Then, hydrochloric acid in 1,4-dioxane (4 M, 4 mL) was added, and the reaction mixture was stirred at room temperature for 4 hours. After the reaction was completed, the reaction mixture was concentrated. The resulting crude product was purified by normal-phase column chromatography (ethyl acetate: petroleum ether = 50%) to obtain compound **49-8** (128 mg, yield: 99%) as a yellow oil. MS (ESI, m/z): 492.2 [M+H]$^+$.

Compound **49-8** (64 mg, 0.12 mmol), triethylamine (37 mg, 0.36 mmol), and 2-(7-azabenzotriazol-1-yl)-*N*,*N*,*N*',*N*'-tetramethyluronium hexafluorophosphate (92 mg, 0.24 mmol) were added to N,N-dimethylformamide (10 mL) and stirred. Then, 3,3,3-trifluoro-2,2-dimethylpropanoic acid (38 mg, 0.36 mmol) was added, and the reaction mixture was stirred at room temperature for 16 hours. After the reaction was completed, ethyl acetate (200 mL) was added for dilution, and the organic phase was washed with water (40 mL × 3). The organic phase was dried over anhydrous sodium sulfate, filtered, and the filtrate was concentrated. The resulting crude product was purified by normal-phase column chromatography (ethyl acetate: petroleum ether = 50%) to obtain compound **49-9** (72 mg, yield: 95%) as a yellow oil. MS (ESI, m/z): 630.2 [M+H]$^+$.

Compound **49-9** (72 mg, 0.11 mmol) and *p*-toluenesulfonyl chloride (33 mg, 0.17 mmol) were added to dichloromethane (5 mL), and triethylamine (35 mg, 0.34 mmol) was added at 0°C. The reaction mixture was reacted at room temperature for 2 hours. After the reaction was completed, the reaction mixture was concentrated to obtain a crude product. The resulting crude product was then purified by normal-phase column chromatography (petroleum ether: ethyl acetate = 8:2) to obtain compound **49-10** (65 mg, yield: 93%) as a yellow oil. MS (ESI, m/z): 612.2 [M+H]$^+$.

Compound **49-10** (65 mg, 0.11 mmol) was added to methanol (2 mL) and stirred. 4 M sodium hydroxide solution (2 mL) was added, and the reaction mixture was stirred at 60°C for 16 hours. After the reaction was completed, the reaction mixture was concentrated to obtain a crude product. The resulting crude product was then purified by reverse-phase column chromatography [acetonitrile/water (0.05% formic acid) = 0% to 100%] to obtain compound **49** (21.05 mg, yield: 33%) as a yellow solid. MS (ESI, m/z): 598.3 [M+H]$^+$.

$^1$H NMR (400 MHz, $CDCl_3$) δ 7.98 - 7.87 (m, 1H), 7.42 (s, 1H), 7.32 - 7.27 (m, 1H), 7.24 - 7.15 (m, 1H), 6.66 - 6.59 (m, 1H), 6.55 (s, 1H), 6.49 - 6.41 (m, 1H), 3.91 - 3.73 (m, 6H), 2.98 - 2.80 (m, 4H), 2.55 - 2.46 (m, 1H), 2.26 - 2.16 (m, 2H), 2.08 - 2.02 (m, 2H), 1.77 (s, 6H), 1.16 - 1.03 (m, 1H), 0.69 - 0.59 (m, 1H), 0.48-0.42 (m, 1H), 0.39 - 0.30 (m, 1H), 0.21-0.16 (m, 1H).

**Example 50:**

Synthetic Route:

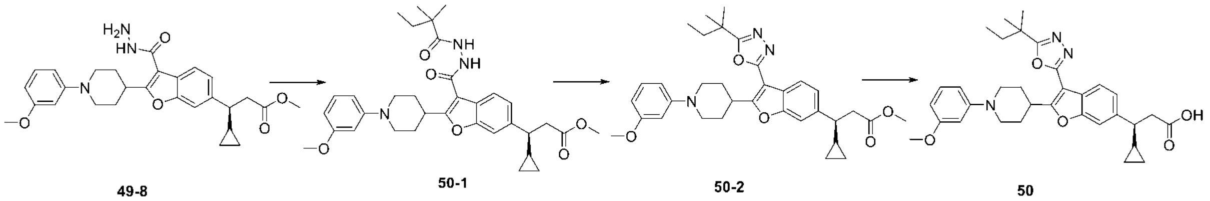

49-8 50-1 50-2 50

Referring to the synthetic route of compound **49**, 3,3,3-trifluoro-2,2-dimethylpropionic acid was replaced with 2,2-dimethylbutyric acid to carry out the synthesis. The resulting crude product was purified by reverse-phase column chromatography [acetonitrile/water (0.05% formic acid) = 0% to 100%] to obtain compound **50** (11 mg, yield: 19%) as a white solid. MS (ESI, m/z): 558.3 [M+H]$^+$.

$^1$H NMR (400 MHz, $CDCl_3$) δ 7.95 - 7.90 (m, 1H), 7.41 (s, 1H), 7.27 - 7.16 (m, 2H), 6.68 - 6.60 (m, 1H), 6.56 (s, 1H), 6.50 - 6.42 (m, 1H), 3.93 - 3.68 (m, 6H), 3.00 - 2.75 (m, 4H), 2.58 - 2.43 (m, 1H), 2.31 - 2.15 (m, 2H), 2.07 -2.04 (m, 2H), 1.90 -

1.83 (m, 2H), 1.49 (s, 6H), 1.14 - 1.04 (m, 1H), 0.92 (t, $J$ = 7.6 Hz, 3H), 0.69 - 0.59 (m, 1H), 0.51 - 0.40 (m, 1H), 0.40 - 0.30 (m, 1H), 0.25 - 0.13 (m, 1H).

**Example 51:**

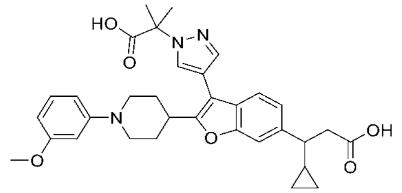

Synthetic Route:

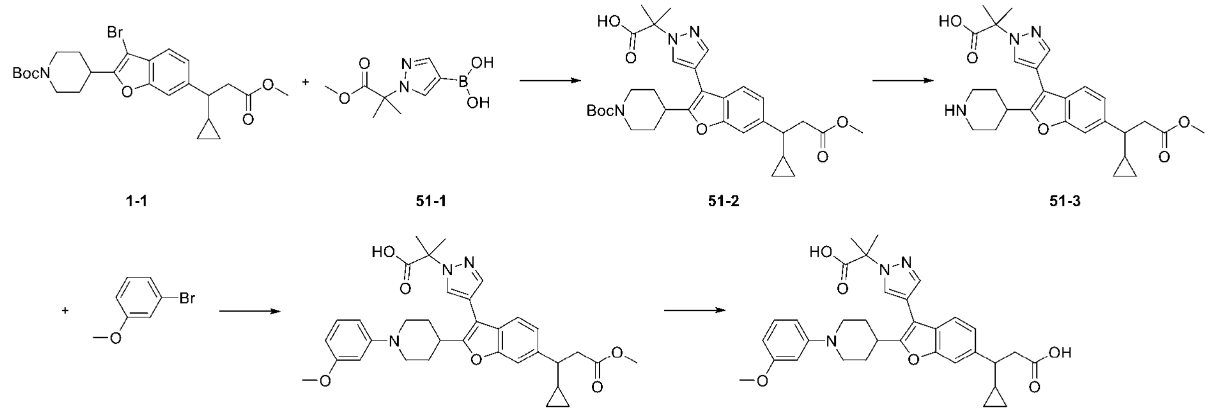

1-1 51-1 51-2 51-3

11-1 51-4 51

Referring to the synthetic route of compound **6**, compound **6-1** was replaced with compound 51-1 to carry out the synthesis. The resulting crude product was purified by reverse-phase column chromatography [acetonitrile/water (0.05% formic acid) = 0% to 100%] to obtain compound **51** (5 mg, yield: 15%) as a white solid. MS (ESI, m/z): 572.3 $[M+H]^+$.

$^1$H NMR (400 MHz, $CDCl_3$) δ 7.80 - 7.70 (m, 2H), 7.41 (d, $J$ = 8.0 Hz, 1H), 7.35 (s, 1H), 7.23 - 7.17 (m, 1H), 7.09 (d, $J$ = 8.0 Hz, 1H), 6.67 - 6.60 (m, 1H), 6.56 (s, 1H), 6.48 - 6.42 (m, 1H), 3.83 - 3.77 (m, 5H), 3.13 - 3.00 (m, 1H), 2.90 - 2.80 (m, 4H), 2.49 - 2.40 (m, 1H), 2.27 - 2.12 (m, 2H), 1.95 (s, 6H), 1.92 - 1.87 (m, 2H), 1.12 - 1.01 (m, 1H), 0.66 - 0.57 (m, 1H), 0.48 - 0.39 (m, 1H), 0.36 - 0.26 (m, 1H), 0.25 - 0.16 (m, 1H).

**Example 52:**

Synthetic Route:

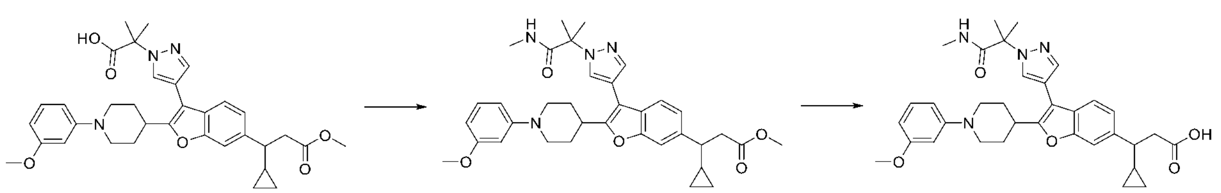

51-4 52-1 52

Referring to the synthetic route of compound **51**, compound **51-4** (100 mg, 0.172 mol) was obtained. Compound **51-4** (100 mg, 0.172 mol), methylamine hydrochloride (14 mg, 0.207 mmol), HATU (79 mg, 0.207 mmol), and TEA (52 mg,

0.517 mmol) were added to dichloromethane (5 mL), and the reaction was carried out at room temperature for 16 hours. After the reaction was completed, the reaction mixture was directly concentrated. The resulting crude product was purified by normal-phase column chromatography (ethyl acetate: petroleum ether = 0% to 100%) to obtain compound **52-1** (109 mg, yield: 99%) as a white solid. MS (ESI, m/z): 599.2 $[M+H]^+$.

Referring to the synthetic route of compound **51**, the synthesis was carried out, and the resulting crude product was purified by reverse-phase column chromatography [acetonitrile/water (0.05% formic acid) = 0% to 100%] to obtain compound **52** (30 mg, yield: 38%) as a white solid. MS (ESI, m/z): 585.3 $[M+H]^+$.

$^1$H NMR (400 MHz, $CDCl_3$) $\delta$ 7.84 - 7.77 (m, 2H), 7.45 (d, $J$ = 8.0 Hz, 1H), 7.37 (s, 1H), 7.25 - 7.12 (m, 2H), 6.67 - 6.59 (m, 1H), 6.55 (s, 1H), 6.49 - 6.43 (m, 1H),6.42 - 6.35 (m, 1H), 3.87 - 3.81 (m, 5H), 3.14 - 3.00 (m, 1H), 2.90 - 2.82 (m, 4H), 2.81 - 2.74 (m, 3H), 2.57 - 2.46 (m, 1H), 2.33 - 2.16 (m, 2H), 1.97 - 1.91 (m, 8H), 1.16 - 0.98 (m, 1H), 0.70 - 0.59 (m, 1H), 0.50 - 0.41 (m, 1H), 0.40 - 0.29 (m, 1H), 0.26 - 0.14 (m, 1H).

**Example 53:**

Synthetic Route:

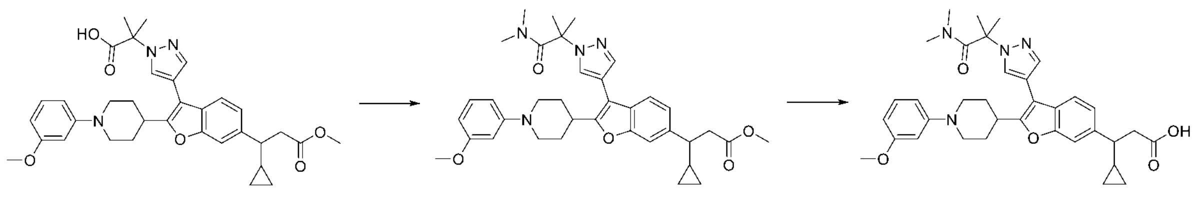

51-4 53-1 53

Referring to the synthetic route of compound **52**, methylamine hydrochloride was replaced with dimethylamine hydrochloride to carry out the synthesis. The resulting crude product was purified by reverse-phase column chromatography [acetonitrile/water (0.05% formic acid) = 0% to 100%] to obtain compound **53** (22 mg, yield: 41%) as a white solid. MS (ESI, m/z): 599.3 $[M+H]^+$.

$^1$H NMR (400 MHz, DMSO-$d_6$) $\delta$ 8.18 - 8.12 (m, 1H), 7.84 - 7.78 (m, 1H), 7.54 - 7.48 (m, 1H), 7.44 (s, 1H), 7.17 - 7.09 (m, 2H), 6.67 - 6.59 (m, 1H), 6.55 (s, 1H), 6.49 - 6.43 (m, 1H), 3.84 - 3.76 (m, 2H), 3.72 (s, 3H), 3.20 - 3.16 (m, 1H), 2.89 - 2.79 (m, 4H), 2.71 - 2.59 (m, 3H), 2.40 - 2.32 (m, 3H), 1.97 - 1.93 (m, 1H), 1.89 - 1.83 (m, 2H), 1.76 (s, 6H), 1.07 - 1.00 (m, 1H), 0.53 - 0.45 (m, 1H), 0.28 - 0.21 (m, 2H), 0.15 - 0.08 (m, 1H).

**Example 54:**

Synthetic Route:

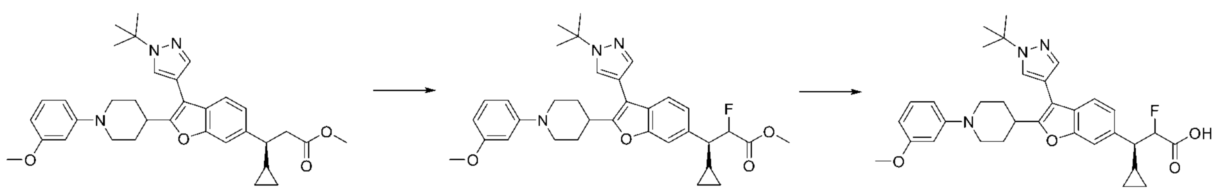

38-4 54-1 54

Referring to the synthetic route of compound **38**, compound **38-4** (210 mg, 0.382 mmol) was obtained and dissolved in tetrahydrofuran (5 mL). Lithium bis(trimethylsilyl)amide (0.77 mL, 2 M in THF) was added at -78°C, and the reaction was transferred to room temperature and carried out for 1 hour. After cooling back to -78°C, N-fluorobenzenesulfonimide (240 mg, 7.64 mmol) was added, and the reaction was carried out at room temperature overnight. The reaction was quenched with saturated ammonium chloride solution (20 mL) and extracted with ethyl acetate (20 mL × 3). The organic phase was dried over anhydrous sodium sulfate, filtered, and concentrated. The resulting crude product was purified by normal-phase column chromatography (ethyl acetate: petroleum ether = 5%) to obtain compound **54-1** (41 mg, yield: 19%) as a white solid. MS (ESI, m/z): 574.3 $[M+H]^+$.

Compound **54-1** (41 mg, 0.0897 mmol) and lithium hydroxide (9.0 mg, 0.358 mmol) were dissolved in THF (2 mL),

methanol (2 mL), and water (2 mL). The reaction was carried out at 50°C for 2 hours. After concentration, the resulting crude product was purified by reverse-phase column chromatography [acetonitrile/water (0.05% formic acid) = 50% to 70%] to obtain compound **54** (8.0 mg, yield: 20%) as a white solid. MS (ESI, m/z): 560.3 [M+H]$^+$.

$^1$H NMR (400 MHz, $CDCl_3$) δ 7.75 - 7.68 (m, 2H), 7.54 - 7.48 (m, 2H), 7.25 - 7.20 (m, 2H), 6.70 - 6.58 (m, 2H), 6.51 - 6.45 (m, 1H), 5.22 (d, *J* = 48.1 Hz, 1H), 3.86 - 3.79 (m, 5H), 3.15 - 3.08 (m, 1H), 2.92 - 2.86 (m, 2H), 2.67 - 2.61 (m, 1H), 2.32 - 2.23 (m, 2H), 1.99 - 1.90 (m, 2H), 1.69 (s, 9H), 1.56 - 1.50 (m, 1H), 0.77 - 0.70 (m, 1H), 0.63 - 0.56 (m, 1H), 0.52 - 0.45 (m, 1H), 0.17 - 0.10 (m, 1H).

**Example 55:**

Synthetic Route:

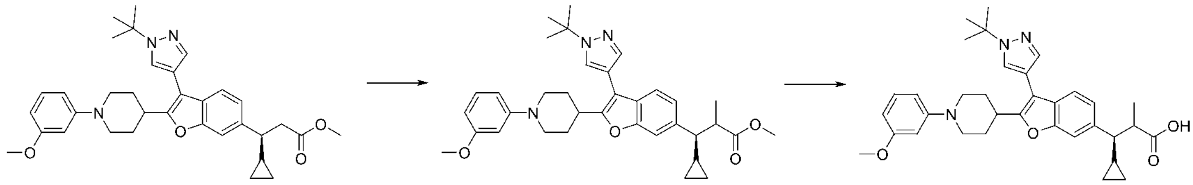

38-4 55-1 55

Referring to the synthetic route of compound **38**, compound **38-4** (253 mg, 0.46 mmol) was obtained and dissolved in tetrahydrofuran (5 mL). Lithium bis(trimethylsilyl)amide (1.0 mL, 2 M in THF) was added at -40°C, and the reaction was carried out for 1 hour. Then iodomethane (196 mg, 1.4 mmol) was added. The reaction was carried out at room temperature overnight. The reaction was quenched with saturated ammonium chloride solution (20 mL) and extracted with ethyl acetate (20 mL × 3). The organic phase was dried over anhydrous sodium sulfate, filtered, and concentrated. The resulting crude product was purified by normal-phase column chromatography (ethyl acetate: petroleum ether = 5%) to obtain compound **55-1** (192 mg, yield: 74%) as a white solid. MS (ESI, m/z): 570.3 [M+H]$^+$.

Referring to the synthetic route of compound **6**, compound **6-5** was replaced with compound **55-1** to carry out the synthesis. The resulting crude product was purified by reverse-phase column chromatography [acetonitrile/water (0.05% formic acid) = 0% to 100%] to obtain compound **55** (122 mg, yield: 64%) as a white solid. MS (ESI, m/z): 556.3 [M+H]$^+$.

$^1$H NMR (400 MHz, $CDCl_3$) δ 7.73 - 7.63 (m, 2H), 7.50 - 7.40 (m, 1H), 7.34 (s, 1H), 7.23 - 7.06 (m, 2H), 6.65 - 6.36 (m, 3H), 3.81 (s, 5H), 3.16 - 3.02 (m, 1H), 3.06 - 2.94 (m, 1H), 2.88 - 2.79 (m, 2H), 2.40 - 2.30 (m, 1H), 2.26 - 2.15 (m, 2H), 1.97 - 1.88 (m, 2H), 1.66 (s, 9H), 1.31 (d, *J* = 7.2 Hz, 3H), 1.19 - 1.06 (m, 1H), 0.74 - 0.67 (m, 1H), 0.45 - 0.31 (m, 2H), 0.09 - 0.03 (m, 1H).

**Example 56:**

Synthetic Route:

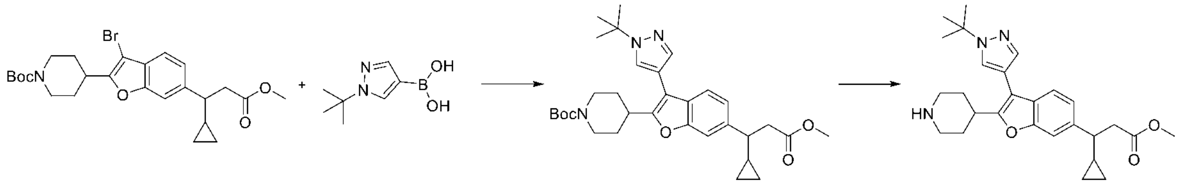

1-1 38-1 56-1 56-2

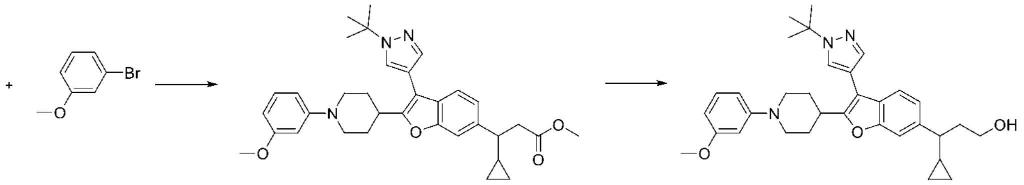

11-1 56-3 56

Referring to the synthetic route of compound **38**, compound **12-1** was replaced with compound 1-1 to synthesize compound 56-3 (560 mg, 1.0 mmol) as a white solid. Compound **56-3** (560 mg, 1.0 mmol) was added to tetrahydrofuran (10 mL), and lithium aluminum hydride (84 mg, 2.22 mmol) was slowly added to the reaction mixture under an ice bath. The reaction mixture was then stirred at room temperature for 1 hour. After the reaction was completed, the system was quenched sequentially with 0.1 mL of water, 0.1 mL of 15% sodium hydroxide solution, and 0.3 mL of water. Anhydrous sodium sulfate was added, and the mixture was filtered. The filtrate was concentrated to obtain a crude product, which was then purified by reverse-phase column chromatography [acetonitrile/water (0.05% formic acid) = 0% to 100%] to obtain compound 56 (377 mg, yield: 71%) as a white solid. MS (ESI, m/z): 528.1 $[M+H]^+$.

$^1H$ NMR (400 MHz, DMSO-$d_6$) δ 8.13 - 8.06 (m, 1H), 7.76 - 7.71 (m, 1H), 7.59 - 7.53 (m, 1H), 7.41 - 7.37 (m, 1H), 7.16 - 7.08 (m, 2H), 6.61 - 6.54 (m, 1H), 6.51 - 6.46 (m, 1H), 6.40 - 6.33 (m, 1H), 4.33 - 4.28 (m, 1H), 3.84 (d, $J$ = 12.0 Hz, 2H), 3.74 (s, 3H), 3.28 - 3.16 (m, 1H), 2.95 - 2.85 (m, 2H), 2.06 - 1.81 (m, 8H), 1.61 (s, 9H), 1.10 - 0.95 (m, 1H), 0.59 - 0.49 (m, 1H), 0.30 - 0.19 (m, 2H), 0.06 - 0.03 (m, 1H).

**Example 57:**

Synthetic Route:

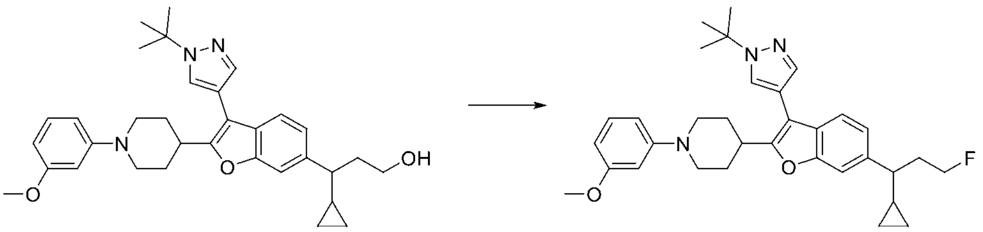

56 57

Compound **56** (45 mg, 0.085 mmol) was added to dichloromethane (5 mL). Diethylaminosulfur trifluoride (27 mg, 0.17 mmol) was slowly added to the reaction mixture under an ice bath, and the reaction mixture was stirred at room temperature for 2 hours. After the reaction was completed, the reaction mixture was directly concentrated and purified by reverse-phase column chromatography [acetonitrile/water (0.05% formic acid) = 0% to 100%] to obtain compound 57 (7 mg, yield: 16%) as a white solid. MS (ESI, m/z): 530.1 $[M+H]^+$.

$^1H$ NMR (400 MHz, DMSO-$d_6$) δ 8.10 (s, 1H), 7.75 (s, 1H), 7.63 - 7.54 (m, 1H), 7.45 (s, 1H), 7.14 - 7.10 (m, 2H), 6.63 - 6.54 (m, 1H), 6.49 (s, 1H), 6.39 - 6.31 (m, 1H), 4.54 - 4.18 (m, 1H), 3.89 - 3.78 (m, 2H), 3.73 (s, 3H), 3.22 - 3.18 (m, 1H), 2.95 - 2.81 (m, 2H), 2.26 - 1.82 (m, 8H), 1.60 (s, 9H), 1.15 - 1.02 (m, 1H), 0.62 - 0.53 (m, 1H), 0.33 - 0.19 (m, 2H), 0.11 - 0.02 (m, 1H).

**Example 58:**

Synthetic Route:

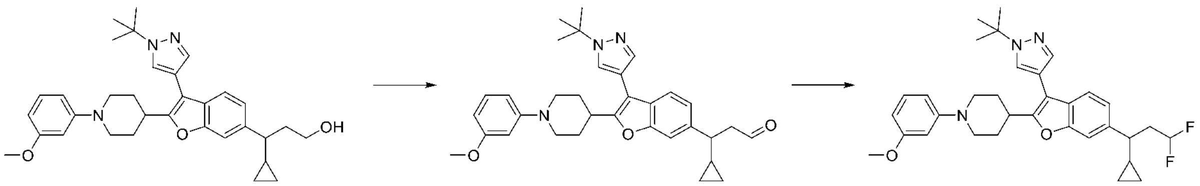

56 58-1 58

Compound **56** (300 mg, 0.57 mmol) was added to dichloromethane (15 mL). Under an ice bath, Dess-Martin periodinane (483 mg, 1.14 mmol) was slowly added to the reaction mixture and stirred at room temperature for 1 hour. After the reaction was completed, the reaction mixture was directly concentrated and purified by reverse-phase column chromatography [acetonitrile/water (0.05% formic acid) = 0% to 100%] to obtain compound **58-1** (53 mg, yield: 18%) as a white solid. MS (ESI, m/z): 526.1 $[M+H]^+$.

Compound **58-1** (53 mg, 0.1 mmol) was added to dichloromethane (15 mL). Diethylaminosulfur trifluoride (33 mg,

0.2 mmol) was slowly added to the reaction mixture under an ice bath, and the reaction mixture was stirred at room temperature for 1 hour. After the reaction was completed, the reaction mixture was directly concentrated and purified by reverse-phase column chromatography [acetonitrile/water (0.05% formic acid) = 0% to 100%] to obtain compound **58** (6 mg, yield: 11%) as a yellow solid. MS (ESI, m/z): 548.1 [M+H]$^+$.

$^1$H NMR (400 MHz, DMSO-$d_6$) δ 7.76 - 7.65 (m, 2H), 7.54 - 7.46 (m, 1H), 7.38 - 7.31 (m, 1H), 7.26 - 7.17 (m, 2H), 7.15 - 7.08 (m, 1H), 6.69 - 6.38 (m, 2H), 5.91 - 5.57 (m, 1H), 3.89 - 3.76 (m, 5H), 3.13 - 2.83 (m, 1H), 2.42 - 2.09 (m, 5H), 2.05 - 1.89 (m, 2H), 1.69 (s, 9H), 1.57 - 1.49 (m, 2H), 1.10 - 1.06 (m, 1H), 0.75 - 0.64 (m, 1H), 0.50 - 0.39 (m, 1H), 0.38 - 0.29 (m, 1H), 0.22 - 0.13 (m, 1H).

**Example 59:**

Synthetic Route:

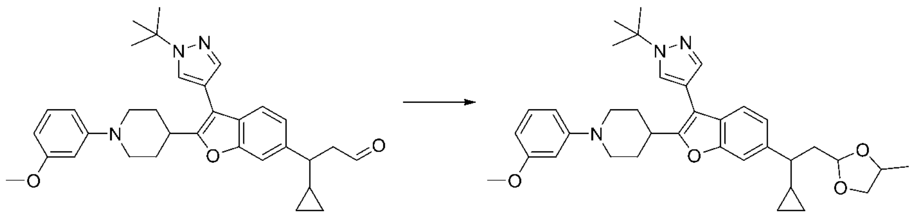

58-1 59

Compound **58-1** (94 mg, 0.18 mmol), propylene glycol (82 mg, 1.07 mmol), and a catalytic amount of *p*-toluenesulfonic acid were added to toluene (5 mL). The reaction mixture was heated to 120°C and stirred overnight. After the reaction was completed, the reaction mixture was directly concentrated and purified by reverse-phase column chromatography [acetonitrile/water (0.05% formic acid) = 0% to 100%] to obtain compound **59** (5 mg, yield: 5%) as a white solid. MS (ESI, m/z): 584.2 [M+H]$^+$.

$^1$H NMR (400 MHz, DMSO-$d_6$) δ 7.71 (s, 2H), 7.53 - 7.45 (m, 1H), 7.35 (s, 1H), 7.25 - 7.18 (m, 1H), 7.17 - 7.10 (m, 1H), 6.67 - 6.59 (m, 1H), 6.55 (s, 1H), 6.49 - 6.38 (m, 1H), 4.84 - 4.76 (m, 1H), 4.14 - 4.03 (m, 1H), 3.92 - 3.74 (m, 6H), 3.40 - 3.31 (m, 1H), 3.17 - 3.06 (m, 1H), 2.92 - 2.71 (m, 2H), 2.30 - 2.10 (m, 5H), 1.97 - 1.89 (m, 2H), 1.69 (s, 9H), 1.32 - 1.28 (m, 3H), 1.08 - 1.01 (m, 1H), 0.68 - 0.59 (m, 1H), 0.43 - 0.31 (m, 2H), 0.21 - 0.11 (m, 1H).

**Example 60:**

Synthetic Route:

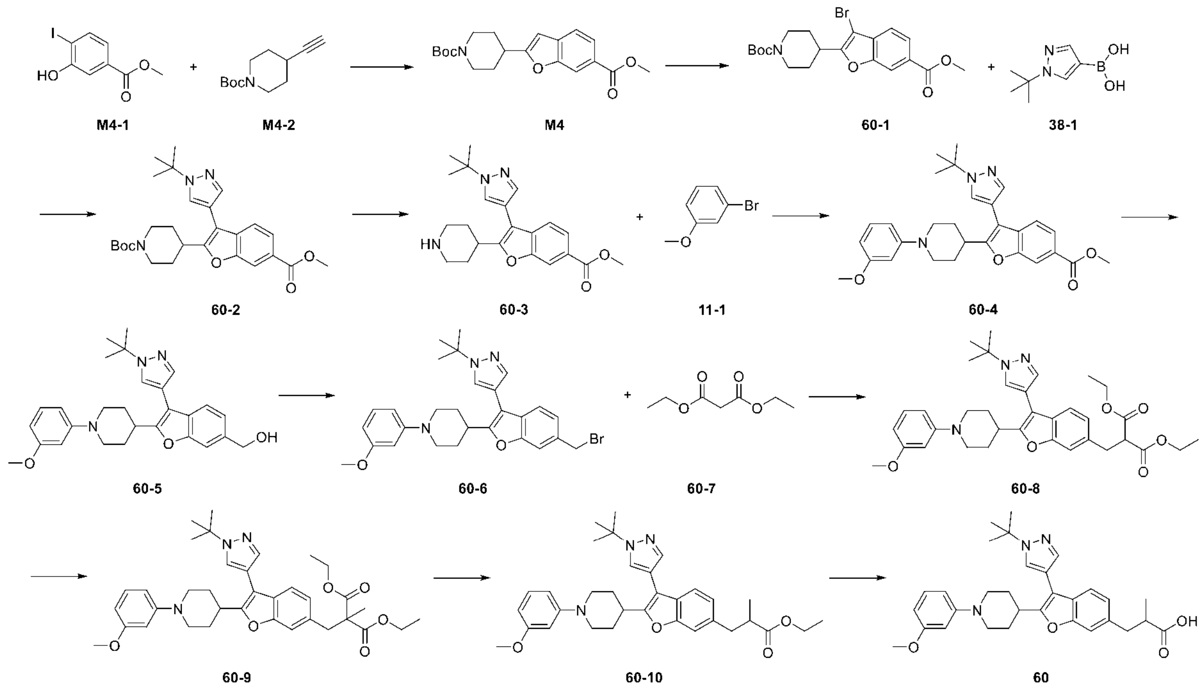

M4-1 M4-2 M4 60-1 38-1

60-2 60-3 11-1 60-4

60-5 60-6 60-7 60-8

60-9 60-10 60

Intermediate **M4** was synthesized by referring to the synthetic route of intermediate **M1**. Then, referring to the synthetic route of compound **38** and replacing compound **12-1** with compound **60-1**, compound **60-4** (3.42 g, 7.02 mmol) was obtained. Compound **60-4** (3.42 g, 7.02 mmol) was added to tetrahydrofuran (20 mL), and lithium aluminum hydride (293 mg, 7.72 mmol) was added under an ice bath. The reaction mixture was stirred at 0°C for 1 hour, then quenched with water (0.3 mL), followed by the addition of 15% sodium hydroxide (0.3 mL) and water (0.9 mL). The mixture was dried over magnesium sulfate, filtered, and the filtrate was concentrated. The resulting crude product was purified by normal-phase column chromatography (ethyl acetate: petroleum ether = 0% to 100%) to obtain compound **60-5** (2.5 g, yield: 77%) as a yellow solid. MS (ESI, m/z): 460.2 $[M+H]^+$.

Compound **60-5** (1.5 g, 3.27 mmol), phosphorus tribromide (1.5 mL), and dichloromethane (25 mL) were stirred and reacted at room temperature for 2 hours. The reaction mixture was directly concentrated, and the resulting crude product was purified by normal-phase column chromatography (ethyl acetate: petroleum ether = 0% to 100%) to obtain compound **60-6** (1.2 g, yield: 70%) as a yellow oil. MS (ESI, m/z): 522.2 $[M+H]^+$.

Compound **60-6** (1.2 g, 2.3 mmol), compound **60-7** (3.08 g, 323 mmol), potassium carbonate (634 mg, 4.6 mmol), and *N,N*-dimethylformamide (10 mL) were stirred at 80°C for 3 hours. The reaction mixture was directly concentrated, and the resulting crude product was purified by normal-phase column chromatography (ethyl acetate: petroleum ether = 0% to 100%) to obtain compound **60-8** (0.9 g, yield: 65%) as a yellow oil. MS (ESI, m/z): 602.2 $[M+H]^+$.

Compound **60-8** (20 mg, 0.0333 mmol), iodomethane (14 mg, 0.0998 mmol), cesium carbonate (33 mg, 0.0999 mmol), and N,N-dimethylformamide (2 mL) were stirred at room temperature for 2 hours. The reaction mixture was directly concentrated, and the resulting crude product was purified by normal-phase column chromatography (ethyl acetate: petroleum ether = 0% to 100%) to obtain compound **60-9** (15 mg, yield: 73%) as a yellow oil. MS (ESI, m/z): 616.3 $[M+H]^+$.

Compound **60-9** (15 mg, 0.0244 mmol), water (1 mL), and *N,N*-dimethylformamide (2 mL) were stirred at room temperature for 2 hours. The reaction mixture was directly concentrated, and the resulting crude product was purified by normal-phase column chromatography (ethyl acetate: petroleum ether = 0% to 100%) to obtain compound **60-10** (10 mg, yield: 75%) as a white solid. MS (ESI, m/z): 544.2 $[M+H]^+$.

Compound **60-10** (15 mg, 0.0184 mmol), lithium hydroxide (22 mg, 0.921 mmol), water (1 mL), tetrahydrofuran (2 mL), and methanol (2 mL) were stirred at 60°C for 2 hours. The reaction mixture was directly concentrated, and the resulting crude product was purified by reverse-phase column chromatography [acetonitrile/water (0.05% formic acid) = 0% to 100%] to obtain compound **60** (8.85 mg, yield: 93%) as a white solid. MS (ESI, m/z): 516.3 $[M+H]^+$.

$^1H$ NMR (400 MHz, $CDCl_3$) δ 7.71 (s, 2H), 7.48 - 7.42 (m, 1H), 7.29 (s, 1H), 7.23 - 7.16 (m, 1H), 7.11 - 7.05 (m, 1H), 6.64 - 6.58 (m, 1H), 6.54 (s, 1H), 6.46 - 6.40 (m, 1H), 3.86 - 3.78 (m, 5H), 3.25 - 3.15 (m, 1H), 3.15 - 3.04 (m, 1H), 2.90 - 2.76 (m, 4H), 2.27 - 2.18 (m, 2H), 1.98 - 1.89 (m, 2H), 1.68 (s, 9H), 1.24 - 1.17 (m, 2H).

**Example 61:**

Synthetic Route:

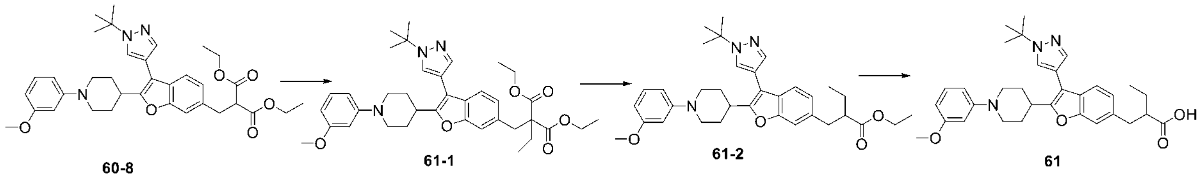

60-8 61-1 61-2 61

Referring to the synthetic route of compound **60**, iodomethane was replaced with iodoethane to carry out the synthesis. The resulting crude product was purified by reverse-phase column chromatography [acetonitrile/water (0.05% formic acid) = 0% to 100%] to obtain compound **51** (7 mg, yield: 22%) as a white solid. MS (ESI, m/z): 530.3 $[M+H]^+$.

$^1H$ NMR (400 MHz, $CDCl_3$) δ 7.76 - 7.60 (m, 3H), 7.50 - 7.42 (m, 1H), 7.33 - 7.29 (m, 1H), 7.24 - 7.20 (m, 1H), 7.12 - 7.06 (m, 1H), 6.72 - 6.37 (m, 2H), 3.83 (s, 3H), 3.82 - 3.78 (m, 2H), 3.16 - 3.11 (m, 1H), 3.11 - 3.07 (m, 1H), 2.93 - 2.86 (m, 2H), 2.79 - 2.60 (m, 2H), 2.34 - 2.12 (m, 2H), 1.98 - 1.89 (m, 2H), 1.68 (s, 9H), 1.64 - 1.62 (m, 2H), 0.99 (t, *J* = 7.2 Hz, 3H).

**Example 62:**

Synthetic Route:

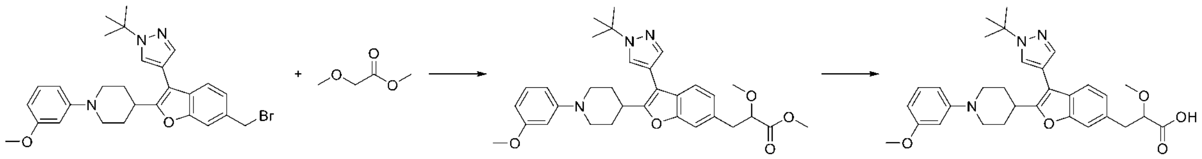

60-6 62-1 62-2 62

Referring to the synthetic route of compound **60**, compound **60-6** (116 mg, 0.222 mmol) and compound **62-1** (46 mg, 0.444 mmol) were synthesized and added to tetrahydrofuran (5 mL). Sodium bis(trimethylsilyl)amide (444 μL, 0.444 mmol) was added at -70°C, and the reaction was stirred at -70°C for 1 hour, followed by stirring at room temperature overnight. The reaction mixture was quenched with water at 0°C. The organic phase was dried over sodium sulfate and purified by normal-phase column chromatography (ethyl acetate: petroleum ether = 0% to 100%) to obtain compound **62-2** (42 mg, yield: 35%) as a yellow oil. MS (ESI, m/z): 546.3 $[M+H]^+$.

Referring to the synthetic route of compound **60**, the synthesis was carried out, and the resulting crude product was purified by reverse-phase column chromatography [acetonitrile/water (0.05% formic acid) = 0% to 100%] to obtain compound **62** (15 mg, yield: 38%) as a yellow solid. MS (ESI, m/z): 532.3 $[M+H]^+$.

$^1H$ NMR (400 MHz, $CDCl_3$) δ 7.76 (s, 1H), 7.73 (s, 1H), 7.49 (d, *J* = 8.0 Hz, 1H), 7.40 (s, 1H), 7.26 - 7.16 (m, 2H), 6.72 - 6.64 (m, 1H), 6.61 (s, 1H), 6.53 - 6.45 (m, 1H), 4.13 - 4.05 (m, 1H), 3.86 - 3.80 (m, 5H), 3.44 (s, 3H), 3.34 - 3.24 (m, 1H), 3.23 - 3.07 (m, 2H), 2.96 - 2.83 (m, 2H), 2.35 - 2.20 (m, 2H), 2.02 - 1.91 (m, 2H), 1.71 (s, 9H).

**Example 63:**

Synthetic Route:

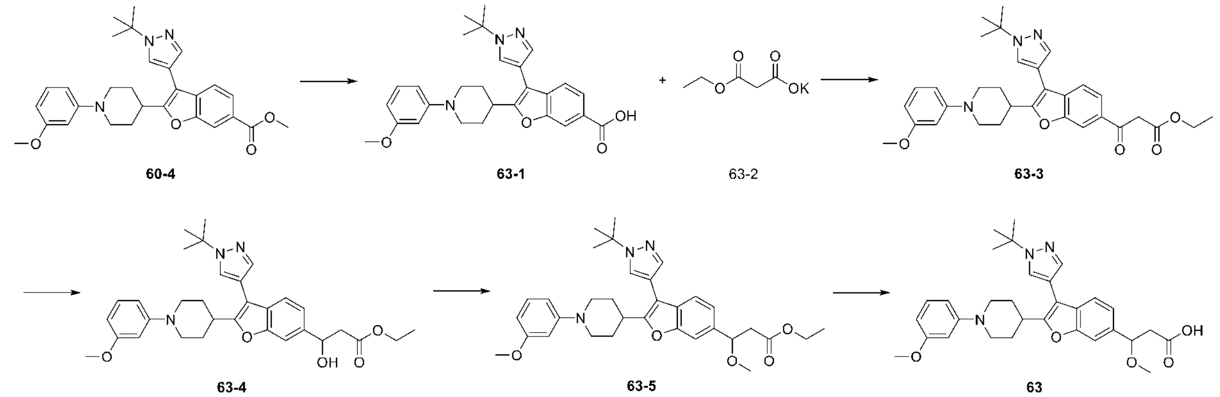

60-4 63-1 63-2 63-3

63-4 63-5 63

Referring to the synthetic route of compound **60**, compound **60-4** (1.7 g, 3.5 mmol) was synthesized. Compound **60-4** (1.7 g, 3.5 mmol) and lithium hydroxide (170 mg, 7.0 mmol) were added to tetrahydrofuran (16 mL) and water (4 mL). The reaction was carried out at room temperature for 16 hours, followed by dilution with water (20 mL) and extraction with ethyl acetate (20 mL × 3). The organic phase was dried over sodium sulfate and purified by normal-phase column chromatography (ethyl acetate: petroleum ether = 0% to 100%) to obtain compound **63-1** (1.7 g, yield: 91%) as a yellow solid. MS (ESI, m/z): 474.2 $[M+H]^+$.

Compound **63-2** (327 mg, 2.38 mmol) was dissolved in ethyl acetate (8 mL). Triethylamine (421 mg, 4.06 mmol) and magnesium chloride (217 mg, 2.28 mmol) were added at 0°C, and the reaction was carried out at room temperature for 3 hours to obtain system **1**. Compound **63-1** (600 mg, 1.27 mmol) and *N,N'*-carbonyldiimidazole (272 mg, 1.65 mmol) were dissolved in THF (8 mL) and reacted at room temperature for 2 hours. The resulting mixture was added to system **1** and reacted at 45°C for 8 hours. Water (30 mL) was added for dilution, followed by extraction with ethyl acetate (30 mL × 3). The organic phase was dried over anhydrous sodium sulfate, filtered, and concentrated. The resulting crude product was purified by normal-phase column chromatography (ethyl acetate: petroleum ether = 5% to 10%) to obtain compound **63-3** (450 mg, yield: 65%) as a white solid. MS (ESI, m/z): 544.2 $[M+H]^+$.

Compound **63-3** (450 mg, 0.829 mmol) was dissolved in ethanol (4 mL), and sodium borohydride (35 mg, 0.912 mmol) was added at 0°C. The reaction was carried out at room temperature for 2 hours, then diluted with 1 N hydrochloric acid aqueous solution (30 mL) and extracted with ethyl acetate (30 mL × 3). The organic phase was dried over anhydrous sodium sulfate, filtered, and concentrated. The resulting crude product was purified by normal-phase column chromatography (ethyl acetate: petroleum ether = 30% to 100%) to obtain compound 63-4 (400 mg, yield: 89%) as a white solid. MS (ESI, m/z): 546.2 $[M+H]^+$.

Compound **63-4** (200 mg, 0.366 mmol) was dissolved in dichloromethane (3 mL), followed by the addition of trimethyloxonium tetrafluoroborate (108 mg, 0.733 mmol), proton sponge (156 mg, 0.733 mmol), and molecular sieves (200 mg). The reaction was carried out at room temperature overnight. Water (30 mL) was added for dilution, followed by extraction with ethyl acetate (30 mL × 3). The organic phase was dried over anhydrous sodium sulfate, filtered, and concentrated. The resulting crude product was purified by normal-phase column chromatography (ethyl acetate: petroleum ether = 30% to 100%) to obtain compound **63-5** (180 mg, yield: 91%) as a white solid. MS (ESI, m/z): 560.2 $[M+H]^+$.

To a reaction tube, compound **63-5** (180 mg, 0.330 mmol), lithium hydroxide (40 mg, 1.65 mmol), tetrahydrofuran (3 mL), methanol (3 mL), and water (3 mL) were added. The reaction was carried out at 50°C for 2 hours. After the reaction mixture was rotary evaporated to remove the solvent, the resulting crude product was purified by reverse-phase column chromatography [acetonitrile/water (0.05% formic acid) = 0% to 100%] to obtain compound **63** (110 mg, yield: 61%) as a white solid. MS (ESI, m/z): 532.2 $[M+H]^+$.

$^1H$ NMR (400 MHz, DMSO-$d_6$) δ 8.15 (s, 1H), 7.81 (s, 1H), 7.73 - 7.66 (m, 1H), 7.61 - 7.56 (m, 3H), 7.36 (s, 1H), 7.30 - 7.26 (m, 1H), 7.24 - 7.20 (m, 1H), 4.82 - 4.70 (m, 3H), 4.15 - 4.02 (m, 2H), 3.92 (s, 3H), 3.47 (s, 3H), 2.27 - 2.20 (m, 1H), 2.19 - 2.11 (m, 2H), 2.07 - 1.92 (m, 4H), 1.64 (s, 9H).

**Example 64:**

Synthetic Route:

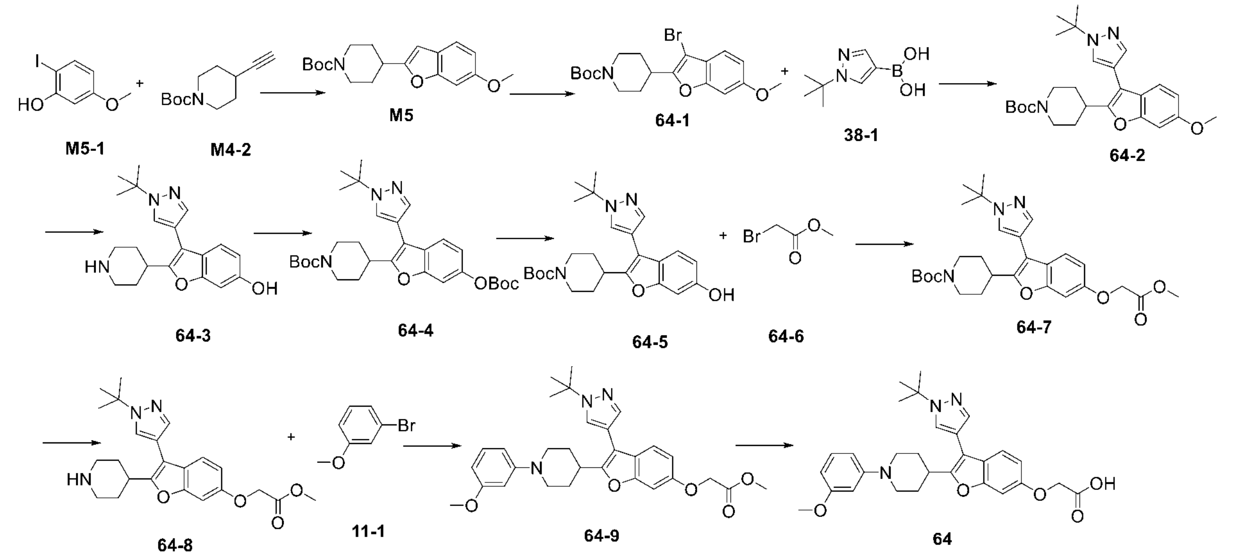

M5-1 M4-2 M5 64-1 38-1 64-2 64-3 64-4 64-5 64-6 64-7 64-8 11-1 64-9 64

Intermediate **M5** was synthesized by referring to the synthetic route of intermediate **M1**. Then, referring to the synthetic route of compound **38**, compound **12-1** was replaced with compound **64-1** to obtain compound **64-2** (60 mg, 1.32 mmol). Compound **64-2** (60 mg, 1.32 mmol) was added to boron tribromide (4 mL), and the reaction mixture was stirred at 0°C for 2 hours, and then quenched with saturated sodium bicarbonate (20 mL). The mixture was extracted with ethyl acetate (20 mL × 3), dried over magnesium sulfate, filtered, and the organic phase was concentrated. The resulting crude product was purified by normal-phase column chromatography (ethyl acetate: petroleum ether = 5% to 20%) to obtain compound **64-3** (40 mg, yield: 89%) as a yellow oil. MS (ESI, m/z): 340.2 $[M+H]^+$.

Compound **64-3** (30 mg, 0.09 mmol), di-*tert*-butyl dicarbonate (39 mg, 0.18 mmol), 4-dimethylaminopyridine (2.16 mg, 0.02 mmol), and dichloromethane (5 mL) were stirred at room temperature for 16 hours. The reaction mixture was directly concentrated, and the resulting crude product was purified by normal-phase column chromatography (ethyl acetate: petroleum ether = 0% to 100%) to obtain compound **64-4** (40 mg, yield: 84%) as a yellow oil. MS (ESI, m/z): 484.2 $[M-55]^+$.

Compound **64-4** (40 mg, 0.074 mmol), sodium hydroxide (6 mg, 0.15 mmol), and methanol (10 mL) were stirred at room temperature for 16 hours. The reaction mixture was directly concentrated, and the resulting crude product was purified by normal -phase column chromatography (ethyl acetate: petroleum ether = 0% to 100%) to obtain compound **64-5** (30 mg, yield: 92%) as a yellow oil. MS (ESI, m/z): 384.2 $[M-55]^+$.

Compound **64-5** (30 mg, 0.068 mmol), compound **64-6** (12.5 mg, 0.082 mmol), potassium carbonate (28.3 mg, 0.205 mmol), and *N,N*-dimethylformamide (2 mL) were stirred at room temperature for 16 hours. The reaction mixture was directly concentrated, and the resulting crude product was purified by normal-phase column chromatography (ethyl acetate: petroleum ether = 0% to 100%) to obtain compound **64-7** (32 mg, yield: 91%) as a yellow oil. MS (ESI, m/z): 456.2 $[M-55]^+$.

Compound **64-7** (32 mg, 0.063 mmol), trifluoroacetic acid (14.3 mg, 0.125 mmol), and dichloromethane (2 mL) were stirred at room temperature for 16 hours. The reaction mixture was directly concentrated, and the resulting crude product was purified by normal-phase column chromatography (methanol: dichloromethane = 5% to 30%) to obtain compound **64-8** (23 mg, yield: 89%) as a yellow oil. MS (ESI, m/z): 412.2 $[M+H]^+$.

Referring to the synthetic route of compound **38**, compound **38-3** was replaced with compound **64-8** to carry out the synthesis. The resulting crude product was purified by reverse-phase column chromatography [acetonitrile/water (0.05% ammonia) = 0% to 100%] to obtain compound **64** (14 mg, yield: 20%) as a white solid. MS (ESI, m/z): 504.3 $[M+H]^+$.

$^1H$ NMR (400 MHz, MeOD) δ 8.01 - 7.91 (m, 1H), 7.71 (s, 1H), 7.43 (d, *J* = 8.8Hz, 1H), 7.17 (t, *J* = 8.0Hz, 1H), 7.06 (d, *J* = 2.0Hz, 1H), 6.99 - 6.90 (m, 1H), 6.69 - 6.63 (m, 1H), 6.61 - 6.56 (m, 1H), 6.49 - 6.43 (m, 1H), 4.58 (s, 2H), 3.86 - 3.77 (m, 5H), 3.17 - 3.07 (m, 1H), 2.90 - 2.80 (m, 2H), 2.26 - 2.12 (m, 2H), 2.01 - 1.90 (m, 2H), 1.68 (s, 9H).

**Example 65**:

Synthetic Route:

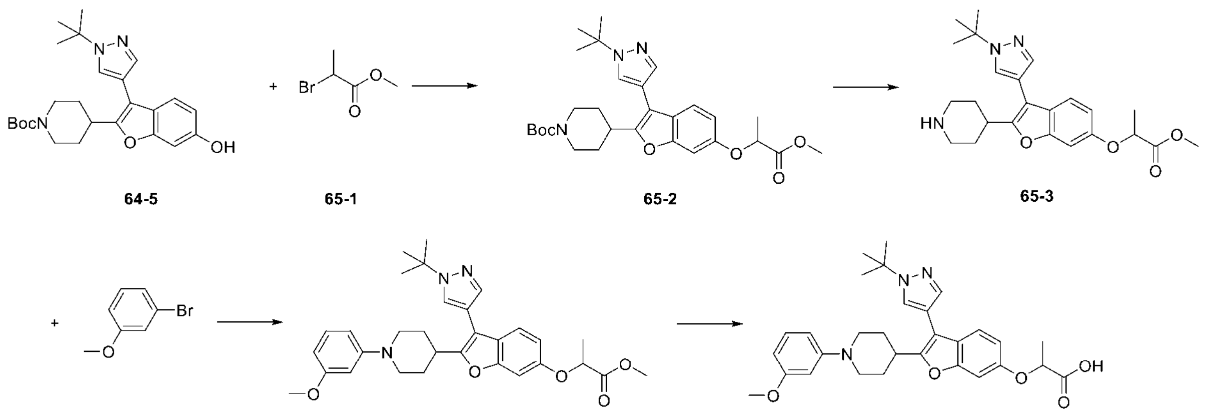

64-5 65-1 65-2 65-3

11-1 65-4 65

Referring to the synthetic route of compound **64**, compound **64-6** was replaced with compound **65-1** to carry out the synthesis. The resulting crude product was purified by reverse-phase column chromatography [acetonitrile/water (0.05% ammonia) = 0% to 100%] to obtain compound **65** (2.4 mg, yield: 8%) as a white solid. MS (ESI, m/z): 518.3 $[M+H]^+$.

$^1H$ NMR (400 MHz, $CDCl_3$) δ 7.72 - 7.67 (m, 2H), 7.46 - 7.41 (m, 1H), 7.24 - 7.18 (m, 1H), 7.02 (s, 1H), 6.96 - 6.90 (m, 1H), 6.67 - 6.60 (m, 1H), 6.56 (s, 1H), 6.49 - 6.42 (m, 1H), 4.90 - 4.82 (m, 1H), 3.86 - 3.78 (m, 5H), 3.13 - 3.03 (m, 1H), 2.92 - 2.81 (m, 2H), 2.26 - 2.20 (m, 2H), 1.97 - 1.90 (m, 2H), 1.73 - 1.67 (m, 12H).

**Example 66:**

Synthetic Route:

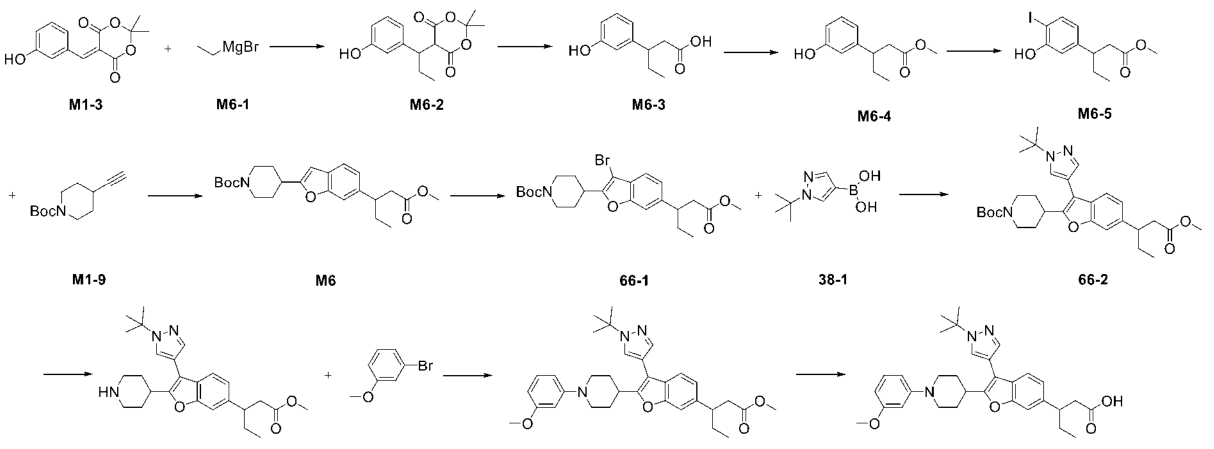

M1-3 M6-1 M6-2 M6-3 M6-4 M6-5

M1-9 M6 66-1 38-1 66-2

66-3 11-1 66-4 66

The synthesis route of intermediate M1 was referred to obtain intermediate M6. Then, referring to the synthetic route of compound 38, compound 12-1 was replaced with compound 66-1 to carry out the synthesis. The resulting crude product was purified by reverse-phase column chromatography [acetonitrile/water (0.05% ammonia) = 0% to 100%] to obtain compound 66 (65 mg, yield: 79%) as a white solid. MS (ESI, m/z): 530.3 $[M+H]^+$.

$^1H$ NMR (400 MHz, $CDCl_3$) δ 7.71 (s, 2H), 7.47 (d, *J* = 8.0 Hz, 1H), 7.29 (s, 1H), 7.24 - 7.17 (m, 1H), 7.13 - 7.07 (m, 1H), 6.66 - 6.59 (m, 1H), 6.58 - 6.52 (m, 1H), 6.48 - 6.42 (m, 1H), 3.86 - 3.80 (m, 5H), 3.20 - 3.02 (m, 2H), 2.92 - 2.79 (m, 2H), 2.77 - 2.64 (m, 2H), 2.33 - 2.15 (m, 2H), 2.01 - 1.88 (m, 2H), 1.87 - 1.73 (m, 2H), 1.68 (s, 9H), 0.82 (t, *J* = 7.2 Hz, 3H).

**Example 67:**

Synthetic Route:

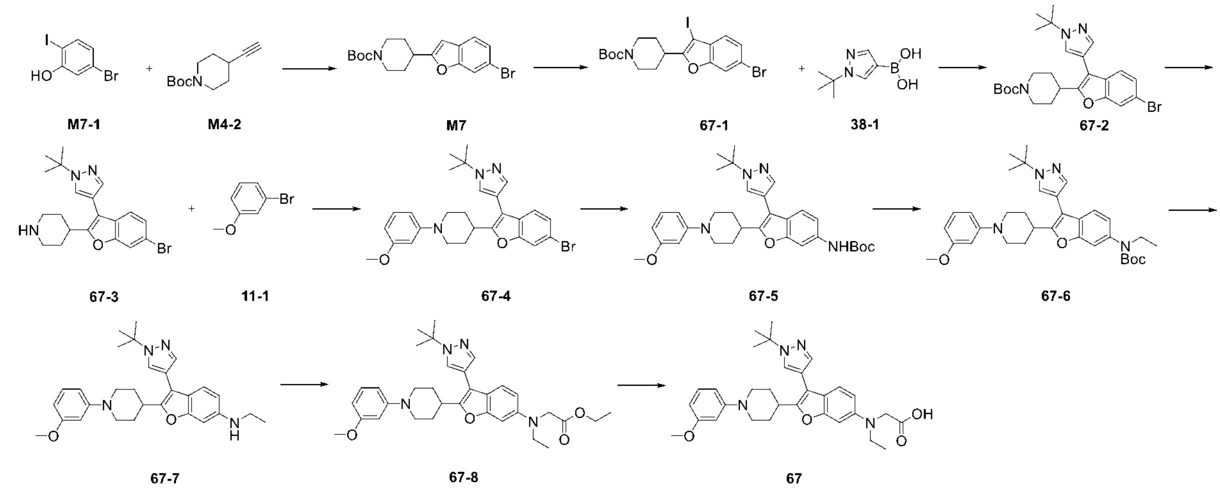

M7-1 M4-2 M7 67-1 38-1 67-2

67-3 11-1 67-4 67-5 67-6

67-7 67-8 67

Referring to the synthetic route of intermediate **M1,** intermediate **M7** was synthesized. Then, referring to the synthetic route of compound **38**, *N*-bromosuccinimide was replaced with N-iodosuccinimide to synthesize compound 67-4 (40 mg, 0.08 mmol). Compound **67-4** (40 mg, 0.08 mmol) and *tert*-butyl carbamate (19 mg, 0.16 mmol), 4,5-bis(diphenylphosphino)-9,9-dimethylxanthene (9 mg, 0.02 mmol), and cesium carbonate (51 mg, 0.16 mmol) were added to 1,4-dioxane (10 mL). The reaction mixture was stirred at 90°C for 4 hours and then directly concentrated. The resulting crude product was purified by normal-phase column chromatography (ethyl acetate: petroleum ether = 0% to 100%) to obtain compound **67-5** (40 mg, yield: 93%) as a yellow solid. MS (ESI, m/z): 545.2 $[M+H]^+$.

Under an ice bath, compound **67-5** (40 mg, 0.07 mmol), sodium hydride (2 mg, 0.09 mmol), and iodoethane (14 mg, 0.09 mmol) were added to tetrahydrofuran (5 mL), and the reaction mixture was stirred at room temperature for 16 hours. The reaction mixture was directly concentrated, and the resulting crude product was purified by normal-phase column chromatography (ethyl acetate: petroleum ether = 0% to 100%) to obtain compound **67-6** (40 mg, yield: 95%) as a colorless oil. MS (ESI, m/z): 573.3 $[M+H]^+$.

Compound **67-6** (40 mg, 0.07 mmol), trifluoroacetic acid (40 mg, 0.35 mmol), and dichloromethane (5 mL) were stirred at room temperature for 6 hours. The reaction mixture was directly concentrated, and the resulting crude product was purified by normal-phase column chromatography (ethyl acetate: petroleum ether = 0% to 100%) to obtain compound **67-7** (30 mg, yield: 91%) as a yellow oil. MS (ESI, m/z): 473.3 $[M+H]^+$.

Compound **67-7** (30 mg, 0.06 mmol), ethyl bromoacetate (21 mg, 0.13 mmol), sodium carbonate (20 mg, 0.19 mmol), and N,N-dimethylformamide (5 mL) were stirred at room temperature for 6 hours. The reaction mixture was diluted with water (20 mL) and extracted with ethyl acetate (20 mL × 3). The organic phase was dried over magnesium sulfate, filtered, and concentrated. The resulting crude product was purified by normal-phase column chromatography (ethyl acetate: petroleum ether = 0% to 100%) to obtain compound **67-8** (20 mg, yield: 56%) as a yellow solid. MS (ESI, m/z): 559.3 $[M+H]^+$.

Then, referring to the synthetic route of compound **38**, the synthesis was carried out, and the resulting crude product was purified by reverse-phase column chromatography [acetonitrile/water (0.05% ammonia) = 0% to 100%] to obtain compound 67 (4 mg, yield: 19%) as a white solid. MS (ESI, m/z): 531.2 $[M+H]^+$.

$^1$H NMR (400 MHz, DMSO-$d_6$) δ 8.02 (s, 1H), 7.70 (s, 1H), 7.34 (d, *J* = 8.4 Hz, 1H), 7.15 - 7.08 (m, 1H), 6.60 - 6.52 (m, 3H), 6.50 - 6.47 (m, 1H), 6.38 - 6.32 (m, 1H), 3.86 - 3.77 (m, 2H), 3.73 (s, 3H), 3.56 (s, 2H), 3.40 (q, *J* = 7.2 Hz, 2H), 3.16 - 3.06 (m, 1H), 2.91 - 2.81 (m, 2H), 2.02 - 1.89 (m, 2H), 1.88 - 1.79 (m, 2H), 1.59 (s, 9H), 1.10 (t, *J* = 7.2 Hz, 3H).

**Example 68:**

Synthetic Route:

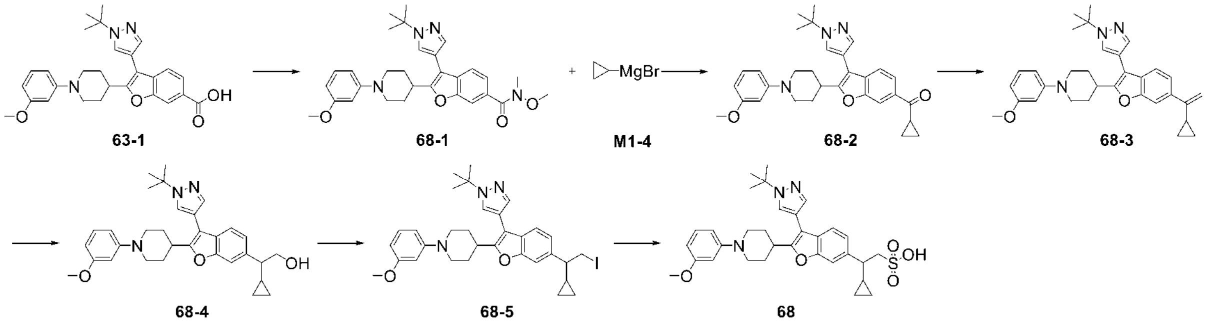

63-1 68-1 M1-4 68-2 68-3

68-4 68-5 68

Referring to the synthetic route of compound 63, compound 63-1 (2.6 g, 5.50 mmol) was synthesized. Compound 63-1 (2.6 g, 5.50 mmol), triethylamine (2.78 g, 27.48 mmol), and 2-(7-azabenzotriazol-1-yl)-*N*,*N*,*N*,*N*'-tetramethyluronium hexafluorophosphate (4.18 g, 10.99 mmol) were added to *N*,*N*-dimethylformamide (80 mL) and stirred. Dimethylhydroxylamine hydrochloride (808 mg, 8.25 mmol) was then added, and the reaction mixture was stirred at room temperature for 8 hours. After the reaction was completed, the reaction mixture was diluted with ethyl acetate (600 mL), and the organic phase was washed with water (180 mL × 6), dried over anhydrous sodium sulfate, filtered, and concentrated. The resulting crude product was purified by normal-phase column chromatography (ethyl acetate: petroleum ether = 0% to 100%) to obtain compound **68-1** (2.6 g, yield: 92%) as a yellow solid. MS (ESI, m/z): 517.2 $[M+H]^+$.

Under an ice bath, compound **68-1** (2.6 g, 5.04 mmol) was added to anhydrous tetrahydrofuran (50 mL) and stirred. Cyclopropylmagnesium bromide (1.0 M in tetrahydrofuran, 10.1 mL, 10.08 mmol) was then added, and the reaction mixture was stirred at room temperature for 3 hours. After the reaction was completed, the reaction was quenched with saturated sodium bicarbonate solution (2 mL), and a crude product was obtained by concentration. The resulting crude product was then purified by normal-phase column chromatography (ethyl acetate: petroleum ether = 5% to 30%) to obtain compound **68-2** (2.1 g, yield: 84%) as a yellow oil. MS (ESI, m/z): 498.3 $[M+H]^+$.

Methyltriphenylphosphonium bromide (4.31 g, 12.07 mmol) was added to anhydrous tetrahydrofuran (40 mL) and stirred. Under a nitrogen atmosphere at 0°C, *n*-butyllithium solution (2.5 M in tetrahydrofuran, 4.83 mL, 12.07 mmol) was added and reacted for 0.5 hours. Then, a solution of compound **68-2** (2.0 g, 4.0 mmol) in tetrahydrofuran (10 mL) was slowly added. The reaction was carried out at room temperature for 14 hours, quenched with water (1.5 mL), and concentrated. The resulting crude product was purified by normal-phase column chromatography (ethyl acetate: petroleum ether = 0% to 100%) to obtain compound **68-3** (1.8 g, yield: 90%) as a yellow oil. MS (ESI, m/z): 496.3 $[M+H]^+$.

Compound **68-3** (800 mg, 1.62 mmol) was added to tetrahydrofuran (40 mL) and stirred. At 0°C, borane-dimethyl sulfide complex (2.0 M in tetrahydrofuran, 2.43 mL, 4.85 mmol) was added, and the reaction was carried out at room temperature for 16 hours. Then, 6 M sodium hydroxide solution (1.62 mL, 9.70 mmol) and hydrogen peroxide (30% in water, 3.12 mL, 32.32 mmol) were slowly added, and the mixture was stirred at room temperature for 16 hours. After the reaction was completed, the reaction was quenched with saturated sodium thiosulfate solution (100 mL) and extracted with ethyl acetate (80 mL × 3). The combined organic phases were dried over anhydrous sodium sulfate and concentrated. The resulting crude product was purified by normal-phase column chromatography (ethyl acetate: petroleum ether = 0% to 100%) to obtain compound **68-4** (670 mg, yield: 80%) as a colorless oil. MS (ESI, m/z): 514.3 $[M+H]^+$.

Compound **68-4** (98 mg, 0.19 mmol), triphenylphosphine (75 mg, 0.29 mmol), and imidazole (19 mg, 0.29 mmol) were added to anhydrous tetrahydrofuran (10 mL) and stirred. Iodine (73 mg, 0.29 mmol) was added, and the reaction was carried out at room temperature for 16 hours. After the reaction was completed, the reaction mixture was concentrated, and the resulting crude product was purified by normal-phase column chromatography (ethyl acetate: petroleum ether = 0% to 100%) to obtain compound **68-5** (52 mg, yield: 44%) as a yellow oil. MS (ESI, m/z): 624.3 $[M+H]^+$.

Compound **68-5** (52 mg, 0.08 mmol) was added to ethanol (10 mL)/water (1 mL) and stirred. Sodium sulfite (158 mg, 1.25 mmol) was added, and the reaction mixture was stirred at 90°C for 72 hours. After the reaction was completed, the reaction mixture was concentrated, and the resulting crude product was purified by reverse-phase column chromatography [acetonitrile/water (0.05% formic acid) = 0% to 100%] to obtain compound **68** (6 mg, yield: 13%) as a yellow solid. MS (ESI, m/z): 578.2 $[M+H]^+$.

$^1H$ NMR (400 MHz, $CDCl_3$) δ 7.74 (s, 1H), 7.72 - 7.65 (m, 1H), 7.58 (s, 1H), 7.54 - 7.33 (m, 3H), 7.29 - 7.25 (m, 1H), 7.17 - 7.05 (m, 1H), 6.97 - 6.93 (m, 1H), 3.94 (s, 3H), 3.79 - 3.47 (m, 4H), 3.39 - 3.02 (m, 3H), 2.95 - 2.84 (m, 1H), 2.53 - 2.34 (m, 2H), 2.00 - 1.86 (m, 2H), 1.69 (s, 9H), 1.38 - 1.33 (m, 1H), 0.70 - 0.60 (m, 1H), 0.60 - 0.50 (m, 1H), 0.50 - 0.41 (m, 1H), 0.30 - 0.19 (m, 1H).

**Example 69:**

Synthetic Route:

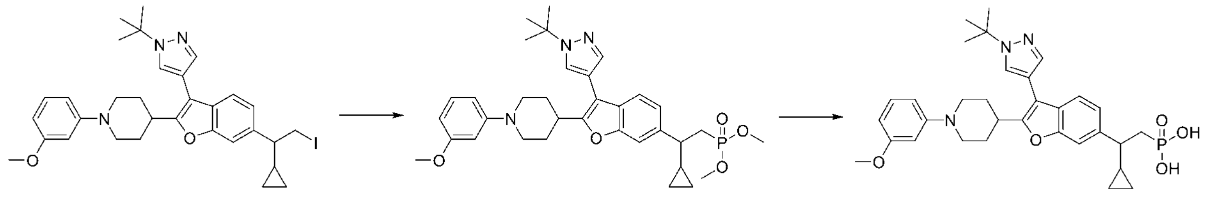

68-5 69-1 69

Referring to the synthetic route of compound **68**, compound **68-5** (300 mg, 0.48 mmol) was synthesized and added to trimethyl phosphite (2 mL) with stirring. The reaction was carried out in a sealed tube at 100°C under a nitrogen atmosphere for 18 hours. After the reaction was completed, the reaction mixture was concentrated, and the resulting crude product was purified by reverse-phase column chromatography [acetonitrile/water (0.05% formic acid) = 0% to 100%] to obtain compound **69-1** (40 mg, yield: 14%) as a yellow oil. MS (ESI, m/z): 606.2 $[M+H]^+$.

Compound **69-1** (40 mg, 0.07 mmol) was added to dichloromethane (10 mL) and stirred. Trimethylbromosilane (0.5 mL) was added, and the reaction mixture was stirred at room temperature for 16 hours. After the reaction was completed, the reaction mixture was concentrated, and the resulting crude product was purified by reverse-phase column chromatography [acetonitrile/water (0.05% formic acid) = 0% to 100%] to obtain compound **69** (1 mg, yield: 2%) as a white solid. MS (ESI, m/z): 578.2 $[M+H]^+$.

$^1H$ NMR (400 MHz, $CDCl_3$) δ 7.75 - 7.60 (m, 2H), 7.45 - 7.37 (m, 1H), 7.27 - 7.04 (m, 3H), 6.80 - 6.47 (m, 3H), 3.90 - 3.81 (m, 4H), 3.74 - 3.66 (m, 2H), 3.14 - 3.06 (m, 1H), 2.99 - 2.77 (m, 4H), 2.09 - 2.03 (m, 2H), 1.95 - 1.87 (m, 2H), 1.66 (s, 9H), 1.17 - 1.04 (m, 1H), 0.63 - 0.51 (m, 1H), 0.45 - 0.27 (m, 2H), 0.20 - 0.07 (m, 1H).

**Example 70:**

Synthetic Route:

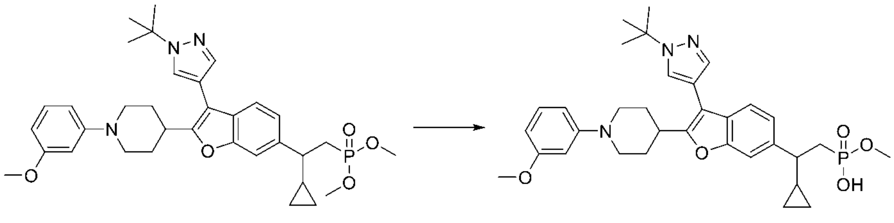

69-1 70

Referring to the synthetic route of compound **69**, compound **69-1** (25 mg, 0.04 mmol) was synthesized and stirred in a mixture of tetrahydrofuran (1 mL)/methanol (1 mL)/water (1 mL). Lithium hydroxide (30 mg, 1.24 mmol) was added, and the reaction was stirred at 70°C under a nitrogen atmosphere for 16 hours. After the reaction was completed, the reaction mixture was concentrated, and the resulting crude product was purified by reverse-phase column chromatography [acetonitrile/water (0.05% formic acid) = 0% to 100%] to obtain compound **70** (9 mg, yield: 38%) as a white solid. MS (ESI, m/z): 592.2 $[M+H]^+$.

$^1H$ NMR (400 MHz, $CDCl_3$) δ 7.71 (d, *J* = 5.6 Hz, 2H), 7.50 - 7.43 (m, 1H), 7.33 (s, 1H), 7.25 - 7.16 (m, 1H), 7.15 - 7.06 (m, 1H), 6.65 - 6.59 (m, 1H), 6.55 (s, 1H), 6.50 - 6.41 (m, 1H), 3.82 - 3.78 (m, 5H), 3.47 - 3.34 (m, 3H), 3.12 - 3.07 (m, 1H), 2.89 - 2.79 (m, 2H), 2.45 - 2.39 (m, 1H), 2.30 - 2.17 (m, 4H), 1.99 - 1.87 (m, 2H), 1.68 (s, 9H), 1.18 - 1.04 (m, 1H), 0.66 - 0.51 (m, 1H), 0.46 - 0.29 (m, 2H), 0.16 - 0.09 (m, 1H).

**Example 71:**

Synthetic Route:

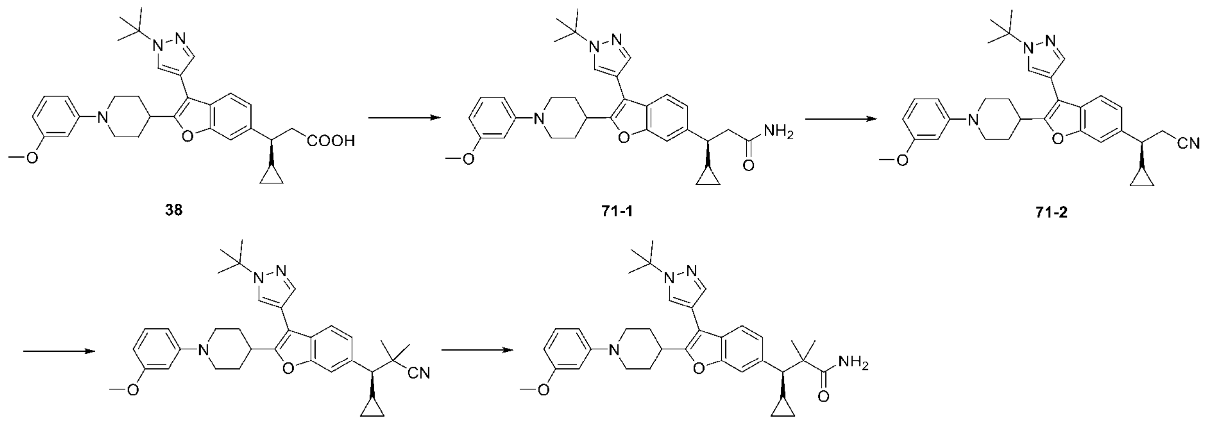

38 71-1 71-2

71-3 71

Compound **38** (304 mg, 0.56 mmol), HATU (426 mg, 1.12 mmol, 2.0 eq), ammonium chloride (90 mg, 1.68 mmol, 3.0 eq), and triethylamine (329 mg, 3.36 mmol, 6.0 eq) were added to dichloromethane (10 mL). The reaction was carried out at room temperature overnight. After the reaction was completed, water (10 mL) was added, and the mixture was extracted with ethyl acetate (20 mL × 3). The combined organic phases were dried over anhydrous sodium sulfate and concentrated. The resulting crude product was purified by normal-phase column chromatography (ethyl acetate: petroleum ether = 0% to 100%) to obtain compound **71-1** (263 mg, yield: 87%) as a white solid. MS (ESI, m/z): 541.3 $[M+H]^+$.

To a reaction tube, compound **71-1** (263 mg, 0.49 mmol), triethylamine (303 mg, 3.0 mmol), and dry dichloromethane (10 mL) were added. Trifluoroacetic anhydride (315 mg, 1.5 mmol) was slowly added dropwise at room temperature, and the reaction was carried out at room temperature for 4 hours. After the reaction was completed, water (10 mL) was added, and the reaction mixture was extracted with ethyl acetate (20 mL × 3). The combined organic phases were dried over anhydrous sodium sulfate and concentrated. The resulting crude product was purified by normal-phase column chromatography (ethyl acetate: petroleum ether = 0% to 100%) to obtain compound **71-2** (217 mg, yield: 86%) as a white solid. MS (ESI, m/z): 523.3 $[M+H]^+$.

To a reaction tube, compound **71-2** (217 mg, 0.41 mmol) and tetrahydrofuran (6 mL) were added under a nitrogen atmosphere. After cooling to -80°C, 1 M solution of lithium bis(trimethylsilyl)amide (1 mg, 1.0 mmol, 2.5 eq) was slowly added dropwise. The reaction mixture was stirred for 1 hour, and then iodomethane (233 mg, 1.64 mmol, 4.0 eq) was added. The reaction was carried out at room temperature for 24 hours. The reaction mixture was directly concentrated, and the resulting crude product was purified by normal-phase column chromatography (ethyl acetate: petroleum ether = 0% to 100%) to obtain compound **71-3** (118 mg, yield: 52%) as a white solid. MS (ESI, m/z): 551.4 $[M+H]^+$.

To a reaction tube, compound **71-3** (41 mg, 0.075 mmol), dioxane (2 mL), and water (2 mL) were added. Then, KOH (84 mg, 1.5 mmol) was added. The reaction was carried out at 140°C overnight. Dilute hydrochloric acid was added to adjust the pH to 3, and the mixture was extracted with ethyl acetate (20 mL × 3). The combined organic phases were dried over anhydrous sodium sulfate and concentrated. The resulting crude product was purified by reverse-phase column chromatography [acetonitrile/water (0.05% formic acid) = 0% to 100%] to obtain compound **71** (8 mg, yield: 19%) as a white solid. MS (ESI, m/z): 569.3 $[M+H]^+$.

$^1H$ NMR (400 MHz, $CDCl_3$) δ 7.70 (d, $J$ = 8.0 Hz, 2H), 7.43 (d, $J$ = 7.6 Hz, 1H), 7.33 (s, 1H), 7.25 - 7.16 (m, 1H), 7.14 - 7.08 (m, 1H), 6.67 - 6.57 (m, 1H), 6.53 (s, 1H), 6.47 - 6.37 (m, 1H), 5.50 (s, 1H), 5.33 (s, 1H), 3.88 - 3.76 (m, 5H), 3.17 - 3.05 (m, 1H), 2.91 - 2.79 (m, 2H), 2.40 - 2.33 (m, 1H), 2.28 - 2.15 (m, 2H), 1.98 - 1.87 (m, 2H), 1.67 (s, 9H), 1.33 (s, 3H), 1.35 - 1.24 (m, 1H), 1.16 (s, 3H), 0.80 -0.72 (m, 1H), 0.57 - 0.49 (m, 1H), 0.46 - 0.35 (m, 1H), -0.09 - -0.18 (m, 1H).

**Example 72:**

Synthetic Route:

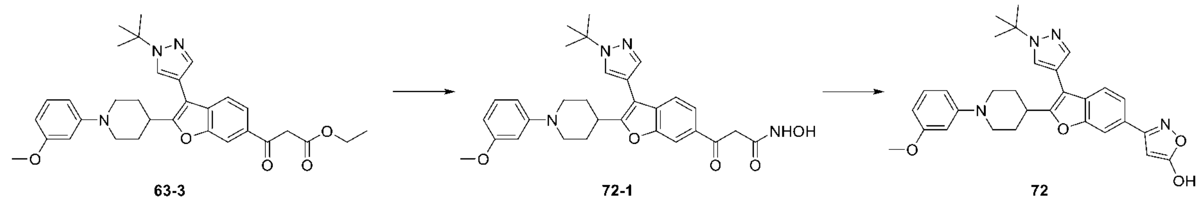

63-3 72-1 72

Referring to the synthetic route of compound **63**, compound **63-3** (50 mg, 0.094 mmol) was synthesized. Compound **63-3** (50 mg, 0.094 mmol) and hydroxylamine hydrochloride (32 mg, 0.47 mmol) were added to 2 N sodium hydroxide aqueous solution (20 mL). The reaction was carried out at room temperature for 1 hour. After the reaction was completed, the reaction mixture was concentrated to obtain crude compound **72-1** (50 mg, yield: 99%). MS (ESI, m/z): 531.2 $[M+H]^+$.

Compound **72-1** (50 mg, 0.094 mmol) was added to concentrated hydrochloric acid (2 mL) and reacted at room temperature for 16 hours. After the reaction was completed, the reaction mixture was concentrated, and the resulting crude product was purified by reverse-phase column chromatography [acetonitrile/water (0.05% formic acid) = 0% to 100%] to obtain compound 72 (6 mg, yield: 3%) as a white solid. MS (ESI, m/z): 513.2 $[M+H]^+$.

$^1$H NMR (400 MHz, DMSO-$d_6$) $\delta$ 8.21 (s, 1H), 8.13 (s, 1H), 7.76 (s, 1H), 7.68 - 7.59 (m, 2H), 7.54 (d, *J* = 8.0 Hz, 1H), 7.16 - 7.09 (m, 1H), 6.63 - 6.55 (m, 1H), 6.50 (s, 1H), 6.39 - 6.32 (m, 1H), 3.88 - 3.80 (m, 2H), 3.73 (s, 3H), 3.24 - 3.18 (m, 1H), 2.94 - 2.84 (m, 2H), 2.04 - 1.88 (m, 4H), 1.61 (s, 9H).

**Example 73:**

Synthetic Route:

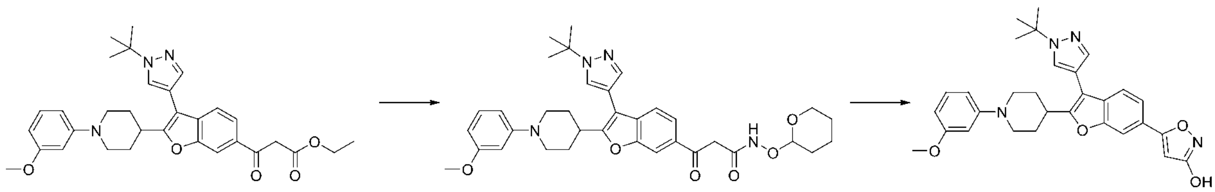

63-3 73-1 73

Referring to the synthetic route of compound **72**, hydroxylamine hydrochloride was replaced with O-(tetrahydro-2*H*-pyran-2-yl)hydroxylamine to synthesize compound **73-1** (200 mg, 0.33 mmol). Compound **73-1** (200 mg, 0.33 mmol) was added to 2 N hydrochloric acid (1 mL) and methanol (10 mL). The reaction was carried out at room temperature for 1 hour. After the reaction was completed, the reaction mixture was concentrated, and the resulting crude product was purified by reverse-phase column chromatography [acetonitrile/water (0.05% formic acid) = 0% to 100%] to obtain compound **73** (7 mg, yield: 3%) as a white solid. MS (ESI, m/z): 513.2 $[M+H]^+$.

$^1$H NMR (400 MHz, DMSO-$d_6$) $\delta$ 11.37 (s, 1H), 8.17 (s, 1H), 8.04 (s, 1H), 7.82 - 7.77 (m, 2H), 7.73 - 7.67 (m, 1H), 7.16 - 7.09 (m, 1H), 6.63 - 6.56 (m, 2H), 6.52 - 6.48 (m, 1H), 6.40 - 6.32 (m, 1H), 3.89 - 3.80 (m, 2H), 3.73 (s, 3H), 3.32 - 3.22 (m, 1H), 2.95 - 2.86 (m, 2H), 2.03 - 1.89 (m, 4H), 1.62 (s, 9H).

**Example 73:**

Synthetic Route:

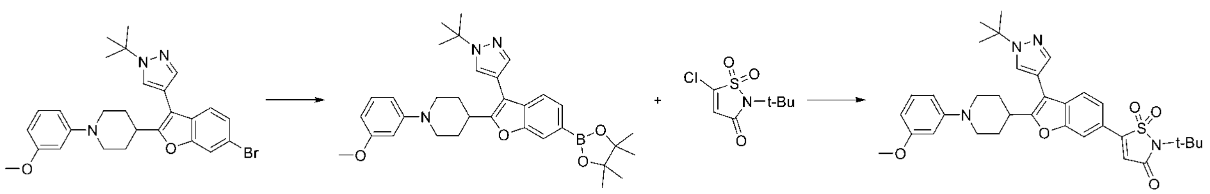

67-4 74-1 74-2 74

Referring to the synthetic route of compound **67**, compound **67-4** (360 mg, 0.70 mmol) was synthesized. Compound **67-4** (360 mg, 0.70 mmol), bis(pinacolato)diboron (198 mg, 0.77 mmol), [1,1'-bis(diphenylphosphino)ferrocene]palladium(II) dichloride dichloromethane complex (102.75 mg, 0.14 mmol), and potassium acetate (208.45 mg, 2.12 mmol) were added to 1,4-dioxane (10 mL). The reaction was carried out at 90°C for 16 hours. After the reaction was completed, the reaction mixture was concentrated, and the resulting crude product was purified by normal-phase column chromatography (ethyl acetate: petroleum ether = 0% to 100%) to obtain compound **74-1** (220 mg, yield: 50%) as a yellow oil. MS (ESI, m/z): 556.3 $[M+H]^+$.

Compound **74-1** (90 mg, 0.16 mmol), compound **74-2** (36.6 mg, 0.16 mmol), and potassium carbonate (67.2 mg, 0.48 mmol) were added to 1,4-dioxane (5 mL). The reaction was carried out at 80°C for 12 hours. After the reaction was completed, the reaction mixture was concentrated, and the resulting crude product was purified by reverse-phase column chromatography [acetonitrile/water (0.05% formic acid) = 0% to 100%] to obtain compound **74** (3 mg, yield: 3%) as a white solid. MS (ESI, m/z): 617.3 $[M+H]^+$.

$^1H$ NMR (400 MHz, DMSO-$d_6$) $\delta$ 8.20 (s, 1H), 8.15 - 8.12 (m, 1H), 7.95 - 7.91 (m, 1H), 7.82 (s, 1H), 7.80 - 7.76 (m, 1H), 7.51 (s, 1H), 7.15 - 7.10 (m, 1H), 6.61 - 6.56 (m, 1H), 6.52 - 6.49 (m, 1H), 6.39 - 6.35 (m, 1H), 3.88 - 3.80 (m, 2H), 3.73 (s, 3H), 3.30 - 3.26 (m, 1H), 2.95 - 2.86 (m, 2H), 2.03 - 1.91 (m, 4H), 1.66 (s, 9H), 1.62 (s, 9H).

**Example 75:**

Synthetic Route:

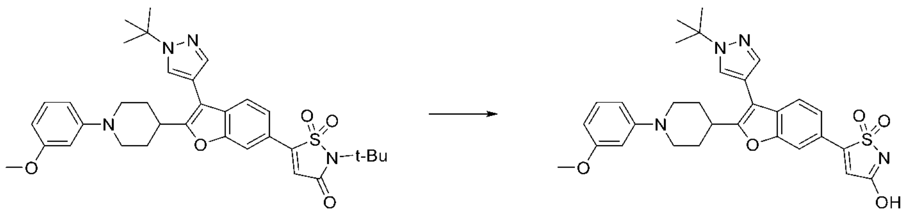

74 75

Compound **74** (20 mg, 0.03 mmol) was added to trifluoroacetic acid (3 mL) and reacted at 100°C for 12 hours. After the reaction was completed, the reaction mixture was concentrated, and the resulting crude product was purified by reverse-phase column chromatography [acetonitrile/water (0.05% formic acid) = 0% to 100%] to obtain compound **75** (6 mg, yield: 31%) as a white solid. MS (ESI, m/z): 561.2 $[M+H]^+$.

$^1H$ NMR (400 MHz, DMSO-$d_6$) $\delta$ 8.18 (s, 1H), 8.08 - 8.03 (m, 1H), 7.85 - 7.76 (m, 3H), 7.18 - 7.12 (m, 1H), 7.01 (s, 1H), 6.65 - 6.60 (m, 1H), 6.55 (s, 1H), 6.43 - 6.37 (m, 1H), 3.88 - 3.80 (m, 2H), 3.74 (s, 3H), 3.30 - 3.27 (m, 1H), 2.99 - 2.91 (m, 2H), 2.06 - 1.94 (m, 4H), 1.62 (s, 9H).

**Example 76:**

Synthetic Route:

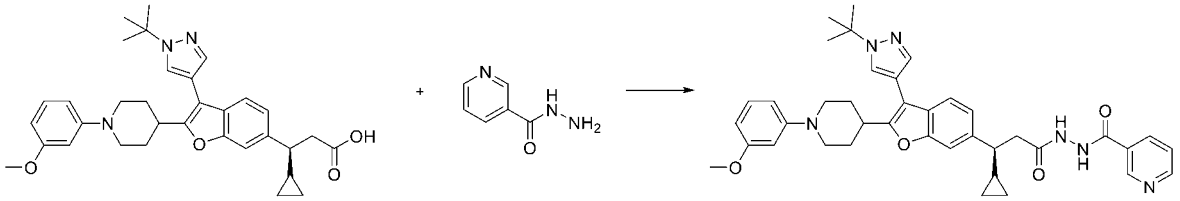

38 76-1 76

Compound **38** (325 mg, 0.6 mmol), compound **76-1** (123 mg, 0.9 mmol), 2-(7-azabenzotriazol-1-yl)-*N,N,N',N'*-tetramethyluronium hexafluorophosphate (456 mg, 1.2 mmol), and triethylamine (182 mg, 1.8 eq) were added to *N,N*-dimethylformamide (10 mL). The reaction was carried out at room temperature for 12 hours. After the reaction was completed, the reaction mixture was concentrated, and the resulting crude product was purified by reverse-phase column chromatography [acetonitrile/water (0.05% formic acid) = 0% to 100%] to obtain compound **75** (187 mg, yield: 47%) as a

white solid. MS (ESI, m/z): 661.2 $[M+H]^+$.

$^1$H NMR (400 MHz, $CDCl_3$) δ 9.91 (s, 1H), 9.06 (s, 1H), 8.99 (s, 1H), 8.68 (d, *J* = 3.6 Hz, 1H), 8.10 - 8.01 (m, 1H), 7.73 (s, 1H), 7.70 (s, 1H), 7.49 - 7.42 (m, 1H), 7.33 (s, 1H), 7.32 - 7.28 (m, 1H), 7.26 - 7.17 (m, 1H), 7.14 - 7.08 (m, 1H), 6.71 - 6.65 (m, 1H), 6.64 - 6.61 (m, 1H), 6.53 - 6.49 (m, 1H), 3.85 - 3.77 (m, 5H), 3.19 - 3.07 (m, 1H), 2.98 - 2.71 (m, 4H), 2.59 - 2.52 (m, 1H), 2.33 - 2.18 (m, 2H), 1.99 - 1.89 (m, 2H), 1.69 (s, 9H), 1.15 - 1.02 (m, 1H), 0.70 - 0.57 (m, 1H), 0.50 - 0.39 (m, 1H), 0.38 - 0.27 (m, 1H), 0.24 - 0.14 (m, 1H).

**Example 77**:

Synthetic Route:

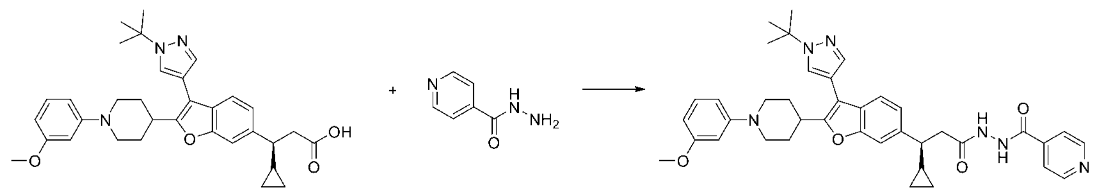

38 77-1 77

Referring to the synthetic route of compound **76**, compound **76-1** was replaced with compound **77-1** to carry out the synthesis. The resulting crude product was purified by reverse-phase column chromatography [acetonitrile/water (0.05% ammonia) = 0% to 100%] to obtain compound **77** (118 mg, yield: 65%) as a white solid. MS (ESI, m/z): 661.2 $[M+H]^+$.

$^1$H NMR (400 MHz, $CDCl_3$) δ 9.67 (s, 1H), 8.73 - 8.63 (m, 3H), 7.72 (s, 1H), 7.70 (s, 1H), 7.58 (d, *J* = 5.6 Hz, 2H), 7.50 - 7.44 (m, 1H), 7.34 (s, 1H), 7.26 - 7.19 (m, 1H), 7.15 -7.09 (m, 1H), 6.71 - 6.65 (m, 1H), 6.63 - 6.57 (m, 1H), 6.54 - 6.47 (m, 1H), 3.84 - 3.79 (m, 5H), 3.18 - 3.08 (m, 1H), 2.95 - 2.69 (m, 4H), 2.60 - 2.51 (m, 1H), 2.33 - 2.18 (m, 2H), 1.96 - 1.91 (m, 2H), 1.69 (s, 9H), 1.15 - 1.04 (m, 1H), 0.71 - 0.60 (m, 1H), 0.52 - 0.41 (m, 1H), 0.39 - 0.30 (m, 1H), 0.26 - 0.16 (m, 1H).

**Example 78:**

Synthetic Route:

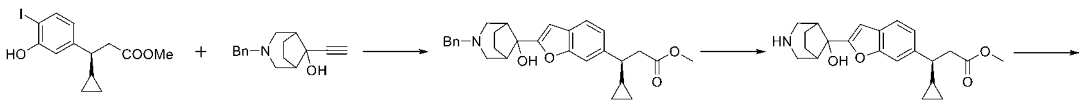

78-1 78-2 78-3 78-4

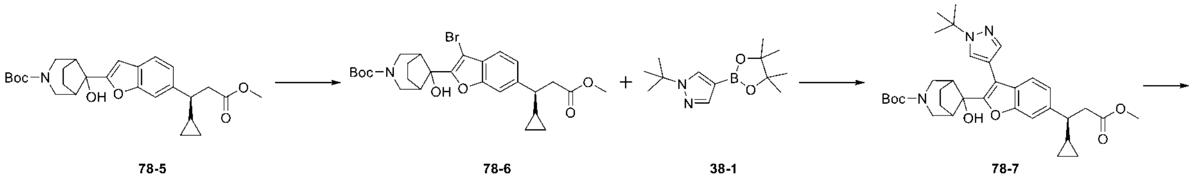

78-5 78-6 38-1 78-7

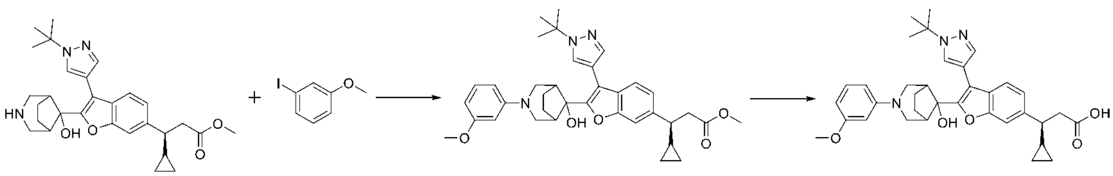

78-8 78-9 78-10 78

To a reaction flask, compound **78-1** (5.53 g, 16.2 mmol), compound **78-2** (3.91 g, 16.2 mmol), bis(triphenylphosphine)palladium(II) chloride (562 mg, 0.8 mmol), copper(I) iodide (309 mg, 1.6 mmol), triethylamine (4.9 g, 48.6 mmol), and dry acetonitrile (60 mL) were added. The reaction was then carried out at 85°C overnight. After the reaction was completed, saturated ammonium chloride solution (10 mL) was added to quench the reaction, and the mixture was

extracted with ethyl acetate (50 mL × 3). The combined organic phases were dried over anhydrous sodium sulfate and concentrated. The resulting crude product was purified by normal-phase column chromatography (ethyl acetate: petroleum ether = 0% to 100%) to obtain compound **78-3** (5.65 g, yield: 76%) as a white solid. MS (ESI, m/z): 460.2 $[M+H]^+$.

To a 100 mL reaction flask, compound **78-3** (5.65 g, 12.3 mmol), 10% palladium hydroxide on carbon (200 mg), Raney nickel (400 mg), tetrahydrofuran (25 mL), and isopropanol (25 mL) were added. The reaction was then carried out at 60°C overnight. After the reaction was completed, the reaction mixture was quenched with saturated ammonium chloride solution (10 mL) at 0°C and directly concentrated. The resulting crude product was purified by normal-phase column chromatography (ethyl acetate: petroleum ether = 0% to 100%) to obtain compound **78-4** (4.14 g, yield: 91%) as a white solid. MS (ESI, m/z): 370.3 $[M+H]^+$.

To a reaction flask, compound **78-4** (4.14 g, 11.3 mmol), triethylamine (2.3 g, 22.6 mmol), and dichloromethane (50 mL) were added. Di-tert-butyl dicarbonate (4.9 g, 22.6 mmol) was slowly added, and the reaction was carried out at room temperature overnight. After the reaction was completed, saturated ammonium chloride solution (10 mL) was added to quench the reaction, and the mixture was extracted with ethyl acetate (50 mL × 3). The combined organic phases were dried over anhydrous sodium sulfate and concentrated. The resulting crude product was purified by normal-phase column chromatography (ethyl acetate: petroleum ether = 0% to 100%) to obtain compound **78-5** (4.87 g, yield: 92%) as a white solid. MS (ESI, m/z): 470.2 $[M+H]^+$.

To a reaction flask, compound **78-5** (4.37 g, 9.3 mmol) and acetonitrile (50 mL) were added. N-Bromosuccinimide (2.5 g, 14 mmol) was then rapidly added, and the reaction was carried out at room temperature for 30 minutes. After the reaction was completed, saturated ammonium chloride solution (10 mL) was added to quench the reaction, and the mixture was extracted with ethyl acetate (50 mL × 3). The combined organic phases were dried over anhydrous sodium sulfate and concentrated. The resulting crude product was purified by normal-phase column chromatography (ethyl acetate: petroleum ether = 0% to 100%) to obtain compound **78-6** (4.25 g, yield: 83%) as a white solid. MS (ESI, m/z): 548.3 $[M+H]^+$.

To a reaction flask, compound **78-6** (1.94 g, 3.5 mmol), compound **38-1** (1.1 g, 4.2 mmol), tetrakis(triphenylphosphine)palladium (202 mg, 0.18 mmol), potassium phosphate (2.23 g, 10.5 mmol), dioxane (30 mL), and water (10 mL) were added under a nitrogen atmosphere. The reaction mixture was stirred at 100°C for 24 hours, then cooled to room temperature and diluted with ethyl acetate (100 mL). The organic phase was washed with water, dried over anhydrous sodium sulfate, filtered, and concentrated. The resulting crude product was purified by normal-phase column chromatography (ethyl acetate: petroleum ether = 0% to 100%) to obtain compound **78-7** (4.25 g, yield: 83%) as a white solid. MS (ESI, m/z): 592.3 $[M+H]^+$.

To a reaction flask, compound **78-7** (1.16 g, 1.96 mmol) and dichloromethane (30 mL) were added, followed by the addition of trifluoroacetic acid (3 mL). The reaction mixture was stirred overnight at room temperature, and the pH was adjusted to approximately 8 with saturated sodium bicarbonate aqueous solution. The mixture was then extracted with dichloromethane (50 mL × 3). The combined organic phases were dried over anhydrous sodium sulfate and concentrated. The resulting crude product was purified by normal-phase column chromatography (ethyl acetate: petroleum ether = 0% to 100%) to obtain compound **78-**8 (876 mg, yield: 91%) as a white solid. MS (ESI, m/z): 492.3 $[M+H]^+$.

To a reaction flask, compound **78-8** (536 mg, 1.1 mmol), [dicyclohexyl[3-(1-methylethoxy)-2',4',6'-tris(1-methylethyl)[1,1'-biphenyl]-2-yl]phosphine-κP](methanesulfonato-κO)[2'-(methylamino-κN)[1,1'-biphenyl]-2-yl-κC]palladium (EPhos Pd G4) (10 mg, 0.011 mmol), dicyclohexyl(3-isopropoxy-2',4',6'-triisopropyl-[1,1'-biphenyl]-2-yl)phosphine (11.8 mg, 0.022 mmol), 3-iodoanisole **78-9** (309 mg, 1.3 mmol), cesium carbonate (1.1 g, 3.3 mmol), and dioxane (10 mL) were added. The reaction was then carried out at 100°C overnight. After the reaction was completed, saturated ammonium chloride solution (10 mL) was added to quench the reaction, and the mixture was extracted with ethyl acetate (20 mL × 3). The combined organic phases were dried over anhydrous sodium sulfate and concentrated. The resulting crude product was purified by normal-phase column chromatography (ethyl acetate: petroleum ether = 0% to 100%) to obtain compound **78-10** (96 mg, yield: 45%) as a white solid. MS (ESI, m/z): 598.3 $[M+H]^+$.

To a reaction flask, compound **78-10** (96 mg, 0.16 mmol), tetrahydrofuran (3 mL), and methanol (3 mL) were added. 1 mL of sodium hydroxide (64 mg, 1.6 mmol, 10.0 eq) aqueous solution was then added dropwise to the reaction. The reaction was carried out at room temperature overnight. Dilute hydrochloric acid was added to adjust the pH to 3, followed by extraction with ethyl acetate (10 mL × 3). The combined organic phases were dried over anhydrous sodium sulfate and concentrated. The resulting crude product was purified by reverse-phase column chromatography [acetonitrile/water (0.05% ammonia) = 0% to 100%] to obtain compound **78** (32 mg, yield: 34%) as a white solid. MS (ESI, m/z): 584.3 $[M+H]^+$.

$^1H$ NMR (400 MHz, $CDCl_3$) δ 7.95 (s, 1H), 7.85 (s, 1H), 7.52 - 7.44 (m, 1H), 7.35 (s, 1H), 7.20 - 7.11 (m, 2H), 6.55 - 6.47 (m, 1H), 6.42 (s, 1H), 6.38 - 6.30 (m, 1H), 3.80 (s, 3H), 3.47 - 3.37 (m, 3H), 2.86 - 2.76 (m, 4H), 2.52 - 2.42 (m, 1H), 1.70 - 1.55 (m, 15H), 1.12 - 1.02 (m, 1H), 0.64 - 0.56 (m, 1H), 0.46 - 0.38 (m, 1H), 0.35 - 0.27 (m, 1H), 0.21 - 0.13 (m, 1H).

**Example 79:**

Synthetic Route:

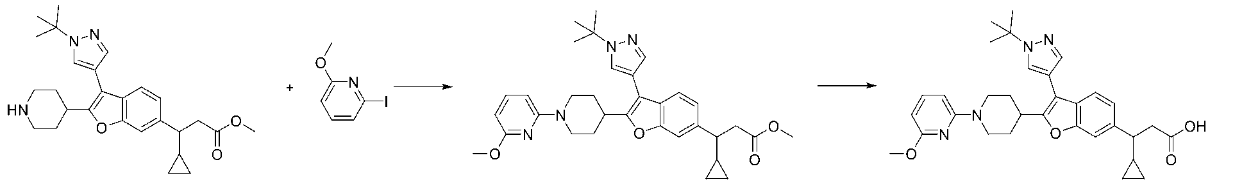

39-3 79-1 79-2 79

Referring to the synthetic route of compound 39, compound 39-4 was replaced with compound 79-1 to carry out the synthesis. The resulting crude product was purified by reverse-phase column chromatography [acetonitrile/water (0.05% formic acid) = 0% to 100%] to obtain compound 79 (8 mg, yield: 41%) as a white solid. MS (ESI, m/z): 543.2 [M+H]$^+$.

$^1$H NMR (400 MHz, $CDCl_3$) δ 7.74 - 7.69 (m, 2H), 7.50 - 7.41 (m, 2H), 7.34 (s, 1H), 7.16 - 7.11 (m, 1H), 6.27 - 6.21 (m, 1H), 6.12 - 6.06 (m, 1H), 4.51 - 4.41 (m, 2H), 3.90 (s, 3H), 3.25 - 3.15 (m, 1H), 3.01 - 2.83 (m, 4H), 2.55 - 2.45 (m, 1H), 2.19 - 2.06 (m, 2H), 1.93 - 1.88 (m, 2H), 1.68 (s, 9H), 1.16 - 1.05 (m, 1H), 0.65 - 0.57 (m, 1H), 0.49 - 0.42 (m, 1H), 0.39 - 0.32 (m, 1H), 0.23 - 0.15 (m, 1H).

**Example 80:**

Synthetic Route:

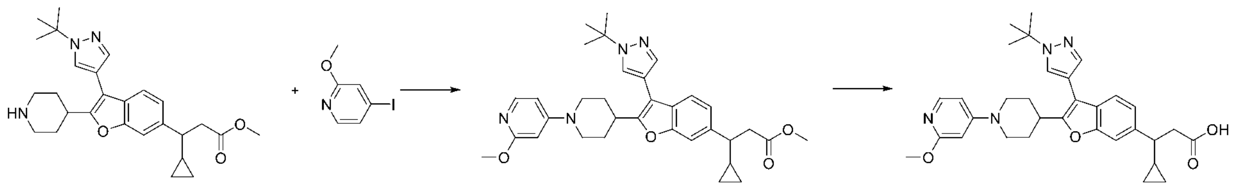

39-3 80-1 80-2 80

Referring to the synthetic route of compound **39**, compound **39-4** was replaced with compound **80-1** to carry out the synthesis. The resulting crude product was purified by reverse-phase column chromatography [acetonitrile/water (0.05% formic acid) = 0% to 100%] to obtain compound **80** (80 mg, yield: 73%) as a white solid. MS (ESI, m/z): 543.2 [M+H]$^+$.

$^1$H NMR (400 MHz, $CDCl_3$) δ 7.94 - 7.89 (m, 1H), 7.72 - 7.65 (m, 2H), 7.50 - 7.43 (m, 1H), 7.34 (s, 1H), 7.17 - 7.10 (m, 1H), 6.49 - 6.42 (m, 1H), 6.11 (s, 1H), 4.01 - 3.95 (m, 2H), 3.92 (s, 3H), 3.26 - 3.16 (m, 1H), 3.05 - 2.97 (m, 2H), 2.90 - 2.81 (m, 2H), 2.55 - 2.46 (m, 1H), 2.19 - 2.07 (m, 2H), 1.93 - 1.87 (m, 2H), 1.68 (s, 9H), 1.15 - 1.05 (m, 1H), 0.66 - 0.60 (m, 1H), 0.47 - 0.40 (m, 1H), 0.38 - 0.31 (m, 1H), 0.23 - 0.15 (m, 1H).

**Example 81:**

Synthetic Route:

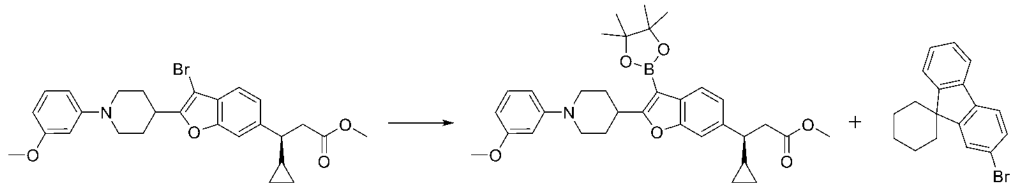

49-2 81-1 81-2

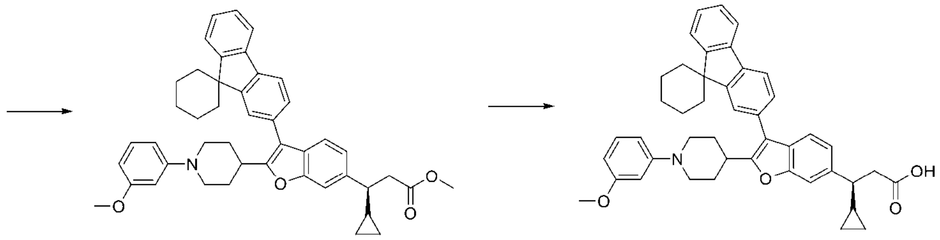

81-3 81

Referring to the synthetic route of compound **49**, compound **49-2** (358 mg, 0.70 mmol) was synthesized. Compound **49-2** (358 mg, 0.70 mmol), bis(pinacolato)diboron (198 mg, 0.77 mmol), [1,1'-bis(diphenylphosphino)ferrocene]palladium(II) dichloride dichloromethane complex (102.75 mg, 0.14 mmol), and potassium acetate (208.45 mg, 2.12 mmol) were added to 1,4-dioxane (10 mL). The reaction was carried out at 90°C for 16 hours. After the reaction was completed, the reaction mixture was concentrated, and the resulting crude product was purified by normal-phase column chromatography (ethyl acetate: petroleum ether = 0% to 100%) to obtain compound **81-1** (196 mg, yield: 50%) as a yellow oil. MS (ESI, m/z): 560.3 $[M+H]^+$.

Compound **81-1** (196 mg, 0.35 mmol), compound **81-2** (110 mg, 0.35 mmol), tetrakis(triphenylphosphine) palladium (40 mg, 0.0035 mmol), and potassium carbonate (223 mg, 1.05 mmol) were added to 1,4-dioxane (5 mL). The reaction was carried out at 100°C for 16 hours. After the reaction was completed, the reaction mixture was cooled to room temperature and diluted with ethyl acetate (50 mL). The organic phase was washed with saturated brine, dried over anhydrous sodium sulfate, filtered, and concentrated. The resulting crude product was purified by normal-phase column chromatography (ethyl acetate: petroleum ether = 0% to 100%) to obtain compound **81-3** (137 mg, yield: 59%) as a white solid. MS (ESI, m/z): 666.3 $[M+H]^+$.

To a reaction tube, compound **81-3** (137 mg, 0.21 mmol), methanol (3 mL), and THF (3 mL) were added. Then, 1 mL of NaOH (84 mg, 1.0 mmol) aqueous solution was added dropwise to the reaction, and the reaction mixture was stirred at room temperature overnight. The pH was adjusted to 3 with dilute hydrochloric acid, and the mixture was extracted with ethyl acetate (10 mL × 3). The organic phase was dried over anhydrous sodium sulfate, filtered, and concentrated. The resulting crude product was purified by reverse-phase column chromatography [acetonitrile/water (0.05% formic acid) = 0% to 100%] to obtain compound **81** (107 mg, yield: 79%) as a white solid. MS (ESI, m/z): 652.3 $[M+H]^+$.

$^1$H NMR (400 MHz, $CDCl_3$) δ 7.88 - 7.83 (m, 1H), 7.82 - 7.74 (m, 2H), 7.65 (d, $J$ = 8.0 Hz, 1H), 7.57 - 7.51 (m, 1H), 7.49 - 7.44 (m, 1H), 7.43 - 7.27 (m, 4H), 7.24 - 7.12 (m, 2H), 6.82 - 6.26 (m, 2H), 3.88 - 3.75 (m, 5H), 3.25 - 3.13 (m, 1H), 2.94 - 2.81 (m, 3H), 2.55 - 2.48 (m, 1H), 2.38 - 2.21 (m, 2H), 2.04 - 1.90 (m, 6H), 1.88 - 1.71 (m, 7H), 1.14 - 1.06 (m, 1H), 0.66 - 0.59 (m, 1H), 0.50 - 0.42 (m, 1H), 0.41 - 0.32 (m, 1H), 0.26 - 0.18 (m, 1H).

**Example 82:**

Synthetic Route:

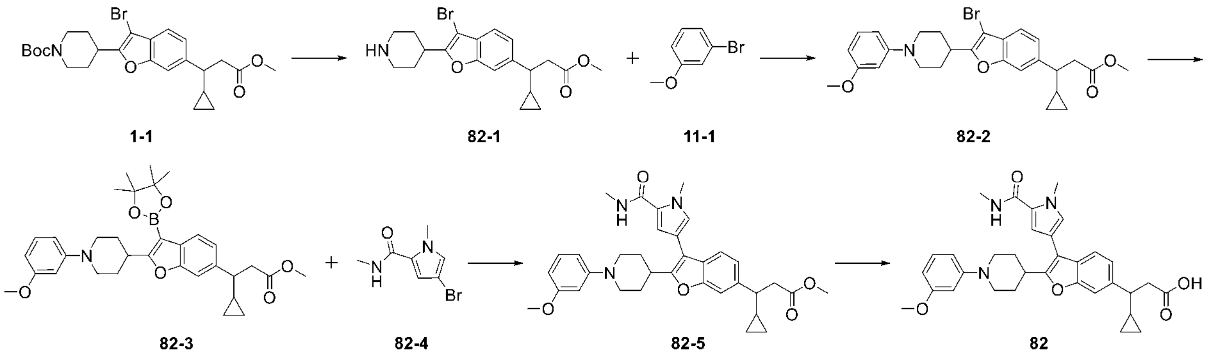

1-1 82-1 11-1 82-2

82-3 82-4 82-5 82

Referring to the synthetic route of compound **49**, compound **12-1** was replaced with compound **1-1** to synthesize compound **82-1**. Then, referring to the synthetic route of compound **81**, compound **81-2** was replaced with compound **82-4** to carry out the synthesis, and the resulting crude product was purified by reverse-phase column chromatography [acetonitrile/water (0.05% formic acid) = 0% to 100%] to obtain compound **82** (14 mg, yield: 27%) as a white solid. MS (ESI, m/z): 556.2 $[M+H]^+$.

$^1H$ NMR (400 MHz, DMSO-$d_6$) δ 8.14 - 8.06 (m, 1H), 7.61 (d, $J$ = 8.0 Hz, 1H), 7.44 (s, 1H), 7.24 (d, $J$ = 1.6 Hz, 1H), 7.17 - 7.10 (m, 2H), 7.02 (d, $J$ = 1.6 Hz, 1H), 6.61 - 6.57 (m, 1H), 6.52 - 6.49 (m, 1H), 6.38 - 6.34 (m, 1H), 3.93 (s, 3H), 3.88 - 3.81 (m, 2H), 3.73 (s, 3H), 3.30 - 3.19 (m, 1H), 2.91 (m, 2H), 2.75 (d, $J$ = 4.4 Hz, 3H), 2.72 - 2.67 (m, 2H), 2.42 - 2.32 (m, 1H), 2.06 - 1.93 (m, 2H), 1.91 - 1.81 (m, 2H), 1.12 - 1.01 (m, 1H), 0.56 - 0.46 (m, 1H), 0.33 - 0.23 (m, 2H), 0.18 - 0.11 (m, 1H).

**Example 83:**

Synthetic Route:

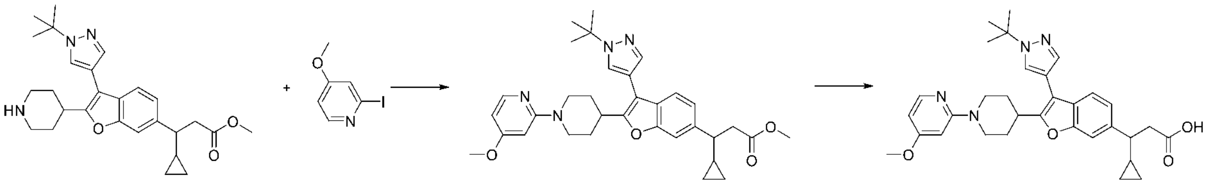

39-3 83-1 83-2 83

Referring to the synthetic route of compound **39**, compound **39-4** was replaced with compound **83-1** to carry out the synthesis. The resulting crude product was purified by reverse-phase column chromatography [acetonitrile/water (0.05% formic acid) = 0% to 100%] to obtain compound **83** (11 mg, yield: 61%) as a white solid. MS (ESI, m/z): 543.2 $[M+H]^+$.

$^1H$ NMR (400 MHz, $CDCl_3$) δ 8.07 (d, $J$ = 6.0 Hz, 1H), 7.69 (s, 2H), 7.48 (d, $J$ = 8.0 Hz, 1H), 7.34 (s, 1H), 7.14 (d, $J$ = 8.0 Hz, 1H), 6.30 - 6.26 (m, 1H), 6.20 - 6.14 (m, 1H), 4.42 - 4.32 (m, 2H), 3.85 (s, 3H), 3.24 - 3.11 (m, 1H), 2.98 - 2.82 (m, 4H), 2.55 - 2.46 (m, 1H), 2.14 - 2.05 (m, 2H), 1.93 - 1.88 (m, 2H), 1.68 (s, 9H), 1.13 - 1.05 (m, 1H), 0.66 - 0.58 (m, 1H), 0.48 - 0.41 (m, 1H), 0.37 - 0.31 (m, 1H), 0.23 - 0.16 (m, 1H).

**Example 84:**

Synthetic Route:

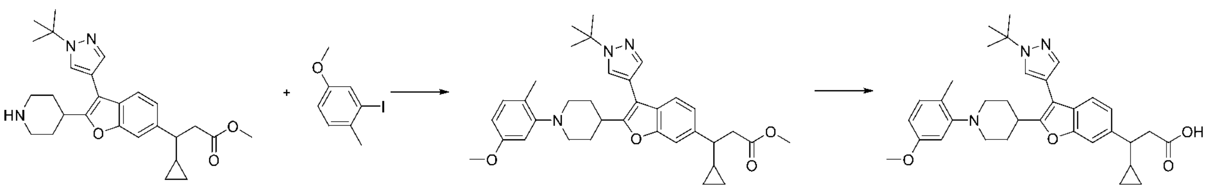

39-3 84-1 84-2 84

Referring to the synthetic route of compound **39**, compound **39-4** was replaced with compound **84-1** to carry out the synthesis. The resulting crude product was purified by reverse-phase column chromatography [acetonitrile/water (0.05% formic acid) = 0% to 100%] to obtain compound **84** (22 mg, yield: 49%) as a white solid. MS (ESI, m/z): 556.3 $[M+H]^+$.

$^1H$ NMR (400 MHz, $CDCl_3$) δ 7.74 - 7.69 (m, 2H), 7.49 (d, *J*= 8.0 Hz, 1H), 7.40 (s, 1H), 7.17 - 7.10 (m, 2H), 6.66 - 6.63 (m, 1H), 6.59 - 6.54 (m, 1H), 3.81 (s, 3H), 3.31 - 3.25 (m, 2H), 3.13 - 3.03 (m, 1H), 2.91 - 2.82 (m, 2H), 2.78 - 2.70 (m, 2H), 2.58 - 2.50 (m, 1H), 2.31 - 2.24 (m, 5H), 1.96 - 1.89 (m, 2H), 1.69 (s, 9H), 1.16 - 1.08 (m, 1H), 0.66 - 0.59 (m, 1H), 0.47 - 0.41 (m, 1H), 0.37 - 0.32 (m, 1H), 0.23 - 0.18 (m, 1H).

**Example 85:**

Synthetic Route:

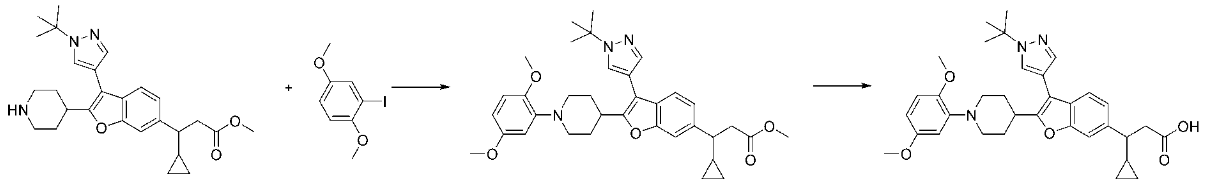

39-3 85-1 85-2 85

Referring to the synthetic route of compound **39,** compound **39-4** was replaced with compound **85-1** to carry out the synthesis. The resulting crude product was purified by reverse-phase column chromatography [acetonitrile/water (0.05% formic acid) = 0% to 100%] to obtain compound **85** (13 mg, yield: 33%) as a white solid. MS (ESI, m/z): 572.3 $[M+H]^+$.

$^1H$ NMR (400 MHz, $CDCl_3$) δ 7.74 - 7.68 (m, 2H), 7.48 (d, *J*= 8.0 Hz, 1H), 7.36 (s, 1H), 7.14 (d, *J* = 8.0 Hz, 1H), 6.85 - 6.75 (m, 1H), 6.64 - 6.57 (m, 1H), 6.53 - 6.46 (m, 1H), 3.87 (s, 3H), 3.79 (s, 3H), 3.70 - 3.63 (m, 2H), 3.12 - 3.05 (m, 1H), 2.90 - 2.85 (m, 2H), 2.72 - 2.64 (m, 2H), 2.54 - 2.47 (m, 1H), 2.40 - 2.31 (m, 2H), 1.96 - 1.90 (m, 2H), 1.69 (s, 9H), 1.15 - 1.05 (m, 1H), 0.66 - 0.57 (m, 1H), 0.49 - 0.42 (m, 1H), 0.39 - 0.32 (m, 1H), 0.28 - 0.16 (m, 1H).

**Example 86:**

Synthetic Route:

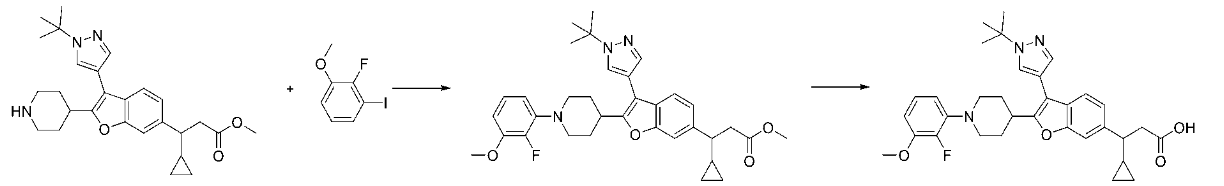

39-3 86-1 86-2 86

Referring to the synthetic route of compound 39, compound 39-4 was replaced with compound 86-1 to carry out the synthesis. The resulting crude product was purified by reverse-phase column chromatography [acetonitrile/water (0.05% formic acid) = 0% to 100%] to obtain compound **86** (4 mg, yield: 13%) as a white solid. MS (ESI, m/z): 560.3 $[M+H]^+$.

$^1H$ NMR (400 MHz, $CDCl_3$) δ 7.72 - 7.69 (m, 2H), 7.48 (d, *J*= 8.0 Hz, 1H), 7.37 (s, 1H), 7.14 (d, *J*= 8.0 Hz, 1H), 7.03 - 6.96 (m, 1H), 6.69 - 6.62 (m, 2H), 3.90 (s, 3H), 3.64 - 3.56 (m, 2H), 3.14 - 3.03 (m, 1H), 2.92 - 2.79 (m, 4H), 2.55 - 2.47 (m, 1H), 2.38 - 2.26 (m, 2H), 1.97 - 1.90 (m, 2H), 1.68 (s, 9H), 1.14 - 1.06 (m, 1H), 0.66 - 0.57 (m, 1H), 0.50 - 0.41 (m, 1H), 0.38 - 0.28 (m, 1H), 0.24 - 0.15 (m, 1H).

**Example 87:**

Synthetic Route:

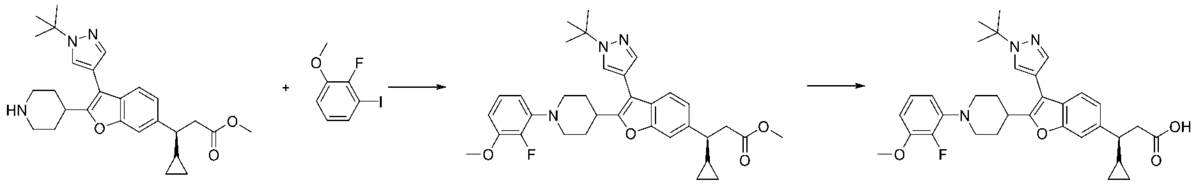

38-3 86-1 87-1 87

Referring to the synthetic route of compound **86,** compound **6-3** was replaced with compound **38-3** to carry out the synthesis. The resulting crude product was purified by reverse-phase column chromatography [acetonitrile/water (0.05% formic acid) = 0% to 100%] to obtain compound **87** (13 mg, yield: 15%) as a white solid. MS (ESI, m/z): 560.3 $[M+H]^+$.

$^1H$ NMR (400 MHz, $CDCl_3$) δ 7.73 - 7.67 (m, 2H), 7.48 (d, *J* = 8.0 Hz, 1H), 7.37 (s, 1H), 7.14 (d, *J* = 8.4 Hz, 1H), 7.05 - 6.96 (m, 1H), 6.73 - 6.61 (m, 2H), 3.90 (s, 3H), 3.65 - 3.56 (m, 2H), 3.16 - 3.01 (m, 1H), 2.94 - 2.80 (m, 4H), 2.57 - 2.46 (m, 1H), 2.39 - 2.29 (m, 2H), 1.99 - 1.91 (m, 2H), 1.69 (s, 9H), 1.15 - 1.06 (m, 1H), 0.69 - 0.61 (m, 1H), 0.47 - 0.43 (m, 1H), 0.36 - 0.32 (m, 1H), 0.23 - 0.19 (m, 1H).

**Example 88:**

Synthetic Route:

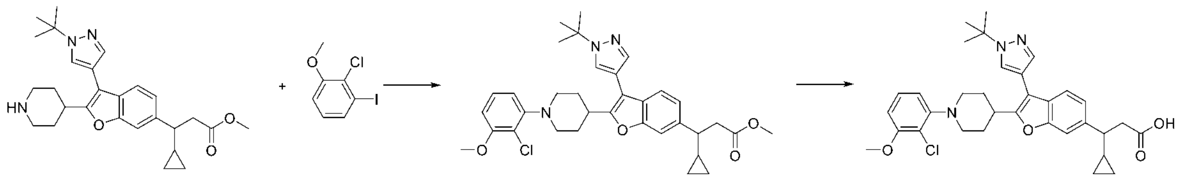

39-3 88-1 88-2 88

Referring to the synthetic route of compound **39,** compound **39-4** was replaced with compound **88-1** to carry out the synthesis. The resulting crude product was purified by reverse-phase column chromatography [acetonitrile/water (0.05% formic acid) = 0% to 100%] to obtain compound **88** (8 mg, yield: 36%) as a white solid. MS (ESI, m/z): 576.3 $[M+H]^+$.

$^1H$ NMR (400 MHz, $CDCl_3$) δ 7.74 - 7.69 (m, 2H), 7.48 (d, *J* = 8.0 Hz, 1H), 7.39 (s, 1H), 7.22 - 7.16 (m, 1H), 7.14 (d, *J* = 8.0 Hz, 1H), 6.79 - 6.73 (m, 1H), 6.72 - 6.66 (m, 1H), 3.92 (s, 3H), 3.57 - 3.48 (m, 2H), 3.15 - 3.05 (m, 1H), 2.91 - 2.75 (m, 4H), 2.57 - 2.48 (m, 1H), 2.42 - 2.29 (m, 2H), 1.98 - 1.90 (m, 2H), 1.69 (s, 9H), 1.19 - 1.07 (m, 1H), 0.68 - 0.60 (m, 1H), 0.48 - 0.42 (m, 1H), 0.39 - 0.31 (m, 1H), 0.24 - 0.17 (m, 1H).

**Example 89:**

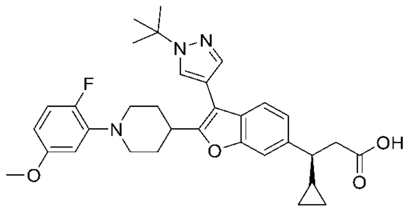

Synthetic Route:

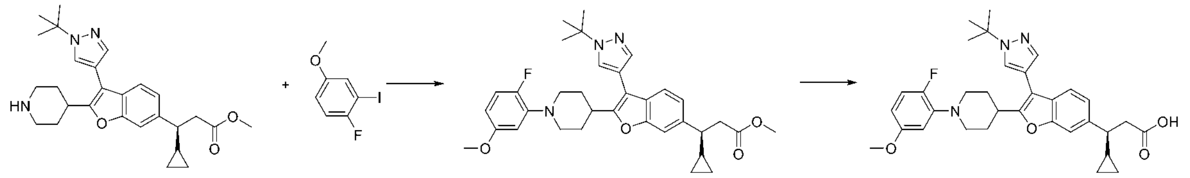

38-3 89-1 89-2 89

Referring to the synthetic route of compound 38, compound 11-1 was replaced with compound **89-1** to carry out the synthesis. The resulting crude product was purified by reverse-phase column chromatography [acetonitrile/water (0.05% formic acid) = 0% to 100%] to obtain compound **89** (19 mg, yield: 33%) as a white solid. MS (ESI, m/z): 560.3 $[M+H]^+$.

$^1H$ NMR (400 MHz, $CDCl_3$) δ 7.71 (s, 2H), 7.52 - 7.44 (m, 1H), 7.36 (s, 1H), 7.20 - 7.08 (m, 1H), 7.03 - 6.88 (m, 1H), 6.63 - 6.50 (m, 1H), 6.48 - 6.38 (m, 1H), 3.79 (s, 3H), 3.68 - 3.54 (m, 2H), 3.16 - 3.03 (m, 1H), 2.96 - 2.71 (m, 4H), 2.58 - 2.44 (m, 1H), 2.40 - 2.24 (m, 2H), 2.01 - 1.86 (m, 2H), 1.68 (s, 9H), 1.16 - 1.04 (m, 1H), 0.70 - 0.57 (m, 1H), 0.50 - 0.40 (m, 1H), 0.39 - 0.30 (m, 1H), 0.26 - 0.14 (m, 1H).

**Example 90:**

Synthetic Route:

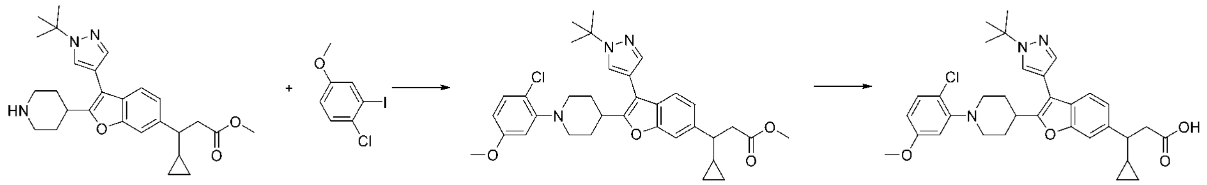

39-3 90-1 90-2 90

Referring to the synthetic route of compound **39,** compound **39-4** was replaced with compound **90-1** to carry out the synthesis. The resulting crude product was purified by reverse-phase column chromatography [acetonitrile/water (0.05% formic acid) = 0% to 100%] to obtain compound **90** (8 mg, yield: 53%) as a white solid. MS (ESI, m/z): 576.3 $[M+H]^+$.

$^1H$ NMR (400 MHz, $CDCl_3$) δ 7.74 - 7.69 (m, 2H), 7.48 (d, *J*= 8.0 Hz, 1H), 7.39 (s, 1H), 7.27 (d, *J*= 2.4 Hz, 1H), 7.18 - 7.12 (m, 1H), 6.69 - 6.62 (m, 1H), 6.58 - 6.51 (m, 1H), 3.81 (s, 3H), 3.58 - 3.50 (m, 2H), 3.15 - 3.05 (m, 1H), 2.92 - 2.72 (m, 4H), 2.58 - 2.48 (m, 1H), 2.42 - 2.29 (m, 2H), 2.00 - 1.91 (m, 2H), 1.69 (s, 9H), 1.17 - 1.08 (m, 1H), 0.67 - 0.60 (m, 1H), 0.48 - 0.41 (m, 1H), 0.38 - 0.31 (m, 1H), 0.25 - 0.18 (m, 1H).

**Example 91:**

Synthetic Route:

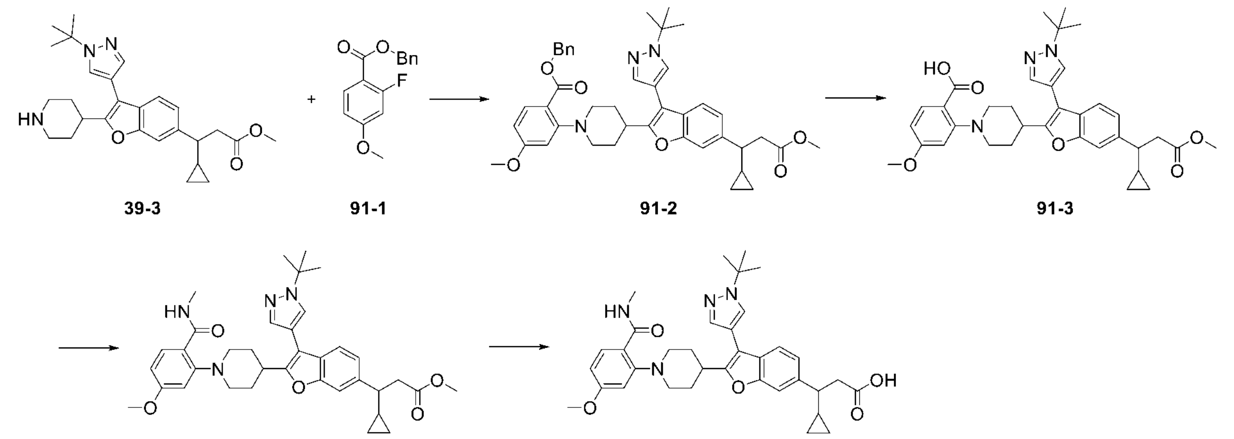

39-3 91-1 91-2 91-3

91-4 91

Referring to the synthetic route of compound **39,** compound **39-4** was replaced with compound **91-1** to synthesize compound **91-2** (184 mg, 0.27 mmol) as a yellow oil. Compound **91-2** (184 mg, 0.27 mmol) and palladium hydroxide (20 mg, 10%) were added to a mixture of methanol (10 mL) and ethyl acetate (10 mL), and the reaction mixture was heated to 60°C and stirred overnight. After the reaction was completed, the reaction mixture was concentrated, and the resulting crude product was purified by normal-phase column chromatography (ethyl acetate: petroleum ether = 0% to 100%) to obtain compound **91-3** (69 mg, yield: 43%) as a white solid. MS (ESI, m/z): 600.1 $[M+H]^+$.

Compound **91-3** (47 mg, 0.078 mmol), methylamine hydrochloride (26 mg, 0.39 mmol), 2-(7-azabenzotriazol-1-yl)-*N*,*N*,*N'*,*N*-tetramethyluronium hexafluorophosphate (45 mg, 0.12 mmol), and *N*,*N*-diisopropylethylamine (81 mg, 0.63 mmol) were added to *N,N*-dimethylformamide (3 mL) and stirred at room temperature overnight. After the reaction was completed, ethyl acetate (50 mL) was added, and the organic phase was washed with saturated brine. The combined organic phases were concentrated to obtain a crude product. The resulting crude product was purified by normal-phase column chromatography (ethyl acetate: petroleum ether = 0% to 100%) to obtain compound **91-4** (30 mg, yield: 63%) as a yellow oil. MS (ESI, m/z): 613.1 $[M+H]^+$.

Compound **91-4** (30 mg, 0.049 mmol) was added to a mixture of tetrahydrofuran (4 mL) and methanol (4 mL). Then, an aqueous solution (1.5 mL) of lithium hydroxide (24 mg, 0.98 mmol) was added to the reaction mixture, and the reaction mixture was heated to 60°C and stirred for 1 hour. The reaction mixture was then purified by reverse-phase column chromatography [acetonitrile/water (0.05% formic acid) = 0% to 100%] to obtain compound **91** (8.8 mg, yield: 30%) as a white solid. MS (ESI, m/z): 599.1 $[M+H]^+$.

$^1H$ NMR (400 MHz, DMSO-$d_6$) δ 9.63 (s, 1H), 8.17 (d, *J* = 8.4 Hz, 1H), 7.76 - 7.64 (m, 2H), 7.48 (d, *J* = 8.0 Hz, 1H), 7.41 (s, 1H), 7.17 (d, *J*= 8.0 Hz, 1H), 6.79 - 6.68 (m, 2H), 3.86 (m, 3H), 3.36 - 3.22 (m, 2H), 3.15 - 3.07 (m, 4H), 2.94 - 2.81 (m, 4H), 2.56 - 2.50 (m, 1H), 2.27 - 2.14 (m, 2H), 2.08 - 1.99 (m, 2H), 1.69 (s, 9H), 1.16 - 1.07 (m, 1H), 0.67 - 0.59 (m, 1H), 0.50 - 0.42 (m, 1H), 0.39 - 0.32 (m, 1H), 0.24 - 0.18 (m, 1H).

**Example 92:**

Synthetic Route:

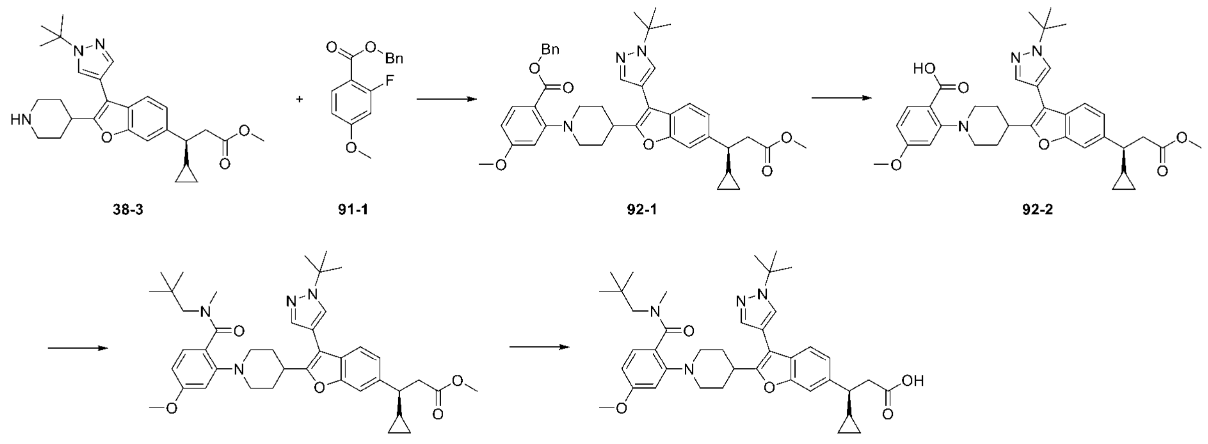

38-3 91-1 92-1 92-2

92-3 92

Referring to the synthetic route of compound **91,** compound **39-3** was replaced with compound **38-3,** and methylamine hydrochloride was replaced with *N*,2,2-trimethylpropan-1-amine to carry out the synthesis. The reaction mixture was purified by reverse-phase column chromatography [acetonitrile/water (0.05% formic acid) = 0% to 100%] to obtain compound **92** (8 mg, yield: 28%) as a white solid. MS (ESI, m/z): 669.4 $[M+H]^+$.

$^1H$ NMR (400 MHz, $CDCl_3$) δ 7.70 - 7.62 (m, 2H), 7.46 (d, *J*= 8.0 Hz, 1H), 7.41 - 7.32 (m, 1H), 7.30 - 7.26 (m, 1H), 7.18 - 7.10 (m, 1H), 6.64 - 6.45 (m, 2H), 3.82 (s, 3H), 3.69 - 3.49 (m, 2H), 3.43 - 3.27 (m, 1H), 3.25 - 3.16 (m, 2H), 3.09 - 2.91 (m, 4H), 2.89 - 2.78 (m, 2H), 2.67 - 2.56 (m, 1H), 2.54 - 2.46 (m, 1H), 2.23 - 2.06 (m, 2H), 2.04 - 1.88 (m, 2H), 1.87 - 1.79 (m, 1H), 1.66 (s, 9H), 1.15 - 1.02 (m, 6H), 0.82 - 0.72 (m, 3H), 0.65 - 0.57 (m, 1H), 0.47 - 0.38 (m, 1H), 0.36 - 0.29 (m, 1H), 0.23 - 0.15 (m 1H).

**Example 93:**

Synthetic Route:

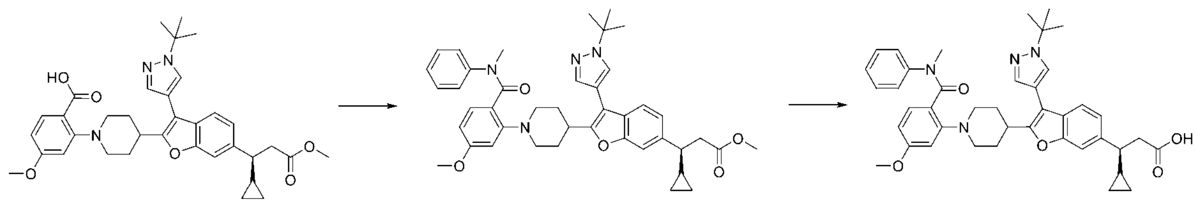

92-2 93-1 93

Referring to the synthetic route of compound **92,** *N*,2,2-trimethylpropan-1-amine was replaced with *N*-methylaniline to carry out the synthesis. The reaction mixture was purified by reverse-phase column chromatography [acetonitrile/-water (0.05% formic acid) = 0% to 100%] to obtain compound 93 (12 mg, yield: 36%) as a white solid. MS (ESI, m/z): 675.3 $[M+H]^+$.

$^1H$ NMR (400 MHz, $CDCl_3$) δ 7.70 - 7.62 (m, 2H), 7.47 (d, *J*= 8.4 Hz, 1H), 7.42 (s, 1H), 7.34 (d, *J*= 8.4 Hz, 1H), 7.25 - 6.85 (m, 6H), 6.60 - 6.45 (m, 1H), 6.28 - 6.08 (m, 1H), 3.75 (s, 3H), 3.54 (s, 3H), 3.04 - 2.81 (m, 4H), 2.69 - 2.46 (m, 3H), 2.23 - 2.09 (m, 2H), 1.90 - 1.72 (m, 3H), 1.66 (s, 9H), 1.16 - 1.05 (m, 1H), 0.66 - 0.57 (m, 1H), 0.49 - 0.40 (m, 1H), 0.38 - 0.30 (m, 1H), 0.25 - 0.16 (m, 1H).

**Example 94:**

Synthetic Route:

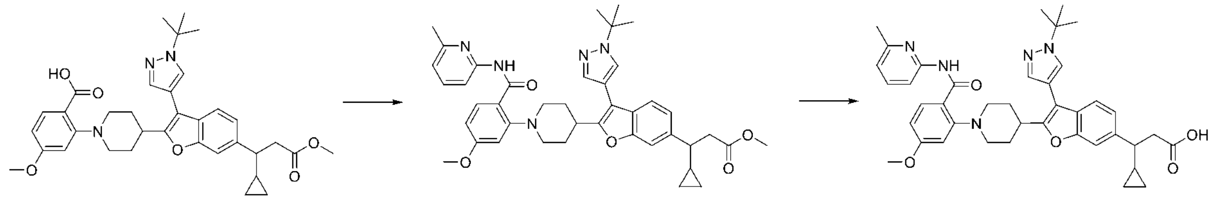

91-3 94-1 94

Referring to the synthetic route of compound 91, methylamine hydrochloride was replaced with 2-amino-6-methylpyridine to carry out the synthesis. The reaction mixture was purified by reverse-phase column chromatography [acetonitrile/water (0.05% formic acid) = 0% to 100%] to obtain compound **94** (5 mg, yield: 49%) as a white solid. MS (ESI, m/z): 676.3 $[M+H]^+$.

$^1$H NMR (400 MHz, DMSO-$d_6$) δ 12.86 (s, 1H), 8.29 - 8.25 (m, 1H), 8.24 - 8.18 (m, 1H),7.74 - 7.69 (m, 2H), 7.66 - 7.59 (m, 1H), 7.50 (d, *J*= 7.6 Hz, 1H), 7.36 (s, 1H), 7.16 (d, *J*= 7.6 Hz, 1H), 6.94 - 6.89 (m, 1H), 6.87 - 6.80 (m, 2H), 3.90 (s, 3H), 3.44 - 3.36 (m, 2H), 3.19 - 3.11 (m, 1H), 3.00 - 2.85 (m, 4H), 2.84 - 2.74 (m, 2H), 2.70 (s, 3H), 2.60 - 2.50 (m, 1H), 2.03 - 1.93 (m, 2H), 1.69 (s, 9H), 1.15 - 1.08 (m, 1H), 0.69 - 0.61 (m, 1H), 0.51 - 0.43 (m, 1H), 0.41 - 0.32 (m, 1H), 0.26 - 0.19 (m, 1H)

**Example 95:**

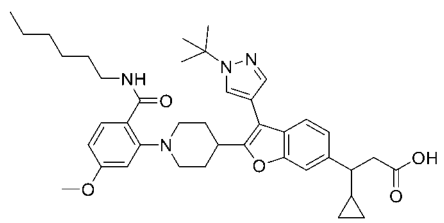

Synthetic Route:

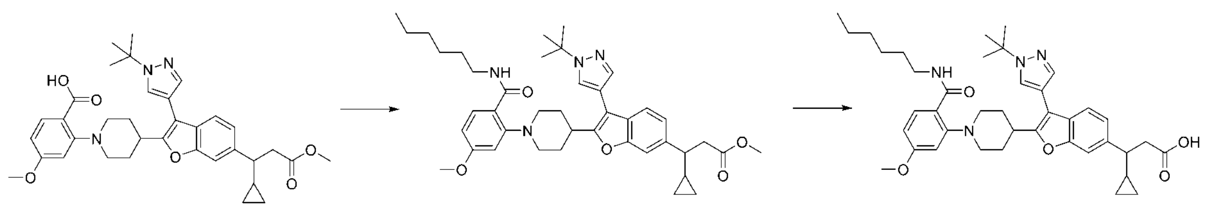

91-3 95-1 95

Referring to the synthetic route of compound 91, methylamine hydrochloride was replaced with n-hexylamine to carry out the synthesis. The reaction mixture was purified by reverse-phase column chromatography [acetonitrile/water (0.05% formic acid) = 0% to 100%] to obtain compound **95** (7 mg, yield: 19%) as a white solid. MS (ESI, m/z): 669.3 $[M+H]^+$.

$^1$H NMR (400 MHz, $CDCl_3$) δ 9.91 - 9.81 (m, 1H), 8.26 - 8.14 (m, 1H), 7.70 (d, *J* = 7.2 Hz, 2H), 7.49 (d, *J* = 8.0 Hz, 1H), 7.36 (s, 1H), 7.17 (d, *J* = 8.0 Hz, 1H), 6.82 - 6.70 (m, 2H), 3.86 (s, 3H), 3.60 - 3.46 (m, 2H), 3.36 - 3.24 (m, 2H), 3.18 - 3.05 (m, 1H), 2.98 - 2.75 (m, 4H), 2.60 - 2.44 (m, 1H), 2.36 - 2.13 (m, 2H), 1.83 - 1.74 (m, 2H), 1.69 (s, 9H), 1.54 - 1.33 (m, 8H), 1.14 - 1.07 (m, 1H), 0.92 (t, *J* = 7.2 Hz, 3H), 0.70 - 0.59 (m, 1H), 0.50 - 0.40 (m, 1H), 0.40 - 0.31 (m, 1H), 0.26 - 0.16 (m, 1H).

**Example 96:**

Synthetic Route:

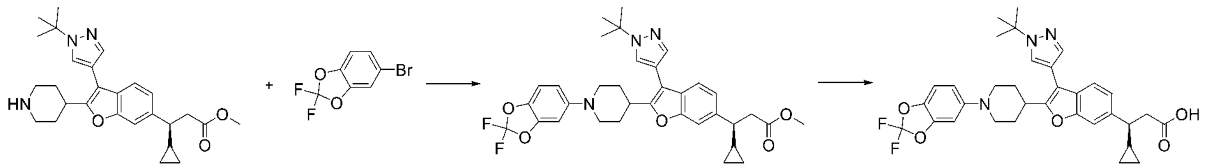

38-3 96-1 96-2 96

Referring to the synthetic route of compound **38,** compound **11-1** was replaced with compound **96-1** to carry out the synthesis. The resulting crude product was purified by reverse-phase column chromatography [acetonitrile/water (0.05% formic acid) = 0% to 100%] to obtain compound **96** (68 mg, yield: 67%) as a white solid. MS (ESI, m/z): 592.3 [M+H]+.

$^1$H NMR (400 MHz, $CDCl_3$) δ 7.68 (d, *J* = 8.0 Hz, 2H), 7.46 (d, *J* = 8.4 Hz, 1H), 7.35 (s, 1H), 7.13 (d, *J*= 8.0 Hz, 1H), 6.99 - 6.90 (m, 1H), 6.85 - 6.75 (m, 1H), 6.74 - 6.60 (m, 1H), 3.70 - 3.57 (m, 2H), 3.19 - 3.00 (m, 1H), 2.92 - 2.76 (m, 4H), 2.55 - 2.44 (m, 1H), 2.35 - 2.15 (m, 2H), 2.02 - 1.86 (m, 1H), 1.67 (s, 9H), 1.14 - 1.02 (m, 1H), 0.68 - 0.53 (m, 1H), 0.49 - 0.38 (m, 1H), 0.37 - 0.27 (m, 1H), 0.24 - 0.12 (m, 1H).

**Example 97:**

Synthetic Route:

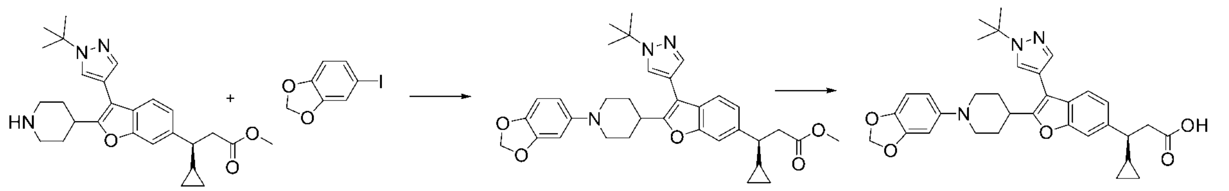

38-3 97-1 97-2 97

Referring to the synthetic route of compound **38,** compound **11-1** was replaced with compound **97-1** to carry out the synthesis. The resulting crude product was purified by reverse-phase column chromatography [acetonitrile/water (0.05% formic acid) = 0% to 100%] to obtain compound **97** (17 mg, yield: 39%) as a white solid. MS (ESI, m/z): 556.3 [M+H]+.

$^1$H NMR (400 MHz, $CDCl_3$) δ 7.68 (s, 2H), 7.46 (d, *J* = 8.0 Hz, 1H), 7.35 (s, 1H), 7.13 (d, *J*= 8.0 Hz, 1H), 6.78 - 6.68 (m, 1H), 6.66 - 6.56 (m, 1H), 6.48 - 6.36 (m, 1H), 5.91 (s, 2H), 3.67 - 3.53 (m, 2H), 3.11 - 2.97 (m, 1H), 2.92 - 2.69 (m, 4H), 2.55 - 2.41 (m, 1H), 2.34 - 2.14 (m, 2H), 2.00 - 1.86 (m, 2H), 1.67 (s, 9H), 1.15 - 1.03 (m, 1H), 0.68 - 0.53 (m, 1H), 0.49 - 0.39 (m, 1H), 0.38 - 0.27 (m, 1H), 0.25 - 0.13 (m, 1H).

**Example 98:**

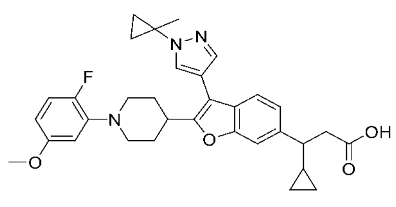

Synthetic Route:

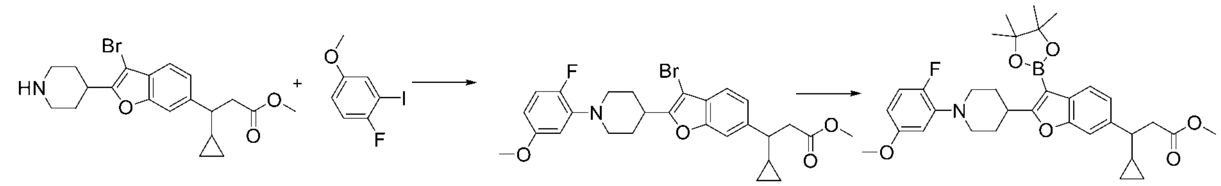

**82-1** **89-1** **98-1** **98-2**

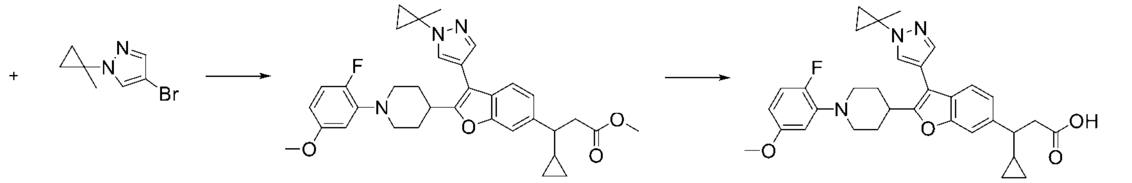

**98-3** **98-4** **98**

Referring to the synthetic route of compound **82,** compound **11-1** was replaced with compound **89-1** and compound **82-4** was replaced with compound **98-3** to carry out the synthesis. The resulting crude product was purified by reverse-phase column chromatography [acetonitrile/water (0.05% ammonia) = 0% to 100%] to obtain compound **98** (6 mg, yield: 19%) as a white solid. MS (ESI, m/z): 558.3 $[M+H]^+$.

$^1$H NMR (400 MHz, MeOD) δ 7.97 (s, 1H), 7.69 (s, 1H), 7.43 (d, *J* = 8.0 Hz, 1H), 7.38 (s, 1H), 7.19 (d, *J*= 8.4 Hz, 1H), 6.99 - 6.91 (m, 1H), 6.64 - 6.58 (m, 1H), 6.51 - 6.45 (m, 1H), 3.76 (s, 3H), 3.58 - 3.51 (m, 2H), 3.14 - 3.05 (m, 1H), 2.85 - 2.77 (m, 2H), 2.71 - 2.58 (m, 2H), 2.55 - 2.46 (m, 1H), 2.29 - 2.17 (m, 2H), 1.90 - 1.98 (m, 2H), 1.67 (s, 3H), 1.35 - 1.30 (m, 2H), 1.13 - 0.99 (m, 3H), 0.62 - 0.53 (m, 1H), 0.42 - 0.33 (m, 2H), 0.15 - 0.10 (m, 1H).

**Example 99:**

Synthetic Route:

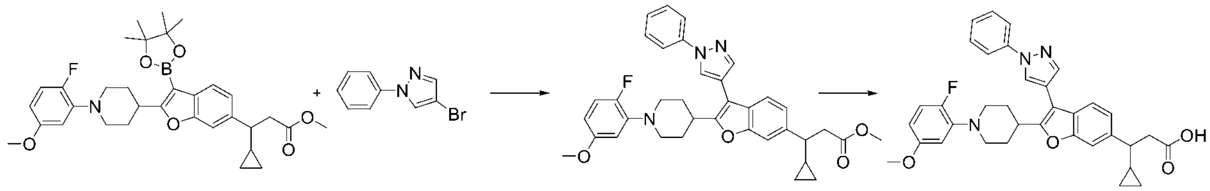

**98-2** **99-1** **99-2** **99**

Referring to the synthetic route of compound **98,** compound **98-3** was replaced with compound **99-1** to carry out the synthesis. The resulting crude product was purified by reverse-phase column chromatography [acetonitrile/water (0.05% ammonia) = 0% to 100%] to obtain compound **99** (26 mg, yield: 87%) as a white solid. MS (ESI, m/z): 580.3 $[M+H]^+$.

$^1$H NMR (400 MHz, $CDCl_3$) δ 8.09 (s, 1H), 7.89 (s, 1H), 7.77 (d, *J* = 7.6 Hz, 2H), 7.54 - 7.47 (m, 3H), 7.40 - 7.31 (m, 2H), 7.16 (d, *J*= 7.2 Hz, 1H), 6.99 - 6.92 (m, 1H), 6.55 - 6.53 (m, 1H), 6.47 - 6.36 (m, 1H), 3.78 (s, 3H), 3.60 - 3.58 (m, 2H), 3.14 - 3.08 (m, 1H), 2.87 - 2.82 (m, 4H), 2.52 - 2.47 (m, 1H), 2.37 - 2.28 (m, 2H), 1.96 - 1.93 (m, 2H), 1.16 - 1.06 (m, 1H), 0.68 - 0.59 (m, 1H), 0.52 - 0.42 (m, 1H), 0.38 - 0.32 (m, 1H), 0.25 - 0.17 (m, 1H).

**Example 100:**

Synthetic Route:

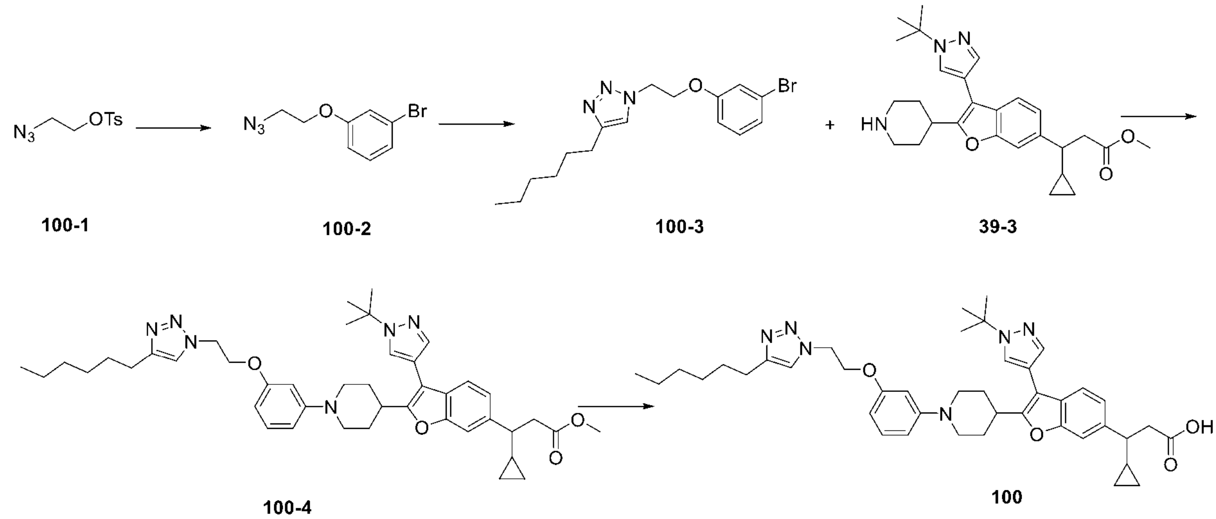

100-1 100-2 100-3 39-3

100-4 100

Compound **100-1** (700 mg, 2.90 mmol), 3-bromophenol (502 mg, 2.90 mmol), and potassium carbonate (601 mg, 4.36 mmol) were added to N,N-dimethylformamide (10 mL), and the reaction mixture was heated to 80°C and stirred overnight. After the reaction was completed, 1 N dilute hydrochloric acid (50 mL) was added, and then ethyl acetate (100 mL) was added. The organic phase was washed with saturated brine, and the combined organic phases were concentrated to obtain a crude product. The resulting crude product was purified by normal-phase column chromatography (ethyl acetate: petroleum ether = 0% to 100%) to obtain compound **100-2** (529 mg, yield: 76%) as a yellow oil. MS (ESI, m/z): 241.8 $[M+H]^+$.

Compound **100-2** (529 mg, 2.19 mmol), 1-octyne (289 mg, 2.62 mmol), copper(II) sulfate pentahydrate (109 mg, 0.44 mmol), and sodium ascorbate (173 mg, 0.87 mmol) were added to a mixture of tert-butanol (10 mL) and water (10 mL). The reaction mixture was heated to 45°C and stirred for 2 hours. After the reaction was completed, ethyl acetate (100 mL) was added. The organic phase was washed with saturated brine, and the combined organic phases were concentrated to obtain a crude product. The resulting crude product was purified by normal-phase column chromatography (ethyl acetate: petroleum ether = 0% to 100%) to obtain compound **100-3** (400 mg, yield: 52%) as a yellow oil. MS (ESI, m/z): 351.8 $[M+H]^+$.

Referring to the synthetic route of compound **39,** compound **39-4** was replaced with compound **100-3** to carry out the synthesis. The resulting crude product was purified by reverse-phase column chromatography [acetonitrile/water (0.05% ammonia) = 0% to 100%] to obtain compound **100** (87 mg, yield: 37%) as a white solid. MS (ESI, m/z): 706.8 $[M+H]^+$.

$^1$H NMR (400 MHz, $CDCl_3$) $\delta$ 7.71 (s, 2H), 7.54 - 7.42 (m, 2H), 7.36 (s, 1H), 7.21 - 7.15 (m, 2H), 6.64 (d, *J* = 8.0 Hz, 1H), 6.50 (s, 1H), 6.38 (d, *J* = 8.0 Hz, 1H), 4.77 - 4.65 (m, 2H), 4.40 - 4.25 (m, 2H), 3.88 - 3.73 (m, 2H), 3.16 - 3.02 (m, 1H), 2.93 - 2.79 (m, 4H), 2.75 - 2.65 (m, 2H), 2.55 - 2.49 (m, 1H), 2.26 - 2.18 (m, 2H), 1.97 - 1.85 (m, 2H), 1.73 - 1.63 (m, 11H), 1.39 - 1.28 (m, 7H), 1.15 - 1.05 (m, 1H), 0.88 (t, *J* = 6.4 Hz, 3H), 0.67 - 0.58 (m, 1H), 0.49 - 0.32 (m, 2H), 0.24 - 0.18 (m, 1H).

**Example 101:**

Synthetic Route:

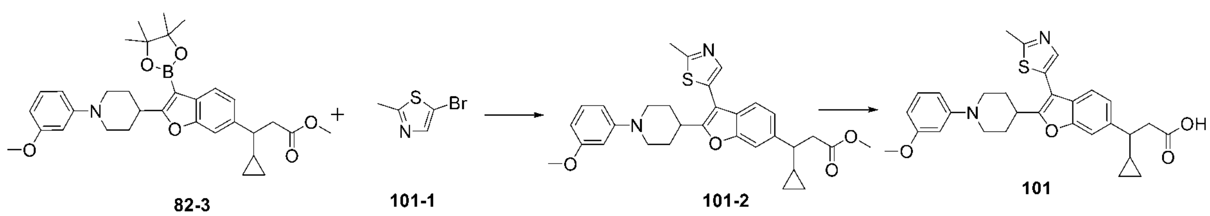

82-3 101-1 101-2 101

Referring to the synthetic route of compound **82,** compound **82-4** was replaced with compound **101-1** to carry out the synthesis. The resulting crude product was purified by reverse-phase column chromatography [acetonitrile/water (0.05% ammonia) = 0% to 100%] to obtain compound **101** (5 mg, yield: 24%) as a white solid. MS (ESI, m/z): 517.2 $[M+H]^+$.

$^1$H NMR (400 MHz, DMSO-$d_6$) $\delta$ 7.88 (s, 1H), 7.58 - 7.50 (m, 2H), 7.24 - 7.19 (m, 1H), 7.16 - 7.09 (m, 1H), 6.61 -

6.55 (m, 1H), 6.51 - 6.48 (m, 1H), 6.39 - 6.34 (m, 1H), 3.89 - 3.80 (m, 2H), 3.73 (s, 3H), 3.31 - 3.20 (m, 1H), 2.92 - 2.80 (m, 2H), 2.77 - 2.69 (m, 5H), 2.42 - 2.34 (m, 1H), 2.07 - 1.92 (m, 2H), 1.92 - 1.83 (m, 2H), 1.12 - 1.02 (m, 1H), 0.55 - 0.47 (m, 1H), 0.32 - 0.23 (m, 2H), 0.18 - 0.09 (m, 1H).

**Example 102:**

Synthetic Route:

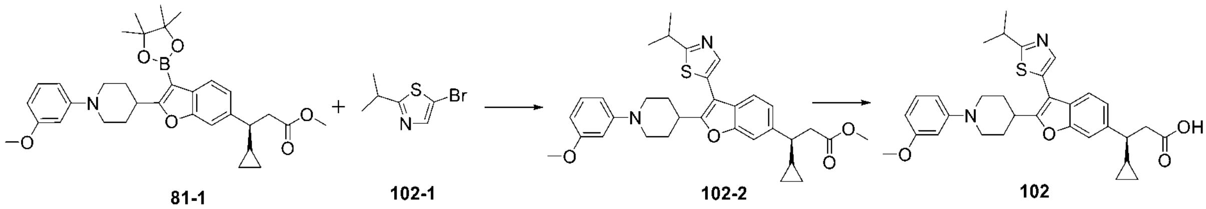

81-1 102-1 102-2 102

Referring to the synthetic route of compound **81,** compound **81-2** was replaced with compound **102-1** to carry out the synthesis. The resulting crude product was purified by reverse-phase column chromatography [acetonitrile/water (0.05% ammonia) = 0% to 100%] to obtain compound **102** (147 mg, yield: 82%) as a white solid. MS (ESI, m/z): 545.2 $[M+H]^+$.

$^1H$ NMR (400 MHz, $CDCl_3$) $\delta$ 7.73 - 7.66 (m, 1H), 7.57 - 7.47 (m, 1H),7.38 (s, 1H), 7.25 - 7.13 (m, 2H), 6.99 - 6.26 (m, 3H), 3.86 - 3.74 (m, 5H), 3.44 - 3.33 (m, 1H), 3.30 - 3.15 (m, 1H), 3.07 - 2.76 (m, 4H), 2.56 - 2.45 (m, 1H), 2.42 - 2.16 (m, 2H), 2.10 - 1.84 (m, 2H), 1.47 (d, $J$= 6.8 Hz, 6H), 1.13 - 1.03 (m, 1H), 0.67 - 0.57 (m, 1H), 0.48 - 0.39 (m, 1H), 0.38 - 0.29 (m, 1H), 0.23 - 0.13 (m, 1H).

**Example 103:**

Synthetic Route:

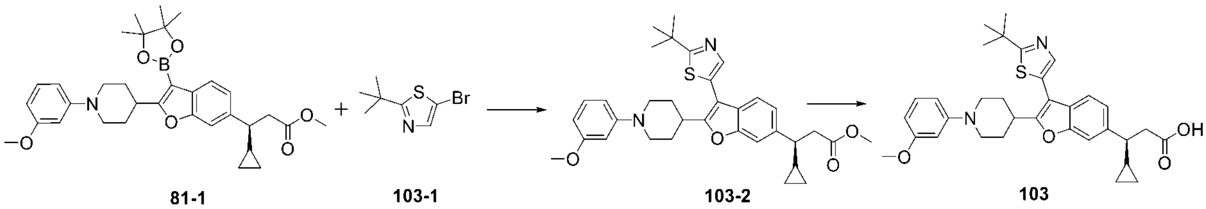

81-1 103-1 103-2 103

Referring to the synthetic route of compound **81,** compound **81-2** was replaced with compound **103-1** to carry out the synthesis. The resulting crude product was purified by reverse-phase column chromatography [acetonitrile/water (0.05% ammonia) = 0% to 100%] to obtain compound **103** (77 mg, yield: 78%) as a white solid. MS (ESI, m/z): 559.2 $[M+H]^+$.

$^1H$ NMR (400 MHz, $CDCl_3$) $\delta$ 7.72 (s, 1H), 7.55 (d, $J$ = 8.0 Hz, 1H), 7.37 (s, 1H), 7.24 - 7.11 (m, 2H), 6.62 - 6.45 (m, 3H), 3.87 - 3.75 (m, 5H), 3.24 - 3.17 (m, 1H), 2.94 - 2.76 (m, 4H), 2.54 - 2.46 (m, 1H), 2.32 - 2.19 (m, 2H), 2.06 - 1.90 (m, 2H), 1.51 (s, 9H), 1.15 - 1.06 (m, 1H), 0.67 - 0.57 (m, 1H), 0.51 - 0.42 (m, 1H), 0.39 - 0.30 (m, 1H), 0.23 - 0.14 (m, 1H).

**Example 104:**

Synthetic Route:

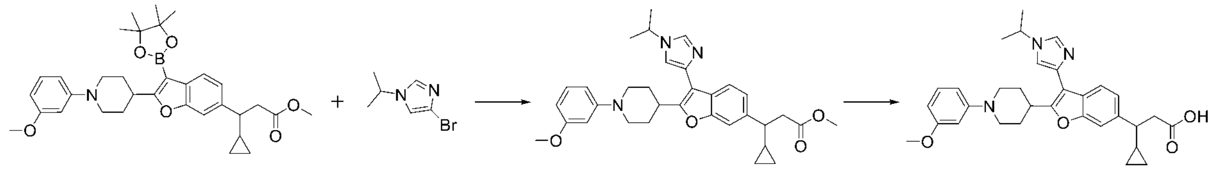

82-3 104-1 104-2 104

Referring to the synthetic route of compound **82,** compound **82-4** was replaced with compound **104-1** to carry out the synthesis. The resulting crude product was purified by reverse-phase column chromatography [acetonitrile/water (0.05% ammonia) = 0% to 100%] to obtain compound **104** (3 mg, yield: 17%) as a white solid. MS (ESI, m/z): 528.2 $[M+H]^+$.

$^1H$ NMR (400 MHz, MeOD) $\delta$ 7.69 (s, 1H), 7.56 (d, $J$ = 8.0 Hz, 1H), 7.33 (s, 1H), 7.21 - 7.13 (m, 3H), 6.65 - 6.61 (m, 1H), 6.56 - 6.52 (m, 1H), 6.47 - 6.40 (m, 1H), 4.48 - 4.40 (m, 1H), 3.81 - 3.77 (m, 6H), 2.91 - 2.76 (m, 4H), 2.53 - 2.43 (m, 1H), 2.24 - 2.13 (m, 2H), 2.03 - 1.94 (m, 2H), 1.58 (d, $J$ = 6.8 Hz, 6H), 1.10 - 1.04 (m, 1H), 0.67 - 0.57 (m, 1H), 0.45 - 0.39 (m, 1H), 0.35 - 0.29 (m, 1H), 0.21 - 0.14 (m, 1H).

**Example 105:**

Synthetic Route:

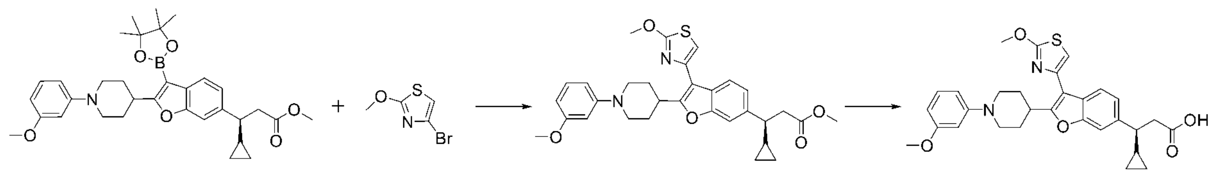

81-1 105-1 105-2 105

Referring to the synthetic route of compound **81,** compound **81-2** was replaced with compound **105-1** to carry out the synthesis. The resulting crude product was purified by reverse-phase column chromatography [acetonitrile/water (0.05% ammonia) = 0% to 100%] to obtain compound **105** (64 mg, yield: 75%) as a white solid. MS (ESI, m/z): 532.9 $[M+H]^+$.

$^1H$ NMR (400 MHz, $CDCl_3$) $\delta$ 7.70 (d, $J$= 8.0 Hz, 1H), 7.35 (s, 1H), 7.24 - 7.15 (m, 2H), 6.79 (s, 1H), 6.67 - 6.38 (m, 3H), 4.13 (s, 3H), 3.89 - 3.63 (m, 6H), 2.91 - 2.82 (m, 3H), 2.53 - 2.47 (m, 1H), 2.25 - 2.17 (m, 2H), 2.08 - 2.01 (m, 2H), 1.29 - 1.19 (m, 1H), 1.12 - 1.04 (m, 1H), 0.66 - 0.55 (m, 1H), 0.48 - 0.39 (m, 1H), 0.37 - 0.29 (m, 1H), 0.23 - 0.13 (m, 1H)

**Example 106:**

Synthetic Route:

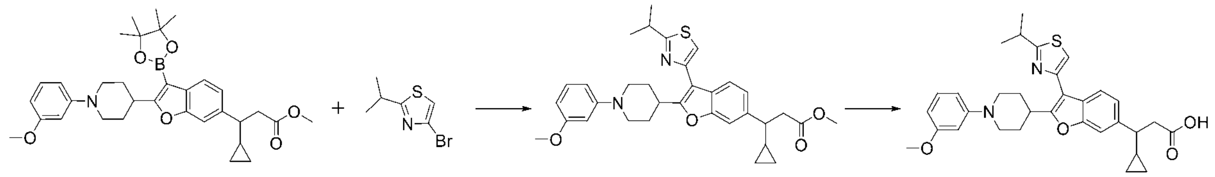

82-3 106-1 106-2 106

Referring to the synthetic route of compound **82,** compound **82-4** was replaced with compound **106-1** to carry out the synthesis. The resulting crude product was purified by reverse-phase column chromatography [acetonitrile/water (0.05% ammonia) = 0% to 100%] to obtain compound **106** (54 mg, yield: 76%) as a white solid. MS (ESI, m/z): 545.2 $[M+H]^+$.

$^1H$ NMR (400 MHz, $CDCl_3$) $\delta$ 7.71 (d, $J$ = 8.0 Hz, 1H), 7.38 - 7.33 (m, 1H), 7.27 - 7.24 (m, 1H), 7.23 - 7.12 (m, 2H), 6.63 (d, $J$ = 8.0 Hz, 1H), 6.56 (s, 1H), 6.44 (d, $J$ = 8.0 Hz, 1H), 3.89 - 3.77 (m, 5H), 3.76 - 3.64 (m, 1H), 3.43 - 3.34 (m, 1H), 2.92 - 2.79 (m, 4H), 2.58 - 2.46 (m, 1H), 2.28 - 2.15 (m, 2H), 2.10 - 1.96 (m, 2H), 1.47 (d, $J$ = 6.8 Hz, 6H), 1.18 - 1.06 (m, 1H), 0.69 - 0.58 (m, 1H), 0.47 - 0.39 (m, 1H), 0.37 - 0.26 (m, 1H), 0.22 - 0.15 (m, 1H).

**Example 107:**

Synthetic Route:

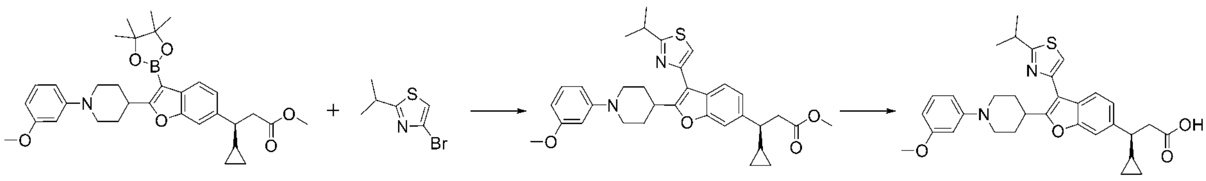

81-1 106-1 107-1 107

Referring to the synthetic route of compound **81,** compound **81-2** was replaced with compound **106-1** to carry out the synthesis. The resulting crude product was purified by reverse-phase column chromatography [acetonitrile/water (0.05% ammonia) = 0% to 100%] to obtain compound **107** (147 mg, yield: 82%) as a white solid. MS (ESI, m/z): 545.2 $[M+H]^+$.

$^1$H NMR (400 MHz, $CDCl_3$) $\delta$ 7.71 (d, *J* = 8.0 Hz, 1H), 7.37 - 7.33 (m, 1H), 7.28 - 7.26 (m, 1H), 7.23 - 7.12 (m, 2H), 6.73 - 6.35 (m, 3H), 3.89 - 3.78 (m, 5H), 3.76 - 3.63 (m, 1H), 3.41 - 3.30 (m, 1H), 2.95 - 2.77 (m, 4H), 2.55 - 2.45 (m, 1H), 2.31 - 2.15 (m, 2H), 2.09 - 1.97 (m, 2H), 1.47 (d, *J*= 6.8 Hz, 6H), 1.16 - 1.03 (m, 1H), 0.66 - 0.55 (m, 1H), 0.48 - 0.39 (m, 1H), 0.37 - 0.27 (m, 1H), 0.24 - 0.13 (m, 1H).

**Example 108:**

Synthetic Route:

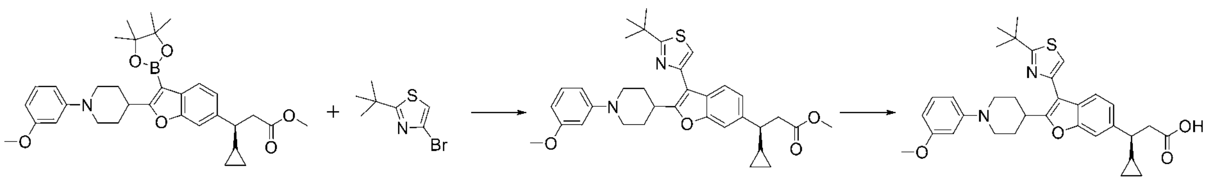

81-1 108-1 108-2 108

Referring to the synthetic route of compound **81,** compound **81-2** was replaced with compound **108-1** to carry out the synthesis. The resulting crude product was purified by reverse-phase column chromatography [acetonitrile/water (0.05% ammonia) = 0% to 100%] to obtain compound **108** (89 mg, yield: 73%) as a white solid. MS (ESI, m/z): 559.2 $[M+H]^+$.

$^1$H NMR (400 MHz, $CDCl_3$) $\delta$ 7.72 (d, *J* = 8.0 Hz, 1H), 7.35 (s, 1H), 7.28 (s, 1H), 7.23 - 7.13 (m, 2H), 6.68 - 6.60 (m, 1H), 6.56 (s, 1H), 6.48 - 6.38 (m, 1H), 3.91 - 3.77 (m, 5H), 3.75 - 3.64 (m, 1H), 2.93 - 2.76(m, 4H), 2.57 - 2.48 (m, 1H), 2.32 - 2.17 (m, 2H), 2.12 - 2.04 (m, 2H), 1.49 (s, 9H), 1.15 - 1.06 (m, 1H), 0.66 - 0.55 (m, 1H), 0.48 - 0.38 (m, 1H), 0.36 - 0.27 (m, 1H), 0.22 -0.12(m, 1H).

**Example 109:**

Synthetic Route:

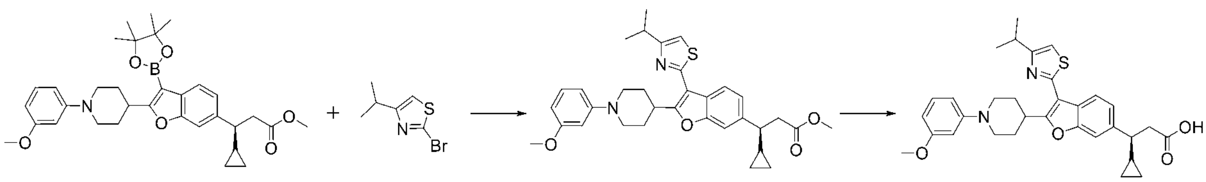

81-1 109-1 109-2 109

Referring to the synthetic route of compound **81,** compound **81-2** was replaced with compound **109-1** to carry out the synthesis. The resulting crude product was purified by reverse-phase column chromatography [acetonitrile/water (0.05% ammonia) = 0% to 100%] to obtain compound 109 (133 mg, yield: 77%) as a white solid. MS (ESI, m/z): 545.2 $[M+H]^+$.

$^1H$ NMR (400 MHz, $CDCl_3$) $\delta$ 7.97 (d, *J*= 8.0 Hz, 1H), 7.38 (s, 1H), 7.25 - 7.21 (m, 2H), 6.91 (s, 1H), 6.78 - 6.33 (m, 3H), 3.90 - 3.74 (m, 6H), 3.24 - 3.09 (m, 1H), 3.01 - 2.76 (m, 4H), 2.57 - 2.43 (m, 1H), 2.35 - 2.00 (m, 4H), 1.38 (d, *J* = 6.8 Hz, 6H), 1.15 - 1.01 (m, 1H), 0.69 - 0.55 (m, 1H), 0.49 - 0.39 (m, 1H), 0.38 - 0.26 (m, 1H), 0.22 - 0.14 (m, 1H).

**Example 110:**

Synthetic Route:

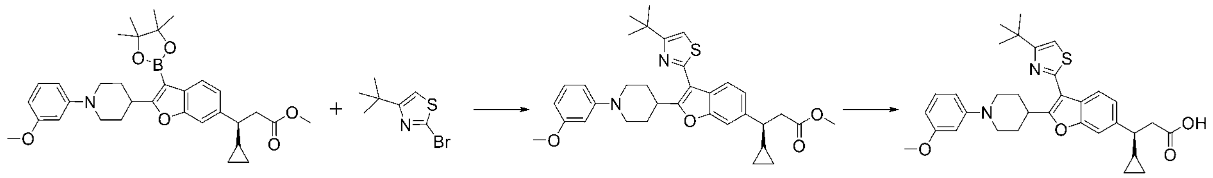

81-1 110-1 110-2 110

Referring to the synthetic route of compound **81,** compound **81-2** was replaced with compound **110-1** to carry out the synthesis. The resulting crude product was purified by reverse-phase column chromatography [acetonitrile/water (0.05% ammonia) = 0% to 100%] to obtain compound **110** (88 mg, yield: 73%) as a white solid. MS (ESI, m/z): 559.2 $[M+H]^+$.

$^1H$ NMR (400 MHz, $CDCl_3$) $\delta$ 7.97 (d, *J* = 8.0 Hz, 1H), 7.38 (s, 1H), 7.22 (d, *J*= 8.0 Hz, 2H), 6.93 (s, 1H), 6.78 - 6.31 (m, 3H), 3.88 - 3.78 (m, 6H), 3.02 - 2.77 (m, 4H), 2.55 - 2.47 (m, 1H), 2.36 - 2.11 (m, 4H), 1.41 (s, 9H), 1.17 - 1.08 (m, 1H), 0.67 - 0.57 (m, 1H), 0.48 - 0.40 (m, 1H), 0.37 - 0.26 (m, 1H), 0.22 - 0.16 (m, 1H).

**Example 111:**

Synthetic Route:

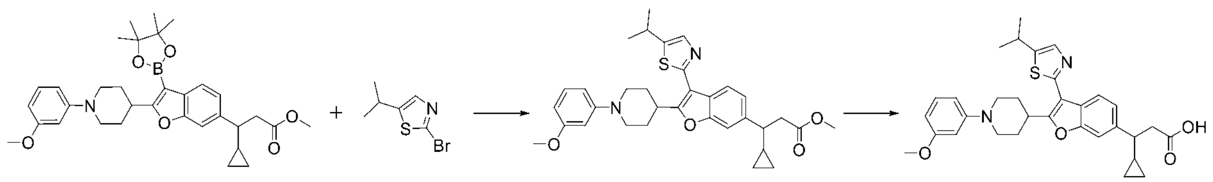

82-3 111-1 111-2 111

Referring to the synthetic route of compound **82,** compound **82-4** was replaced with compound **111-1** to carry out the synthesis. The resulting crude product was purified by reverse-phase column chromatography [acetonitrile/water (0.05% ammonia) = 0% to 100%] to obtain compound **111** (69 mg, yield: 79%) as a white solid. MS (ESI, m/z): 545.2 $[M+H]^+$.

$^1H$ NMR (400 MHz, $CDCl_3$) $\delta$ 7.94 (d, *J* = 8.0 Hz, 1H), 7.64 - 7.56 (m, 1H), 7.41 - 7.34 (m, 2H), 7.23 - 7.15 (m, 2H), 6.68 - 6.60 (m, 1H), 6.56 (s, 1H), 6.50 - 6.39 (m, 1H), 3.87 - 3.78 (m, 5H), 3.77 - 3.70 (m, 1H), 3.35 - 3.25 (m, 1H), 2.99 - 2.88 (m, 2H), 2.85 - 2.76 (m, 2H), 2.56 - 2.42 (m, 1H), 2.29 - 2.20 (m, 2H), 2.07 - 1.98 (m, 2H), 1.41 (d, *J = 6.8* Hz, 6H), 1.13 - 1.04 (m, 1H), 0.65 - 0.55 (m, 1H), 0.47 - 0.39 (m, 1H), 0.35 - 0.24 (m, 1H), 0.21 - 0.14 (m, 1 H).

**Example 112:**

Synthetic Route:

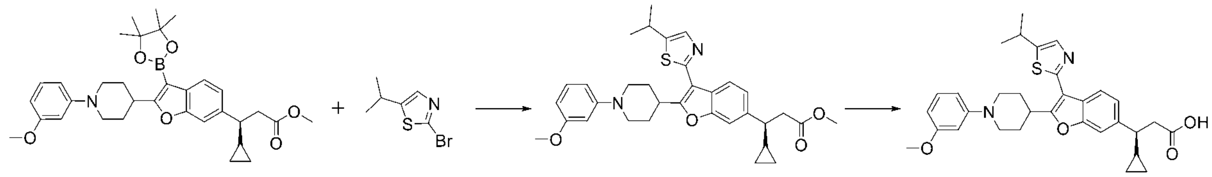

81-1 111-1 112-1 112

Referring to the synthetic route of compound **81,** compound **81-2** was replaced with compound **111-1** to carry out the synthesis. The resulting crude product was purified by reverse-phase column chromatography [acetonitrile/water (0.05% ammonia) = 0% to 100%] to obtain compound **112** (92 mg, yield: 89%) as a white solid. MS (ESI, m/z): 545.2 $[M+H]^+$.

$^1H$ NMR (400 MHz, $CDCl_3$) $\delta$ 7.94 (d, $J$ = 8.0 Hz, 1H), 7.62 - 7.57 (m, 1H), 7.40 - 7.35 (m, 1H), 7.25 - 7.15 (m, 3H), 6.74 - 6.35 (m, 2H), 3.90 - 3.70 (m, 6H), 3.34 - 3.22 (m, 1H), 3.04 - 2.76 (m, 4H), 2.55 - 2.45 (m, 1H), 2.36 - 2.13 (m, 2H), 2.12 - 1.97 (m, 2H), 1.41 (d, $J$ = 6.8 Hz, 6H), 1.14 - 1.03 (m, 1H), 0.66 - 0.55 (m, 1H), 0.48 - 0.39 (m, 1H), 0.37 - 0.28 (m, 1H), 0.24 - 0.14 (m, 1H).

**Example 113:**

Synthetic Route:

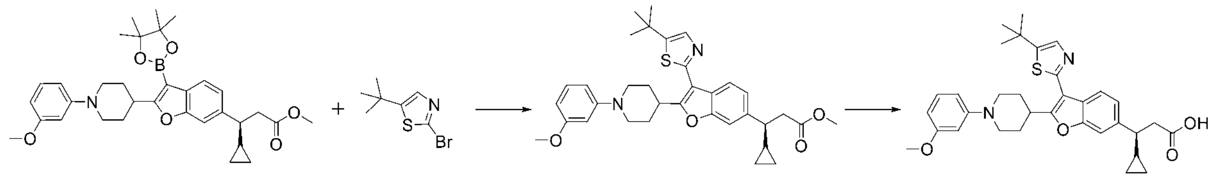

81-1 113-1 113-2 113

Referring to the synthetic route of compound **81,** compound **81-2** was replaced with compound **113-1** to carry out the synthesis. The resulting crude product was purified by reverse-phase column chromatography [acetonitrile/water (0.05% ammonia) = 0% to 100%] to obtain compound **113** (31 mg, yield: 33%) as a white solid. MS (ESI, m/z): 559.2 $[M+H]^+$.

$^1H$ NMR (400 MHz, $CDCl_3$) $\delta$ 7.95 (d, $J$ = 8.0 Hz, 1H), 7.63 - 7.57 (m, 1H), 7.42 - 7.35 (m, 1H), 7.24 - 7.16 (m, 2H), 6.70 - 6.40 (m, 3H), 3.90 - 3.70 (m, 6H), 3.04 - 2.87 (m, 2H), 2.85 - 2.62 (m, 2H), 2.55 - 2.46 (m, 1H), 2.36 - 2.22 (m, 2H), 2.12 - 1.98 (m, 2H), 1.46 (s, 9H), 1.14 - 1.04 (m, 1H), 0.66 - 0.56 (m, 1H), 0.49 - 0.41 (m, 1H), 0.38 - 0.27 (m, 1H), 0.23 - 0.14 (m, 1H).

**Example 114:**

Synthetic Route:

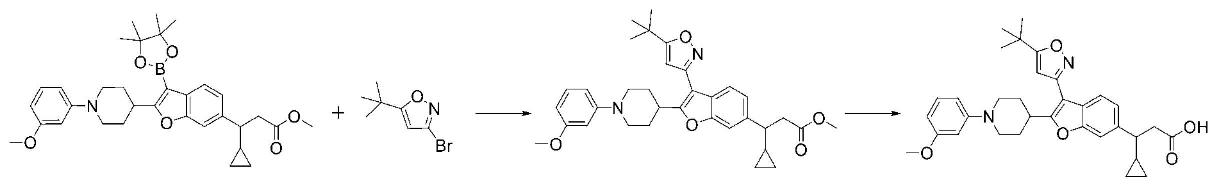

82-3 114-1 114-2 114

Referring to the synthetic route of compound **82,** compound **82-4** was replaced with compound **114-1** to carry out the synthesis. The resulting crude product was purified by reverse-phase column chromatography [acetonitrile/water (0.05% ammonia) = 0% to 100%] to obtain compound **114** (12 mg, yield: 28%) as a white solid. MS (ESI, m/z): 543.2 $[M+H]^+$.

$^1H$ NMR (400 MHz, $CDCl_3$) $\delta$ 7.66 (d, $J$= 8.0 Hz, 1H), 7.38 (s, 1H), 7.24 - 7.15 (m, 2H), 6.68 - 6.58 (m, 1H), 6.57 -

6.50 (m, 1H), 6.49 - 6.41 (m, 1H), 6.40 (s, 1H), 3.89 - 3.76 (m, 5H), 3.66 - 3.50 (m, 1H), 3.00 - 2.76 (m, 4H), 2.56 - 2.44 (m, 1H), 2.30 - 2.12 (m, 2H), 2.07 - 1.95 (m, 2H), 1.44 (s, 9H), 1.13 - 1.01 (m, 1H), 0.68 - 0.56 (m, 1H), 0.50 - 0.39 (m, 1H), 0.38 - 0.28 (m, 1H), 0.24 - 0.14 (m, 1H).

**Example 115:**

Synthetic Route:

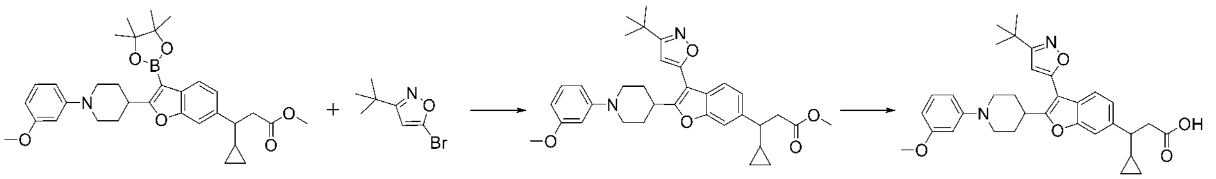

82-3 115-1 115-2 115

Referring to the synthetic route of compound **82,** compound **82-4** was replaced with compound **115-1** to carry out the synthesis. The resulting crude product was purified by reverse-phase column chromatography [acetonitrile/water (0.05% ammonia) = 0% to 100%] to obtain compound **115** (21 mg, yield: 64%) as a white solid. MS (ESI, m/z): 543.2 $[M+H]^+$.

$^1$H NMR (400 MHz, $CDCl_3$) δ 7.69 (d, *J* = 7.6 Hz, 1H), 7.42 - 7.35 (m, 1H), 7.23 - 7.12 (m, 2H), 6.67 - 6.58 (m, 1H), 6.58 - 6.49 (m, 1H), 6.48 - 6.39 (m, 1H), 6.31 - 6.24 (m, 1H), 3.86 - 3.80 (m, 5H), 3.55 - 3.41 (m, 1H), 3.00 - 2.76 (m, 5H), 2.30 - 2.16 (m, 2H), 2.05 - 1.97 (m, 2H), 1.45 (s, 9H), 1.16 - 1.01 (m, 1H), 0.68 - 0.57 (m, 1H), 0.51 - 0.39 (m, 1H), 0.38 - 0.28 (m, 1H), 0.26 - 0.13 (m, 1H).

**Example 116:**

Synthetic Route:

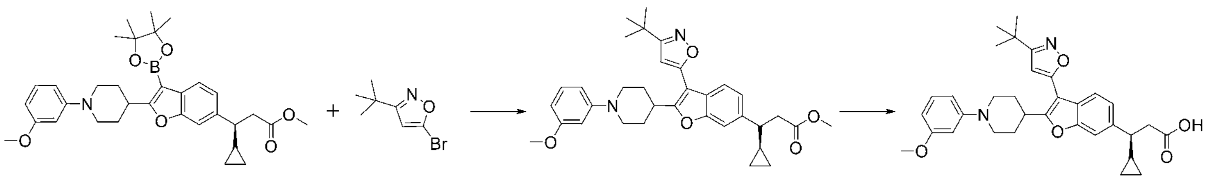

81-1 115-1 116-1 116

Referring to the synthetic route of compound **81,** compound **81-2** was replaced with compound **115-1** to carry out the synthesis. The resulting crude product was purified by reverse-phase column chromatography [acetonitrile/water (0.05% ammonia) = 0% to 100%] to obtain compound **116** (16 mg, yield: 31%) as a white solid. MS (ESI, m/z): 543.2 $[M+H]^+$.

$^1$H NMR (400 MHz, $CDCl_3$) δ 7.67 (d, *J*= 8.0 Hz, 1H), 7.38 (s, 1H), 7.23 - 7.14 (m, 2H), 6.67 - 6.60 (m, 1H), 6.55 (s, 1H), 6.48 - 6.40 (m, 1H), 6.26 (s, 1H), 3.86 - 3.77 (m, 5H), 3.54 - 3.42 (m, 1H), 3.01 - 2.76 (m, 4H), 2.55 - 2.44 (m, 1H), 2.32 - 2.14 (m, 2H), 2.08 - 1.93 (m, 2H), 1.46 (s, 9H), 1.12 - 1.04 (m, 1H), 0.66 - 0.56 (m, 1H), 0.49 - 0.39 (m, 1H), 0.37 - 0.28 (m, 1H), 0.23 - 0.14 (m, 1H).

**Example 117:**

Synthetic Route:

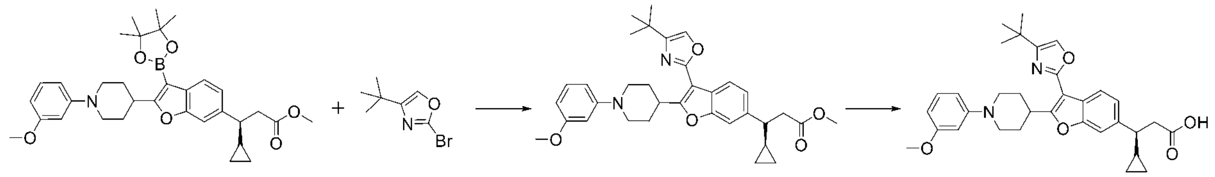

81-1 117-1 117-2 117

Referring to the synthetic route of compound **81,** compound **81-2** was replaced with compound **117-1** to carry out the synthesis. The resulting crude product was purified by reverse-phase column chromatography [acetonitrile/water (0.05% ammonia) = 0% to 100%] to obtain compound **117** (39 mg, yield: 72%) as a white solid. MS (ESI, m/z): 543.2 $[M+H]^+$.

$^1$H NMR (400 MHz, DMSO-$d_6$) δ 11.97 (s, 1H), 7.97 (d, *J* = 8.0 Hz, 1H), 7.93 (s, 1H), 7.56 (s, 1H), 7.31 - 7.26 (m, 1H), 7.13 (t, *J* = 8.4 Hz, 1H), 6.62 - 6.57 (m, 1H), 6.54 - 6.50 (m, 1H), 6.40 - 6.34 (m, 1H), 3.93 - 3.85 (m, 2H), 3.84 - 3.76 (m, 1H), 3.73 (s, 3H), 2.92 - 2.81 (m, 2H), 2.76 - 2.67 (m, 2H), 2.45 - 2.39 (m, 1H), 2.08 - 1.95 (m, 4H), 1.30 (s, 9H), 1.12 - 1.04 (m, 1H), 0.58 - 0.48 (m, 1H), 0.38 - 0.25 (m, 2H), 0.21 - 0.10 (m, 1H).

**Example 118:**

Synthetic Route:

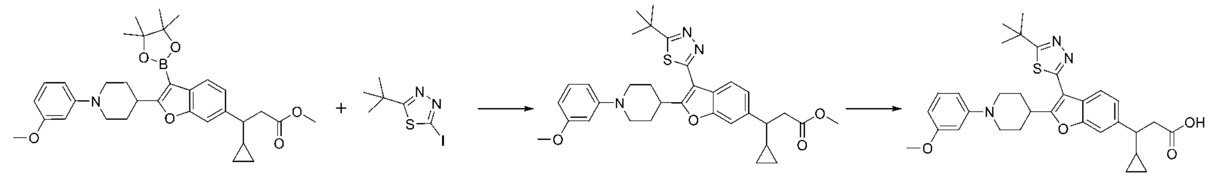

82-3 118-1 118-2 118

Referring to the synthetic route of compound **82,** compound **82-4** was replaced with compound **118-1** to carry out the synthesis. The resulting crude product was purified by reverse-phase column chromatography [acetonitrile/water (0.05% ammonia) = 0% to 100%] to obtain compound **118** (8 mg, yield: 9%) as a white solid. MS (ESI, m/z): 560.2 $[M+H]^+$.

$^1$H NMR (400 MHz, $CDCl_3$) *δ* 7.89 (d, *J* = 8.0 Hz, 1H), 7.42 (s, 1H), 7.27 - 7.17 (m, 2H), 6.69 - 6.61 (m, 1H), 6.57 (s, 1H), 6.49 - 6.40 (m, 1H), 3.96 - 3.73 (m, 6H), 3.82 - 3.75 (m, 1H), 3.04 - 2.92 (m, 2H), 2.92 - 2.80 (m, 2H), 2.58 - 2.44 (m, 1H), 2.31 - 2.17 (m, 2H), 2.10 - 2.03 (m, 2H), 1.59 (s, 9H), 1.16 - 1.04 (m, 1H), 0.70 - 0.55 (m, 1H), 0.52 - 0.40 (m, 1H), 0.39 - 0.29 (m, 1H), 0.26 - 0.12 (m, 1H).

**Example 119:**

Synthetic Route:

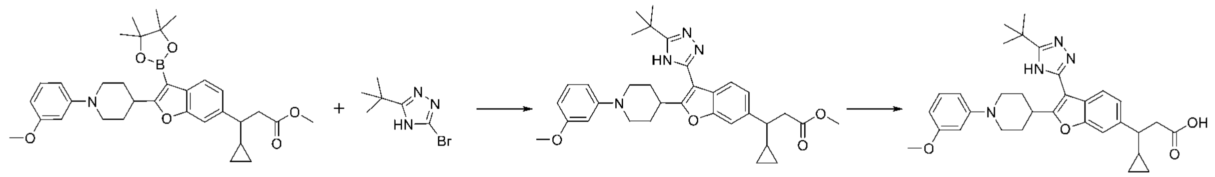

82-3 119-1 119-2 119

Referring to the synthetic route of compound **82,** compound **82-4** was replaced with compound **119-1** to carry out the synthesis. The resulting crude product was purified by reverse-phase column chromatography [acetonitrile/water (0.05% formic acid) = 0% to 100%] to obtain compound **119** (4 mg, yield: 4%) as a yellow solid. MS (ESI, m/z): 543.2 $[M+H]^+$.

$^1$H NMR (400 MHz, $CDCl_3$) *δ* 7.91 (d, *J* = 8.0 Hz, 1H), 7.49 - 7.43 (m, 2H), 7.22 (t, *J* = 8.0 Hz, 1H), 6.69 - 6.62 (m, 1H), 6.61 - 6.56 (m, 1H), 6.49 - 6.43 (m, 1H), 4.11 - 3.99 (m, 1H), 3.89 - 3.80 (m, 5H), 3.10 - 3.01 (m, 1H), 2.97 - 2.88 (m, 3H),

2.63 - 2.54 (m, 2H), 2.25 - 2.12 (m, 2H), 2.08 - 2.04 (m, 2H), 1.43 (s, 9H), 1.22 - 1.16 (m, 1H), 0.74 - 0.67 (m, 1H), 0.57 - 0.46 (m, 1H), 0.41 - 0.36 (m, 1H), 0.33 - 0.26 (m, 1H).

**Example 120:**

Synthetic Route:

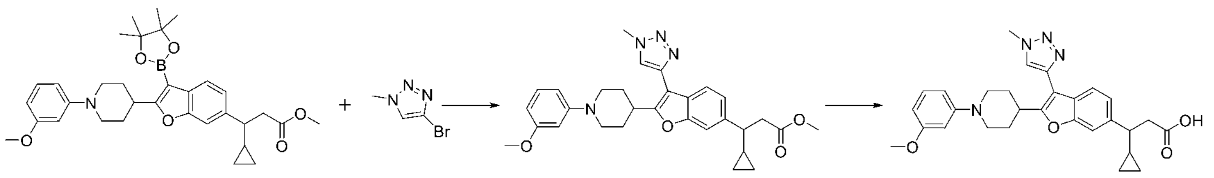

82-3 120-1 120-2 120

Referring to the synthetic route of compound **82,** compound **82-4** was replaced with compound **120-1** to carry out the synthesis. The resulting crude product was purified by reverse-phase column chromatography [acetonitrile/water (0.05% ammonia) = 0% to 100%] to obtain compound **120** (19 mg, yield: 66%) as a white solid. MS (ESI, m/z): 501.2 $[M+H]^+$.

$^1H$ NMR (400 MHz, DMSO-$d_6$) δ 8.57 (s, 1H), 7.79 (d, *J* = 8.0 Hz, 1H), 7.50 (s, 1H), 7.21 (d, *J*= 8.0 Hz, 1H), 7.12 - 7.08 (m, 1H), 6.64 - 6.55 (m, 1H), 6.54 - 6.46 (m, 1H), 6.41 - 6.32 (m, 1H), 4.15 (s, 3H), 3.90 - 3.81 (m, 2H), 3.73 (s, 3H), 3.65 - 3.55 (m, 1H), 2.94 - 2.84 (m, 2H), 2.76 - 2.64 (m, 2H), 2.44 - 2.35 (m, 1H), 2.07 - 1.87 (m, 4H), 1.12 - 1.03 (m, 1H), 0.57 - 0.47 (m, 1H), 0.35 - 0.22 (m, 2H), 0.19 - 0.10 (m, 1H).

**Example 121:**

Synthetic Route:

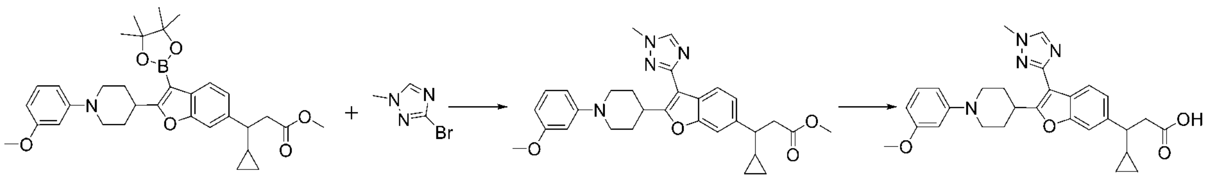

82-3 121-1 121-2 121

Referring to the synthetic route of compound **82,** compound **82-4** was replaced with compound **121-1** to carry out the synthesis. The resulting crude product was purified by reverse-phase column chromatography [acetonitrile/water (0.05% ammonia) = 0% to 100%] to obtain compound **121** (10 mg, yield: 34%) as a white solid. MS (ESI, m/z): 501.2 $[M+H]^+$.

$^1H$ NMR (400 MHz, DMSO-$d_6$) δ 8.60 (s, 1H), 8.04 (d, *J* = 8.0 Hz, 1H), 7.49 (s, 1H), 7.21 (d, *J*= 8.0 Hz, 1H), 7.16 - 7.09 (m, 1H), 6.63 - 6.55 (m, 1H), 6.54 - 6.47 (m, 1H), 6.41 - 6.33 (m, 1H), 4.09 - 3.94 (m, 4H), 3.91 - 3.82 (m, 2H), 3.73 (s, 3H), 2.90 - 2.80 (m, 2H), 2.72 - 2.65 (m, 2H), 2.43 - 2.34 (m, 1H), 2.09 - 1.88 (m, 4H), 1.10 - 1.03 (m, 1H), 0.55 - 0.48 (m, 1H), 0.34 - 0.23 (m, 2H), 0.19 - 0.11 (m, 1H).

**Example 122:**

Synthetic Route:

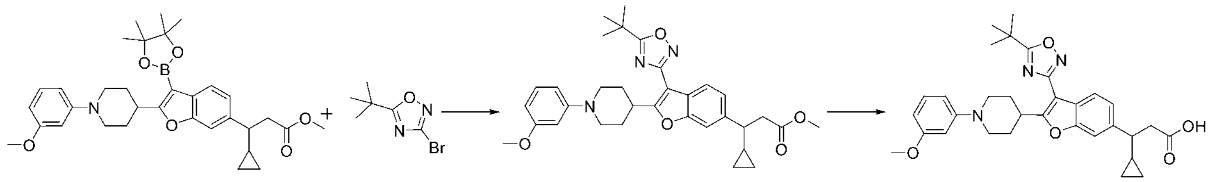

82-3 122-1 122-2 122

Referring to the synthetic route of compound **82,** compound **82-4** was replaced with compound **122-1** to carry out the synthesis. The resulting crude product was purified by reverse-phase column chromatography [acetonitrile/water (0.05% ammonia) = 0% to 100%] to obtain compound **122** (8 mg, yield: 15%) as a white solid. MS (ESI, m/z): 544.2 [M+H]$^+$.

$^1$H NMR (400 MHz, $CDCl_3$) $\delta$ 8.08 (d, *J* = 8.0 Hz, 1H), 7.45 - 7.36 (m, 1H), 7.24 (d, *J = 8.4* Hz, 2H), 6.82 - 6.31 (m, 3H), 4.01 - 3.69 (m, 6H), 3.06 - 2.77 (m, 4H), 2.59 - 2.45 (m, 1H), 2.39 - 2.13 (m, 2H), 2.14 - 1.97 (m, 2H), 1.53 (s, 9H), 1.14 - 1.06 (m, 1H), 0.68 - 0.57 (m, 1H), 0.51 - 0.41 (m, 1H), 0.38 - 0.30 (m, 1H), 0.27 - 0.15 (m, 1H).

**Example 123:**

Synthetic Route:

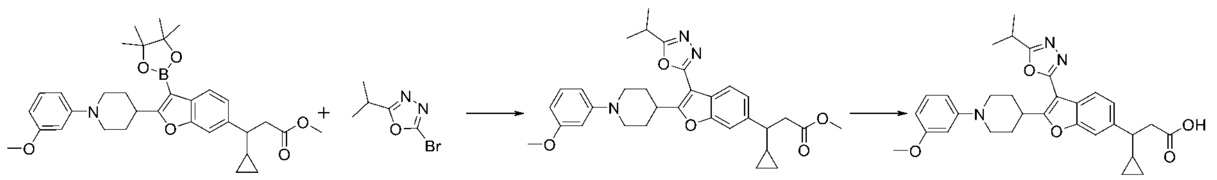

82-3 123-1 123-2 123

Referring to the synthetic route of compound **82,** compound **82-4** was replaced with compound **123-1** to carry out the synthesis. The resulting crude product was purified by reverse-phase column chromatography [acetonitrile/water (0.05% formic acid) = 0% to 100%] to obtain compound **123** (4 mg, yield: 11%) as a white solid. MS (ESI, m/z): 530.2 [M+H]$^+$.

$^1$H NMR (400 MHz, $CDCl_3$) $\delta$ 7.97 - 7.92 (m, 1H), 7.44 (s, 1H), 7.26 - 7.24 (m, 2H), 6.70 - 6.59 (m, 1H), 6.60 - 6.54 (m, 1H), 6.54 - 6.40 (m, 1H), 3.96 - 3.86 (m, 2H), 3.84 (s, 3H), 3.82 - 3.79 (m, 1H), 3.41 - 3.28 (m, 1H), 3.04 - 2.91 (m, 2H), 2.91 - 2.80 (m, 2H), 2.57 - 2.48 (m, 1H), 2.34 - 2.15 (m, 2H), 2.15 - 2.02 (m, 2H), 1.52 (s, 6H), 1.17 - 1.07 (m, 1H), 0.72 - 0.61 (m, 1H), 0.53 - 0.42 (m, 1H), 0.40 - 0.32 (m, 1H), 0.28 - 0.16 (m, 1H).

**Example 124:**

Synthetic Route:

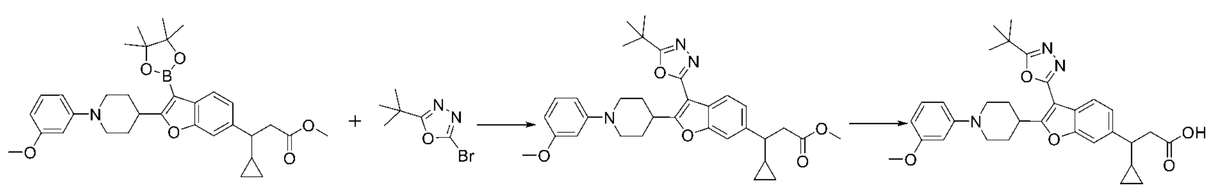

82-3 124-1 124-2 124

Referring to the synthetic route of compound **82,** compound **82-4** was replaced with compound **124-1** to carry out the synthesis. The resulting crude product was purified by reverse-phase column chromatography [acetonitrile/water (0.05% formic acid) = 0% to 100%] to obtain compound **124** (10 mg, yield: 31%) as a white solid. MS (ESI, m/z): 544.3 [M+H]$^+$.

$^1$H NMR (400 MHz, $CDCl_3$) $\delta$ 7.94 (d, *J*= 8.0 Hz, 1H), 7.42 (s, 1H), 7.26 - 7.19 (m, 2H), 6.68 - 6.61 (m, 1H), 6.61 - 6.54 (m, 1H), 6.50 - 6.40 (m, 1H), 3.87 - 3.83 (m, 5H), 3.01 - 2.80 (m, 5H), 2.57 - 2.47 (m, 1H), 2.27 - 2.15 (m, 2H), 2.10 - 2.03 (m, 2H), 1.54 (s, 9H), 1.13 - 1.08 (m, 1H), 0.68 - 0.61 (m, 1H), 0.48 - 0.42 (m, 1H), 0.38 - 0.32 (m, 1H), 0.23 - 0.18 (m, 1H).

**Example 125:**

Synthetic Route:

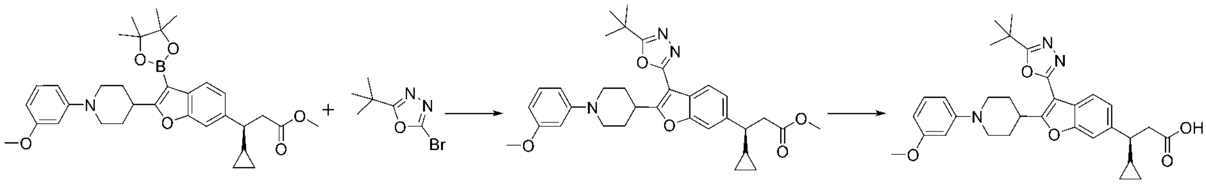

81-1 124-1 125-1 125

Referring to the synthetic route of compound **81,** compound **81-2** was replaced with compound **124-1** to carry out the synthesis. The resulting crude product was purified by reverse-phase column chromatography [acetonitrile/water (0.05% formic acid) = 0% to 100%] to obtain compound **125** (118 mg, yield: 68%) as a white solid. MS (ESI, m/z): 544.3 $[M+H]^+$.

$^1H$ NMR (400 MHz, $CDCl_3$) $\delta$ 7.92 (d, $J$ = 8.4 Hz, 1H), 7.40 (s, 1H), 7.26 - 7.14 (m, 2H), 6.66 - 6.58 (m, 1H), 6.55 (s, 1H), 6.48 - 6.38 (m, 1H), 3.91 - 3.71 (m, 6H), 2.98 - 2.79 (m, 4H), 2.56 - 2.47 (m, 1H), 2.31 - 2.13 (m, 2H), 2.12 - 1.99 (m, 2H), 1.52 (s, 9H), 1.14 - 1.05 (m, 1H), 0.69 - 0.57 (m, 1H), 0.49 - 0.39 (m, 1H), 0.36 - 0.28 (m, 1H), 0.21 - 0.16 (m, 1H).

**Example 126:**

Synthetic Route:

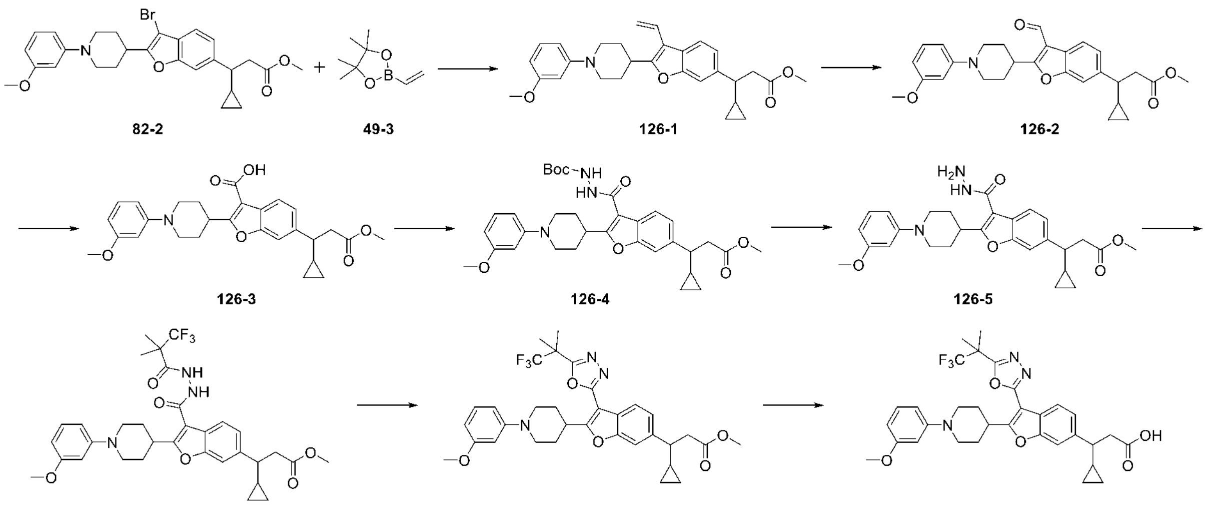

126-6 126-7 126

Referring to the synthetic route of compound **49,** compound **49-2** was replaced with compound **82-2** to carry out the synthesis. The resulting crude product was purified by reverse-phase column chromatography [acetonitrile/water (0.05% formic acid) = 0% to 100%] to obtain compound **126** (4 mg, yield: 9%) as a white solid. MS (ESI, m/z): 598.3 $[M+H]^+$.

$^1H$ NMR (400 MHz, $CDCl_3$) $\delta$ 7.94 (d, $J$ = 8.0 Hz, 1H), 7.41 (s, 1H), 7.30 - 7.19 (m, 2H), 6.68 - 6.59 (m, 1H), 6.59 - 6.50 (m, 1H), 6.49 - 6.38 (m, 1H), 3.90 - 3.83 (m, 2H), 3.80 (s, 3H), 3.77 - 3.75 (m, 1H), 2.98 - 2.82 (m, 4H), 2.58 - 2.43 (m, 1H), 2.30 - 2.16 (m, 2H), 2.10 - 2.03 (m, 2H), 1.78 (s, 6H), 1.11-1.07 (m, 1H), 0.67 - 0.60 (m, 1H), 0.49 - 0.41 (m, 1H), 0.37 - 0.32 (m, 1H), 0.25 - 0.16 (m, 1H).

**Example 127:**

Synthetic Route:

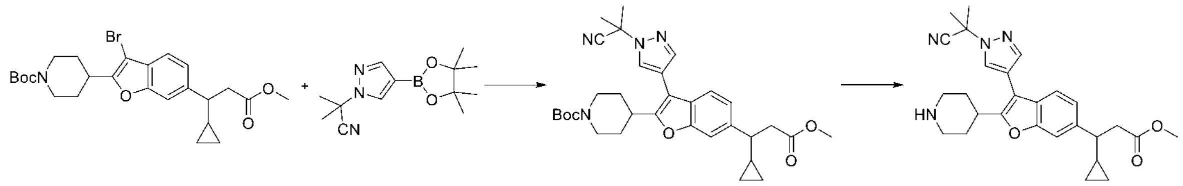

1-1 127-1 127-2 127-3

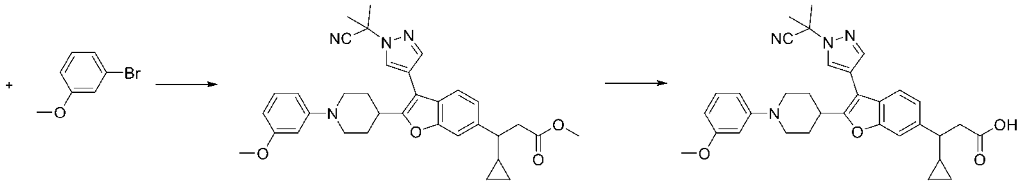

11-1 127-4 127

Referring to the synthetic route of compound **6,** compound **6-1** was replaced with compound **127-1** to carry out the synthesis. The resulting crude product was purified by reverse-phase column chromatography [acetonitrile/water (0.05% formic acid) = 0% to 100%] to obtain compound **127** (8 mg, yield: 14%) as a white solid. MS (ESI, m/z): 553.2 $[M+H]^+$.

$^1$H NMR (400 MHz, $CDCl_3$) $\delta$ 7.77 (s, 1H), 7.70 (s, 1H), 7.36 (d, *J* = 8.0 Hz, 1H), 7.25 - 7.00 (m, 3H), 6.59 - 6.49 (m, 1H), 6.47 - 6.39 (m, 1H), 6.38 - 6.27 (m, 1H), 3.85 - 3.65 (m, 5H), 3.05 - 2.91 (m, 1H), 2.82 - 2.72 (m, 4H), 2.49 - 2.32 (m, 1H), 2.25 - 2.10 (m, 2H), 2.02 (s, 6H), 1.87 - 1.76 (m, 2H), 1.11 - 0.93 (m, 1H), 0.57 - 0.52 (m, 1H), 0.39 - 0.33 (m, 1H), 0.28 - 0.25 (m, 1H), 0.14 - 0.10 (m, 1H).

**Example 128:**

Synthetic Route:

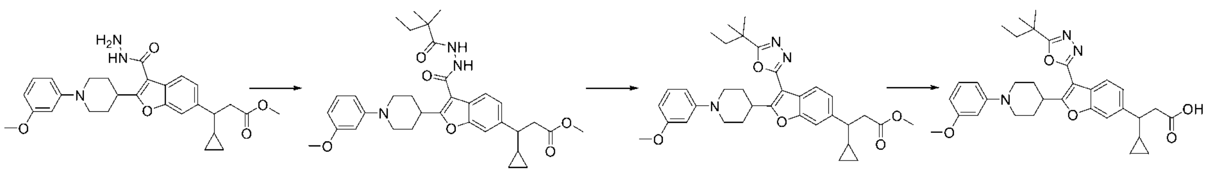

126-5 128-1 128-2 128

Referring to the synthetic route of compound **126,** 3,3,3-trifluoro-2,2-dimethylpropionic acid was replaced with 2,2-dimethylbutyric acid to carry out the synthesis. The resulting crude product was purified by reverse-phase column chromatography [acetonitrile/water (0.05% formic acid) = 0% to 100%] to obtain compound **128** (5 mg, yield: 14%) as a white solid. MS (ESI, m/z): 558.3 $[M+H]^+$.

$^1$H NMR (400 MHz, $CDCl_3$) $\delta$ 7.94 (d, *J* = 8.0 Hz, 1H), 7.42 (s, 1H), 7.27 - 7.20 (m, 2H), 6.68 - 6.59 (m, 1H), 6.56 (s, 1H), 6.48 - 6.40 (m, 1H), 3.91 - 3.84 (m, 2H), 3.83 (s, 3H), 3.81 - 3.79 (m, 1H), 2.97 - 2.82 (m, 4H), 2.54 - 2.51 (m, 1H), 2.29 - 2.17 (m, 2H), 2.09 - 2.01 (m, 2H), 1.88 - 1.82 (m, 2H), 1.50 (s, 6H), 1.13 - 1.05 (m, 1H), 0.95 - 0.88 (m, 3H), 0.69 - 0.57 (m, 1H), 0.52 - 0.40 (m, 1H), 0.37 - 0.30 (m, 1H), 0.24 - 0.15 (m, 1H).

**Example 129:**

Synthetic Route:

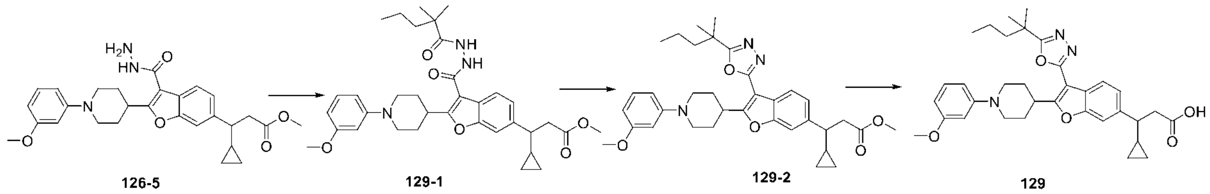

126-5 129-1 129-2 129

Referring to the synthetic route of compound **126,** 3,3,3-trifluoro-2,2-dimethylpropionic acid was replaced with 2,2-dimethylpentanoic acid to carry out the synthesis. The resulting crude product was purified by reverse-phase column chromatography [acetonitrile/water (0.05% formic acid) = 0% to 100%] to obtain compound **129** (15 mg, yield: 37%) as a white solid. MS (ESI, m/z): 572.3 $[M+H]^+$.

$^1H$ NMR (400 MHz, $CDCl_3$) δ 7.95 (d, *J*= 8.0 Hz, 1H), 7.44 (s, 1H), 7.33 - 7.30 (m, 1H), 7.29 - 7.20 (m, 1H), 6.91 - 6.40 (m, 3H), 3.94 - 3.79 (m, 6H), 3.06 - 2.83 (m, 4H), 2.54 (q, *J*= 8.0 Hz, 1H), 2.32 - 2.19 (m, 2H), 2.13 - 2.06 (m, 2H), 1.82 - 1.78 (m, 2H), 1.53 (s, 6H), 1.39 - 1.33 (m, 2H), 1.17 - 1.07 (m, 1H), 0.94 (t, *J*= 7.2 Hz, 3H), 0.70 - 0.63 (m, 1H), 0.54 - 0.44 (m, 1H), 0.41 - 0.34 (m, 1H), 0.25 - 0.21 (m, 1H).

**Example 130:**

Synthetic Route:

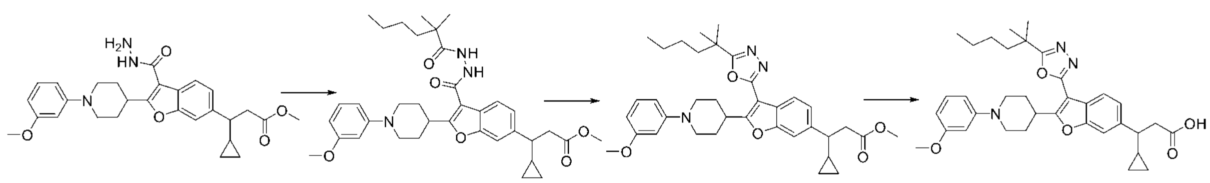

126-5 130-1 130-2 130

Referring to the synthetic route of compound **126,** 3,3,3-trifluoro-2,2-dimethylpropionic acid was replaced with 2,2-dimethylhexanoic acid to carry out the synthesis. The resulting crude product was purified by reverse-phase column chromatography [acetonitrile/water (0.05% formic acid) = 0% to 100%] to obtain compound **130** (29 mg, yield: 36%) as a white solid. MS (ESI, m/z): 586.3 $[M+H]^+$.

$^1H$ NMR (400 MHz, $CDCl_3$) δ 7.94 (d, *J* = 8.0 Hz, 1H), 7.41 (s, 1H), 7.27 - 7.13 (m, 2H), 6.68 - 6.60 (m, 1H), 6.56 (s, 1H), 6.50 - 6.41 (m, 1H), 3.93 - 3.72 (m, 6H), 3.01 - 2.78 (m, 4H), 2.51 (q, *J* = 8.0 Hz, 1H), 2.30 - 2.14 (m, 2H), 2.12 - 2.00 (m, 2H), 1.85 - 1.73 (m, 2H), 1.50 (s, 6H), 1.37 - 1.27 (m, 4H), 1.13 - 1.05 (m, 1H), 0.89 (t, *J* = 6.8 Hz, 3H), 0.70 - 0.58 (m, 1H), 0.48- 0.41 (m, 1H), 0.38 - 0.30 (m, 1H), 0.23 - 0.14 (m, 1H).

**Example 131:**

Synthetic Route:

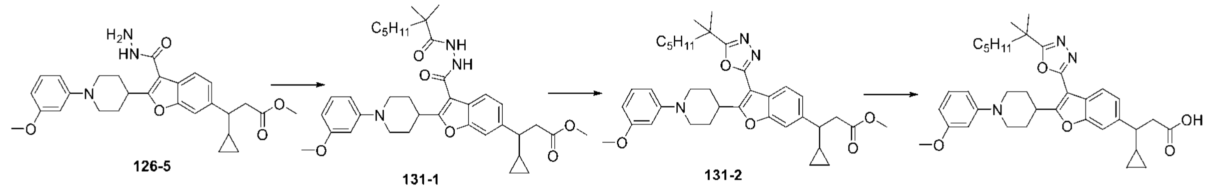

126-5 131-1 131-2 131

Referring to the synthetic route of compound **126,** 3,3,3-trifluoro-2,2-dimethylpropionic acid was replaced with 2,2-dimethylheptanoic acid to carry out the synthesis. The resulting crude product was purified by reverse-phase column chromatography [acetonitrile/water (0.05% formic acid) = 0% to 100%] to obtain compound **131** (26 mg, yield: 32%) as a white solid. MS (ESI, m/z): 600.3 $[M+H]^+$.

$^1H$ NMR (400 MHz, $CDCl_3$) δ 7.97 (d, *J* = 8.0 Hz, 1H), 7.44 (s, 1H), 7.33 - 7.28 (m, 1H), 7.24 (t, *J* = 8.0 Hz, 1H), 6.69 -

6.62 (m, 1H), 6.61 - 6.56 (m, 1H), 6.52 - 6.44 (m, 1H), 3.90 - 3.80 (m, 6H), 3.00 - 2.86 (m, 4H), 2.56 (q, *J* = 8.2 Hz, 1H), 2.29 - 2.20 (m, 2H), 2.11 - 2.06 (m, 2H), 1.83 - 1.79 (m, 2H), 1.53 (s, 6H), 1.32 - 1.30 (m, 6H), 1.14 - 1.10 (m, 1H), 0.94 - 0.88 (m, 3H), 0.69 - 0.62 (m, 1H), 0.51 - 0.44 (m, 1H), 0.41 - 0.35 (m, 1H), 0.25 - 0.19 (m, 1H).

**Example 132:**

Synthetic Route:

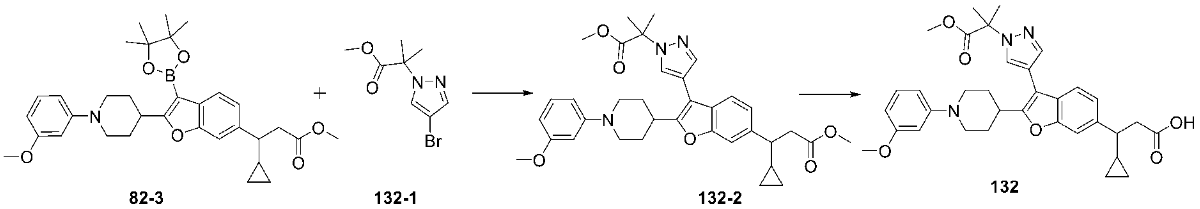

82-3 132-1 132-2 132

Referring to the synthetic route of compound **82,** compound **82-4** was replaced with compound **132-1** to carry out the synthesis. The resulting crude product was purified by reverse-phase column chromatography [acetonitrile/water (0.05% ammonia) = 0% to 100%] to obtain compound **132** (8 mg, yield: 40%) as a white solid. MS (ESI, m/z): 586.3 $[M+H]^+$.

$^1$H NMR (400 MHz, $CDCl_3$) $\delta$ 7.77 - 7.69 (m, 2H), 7.47 (d, *J* = 6.8 Hz, 1H), 7.35 (s, 1H), 7.23 - 7.16 (m, 1H), 7.14 (d, *J* = 7.2 Hz, 1H), 6.64 - 6.57 (m, 1H), 6.54 (s, 1H), 6.47 - 6.39 (m, 1H), 3.84 - 3.80 (m, 5H), 3.76 (s, 3H), 3.15 - 3.05 (m, 1H), 2.87 - 2.80 (m, 4H), 2.54 - 2.45 (m, 1H), 2.27 - 2.18 (m, 2H), 1.96 - 1.93 (m, 8H), 1.15 - 1.03 (m, 1H), 0.68 - 0.56 (m, 1H), 0.50 - 0.40 (m, 1H), 0.37 - 0.28 (m, 1H), 0.25 - 0.13 (m, 1H).

**Example 133:**

Synthetic Route:

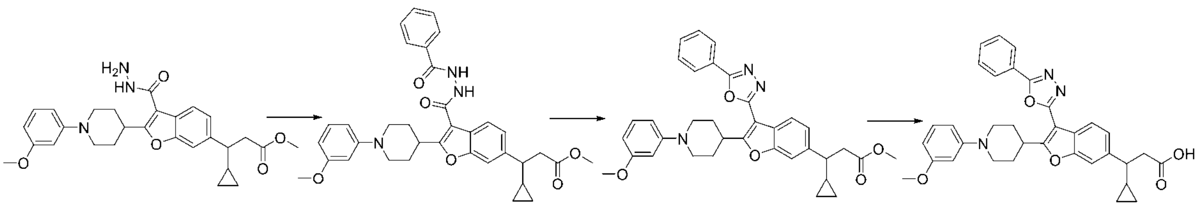

126-5 133-1 133-2 133

Referring to the synthetic route of compound 126, 3,3,3-trifluoro-2,2-dimethylpropionic acid was replaced with benzoic acid to carry out the synthesis. The resulting crude product was purified by reverse-phase column chromatography [acetonitrile/water (0.05% formic acid) = 0% to 100%] to obtain compound 133 (4 mg, yield: 10%) as a white solid. MS (ESI, m/z): 564.3 $[M+H]^+$.

$^1$H NMR (400 MHz, $CDCl_3$) $\delta$ 8.22 - 8.13 (m, 2H), 8.09 (d, *J*= 8.0 Hz, 1H), 7.62 - 7.57 (m, 3H), 7.46 (s, 1H), 7.35 - 7.30 (m, 1H), 7.25 - 7.21 (m, 1H), 6.70 - 6.62 (m, 1H), 6.61 - 6.55 (m, 1H), 6.50 - 6.44 (m, 1H), 3.96 - 3.86 (m, 3H), 3.84 (s, 3H), 3.03 - 2.96 (m, 2H), 2.95 - 2.88 (m, 2H), 2.58 - 2.52 (m, 1H), 2.27 - 2.23 (m, 2H), 2.14 - 2.11 (m, 2H), 1.15 - 1.11 (m, 1H), 0.70 - 0.64 (m, 1H), 0.53 - 0.45 (m, 1H), 0.41 - 0.37 (m, 1H), 0.26 - 0.21 (m, 1H).

**Example 134:**

Synthetic Route:

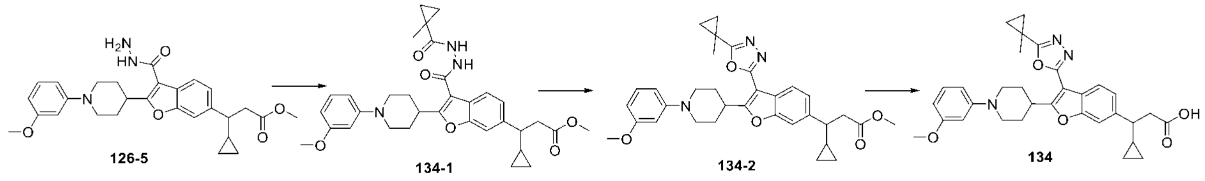
126-5 134-1 134-2 134

Referring to the synthetic route of compound 126, 3,3,3-trifluoro-2,2-dimethylpropionic acid was replaced with 1-methylcyclopropane-1-carboxylic acid to carry out the synthesis. The resulting crude product was purified by reverse-phase column chromatography [acetonitrile/water (0.05% formic acid) = 0% to 100%] to obtain compound 134 (4 mg, yield: 9%) as a white solid. MS (ESI, m/z): 542.3 $[M+H]^+$.

$^1H$ NMR (400 MHz, $CDCl_3$) δ 7.80 (d, *J* = 8.0 Hz, 1H), 7.32 (s, 1H), 7.25 -7.10 (m, 2H), 6.60 - 6.51 (m, 1H), 6.50 - 6.44 (m, 1H), 6.40 - 6.32 (m, 1H), 3.84 - 3.71 (m, 5H), 3.70 - 3.63 (m, 1H), 2.88 - 2.74 (m, 4H), 2.49 - 2.36 (m, 1H), 2.20 - 2.05 (m, 2H), 2.00 - 1.89 (m, 2H), 1.37 - 1.28 (m, 2H), 1.18 (s, 3H), 1.06 - 0.98 (m, 1H), 0.97- 0.95 (m, 2H), 0.63 - 0.49 (m, 1H), 0.44 - 0.33 (m, 1H), 0.28-0.24 (m, 1H), 0.17 - 0.07 (m, 1H).

**Example 135:**

Synthetic Route:

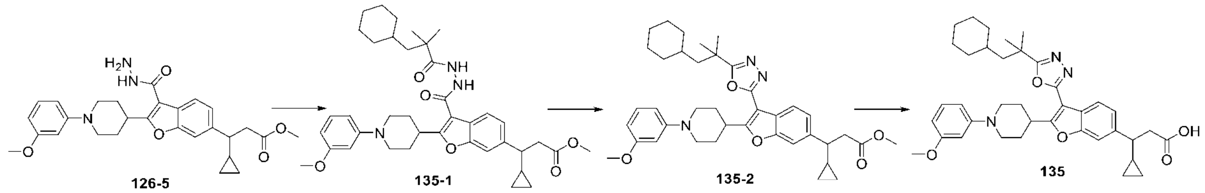
126-5 135-1 135-2 135

Referring to the synthetic route of compound 126, 3,3,3-trifluoro-2,2-dimethylpropionic acid was replaced with 1-cyclohexyl-2,2-dimethylpropanoic acid to carry out the synthesis. The resulting crude product was purified by reverse-phase column chromatography [acetonitrile/water (0.05% formic acid) = 0% to 100%] to obtain compound 135 (16 mg, yield: 80%) as a white solid. MS (ESI, m/z): 626.3 $[M+H]^+$.

$^1H$ NMR (400 MHz, $CDCl_3$) δ 7.97 (d, *J*= 8.0 Hz, 1H), 7.45 (s, 1H), 7.32 - 7.30 (m, 1H), 7.28 - 7.23 (m, 1H), 6.83 - 6.42 (m, 3H), 3.91 - 3.84 (m, 6H), 3.09 - 2.87 (m, 4H), 2.60 - 2.52 (m, 1H), 2.39 - 2.21 (m, 2H), 2.14 - 2.06 (m, 2H), 1.77 - 1.73 (m, 2H), 1.64 - 1.57 (m, 4H), 1.53 (s, 6H), 1.50 - 1.42 (m, 3H), 1.20 - 1.14 (m, 2H), 1.11 - 1.07 (m, 1H), 0.93 - 0.87 (m, 2H), 0.70 - 0.64 (m, 1H), 0.51 - 0.46 (m, 1H), 0.41 - 0.36 (m, 1H), 0.26 - 0.20 (m, 1H).

**Example 136:**

Synthetic Route:

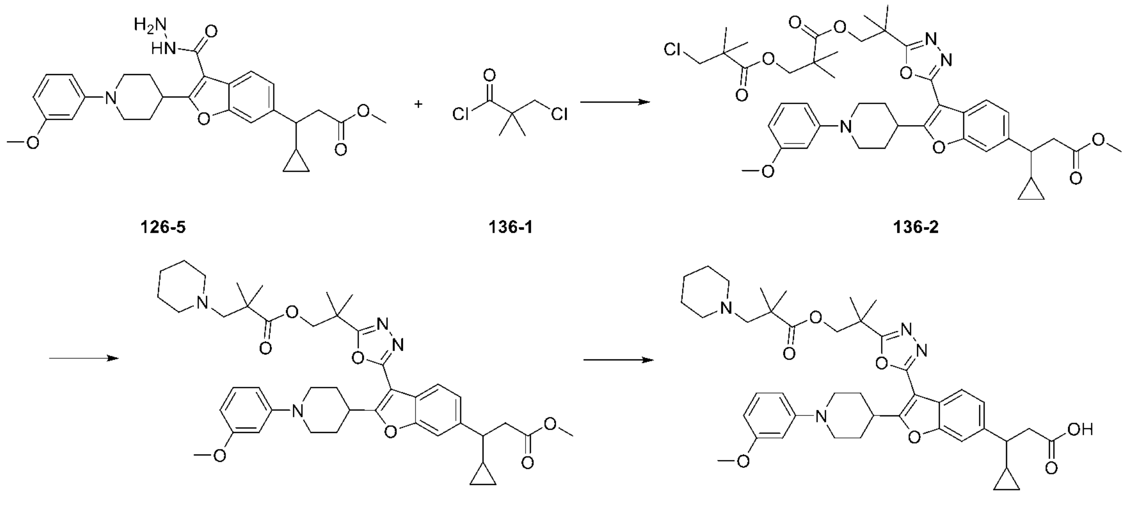

126-5 136-1 136-2

136-3 136

Referring to the synthetic route of compound 126, compound **126-5** (100 mg, 0.203 mmol) was synthesized. Compound **126-5** (100 mg, 0.203 mmol) and triethylamine (41 mg, 0.407 mmol) were dissolved in dichloromethane (5 mL). Compound **136-1** (41 mg, 0.264 mmol) was added, and the reaction was carried out at room temperature overnight. After concentration, the resulting crude product was purified by normal-phase column chromatography (ethyl acetate: petroleum ether = 10%) to obtain compound **136-2** (110 mg, yield: 78%) as a white solid. MS (ESI, m/z): 792.2 $[M+H]^+$.

Compound **136-2** (20 mg, 0.0253 mmol), sodium iodide (5.0 mg, 0.0339 mmol), potassium carbonate (5.6 mg, 0.0401 mmol), and piperidine (14 mg, 0.169 mmol) were dissolved in dimethyl sulfoxide (2 mL) and reacted at 85°C overnight. After concentration, the resulting crude product was purified by reverse-phase column chromatography [acetonitrile/water (0.05% formic acid) = 90% to 100%] to obtain compound **136-3** (18 mg, yield: 96%) as a white solid. MS (ESI, m/z): 741.2 $[M+H]^+$.

To a reaction tube, compound **136-3** (18 mg, 0.0243 mmol), lithium hydroxide (4.1 mg, 0.171 mmol), tetrahydrofuran (3 mL), methanol (3 mL), and water (3 mL) were added. The reaction was carried out at 50°C for 2 hours. After concentration, the resulting crude product was purified by reverse-phase column chromatography [acetonitrile/water (0.05% formic acid) = 50% to 70%] to obtain compound 136 (3 mg, yield: 18%) as a white solid. MS (ESI, m/z): 727.2 $[M+H]^+$.

$^1H$ NMR (400 MHz, $CDCl_3$) δ 7.42 (s, 1H), 7.32 -7.27 (m, 1H), 7.25 - 7.19 (m, 1H), 7.04 (d, *J*= 8.0 Hz, 1H), 6.68 - 6.40 (m, 3H), 4.09 - 3.93 (m, 4H), 3.86 - 3.82 (m, 5H), 3.56 - 3.51 (m, 4H), 3.50 - 3.43 (m, 1H), 2.97 - 2.82 (m, 3H), 2.70 - 2.61 (m, 1H), 2.46 - 2.40 (m, 1H), 2.28 - 2.03 (m, 4H), 1.69 - 1.63 (m, 2H), 1.62 - 1.54 (m, 4H), 1.40 (d, *J*= 10.4 Hz, 6H), 1.34 (d, *J* = *7.6* Hz, 6H), 1.26 - 1.21 (m, 1H), 0.71 - 0.64 (m, 1H), 0.56 - 0.48 (m, 1H), 0.40 - 0.33 (m, 1H), 0.28 - 0.20 (m, 1H).

**Example 137:**

Synthetic Route:

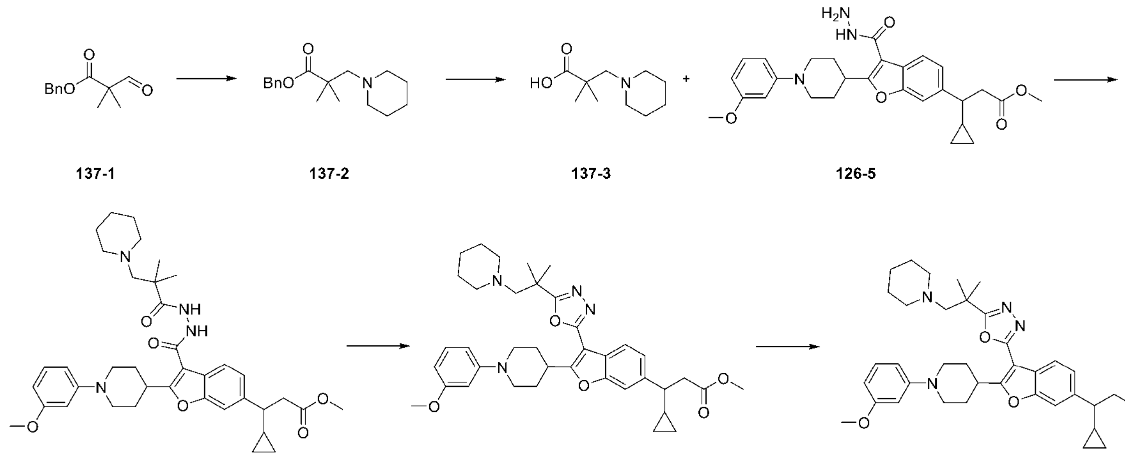

137-1 137-2 137-3 126-5

137-4 137-5 137

Compound **137-1** (250 mg, 1.19 mmol) was dissolved in dichloromethane (5 mL). Piperidine (121 mg, 1.43 mmol) and sodium triacetoxyborohydride (504 mg, 2.20 mmol) were added, and the reaction was carried out at room temperature overnight. After concentration, the resulting crude product was purified by normal-phase column chromatography (ethyl acetate: petroleum ether = 40%) to obtain compound **137-2** (100 mg, yield: 30%) as a yellow oil. MS (ESI, m/z): 276.1 $[M+H]^+$.

Compound **137-2** (45 mg, 0.163 mmol) was dissolved in tetrahydrofuran (3 mL), and 5% palladium on carbon (10 mg) was added. The reaction was carried out under a hydrogen atmosphere at room temperature overnight. The reaction mixture was rotary evaporated to dryness to remove the solvent to obtain compound **137-3** (42 mg, yield: 99%) as a yellow oil. MS (ESI, m/z): 186.1 $[M+H]^+$.

Then, referring to the synthetic route of compound 126, 3,3,3-trifluoro-2,2-dimethylpropionic acid was replaced with compound **137-3** to carry out the synthesis. The resulting crude product was purified by reverse-phase column chromatography [acetonitrile/water (0.05% formic acid) = 0% to 100%] to obtain compound 137 (16 mg, yield: 68%) as a white solid. MS (ESI, m/z): 627.3 $[M+H]^+$.

$^1H$ NMR (400 MHz, $CDCl_3$) δ 7.91 (d, *J* = 8.0 Hz, 1H), 7.43 (s, 1H), 7.23 (t, *J* = 8.4 Hz, 1H), 6.98 (d, *J* = 8.0 Hz, 1H), 6.69 - 6.62 (m, 1H), 6.60 - 6.55 (m, 1H), 6.51 - 6.44 (m, 1H), 3.87 - 3.76 (m, 6H), 3.41 - 3.23 (m, 2H), 2.98 - 2.82 (m, 6H), 2.57 - 2.53 (m, 1H), 2.24 - 2.16 (m, 4H), 2.07 - 2.02 (m, 2H), 1.88 - 1.71 (m, 4H), 1.64 (s, 6H), 1.58 - 1.46 (m, 2H), 1.17 - 1.06 (m, 1H), 0.72 - 0.64 (m, 1H), 0.51 - 0.43 (m, 1H), 0.42 - 0.34 (m, 1H), 0.26 - 0.18 (m, 1H).

**Example 138:**

Synthetic Route:

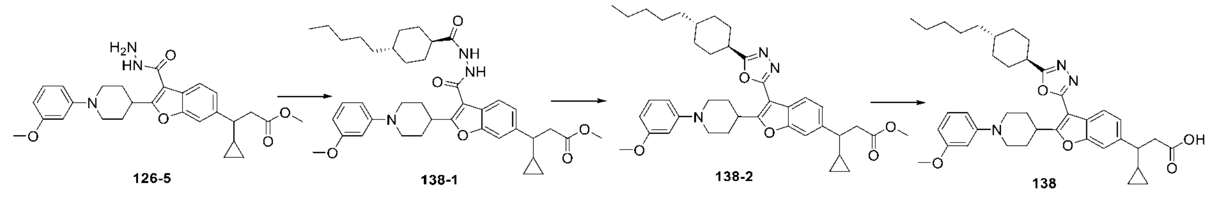

126-5 138-1 138-2 138

Referring to the synthetic route of compound 126, 3,3,3-trifluoro-2,2-dimethylpropionic acid was replaced with trans-4-pentylcyclohexanecarboxylic acid to carry out the synthesis. The resulting crude product was purified by reverse-phase column chromatography [acetonitrile/water (0.05% formic acid) = 0% to 100%] to obtain compound 138 (3 mg, yield: 16%) as a white solid. MS (ESI, m/z): 640.3 $[M+H]^+$.

$^1H$ NMR (400 MHz, $CDCl_3$) δ 7.95 (d, *J* = 8.0 Hz, 1H), 7.42 (s, 1H), 7.26 - 7.17 (m, 2H), 6.67 - 6.60 (m, 1H), 6.56 (s, 1H), 6.49 - 6.42 (m, 1H), 3.91 - 3.75 (m, 7H), 3.00 - 2.81 (m, 5H), 2.57 - 2.48 (m, 1H), 2.29 - 2.15 (m, 4H), 2.09 - 2.02 (m, 2H), 1.99 - 1.80 (m, 4H), 1.75 - 1.57(m, 4H), 1.22 - 1.00 (m, 5H), 0.97 - 0.84 (m, 5H), 0.65 - 0.63 (m, 1H), 0.50 - 0.43 (m, 1H), 0.44 - 0.32 (m, 1H), 0.23 - 0.19 (m, 1H).

**Example 139:**

Synthetic Route:

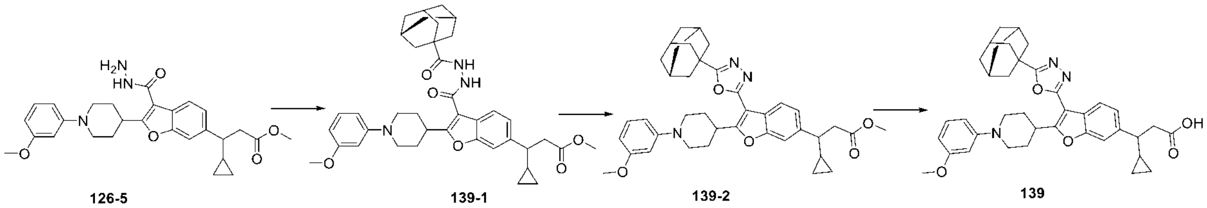

126-5 139-1 139-2 139

Referring to the synthetic route of compound 126, 3,3,3-trifluoro-2,2-dimethylpropionic acid was replaced with 1-adamantanecarboxylic acid to carry out the synthesis. The resulting crude product was purified by reverse-phase column chromatography [acetonitrile/water (0.05% formic acid) = 0% to 100%] to obtain compound 139 (20 mg, yield: 48%) as a white solid. MS (ESI, m/z): 622.3 $[M+H]^+$.

$^1$H NMR (400 MHz, $CDCl_3$) δ 7.85 (d, *J* = 8.0 Hz, 1H), 7.36 (s, 1H), 7.21 -7.10 (m, 2H), 6.58 - 6.52 (m, 1H), 6.49 (s, 1H), 6.45 - 6.37 (m, 1H), 3.79 (s, 3H), 3.77 - 3.68 (m, 2H), 3.68 - 3.55 (m, 1H), 2.89 - 2.69 (m, 2H), 2.70 - 2.52 (m, 2H), 2.49 - 2.43 (m, 1H), 2.28 - 2.03 (m, 11H), 1.98 - 1.92 (m, 2H), 1.83 - 1.72 (m, 6H), 0.94 - 0.89 (m, 1H), 0.49 - 0.38 (m, 1H), 0.33 - 0.17 (m, 2H), 0.08 - 0.05 (m, 1H).

**Example** 140:

Synthetic Route:

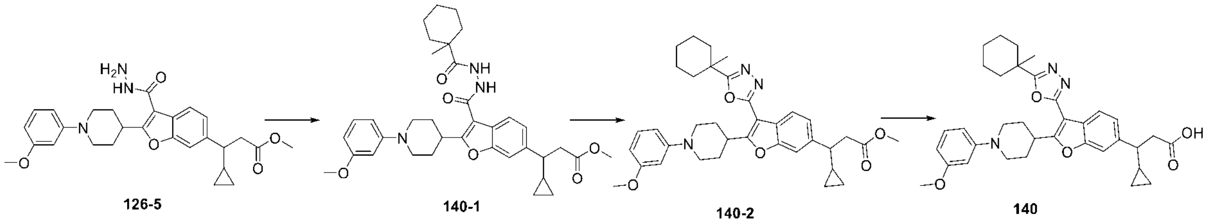

126-5 140-1 140-2 140

Referring to the synthetic route of compound 126, 3,3,3-trifluoro-2,2-dimethylpropionic acid was replaced with 1-methylcyclohexanecarboxylic acid to carry out the synthesis. The resulting crude product was purified by reverse-phase column chromatography [acetonitrile/water (0.05% formic acid) = 0% to 100%] to obtain compound 140 (17 mg, yield: 47%) as a white solid. MS (ESI, m/z): 584.3 $[M+H]^+$.

$^1$H NMR (400 MHz, $CDCl_3$) δ 7.94 (d, *J* = 8.0 Hz, 1H), 7.42 (s, 1H), 7.28 - 7.15 (m, 2H), 6.68 - 6.61 (m, 1H), 6.56 (s, 1H), 6.50 - 6.41 (m, 1H), 3.95 - 3.85 (m, 2H), 3.83 (s, 3H), 3.82 - 3.75 (m, 1H), 3.05 - 2.79 (m, 4H), 2.57 - 2.46 (m, 1H), 2.38 - 2.26 (m, 2H), 2.23 - 2.17 (m, 2H), 2.12 - 2.02 (m, 2H), 1.75 - 1.52 (m, 8H), 1.44 (s, 3H), 1.14 - 1.05 (m, 1H), 0.70 - 0.59 (m, 1H), 0.53 - 0.42 (m, 1H), 0.41 - 0.31 (m, 1H), 0.24 - 0.14 (m, 1H).

**Example 141:**

Synthetic Route:

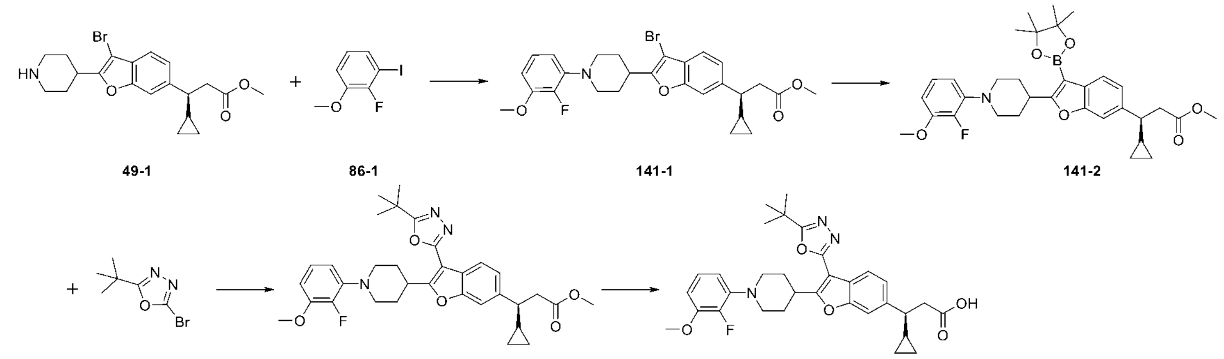

49-1 86-1 141-1 141-2

124-1 141-3 141

Referring to the synthetic route of compound **81,** compound **11-1** was replaced with compound **86-1** and compound **81-2** was replaced with compound **124-1** to carry out the synthesis. The resulting crude product was purified by reverse-phase column chromatography [acetonitrile/water (0.05% formic acid) = 0% to 100%] to obtain compound 141 (24 mg, yield: 39%) as a white solid. MS (ESI, m/z): 562.3 $[M+H]^+$.

$^1H$ NMR (400 MHz, $CDCl_3$) δ 7.93 (d, *J*= 8.0 Hz, 1H), 7.42 (s, 1H), 7.27 - 7.21 (m, 1H), 7.05 - 6.94 (m, 1H), 6.74 - 6.60 (m, 2H), 3.90 (s, 3H), 3.82 - 3.76 (m, 1H), 3.67 - 3.54 (m, 2H), 2.96 - 2.75 (m, 4H), 2.58 - 2.45 (m, 1H), 2.35 - 2.26 (m, 2H), 2.13 - 2.00 (m, 2H), 1.53 (s, 9H), 1.16 - 1.05 (m, 1H), 0.67 - 0.59 (m, 1H), 0.52 - 0.41 (m, 1H), 0.40 - 0.30 (m, 1H), 0.26 - 0.15 (m, 1H).

**Example 142:**

Synthetic Route:

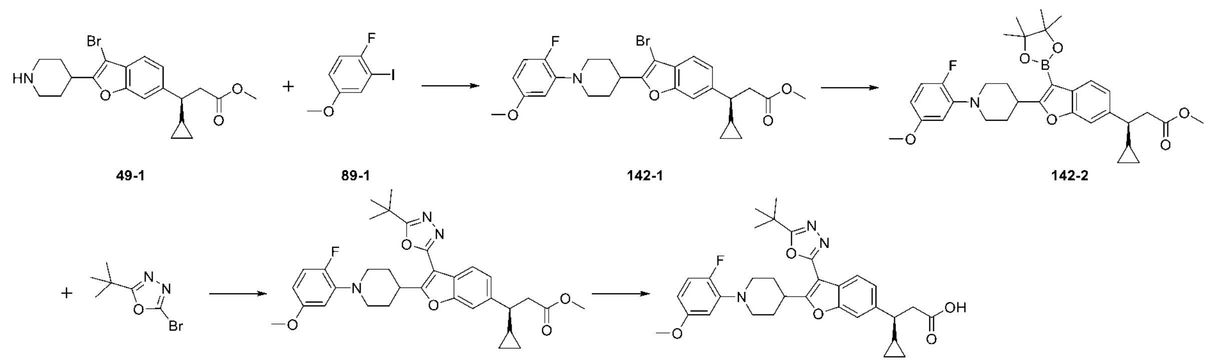

49-1 89-1 142-1 142-2

124-1 142-3 142

Referring to the synthetic route of compound **141,** compound **86-1** was replaced with compound **89-1** to carry out the synthesis. The resulting crude product was purified by reverse-phase column chromatography [acetonitrile/water (0.05% formic acid) = 0% to 100%] to obtain compound **142** (91 mg, yield: 63%) as a white solid. MS (ESI, m/z): 562.3 $[M+H]^+$.

$^1H$ NMR (400 MHz, $CDCl_3$) δ 7.92 (d, *J*= 8.0 Hz, 1H), 7.41 (s, 1H), 7.27 - 7.21 (m, 1H), 7.06 - 6.95 (m, 1H), 6.60 - 6.52 (m, 1H), 6.48 - 6.37 (m, 1H), 3.86 - 3.71 (m, 4H), 3.67 - 3.53 (m, 2H), 2.92 - 2.84 (m, 4H), 2.58 - 2.44 (m, 1H), 2.37 - 2.20 (m, 2H), 2.12 - 1.98 (m, 2H), 1.57 - 1.46 (m, 9H), 1.14 - 1.05 (m, 1H), 0.69 - 0.61 (m, 1H), 0.50 - 0.41 (m, 1H), 0.38 - 0.31 (m, 1H), 0.22 - 0.16 (m, 1H).

**Example 143:**

Synthetic Route:

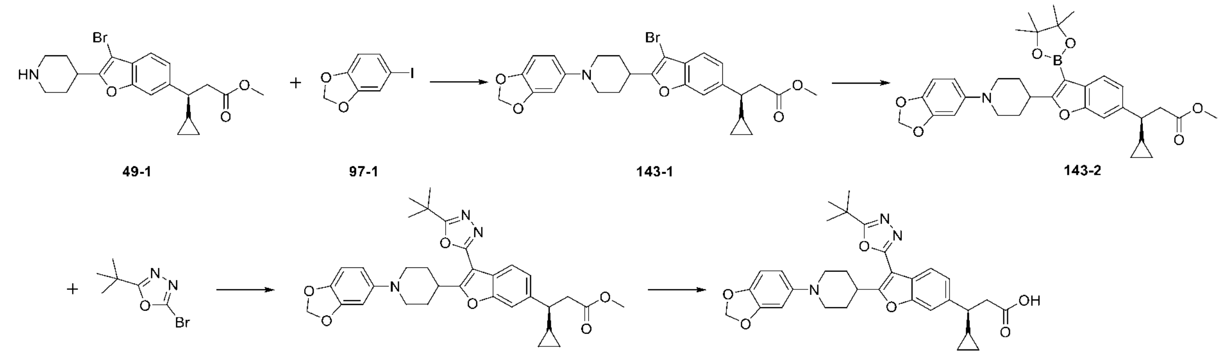

49-1 97-1 143-1 143-2

124-1 143-3 143

Referring to the synthetic route of compound 141, compound 86-1 was replaced with compound 97-1 to carry out the synthesis. The resulting crude product was purified by reverse-phase column chromatography [acetonitrile/water (0.05% formic acid) = 0% to 100%] to obtain compound **143** (97 mg, yield: 64%) as a white solid. MS (ESI, m/z): 558.3 $[M+H]^+$.

$^1H$ NMR (400 MHz, $CDCl_3$) δ 7.92 (d, *J* = 8.0 Hz, 1H), 7.41 (s, 1H), 7.26 - 7.22 (m, 1H), 6.78 - 6.71 (m, 1H), 6.64 (s, 1H), 6.50 - 6.40 (m, 1H), 5.91 (s, 2H), 3.86 - 3.55 (m, 3H), 2.96 - 2.76 (m, 4H), 2.56 - 2.48 (m, 1H), 2.32 - 2.14 (m, 2H), 2.08 - 1.99 (m, 2H), 1.52 (s, 9H), 1.13 - 1.06 (m, 1H), 0.68 - 0.57 (m, 1H), 0.49 - 0.42 (m, 1H), 0.37 - 0.30 (m 1H), 0.23 - 0.18 (m, 1H).

**Example 144:**

Synthetic Route:

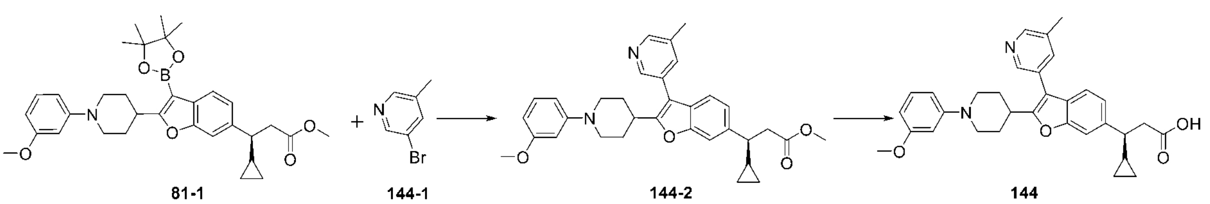

81-1 144-1 144-2 144

Referring to the synthetic route of compound **81,** compound **81-2** was replaced with compound **144-1** to carry out the synthesis. The resulting crude product was purified by reverse-phase column chromatography [acetonitrile/water (0.05% ammonia) = 0% to 100%] to obtain compound **144** (226 mg, yield: 98%) as a white solid. MS (ESI, m/z): 511.2 $[M+H]^+$.

$^1H$ NMR (400 MHz, $CDCl_3$) δ 8.46 (d, *J* = 8.0 Hz, 2H), 7.62 (s, 1H), 7.46 - 7.34 (m, 2H), 7.25 - 7.14 (m, 2H), 6.62 - 6.56 (m, 1H), 6.52 (s, 1H), 6.47 - 6.38 (m, 1H), 3.84 - 3.71 (m, 5H), 3.03 - 2.93 (m, 1H), 2.92 - 2.74 (m, 4H), 2.57 - 2.46 (m, 1H), 2.42 (s, 3H), 2.30 - 2.17 (m, 2H), 1.94 - 1.81 (m, 2H), 1.16 - 1.03 (m, 1H), 0.68 - 0.56 (m, 1H), 0.49 - 0.41 (m, 1H), 0.40 - 0.31 (m, 1H), 0.24 - 0.16 (m, 1H).

**Example 145:**

Synthetic Route:

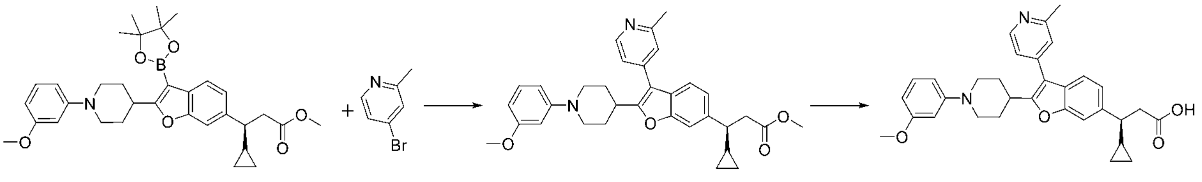

81-1 145-1 145-2 145

Referring to the synthetic route of compound **81,** compound **81-2** was replaced with compound **145-1** to carry out

the synthesis. The resulting crude product was purified by reverse-phase column chromatography [acetonitrile/water (0.05% ammonia) = 0% to 100%] to obtain compound **145** (183 mg, yield: 92%) as a white solid. MS (ESI, m/z): 511.2 $[M+H]^+$.

$^1H$ NMR (400 MHz, $CDCl_3$) $\delta$ 8.57 (d, $J$ = 7.2 Hz, 1H), 7.47 - 7.37 (m, 2H), 7.26 (s, 1H), 7.22 - 7.13 (m, 3H), 6.62 - 6.56 (m, 1H), 6.54 - 6.48 (m, 1H), 6.47 - 6.38 (m, 1H), 3.86 - 3.71 (m, 5H), 3.11 - 2.96 (m, 1H), 2.93 - 2.74 (m, 4H), 2.63 (s, 3H), 2.57 - 2.48 (m, 1H), 2.29 - 2.17 (m, 2H), 1.96 - 1.80 (m, 2H), 1.17 - 1.06 (m, 1H), 0.69 - 0.58 (m, 1H), 0.49 - 0.42 (m, 1H), 0.40 - 0.31 (m, 1H), 0.26 - 0.13 (m, 1H).

**Example 146:**

Synthetic Route:

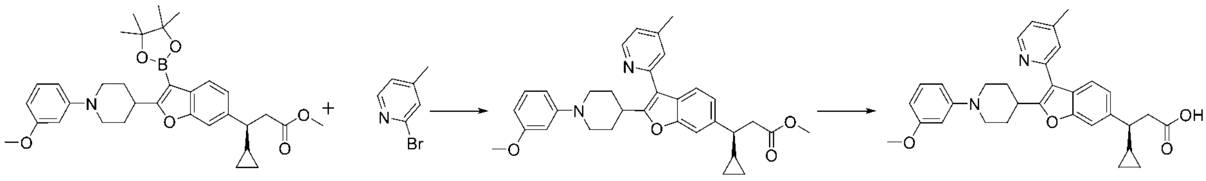

81-1 146-1 146-2 146

Referring to the synthetic route of compound **81,** compound **81-2** was replaced with compound **146-1** to carry out the synthesis. The resulting crude product was purified by reverse-phase column chromatography [acetonitrile/water (0.05% ammonia) = 0% to 100%] to obtain compound **146** (51 mg, yield: 76%) as a white solid. MS (ESI, m/z): 511.2 $[M+H]^+$.

$^1H$ NMR (400 MHz, $CDCl_3$) $\delta$ 7.76 - 7.61 (m, 2H), 7.42 - 7.35 (m, 2H), 7.23 - 7.05 (m, 3H), 6.65 - 6.50 (m, 2H), 6.47 - 6.38 (m, 1H), 3.87 - 3.77 (m, 5H), 3.55 - 3.41 (m, 1H), 2.90 - 2.77 (m, 4H), 2.61 (s, 3H), 2.54 - 2.43 (m, 1H), 2.29 - 2.16 (m, 2H), 2.08 - 1.98 (m, 2H), 1.12 - 1.02 (m, 1H), 0.66 - 0.54 (m, 1H), 0.46 - 0.38 (m, 1H), 0.36 - 0.27 (m, 1H), 0.21 - 0.14 (m, 1H).

**Example 147:**

Synthetic Route:

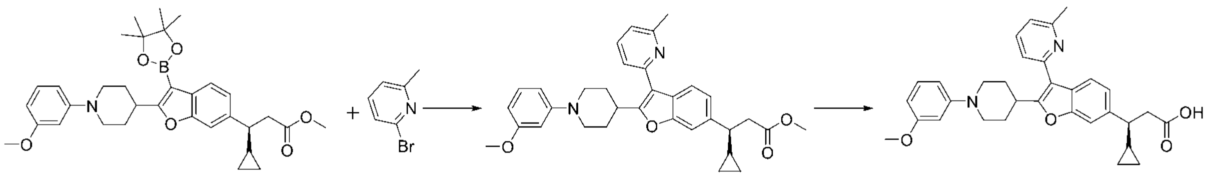

81-1 147-1 147-2 147

Referring to the synthetic route of compound **81,** compound **81-2** was replaced with compound **147-1** to carry out the synthesis. The resulting crude product was purified by reverse-phase column chromatography [acetonitrile/water (0.05% ammonia) = 0% to 100%] to obtain compound **147** (59 mg, yield: 79%) as a white solid. MS (ESI, m/z): 511.2 $[M+H]^+$.

$^1H$ NMR (400 MHz, $CDCl_3$) $\delta$ 8.59 (d, $J$ = 8.0 Hz, 1H), 7.63 (d, $J$= 8.0 Hz, 1H), 7.36 (s, 2H), 7.21 - 7.11 (m, 2H), 7.07 (d, $J$= 8.4 Hz, 1H), 6.64 - 6.56 (m, 1H), 6.53 (s, 1H), 6.45 - 6.38 (m, 1H), 3.85 - 3.69 (m, 5H), 3.53 - 3.38 (m, 1H), 2.92 - 2.72 (m, 4H), 2.55 - 2.45 (m, 1H), 2.43 (s, 3H), 2.26 - 2.17 (m, 2H), 2.05 - 1.94 (m, 2H), 1.14 - 1.03 (m, 1H), 0.65 - 0.54 (m, 1H), 0.48 - 0.40 (m, 1H), 0.36 - 0.27 (m, 1H), 0.21 - 0.13 (m, 1H).

**Example 148:**

Synthetic Route:

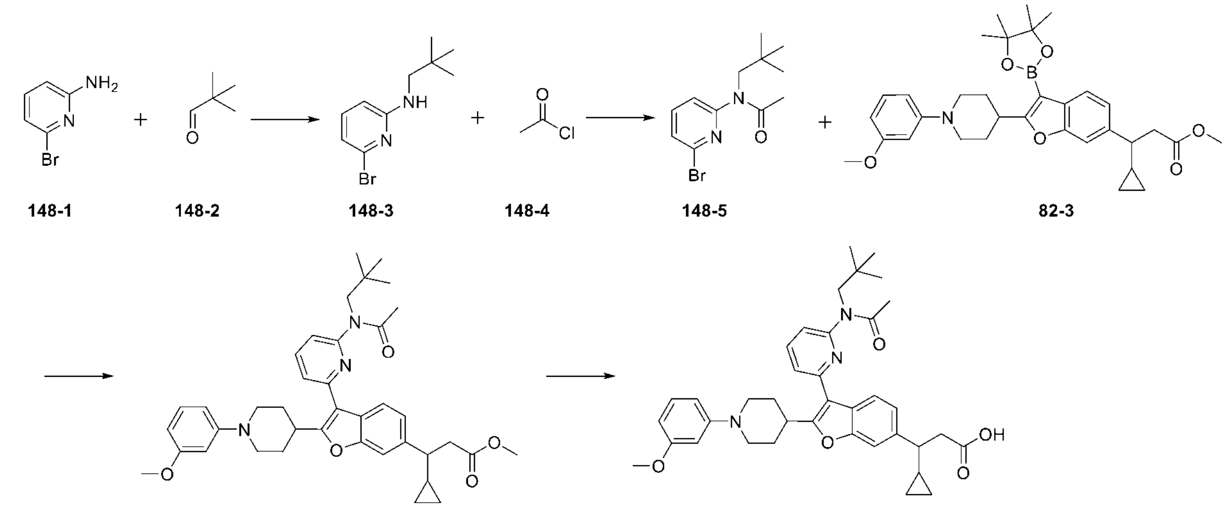

148-1 148-2 148-3 148-4 148-5 82-3

148-6 148

Compound **148-1** (2 g, 11.6 mmol) was dissolved in dichloromethane (20 mL). Compound **148-2** (994 mg, 11.6 mmol), acetic acid (0.02 mL), and sodium triacetoxyborohydride (3.2 g, 15.1 mmol) were added, and the reaction was carried out at room temperature for 4 hours. After concentration, the resulting crude product was purified by normal-phase column chromatography (ethyl acetate: petroleum ether = 40%) to obtain compound **148-3** (1.6 g, yield: 57%) as a colorless oil. MS (ESI, m/z): 243.1 $[M+H]^+$.

Compound **148-3** (1.6 g, 6.6 mmol) was dissolved in dichloromethane (10 mL), and compound **148-4** (779 mg, 9.9 mmol) and triethylamine (1.33 g, 13.2 mmol) were added. The reaction was carried out at room temperature for 4 hours. After concentration, the resulting crude product was purified by normal-phase column chromatography (ethyl acetate: petroleum ether = 40%) to obtain compound **148-5** (938 mg, yield: 50%) as a yellow oil. MS (ESI, m/z): 285.1 $[M+H]^+$.

Then, referring to the synthetic route of compound **82,** compound **82-4** was replaced with compound **148-5** to carry out the synthesis. The resulting crude product was purified by reverse-phase column chromatography [acetonitrile/-water (0.05% ammonia) = 0% to 100%] to obtain compound **148** (21 mg, yield: 84%) as a white solid. MS (ESI, m/z): 624.2 $[M+H]^+$.

$^1$H NMR (400 MHz, $CDCl_3$) $\delta$ 7.86 (t, $J$ = 7.6 Hz, 1H), 7.73 (d, $J$ = 7.6 Hz, 1H), 7.56 (d, $J$= 7.6 Hz, 1H), 7.41 (s, 1H), 7.28 - 7.27 (m, 1H), 7.25 - 7.13 (m, 3H), 6.69 - 6.38 (m, 2H), 3.94 (s, 2H), 3.88 - 3.78 (m, 5H), 3.72 - 3.57 (m, 1H), 2.98 - 2.75 (m, 4H), 2.58 - 2.47 (m, 1H), 2.38 - 2.16 (m, 2H), 2.09 (s, 3H), 2.06 - 1.97 (m, 2H), 1.18 - 1.05 (m, 1H), 0.88 (s, 9H), 0.68 - 0.59 (m, 1H), 0.52 - 0.41 (m, 1H), 0.40 - 0.30 (m, 1H), 0.26 - 0.16 (m, 1H).

**Example 149:**

Synthetic Route:

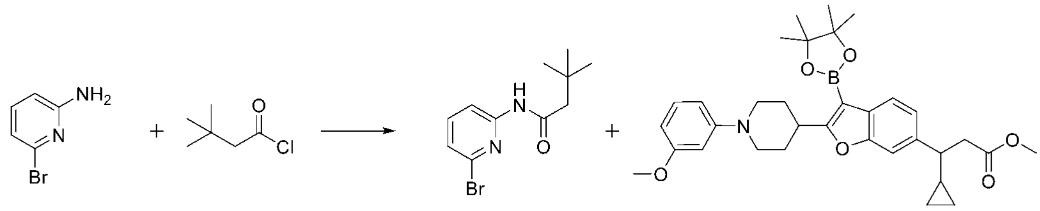

148-1 149-1 149-2 82-3

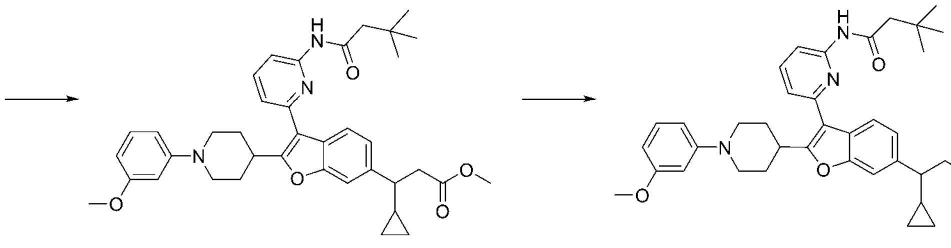

149-3 149

Referring to the synthetic route of compound 148, compound **148-3** was replaced with compound **148-1** and compound **148-4** was replaced with compound **149-1** to carry out the synthesis. The resulting crude product was purified by reverse-phase column chromatography [acetonitrile/water (0.05% ammonia) = 0% to 100%] to obtain compound 149 (14 mg, yield: 23%) as a white solid. MS (ESI, m/z): 610.2 $[M+H]^+$.

$^1H$ NMR (400 MHz, $CDCl_3$) $\delta$ 8.96 - 8.75 (m, 1H), 8.30 - 8.13 (m, 1H), 7.86 - 7.78 (m, 1H), 7.58 (d, *J*= 8.0 Hz, 1H), 7.37 (s, 1H), 7.25 - 7.16 (m, 2H), 7.14 - 7.08 (m, 1H), 6.66 - 6.58 (m, 1H), 6.54 (s, 1H), 6.49 - 6.42 (m, 1H), 3.87 - 3.65 (m, 5H), 3.36 - 3.16 (m, 1H), 2.90 - 2.61 (m, 4H), 2.53 - 2.35 (m, 1H), 2.33 - 2.07 (m, 4H), 2.02 - 1.80 **(m,** 2H), 1.32 - 1.25 (m, 1H), 1.07 (s, 9H), 0.68 - 0.55 (m, 1H), 0.49 - 0.38 (m, 1H), 0.37 - 0.25 (m, 1H), 0.22 - 0.05 (m, 1H).

**Example 150:**

Synthetic Route:

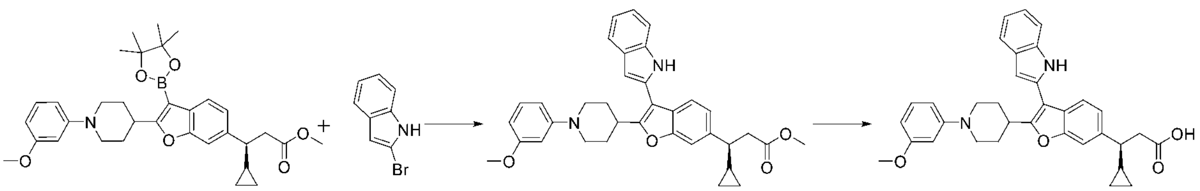

81-1 150-1 150-2 150

Referring to the synthetic route of compound 81, compound 81-2 was replaced with compound **150-1** to carry out the synthesis. The resulting crude product was purified by reverse-phase column chromatography [acetonitrile/water (0.05% ammonia) = 0% to 100%] to obtain compound 150 (30 mg, yield: 58%) as a white solid. MS (ESI, m/z): 535.2 $[M+H]^+$.

$^1H$ NMR (400 MHz, $CDCl_3$) $\delta$ 8.27 (s, 1H), 7.68 (d, *J* = 8.0 Hz, 1H), 7.58 (d,*J* = 8.0 Hz, 1H), 7.43 (d, *J*= 8.0 Hz, 1H), 7.39 (s, 1H), 7.22 (d, *J*= 8.0 Hz, 1H), 7.26 - 7.13 (m, 3H), 6.64 - 6.58 (m, 2H), 6.54 (s, 1H), 6.48 - 6.40 (m, 1H), 3.90 - 3.76 (m, 5H), 3.31 - 3.23 (m, 1H), 2.94 - 2.78 (m, 4H), 2.55 - 2.44 (m, 1H), 2.29 - 2.18 (m, 2H), 1.97 - 1.86 (m, 2H), 1.15 - 1.05 (m, 1H), 0.69 - 0.58 (m, 1H), 0.51 - 0.42 (m, 1H), 0.39 - 0.28 (m, 1H), 0.22 - 0.13 (m, 1H).

**Example 151:**

Synthetic Route:

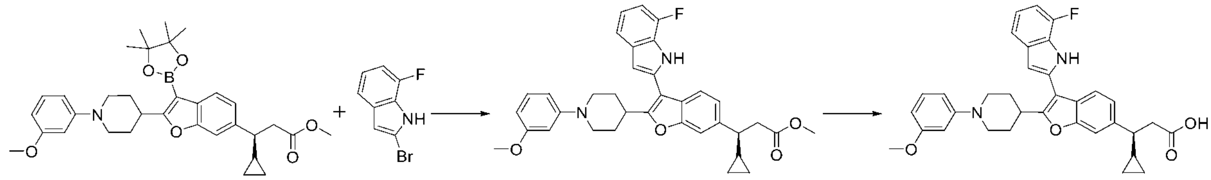

81-1 151-1 151-2 151

Referring to the synthetic route of compound **81,** compound **81-2** was replaced with compound **151-1** to carry out the synthesis. The resulting crude product was purified by reverse-phase column chromatography [acetonitrile/water (0.05% ammonia) = 0% to 100%] to obtain compound **151** (103 mg, yield: 84%) as a white solid. MS (ESI, m/z): 553.2 $[M+H]^+$.

$^1$H NMR (400 MHz, $CDCl_3$) $\delta$ 8.46 (s, 1H), 7.58 (d, *J* = 8.0 Hz, 1H), 7.43 (d, *J* = 8.0 Hz, 1H), 7.40 (s, 1H), 7.24 - 7.15 (m, 2H), 7.17 - 7.04 (m, 1H), 6.99 - 6.90 (m, 1H), 6.76 - 6.33 (m, 4H), 3.88 - 3.76 (m, 5H), 3.34 - 3.25 (m, 1H), 2.95 - 2.79 (m, 4H), 2.53 - 2.45 (m, 1H), 2.33 - 2.16 (m, 2H), 2.02 - 1.89 (m, 2H), 1.15 - 1.03 (m, 1H), 0.68 - 0.57 (m, 1H), 0.51 - 0.40 (m, 1H), 0.38 - 0.29 (m, 1H), 0.24 - 0.16 (m, 1H).

**Example** 152

Synthetic Route:

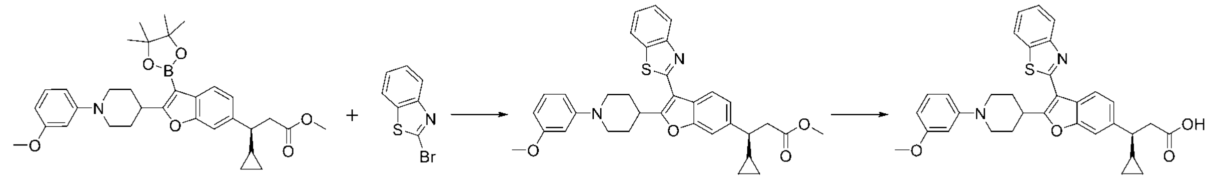

81-1 152-1 152-2 152

Referring to the synthetic route of compound 81, compound 81-2 was replaced with compound **152-1** to carry out the synthesis. The resulting crude product was purified by reverse-phase column chromatography [acetonitrile/water (0.05% ammonia) = 0% to 100%] to obtain compound 152 (2 mg, yield: 11%) as a white solid. MS (ESI, m/z): 553.2 $[M+H]^+$.

$^1$H NMR (400 MHz, MeOD) $\delta$ 8.15 - 7.99 (m, 3H), 7.58 - 7.52 (m, 1H), 7.49 - 7.42 (m, 2H), 7.35 - 7.30 (m, 1H), 7.16 (t, *J*= 8.0 Hz, 1H), 6.68 - 6.63 (m, 1H), 6.59 - 6.58 (m, 1H), 6.46 - 6.44 (m, 1H), 3.99 - 3.91 (m, 1H), 3.86 (m, 2H), 3.78 (s, 3H), 2.99 - 2.89 (m, 2H), 2.79 - 2.74 (m, 2H), 2.54 - 2.48 (m, 1H), 2.30 - 2.20 (m, 2H), 2.11 (m, 2H),1.17 - 1.09 (m, 1H), 0.62 - 0.60 (m, 1H), 0.43 - 0.33 (m, 2H), 0.20 - 0.16 (m, 1H).

**Example 153:**

Synthetic Route:

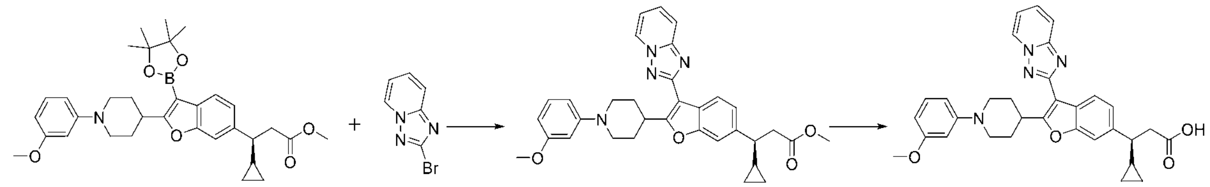

81-1 153-1 153-2 153

Referring to the synthetic route of compound **81,** compound **81-2** was replaced with compound **153-1** to carry out the synthesis. The resulting crude product was purified by reverse-phase column chromatography [acetonitrile/water (0.05% ammonia) = 0% to 100%] to obtain compound **153** (4 mg, yield: 20%) as a white solid. MS (ESI, m/z): 537.2 $[M+H]^+$.

$^1$H NMR (400 MHz, MeOD) $\delta$ 8.84 (d, *J* = 6.8 Hz, 1H), 8.18 (d, *J* = 8.0 Hz, 1H), 7.81 (d, *J*= 8.8 Hz, 1H), 7.72 - 7.65 (m, 1H), 7.45 (s, 1H), 7.30 - 7.28 (m, 1H), 7.21 - 7.13 (m, 2H), 6.67 - 6.65 (m, 1H), 6.60 - 6.59 (m, 1H), 6.46 - 6.44 (m, 1H),

4.18 - 4.12 (m, 1H), 3.86 - 3.83 (m, 2H), 3.78 (s, 3H), 2.96 - 2.93 (m, 2H), 2.74 - 2.63 (m, 2H), 2.57 - 2.51 (m, 1H), 2.26 - 2.18 (m, 2H), 2.09 - 2.03 (m, 2H), 1.14 - 1.07 (m, 1H), 0.62 - 0.55 (m, 1H), 0.42 - 0.36 (m, 2H), 0.18 - 0.12 (m, 1H).

**Example 154:**

Synthetic Route:

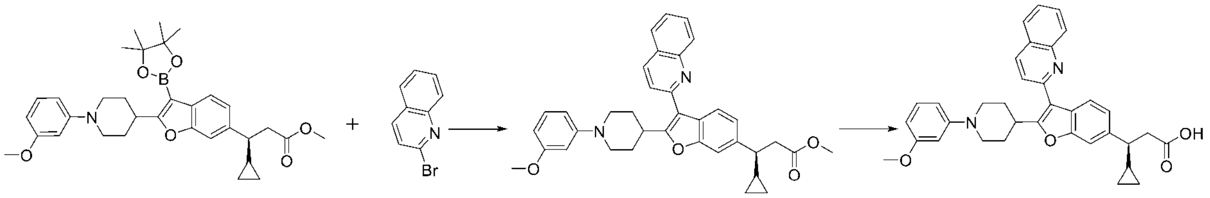

81-1 154-1 154-2 154

Referring to the synthetic route of compound **81,** compound **81-2** was replaced with compound **154-1** to carry out the synthesis. The resulting crude product was purified by reverse-phase column chromatography [acetonitrile/water (0.05% ammonia) = 0% to 100%] to obtain compound **154** (27 mg, yield: 38%) as a white solid. MS (ESI, m/z): 547.2 $[M+H]^+$.

$^1$H NMR (400 MHz, $CDCl_3$) $\delta$ 8.25 (d, *J* = 8.4 Hz, 1H), 8.11 (d, *J* = 8.4 Hz, 1H), 7.88 - 7.79 (m, 2H), 7.77 - 7.70 (m, 2H), 7.55 (t, *J*= 8.0 Hz, 1H), 7.46 - 7.39 (m, 1H), 7.23 - 7.15 (m, 2H), 6.79 - 6.27 (m, 3H), 3.90 - 3.76 (m, 5H), 3.72 - 3.64 (m, 1H), 2.96 - 2.80 (m, 4H), 2.56 - 2.47 (m, 1H), 2.40 - 2.23 (m, 2H), 2.17 - 2.08 (m, 2H), 1.18 - 1.05 (m, 1H), 0.68 - 0.57 (m, 1H), 0.51 - 0.43 (m, 1H), 0.39 - 0.29 (m, 1H), 0.23 - 0.15 (m, 1H).

**Example 155:**

Synthetic Route:

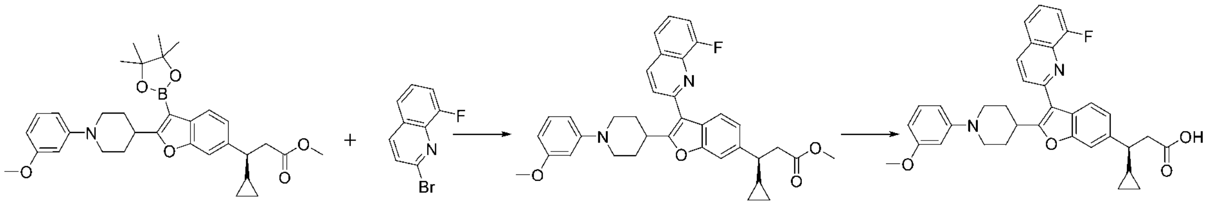

81-1 155-1 155-2 155

Referring to the synthetic route of compound **81,** compound **81-2** was replaced with compound **155-1** to carry out the synthesis. The resulting crude product was purified by reverse-phase column chromatography [acetonitrile/water (0.05% ammonia) = 0% to 100%] to obtain compound **155** (36 mg, yield: 53%) as a white solid. MS (ESI, m/z): 565.2 $[M+H]^+$.

$^1$H NMR (400 MHz, $CDCl_3$) $\delta$ 8.26 (d, *J* = 8.4 Hz, 1H), 7.86 (t, *J*= 8.4 Hz, 2H), 7.63 (d, *J* = 7.6 Hz, 1H), 7.50 - 7.37 (m, 3H), 7.23 - 7.15 (m, 2H), 6.67 - 6.60 (m, 1H), 6.56 (s, 1H), 6.46 - 6.39 (m, 1H), 3.90 - 3.73 (m, 6H), 2.98 - 2.80 (m, 4H), 2.58 - 2.47 (m, 1H), 2.35 - 2.21 (m, 2H), 2.19 - 2.10 (m, 2H), 1.18 - 1.08 (m, 1H), 0.69 - 0.57 (m, 1H), 0.50 - 0.39 (m, 1H), 0.37 - 0.29 (m, 1H), 0.22 - 0.13 (m, 1H).

**Example 156:**

Synthetic Route:

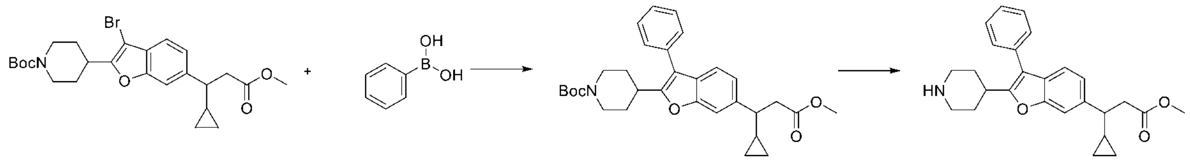

1-1 156-1 156-2 156-3

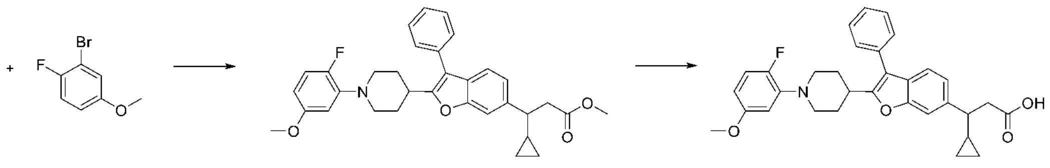

156-4 156-5 156

Referring to the synthetic route of compound 6, compound **6-1** was replaced with compound **156-1** and compound **6-4** was replaced with compound **156-4** to carry out the synthesis. The resulting crude product was purified by reverse-phase column chromatography [acetonitrile/water (0.05% formic acid) = 0% to 100%] to obtain compound 156 (7 mg, yield: 5%) as a white solid. MS (ESI, m/z): 514.3 $[M+H]^+$.

$^1H$ NMR (400 MHz, $CDCl_3$) $\delta$ 7.59 - 7.42 (m, 5H), 7.42 - 7.32 (m, 2H), 7.13 (d, *J*= 8.0 Hz, 1H), 7.00 - 6.91 (m, 1H), 6.61 - 6.50 (m, 1H), 6.48 - 6.41 (m, 1H), 3.82 - 3.76 (m, 3H), 3.64 - 3.52 (m, 2H), 3.16 - 3.01 (m, 1H), 2.94 - 2.69 (m, 4H), 2.59 - 2.47 (m, 1H), 2.43 - 2.26 (m, 2H), 1.99 - 1.90 (m, 2H), 1.16 - 1.06 (m, 1H), 0.70 - 0.58 (m, 1H), 0.52 - 0.41 (m, 1H), 0.40 - 0.30 (m, 1H), 0.27 - 0.15 (m, 1H).

**Example 157:**

Synthetic Route:

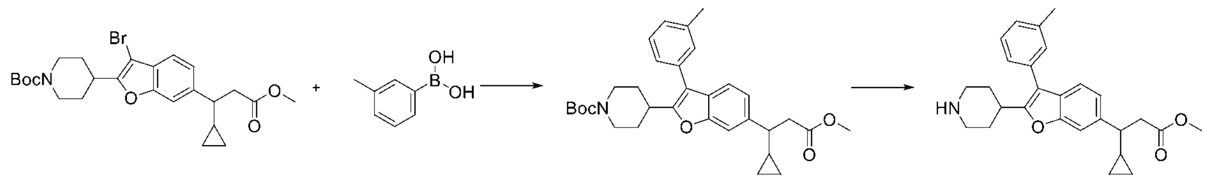

1-1 157-1 157-2 157-3

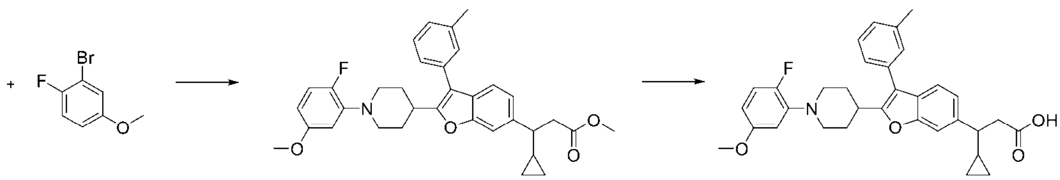

156-4 157-4 157

Referring to the synthetic route of compound **156,** compound **156-1** was replaced with compound **157-1** to carry out the synthesis. The resulting crude product was purified by reverse-phase column chromatography [acetonitrile/water (0.05% formic acid) = 0% to 100%] to obtain compound **157** (10 mg, yield: 40%) as a white solid. MS (ESI, m/z): 528.2 $[M+H]^+$.

$^1H$ NMR (400 MHz, $CDCl_3$) $\delta$ 7.47 (d, *J* = 8.0 Hz, 1H), 7.42 - 7.37 (m, 2H), 7.33 - 7.28 (m, 1H), 7.27 - 7.19 (m, 1H), 7.13 (d, *J = 8.0* Hz, 1H), 7.01 - 6.91 (m, 1H), 6.58 - 6.51 (m, 1H), 6.48 - 6.39 (m, 1H), 3.79 (s, 3H), 3.62 - 3.54 (m, 2H), 3.13 - 3.03 (m, 1H), 2.92 - 2.83 (m, 2H), 2.80 - 2.70 (m, 2H), 2.57 - 2.48 (m, 1H), 2.46 (s, 3H), 2.39 - 2.28 (m, 2H), 1.98 - 1.88 (m, 2H), 1.15 - 1.03 (m, 1H), 0.68 - 0.57 (m, 1H), 0.49 - 0.42 (m, 1H), 0.39 - 0.29 (m, 1H), 0.25 - 0.17 (m, 1H).

**Example 158:**

Synthetic Route:

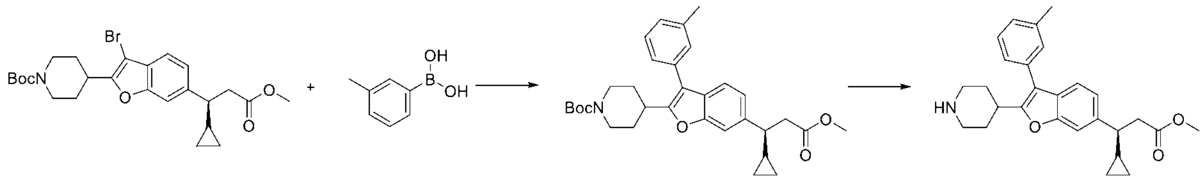

12-1 157-1 158-1 158--2

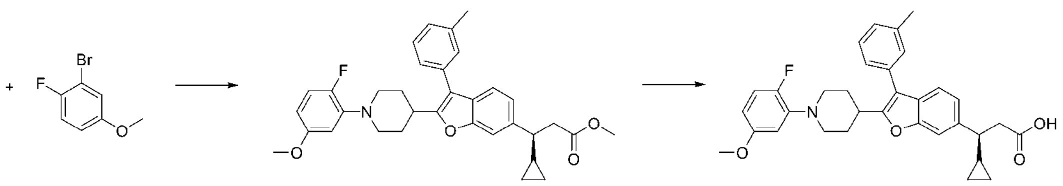

156-4 158-3 158

Referring to the synthetic route of compound 157, compound 1-1 was replaced with compound 12-1 to carry out the synthesis. The resulting crude product was purified by reverse-phase column chromatography [acetonitrile/water (0.05% formic acid) = 0% to 100%] to obtain compound 158 (20 mg, yield: 42%) as a white solid. MS (ESI, m/z): 528.2 $[M+H]^+$.

$^1H$ NMR (400 MHz, $CDCl_3$) δ 7.47 (d, *J* = 8.0 Hz, 1H), 7.41 - 7.36 (m, 2H), 7.30 (s, 1H), 7.27 - 7.20 (m, 2H), 7.13 (d, *J* = 7.6 Hz, 1H), 7.01 - 6.91 (m, 1H), 6.59 - 6.50 (m, 1H), 6.48 - 6.39 (m, 1H), 3.79 (s, 3H), 3.64 - 3.53 (m, 2H), 3.13 - 3.00 (m, 1H), 2.92 - 2.84 (m, 2H), 2.81 - 2.69 (m, 2H), 2.55 - 2.48 (m, 1H), 2.45 (s, 3H), 2.40 - 2.28 (m, 2H), 1.96 - 1.90 (m, 2H), 1.18 - 1.06 (m, 1H), 0.68 - 0.57 (m, 1H), 0.49 - 0.41 (m, 1H), 0.39 - 0.26 (m, 1H), 0.26 - 0.15 (m, 1H).

## Example 159:

Synthetic Route:

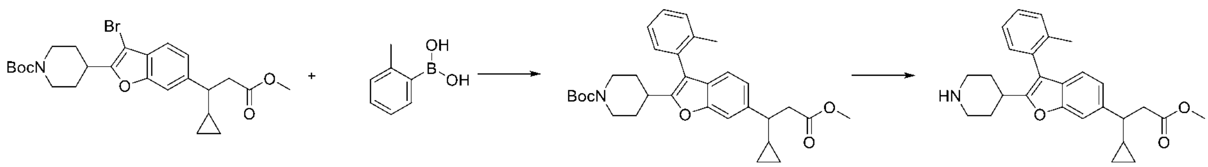

1-1 159-1 159-2 159-3

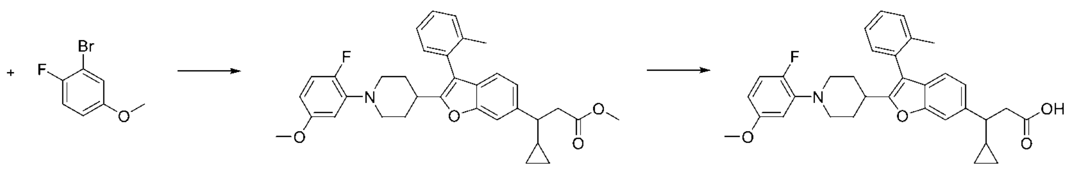

156-4 159-4 159

Referring to the synthetic route of compound **156,** compound **156-1** was replaced with compound **159-1** to carry out the synthesis. The resulting crude product was purified by reverse-phase column chromatography [acetonitrile/water (0.05% formic acid) = 0% to 100%] to obtain compound **159** (2 mg, yield: 23%) as a white solid. MS (ESI, m/z): 528.2 $[M+H]^+$.

$^1H$ NMR (400 MHz, $CDCl_3$) δ 7.39 (s, 1H), 7.37 - 7.33 (m, 2H), 7.28 - 7.21 (m, 2H), 7.14 - 7.05 (m, 2H), 6.99 - 6.90 (m, 1H), 6.55 - 6.46 (m, 1H), 6.45 - 6.37 (m, 1H), 3.77 (s, 3H), 3.58 - 3.49 (m, 2H), 2.92 - 2.84 (m, 2H), 2.84 - 2.62 (m, 3H), 2.55 - 2.46 (m, 1H), 2.31 - 2.18 (m, 5H), 1.94 - 1.83 (m, 2H), 1.15 - 1.05 (m, 1H), 0.68 - 0.57 (m, 1H), 0.52 - 0.42 (m, 1H), 0.39 - 0.31 (m, 1H), 0.27 - 0.19 (m, 1H).

**Example 160:**

Synthetic Route:

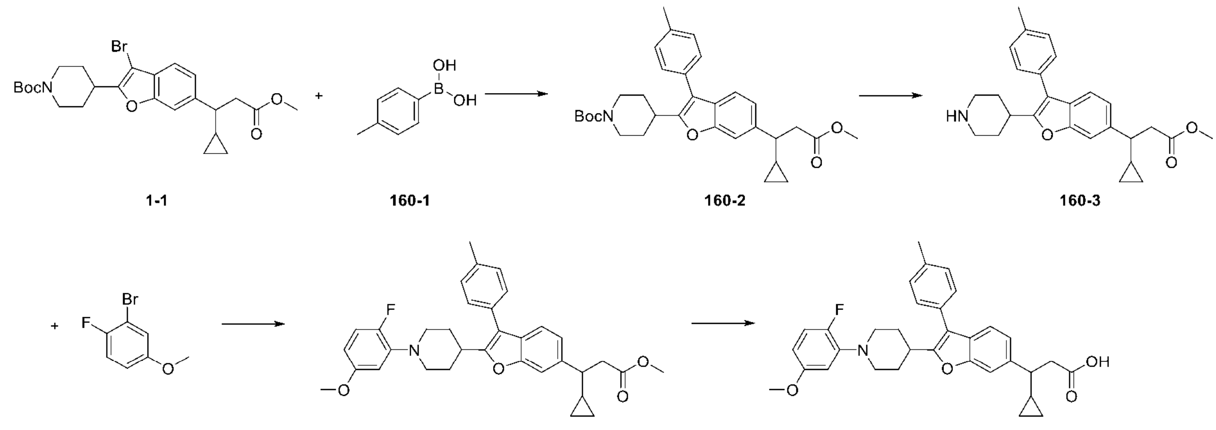

1-1 160-1 160-2 160-3

156-4 160-4 160

Referring to the synthetic route of compound **156,** compound **156-1** was replaced with compound **160-1** to carry out the synthesis. The resulting crude product was purified by reverse-phase column chromatography [acetonitrile/water (0.05% formic acid) = 0% to 100%] to obtain compound **160** (15 mg, yield: 73%) as a white solid. MS (ESI, m/z): 528.2 $[M+H]^+$.

$^1H$ NMR (400 MHz, $CDCl_3$) $\delta$ 7.46 (d, $J$ = 8.0 Hz, 1H), 7.40 -7.36 (m, 3H), 7.34 - 7.30 (m, 2H), 7.12 (d, $J$ = 8.0 Hz, 1H), 7.01 - 6.91 (m, 1H), 6.58 - 6.49 (m, 1H), 6.47 - 6.36 (m, 1H), 3.79 (s, 3H), 3.63 - 3.55 (m, 2H), 3.13 - 3.00 (m, 1H), 2.91 - 2.84 (m, 2H), 2.79 - 2.70 (m, 2H), 2.56 - 2.47 (m, 1H), 2.45 (s, 3H), 2.41 - 2.30 (m, 2H), 1.95 - 1.88 (m, 2H), 1.17 - 1.03 (m, 1H), 0.68 - 0.58 (m, 1H), 0.52 - 0.41 (m, 1H), 0.41 - 0.32 (m, 1H), 0.28 - 0.18 (m, 1H).

**Example 161:**

Synthetic Route:

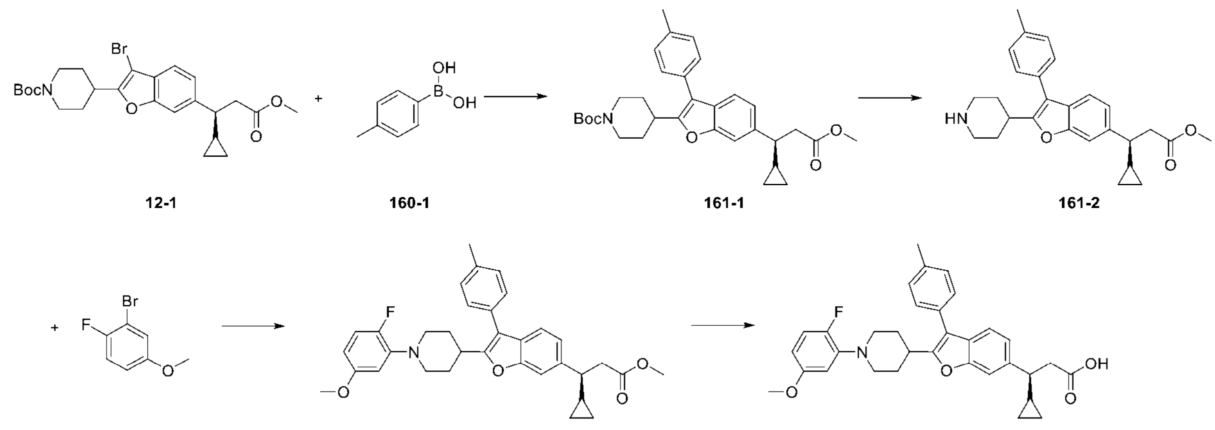

12-1 160-1 161-1 161-2

156-4 161-3 161

Referring to the synthetic route of compound 160, compound 1-1 was replaced with compound **12-1** to carry out the synthesis. The resulting crude product was purified by reverse-phase column chromatography [acetonitrile/water (0.05% formic acid) = 0% to 100%] to obtain compound **161** (9 mg, yield: 28%) as a white solid. MS (ESI, m/z): 528.2 $[M+H]^+$.

$^1H$ NMR (400 MHz, $CDCl_3$) $\delta$ 7.46 (d, J= 7.6 Hz, 1H), 7.41 - 7.35 (m, 3H), 7.34 - 7.30 (m, 2H), 7.12 (d, $J$ = 8.8 Hz, 1H), 6.99 - 6.93 (m, 1H), 6.61 - 6.48 (m, 1H), 6.49 - 6.39 (m, 1H), 3.79 (s, 3H), 3.66 - 3.53 (m, 2H), 3.13 - 3.02 (m, 1H), 2.95 -

2.71 (m, 4H), 2.57 - 2.49 (m, 1H), 2.45 (s, 3H), 2.41 - 2.29 (m, 2H), 1.99 - 1.90 (m, 2H), 1.17 - 1.06 (m, 1H), 0.68 - 0.59 (m, 1H), 0.50 - 0.43 (m, 1H), 0.39 - 0.31 (m, 1H), 0.27 - 0.19 (m, 1H).

**Example 162:**

Synthetic Route:

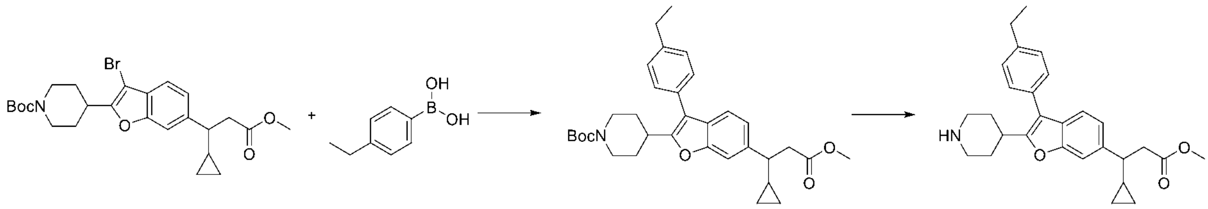

1-1 162-1 162-2 162-3

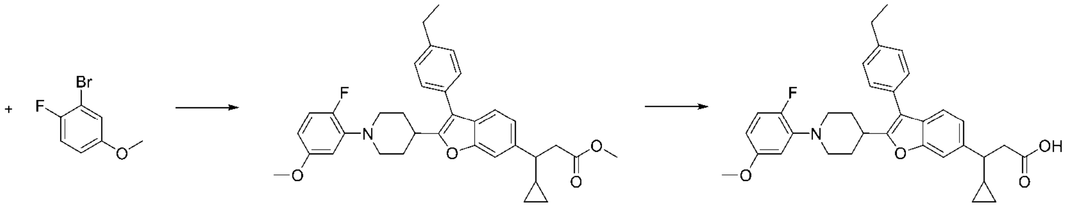

156-4 162-4 162

Referring to the synthetic route of compound **156,** compound **156-1** was replaced with compound **162-1** to carry out the synthesis. The resulting crude product was purified by reverse-phase column chromatography [acetonitrile/water (0.05% formic acid) = 0% to 100%] to obtain compound **162** (9 mg, yield: 49%) as a white solid. MS (ESI, m/z): 542.2 $[M+H]^+$.

$^1H$ NMR (400 MHz, $CDCl_3$) δ 7.48 (d, *J* = 7.2 Hz, 1H), 7.42 - 7.37 (m, 3H), 7.36 - 7.31 (m, 2H), 7.12 (d, *J*= 8.0 Hz, 1H), 7.01 - 6.91 (m, 1H), 6.59 - 6.49 (m, 1H), 6.47 - 6.39 (m, 1H), 3.79 (s, 3H), 3.62 - 3.54 (m, 2H), 3.14 - 3.02 (m, 1H), 2.91 - 2.83 (m, 2H), 2.80 - 2.70 (m, 4H), 2.56 - 2.46 (m, 1H), 2.42 - 2.25 (m, 2H), 1.97 - 1.89 (m, 2H), 1.33 (t, *J* = 7.2 Hz, 3H), 1.16 - 1.06 (m, 1H), 0.68 - 0.60 (m, 1H), 0.52 - 0.39 (m, 1H), 0.39 - 0.29 (m, 1H), 0.27 - 0.17 (m, 1H).

**Example 163:**

Synthetic Route:

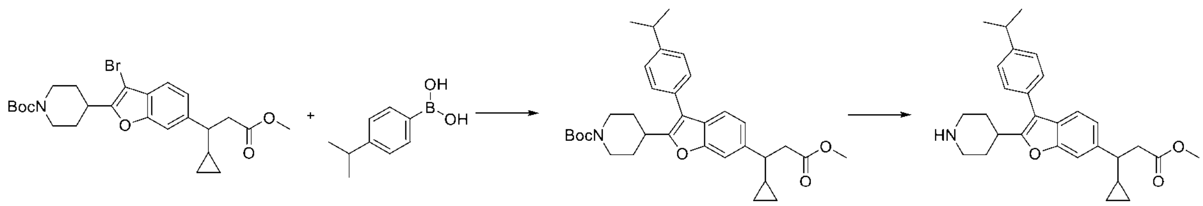

1-1 163-1 163-2 163-3

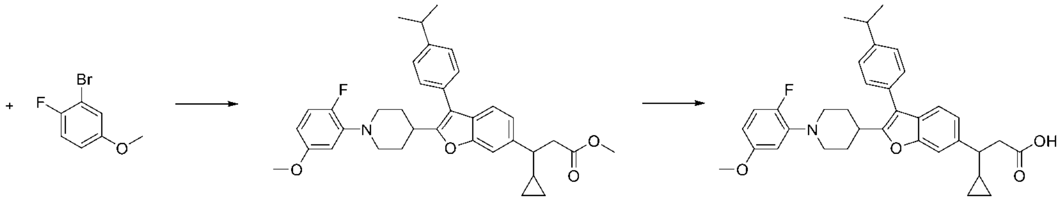

156-4 163-4 163

Referring to the synthetic route of compound **156,** compound **156-1** was replaced with compound **163-1** to carry out the synthesis. The resulting crude product was purified by reverse-phase column chromatography [acetonitrile/water

(0.05% formic acid) = 0% to 100%] to obtain compound **163** (9 mg, yield: 47%) as a white solid. MS (ESI, m/z): 556.3 $[M+H]^+$.

$^1$H NMR (400 MHz, DMSO-$d_6$) δ 7.51 (s, 1H), 7.47 - 7.39 (m, 2H), 7.36 (s, 1H), 7.32 - 7.28 (m, 2H), 7.18 (d, *J* = 8.0 Hz, 1H), 7.07 - 7.02(m, 1H), 6.57 - 6.55 (m, 1H), 6.51 - 6.47 (m, 1H), 3.72 (s, 3H), 3.49 - 3.46 (m, 2H), 3.03 - 2.97 (m, 2H), 2.79 - 2.65 (m, 4H), 2.43 - 2.37 (m, 1H), 2.15 - 2.05 (m, 2H), 1.95 - 1.92 (m, 2H), 1.27 (d, *J*= 7.2 Hz, 6H), 1.09 - 1.06 (m, 1H), 0.53 - 0.51 (m, 1H), 0.32 - 0.28 (m, 2H), 0.16 - 0.14 (m, 1H).

**Example 164:**

Synthetic Route:

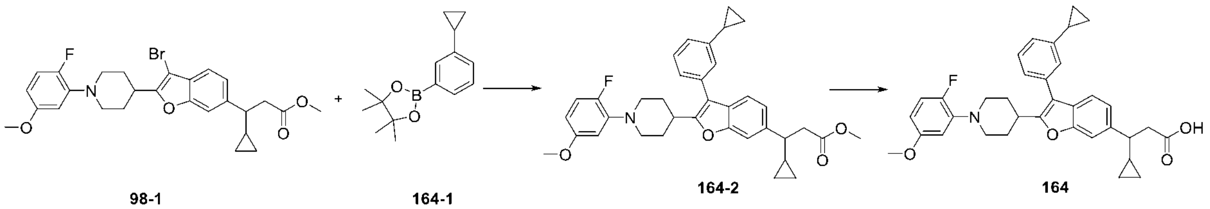

98-1 164-1 164-2 164

Referring to the synthetic route of compound **98,** compound **98-2** was replaced with compound **164-1** and compound **98-3** was replaced with compound **98-1.** The resulting crude product was purified by reverse-phase column chromatography [acetonitrile/water (0.05% formic acid) = 0% to 100%] to obtain compound **164** (21 mg, yield: 61%) as a white solid. MS (ESI, m/z): 554.3 $[M+H]^+$.

$^1$H NMR (400 MHz, MeOD) δ 7.41 (s, 1H), 7.39 -7.31 (m, 2H), 7.23 (d, *J*= 7.6 Hz, 1H), 7.20 - 7.14 (m, 2H), 7.09 (d, *J* = 7.6 Hz, 1H), 6.98 - 6.88 (m, 1H), 6.62 - 6.56 (m, 1H), 6.56 - 6.44 (m, 1H), 3.75 (s, 3H), 3.57 - 3.49 (m, 2H), 3.10 - 3.01 (m, 1H), 2.77 - 2.69 (m, 2H), 2.69 - 2.50 (m, 3H), 2.33 - 2.19 (m, 2H), 2.04 - 1.88 (m, 3H), 1.14 - 1.06 (m, 1H), 1.05 - 0.98 (m, 2H), 0.76 - 0.69 (m, 2H), 0.63 - 0.54 (m, 1H), 0.44 - 0.33 (m, 2H), 0.18 - 0.10 (m, 1H).

**Example 165:**

Synthetic Route:

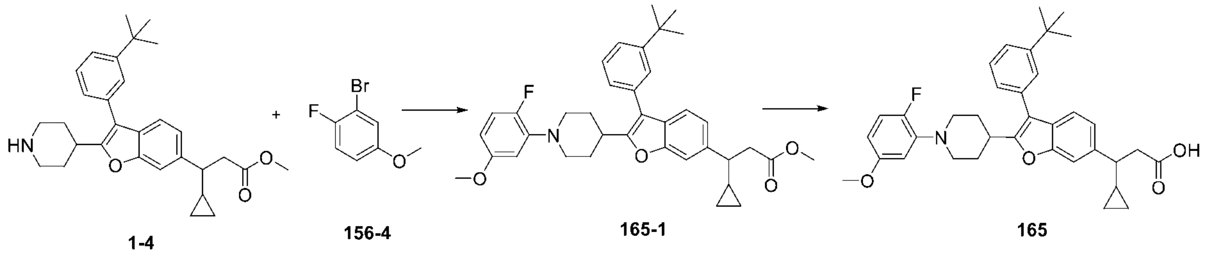

1-4 156-4 165-1 165

Referring to the synthetic route of compound 156, compound 156-3 was replaced with compound **1-4** to carry out the synthesis. The resulting crude product was purified by reverse-phase column chromatography [acetonitrile/water (0.05% formic acid) = 0% to 100%] to obtain compound **165** (7 mg, yield: 5%) as a white solid. MS (ESI, m/z): 570.3 $[M+H]^+$.

$^1$H NMR (400 MHz, $CDCl_3$) δ 7.59 - 7.47 (m, 2H), 7.47 - 7.36 (m, 3H), 7.36 - 7.30 (m, 1H), 7.16 (d, *J*= 8.0 Hz, 1H), 6.99 - 6.89 (m, 1H), 6.62 - 6.51 (m, 1H), 6.49 - 6.38 (m, 1H), 3.79 (s, 3H), 3.61 (d, *J* = 11.2 Hz, 2H), 3.18 - 3.05 (m, 1H), 2.97 - 2.82 (m, 2H), 2.81 - 2.66 (m, 2H), 2.59 - 2.47 (m, 1H), 2.45 - 2.31 (m, 2H), 2.01 - 1.92 (m, 2H), 1.40 (s, 9H), 1.18 - 1.03 (m, 1H), 0.70 - 0.59 (m, 1H), 0.55 - 0.43 (m, 1H), 0.41 - 0.32 (m, 1H), 0.28 - 0.17 (m, 1H).

**Example 166:**

Synthetic Route:

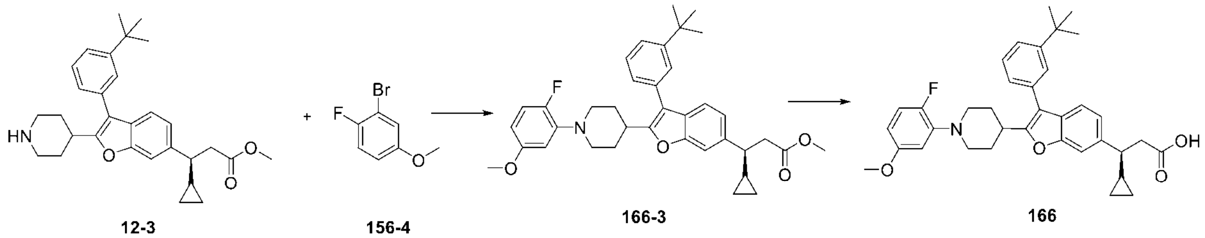

12-3 156-4 166-3 166

Referring to the synthetic route of compound **165,** compound **1-4** was replaced with compound **12-3** to carry out the synthesis. The resulting crude product was purified by reverse-phase column chromatography [acetonitrile/water (0.05% formic acid) = 0% to 100%] to obtain compound **166** (30 mg, yield: 18%) as a white solid. MS (ESI, m/z): 570.3 [M+H]$^+$.

$^1$H NMR (400 MHz, $CDCl_3$) δ 7.54 - 7.47 (m, 2H), 7.45 - 7.41 (m, 2H), 7.39 (s, 1H), 7.33 - 7.28 (m, 1H), 7.14 (d, *J*= 8.0 Hz, 1H), 7.00 - 6.91 (m, 1H), 6.61 - 6.50 (m, 1H), 6.48 - 6.38 (m, 1H), 3.78 (s, 3H), 3.60 (d, *J* = 11.6 Hz, 2H), 3.16 - 3.03 (m, 1H), 2.94 - 2.82 (m, 2H), 2.80 - 2.67 (m, 2H), 2.57 - 2.46 (m, 1H), 2.45 - 2.28 (m, 2H), 2.00 - 1.89 (m, 2H), 1.39 (s, 9H), 1.15 - 1.06 (m, 1H), 0.69 - 0.56 (m, 1H), 0.52 - 0.40 (m, 1H), 0.40 - 0.29 (m, 1H), 0.26 - 0.16 (m, 1H).

**Example 167:**

Synthetic Route:

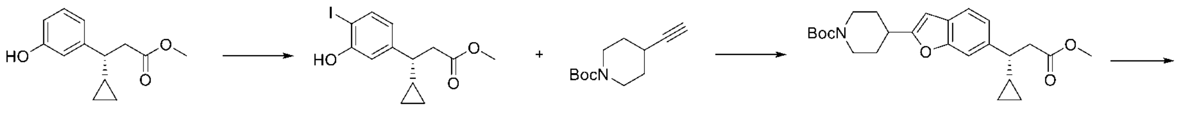

M8-1 M8-2 M1-9 M8

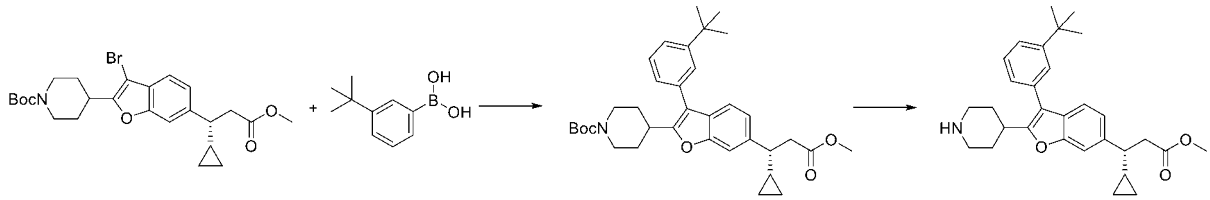

167-1 1-2 167-2 167-3

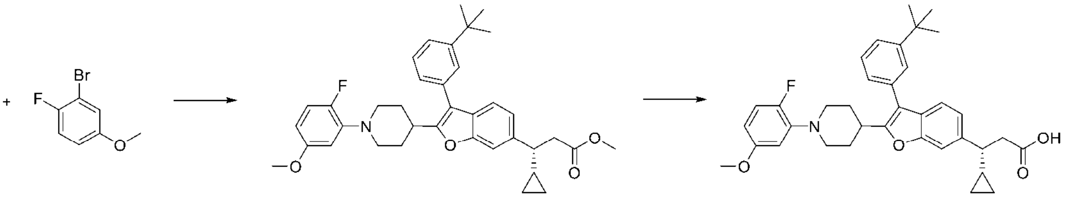

156-4 167-4 167

The synthesis of intermediate **M1** was referred to obtain intermediate **M8.** Then, referring to the synthetic route of compound **165,** compound **1-1** was replaced with compound **167-1** to carry out the synthesis. The resulting crude product was purified by reverse-phase column chromatography [acetonitrile/water (0.05% formic acid) = 0% to 100%] to obtain compound **167** (26 mg, yield: 16%) as a white solid. MS (ESI, m/z): 570.3 [M+H]$^+$.

$^1$H NMR (400 MHz, DMSO-$d_6$) δ 7.53 - 7.41 (m, 5H), 7.33 (d, *J* = 6.4 Hz, 1H), 7.18 (d, *J* = 8.0 Hz, 1H), 7.09 - 7.00 (m, 1H), 6.58 - 6.47 (m, 2H), 3.72 (s, 3H), 3.54 - 3.43 (m, 2H), 3.11 - 2.98 (m, 1H), 2.78 - 2.67 (m, 4H), 2.42 - 2.38 (m, 1H), 2.12 - 2.08 (m, 2H), 1.94 - 1.92 (m, 2H), 1.35 (s, 9H), 1.11 - 1.03 (m, 1H), 0.52 - 0.48 (m, 1H), 0.35 - 0.24 (m, 2H), 0.19 - 0.12 (m, 1H).

**Example 168:**

Synthetic Route:

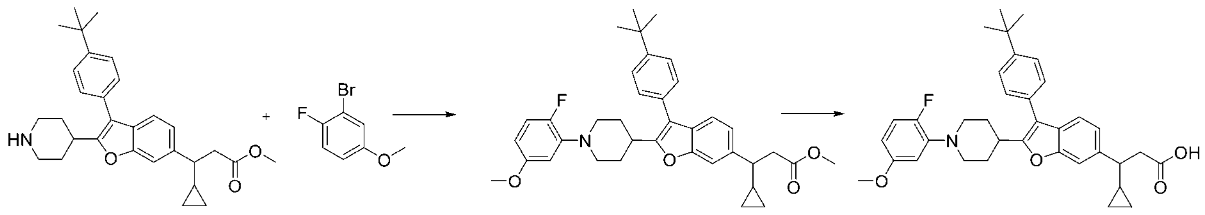

6-3 156-4 168-1 168

Referring to the synthetic route of compound **156,** compound **156-3** was replaced with compound **6-3** to carry out the synthesis. The resulting crude product was purified by reverse-phase column chromatography [acetonitrile/water (0.05% formic acid) = 0% to 100%] to obtain compound **168** (40 mg, yield: 7%) as a white solid. MS (ESI, m/z): 570.3 $[M+H]^+$.

$^1$H NMR (400 MHz, $CDCl_3$) δ 7.56 (d, *J* = 8.4 Hz, 2H), 7.48 -7.39 (m, 4H), 7.15 (d, *J = 8.4* Hz, 1H), 7.08-7.02 (m, 1H), 6.58-6.55 (m, 1H), 6.52 - 6.45 (m, 1H), 3.72 (s, 3H), 3.50 - 3.42 (m, 2H), 3.28 - 3.13 (m, 1H), 2.82 - 2.72 (m, 2H), 2.61 - 2.36 (m, 3H), 2.18 - 2.05 (m, 2H), 1.95 - 1.87 (m, 2H), 1.35 (s, 9H), 1.10 - 0.98 (m, 1H), 0.53 - 0.45 (m, 1H), 0.33 - 0.20 (m, 2H), 0.16 - 0.08 (m, 1H).

**Example 169:**

Synthetic Route:

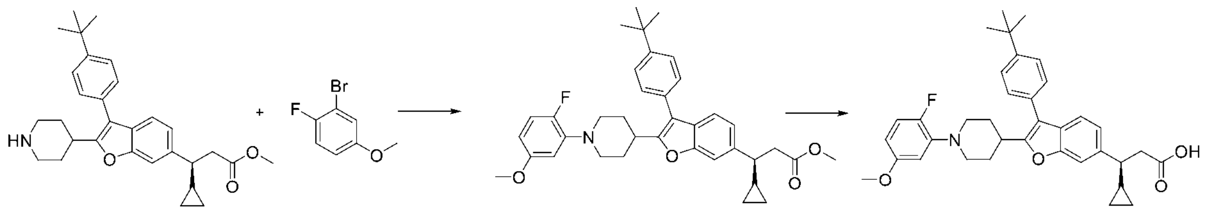

14-2 156-4 169-1 169

Referring to the synthetic route of compound **168,** compound **6-3** was replaced with compound **14-2** to carry out the synthesis. The resulting crude product was purified by reverse-phase column chromatography [acetonitrile/water (0.05% formic acid) = 0% to 100%] to obtain compound **169** (30 mg, yield: 27%) as a white solid. MS (ESI, m/z): 570.3 $[M+H]^+$.

$^1$H NMR (400 MHz, $CDCl_3$) δ 7.56 (d, *J*= 8.4 Hz, 2H), 7.51 (s, 1H), 7.48 - 7.40 (m, 3H), 7.16 (d, *J* = 8.4 Hz, 1H), 7.08 - 7.02 (m, 1H), 6.58 - 6.55 (m, 1H), 6.52 - 6.47 (m, 1H), 3.72 (s, 3H), 3.48 - 3.45 (m, 2H), 3.10 - 3.03 (m, 1H), 2.89 - 2.59 (m, 4H), 2.45 - 2.29 (m, 1H), 2.16 - 2.07 (m, 2H), 1.94 - 1.91 (m, 2H), 1.35 (s, 9H), 1.12 - 1.04 (m, 1H), 0.59 - 0.48 (m, 1H), 0.35 - 0.25 (m, 2H), 0.20 - 0.11 (m, 1H).

**Example 170:**

Synthetic Route:

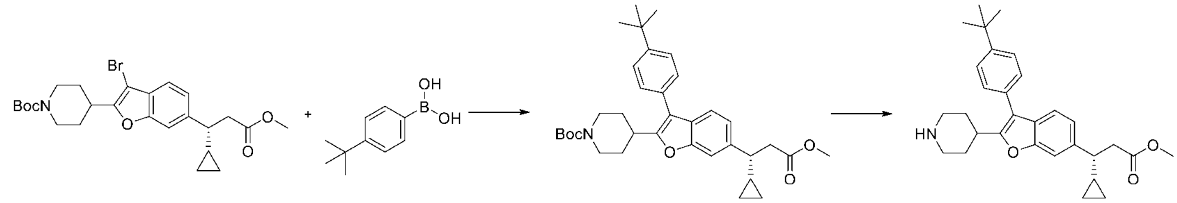

167-1 6-1 170-1 170-2

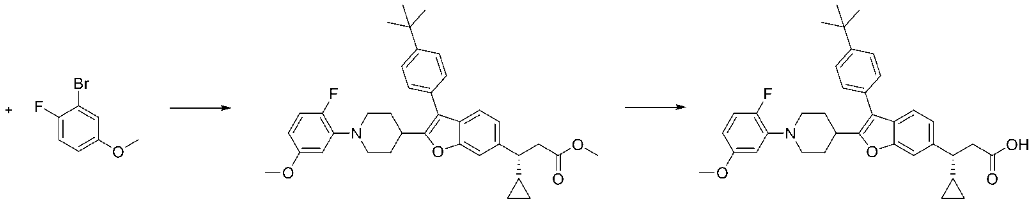

156-4 170-3 170

Referring to the synthetic route of compound **167,** compound **1-2** was replaced with compound **6-1** to carry out the synthesis. The resulting crude product was purified by reverse-phase column chromatography [acetonitrile/water (0.05% formic acid) = 0% to 100%] to obtain compound **170** (28 mg, yield: 25%) as a white solid. MS (ESI, m/z): 570.3 $[M+H]^+$.

$^1$H NMR (400 MHz, DMSO-$d_6$) δ 7.56 (d, $J$ = 8.4 Hz, 2H), 7.51 (s, 1H), 7.46 - 7.42 (m, 3H), 7.17 (d, $J$ = 8.4 Hz, 1H), 7.08 - 7.02 (m, 1H), 6.58 - 6.55 (m, 1H), 6.51 - 6.47 (m, 1H), 3.72 (s, 3H), 3.51 - 3.42 (m, 2H), 3.12 - 3.02 (m, 1H), 2.81 - 2.70 (m, 4H), 2.42 - 2.35 (m, 1H), 2.13 - 2.09 (m, 2H), 1.94 - 1.90 (m, 2H), 1.35 (s, 9H), 1.11 - 1.06 (m, 1H), 0.56 - 0.50 (m, 1H), 0.33 - 0.26 (m, 2H), 0.16 - 0.14 (m, 1H).

**Example 171:**

Synthetic Route:

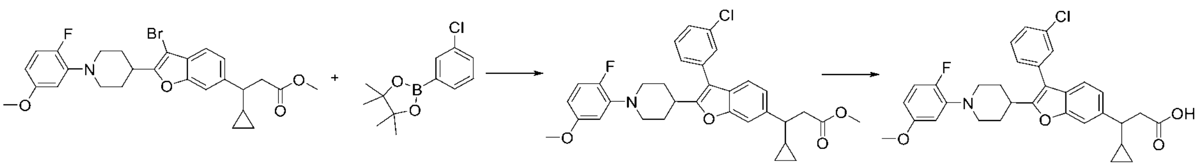

98-1 171-1 171-2 171

Referring to the synthetic route of compound **164,** compound **164-1** was replaced with compound **171-1** to carry out the synthesis. The resulting crude product was purified by reverse-phase column chromatography [acetonitrile/water (0.05% formic acid) = 0% to 100%] to obtain compound **171** (15 mg, yield: 34%) as a white solid. MS (ESI, m/z): 548.2 $[M+H]^+$.

$^1$H NMR (400 MHz, MeOD) δ 7.53 - 7.48 (m, 2H), 7.45 - 7.34 (m, 4H), 7.18 (d, $J$= 8.0 Hz, 1H), 6.94 (dd, $J$ = 12.4, 8.8 Hz, 1H), 6.59 (dd, $J$ = 7.2, 2.8 Hz, 1H), 6.51 - 6.45 (m, 1H), 3.75 (s, 3H), 3.53 - 3.48 (m, 2H), 3.12 - 2.99 (m, 1H), 2.87 - 2.70 (m, 4H), 2.55 - 2.42 (m, 1H), 2.39 - 2.18 (m, 2H), 1.95 - 1.90 (m, 2H), 1.19 - 1.06 (m, 1H), 0.68 - 0.54 (m, 1H), 0.48 - 0.28 (m, 2H), 0.24 - 0.12 (m, 1H).

**Example 172:**

Synthetic Route:

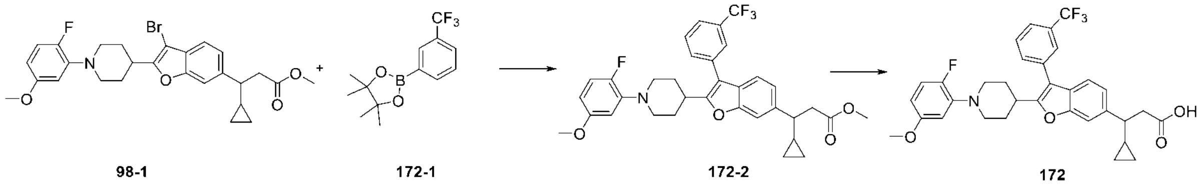

98-1 172-1 172-2 172

Referring to the synthetic route of compound **164,** compound **164-1** was replaced with compound **172-1** to carry out the synthesis. The resulting crude product was purified by reverse-phase column chromatography [acetonitrile/water (0.05% formic acid) = 0% to 100%] to obtain compound **172** (18 mg, yield: 36%) as a white solid. MS (ESI, m/z): 582.2 $[M+H]^+$.

$^1H$ NMR (400 MHz, $CDCl_3$) $\delta$ 7.72 (s, 1H), 7.65 - 7.60 (m, 3H), 7.46 - 7.37 (m, 2H), 7.14 (d, *J*= 7.6 Hz, 1H), 6.96 - 6.91 (m, 1H), 6.55 - 6.48 (m, 1H), 6.44 - 6.40 (m, 1H), 3.77 (s, 3H), 3.59 - 3.56 (m, 2H), 2.98 - 2.96 (m, 1H), 2.90 - 2.84 (m, 2H), 2.75 - 2.67 (m, 2H), 2.54 - 2.44 (m, 1H), 2.40 - 2.26 (m, 2H), 1.96 - 1.85 (m, 2H), 1.15 - 1.04 (m, 1H), 0.67 - 0.58 (m, 1H), 0.50 - 0.41 (m, 1H), 0.38 - 0.30 (m, 1H), 0.24 - 0.16 (m, 1H).

**Example 173:**

Synthetic Route:

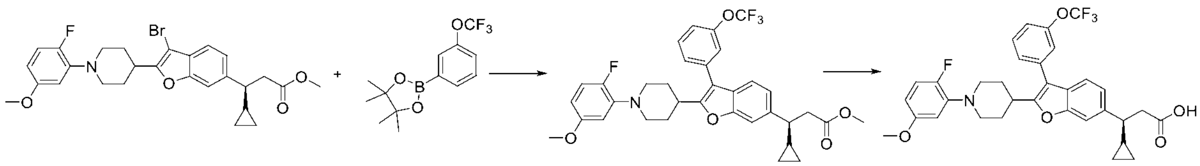

142-1 173-1 173-2 173

Referring to the synthetic route of compound **164,** compound **98-1** was replaced with compound **142-1** and compound **164-1** was replaced with compound **173-1** to carry out the synthesis. The resulting crude product was purified by reverse-phase column chromatography [acetonitrile/water (0.05% formic acid) = 0% to 100%] to obtain compound **173** (14 mg, yield: 48%) as a white solid. MS (ESI, m/z): 598.1 $[M+H]^+$.

$^1H$ NMR (400 MHz, MeOD) $\delta$ 7.65 - 7.59 (m, 1H), 7.51 (d, *J* = 7.6 Hz, 1H), 7.46 - 7.36 (m, 3H), 7.35 - 7.28 (m, 1H), 7.22 - 7.16 (m, 1H), 6.94 (dd, *J* = 12.4, 8.8 Hz, 1H), 6.59 (dd, *J* = 7.2, 3.2 Hz, 1H), 6.52 - 6.46 (m, 1H), 3.75 (s, 3H), 3.59 - 3.49 (m, 2H), 3.12 - 3.02 (m, 1H), 2.87 - 2.68 (m, 4H), 2.52 - 2.44 (m, 1H), 2.34 - 2.21 (m, 2H), 2.00 - 1.90 (m, 2H), 1.18 - 1.07 (m, 1H), 0.66 - 0.51 (m, 1H), 0.46 - 0.29 (m, 2H), 0.21- 0.15 (m, 1H).

**Example 174:**

Synthetic Route:

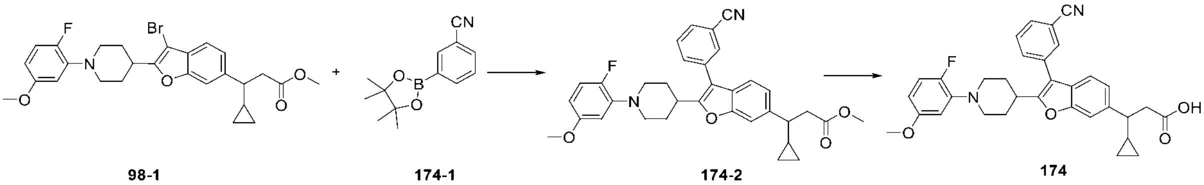

98-1 174-1 174-2 174

Referring to the synthetic route of compound **164,** compound **164-1** was replaced with compound **174-1** to carry out the synthesis. The resulting crude product was purified by reverse-phase column chromatography [acetonitrile/water (0.05% formic acid) = 0% to 100%] to obtain compound **174** (7 mg, yield: 32%) as a white solid. MS (ESI, m/z): 539.2 $[M+H]^+$.

$^1H$ NMR (400 MHz, DMSO-$d_6$) $\delta$ 7.96 (s, 1H), 7.90 - 7.81 (m, 2H), 7.78 - 7.71 (m, 1H), 7.46 (s, 1H), 7.37 (d, *J* = 8.0 Hz, 1H), 7.16 (d, *J* = 8.0 Hz, 1H), 7.04 (dd, *J* = 12.4, 8.8 Hz, 1H), 6.56 (dd, *J* = 7.6, 2.8 Hz, 1H), 6.52 - 6.46 (m, 1H), 3.72 (s, 3H), 3.49 - 3.40 (m, 2H), 3.09 - 2.98 (m, 1H), 2.82 - 2.72 (m, 2H), 2.50 - 2.28 (m, 3H), 2.15 - 2.02 (m, 2H), 1.99 - 1.88 (m, 2H),

1.06 - 0.94 (m, 1H), 0.49 - 0.39 (m, 1H), 0.32 - 0.22 (m, 2H), 0.12 - 0.03 (m, 1H).

**Example 175:**

Synthetic Route:

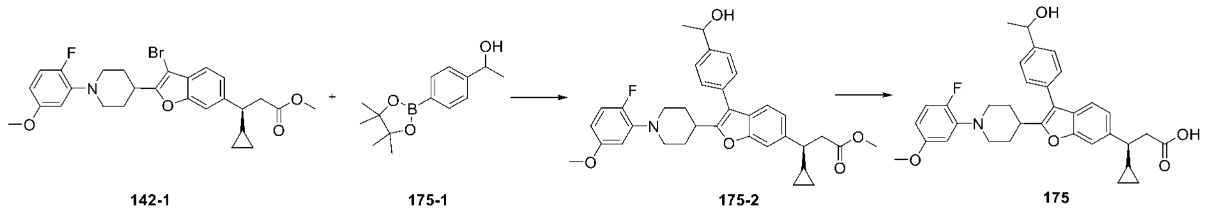

142-1 175-1 175-2 175

Referring to the synthetic route of compound **173,** compound **173-1** was replaced with compound **175-1** to carry out the synthesis. The resulting crude product was purified by reverse-phase column chromatography [acetonitrile/water (0.05% formic acid) = 0% to 100%] to obtain compound **175** (25 mg, yield: 61%) as a white solid. MS (ESI, m/z): 558.3 $[M+H]^+$.

$^1H$ NMR (400 MHz, DMSO-$d_6$) $\delta$ 7.51 - 7.42 (m, 5H), 7.36 (d, $J$ = 8.0 Hz, 1H), 7.14 (d, $J$ = 8.0 Hz, 1H), 7.08 - 7.01 (m, 1H), 6.57 - 6.54 (m, 1H), 6.51 - 6.47 (m, 1H), 5.24 (s, br, 1H), 4.80 - 4.77 (m, 1H), 3.71 (s, 3H), 3.47 - 3.42 (m, 2H), 3.04 - 3.01 (m, 1H), 2.75 - 2.70 (m, 2H), 2.49 - 2.37 (m, 3H), 2.12 - 2.08 (m, 2H), 1.90 - 1.87 (m, 2H), 1.39 (d, $J$ = 6.8 Hz, 3H), 1.02 - 0.99 (m, 1H), 0.48 - 0.43 (m, 1H), 0.33 - 0.17 (m, 2H), 0.11 - 0.07 (m, 1H).

**Example 176:**

Synthetic Route:

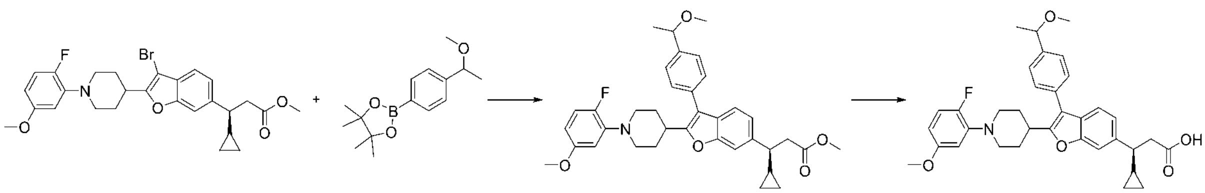

142-1 176-1 176-2 176

Referring to the synthetic route of compound **173,** compound **173-1** was replaced with compound **176-1** to carry out the synthesis. The resulting crude product was purified by reverse-phase column chromatography [acetonitrile/water (0.05% formic acid) = 0% to 100%] to obtain compound **176** (12 mg, yield: 37%) as a white solid. MS (ESI, m/z): 572.2 $[M+H]^+$.

$^1H$ NMR (400 MHz, DMSO-$d_6$) $\delta$ 7.56 - 7.35 (m, 6H), 7.16 (d, $J$ = 8.0 Hz, 1H), 7.07 - 7.01 (m, 1H), 6.58 - 6.54 (m, 1H), 6.52 - 6.46 (m, 1H), 4.42 - 4.36 (m, 1H), 3.71 (s, 3H), 3.46 - 3.44 (m, 2H), 3.19 (s, 3H), 3.09 - 3.05 (m, 1H), 2.80 - 2.74 (m, 2H), 2.47 - 2.41 (m, 3H), 2.13 - 2.08 (m, 2H), 1.95 - 1.89 (m, 2H), 1.40 (d, $J$ = 6.4 Hz, 3H), 1.01 - 0.99 (m, 1H), 0.49 - 0.44 (m, 1H), 0.33 - 0.21 (m, 2H), 0.16 - 0.06 (m, 1H).

**Example 177:**

Synthetic Route:

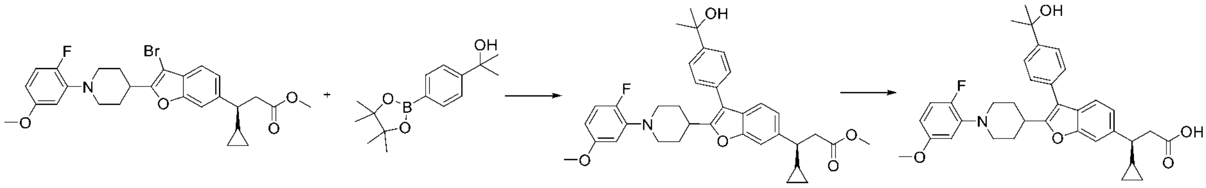

142-1 177-1 177-2 177

Referring to the synthetic route of compound **173,** compound **173-1** was replaced with compound **177-1** to carry out the synthesis. The resulting crude product was purified by reverse-phase column chromatography [acetonitrile/water (0.05% formic acid) = 0% to 100%] to obtain compound **177** (41 mg, yield: 35%) as a white solid. MS (ESI, m/z): 572.3 $[M+H]^+$.

$^1H$ NMR (400 MHz, DMSO-$d_6$) δ 7.62 (d, *J* = 8.4 Hz, 2H), 7.48 - 7.40 (m, 3H), 7.36 (d, *J*= 8.0 Hz, 1H), 7.17 - 7.11 (m, 1H), 7.04 (dd, *J*= 12.4, 8.8 Hz, 1H), 6.56 (dd, *J* = 7.6, 2.8 Hz, 1H), 6.52 - 6.45 (m, 1H), 5.07 (s, br, 1H), 3.72 (s, 3H), 3.50 - 3.42 (m, 2H), 3.11 - 2.99 (m, 1H), 2.83 - 2.70 (m, 2H), 2.48 - 2.29 (m, 3H), 2.20 - 2.03 (m, 2H), 1.95 - 1.87 (m, 2H), 1.49 (s, 6H), 1.05 - 0.94 (m, 1H), 0.50 - 0.38 (m, 1H), 0.32 - 0.20 (m, 2H), 0.12 - 0.04 (m, 1H).

**Example 178:**

Synthetic Route:

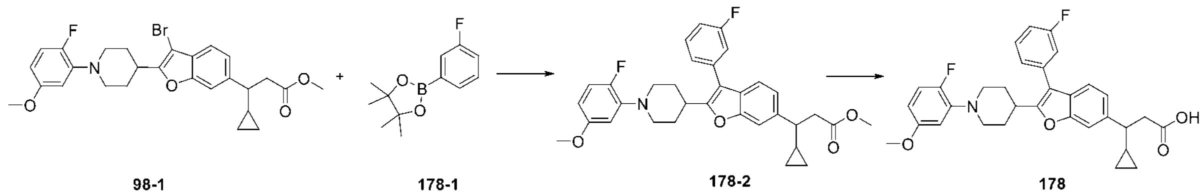

98-1 178-1 178-2 178

Referring to the synthetic route of compound **164,** compound **164-1** was replaced with compound **178-1** to carry out the synthesis. The resulting crude product was purified by reverse-phase column chromatography [acetonitrile/water (0.05% formic acid) = 0% to 100%] to obtain compound **178** (11 mg, yield: 54%) as a white solid. MS (ESI, m/z): 532.2 $[M+H]^+$.

$^1H$ NMR (400 MHz, DMSO-$d_6$) δ 7.62 - 7.54 (m, 1H), 7.45 (s, 1H), 7.39 - 7.29 (m, 3H), 7.29 - 7.22 (m, 1H), 7.16 (d, *J* = 8.0 Hz, 1H), 7.04 (dd, *J* = 12.4, 8.8 Hz, 1H), 6.56 (dd, *J* = 7.6, 2.8 Hz, 1H), 6.52 - 6.46 (m, 1H), 3.72 (s, 3H), 3.50 - 3.41 (m, 2H), 3.10 - 3.00 (m, 1H), 2.82 - 2.72 (m, 2H), 2.47 - 2.37 (m, 3H), 2.16 - 2.02 (m, 2H), 1.98 - 1.87 (m, 2H), 1.05 - 0.94 (m, 1H), 0.49 - 0.41 (m, 1H), 0.30 - 0.21 (m, 2H), 0.11 - 0.04 (m, 1H).

**Example 179:**

Synthetic Route:

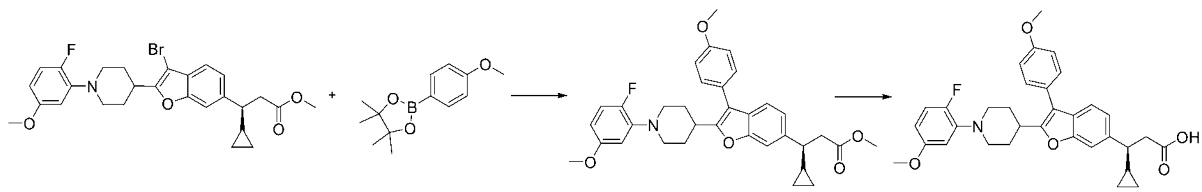

142-1 179-1 179-2 179

Referring to the synthetic route of compound **173,** compound **173-1** was replaced with compound **179-1** to carry out the synthesis. The resulting crude product was purified by reverse-phase column chromatography [acetonitrile/water (0.05% formic acid) = 0% to 100%] to obtain compound **179** (10 mg, yield: 48%) as a white solid. MS (ESI, m/z): 544.2 $[M+H]^+$.

$^1H$ NMR (400 MHz, $CDCl_3$) δ 7.44 (d, *J* = 8.0 Hz, 1H), 7.41 - 7.37 (m, 3H), 7.12 (d, *J* = 8.0 Hz, 1H), 7.08 - 7.01 (m,

2H), 7.00 - 6.90 (m, 1H), 6.57 - 6.50 (m, 1H), 6.48 - 6.38 (m, 1H), 3.89 (s, 3H), 3.78 (s, 3H), 3.61 - 3.55 (m, 2H), 3.08 - 3.00 (m, 1H), 2.90 - 2.83 (m, 2H), 2.79 - 2.71 (m, 2H), 2.53 - 2.46 (m, 1H), 2.39 - 2.29 (m, 2H), 1.95 - 1.89 (m, 2H), 1.15 - 1.01 (m, 1H), 0.69 - 0.58 (m, 1H), 0.51 - 0.41 (m, 1H), 0.37 - 0.29 (m, 1H), 0.26 - 0.16 (m, 1H).

**Example 180:**

Synthetic Route:

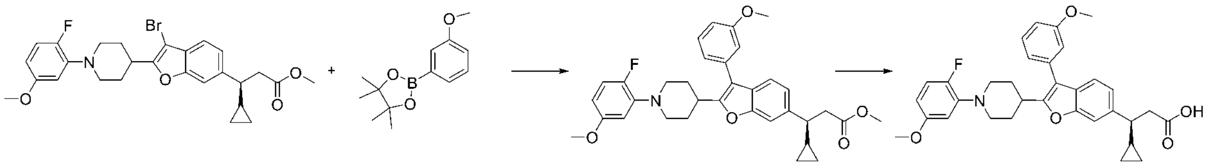

142-1 180-1 180-2 180

Referring to the synthetic route of compound **173,** compound **173-1** was replaced with compound **180-1** to carry out the synthesis. The resulting crude product was purified by reverse-phase column chromatography [acetonitrile/water (0.05% formic acid) = 0% to 100%] to obtain compound **180** (43 mg, yield: 23%) as a white solid. MS (ESI, m/z): 543.3 $[M+H]^+$.

$^1H$ NMR (400 MHz, MeOD) δ 7.45 - 7.36 (m, 3H), 7.15 - 6.89 (m, 5H), 6.57 - 6.49 (m, 1H), 6.51 - 6.45 (m, 1H), 3.85 (s, 3H), 3.74 (s, 3H), 3.59 - 3.47 (m, 2H), 3.15 - 3.06 (m, 1H), 2.86 - 2.69 (m, 4H), 2.45 - 2.43 (m, 1H), 2.32 - 2.20 (m, 2H), 1.99 - 1.89 (m, 2H), 1.17 - 1.07 (m, 1H), 0.65 - 0.56 (m, 1H), 0.46 - 0.29 (m, 2H), 0.21 - 0.13 (m, 1H).

**Example 181:**

Synthetic Route:

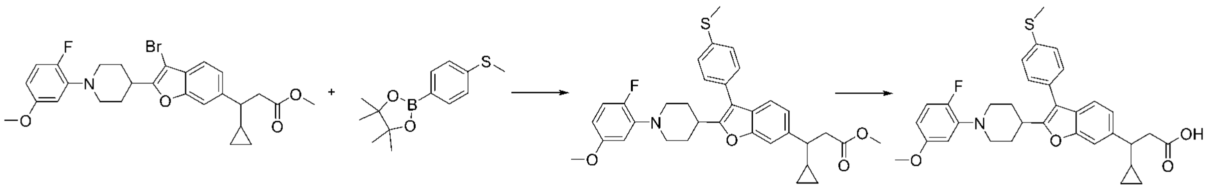

164-1 181-1 181-2 181

Referring to the synthetic route of compound **164,** compound **164-1** was replaced with compound **181-1** to carry out the synthesis. The resulting crude product was purified by reverse-phase column chromatography [acetonitrile/water (0.05% formic acid) = 0% to 100%] to obtain compound **181** (35 mg, yield: 62%) as a white solid. MS (ESI, m/z): 560.2 $[M+H]^+$.

$^1H$ NMR (400 MHz, MeOD) δ 7.45 - 7.38 (m, 5H), 7.36 (d, *J* = 8.0 Hz, 1H), 7.21 - 7.14 (m, 1H), 6.94 (dd, *J* = 12.4, 8.8 Hz, 1H), 6.58 (dd, *J* = 7.2, 3.2 Hz, 1H), 6.50 - 6.45 (m, 1H), 3.75 (s, 3H), 3.56 - 3.49 (m, 2H), 3.11 - 3.01 (m, 1H), 2.80 - 2.45 (m, 8H), 2.32 - 2.18 (m, 2H), 1.98 - 1.86 (m, 2H), 1.13 - 1.02 (m, 1H), 0.62 - 0.52 (m, 1H), 0.44 - 0.31 (m, 2H), 0.18 - 0.08 (m, 1H).

**Example 182:**

Synthetic Route:

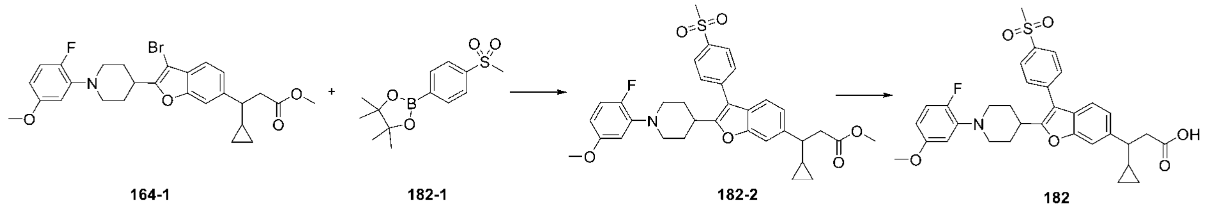

164-1 182-1 182-2 182

Referring to the synthetic route of compound **164,** compound **164-1** was replaced with compound **182-1** to carry out the synthesis. The resulting crude product was purified by reverse-phase column chromatography [acetonitrile/water (0.05% formic acid) = 0% to 100%] to obtain compound **182** (23 mg, yield: 79%) as a white solid. MS (ESI, m/z): 592.2 $[M+H]^+$.

$^1$H NMR (400 MHz, MeOD) $\delta$ 8.15 - 8.05 (m, 2H), 7.78 (d, *J* = 8.4 Hz, 2H), 7.50 - 7.40 (m, 2H), 7.25 - 7.19 (m, 1H), 6.94 (dd, *J* = 12.4, 8.8 Hz, 1H), 6.59 (dd, *J* = 7.2, 2.8 Hz, 1H), 6.52 - 6.45 (m, 1H), 3.75 (s, 3H), 3.59 - 3.50 (m, 2H), 3.19 (s, 3H), 3.15 - 3.06 (m, 1H), 2.82 - 2.63 (m, 4H), 2.56 - 2.45 (m, 1H), 2.34 - 2.21 (m, 2H), 2.01 - 1.92 (m, 2H), 1.15 - 1.03 (m, 1H), 0.64 - 0.53 (m, 1H), 0.43 - 0.33 (m, 2H), 0.19 - 0.11 (m, 1H).

**Example 183:**

Synthetic Route:

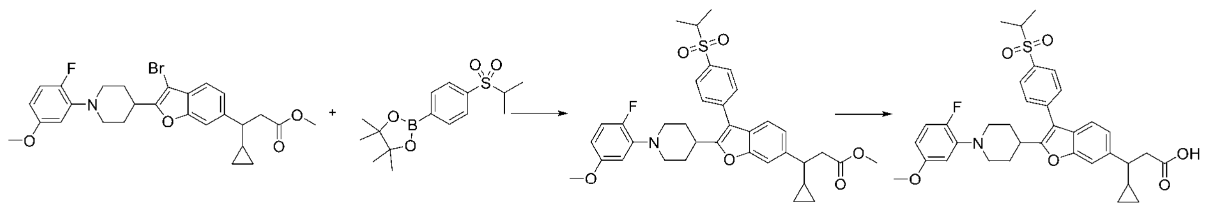

98-1 183-1 183-2 183

Referring to the synthetic route of compound **164,** compound **164-1** was replaced with compound **183-1** to carry out the synthesis. The resulting crude product was purified by reverse-phase column chromatography [acetonitrile/water (0.05% formic acid) = 0% to 100%] to obtain compound **183** (5 mg, yield: 12%) as a white solid. MS (ESI, m/z): 620.2 $[M+H]^+$.

$^1$H NMR (400 MHz, DMSO-$d_6$) $\delta$ 8.01 - 7.96 (m, 2H), 7.81 (d, *J* = 8.4 Hz, 2H), 7.47 - 7.40 (m, 2H), 7.17 (d, *J* = 8.0 Hz, 1H), 7.09 - 7.00 (m, 1H), 6.59 - 6.53 (m, 1H), 6.51 - 6.46 (m, 1H), 3.72 (s, 3H), 3.56 - 3.45 (m, 2H), 3.15 - 3.05 (m, 1H), 2.83 - 2.75 (m, 2H), 2.50 - 2.41 (m, 2H), 2.32 - 2.28 (m, 1H), 2.25 - 2.18 (m, 1H), 2.16 - 2.07 (m, 2H), 1.99 - 1.91 (m, 2H), 1.23 (d, *J* = 6.8 Hz, 6H), 1.04 - 0.93 (m, 1H), 0.46 - 0.39 (m, 1H), 0.30 - 0.20 (m, 2H), 0.10 - 0.03 (m, 1H).

**Example 184:**

Synthetic Route:

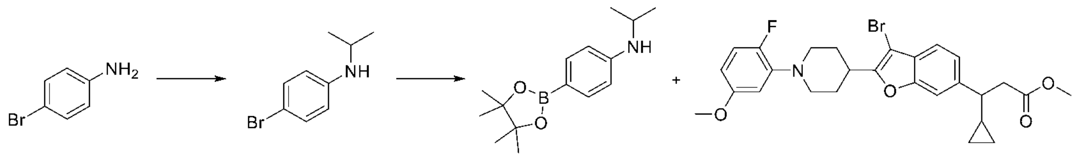

184-1 184-2 184-3 98-1

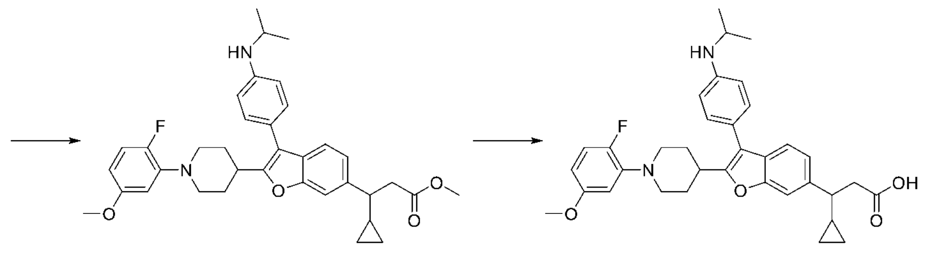

184-4 184

To a solution of compound **184-1** (1.0 g, 5.80 mmol) and sodium acetate (0.95 g, 11.60 mmol) in ethanol/acetic acid/water/acetone (5/8/5/14/ = 3.5 mL) at 0°C, sodium borohydride (1.65 g, 43.50 mmol) was added. The reaction was stirred at 0°C for 3 hours. LCMS monitoring confirmed the complete consumption of the starting material. The reaction mixture was added with saturated sodium bicarbonate solution (20 mL) and extracted with ethyl acetate (30 mL × 3). The combined organic phases were concentrated, and the resulting crude product was purified by normal-phase column chromatography (ethyl acetate: petroleum ether = 0% to 100%) to obtain compound **184-2** (880 mg, yield: 70%) as a colorless oil. MS (ESI, m/z): 214.1 $[M+H]^+$.

At room temperature, in a 25 mL three-necked flask under a nitrogen atmosphere, compound **184-2** (100 mg, 0.46 mmol), potassium acetate (137 mg, 1.40 mmol), [1,1'-bis(diphenylphosphino)ferrocene]palladium(II) chloride (68 mg, 0.09 mmol), and bis(pinacolato)diboron (130 mg, 0.51 mmol) were added. Then, 1,4-dioxane (3 mL) was added and stirred. The reaction mixture was placed in a 90°C oil bath and reacted for 16 hours. LCMS monitoring confirmed the complete consumption of the starting material. The reaction mixture was diluted with ethyl acetate (50 mL), filtered, and concentrated. The resulting crude product was purified by normal-phase column chromatography (ethyl acetate: petroleum ether = 0% to 100%) to obtain compound **184-3** (130 mg, yield: 95%) as a yellow solid. MS (ESI, m/z): 262.3 $[M+H]^+$.

Then, referring to the synthetic route of compound **164,** compound **164-1** was replaced with compound **184-3** to carry out the synthesis. The resulting crude product was purified by reverse-phase column chromatography [acetonitrile/-water (0.05% formic acid) = 0% to 100%] to obtain compound **184** (7 mg, yield: 26%) as a white solid. MS (ESI, m/z): 571.3 $[M+H]^+$.

$^1$H NMR (400 MHz, MeOD) $\delta$ 7.37 - 7.34 (m, 2H), 7.23 (d, $J$ = 8.4 Hz, 2H), 7.14 (d, $J$ = 8.0 Hz, 1H), 6.94 (dd, $J$ = 12.4, 8.8 Hz, 1H), 6.77 (d, $J$ = 8.4 Hz, 2H), 6.59 (dd, $J$ = 7.2, 2.8 Hz, 1H), 6.49 - 6.46 (m, 1H), 3.75 (s, 3H), 3.70 - 3.62 (m, 1H), 3.58 - 3.44 (m, 2H), 3.15 - 3.01 (m, 2H), 2.77 - 2.65 (m, 4H), 2.54 - 2.47 (m, 1H), 2.29 - 2.20 (m, 2H), 1.94 - 1.91(m , 2H), 1.23 (d, $J$ = 6.4 Hz, 6H), 1.10 - 1.06 (m, 1H), 0.58 - 0.54 (m, 1H), 0.39 - 0.35 (m, 2H), 0.15 - 0.11 (m, 1H).

**Example 185:**

Synthetic Route:

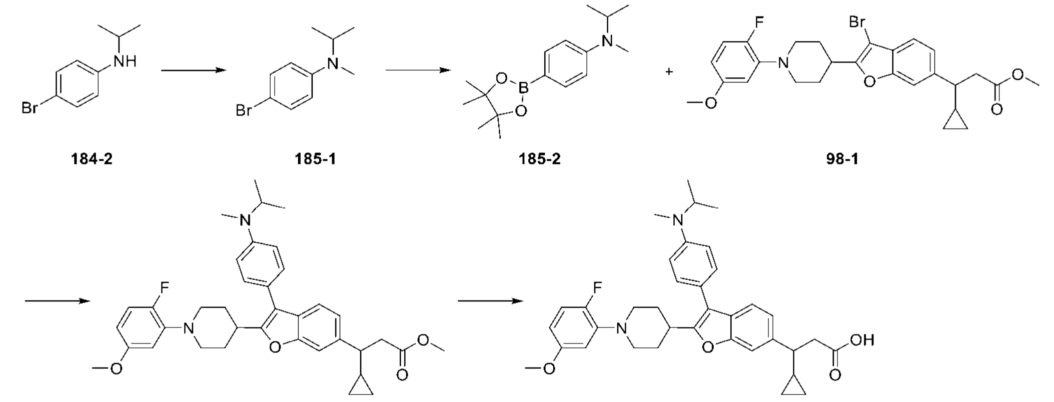

184-2 185-1 185-2 98-1

185-3 185

To a solution of compound **184-2** (200 mg, 0.93 mmol) in *N,N*-dimethylformamide (5 mL) at 0°C, sodium hydride (112 mg, 4.67 mmol) was added. The reaction was stirred at 0°C for 0.5 hours. Then, iodomethane (398 mg, 2.8 mmol) was added, and the reaction mixture was stirred at room temperature for an additional 1.5 hours. LCMS monitoring confirmed the complete consumption of the starting material. The reaction mixture was quenched with water (20 mL) and extracted with ethyl acetate (50 mL × 3). The combined organic phases were concentrated, and the resulting crude product was purified by normal-phase column chromatography (ethyl acetate: petroleum ether = 0% to 20%) to obtain compound **185-1** (100 mg, yield: 47%) as a yellow oil. MS (ESI, m/z): 228.1 $[M+H]^+$.

Then, referring to the synthetic route of compound **184,** compound **184-2** was replaced with compound **185-1** to carry out the synthesis. The resulting crude product was purified by reverse-phase column chromatography [acetonitrile/-water (0.05% formic acid) = 0% to 100%] to obtain compound **185** (32 mg, yield: 33%) as a white solid. MS (ESI, m/z): 585.2 $[M+H]^+$.

$^1$H NMR (400 MHz, MeOD) $\delta$ 7.40 - 7.34 (m, 2H), 7.31 (d, *J* = 7.6 Hz, 2H), 7.15 (d, *J* = 8.0 Hz, 1H), 7.02 - 6.89 (m, 3H), 6.64 - 6.55 (m, 1H), 6.52 - 6.43 (m, 1H), 4.24 - 4.14 (m, 1H), 3.75 (s, 3H), 3.57 - 3.48 (m, 2H), 3.15 - 3.02 (m, 1H), 2.82 - 2.70 (m, 5H), 2.70 - 2.56 (m, 2H), 2.56 - 2.47 (m, 1H), 2.32 - 2.17 (m, 2H), 1.97 - 1.87 (m, 2H), 1.21 (d, *J* = 6.8 Hz, 6H), 1.11 - 1.01 (m, 1H), 0.62 - 0.53 (m, 1H), 0.44 - 0.33 (m, 2H), 0.17 - 0.09 (m, 1H).

**Example 186:**

Synthetic Route:

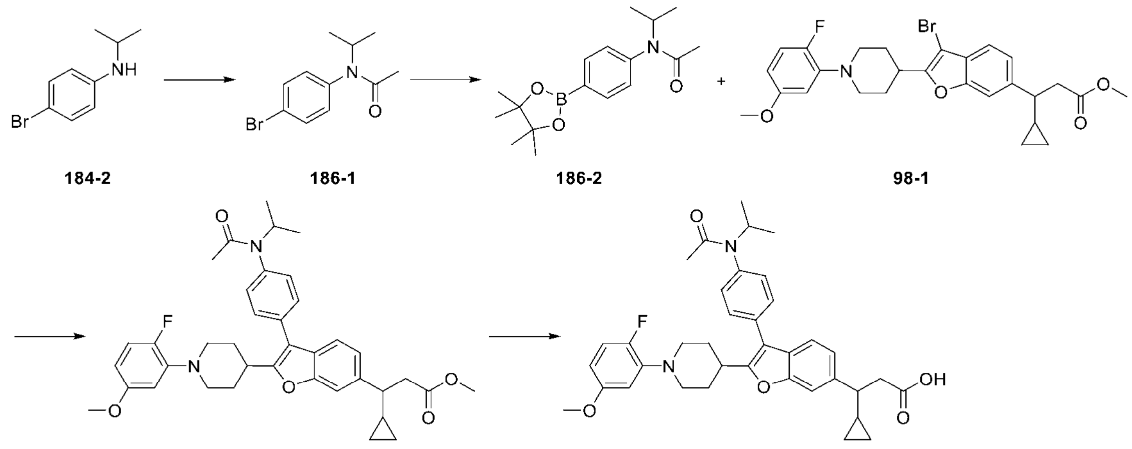

184-2 186-1 186-2 98-1

186-3 186

To a solution of compound **184-2** (200 mg, 0.93 mmol) in dichloromethane (5 mL) at 0°C, acetyl chloride (220 mg, 2.80 mol) was added. The reaction was stirred at room temperature for 1 hour. LCMS monitoring confirmed the complete consumption of the starting material. The reaction mixture was concentrated, and the resulting crude product was purified by normal-phase column chromatography (ethyl acetate: petroleum ether = 0% to 100%) to obtain compound **186-1** (200 mg, yield: 83%) as a yellow solid. MS (ESI, m/z): 256.1 $[M+H]^+$.

Then, referring to the synthetic route of compound **184,** compound **184-2** was replaced with compound **186-1** to carry out the synthesis. The resulting crude product was purified by reverse-phase column chromatography [acetonitrile/-water (0.05% formic acid) = 0% to 100%] to obtain compound **186** (5 mg, yield: 19%) as a white solid. MS (ESI, m/z): 613.3 $[M+H]^+$.

$^1$H NMR (400 MHz, MeOD) $\delta$ 7.63 (d, $J$ = 8.0 Hz, 2H), 7.46 - 7.36 (m, 4H), 7.21 (d, $J$ = 8.0 Hz, 1H), 6.97 - 6.92 (m, 1H), 6.61 - 6.58 (m, 1H), 6.49 - 6.47 (m, 1H), 4.98 - 4.92 (m, 1H), 3.75 (s, 3H), 3.55 - 3.48 (m, 2H), 3.14 - 3.10 (m, 1H), 2.80 - 2.61 (m, 4H), 2.56 - 2.58 (m, 1H), 2.30 - 2.28 (m, 2H), 2.00 - 1.93 (m, 2H), 1.83 (s, 3H), 1.14 (d, $J$ = 6.8 Hz, 6H), 1.10 - 1.08 (m, 1H), 0.59 - 0.58 (m, 1H), 0.41 - 0.36 (m, 2H), 0.15 - 0.13 (m, 1H).

**Example 187:**

Synthetic Route:

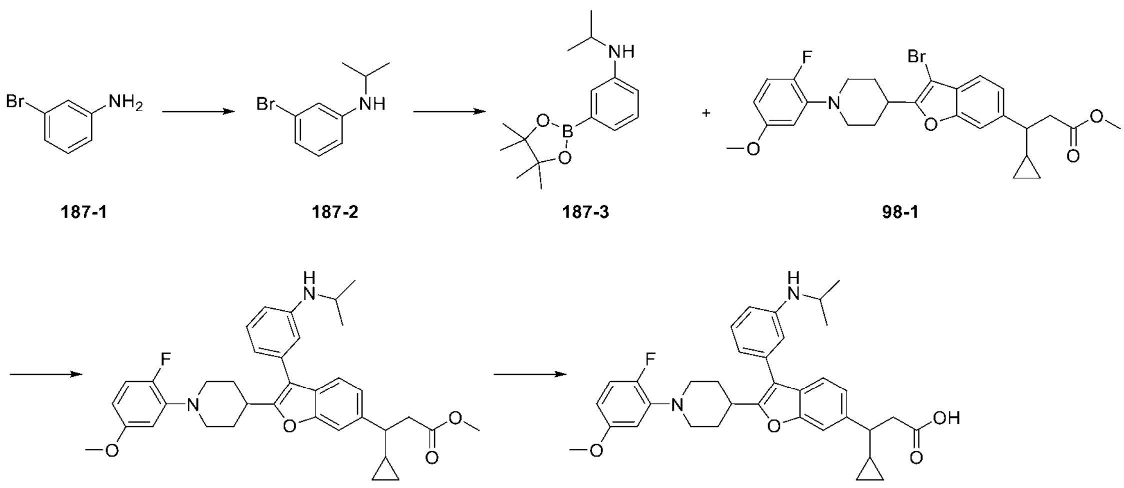

187-1 187-2 187-3 98-1

187-4 187

Referring to the synthetic route of compound **184,** compound **184-1** was replaced with compound **187-1** to carry out the synthesis. The resulting crude product was purified by reverse-phase column chromatography [acetonitrile/water (0.05% formic acid) = 0% to 100%] to obtain compound **187** (13 mg, yield: 27%) as a white solid. MS (ESI, m/z): 571.3 $[M+H]^+$.

$^1$H NMR (400 MHz, DMSO-$d_6$) $\delta$ 7.46 (s, 1H), 7.40 (d, $J$ = 8.0 Hz, 1H), 7.22 - 7.13 (m, 2H), 7.07 - 7.02 (m, 1H), 6.65 - 6.55 (m, 4H), 6.51 - 6.47 (m, 1H), 5.59 (d, $J$ = 7.2 Hz, 1H), 3.72 (s, 3H), 3.60 - 3.43 (m, 3H), 3.11 - 3.09 (m, 1H), 2.79 - 2.71 (m, 2H), 2.54 - 2.37 (s, 3H), 2.16 - 2.04 (m, 2H), 1.93 - 1.88 (m, 2H), 1.17 (d, $J$ = 6.4 Hz, 6H), 1.07 - 0.99 (m, 1H), 0.52 - 0.48 (m, 1H), 0.30 - 0.25 (m, 2H), 0.15 - 0.11 (m, 1H).

**Example 188:**

Synthetic Route:

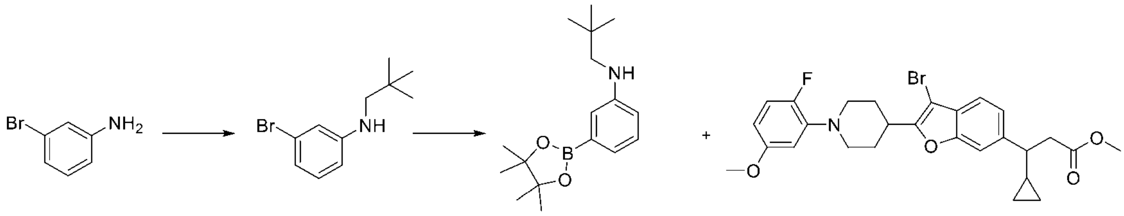

187-1 188-1 188-2 98-1

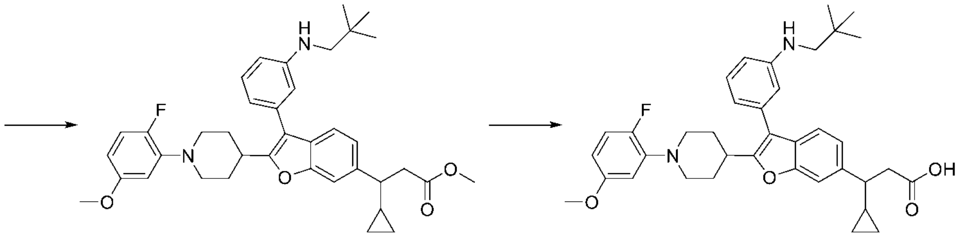

188-3 188

To a solution of compound **187-1** (171 mg, 1 mmol) in 1,2-dichloroethane (2 mL) at 0°C, pivaldehyde (86 mg, 1 mmol), sodium triacetoxyborohydride (636 mg, 3 mmol), acetic acid (60 mg, 1 mmol), and anhydrous magnesium sulfate (120 mg, 1 mmol) were added. The reaction was stirred at room temperature for 2 hours. LCMS monitoring confirmed the complete consumption of the starting material. The reaction mixture was added with saturated sodium bicarbonate solution (5 mL) and extracted with ethyl acetate (10 mL × 3). The combined organic phases were concentrated, and the resulting crude product was purified by normal-phase column chromatography (ethyl acetate: petroleum ether = 0% to 100%) to obtain compound **188-1** (213 mg, yield: 88%) as a colorless oil. MS (ESI, m/z): 242.2 $[M+H]^+$.

Referring to the synthetic route of compound **184,** compound **184-2** was replaced with compound **188-1** to carry out the synthesis. The resulting crude product was purified by reverse-phase column chromatography [acetonitrile/water (0.05% formic acid) = 0% to 100%] to obtain compound **188** (5 mg, yield: 20%) as a white solid. MS (ESI, m/z): 599.4 $[M+H]^+$.

$^1$H NMR (400 MHz, MeOD) $\delta$ 7.43 (d, $J$ = 8.0 Hz, 1H), 7.37 (s, 1H), 7.22 - 7.18 (m, 1H), 7.13 (d, $J$ = 8.0 Hz, 1H), 6.94 (dd, $J$ = 12.4, 8.8 Hz, 1H), 6.77 - 6.74 (m, 1H), 6.71 - 6.64 (m, 2H), 6.61 - 6.56 (m, 1H), 6.51 - 6.46 (m, 1H), 3.75 (s, 3H), 3.54 - 3.48 (m, 2H), 3.18 - 3.13 (m, 1H), 2.94 (s, 2H), 2.84 - 2.70 (m, 4H), 2.51 - 2.45 (m, 1H), 2.31 - 2.22 (m, 2H), 1.96 - 1.93 (m, 2H), 1.14 - 1.11 (m, 1H), 0.96 (s, 9H), 0.63 - 0.58 (m, 1H), 0.42 - 0.32 (m, 2H), 0.20 - 0.15 (m, 1H).

## Example 189

Synthetic Route:

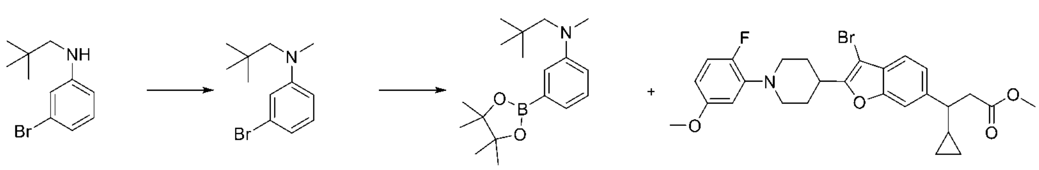

188-1 189-1 189-2 98-1

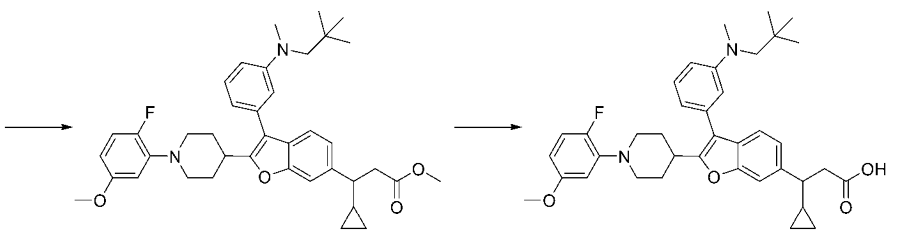

189-3 189

To a solution of compound **188-1** (300 mg, 1.24 mmol) in dichloromethane (5 mL), paraformaldehyde (37.2 mg, 1.24 mmol) and sodium triacetoxyborohydride (787.7 mg, 3.72 mmol) were added. The reaction was stirred at room

temperature for 16 hours. LCMS monitoring confirmed the complete consumption of the starting material. The reaction mixture was added with saturated sodium bicarbonate solution (20 mL) and extracted with ethyl acetate (50 mL × 3). The combined organic phases were concentrated, and the resulting crude product was purified by normal-phase column chromatography (ethyl acetate: petroleum ether = 0% to 100%) to obtain compound **189-1** (100 mg, yield: 32%) as a colorless oil. MS (ESI, m/z): 256.1 [M+H]+.

Then, referring to the synthetic route of compound **184,** compound **184-2** was replaced with compound **189-1** to carry out the synthesis. The resulting crude product was purified by reverse-phase column chromatography [acetonitrile/water (0.05% formic acid) = 0% to 100%] to obtain compound **189** (8 mg, yield: 24%) as a white solid. MS (ESI, m/z): 613.3 [M+H]+.

$^1$H NMR (400 MHz, DMSO-$d_6$) $\delta$ 7.50 (s, 1H), 7.41 (d, *J* = 8.0 Hz, 1H), 7.31 - 7.27 (m, 1H), 7.15 (d, *J* = 8.0 Hz, 1H), 7.07 - 7.02 (m, 1H), 6.81 - 6.79 (m, 2H), 6.71 (d, *J* = 7.6 Hz, 1H), 6.56 - 6.54 (m, 1H), 6.52 - 6.45 (m, 1H), 3.71 (s, 3H), 3.49 - 3.46 (m, 2H), 3.19 (s, 2H), 3.13 - 3.08 (m, 1H), 2.99 (s, 3H), 2.77 - 2.70 (m, 4H), 2.46 - 2.41 (m, 1H), 2.15 - 2.05 (m, 2H), 1.93 - 1.90 (m, 2H), 1.11 - 1.07 (m, 1H), 0.97 (s, 9H), 0.55 - 0.50 (m, 1H), 0.32 - 0.27 (m, 2H), 0.18 - 0.15 (m, 1H).

**Example 190:**

Synthetic Route:

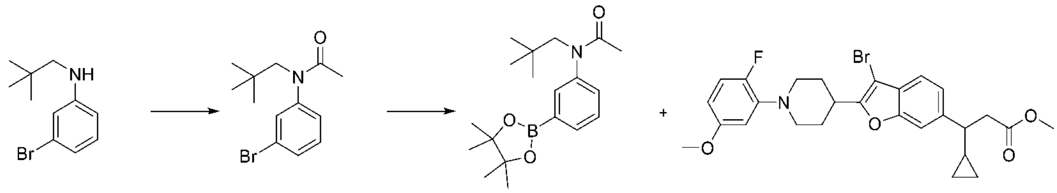

188-1 190-1 190-2 98-1

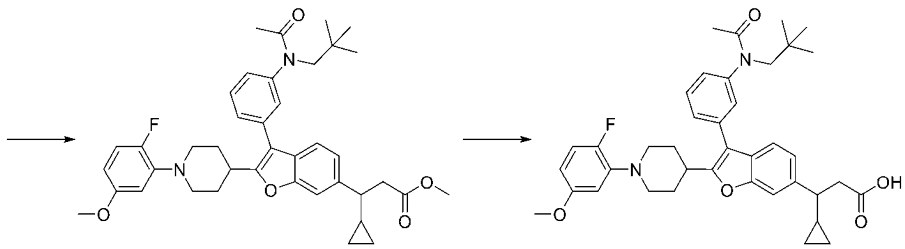

190-3 190

Referring to the synthetic route of compound **186,** compound **184-2** was replaced with compound **188-1** to carry out the synthesis. The resulting crude product was purified by reverse-phase column chromatography [acetonitrile/water (0.05% formic acid) = 0% to 100%] to obtain compound **190** (8 mg, yield: 24%) as a white solid. MS (ESI, m/z): 641.3 [M+H]+.

$^1$H NMR (400 MHz, DMSO-$d_6$) $\delta$ 7.50 (s, 1H), 7.41 (d, *J* = 8.0 Hz, 1H), 7.31 - 7.27 (m, 1H), 7.15 (d, *J* = 8.0 Hz, 1H), 7.07 - 7.02 (m, 1H), 6.81 - 6.79 (m, 2H), 6.71 (d, *J* = 7.6 Hz, 1H), 6.56 - 6.54 (m, 1H), 6.52 - 6.45 (m, 1H), 3.71 (s, 3H), 3.49 - 3.46 (m, 2H), 3.19 (s, 2H), 3.13 - 3.08 (m, 1H), 2.99 (s, 3H), 2.77 - 2.70 (m, 4H), 2.46 - 2.41 (m, 1H), 2.15 - 2.05 (m, 2H), 1.93 - 1.90 (m, 2H), 1.11 - 1.07 (m, 1H), 0.97 (s, 9H), 0.55 - 0.50 (m, 1H), 0.32 - 0.27 (m, 2H), 0.18 - 0.15 (m, 1H).

**Example 191:**

Synthetic Route:

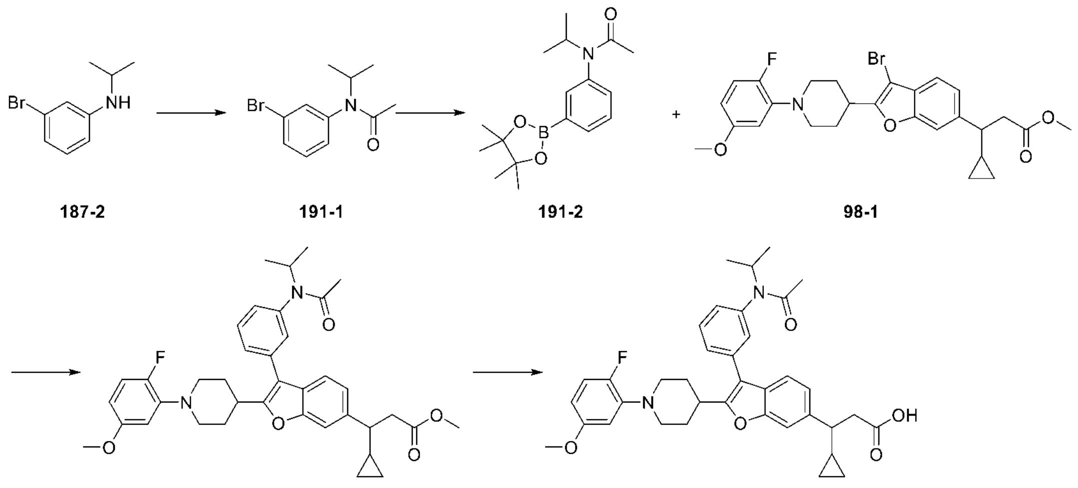

187-2 191-1 191-2 98-1

191-3 191

Referring to the synthetic route of compound **186,** compound **184-2** was replaced with compound **187-2** to carry out the synthesis. The resulting crude product was purified by reverse-phase column chromatography [acetonitrile/water (0.05% formic acid) = 0% to 100%] to obtain compound **191** (25 mg, yield: 51%) as a white solid. MS (ESI, m/z): 613.3 $[M+H]^+$.

$^1H$ NMR (400 MHz, MeOD) $\delta$ 7.69 - 7.58 (m, 2H), 7.45 - 7.37 (m, 2H), 7.32 (s, 1H), 7.27 (d, $J$ = 7.6 Hz, 1H), 7.18 (d, $J$ = 8.0 Hz, 1H), 6.94 (dd, $J$ = 12.4, 8.8 Hz, 1H), 6.56 (dd, $J$ = 7.2, 2.8 Hz, 1H), 6.52 - 6.45 (m, 1H), 5.04 - 4.92 (m, 1H), 3.75 (s, 3H), 3.59 - 3.49 (m, 2H), 3.13 - 3.04 (m, 1H), 2.85 - 2.65 (m, 4H), 2.52 - 2.44 (m, 1H), 2.32 - 2.20 (m, 2H), 2.07 - 1.88 (m, 2H), 1.83 (s, 3H), 1.18 - 1.06 (m, 7H), 0.67 - 0.56 (m, 1H), 0.44 - 0.32 (m, 2H), 0.21 - 0.14 (m, 1H).

**Example 192:**

Synthetic Route:

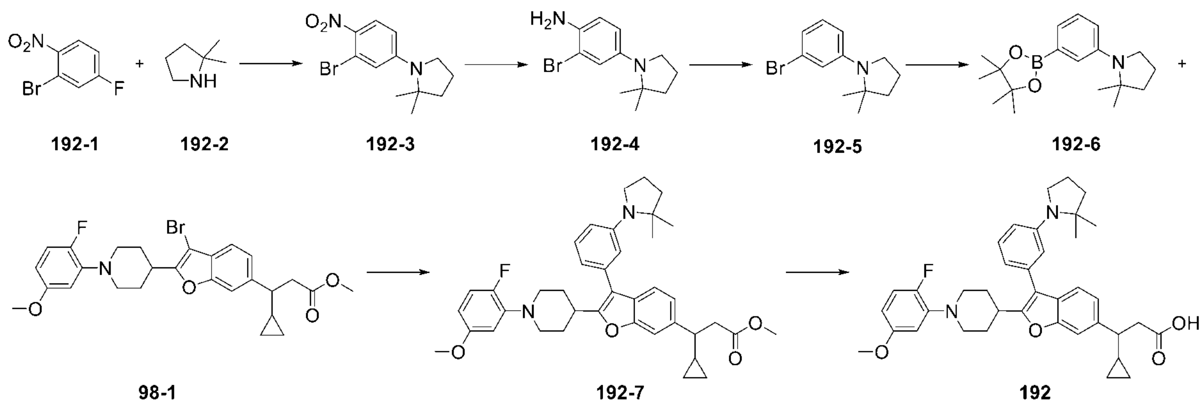

192-1 192-2 192-3 192-4 192-5 192-6

98-1 192-7 192

To a solution of compound **192-1** (200 mg, 0.91 mmol) in *N,N*-dimethylformamide (5 mL), compound **192-2** (90 mg, 0.91 mmol) and potassium carbonate (376 mg, 2.73 mmol) were added. The reaction was stirred at 80°C for 24 hours. LCMS monitoring confirmed the complete consumption of the starting material. The reaction mixture was directly concentrated, and the resulting crude product was purified by normal-phase column chromatography (ethyl acetate: petroleum ether = 0% to 100%) to obtain compound **192-3** (150 mg, yield: 55%) as a yellow oil. MS (ESI, m/z): 299.0 $[M+H]^+$.

To a solution of compound **192-3** (100 mg, 0.33 mmol) in ethanol/water = 1/1 (10 mL), iron powder (93 mg, 1.67 mmol) and ammonium chloride (178 mg, 3.34 mmol) were added. The reaction was stirred at 60°C for 2 hours. LCMS monitoring confirmed the complete consumption of the starting material. The reaction mixture was directly concentrated, and the resulting crude product was purified by normal-phase column chromatography (ethyl acetate: petroleum ether = 0% to 100%) to obtain compound **192-4** (60 mg, yield: 67%) as a pale yellow oil. MS (ESI, m/z): 269.1 $[M+H]^+$.

To a solution of compound **192-4** (50 mg, 0.185 mmol) in tetrahydrofuran (10 mL) at 0°C, a solution of 3 M

hydrochloric acid (0.6 mL) and sodium nitrite (19 mg, 0.28 mmol) in water (1 mL) was added. The reaction was stirred at 0°C for 15 minutes. Then, a solution of potassium iodide (61.5 mg, 0.37 mmol) in water (0.5 mL) was added, and the reaction was stirred at room temperature for 48 hours. LCMS monitoring confirmed the complete consumption of the starting material. The reaction was quenched with saturated sodium sulfite aqueous solution (10 mL) and extracted with ethyl acetate (20 mL × 3). The combined organic phases were concentrated, and the resulting crude product was purified by normal-phase column chromatography (ethyl acetate: petroleum ether = 0% to 100%) to obtain compound **192-5** (25 mg, yield: 53%) as a pale yellow oil. MS (ESI, m/z): 254.1 $[M+H]^+$.

Then, referring to the synthetic route of compound **184,** compound **184-2** was replaced with compound **192-5** to carry out the synthesis. The resulting crude product was purified by reverse-phase column chromatography [acetonitrile/-water (0.05% formic acid) = 0% to 100%] to obtain compound **192** (14 mg, yield: 30%) as a white solid. MS (ESI, m/z): 611.2 $[M+H]^+$.

$^1H$ NMR (400 MHz, MeOD) $\delta$ 7.44 - 7.37 (m, 2H), 7.29 -7.22 (m, 1H), 7.19 - 7.13 (m, 1H), 6.94 (dd, *J* = 12.4, 8.8 Hz, 1H), 6.85 - 6.81 (m, 1H), 6.80 - 6.75 (m, 1H), 6.72 (d, *J* = 7.6 Hz, 1H), 6.58 (dd, *J* = 7.2, 2.8 Hz, 1H), 6.50 - 6.45 (m, 1H), 3.75 (s, 3H), 3.58 - 3.49 (m, 2H), 3.43 - 3.36 (m, 2H), 3.20 - 3.13 (m, 1H), 2.77 - 2.48 (m, 5H), 2.34 - 2.21 (m, 2H), 2.00 - 1.88 (m, 6H), 1.47 (s, 6H), 1.14 - 1.02 (m, 1H), 0.62 - 0.52 (m, 1H), 0.43 - 0.32 (m, 2H), 0.18 - 0.09 (m, 1H).

**Example 193:**

Synthetic Route:

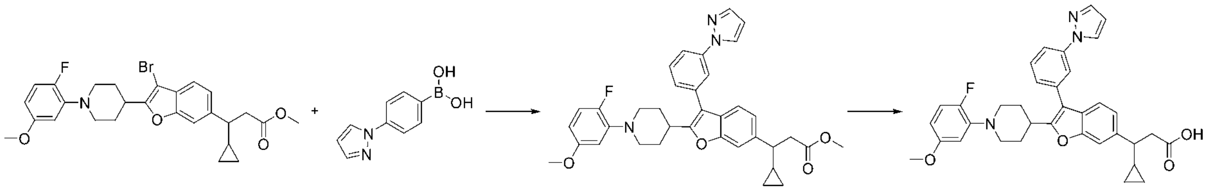

98-1 193-1 193-2 193

Referring to the synthetic route of compound **164,** compound **164-1** was replaced with compound **193-1** to carry out the synthesis. The resulting crude product was purified by reverse-phase column chromatography [acetonitrile/water (0.05% formic acid) = 0% to 100%] to obtain compound **193** (60 mg, yield: 75%) as a white solid. MS (ESI, m/z): 580.3 $[M+H]^+$.

$^1H$ NMR (400 MHz, $CDCl_3$) $\delta$ 8.02 - 7.94 (m, 1H), 7.87 - 7.81 (m, 1H), 7.80 - 7.77 (m, 1H), 7.77 - 7.70 (m, 1H), 7.58 (t, *J* = 8.0 Hz, 1H), 7.50 (d, *J* = 8.0 Hz, 1H), 7.44 - 7.40 (m, 1H), 7.39 - 7.32 (m, 1H), 7.18 - 7.12 (m, 1H), 7.03 - 6.90 (m, 1H), 6.60 - 6.49 (m, 2H), 6.47 - 6.39 (m, 1H), 3.79 (s, 3H), 3.67 - 3.53 (m, 2H), 3.15 - 3.07 (m, 1H), 2.91 - 2.84 (m, 2H), 2.84 - 2.71 (m, 2H), 2.57 - 2.47 (m, 1H), 2.45 - 2.30 (m, 2H), 2.02 - 1.90 (m, 2H), 1.18 - 1.04 (m, 1H), 0.70 - 0.59 (m, 1H), 0.52 - 0.42 (m, 1H), 0.40 - 0.31 (m, 1H), 0.27 - 0.16 (m, 1H).

**Example 194:**

Synthetic Route:

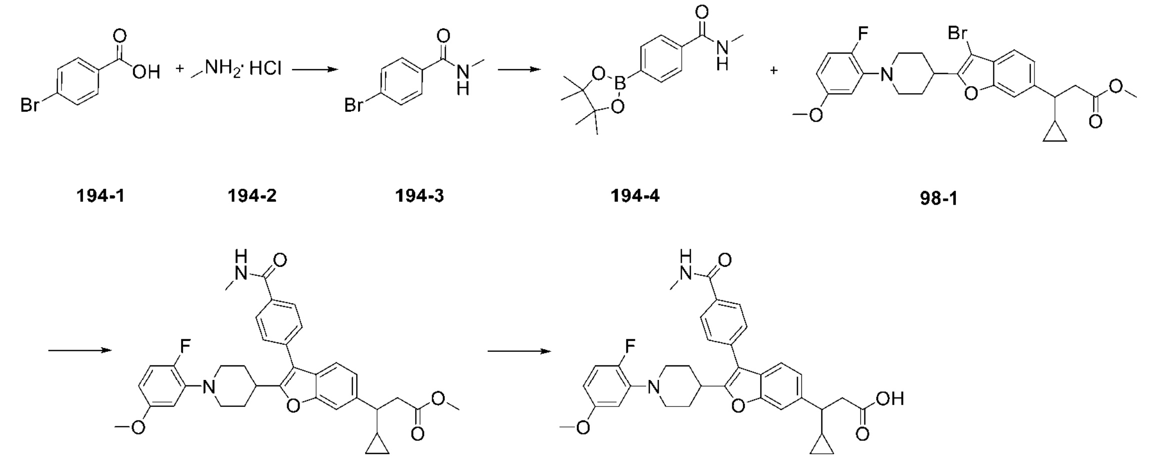

194-1 194-2 194-3 194-4 98-1

194-5 194

To a solution of 4-bromobenzoic acid **194-1** (500 mg, 2.48 mmol) in *N,N*-dimethylformamide (20 mL), 2-(7-azabenzotriazol-1-yl)-*N,N,N',N'*-tetramethyluronium hexafluorophosphate (1.41 g, 3.73 mmol), methylamine hydrochloride **194-2** (839.7 mg, 12.4 mmol), and *N,N*-diisopropylethylamine (964 mg, 7.46 mmol) were added. The reaction was stirred at 25°C for 4 hours. LCMS monitoring confirmed the complete consumption of the starting material. The reaction mixture was diluted with ethyl acetate (50 mL) and washed with water (30 mL × 3). The organic phase was dried over anhydrous sodium sulfate and concentrated under reduced pressure. The resulting crude product was purified by normal-phase column chromatography (ethyl acetate: petroleum ether = 0% to 100%) to obtain compound **194-3** (500 mg, yield: 94%) as a white solid. MS (ESI, m/z): 213.9 $[M+H]^+$.

Then, referring to the synthetic route of compound **184,** compound **184-2** was replaced with compound **194-3** to carry out the synthesis. The resulting crude product was purified by reverse-phase column chromatography [acetonitrile/-water (0.05% formic acid) = 0% to 100%] to obtain compound **194** (14 mg, yield: 27%) as a white solid. MS (ESI, m/z): 571.2 $[M+H]^+$.

$^1H$ NMR (400 MHz, MeOD) δ 7.96 (d, *J* = 8.4 Hz, 2H), 7.60 (d, *J* = 8.4 Hz, 2H), 7.45 -7.40 (m, 2H), 7.18 (d, *J* = 8.4 Hz, 1H), 6.95 (dd, *J* = 12.4, 8.8 Hz, 1H), 6.58 (dd, *J* = 7.2, 2.8 Hz, 1H), 6.52 - 6.44 (m, 1H), 3.74 (s, 3H), 3.53 - 3.50 (m, 2H), 3.14 - 3.06 (m, 1H), 2.96 (s, 3H), 2.87 - 2.69 (m, 4H), 2.53 - 2.43 (m, 1H), 2.33 - 2.23 (m, 2H), 1.95 - 1.92 (m, 2H), 1.21 - 1.01 (m, 1H), 0.67 - 0.57 (m, 1H), 0.46 - 0.30 (m, 2H), 0.22 - 0.14 (m, 1H).

**Example 195:**

Synthetic Route:

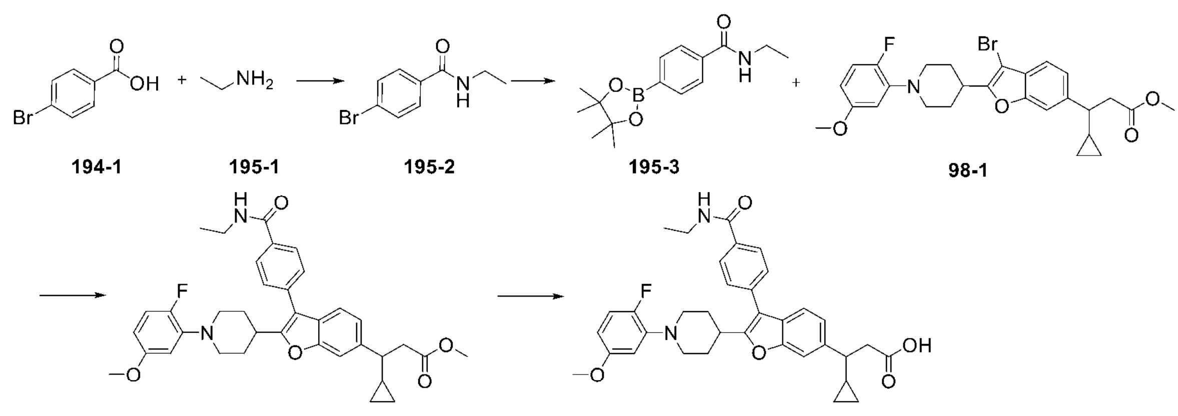

194-1 195-1 195-2 195-3 98-1

195-4 195

Referring to the synthetic route of compound **194,** compound **194-2** was replaced with compound **195-1** to carry out the synthesis. The resulting crude product was purified by reverse-phase column chromatography [acetonitrile/water (0.05% formic acid) = 0% to 100%] to obtain compound **195** (12 mg, yield: 10%) as a white solid. MS (ESI, m/z): 585.3 $[M+H]^+$.

$^1$H NMR (400 MHz, MeOD) δ 7.96 (d, *J* = 8.4 Hz, 2H), 7.59 (d, *J* = 8.4 Hz, 2H), 7.45 - 7.39 (m, 2H), 7.18 (d, *J* = 8.0 Hz, 1H), 6.95 (dd, *J* = 12.4, 8.8 Hz, 1H), 6.58 (dd, *J* = 7.2, 2.8 Hz, 1H), 6.51 - 6.46 (m, 1H), 3.75 (s, 3H), 3.58 - 3.49 (m, 2H), 3.49 - 3.41 (m, 2H), 3.15 - 3.16 (m, 1H), 2.89 - 2.67 (m, 4H), 2.54 - 2.42 (m, 1H), 2.39 - 2.18 (m, 2H), 2.01 - 1.89 (m, 2H), 1.26 (t, *J* = 7.2 Hz, 3H), 1.17 - 1.07 (m, 1H), 0.68 - 0.57 (m, 1H), 0.46 - 0.31 (m, 2H), 0.21 - 0.13 (m, 1H).

**Example 196:**

Synthetic Route:

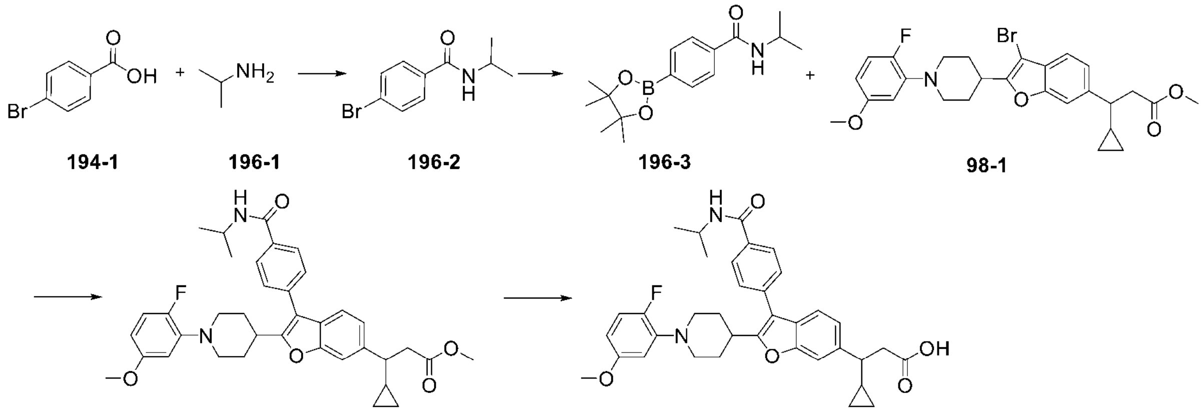

194-1 196-1 196-2 196-3 98-1

196-4 196

Referring to the synthetic route of compound **194,** compound **194-2** was replaced with compound **196-1** to carry out the synthesis. The resulting crude product was purified by reverse-phase column chromatography [acetonitrile/water (0.05% formic acid) = 0% to 100%] to obtain compound **196** (24 mg, yield: 14%) as a white solid. MS (ESI, m/z): 599.3 [M+H]$^+$.

$^1$H NMR (400 MHz, MeOD) δ 7.95 (d, *J* = 8.4 Hz, 2H), 7.59 (d, *J* = 8.4 Hz, 2H), 7.45 - 7.39 (m, 2H), 7.25 - 7.14 (m, 1H), 6.94 (dd, *J* = 12.4, 8.8 Hz, 1H), 6.58 (dd, *J* = 7.2, 3.2 Hz, 1H), 6.53 - 6.44 (m, 1H), 4.38 - 4.12 (m, 1H), 3.75 (s, 3H), 3.53 - 3.50 (m, 2H), 3.14 - 3.07 (m, 1H), 2.80 - 2.67 (m, 4H), 2.55 - 2.45 (m, 1H), 2.33 - 2.20 (m, 2H), 1.99 - 1.90 (m, 2H), 1.28 (d, *J* = 6.4 Hz, 6 H), 1.18 - 1.05 (m, 1H), 0.54 - 0.67 (m, 1H), 0.45 - 0.29 (m, 2H), 0.21 - 0.12 (m, 1H).

**Example 197:**

Synthetic Route:

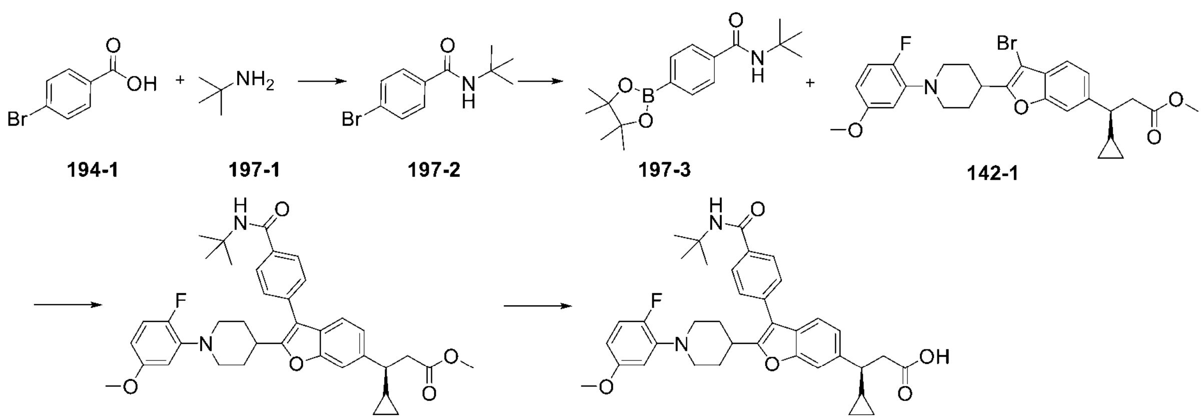

194-1 197-1 197-2 197-3 142-1

197-4 197

Referring to the synthetic route of compound **194,** compound **194-2** was replaced with compound **197-1** and compound **98-1** was replaced with compound **142-1** to carry out the synthesis. The resulting crude product was purified by

reverse-phase column chromatography [acetonitrile/water (0.05% formic acid) = 0% to 100%] to obtain compound **197** (10 mg, yield: 51%) as a white solid. MS (ESI, m/z): 613.3 $[M+H]^+$.

$^1H$ NMR (400 MHz, $CDCl_3$) δ 7.80 (d, *J* = 7.6 Hz, 2H), 7.48 (d, *J* = 7.6 Hz, 2H), 7.42 - 7.31 (m, 2H), 7.11 (d, *J* = 8.0 Hz, 1H), 6.96 - 6.84 (m, 1H), 6.55 - 6.45 (m, 1H), 6.44 - 6.31 (m, 1H), 6.15 (s, br, 1H), 3.74 (s, 3H), 3.58 - 3.46 (m, 2H), 3.05 - 2.91 (m, 1H), 2.72 - 2.63 (m, 4H), 2.50 - 2.40 (m, 1H), 2.37 - 2.20 (m, 2H), 1.93 - 1.82 (m, 2H), 1.46 (s, 9H), 1.11 - 0.97 (m, 1H), 0.65 - 0.52 (m, 1H), 0.46 - 0.36 (m, 1H), 0.35 - 0.27 (m, 1H), 0.20 - 0.10 (m, 1H).

**Example 198:**

Synthetic Route:

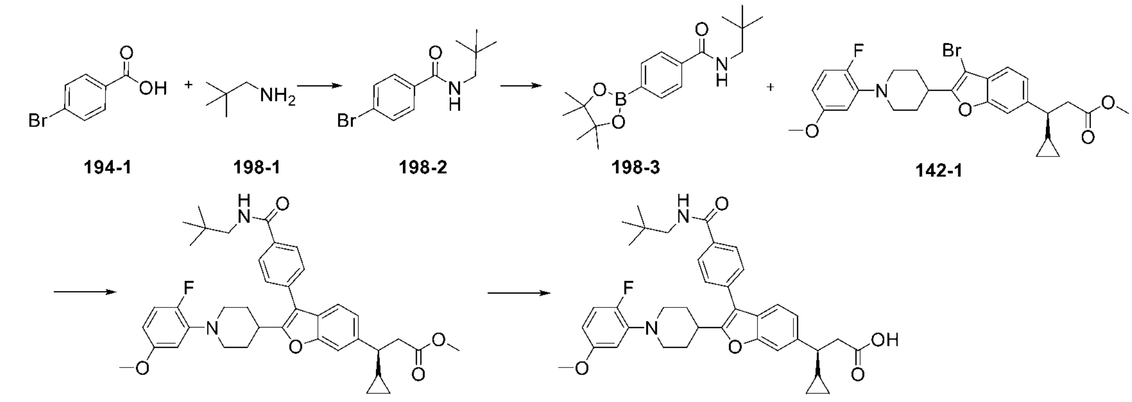

194-1 198-1 198-2 198-3 142-1

198-4 198

Referring to the synthetic route of compound **197,** compound **197-1** was replaced with compound **198-1** to carry out the synthesis. The resulting crude product was purified by reverse-phase column chromatography [acetonitrile/water (0.05% formic acid) = 0% to 100%] to obtain compound **198** (60 mg, yield: 59%) as a white solid. MS (ESI, m/z): 627.3 $[M+H]^+$.

$^1H$ NMR (400 MHz, $CDCl_3$) δ 7.90 (d, *J* = 7.2 Hz, 2H), 7.54 (d, *J* = 7.2 Hz, 2H), 7.48 - 7.37 (m, 2H), 7.18 - 7.10 (m, 1H), 7.02 - 6.89 (m, 1H), 6.54 (s, br, 1H), 6.49 - 6.39 (m, 1H), 6.36 - 6.26 (m, 1H), 3.78 (s, 3H), 3.68 - 3.51 (m, 2H), 3.42 - 3.28 (m, 2H), 3.14 - 2.98 (m, 1H), 2.95 - 2.82 (m, 2H), 2.81 - 2.67 (m, 2H), 2.60 - 2.45 (m, 1H), 2.42 - 2.26 (m, 2H), 1.98 - 1.84 (m, 2H), 1.16 - 1.07 (m, 1H), 1.03 (s, 9H), 0.70 - 0.57 (m, 1H), 0.52 - 0.41 (m, 1H), 0.41 - 0.29 (m, 1H), 0.26 - 0.17 (m, 1H).

**Example 199:**

Synthetic Route:

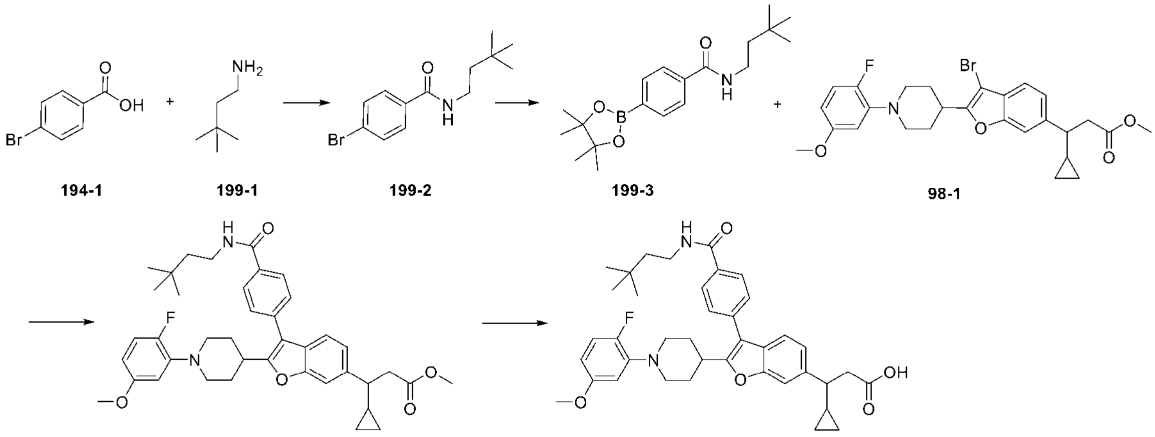

194-1 199-1 199-2 199-3 98-1

199-4 199

Referring to the synthetic route of compound **194,** compound **194-2** was replaced with compound **199-1** to carry out the synthesis. The resulting crude product was purified by reverse-phase column chromatography [acetonitrile/water (0.05% formic acid) = 0% to 100%] to obtain compound **199** (60 mg, yield: 62%) as a white solid. MS (ESI, m/z): 641.3 $[M+H]^+$.

$^1H$ NMR (400 MHz, $CDCl_3$) δ 7.88 (d, *J* = 6.8 Hz, 1H), 7.52 (d, *J* = 6.8 Hz, 1H), 7.46 - 7.37 (m, 2H), 7.13 (d, *J* = 7.2 Hz, 1H), 7.02 - 6.90 (m, 1H), 6.55 - 6.45 (m, 2H), 6.24 - 6.18 (m, 1H), 3.79 (s, 3H), 3.65 - 3.45 (m, 4H), 3.14 - 2.97 (m, 1H), 2.94 - 2.83 (m, 2H), 2.82 - 2.66 (m, 2H), 2.56 - 2.45 (m, 1H), 2.44 - 2.24 (m, 2H), 1.98 - 1.84 (m, 2H), 1.65 - 1.53 (m, 2H), 1.16 - 1.06 (m, 1H), 1.02 (s, 9H), 0.70 - 0.57 (m, 1H), 0.52 - 0.41 (m, 1H), 0.40 - 0.28 (m, 1H), 0.28 - 0.16 (m, 1H).

**Example 200:**

Synthetic Route:

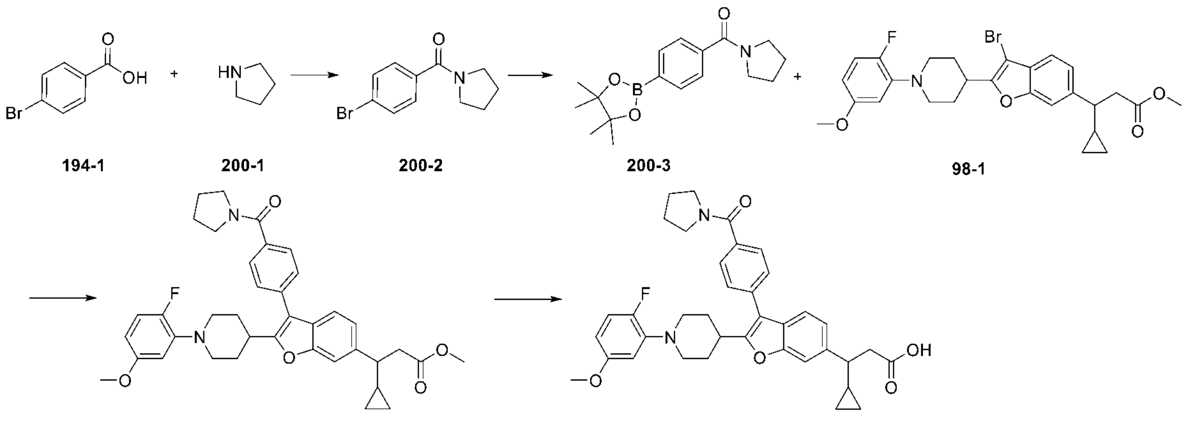

194-1 200-1 200-2 200-3 98-1

200-4 200

Referring to the synthetic route of compound **194,** compound **194-2** was replaced with compound **200-1** to carry out the synthesis. The resulting crude product was purified by reverse-phase column chromatography [acetonitrile/water (0.05% formic acid) = 0% to 100%] to obtain compound **200** (70 mg, yield: 58%) as a white solid. MS (ESI, m/z): 611.3 $[M+H]^+$.

$^1H$ NMR (400 MHz, $CDCl_3$) δ 7.66 (d, *J* = 8.0 Hz, 2H), 7.53 - 7.44 (m, 3H), 7.41 (s, 1H), 7.15 (d, *J* = 8.0 Hz, 1H), 7.05 - 6.87 (m, 1H), 6.73 - 6.30 (m, 2H), 3.80 (s, 3H), 3.76 - 3.66 (m, 2H), 3.65 - 3.49 (m, 4H), 3.11 - 3.03 (m, 1H), 2.92 - 2.72 (m, 4H), 2.59 - 2.45 (m, 1H), 2.45 - 2.19 (m, 2H), 2.13 - 1.78 (m, 6H), 1.19 - 1.00 (m, 1H), 0.71 - 0.57 (m, 1H), 0.55 - 0.40 (m, 1H), 0.39 - 0.30 (m, 1H), 0.28 - 0.12 (m, 1H).

**Example 201:**

Synthetic Route:

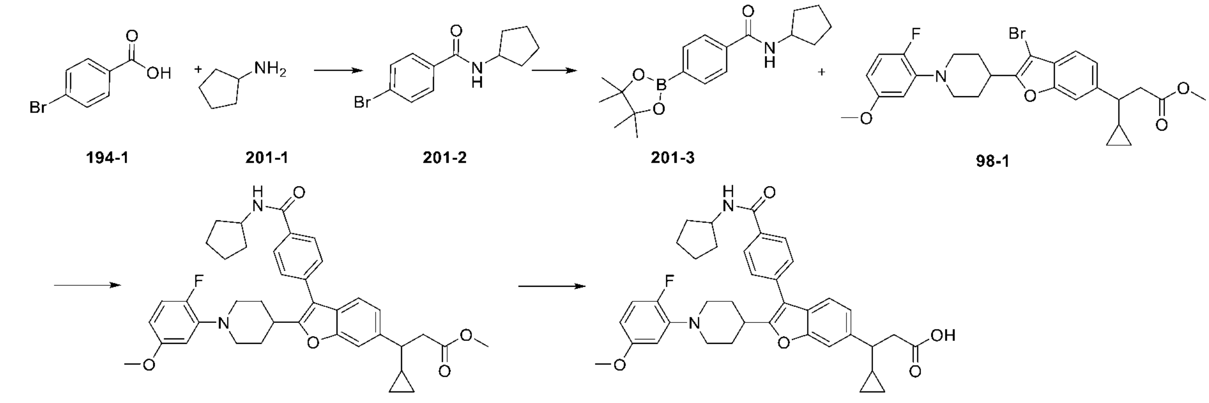

194-1 201-1 201-2 201-3 98-1

201-4 201

Referring to the synthetic route of compound **194,** compound **194-2** was replaced with compound **201-1** to carry out the synthesis. The resulting crude product was purified by reverse-phase column chromatography [acetonitrile/water (0.05% formic acid) = 0% to 100%] to obtain compound **201** (21 mg, yield: 10%) as a white solid. MS (ESI, m/z): 625.3 $[M+H]^+$.

$^1$H NMR (400 MHz, DMSO-$d_6$) δ 8.40 - 8.31 (m, 1H), 7.97 (d, $J$ = 8.4 Hz, 2H), 7.57 (d, $J$ = 8.4 Hz, 2H), 7.47 (s, 1H), 7.41 - 7.34 (m, 1H), 7.17 (d, $J$ = 8.4 Hz, 1H), 7.04 (dd, $J$ = 12.4, 8.8 Hz, 1H), 6.58 - 6.44 (m, 2H), 4.26 - 4.23 (m, 1H), 4.33 - 4.19 (m, 1H), 3.73 (s, 3H), 3.47 - 3.44 (m, 2H), 3.05 - 3.01 (m, 1H), 2.76 - 2.70 (m, 2H), 2.56 - 2.39 (m, 2H), 2.10 - 2.08 (m, 2H), 1.93 - 1.88 (m, 4H), 1.78 - 1.64 (m, 2H), 1.61 - 1.50 (m, 4H), 1.00 - 0.97 (m, 1H), 0.50 - 0.43 (m, 1H), 0.33 - 0.22 (m, 2H), 0.09 - 0.07 (m, 1H).

**Example 202:**

Synthetic Route:

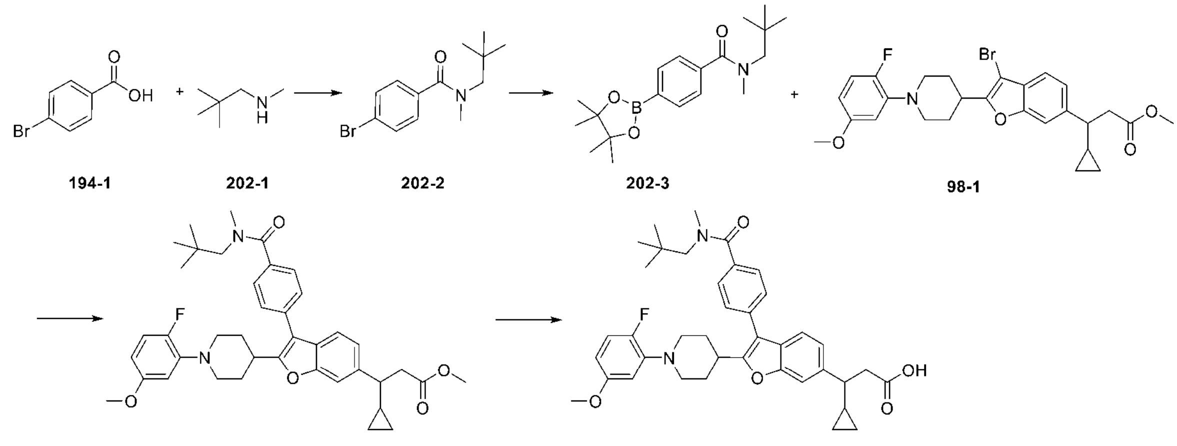

194-1 202-1 202-2 202-3 98-1

202-4 202

Referring to the synthetic route of compound **194,** compound **194-2** was replaced with compound **202-1** to carry out the synthesis. The resulting crude product was purified by reverse-phase column chromatography [acetonitrile/water (0.05% formic acid) = 0% to 100%] to obtain compound **202** (25 mg, yield: 63%) as a white solid. MS (ESI, m/z): 641.3 $[M+H]^+$.

$^1$H NMR (400 MHz, $CDCl_3$) δ 7.63 - 7.42 (m, 5H), 7.40 (s, 1H), 7.14 (d, $J$ = 8.0 Hz, 1H), 7.03 - 6.88 (m, 1H), 6.62 - 6.49 (m, 1H), 6.48 - 6.39 (m, 1H), 3.79 (s, 3H), 3.64 - 3.52 (m, 2H), 3.50 - 3.34 (m, 2H), 3.26 - 3.13 (m, 3H), 3.12 - 2.99 (m, 1H), 2.95 - 2.82 (m, 2H), 2.81 - 2.67 (m, 2H), 2.58 - 2.46 (m, 1H), 2.43 - 2.21 (m, 2H), 1.97 - 1.85 (m, 2H), 1.18 - 0.81 (m, 10H), 0.70 - 0.56 (m, 1H), 0.54 - 0.40 (m, 1H), 0.39 - 0.30 (m, 1H), 0.25 - 0.15 (m, 1H).

**Example 203:**

Synthetic Route:

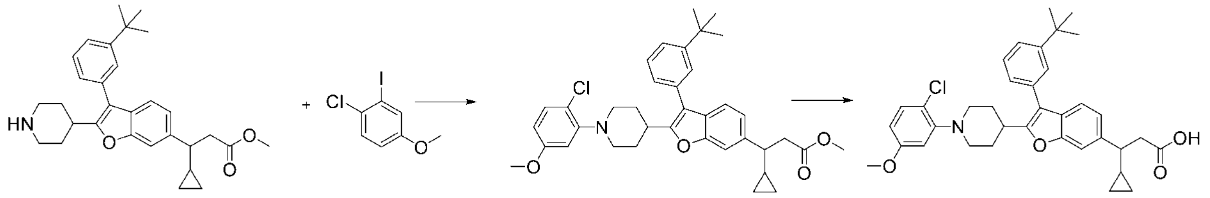

1-4 90-1 203-1 203

Referring to the synthetic route of compound **3,** compound **3-1** was replaced with compound **90-1** to carry out the synthesis. The resulting crude product was purified by reverse-phase column chromatography [acetonitrile/water (0.05% formic acid) = 0% to 100%] to obtain compound **203** (19 mg, yield: 50%) as a white solid. MS (ESI, m/z): 586.3 $[M+H]^+$.

$^1H$ NMR (400 MHz, $CDCl_3$) δ 7.56 - 7.48 (m, 2H), 7.46 - 7.40 (m, 3H), 7.36 - 7.29 (m, 2H), 7.15 (d, *J* = 8.0 Hz, 1H), 6.66 - 6.60 (m, 1H), 6.56 - 6.50 (m, 1H), 3.80 (s, 3H), 3.59 - 3.50 (m, 2H), 3.17 - 3.04 (m, 1H), 2.94 - 2.81 (m, 2H), 2.75 - 2.65 (m, 2H), 2.59 - 2.47 (m, 1H), 2.46 - 2.32 (m, 2H), 1.99 - 1.92 (m, 2H), 1.40 (s, 9H), 1.17 - 1.05 (m, 1H), 0.70 - 0.65 (m, 1H), 0.49 - 0.44 (m, 1H), 0.38 - 0.34 (m, 1H), 0.25 - 0.21 (m, 1H).

**Example 204:**

Synthetic Route:

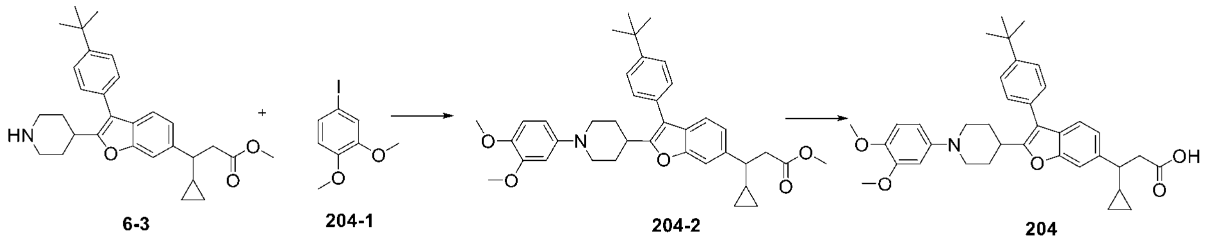

6-3 204-1 204-2 204

Referring to the synthetic route of compound **6,** compound **6-4** was replaced with compound **204-1** to carry out the synthesis. The resulting crude product was purified by reverse-phase column chromatography [acetonitrile/water (0.05% formic acid) = 0% to 100%] to obtain compound **204** (6 mg, yield: 31%) as a white solid. MS (ESI, m/z): 582.3 $[M+H]^+$.

$^1H$ NMR (400 MHz, MeOD) δ 7.55 (d, *J* = 8.0 Hz, 2H), 7.42 -7.37 (m, 4H), 7.14 (d, *J* = 8.0 Hz, 1H), 6.86 (d, *J* = 8.8 Hz, 1H), 6.72 (d, *J* = 2.4 Hz, 1H),6.60 - 6.56 (m, 1H), 3.82 (s, 3H), 3.78 (s, 3H), 3.64 - 3.60 (m, 2H), 3.11 - 3.05 (m, 1H), 2.79 - 2.69 (m, 4H), 2.48 - 2.46 (m, 1H), 2.26 - 2.21 (m, 2H),1.97 - 1.93 (m, 2H), 1.39 (s, 9H), 1.15 - 1.09 (m, 1H), 0.63 - 0.57 (m, 1H), 0.42 - 0.39 (m, 1H), 0.35 - 0.31 (m, 1H), 0.20 - 0.16 (m, 1H).

**Example 205:**

Synthetic Route:

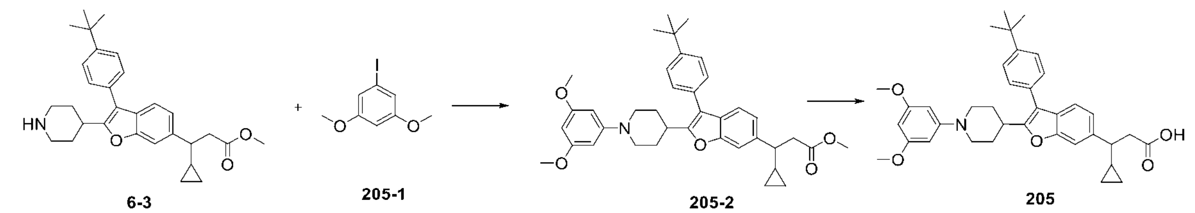

6-3 205-1 205-2 205

Referring to the synthetic route of compound **6,** compound **6-4** was replaced with compound **205-1** to carry out the synthesis. The resulting crude product was purified by reverse-phase column chromatography [acetonitrile/water (0.05% formic acid) = 0% to 100%] to obtain compound **205** (12 mg, yield: 24%) as a white solid. MS (ESI, m/z): 582.3 $[M+H]^+$.

$^1$H NMR (400 MHz, DMSO-$d_6$) δ 7.55 (d, *J* = 8.2 Hz, 2H), 7.46 - 7.41 (m, 3H), 7.37 (d, *J* = 8.0 Hz, 1H), 7.11 (d, *J* = 8.0 Hz, 1H), 6.11 - 6.08 (m, 2H), 5.98 - 5.93 (m, 1H), 3.82 - 3.79 (m, 2H), 3.71 (s, 6H), 3.14 - 3.04 (m, 1H), 2.83 - 2.76 (m, 2H), 2.73 - 2.35 (m, 3H), 2.03 - 2.01 (m, 2H), 1.86 - 1.83 (m, 2H), 1.35 (s, 9H), 1.03 - 0.93 (m, 1H), 0.48 - 0.38 (m, 1H), 0.29 - 0.20 (m, 2H), 0.11 - 0.03 (m, 1H).

**Example 206:**

Synthetic Route:

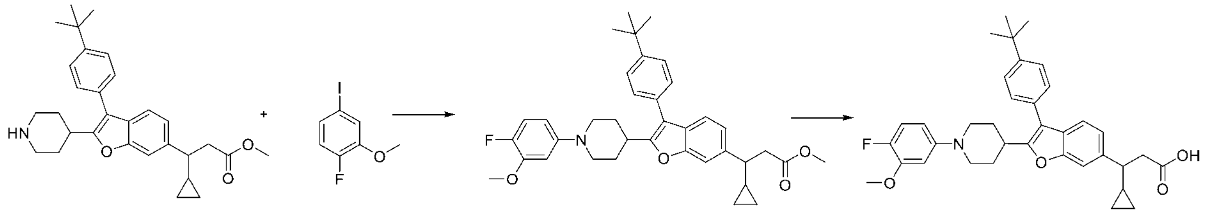

6-3 206-1 206-2 206

Referring to the synthetic route of compound **6,** compound **6-4** was replaced with compound **206-1** to carry out the synthesis. The resulting crude product was purified by reverse-phase column chromatography [acetonitrile/water (0.05% formic acid) = 0% to 100%] to obtain compound **206** (29 mg, yield: 49%) as a white solid. MS (ESI, m/z): 570.2 $[M+H]^+$.

$^1$H NMR (400 MHz, MeOD) δ 7.55 (d, *J* = 8.4 Hz, 2H), 7.43 -7.37 (m, 4H), 7.14 (d, *J = 8.0* Hz, 1H), 6.97 - 6.92 (m, 1H), 6.76 - 6.73 (m, 1H), 6.55 - 6.51 (m, 1H), 3.85 (s, 3H), 3.69 - 3.66 (m, 2H), 3.13 - 3.09 (m, 1H), 2.78 - 2.65 (m, 4H), 2.47 - 2.45 (m, 1H), 2.28 - 2.19 (m, 2H),1.97 - 1.94 (m, 2H), 1.39 (s, 9H), 1.15 - 1.10 (m, 1H), 0.63 - 0.59 (m, 1H), 0.43 - 0.38 (m, 1H), 0.36 - 0.32 (m, 1H), 0.19 - 0.15 (m, 1H).

**Example 207:**

Synthetic Route:

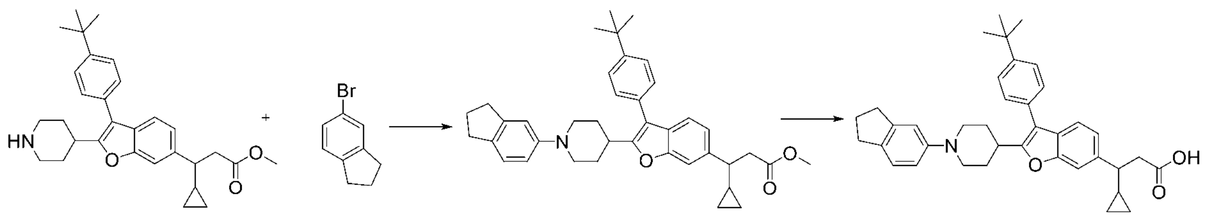

6-3 207-1 207-2 207

Referring to the synthetic route of compound **6,** compound **6-4** was replaced with compound **207-1** to carry out the synthesis. The resulting crude product was purified by reverse-phase column chromatography [acetonitrile/water (0.05% formic acid) = 0% to 100%] to obtain compound **207** (13 mg, yield: 44%) as a white solid. MS (ESI, m/z): 562.2 $[M+H]^+$.

$^1$H NMR (400 MHz, MeOD) δ 7.54 (d, *J* = 8.4 Hz, 2H), 7.43 - 7.37 (m, 4H), 7.15 (dd, *J* = 8.0, 1.2 Hz, 1H), 7.08 (d, *J* = 8.4 Hz, 1H), 6.93 (s, 1H), 6.85 - 6.78 (m, 1H), 3.68 - 3.65 (m, 2H), 3.12 - 3.03 (m, 1H), 2.86 - 2.67 (m, 8H), 2.53 - 2.43 (m, 1H), 2.31 - 2.49 (m, 2H), 2.07 - 2.02 (m, 2H), 1.94 - 1.92 (m, 2H), 1.39 (s, 9H), 1.15 - 1.03 (m, 1H), 0.64 - 0.52 (m, 1H), 0.45 - 0.33 (m, 2H), 0.19 - 0.13 (m, 1H).

**Example 208:**

Synthetic Route:

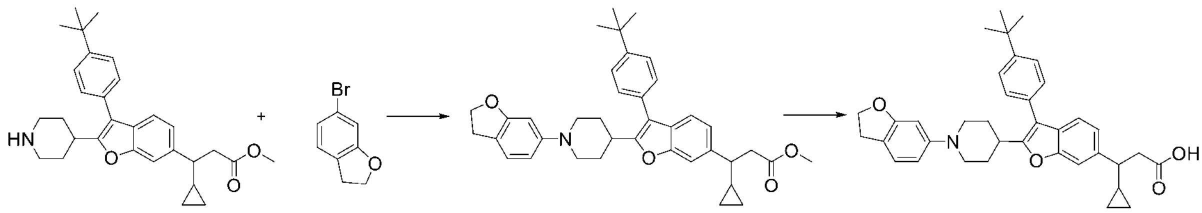

6-3 208-1 208-2 208

Referring to the synthetic route of compound **6,** compound **6-4** was replaced with compound **208-1** to carry out the synthesis. The resulting crude product was purified by reverse-phase column chromatography [acetonitrile/water (0.05% formic acid) = 0% to 100%] to obtain compound **208** (13 mg, yield: 42%) as a white solid. MS (ESI, m/z): 564.2 $[M+H]^+$.

$^1$H NMR (400 MHz, MeOD) δ 7.55 (d, *J* = 8.4 Hz, 2H), 7.43 -7.34 (m, 4H), 7.14 (d, *J* = 8.0 Hz, 1H), 7.05 (d, *J* = 8.0 Hz, 1H), 6.52 - 6.50 (m, 1H),6.47 - 6.43 (m, 1H), 4.50 (t, *J* = 8.0 Hz, 2H), 3.71 - 3.68 (m, 2H), 3.15 - 3.05 (m, 3H), 2.78 - 2.65 (m, 4H), 2.51 - 2.45 (m, 1H), 2.25 - 2.17 (m, 2H),1.94 - 1.91 (m, 2H), 1.39 (s, 9H), 1.14 - 1.08 (m, 1H), 0.69 - 0.57 (m, 1H), 0.41 - 0.32 (m, 2H), 0.18 - 0.15 (m, 1H).

**Example 209:**

Synthetic Route:

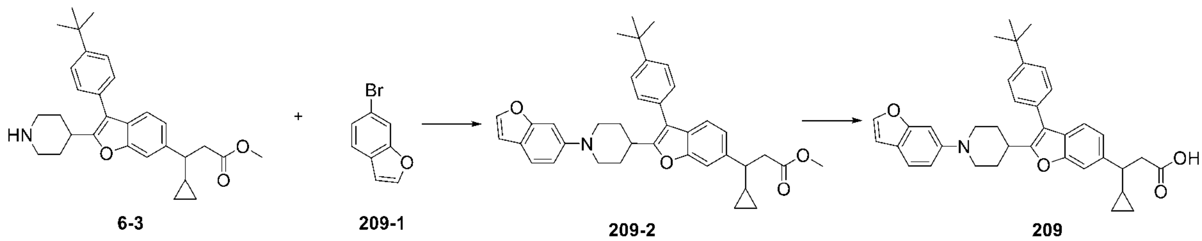

6-3 209-1 209-2 209

Referring to the synthetic route of compound **6,** compound **6-4** was replaced with compound **209-1** to carry out the synthesis. The resulting crude product was purified by reverse-phase column chromatography [acetonitrile/water (0.05% formic acid) = 0% to 100%] to obtain compound **209** (11 mg, yield: 37%) as a white solid. MS (ESI, m/z): 562.2 $[M+H]^+$.

$^1$H NMR (400 MHz, DMSO-$d_6$) δ 7.08 - 7.77 (m, 1H), 7.55 (d, *J* = 8.0 Hz, 2H), 7.49 - 7.36 (m, 5H), 7.13 - 7.11 (m, 2H), 7.03 - 6.99 (m, 1H), 6.82 - 6.78 (m, 1H), 3.84 - 3.81 (m, 2H), 3.50 - 3.07 (m, 1H), 2.87 - 2.81 (m, 2H), 2.49 - 2.36 (m, 3H), 2.11 - 2.08 (m, 2H), 1.91 - 1.88 (m, 2H), 1.35 (s, 9H), 1.02 - 0.94 (m, 1H), 0.47 - 0.41 (m, 1H), 0.28 - 0.22 (m, 2H), 0.10 - 0.08 (m, 1H).

**Example 210:**

Synthetic Route:

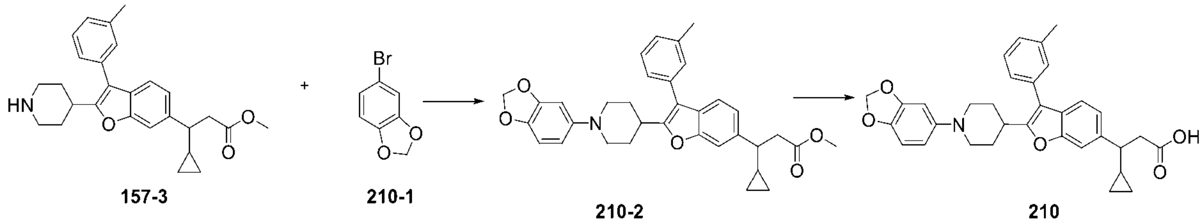

157-3 210-1 210-2 210

Referring to the synthetic route of compound **157,** compound **157-4** was replaced with compound **210-1** to carry out the synthesis. The resulting crude product was purified by reverse-phase column chromatography [acetonitrile/water (0.05% formic acid) = 0% to 100%] to obtain compound **210** (17 mg, yield: 59%) as a white solid. MS (ESI, m/z): 524.2 $[M+H]^+$.

$^1$H NMR (400 MHz, $CDCl_3$) δ 7.46 (d, *J* = 8.0 Hz, 1H), 7.42 - 7.34 (m, 2H), 7.30 - 7.16 (m, 3H), 7.11 (d, *J*= 8.0 Hz, 1H), 6.76 - 6.69 (m, 1H), 6.62 (s, 1H), 6.49 - 6.35 (m, 1H), 5.91 (s, 2H), 3.65 - 3.52 (m, 2H), 3.10 - 2.97 (m, 1H), 2.92 - 2.64 (m, 4H), 2.54 - 2.39 (m, 4H), 2.35 - 2.15 (m, 2H), 1.98 - 1.85 (m, 2H), 1.15 -1.04 (m, 1H), 0.67 - 0.55 (m, 1H), 0.50 - 0.27 (m, 2H), 0.24 - 0.15 (m, 1H).

**Example 211:**

Synthetic Route:

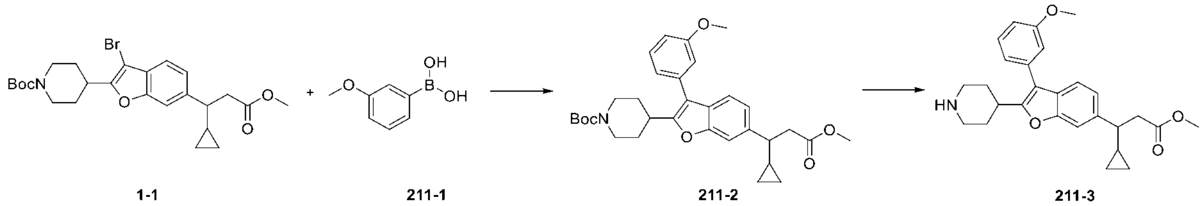

1-1 211-1 211-2 211-3

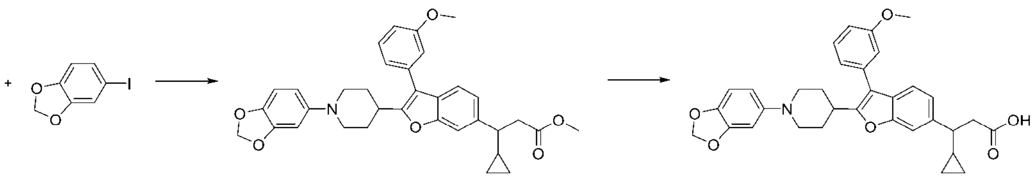

210-1 211-4 211

Referring to the synthetic route of compound **6,** compound **6-1** was replaced with compound **211-1,** and compound **6-4** was replaced with compound **210-1** to synthesize compound **211** (3.9 mg, yield: 13%) as a white solid. MS (ESI, m/z): 540.2 $[M+H]^+$.

$^1$H NMR (400 MHz, $CDCl_3$) δ 7.49 (d, *J* = 8.0 Hz, 1H), 7.44 - 7.38 (m, 2H), 7.13 (d, *J* = 8.0 Hz, 1H), 7.08 - 6.99 (m, 2H), 6.96 - 6.89 (m, 1H), 6.76 - 6.69 (m, 1H), 6.63 (s, 1H), 6.47 - 6.35 (m, 1H), 5.92 (s, 2H), 3.87 (s, 3H), 3.64 - 3.54 (m, 2H), 3.13 - 3.01 (m, 1H), 2.91 - 2.84 (m, 2H), 2.77 - 2.68 (m, 2H), 2.56 - 2.47 (m, 1H), 2.34 - 2.23 (m, 2H), 1.96 - 1.89 (m, 2H), 1.18 - 1.05 (m, 1H), 0.70 - 0.58 (m, 1H), 0.52 - 0.41 (m, 1H), 0.39 - 0.28 (m, 1H), 0.25 - 0.16 (m, 1H).

**Example 212:**

Synthetic Route:

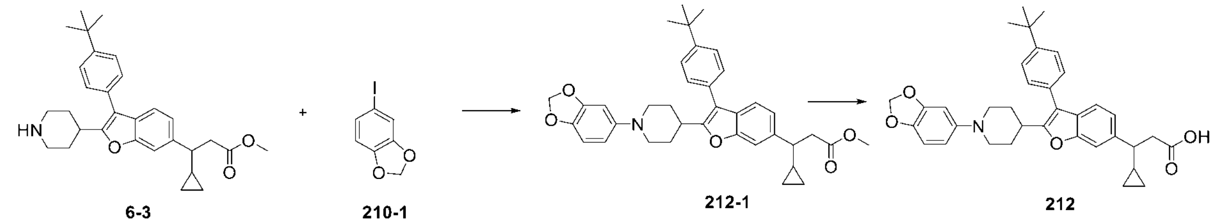

6-3 210-1 212-1 212

Referring to the synthetic route of compound **6,** compound **6-4** was replaced with compound **210-1** to carry out the synthesis. The resulting crude product was purified by reverse-phase column chromatography [acetonitrile/water (0.05% formic acid) = 0% to 100%] to obtain compound **212** (17 mg, yield: 59%) as a white solid. MS (ESI, m/z): 566.2 $[M+H]^+$.

$^1$H NMR (400 MHz, $CDCl_3$) δ 7.55 (d, *J* = 8.4 Hz, 2H), 7.44-7.35 (m, 4H), 7.14 (d, *J* = 8.0 Hz, 1H), 6.71 (d, *J* = 8.4 Hz, 1H), 6.67 (d, *J*= 2.4 Hz, 1H), 6.52 - 6.46 (m, 1H), 5.87(s, 2H), 3.60 - 3.52 (m, 2H), 3.10 - 3.00 (m, 1H), 2.81 - 2.69 (m, 4H), 2.51 - 2.42 (m, 1H), 2.28 - 2.19 (m, 2H), 1.98 - 1.90 (m, 2H), 1.39 (s, 9H), 1.15 - 1.10 (m, 1H), 0.63 - 0.58 (m, 1H), 0.43 - 0.31 (m, 2H), 0.20 - 0.15 (m, 1H).

**Example 213:**

Synthetic Route:

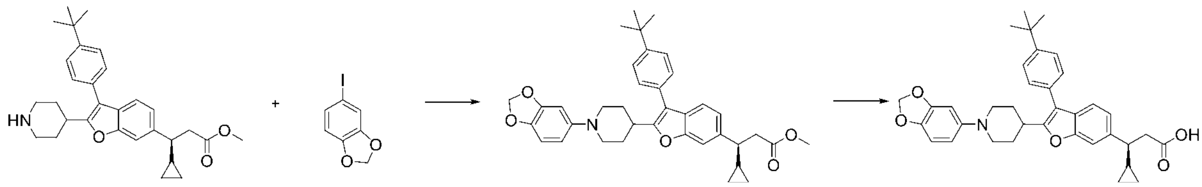

14-2 210-1 213-1 213

Referring to the synthetic route of compound **14,** compound **11-1** was replaced with compound **210-1** to carry out the synthesis. The resulting crude product was purified by reverse-phase column chromatography [acetonitrile/water (0.05% formic acid) = 0% to 100%] to obtain compound **213** (18 mg, yield: 60%) as a white solid. MS (ESI, m/z): 566.2 $[M+H]^+$.

$^1$H NMR (400 MHz, $CDCl_3$) δ 7.53 - 7.49 (m, 3H), 7.42 - 7.38 (m, 3H), 7.12 (d, *J* = 8.6 Hz, 1H), 6.75 (d, *J*= 8.0 Hz, 1H), 6.68 - 6.57 (m, 1H), 6.51 - 6.39 (m, 1H), 5.93 (s, 2H), 3.67 - 3.55 (m, 2H), 3.14 - 2.97 (m, 1H), 2.91 - 2.85 (m, 2H), 2.84 - 2.66 (m, 2H), 2.53 - 2.46 (m, 1H), 2.38 - 2.20 (m, 2H), 1.97 - 1.90 (m, 2H), 1.41 (s, 9H), 1.14 - 1.07 (m, 1H), 0.67 - 0.56 (m, 1H), 0.52 - 0.41 (m, 1H), 0.38 - 0.30 (m, 1H), 0.27 - 0.16 (m, 1H).

**Example 214:**

Synthetic Route:

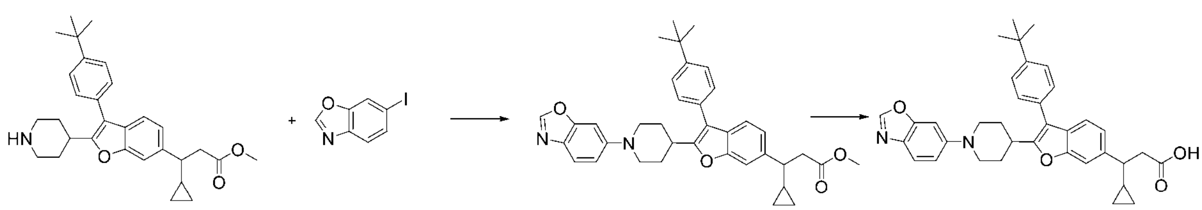

6-3 214-1 214-2 214

Referring to the synthetic route of compound **6,** compound **6-4** was replaced with compound **214-1** to carry out the synthesis. The resulting crude product was purified by reverse-phase column chromatography [acetonitrile/water (0.05% formic acid) = 0% to 100%] to obtain compound **214** (1 mg, yield: 8%) as a white solid. MS (ESI, m/z): 563.2 $[M+H]^+$.

$^1$H NMR (400 MHz, MeOD) δ 8.29 (s, 1H), 7.58 (d, *J* = 8.8 Hz, 1H), 7.54 (d, *J* = 8.0 Hz, 2H), 7.46 - 7.35 (m, 4H), 7.25

(d, $J$ = 2.0 Hz, 1H), 7.20 - 7.14 (m, 2H), 3.89 - 3.81 (m, 2H), 3.18 - 3.09 (m, 1H), 2.92 - 2.81 (m, 2H), 2.70 - 2.56 (m, 2H), 2.55 - 2.45 (m, 1H), 2.31 - 2.20 (m, 2H), 2.02 - 1.92 (m, 2H), 1.39 (s, 9H), 1.11 - 1.01 (m, 1H), 0.60 - 0.52 (m, 1H), 0.41 - 0.32 (m, 2H), 0.15 - 0.10 (m, 1H).

**Example 215:**

Synthetic Route:

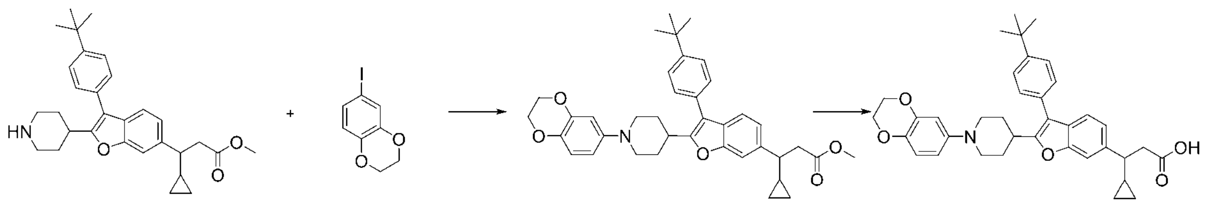

6-3 215-1 215-2 215

Referring to the synthetic route of compound **6,** compound **6-4** was replaced with compound **215-1** to carry out the synthesis. The resulting crude product was purified by reverse-phase column chromatography [acetonitrile/water (0.05% formic acid) = 0% to 100%] to obtain compound **215** (15 mg, yield: 37%) as a white solid. MS (ESI, m/z): 580.3 $[M+H]^+$.

$^1H$ NMR (400 MHz, $CD_3OD$) $\delta$ 7.54 (d, $J$ = 8.4 Hz, 2H), 7.43 - 7.35 (m, 4H), 7.16 (d, $J$ = 8.8 Hz, 1H), 6.72 (d, $J$ = 8.8 Hz, 1H), 6.61 - 6.49 (m, 2H), 4.24 - 4.11 (m, 4H), 3.62 - 3.52 (m, 2H), 3.11 - 3.01 (m, 1H), 2.71 - 2.60 (m, 4H), 2.56 - 2.45 (m, 1H), 2.29 - 2.16 (m, 2H), 1.97 - 1.87 (m, 2H), 1.39 (s, 9H), 1.14 - 0.99 (m, 1H), 0.63 - 0.51 (m, 1H), 0.42 - 0.32 (m, 2H), 0.20 - 0.11 (m, 1H).

**Example 216:**

Synthetic Route:

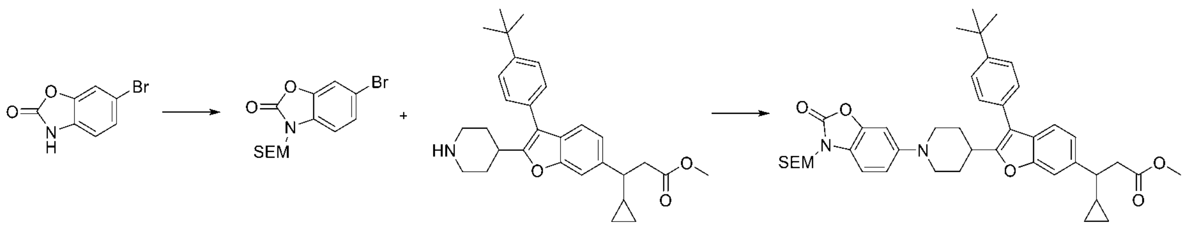

216-1 216-2 6-3 216-3

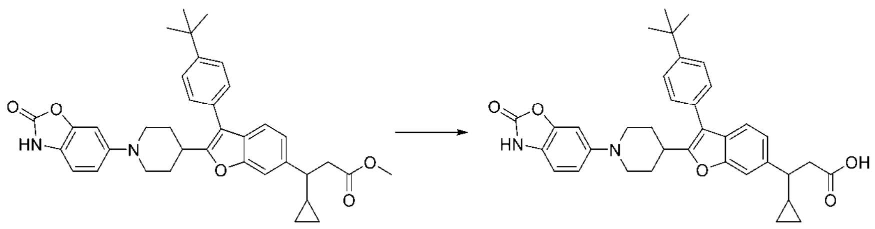

216-4 216

Under a nitrogen atmosphere, sodium hydride (67 mg, 1.68 mmol) was added to a solution of compound **216-1** (300 mg, 1.4 mmol) in *N,N*-dimethylformamide (5 mL) at 0°C, and the mixture was stirred for 0.5 hours. Then, 2-(trimethylsilyl)ethoxymethyl chloride (351 mg, 2.1 mmol) was added, and stirring was continued at room temperature for 1.5 hours. The resulting mixture was added to water (50 mL) and extracted with ethyl acetate (30 mL × 3). The combined organic phases were washed with saturated brine (50 mL), dried over anhydrous sodium sulfate, and concentrated. The resulting crude product was purified by normal-phase column chromatography (ethyl acetate: petroleum ether = 0% to 100%) to obtain compound **216-2** (300 mg, yield: 62%) as a pale red solid.

Referring to the synthetic route of compound **6,** compound **6-4** was replaced with compound **216-2** to synthesize compound **216-3** (50 mg, yield: 63%) as a yellow oil. MS (ESI, m/z): 723.4 [M+H]$^+$.

At room temperature, trifluoroacetic acid (1 mL) was added to a solution of compound **216**-3 (50 mg, 0.069 mmol) in dichloromethane (2 mL), and the reaction mixture was stirred for 2.5 hours. The solvent was then removed by concentration, followed by the addition of ammonia in methanol (7 M, 1 mL). The mixture was stirred at room temperature for 0.5 hours. After concentration, the resulting crude product was purified by normal-phase column chromatography (ethyl acetate: petroleum ether = 0% to 100%) to obtain compound **216-4** (30 mg, yield: 73%) as a yellow solid. MS (ESI, m/z): 593.3 [M+H]$^+$.

Referring to the synthetic route of compound **6,** compound **6-5** was replaced with compound **216-4** to carry out the synthesis. The resulting crude product was purified by reverse-phase column chromatography [acetonitrile/water (0.05% formic acid) = 0% to 100%] to obtain compound **216** (2 mg, yield: 8%) as a white solid. MS (ESI, m/z): 579.2 [M+H]$^+$.

$^1$HNMR (400 MHz, $CD_3OD$) $\delta$ 7.59 - 7.57 (m, 3H), 7.48 - 7.35 (m, 5H), 7.26 - 7.15 (m, 2H), 3.80 - 3.68 (m, 4H), 3.48 - 3.44 (m, 1H), 2.81 - 2.73(m, 2H), 2.66 - 2.42 (m, 3H), 2.27 - 2.24 (m, 2H), 1.39 (s, 9H), 1.14 - 1.10 (m,1H), 0.64 - 0.61(m, 1H), 0.46 - 0.30 (m, 2H), 0.22 - 0.14 (m, 1H). **Example 217:**

Synthetic Route:

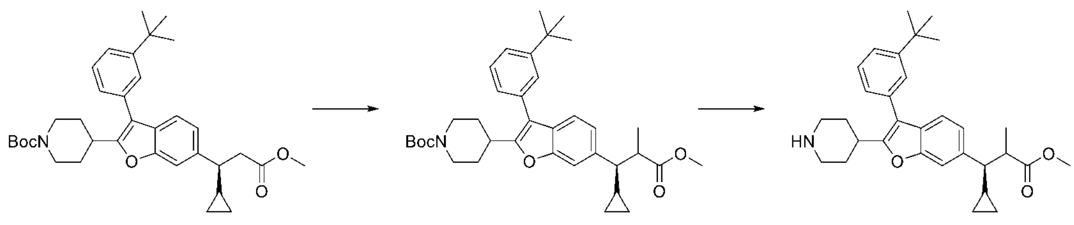

12-2 217-1 217-2

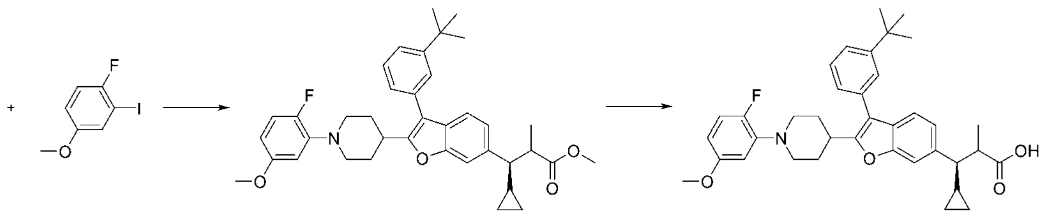

89-1 217-3 217

Referring to the synthetic route of compound **12,** compound **12-2** (160 mg, 0.29 mmol) was obtained and added to tetrahydrofuran (10 mL). Lithium bis(trimethylsilyl)amide (0.87 mL, 1 mol/L) was then slowly added to the reaction mixture at -78°C and stirred for 30 minutes, followed by warming to 0°C and stirring for 80 minutes. After cooling to -78°C, a solution of iodomethane (203 mg, 1.45 mmol) in tetrahydrofuran (5 mL) was slowly added to the reaction mixture and stirred at room temperature for 2 hours. After the reaction was completed, the reaction mixture was quenched with saturated ammonium chloride solution (10 mL). Ethyl acetate (100 mL) was added, and the organic phase was washed with saturated brine. The organic phase was concentrated to obtain a crude product. The resulting crude product was purified by reverse-phase column chromatography [acetonitrile/water (0.05% formic acid) = 0% to 100%] to obtain compound **217-1** (162 mg, yield: 97%) as a yellow oil. MS (ESI, m/z): 574.3 [M+H]$^+$.

Then, referring to the synthetic route of compound **89,** compound **38-3** was replaced with compound **217-2** to carry out the synthesis. The resulting crude product was purified by reverse-phase column chromatography [acetonitrile/-water (0.05% formic acid) = 0% to 100%] to obtain compound **217** (153 mg, yield: 93%) as a white solid. MS (ESI, m/z): 584.3 [M+H]$^+$.

$^1$H NMR (400 MHz, $CDCl_3$) $\delta$ 7.54 - 7.34 (m, 5H), 7.33 - 7.27 (m, 1H), 7.17 - 7.09 (m, 1H), 6.98 - 6.88 (m, 1H), 6.57 - 6.48 (m, 1H), 6.45 - 6.38 (m, 1H), 3.77 (s, 3H), 3.65 - 3.51 (m, 2H), 3.14 - 3.01 (m, 1H), 2.84 - 2.63 (m, 3H), 2.35 - 2.24 (m, 2H), 2.06 - 1.98 (m, 1H), 1.98 - 1.85 (m, 2H), 1.40 - 1.36 (m, 12H), 1.15 - 1.06 (m, 1H), 0.74 - 0.66 (m, 1H), 0.55 - 0.46 (m, 1H), 0.44 - 0.37 (m, 1H), 0.34 - 0.25 (m, 1H).

**Example 218:**

Synthetic Route:

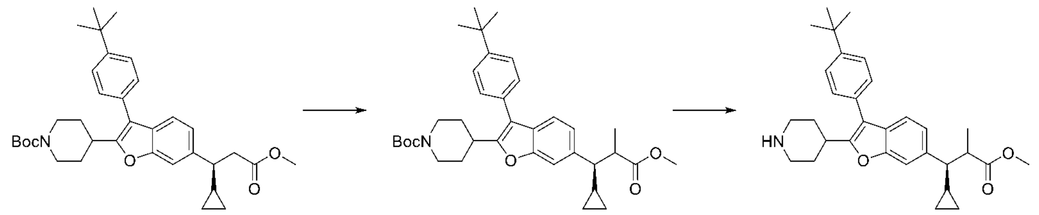

14-1 218-1 218-2

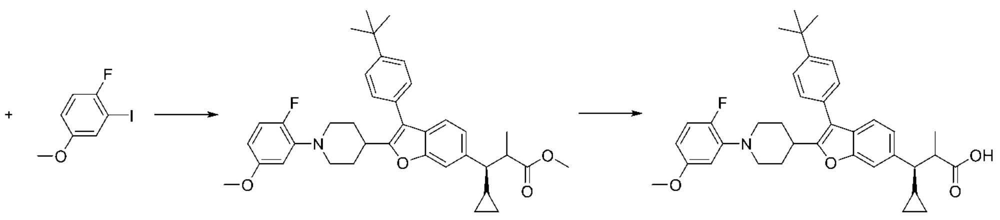

89-1 218-3 218

Referring to the synthetic route of compound **217,** compound **12-2** was replaced with compound **14-1** to carry out the synthesis. The resulting crude product was purified by reverse-phase column chromatography [acetonitrile/water (0.05% formic acid) = 0% to 100%] to obtain compound **218** (20 mg, yield: 18%) as a white solid. MS (ESI, m/z): 584.3 $[M+H]^+$.

$^1H$ NMR (400 MHz, DMSO-$d_6$) δ .56 (d, $J$ = 8.0 Hz, 2H), 7.45 - 7.39 (m, 4H), 7.12 (d, $J$ = 8.0 Hz, 1H), 7.07 - 7.02 (m, 1H), 6.57 - 6.48 (m, 2H), 3.71 (s, 3H), 3.56 - 3.37 (m, 2H), 3.09 - 3.03 (m, 1H), 2.86 - 2.74 (m, 3H), 2.19 - 2.06 (m, 3H), 1.97-1.87 (m, 2H), 1.35 (s, 9H), 1.29 - 1.19 (m, 3H), 1.15 - 1.07 (m, 1H), 0.70 - 0.61 (m, 1H), 0.41 - 0.23 (m, 2H), -0.06 - -0.16 (m, 1H).

**Example 219:**

Synthetic Route:

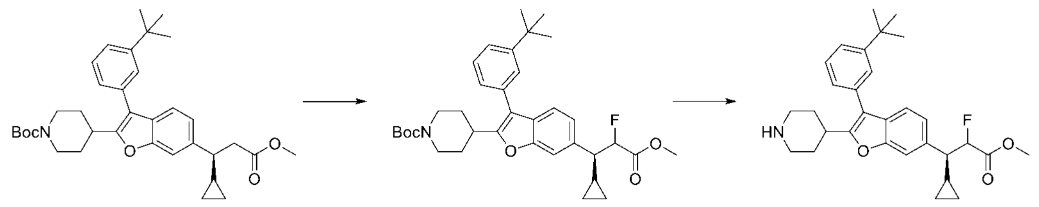

12-2 219-1 219-2

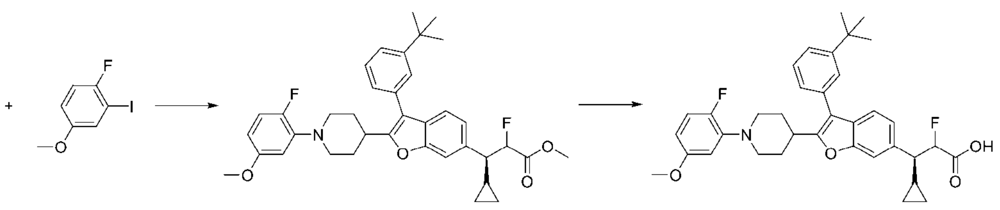

89-1 219-3 219

Referring to the synthetic route of compound **217,** iodomethane was replaced with *N*-fluorobenzenesulfonimide to carry out the synthesis. The resulting crude product was purified by reverse-phase column chromatography [acetonitrile/water (0.05% formic acid) = 0% to 100%] to obtain compound **219** (10 mg, yield: 48%) as a white solid. MS (ESI, m/z): 588.3 $[M+H]^+$.

$^1$H NMR (400 MHz, $CDCl_3$) δ 7.62 - 7.18 (m, 6H), 7.06 - 6.89 (m, 2H), 6.62 - 6.50 (m, 1H), 6.49 - 6.38 (m, 1H), 5.40 - 4.80 (m, 1H), 3.79 (s, 3H), 3.69 - 3.52 (m, 2H), 3.20 - 3.03 (m, 1H), 2.85 - 2.63 (m, 2H), 2.50 - 2.24 (m, 1H), 2.14 - 1.82 (m, 4H), 1.38 - 1.33 (m, 10H), 0.72- 0.20 (m, 3H), 0.14 - 0.00 (m, 1H).

**Example 220:**

Synthetic Route:

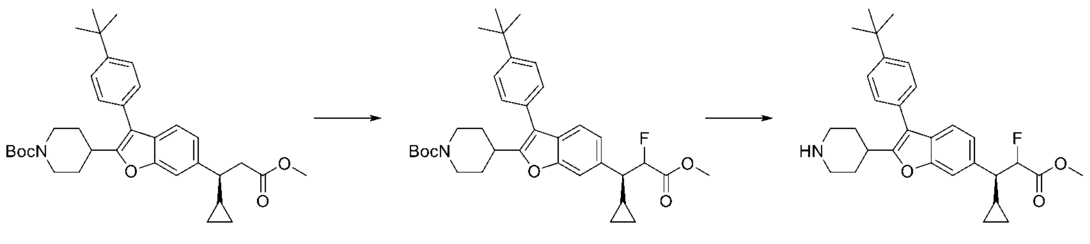

**14-1** **220-1** **220-2**

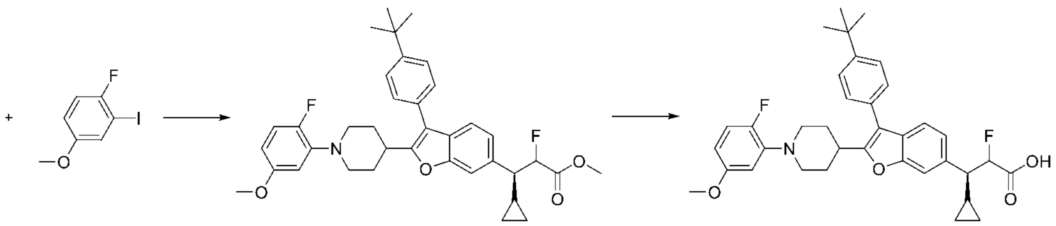

**89-1** **220-3** **220**

Referring to the synthetic route of compound **219,** compound **12-2** was replaced with compound **14-1** to carry out the synthesis. The resulting crude product was purified by reverse-phase column chromatography [acetonitrile/water (0.05% formic acid) = 0% to 100%] to obtain compound **220** (15 mg, yield: 43%) as a white solid. MS (ESI, m/z): 588.3 $[M+H]^+$.

$^1$H NMR (400 MHz, DMSO-$d_6$) δ 7.58 - 7.45 (m, 6H), 7.25 (d, *J* = 8.4 Hz, 1H), 7.08 - 7.03 (m, 1H), 6.59 - 6.54 (m, 1H), 6.52 - 6.47 (m, 1H), 5.18 (d, *J* = 48.0 Hz, 1H), 3.72 (s, 3H), 3.52 - 3.42 (m, 2H), 3.14 - 3.02 (m, 1H), 2.86 - 2.72 (m, 2H), 2.69 - 2.55 (m, 1H), 2.18 - 2.06 (m, 2H), 1.99 - 1.87 (m, 2H), 1.38 - 1.33 (m, 10H), 0.66 - 0.56 (m, 1H), 0.50 - 0.34 (m, 2H), 0.08 - 0.01 (m, 1H).

**Example 221:**

Synthetic Route:

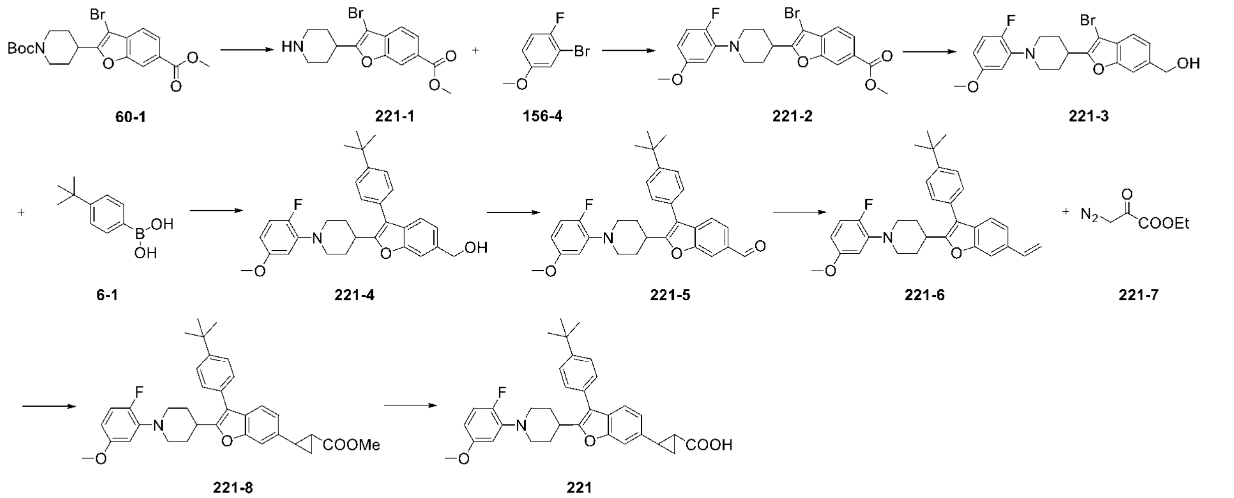

60-1 221-1 156-4 221-2 221-3

6-1 221-4 221-5 221-6 221-7

221-8 221

Referring to the synthetic route of compound **60,** compound **60-1** (12.5 g, 28.60 mmol) was synthesized and added to dichloromethane (100 mL) with stirring. Trifluoroacetic acid (12 mL) was then added, and the reaction was carried out at room temperature for 3 hours. After the reaction was completed, the reaction mixture was concentrated and diluted with ethyl acetate (200 mL). The organic phase was washed with water (80 mL) and then with saturated sodium bicarbonate solution (80 mL). The organic phase was dried over anhydrous sodium sulfate and concentrated to obtain compound **221-1** (9.6 g, crude product) as a yellow solid. MS (ESI, m/z): 338.3 $[M+H]^+$.

Compound **221-1** (9.6 g, 28.49 mmol), 3-bromo-4-fluoroanisole **156-4** (5.84 g, 28.49 mmol), 4,5-bis(diphenylphosphino)-9,9-dimethylxanthene (2.97 g, 5.13 mmol), and cesium carbonate (24.07 g, 78.07 mmol) were added to anhydrous 1,4-dioxane (500 mL) and stirred. Under a nitrogen atmosphere, tris(dibenzylideneacetone)dipalladium (2.35 g, 2.56 mmol) was added, and the reaction was carried out at 100°C for 16 hours. After the reaction was completed, the reaction mixture was diluted with dichloromethane: methanol (5:1) (600 mL) and filtered. The filtrate was concentrated to obtain a crude product. The resulting crude product was then purified by column chromatography to obtain compound **221-2** (7.2 g, crude product) as a yellow oil. MS (ESI, m/z): 462.3 $[M+H]^+$.

Compound **221-2** (7.2 g, 15.61 mmol) was added to anhydrous tetrahydrofuran (300 mL), followed by the portion-wise addition of lithium borohydride (7.5 g, 344.35 mmol). The reaction mixture was stirred at room temperature for 8 hours. After the reaction was completed, the system was slowly poured into an ice-cold sodium bicarbonate solution (400 mL) with stirring. The mixture was extracted with ethyl acetate (200 mL), and the organic phase was dried over anhydrous sodium sulfate, filtered, and concentrated to obtain a crude product. The resulting crude product was then purified by normal-phase column chromatography (ethyl acetate: petroleum ether = 0% to 100%) to obtain compound **221-3** (6.6 g, yield: 98%) as a yellow oil. MS (ESI, m/z): 434.3 $[M+H]^+$.

Compound **221-3** (800 mg, 1.85 mmol), 4-*tert*-butylphenylboronic acid **12-2** (493 mg, 2.77 mmol), bis(triphenylphosphine)palladium(II) chloride (130 mg, 0.18 mmol), and potassium carbonate (765 mg, 5.54 mmol) were added to a mixture of *N,N*-dimethylformamide (6 mL) and water (2 mL) and stirred. Under a nitrogen atmosphere, the reaction mixture was stirred at 100°C for 16 hours. After the reaction was completed, the reaction mixture was diluted with ethyl acetate (50 mL) and filtered. The organic phase was washed with water (30 mL), dried over anhydrous sodium sulfate, and concentrated to obtain a crude product. The resulting crude product was then purified by normal-phase column chromatography (ethyl acetate: petroleum ether = 0% to 100%) to obtain compound **221-4** (830 mg, yield: 92%) as a yellow oil. MS (ESI, m/z): 488.3 $[M+H]^+$.

Compound **221-4** (820 mg, 1.68 mmol) was added to dichloromethane (20 mL) and stirred. At 0°C, Dess-Martin periodinane (857 mg, 2.02 mmol) was added, and the reaction mixture was stirred at room temperature for 1 hour. After the reaction was completed, the reaction mixture was quenched with saturated sodium bicarbonate solution (10 mL) and concentrated to obtain a crude product. The resulting crude product was then purified by normal-phase column chromatography (ethyl acetate: petroleum ether = 0% to 100%) to obtain compound **221-5** (760 mg, yield: 93%) as a yellow solid. MS (ESI, m/z): 486.3 $[M+H]^+$.

To a reaction tube, under a nitrogen atmosphere, triphenylphosphine (164 mg, 0.46 mmol) and THF (2 mL) were added. After cooling to 0°C, a 1 mmol/L solution of potassium *tert*-butoxide (460 μL, 0.46 mmol) was slowly added dropwise. The reaction mixture was stirred for 1 hour, and then compound **221-5** (97 g, 0.2 mmol) was added. The reaction was heated to 60°C and stirred for 24 hours. After filtration and rotary evaporation to dryness, compound **221-6** (82 mg, crude product) was obtained as a white solid. MS (ESI, m/z): 484.3 $[M+H]^+$.

To a reaction tube, under a nitrogen atmosphere, compound **221-6** (70 mg, 0.15 mmol), ethyl diazoacetate **221-7** (45 mg, 0.24 mmol), $Pd(OAc)_2$ (7 mg, 0.03 mmol), and toluene (1 mL) were added. The reaction was carried out at 80°C for

4 hours. After cooling to room temperature, water (20 mL) was added, and the mixture was extracted with ethyl acetate (20 mL × 3). The combined organic phases were dried over anhydrous sodium sulfate and concentrated. The resulting crude product was purified by normal-phase column chromatography (ethyl acetate: petroleum ether = 0% to 100%) to obtain compound **221-8** (36 mg, yield: 42%) as a white solid. MS (ESI, m/z): 570.3 $[M+H]^+$.

To a reaction tube, compound **221-8** (36 mg, 63 μmol) and methanol (2 mL) were added. A solution of NaOH (51 mg, 1.26 mmol) in water (0.5 mL) was then added dropwise to the reaction, and the mixture was stirred at room temperature overnight. The pH was adjusted to 3 with 1 N dilute hydrochloric acid, and the mixture was extracted with ethyl acetate (10 mL). The organic phase was dried over anhydrous sodium sulfate, filtered, and concentrated. The resulting crude product was purified by normal-phase column chromatography (ethyl acetate: petroleum ether = 0% to 100%) to obtain compound **221** (24 mg, yield: 70%) as a white solid. MS (ESI, m/z): 542.3 $[M+H]^+$.

$^1$H NMR (400 MHz, $CDCl_3$) δ 7.50 (d, *J* = 7.6 Hz, 2H), 7.45 -7.35 (m, 4H), 7.13 (d, *J* = 8.0 Hz, 1H), 7.00 - 6.89 (m, 1H), 6.57 - 6.49 (m, 1H), 6.45 - 6.37 (m, 1H), 3.77 (s, 3H), 3.61 - 3.52 (m, 2H), 3.12 - 3.02 (m, 1H), 2.79 - 2.71 (m, 3H), 2.39 - 2.26 (m, 2H), 2.15 - 2.06 (m, 1H), 1.96 - 1.87 (m, 2H), 1.78 - 1.69 (m, 1H), 1.47 - 1.36 (m, 10H).

**Example 222:**

Synthetic Route:

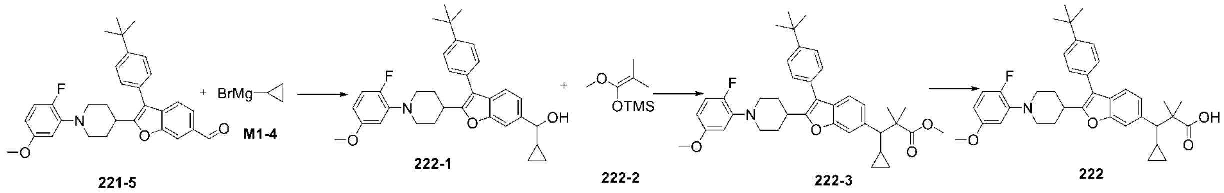

221-5 M1-4 222-1 222-2 222-3 222

Referring to the synthetic route of compound **221,** compound **221-5** (165 mg, 0.34 mmol) was synthesized and added to anhydrous tetrahydrofuran (20 mL) with stirring. At 0°C, cyclopropylmagnesium bromide **M1-4** (1.0 M in THF, 0.51 mL, 0.51 mmol) was slowly added, and the reaction mixture was stirred at 0°C for 1 hour. After the reaction was completed, the reaction mixture was quenched with saturated sodium bicarbonate solution (20 mL) and concentrated to obtain a crude product. The resulting crude product was purified by normal-phase column chromatography (ethyl acetate: petroleum ether = 0% to 100%) to obtain compound **222-1** (168 mg, yield: 94%) as a yellow oil. MS (ESI, m/z): 528.3 $[M+H]^+$.

Compound **222-1** (160 mg, 0.30 mmol) was added to anhydrous acetonitrile (20 mL) under a nitrogen atmosphere. Trichloroacetonitrile (87.2 mg, 0.60 mmol) was added, and then 1,8-diazabicyclo[5.4.0]undec-7-ene (4 mg, 0.03 mmol) was added. The reaction mixture was stirred at room temperature for 40 minutes. Then, 1-methoxy-1-(trimethylsiloxy)-2-methyl-1-propene **222-2** (156.4 mg, 0.90 mmol) was added, and then bis(trifluoromethanesulfonyl)imide (18 mg, 0.06 mmol) was added. The reaction was carried out at room temperature for 2 hours. After the reaction was completed, the reaction mixture was quenched with saturated ammonium chloride solution and concentrated to obtain a crude product. The resulting crude product was then purified by normal-phase column chromatography (ethyl acetate: petroleum ether = 0% to 100%) to obtain compound **222-3** (80 mg, yield: 44%) as a colorless oil. MS (ESI, m/z): 612.3 $[M+H]^+$.

Compound **222-3** (80 mg, 0.13 mmol) was added to methanol (2 mL) and tetrahydrofuran (2 mL) and stirred. A 4 M sodium hydroxide solution (2 mL) was added, and the reaction mixture was stirred at 85°C for 3 days. After the reaction was completed, the pH was adjusted to 3-4 with 1 N hydrochloric acid, and the mixture was extracted with ethyl acetate (10 mL). The organic phase was concentrated to obtain a crude product. The resulting crude product was purified by reverse-phase column chromatography [acetonitrile/water (0.05% formic acid) = 0% to 100%] to obtain compound **222** (38 mg, yield: 49%) as a white solid. MS (ESI, m/z): 598.3 $[M+H]^+$.

$^1$H NMR (400 MHz, DMSO-$d_6$) δ 7.59 - 7.53 (m, 2H), 7.48 - 7.36 (m, 4H), 7.12 (d, *J* = 8.0 Hz, 1H), 7.07 - 7.02 (m, 1H), 6.64 - 6.53 (m, 1H), 6.50 - 6.47 (m, 1H), 3.72 (s, 3H), 3.51 - 3.43 (m, 2H), 3.13 - 3.02 (m, 1H), 2.83 - 2.73 (m, 2H), 2.28 (d, *J* = 10.8 Hz, 1H), 2.16 - 2.08 (m, 2H), 1.97 - 1.89 (m, 2H), 1.47 - 1.39 (m, 1H), 1.36 (s, 9H), 1.18 (s, 3H), 1.04 (s, 3H), 0.72 - 0.60 (m, 1H), 0.47 - 0.40 (m, 1H), 0.39 - 0.25 (m, 1H), -0.21 - -0.32 (m, 1H).

**Example 223:**

Synthetic Route:

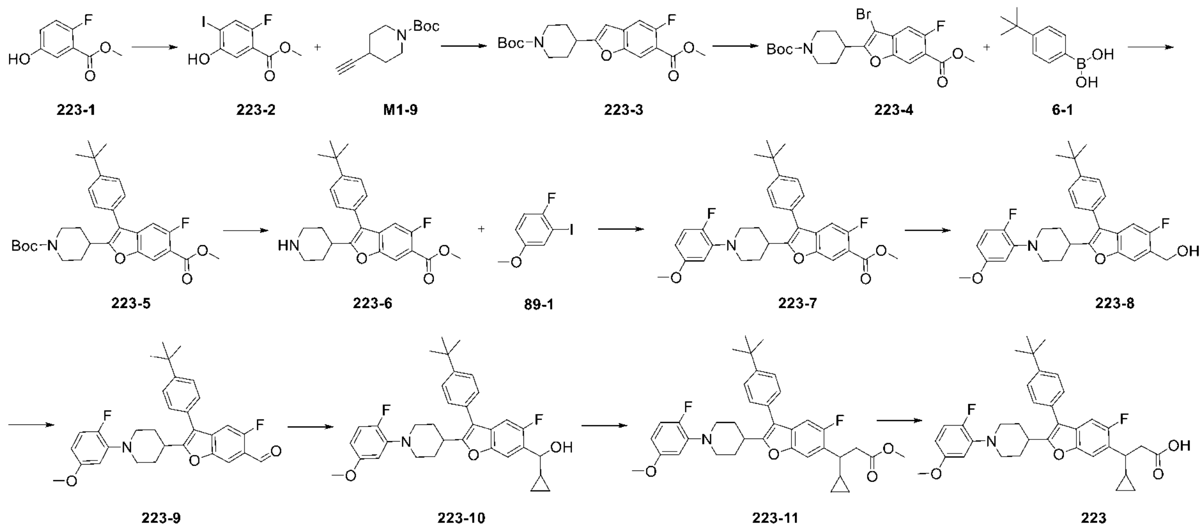

223-1 223-2 M1-9 223-3 223-4 6-1

223-5 223-6 89-1 223-7 223-8

223-9 223-10 223-11 223

Compound **223-1** (2 g, 11.8 mmol) and N-iodosuccinimide (2.9 g, 12.9 mmol) were added to acetic acid (20 mL) and stirred at room temperature for 2 hours. After the reaction was completed, most of the acetic acid was removed by concentration, and ethyl acetate (100 mL) was added. The organic phase was washed with saturated sodium bicarbonate solution (100 mL) and saturated brine (50 mL). The organic phase was concentrated to obtain a crude product, which was purified by normal-phase column chromatography (ethyl acetate: petroleum ether = 0% to 100%) to obtain compound **223-2** (1.95 g, yield: 56%) as a yellow solid. MS (ESI, m/z): 297.3 $[M+H]^+$.

Under a nitrogen atmosphere, compound **223-2** (1.95 g, 6.59 mmol), compound **M1-9** (1.38 g, 6.59 mmol), copper(I) iodide (125 mg, 0.66 mmol), bis(triphenylphosphine)palladium(II) chloride (231 mg, 0.33 mmol), and triethylamine (2 g, 19.8 mmol) were added to acetonitrile (240 mL). The reaction mixture was heated to 100°C and stirred overnight. After the reaction was completed, the reaction mixture was directly concentrated and purified by normal-phase column chromatography (ethyl acetate: petroleum ether = 0% to 100%) to obtain compound **223-3** (1.7 g, yield: 69%) as a yellow oil. MS (ESI, m/z): 378.3 $[M+H]^+$.

Compound **223-3** (1.35 g, 3.58 mmol) was added to tetrahydrofuran (20 mL), followed by the slow addition of liquid bromine (3.6 mL, 1 M in acetic acid) under an ice-water bath. The reaction mixture was then stirred at room temperature for 1 hour. After the reaction was completed, ethyl acetate (100 mL) was added. The organic phase was washed with saturated sodium bicarbonate solution (100 mL) and saturated brine (50 mL), then concentrated to obtain a crude product. The resulting crude product was purified by normal-phase column chromatography (ethyl acetate: petroleum ether = 0% to 100%) to obtain compound **223-4** (729 mg, yield: 45%) as a yellow oil. MS (ESI, m/z): 456.3 $[M+H]^+$.

Under a nitrogen atmosphere, compound **223-4** (729 mg, 1.6 mmol), compound **6-1** (341 mg, 1.92 mmol), tetrakis(triphenylphosphine)palladium (185 mg, 0.16 mmol), and sodium carbonate (508 mg, 4.8 mmol) were added to a mixture of 1,4-dioxane (8 mL) and water (1 mL). The reaction mixture was heated to 100°C and stirred overnight. After the reaction was completed, ethyl acetate (100 mL) was added, and the organic phase was washed with saturated brine (50 mL). The organic phase was concentrated to obtain a crude product, which was purified by normal-phase column chromatography (ethyl acetate: petroleum ether = 0% to 100%) to obtain compound **223-5** (784 mg, yield: 96%) as a yellow oil. MS (ESI, m/z): 510.3 $[M+H]^+$.

Compound **223-5** (784 mg, 1.54 mmol) was added to dichloromethane (15 mL), followed by the addition of a 4 N hydrochloric acid/1,4-dioxane solution (5 mL) to the reaction mixture, which was then stirred at room temperature for 2 hours. After the reaction was completed, the organic phase was directly concentrated to obtain compound **223-6** (630 mg, crude product) as a yellow oil. MS (ESI, m/z): 410.3 $[M+H]^+$.

Under a nitrogen atmosphere, compound **223-6** (630 mg, 1.54 mmol), 4-fluoro-3-iodoanisole **89-1** (504 mg, 2.0 mmol), tris(dibenzylideneacetone)dipalladium (141 mg, 0.15 mmol), 2-dicyclohexylphosphino-2',6'-diisopropoxybiphenyl (144 mg, 0.31 mmol), and cesium carbonate (2 g, 6.16 mmol) were added to a solution of 1,4-dioxane (15 mL). The reaction mixture was heated to 100°C and stirred overnight. After the reaction was completed, ethyl acetate (100 mL) was added, and the organic phase was washed with saturated brine (50 mL). The organic phase was concentrated to obtain a crude product, which was purified by normal-phase column chromatography (ethyl acetate: petroleum ether = 0% to

100%) to obtain compound **223-7** (370 mg, yield: 45%) as a yellow oil. MS (ESI, m/z): 534.3 $[M+H]^+$.

Under a nitrogen atmosphere, compound **223-7** (370 mg, 0.69 mmol) was added to a mixture of tetrahydrofuran (20 mL) and methanol (10 mL). Then, lithium aluminum hydride (40 mg, 1.04 mmol) was slowly added to the reaction mixture under an ice-water bath and stirred for 2 hours. After the reaction was completed, water (0.1 mL), 15% sodium hydroxide aqueous solution (0.1 mL), and water (0.3 mL) were added separately and stirred for 0.5 hours. After filtration, the reaction mixture was directly concentrated to obtain compound **223-8** (400 mg, crude product) as a yellow oil. MS (ESI, m/z): 506.3 $[M+H]^+$.

Compound **223-8** (400 mg, 0.79 mmol) was added to a mixture of tetrahydrofuran (10 mL) and dichloromethane (10 mL). Then, Dess-Martin periodinane (671 mg, 1.58 mmol) was slowly added to the reaction mixture under an ice-water bath, and the reaction mixture was stirred at room temperature for 5 hours. After the reaction was completed, ethyl acetate (100 mL) was added. The organic phase was washed with saturated sodium bicarbonate solution (100 mL) and saturated brine (500 mL), then concentrated to obtain a crude product. The resulting crude product was purified by normal-phase column chromatography (ethyl acetate: petroleum ether = 0% to 100%) to obtain compound **223-9** (245 mg, yield: 62%) as a yellow oil. MS (ESI, m/z): 504.3 $[M+H]^+$.

Under a nitrogen atmosphere, compound **223-9** (245 mg, 0.49 mmol) was added to a tetrahydrofuran (10 mL) solution. Then, cyclopropylmagnesium bromide **M1-4** (1.9 mL, 0.97 mmol, 0.5 mol/L) was slowly added dropwise to the reaction mixture under an ice-water bath and stirred for half an hour. After the reaction was completed, the reaction mixture was quenched with saturated ammonium chloride solution (10 mL). Ethyl acetate (100 mL) was added, and the organic phase was washed with saturated brine (50 mL). The organic phase was concentrated to obtain a crude product, which was purified by normal-phase column chromatography (ethyl acetate: petroleum ether = 0% to 100%) to obtain compound **223-10** (185 mg, yield: 62%) as a yellow oil. MS (ESI, m/z): 546.3 $[M+H]^+$.

Under a nitrogen atmosphere, compound **223-10** (185 mg, 0.34 mmol), 1-(*tert*-butyldimethylsilyloxy)-1-methoxyethene (128 mg, 0.68 mmol), and rhenium carbonyl bromide (14 mg, 0.034 mmol) were added to a toluene (10 mL) solution and heated to 120°C with stirring overnight. After the reaction was completed, ethyl acetate (100 mL) was added, and the organic phase was washed with saturated brine (50 mL). The organic phase was concentrated to obtain a crude product, which was purified by normal-phase column chromatography (ethyl acetate: petroleum ether = 0% to 100%) to obtain compound **223-11** (136 mg, yield: 67%) as a yellow oil. MS (ESI, m/z): 602.3 $[M+H]^+$.

Compound **223-11** (136 mg, 0.23 mmol) was added to a mixture of tetrahydrofuran (6 mL) and methanol (4 mL). Then, a solution of lithium hydroxide (109 mg, 4.5 mmol) in water (1 mL) was added to the reaction mixture, which was heated to 60°C and stirred for 2 hours. After the reaction was completed, 1 N dilute hydrochloric acid (10 mL) and ethyl acetate (100 mL) were added. The organic phase was washed with saturated brine (50 mL), concentrated to obtain a crude product, and purified by reverse-phase column chromatography [acetonitrile/water (0.05% formic acid) = 0% to 100%] to obtain compound 223 (2 mg, yield: 2%) as a white solid. MS (ESI, m/z): 588.3 $[M+H]^+$.

$^1H$ NMR (400 MHz, $CDCl_3$) δ 7.53 (d, *J* = 8.0 Hz, 2H), 7.41 - 7.35 (m, 3H), 7.21 (d, *J* = 10.4 Hz, 1H), 6.99 - 6.94 (m, 1H), 6.58 - 6.51 (m, 1H), 6.48 -6.40 (m, 1H), 3.79 (s, 3H), 3.64-3.53 (m, 2H), 3.13 -3.05 (m, 1H), 2.92 - 2.88 (m, 2H), 2.82 - 2.71 (m, 3H), 2.40 - 2.28 (m, 2H), 1.99-1.90 (m, 2H), 1.41 (s, 9H), 1.27 - 1.17 (m, 1H), 0.69 -0 .61 (m, 1H), 0.52 - 0.42 (m, 1H), 0.40 - 0.34 (m, 1H), 0.26 - 0.19 (m, 1H).

**Example 224:**

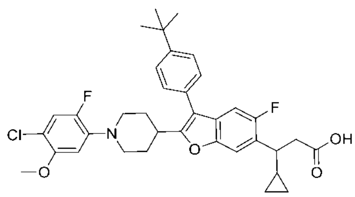

Synthetic Route:

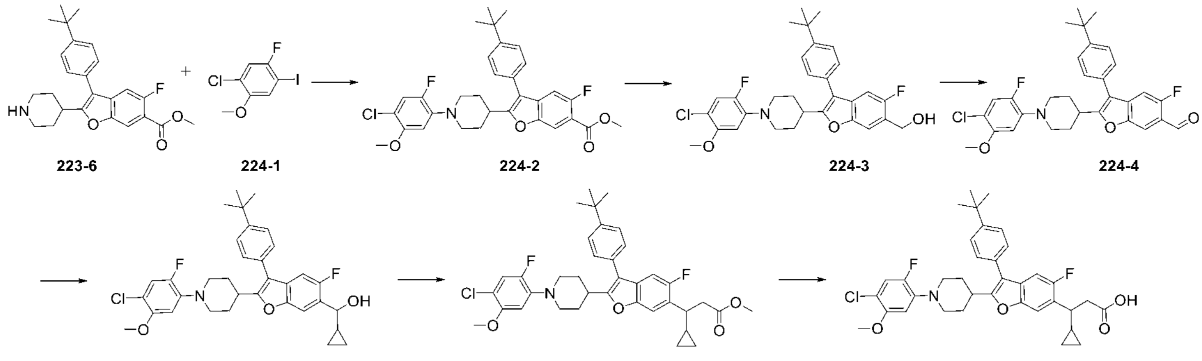

223-6 224-1 224-2 224-3 224-4

224-5 224-6 224

Referring to the synthetic route of compound **223,** compound **89-1** was replaced with compound **224-1** to carry out the synthesis. The resulting crude product was purified by reverse-phase column chromatography [acetonitrile/water (0.05% formic acid) = 0% to 100%] to obtain compound **224** (4 mg, yield: 7%) as a white solid. MS (ESI, m/z): 622.3 $[M+H]^+$.

$^1$H NMR (400 MHz, $CDCl_3$) δ 7.53 (d, *J* = 7.2 Hz, 2H), 7.41 - 7.35 (m, 3H), 7.21 (d, *J* = 10.4 Hz, 1H), 7.10 (d, *J* = 11.2 Hz, 1H), 6.59 (s, 1H), 3.88 (s, 3H), 3.63 - 3.51 (m, 2H), 3.16 - 3.05 (m, 1H), 2.95 - 2.69 (m, 5H), 2.43 - 2.28 (m, 2H), 2.00 - 1.89 (m, 2H), 1.41 (s, 9H), 1.17 - 1.08 (m, 1H), 0.68 - 0.63 (m, 1H), 0.50 - 0.42 (m, 1H), 0.41 - 0.33 (m, 1H), 0.26 - 0.19 (m, 1H).

**Example 225:**

Synthetic Route:

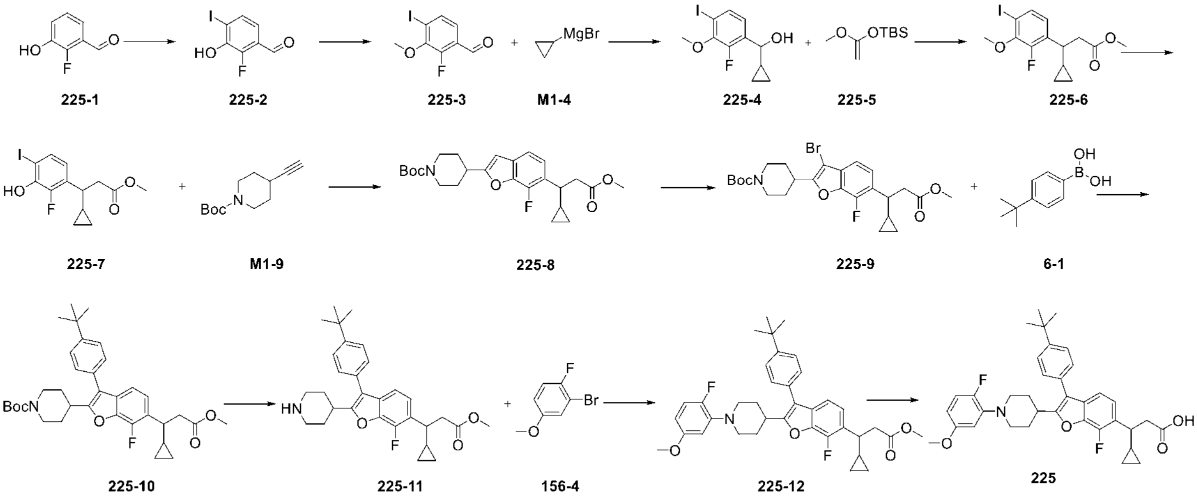

225-1 225-2 225-3 M1-4 225-4 225-5 225-6

225-7 M1-9 225-8 225-9 6-1

225-10 225-11 156-4 225-12 225

Compound **225-1** (1.2 g, 8.57 mmol) and N-iodosuccinimide (1.93 g, 8.57 mmol) were added to dichloromethane (20 mL) and stirred at room temperature for 6 hours. After the reaction was completed, the reaction mixture was directly concentrated to obtain a crude product, which was purified by normal-phase column chromatography (ethyl acetate: petroleum ether = 0% to 100%) to obtain compound **225-2** (330 mg, yield: 15%) as a yellow solid. MS (ESI, m/z): 267.3 $[M+H]^+$.

Compound **225-2** (322 mg, 1.21 mmol), iodomethane (206 mg, 1.45 mmol), and potassium carbonate (334 mg, 2.42 mmol) were added to *N,N*-dimethylformamide (5 mL) and stirred at room temperature for 3 hours. After the reaction was completed, saturated brine (20 mL) was added, and the reaction mixture was extracted with ethyl acetate (20 mL × 3). The combined organic phases were concentrated to obtain a crude product, which was purified by normal-phase column chromatography (ethyl acetate: petroleum ether = 0% to 100%) to obtain compound **225-3** (338 mg, yield: 99%) as a white solid. MS (ESI, m/z): 281.3 $[M+H]^+$.

At -20°C, **M1-4** (12 mL, 0.5 mmol/mL in THF) was slowly added dropwise to a mixture of compound **225-3** (339 mg, 1.21 mmol) and tetrahydrofuran (10 mL). After the addition was completed, the reaction mixture was stirred at -20°C for 2 hours. After the reaction was completed, the reaction mixture was quenched with saturated ammonium chloride aqueous solution (20 mL) and extracted with ethyl acetate (20 mL × 3). The combined organic phases were concentrated

to obtain compound **225-4** (330 mg, crude product) as a colorless oil. MS (ESI, m/z): 323.3 [M+H]$^+$.

Compound **225-4** (330 mg, 1.03 mmol), compound **225-5** (771 mg, 4.1 mmol), and rhenium pentacarbonyl bromide (83 mg, 0.205 mmol) were added to toluene (5 mL) and stirred at room temperature for 16 hours. After the reaction was completed, the reaction mixture was directly concentrated to obtain a crude product, which was purified by column chromatography to obtain compound **225-6** (310 mg, yield: 80%) as a yellow oil. MS (ESI, m/z): 379.3 [M+H]$^+$.

Compound **225-6** (305 mg, 0.807 mmol) and boron tribromide (1 mL) were added to dichloromethane (5 mL) and stirred at room temperature for 16 hours. After the reaction was completed, the reaction mixture was directly concentrated to obtain a crude product, which was purified by normal-phase column chromatography (ethyl acetate: petroleum ether = 0% to 100%) to obtain compound **225-7** (140 mg, yield: 48%) as a yellow oil. MS (ESI, m/z): 365.3 [M+H]$^+$.

Referring to the synthetic route of intermediate **M1,** compound **M1-8** was replaced with compound **225-7** to synthesize intermediate **225-8.** Referring to the synthetic route of compound **6,** intermediate **M1** was replaced with intermediate **225-8** to carry out the synthesis. The resulting crude product was purified by reverse-phase column chromatography [acetonitrile/water (0.05% formic acid) = 0% to 100%] to obtain compound **225** (2 mg, yield: 9%) as a white solid. MS (ESI, m/z): 584.3 [M+H]$^+$.

$^1$H NMR (400 MHz, $CDCl_3$) δ 7.53 (d, *J* = 6.6 Hz, 2H), 7.40 (d, *J* = 6.6 Hz, 2H), 7.29 - 7.24 (m, 1H), 7.12 - 7.04 (m, 1H), 7.00 - 6.91 (m, 1H), 6.57 - 6.51 (m, 1H), 6.48 - 6.39 (m, 1H), 3.78 (s, 3H), 3.64 - 3.54 (m, 2H), 3.17 - 3.04 (m, 1H), 2.93 (d, *J* = 6.8 Hz, 2H), 2.84 - 2.71 (m, 3H), 2.47 - 2.32 (m, 2H), 2.00 - 1.91 (m, 2H), 1.41 (s, 9H), 1.21 - 1.17 (m, 1H), 0.68 - 0.58 (m, 1H), 0.49 - 0.32 (m, 2H), 0.29 - 0.19 (m, 1H).

**Example 226:**

Synthetic Route:

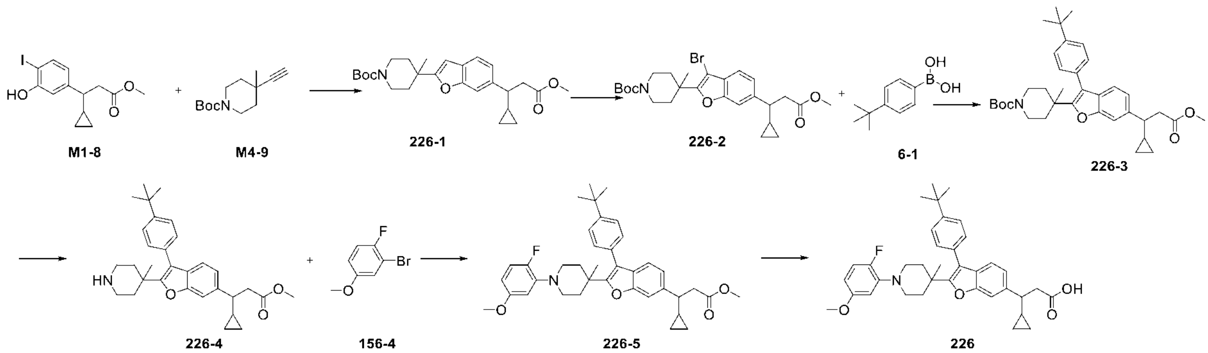

M1-8 M4-9 226-1 226-2 6-1 226-3 226-4 156-4 226-5 226

Referring to the synthetic route of intermediate **M1, M1-9** was replaced with commercially available starting material **M4-9** to synthesize compound **226-1.** Referring to the synthetic route of compound **6,** the synthesis was carried out, and the resulting crude product was purified by reverse-phase column chromatography [acetonitrile/water (0.05% formic acid) = 0% to 100%] to obtain compound **226** (18 mg, yield: 35%) as a white solid. MS (ESI, m/z): 584.3 [M+H]$^+$.

$^1$H NMR (400 MHz, $CDCl_3$) δ 7.42 (d, *J* = 8.0 Hz, 2H), 7.36 (s, 1H), 7.30 - 7.23 (m, 2H), 7.16 - 7.10 (m, 1H), 7.08 -7.00 (m, 1H), 6.85 (m, 1H), 6.43 - 6.28 (m, 2H), 3.72 (s, 3H), 3.15 - 3.02 (m, 2H), 2.90 - 2.79 (m, 2H), 2.77 - 2.65 (m, 2H), 2.51 -2.43 (m, 1H), 2.41 - 2.30 (m, 2H), 1.77 - 1.63 (m, 2H), 1.44 (s, 3H), 1.37 s, 9H), 1.15 - 1.04 (m, 1H), 0.65 - 0.55 (m, 1H), 0.50 - 0.41 (m, 1H), 0.38 - 0.29 (m, 1H), 0.24 - 0.16 (m, 1H).

**Example 227:**

Synthetic Route:

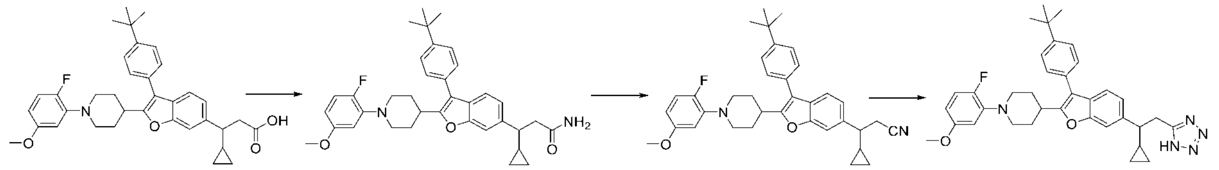

168 227-1 227-2 227

Compound **168** (300 mg, 0.53 mmol), ammonium chloride (42 mg, 0.79 mmol), 2-(7-azabenzotriazol-1-yl)-*N,N,N',N'*-tetramethyluronium hexafluorophosphate (400 mg, 1.05 mmol), and *N,N*-diisopropylethylamine (170 mg, 1.32 mmol) were added to *N,N*-dimethylformamide (10 mL) and stirred at room temperature for 2 hours. After the reaction was completed, saturated brine (50 mL) was added, and the reaction mixture was extracted with ethyl acetate (20 mL × 3). The combined organic phases were concentrated to obtain a crude product, which was purified by normal-phase column chromatography (ethyl acetate: petroleum ether = 0% to 100%) to obtain compound **227-1** (280 mg, yield: 93%) as a yellow oil. MS (ESI, m/z): 569.3 $[M+H]^+$.

To a reaction flask containing compound **227-1** (280 mg, 0.49 mmol), triethylamine (125 mg, 1.23 mmol), and 1,4-dioxane (15 mL), trifluoroacetic acid (207 mg, 0.98 mmol) was added dropwise, and the reaction mixture was stirred at room temperature for 16 hours. After the reaction was completed, the reaction mixture was directly concentrated to obtain a crude product, which was purified by normal-phase column chromatography (ethyl acetate: petroleum ether = 0% to 100%) to obtain compound **227-2** (220 mg, yield: 81%) as a yellow oil. MS (ESI, m/z): 551.2 $[M+H]^+$.

To a reaction flask containing compound **227-2** (120 mg, 0.22 mmol) and toluene (15 mL), trimethylsilyl azide (88 mg, 0.76 mmol) and dibutyltin oxide (81 mg, 0.33 mmol) were added separately. The reaction mixture was stirred under a nitrogen atmosphere at 120°C for 16 hours. After the reaction was completed, the reaction mixture was directly concentrated to obtain a crude product, which was purified by reverse-phase column chromatography [acetonitrile/water (0.05% formic acid) = 0% to 100%] to obtain compound **227** (18 mg, yield: 14%) as a yellow solid. MS (ESI, m/z): 594.3 $[M+H]^+$.

$^1H$ NMR (400 MHz, DMSO-$d_6$) $\delta$ 7.56 (d, *J* = 8.4 Hz, 2H), 7.45 - 7.38 (m 3H), 7.35 (s, 1H), 7.16 (dd, *J* = 11.6, 9.2 Hz, 1H), 7.09 (d, *J* = 8.0 Hz, 1H), 6.94 (dd, *J* = 6.4, 2.8 Hz, 1H), 6.84 - 6.77(m, 1H), 3.80 (s, 3H), 3.74 - 3.64 (m, 2H), 3.50 - 3.40 (m, 2H), 3.36 - 3.18 (m, 3H), 2.50 - 2.35 (m, 3H), 2.15 - 2.05 (m, 2H), 1.35 (s, 9H), 1.27 - 1.17 (m, 1H), 0.54 - 0.44 (m, 1H), 0.38 - 0.29 (m, 1H), 0.14 - 0.07 (m, 2H).

**Example 228:**

Synthetic Route:

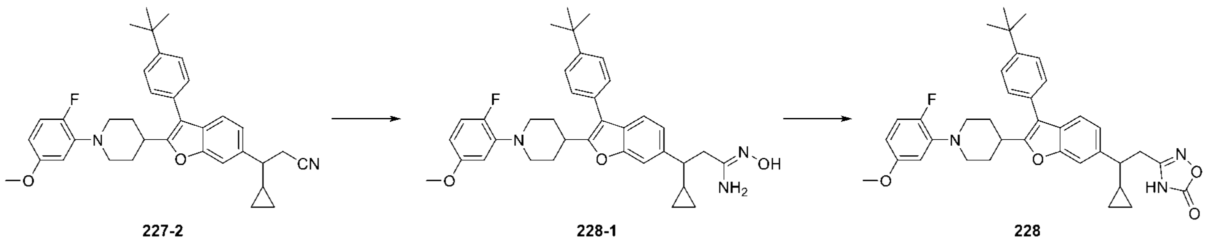

227-2 228-1 228

Referring to the synthetic route of compound **227,** compound **227-2** (60 mg, 0.11 mmol) was synthesized. Compound **227-2** (60 mg, 0.11 mmol) and 50% hydroxylamine aqueous solution (18 mg, 0.55 mmol) were added to dimethyl sulfoxide (5 mL). The reaction mixture was stirred at 90°C under a nitrogen atmosphere for 4 hours. After the reaction was completed, saturated brine (30 mL) was added, and the reaction mixture was extracted with ethyl acetate (20 mL × 3). The combined organic phases were concentrated to obtain a crude product, which was purified by normal-phase column chromatography (ethyl acetate: petroleum ether = 0% to 100%) to obtain compound **228-1** (40 mg, yield: 62%) as a yellow oil. MS (ESI, m/z): 584.3 $[M+H]^+$.

Compound **228-1** (40 mg, 0.069 mmol) and N,N'-carbonyldiimidazole (22 mg, 0.14 mmol) were added to 1,4-dioxane (5 mL) and stirred under a nitrogen atmosphere at 90°C for 4 hours. After the reaction was completed, the reaction mixture was directly concentrated and purified by reverse-phase column chromatography [acetonitrile/water (0.05% formic acid) = 0% to 100%] to obtain compound **228** (3 mg, yield: 5%) as a yellow solid. MS (ESI, m/z): 610.3 $[M+H]^+$.

$^1H$ NMR (400 MHz, $CD_3OD$) $\delta$ 7.55 (d, *J* = 8.4 Hz, 2H), 7.45 - 7.38 (m, 4H), 7.11 (d, *J* = 8.0 Hz, 1H), 6.97 - 6.90 (m, 1H), 6.66 - 6.56 (m, 1H), 6.53 - 6.44 (m, 1H), 3.75 (s, 3H), 3.58 - 3.49 (m, 2H), 3.15 - 3.07 (m,1H), 3.05 - 2.91 (m, 2H), 2.81 -

2.69 (m, 2H), 2.42 - 2.20 (m, 3H), 2.00 - 1.87 (m, 2H), 1.39 (s, 9H), 1.23 - 1.11 (m, 1H), 0.70 - 0.59 (m, 1H), 0.48 - 0.38 (m, 1H), 0.33 - 0.23 (m, 1H), 0.20 - 0.13 (m, 1H).

**Example 229:**

Synthetic Route:

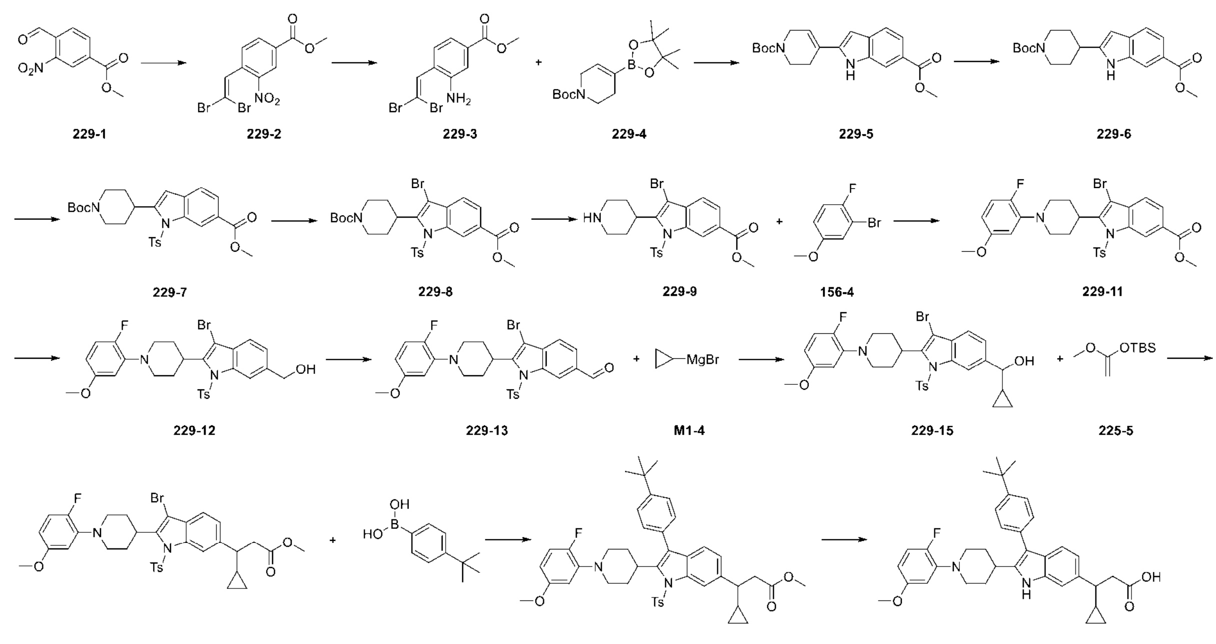

229-1 229-2 229-3 229-4 229-5 229-6

229-7 229-8 229-9 156-4 229-11

229-12 229-13 M1-4 229-15 225-5

229-16 6-1 229-18 229

Compound **229-1** (10.0 g, 47.9 mmol) and carbon tetrabromide (17.5 g, 52.6 mmol) were added to dichloromethane (350 mL) and stirred. Triphenylphosphine (25.1 g, 95.7 mmol) was added in portions at 0°C, and the reaction was carried out at 0°C for 2 hours. After the reaction was completed, petroleum ether (350 mL) was added, and the mixture was filtered. The filtrate was concentrated to obtain a crude product. The resulting crude product was then purified by normal -phase column chromatography (ethyl acetate: petroleum ether = 0% to 100%) to obtain compound **229-2** (14.5 g, yield: 83%) as a yellow solid. MS (ESI, m/z): 365.9 $[M+H]^+$.

Compound **229-2** (14.5 g, 39.9 mmol) was added to anhydrous ethanol (160 mL) and stirred. Stannous chloride (30.2 g, 159.8 mmol) was added, and the reaction was carried out at 100°C for 1 hour. After the reaction was completed, the reaction mixture was concentrated to obtain a crude product. The resulting crude product was diluted with ethyl acetate (400 mL) and water (100 mL), adjusted to pH = 9 with saturated sodium carbonate solution, extracted with ethyl acetate, and the combined organic phases were dried over anhydrous sodium sulfate and concentrated to obtain a crude product. The resulting crude product was then purified by normal -phase column chromatography (ethyl acetate: petroleum ether = 0% to 100%) to obtain compound **229-3** (13.1 g, yield: 98%) as a yellow oil. MS (ESI, m/z): 335.9 $[M+H]^+$.

Compound **229-3** (13.1 g, 39.3 mmol), *N*-Boc-1,2,5,6-tetrahydropyridine-4-boronic acid pinacol ester **229-4** (18.2 g, 59.0 mmol), potassium phosphate hydrate (45.2 g, 196.8 mmol), and 2-dicyclohexylphosphino-2',6'-dimethoxybiphenyl (970 mg, 2.4 mmol) were added to toluene (300 mL) and stirred. Under a nitrogen atmosphere, palladium acetate (266 mg, 1.2 mmol) was added, and the reaction was carried out at 90°C for 24 hours. After the reaction was completed, the reaction mixture was diluted with ethyl acetate and filtered. The filtrate was concentrated to obtain a crude product. The resulting crude product was then purified by normal-phase column chromatography (ethyl acetate: petroleum ether = 0% to 100%) to obtain compound **229-5** (7.6 g, yield: 54%) as a yellow oil. MS (ESI, m/z): 357.3 $[M+H]^+$.

Compound **229-5** (7.6 g, 21.4 mmol) was added to methanol (50 mL) and tetrahydrofuran (50 mL) and stirred. 10% palladium on carbon (1.52 g) was added, and the reaction was carried out under hydrogen (1 atm) at room temperature for 24 hours. After the reaction was completed, the reaction mixture was filtered, and the filtrate was concentrated. The resulting crude product was purified by normal-phase column chromatography (ethyl acetate: petroleum ether = 0% to 100%) to obtain compound **229-6** (7.6 g, yield: 99%) as a yellow solid. MS (ESI, m/z): 359.3 $[M+H]^+$.

Compound **229-6** (7.6 g, 21.2 mmol) was added to anhydrous tetrahydrofuran (80 mL). Sodium hydride (60%, 2.6

g, 63.7 mmol) was added in portions at 0°C, and the mixture was stirred at 0°C for 1 hour. Then, a solution of *p*-toluenesulfonyl chloride (6.1 g, 31.8 mmol) in anhydrous tetrahydrofuran (20 mL) was slowly added dropwise at 0°C, followed by stirring at room temperature for 16 hours. After the reaction was completed, the reaction mixture was quenched with saturated ammonium chloride solution and extracted with ethyl acetate. The organic phase was dried over anhydrous sodium sulfate, filtered, and concentrated to obtain a crude product. The resulting crude product was then purified by normal-phase column chromatography (ethyl acetate: petroleum ether = 0% to 100%) to obtain compound **229-7** (5.2 g, yield: 48%) as a yellow solid. MS (ESI, m/z): 513.3 $[M+H]^+$.

Compound **229-7** (5.2 g, 10.2 mmol) was added to anhydrous dichloromethane (100 mL) and stirred. *N*-Bromosuccinimide (2.7 g, 15.2 mmol) was added in portions at 0°C, and the reaction was carried out at 0°C for 2 hours. After the reaction was completed, the reaction mixture was quenched with saturated sodium bicarbonate solution and concentrated to obtain a crude product. The resulting crude product was then purified by normal-phase column chromatography (ethyl acetate: petroleum ether = 0% to 100%) to obtain compound **229-8** (4.1 g, yield: 68%) as a brown oil. MS (ESI, m/z): 591.2 $[M+H]^+$.

Compound **229-8** (4.1 g, 7.0 mmol) was added to dichloromethane (40 mL) and stirred, followed by the addition of trifluoroacetic acid (4 mL). The reaction was carried out at room temperature for 2 hours. After the reaction was completed, the reaction mixture was concentrated, diluted with ethyl acetate, and the organic phase was washed with saturated sodium bicarbonate solution. The organic phase was dried over anhydrous sodium sulfate, concentrated, and then purified by normal-phase column chromatography (ethyl acetate: petroleum ether = 0% to 100%) to obtain compound **229-9** (3.4 g, yield: 99%) as a yellow solid. MS (ESI, m/z): 491.2 $[M+H]^+$.

Compound **229-9** (3.4 g, 7.0 mmol), 3-bromo-4-fluoroanisole **156-4** (4.3 g, 20.8 mmol), 4,5-bis(diphenylphosphino)-9,9-dimethylxanthene (804 mg, 1.4 mmol), and cesium carbonate (6.8 g, 20.8 mmol) were added to anhydrous 1,4-dioxane (30 mL) and stirred. Under a nitrogen atmosphere, tris(dibenzylideneacetone)dipalladium (636 mg, 0.7 mmol) was added, and the reaction was carried out in a sealed tube and heated to 120°C for 36 hours. After the reaction was completed, the reaction mixture was diluted with dichloromethane: methanol (5:1) (200 mL), filtered, and the filtrate was concentrated. The resulting crude product was then purified by normal-phase column chromatography (ethyl acetate: petroleum ether = 0% to 100%). The resulting crude product was then purified by column chromatography to obtain compound **229-11** (900 mg, crude product) as a yellow oil. MS (ESI, m/z): 615.2 $[M+H]^+$.

Compound **229-11** (650 mg, 1.1 mmol) was added to anhydrous tetrahydrofuran (20 mL), followed by the portion-wise addition of lithium borohydride (312 mg, 5.3 mmol). The reaction mixture was stirred at room temperature for 16 hours. After the reaction was completed, the system was slowly poured into ice water with stirring. The mixture was extracted with ethyl acetate, and the organic phase was dried over anhydrous sodium sulfate, filtered, and concentrated to obtain a crude product. The resulting crude product was then purified by normal-phase column chromatography (ethyl acetate: petroleum ether = 0% to 100%) to obtain compound **229-12** (320 mg, yield: 52%) as a yellow oil. MS (ESI, m/z): 587.2 $[M+H]^+$.

Compound **229-12** (320 mg, 0.55 mmol) was added to dichloromethane (15 mL) and stirred. At 0°C, Dess-Martin periodinane (278 mg, 0.66 mmol) was added, and the reaction mixture was stirred at room temperature for 4 hours at 0°C. After the reaction was completed, the reaction mixture was quenched with saturated sodium bicarbonate solution and concentrated to obtain a crude product. The resulting crude product was then purified by normal-phase column chromatography (ethyl acetate: petroleum ether = 0% to 100%) to obtain compound **229-13** (170 mg, yield: 53%) as a yellow oil. MS (ESI, m/z): 585.2 $[M+H]^+$.

Compound **229-13** (170 mg, 0.29 mmol) was added to anhydrous tetrahydrofuran (10 mL) and stirred. At 0°C, cyclopropylmagnesium bromide **M1-4** (1.0 M in tetrahydrofuran, 0.87 mL, 0.87 mmol) was slowly added, and the reaction mixture was stirred at room temperature for 2 hours. After the reaction was completed, the reaction mixture was quenched with saturated sodium bicarbonate solution and concentrated to obtain a crude product. The resulting crude product was then purified by normal-phase column chromatography (ethyl acetate: petroleum ether = 0% to 100%) to obtain compound **229-15** (170 mg, yield: 93%) as a yellow oil. MS (ESI, m/z): 627.2 $[M+H]^+$.

Compound **229-15** (170 mg, 0.27 mmol), 1-(*tert*-butyldimethylsilyloxy)-1-methoxyethene **225-5** (153 mg, 0.81 mmol), and rhenium pentacarbonyl bromide (22 mg, 0.05 mmol) were added to anhydrous toluene (5 mL). The reaction was carried out in a sealed tube under a nitrogen atmosphere and heated at 120°C for 24 hours. After the reaction was completed, the reaction mixture was concentrated and purified by normal-phase column chromatography (ethyl acetate: petroleum ether = 0% to 100%) to obtain compound **229-16** (110 mg, yield: 59%) as a yellow oil. MS (ESI, m/z): 683.3 $[M+H]^+$.

Compound **229-16** (110 mg, 0.16 mmol), 4-*tert*-butylbenzeneboronic acid **6-1** (43 mg, 0.24 mmol), bis(triphenylphosphine)palladium(II) chloride (11 mg, 0.02 mmol), and potassium carbonate (67 mg, 0.48 mmol) were added to *N,N*-dimethylformamide (4 mL) and water (1 mL) and stirred. Under a nitrogen atmosphere, the reaction mixture was stirred at 100°C for 16 hours. After the reaction was completed, the reaction mixture was filtered, concentrated, and purified by normal -phase column chromatography (ethyl acetate: petroleum ether = 0% to 100%) to obtain compound **229-17** (117 mg, yield: 99%) as a yellow oil. MS (ESI, m/z): 737.3 $[M+H]^+$.

Compound **229-17** (118 mg, 0.16 mmol) was added to methanol (5 mL) and stirred. 4 M sodium hydroxide solution (5 mL) was added, and the reaction mixture was stirred at 100°C for 36 hours. After the reaction was completed, the reaction mixture was concentrated to obtain a crude product, which was purified by reverse-phase column chromatography [acetonitrile/water (0.05% formic acid) = 0% to 100%] to obtain compound **229** (1.8 mg, yield: 2%) as a white solid. MS (ESI, m/z): 569.3 $[M+H]^+$.

$^1H$ NMR (400 MHz, DMSO-$d_6$) $\delta$ 11.10 (s, 1H), 7.49 (d, $J$ = 7.6 Hz, 2H), 7.37-7.35 (m, 3H), 7.17 (s, 1H), 7.07 - 7.02 (m, 1H), 6.90 (d, $J$ = 7.6 Hz, 1H), 6.59 - 6.57 (m, 1H), 6.50 - 6.48 (m, 1H), 3.72 (s, 3H), 3.60 - 3.46 (m, 2H), 3.07 - 3.00 (m, 1H), 2.79 - 2.63 (m, 4H), 2.39 - 2.31 (m, 1H), 2.25 - 2.11 (m, 2H), 1.92 - 1.83 (m, 2H), 1.35 (s, 9H), 1.09 - 0.98 (m, 1H), 0.53 - 0.44 (m, 1H), 0.36 - 0.21 (m, 2H), 0.14 - 0.04 (m, 1H).

**Example 230:**

Synthetic Route:

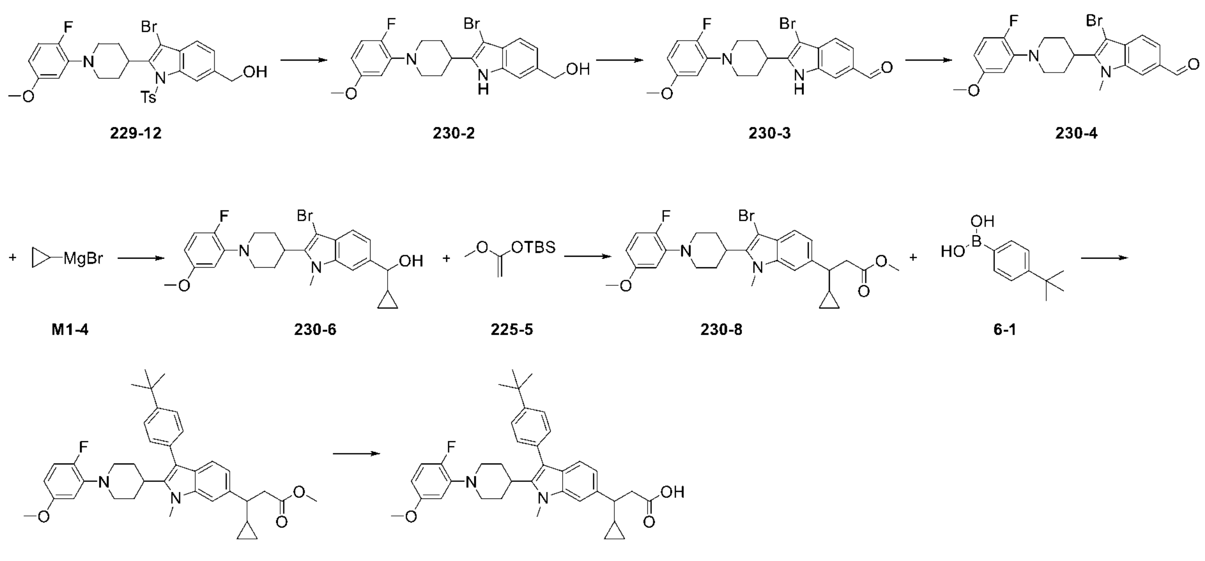

229-12, 230-2, 230-3, 230-4

M1-4, 230-6, 225-5, 230-8, 6-1

230-10, 230

Compound **229-12** (1 g, 1.7 mmol) was added to methanol (20 mL) and stirred. 4 M sodium hydroxide solution (20 mL) was added, and the reaction mixture was stirred at 100°C for 6 hours. After the reaction was completed, the reaction mixture was concentrated to obtain a crude product. The resulting crude product was then purified by normal-phase column chromatography (ethyl acetate: petroleum ether = 0% to 100%) to obtain compound **230-2** (330 mg, yield: 45%) as a white solid. MS (ESI, m/z): 433.3 $[M+H]^+$.

Compound **230-2** (330 mg, 0.76 mmol) was added to dichloromethane (10 mL) and stirred. At 0°C, Dess-Martin periodinane (389 mg, 0.92 mmol) was added, and the reaction mixture was stirred at room temperature for 4 hours. After the reaction was completed, the reaction mixture was quenched with saturated sodium bicarbonate solution and concentrated to obtain a crude product. The resulting crude product was then purified by normal-phase column chromatography (ethyl acetate: petroleum ether = 0% to 100%) to obtain compound **230-3** (220 mg, yield: 67%) as a yellow oil. MS (ESI, m/z): 431.3 $[M+H]^+$.

Compound **230-3** (200 mg, 0.47 mmol) was added to *N,N*-dimethylformamide (5 mL), and sodium hydride (60%, 37 mg, 0.93 mmol) was added at 0°C. The reaction mixture was stirred at 0°C for 0.5 hours, followed by the addition of iodomethane (201 mg, 1.40 mmol) at 0°C. The reaction mixture was then stirred at room temperature for 2 hours. After the reaction was completed, the reaction mixture was quenched with water and extracted with ethyl acetate. The organic phase was dried over anhydrous sodium sulfate, filtered, and concentrated to obtain a crude product. The resulting crude product was then purified by normal-phase column chromatography (ethyl acetate: petroleum ether = 0% to 100%) to obtain compound **230-4** (200 mg, yield: 97%) as a yellow oil. MS (ESI, m/z): 445.3 $[M+H]^+$.

Then, referring to the synthetic route of compound **229,** compound **229-13** was replaced with compound **230-4** to carry out the synthesis. The resulting crude product was purified by reverse-phase column chromatography [acetonitrile/-water (0.05% formic acid) = 0% to 100%] to obtain compound **230** (11 mg, yield: 6%) as a white solid. MS (ESI, m/z): 583.3 $[M+H]^+$.

$^1H$ NMR (400 MHz, DMSO-$d_6$) $\delta$ 7.49 - 7.47 (m, 2H), 7.31 - 7.28 (m, 3H), 7.16 (d, $J$ = 8.0 Hz, 1H), 7.09 - 6.97 (m,

1H), 6.95 - 6.85 (m, 1H), 6.61 - 6.44 (m, 2H), 3.85 (s, 3H), 3.71 (s, 3H), 3.47 - 3.38 (m, 2H), 3.20 - 3.16 (m, 1H), 2.78 - 2.63 (m, 4H), 2.44 - 2.38 (m, 1H), 2.18 - 2.02 (m, 2H), 1.90 - 1.76 (m, 2H), 1.35 (s, 9H), 1.13 - 1.02 (m, 1H), 0.58 - 0.45 (m, 1H), 0.37 - 0.20 (m, 2H), 0.18 - 0.03 (m, 1H).

**Example 231:**

Synthetic Route:

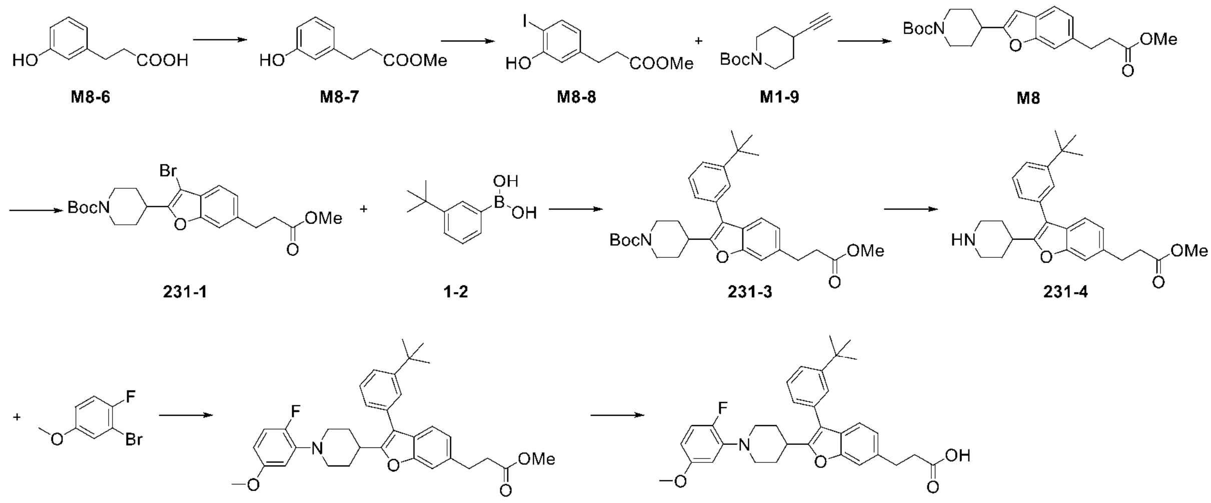

M8-6 M8-7 M8-8 M1-9 M8

231-1 1-2 231-3 231-4

156-4 231-6 231

Referring to the synthetic route of intermediate **M1,** compound **M1-6** was replaced with commercially available starting material **M8-6** to synthesize intermediate **M8.** Then, referring to the synthetic route of compound **12,** the synthesis was carried out, and the resulting crude product was purified by reverse-phase column chromatography [acetonitrile/water (0.05% formic acid) = 0% to 100%] to obtain compound **231** (10 mg, yield: 20%) as a white solid. MS (ESI, m/z): 530.3 $[M+H]^+$.

$^1H$ NMR (400 MHz, $CDCl_3$) δ 7.52 - 7.49 (m, 2H), 7.47 - 7.42 (m, 2H), 7.36 (s, 1H), 7.33 - 7.30 (m, 1H), 7.12 (d, *J* = 8.4 Hz, 1H), 6.96 (dd, *J* = 12.0, 8.8 Hz, 1H), 6.57 - 6.51 (m, 1H), 6.47 - 6.41 (m, 1H), 3.79 (s, 3H), 3.65 - 3.56 (m, 2H), 3.14 - 3.08 (m, 3H), 2.79 - 2.70 (m, 4H), 2.42 - 2.32 (m, 2H), 2.00 - 1.92 (m, 2H), 1.40 (s, 9H).

**Example 232:**

Synthetic Route:

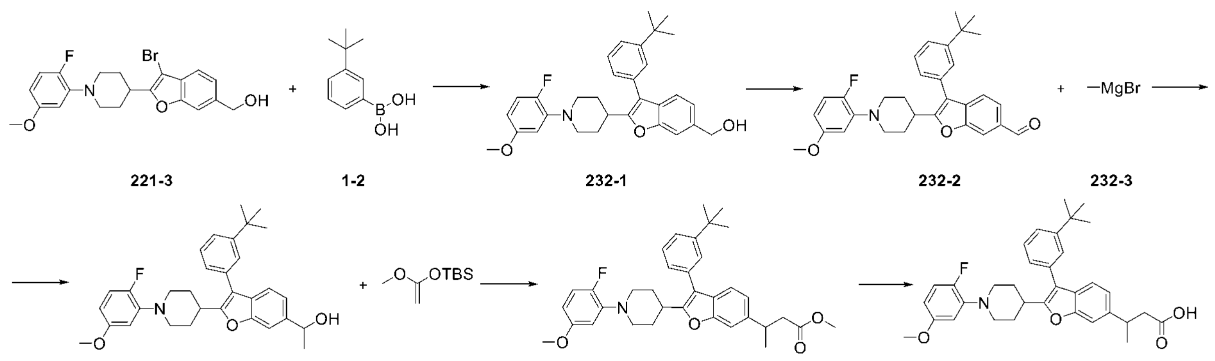

221-3 1-2 232-1 232-2 232-3

232-4 225-5 232-5 232

Referring to the synthetic route of compound **221,** compound **6-1** was replaced with compound **1-2** to synthesize compound **232-2** (60 mg, 0.12 mmol), and compound **232-2** (60 mg, 0.12 mmol) was added to anhydrous tetrahydrofuran

(10 mL) and stirred. Methylmagnesium bromide **232-3** (1.0 M in tetrahydrofuran, 0.37 mL, 0.37 mmol) was slowly added at 0°C, and the reaction mixture was stirred at 0°C for 1 hour. After the reaction was completed, the reaction mixture was quenched with saturated sodium bicarbonate solution (10 mL). The organic layer was concentrated to obtain a crude product, which was purified by normal-phase column chromatography (ethyl acetate: petroleum ether = 0% to 100%) to obtain compound **232-4** (60 mg, yield: 97%) as a yellow oil. MS (ESI, m/z): 502.3 $[M+H]^+$.

Compound **232-4** (60 mg, 0.12 mmol), 1-(*tert*-butyldimethylsilyloxy)-1-methoxyethene **232-5** (68 mg, 0.36 mmol), and rhenium pentacarbonyl bromide (5 mg, 0.01 mmol) were added to anhydrous toluene (4 mL). The reaction was carried out in a sealed tube under a nitrogen atmosphere and heated at 120°C for 16 hours. After the reaction was completed, the reaction mixture was concentrated and purified by normal-phase column chromatography (ethyl acetate: petroleum ether = 0% to 100%) to obtain compound **232-5** (66 mg, yield: 59%) as a yellow oil. MS (ESI, m/z): 558.3 $[M+H]^+$.

Compound **232-5** (66 mg, 0.12 mmol) was added to ethanol (3 mL) and stirred. 4 M sodium hydroxide solution (3 mL) was added, and the reaction mixture was stirred at 50°C for 16 hours. After the reaction was completed, the pH was adjusted to 3-4 with 1 N hydrochloric acid, and the mixture was extracted with ethyl acetate (10 mL). The combined organic phases were dried over anhydrous sodium sulfate, filtered, and concentrated to obtain a crude product, which was purified by reverse-phase column chromatography [acetonitrile/water (0.05% formic acid) = 0% to 100%] to obtain compound **232** (26 mg, yield: 40%) as a white solid. MS (ESI, m/z): 544.3 $[M+H]^+$.

$^1$H NMR (400 MHz, DMSO-$d_6$) δ 7.56 - 7.38 (m, 5H), 7.33 (*d*, *J* = 6.8 Hz, 1H), 7.19 (*d*, *J* = 8.0 Hz, 1H), 7.07 - 7.02 (m, 1H), 6.57 - 6.55 (m, 1H), 6.53 - 6.44 (m, 1H), 3.72 (s, 3H), 3.53-3.44 (m, 2H), 3.27 - 3.26 (m, 1H), 3.11 - 3.02 (m, 1H), 2.77 - 2.71 (m, 2H), 2.58 (*d*, *J* = 7.6 Hz, 2H), 2.16 - 2.07 (m, 2H), 1.92 - 1.90 (m, 2H), 1.35 (s, 9H), 1.28 (*d*, *J* = 6.8 Hz, 3H).

**Example 233:**

Synthetic Route:

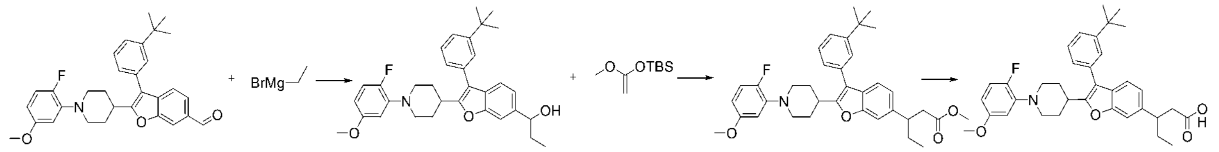

232-2 233-1 233-2 225-5 233-3 233

Referring to the synthetic route of compound **232,** compound **232-3** was replaced with compound **233-1** to carry out the synthesis. The resulting crude product was purified by reverse-phase column chromatography [acetonitrile/water (0.05% formic acid) = 0% to 100%] to obtain compound **233** (7 mg, yield: 15%) as a white solid. MS (ESI, m/z): 558.3 $[M+H]^+$.

$^1$H NMR (400 MHz, DMSO-$d_6$) δ 7.50 - 7.43 (m, 5H), 7.36 - 7.31 (m, 1H), 7.17 - 7.12 (m, 1H), 7.08 - 7.01 (m, 1H), 6.58 - 6.54 (m, 1H), 6.52 - 6.47 (m, 1H), 3.72 (s, 3H), 3.53 - 3.46 (m, 2H), 3.07 - 3.01 (m, 1H), 2.78 - 2.64 (m, 2H), 2.63 - 2.53 (m, 3H), 2.21 - 2.05 (m, 2H), 2.01 - 1.92 (m, 2H), 1.78 - 1.54 (m, 2H), 1.36 (s, 9H), 0.74 (t, *J* = 7.2 Hz, 3H).

**Example 234:**

Synthetic Route:

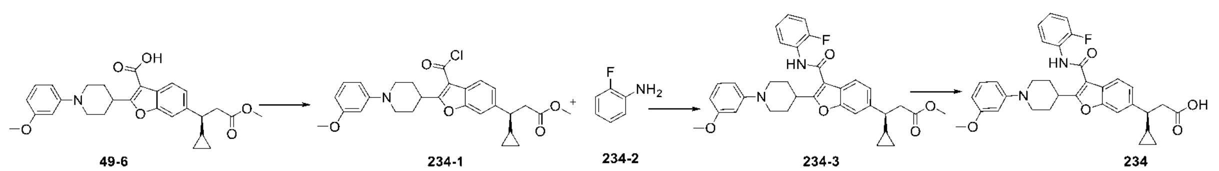

49-6 234-1 234-2 234-3 234

Compound **49-6** (710 mg, 1.49 mmol) and *N,N*-dimethylformamide (0.2 mL) were added to dichloromethane (30 mL) and stirred. Under a nitrogen atmosphere, oxalyl chloride (283 mg, 2.23 mmol) was added dropwise at 0°C, and the reaction was carried out for 4 hours. After the reaction was completed (monitored with methanol), the reaction mixture was directly used in the next step. MS (ESI, m/z): 491.2 $[M-4]^+$.

The reaction mixture containing compound **234-1** (366 mg, 0.74 mmol) obtained from the previous step was

slowly added dropwise under a nitrogen atmosphere at 0°C to a mixture of o-toluidine **234-2** (159 mg, 1.48 mmol) and triethylamine (375 mg, 3.71 mmol) in dichloromethane (40 mL). The reaction mixture was stirred at room temperature for 16 hours. After the reaction was completed, the reaction mixture was concentrated to obtain a crude product. The resulting crude product was purified by normal-phase column chromatography (petroleum ether: ethyl acetate = 8:2) to obtain compound **234-3** (410 mg, yield: 97%) as a yellow oil. MS (ESI, m/z): 571.3 $[M+H]^+$.

Compound **234-3** (400 mg, 0.7 mmol) was added to methanol (20 mL) and stirred. 4 M sodium hydroxide solution (20 mL) was added, and the reaction mixture was stirred at 60°C for 16 hours. After the reaction was completed, the reaction mixture was concentrated to obtain a crude product, which was purified by reverse-phase column chromatography [acetonitrile/water (0.05% formic acid) = 0% to 100%] to obtain compound **234** (350 mg, yield: 90%) as a white solid. MS (ESI, m/z): 557.2 $[M+H]^+$.

$^1H$ NMR (400 MHz, $CDCl_3$) δ 8.51- 8.47 (m, 1H), 8.02 - 7.97 (m, 1H), 7.70 (d, *J* = 8.0 Hz, 1H), 7.45 (s, 1H), 7.30 - 7.24 (m, 1H), 7.26 - 7.08 (m, 4H), 6.63 (dd, *J* = 8.0 Hz, 1H), 6.58 - 6.52 (m, 1H), 6.45 (dd, *J* = 8.0 Hz, 1H), 3.96- 3.76 (m, 6H), 3.05 - 2.75 (m, 4H), 2.54 - 2.45 (m, 1H), 2.30 - 2.12 (m, 2H), 2.08 - 1.99 (m, 2H), 1.18 - 1.03 (m, 1H), 0.68- 0.62 (m, 1H), 0.51- 0.44 (m, 1H), 0.38- 0.33 (m, 1H), 0.23- 0.18 (m, 1H).

**Example 235:**

Synthetic Route:

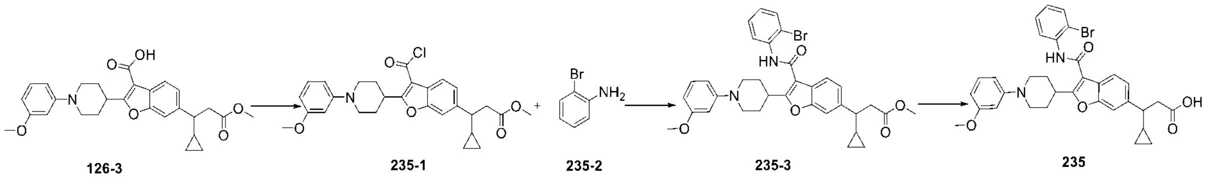

126-3 235-1 235-2 235-3 235

Referring to the synthetic route of compound **234,** compound **49-6** was replaced with compound **126-3,** and compound **234-2** was replaced with compound **235-2.** The synthesis was carried out, and the resulting crude product was purified by reverse-phase column chromatography [acetonitrile/water (0.05% formic acid) = 0% to 100%] to obtain compound **235** (35 mg, yield: 69%) as a white solid. MS (ESI, m/z): 553.2 $[M+H]^+$.

$^1H$ NMR (400 MHz, DMSO-$d_6$) δ 9.71 (s, 1H), 7.84 (d, *J* = 8.0 Hz, 1H), 7.82 - 7.76 (m, 1H), 7.74 (dd, *J* = 8.0, 1.2 Hz, 1H), 7.57 (s, 1H), 7.48 - 7.41 (m, 1H), 7.28 (d, *J* = 8.1 Hz, 1H), 7.26 - 7.20 (m, 1H), 7.15 - 7.09 (m, 1H), 6.62 - 6.56 (m, 1H), 6.52 - 6.48 (m, 1H), 6.37 (dd, *J* = 8.0, 2.0 Hz, 1H), 3.91 - 3.81 (m, 2H), 3.73 (s, 3H), 3.69 - 3.60 (m, 1H), 2.87 - 2.79 (m, 2H), 2.75 - 2.65 (m, 2H), 2.43 - 2.35 (m, 1H), 2.02 - 1.94 (m, 4H), 1.11 - 1.04 (m, 1H), 0.56 - 0.48 (m, 1H), 0.34 - 0.24 (m, 2H), 0.19 - 0.12 (m, 1H).

**Example 236:**

Synthetic Route:

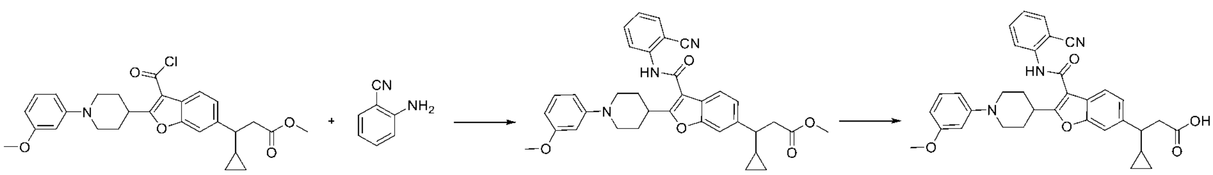

235-1 236-1 236-2 236

Referring to the synthetic route of compound **235,** compound **235-2** was replaced with compound **236-1** to carry out the synthesis. The resulting crude product was purified by reverse-phase column chromatography [acetonitrile/water (0.05% formic acid) = 0% to 100%] to obtain compound **236** (1 mg, yield: 5%) as a white solid. MS (ESI, m/z): 564.2 $[M+H]^+$.

$^1H$ NMR (400 MHz, DMSO-$d_6$) δ 10.50 (s, 1H), 8.49 (s, 1H), 7.92 (d, *J* = 7.7 Hz, 1H), 7.85 - 7.73 (m, 3H), 7.57 (s, 1H), 7.49 - 7.42 (m, 1H), 7.30 (d, *J* = 8.1 Hz, 1H), 7.19 - 7.12 (m, 1H), 6.61 (dd, *J* = 8.2, 1.9 Hz, 1H), 6.56 - 6.51 (m, 1H), 6.40 (dd, *J* = 8.1, 2.1 Hz, 1H), 3.92 - 3.85 (m, 2H), 3.76 (s, 3H), 3.70 - 3.60 (s, 1H), 2.91 - 2.82 (m, 2H), 2.72 - 2.60 (m, 2H), 2.52 - 2.41 (m, 1H), 2.08 - 1.93 (m, 4H), 1.15 - 1.03 (m, 1H), 0.58 - 0.48 (m, 1H), 0.38 - 0.25 (m, 2H), 0.20 - 0.10 (m, 1H).

**Example 237:**

Synthetic Route:

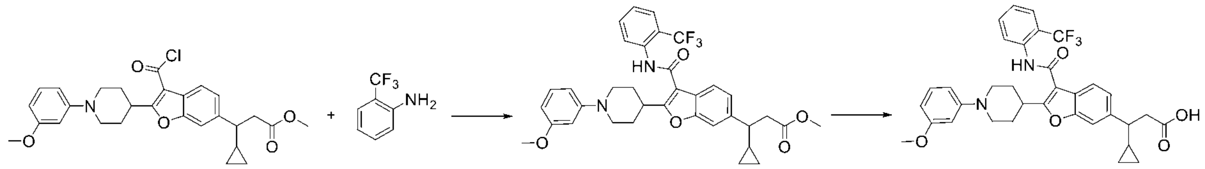

235-1 237-1 237-2 237

Referring to the synthetic route of compound **235,** compound **235-2** was replaced with compound **237-1** to carry out the synthesis. The resulting crude product was purified by reverse-phase column chromatography [acetonitrile/water (0.05% formic acid) = 0% to 100%] to obtain compound **237** (3 mg, yield: 9%) as a white solid. MS (ESI, m/z): 607.2 [M+H]$^+$.

$^1$H NMR (400 MHz, DMSO-$d_6$) δ 9.92 (s, 1H), 8.39 (s, 1H), 7.82 (d, $J$ = 7.6 Hz, 1H), 7.79 - 7.73 (m, 1H), 7.71 (d, $J$ = 8.0 Hz, 1H), 7.64 (d, $J$ = 8.0 Hz, 1H), 7.59 - 7.50 (m, 2H), 7.27 (d, $J$ = 8.2 Hz, 1H), 7.17 - 7.08 (m, 1H), 6.59 (dd, $J$ = 8.2, 2.0 Hz, 1H), 6.54 - 6.46 (m, 1H), 6.37 (dd, $J$ = 8.0, 2.0 Hz, 1H), 3.91 - 3.84 (m, 2H), 3.73 (s, 3H), 3.64 - 3.54 (m, 1H), 2.86 - 2.75 (m, 2H), 2.74 - 2.63 (m, 2H), 2.45 - 2.35 (m, 1H), 2.06 - 1.89 (m, 4H), 1.12 - 1.02 (m, 1H), 0.56 - 0.47 (m, 1H), 0.34 - 0.24 (m, 2H), 0.18 - 0.10 (m, 1H).

**Example 238:**

Synthetic Route:

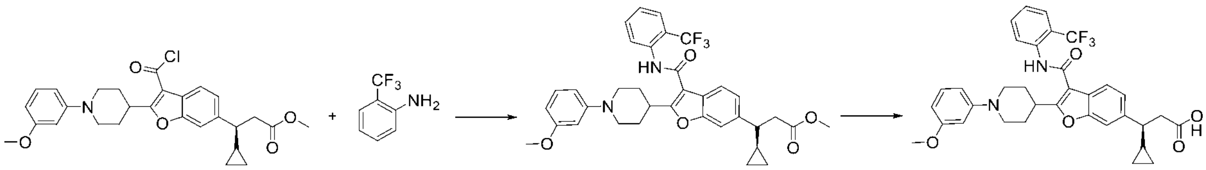

234-1 237-1 238-2 238

Referring to the synthetic route of compound **234,** compound **234-2** was replaced with compound **237-1** to carry out the synthesis. The resulting crude product was purified by reverse-phase column chromatography [acetonitrile/water (0.05% formic acid) = 0% to 100%] to obtain compound **237** (18 mg, yield: 64%) as a white solid. MS (ESI, m/z): 607.2 [M+H]$^+$.

$^1$H NMR (400 MHz, $CDCl_3$) δ 8.43 (d, $J$ = 8.4 Hz, 1H), 8.09 (s, 1H), 7.72 - 7.66 (m, 3H), 7.47 (s, 1H), 7.34 - 7.30 (m, 2H), 7.24 - 7.19 (m, 1H), 6.69 - 6.42 (m, 3H), 3.86 - 3.83 (m, 6H), 2.99 - 2.86 (m, 4H), 2.57 - 2.50 (m, 1H), 2.31 - 2.19 (m, 2H), 2.09 - 2.04 (m, 2H), 1.14 - 1.09 (m, 1H), 0.69 - 0.61 (m, 1H), 0.53 - 0.46 (m, 1H), 0.41 - 0.35 (m, 1H), 0.26 - 0.20 (m, 1H).

**Example 239:**

Synthetic Route:

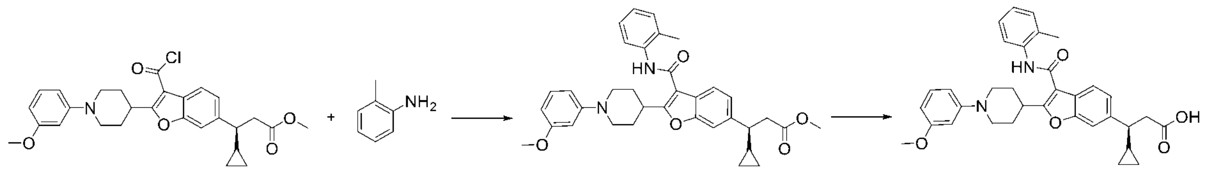

234-1 239-1 239-2 239

Referring to the synthetic route of compound **234,** compound **234-2** was replaced with compound **239-1** to carry

out the synthesis. The resulting crude product was purified by reverse-phase column chromatography [acetonitrile/water (0.05% formic acid) = 0% to 100%] to obtain compound **239** (35 mg, yield: 69%) as a white solid. MS (ESI, m/z): 553.2 $[M+H]^+$.

$^1H$ NMR (400 MHz, $CDCl_3$) δ 8.00 (d, *J* = 8.0 Hz, 1H), 7.68 (d, *J* = 8.0 Hz, 1H), 7.60 (s, 1H), 7.45 (s, 1H), 7.31 (d, *J* = 8.0 Hz, 1H), 7.28 - 7.24 (m, 2H), 7.23 -7.12 (m, 2H), 6.62 (dd, *J* = 8.4, 2.4 Hz, 1H), 6.56 - 6.53 (m, 1H), 6.44 (dd, *J* = 8.4, 2.4 Hz, 1H), 3.93 - 3.78 (m, 6H), 2.97 - 2.78 (m, 4H), 2.57 - 2.45 (m, 1H), 2.38 (s, 3H), 2.29 - 2.12 (m, 2H), 2.10 - 1.95 (m, 2H), 1.16 - 1.03 (m, 1H), 0.69 - 0.62 (m, 1H), 0.51 - 0.44 (m, 1H), 0.39 - 0.33 (m, 1H), 0.24 -0.18 (m, 1H).

**Example 240:**

Synthetic Route:

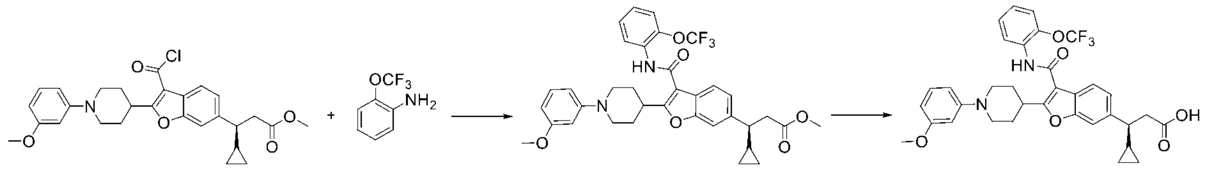

234-1 240-1 240-2 240

Referring to the synthetic route of compound **234,** compound **234-2** was replaced with compound **240-1** to carry out the synthesis. The resulting crude product was purified by reverse-phase column chromatography [acetonitrile/water (0.05% formic acid) = 0% to 100%] to obtain compound **240** (2 mg, yield: 5%) as a white solid. MS (ESI, m/z): 623.2 $[M+H]^+$.

$^1H$ NMR (400 MHz, MeOD) δ 8.03 (d, *J* = 7.0 Hz, 1H), 7.78 (d, *J* = 8.2 Hz, 1H), 7.55 - 7.40 (m, 5H), 7.39 - 7.32 (m, 2H), 7.25 - 7.19 (m, 2H), 7.13 - 7.04 (m, 1H), 4.00 - 3.74 (m 6H), 2.92 - 2.74 (m, 4H), 2.60 - 2.44 (m, 3H), 2.44 - 2.34 (m, 2H), 1.16 - 1.10 (m, 1H), 0.68 - 0.58 (m, 1H), 0.47 - 0.31 (m, 2H), 0.23 - 0.12 (m, 1H).

**Example 241:**

Synthetic Route:

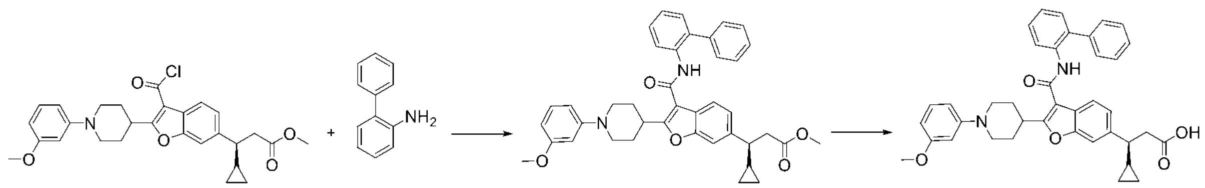

234-1 241-1 241-2 241

Referring to the synthetic route of compound **234,** compound **234-2** was replaced with compound **241-1** to carry out the synthesis. The resulting crude product was purified by reverse-phase column chromatography [acetonitrile/water (0.05% formic acid) = 0% to 100%] to obtain compound **241** (39 mg, yield: 48%) as a white solid. MS (ESI, m/z): 615.2 $[M+H]^+$.

$^1H$ NMR (400 MHz, $CDCl_3$) δ 8.64 (d, *J* = 8.4 Hz, 1H), 7.86 (s, 1H), 7.58 - 7.39 (m, 6H), 7.34 - 7.27 (m, 2H), 7.27 - 7.15 (m, 2H), 6.87 (d, *J* = 8.4 Hz, 1H), 6.62 (d, *J* = 8.4 Hz, 1H), 6.58 - 6.49 (m, 2H), 6.45 (d, *J* = 8.4 Hz, 1H), 3.94 - 3.66 (m, 6H), 2.92 - 2.76 (m, 4H), 2.52 - 2.40 (m, 1H), 2.17 - 2.07 (m, 2H), 1.98 - 1.86 (m, 2H), 1.08 - 1.02 (m, 1H), 0.66 - 0.60 (m, 1H), 0.52 - 0.38 (m, 1H), 0.35 - 0.30 (m, 1H), 0.21 - 0.10 (m, 1H).

**Example 242:**

Synthetic Route:

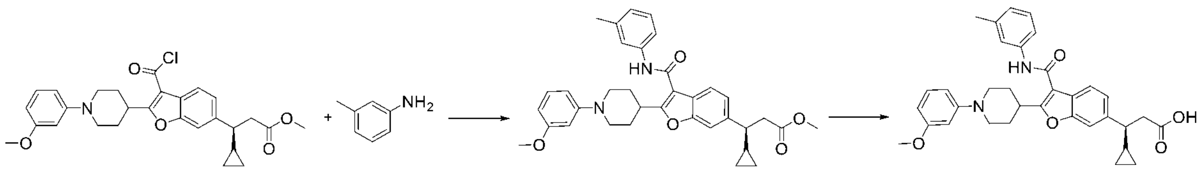

234-1 242-1 242-2 242

Referring to the synthetic route of compound **234,** compound **234-2** was replaced with compound **242-1** to carry out the synthesis. The resulting crude product was purified by reverse-phase column chromatography [acetonitrile/water (0.05% formic acid) = 0% to 100%] to obtain compound **242** (10 mg, yield: 51%) as a white solid. MS (ESI, m/z): 553.2 $[M+H]^+$.

$^1$H NMR (400 MHz, $CDCl_3$) δ 7.56 (d, *J* = 8.0 Hz, 1H), 7.42 (s, 1H), 7.38 - 7.31 (m, 2H), 7.24 - 7.16 (m, 4H), 6.96 - 6.90 (m, 1H), 6.79 - 6.50 (m, 3H), 3.82 - 3.60 (m, 6H), 3.16 - 3.03 (m, 2H), 2.81 - 2.68 (m, 2H), 2.45 - 2.38 (m, 1H), 2.32 (s, 3H), 2.29 - 2.18 (m, 2H), 2.12 - 2.01 (m, 2H), 1.07 - 0.96 (m, 1H), 0.62 - 0.51 (m, 1H), 0.43 - 0.35 (m, 1H), 0.32 - 0.24 (m, 1H), 0.16 - 0.09 (m, 1H).

**Example 243:**

Synthetic Route:

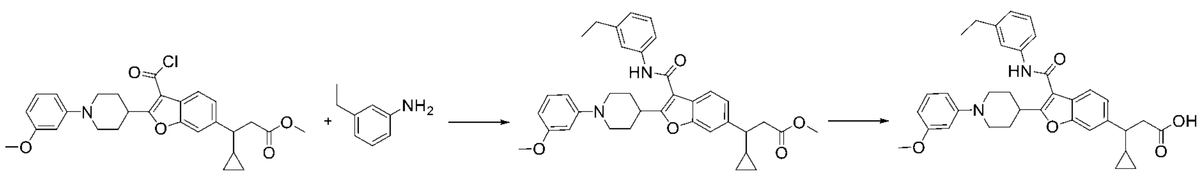

235-1 243-1 243-2 243

Referring to the synthetic route of compound **235,** compound **235-2** was replaced with compound **243-1** to carry out the synthesis. The resulting crude product was purified by reverse-phase column chromatography [acetonitrile/water (0.05% formic acid) = 0% to 100%] to obtain compound **243** (12 mg, yield: 42%) as a white solid. MS (ESI, m/z): 567.2 $[M+H]^+$.

$^1$H NMR (400 MHz, DMSO-$d_6$) δ 10.13 (s, 1H), 7.65 (s, 1H), 7.60 - 7.51 (m, 3H), 7.28 - 7.14 (m, 4H), 6.97 - 6.92 (m, 1H), 6.79 - 6.60 (m, 2H), 6.57 - 6.47 (m, 1H), 3.85 - 3.75 (m, 2H), 3.74 (s, 3H), 3.19 - 3.11 (m, 2H), 3.11 - 3.00 (m, 1H), 2.75 - 2.72 (m, 2H), 2.63 - 2.56 (m, 2H), 2.40 - 2.35 (m, 1H), 2.05 - 1.96 (m, 4H), 1.19 - 1.15 (m, 3H), 1.09 - 1.03 (m, 1H), 0.56 - 0.48 (m, 1H), 0.31 - 0.25 (m, 2H), 0.18 - 0.11 (m, 1H).

**Example 244:**

Synthetic Route:

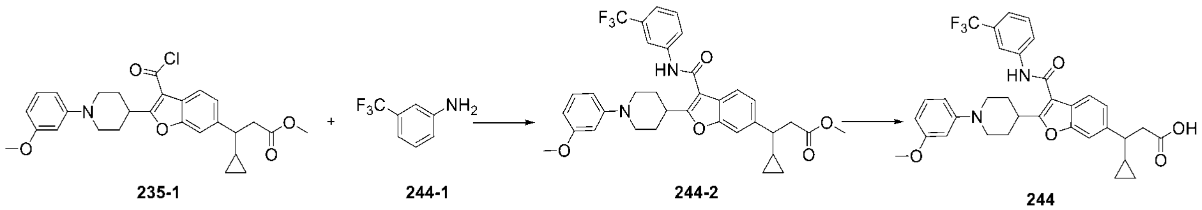

235-1 244-1 244-2 244

Referring to the synthetic route of compound **235,** compound **235-2** was replaced with compound **244-1** to carry out the synthesis. The resulting crude product was purified by reverse-phase column chromatography [acetonitrile/water (0.05% formic acid) = 0% to 100%] to obtain compound **244** (18 mg, yield: 56%) as a white solid. MS (ESI, m/z): 507.2 $[M+H]^+$.

$^1$H NMR (400 MHz, DMSO-$d_6$) δ 10.52 (s, 1H), 8.25 (s, 1H), 7.98 - 7.94 (m, 1H), 7.63 - 7.55 (m, 3H), 7.49 - 7.42 (m, 1H), 7.30 - 7.22 (m, 1H), 7.15 - 7.06 (m, 1H), 6.60 - 6.52 (m, 1H), 6.50 - 6.46 (m, 1H), 6.39 - 6.32 (m, 1H), 3.85 - 3.80 (m, 2H),

3.72 - 3.70 (m, 3H), 3.60 - 3.35 (m, 1H), 2.85 - 2.77 (m, 2H), 2.74 - 2.70 (m, 2H), 2.40 - 2.35 (m, 1H), 2.00 - 1.93 (m, 4H), 1.10 - 1.03 (m, 1H), 0.56 - 0.48 (m, 1H), 0.31 - 0.22 (m, 2H), 0.18 - 0.10 (m, 1H).

**Example 245:**

Synthetic Route:

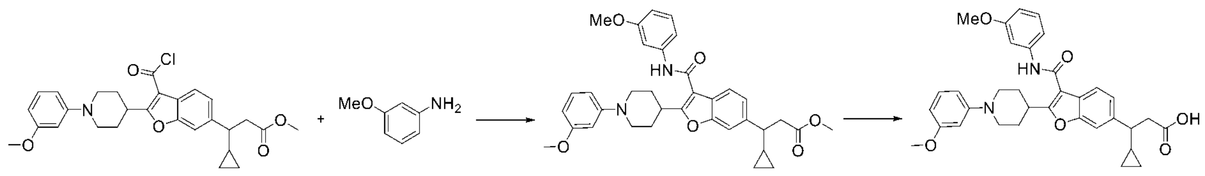

235-1 245-1 245-2 245

Referring to the synthetic route of compound **235,** compound **235-2** was replaced with compound **245-1** to carry out the synthesis. The resulting crude product was purified by reverse-phase column chromatography [acetonitrile/water (0.05% formic acid) = 0% to 100%] to obtain compound **245** (11 mg, yield: 30%) as a white solid. MS (ESI, m/z): 569.2 $[M+H]^+$.

$^1$H NMR (400 MHz, MeOD) δ 7.47 (d, *J* = 8.2 Hz, 1H), 7.36 - 7.26 (m, 2H), 7.19 - 7.07 (m, 3H), 7.03 - 6.99 (m, 1H), 6.63 - 6.57 (m, 1H), 6.51 - 6.48 (m, 1H), 6.44 - 6.42 (m, 1H), 6.31 - 6.29 (m, 1H), 3.73 - 3.62 (m, 8H), 3.43 - 3.35 (m, 1H), 2.74 - 2.71 (m, 2H), 2.60 - 2.35 (m, 3H), 2.09 - 1.98 (m, 2H), 1.93 - 1.90 (m, 2H), 0.96 - 0.93 (m, 1H), 0.50 - 0.41 (m, 1H), 0.30 - 0.18 (m, 2H), 0.03 - 0.05 (m, 1H).

**Example 246:**

Synthetic Route:

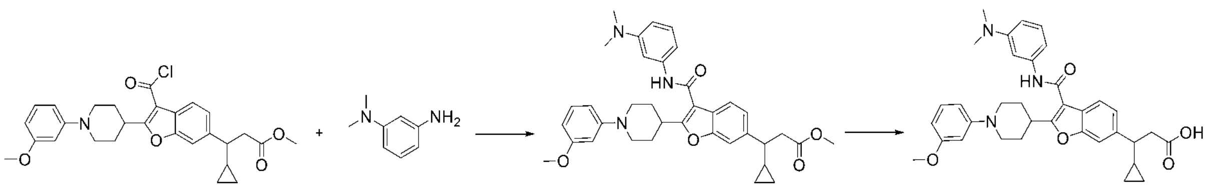

235-1 246-1 246-2 246

Referring to the synthetic route of compound **235,** compound **235-2** was replaced with compound **246-1** to carry out the synthesis. The resulting crude product was purified by reverse-phase column chromatography [acetonitrile/water (0.05% formic acid) = 0% to 100%] to obtain compound **246** (16 mg, yield: 46%) as a white solid. MS (ESI, m/z): 582.2 $[M+H]^+$.

$^1$H NMR (400 MHz, $CDCl_3$) δ 7.72 - 7.58 (m, 2H), 7.43 (s, 1H), 7.26 - 7.15 (m, 4H), 6.94 - 6.84 (m, 1H), 6.71 - 6.44 (m, 4H), 3.85 - 3.76 (m, 6H), 3.00 (s, 6H), 2.97 - 2.85 (m, 4H), 2.55 - 2.45 (m, 1H), 2.34 - 2.15 (m, 2H), 2.06 - 1.95 (m, 2H), 1.12 - 1.04 (m, 1H), 0.67 - 0.59 (m, 1H), 0.51 - 0.40 (m, 1H), 0.38 - 0.30 (m, 1H), 0.25 - 0.17 (m, 1H).

**Example 247:**

Synthetic Route:

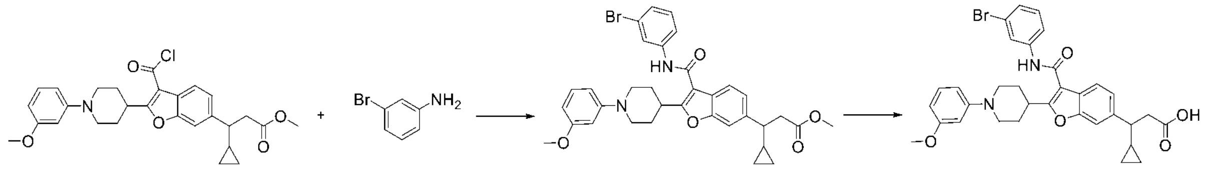

235-1 247-1 247-2 247

Referring to the synthetic route of compound **235,** compound **235-2** was replaced with compound **247-1** to carry out the synthesis. The resulting crude product was purified by reverse-phase column chromatography [acetonitrile/water (0.05% formic acid) = 0% to 100%] to obtain compound **247** (23 mg, yield: 53%) as a white solid. MS (ESI, m/z): 617.2 $[M+H]^+$.

$^1H$ NMR (400 MHz, $CDCl_3$) δ 7.97 (s, 1H), 7.59 - 7.51 (m, 2H), 7.44 (s, 1H), 7.34 - 7.30 (m, 1H), 7.27 - 7.22 (m, 4H), 6.83 - 6.42 (m, 3H), 3.84 - 3.75 (m, 6H), 3.13 - 2.74 (m, 4H), 2.53 - 2.46 (m, 1H), 2.33 - 2.17 (m, 2H), 2.04 - 1.98 (m, 2H), 1.11 - 1.04 (m, 1H), 0.70 - 0.62 (m, 1H), 0.49 - 0.42 (m, 1H), 0.38 - 0.32 (m, 1H), 0.22 - 0.16 (m, 1H).

**Example 248:**

Synthetic Route:

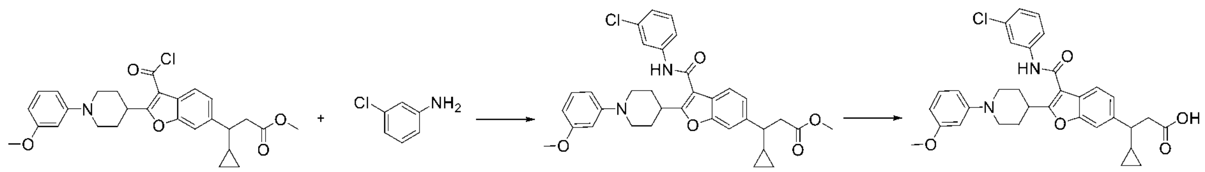

235-1 248-1 248-2 248

Referring to the synthetic route of compound **235,** compound **235-2** was replaced with compound **248-1** to carry out the synthesis. The resulting crude product was purified by reverse-phase column chromatography [acetonitrile/water (0.05% formic acid) = 0% to 100%] to obtain compound **248** (2 mg, yield: 3%) as a white solid. MS (ESI, m/z): 573.2 $[M+H]^+$.

$^1H$ NMR (400 MHz, DMSO-$d_6$) δ 10.39 (s, 1H), 7.98 - 7.92 (m, 1H), 7.69 - 7.63 (m, 1H), 7.61 - 7.55 (m, 2H), 7.41 - 7.35 (m, 1H), 7.29 - 7.23 (m, 1H), 7.20 - 7.14 (m, 1H), 7.14 - 7.09 (m, 1H), 6.60 - 6.55 (m, 1H), 6.51 - 6.47 (m, 1H), 6.39 - 6.33 (m, 1H), 3.90 - 3.77 (m, 2H), 3.72 (s, 3H), 3.49 - 3.46 (m, 1H), 2.86 - 2.78 (m, 2H), 2.77 - 2.69 (m, 2H), 2.42 - 2.35 (m, 1H), 2.00 - 1.91 (m, 4H), 1.11 - 1.03 (m, 1H), 0.56 - 0.48 (m, 1H), 0.32 - 0.24 (m, 2H), 0.18 - 0.11 (m, 1H).

**Example 249:**

Synthetic Route:

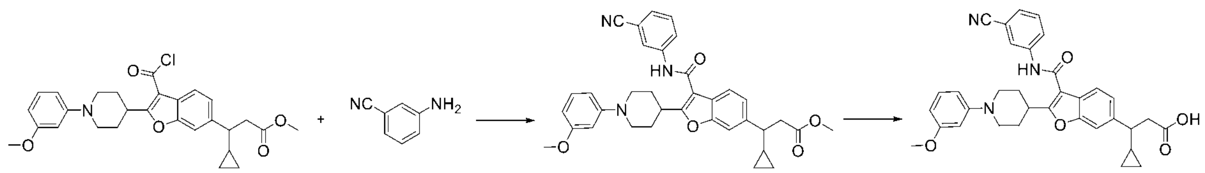

235-1 249-1 249-2 249

Referring to the synthetic route of compound **235,** compound **235-2** was replaced with compound **249-1** to carry out the synthesis. The resulting crude product was purified by reverse-phase column chromatography [acetonitrile/water (0.05% formic acid) = 0% to 100%] to obtain compound **249** (1 mg, yield: 5%) as a white solid. MS (ESI, m/z): 564.2 $[M+H]^+$.

$^1H$ NMR (400 MHz, DMSO-$d_6$) δ 10.53 (s, 1H), 8.23 (s, 1H), 8.06 - 7.97 (m, 1H), 7.66 - 7.53 (m, 4H), 7.26 (d, $J$ = 8.0 Hz, 1H), 7.15 - 7.08 (m, 1H), 6.61 - 6.53 (m, 1H), 6.51 - 6.46 (m, 1H), 6.40 - 6.32 (m, 1H), 3.89 - 3.79 (m, 2H), 3.72 (s, 3H), 3.54 - 3.40 (m, 1H), 2.87 - 2.78 (m, 2H), 2.77 - 2.70 (m, 2H), 2.42 - 2.34 (m, 1H), 2.03 - 1.89 (m, 4H), 1.11 - 1.02 (m, 1H), 0.57 - 0.48 (m, 1H), 0.34 - 0.23 (m, 2H), 0.19 - 0.09 (m, 1H).

**Example 250:**

Synthetic Route:

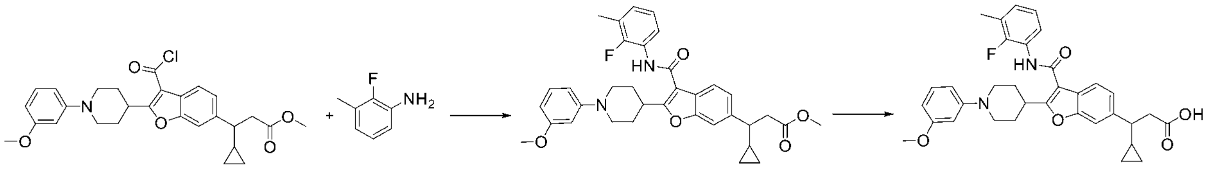

235-1 250-1 250-2 250

Referring to the synthetic route of compound **235,** compound **235-2** was replaced with compound **250-1** to carry out the synthesis. The resulting crude product was purified by reverse-phase column chromatography [acetonitrile/water (0.05% formic acid) = 0% to 100%] to obtain compound **250** (124 mg, yield: 85%) as a white solid. MS (ESI, m/z): 571.2 $[M+H]^+$.

$^1H$ NMR (400 MHz, $CDCl_3$) δ 8.32-8.25 (m, 1H), 8.02-7.97 (m, 1H), 7.70 (d, $J$ = 8.0 Hz, 1H), 7.43 (s, 1H), 7.29-7.25 (m, 1H), 7.23-7.16 (m, 1H), 7.11-7.05 (m, 1H), 6.93-6.99 (m, 1H), 6.73 - 6.35 (m, 3H), 3.93 - 3.75 (m, 6H), 3.02 - 2.77 (m, 4H), 2.58 - 2.47 (m, 1H), 2.33 (s, 3H), 2.28 - 2.12 (m, 2H), 2.08 - 1.95 (m, 2H), 1.14 - 1.04 (m, 1H), 0.68 - 0.57 (m, 1H), 0.53 - 0.45 (m, 1H), 0.40 - 0.30 (m, 1H), 0.26 - 0.16 (m, 1H).

**Example 251:**

Synthetic Route:

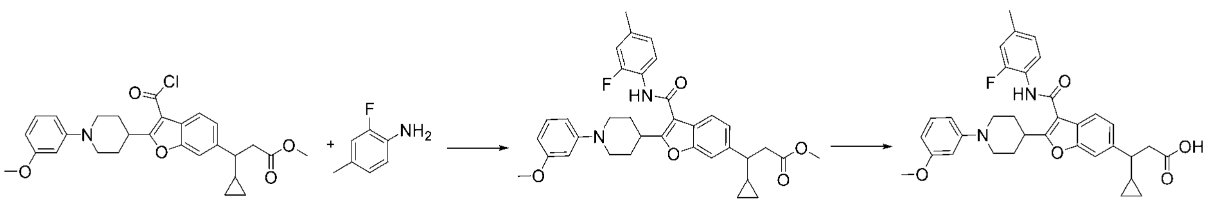

235-1 251-1 251-2 251

Referring to the synthetic route of compound **235,** compound **235-2** was replaced with compound **251-1** to carry out the synthesis. The resulting crude product was purified by reverse-phase column chromatography [acetonitrile/water (0.05% formic acid) = 0% to 100%] to obtain compound **251** (126 mg, yield: 92%) as a white solid. MS (ESI, m/z): 571.2 $[M+H]^+$.

$^1H$ NMR (400 MHz, $CDCl_3$) δ 8.33 - 8.26 (m, 1H), 7.91 (s, 1H), 7.68 (d, $J$ = 8.4 Hz, 1H), 7.43 (s, 1H), 7.29 - 7.24 (m, 1H), 7.23 - 7.16 (m, 1H), 7.08 - 6.95 (m, 2H), 6.77 - 6.33 (m, 3H), 3.96 - 3.77 (m, 6H), 3.08 - 2.76 (m, 4H), 2.57 - 2.46 (m, 1H), 2.35 (s, 3H), 2.28 - 2.12 (m, 2H), 2.08 - 1.96 (m, 2H), 1.15 - 1.07 (m, 1H), 0.70 - 0.59 (m, 1H), 0.54 - 0.43 (m, 1H), 0.38 - 0.29 (m, 1H), 0.24 - 0.15 (m, 1H).

**Example 252:**

Synthetic Route:

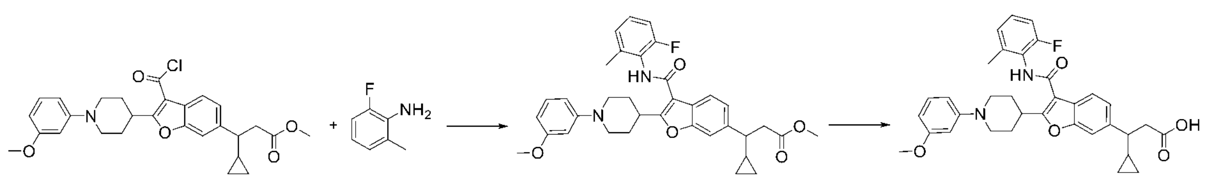

235-1 252-1 252-2 252

Referring to the synthetic route of compound **235,** compound **235-2** was replaced with compound **252-1** to carry out the synthesis. The resulting crude product was purified by reverse-phase column chromatography [acetonitrile/water (0.05% formic acid) = 0% to 100%] to obtain compound **252** (1 mg, yield: 4%) as a white solid. MS (ESI, m/z): 571.2 [M+H]+.

$^1$H NMR (400 MHz, $CDCl_3$) δ 7.74 (d, *J* = 8.0 Hz, 1H), 7.48 (s, 1H), 7.38 - 7.30 (m, 2H), 7.28 - 7.18 (m, 2H), 7.13 (d, *J* = 7.6 Hz, 1H), 7.09 - 7.01 (m, 1H), 6.63 (dd, *J*= 8.0, 2.4 Hz, 1H), 6.58 - 6.53 (m, 1H), 6.45 (dd, *J* = 8.0, 2.4 Hz, 1H), 3.84 - 3.82 (m, 6H), 3.01 - 2.82 (m, 4H), 2.58 - 2.50 (m, 1H), 2.41 (s, 3H), 2.25 - 2.13 (m, 2H), 2.11 - 2.04 (m, 2H), 1.18 - 1.08 (m, 1H), 0.73 - 0.63 (m, 1H), 0.53 - 0.46 (m, 1H), 0.42 - 0.36 (m, 1H), 0.26 - 0.21 (m, 1H).

**Example 253:**

Synthetic Route:

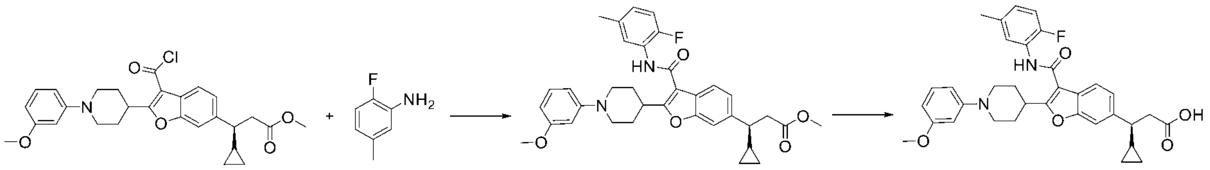

234-1 253-1 253-2 253

Referring to the synthetic route of compound **234,** compound **234-2** was replaced with compound **253-1** to carry out the synthesis. The resulting crude product was purified by reverse-phase column chromatography [acetonitrile/water (0.05% formic acid) = 0% to 100%] to obtain compound **253** (32 mg, yield: 63%) as a white solid. MS (ESI, m/z): 571.2 [M+H]+.

$^1$H NMR (400 MHz, $CDCl_3$) δ 8.30 (d, *J* = 7.6 Hz, 1H), 7.98 - 7.92 (m, 1H), 7.68 (d, *J* = 8.0 Hz, 1H), 7.43 (s, 1H), 7.29 - 7.25 (m, 1H), 7.26 - 7.23 (m, 1H), 7.03 (dd, *J* = 8.0, 7.6 Hz, 1H), 6.93 - 6.85 (m, 1H), 6.73 - 6.35 (m, 3H), 3.93 - 3.76 (m, 6H), 3.02 - 2.77 (m, 4H), 2.53 - 2.46 (m, 1H), 2.37 (s, 3H), 2.28 - 2.12 (m, 2H), 2.08 - 2.00 (m, 2H), 1.15 - 1.04 (m, 1H), 0.68 - 0.59 (m, 1H), 0.51 - 0.41 (m, 1H), 0.39 - 0.30 (m, 1H), 0.23 - 0.15 (m, 1H).

**Example 254:**

Synthetic Route:

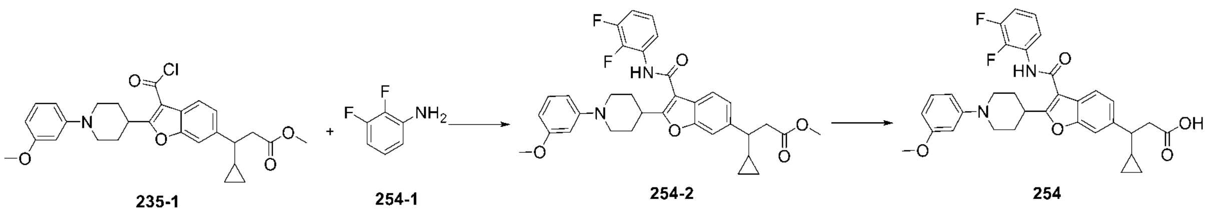

235-1 254-1 254-2 254

Referring to the synthetic route of compound **235,** compound **235-2** was replaced with compound **254-1** to carry out the synthesis. The resulting crude product was purified by reverse-phase column chromatography [acetonitrile/water (0.05% formic acid) = 0% to 100%] to obtain compound **254** (109 mg, yield: 83%) as a white solid. MS (ESI, m/z): 574.9 [M+H]+.

$^1$H NMR (400 MHz, $CDCl_3$) δ 8.30 - 8.19 (m, 1H), 7.98 (s, 1H), 7.67 (d, *J* = 8.0 Hz, 1H), 7.44 (s, 1H), 7.28 (d, *J* = 8.0 Hz, 1H), 7.23 - 7.09 (m, 2H), 7.01 - 6.91 (m, 1H), 6.73 - 6.36 (m, 3H), 3.92 - 3.78 (m, 6H), 3.02 - 2.78 (m, 4H), 2.54 - 2.46 (m, 1H), 2.27 - 2.13 (s, 2H), 2.08 - 1.98 (m, 2H), 1.15 - 1.06 (m, 1H), 0.67 - 0.59 (m, 1H), 0.51 - 0.43 (m, 1H), 0.39 - 0.30 (m, 1H), 0.25 - 0.16 (m, 1H).

**Example 255:**

Synthetic Route:

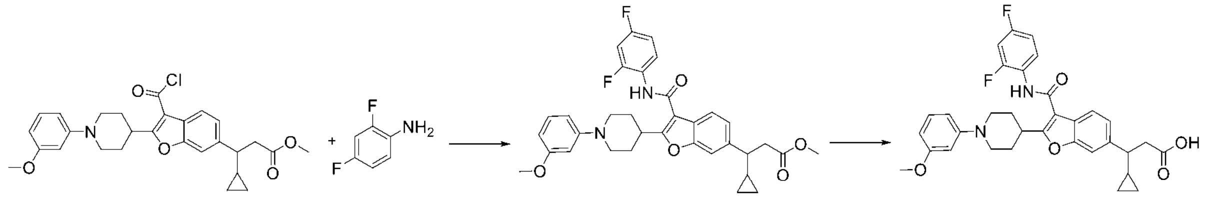

235-1 255-1 255-2 255

Referring to the synthetic route of compound **235,** compound **235-2** was replaced with compound **255-1** to carry out the synthesis. The resulting crude product was purified by reverse-phase column chromatography [acetonitrile/water (0.05% formic acid) = 0% to 100%] to obtain compound **255** (118 mg, yield: 93%) as a white solid. MS (ESI, m/z): 574.9 $[M+H]^+$.

$^1$H NMR (400 MHz, $CDCl_3$) δ 8.46 - 8.38 (m, 1H), 7.86 (s, 1H), 7.66 (d, *J* = 8.0 Hz, 1H), 7.44 (s, 1H), 7.30 - 7.26 (m, 1H), 7.24 - 7.16 (m, 1H), 7.00 - 6.89 (m, 2H), 6.76 - 6.35 (m, 3H), 3.90 - 3.76 (m, 6H), 3.02 - 2.76 (m, 4H), 2.55 - 2.57 (m, 1H), 2.28 - 2.13 (m, 2H), 2.08 - 1.97 (m, 2H), 1.14 - 1.06 (m, 1H), 0.69 - 0.60 (m, 1H), 0.50 - 0.39 (m, 1H), 0.37 - 0.29 (m, 1H), 0.25 - 0.14 (m, 1H).

**Example 256:**

Synthetic Route:

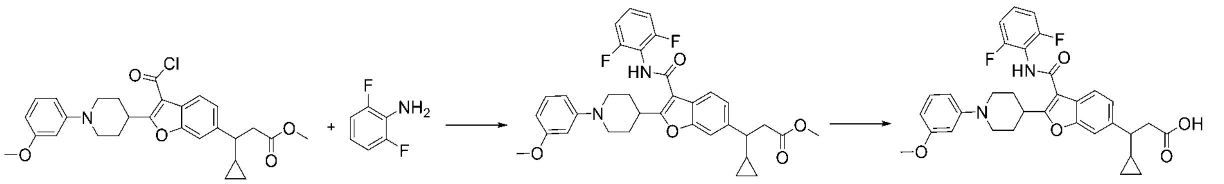

235-1 256-1 256-2 256

Referring to the synthetic route of compound **235,** compound **235-2** was replaced with compound **256-1** to carry out the synthesis. The resulting crude product was purified by reverse-phase column chromatography [acetonitrile/water (0.05% formic acid) = 0% to 100%] to obtain compound **256** (2 mg, yield: 4%) as a white solid. MS (ESI, m/z): 574.9 $[M+H]^+$.

$^1$H NMR (400 MHz, $CDCl_3$) δ 7.73 (d, *J* = 8.0 Hz, 1H), 7.49 - 7.45 (m, 1H), 7.35 - 7.26 (m, 3H), 7.24 - 7.18 (m, 1H), 7.10 - 7.02 (m, 2H), 6.63 (dd, *J* = 8.0, 2.4 Hz, 1H), 6.58 - 6.53 (m, 1H), 6.45 (dd, *J* = 8.0, 2.4 Hz, 1H), 3.84 - 3.81 (m, 6H), 3.01 - 2.83 (m, 4H), 2.58 - 2.49 (m, 1H), 2.25 - 2.17 (m, 2H), 2.08 - 2.06 (m, 2H), 1.16 - 1.08 (m, 1H), 0.71 - 0.65 (m, 1H), 0.53 - 0.46 (m, 1H), 0.43 - 0.34 (m, 1H), 0.26 - 0.20 (m, 1H).

**Example 257:**

Synthetic Route:

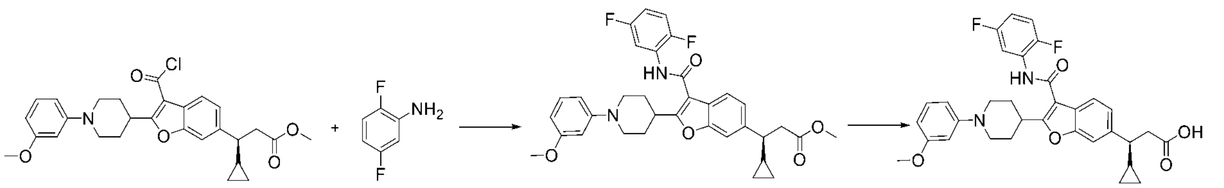

234-1 257-1 257-2 257

Referring to the synthetic route of compound **234,** compound **234-2** was replaced with compound **257-1** to carry out the synthesis. The resulting crude product was purified by reverse-phase column chromatography [acetonitrile/water (0.05% formic acid) = 0% to 100%] to obtain compound **257** (13 mg, yield: 41%) as a white solid. MS (ESI, m/z): 574.9 $[M+H]^+$.

$^1$H NMR (400 MHz, $CDCl_3$) δ 8.37 - 8.31 (m, 1H), 8.02 (s, 1H), 7.66 (d, *J* = 8.0 Hz, 1H), 7.44 (s, 1H), 7.28 (d, *J* = 8.2

Hz, 1H), 7.23 - 7.16 (m, 1H), 7.15 - 7.06 (m, 1H), 6.82 - 6.75 (m, 1H), 6.74 - 6.34 (m, 3H), 3.93 - 3.78 (m, 6H), 3.05 - 2.79 (m, 4H), 2.54 - 2.45 (m, 1H), 2.30 - 2.14 (m, 2H), 2.09 - 1.98 (m, 2H), 1.14 - 1.03 (m, 1H), 0.69 - 0.60 (m, 1H), 0.51 - 0.42 (m, 1H), 0.38 - 0.30 (m, 1H), 0.23 - 0.15 (m, 1H).

**Example 258:**

Synthetic Route:

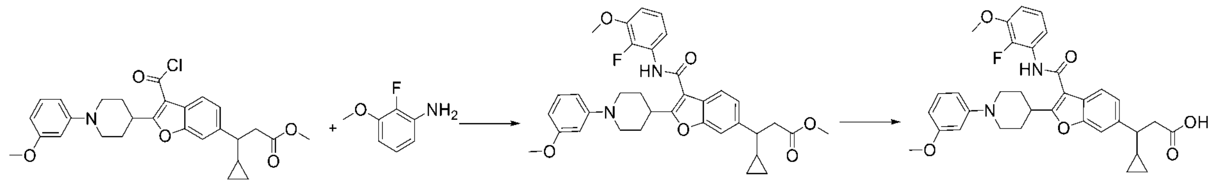

235-1 258-1 258-2 258

Referring to the synthetic route of compound **235,** compound **235-2** was replaced with compound **258-1** to carry out the synthesis. The resulting crude product was purified by reverse-phase column chromatography [acetonitrile/water (0.05% formic acid) = 0% to 100%] to obtain compound **258** (107 mg, yield: 87%) as a white solid. MS (ESI, m/z): 587.2 $[M+H]^+$.

$^1H$ NMR (400 MHz, $CDCl_3$) δ 8.09 - 8.03 (m, 1H), 8.00 - 7.97 (m, 1H), 7.75 - 7.64 (d, $J$ = 8.0 Hz, 1H), 7.43 (s, 1H), 7.28 - 7.24 (m, 2H), 7.23 - 7.17 (m, 1H), 7.15 - 7.08 m, 1H), 6.8 - 6.75 (m, 1H), 6.72 - 6.38 (m, 3H), 3.92 (s, 3H), 3.87 - 3.75 (m, 6H), 3.01 - 2.78 (m, 4H), 2.54 - 2.47 (m, 1H), 2.28 - 2.12 (m, 2H), 2.09 - 1.99 (m, 2H), 1.14 - 1.04 (m, 1H), 0.67 - 0.58 (m, 1H), 0.48 - 0.43 (m, 1H), 0.39 - 0.27 (m, 1H), 0.23 - 0.15 (m, 1H).

**Example 259:**

Synthetic Route:

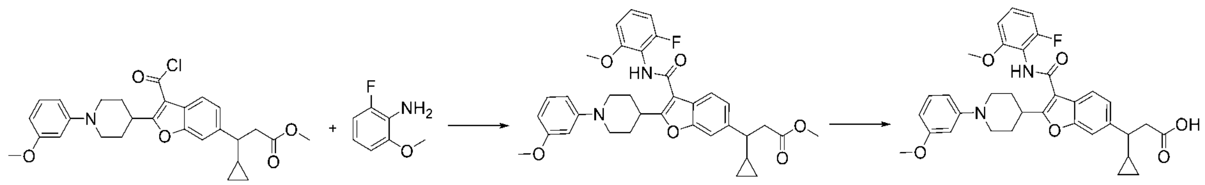

235-1 259-1 259-2 259

Referring to the synthetic route of compound **235,** compound **235-2** was replaced with compound **259-1** to carry out the synthesis. The resulting crude product was purified by reverse-phase column chromatography [acetonitrile/water (0.05% formic acid) = 0% to 100%] to obtain compound **259** (10 mg, yield: 25%) as a white solid. MS (ESI, m/z): 587.2 $[M+H]^+$.

$^1H$ NMR (400 MHz, $CDCl_3$) δ 7.77 (d, $J$ = 8.0 Hz, 1H), 7.46 (s, 1H), 7.36 (s, 1H), 7.30 - 7.15 (m, 3H), 6.92 - 6.84 (m, 1H), 6.79 (d, $J$ = 8.4 Hz, 1H), 6.63 (d, $J$ = 8.6 Hz, 1H), 6.56 (s, 1H), 6.49 - 6.43 (m, 1H), 3.90 (s, 3H), 3.89 - 3.75 (m, 6H), 2.98 - 2.81 (m, 4H), 2.57 - 2.49 (m, 1H), 2.27 - 2.14 (m, 2H), 2.11 - 2.03 (m, 2H), 1.17 - 1.08 (m, 1H), 0.71 - 0.64 (m, 1H), 0.52 - 0.46 (m, 1H), 0.43 - 0.33 (m, 1H), 0.28 - 0.19 (m, 1H).

**Example 260:**

Synthetic Route:

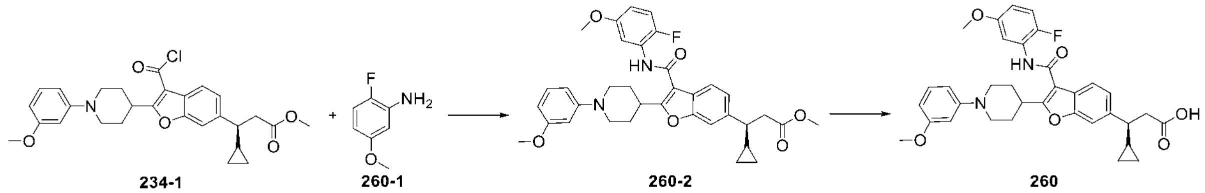

234-1 260-1 260-2 260

Referring to the synthetic route of compound **234,** compound **234-2** was replaced with compound **260-1** to carry out the synthesis. The resulting crude product was purified by reverse-phase column chromatography [acetonitrile/water (0.05% formic acid) = 0% to 100%] to obtain compound **260** (24 mg, yield: 57%) as a white solid. MS (ESI, m/z): 587.2 $[M+H]^+$.

$^1$H NMR (400 MHz, $CDCl_3$) δ 8.17 - 8.12 (m, 1H), 8.00 - 7.96 (m, 1H), 7.68 (d, *J* = 8.0 Hz, 1H), 7.43 (s, 1H), 7.29 - 7.25 (m, 1H), 7.23 - 7.17 (m, 1H), 7.10 - 7.01 (m, 1H), 6.72 - 6.34 (m, 4H), 3.88 - 3.76 (m, 9H), 3.03 - 2.78 (m, 4H), 2.54 - 2.46 (m, 1H), 2.29 - 2.14 (m, 2H), 2.07 - 1.99 (m, 2H), 1.14 - 1.04 (m, 1H), 0.68 - 0.59 (m, 1H), 0.50 - 0.42 (m, 1H), 0.38 - 0.30 (m, 1H), 0.23 - 0.15 (m, 1H).

**Example 261:**

Synthetic Route:

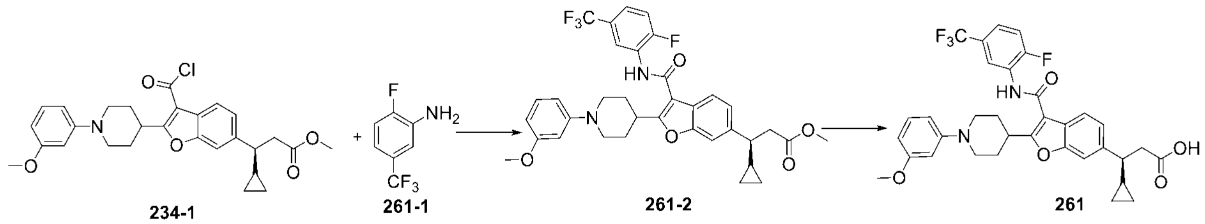

234-1 261-1 261-2 261

Referring to the synthetic route of compound **234,** compound **234-2** was replaced with compound **261-1** to carry out the synthesis. The resulting crude product was purified by reverse-phase column chromatography [acetonitrile/water (0.05% formic acid) = 0% to 100%] to obtain compound **261** (15 mg, yield: 47%) as a white solid. MS (ESI, m/z): 624.9 $[M+H]^+$.

$^1$H NMR (400 MHz, $CDCl_3$) δ 8.94 - 8.86 (m, 1H), 8.11 - 8.02 (m, 1H), 7.66 (d, *J* = 8.0 Hz, 1H), 7.45 (s, 1H), 7.42 - 7.37 (m, 1H), 7.32 - 7.27 (m, 2H), 7.23 - 7.16 (m, 1H), 6.74 - 6.37 (m, 3H), 3.89 - 3.78 (m, 6H), 3.04 - 2.79 (m, 4H), 2.53 - 2.46 (m, 1H), 2.28 - 2.14 (m, 2H), 2.08 - 1.99 (m, 2H), 1.14 - 1.04 (m, 1H), 0.68 - 0.60 (m, 1H), 0.51 - 0.42 (m, 1H), 0.39 - 0.31 (m, 1H), 0.23 - 0.15 (m, 1H).

**Example 262:**

Synthetic Route:

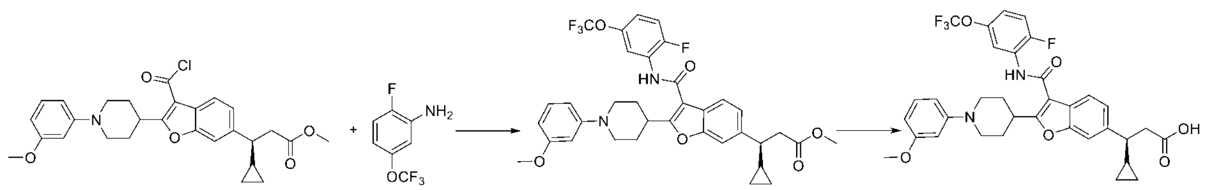

234-1 262-1 262-2 262

Referring to the synthetic route of compound **234,** compound **234-2** was replaced with compound **262-1** to carry out the synthesis. The resulting crude product was purified by reverse-phase column chromatography [acetonitrile/water (0.05% formic acid) = 0% to 100%] to obtain compound **262** (12 mg, yield: 27%) as a white solid. MS (ESI, m/z): 640.9 $[M+H]^+$.

$^1$H NMR (400 MHz, $CDCl_3$) δ 8.57 - 8.48 (m, 1H), 8.03 (s, 1H), 7.65 (d, *J* = 8.0 Hz, 1H), 7.45 (s, 1H), 7.28 (d, *J* = 8.2

Hz, 1H), 7.24 - 7.12 (m, 2H), 7.00 - 6.93 (m, 1H), 6.76 - 6.36 (m, 3H), 3.90 - 3.78 (m, 6H), 3.04 - 2.77 (m, 4H), 2.54 - 2.45 (m, 1H), 2.29 - 2.14 (m, 2H), 2.08 - 1.97 (m, 2H), 1.15 - 1.14 (m 1H), 0.69 - 0.60 (m, 1H), 0.51 - 0.42 (m, 1H), 0.39 - 0.31 (m, 1H), 0.23 - 0.15 (m, 1H).

**Example 263:**

Synthetic Route:

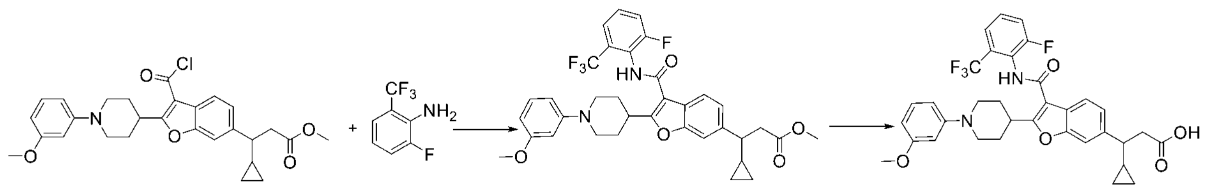

235-1 263-1 263-2 263

Referring to the synthetic route of compound **235,** compound **235-2** was replaced with compound **263-1** to carry out the synthesis. The resulting crude product was purified by reverse-phase column chromatography [acetonitrile/water (0.05% formic acid) = 0% to 100%] to obtain compound **263** (40 mg, yield: 60%) as a white solid. MS (ESI, m/z): 624.9 $[M+H]^+$.

$^1$H NMR (400 MHz, $CDCl_3$) δ 7.71 (d, *J* = 8.0 Hz, 1H), 7.61 - 7.55 (m, 1H), 7.50 - 7.42 (m, 4H), 7.32 - 7.30 (m, 1H), 7.24 - 7.19 (m, 1H), 6.68 - 6.62 (m, 1H), 6.60 - 6.55 (m, 1H), 6.49 - 6.44 (m, 1H), 3.87 - 3.77 (m, 6H), 3.00 - 2.84 (m, 4H), 2.58 - 2.48 (m, 1H), 2.28 - 2.14 (m, 2H), 2.09 - 2.04 (m, 2H), 1.17 - 1.09 (m, 1H), 0.73 - 0.63 (m, 1H), 0.55 - 0.47 (m, 1H), 0.42 - 0.34 (m, 1H), 0.26 - 0.17 (m, 1H).

**Example 264:**

Synthetic Route:

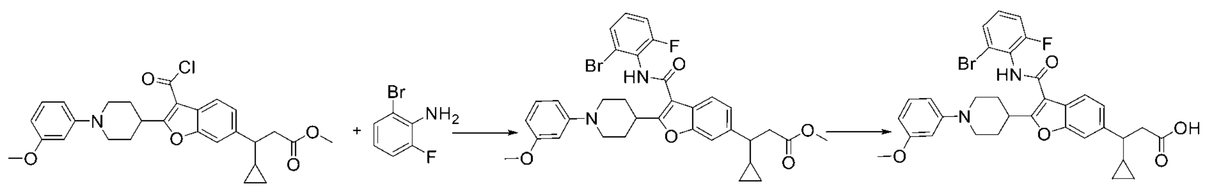

235-1 264-1 264-2 264

Referring to the synthetic route of compound **235,** compound **235-2** was replaced with compound **264-1** to carry out the synthesis. The resulting crude product was purified by reverse-phase column chromatography [acetonitrile/water (0.05% formic acid) = 0% to 100%] to obtain compound **264** (16 mg, yield: 73%) as a white solid. MS (ESI, m/z): 635.2 $[M+H]^+$.

$^1$H NMR (400 MHz, $CDCl_3$) δ 7.80 (d, *J* = 8.0 Hz, 1H), 7.54 - 7.45 (m, 3H), 7.33 - 7.30 (m, 1H), 7.28 - 7.19 (m, 3H), 6.88 - 6.42 (m, 3H), 3.93 - 3.79 (m, 6H), 3.04 - 2.84 (m, 4H), 2.57 - 2.53 (m, 1H), 2.43 - 2.25 (m, 2H), 2.14 - 2.07 (m, 2H), 1.18 - 1.09 (m, 1H), 0.72 - 0.64 (m, 1H), 0.53 - 0.47 (m, 1H), 0.42 - 0.35 (m, 1H), 0.26 - 0.19 (m, 1H).

**Example 265:**

Synthetic Route:

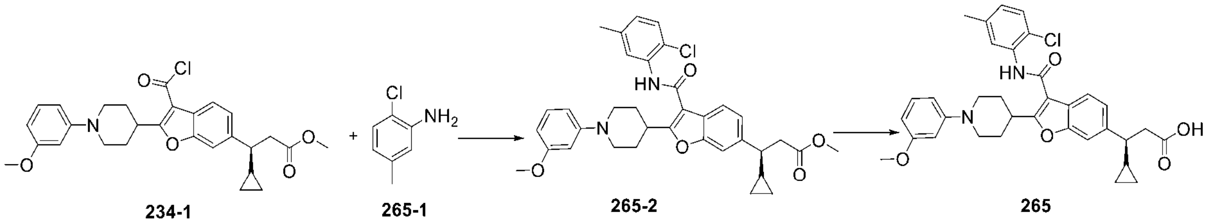

234-1 265-1 265-2 265

Referring to the synthetic route of compound **234,** compound **234-2** was replaced with compound **265-1** to carry out the synthesis. The resulting crude product was purified by reverse-phase column chromatography [acetonitrile/water (0.05% formic acid) = 0% to 100%] to obtain compound **265** (21 mg, yield: 44%) as a white solid. MS (ESI, m/z): 586.9 $[M+H]^+$.

$^1$H NMR (400 MHz, $CDCl_3$) δ 8.46 (s, 1H), 8.33 (s, 1H), 7.82 (d, *J* = 8.2 Hz, 1H), 7.48 (s, 1H), 7.36 - 7.29 (m, 2H), 7.27 - 7.22 (m, 1H), 6.91 (d, *J* = 8.4 Hz, 1H), 6.66 - 6. -59 (m, 1H), 6.55 (s, 1H), 6.47 - 6.41 (m, 1H), 3.96 - 3.81 (m, 6H), 3.10 - 2.82 (m, 4H), 2.58 - 2.50 (m, 1H), 2.42 (s, 3H), 2.37 - 2.16 (m, 2H), 2.14 - 2.15 (m, 2H), 1.17 - 1.08 (m, 1H), 0.71 - 0.63 (m, 1H), 0.53 - 0.47 (m, 1H), 0.41 - 0.34 (m, 1H), 0.26 - 0.18 (m, 1H).

**Example 266:**

Synthetic Route:

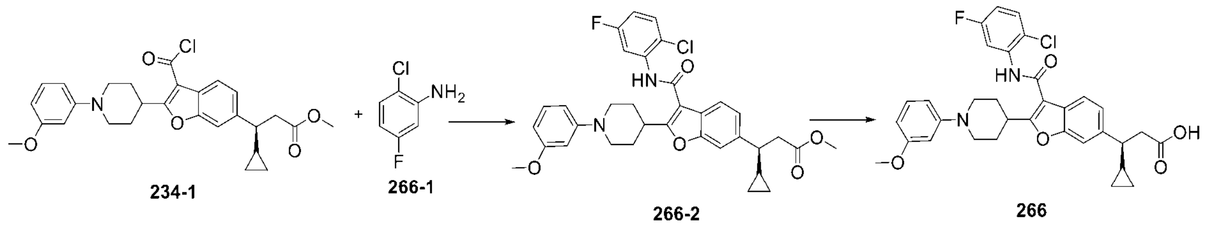

234-1 266-1 266-2 266

Referring to the synthetic route of compound **234,** compound **234-2** was replaced with compound **266-1** to carry out the synthesis. The resulting crude product was purified by reverse-phase column chromatography [acetonitrile/water (0.05% formic acid) = 0% to 100%] to obtain compound **266** (15 mg, yield: 43%) as a white solid. MS (ESI, m/z): 590.9 $[M+H]^+$.

$^1$H NMR (400 MHz, $CDCl_3$) δ 8.50 (dd, *J* = 8.8, 3.2 Hz, 1H), 8.37 (s, 1H), 7.81 (d, *J* = 8.4 Hz, 1H), 7.45 (s, 1H), 7.39 (dd, *J* = 8.4, 3.2 Hz, 1H), 7.28 (d, *J* = 8.0 Hz, 1H), 7.24-6.15 (m, 1H), 6.86-6.78 (m, 1H), 6.78 - 6.29 (m, 3H), 3.91 - 3.77 (m, 6H), 3.04 - 2.78 (m, 4H), 2.54 - 2.46 (m, 1H), 2.29 - 2.15 (m, 2H), 2.09 - 2.0 (m, 2H), 1.15 - 1.03 (m, 1H), 0.69 - 0.60 (m, 1H), 0.53 - 0.42 (m, 1H), 0.39 - 0.31 (m, 1H), 0.24 - 0.16 (m, 1H).

**Example 267:**

Synthetic Route:

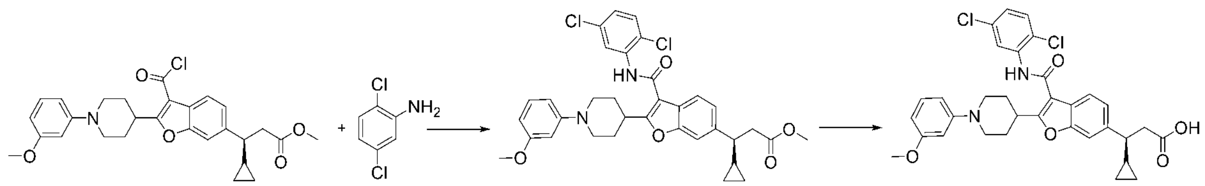

234-1 267-1 267-2 267

Referring to the synthetic route of compound **234,** compound **234-2** was replaced with compound **267-1** to carry out the synthesis. The resulting crude product was purified by reverse-phase column chromatography [acetonitrile/water (0.05% formic acid) = 0% to 100%] to obtain compound **267** (12 mg, yield: 33%) as a white solid. MS (ESI, m/z): 606.9 $[M+H]^+$.

$^1$H NMR (400 MHz, $CDCl_3$) δ 8.75 - 8.71 (m, 1H), 8.32 (s, 1H), 7.79 (d, *J* = 8.0 Hz, 1H), 7.44 (s, 1H), 7.36 (d, *J*= 9.2 Hz, 1H), 7.28 (d, *J*= 9.2 Hz, 1H), 7.23 - 7.16 (m, 1H), 7.08 (dd, *J*= 8.0, 2.4 Hz, 1H), 6.72 - 6.35 (m, 3H), 3.91 - 2.78 (m, 6H), 3.02 - 2.78 (m, 4H), 2.54 - 2.47 (m, 1H), 2.28 - 2.14 (m, 2H), 2.08 - 1.98 (m, 2H), 1.14 - 1.04 (m, 1H), 0.69 - 0.59 (m, 1H), 0.51 - 0.42 (m, 1H), 0.38 - 0.31 (m, 1H), 0.24 - 0.15 (m, 1H).

**Example 268:**

Synthetic Route:

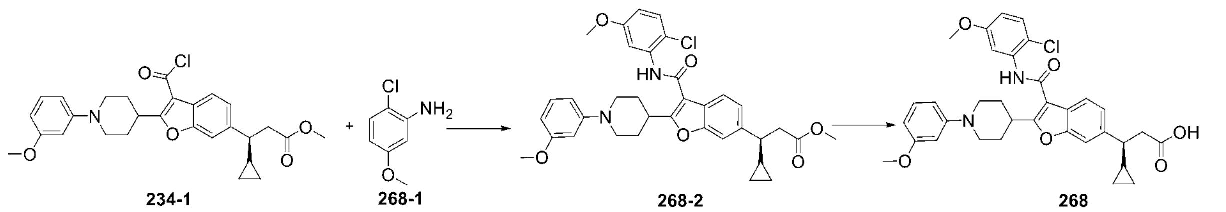

234-1 268-1 268-2 268

Referring to the synthetic route of compound **234,** compound **234-2** was replaced with compound **268-1** to carry out the synthesis. The resulting crude product was purified by reverse-phase column chromatography [acetonitrile/water (0.05% formic acid) = 0% to 100%] to obtain compound **268** (27 mg, yield: 45%) as a white solid. MS (ESI, m/z): 602.9 $[M+H]^+$.

$^1$H NMR (400 MHz, $CDCl_3$) δ 8.35 - 8.27 (m, 2H), 7.83 (d, *J* = 8.4 Hz, 1H), 7.43 (s, 1H), 7.30 (d, *J* = 8.4 Hz, 1H), 7.29 - 7.26 (m, 1H), 7.23 - 7.16 (m, 1H), 6.72 - 6.38 (m, 4H), 3.90 - 3.76 (m, 9H), 3.01 - 2.79 (m, 4H), 2.54 - 2.46 (m, 1H), 2.29 - 2.13 (m, 2H), 2.08 - 2.00 (m, 2H), 1.15 - 1.04 (m, 1H), 0.69 - 0.59 (m, 1H), 0.51 - 0.42 (m, 1H), 0.39 - 0.30 (m, 1H), 0.24 - 0.15 (m, 1H).

**Example 269:**

Synthetic Route:

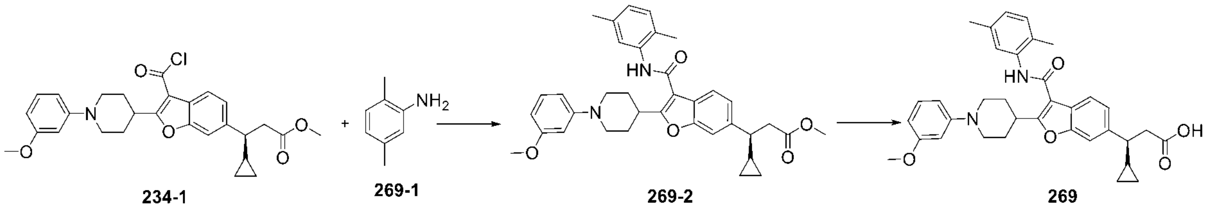

234-1 269-1 269-2 269

Referring to the synthetic route of compound **234,** compound **234-2** was replaced with compound **269-1** to carry out the synthesis. The resulting crude product was purified by reverse-phase column chromatography [acetonitrile/water (0.05% formic acid) = 0% to 100%] to obtain compound **269** (66 mg, yield: 79%) as a white solid. MS (ESI, m/z): 567.3 $[M+H]^+$.

$^1$H NMR (400 MHz, $CDCl_3$) δ 7.84 (s, 1H), 7.65 (d, *J* = 8.0 Hz, 1H), 7.54 (s, 1H), 7.44 (s, 1H), 7.25 - 7.22 (m, 1H), 7.22 - 7.16 (m, 1H), 7.13 (d, *J* = 7.6 Hz, 1H), 6.95 (d, *J* = 7.6 Hz, 1H), 6.72 - 6.34 (m, 3H), 3.94 - 3.77 (m, 6H), 3.02 - 3.80 (m, 4H), 2.55 - 2.45 (m, 1H), 2.37 (s, 3H), 2.31 (s, 3H), 2.27 - 2.10 (m, 2H), 2.08 - 1.97 (m, 2H), 1.15 - 1.02 (m, 1H), 0.71 - 0.58 (m, 1H), 0.51 - 0.42 (m, 1H), 0.39 - 0.31 (m, 1H), 0.23 - 0.15 (m, 1H).

**Example 270:**

Synthetic Route:

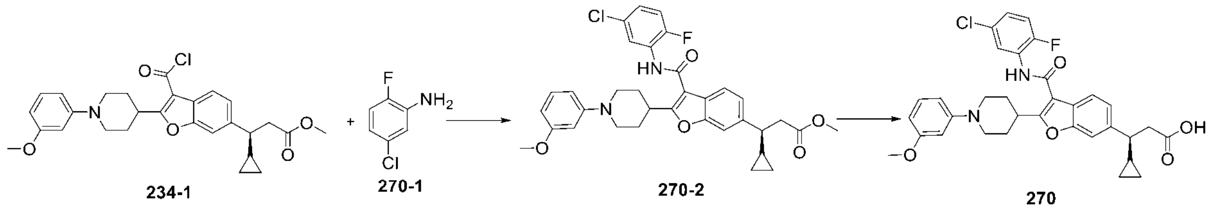

234-1 270-1 270-2 270

Referring to the synthetic route of compound **234,** compound **234-2** was replaced with compound **270-1** to carry out the synthesis. The resulting crude product was purified by reverse-phase column chromatography [acetonitrile/water (0.05% formic acid) = 0% to 100%] to obtain compound **270** (43 mg, yield: 59%) as a white solid. MS (ESI, m/z): 591.1 $[M+H]^+$.

$^1$H NMR (400 MHz, $CDCl_3$) δ 8.63 - 8.57 (m, 1H), 8.00 - 7.95 (m, 1H), 7.64 (d, *J* = 8.0 Hz, 1H), 7.46 (s, 1H), 7.30 - 7.25 (m, 1H), 7.25 - 7.18 (m, 1H), 7.13 - 7.04 (m, 2H), 6.74 - 6.35 (m, 3H), 3.91 - 3.77 (m, 6H), 3.11 - 2.79 (m, 4H), 2.58 - 2.45 (m, 1H), 2.36 - 2.14 (m, 2H), 2.12 - 2.02 (m, 2H), 1.17 - 1.03 (m, 1H), 0.72 - 0.59 (m, 1H), 0.54 - 0.42 (m, 1H), 0.40 - 0.31 (m, 1H), 0.27 - 0.15 (m, 1H).

**Example 271:**

Synthetic Route:

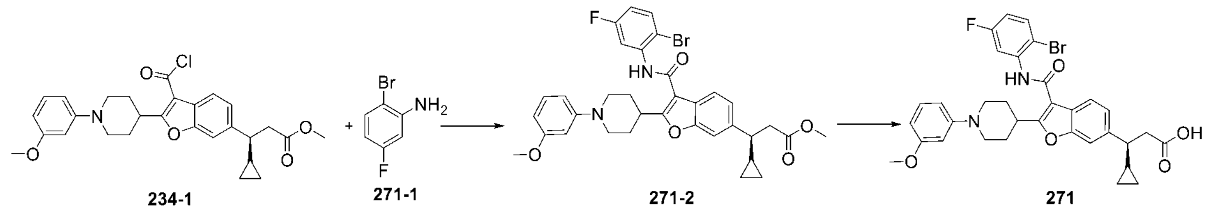

234-1 271-1 271-2 271

Referring to the synthetic route of compound **234,** compound **234-2** was replaced with compound **271-1** to carry out the synthesis. The resulting crude product was purified by reverse-phase column chromatography [acetonitrile/water (0.05% formic acid) = 0% to 100%] to obtain compound **271** (34 mg, yield: 55%) as a white solid. MS (ESI, m/z): 635.1 $[M+H]^+$.

$^1$H NMR (400 MHz, $CDCl_3$) δ 8.52 - 8.46 (m, 1H), 8.37 (s, 1H), 7.88 (d, *J* = 8.0 Hz, 1H), 7.69 - 7.52 (m, 1H), 7.46 (s, 1H), 7.33 - 7.18 (m, 2H), 6.81 - 6.75 (m, 1H), 6.72 - 6.36 (m, 3H), 3.96 - 3.78 (m, 6H), 3.04 - 2.78 (m, 4H), 2.56 - 2.46 (m, 1H), 2.28 - 2.12 (m, 2H), 2.09 - 1.99 (m, 2H), 1.14 - 1.05 (m, 1H), 0.69 - 0.60 (m 1H), 0.51 - 0.42 (m, 1H), 0.39 - 0.31 (m, 1H), 0.24 - 0.15 (m, 1H).

**Example 272:**

Synthetic Route:

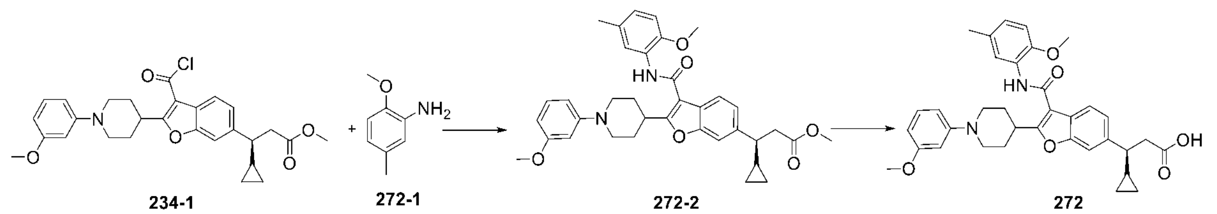

234-1 272-1 272-2 272

Referring to the synthetic route of compound **234,** compound **234-2** was replaced with compound **272-1** to carry out the synthesis. The resulting crude product was purified by reverse-phase column chromatography [acetonitrile/water (0.05% formic acid) = 0% to 100%] to obtain compound **272** (86 mg, yield: 87%) as a white solid. MS (ESI, m/z): 583.3 $[M+H]^+$.

$^1$H NMR (400 MHz, $CDCl_3$) δ 8.46 (s, 1H), 8.37 (s, 1H), 7.77 (d, *J* = 8.0 Hz, 1H), 7.42 (s, 1H), 7.25 (d, *J* = 6.4 Hz,

1H), 7.22 - 7.16 (m, 1H), 6.89 (d, *J* = 6.4 Hz, 1H), 6.83 (d, *J* = 8.0 Hz, 1H), 6.72 - 6.36 (m, 3H), 3.95 - 3.76 (m, 9H), 3.04 - 2.78 (m, 4H), 2.54 - 2.46 (m, 1H), 2.35 (s, 3H), 2.28 - 2.12 (m, 2H), 2.08 - 1.98 (m, 2H), 1.14 - 1.05 (m, 1H), 0.68 - 0.59 (m, 1H), 0.51 - 0.42 (m, 1H), 0.38 - 0.30 (m, 1H), 0.23 - 0.16 (m, 1H).

**Example 273:**

Synthetic Route:

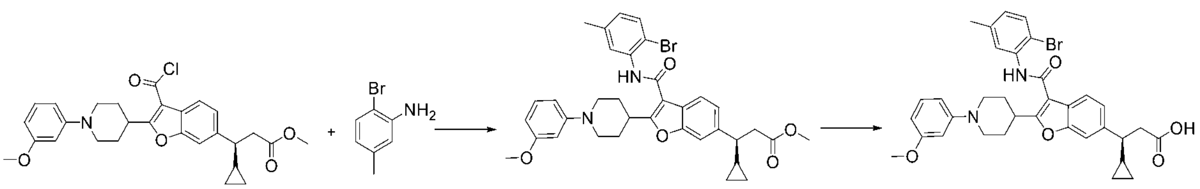

234-1 273-1 273-2 273

Referring to the synthetic route of compound **234,** compound **234-2** was replaced with compound **273-1** to carry out the synthesis. The resulting crude product was purified by reverse-phase column chromatography [acetonitrile/water (0.05% formic acid) = 0% to 100%] to obtain compound **273** (15 mg, yield: 45%) as a white solid. MS (ESI, m/z): 631.1 $[M+H]^+$.

$^1H$ NMR (400 MHz, $CDCl_3$) δ 8.42 (s, 1H), 8.27 (s, 1H), 7.88 (d, *J* = 8.0 Hz, 1H), 7.52 - 7.40 (m, 2H), 7.29 - 7.25 (m, 1H), 7.22 - 7.16 (m, 1H), 6.85 (d, *J* = 8.0 Hz, 1H), 6.67 - 6.37 (m, 3H), 3.89 - 3.77 (m, 6H), 3.01 - 2.79 (m, 4H), 2.55 - 2.46 (m, 1H), 2.38 (s, 3H), 2.27 - 2.13 (m, 2H), 2.09 - 1.98 (m, 2H), 1.15 - 1.04 (m, 1H), 0.70 - 0.59 (m, 1H), 0.53 - 0.44 (m, 1H), 0.38 - 0.30 (m, 1H), 0.24 - 0.15 (m, 1H).

**Example 274:**

Synthetic Route:

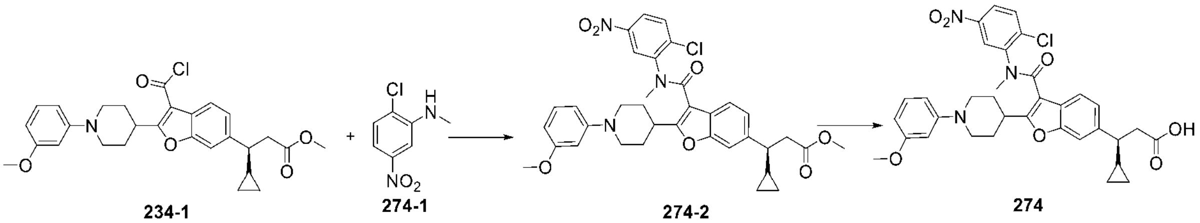

234-1 274-1 274-2 274

Referring to the synthetic route of compound **234,** compound **234-2** was replaced with compound **274-1** to carry out the synthesis. The resulting crude product was purified by reverse-phase column chromatography [acetonitrile/water (0.05% formic acid) = 0% to 100%] to obtain compound **274** (3 mg, yield: 8%) as a white solid. MS (ESI, m/z): 632.2 $[M+H]^+$.

$^1H$ NMR (400 MHz, $CDCl_3$) δ 7.80 - 7.75 (m, 1H), 7.54 - 7.49 (m, 2H), 7.36 (*d*, *J* = 8.4 Hz, 1H), 7.31 (s, 1H), 7.24 - 7.18 (m, 1H), 7.13 (*d, J*= 8.4 Hz, 1H), 6.61 - 6.57 (m, 1H), 6.53 - 6.50 (m, 1H), 6.49 - 6.44 (m, 1H), 4.15 (s, 3H), 3.91 - 3.80 (m, 4H), 3.79 - 3.70 (m, 2H), 2.88 - 2.77 (m, 2H), 2.77 - 2.70 (m, 2H), 2.50 - 2.43 (m, 1H), 2.05 - 1.99 (m, 2H), 1.46 - 1.37 (m, 2H), 1.11 - 1.04 (m, 1H), 0.66 - 0.64 (m, 1H), 0.49 - 0.45 (m, 1H), 0.37 - 0.33 (m, 1H), 0.21 - 0.17 (m, 1H).

**Example 275:**

Synthetic Route:

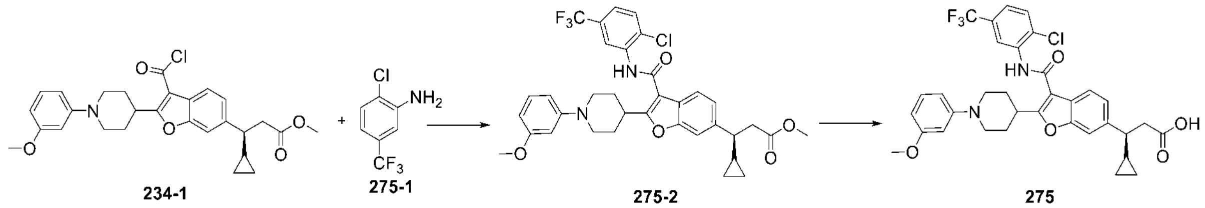

234-1 275-1 275-2 275

Referring to the synthetic route of compound **234,** compound **234-2** was replaced with compound **275-1** to carry out the synthesis. The resulting crude product was purified by reverse-phase column chromatography [acetonitrile/water (0.05% formic acid) = 0% to 100%] to obtain compound **275** (5 mg, yield: 11%) as a white solid. MS (ESI, m/z): 640.9 $[M+H]^+$.

$^1H$ NMR (400 MHz, $CDCl_3$) δ 9.04 (s, 1H), 8.45 (s, 1H), 7.84 (d, *J* = 8.0 Hz, 1H), 7.60 (d, *J*= 8.4 Hz, 1H), 7.49 (s, 1H), 7.39 (d, *J*= 8.4 Hz, 1H), 7.33 (d, *J*= 8.0 Hz, 1H), 7.26 - 7.19 (m, 1H), 6.68 - 6.62 (m, 1H), 6.57 (s, 1H), 6.47 (d, *J* = 8.0 Hz, 1H), 3.88 - 3.84 (m, 6H), 3.03 - 2.84 (m, 4H), 2.59 - 2.50 (*m*, 1H), 2.27 - 2.18 (m, 2H), 2.09 - 2.05 (m, 2H), 1.16 - 1.10 (m, 1H), 0.72 - 0.64 (m, 1H), 0.56 - 0.47 (m, 1H), 0.43 - 0.35 (m, 1H), 0.27 - 0.21 (m, 1H).

**Example 276:**

Synthetic Route:

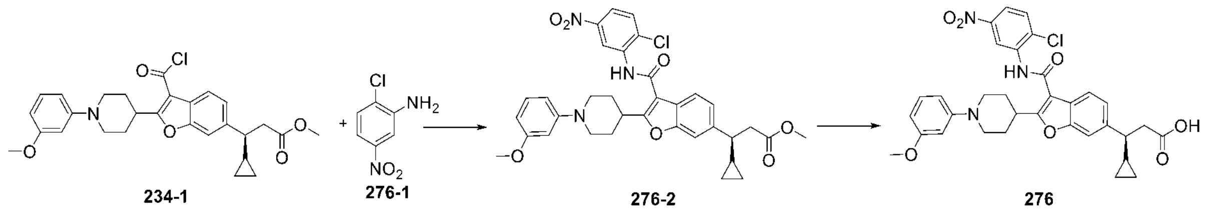

234-1 276-1 276-2 276

Referring to the synthetic route of compound **234,** compound **234-2** was replaced with compound **276-1** to carry out the synthesis. The resulting crude product was purified by reverse-phase column chromatography [acetonitrile/water (0.05% formic acid) = 0% to 100%] to obtain compound **276** (1 mg, yield: 4%) as a white solid. MS (ESI, m/z): 618.2 $[M+H]^+$.

$^1H$ NMR (400 MHz, $CDCl_3$) δ 9.64 - 9.60 (m, 1H), 8.47 (s, 1H), 8.01 (dd, *J* = 8.8, 2.4 Hz, 1H), 7.83 (d, *J* = 8.4 Hz, 1H), 7.65 (d, *J* = 8.8 Hz, 1H), 7.50 (s, 1H), 7.34 (d, *J* = 8.4 Hz, 1H), 7.25 - 7.20 (m, 1H), 6.65 (d, *J* = 8.4 Hz, 1H), 6.58 (s, 1H), 6.51 - 6.45 (m, 1H), 3.92 - 3.83 (m, 6H), 3.01 - 2.87 (m, 4H), 2.60 - 2.54 (m, 1H), 2.27 - 2.19 (m, 2H), 2.10 - 2.05 (m, 2H), 1.19 - 1.09 (m, 1H), 0.73 - 0.61 (m, 1H), 0.55 - 0.48 (m, 1H), 0.45 - 0.35 (m, 1H), 0.28 - 0.21 (m, 1H).

**Example 277:**

Synthetic Route:

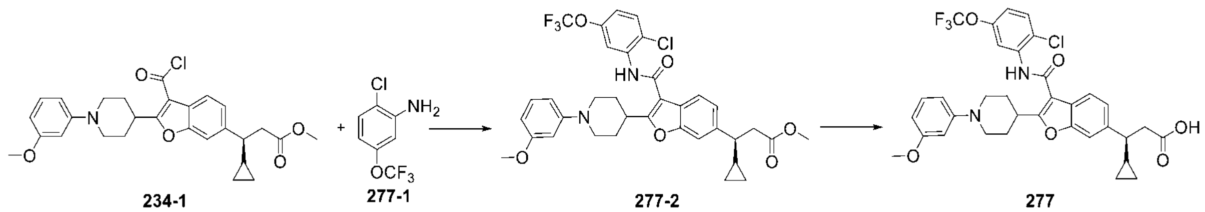

234-1 277-1 277-2 277

Referring to the synthetic route of compound **234,** compound **234-2** was replaced with compound **277-1** to carry out the synthesis. The resulting crude product was purified by reverse-phase column chromatography [acetonitrile/water (0.05% formic acid) = 0% to 100%] to obtain compound **277** (5 mg, yield: 12%) as a white solid. MS (ESI, m/z): 657.2 $[M+H]^+$.

$^1H$ NMR (400 MHz, $CDCl_3$) δ 8.72 - 8.68 (m, 1H), 8.41 (s, 1H), 7.84 (d, *J* = 8.4 Hz, 1H), 7.51 - 7.45 (m, 2H), 7.34 - 7.29 (m, 1H), 7.26 - 7.19 (m, 1H), 7.01 (d, *J* = 8.8 Hz, 1H), 6.65 (d, *J* = 8.4 Hz, 1H), 6.58 (s, 1H), 6.47 (d, *J* = 8.4 Hz, 1H), 3.87

-3.84 (m, 6H), 3.00 - 2.85 (m, 4H), 2.58 - 2.50 (m, 1H), 2.27 -2.18 (m, 2H), 2.08 - 2.05 (m, 2H), 1.16 - 1.06 (m, 1H), 0.72 - 0.63 (m, 1H), 0.53 - 0.47 (m, 1H), 0.41 - 0.35 (m, 1H), 0.26 - 0.20 (m, 1H).

**Example 278:**

Synthetic Route:

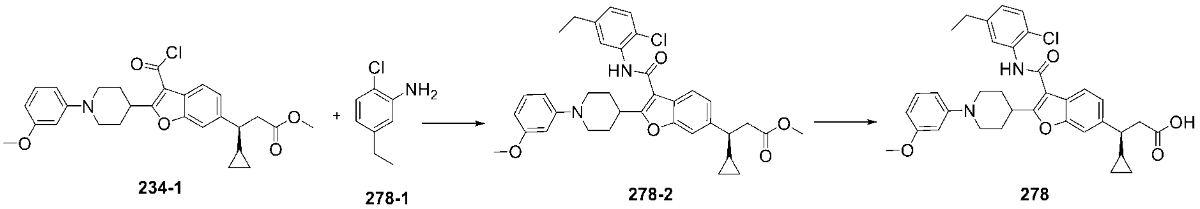

234-1 278-1 278-2 278

Referring to the synthetic route of compound **234,** compound **234-2** was replaced with compound **278-1** to carry out the synthesis. The resulting crude product was purified by reverse-phase column chromatography [acetonitrile/water (0.05% formic acid) = 0% to 100%] to obtain compound **278** (9 mg, yield: 30%) as a white solid. MS (ESI, m/z): 601.2 $[M+H]^+$.

$^1$H NMR (400 MHz, $CDCl_3$) δ 8.52 - 8.49 (m, 1H), 8.33 (s, 1H), 7.86 (d, *J* = 8.4 Hz, 1H), 7.46 (s, 1H), 7.37 (d, *J* = 8.4 Hz, 1H), 7.33 - 7.27 (m, 1H), 7.25 - 7.19 (m, 1H), 6.97 (dd, *J* = 8.4, 2.4 Hz, 1H), 6.65 (dd, *J* = 8.4, 2.4 Hz, 1H), 6.59 - 6.55 (m, 1H), 6.46 (dd, *J* = 8.4, 2.4 Hz, 1H), 3.91 - 3.79 (m, 6H), 3.01 - 2.85 (m, 4H), 2.73 (q, *J* = 7.6 Hz, 2H), 2.57 - 2.51 (m, 1H), 2.27 - 2.19 (m, 2H), 2.12 - 2.02 (m, 2H), 1.30 (t, *J* = 7.2 Hz, 3H), 1.17 - 1.08 (m, 1H), 0.72 - 0.62 (m, 1H), 0.53 - 0.46 (m, 1H), 0.41 - 0.35 (m, 1H), 0.26 - 0.20 (m, 1H).

**Example 279:**

Synthetic Route:

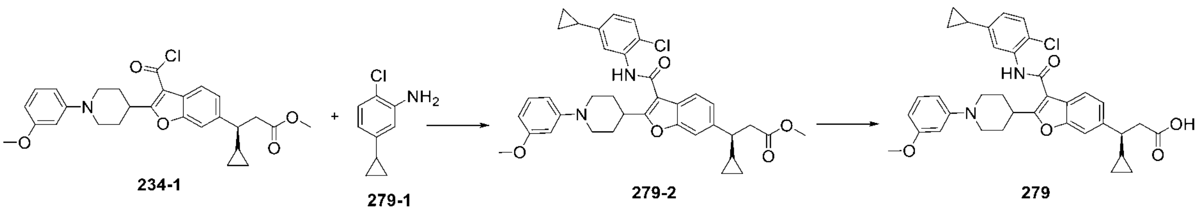

234-1 279-1 279-2 279

Referring to the synthetic route of compound **234,** compound **234-2** was replaced with compound **279-1** to carry out the synthesis. The resulting crude product was purified by reverse-phase column chromatography [acetonitrile/water (0.05% formic acid) = 0% to 100%] to obtain compound **279** (8 mg, yield: 18%) as a white solid. MS (ESI, m/z): 613.2 $[M+H]^+$.

$^1$H NMR (400 MHz, $CDCl_3$) δ 8.43 (s, 1H), 8.31 (s, 1H), 7.85 (d, *J* = 8.4 Hz, 1H), 7.46 (s, 1H), 7.35 - 7.29 (m, 2H), 7.26 - 7.19 (m, 1H), 6.81 (d, *J* = 8.4 Hz, 1H), 6.67 - 6.62 (m, 1H), 6.57 (s, 1H), 6.47 (d, *J* = 8.4 Hz, 1H), 3.91 - 3.83 (m, 6H), 3.00 - 2.83 (m, 4H), 2.58 - 2.51 (m, 1H), 2.30 - 2.16 (m, 2H), 2.11 - 2.03 (m, 2H), 2.02 - 1.94 (m, 1H), 1.17 - 1.08 (m, 1H), 1.06 - 0.99 (m, 2H), 0.81 - 0.75 (m, 2H), 0.72 - 0.61 (m, 1H), 0.53 - 0.46 (m, 1H), 0.44 - 0.34 (m, 1H), 0.25 - 0.20 (m, 1H).

**Example 280:**

Synthetic Route:

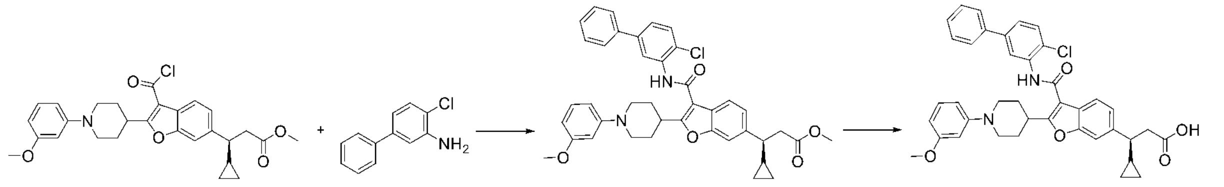

234-1 280-1 280-2 280

Referring to the synthetic route of compound **234,** compound **234-2** was replaced with compound **280-1** to carry out the synthesis. The resulting crude product was purified by reverse-phase column chromatography [acetonitrile/water (0.05% formic acid) = 0% to 100%] to obtain compound **280** (6 mg, yield: 14%) as a white solid. MS (ESI, m/z): 649.2 $[M+H]^+$.

$^1$H NMR (400 MHz, $CDCl_3$) δ 8.95 - 8.92 (m, 1H), 8.40 (s, 1H), 7.89 (d, *J*= 8.4 Hz, 1H), 7.68 (d, *J* = 7.6 Hz, 2H), 7.56 - 7.45 (m, 4H), 7.45 - 7.32 (m, 3H), 7.25 - 7.19 (m, 1H), 6.64 (d, *J* = 8.4 Hz, 1H), 6.56 (s, 1H), 6.46 (d, *J* = 9.6 Hz, 1H), 3.91 - 3.80 (m, 6H), 3.01 - 2.85 (m, 4H), 2.59 - 2.49 (m, 1H), 2.27 - 2.19 (m, 2H), 2.09 - 2.05 (m, 2H), 1.16 - 1.08 (m, 1H), 0.73 - 0.64 (m, 1H), 0.55 - 0.45 (m, 1H), 0.44 - 0.35 (m, 1H), 0.30 - 0.19 (m, 1H).

**Example 281:**

Synthetic Route:

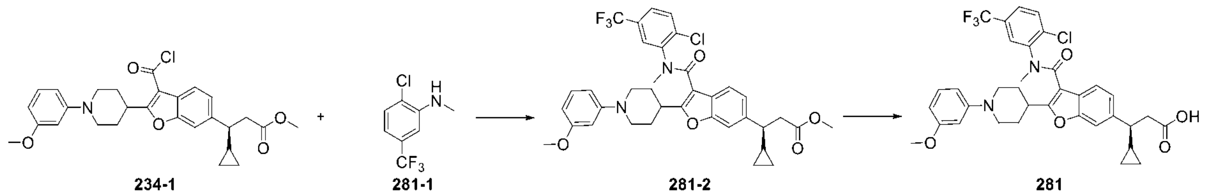

234-1 281-1 281-2 281

Referring to the synthetic route of compound **234,** compound **234-2** was replaced with compound **281-1** to carry out the synthesis. The resulting crude product was purified by reverse-phase column chromatography [acetonitrile/water (0.05% formic acid) = 0% to 100%] to obtain compound **281** (5 mg, yield: 8%) as a white solid. MS (ESI, m/z): 655.2 $[M+H]^+$.

$^1$H NMR (400 MHz, $CDCl_3$) δ 7.46 (d, *J* = 8.0 Hz, 1H), 7.41 (d, *J* = 8.0 Hz, 1H), 7.32 - 7.29 (m, 1H), 7.26 - 7.19 (m, 1H), 7.19 - 7.12 (m, 2H), 6.97 (s, 1H), 6.69 - 6.61 (m, 1H), 6.61 - 6.56 (m, 1H), 6.56 - 6.48 (m, 1H), 4.13 (s, 3H), 3.83 (s, 3H), 3.73 - 3.64 (m, 2H), 3.60 - 3.45 (m, 1H), 2.85 - 2.70 (m, 4H), 2.52 - 2.46 (m, 1H), 2.29 - 2.20 (m, 2H), 2.10 - 2.04 (m, 2H), 1.11 - 1.02 (m, 1H), 0.68 - 0.59 (m, 1H), 0.50 - 0.42 (m, 1H), 0.36 - 0.31 (m, 1H), 0.24 - 0.16 (m, 1H).

**Example 282:**

Synthetic Route:

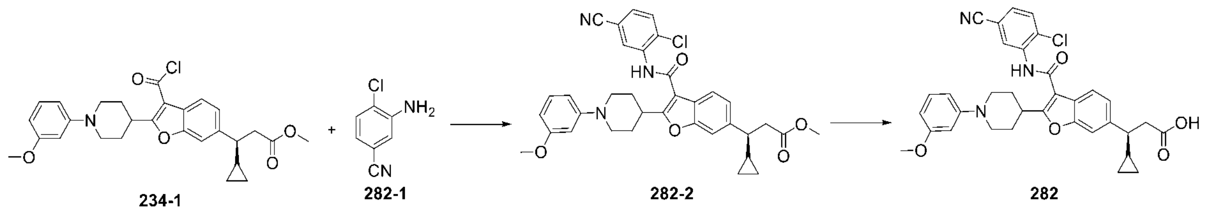

234-1 282-1 282-2 282

Referring to the synthetic route of compound **234,** compound **234-2** was replaced with compound **282-1** to carry out the synthesis. The resulting crude product was purified by reverse-phase column chromatography [acetonitrile/water (0.05% formic acid) = 0% to 100%] to obtain compound **282** (8 mg, yield: 22%) as a white solid. MS (ESI, m/z): 598.2 $[M+H]^+$.

$^1$H NMR (400 MHz, $CDCl_3$) δ 9.09 (d, *J* = 2.0 Hz, 1H), 8.44 (s, 1H), 7.81 (d, *J* = 8.4 Hz, 1H), 7.59 (d, *J* = 8.4 Hz, 1H), 7.49 (s, 1H), 7.41 (dd, *J* = 8.4, 2.0 Hz, 1H), 7.33 (d, *J* = *7.6* Hz, 1H), 7.26 - 7.20 (m, 1H), 6.65 (d, *J* = 7.2 Hz, 1H), 6.58 (s, 1H),

6.48 (d, *J* = 8.4 Hz, 1H), 3.88 - 3.80 (m, 6H), 3.01 - 2.88 (m, 4H), 2.58 - 2.49 (m, 1H), 2.27 - 2.18 (m, 2H), 2.08 - 2.05 (m, 2H), 1.15 - 1.10 (m, 1H), 0.71 - 0.64 (m, 1H), 0.54 - 0.46 (m, 1H), 0.43 - 0.36 (m, 1H), 0.26 - 0.20 (m, 1H).

**Example 283:**

Synthetic Route:

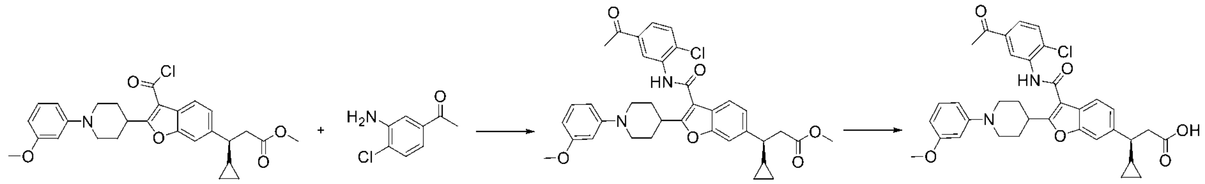

234-1 283-1 283-2 283

Referring to the synthetic route of compound **234,** compound **234-2** was replaced with compound **283-1** to carry out the synthesis. The resulting crude product was purified by reverse-phase column chromatography [acetonitrile/water (0.05% formic acid) = 0% to 100%] to obtain compound **283** (8 mg, yield: 28%) as a white solid. MS (ESI, m/z): 615.2 $[M+H]^+$.

$^1$H NMR (400 MHz, $CDCl_3$) δ 9.27 (s, 1H), 8.40 (s, 1H), 7.85 (d, J = 8.4 Hz, 1H), 7.79 - 7.69 (m, 1H), 7.58 (d, *J* = 8.4 Hz, 1H), 7.49 (s, 1H), 7.39 - 7.31 (m, 1H), 7.27 - 7.19 (m, 1H), 6.65 (d, *J* = 8.4 Hz, 1H), 6.58 (s, 1H), 6.47 (d, *J* = 8.4 Hz, 1H), 3.97 - 3.73 (m, 6H), 3.05 - 2.82 (m, 4H), 2.71 (s, 3H), 2.57 - 2.51(m, 1H), 2.30 - 2.17 (m, 2H), 2.13 - 2.03 (m, 2H), 1.17 - 1.07 (m, 1H), 0.71 - 0.65 (m, 1H), 0.54 - 0.47 (m, 1H), 0.42 - 0.36 (m, 1H), 0.32 - 0.19 (m, 1H).

**Example 284:**

Synthetic Route:

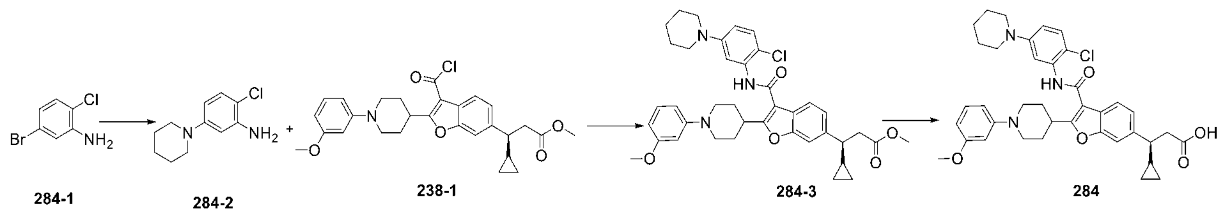

284-1 284-2 238-1 284-3 284

Compound **284-1** (400 mg, 1.93 mmol), piperidine (493 mg, 5.80 mmol), sodium tert-butoxide (557 mg, 5.80 mmol), and 2,2'-bis(diphenylphosphino)-1,1'-binaphthyl (241 mg, 0.39 mmol) were added to toluene (15 mL) and stirred. Tris(dibenzylideneacetone)dipalladium (177 mg, 0.19 mmol) was added, and the reaction was carried out at 100°C under a nitrogen atmosphere for 16 hours. After the reaction was completed, the reaction mixture was concentrated to obtain a crude product. The resulting crude product was then purified by normal-phase column chromatography (petroleum ether: ethyl acetate = 8:2) to obtain another crude product. Another crude product was then purified by reverse-phase column chromatography (water (0.1% formic acid): acetonitrile = 7:3) to obtain compound **284-2** (45 mg, yield: 11%) as a yellow solid. MS (ESI, m/z): 211.2 $[M+H]^+$.

Referring to the synthetic route of compound **234,** compound **234-2** was replaced with compound **284-2** to carry out the synthesis. The resulting crude product was purified by reverse-phase column chromatography [acetonitrile/water (0.05% formic acid) = 0% to 100%] to obtain compound **284** (5 mg, yield: 14%) as a white solid. MS (ESI, m/z): 656.2 $[M+H]^+$.

$^1$H NMR (400 MHz, $CDCl_3$) δ 8.34 - 8.30 (m, 1H), 8.29 (s, 1H), 7.86 (d, *J* = 8.4 Hz, 1H), 7.45 (s, 1H), 7.28 - 7.20 (m, 3H), 6.69 (dd, *J* = 8.8, 2.8 Hz, 1H), 6.64 (d, *J*= 8.4 Hz, 1H), 6.58 - 6.55 (m, 1H), 6.48 - 6.43 (m, 1H), 3.90 - 3.84 (m, 6H), 3.29 - 3.22 (m, 4H), 3.02 - 2.84 (m, 4H), 2.56 - 2.50 (m, 1H), 2.28 - 2.17 (m, 2H), 2.12 - 2.01 (m, 2H), 1.77- 1.72 (m, 4H), 1.64 - 1.60 (m, 2H), 1.15 - 1.05 (m, 1H), 0.71 - 0.62 (m, 1H), 0.52 - 0.46 (m, 1H), 0.40 - 0.35 (m, 1H), 0.26 - 0.20 (m, 1H).

**Example 285:**

Synthetic Route:

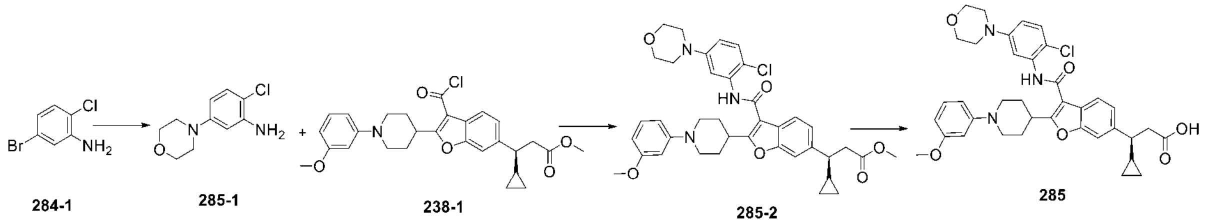

284-1 285-1 238-1 285-2 285

Referring to the synthetic route of compound **284,** piperidine was replaced with morpholine to carry out the synthesis. The resulting crude product was purified by reverse-phase column chromatography [acetonitrile/water (0.05% formic acid) = 0% to 100%] to obtain compound **285** (6 mg, yield: 17%) as a white solid. MS (ESI, m/z): 658.2 $[M+H]^+$.

$^1H$ NMR (400 MHz, $CDCl_3$) δ 8.37 - 8.34 (m, 1H), 8.33 (s, 1H), 7.86 (d, $J$ = 8.4 Hz, 1H), 7.46 (s, 1H), 7.30 - 7.34 (m, 2H), 7.25 - 7.19 (m, 1H), 6.67 - 6.64 (m, 2H), 6.59 - 6.54 (m, 1H), 6.49 - 6.44 (m, 1H), 3.95 - 3.82 (m, 10H), 3.29 - 3.22 (m, 4H), 3.03 - 2.86 (m, 4H), 2.57 - 2.51 (m, 1H), 2.27 - 2.17 (m, 2H), 2.10 - 2.01 (m, 2H), 1.18 - 1.08 (m, 1H), 0.71 - 0.62 (m, 1H), 0.53 - 0.46 (m, 1H), 0.41 - 0.35 (m, 1H), 0.28 - 0.19 (m, 1H).

**Example 286:**

Synthetic Route:

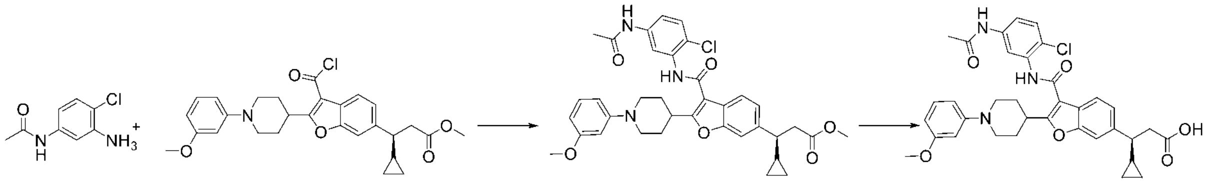

286-1 238-1 286-2 286

Referring to the synthetic route of compound **284,** compound **284-2** was replaced with compound **286-1** to carry out the synthesis. The resulting crude product was purified by reverse-phase column chromatography [acetonitrile/water (0.05% formic acid) = 0% to 100%] to obtain compound **286** (6 mg, yield: 17%) as a white solid. MS (ESI, m/z): 630.2 $[M+H]^+$.

$^1H$ NMR (400 MHz, $CDCl_3$) δ 8.50 - 8.44 (m, 1H), 8.33 (s, 1H), 7.87 - 7.70 (m, 2H), 7.53 (s, 1H), 7.44 (s, 1H), 7.37 (d, $J$ = 8.0 Hz, 1H), 7.30 - 7.25 (m, 1H), 7.23 - 7.17 (m, 1H), 6.76 - 6.36 (m, 3H), 3.87 - 3.73 (m, 6H), 3.00 - 2.77 (m, 4H), 2.55 - 2.43 (m, 1H), 2.30 - 2.20 (m, 2H), 2.17 (s, 3H), 2.09 - 1.98 (m, 2H), 1.14 - 1.04 (m, 1H), 0.68 - 0.59 (m, 1H), 0.51 - 0.41 (m, 1H), 0.39 - 0.30 (m, 1H), 0.25 - 0.15 (m, 1H).

**Example 287:**

Synthetic Route:

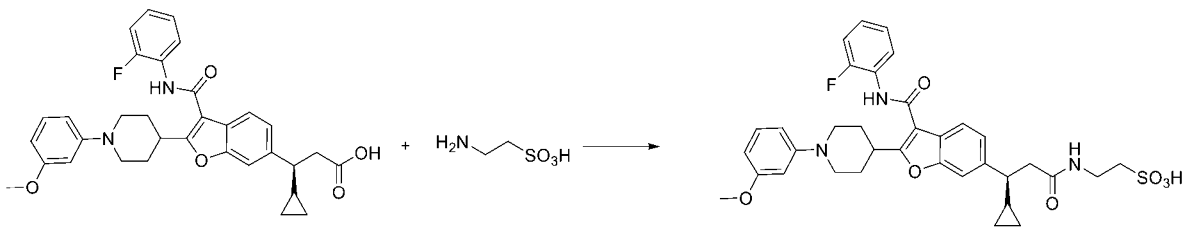

234 287-1 287

To a reaction tube, compound **234** (36 mg, 65 μmol), compound **287-1** (10 mg, 78 μmol), HATU (30 mg, 78 μmol), and DCM (2 mL) were added. The reaction was carried out at room temperature overnight. After the reaction mixture was rotary evaporated to dryness to remove the solvent, the resulting crude product was purified by reverse-phase column chromatography [acetonitrile/water (0.05% formic acid) = 0% to 100%] to obtain compound **287** (31 mg, yield: 69%) as a white solid. MS (ESI, m/z): 663.9 $[M+H]^+$.

$^1H$ NMR (400 MHz, $CDCl_3$) δ 8.29 (m, 2H), 7.61 (d, *J* = 8.0 Hz, 1H), 7.51 (s, 1H), 7.42 - 7.26 (m, 4H), 7.17 - 7.15 (m, 4H), 6.91 (d, *J* = 8.0 Hz, 1H), 4.07 - 3.97 (m, 1H), 3.85 (s, 3H), 3.82 - 3.63 (m, 4H), 3.48 - 3.32 (m, 2H), 3.16 - 2.47 (m, 7H), 2.31 - 2.16 (m, 2H), 1.13 - 1.01 (m, 1H), 0.62 - 0.52 (m, 1H), 0.42 - 0.26 (m, 2H), 0.19 - 0.09 (m, 1H).

**Example 288:**

Synthetic Route:

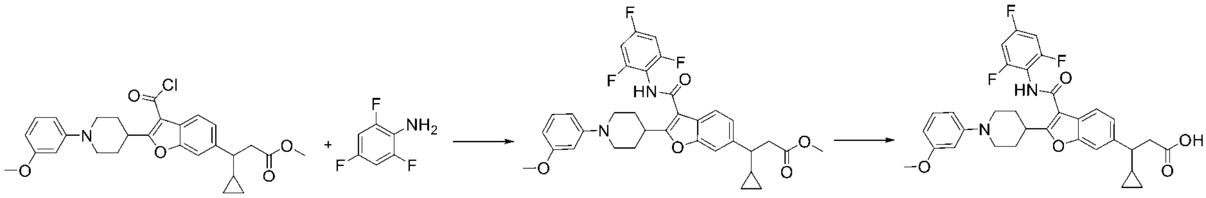

235-1 288-1 288-2 288

Referring to the synthetic route of compound **235,** compound **235-2** was replaced with compound **288-1** to carry out the synthesis. The resulting crude product was purified by reverse-phase column chromatography [acetonitrile/water (0.05% formic acid) = 0% to 100%] to obtain compound **288** (5 mg, yield: 43%) as a white solid. MS (ESI, m/z): 592.9 $[M+H]^+$.

$^1H$ NMR (400 MHz, $CDCl_3$) δ 7.70 (d, *J* = 8.0 Hz, 1H), 7.48 (s, 1H), 7.29 - 7.19 (m, 3H), 6.88 - 6.81 (m, 2H), 6.76 - 6.40 (m, 3H), 3.87 - 3.78 (m, 6H), 2.98 - 2.83 (m, 4H), 2.56 - 2.49 (m, 1H), 2.30 - 2.17 (m, 2H), 2.10 - 2.04 (m, 2H), 1.16 - 1.06 (m, 1H), 0.72 - 0.63 (m, 1H), 0.55 - 0.46 (m, 1H), 0.42 - 0.35 (m, 1H), 0.25 - 0.19 (m, 1H).

**Example 289:**

Synthetic Route:

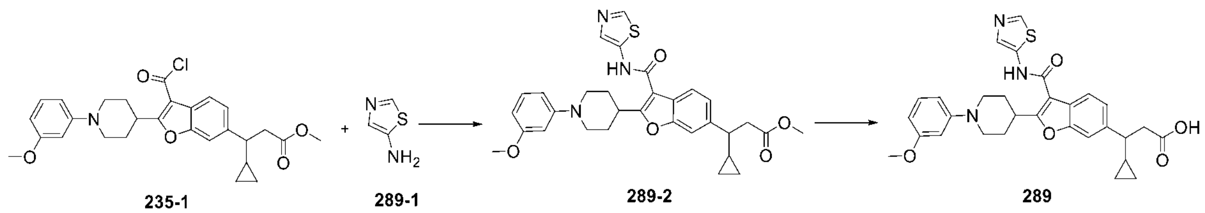

235-1 289-1 289-2 289

Referring to the synthetic route of compound **235,** compound **235-2** was replaced with compound **289-1** to carry out the synthesis. The resulting crude product was purified by reverse-phase column chromatography [acetonitrile/water (0.05% formic acid) = 0% to 100%] to obtain compound **289** (3 mg, yield: 8%) as a white solid. MS (ESI, m/z): 546.2 $[M+H]^+$.

$^1H$ NMR (400 MHz, $CDCl_3$) δ 8.49 (s, 1H), 7.85 -7.70 (m, 1H), 7.55 - 7.30 (m, 2H), 7.22 - 7.07 (m, 2H), 6.57 (d, *J* = 6.2 Hz, 1H), 6.50 (s, 1H), 6.42 (d, *J* = 7.6 Hz, 1H), 3.84 - 3.62 (m, 6H), 3.00 - 2.60 (m, 4H), 2.50 - 2.35 (m, 1H), 2.04 - 1.86 (m, 4H), 1.10 - 1.00 (m, 1H), 0.67 - 0.55 (m, 1H), 0.50 - 0.37 (m, 1H), 0.35 - 0.22 (m, 1H), 0.20 - 0.10 (m, 1H).

**Example 290:**

Synthetic Route:

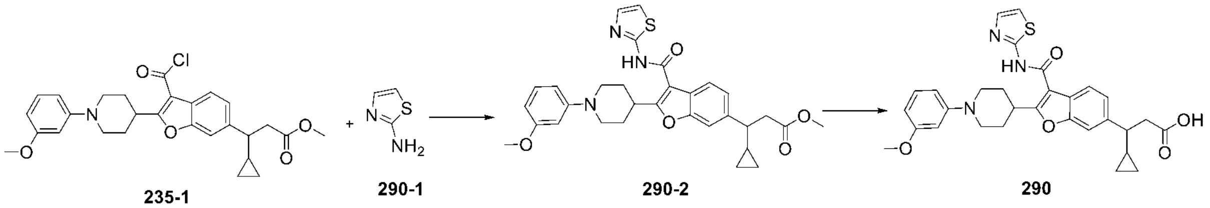

235-1 290-1 290-2 290

Referring to the synthetic route of compound **235,** compound **235-2** was replaced with compound **290-1** to carry out the synthesis. The resulting crude product was purified by reverse-phase column chromatography [acetonitrile/water (0.05% formic acid) = 0% to 100%] to obtain compound **290** (3 mg, yield: 13%) as a white solid. MS (ESI, m/z): 546.2 $[M+H]^+$.

$^1$H NMR (400 MHz, $CD_3OD$) δ 8.46 (s, 1H), 7.77 (d, *J* = 8.0 Hz, 1H), 7.50 (d, *J* = 3.8 Hz, 1H), 7.47 (s, 1H), 7.30 (dd, *J* = 8.2, 1.2 Hz, 1H), 7.20 - 7.11 (m, 2H), 6.64 (dd, *J* = 8.2, 2.0 Hz, 1H), 6.58 - 6.54 (m, 1H), 6.44 (dd, *J* = 8.0, 2.2 Hz, 1H), 3.87 - 3.74 (m, 5H), 3.72 - 3.60 (m, 1H), 2.93 - 2.70 (m, 4H), 2.53 - 2.44 (m, 1H), 2.24 - 2.10 (m, 2H), 2.10 - 2.04 (m, 2H), 1.16 - 1.07 (m, 1H), 0.65 - 0.56 (m, 1H), 0.45 - 0.32 (m, 2H), 0.21 - 0.13 (m, 1H).

**Example 291:**

Synthetic Route:

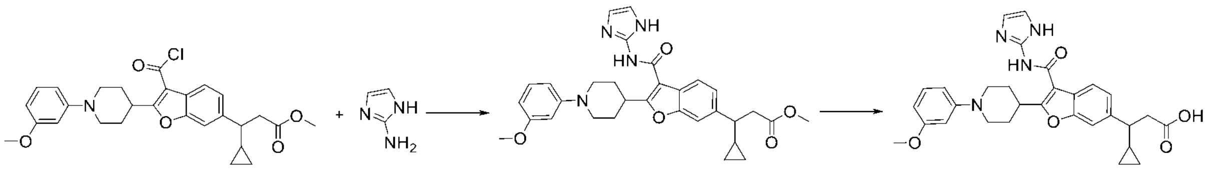

235-1 291-1 291-2 291

Referring to the synthetic route of compound **235,** compound **235-2** was replaced with compound **291-1** to carry out the synthesis. The resulting crude product was purified by reverse-phase column chromatography [acetonitrile/water (0.05% formic acid) = 0% to 100%] to obtain compound **291** (2 mg, yield: 2%) as a white solid. MS (ESI, m/z): 529.2 $[M+H]^+$.

$^1$H NMR (400 MHz, $CDCl_3$) δ 7.66 (d, *J* = 7.6 Hz, 1H), 7.49 (s, 1H), 7.25 - 7.17 (m, 2H), 6.85 (s, 2H), 6.62 (d, *J* = 7.8 Hz, 1H), 6.54 (s, 1H), 6.44 (d, *J*= 8.4 Hz, 1H), 3.86 - 3.80 (m, 5H), 3.71 - 3.62 (m, 1H), 2.92 - 2.85 (m, 3H), 2.78 - 2.70 (m, 1H), 2.58 - 2.50 (m, 1H), 2.24 -2.10 (m, 2H), 2.04 - 1.94 (m, 2H), 1.17 - 1.10 (m, 1H), 0.67 - 0.57 (m, 1H), 0.54 - 0.45 (m, 1H), 0.38 - 0.29 (m, 1H), 0.24 - 0.14 (m, 1H).

**Example 292:**

Synthetic Route:

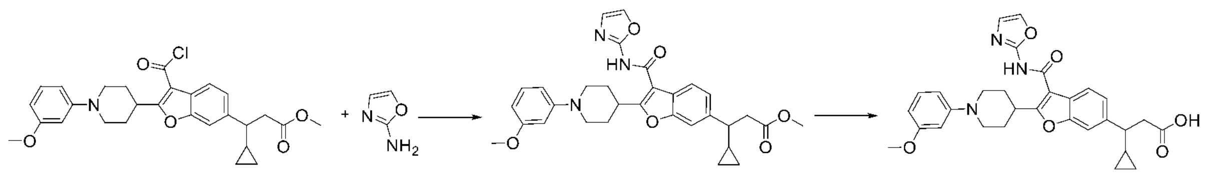

235-1 292-1 292-2 292

Referring to the synthetic route of compound **235,** compound **235-2** was replaced with compound **292-1** to carry out the synthesis. The resulting crude product was purified by reverse-phase column chromatography [acetonitrile/water (0.05% formic acid) = 0% to 100%] to obtain compound **292** (2 mg, yield: 11%) as a white solid. MS (ESI, m/z): 530.2 $[M+H]^+$.

$^1$H NMR (400 MHz, MeOD) δ 7.66 - 7.52 (m, 2H), 7.35 (s, 1H), 7.18 (d, *J* = 8.2 Hz, 1H), 7.08 - 7.02 (m, 2H), 6.53 (dd, *J* = 8.2, 2.0 Hz, 1H), 6.48 - 6.44 (m, 1H), 6.34 (dd, *J* = 8.0, 2.2 Hz, 1H), 3.75 - 3.65 (m, 5H), 3.57 - 3.49 (m, 1H), 2.78 - 2.70 (m, 2H), 2.65 - 2.50 (m, 2H), 2.43 - 2.35 (m, 1H), 2.10 - 2.00 (m, 2H), 1.97 - 1.91 (m, 2H), 1.02 - 0.95(m, 1H), 0.52 - 0.46 (m, 1H),

0.31 - 0.25 (m, 2H), 0.07 - 0.02 (m, 1H).

**Example 293:**

Synthetic Route:

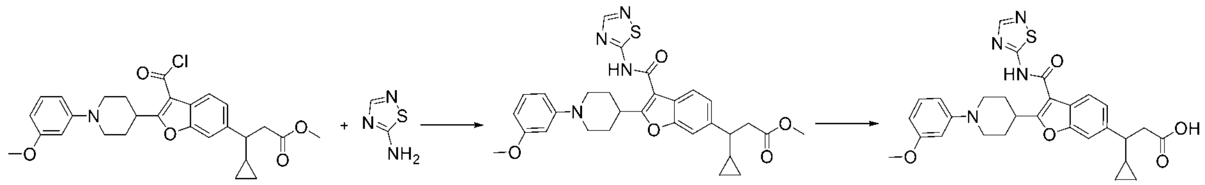

235-1 293-1 293-2 293

Referring to the synthetic route of compound **235,** compound **235-2** was replaced with compound **293-1** to carry out the synthesis. The resulting crude product was purified by reverse-phase column chromatography [acetonitrile/water (0.05% formic acid) = 0% to 100%] to obtain compound **293** (2 mg, yield: 6%) as a white solid. MS (ESI, m/z): 547.2 $[M+H]^+$.

$^1H$ NMR (400 MHz, $CD_3OD$) δ 8.34 (s, 1H), 7.82 (d, *J* = 7.8 Hz, 1H), 7.44 (s, 1H), 7.28 (d, *J* = 7.0 Hz, 1H), 7.16 - 7.09 (m, 1H), 6.62 (dd, *J* = 8.0, 2.0 Hz, 1H), 6.57 - 6.53 (m, 1H), 6.42 (dd, *J* = 8.2, 2.0 Hz, 1H), 4.58 (s, 1H), 3.87 - 3.70 (m, 6H), 2.94 - 2.81 (m, 2H), 2.69 (dd, *J* = 13.8, 6.6 Hz, 1H), 2.63 - 2.57 (m, 1H), 2.52 - 2.45 (m, 1H), 2.21 - 2.07 (m, 2H), 2.07 - 1.98 (m, 2H), 1.10 - 1.03 (m, 1H), 0.61 - 0.53 (m, 1H), 0.40 - 0.31 (m, 2H), 0.15 - 0.08 (m, 1H).

**Example 294:**

Synthetic Route:

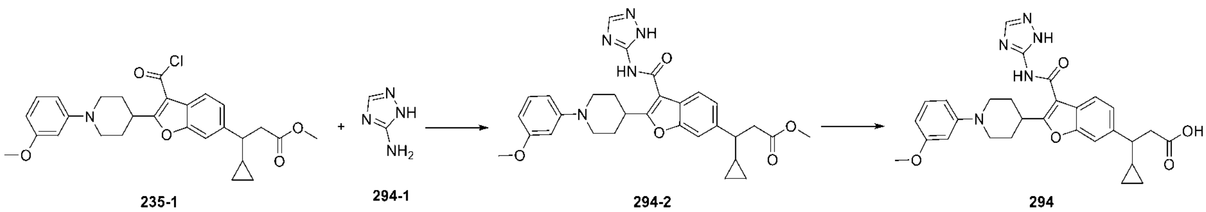

235-1 294-1 294-2 294

Referring to the synthetic route of compound **235,** compound **235-2** was replaced with compound **294-1** to carry out the synthesis. The resulting crude product was purified by reverse-phase column chromatography [acetonitrile/water (0.05% formic acid) = 0% to 100%] to obtain compound **294** (2 mg, yield: 10%) as a white solid. MS (ESI, m/z): 530.2 $[M+H]^+$.

$^1H$ NMR (400 MHz, DMSO-$d_6$) δ 8.16 (s, 1H), 7.55 - 7.46 (m, 1H), 7.30 (s, 1H), 7.15 - 7.06 (m, 2H), 6.59 (dd, *J* = 8.0, 2.0 Hz, 1H), 6.52 - 6.49 (m, 1H), 6.35 (dd, *J* = 8.0, 2.0 Hz, 1H), 3.89 - 3.80 (m, 2H), 3.73 (s, 3H), 3.30 - 3.16 (m, 1H), 2.88 - 2.79 (m, 2H), 2.55 - 2.35 (m, 3H), 2.02 - 1.95 (m, 2H), 1.95 - 1.88 (m, 2H), 0.87 - 0.84 (m, 1H), 0.45 - 0.41 (m, 1H), 0.29 - 0.21 (m, 2H), 0.10 - 0.07 (m, 1H).

**Example 295:**

Synthetic Route:

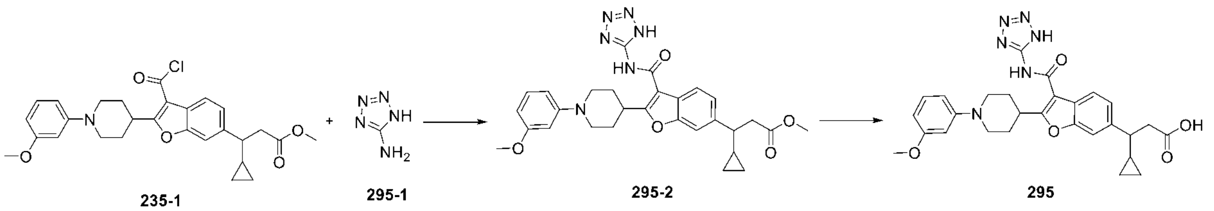

235-1 295-1 295-2 295

Referring to the synthetic route of compound **235,** compound **235-2** was replaced with compound **295-1** to carry out the synthesis. The resulting crude product was purified by reverse-phase column chromatography [acetonitrile/water (0.05% formic acid) = 0% to 100%] to obtain compound **295** (4 mg, yield: 10%) as a white solid. MS (ESI, m/z): 531.2 $[M+H]^+$.

$^1H$ NMR (400 MHz, MeOD) δ 7.73 (d, *J* = 8.0 Hz, 1H), 7.45 (s, 1H), 7.29 (d, *J* = 8.2 Hz, 1H), 7.17 - 7.11 (m, 1H), 6.62 (dd, *J* = 8.0, 1.8 Hz, 1H), 6.58 - 6.53 (m, 1H), 6.43 (dd, *J* = 8.2, 2.0 Hz, 1H), 3.86 - 3.73 (m, 5H), 3.68 - 3.55 (m, 1H), 2.87 - 2.80 (m, 2H), 2.71 - 2.47 (m, 3H), 2.20 - 1.99 (m, 2H), 2.08 - 2.00 (m, 2H), 1.12 - 1.02 (m, 1H), 0.61 - 0.54 (m, 1H), 0.42 - 0.33 (m, 2H), 0.15 - 0.08 (m, 1H).

**Example 296:**

Synthetic Route:

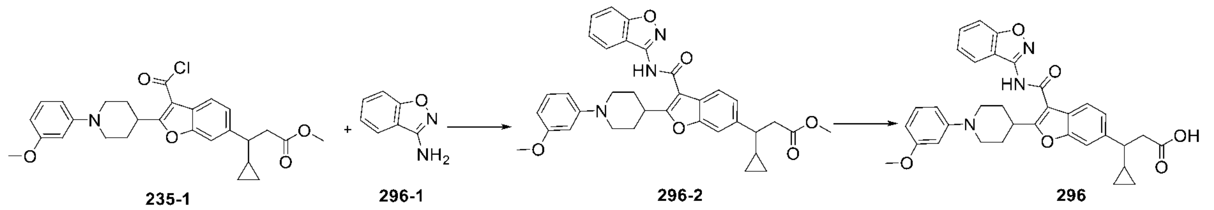

235-1 296-1 296-2 296

Referring to the synthetic route of compound **235,** compound **235-2** was replaced with compound **296-1** to carry out the synthesis. The resulting crude product was purified by reverse-phase column chromatography [acetonitrile/water (0.05% formic acid) = 0% to 100%] to obtain compound **296** (3 mg, yield: 21%) as a white solid. MS (ESI, m/z): 580.2 $[M+H]^+$.

$^1H$ NMR (400 MHz, MeOD) δ 8.11 (dd, *J* = 7.8, 1.6 Hz, 1H), 8.07 (d, *J*= 8.0 Hz, 1H), 7.55 (s, 1H), 7.47 - 7.42 (m, 1H), 7.39 (dd, *J* = 8.2, 1.2 Hz, 1H), 7.19 - 7.13 (m, 1H), 7.08 - 7.02 (m, 2H), 6.66 (dd, *J*= 8.0, 2.0 Hz, 1H), 6.61 - 6.57 (m, 1H), 6.46 (dd, *J* = 7.8, 2.2 Hz, 1H), 4.02 - 3.93 (m, 1H), 3.92 - 3.84 (m, 2H), 3.78 (s, 3H), 2.95 - 2.90 (m, 2H), 2.89 - 2.62 (m, 2H), 2.55 - 2.47 (m, 1H), 2.27 - 2.18 (m, 2H), 2.18 - 2.12 (m, 2H), 1.18 - 1.10 (m, 1H), 0.66 - 0.59 (m, 1H), 0.46 - 0.33 (m, 2H), 0.22 - 0.15 (m, 1H).

**Example 297:**

Synthetic Route:

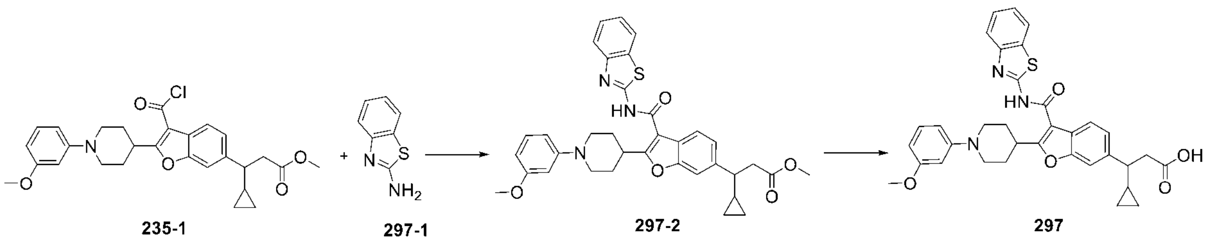

235-1 297-1 297-2 297

Referring to the synthetic route of compound **235,** compound **235-2** was replaced with compound **297-1** to carry out the synthesis. The resulting crude product was purified by reverse-phase column chromatography [acetonitrile/water (0.05% formic acid) = 0% to 100%] to obtain compound **297** (5 mg, yield: 21%) as a white solid. MS (ESI, m/z): 596.2

[M+H]+.

$^1H$ NMR (400 MHz, MeOD) δ 7.89 (d, *J* = 8.0 Hz, 1H), 7.86 - 7.78 (m, 1H), 7.75 (d, *J* = 7.8 Hz, 1H), 7.50 - 7.43 (m, 2H), 7.37 - 7.30 (m, 2H), 7.18 - 7.12 (m, 1H), 6.64 (dd, *J* = 8.2, 2.0 Hz, 1H), 6.60 - 6.56 (m, 1H), 6.44 (dd, *J*= 8.0, 2.0 Hz, 1H), 3.86 - 3.79 (m, 2H), 3.79 - 3.70 (m, 4H), 2.97 - 2.84 (m, 2H), 2.76 - 2.59 (m, 2H), 2.55 - 2.48 (m, 1H), 2.24 - 2.12 (m, 2H), 2.11 - 2.04 (m, 2H), 1.14 - 1.04 (m, 1H), 0.64 - 0.55 (m, 1H), 0.42 - 0.35 (m, 2H), 0.17 - 0.11 (m, 1H).

**Example 298:**

Synthetic Route:

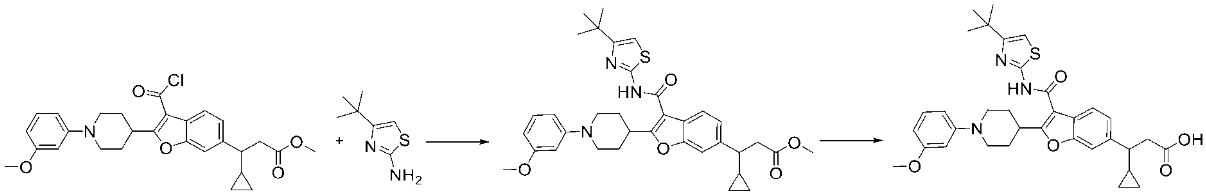

235-1 298-1 298-2 298

Referring to the synthetic route of compound **235,** compound **235-2** was replaced with compound **298-1** to carry out the synthesis. The resulting crude product was purified by reverse-phase column chromatography [acetonitrile/water (0.05% formic acid) = 0% to 100%] to obtain compound **298** (10 mg, yield: 14%) as a white solid. MS (ESI, m/z): 602.2 [M+H]+.

$^1H$ NMR (400 MHz, $CDCl_3$) δ 7.61 (d, *J* = 8.4 Hz, 1H), 7.50 (s, 1H), 7.30 - 7.21 (m, 2H), 7.06 (d, *J* = 8.4 Hz, 1H), 6.88 - 6.42 (m, 3H), 4.02 - 3.66 (m, 6H), 3.20 - 2.95 (m, 2H), 2.91 - 2.82 (m, 1H), 2.79 - 2.66 (m, 1H), 2.60 - 2.50 (m, 1H), 2.46 - 2.22 (m, 2H), 2.17 - 2.02 (m, 2H), 1.41 (s, 9H), 1.18 - 1.06 (m, 1H), 0.68 - 0.55 (m, 1H), 0.52 - 0.42 (m, 1H), 0.42 - 0.31 (m, 1H), 0.16 - 0.13 (m, 1H).

**Example** 299:

Synthetic Route:

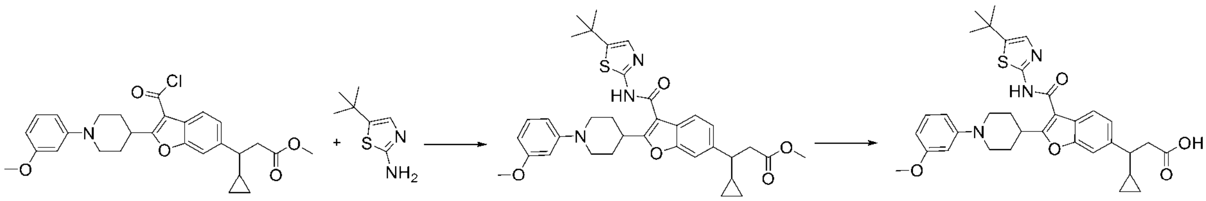

235-1 299-1 299-2 299

Referring to the synthetic route of compound **235,** compound **235-2** was replaced with compound **299-1** to carry out the synthesis. The resulting crude product was purified by reverse-phase column chromatography [acetonitrile/water (0.05% formic acid) = 0% to 100%] to obtain compound **299** (12 mg, yield: 19%) as a white solid. MS (ESI, m/z): 602.2 [M+H]+.

$^1H$ NMR (400 MHz, $CDCl_3$) δ 7.67 (d, *J* = 8.4 Hz, 1H), 7.51 (s, 1H), 7.27 - 7.18 (m, 2H), 7.15 (s, 1H), 6.68 - 6.62 (m, 1H), 6.59 - 6.55 (m, 1H), 6.50 - 6.44 (m, 1H), 3.92 - 3.73 (m, 6H), 3.01 - 2.75 (m, 4H), 2.62 - 2.52 (m, 1H), 2.29 - 2.15 (m, 2H), 2.13 - 2.03 (m, 2H), 1.44 (s, 9H), 1.21 - 1.10 (m, 1H), 0.73 - 0.60 (m, 1H), 0.54 -0.48 (m, 1H), 0.39 -0.34 (m, 1H), 0.26 - 0.17 (m, 1H).

**Example 300:**

Synthetic Route:

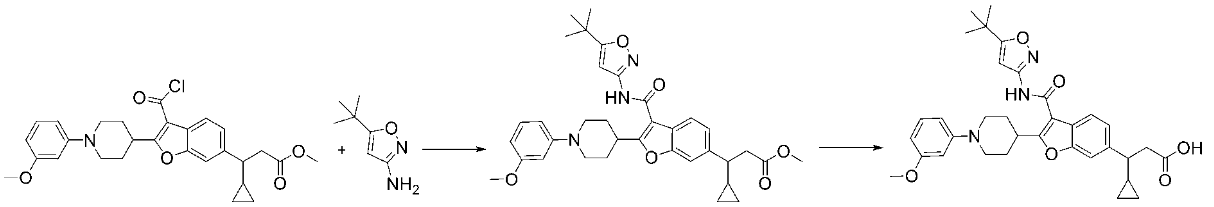

235-1 300-1 300-2 300

Referring to the synthetic route of compound **235,** compound **235-2** was replaced with compound **300-1** to carry out the synthesis. The resulting crude product was purified by reverse-phase column chromatography [acetonitrile/water (0.05% formic acid) = 0% to 100%] to obtain compound **299** (13 mg, yield: 18%) as a white solid. MS (ESI, m/z): 586.2 $[M+H]^+$.

$^1$H NMR (400 MHz, $CDCl_3$) δ 8.84 (s, 1H), 7.67 (*d, J* = 8.4 Hz, 1H), 7.47 (s, 1H), 7.29 - 7.20 (m, 2H), 6.87 (s, 1H), 6.65 (*d, J* = 8.4 Hz, 1H), 6.60 - 6.55 (m, 1H), 6.47 (*dd, J* = 8.4, 2.4 Hz, 1H), 3.84 - 3.74 (m, 6H), 3.02 - 2.79 (m, 4H), 2.58 - 2.48 (m, 1H), 2.25 - 2.17 (m, 2H), 2.08 - 2.02 (m, 2H), 1.41 (s, 9H), 1.18 - 1.08 (m, 1H), 0.71 - 0.64 (m, 1H), 0.54 - 0.46 (m, 1H), 0.43 - 0.33 (m, 1H), 0.25 - 0.16 (m, 1H).

**Example** 301:

Synthetic Route:

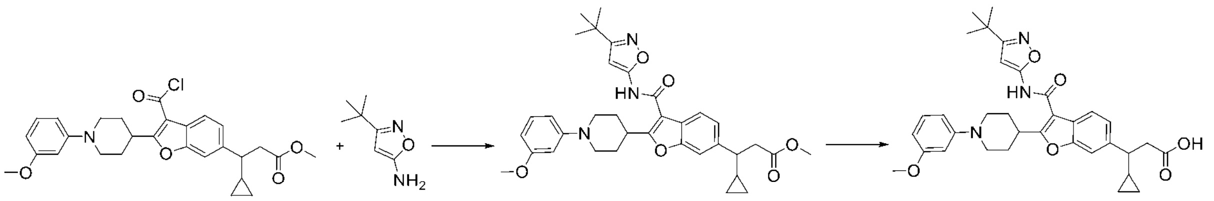

235-1 301-1 301-2 301

Referring to the synthetic route of compound **235,** compound **235-2** was replaced with compound **301-1** to carry out the synthesis. The resulting crude product was purified by reverse-phase column chromatography [acetonitrile/water (0.05% formic acid) = 0% to 100%] to obtain compound **301** (36 mg, yield: 53%) as a white solid. MS (ESI, m/z): 586.2 $[M+H]^+$.

$^1$H NMR (400 MHz, $CDCl_3$) δ 8.54 (s, 1H), 7.63 (d, *J* = 8.4 Hz, 1H), 7.47 (s, 1H), 7.32 (d, *J* = 8.4 Hz, 1H), 7.25 - 7.19 (m, 1H), 6.64 (dd, *J* = 8.4, 2.4 Hz, 1H), 6.58 - 6.54 (m, 1H), 6.50 (s, 1H), 6.47 (dd, *J* = 8.4, 2.4 Hz, 1H), 3.90 - 3.69 (m, 6H), 2.99 - 2.81 (m, 4H), 2.56 - 2.47 (m, 1H), 2.26 - 2.13 (m, 2H), 2.04 - 2.00 (m, 2H), 1.39 (s, 9H), 1.16 - 1.06 (m, 1H), 0.71- 0.64 (m, 1H), 0.56 - 0.43 (m, 1H), 0.40 - 0.34 (m, 1H), 0.24 - 0.18 (m, 1H).

**Example 302:**

Synthetic Route:

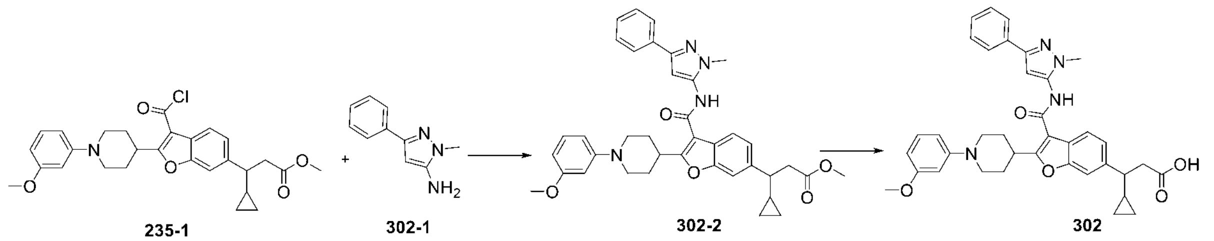

235-1 302-1 302-2 302

Referring to the synthetic route of compound **235,** compound **235-2** was replaced with compound **302-1** to carry out the synthesis. The resulting crude product was purified by reverse-phase column chromatography [acetonitrile/water

(0.05% formic acid) = 0% to 100%] to obtain compound **302** (1 mg, yield: 9%) as a white solid. MS (ESI, m/z): 619.2 [M+H]+.

$^1$H NMR (400 MHz, $cd_3od$) δ 7.88 - 7.78 (m, 3H), 7.53 (s, 1H), 7.48 - 7.41 (m, 2H), 7.41 - 7.34 (m, 2H), 7.23 - 7.17 (m, 1H), 6.78 (s, 1H), 6.68 (dd, *J* = 8.2, 2.0 Hz, 1H), 6.63 - 6.59 (m, 1H), 6.49 (dd, *J* = 8.1, 2.1 Hz, 1H), 3.94 (s, 3H), 3.91 - 3.83 (m, 2H), 3.82 (s, 3H), 3.73 - 3.62 (m, 1H), 2.95 - 2.73 (m, 4H), 2.60 - 2.51 (m, 1H), 2.28 - 2.06 (m, 4H), 1.21 - 1.14 (m, 1H), 0.71 - 0.61 (m, 1H), 0.50 - 0.37 (m, 2H), 0.27 - 0.17 (m, 1H).

**Example 303:**

Synthetic Route:

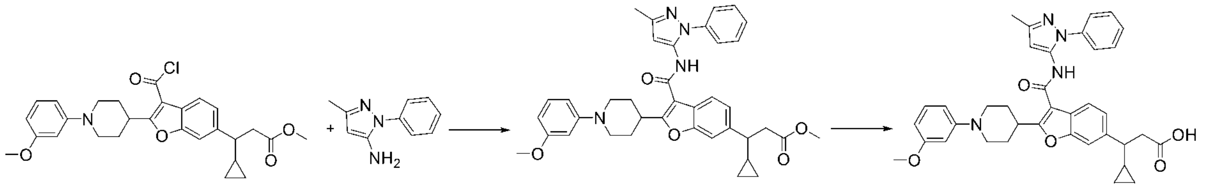

235-1 303-1 303-2 303

Referring to the synthetic route of compound **235,** compound **235-2** was replaced with compound **303-1** to carry out the synthesis. The resulting crude product was purified by reverse-phase column chromatography [acetonitrile/water (0.05% formic acid) = 0% to 100%] to obtain compound **303** (1 mg, yield: 7%) as a white solid. MS (ESI, m/z): 619.2 [M+H]+.

$^1$H NMR (400 MHz, $CD_3OD$) δ 7.57 - 7.50 (m, 4H), 7.48 - 7.45 (m, 1H), 7.42 - 7.40 (m, 2H), 7.19 - 7.13 (m, 2H), 6.61 (dd, *J* = 8.2, 2.2 Hz, 1H), 6.55 - 6.53 (m, 1H), 6.45 - 6.43 (m, 2H), 3.79 - 3.70 (m, 5H), 3.35 - 3.33 (m, 1H), 2.76 - 2.72 (m, 2H), 2.71 - 2.63 (m, 2H), 2.49 - 2.43 (m, 1H), 2.34 (s, 3H), 2.08 - 2.04 (m, 2H), 1.90 - 1.87 (m, 2H), 1.12 - 1.03 (m, 1H), 0.62 - 0.57 (m, 1H), 0.39 - 0.30 (m, 2H), 0.15 - 0.12 (m, 1H).

**Example 304:**

Synthetic Route:

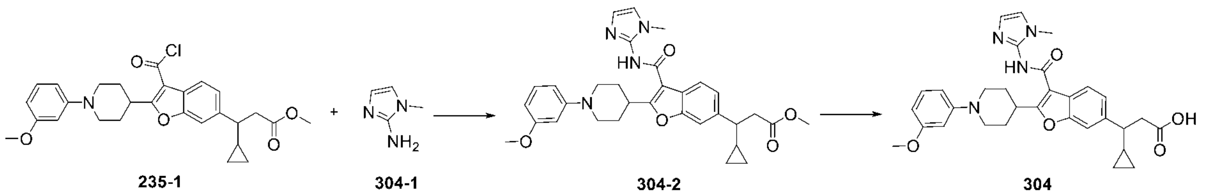

235-1 304-1 304-2 304

Referring to the synthetic route of compound **235,** compound **235-2** was replaced with compound **304-1** to carry out the synthesis. The resulting crude product was purified by reverse-phase column chromatography [acetonitrile/water (0.05% formic acid) = 0% to 100%] to obtain compound **304** (2 mg, yield: 10%) as a white solid. MS (ESI, m/z): 543.2 [M+H]+.

$^1$H NMR (400 MHz, DMSO-$d_6$) δ 12.27 (s, 1H), 8.26-8.18 (m, 1H), 7.42 (s, 1H), 7.19 - 7.10 (m, 2H), 7.01 (d, *J* = 2.0 Hz, 1H), 6.80 (d, *J* = 2.0 Hz, 1H), 6.62 - 6.57 (m, 1H), 6.53 - 6.49 (m, 1H), 6.39 - 6.34 (m, 1H), 4.41 - 4.23 (m, 1H), 3.94 - 3.84 (m, 2H), 3.73 (s, 3H), 3.57 (s, 3H), 2.85 - 2.77 (m, 2H), 2.72 - 2.66 (m, 2H), 2.39 - 2.33 (m, 1H), 2.02 - 1.90 (m, 4H), 1.11 - 1.03 (m, 1H), 0.55 - 0.48 (m, 1H), 0.34 - 0.24 (m, 2H), 0.18 - 0.11 (m, 1H).

**Example** 305:

Synthetic Route:

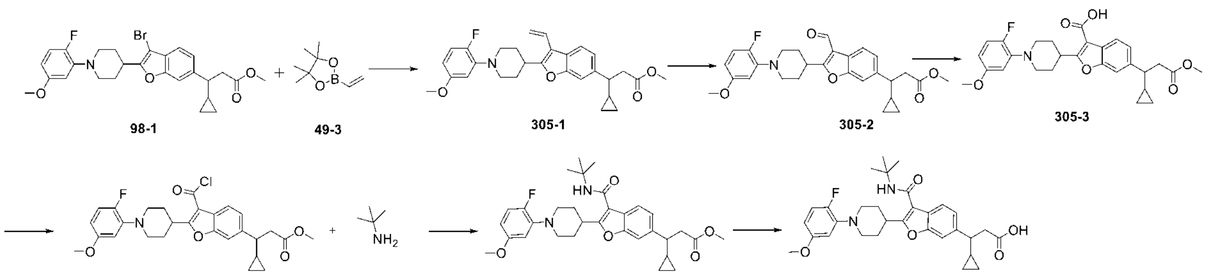

98-1 49-3 305-1 305-2 305-3

305-4 305-5 305-6 305

Referring to the synthetic route of compound **49,** compound **49-2** was replaced with compound **98-1** to synthesize compound **305-3.** Then, referring to the synthetic route of compound 235, compound **126-3** was replaced with compound **305-3,** and compound **235-2** was replaced with compound **305-5.** The synthesis was carried out, and the resulting crude product was purified by reverse-phase column chromatography [acetonitrile/water (0.05% formic acid) = 0% to 100%] to obtain compound **305** (10 mg, yield: 62%) as a white solid. MS (ESI, m/z): 537.2 $[M+H]^+$.

$^1H$ NMR (400 MHz, $CDCl_3$) δ 7.50 (d, *J* = 8.0 Hz, 1H), 7.39 (s, 1H), 7.21 (d, *J* = 8.0 Hz, 1H), 7.03 - 6.88 (m, 1H), 6.61 - 6.51 (m, 1H), 6.45 - 6.43 (m, 1H), 5.84 (s, 1H), 3.80 (s, 3H), 3.77 - 3.69 (m, 1H), 3.62 - 3.52 (m, 2H), 2.95 - 2.76 (m, 4H), 2.55 - 2.43 (m, 1H), 2.32 - 2.16 (m, 2H), 2.07 - 1.96 (m, 2H), 1.52 (s, 9H), 1.15 - 1.02 (m, 1H), 0.70 - 0.58 (m, 1H), 0.51 - 0.40 (m, 1H), 0.39 - 0.28 (m, 1H), 0.24 - 0.14 (m, 1H).

**Example 306:**

Synthetic Route:

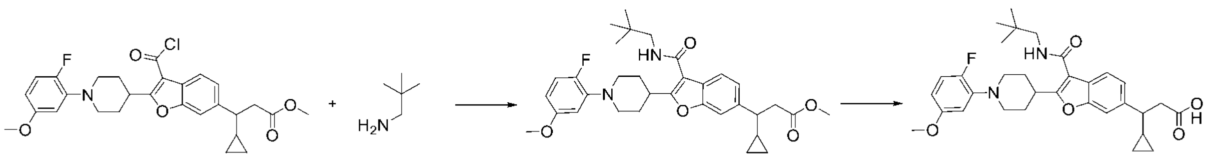

305-4 306-1 306-2 306

Referring to the synthetic route of compound **305,** compound **305-5** was replaced with compound **306-1** to carry out the synthesis. The resulting crude product was purified by reverse-phase column chromatography [acetonitrile/water (0.05% formic acid) = 0% to 100%] to obtain compound **306** (15 mg, yield: 72%) as a white solid. MS (ESI, m/z): 551.2 $[M+H]^+$.

$^1H$ NMR (400 MHz, $CDCl_3$) δ 7.55 (d, J = 8.0 Hz, 1H), 7.41 (s, 1H), 7.24 (d, *J* = 8.0 Hz, 1H), 6.96 (dd, *J* = 12.0, 8.8 Hz, 1H), 6.60 - 6.51 (m, 1H), 6.48 - 6.40 (m, 1H), 6.05 (t, J = 6.4 Hz, 1H), 3.81 - 3.74 (m, 4H), 3.63 - 3.54 (m, 2H), 3.33 (d, J = 6.4 Hz, 2H), 2.97 - 2.78 (m, 4H), 2.55 - 2.44 (m, 1H), 2.32 - 2.17 (m, 2H), 2.05 - 1.97 (m, 2H), 1.15 - 1.07 (m, 1H), 1.05 (s, 9H), 0.69 - 0.60 (m, 1H), 0.52 - 0.41 (m, 1H), 0.39 - 0.30 (m, 1H), 0.25 - 0.14 (m, 1H).

**Example 307:**

Synthetic Route:

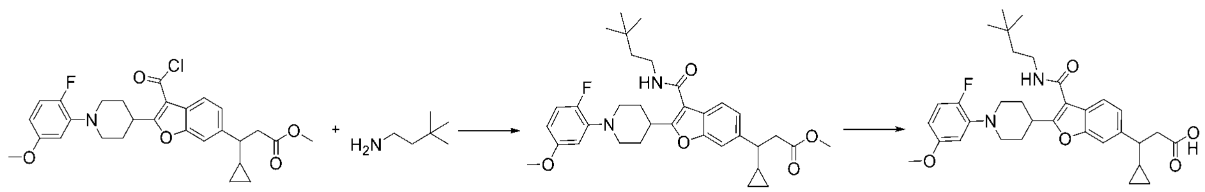

305-4 307-1 307-2 307

Referring to the synthetic route of compound **305,** compound **305-5** was replaced with compound **307-1** to carry out the synthesis. The resulting crude product was purified by reverse-phase column chromatography [acetonitrile/water

(0.05% formic acid) = 0% to 100%] to obtain compound **307** (15 mg, yield: 63%) as a white solid. MS (ESI, m/z): 565.2 $[M+H]^+$.

$^1H$ NMR (400 MHz, $CDCl_3$) δ 7.52 (d, J = 8.0 Hz, 1H), 7.40 (s, 1H), 7.21 (d, J = 8.0 Hz, 1H), 6.96 (dd, *J* = 12.0, 8.8 Hz, 1H), 6.60 - 6.52 (m, 1H), 6.47 - 6.39 (m, 1H), 5.92 (t, J = 6.0 Hz, 1H), 3.81 - 3.73 (m, 4H), 3.63 - 3.48 (m, 4H), 2.90 - 2.78 (m, 4H), 2.56 - 2.46 (m, 1H), 2.28 - 2.19 (m, 2H), 2.05 - 1.96 (m, 2H), 1.63 - 1.54 (m, 2H), 1.16 - 1.05 (m, 1H), 1.01 (s, 9H), 0.69 - 0.59 (m, 1H), 0.51 - 0.41 (m, 1H), 0.39 - 0.29 (m, 1H), 0.25 - 0.14 (m, 1H).

**Example 308:**

Synthetic Route:

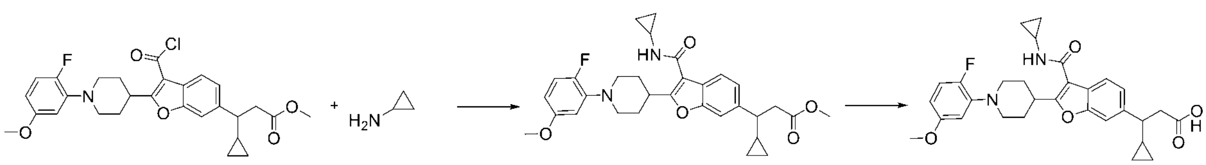

305-4 308-1 308-2 308

Referring to the synthetic route of compound **305,** compound **305-5** was replaced with compound **308-1** to carry out the synthesis. The resulting crude product was purified by reverse-phase column chromatography [acetonitrile/water (0.05% formic acid) = 0% to 100%] to obtain compound **308** (24 mg, yield: 60%) as a white solid. MS (ESI, m/z): 521.2 $[M+H]^+$.

$^1H$ NMR (400 MHz, $CDCl_3$) δ 7.42 (d, *J* = 8.0 Hz, 1H), 7.38 (s, 1H), 7.18 (d, *J* = 6.8 Hz, 1H), 6.99 - 6.90 (m, 1H), 6.57 - 6.51 (m, 1H), 6.45 - 6.37 (m, 1H), 6.15 - 6.06 (m, 1H), 3.90 - 3.73 (m, 4H), 3.61 - 3.51 (m, 2H), 2.95 - 2.75 (m, 5H), 2.53 - 2.43 (m, 1H), 2.29 - 2.14 (m, 2H), 2.07 - 1.95 (m, 2H), 1.13 - 1.00 (m, 1H), 0.95 - 0.85 (m, 2H), 0.69 - 0.58 (m, 3H), 0.48 - 0.40 (m, 1H), 0.36 - 0.36 (m, 1H), 0.23 - 0.12 (m, 1H).

**Example 309:**

Synthetic Route:

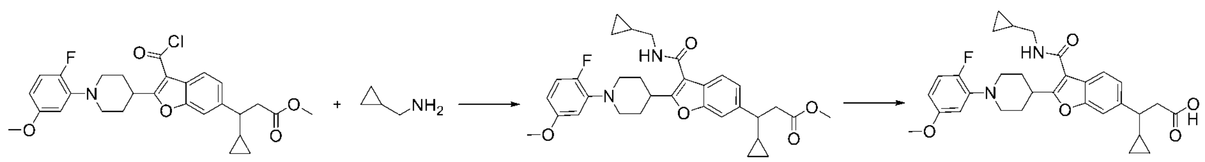

305-4 309-1 309-2 309

Referring to the synthetic route of compound **305,** compound **305-5** was replaced with compound **309-1** to carry out the synthesis. The resulting crude product was purified by reverse-phase column chromatography [acetonitrile/water (0.05% formic acid) = 0% to 100%] to obtain compound **309** (17 mg, yield: 38%) as a white solid. MS (ESI, m/z): 535.2 $[M+H]^+$.

$^1H$ NMR (400 MHz, $CDCl_3$) δ 7.55 (*d, J* = 8.0 Hz, 1H), 7.39 (s, 1H), 7.21 (d, *J* = 8.0 Hz, 1H), 6.98 - 6.89 (m, 1H), 6.55 - 6.51 (m, 1H), 6.45 - 6.38 (m, 1H), 6.12 - 6.03 (m, 1H), 3.82 - 3.70 (m, 4H), 3.61 - 3.51 (m, 2H), 3.41 - 3.31 (m, 2H), 2.90 - 2.80 (m, 4H), 2.54 - 2.43 (m, 1H), 2.30 - 2.16 (m, 2H), 2.05 - 1.94 (m, 2H), 1.17 - 1.01 (m, 2H), 0.68 - 0.53 (m, 3H), 0.51 - 0.39 (m, 1H), 0.39 - 0.27 (m, 3H), 0.22 - 0.09 (m, 1H).

**Example 310:**

Synthetic Route:

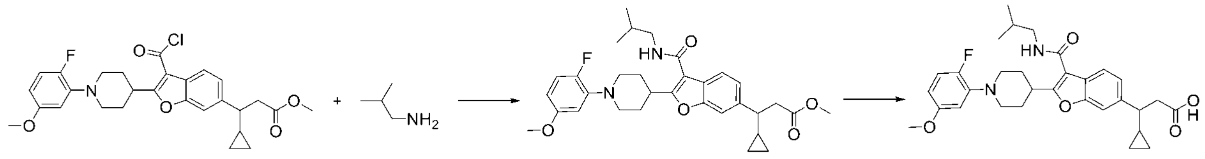

305-4 310-1 310-2 310

Referring to the synthetic route of compound **305,** compound **305-5** was replaced with compound **310-1** to carry out the synthesis. The resulting crude product was purified by reverse-phase column chromatography [acetonitrile/water (0.05% formic acid) = 0% to 100%] to obtain compound **310** (20 mg, yield: 38%) as a white solid. MS (ESI, m/z): 537.2 $[M+H]^+$.

$^1H$ NMR (400 MHz, $CDCl_3$) δ 7.56 - 7.46 (m, 1H), 7.39 (s, 1H), 7.21 (d, *J* = 8.0 Hz, 1H), 6.98 - 6.90 (m, 1H), 6.57 - 6.51 (m, 1H), 6.45 - 6.38 (m, 1H), 6.07 - 5.98 (m, 1H), 3.83 - 3.70 (m, 4H), 3.62 - 3.51 (m, 2H), 3.38 - 3.29 (m, 2H), 2.93 - 2.76 (m, 4H), 2.52 - 2.42 (m, 1H), 2.28 - 2.15 (m, 2H), 2.05 - 1.88 (m, 3H), 1.13 - 0.97 (d, *J*= 5.6 Hz, 7H), 0.66 - 0.57 (m, 1H), 0.49 - 0.40 (m, 1H), 0.37 - 0.29 (m, 1H), 0.22 - 0.14 (m, 1H).

**Example 311:**

Synthetic Route:

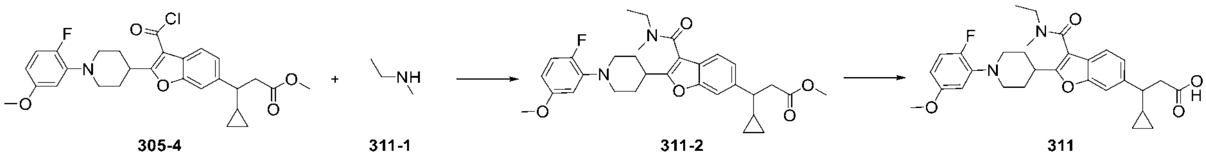

305-4 311-1 311-2 311

Referring to the synthetic route of compound **305,** compound **305-5** was replaced with compound **311-1** to carry out the synthesis. The resulting crude product was purified by reverse-phase column chromatography [acetonitrile/water (0.05% formic acid) = 0% to 100%] to obtain compound **311** (26 mg, yield: 57%) as a white solid. MS (ESI, m/z): 523.2 $[M+H]^+$.

$^1H$ NMR (400 MHz, $CDCl_3$) δ 7.33 (s, 1H), 7.30 (d, *J* = 7.6 Hz, 1H), 7.12 (d, *J* = 7.6 Hz, 1H), 6.98 - 6.89 (m, 1H), 6.56 - 6.49 (m, 1H), 6.45 - 6.38 (m, 1H), 3.77 (s, 3H), 3.70 - 3.31 (m, 4H), 3.22 - 2.91 (m, 4H), 2.85 - 2.75 (m, 4H), 2.50 - 2.43 (m, 1H), 2.25 - 2.11 (m, 2H), 2.08 - 1.98 (m, 2H), 1.28 - 1.02 (m, 4H), 0.64 - 0.57 (m, 1H), 0.47 - 0.38 (m, 1H), 0.36 - 0.28 (m, 1H), 0.21 - 0.14 (m, 1H).

**Example 312:**

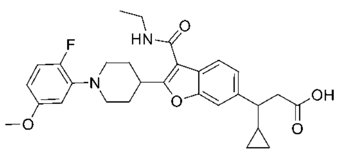

Synthetic Route:

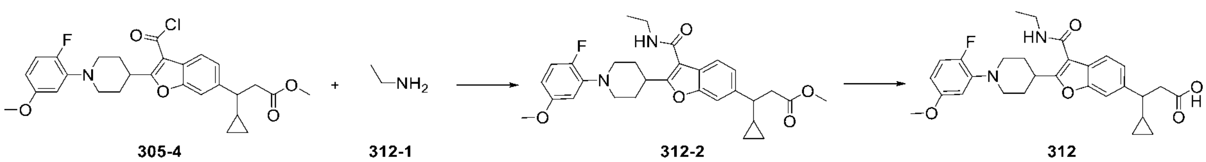

305-4 312-1 312-2 312

Referring to the synthetic route of compound **305,** compound **305-5** was replaced with compound **312-1** to carry

out the synthesis. The resulting crude product was purified by reverse-phase column chromatography [acetonitrile/water (0.05% formic acid) = 0% to 100%] to obtain compound **312** (17 mg, yield: 55%) as a white solid. MS (ESI, m/z): 509.2 $[M+H]^+$.

$^1H$ NMR (400 MHz, $CDCl_3$) δ 7.51 (d, *J* = 8.0 Hz, 1H), 7.38 (s, 1H), 7.19 (d, *J* = 8.0 Hz, 1H), 7.02 - 6.85 (m, 1H), 6.57 - 6.51 (m, 1H), 6.45 - 6.38 (m, 1H), 5.98 - 5.91 (m, 1H), 3.83 - 3.70 (m, 4H), 3.60 - 3.47 (m, 4H), 2.91 - 2.73 (m, 4H), 2.53 - 2.42 (m, 1H), 2.27 - 2.15 (m, 2H), 2.03 - 1.95 (m, 2H), 1.31 - 1.25 (m, 3H), 1.12 - 1.02 (m, 1H), 0.66 - 0.57 (m, 1H), 0.46 - 0.40 (m, 1H), 0.37 - 0.27 (m, 1H), 0.21 - 0.13 (m, 1H).

**Example 313:**

Synthetic Route:

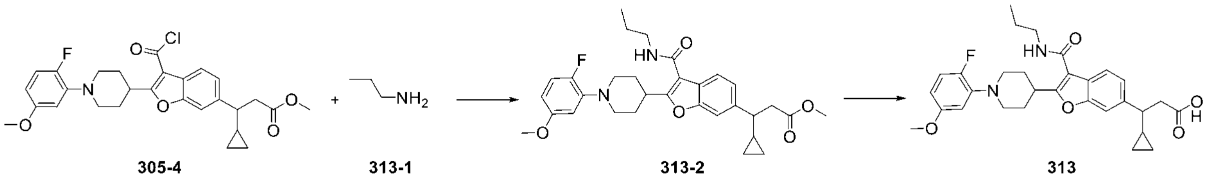

305-4 313-1 313-2 313

Referring to the synthetic route of compound **305,** compound **305-5** was replaced with compound **313-1** to carry out the synthesis. The resulting crude product was purified by reverse-phase column chromatography [acetonitrile/water (0.05% formic acid) = 0% to 100%] to obtain compound **313** (13 mg, yield: 54%) as a white solid. MS (ESI, m/z): 523.2 $[M+H]^+$.

$^1H$ NMR (400 MHz, $CDCl_3$) δ 7.50 (d, *J* = 6.8 Hz, 1H), 7.38 (s, 1H), 7.19 (d, *J* = 6.8 Hz, 1H), 7.01 - 6.87 (m, 1H), 6.58 - 6.50 (m, 1H), 6.46 - 6.36 (m, 1H), 6.00 (s, 1H), 3.80 - 3.70 (m, 4H), 3.61 - 3.40 (m, 4H), 2.92 - 2.71 (m, 4H), 2.53 - 2.41 (m, 1H), 2.26 - 2.13 (m, 2H), 2.03 - 1.92 (m, 2H), 1.73 - 1.61 (m, 2H), 1.11 - 0.97 (m, 4H), 0.65 - 0.56 (m, 1H), 0.47 - 0.39 (m, 1H), 0.35 - 0.27 (m, 1H), 0.20 - 0.11 (m, 1H).

**Example 314:**

Synthetic Route:

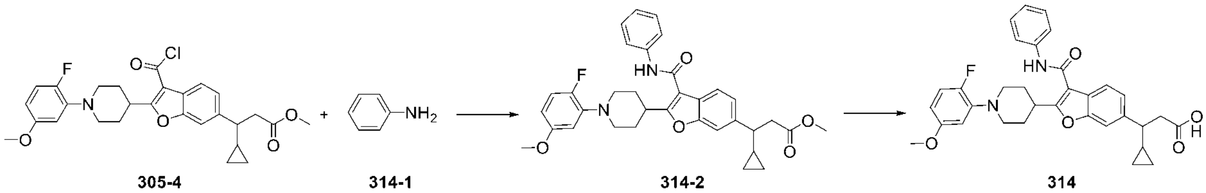

305-4 314-1 314-2 314

Referring to the synthetic route of compound **305,** compound **305-5** was replaced with compound **314-1** to carry out the synthesis. The resulting crude product was purified by reverse-phase column chromatography [acetonitrile/water (0.05% formic acid) = 0% to 100%] to obtain compound **314** (10 mg, yield: 66%) as a white solid. MS (ESI, m/z): 557.2 $[M+H]^+$.

$^1H$ NMR (400 MHz, $CDCl_3$) δ 7.72 (s, 1H), 7.67 - 7.60 (m, 3H), 7.49 - 7.36 (m, 3H), 7.29 - 7.24 (m, 1H), 7.22 - 7.15 (m, 1H), 7.02 - 6.91 (m, 1H), 6.61 - 6.51 (m, 1H), 6.45 - 6.43 (m, 1H), 3.85 - 3.72 (m, 4H), 3.63 - 3.54 (m, 2H), 3.00 - 2.74 (m, 4H), 2.59 - 2.43 (m, 1H), 2.34 - 2.18 (m, 2H), 2.08 - 1.98 (m, 2H), 1.17 - 1.04 (m, 1H), 0.72 - 0.60 (m, 1H), 0.54 - 0.43 (m, 1H), 0.41 - 0.31 (m, 1H), 0.27 - 0.15 (m, 1H).

**Example 315:**

Synthetic Route:

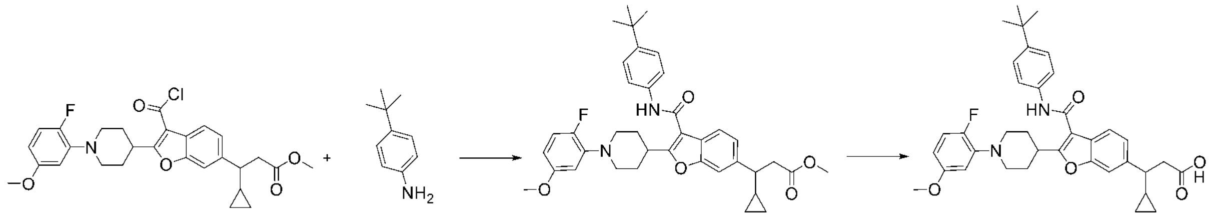

305-4 315-1 315-2 315

Referring to the synthetic route of compound **305,** compound **305-5** was replaced with compound **315-1** to carry out the synthesis. The resulting crude product was purified by reverse-phase column chromatography [acetonitrile/water (0.05% formic acid) = 0% to 100%] to obtain compound **315** (10 mg, yield: 62%) as a white solid. MS (ESI, m/z): 613.2 $[M+H]^+$.

$^1H$ NMR (400 MHz, $CDCl_3$) δ 7.69 - 7.60 (m, 2H), 7.55 (d, *J* = 8.4 Hz, 2H), 7.46 - 7.41 (m, 3H), 7.28 - 7.24 (m, 1H), 7.02 - 6.89 (m, 1H), 6.61 - 6.51 (m, 1H), 6.47 - 6.39 (m, 1H), 3.83 - 3.74 (m, 4H), 3.63 - 3.55 (m, 2H), 2.95 - 2.80 (m, 4H), 2.57 - 2.44 (m, 1H), 2.35 - 2.17 (m, 2H), 2.07 - 1.99 (m, 2H), 1.35 (s, 9H), 1.18 - 1.03 (m, 1H), 0.72 - 0.61 (m, 1H), 0.54 - 0.44 (m, 1H), 0.42 - 0.32 (m, 1H), 0.27 - 0.17 (m, 1H).

**Example 316:**

Synthetic Route:

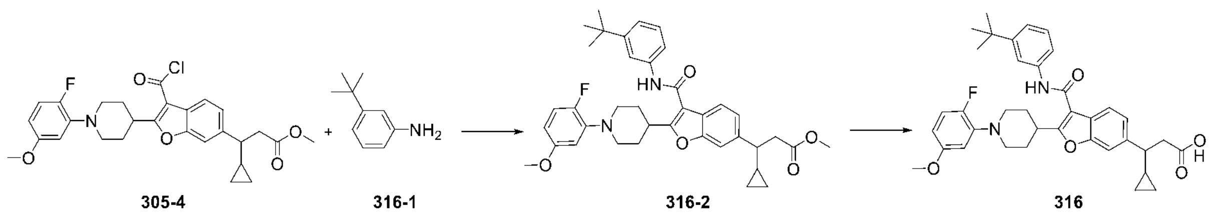

305-4 316-1 316-2 316

Referring to the synthetic route of compound **305,** compound **305-5** was replaced with compound **316-1** to carry out the synthesis. The resulting crude product was purified by reverse-phase column chromatography [acetonitrile/water (0.05% formic acid) = 0% to 100%] to obtain compound **316** (15 mg, yield: 66%) as a white solid. MS (ESI, m/z): 613.2 $[M+H]^+$.

$^1H$ NMR (400 MHz, $CDCl_3$) δ 7.72 - 7.62 (m, 2H), 7.57 (s, 1H), 7.51 (d, *J* = 8.0 Hz, 1H), 7.45 (s, 1H), 7.38 - 7.32 (m, 1H), 7.27 - 7.20 (m, 2H), 7.01 - 6.90 (m, 1H), 6.60 - 6.50 (m, 1H), 6.49 - 6.38 (m, 1H), 3.79 (m, 4H), 3.63 - 3.54 (m, 2H), 3.00 - 2.75 (m, 4H), 2.57 - 2.44 (m, 1H), 2.37 - 2.17 (m, 2H), 2.07 - 1.98 (m, 2H), 1.37 (s, 9H), 1.18 - 1.04 (m, 1H), 0.72 - 0.61 (m, 1H), 0.53 - 0.43 (m, 1H), 0.42 - 0.32 (m, 1H), 0.27 - 0.17 (m, 1H).

**Example 317:**

Synthetic Route:

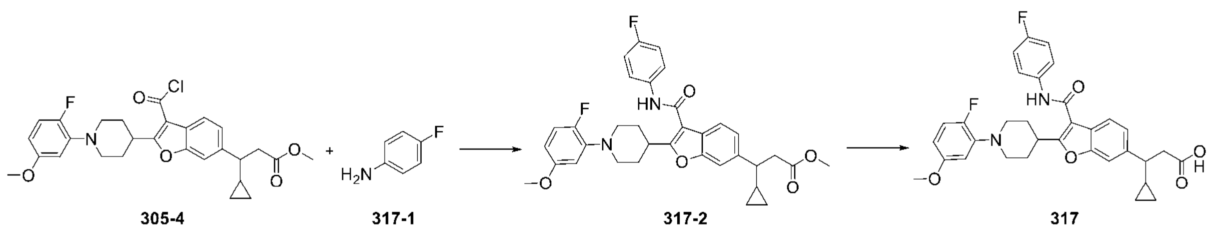

305-4 317-1 317-2 317

Referring to the synthetic route of compound **305,** compound **305-5** was replaced with compound **317-1** to carry out the synthesis. The resulting crude product was purified by reverse-phase column chromatography [acetonitrile/water (0.05% formic acid) = 0% to 100%] to obtain compound **317** (6 mg, yield: 11%) as a white solid. MS (ESI, m/z): 575.2

[M+H]+.

$^1$H NMR (400 MHz, DMSO-$d_6$) δ 10.26 (s, 1H), 8.45 (s, 1H), 7.78 - 7.76 (m, 2H), 7.59 (d, $J$ = 7.6 Hz, 1H), 7.55 (s, 1H), 7.26 (d, $J$ = 8.4 Hz, 1H), 7.21 - 7.17 (m, 2H), 7.09 - 7.00 (m, 1H), 6.59 - 6.57 (m, 1H), 6.51 - 6.48 (m, 1H), 3.73 (s, 3H), 3.60 - 3.30 (m, 3H), 2.81 - 2.76 (m, 2H), 2.69 - 2.67 (m, 2H), 2.44 - 2.40 (m, 1H), 2.04 - 1.99 (m, 4H), 1.12 - 1.05 (m, 1H), 0.55 - 0.49 (m, 1H), 0.32 - 0.27 (m, 2H), 0.17 - 0.12 (m, 1H).

**Example 318:**

Synthetic Route:

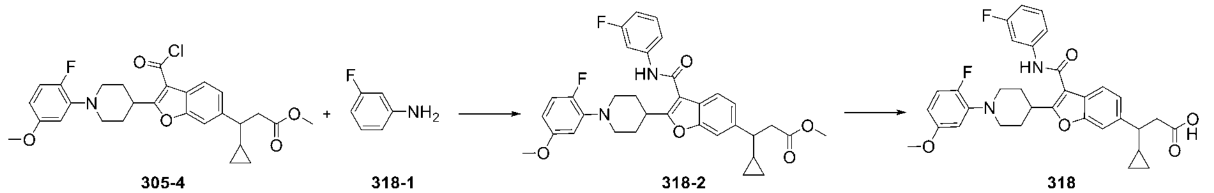

305-4 318-1 318-2 318

Referring to the synthetic route of compound **305,** compound **305-5** was replaced with compound **318-1** to carry out the synthesis. The resulting crude product was purified by reverse-phase column chromatography [acetonitrile/water (0.05% formic acid) = 0% to 100%] to obtain compound **318** (4 mg, yield: 7%) as a white solid. MS (ESI, m/z): 575.2 [M+H]+.

$^1$H NMR (400 MHz, $CDCl_3$) δ 7.75 (s, 1H), 7.66 - 7.58 (m, 2H), 7.46 (s, 1H), 7.40 - 7.31 (m, 1H), 7.30 - 7.24 (m, 2H), 6.98 - 6.93 (m, 1H), 6.89 - 6.87 (m, 1H), 6.57 - 6.55 (m, 1H), 6.46 -6.42 (m, 1H), 3.79 (s, 3H), 3.77 - 3.73 (m, 1H), 3.61 - 3.58 (m, 2H), 2.89 - 2.82 (m, 4H), 2.58 - 2.48 (m, 1H), 2.32 - 2.21 (m, 2H), 2.05 - 2.00 (m, 2H), 1.14 - 1.08 (m, 1H), 0.70 - 0.63 (m, 1H), 0.52 - 0.45 (m, 1H), 0.39 - 0.33 (m, 1H), 0.24 - 0.18 (m, 1H).

**Example 319:**

Synthetic Route:

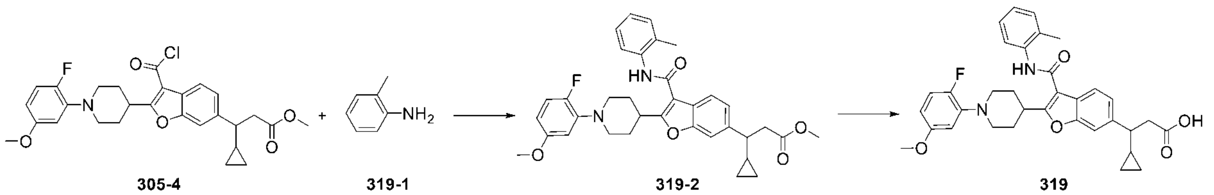

305-4 319-1 319-2 319

Referring to the synthetic route of compound **305,** compound **305-5** was replaced with compound **319-1** to carry out the synthesis. The resulting crude product was purified by reverse-phase column chromatography [acetonitrile/water (0.05% formic acid) = 0% to 100%] to obtain compound **319** (9 mg, yield: 42%) as a white solid. MS (ESI, m/z): 571.2 [M+H]+.

$^1$H NMR (400 MHz, $CDCl_3$) δ 8.03 - 7.95 (m, 1H), 7.64 - 7.59 (m, 1H), 7.57 (s, 1H), 7.46 (s, 1H), 7.31 - 7.23 (m, 2H), 7.18 - 7.08 (m, 1H), 6.99 - 6.88 (m, 1H), 6.57 - 6.49 (m, 1H), 6.46 - 6.37 (m, 1H), 3.84 - 3.69 (m, 4H), 3.61 - 3.50 (m, 2H), 2.90 - 2.74 (m, 4H), 2.56 - 2.46 (m, 1H), 2.36 (s, 3H), 2.33 - 2.16 (m, 2H), 2.07 - 1.98 (m, 2H), 1.15 - 1.06 (m, 1H), 0.68 - 0.61 (m, 1H), 0.49 - 0.42 (m, 1H), 0.39 - 0.30 (m, 1H), 0.23 - 0.17 (m, 1H).

**Example 320:**

Synthetic Route:

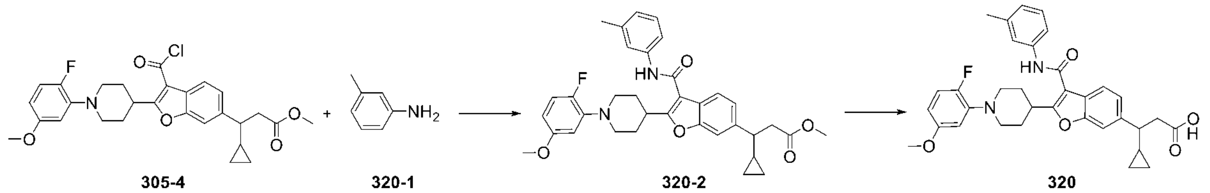

305-4 320-1 320-2 320

Referring to the synthetic route of compound **305,** compound **305-5** was replaced with compound **320-1** to carry out the synthesis. The resulting crude product was purified by reverse-phase column chromatography [acetonitrile/water (0.05% formic acid) = 0% to 100%] to obtain compound **320** (18 mg, yield: 70%) as a white solid. MS (ESI, m/z): 571.2 $[M+H]^+$.

$^1H$ NMR (400 MHz, $CDCl_3$) δ 7.68 - 7.57 (m, 1H), 7.51 (s, 1H), 7.43 (s, 1H), 7.40 - 7.33 (m, 1H), 7.31 - 7.20 (m, 2H), 7.02 - 6.90 (m, 2H), 6.61 - 6.51 (m, 1H), 6.48 - 6.39 (m, 1H), 3.84 - 3.70 (m, 4H), 3.62 - 3.49 (m, 2H), 2.96 - 2.76 (m, 4H), 2.52 - 2.45 (m, 1H), 2.39 (s, 3H), 2.34 - 2.19 (m, 2H), 2.07 - 1.97 (m, 2H), 1.13 - 1.00 (m, 1H), 0.70 - 0.57 (m, 1H), 0.51 - 0.41 (m, 1H), 0.38 - 0.28 (m, 1H), 0.23 - 0.14 (m, 1H).

**Example 321:**

Synthetic Route:

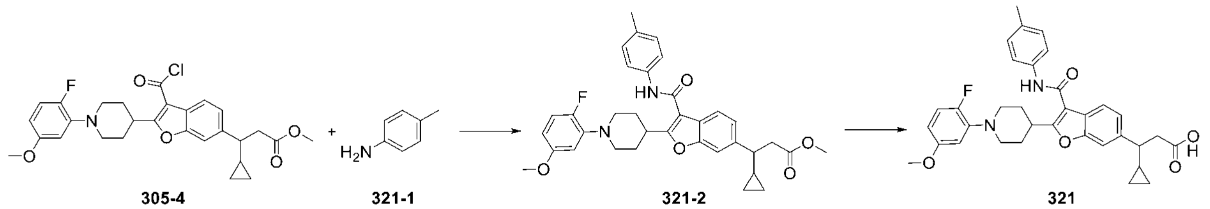

305-4 321-1 321-2 321

Referring to the synthetic route of compound **305,** compound **305-5** was replaced with compound **321-1** to carry out the synthesis. The resulting crude product was purified by reverse-phase column chromatography [acetonitrile/water (0.05% formic acid) = 0% to 100%] to obtain compound **321** (23 mg, yield: 74%) as a white solid. MS (ESI, m/z): 571.2 $[M+H]^+$.

$^1H$ NMR (400 MHz, $CDCl_3$) δ 7.68 - 7.57 (m, 2H), 7.50 (d, *J* = 7.6 Hz, 2H), 7.43 (s, 1H), 7.27 - 7.22 (m, 1H), 7.19 (d, *J* = 7.6 Hz, 2H), 7.00 - 6.88 (m, 1H), 6.64 - 6.51 (m, 1H), 6.47-6.39 (m, 1H), 3.83 - 3.71 (m, 4H), 3.62 - 3.49 (m, 2H), 2.96-2.73 (m, 4H), 2.54 - 2.44 (m, 1H), 2.35 (s, 3H), 2.31 - 2.15 (m, 2H), 2.06 - 1.95 (m, 2H), 1.12 - 1.01 (m, 1H), 0.68 - 0.56 (m, 1H), 0.52 - 0.40 (m, 1H), 0.38 - 0.29 (m, 1H), 0.22 - 0.14 (m, 1H).

**Example 322:**

Synthetic Route:

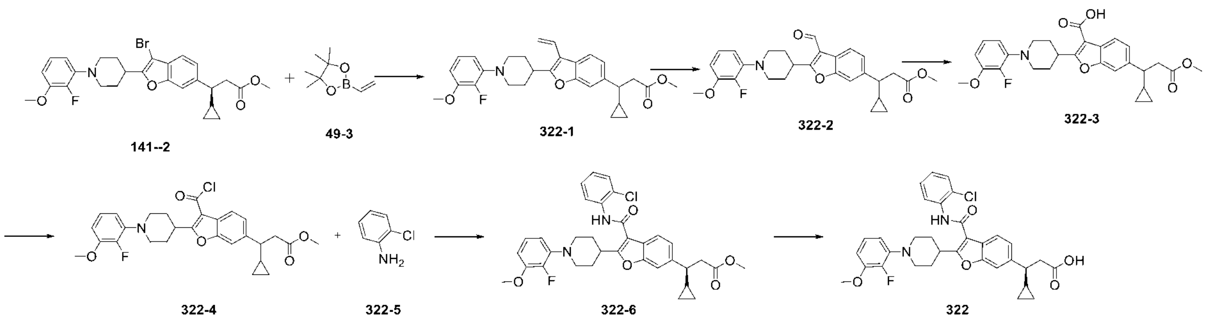

141--2 49-3 322-1 322-2 322-3

322-4 322-5 322-6 322

Referring to the synthetic route of compound **305,** compound **98-1** was replaced with compound **141-2,** and compound **305-5** was replaced with compound **322-5.** The synthesis was carried out, and the resulting crude product was

purified by reverse-phase column chromatography [acetonitrile/water (0.05% formic acid) = 0% to 100%] to obtain compound **322** (1 mg, yield: 4%) as a white solid. MS (ESI, m/z): 591.2 [M+H]$^+$.

$^1$H NMR (400 MHz, $CDCl_3$) δ 8.63 (d, $J$ = 8.0 Hz, 1H), 8.35 (s, 1H), 7.85 (d, $J$ = 8.0 Hz, 1H), 7.50 - 7.43 (m, 2H), 7.38 - 7.34 (m, 1H), 7.33 - 7.27 (m, 1H), 7.13 - 7.08 (m, 1H), 7.03 - 6.99 (m, 1H), 6.69 - 6.61 (m, 2H), 3.90 (s, 3H), 3.88 - 3.80 (m, 1H), 3.66 - 3.54 (m, 2H), 2.98 - 2.84 (m, 4H), 2.59 - 2.49 (m, 1H), 2.34 - 2.27 (m, 2H), 2.08 - 2.04 (m, 2H), 1.15 - 1.11 (m, 1H), 0.73 - 0.62 (m, 1H), 0.53 - 0.44 (m, 1H), 0.43 - 0.33 (m, 1H), 0.28 - 0.19 (m, 1H).

**Example 323:**

Synthetic Route:

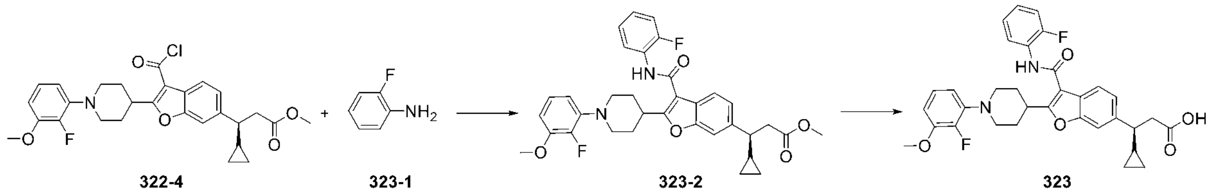

322-4 323-1 323-2 323

Referring to the synthetic route of compound **322,** compound **322-5** was replaced with compound **323-1** to carry out the synthesis. The resulting crude product was purified by reverse-phase column chromatography [acetonitrile/water (0.05% formic acid) = 0% to 100%] to obtain compound **323** (3 mg, yield: 9%) as a white solid. MS (ESI, m/z): 575.2 [M+H]$^+$.

$^1$H NMR (400 MHz, $CDCl_3$) δ 8.55 - 8.47 (m, 1H), 8.01 (s, 1H), 7.71 (d, $J$= 8.0 Hz, 1H), 7.47 (s, 1H), 7.34 - 7.29 (m, 1H), 7.24 -7.08 (m, 3H), 7.04 - 6.98 (m, 1H), 6.68 - 6.63 (m, 2H), 3.90 (s, 3H), 3.87 - 3.77 (m, 1H), 3.66 - 2.55 (m, 2H), 3.05 - 2.80 (m, 4H), 2.59 - 2.49 (m, 1H), 2.34 - 2.25 (m, 2H), 2.11 - 2.01 (m, 2H), 1.15 - 1.08 (m, 1H), 0.72 - 0.60 (m, 1H), 0.53 - 0.42 (m, 1H), 0.41 - 0.31 (m, 1H), 0.24 - 0.18 (m, 1H).

**Example 324:**

Synthetic Route:

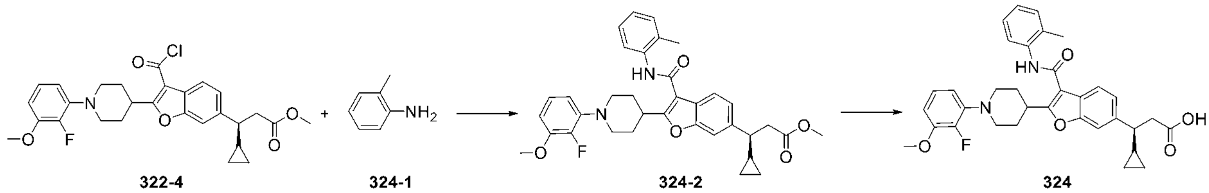

322-4 324-1 324-2 324

Referring to the synthetic route of compound **322,** compound **322-5** was replaced with compound **324-1** to carry out the synthesis. The resulting crude product was purified by reverse-phase column chromatography [acetonitrile/water (0.05% formic acid) = 0% to 100%] to obtain compound **324** (2 mg, yield: 6%) as a white solid. MS (ESI, m/z): 571.2 [M+H]$^+$.

$^1$H NMR (400 MHz, $CDCl_3$) δ 8.02 - 7.95 (m, 1H), 7.68 (d, $J$ = 8.0 Hz, 1H), 7.60 (s, 1H), 7.47 (s, 1H), 7.33 - 7.22 (m, 3H), 7.19 - 7.11 (m, 1H), 7.02 -6.97 (m, 1H), 6.68 - 6.62 (m, 2H), 3.90 (s, 3H), 3.87 - 3.74 (m, 1H), 3.63 - 3.52 (m, 2H), 2.97 - 2.80 (m, 4H), 2.54 - 2.49 (m, 1H), 2.38 (s, 3H), 2.29 -2.24 (m, 2H), 2.08 - 1.97 (m, 2H), 1.15 - 1.08 (m, 1H), 0.73 - 0.60 (m, 1H), 0.51 - 0.45 (m, 1H), 0.40 - 0.32 (m, 1H), 0.24 - 0.18 (m, 1H).

**Example 325:**

Synthetic Route:

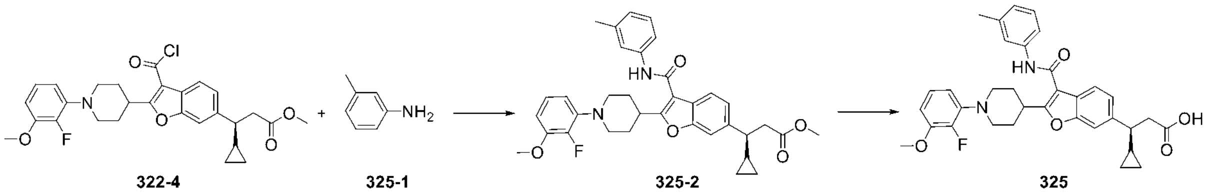

322-4 325-1 325-2 325

Referring to the synthetic route of compound **322,** compound **322-5** was replaced with compound **325-1** to carry out the synthesis. The resulting crude product was purified by reverse-phase column chromatography [acetonitrile/water (0.05% formic acid) = 0% to 100%] to obtain compound **325** (10 mg, yield: 51%) as a white solid. MS (ESI, m/z): 571.2 $[M+H]^+$.

$^1H$ NMR (400 MHz, $CDCl_3$) δ 7.69 - 7.60 (m, 2H), 7.54 - 7.49 (m, 1H), 7.48 - 7.42 (m, 1H), 7.41 - 7.34 (m, 1H), 7.27 - 7.24 (m, 2H), 7.04 - 6.95 (m, 2H), 6.69 - 6.60 (m, 2H), 3.89 (s, 3H), 3.85 - 3.74 (m, 1H), 3.62 - 3.49 (m, 2H), 2.93 - 2.81 (m, 4H), 2.56 - 2.45 (m, 1H), 2.40 (s, 3H), 2.30 - 2.20 (m, 2H), 2.07 - 1.99 (m, 2H), 1.15 - 1.07 (m, 1H), 0.70 - 0.60 (m, 1H), 0.51 - 0.43 (m, 1H), 0.40 - 0.30 (m, 1H), 0.26 - 0.17 (m, 1H).

**Example 326:**

Synthetic Route:

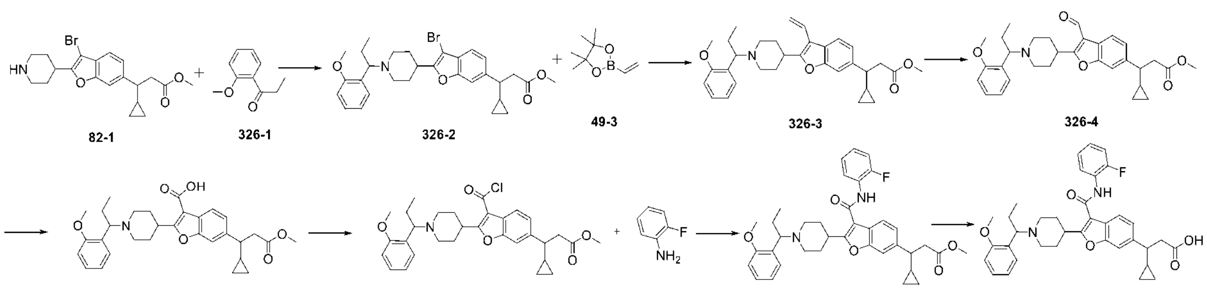

82-1 326-1 326-2 49-3 326-3 326-4

326-5 326-6 323-1 326-7 326

Compound **82-1** (45 mg, 0.11 mmol), compound **326-1** (100 mg, 0.61 mmol), and sodium cyanoborohydride (17 mg, 0.27 mmol) were dissolved in a mixture of methanol (10 mL) and acetic acid (1 mL). The reaction was carried out at 60°C for 48 hours. After the reaction was completed, saturated sodium bicarbonate solution (20 mL) was added, and the mixture was extracted with ethyl acetate (20 mL × 3). The combined organic phases were concentrated and purified by normal-phase column chromatography (ethyl acetate: petroleum ether = 0% to 100%) to obtain compound **326-2** (40 mg, yield: 66%). MS (ESI, m/z): 554.3 $[M+H]^+$.

Referring to the synthetic route of compound **323,** compound **322-4** was replaced with compound **326-2** to carry out the synthesis. The resulting crude product was purified by reverse-phase column chromatography [acetonitrile/water (0.05% formic acid) = 0% to 100%] to obtain compound **326** (1 mg, yield: 0.5%) as a white solid. MS (ESI, m/z): 599.3 $[M+H]^+$.

$^1H$ NMR (400 MHz, MeOD) δ 7.77 - 7.70 (m, 1H), 7.58 (d, *J* = 8.2 Hz, 1H), 7.37 - 7.31 (m, 1H), 7.30 - 7.22 (m, 2H), 7.15 (d, *J* = 8.2 Hz, 1H), 7.10 - 6.99 (m, 4H), 6.97 - 6.92 (m, 1H), 4.50 - 4.37 (m, 1H), 3.85 - 3.74 (m, 4H), 3.60 - 3.45 (m, 1H), 3.32 - 3.22 (m, 1H), 3.00 - 2.76 (m, 2H), 2.73 - 2.54 (m, 2H), 2.37 - 2.25 (m, 1H), 2.25 - 1.96 (m, 6H), 0.99 - 0.91 (m, 1H), 0.61 (t, *J* = 7.2 Hz, 3H), 0.50 - 0.40 (m, 1H), 0.29 - 0.13 (m, 2H), 0.04 - -0.06 (m, 1H).

**Example 327:**

Synthetic Route:

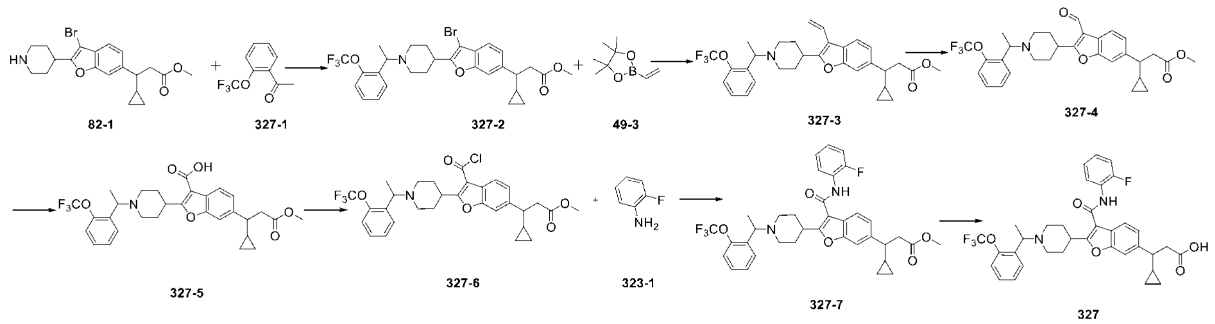

82-1 327-1 327-2 49-3 327-3 327-4

327-5 327-6 323-1 327-7 327

Referring to the synthetic route of compound **326,** compound **326-1** was replaced with compound **327-1** to carry out the synthesis. The resulting crude product was purified by reverse-phase column chromatography [acetonitrile/water (0.05% formic acid) = 0% to 100%] to obtain compound **327** (4 mg, yield: 14%) as a white solid. MS (ESI, m/z): 639.2 $[M+H]^+$.

$^1$H NMR (400 MHz, MeOD) δ 7.95 - 7.90 (m, 1H), 7.70 - 7.69 (m, 2H), 7.44 - 7.33 (m, 3H), 7.38 - 7.32 (m, 1H), 7.29 (d, *J* = 8.0 Hz, 1H), 7.27 - 7.17 (m, 3H), 4.24 - 4.15 (m, 1H), 3.56 - 3.45 (m, 2H), 3.09 - 3.00 (m, 1H), 2.87 - 2.70 (m, 2H), 2.52 - 2.27 (m, 3H), 2.25 - 1.92 (m, 4H), 1.51 (d, *J* = 6.8 Hz, 3H), 1.18 - 1.04 (m, 1H), 0.69 - 0.53 (m, 1H), 0.46 - 0.29 (m, 2H), 0.20 - 0.14 (m, 1H).

**Example 328:**

Synthetic Route:

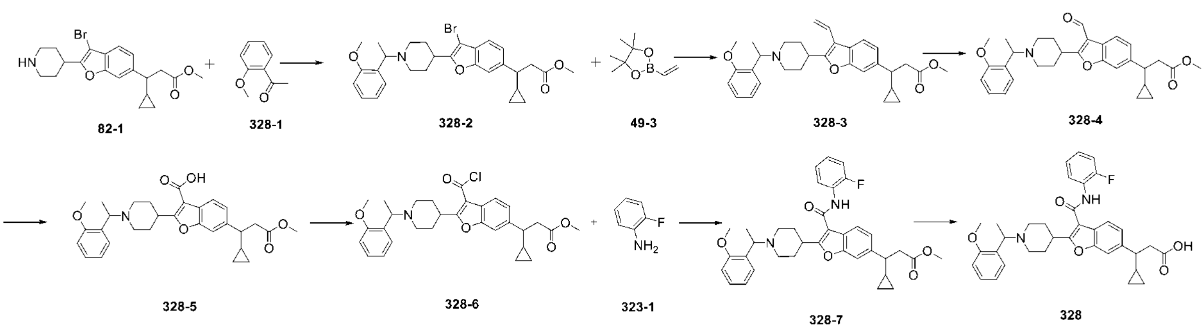

82-1 328-1 328-2 49-3 328-3 328-4

328-5 328-6 323-1 328-7 328

Referring to the synthetic route of compound **326,** compound **326-1** was replaced with compound **328-1** to carry out the synthesis. The resulting crude product was purified by reverse-phase column chromatography [acetonitrile/water (0.05% formic acid) = 0% to 100%] to obtain compound **328** (2 mg, yield: 3%) as a white solid. MS (ESI, m/z): 585.2 $[M+H]^+$.

$^1$H NMR (400 MHz, MeOD) δ 7.94 - 7.88 (m, 1H), 7.70 (d, *J* = 8.0 Hz, 1H), 7.48 - 7.41 (m, 3H), 7.33 (d, *J* = 8.2 Hz, 1H), 7.28 - 7.18 (m, 3H), 7.12 (d, *J* = 8.0 Hz, 1H), 7.10 - 7.04 (m, 1H), 4.79 - 4.65 (m, 1H), 3.93 (s, 3H), 3.80 - 3.61 (m, 2H), 3.43 - 3.31 (m, 1H), 3.02 - 2.82 (m, 2H), 2.78 - 2.60 (m, 2H), 2.55 - 2.43 (m, 1H), 2.38 - 2.08 (m, 4H), 1.68 (d, *J* = 6.8 Hz, 3H), 1.16 - 1.04 (m, 1H), 0.64 - 0.56 (m, 1H), 0.42 - 0.32 (m, 2H), 0.18 - 0.08 (m, 1H).

**Example 329:**

Synthetic Route:

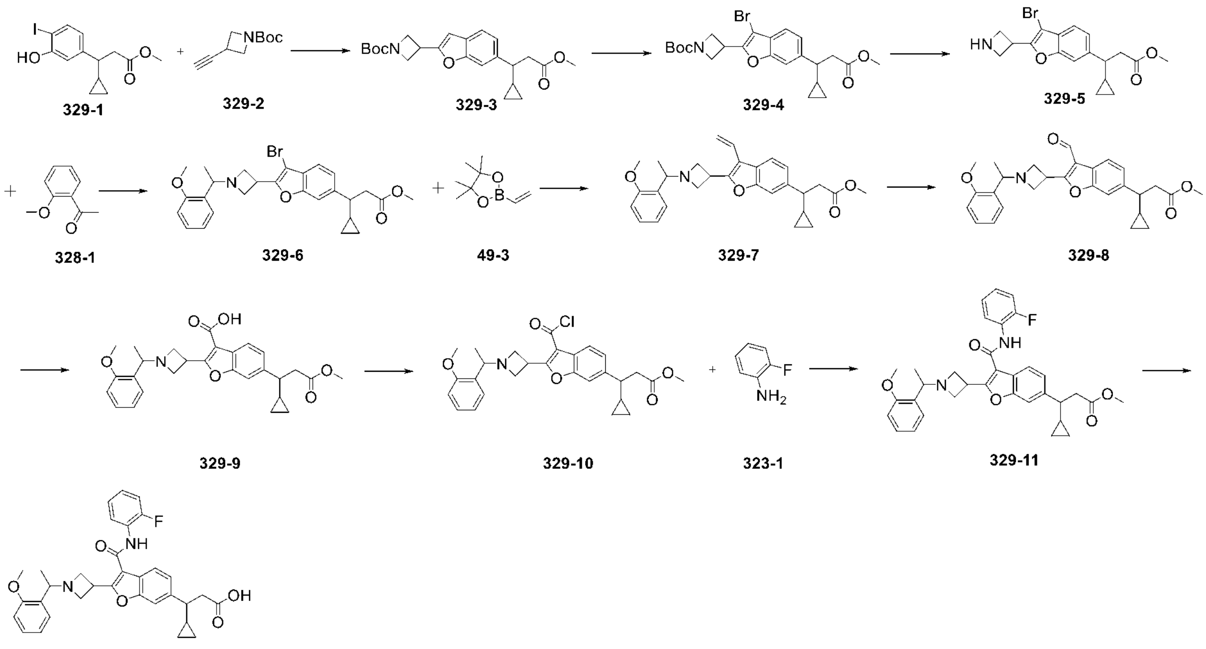

329-1 329-2 329-3 329-4 329-5

328-1 329-6 49-3 329-7 329-8

329-9 329-10 323-1 329-11

329

Referring to the synthetic route of compound **60,** compound **329-4** was synthesized. Then, referring to the synthetic route of compound **328** and replacing compound **82-1** with compound **329-5,** the synthesis was carried out, and the resulting crude product was purified by reverse-phase column chromatography [acetonitrile/water (0.05% formic acid) = 0% to 100%] to obtain compound **329** (20 mg, yield: 41%) as a white solid. MS (ESI, m/z): 557.2 $[M+H]^+$.

$^1H$ NMR (400 MHz, $CDCl_3$) δ 8.33 - 8.24 (m, 1H), 8.18 - 8.03 (m, 1H), 7.41 - 7.32 (m, 2H), 7.19 - 7.00 (m, 6H), 6.94 - 6.88 (m, 1H), 6.82 - 6.75 (m, 1H), 4.37 - 4.25 (m, 1H), 3.88 - 3.81 (m, 1H), 3.75 (s, 3H), 3.69 - 3.63 (m, 1H), 3.59 - 3.53 (m, 1H), 3.43 - 3.33 (m, 2H), 2.61 - 2.44 (m, 3H), 1.24 - 1.16 (m, 3H), 0.93 - 0.79 (m, 1H), 0.48 - 0.34 (m, 1H), 0.33 - 0.20 (m, 2H) 0.10 - 0.00 (m, 1H).

**Example 330:**

Synthetic Route:

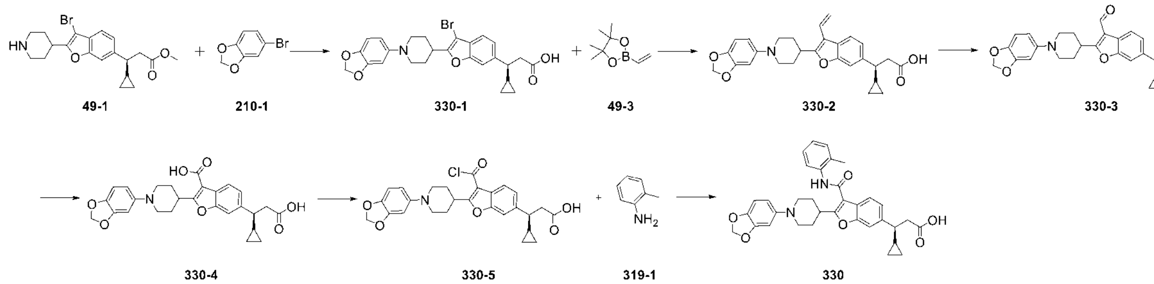

49-1 210-1 330-1 49-3 330-2 330-3

330-4 330-5 319-1 330

Referring to the synthetic route of compound **49,** compound 11-1 was replaced with compound **210-1** to synthesize compound **330-4.** Then, referring to the synthetic route of compound **319,** compound **305-4** was replaced with compound **330-4** to carry out the synthesis, and the resulting crude product was purified by reverse-phase column chromatography [acetonitrile/water (0.05% formic acid) = 0% to 100%] to obtain compound **330** (24 mg, yield: 54%) as a white solid. MS (ESI, m/z): 567.2 $[M+H]^+$.

$^1H$ NMR (400 MHz, $CDCl_3$) δ 8.04 - 7.94 (m, 1H), 7.68 (d, *J* = 8.0 Hz, 1H), 7.61 (s, 1H), 7.47 (s, 1H), 7.33 - 7.25 (m, 3H), 7.17 - 7.13 (m, 1H), 6.74 (d, *J* = 8.4 Hz, 1H), 6.64 (d, J = 2.4 Hz, 1H), 6.45 (dd, *J* = 8.4, 2.4 Hz, 1H), 5.92 (s, 2H), 3.78 - 3.73 (m, 1H), 3.64 - 3.56 (m, 2H), 3.05 - 2.69 (m, 4H), 2.55 - 2.47 (m, 1H), 2.37 (s, 3H), 2.29 - 2.13 (m, 2H), 2.08 - 1.98 (m, 2H), 1.27- 1.06 (m, 1H), 0.69 - 0.62 (m, 1H), 0.51- 0.45 (m, 1H), 0.39 - 0.33 (m, 1H), 0.24 - 0.18 (m, 1H).

**Example 331:**

Synthetic Route:

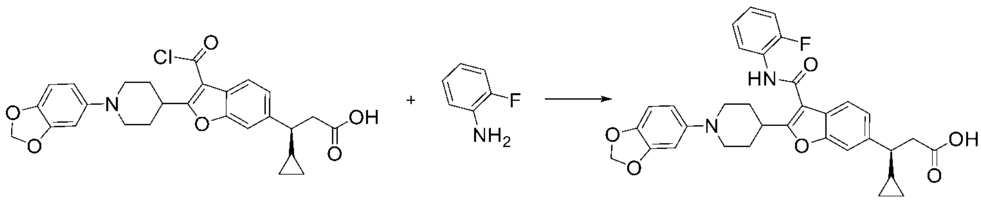

330-5 323-1 331

Referring to the synthetic route of compound **330,** compound **319-1** was replaced with compound **323-1** to carry out the synthesis. The resulting crude product was purified by reverse-phase column chromatography [acetonitrile/water (0.05% formic acid) = 0% to 100%] to obtain compound **331** (15 mg, yield: 54%) as a white solid. MS (ESI, m/z): 571.2 $[M+H]^+$.

$^1H$ NMR (400 MHz, $CDCl_3$) δ 8.52 - 8.44 (m, 1H), 8.01 (s, 1H), 7.70 (d, *J* = 8.0 Hz, 1H), 7.46 (s, 1H), 7.33 - 7.27 (m, 1H), 7.24 - 7.11 (m, 3H), 6.75 (d, *J* = 8.8 Hz, 1H), 6.70 - 6.61 (m, 1H), 6.54 - 6.42 (m, 1H), 5.92 (s, 2H), 3.84 - 3.74 (m, 1H), 3.68 - 3.56 (m, 2H), 2.97 - 2.81 (m, 4H), 2.57 - 2.47 (m, 1H), 2.33 - 2.17 (m, 2H), 2.11 - 1.99 (m, 2H), 1.15 - 1.06 (m, 1H), 0.69 - 0.59 (m, 1H), 0.53 - 0.43 (m, 1H), 0.40 - 0.31 (m, 1H), 0.28 - 0.16 (m, 1H).

**Example 332:**

Synthetic Route:

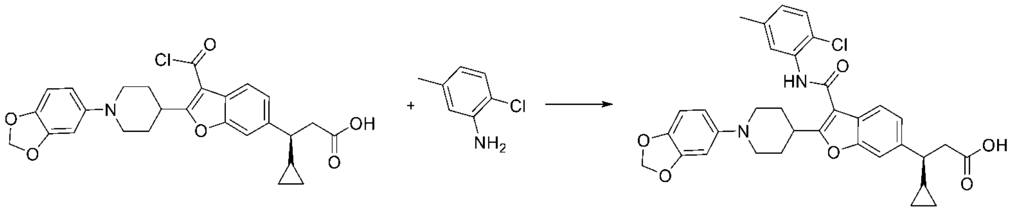

330-5 265-1 332

Referring to the synthetic route of compound **330,** compound **319-1** was replaced with compound **265-1** to carry out the synthesis. The resulting crude product was purified by reverse-phase column chromatography [acetonitrile/water (0.05% formic acid) = 0% to 100%] to obtain compound **332** (22 mg, yield: 62%) as a white solid. MS (ESI, m/z): 601.2 $[M+H]^+$.

$^1H$ NMR (400 MHz, $CDCl_3$) δ 8.48 (s, 1H), 8.33 (s, 1H), 7.86 (d, *J* = 8.0 Hz, 1H), 7.48 (s, 1H), 7.36 - 7.29 (m, 2H), 6.97 - 6.91 (m, 1H), 6.77 (d, *J* = 8.4 Hz, 1H), 6.67 (s, 1H), 6.52 - 6.45 (m, 1H), 5.95 (s, 2H), 3.89 - 3.75 (m, 1H), 3.70 - 3.58 (m, 2H), 2.95 - 2.81 (m, 4H), 2.60 - 2.51 (m, 1H), 2.43 (s, 3H), 2.34 - 2.21 (m, 2H), 2.10 - 2.03 (m, 2H), 1.18 - 1.08 (m, 1H), 0.73 - 0.64 (m, 1H), 0.55 - 0.46 (m, 1H), 0.44 - 0.34 (m, 1H), 0.28 - 0.18 (m, 1H).

**Example 333:**

Synthetic Route:

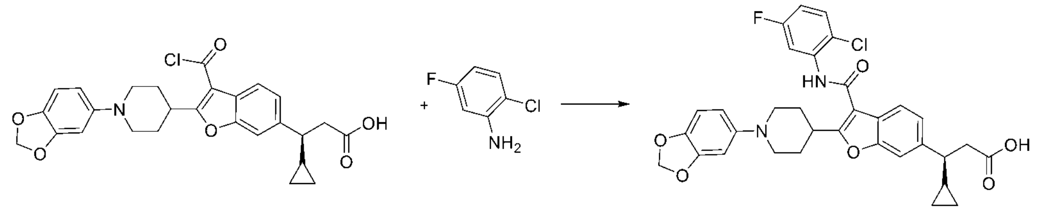

330-5 266-1 333

Referring to the synthetic route of compound **330,** compound **319-1** was replaced with compound **266-1** to carry out the synthesis. The resulting crude product was purified by reverse-phase column chromatography [acetonitrile/water (0.05% formic acid) = 0% to 100%] to obtain compound **333** (20 mg, yield: 71%) as a white solid. MS (ESI, m/z): 605.2 $[M+H]^+$.

$^1$H NMR (400 MHz, $CDCl_3$) δ 8.57-8.49 (m, 1H), 8.40 (s, 1H), 7.84 (d, *J* = 8.0 Hz, 1H), 7.48 (s, 1H), 7.42 (dd, *J* = 8.8, 5.6 Hz, 1H), 7.32 (d, *J* = 8.4 Hz, 1H), 6.91 - 6.81 (m, 1H), 6.77 (d, *J* = 8.4 Hz, 1H), 6.68-6.64 (m, 1H), 6.52 - 6.46 (m, 1H), 5.94 (s, 2H),3.86 - 3.73 (m, 1H), 3.70 - 3.60 (m, 2H), 2.97 - 2.84 (m, 4H), 2.58 - 2.49 (m, 1H), 2.31 - 2.17 (m, 2H), 2.12 - 2.03 (m, 2H), 1.16 - 1.08 (m, 1H), 0.72 - 0.64 (m, 1H), 0.55 - 0.46 (m, 1H), 0.42 - 0.33 (m, 1H), 0.28 - 0.16 (m, 1H).

**Example 334:**

Synthetic Route:

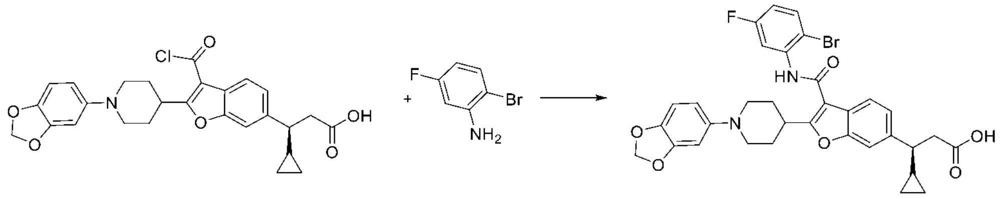

330-5 271-1 334

Referring to the synthetic route of compound **330,** compound **319-1** was replaced with compound **271-1** to carry out the synthesis. The resulting crude product was purified by reverse-phase column chromatography [acetonitrile/water (0.05% formic acid) = 0% to 100%] to obtain compound **334** (18 mg, yield: 55%) as a white solid. MS (ESI, m/z): 649.1 $[M+H]^+$.

$^1$H NMR (400 MHz, $CDCl_3$) δ 8.56 - 8.49 (m, 1H), 8.39 (s, 1H), 7.91 (d, *J* = 8.4 Hz, 1H), 7.58 (dd, *J* = 8.8, 5.6 Hz, 1H), 7.49 (s, 1H), 7.34 - 7.30 (m, 1H), 6.85 - 6.75 (m, 2H), 6.67 (d, *J* = 2.0 Hz, 1H), 6.51 - 6.45 (m, 1H), 5.94 (s, 2H), 3.87 - 3.74 (m, 1H), 3.69 - 3.60 (m, 2H), 2.94 - 2.83 (m, 4H), 2.58 - 2.50 (m, 1H), 2.33 - 2.18 (m, 2H), 2.12 - 2.03 (m, 2H), 1.18 - 1.07 (m, 1H), 0.71 - 0.61 (m, 1H), 0.56 - 0.45 (m, 1H), 0.43 - 0.34 (m, 1H), 0.29 - 0.18 (m, 1H).

**Example 335:**

Synthetic Route:

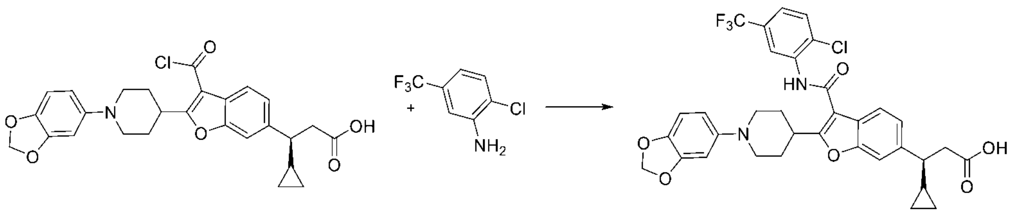

330-5 275-1 335

Referring to the synthetic route of compound **330,** compound **319-1** was replaced with compound **275-1** to carry

out the synthesis. The resulting crude product was purified by reverse-phase column chromatography [acetonitrile/water (0.05% formic acid) = 0% to 100%] to obtain compound **335** (34 mg, yield: 83%) as a white solid. MS (ESI, m/z): 655.2 $[M+H]^+$.

$^1$H NMR (400 MHz, $CDCl_3$) δ 9.06 - 9.02 (m, 1H), 8.45 (s, 1H), 7.83 (d, *J* = 8.0 Hz, 1H), 7.60 (d, *J* = 8.4 Hz, 1H), 7.50 (s, 1H), 7.42 - 7.37 (m, 1H), 7.33 (d, *J* = 8.0 Hz, 1H), 6.77 (d, *J* = 8.4 Hz, 1H), 6.72 - 6.65 (m, 1H), 6.54 - 6.44 (m, 1H), 5.95 (s, 2H), 3.89 - 3.78 (m, 1H), 3.69 - 3.60 (m, 2H), 2.94 - 2.83 (m, 4H), 2.59 - 2.49 (m, 1H), 2.34 - 2.22 (m, 2H), 2.11 - 2.03 (m, 2H), 1.18 - 1.07 (m, 1H), 0.74 - 0.63 (m, 1H), 0.56 - 0.46 (m, 1H), 0.43 - 0.34 (m, 1H), 0.31 - 0.20 (m, 1H).

**Example 336:**

Synthetic Route:

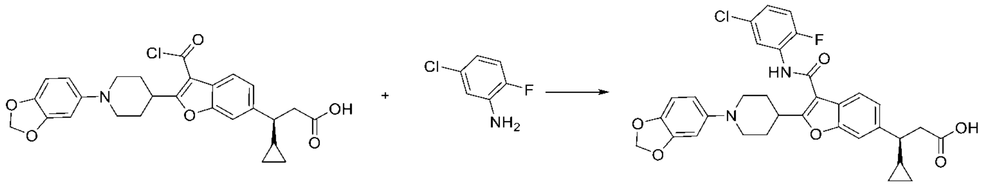

330-5 270-1 336

Referring to the synthetic route of compound **330,** compound **319-1** was replaced with compound **270-1** to carry out the synthesis. The resulting crude product was purified by reverse-phase column chromatography [acetonitrile/water (0.05% formic acid) = 0% to 100%] to obtain compound **336** (25 mg, yield: 60%) as a white solid. MS (ESI, m/z): 655.2 $[M+H]^+$.

$^1$H NMR (400 MHz, $CDCl_3$) δ 8.66 - 8.61 (m, 1H), 8.05 - 7.99 (m, 1H), 7.68 (d, *J* = 8.0 Hz, 1H), 7.49 (s, 1H), 7.34 - 7.30 (m, 1H), 7.14 - 7.08 (m, 2H), 6.77 (d, *J* = 8.4 Hz, 1H), 6.68 (s, 1H), 6.56 - 6.45 (m, 1H), 5.95 (s, 2H), 3.84 - 3.73 (m, 1H), 3.68 - 3.59 (m, 2H), 2.97 - 2.82 (m, 4H), 2.59 - 2.48 (m, 1H), 2.32 - 2.17 (m, 2H), 2.09 - 2.02 (m, 2H), 1.17 - 1.07 (m, 1H), 0.75 - 0.63 (m, 1H), 0.54 - 0.45 (m, 1H), 0.43 - 0.34 (m, 1H), 0.27 - 0.19 (m, 1H).

**Example 337:**

Synthetic Route:

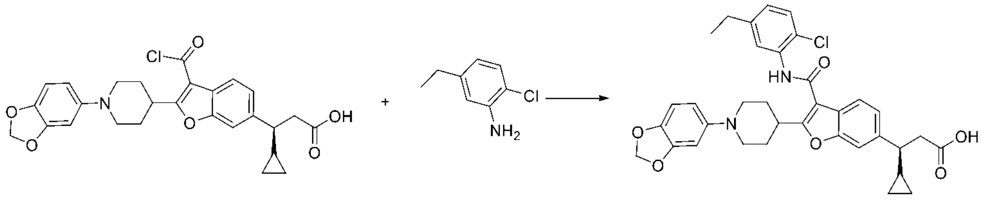

330-5 276-1 337

Referring to the synthetic route of compound **330,** compound **319-1** was replaced with compound **276-1** to carry out the synthesis. The resulting crude product was purified by reverse-phase column chromatography [acetonitrile/water (0.05% formic acid) = 0% to 100%] to obtain compound **337** (18 mg, yield: 53%) as a white solid. MS (ESI, m/z): 615.2 $[M+H]^+$.

$^1$H NMR (400 MHz, $CDCl_3$) δ 8.53 - 8.48 (m, 1H), 8.34 (s, 1H), 7.86 (d, *J* = 8.4 Hz, 1H), 7.48 (s, 1H), 7.37 (d, *J* = 8.4 Hz, 1H), 7.32 - 7.29 (m, 1H), 6.97 (dd, *J* = 8.4, 2.0 Hz, 1H), 6.77 (d, *J* = 8.4 Hz, 1H), 6.71 - 6.66 (m, 1H), 6.53 - 6.44 (m, 1H), 5.95 (s, 2H), 3.88 - 3.78 (m, 1H), 3.68 - 3.60 (m, 2H), 2.94 - 2.83 (m, 4H), 2.72 (q, *J* = 7.6 Hz, 2H), 2.58 - 2.50 (m, 1H), 2.33 - 2.20 (m, 2H), 2.12 - 2.02 (m, 2H), 1.30 (t, *J* = 7.6 Hz, 3H), 1.18 - 1.08 (m, 1H), 0.71 - 0.61 (m, 1H), 0.55 - 0.46 (m, 1H), 0.42 - 0.34 (m, 1H), 0.29 - 0.20 (m, 1H).

**Example 338:**

Synthetic Route:

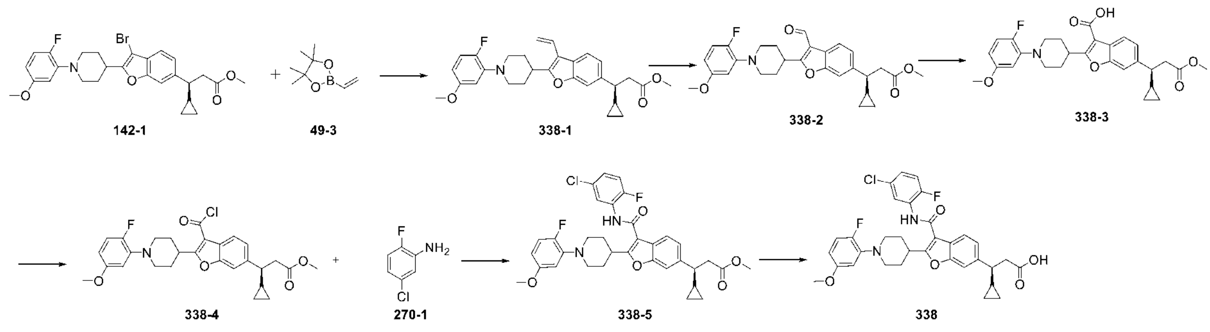

Referring to the synthetic route of compound **305,** compound **98-1** was replaced with compound **142-1** to synthesize compound **338-4.** Then, referring to the synthetic route of compound **270,** compound **234-1** was replaced with compound **338-4** to carry out the synthesis, and the resulting crude product was purified by reverse-phase column chromatography [acetonitrile/water (0.05% formic acid) = 0% to 100%] to obtain compound **338** (32 mg, yield: 68%) as a white solid. MS (ESI, m/z): 609.2 [M+H]$^+$.

$^1$H NMR (400 MHz, $CDCl_3$) δ 8.66 - 8.62 (m, 1H), 8.03 (d, *J* = 3.2 Hz, 1H), 7.69 (d, *J* = 8.0 Hz, 1H), 7.48 (s, 1H), 7.32 (d, *J* = 8.4 Hz, 1H), 7.17 - 7.08 (m, 2H), 6.99 (dd, *J* = 12.0, 8.8 Hz, 1H), 6.63 - 6.54 (m, 1H), 6.51 - 6.43 (m, 1H), 3.87 - 3.78 (m, 4H), 3.67 - 3.58 (m, 2H), 2.96 - 2.84 (m, 4H), 2.58 - 2.50 (m, 1H), 2.35 - 2.22 (m, 2H), 2.09 - 2.04 (m, 2H), 1.15 - 1.09 (m, 1H), 0.72 - 0.64 (m, 1H), 0.53 - 0.46 (m, 1H), 0.42 - 0.34 (m, 1H), 0.29 - 0.19 (m, 1H).

**Example 339:**

Synthetic Route:

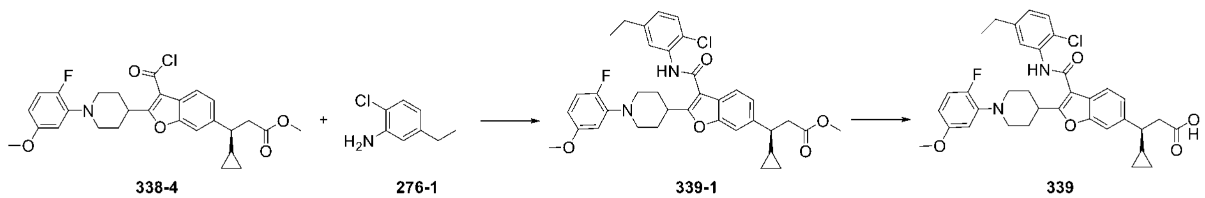

Referring to the synthetic route of compound **338,** compound **270-1** was replaced with compound **276-1** to carry out the synthesis. The resulting crude product was purified by reverse-phase column chromatography [acetonitrile/water (0.05% formic acid) = 0% to 100%] to obtain compound **339** (22 mg, yield: 58%) as a white solid. MS (ESI, m/z): 619.2 [M+H]$^+$.

$^1$H NMR (400 MHz, $CDCl_3$) δ 8.54 - 6.48 (m, 1H), 8.34 (s, 1H), 7.86 (d, *J* = 8.4 Hz, 1H), 7.47 (s, 1H), 7.37 (d, *J* = 8.4 Hz, 1H), 7.32 - 7.30 (m, 1H), 7.04 - 6.94 (m, 2H), 6.59 (dd, *J* = 7.2, 2.8 Hz, 1H), 6.50-6.43 (m, 1H), 3.90 - 3.78 (m, 4H), 3.66 - 3.59 (m, 2H), 2.95 - 2.85 (m, 4H), 2.72 (q, *J* = 7.6 Hz, 2H), 2.59 - 2.49 (m, 1H), 2.36 - 2.25 (m, 2H), 2.14 - 2.03 (m, 2H), 1.31 (t, *J* = 7.6 Hz, 3H), 1.18 - 1.08 (m, 1H), 0.72 - 0.63 (m, 1H), 0.52 - 0.45 (m, 1H), 0.41 - 0.33 (m, 1H), 0.29 - 0.20 (m, 1H).

**Example 340:**

Synthetic Route:

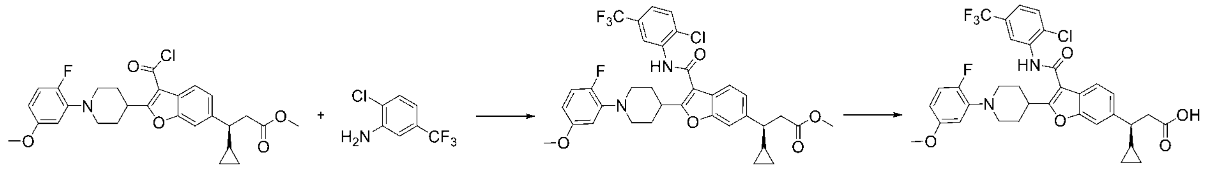

338-4 275-1 340-1 340

Referring to the synthetic route of compound **338,** compound **270-1** was replaced with compound **275-1** to carry out the synthesis. The resulting crude product was purified by reverse-phase column chromatography [acetonitrile/water (0.05% formic acid) = 0% to 100%] to obtain compound **340** (17 mg, yield: 53%) as a white solid. MS (ESI, m/z): 659.2 $[M+H]^+$.

$^1H$ NMR (400 MHz, $CDCl_3$) δ 9.06 - 9.03 (m, 1H), 8.46 (s, 1H), 7.84 (d, *J* = 8.4 Hz, 1H), 7.60 (d, *J* = 8.4 Hz, 1H), 7.49 (s, 1H), 7.39 (dd, *J* = 6.8, 2.4 Hz, 1H), 7.33 (dd, *J* = 8.4, 1.2 Hz, 1H), 6.99 (dd, *J* = 12.0, 8.8 Hz, 1H), 6.69 - 6.62 (m, 1H), 6.52 - 6.43 (m, 1H), 3.90 - 3.79 (m, 4H), 3.66 - 3.60 (m, 2H), 2.99 - 2.83 (m, 4H), 2.61 - 2.50 (m, 1H), 2.41 - 2.25 (m, 2H), 2.15 - 2.05 (m, 2H), 1.18 - 1.10 (m, 1H), 0.75 - 0.65 (m, 1H), 0.56 - 0.45 (m, 1H), 0.42 - 0.33 (m, 1H), 0.27 - 0.20 (m, 1H).

**Example 341:**

Synthetic Route:

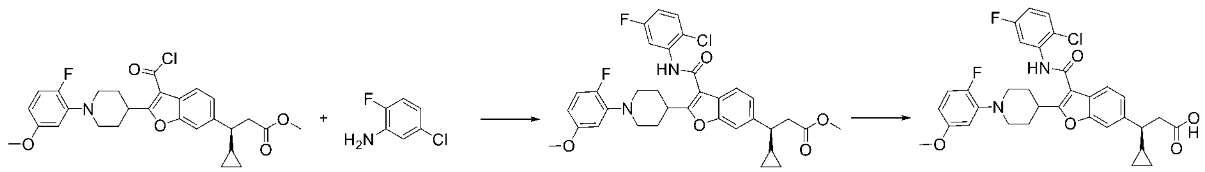

338-4 266-1 341-1 341

Referring to the synthetic route of compound **338,** compound **270-1** was replaced with compound **266-1** to carry out the synthesis. The resulting crude product was purified by reverse-phase column chromatography [acetonitrile/water (0.05% formic acid) = 0% to 100%] to obtain compound **341** (15 mg, yield: 50%) as a white solid. MS (ESI, m/z): 609.2 $[M+H]^+$.

$^1H$ NMR (400 MHz, $CDCl_3$) δ 8.54 (dd, *J* = 10.8, 3.2 Hz, 1H), 8.41 (s, 1H), 7.85 (d, *J* = 8.0 Hz, 1H), 7.49 (s, 1H), 7.42 (dd, *J* = 8.8, 5.6 Hz, 1H), 7.32 (d, *J* = 8.4 Hz, 1H), 6.99 (dd, *J* = 12.0, 8.8 Hz, 1H), 6.89-6.82 (m, 1H), 6.63-6.57 (m, 1H), 6.51-6.44 (m, 1H), 3.89 - 3.80 (m, 4H), 3.68 - 3.59 (m, 2H), 2.99 - 2.86 (m, 4H), 2.60 - 2.48 (m, 1H), 2.40 - 2.27 (m, 2H), 2.10 - 2.03 (m, 2H), 1.18 - 1.09 (m, 1H), 0.76 - 0.64 (m, 1H), 0.58 - 0.48 (m, 1H), 0.43 - 0.31 (m, 1H), 0.27 - 0.19 (m, 1H).

**Example 342:**

Synthetic Route:

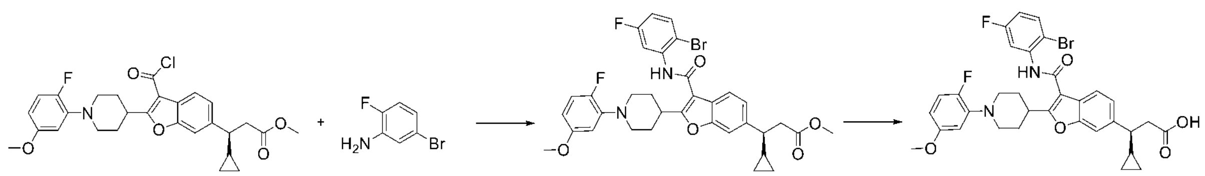

338-4 271-1 342-1 342

Referring to the synthetic route of compound **338,** compound **270-1** was replaced with compound **271-1** to carry out the synthesis. The resulting crude product was purified by reverse-phase column chromatography [acetonitrile/water (0.05% formic acid) = 0% to 100%] to obtain compound **342** (15 mg, yield: 54%) as a white solid. MS (ESI, m/z): 653.1 $[M+H]^+$.

$^1H$ NMR (400 MHz, $CDCl_3$) δ 8.54 (dd, *J* = 10.8, 3.2 Hz, 1H), 8.41 (s, 1H), 7.85 (d, *J* = 8.0 Hz, 1H), 7.49 (s, 1H), 7.42 (dd, *J* = 8.8, 5.6 Hz, 1H), 7.32 (d, *J* = 8.4 Hz, 1H), 6.99 (dd, *J* = 12.0, 8.8 Hz, 1H), 6.88 - 6.83 (m, 1H), 6.63 - 6.57 (m, 1H), 6.51

- 6.44 (m, 1H), 3.88 - 3.79 (m, 4H), 3.69 - 3.60 (m, 2H), 2.97 - 2.86 (m, 4H), 2.60 - 2.50 (m, 1H), 2.37 - 2.26 (m, 2H), 2.10 - 2.04 (m, 2H), 1.19 - 1.11 (m, 1H), 0.72 - 0.63 (m, 1H), 0.57 - 0.48 (m, 1H), 0.45 - 0.35 (m, 1H), 0.30 - 0.20 (m, 1H).

**Example 343:**

Synthetic Route:

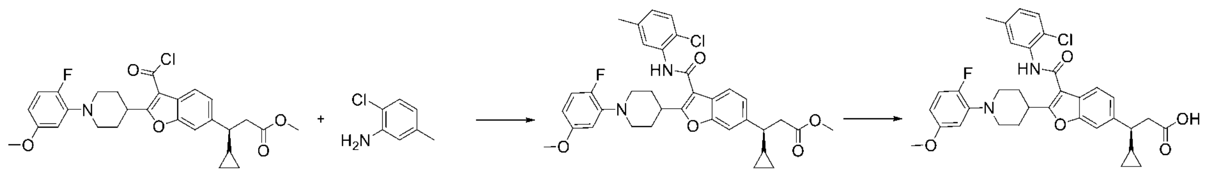

338-4 265-1 343-1 343

Referring to the synthetic route of compound **338,** compound **270-1** was replaced with compound **265-1** to carry out the synthesis. The resulting crude product was purified by reverse-phase column chromatography [acetonitrile/water (0.05% formic acid) = 0% to 100%] to obtain compound **343** (17 mg, yield: 50%) as a white solid. MS (ESI, m/z): 605.1 $[M+H]^+$.

$^1H$ NMR (400 MHz, $CDCl_3$) δ 8.49 (s, 1H), 8.34 (s, 1H), 7.87 (d, *J* = 8.0 Hz, 1H), 7.48 (s, 1H), 7.37 - 7.31 (m, 2H), 7.03 - 6.92 (m, 2H), 6.65 - 6.55 (m, 1H), 6.51 - 6.42 (m, 1H), 3.91 - 3.80 (m, 4H), 3.66 - 3.58 (m, 2H), 2.97 - 2.85 (m, 4H), 2.59 - 2.51 (m, 1H), 2.43 (s, 3H), 2.37 - 2.26 (m, 2H), 2.13 - 2.06 (m, 2H), 1.18 - 1.08 (m, 1H), 0.72 - 0.61 (m, 1H), 0.57 - 0.46 (m, 1H), 0.43 - 0.35 (m, 1H), 0.31 - 0.19 (m, 1H).

**Example 344:**

Synthetic Route:

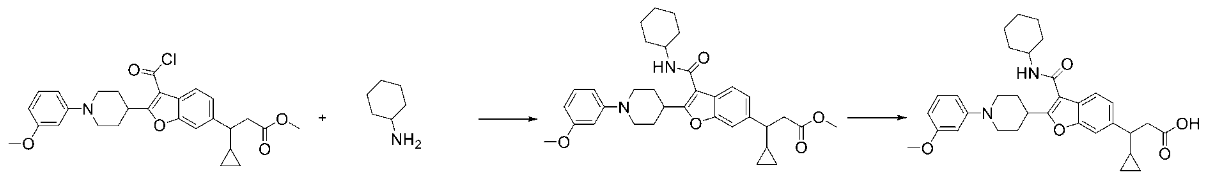

235-1 344-1 344-2 344

Referring to the synthetic route of compound **235,** compound **235-2** was replaced with compound **344-1** to carry out the synthesis. The resulting crude product was purified by reverse-phase column chromatography [acetonitrile/water (0.05% formic acid) = 0% to 100%] to obtain compound **344** (12 mg, yield: 50%) as a white solid. MS (ESI, m/z): 545.3 $[M+H]^+$.

$^1H$ NMR (400 MHz, DMSO-$d_6$) δ 7.91 (d, *J* = 8.0 Hz, 1H), 7.51 - 7.37 (m, 2H), 7.18 (d, *J* = 8.0 Hz, 1H), 7.14 - 7.06 (m, 1H), 6.56 (dd, *J* = 8.2, 2.0 Hz, 1H), 6.51 - 6.45 (m, 1H), 6.35 (dd, *J* = 8.0, 2.1 Hz, 1H), 3.87 - 3.74 (m, 3H), 3.71 (s, 3H), 3.47 - 3.39 (m, 1H), 2.82 - 2.72 (m, 2H), 2.61 - 2.50 (m, 2H), 2.42 - 2.36 (m, 1H), 2.03 - 1.64 (m, 8H), 1.61 - 1.54 (m, 1H), 1.40 - 1.24 (m, 4H), 1.19 - 1.08 (m, 1H), 1.04 - 0.95 (m, 1H), 0.51 - 0.42 (m, 1H), 0.29 - 0.21 (m, 2H), 0.11 - 0.04 (m, 1H).

**Example 345:**

Synthetic Route:

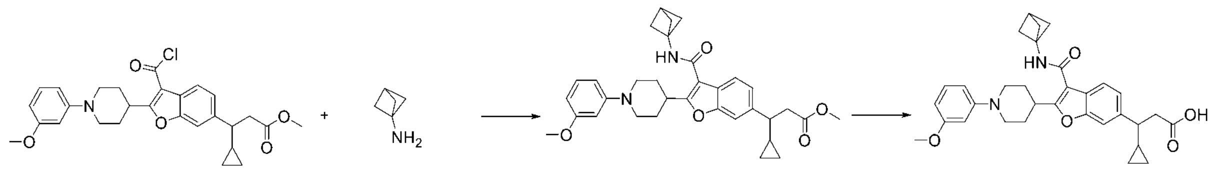

235-1 345-1 345-2 345

Referring to the synthetic route of compound **235,** compound **235-2** was replaced with compound **345-1** to carry out the synthesis. The resulting crude product was purified by reverse-phase column chromatography [acetonitrile/water (0.05% formic acid) = 0% to 100%] to obtain compound **345** (4 mg, yield: 8%) as a white solid. MS (ESI, m/z): 529.3 [M+H]$^+$.

$^1$H NMR (400 MHz, DMSO-$d_6$) δ 8.55 (s, 1H), 7.48 (d, *J* = 8.0 Hz, 1H), 7.39 (s, 1H), 7.18 (d, *J* = 8.2 Hz, 1H), 7.15 - 7.08 (m, 1H), 6.57 (dd, *J* = 8.2, 2.0 Hz, 1H), 6.52 - 6.46 (m, 1H), 6.36 (dd, *J* = 8.0, 2.0 Hz, 1H), 3.88 - 3.77 (m, 2H), 3.73 (s, 3H), 3.52 - 3.44 (m, 1H), 2.86 - 2.75 (m, 2H), 2.48 - 2.42 (m, 2H), 2.40 - 2.22 (m, 2H), 2.10 (s, 6H), 2.00 - 1.84 (m, 4H), 1.01 - 0.91 (m, 1H), 0.47 - 0.38 (m, 1H), 0.29 - 0.19 (m, 2H), 0.10 - 0.02 (m, 1H).

**Example 346:**

Synthetic Route:

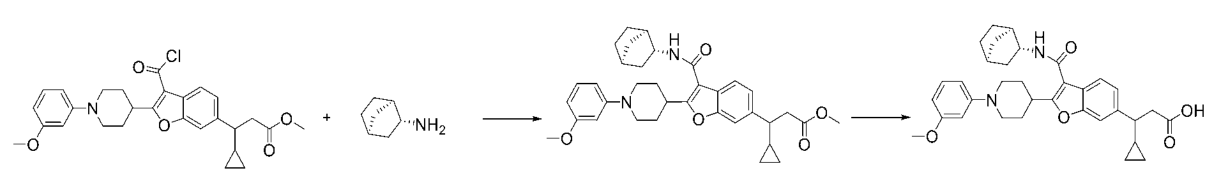

235-1 346-1 346-2 346

Referring to the synthetic route of compound **235,** compound **235-2** was replaced with compound **346-1** to carry out the synthesis. The resulting crude product was purified by reverse-phase column chromatography [acetonitrile/water (0.05% formic acid) = 0% to 100%] to obtain compound **346** (9 mg, yield: 31%) as a white solid. MS (ESI, m/z): 557.3 [M+H]$^+$.

$^1$H NMR (400 MHz, DMSO-$d_6$) δ 8.09 (d, *J* = 6.4 Hz, 1H), 7.52 - 7.45 (m, 2H), 7.22 (d, *J* = 8.4 Hz, 1H), 7.15 - 7.08 (m, 1H), 6.57 (dd, *J* = 8.4, 2.0 Hz, 1H), 6.52 - 6.47 (m, 1H), 6.36 (dd, *J* = 8.0, 2.0 Hz, 1H), 4.19 - 4.09 (m, 1H), 3.87-3.78 (m, 2H), 3.73 (s, 3H), 3.46 - 3.38 (m, 1H), 2.84 - 2.62 (m, 4H), 2.48 - 2.44 (m, 1H), 2.42 - 2.34 (m, 1H), 2.20 - 2.15 (m, 1H), 2.01 - 1.82 (m, 5H), 1.74 - 1.66 (m, 1H), 1.56 - 1.25 (m, 5H), 1.15 - 0.99 (m, 2H), 0.55 - 0.47 (m, 1H), 0.33 - 0.22 (m, 2H), 0.18 - 0.09 (m, 1H).

**Example 347:**

Synthetic Route:

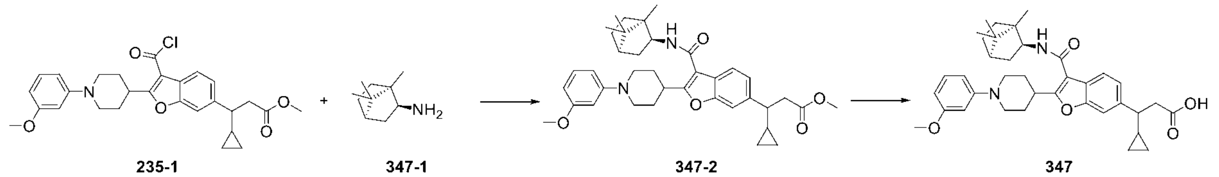

235-1 347-1 347-2 347

Referring to the synthetic route of compound **235,** compound **235-2** was replaced with compound **347-1** to carry out the synthesis. The resulting crude product was purified by reverse-phase column chromatography [acetonitrile/water (0.05% formic acid) = 0% to 100%] to obtain compound **347** (2 mg, yield: 5%) as a white solid. MS (ESI, m/z): 599.4 [M+H]$^+$.

$^1$H NMR (400 MHz, DMSO-$d_6$) δ 11.97 (s, 1H), 8.10 (d, *J* = 8.6 Hz, 1H), 7.54 - 7.46 (m, 2H), 7.24 (d, *J* = 8.2 Hz, 1H), 7.20 - 7.10 (m, 1H), 6.81 - 6.26 (m, 3H), 4.48 - 4.35 (m, 1H), 3.88 - 3.76 (m, 2H), 3.73 (s, 3H), 2.97 - 2.65 (m, 4H), 2.42 - 2.33 (m, 1H), 2.24 - 2.12 (m, 1H), 2.03 - 1.97 (m, 2H), 1.92 - 1.77 (m, 2H), 1.72 - 1.60 (m, 2H), 1.41 - 1.29 (m, 2H), 1.26 - 1.19 (m, 2H), 1.15 - 1.02 (m, 2H), 0.96 (s, 3H), 0.88 (s, 3H), 0.83 (s, 3H), 0.57 - 0.47 (m, 1H), 0.32 - 0.23 (m, 2H), 0.18 - 0.09 (m, 1H).

**Example 348:**

Synthetic Route:

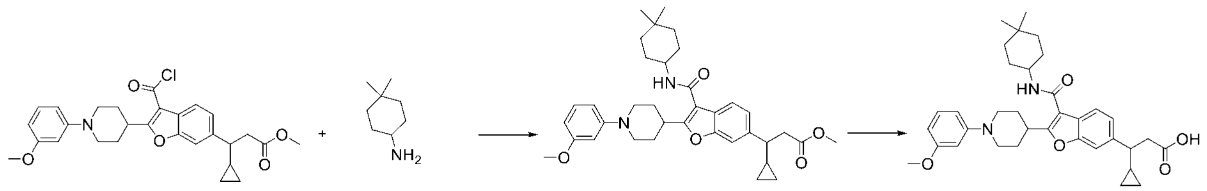

235-1 348-1 348-2 348

Referring to the synthetic route of compound **235,** compound **235-2** was replaced with compound **348-1** to carry out the synthesis. The resulting crude product was purified by reverse-phase column chromatography [acetonitrile/water (0.05% formic acid) = 0% to 100%] to obtain compound **348** (5 mg, yield: 10%) as a white solid. MS (ESI, m/z): 573.2 $[M+H]^+$.

$^1$H NMR (400 MHz, DMSO-$d_6$) δ 7.92 (d, *J* = 8.0 Hz, 1H), 7.56 - 7.45 (m, 2H), 7.21 (d, *J* = 8.4 Hz, 1H), 7.15 - 7.08 (m, 1H), 6.61 - 6.55 (m, 1H), 6.51 - 6.47 (m, 1H), 6.36 (dd, *J* = 8.0, 2.0 Hz, 1H), 3.88 - 3.78 (m, 2H), 3.78 - 3.67 (m, 4H), 3.41 - 3.52 (m, 1H), 2.86 - 2.74 (m, 2H), 2.74 - 2.64 (m, 2H), 2.39 - 2.33 (m, 1H), 2.01 - 1.85 (m, 4H), 1.74 - 1.63 (m, 2H), 1.63 - 1.58 (m, 2H), 1.46 - 1.36 (m, 2H), 1.31 - 1.20 (m, 2H), 1.09 - 1.00 (m, 1H), 0.93 (s, 6H), 0.55 - 0.48 (m, 1H), 0.32 - 0.21 (m, 2H), 0.16 - 0.09 (m, 1H).

**Example 349:**

Synthetic Route:

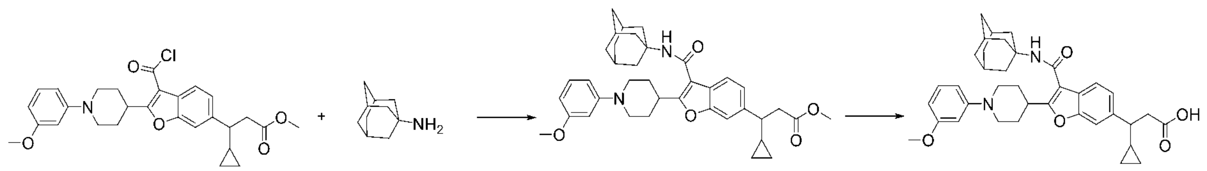

235-1 349-1 349-2 349

Referring to the synthetic route of compound **235,** compound **235-2** was replaced with compound **349-1** to carry out the synthesis. The resulting crude product was purified by reverse-phase column chromatography [acetonitrile/water (0.05% formic acid) = 0% to 100%] to obtain compound **349** (9 mg, yield: 10%) as a white solid. MS (ESI, m/z): 597.4 $[M+H]^+$.

$^1$H NMR (400 MHz, DMSO-$d_6$) δ 12.14 (s, 1H), 7.50 - 7.40 (m, 3H), 7.16 (d, *J* = 8.6 Hz, 1H), 7.12 - 7.04 (m, 1H), 6.54 (dd, *J* = 8.2, 2.0 Hz, 1H), 6.48 - 6.43 (m, 1H), 6.33 (dd, *J* = 8.0, 2.0 Hz, 1H), 3.84 - 3.75 (m, 2H), 3.69 (s, 3H), 3.43 - 3.28 (m, 3H), 2.85 - 2.73 (m, 2H), 2.71 - 2.59 (m, 2H), 2.38 - 2.28 (m, 1H), 2.07 - 2.01 (m, 8H), 1.93 - 1.85 (m, 3H), 1.64 (s, 6H), 1.09 - 0.96 (m, 1H), 0.52 - 0.43 (m, 1H), 0.28 - 0.19 (m, 2H), 0.14 - 0.04 (m, 1H).

**Example 350:**

Synthetic Route:

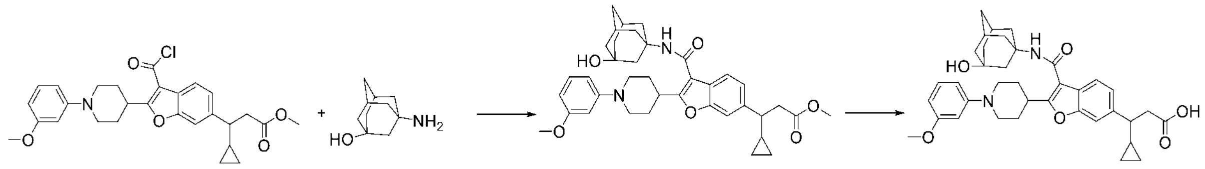

235-1 350-1 350-2 350

Referring to the synthetic route of compound **235,** compound **235-2** was replaced with compound **350-1** to carry out the synthesis. The resulting crude product was purified by reverse-phase column chromatography [acetonitrile/water (0.05% formic acid) = 0% to 100%] to obtain compound **350** (6 mg, yield: 20%) as a white solid. MS (ESI, m/z): 613.3 $[M+H]^+$.

$^1H$ NMR (400 MHz, DMSO-$d_6$) δ 7.56 (s, 1H), 7.46 (d, *J* = 8.0 Hz, 1H), 7.42 (s, 1H), 7.21 (d, *J* = 8.0 Hz, 1H), 7.19 - 7.13 (m, 1H), 6.62 (dd, *J* = 8.2, 2.0 Hz, 1H), 6.56 - 6.52 (m, 1H), 6.40 (dd, *J* = 8.0, 2.0 Hz, 1H), 4.57 (s, 1H), 3.92 - 3.81 (s, 2H), 3.77 (s, 3H), 3.48 - 3.40 (m, 1H), 2.90 - 2.79 (m, 2H), 2.53 - 2.47 (m, 1H), 2.41 - 2.25 (m, 2H), 2.25 - 2.18 (m, 2H), 2.08 - 1.88 (m, 10H), 1.61 (d, *J* = 8.6 Hz, 6H), 1.06 - 0.96 (m, 1H), 0.51 - 0.42 (m, 1H), 0.32 - 0.23 (m, 2H), 0.11 - 0.06 (m, 1H).

**Example 351:**

Synthetic Route:

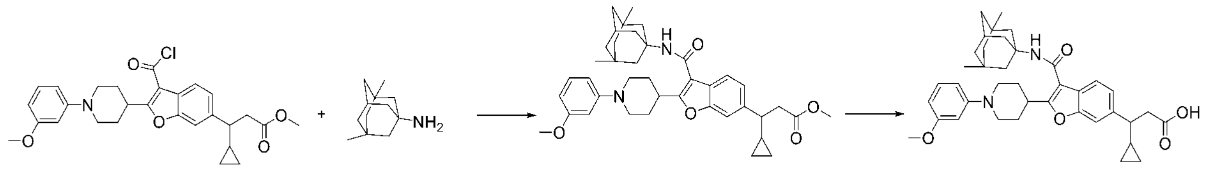

235-1 351-1 351-2 351

Referring to the synthetic route of compound **235,** compound **235-2** was replaced with compound **351-1** to carry out the synthesis. The resulting crude product was purified by reverse-phase column chromatography [acetonitrile/water (0.05% formic acid) = 0% to 100%] to obtain compound **351** (3 mg, yield: 6%) as a white solid. MS (ESI, m/z): 625.3 $[M+H]^+$.

$^1H$ NMR (400 MHz, $CDCl_3$) δ 7.48 (d, *J* = 8.0 Hz, 1H), 7.36 (s, 1H), 7.22 - 7.15 (m, 2H), 6.60 (dd, *J* = 8.2, 2.0 Hz, 1H), 6.54 - 6.50 (m, 1H), 6.41 (dd, *J* = 8.2, 2.2 Hz, 1H), 5.72 (s, 1H), 3.82 - 3.71 (m, 6H), 2.98 - 2.88 (m, 2H), 2.88 - 2.76 (m, 2H), 2.54 - 2.44 (m, 1H), 2.18 - 2.06 (m, 2H), 2.01 - 1.94 (m, 4H), 1.85 - 1.76 (m, 4H), 1.48 - 1.43 (m, 2H), 1.38 - 1.25 (m, 5H), 1.09 - 1.03 (m, 1H), 0.90 (s, 6H), 0.66 - 0.58 (m, 1H), 0.48 - 0.39 (m, 1H), 0.37 - 0.29 (m, 1H), 0.22 - 0.12 (m, 1H).

**Example 352:**

Synthetic Route:

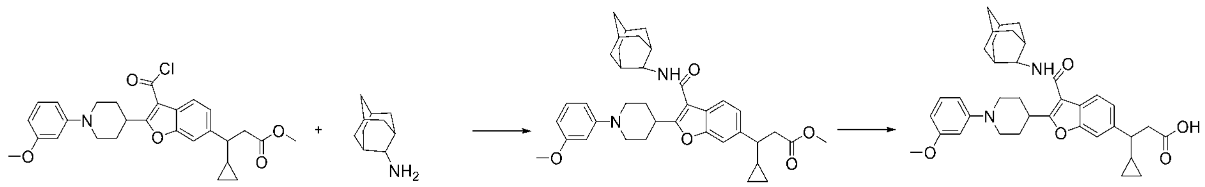

235-1 352-1 352-2 352

Referring to the synthetic route of compound **235,** compound **235-2** was replaced with compound **352-1** to carry out the synthesis. The resulting crude product was purified by reverse-phase column chromatography [acetonitrile/water (0.05% formic acid) = 0% to 100%] to obtain compound **352** (8 mg, yield: 28%) as a white solid. MS (ESI, m/z): 597.3 $[M+H]^+$.

$^1H$ NMR (400 MHz, $CD_3OD$) δ 7.51 (d, *J* = 8.2 Hz, 1H), 7.40 (s, 1H), 7.24 (dd, *J* = 8.2, 1.2 Hz, 1H), 7.14 - 7.07 (m, 1H), 6.58 (dd, *J*= 8.0, 2.0 Hz, 1H), 6.54 - 6.50 (m, 1H), 6.40 (dd, *J* = 8.0, 2.0 Hz, 1H), 4.20 - 4.14 (m, 1H), 3.81 - 3.70 (m, 5H), 3.53 - 3.44 (m, 1H), 2.83 - 2.72 (m, 2H), 2.64 (dd, *J* = 13.4, 6.8 Hz, 1H), 2.57 - 2.44 (m, 2H), 2.17 - 1.77 (m, 16H), 1.75 - 1.66

(m, 2H), 1.10 - 0.98 (m, 1H), 0.57 - 0.47 (m, 1H), 0.38 - 0.27 (m, 2H), 0.10 - 0.05 (m, 1H).

**Example 353:**

Synthetic Route:

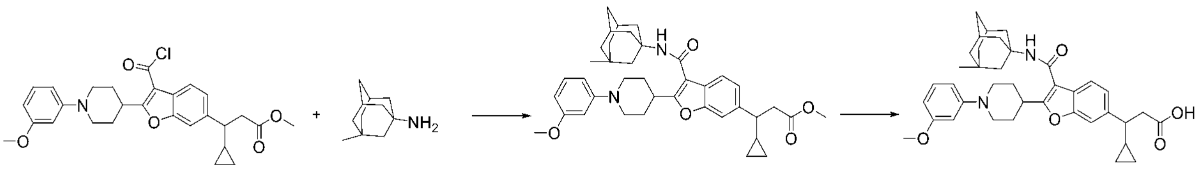

235-1 353-1 353-2 353

Referring to the synthetic route of compound **235,** compound **235-2** was replaced with compound **353-1** to carry out the synthesis. The resulting crude product was purified by reverse-phase column chromatography [acetonitrile/water (0.05% formic acid) = 0% to 100%] to obtain compound **353** (8 mg, yield: 38%) as a white solid. MS (ESI, m/z): 611.3 $[M+H]^+$.

$^1$H NMR (400 MHz, DMSO-$d_6$) δ 7.53 - 7.43 (m, 3H), 7.19 (d, *J* = 8.4 Hz, 1H), 7.14 - 7.09 (m, 1H), 6.57 (d, *J* = 8.4 Hz, 1H), 6.51 - 6.47 (m, 1H), 6.36 (dd, *J* = 8.0, 2.0 Hz, 1H), 3.86 - 3.80 (m, 2H), 3.73 (s, 3H), 3.45 - 3.33 (m, 1H), 2.86 - 2.76 (m, 2H), 2.72 - 2.63 (m, 2H), 2.11 - 1.88 (m, 10H), 1.84 - 1.79 (m, 2H), 1.65 - 1.49 (m, 2H), 1.46 - 1.36 (m, 4H), 1.08 - 0.99 (m, 1H), 0.84 (s, 3H), 0.58 - 0.47 (m, 1H), 0.30 - 0.22 (m, 2H), 0.15 - 0.07 (m, 1H).

**Example 354:**

Synthetic Route:

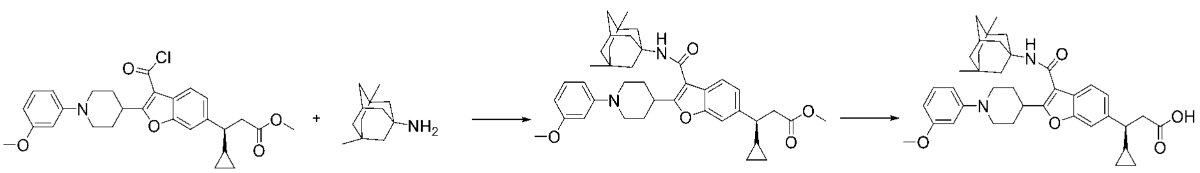

234-1 351-1 354-1 354

Referring to the synthetic route of compound **234,** compound **234-2** was replaced with compound **351-1** to carry out the synthesis. The resulting crude product was purified by reverse-phase column chromatography [acetonitrile/water (0.05% formic acid) = 0% to 100%] to obtain compound **354** (4 mg, yield: 13%) as a white solid. MS (ESI, m/z): 625.3 $[M+H]^+$.

$^1$H NMR (400 MHz, $CDCl_3$) δ 7.49 (d, *J* = 8.4 Hz, 1H), 7.37 (s, 1H), 7.23 - 7.16 (m, 2H), 6.70 - 6.58 (m, 1H), 6.57 - 6.51 (m, 1H), 6.48 - 6.38 (m, 1H), 5.74 (s, 1H), 3.89 - 3.69 (m, 6H), 3.05 - 2.75 (m, 4H), 2.53 - 2.43 (m, 1H), 2.25 - 2.20 (m, 1H), 2.19 - 2.08 (m, 2H), 2.03 - 1.96 (m, 4H), 1.85 - 1.78 (m, 4H), 1.48 - 1.46 (m, 2H), 1.37 - 1.33 (m, 2H), 1.23 - 1.17 (m, 2H), 1.10 - 1.05 (m, 1H), 0.92 (s, 6H), 0.65 - 0.60 (m, 1H), 0.50 - 0.42 (m, 1H), 0.37 - 0.30 (m, 1H), 0.21 - 0.15 (m, 1H).

**Example 355:**

Synthetic Route:

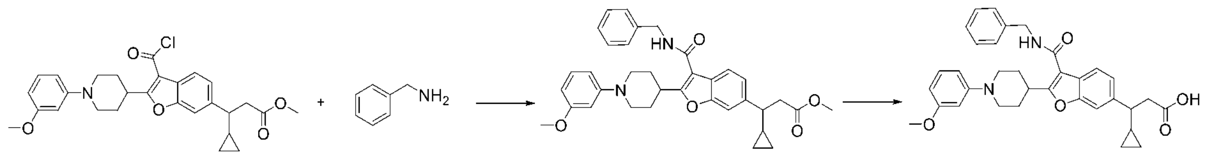

235-1 355-1 355-2 355

Referring to the synthetic route of compound **235,** compound **235-2** was replaced with compound **355-1** to carry out the synthesis. The resulting crude product was purified by reverse-phase column chromatography [acetonitrile/water (0.05% formic acid) = 0% to 100%] to obtain compound **355** (6 mg, yield: 23%) as a white solid. MS (ESI, m/z): 553.3 $[M+H]^+$.

$^1$H NMR (400 MHz, DMSO-$d_6$) δ 12.22 - 11.51 (m, 1H), 8.61 (t, *J* = 5.6 Hz, 1H), 7.62 (d, *J* = 8.0 Hz, 1H), 7.51 (s, 1H), 7.39 - 7.34 (m, 4H), 7.28 - 7.21 (m, 2H), 7.15 - 7.09 (m, 1H), 6.60 - 6.54 (m, 1H), 6.50 - 6.47 (m, 1H), 6.38 - 6.36 (m, 1H), 4.51 (d, *J* = 6.0 Hz, 2H), 3.84 - 3.81 (m, 2H), 3.73 (s, 3H), 3.57 - 3.52 (m, 1H), 2.82 - 2.69 (m, 4H), 2.41 - 2.34 (m, 1H), 1.96 - 1.87 (m, 4H), 1.06 - 1.03 (m, 1H), 0.54 - 0.49 (m, 1H), 0.31 - 0.25(m, 2H), 0.16 - 0.11 (m, 1H).

**Example 356:**

Synthetic Route:

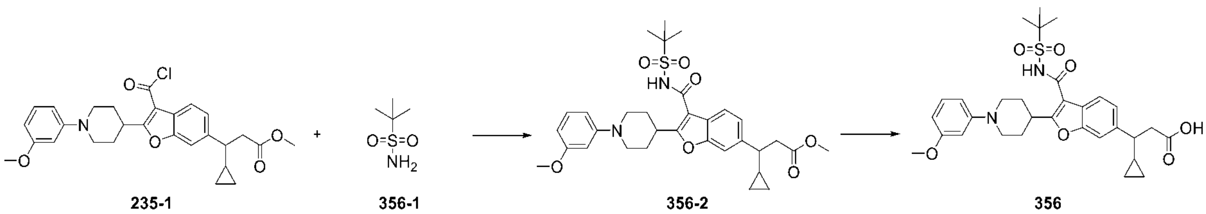

235-1 356-1 356-2 356

Referring to the synthetic route of compound **235,** compound **235-2** was replaced with compound **356-1** to carry out the synthesis. The resulting crude product was purified by reverse-phase column chromatography [acetonitrile/water (0.05% formic acid) = 0% to 100%] to obtain compound **356** (20 mg, yield: 34%) as a white solid. MS (ESI, m/z): 583.3 $[M+H]^+$.

$^1$H NMR (400 MHz, $CDCl_3$) δ 7.58 (d, *J* = 8.0 Hz, 1H), 7.45 (s, 1H), 7.32 - 7.29 (m, 1H), 7.25 - 7.20 (m, 1H), 6.79 - 6.39 (m, 3H), 3.85 - 3.80 (m, 6H), 2.98 - 2.81 (m, 4H), 2.52 - 2.45 (m, 1H), 2.32 - 2.11 (m, 2H), 2.07 - 2.02 (m, 2H), 1.61 (s, 9H), 1.12 - 1.07 (m, 1H), 0.68 - 0.64 (m, 1H), 0.49 - 0.44 (m, 1H), 0.38 - 0.33 (m, 1H), 0.22 - 0.17 (m, 1H).

**Example 357:**

Synthetic Route:

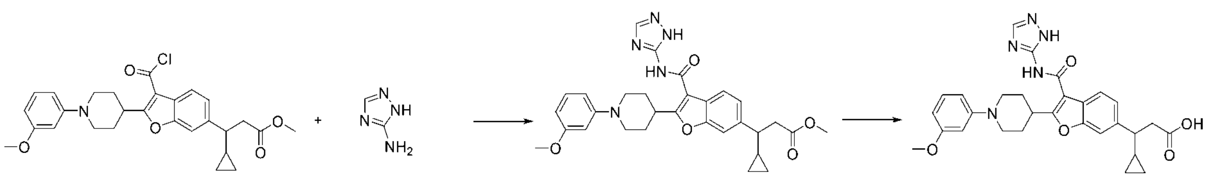

235-1 357-1 357-2 357

Referring to the synthetic route of compound **235,** compound **235-2** was replaced with compound **357-1** to carry out the synthesis. The resulting crude product was purified by reverse-phase column chromatography [acetonitrile/water (0.05% formic acid) = 0% to 100%] to obtain compound **357** (2 mg, yield: 11%) as a white solid. MS (ESI, m/z): 530.2 $[M+H]^+$.

$^1$H NMR (400 MHz, DMSO-$d_6$) δ 8.16 (s, 1H), 7.55 - 7.46 (m, 1H), 7.30 (s, 1H), 7.15 - 7.06 (m, 2H), 6.59 (dd, *J* = 8.2, 2.0 Hz, 1H), 6.52 - 6.49 (m, 1H), 6.35 (dd, *J* = 8.0, 2.0 Hz, 1H), 3.89 - 3.80 (m, 2H), 3.73 (s, 3H), 3.30 - 3.16 (m, 1H), 2.88 - 2.79 (m, 2H), 2.55 - 2.35 (m, 3H), 2.02 - 1.95 (m, 2H), 1.95 - 1.88 (m, 2H), 0.87 - 0.84 (m, 1H), 0.45 - 0.41 (m, 1H), 0.29 - 0.21

(m, 2H), 0.10 - 0.07 (m, 1H).

**Example 358:**

Synthetic Route:

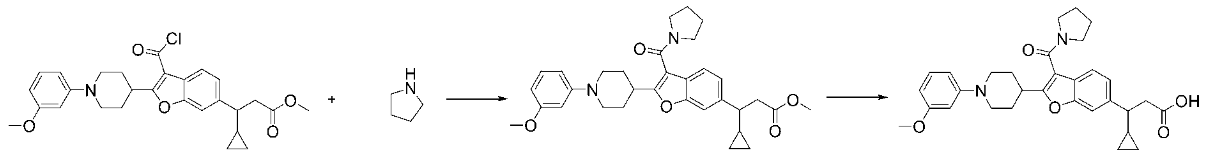

235-1 200-1 358-1 358

Referring to the synthetic route of compound **235,** compound **235-2** was replaced with compound **200-1** to carry out the synthesis. The resulting crude product was purified by reverse-phase column chromatography [acetonitrile/water (0.05% formic acid) = 0% to 100%] to obtain compound **358** (31 mg, yield: 41%) as a white solid. MS (ESI, m/z): 517.2 $[M+H]^+$.

$^1H$ NMR (400 MHz, $CDCl_3$) δ 7.37 - 7.32 (m, 2H), 7.24 - 7.17 (m, 1H), 7.16-7.12 (m, 1H), 6.62 (dd, *J* = 8.0, 2.4 Hz, 1H), 6.57 - 6.52 (m, 1H), 6.45 (dd, *J* = 8.0, 2.4 Hz, 1H), 3.83 - 3.78 (m, 5H), 3.77 - 3.67 (m, 2H), 3.45 - 3.35 (m, 2H), 3.19 - 3.11 (m, 1H), 2.94 - 2.74 (m, 4H), 2.55 - 2.45 (m, 1H), 2.22 - 1.84 (m, 8H), 1.13 - 1.04 (m, 1H), 0.66 - 0.59 (m, 1H), 0.48 - 0.42 (m, 1H), 0.37 - 0.31 (m, 1H), 0.22 - 0.16 (m, 1H).

**Example 359:**

Synthetic Route:

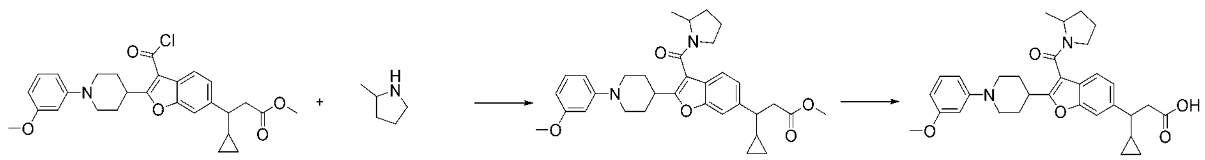

235-1 359-1 359-2 359

Referring to the synthetic route of compound **235,** compound **235-2** was replaced with compound **359-1** to carry out the synthesis. The resulting crude product was purified by reverse-phase column chromatography [acetonitrile/water (0.05% formic acid) = 0% to 100%] to obtain compound **359** (55 mg, yield: 73%) as a white solid. MS (ESI, m/z): 531.2 $[M+H]^+$.

$^1H$ NMR (400 MHz, $CDCl_3$) δ 7.38 - 7.31 (m, 2H), 7.25 - 7.18 (m, 1H), 7.15 (d, *J* = 8.0 Hz, 1H), 6.62 (dd, *J* = 8.0, 2.4 Hz, 1H), 6.58 - 6.53 (m, 1H), 6.45 (dd, *J* = 8.0, 2.4 Hz, 1H), 4.50 - 4.35 (m, 1H), 3.83 - 3.79 (m, 5H), 3.55 - 3.28 (m, 2H), 3.23 - 3.09 (m, 1H), 2.96 - 2.78 (m, 4H), 2.56 - 2.46 (m, 1H), 2.28 - 2.10 (m, 4H), 2.08 - 1.92 (m, 2H), 1.87 - 1.73 (m, 1H), 1.71 - 1.65 (m, 1H), 1.53 - 1.27 (m, 3H), 1.17 - 1.06 (m, 1H), 0.66 - 0.60 (m, 1H), 0.49 - 0.42 (m, 1H), 0.37 - 0.31 (m, 1H), 0.22 - 0.16 (m, 1H).

**Example 360:**

Synthetic Route:

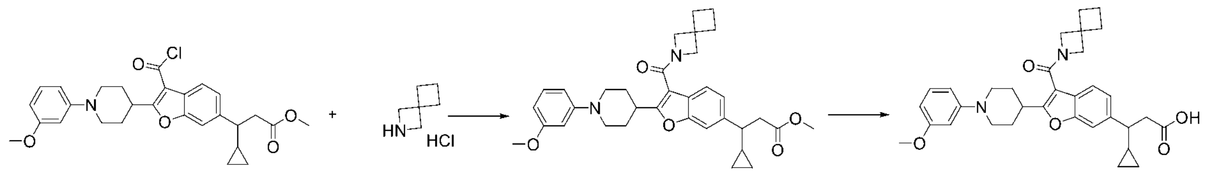

235-1 360-1 360-2 360

Referring to the synthetic route of compound **235,** compound **235-2** was replaced with compound **360-1** to carry out the synthesis. The resulting crude product was purified by reverse-phase column chromatography [acetonitrile/water (0.05% formic acid) = 0% to 100%] to obtain compound **360** (15 mg, yield: 36%) as a white solid. MS (ESI, m/z): 543.2 [M+H]⁺.

$^1$H NMR (400 MHz, $CDCl_3$) δ 7.44 (d, *J* = 8.0 Hz, 1H), 7.36 (s, 1H), 7.25 - 7.16 (m, 2H), 6.63 (dd, *J* = 8.0, 2.4 Hz, 1H), 6.58 - 6.54 (m, 1H), 6.46 (dd, *J* = 8.0, 2.4 Hz, 1H), 4.20 - 4.02 (m, 4H), 3.86 - 3.80 (m, 5H), 3.36 - 3.29 (m, 1H), 2.97 - 2.81 (m, 4H), 2.56 - 2.46 (m, 1H), 2.31 - 2.13 (m, 6H), 2.07 - 1.96 (m, 2H), 1.93 - 1.80 (m, 2H), 1.16 - 1.03 (m, 1H), 0.70 - 0.59 (m, 1H), 0.52 - 0.42 (m, 1H), 0.39 - 0.35 (m, 1H), 0.24 - 0.19 (m, 1H).

**Example 361:**

Synthetic Route:

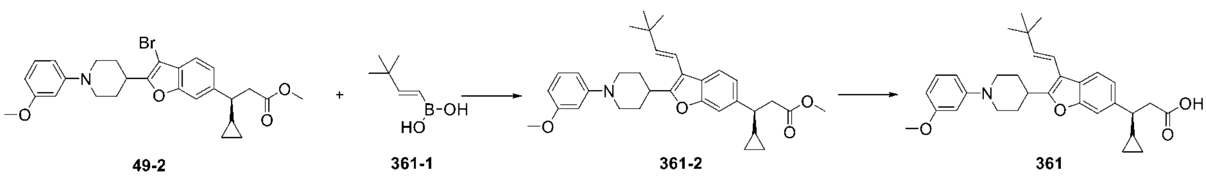

49-2 361-1 361-2 361

Referring to the synthetic route of compound **49,** compound **49-3** was replaced with compound **361-1,** and compound **49-10** was replaced with compound **361-2.** The synthesis was carried out, and the resulting crude product was purified by reverse-phase column chromatography [acetonitrile/water (0.05% formic acid) = 0% to 100%] to obtain compound **361** (48 mg, yield: 56%) as a white solid. MS (ESI, m/z): 502.2 [M+H]⁺.

$^1$H NMR (400 MHz, $CDCl_3$) δ 7.70 (d, *J* = 8.0 Hz, 1H), 7.31 (s, 1H), 7.24 - 7.18 (m, 1H), 7.14 (d, *J* = 8.0 Hz, 1H), 6.63 (d, *J* = 7.6 Hz, 1H), 6.56 (s, 1H), 6.45 (d, *J* = 7.6 Hz, 1H), 6.33 - 6.31 (m, 2H), 3.86 - 3.82 (m, 5H), 3.10 - 3.02 (m, 1H), 2.93 - 2.83 (m, 4H), 2.54 - 2.45 (m, 1H), 2.26 - 2.12 (m, 2H), 1.96 - 1.90 (m, 2H), 1.18 (s, 9H), 1.12 - 1.06 (m, 1H), 0.66 - 0.56 (m, 1H), 0.46 - 0.40 (m, 1H), 0.35 - 0.30 (m, 1H), 0.20 - 0.16 (m, 1H).

**Example 362:**

Synthetic Route:

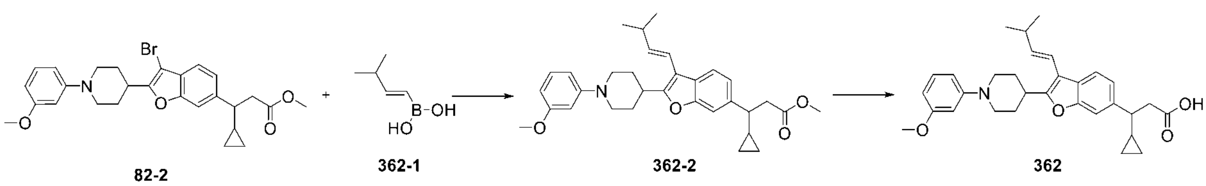

82-2 362-1 362-2 362

Referring to the synthetic route of compound **361,** compound **49-2** was replaced with compound **82-2,** and compound **361-1** was replaced with compound **362-1.** The synthesis was carried out, and the resulting crude product was purified by reverse-phase column chromatography [acetonitrile/water (0.05% formic acid) = 0% to 100%] to obtain compound **362** (45 mg, yield: 45%) as a white solid. MS (ESI, m/z): 588.2 [M+H]⁺.

$^1$H NMR (400 MHz, $CDCl_3$) δ 7.73 - 7.30 (m, 2H), 7.24 - 7.17 (m, 1H), 7.16 - 7.06 (m, 1H), 6.73 - 6.39 (m, 3H), 6.39 - 5.66 (m, 2H), 3.93 - 3.74 (m, 6H), 3.01 - 2.79 (m, 4H), 2.59 - 2.43 (m, 2H), 2.30 - 2.08 (m, 2H), 1.99 - 1.84 (m, 2H), 1.17 - 1.11 (m, 3H), 1.12 - 1.06 (m, 1H), 1.02 - 0.97 (m, 3H), 0.67 - 0.58 (m, 1H), 0.49 - 0.42 (m, 1H), 0.38 - 0.30 (m, 1H), 0.24 - 0.18 (m,

1H).

**Example 363:**

Synthetic Route:

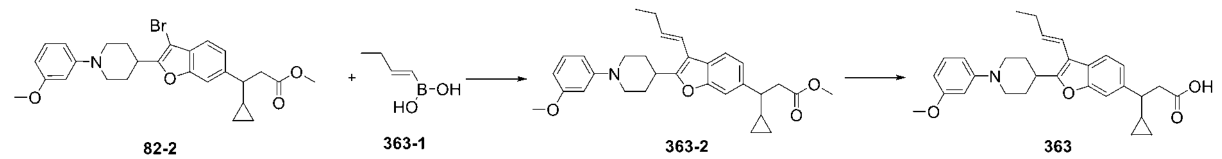

82-2 363-1 363-2 363

Referring to the synthetic route of compound **362,** compound **362-1** was replaced with compound **363-1** to carry out the synthesis. The resulting crude product was purified by reverse-phase column chromatography [acetonitrile/water (0.05% formic acid) = 0% to 100%] to obtain compound **363** (8 mg, yield: 39%) as a white solid. MS (ESI, m/z): 574.2 $[M+H]^+$.

$^1$H NMR (400 MHz, $CDCl_3$) δ 7.71 - 7.30 (m, 2H), 7.24 - 7.17 (m, 1H), 7.15 - 7.08 (m, 1H), 6.65 - 6.58 (m, 1H), 6.58 - 6.52 (m, 1H), 6.47 - 5.79 (m, 3H), 3.86 - 3.78 (m, 5H), 3.09 - 2.81 (m, 5H), 2.57 - 2.44 (m, 1H), 2.35 - 2.26 (m, 1H), 2.21 - 2.10 (m, 3H), 1.95 - 1.88 (m, 2H), 1.19 - 0.98 (m, 4H), 0.66 - 0.57 (m, 1H), 0.49 - 0.39 (m, 1H), 0.38 - 0.29 (m, 1H), 0.24 - 0.14 (m, 1H).

**Example 364:**

Synthetic Route:

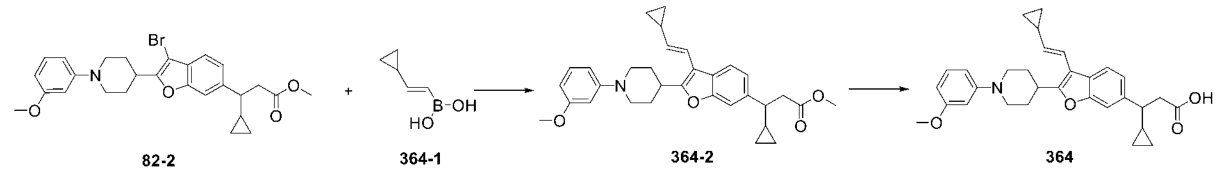

82-2 364-1 364-2 364

Referring to the synthetic route of compound **362,** compound **362-1** was replaced with compound **364-1** to carry out the synthesis. The resulting crude product was purified by reverse-phase column chromatography [acetonitrile/water (0.05% formic acid) = 0% to 100%] to obtain compound **364** (40 mg, yield: 80%) as a white solid. MS (ESI, m/z): 486.2 $[M+H]^+$.

$^1$H NMR (400 MHz, $CDCl_3$) δ 7.61 (d, *J* = 8.0 Hz, 1H), 7.31 (s, 1H), 7.25 - 7.18 (m, 1H), 7.11 (d, *J* = 7.6 Hz, 1H), 6.68 - 6.59 (m, 1H), 6.58 - 6.50 (m, 2H), 6.48 - 6.40 (m, 1H), 5.82 - 5.73 (m, 1H), 3.87 - 3.80 (m, 5H), 3.15 - 2.73 (m, 5H), 2.54 - 2.43 (m, 1H), 2.26 - 2.06 (m, 2H), 1.96 - 1.87 (m, 2H), 1.64 - 1.59 (m, 1H), 1.11 - 1.04 (m, 1H), 0.91 - 0.84 (m, 2H), 0.65 - 0.59 (m, 1H), 0.57 - 0.52 (m, 2H), 0.47 - 0.40 (m, 1H), 0.37 - 0.31 (m, 1H), 0.23 - 0.16 (m, 1H).

**Example 365:**

Synthetic Route:

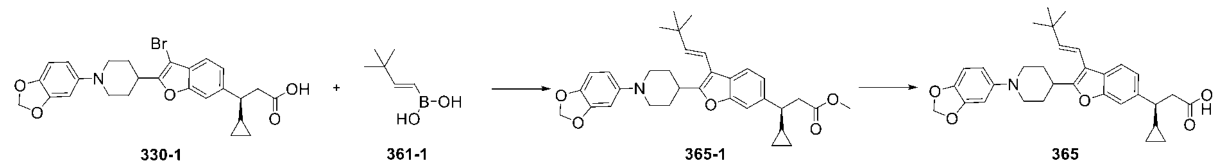

330-1 361-1 365-1 365

Referring to the synthetic route of compound **361,** compound **49-2** was replaced with compound **330-1** to carry out the synthesis. The resulting crude product was purified by reverse-phase column chromatography [acetonitrile/water

(0.05% formic acid) = 0% to 100%] to obtain compound **365** (90 mg, yield: 39%) as a white solid. MS (ESI, m/z): 530.2 [M+H]+.

$^1$H NMR (400 MHz, $CDCl_3$) δ 7.70 (d, *J* = 8.4 Hz, 1H), 7.32 (s, 1H), 7.14 (d, *J* = 7.6 Hz, 1H), 6.76 (d, *J* = 8.8 Hz, 1H), 6.65 (s, 1H), 6.51 - 6.42 (m, 1H), 6.34 - 6.31 (m, 2H), 5.93 (s, 2H), 3.68 - 3.57 (m, 2H), 3.05 - 2.95 (m, 1H), 2.92 - 2.77 (m, 4H), 2.57 - 2.44 (m, 1H), 2.29 - 2.14 (m, 2H), 2.01 - 1.88 (m, 2H), 1.18 (s, 9H), 1.12 - 1.06 (m, 1H), 0.66 - 0.57 (m, 1H), 0.48 - 0.39 (m, 1H), 0.35 - 0.30 (m, 1H), 0.24 - 0.16 (m, 1H).

**Example 366:**

Synthetic Route:

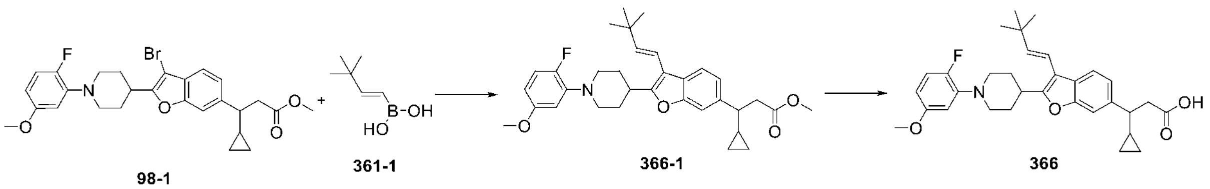

98-1 361-1 366-1 366

Referring to the synthetic route of compound **361,** compound **49-2** was replaced with compound **98-1** to carry out the synthesis. The resulting crude product was purified by reverse-phase column chromatography [acetonitrile/water (0.05% formic acid) = 0% to 100%] to obtain compound **366** (2 mg, yield: 8%) as a white solid. MS (ESI, m/z): 520.2 [M+H]+.

$^1$H NMR (400 MHz, DMSO-$d_6$) δ 7.72 (d, *J* = 8.0 Hz, 1H), 7.44 (s, 1H), 7.17 (d, *J* = 8.0 Hz, 1H), 7.05 (dd, *J* = 12.4, 8.8 Hz, 1H), 6.61 - 6.57 (m, 1H), 6.51- 6.49 (m, 1H), 6.47 - 6.34 (m, 2H), 3.73 (s, 3H), 3.50 - 3.47 (m, 2H), 3.25 - 3.14 (m, 1H), 2.92 - 2.80 (m, 2H), 2.79 - 2.59 (m, 2H), 2.43 - 2.33 (m, 1H), 2.12 - 1.87 (m, 2H), 1.87 - 1.85 (m, 2H), 1.16 (s, 9H), 1.12 - 1.04 (m, 1H), 0.55-0.48 (m, 1H), 0.35 - 0.24 (m, 2H), 0.19 - 0.10 (m, 1H).

**Example 367:**

Synthetic Route:

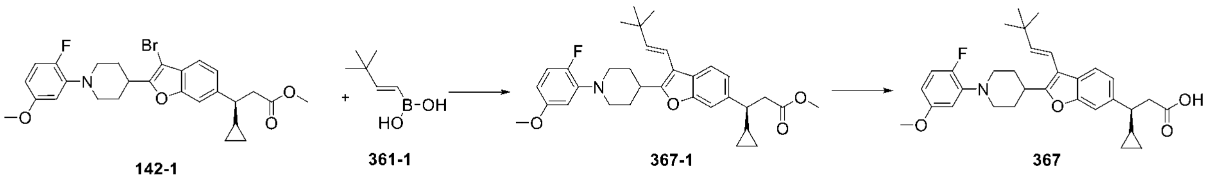

142-1 361-1 367-1 367

Referring to the synthetic route of compound **361,** compound **49-2** was replaced with compound **142-1** to carry out the synthesis. The resulting crude product was purified by reverse-phase column chromatography [acetonitrile/water (0.05% formic acid) = 0% to 100%] to obtain compound **367** (3 mg, yield: 21%) as a white solid. MS (ESI, m/z): 520.2 [M+H]+.

$^1$H NMR (400 MHz, DMSO-$d_6$) δ 7.72 (d, *J* = 8.4 Hz, 1H), 7.43 (s, 1H), 7.16 (d, *J* = 8.4 Hz, 1H), 7.05 (dd, *J* = 12.4, 8.8 Hz, 1H), 6.59 - 6.57 (m, 1H), 6.52 - 6.47 (m, 1H), 6.47 - 6.32 (m, 2H), 3.73 (s, 3H), 3.52 - 3.48 (m, 2H), 3.19 - 3.15 (m, 1H), 2.88 - 2.82 (m, 2H), 2.71 - 2.62 (m, 2H), 2.41 - 2.33 (m, 1H), 2.06 - 1.96 (m, 2H), 1.87 - 1.84 (m, 2H), 1.15 (s, 9H), 1.09 - 1.01 (m, 1H), 0.53 - 0.43 (m, 1H), 0.31 - 0.25 (m, 2H), 0.16 - 0.09 (m, 1H).

**Example 368:**

Synthetic Route:

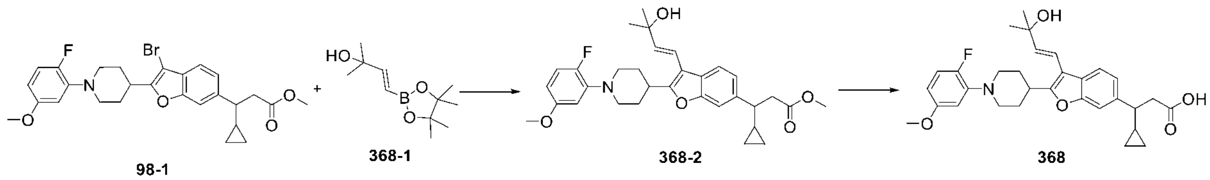

98-1 368-1 368-2 368

Referring to the synthetic route of compound **366,** compound **361-1** was replaced with compound **368-1** to carry out the synthesis. The resulting crude product was purified by reverse-phase column chromatography [acetonitrile/water (0.05% formic acid) = 0% to 100%] to obtain compound **368** (8 mg, yield: 13%) as a white solid. MS (ESI, m/z): 522.2 $[M+H]^+$.

$^1H$ NMR (400 MHz, DMSO-$d_6$) δ 7.76-7.69 (m, 1H), 7.47-7.43 (m, 1H), 7.19 - 7.16 (m, 1H), 7.08 - 7.03 (m, 1H), 6.67 - 6.24 (m, 4H), 3.73 (s, 3H), 3.50 - 3.47 (m,2H), 3.19 - 3.17 (m, 1H), 2.88 - 2.82 (m, 2H), 2.70 - 2.63 (m, 2H), 2.44 - 2.35 (m, 1H), 2.08 - 1.98 (m, 2H), 1.88 - 1.82 (m, 2H), 1.32 (s, 6H), 1.07 - 1.02 (m, 1H), 0.55 - 0.48 (m, 1H), 0.33 - 0.25 (m, 2H), 0.17 - 0.11 (m, 1H).

**Example 369:**

Synthetic Route:

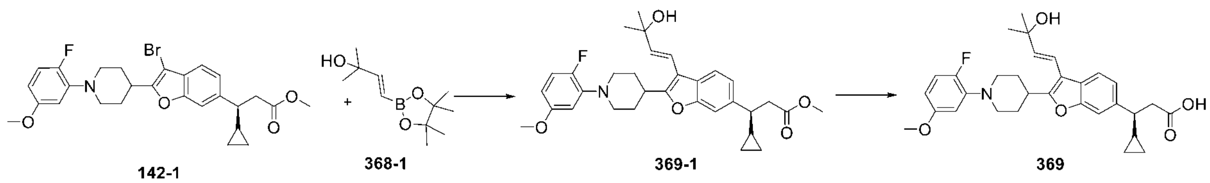

142-1 368-1 369-1 369

Referring to the synthetic route of compound **368,** compound **98-1** was replaced with compound **142-1** to carry out the synthesis. The resulting crude product was purified by reverse-phase column chromatography [acetonitrile/water (0.05% formic acid) = 0% to 100%] to obtain compound **369** (6 mg, yield: 11%) as a white solid. MS (ESI, m/z): 522.2 $[M+H]^+$.

$^1H$ NMR (400 MHz, DMSO-$d_6$) δ 12.8-11.76 (m, 1H), 7.80 - 7.70 (m, 1H), 7.48 - 7.40 (m, 1H), 7.21 - 7.13 (m, 1H), 7.10 - 7.01 (m, 1H), 6.66 - 6.55 (m, 2H), 6.54 - 6.44 (m, 2H), 4.73 (s, 1H), 3.73 (s, 3H), 3.55 - 3.44 (m, 2H), 3.21 - 3.13 (m, 1H), 2.86 - 2.80 (m, 2H), 2.77 - 2.66 (m, 2H), 2.41 - 2.36 (m, 1H), 2.06 - 1.94 (m, 2H), 1.90 - 1.81 (m, 2H), 1.32 (s, 6H), 1.12 - 1.02 (m, 1H), 0.55 - 0.43 (m, 1H), 0.34 - 0.23 (m, 2H), 0.18 - 0.09 (m, 1H).

**Example 370:**

Synthetic Route:

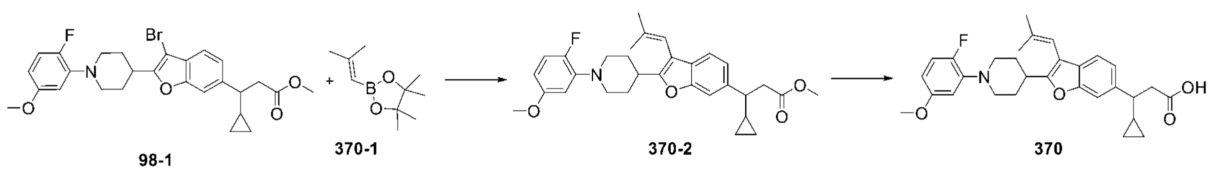

98-1 370-1 370-2 370

Referring to the synthetic route of compound **366,** compound **361-1** was replaced with compound **370-1** to carry out the synthesis. The resulting crude product was purified by reverse-phase column chromatography [acetonitrile/water (0.05% formic acid) = 0% to 100%] to obtain compound **370** (1 mg, yield: 3%) as a white solid. MS (ESI, m/z): 492.2 $[M+H]^+$.

$^1H$ NMR (400 MHz, MeOD) δ 7.31 (s, 1H), 7.23 (d, *J* = 8.0 Hz, 1H), 7.10 (d, *J* = 8.0 Hz, 1H), 6.95 (dd, *J*= 12.2, 8.8 Hz, 1H), 6.60 (dd, *J* = 7.2, 2.8 Hz, 1H), 6.52 - 6.46 (m, 1H), 6.11 - 6.08 (m, 1H), 3.76 (s, 3H), 3.55 - 3.48 (m, 2H), 2.93 - 2.70 (m, 5H), 2.50 - 2.40 (m, 1H), 2.21 - 2.10 (m, 2H), 1.97 (s, 3H), 1.95 - 1.85 (m, 2H), 1.68 (s, 3H), 1.14 - 1.07 (m, 1H), 0.64 - 0.55 (m, 1H), 0.43 - 0.29 (m, 2H), 0.19 - 0.11 (m, 1H).

**Example 371:**

Synthetic Route:

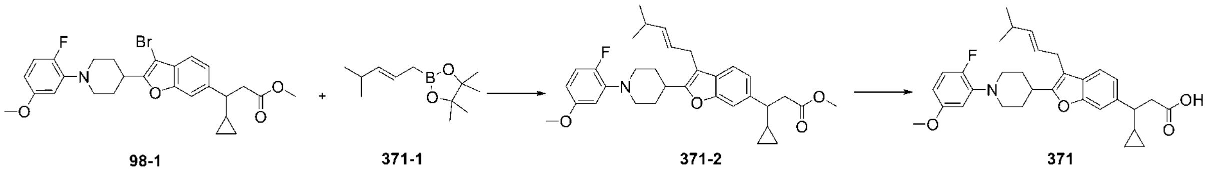

98-1 371-1 371-2 371

Referring to the synthetic route of compound **366,** compound **361-1** was replaced with compound **371-1** to carry out the synthesis. The resulting crude product was purified by reverse-phase column chromatography [acetonitrile/water (0.05% formic acid) = 0% to 100%] to obtain compound **371** (5 mg, yield: 45%) as a white solid. MS (ESI, m/z): 520.2 $[M+H]^+$.

$^1$H NMR (400 MHz, $CDCl_3$) δ 7.40 (d, *J* = 8.0 Hz, 1H), 7.31 (s, 1H), 7.10 (d, *J*= 7.6 Hz, 1H), 6.96 (dd, *J* = 12.0, 8.4 Hz, 1H), 6.59 - 6.54 (m, 1H), 6.48 - 6.40 (m, 1H), 5.40 - 5.27 (m, 2H), 3.80 (s, 3H), 3.63 - 3.57 (m, 2H), 3.42 (d, *J* = 5.6 Hz, 2H), 3.02 - 2.77 (m, 6H), 2.51 - 2.45 (m, 1H), 2.27 - 2.21 (m, 2H), 1.90 - 1.86 (m, 2H), 1.11 - 1.04 (m, 7H), 0.63 - 0.59 (m, 1H), 0.46 - 0.41 (m, 1H), 0.38 - 0.31 (m, 1H), 0.22 - 0.17 (m, 1H).

**Example 372:**

Synthetic Route:

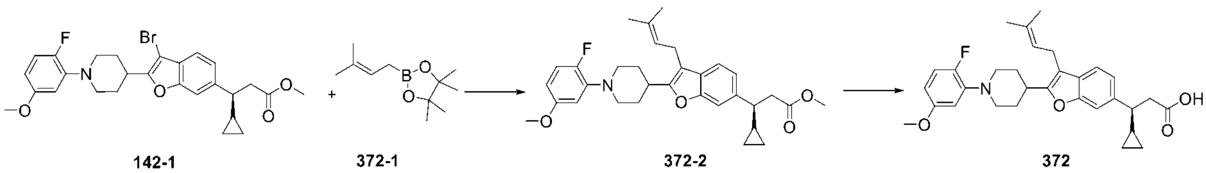

142-1 372-1 372-2 372

Referring to the synthetic route of compound **367,** compound **361-1** was replaced with compound **372-1** to carry out the synthesis. The resulting crude product was purified by reverse-phase column chromatography [acetonitrile/water (0.05% formic acid) = 0% to 100%] to obtain compound **372** (40 mg, yield: 57%) as a white solid. MS (ESI, m/z): 506.3 $[M+H]^+$.

$^1$H NMR (400 MHz, $CDCl_3$) δ 7.36 (d, *J* = 8.4 Hz, 1H), 7.29 (s, 1H), 7.07 (d, *J* = 8.4 Hz, 1H), 6.99 - 6.91 (m, 1H), 6.58 - 6.53 (m, 1H), 6.46 - 6.39 (m, 1H), 5.29 - 5.20 (m, 1H), 3.79 (s, 3H), 3.64 - 3.54 (m, 2H), 3.33 (d, *J* = 6.0 Hz, 2H), 2.98 - 2.89 (m, 1H), 2.86 - 2.76 (m, 4H), 2.51 - 2.42 (m, 1H), 2.33 - 2.19 (m, 2H), 1.90 - 1.84 (m, 2H), 1.81 (s, 3H), 1.72 (s, 3H), 1.13 - 1.02 (m, 1H), 0.64 - 0.57 (m, 1H), 0.47 - 0.39 (m, 1H), 0.35 - 0.28 (m, 1H), 0.23 - 0.14 (m, 1H).

**Example 373:**

Synthetic Route:

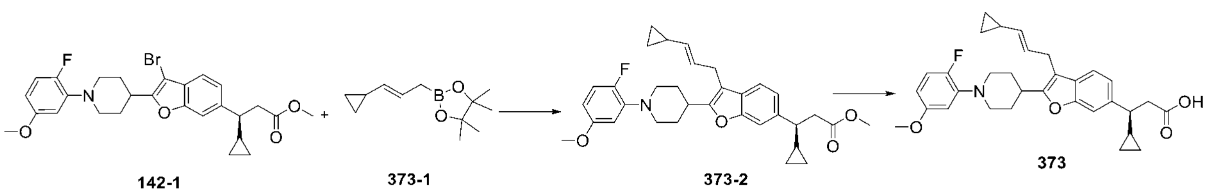

142-1 373-1 373-2 373

Referring to the synthetic route of compound **367,** compound **361-1** was replaced with compound **373-1** to carry out the synthesis. The resulting crude product was purified by reverse-phase column chromatography [acetonitrile/water

(0.05% formic acid) = 0% to 100%] to obtain compound **373** (15 mg, yield: 76%) as a white solid. MS (ESI, m/z): 518.2 [M+H]+.

$^1$H NMR (400 MHz, $CDCl_3$) δ 7.50 - 7.37 (m, 1H), 7.33 - 7.29 (m, 1H), 7.14 - 7.06 (m, 1H), 7.01 - 6.92 (m, 1H), 6.61 - 6.54 (m, 1H), 6.49 - 6.41 (m, 1H), 5.69 - 5.36 (m, 1H), 5.17 - 4.80 (m, 1H), 3.80 (s, 3H), 3.64 - 3.57 (m, 2H), 3.55 - 3.32 (m, 2H), 3.07 - 2.92 (m, 1H), 2.89 - 2.74 (m, 4H), 2.53 - 2.42 (m, 1H), 2.35 - 2.19 (m, 2H), 1.96 - 1.84 (m, 2H), 1.85 - 1.77 (m, 1H), 1.16 - 1.03 (m, 1H), 0.94 - 0.66 (m, 2H), 0.63 - 0.57 (m, 1H), 0.49 - 0.26 (m, 4H), 0.24 - 0.15 (m, 1H).

**Example 374:**

Synthetic Route:

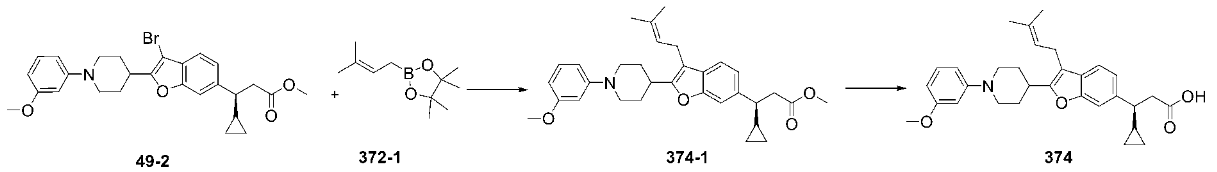

49-2 372-1 374-1 374

Referring to the synthetic route of compound **372,** compound **142-1** was replaced with compound **49-2** to carry out the synthesis. The resulting crude product was purified by reverse-phase column chromatography [acetonitrile/water (0.05% formic acid) = 0% to 100%] to obtain compound **374** (12 mg, yield: 57%) as a white solid. MS (ESI, m/z): 488.3 [M+H]+.

$^1$H NMR (400 MHz, $CDCl_3$) δ 7.39 - 7.34 (m, 1H), 7.29 - 7.27 (m, 1H), 7.22 - 7.17 (m, 1H), 7.07 (d, *J* = 7.6 Hz, 1H), 6.64 - 6.58 (m, 1H), 6.55 - 6.51 (m, 1H), 6.48 - 6.39 (m, 1H), 5.29 - 5.19 (m, 1H), 3.85 - 3.79 (m, 5H), 3.36 - 3.30 (m, 2H), 3.01 - 2.90 (m, 1H), 2.88 - 2.80 (m, 4H), 2.51 - 2.39 (m, 1H), 2.24 - 2.10 (m, 2H), 1.91 - 1.84 (m, 2H), 1.81 (s, 3H), 1.72 (s, 3H), 1.12 - 1.03 (m, 1H), 0.66 - 0.53 (m, 1H), 0.46 - 0.37 (m, 1H), 0.35 - 0.28 (m, 1H), 0.22 - 0.15 (m, 1H).

**Example 375:**

Synthetic Route:

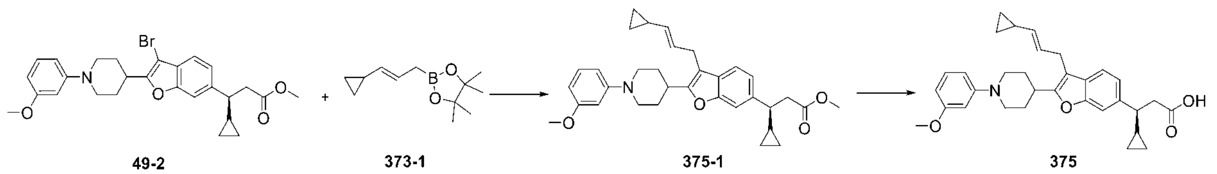

49-2 373-1 375-1 375

Referring to the synthetic route of compound **373,** compound **142-1** was replaced with compound **49-2** to carry out the synthesis. The resulting crude product was purified by reverse-phase column chromatography [acetonitrile/water (0.05% formic acid) = 0% to 100%] to obtain compound **375** (4 mg, yield: 18%) as a white solid. MS (ESI, m/z): 500.2 [M+H]+.

$^1$H NMR (400 MHz, $CDCl_3$) δ 7.47 (d, *J* = 7.4 Hz, 1H), 7.31 (s, 1H), 7.23 - 7.18 (m, 1H), 7.10 (d, *J* = 7.4 Hz, 1H), 6.66 - 6.59 (m, 1H), 6.57 - 6.51 (m, 1H), 6.49 - 6.39 (m, 1H), 5.68 - 5.34 (m, 1H), 5.18 - 4.80 (m, 1H), 3.86 - 3.81 (m, 5H), 3.53 (d, *J* = 7.0 Hz, 2H), 3.06 - 2.98 (m, 1H), 2.88 - 2.81 (m, 4H), 2.50 - 2.45 (m, 1H), 2.22 - 2.14 (m, 2H), 1.93 - 1.88 (m, 2H), 1.83 - 1.78 (m, 1H), 1.12 - 1.06 (m, 1H), 0.89 - 0.82 (m, 2H), 0.65 - 0.59 (m, 1H), 0.47 - 0.40 (m, 3H), 0.35 - 0.30 (m, 1H), 0.22 - 0.17 (m, 1H).

**Example 376:**

Synthetic Route:

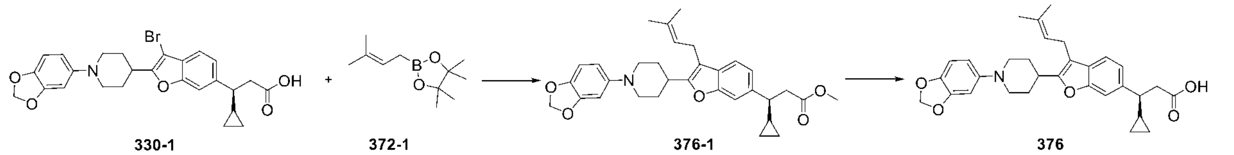

330-1 372-1 376-1 376

Referring to the synthetic route of compound **330,** compound **330-2** was replaced with compound **372-1** to carry out the synthesis. The resulting crude product was purified by reverse-phase column chromatography [acetonitrile/water (0.05% formic acid) = 0% to 100%] to obtain compound **376** (3 mg, yield: 20%) as a white solid. MS (ESI, m/z): 502.3 $[M+H]^+$.

$^1H$ NMR (400 MHz, $CDCl_3$) δ 7.39 - 7.33 (m, 1H), 7.32 - 7.28 (m, 1H), 7.10 - 7.06 (m, 1H), 6.77 - 6.70 (m, 1H), 6.68 - 6.60 (m, 1H), 6.48 - 6.38 (m, 1H), 5.92 (s, 2H), 5.28 - 5.20 (m, 1H), 3.69 - 3.52 (m, 2H), 3.34 - 3.30 (m, 2H), 2.90 - 2.72 (m, 5H), 2.50 - 2.43 (m, 1H), 2.31 - 2.04 (m, 2H), 1.90 - 1.82 (m, 2H), 1.81 (s, 3H), 1.72 (s, 3H), 1.11 - 1.04 (m, 1H), 0.64 - 0.59 (m, 1H), 0.45 - 0.39 (m, 1H), 0.35 - 0.30 (m, 1H), 0.21 - 0.16 (m, 1H).

**Example 377:**

Synthetic Route:

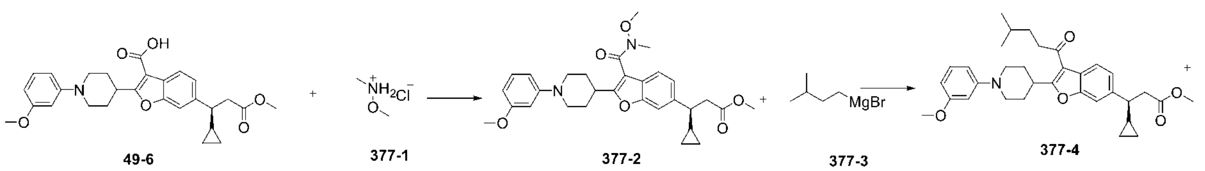

49-6 377-1 377-2 377-3 377-4

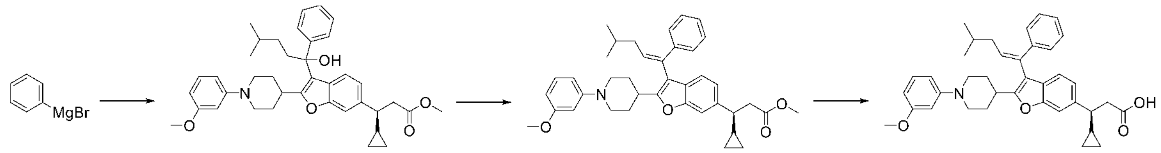

377-5 377-6 377-7 377

Compound **49-6** (1100 mg, 2.31 mmol) and compound **377-1** (339 mg, 3.46 mmol) were dissolved in dichloromethane (20 mL). 2-(7-Azabenzotriazol-1-yl)-*N,N,N',N'*-tetramethyluronium hexafluorophosphate (1100 mg, 3.0 mmol) and triethylamine (698 mg, 6.92 mmol) were added, and the reaction was carried out at room temperature overnight. After concentration, the resulting crude product was purified by normal-phase column chromatography (ethyl acetate: petroleum ether = 30%) to obtain compound **377-2** (1250 mg, yield: 99%) as a white solid. MS (ESI, m/z): 521.3 $[M+H]^+$.

Compound **377-2** (500 mg, 0.96 mmol) was dissolved in tetrahydrofuran (5 mL), and compound **377-3** (1.0 M, 1.5 mL) was added at 0°C. The reaction was carried out at 0°C for 1 hour, then quenched with saturated ammonium chloride solution (1 mL). After concentration, the resulting crude product was purified by normal-phase column chromatography (ethyl acetate: petroleum ether = 10%) to obtain compound **377-4** (350 mg, yield: 68%) as a white solid. MS (ESI, m/z): 532.3 $[M+H]^+$.

Compound **377-4** (130 mg, 0.244 mmol) was dissolved in tetrahydrofuran (5 mL). Phenyl Grignard reagent (1.0 M, 0.32 mL) was added at 0°C, and the reaction was carried out at 0°C for 1 hour. The reaction mixture was quenched with saturated ammonium chloride solution (1 mL), concentrated, and purified by normal-phase column chromatography (ethyl acetate: petroleum ether = 10%) to obtain compound **377-6** (70 mg, yield: 47%) as a white solid. MS (ESI, m/z): 610.3 $[M+H]^+$.

Compound **377-6** (70 mg, 0.114 mmol) was dissolved in dichloromethane (3 mL), and trifluoroacetic acid (3 mL) was added. The reaction was carried out at room temperature for 2 hours, and after concentration, the resulting crude product was directly used in the next step. MS (ESI, m/z): 592.3 $[M+H]^+$.

To a reaction tube, compound **377-7** (35 mg, 0.0589 mmol), lithium hydroxide (8.2 mg, 0.341 mmol), tetrahydrofuran (3 mL), methanol (3 mL), and water (3 mL) were added. The reaction was carried out at 50°C for 2 hours. After concentration, the resulting crude product was purified by reverse-phase column chromatography [acetonitrile/water (0.05% formic acid) = 50% to 70%] to obtain compound 377 (18 mg, yield: 51%) as a white solid. MS (ESI, m/z): 578.4 $[M+H]^+$.

$^1H$ NMR (400 MHz, $CDCl_3$) δ 7.35 - 7.26 (m, 4H), 7.25 - 7.12 (m, 4H), 7.08 - 6.97 (m, 1H), 6.60 - 6.54 (m, 1H), 6.51 - 6.45 (m, 1H), 6.44 - 6.39 (m, 1H), 5.95 (t, *J* = 7.6 Hz, 1H), 3.81- 3.68 (m, 5H), 2.83 - 2.55 (m, 5H), 2.49 - 2.39 (m, 1H), 2.27 -

2.19 (m, 2H), 2.17 - 2.06 (m, 2H), 1.80 - 1.68 (m, 3H), 1.12 - 1.02 (m, 1H), 0.96 - 0.88 (m, 6H), 0.67 - 0.52 (m, 1H), 0.52 - 0.37 (m, 1H), 0.35 - 0.27 (m, 1H), 0.24 - 0.12 (m, 1H).

**Example 378:**

Synthetic Route:

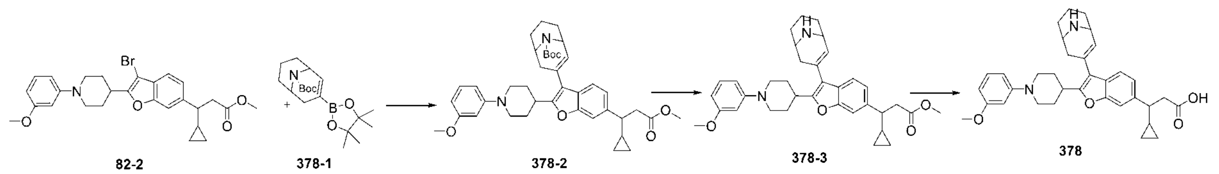

82-2 378-1 378-2 378-3 378

Referring to the synthetic route of compound **362,** compound **362-1** was replaced with compound **378-1** to obtain synthesize compound **378-2** (100 mg, 0.188 mmol), and compound **378-2** (100 mg, 0.188 mmol) was added to a mixture of dichloromethane (3 mL) and trifluoroacetic acid (1 mL). The reaction was carried out at room temperature for 2 hours, and after concentration, the resulting crude product was directly used in the next step. LCMS: $[M+H]^+$ = 555.0.
Then, referring to the synthetic route of compound **362,** compound **362-2** was replaced with compound **378-3** to carry out the synthesis. The resulting crude product was purified by reverse-phase column chromatography [acetonitrile/-water (0.05% formic acid) = 0% to 100%] to obtain compound **378** (60 mg, yield: 63%) as a white solid. MS (ESI, m/z): 541.2 $[M+H]^+$.
$^1$H NMR (400 MHz, DMSO-$d_6$) δ 7.38 - 7.32 (m, 2H), 7.15 - 7.08 (m, 2H), 6.61 - 6.54 (m, 1H), 6.52 - 6.48 (m, 1H), 6.40 - 6.33 (m, 1H), 5.90 - 5.84 (m, 1H), 3.86 - 3.81 (m, 2H), 3.74 (s, 3H), 3.57 - 3.46 (m, 1H), 3.31 - 3.24 (m, 1H), 3.14 - 3.03 (m, 1H), 2.84 - 2.68 (m, 4H), 2.48 - 2.42 (m, 1H), 2.40 - 2.24 (m, 2H), 2.09 - 1.95 (m, 4H), 1.83 - 1.72 (m, 4H), 1.67 - 1.46 (m, 2H), 1.02 - 0.90 (m, 1H), 0.47 - 0.38 (m, 1H), 0.30 - 0.20 (m, 2H), 0.10 - 0.01 (m, 1H).

**Example 379:**

Synthetic Route:

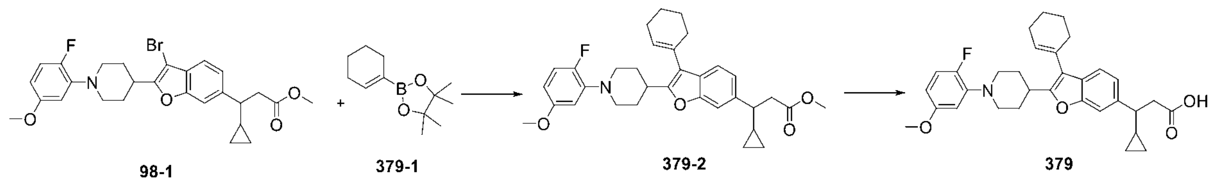

98-1 379-1 379-2 379

Referring to the synthetic route of compound **366,** compound **361-1** was replaced with compound **379-1** to carry out the synthesis. The resulting crude product was purified by reverse-phase column chromatography [acetonitrile/water (0.05% formic acid) = 0% to 100%] to obtain compound **379** (13 mg, yield: 44%) as a white solid. MS (ESI, m/z): 518.2 $[M+H]^+$.
$^1$H NMR (400 MHz, $CDCl_3$) δ 7.40 (d, *J* = 8.0 Hz, 1H), 7.29 (s, 1H), 7.07 (dd, *J* = 8.0, 1.2 Hz, 1H), 6.94 (dd, *J* = 12.0, 8.8 Hz, 1H), 6.57 - 6.52 (m, 1H), 6.45 - 6.38 (m, 1H), 5.80 - 5.75 (m, 1H), 3.78 (s, 3H), 3.61 - 3.53 (m, 2H), 3.03 - 2.92 (m, 1H), 2.91 - 2.73 (m, 4H), 2.51 - 2.42 (m, 1H), 2.36 - 2.19 (m, 6H), 1.91 - 1.83 (m, 2H), 1.83 - 1.69 (m, 4H), 1.13 - 1.03 (m, 1H), 0.67 - 0.55 (m, 1H), 0.47 - 0.38 (m, 1H), 0.37 - 0.28 (m, 1H), 0.22 - 0.14 (m, 1H).

**Example 380:**

Synthetic Route:

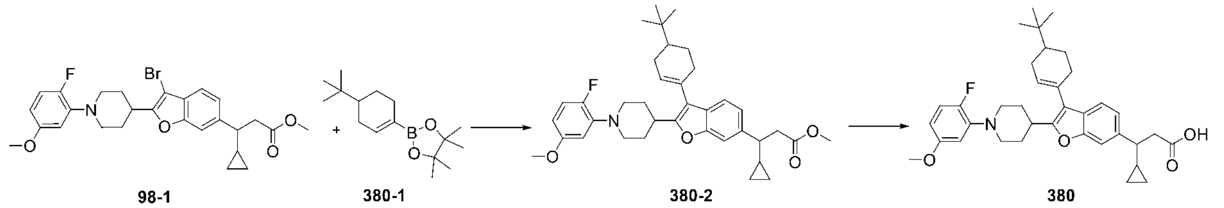

98-1 380-1 380-2 380

Referring to the synthetic route of compound **366,** compound **361-1** was replaced with compound **380-1** to carry out the synthesis. The resulting crude product was purified by reverse-phase column chromatography [acetonitrile/water (0.05% formic acid) = 0% to 100%] to obtain compound **380** (3 mg, yield: 18%) as a white solid. MS (ESI, m/z): 574.2 $[M+H]^+$.

$^1$H NMR (400 MHz, DMSO-$d_6$) δ 7.41 (d, $J$ = 8.0 Hz, 1H), 7.30 (s, 1H), 7.08 (d, $J$ = 8.4 Hz, 1H), 6.98 - 6.93 (m, 1H), 6.58 - 6.54 (m, 1H), 6.45 - 6.40 (m, 1H), 5.82 - 5.77 (m, 1H), 3.79 (s, 3H), 3.63 - 3.53 (m, 2H), 3.04 - 2.93 (m, 1H), 2.90 - 2.77 (m, 4H), 2.53 - 2.44 (m, 1H), 2.43 - 2.39 (m, 2H), 2.28 - 2.23 (m, 4H), 2.07 - 1.94 (m, 4H), 1.93 - 1.84 (m, 2H), 1.11 - 1.05 (m, 1H), 0.95 (s, 9H), 0.65 - 0.56 (m, 1H), 0.47 - 0.40 (m, 1H), 0.38 - 0.30 (m, 1H), 0.22 - 0.16 (m, 1H).

**Example 381:**

Synthetic Route:

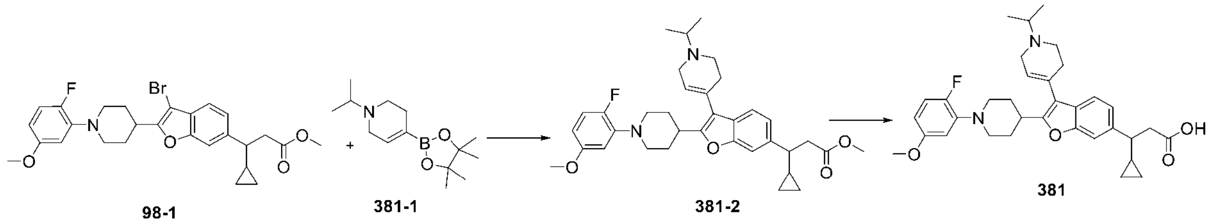

381

98-1 381-1 381-2

Referring to the synthetic route of compound **366,** compound **361-1** was replaced with compound **381-1** to carry out the synthesis. The resulting crude product was purified by reverse-phase column chromatography [acetonitrile/water (0.05% formic acid) = 0% to 100%] to obtain compound **381** (8 mg, yield: 13%) as a white solid. MS (ESI, m/z): 561.2 $[M+H]^+$.

$^1$H NMR (400 MHz, $CDCl_3$) δ 7.41 - 7.39 (m, 2H), 7.10 - 7.00 (m, 2H), 6.56 (dd, $J$ = 7.6 Hz, 3.2 Hz, 1H), 6.50 - 6.44 (m, 1H), 5.82 - 5.77 (m, 1H), 3.71 (s, 3H), 3.49 - 3.40 (m, 2H), 3.19 - 3.17 (m, 2H), 3.06 - 3.00 (m, 1H), 2.82 - 2.74 (m, 3H), 2.71 - 2.64 (m, 4H), 2.46 - 2.40 (m, 2H), 2.37 - 2.30 (m, 1H), 2.07 - 1.95 (m, 2H), 1.88 - 1.79 (m, 2H), 1.10 - 0.98 (m, 7H), 0.54 - 0.45 (m, 1H), 0.31 - 0.22 (m, 2H), 0.15 - 0.07 (m, 1H).

**Example 382:**

Synthetic Route:

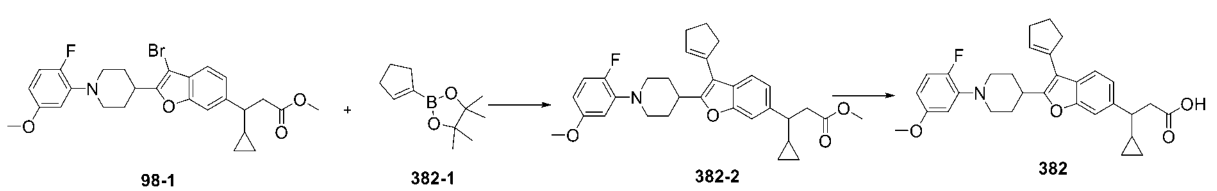

382

98-1 382-1 382-2

Referring to the synthetic route of compound **366,** compound **361-1** was replaced with compound **382-1** to carry out the synthesis. The resulting crude product was purified by reverse-phase column chromatography [acetonitrile/water (0.05% formic acid) = 0% to 100%] to obtain compound **382** (8 mg, yield: 15%) as a white solid. MS (ESI, m/z): 504.2 $[M+H]^+$.

$^1$HNMR (400 MHz, MeOD) δ 7.50 (d, $J$ = 8.0 Hz, 1H), 7.32 (s, 1H), 7.13 (dd, $J$ = 8.0, 1.2 Hz, 1H), 6.95 (dd, $J$ = 12.2,

8.8 Hz, 1H), 6.61 (dd, $J$ = 7.2, 2.8 Hz, 1H), 6.53 - 6.46 (m, 1H), 6.03 - 5.98 (m, 1H), 3.76 (s, 3H), 3.59 - 3.50 (m, 2H), 3.21 - 3.13 (m, 1H), 2.84 - 2.70 (m, 6H), 2.60 - 2.52 (m, 2H), 2.50 - 2.41 (m, 1H), 2.28 - 2.14 (m, 2H), 2.11 - 2.02 (m, 2H), 1.96 - 1.86 (m, 2H), 1.15 - 1.05 (m, 1H), 0.63 - 0.55 (m, 1H), 0.44 - 0.29 (m, 2H), 0.19 - 0.10 (m, 1H).

**Example 383:**

Synthetic Route:

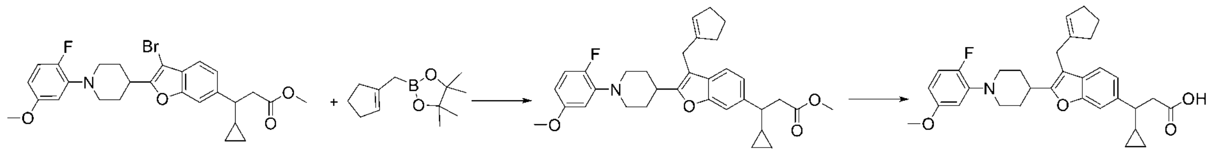

98-1 383-1 383-2 383

Referring to the synthetic route of compound **366,** compound **361-1** was replaced with compound **383-1** to carry out the synthesis. The resulting crude product was purified by reverse-phase column chromatography [acetonitrile/water (0.05% formic acid) = 0% to 100%] to obtain compound **383** (12 mg, yield: 45%) as a white solid. MS (ESI, m/z): 518.2 [M+H]$^+$.

$^1$H NMR (400 MHz, $CDCl_3$) δ 7.40 (d, $J$ = 7.6 Hz, 1H), 7.30 (s, 1H), 7.09 (d, $J$ = 7.6 Hz, 1H), 7.00 - 6.93 (m, 1H), 6.62 - 6.53 (m, 1H), 6.48 - 6.40 (m, 1H), 5.59 - 5.36 (m, 1H), 3.80 (s, 3H), 3.66 - 3.56 (m, 2H), 2.98 - 2.76 (m, 5H), 2.74 - 2.60 (m, 2H), 2.53 - 2.43 (m, 1H), 2.36 - 2.18 (m, 3H), 2.12 - 1.96 (m, 2H), 1.92 - 1.83 (m, 2H), 1.76 - 1.71 (m, 1H), 1.13 - 1.04 (m, 1H), 0.99 - 0.94 (m, 1H), 0.92 - 0.85 (m, 1H), 0.65 - 0.56 (m, 1H), 0.47 - 0.40 (m, 1H), 0.41 - 0.29 (m, 1H), 0.25 - 0.15 (m, 1H).

**Example 384:**

Synthetic Route:

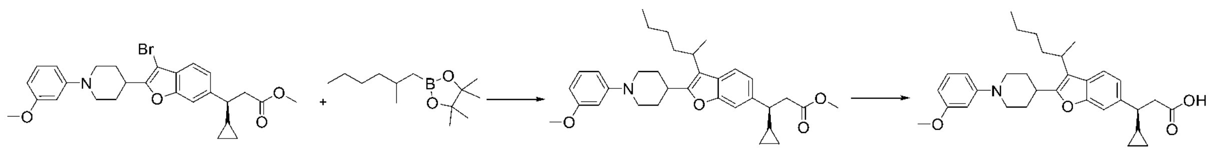

49-2 384-1 384-2 384

Referring to the synthetic route of compound **373,** compound **142-1** was replaced with compound **49-2** to carry out the synthesis. The resulting crude product was purified by reverse-phase column chromatography [acetonitrile/water (0.05% formic acid) = 0% to 100%] to obtain compound **375** (5 mg, yield: 26%) as a white solid. MS (ESI, m/z): 504.2 [M+H]$^+$.

$^1$H NMR (400 MHz, $CDCl_3$) δ 7.53 (d, $J$ = 8.0 Hz, 1H), 7.32 (s, 1H), 7.26 - 7.20 (m, 1H), 7.07 (d, $J$ = 7.6 Hz, 1H), 6.65 (d, $J$ = 6.8 Hz, 1H), 6.57 (s, 1H), 6.47 (d, $J$ = 8.0 Hz, 1H), 3.89 - 3.82 (m, 5H), 2.97 - 2.81 (m, 6H), 2.56 - 2.46 (m, 1H), 2.32 - 2.17 (m, 2H), 1.92 - 1.81 (m, 3H), 1.79 - 1.71 (m, 1H), 1.40 (d, $J$ = 7.0 Hz, 3H), 1.32 - 1.23 (m, 4H), 1.16 - 1.09 (m, 1H), 0.89 (t, $J$ = 7.2 Hz, 3H), 0.69 - 0.60 (m, 1H), 0.53 - 0.44 (m, 1H), 0.41 - 0.31 (m, 1H), 0.28 - 0.18 (m, 1H).

**Example 385:**

Synthetic Route:

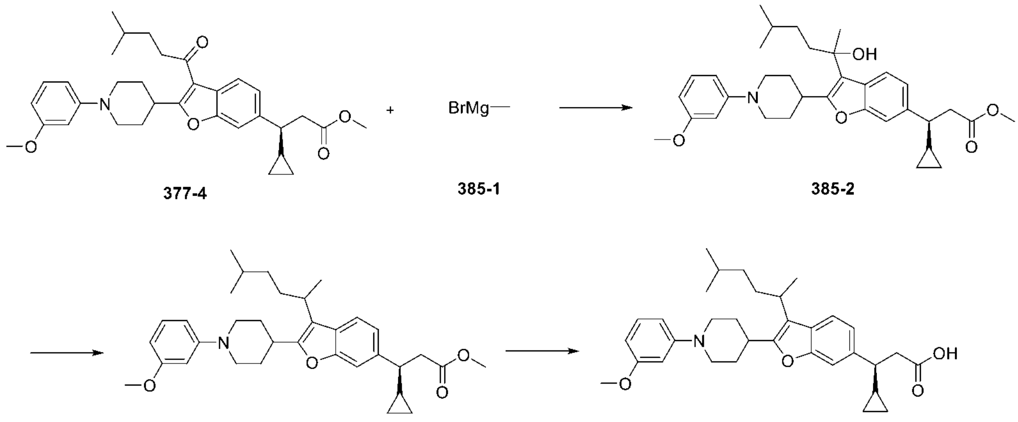

377-4 385-1 385-2

385-3 385

Compound **377-4** (130 mg, 0.244 mmol) was dissolved in tetrahydrofuran (5 mL). Methyl Grignard reagent **385-1** (1.0 M, 0.32 mL) was added at 0°C, and the reaction was carried out at 0°C for 1 hour. The reaction mixture was quenched with saturated ammonium chloride solution (1 mL), concentrated, and purified by normal -phase column chromatography (ethyl acetate: petroleum ether = 10%) to obtain compound **385-2** (20 mg, yield: 15%) as a white solid. LCMS: $[M+H]^+$ = 548.3.

Compound **385-2** (20 mg, 0.0376 mmol) was dissolved in methanol (3 mL), and one drop of concentrated hydrochloric acid was added as a catalyst along with palladium on carbon (10 mg). The reaction was carried out under a hydrogen atmosphere at 50°C overnight, followed by filtration and concentration. The resulting crude product was purified by normal-phase column chromatography (ethyl acetate: petroleum ether = 10%) to obtain compound **385-3** (10 mg, yield: 50%) as a white solid. LCMS: $[M+H]^+$ = 532.3.

To a reaction tube, compound **385-3** (10 mg, 0.0187 mmol), lithium hydroxide (8.2 mg, 0.341 mmol), methanol (1 mL), water (1 mL), and tetrahydrofuran (1 mL) were added. The reaction was carried out at 50°C for 2 hours. After the reaction mixture was rotary evaporated to remove the solvent, the resulting crude product was purified by reverse-phase column chromatography [acetonitrile/water (0.05% formic acid) = 50% to 70%] to obtain compound **385** (4.2 mg, yield: 42%) as a white solid. LCMS: $[M+H]^+$ = 518.3.

$^1$H NMR (400 MHz, $CDCl_3$) δ 7.52 (d, $J$ = 8.0 Hz, 1H), 7.32 (s, 1H), 7.26 - 7.19 (m, 1H), 7.07 (d, $J$ = 8.4 Hz, 1H), 6.65 (d, $J$ = 7.6 Hz, 1H), 6.57 (s, 1H), 6.47 (d, $J$ = 7.6 Hz, 1H), 3.89 - 3.83 (m, 5H), 2.98 - 2.84 (m, 6H), 2.54 - 2.45 (m, 1H), 2.28 - 2.16 (m, 2H), 1.91 - 1.83 (m, 2H), 1.82 - 1.71 (m, 2H), 1.60 - 1.52 (m, 1H), 1.40 (d, $J$ = 7.2 Hz, 3H), 1.23 - 1.07 (m, 3H), 0.92 - 0.85 (m, 6H), 0.70 - 0.60 (m, 1H), 0.52 - 0.43 (m, 1H), 0.40 - 0.31 (m, 1H), 0.27 - 0.19 (m, 1H).

**Example 386:**

Synthetic Route:

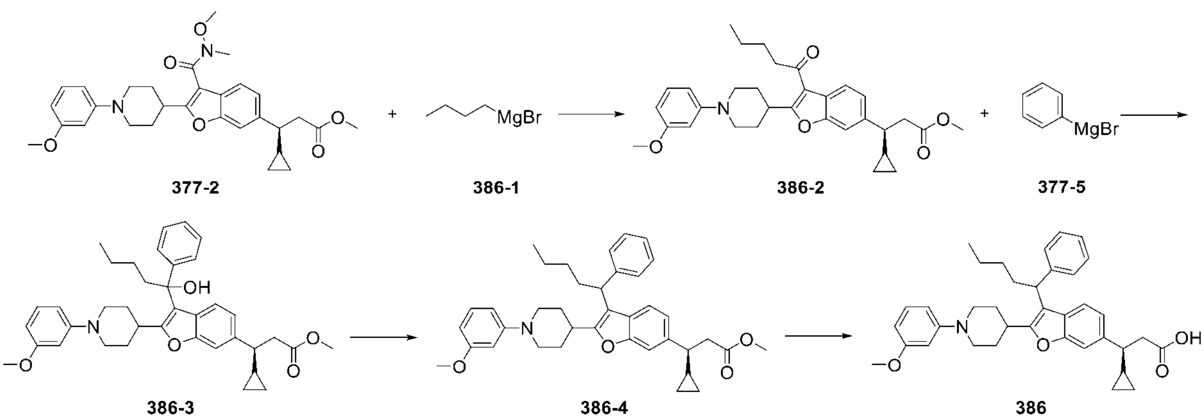

377-2 386-1 386-2 377-5

386-3 386-4 386

Referring to the synthetic route of compound **377,** compound **377-3** was replaced with compound **386-1** to synthesize compound **386-3.** Then, referring to the synthetic route of compound **385,** compound **385-2** was replaced with compound **386-3,** and the resulting crude product was purified by reverse-phase column chromatography [acetonitrile/-

water (0.05% formic acid) = 0% to 100%] to obtain compound **386** (12 mg, yield: 37%) as a white solid. MS (ESI, m/z): 566.2 $[M+H]^+$.

$^1$H NMR (400 MHz, $CDCl_3$) δ 7.40 - 7.30 (m, 3H), 7.28 - 7.26 (m, 2H), 7.21 - 7.13 (m, 3H), 6.99 (d, *J* = 8.0 Hz, 1H), 6.59 (dd, *J* = 8.0, 2.0 Hz, 1H), 6.54 - 6.49 (m, 1H), 6.42 (dd, *J* = 8.0, 2.0 Hz, 1H), 4.13 - 4.05 (m, 1H), 3.84 - 3.75 (m, 5H), 3.00 - 2.90 (m, 1H), 2.87 - 2.76 (m, 4H), 2.49 - 2.41 (m, 1H), 2.26 - 2.11 (m, 4H), 1.82 - 1.73 (m, 2H), 1.34 - 1.26 (m, 4H), 1.10 - 1.01 (m, 1H), 0.87 (t, *J* = 7.2 Hz, 3H), 0.64 - 0.57 (m, 1H), 0.48 - 0.40 (m, 1H), 0.34 - 0.29 (m, 1H), 0.23 - 0.14 (m, 1H).

**Example 387:**

Synthetic Route:

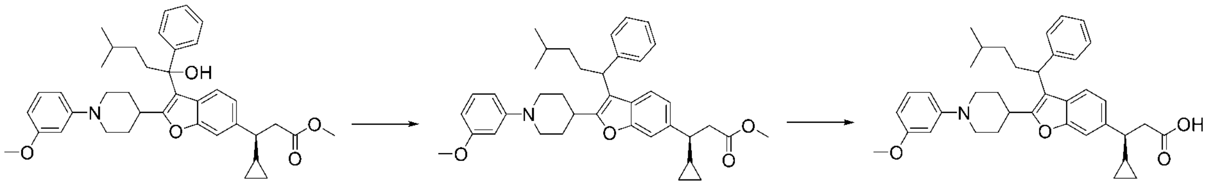

377-6 387-1 387

Referring to the synthetic route of compound **385,** compound **385-2** was replaced with compound **377-6.** The resulting crude product was purified by reverse-phase column chromatography [acetonitrile/water (0.05% formic acid) = 0% to 100%] to obtain compound **387** (20 mg, yield: 57%) as a white solid. MS (ESI, m/z): 580.2 $[M+H]^+$.

$^1$H NMR (400 MHz, $CDCl_3$) δ 7.39 (d, *J* = 8.0 Hz, 1H), 7.35 - 7.30 (m, 2H), 7.29 - 7.26 (m, 3H), 7.21 - 7.13 (m, 2H), 6.99 (d, *J* = 8.4 Hz, 1H), 6.59 (d, *J* = 8.0 Hz, 1H), 6.52 (s, 1H), 6.42 (d, *J* = 7.6 Hz, 1H), 4.11 - 4.03 (m, 1H), 3.85 - 3.74 (m, 5H), 3.01 - 2.89 (m, 1H), 2.85 - 2.73 (m, 4H), 2.47 - 2.39 (m, 1H), 2.28 - 2.08 (m, 4H), 1.81 - 1.71 (m, 2H), 1.62 - 1.53 (m, 1H), 1.25 - 1.15 (m, 2H), 1.09 - 1.01 (m, 1H), 0.90 - 0.82 (m, 6H), 0.63 - 0.54 (m, 1H), 0.48 - 0.38 (m, 1H), 0.38 - 0.27 (m, 1H), 0.23 - 0.12 (m, 1H).

**Example 388:**

Synthetic Route:

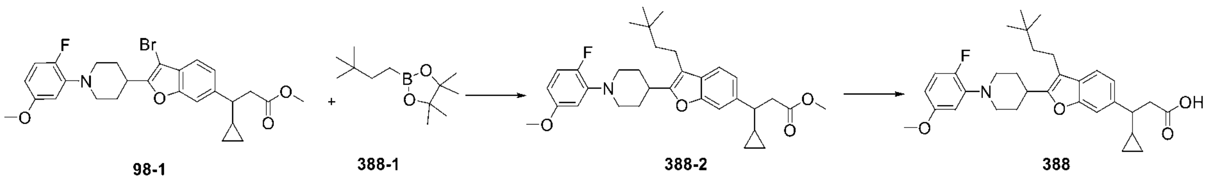

98-1 388-1 388-2 388

Referring to the synthetic route of compound **366,** compound **361-1** was replaced with compound **388-1** to carry out the synthesis. The resulting crude product was purified by reverse-phase column chromatography [acetonitrile/water (0.05% formic acid) = 0% to 100%] to obtain compound **388** (3 mg, yield: 6%) as a white solid. MS (ESI, m/z): 522.2 $[M+H]^+$.

$^1$H NMR (400 MHz, DMSO-$d_6$) δ 7.37 - 7.35 (m, 2H), 7.10 (d, *J* = 7.6 Hz, 1H), 7.05 (dd, *J* = 12.4, 8.8 Hz, 1H), 6.58 (dd, *J* = 7.2, 2.8 Hz, 1H), 6.52 - 6.46 (m, 1H), 3.73 (s, 3H), 3.53 - 3.44 (m, 2H), 3.04 - 2.96 (m, 1H), 2.86 - 2.76 (m, 2H), 2.75 - 2.63 (m, 2H), 2.59 - 2.55 (m, 2H), 2.39 - 2.31 (m, 1H), 2.08 - 1.94 (m, 2H), 1.85 - 1.82 (m, 2H), 1.48 - 1.39 (m, 2H), 1.08 - 1.03 (m, 1H), 1.00 (s, 9H), 0.55 - 0.46 (m, 1H), 0.31 - 0.24 (m, 2H), 0.15 - 0.10 (m, 1H).

**Example 389:**

Synthetic Route:

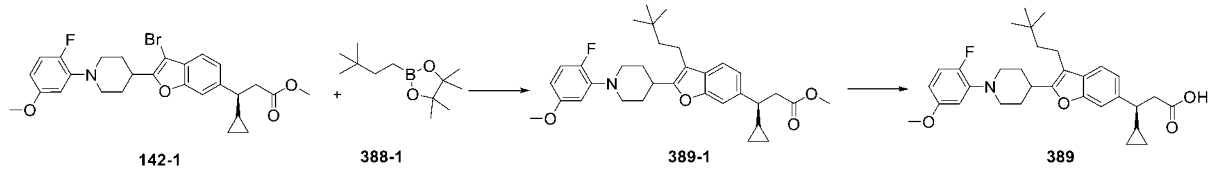

142-1 388-1 389-1 389

Referring to the synthetic route of compound **389,** compound **98-1** was replaced with compound **142-1** to carry out the synthesis. The resulting crude product was purified by reverse-phase column chromatography [acetonitrile/water (0.05% formic acid) = 0% to 100%] to obtain compound **389** (3 mg, yield: 9%) as a white solid. MS (ESI, m/z): 522.3 [M+H]+.

$^1$H NMR (400 MHz, $CDCl_3$) δ 7.39 (d, $J$ = 8.0 Hz, 1H), 7.30 (s, 1H), 7.10 (d, $J$ = 8.0 Hz, 1H), 7.01 - 6.90 (m, 1H), 6.62 - 6.43 (m, 2H), 3.80 (s, 3H), 3.65 - 3.56 (m, 2H), 2.93 - 2.77 (m, 5H), 2.64 - 2.55 (m, 2H), 2.51 - 2.45 (m, 1H), 2.27 - 2.24 (m, 2H), 1.94 - 1.84 (m, 2H), 1.54 - 1.49 (m, 2H), 1.03 (s, 9H), 0.91 - 0.88 (m, 1H), 0.62 - 0.58 (m, 1H), 0.45 - 0.43 (m, 1H), 0.35 - 0.33 (m, 1H), 0.21 - 0.18 (m, 1H).

**Example 390:**

Synthetic Route:

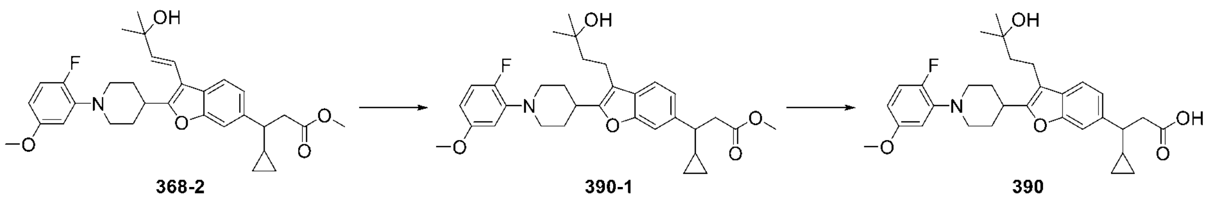

368-2 390-1 390

Compound **368-2** (80 mg, 0.15 mmol) was added to methanol (4 mL) and tetrahydrofuran (4 mL) and stirred. Palladium on carbon (20 mg) was then added, and the reaction mixture was stirred under hydrogen (1 atm) at room temperature for 40 minutes. After the reaction was completed, the reaction mixture was filtered, concentrated, and purified by normal-phase column chromatography (ethyl acetate: petroleum ether = 0% to 100%) to obtain compound **390-1** (80 mg, yield: 99%) as a yellow solid. MS (ESI, m/z): 538.2 [M+H]+.

Compound **390-1** (80 mg, 0.15 mmol) was added to methanol (1 mL), water (1 mL), and tetrahydrofuran (1 mL) and stirred. 4 M sodium hydroxide solution (3 mL) was added, and the reaction mixture was stirred at 70°C for 16 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound **390** (41 mg, yield: 52%) as a white solid. MS (ESI, m/z): 524.2 [M+H]+.

$^1$H NMR (400 MHz, DMSO-$d_6$) δ 7.43 - 7.38 (m, 2H), 7.14 - 7.03 (m, 2H), 6.62 - 6.54 (m, 1H), 6.52 - 6.45 (m, 1H), 4.34 (s, 1H), 3.73 (s, 3H), 3.53 - 3.44 (m, 2H), 3.04 - 2.98 (m, 1H), 2.87 - 2.76 (m, 2H), 2.72 - 2.64 (m, 4H), 2.37 - 2.31 (m, 1H), 2.06 - 1.97 (m, 2H), 1.90 - 1.81 (m, 2H), 1.64 - 1.60 (m, 2H), 1.19 (s, 6H), 1.09 - 1.01 (m, 1H), 0.54 - 0.51 (m, 1H), 0.32 - 0.22 (m, 2H), 0.14 - 0.11 (m, 1H).

**Example 391:**

Synthetic Route:

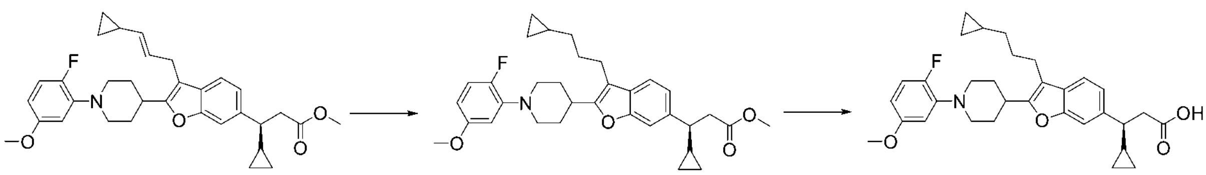

373-2 391-1 391

Referring to the synthetic route of compound **390,** compound **368-2** was replaced with compound **373-2** to carry out the synthesis. The resulting crude product was purified by reverse-phase column chromatography [acetonitrile/water

(0.05% formic acid) = 0% to 100%] to obtain compound **391** (3 mg, yield: 23%) as a white solid. MS (ESI, m/z): 520.3 $[M+H]^+$.

$^1$H NMR (400 MHz, $CDCl_3$) δ 7.41 (d, *J* = 8.0 Hz, 1H), 7.30 (s, 1H), 7.10 (d, *J* = 8.0 Hz, 1H), 6.96 (dd, *J* = 12.0, 9.2 Hz, 1H), 6.59 - 6.54 (m, 1H), 6.47 - 641 (m, 1H), 3.80 (s, 3H), 3.66 - 3.56 (m, 2H), 2.96 - 2.78 (m, 5H), 2.70 - 2.63 (m, 2H), 2.53 - 2.44 (m, 1H), 2.31 - 2.25 (m, 2H), 1.89 - 1.84 (m, 2H), 1.78 - 1.74 (m, 1H), 1.32 - 1.26 (m, 4H), 1.14 - 1.07 (m, 1H), 0.78 - 0.70 (m, 1H), 0.65 - 0.57 (m, 1H), 0.45 - 0.42 (m, 2H), 0.37 - 0.32 (m, 1H), 0.25 - 0.17 (m, 1H), 0.05 - 0.00 (m, 2H).

**Example 392:**

Synthetic Route:

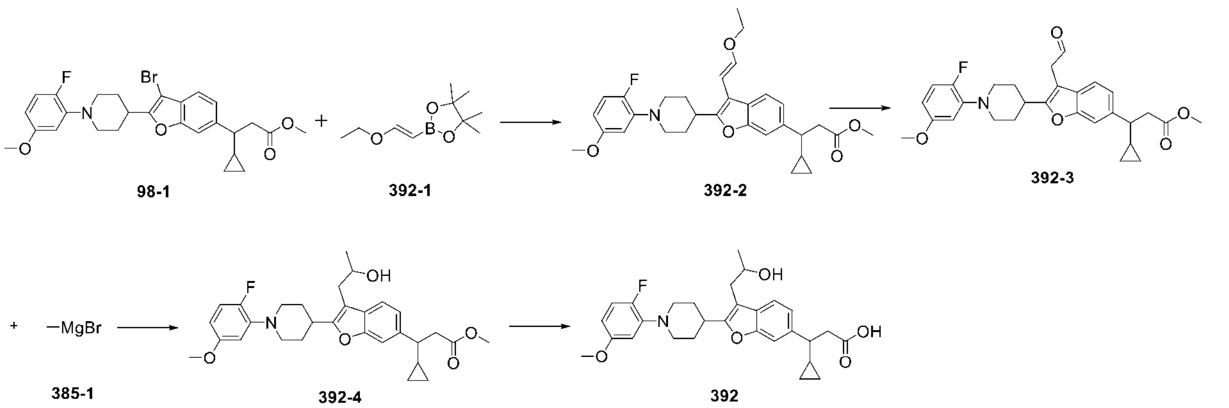

98-1 392-1 392-2 392-3

385-1 392-4 392

Compound **98-1** (200 mg, 0.37 mmol), compound **392-1** (373 mg, 1.88 mmol), [1,1'-bis(diphenylphosphino) ferrocene]palladium(II) chloride dichloromethane complex (26 mg, 0.04 mmol), and potassium carbonate (156 mg, 1.13 mmol) were added to a mixture of 1,4-dioxane: water = 5:1 (25 mL). The reaction mixture was stirred at 80°C for 16 hours. LCMS monitoring confirmed the complete consumption of the starting material. The reaction mixture was extracted with ethyl acetate (50 mL) and concentrated. The resulting crude product was purified by normal-phase column chromatography (ethyl acetate: petroleum ether = 20%) to obtain compound **392-2** (100 mg, yield: 51%) as a yellow oil. MS (ESI, m/z): 522.2 $[M+H]^+$.

Compound **392-2** (41 mg, 0.07 mmol) was added to a mixture of trifluoroacetic acid (4.48 mg, 0.04 mmol) and dichloromethane (4 mL) with stirring. The reaction mixture was stirred at 0°C for 40 minutes. After the reaction was completed, the reaction mixture was filtered, concentrated, and purified by normal-phase column chromatography (ethyl acetate: petroleum ether = 0% to 100%) to obtain compound **392-3** (30 mg, yield: 77%) as a yellow oil. MS (ESI, m/z): 494.2 $[M+H]^+$.

Compound **392-3** (30 mg, 0.06 mmol) was dissolved in tetrahydrofuran (5 mL). Methyl Grignard reagent **385-1** (1.0 M, 0.07 mL) was added at 0°C, and the reaction was carried out at 0°C for 2 hours. The reaction mixture was quenched with saturated ammonium chloride solution (1 mL), concentrated, and purified by normal-phase column chromatography (ethyl acetate: petroleum ether = 10%) to obtain compound **392-4** (30 mg, yield: 96%) as a white solid. LCMS: $[M+H]^+$ = 510.3.

Compound **392-4** (20 mg, 0.04 mmol) was added to methanol (1 mL), water (1 mL), and tetrahydrofuran (1 mL) and stirred. 4 M sodium hydroxide solution (3 mL) was added, and the reaction mixture was stirred at 70°C for 16 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound 392 (18 mg, yield: 54%) as a white solid. MS (ESI, m/z): 496.2 $[M+H]^+$.

$^1$H NMR (400 MHz, MeOD) δ 7.38 (d, *J* = 8.0 Hz, 1H), 7.32 (s, 1H), 7.13 (d, *J* = 8.0 Hz, 1H), 6.95 (dd, *J* = 12.2, 8.8 Hz, 1H), 6.61 (dd, *J* = 7.2, 3.0 Hz, 1H), 6.52 - 6.46 (m, 1H), 4.09 - 4.02 (m, 1H), 3.76 (s, 3H), 3.58 - 3.50 (m, 2H), 3.08 - 2.97 (m, 1H), 2.87 - 2.76 (m, 3H), 2.76 - 2.55 (m, 3H), 2.52 - 2.43 (m, 1H), 2.26 - 2.15 (m, 2H), 1.94 - 1.84 (m, 2H), 1.18 (d, *J*= 6.2 Hz, 3H), 1.10 - 1.02 (m, 1H), 0.60 - 0.52 (m, 1H), 0.41 - 0.30 (m, 2H), 0.14 - 0.06 (m, 1H).

**Example 393:**

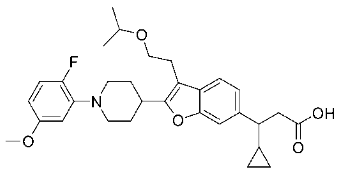

Synthetic Route:

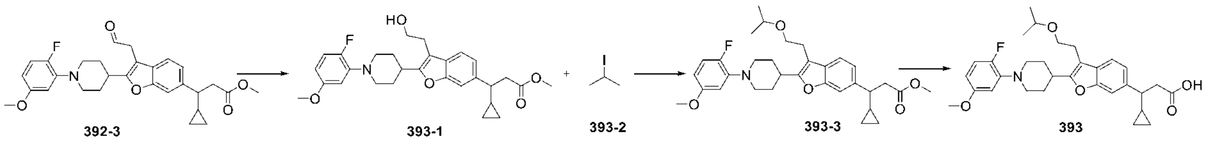

392-3 393-1 393-2 393-3 393

Compound **392-3** (30 mg, 0.06 mmol) was dissolved in tetrahydrofuran (2 mL) and methanol (2 mL). Sodium borohydride (6 mg, 0.16 mmol) was added at 0°C, and the reaction was carried out at 25°C for 2 hours. The reaction mixture was quenched with saturated ammonium chloride solution (1 mL), concentrated, and purified by normal - phase column chromatography (ethyl acetate: petroleum ether = 0% to 100%) to obtain compound **393-1** (20 mg, yield: 67%) as a yellow solid. MS (ESI, m/z): 496.2 $[M+H]^+$.

Compound **393-1** (20 mg, 0.04 mmol) was dissolved in N,N-dimethylformamide (3 mL). Sodium hydride (2 mg, 0.06 mmol) was added at 0°C, and the reaction was carried out at 25°C for 1 hour. Compound **393-2** (55 mg, 0.32 mmol) was then added, followed by quenching with saturated sodium bicarbonate solution (1 mL). The reaction mixture was concentrated and purified by normal-phase column chromatography (ethyl acetate: petroleum ether = 0% to 100%) to obtain compound **393-3** (15 mg, yield: 62%) as a yellow solid. MS (ESI, m/z): 538.3 $[M+H]^+$.

Compound **393-3** (15 mg, 0.03 mmol) was added to methanol (1 mL), water (1 mL), and tetrahydrofuran (1 mL) and stirred. Lithium hydroxide (4 mg, 0.14 mmol) was added, and the reaction mixture was stirred at 25°C for 16 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound 393 (2 mg, yield: 11%) as a white solid. MS (ESI, m/z): 524.2 $[M+H]^+$.

$^1$H NMR (400 MHz, MeOD) δ 7.45 (d, *J* = 8.0 Hz, 1H), 7.31 (s, 1H), 7.20 (dd, *J* = 11.6, 9.0 Hz, 1H), 7.13 (dd, *J* = 8.0, 1.2 Hz, 1H), 7.07 - 6.98 (m, 1H), 6.89 - 6.83 (m, 1H), 3.83 (s, 3H), 3.78 - 3.70 (m, 2H), 3.66 (t, *J* = 6.6 Hz, 2H), 3.63 - 3.55 (m, 1H), 3.46 - 3.35 (m, 2H), 3.31 - 3.23 (m, 1H), 2.92 (t, *J* = 6.6 Hz, 2H), 2.84 - 2.70 (m, 2H), 2.48 - 2.32 (m, 3H), 2.16 - 2.08 (m, 2H), 1.14 - 1.06 (m, 7H), 0.65 - 0.57 (m, 1H), 0.43 - 0.29 (m, 2H), 0.19 - 0.11 (m, 1H).

**Example 394:**

Synthetic Route:

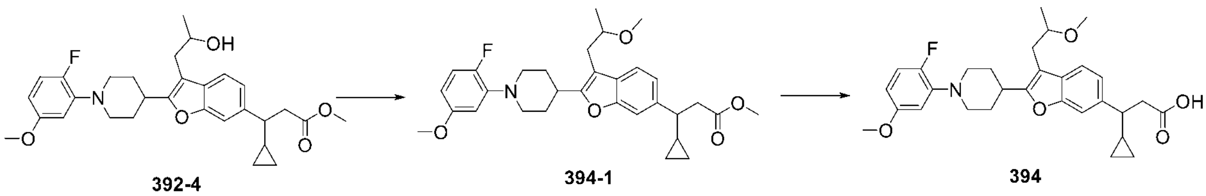

392-4 394-1 394

Compound **392-4** (20 mg, 0.04 mmol) was dissolved in N,N-dimethylformamide (3 mL). Sodium hydride (2 mg, 0.06 mmol) was added at 0°C, and the reaction was carried out at 25°C for 1 hour. Iodomethane (45 mg, 0.31 mmol) was then added, followed by quenching with saturated sodium bicarbonate solution (1 mL). The reaction mixture was concentrated and purified by normal-phase column chromatography (ethyl acetate: petroleum ether = 0% to 100%) to obtain compound **394-1** (15 mg, yield: 73%) as a yellow solid. MS (ESI, m/z): 524.3 $[M+H]^+$.

Compound **394-1** (15 mg, 0.02 mmol) was added to methanol (1 mL), water (1 mL), and tetrahydrofuran (1 mL) and stirred. Lithium hydroxide (4 mg, 0.14 mmol) was added, and the reaction mixture was stirred at 25°C for 4 hours. After the reaction was completed, the reaction mixture was concentrated to obtain a crude product. The resulting crude product

was purified by reverse-phase column chromatography [acetonitrile/water (0.05% formic acid) = 0% to 100%] to obtain compound 394 (3 mg, yield: 18%) as a white solid. MS (ESI, m/z): 510.2 $[M+H]^+$.

$^1H$ NMR (400 MHz, DMSO-$d_6$) δ 7.36 (d, *J* = 8.0 Hz, 1H), 7.28 (s, 1H), 7.09 - 7.00 (m, 2H), 6.58 (dd, *J* = 7.6, 2.8 Hz, 1H), 6.52 - 6.45 (m, 1H), 3.73 (s, 3H), 3.58 - 3.43 (m, 3H), 3.25 (s, 3H), 3.05 - 2.96 (m, 1H), 2.88 - 2.76 (m, 3H), 2.68 - 2.62 (m, 1H), 2.51 - 2.40 (m, 3H), 2.08 - 1.96 (m, 2H), 1.87 - 1.77 (m, 2H), 1.08 (d, *J* = 6.0 Hz, 3H), 0.99 - 0.90(m, 1H), 0.45 - 0.37 (m, 1H), 0.28 - 0.19 (m, 2H), 0.08 - 0.03 (m, 1H).

**Example 395:**

Synthetic Route:

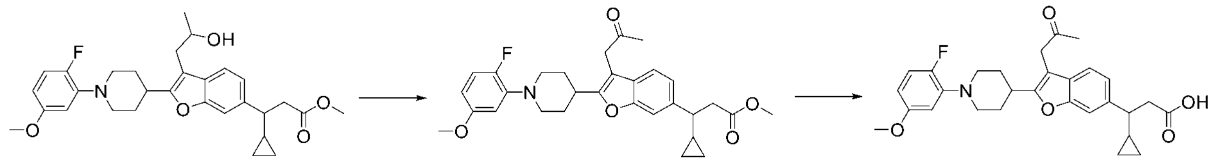

392-4 395-1 395

Compound **392-4** (25 mg, 0.05 mmol) was dissolved in *N,N*-dimethylformamide (3 mL). Dess-Martin periodinane (31 mg, 0.07 mmol) was added at 0°C, and the reaction was carried out at 25°C for 1 hour. The reaction was quenched with water (10 mL) and extracted with ethyl acetate (10 mL × 3). The organic phase was concentrated and purified by normal-phase column chromatography (ethyl acetate: petroleum ether = 0% to 100%) to obtain compound **395-1** (15 mg, yield: 60%) as a yellow solid. MS (ESI, m/z): 508.3 $[M+H]^+$.

Compound **395-1** (15 mg, 0.03 mmol) was added to methanol (1 mL), water (1 mL), and tetrahydrofuran (1 mL) and stirred. Lithium hydroxide (4 mg, 0.14 mmol) was added, and the reaction mixture was stirred at 25°C for 16 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound **395** (1 mg, yield: 8%) as a white solid. MS (ESI, m/z): 494.2 $[M+H]^+$.

$^1H$ NMR (400 MHz, MeOD) δ 7.37 - 7.29 (m, 2H), 7.13 (d, *J* = 8.0 Hz, 1H), 6.95 (dd, *J* = 12.2, 8.8 Hz, 1H), 6.61 (dd, *J* = 7.2, 3.0 Hz, 1H), 6.52 - 6.47 (m, 1H), 3.83 (s, 2H), 3.76 (s, 3H), 3.58 - 3.50 (m, 2H), 3.06 - 2.95 (m, 1H), 2.86 - 2.69 (m, 4H), 2.50 - 2.41 (m, 1H), 2.24 - 2.12 (m, 5H), 1.94 - 1.83 (m, 2H), 1.12 - 1.04 (m, 1H), 0.62 - 0.54 (m, 1H), 0.42 - 0.29 (m, 2H), 0.18 - 0.11 (m, 1H).

**Example 396:**

Synthetic Route:

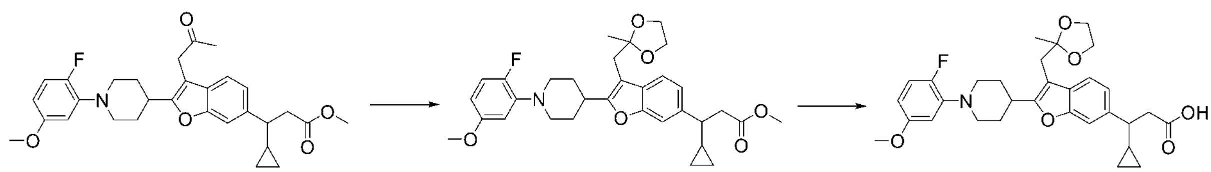

395-1 396-1 396

Compound **395-1** (20 mg, 0.04 mmol) was dissolved in dichloromethane (5 mL), followed by the addition of 1,2-ethanediol (25 mg, 0.39 mmol), triethyl orthoformate (8 mg, 0.06 mmol), and D-camphorsulfonic acid (0.9 mg, 0.01 mmol). The reaction was carried out at 25°C for 24 hours, then quenched with saturated sodium bicarbonate (10 mL) and extracted with ethyl acetate (10 mL × 3). The organic phase was concentrated and purified by normal-phase column chromatography (ethyl acetate: petroleum ether = 0% to 100%) to obtain compound **396-1** (15 mg, yield: 55%) as a yellow solid. MS (ESI, m/z): 552.3 $[M+H]^+$.

Compound **396-1** (15 mg, 0.03 mmol) was added to methanol (1 mL), water (1 mL), and tetrahydrofuran (1 mL) and stirred. Lithium hydroxide (4 mg, 0.14 mmol) was added, and the reaction mixture was stirred at 25°C for 16 hours. After the reaction was completed, the reaction mixture was concentrated to obtain a crude product. The resulting crude product was purified by reverse-phase column chromatography [acetonitrile/water (0.05% formic acid) = 0% to 100%] to

obtain compound **396** (1 mg, yield: 3%) as a white solid. MS (ESI, m/z): 538.2 [M+H]$^+$.

$^1$H NMR (400 MHz, MeOD) δ 7.44 (d, *J* = 8.0 Hz, 1H), 7.30 (s, 1H), 7.12 (d, *J* = 7.4 Hz, 1H), 6.95 (dd, *J* = 12.2, 9.0 Hz, 1H), 6.62 (dd, *J* = 7.4, 2.8 Hz, 1H), 6.51 - 6.46 (m, 1H), 3.88 - 3.83 (m, 2H), 3.78 - 3.73 (m, 5H), 3.58 - 3.50 (m, 2H), 3.07 - 2.98 (m, 1H), 2.97 (s, 2H), 2.84 - 2.76 (m, 2H), 2.68 - 2.44 (m, 3H), 2.24 - 2.12 (m, 2H), 1.93 - 1.88 (m, 2H), 1.34 (s, 3H), 1.09 - 1.02 (m, 1H), 0.58 - 0.51 (m, 1H), 0.40 - 0.33 (m, 2H), 0.14 - 0.09 (m, 1H).

**Example 397:**

Synthetic Route:

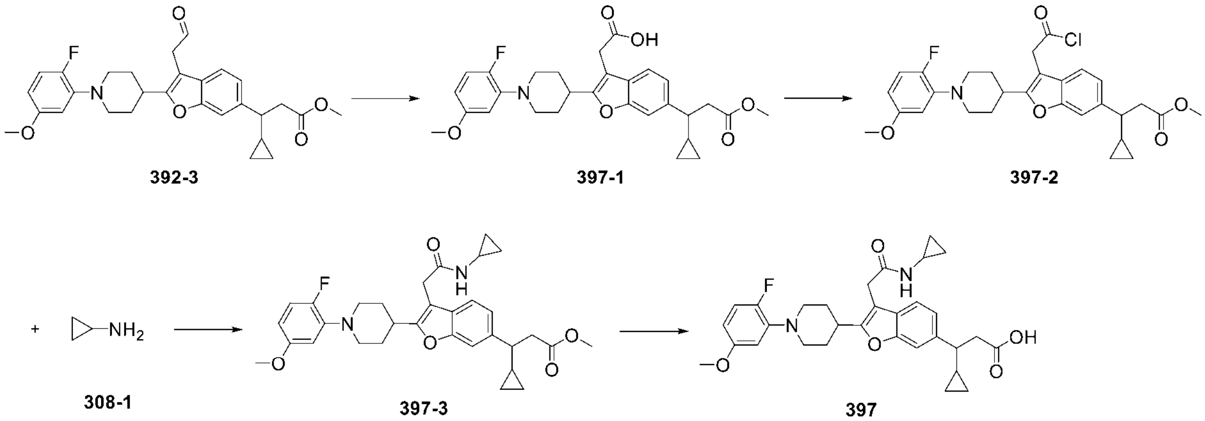

392-3 397-1 397-2

308-1 397-3 397

Compound **392-3** (90 mg, 0.15 mmol) was added to methanol (4 mL) and tetrahydrofuran (4 mL) and stirred. Platinum dioxide (10 mg, 0.044 mmol) was then added, and the reaction mixture was stirred under hydrogen (1 atm) at room temperature for 2 hours. After the reaction was completed, the reaction mixture was filtered, concentrated, and purified by normal-phase column chromatography (methanol: dichloromethane = 0% to 20%) to obtain compound **397-1** (78 mg, yield: 99%) as a white solid. MS (ESI, m/z): 510.2 [M+H]$^+$.

Referring to the synthetic route of compound **308,** compound **305-3** was replaced with compound **397-1** to carry out the synthesis. The resulting crude product was purified by reverse-phase column chromatography [acetonitrile/water (0.05% formic acid) = 0% to 100%] to obtain compound **397** (12 mg, yield: 20%) as a white solid. MS (ESI, m/z): 535.2 [M+H]$^+$.

$^1$H NMR (400 MHz, $CDCl_3$) δ 7.36 (s, 1H), 7.32 (d, *J* = 8.0 Hz, 1H), 7.13 (d, *J* = 8.0 Hz, 1H), 6.97 (dd, *J* = 12.0, 8.8 Hz, 1H), 6.60 - 6.54 (m, 1H), 6.50 - 6.42 (m, 1H), 5.82 - 5.74 (m, 1H), 3.80 (s, 3H), 3.60 - 3.55 (m, 2H), 3.53 (s, 2H), 2.92 - 2.88 (m, 1H), 2.88 - 2.77 (m, 4H), 2.68 - 2.61 (m, 1H), 2.53 - 2.45 (m, 1H), 2.30 - 2.17 (m, 2H), 1.89 - 1.79 (m, 2H), 1.15 - 1.03 (m, 1H), 0.76 - 0.69 (m, 2H), 0.67 - 0.60 (m, 1H), 0.48 - 0.42 (m, 1H), 0.41 - 0.37 (m, 2H), 0.37 - 0.32 (m, 1H), 0.25 - 0.16 (m, 1H).

**Example 398:**

Synthetic Route:

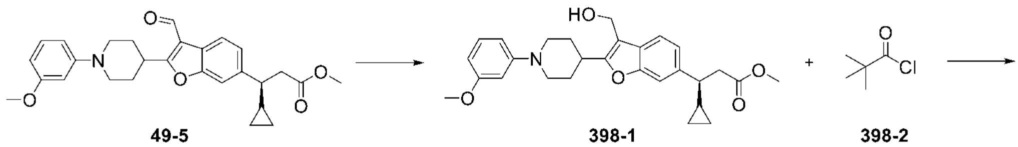

49-5 398-1 398-2

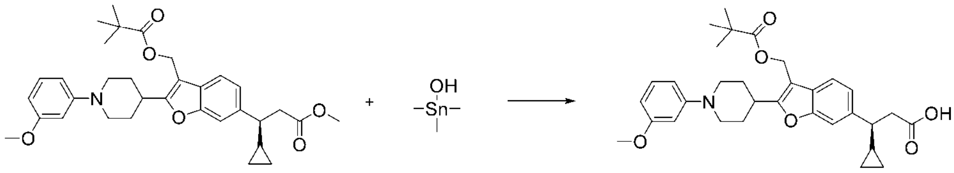

398-3 398-4 398

Compound **49-5** (168 mg, 0.364 mmol) was dissolved in methanol (4 mL) and tetrahydrofuran (4 mL). Sodium borohydride (15 mg, 0.4 mmol) was added at 0°C, and the reaction was carried out at 0°C for 3 hours. After concentration, the resulting crude product was purified by normal-phase column chromatography (ethyl acetate: petroleum ether = 0% to 100%) to obtain compound **398-1** (136 mg, yield: 81%) as a yellow oil. MS (ESI, m/z): 464.3 $[M+H]^+$.

Compound **398-1** (68 mg, 0.147 mmol), compound **398-2** (100 μL, 0.2 mmol, 2 M), and triethylamine (100 μL) were dissolved in dichloromethane (4 mL). The reaction was carried out at 25°C for 24 hours. After concentration, the resulting crude product was purified by normal-phase column chromatography (ethyl acetate: petroleum ether = 0% to 100%) to obtain compound **398-3** (71 mg, yield: 88%) as a yellow oil. MS (ESI, m/z): 548.3 $[M+H]^+$.

Compound **398-3** (71 mg, 0.13 mmol) and compound **398-4** (47 mg, 0.26 mmol) were dissolved in dichloroethane (5 mL) and reacted at 25°C for 24 hours. After concentration, the resulting crude product was purified by reverse-phase column chromatography [acetonitrile/water (0.05% formic acid) = 0% to 100%] to obtain compound **398** (14 mg, yield: 20%) as a white solid. MS (ESI, m/z): 534.2 $[M+H]^+$.

$^1$H NMR (400 MHz, $CDCl_3$) δ 7.51 (d, *J* = 8.0 Hz, 1H), 7.35 (s, 1H), 7.25 - 7.19 (m, 1H), 7.15 (d, *J* = 8.0 Hz, 1H), 6.66 - 6.62 (m, 1H), 6.58 - 6.55 (m, 1H), 6.49 - 6.44 (m, 1H), 5.24 (s, 2H), 3.91 - 3.78 (m, 5H), 3.19 - 3.04 (m, 1H), 2.97 - 2.80 (m, 4H), 2.57 - 2.45 (m, 1H), 2.30 - 2.12 (m, 2H), 1.99 - 1.88 (m, 2H), 1.22 (s, 9H), 1.17 - 1.06 (m, 1H), 0.72 - 0.58 (m, 1H), 0.53 - 0.42 (m, 1H), 0.41 - 0.31 (m, 1H), 0.27 - 0.16 (m, 1H).

**Example 399:**

Synthetic Route:

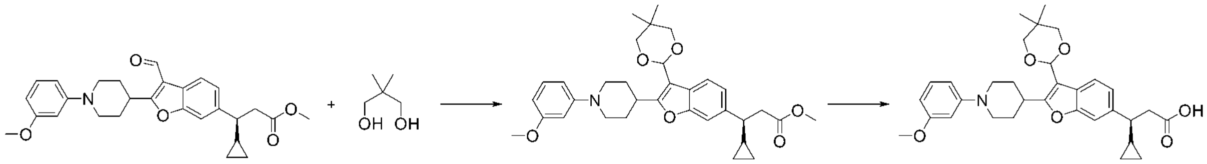

49-5 399-1 399-2 399

Compound **49-5** (138 mg, 0.3 mmol), compound **399-1** (312 mg, 3.0 mmol), and p-toluenesulfonic acid monohydrate (8 mg, 0.03 mmol) were dissolved in toluene (2 mL) and refluxed for 2 hours. After concentration, the resulting crude product was purified by normal-phase column chromatography (ethyl acetate: petroleum ether = 0% to 100%) to obtain compound **399-2** (148 mg, yield: 90%) as a white solid. MS (ESI, m/z): 548.3 $[M+H]^+$.

Compound **399-2** (148 mg, 0.27 mmol) was added to methanol (5 mL). Then, 1 mL of an aqueous solution of NaOH (108 mg, 2.7 mmol) was added dropwise to the reaction, and the reaction mixture was stirred at room temperature overnight. Dilute hydrochloric acid was added to adjust the pH to 3, followed by extraction with ethyl acetate. The organic phase was dried over anhydrous sodium sulfate, filtered, and concentrated. The resulting crude product was purified by reverse-phase column chromatography [acetonitrile/water (0.05% formic acid) = 0% to 100%] to obtain compound **399** (122 mg, yield: 85%) as a white solid. MS (ESI, m/z): 534.2 $[M+H]^+$.

$^1$H NMR (400 MHz, $CDCl_3$) δ 7.72 (d, *J* = 8.0 Hz, 1H), 7.26 (s, 1H), 7.26 - 7.22 (m, 1H), 7.09 (d, *J* = 8.4 Hz, 1H), 6.62 (d, *J* = 8.0 Hz, 1H), 6.54 (s, 1H), 6.43 (d, *J* = 8.4 Hz, 1H), 5.68 (s, 1H), 3.87 - 3.75 (m, 7H), 3.71 - 3.64 (m, 2H), 3.22 - 3.08 (m, 1H), 2.91 - 2.73 (m, 4H), 2.52 - 2.42 (m, 1H), 2.24 - 2.09 (m, 2H), 2.00 - 1.91 (m, 2H), 1.39 (s, 3H), 1.09 - 0.99 (m, 1H), 0.83 (s, 3H), 0.62 - 0.53 (m, 1H), 0.42 - 0.34 (m, 1H), 0.32 - 0.25 (m, 1H), 0.16 - 0.08 (m, 1H).

**Example 400:**

Synthetic Route:

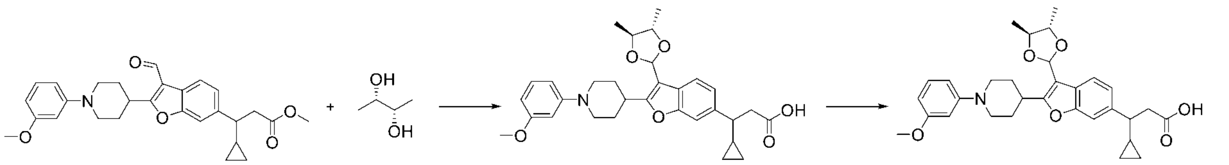

126-2 400-1 400-2 400

Referring to the synthetic route of compound **399,** compound **399-1** was replaced with compound **400-1** to carry out the synthesis. The resulting crude product was purified by reverse-phase column chromatography [acetonitrile/water (0.05% formic acid) = 0% to 100%] to obtain compound **400** (116 mg, yield: 97%) as a white solid. MS (ESI, m/z): 520.2 $[M+H]^+$.

$^1$H NMR (400 MHz, $CDCl_3$) δ 7.58 (d, *J* = 8.0 Hz, 1H), 7.29 (s, 1H), 7.24 - 7.16 (m, 1H), 7.09 (d, *J* = 8.0 Hz, 1H), 6.75 - 6.36 (m, 3H), 6.22 (s, 1H), 3.97 - 3.72 (m, 7H), 3.21-3.05 (m, 1H), 2.99 - 2.73 (m, 4H), 2.52 - 2.46 (m, 1H), 2.25 - 2.08 (m, 2H), 2,02 - 1.93 (m, 2H), 1.44 (d, *J* = 5.6 Hz, 3H), 1.36 (d, *J* = 5.6 Hz, 3H), 1.09 - 1.01 (m 1H), 0.65 - 0.53 (m, 1H), 0.44 - 0.36 (m, 1H), 0.34 - 0.27 (m, 1H), 0.18 - 0.11 (m, 1H).

**Example 401:**

Synthetic Route:

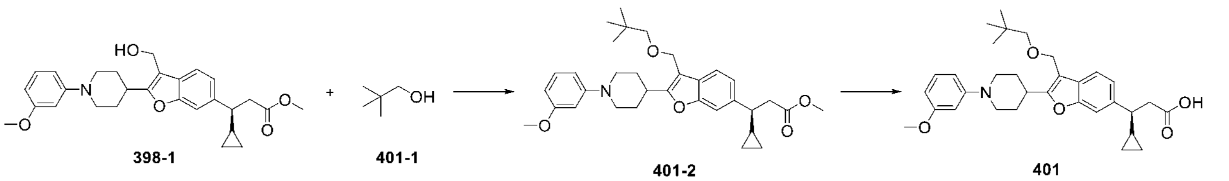

398-1 401-1 401-2 401

Compound **398-1** (30 mg, 0.0650 mmol) was dissolved in dichloromethane (1 mL), and trichloroacetonitrile (10 mg, 0.0679 mmol) and 1,8-diazabicyclo[5,4,0]undec-7-ene (0.1 mL) were added. The reaction was carried out at room temperature for 2 hours, then concentrated and dissolved in dichloromethane (2 mL). 2-Methyl-2-butanol (0.5 mL) and trifluoromethanesulfonic acid (0.05 mL) were added, and the reaction was carried out at room temperature overnight. After concentration, the resulting crude product was purified by normal-phase column chromatography (ethyl acetate: petroleum ether = 10%) to obtain compound **401-2** (28 mg, yield: 82%) as a white solid. MS (ESI, m/z): 634.3 $[M+H]^+$.

Compound **401-2** (28 mg, 0.044 mmol), lithium hydroxide (8.2 mg, 0.341 mmol), tetrahydrofuran (1 mL), methanol (1 mL), and water (1 mL) were reacted at 50°C for 2 hours. After concentration, the resulting crude product was purified by reverse-phase column chromatography [acetonitrile/water (0.05% formic acid) = 0% to 100%] to obtain compound **401** (19 mg, yield: 68%) as a white solid. MS (ESI, m/z): 620.2 $[M+H]^+$.

$^1$H NMR (400 MHz, $CDCl_3$) δ 7.53 (d, *J* = 8.0 Hz, 1H), 7.33 (s, 1H), 7.25 - 7.20 (m, 1H), 7.13 (d, *J* = 8.0 Hz, 1H), 6.65 (d, *J* = 8.4 Hz, 1H), 6.58 (s, 1H), 6.47 (d, *J* = 7.6 Hz, 1H), 4.64 (s, 2H), 3.87 - 3.82 (m, 5H), 3.18 (s, 2H), 3.13 - 3.05 (m, 1H), 2.94 - 2.82 (m, 4H), 2.55 - 2.47 (m, 1H), 2.26 - 2.15 (m, 2H), 1.99 - 1.91 (m, 2H), 1.16 - 1.07 (m, 1H), 0.95 (s, 9H), 0.68 - 0.60 (m, 1H), 0.53 - 0.43 (m, 1H), 0.39 - 0.33 (m, 1H), 0.24 - 0.17 (m, 1H).

**Example 402:**

Synthetic Route:

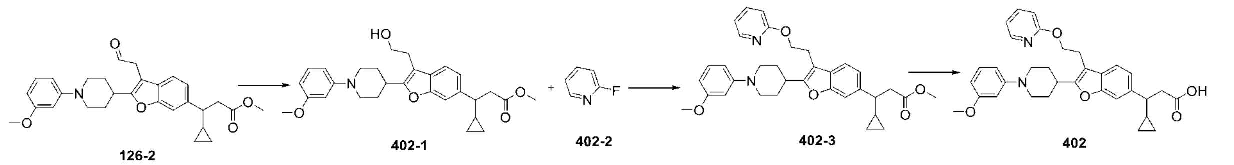

126-2 402-1 402-2 402-3 402

Referring to the synthetic route of compound **393,** compound **392-3** was replaced with compound **126-2,** and compound **393-2** was replaced with compound **402-3.** The synthesis was carried out, and the resulting crude product was purified by reverse-phase column chromatography [acetonitrile/water (0.05% formic acid) = 0% to 100%] to obtain compound **400** (60 mg, yield: 50%) as a white solid. MS (ESI, m/z): 555.2 [M+H]$^+$.

$^1$H NMR (400 MHz, $CDCl_3$) δ 8.15 (d, *J*= 5.2 Hz, 1H), 7.60 - 7.54 (m, 1H), 7.50 (d, *J* = 8.0 Hz, 1H), 7.31 (s, 1H), 7.25 - 7.17 (m, 1H), 7.12 (d, *J* = 8.0 Hz, 1H), 6.90 - 6.83 (m, 1H), 6.69 (d, *J* = 8.4 Hz, 1H), 6.66 - 6.59 (m, 1H), 6.59 - 6.51 (m, 1H), 6.49 - 6.39 (m, 1H), 4.52 (t, *J* = 6.8 Hz, 2H), 3.90 - 3.75 (m, 5H), 3.13 (t, *J* = 6.8 Hz, 2H), 3.06 - 2.94 (m, 1H), 2.93 - 2.75 (m, 4H), 2.56 - 2.41 (m, 1H), 2.31 - 2.07 (m, 2H), 1.96 - 1.85 (m, 2H), 1.15 - 1.01 (m, 1H), 0.68 - 0.56 (m, 1H), 0.48 - 0.38 (m, 1H), 0.37 - 0.30 (m, 1H), 0.26 - 0.10 (m, 1H).

**Example 403:**

Synthetic Route:

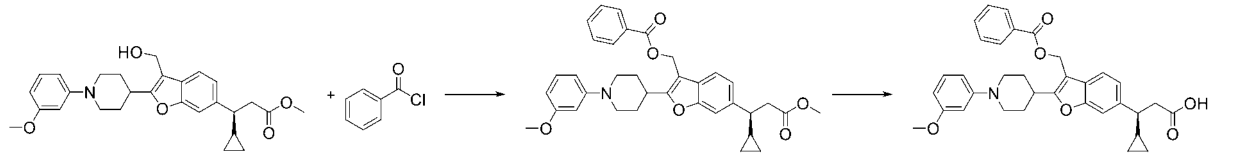

398-1 403-1 403-2 403

Referring to the synthetic route of compound **398,** compound **398-2** was replaced with compound **403-1** to carry out the synthesis. The resulting crude product was purified by reverse-phase column chromatography [acetonitrile/water (0.05% formic acid) = 0% to 100%] to obtain compound **403** (3 mg, yield: 3%) as a white solid. MS (ESI, m/z): 554.2 [M+H]$^+$.

$^1$H NMR (400 MHz, $CDCl_3$) δ 8.07 (d, *J* = 8.0 Hz, 2H), 7.63 - 7.54 (m, 2H), 7.48 - 7.42 (m, 2H), 7.36 (s, 1H), 7.25 - 7.20 (m, 1H), 7.17 (d, *J* = 8.0 Hz, 1H), 6.67 - 6.61 (m, 1H), 6.60 - 6.54 (m, 1H), 6.50 - 6.43 (m, 1H), 5.52 (s, 2H), 3.90 - 3.81 (m, 5H), 3.27 - 3.16 (m, 1H), 2.98 - 2.79 (m, 4H), 2.55 - 2.46 (m, 1H), 2.30 - 2.17 (m, 2H), 2.02 - 1.92 (m, 2H), 1.16 - 1.04 (m, 1H), 0.69 - 0.59 (m, 1H), 0.50 - 0.41 (m, 1H), 0.40 - 0.30 (m, 1H), 0.25 - 0.16 (m, 1H).

**Example 404:**

Synthetic Route:

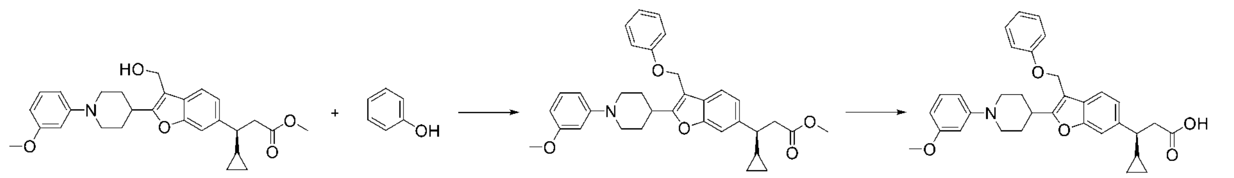

398-1 404-1 404-2 404

Referring to the synthetic route of compound **401,** compound **401-1** was replaced with compound **404-1** to carry out the synthesis. The resulting crude product was purified by reverse-phase column chromatography [acetonitrile/water (0.05% formic acid) = 0% to 100%] to obtain compound **404** (8 mg, yield: 40%) as a white solid. MS (ESI, m/z): 526.2 [M+H]$^+$.

$^1$H NMR (400 MHz, $CDCl_3$) δ 7.55 (d, *J* = 8.0 Hz, 1H), 7.38 - 7.31 (m, 4H), 7.17 (d, *J* = 8.0 Hz, 1H), 7.07 - 7.00 (m, 3H), 6.79 - 6.38 (m, 3H), 5.18 (s, 2H), 3.89 - 3.77 (m, 6H), 2.95 - 2.83 (m, 4H), 2.56 - 2.49 (m, 1H), 2.28 - 2.20 (m, 2H), 1.96 - 1.90 (m, 2H), 1.15 - 1.10 (m, 1H), 0.69 - 0.63 (m, 1H), 0.50 - 0.45 (m, 1H), 0.39 - 0.32 (m, 1H), 0.26 - 0.19 (m, 1H).

**Example 405:**

Synthetic Route:

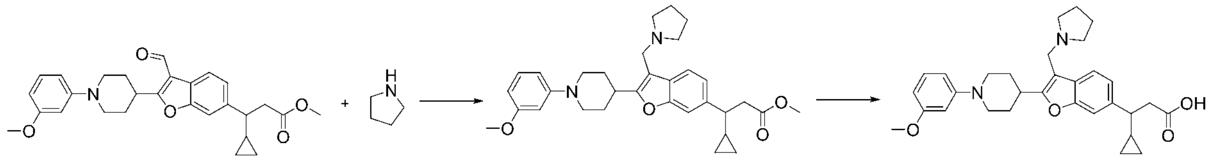

126-2 200-1 405-1 405

Compound **126-2** (20 mg, 0.04 mmol) was dissolved in 1,2-dichloroethane (5 mL), followed by the addition of pyrrolidine (15.4 mg, 0.21 mmol) and anhydrous magnesium sulfate (15.59 mg, 0.13 mmol). The reaction was carried out at room temperature for 4 hours. Sodium triacetoxyborohydride (27.53 mg, 0.13 mmol) and acetic acid (0.1 mL) were then added, and the reaction was carried out at room temperature overnight. The reaction mixture was quenched with saturated sodium bicarbonate (10 mL) and extracted with dichloromethane (10 mL × 3). The combined organic phases were concentrated and purified by normal-phase column chromatography (ethyl acetate: petroleum ether = 0% to 100%) to obtain compound **405-1** (20 mg, yield: 71%) as a colorless oil. MS (ESI, m/z): 517.3 [M+H]+.

Compound **405-1** (20 mg, 0.03 mmol) was added to methanol (1 mL), water (1 mL), and tetrahydrofuran (1 mL) and stirred. Lithium hydroxide (5.56 mg, 0.23 mmol) was added, and the reaction mixture was stirred at 25°C for 16 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound **405** (13 mg, yield: 27%) as a white solid. MS (ESI, m/z): 515.2 [M+H]+.

$^1H$ NMR (400 MHz, MeOD) δ 7.57 (d, *J* = 8.0 Hz, 1H), 7.40 (s, 1H), 7.23 (d, *J* = 8.0 Hz, 1H), 7.19 - 7.12 (m, 1H), 6.63 (dd, *J* = 8.2, 2.0 Hz, 1H), 6.58 - 6.53 (m, 1H), 6.45 (dd, *J* = 8.0, 2.1 Hz, 1H), 4.39 (s, 2H), 3.87 - 3.72 (m, 5H), 3.27 - 3.14 (m, 4H), 2.92 - 2.82 (m, 2H), 2.78 - 2.58 (m, 2H), 2.52 - 2.40 (m, 1H), 2.24 - 2.11 (m, 2H), 2.09 - 1.98 (m, 4H), 1.95 - 1.83 (m, 2H), 1.13 - 0.99 (m, 1H), 0.66 - 0.51 (m, 1H), 0.42 - 0.29 (m, 2H), 0.17 - 0.07 (m, 1H).

**Example 406:**

Synthetic Route:

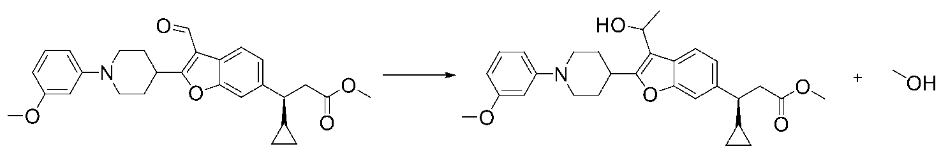

49-5 406-1 406-2

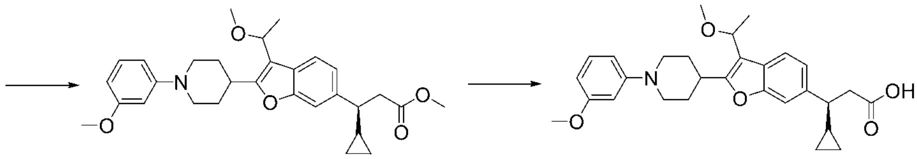

406-3 406

Compound **49-5** (1000 mg, 2.2 mmol) was dissolved in tetrahydrofuran (10 mL), and methyl Grignard reagent (1.0 M, 4.3 mL) was added dropwise. The reaction was carried out at room temperature overnight, then quenched with saturated ammonium chloride solution (1 mL). After concentration, the resulting crude product was purified by normal-phase column chromatography (ethyl acetate: petroleum ether = 0% to 100%) to obtain compound **406-1** (620 mg, yield = 62%) as a white solid. MS (ESI, m/z): 478.3 [M+H]+.

Referring to the synthetic route of compound **401,** compound **398-1** was replaced with compound **406-1,** and compound **401-1** was replaced with compound **406-2.** The synthesis was carried out, and the resulting crude product was purified by reverse-phase column chromatography [acetonitrile/water (0.05% formic acid) = 0% to 100%] to obtain compound **406** (38 mg, yield: 65%) as a white solid. MS (ESI, m/z): 478.3 [M+H]+.

$^1$H NMR (400 MHz, $CDCl_3$) δ 7.41 (d, $J$ = 8.0 Hz, 1H), 7.11 (s, 1H), 7.05 - 6.96 (m, 1H), 6.89 (dd, $J$ = 8.0, 1.2 Hz, 1H), 6.42 (d, $J$ = 8.0 Hz, 1H), 6.34 (s, 1H), 6.25 (d, $J$ = 7.2 Hz, 1H), 4.49 - 4.37 (m, 1H), 3.65 - 3.58 (m, 5H), 3.06 (s, 3H), 2.85 - 2.75 (m, 1H), 2.72 - 2.58 (m, 4H), 2.33 - 2.23 (m, 1H), 2.07 - 1.94 (m, 2H), 1.76 - 1.61 (m, 2H), 1.39 (d, $J$ = 6.4 Hz, 3H), 0.92 - 0.85 (m, 1H), 0.47 - 0.35 (m, 1H), 0.28 - 0.18 (m, 1H), 0.15 - 0.08 (m, 1H), 0.03 - 0.03 (m, 1H).

**Example 407:**

Synthetic Route:

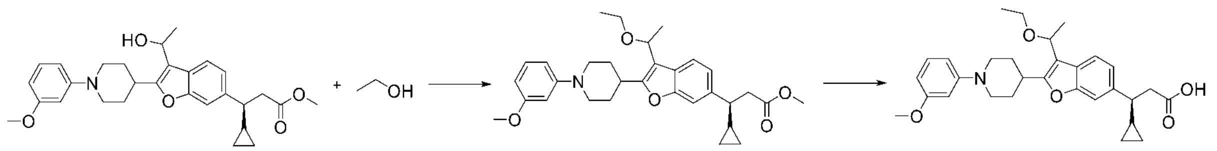

406-1 407-1 407-2 407

Referring to the synthetic route of compound **406,** compound **406-2** was replaced with compound **407-1** to carry out the synthesis. The resulting crude product was purified by reverse-phase column chromatography [acetonitrile/water (0.05% formic acid) = 0% to 100%] to obtain compound **407** (27 mg, yield: 66%) as a white solid. MS (ESI, m/z): 492.3 [M+H]$^+$.

$^1$H NMR (400 MHz, $CDCl_3$) δ 7.61 (d, $J$ = 8.0 Hz, 1H), 7.29 (s, 1H), 7.22 - 7.16 (m, 1H), 7.07 (d, $J$ = 8.0 Hz, 1H), 6.61 (d, $J$ = 8.4 Hz, 1H), 6.53 (s, 1H), 6.44 (d, $J$ = 6.4 Hz, 1H), 4.72 (q, $J$ = 6.4 Hz, 1H), 3.81 (m, 5H), 3.46 - 3.32 (m, 2H), 3.06 - 2.95 (m, 1H), 2.91 - 2.76 (m, 4H), 2.51 - 2.42 (m, 1H), 2.27 - 2.12 (m, 2H), 1.94 - 1.77 (m, 2H), 1.58 (d, $J$ = 6.8 Hz, 3H), 1.18 (t, $J$ = 6.8 Hz, 3H), 1.12 - 1.01 (m, 1H), 0.65 - 0.56 (m, 1H), 0.48 - 0.39 (m, 1H), 0.36 - 0.28 (m, 1H), 0.23 - 0.14 (m, 1H).

**Example 408:**

Synthetic Route:

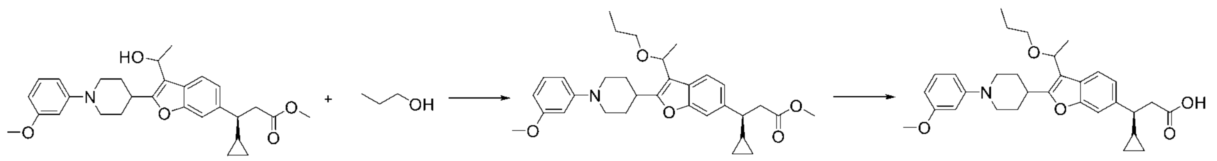

406-1 408-1 408-2 408

Referring to the synthetic route of compound **406,** compound **406-2** was replaced with compound **408-1** to carry out the synthesis. The resulting crude product was purified by reverse-phase column chromatography [acetonitrile/water (0.05% formic acid) = 0% to 100%] to obtain compound **408** (58 mg, yield: 71%) as a white solid. MS (ESI, m/z): 506.3 [M+H]$^+$.

$^1$H NMR (400 MHz, $CDCl_3$) δ 7.41 (d, $J$ = 8.0 Hz, 1H), 7.10 (s, 1H), 7.03 - 6.95 (m, 1H), 6.88 (dd, $J$ = 8.0, 1.2 Hz, 1H), 6.41 (d, $J$ = 8.0 Hz, 1H), 6.34 (s, 1H), 6.24 (d, $J$ = 8.0 Hz, 1H), 4.51 (q, $J$ = 6.4 Hz, 1H), 3.68 - 3.53 (m, 5H), 3.14 - 3.03 (m, 2H), 2.88 - 2.76 (m, 1H), 2.70 - 2.59 (m, 4H), 2.31 - 2.19 (m, 1H), 2.09 - 1.90 (m, 2H), 1.72 - 1.58 (m, 2H), 1.45 - 1.32 (m, 5H), 0.94 - 0.82 (m, 1H), 0.68 (t, $J$ = 7.6 Hz, 3H), 0.46 - 0.46 (m, 1H), 0.29 - 0.21 (m, 1H), 0.16 - 0.09 (m, 1H), 0.03 - -0.07 (m, 1H).

**Example 409:**

Synthetic Route:

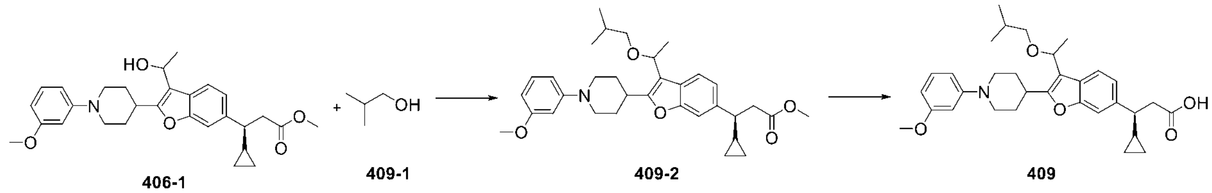

406-1 409-1 409-2 409

Referring to the synthetic route of compound **406,** compound **406-2** was replaced with compound **409-1** to carry out the synthesis. The resulting crude product was purified by reverse-phase column chromatography [acetonitrile/water (0.05% formic acid) = 0% to 100%] to obtain compound **409** (32 mg, yield: 64%) as a white solid. MS (ESI, m/z): 520.3 $[M+H]^+$.

$^1H$ NMR (400 MHz, $CDCl_3$) δ 7.62 (d, *J* = 8.0 Hz, 1H), 7.32 (s, 1H), 7.26 - 7.20 (m, 1H), 7.10 (d, *J* = 8.0 Hz, 1H), 6.69 - 6.60 (m, 1H), 6.59 - 6.53 (m, 1H), 6.51 - 6.43 (m, 1H), 4.71 (q, *J* = 6.4 Hz, 1H), 3.89 - 3.81 (m, 5H), 3.13 (d, *J* = 8.0 Hz, 2H), 3.08 - 3.00 (m, 1H), 2.94 - 2.83 (m, 4H), 2.55 - 2.46 (m, 1H), 2.28 - 2.15 (m, 2H), 1.95 - 1.83 (m, 3H), 1.60 (d, *J* = 6.4 Hz, 3H), 1.15 - 1.06 (m, 1H), 0.93 - 0.87 (m, 6H), 0.67 - 0.60 (m, 1H), 0.51 - 0.42 (m, 1H), 0.40 - 0.32 (m, 1H), 0.27 - 0.19 (m, 1H).

**Example 410:**

Synthetic Route:

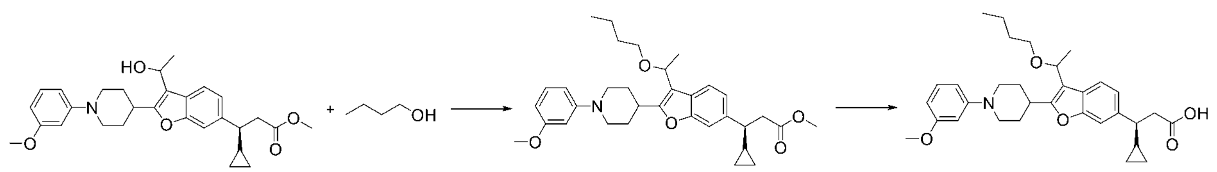

406-1 410-1 410-2 410

Referring to the synthetic route of compound **406,** compound **406-2** was replaced with compound **410-1** to carry out the synthesis. The resulting crude product was purified by reverse-phase column chromatography [acetonitrile/water (0.05% formic acid) = 0% to 100%] to obtain compound **410** (32 mg, yield: 57%) as a white solid. MS (ESI, m/z): 520.3 $[M+H]^+$.

$^1H$ NMR (400 MHz, $CDCl_3$) δ 7.63 (d, *J* = 8.0 Hz, 1H), 7.32 (s, 1H), 7.27 - 7.20 (m, 1H), 7.11 (dd, *J* = 8.0, 1.2 Hz, 1H), 6.65 (d, *J* = 7.6 Hz, 1H), 6.57 (s, 1H), 6.47 (d, *J* = 7.6 Hz, 1H), 4.73 (q, *J* = 6.8 Hz, 1H), 3.91 - 3.81 (m, 5H), 3.40 - 3.30 (m, 2H), 3.09 - 3.01 (m, 1H), 2.94 - 2.81 (m, 4H), 2.55 - 2.45 (m, 1H), 2.29 - 2.15 (m, 2H), 1.97 - 1.81 (m, 2H), 1.62 - 1.51 (m, 5H), 1.44 - 1.29 (m, 2H), 1.16 - 1.05 (m, 1H), 0.89 (t, *J* = 7.2 Hz, 3H), 0.69 - 0.57 (m, 1H), 0.53 - 0.43 (m, 1H), 0.38 - 0.31 (m, 1H), 0.29 - 0.17 (m, 1H).

**Example 411:**

Synthetic Route:

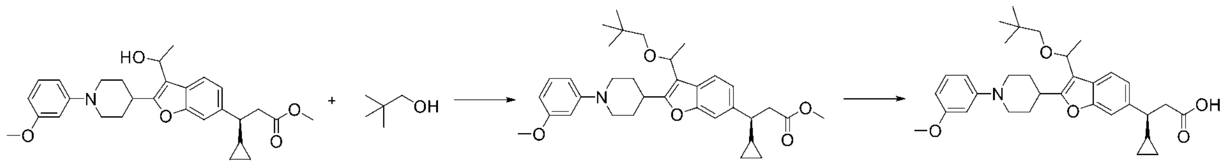

406-1 401-1 411-1 411

Referring to the synthetic route of compound **406,** compound **406-2** was replaced with compound **401-1** to carry out the synthesis. The resulting crude product was purified by reverse-phase column chromatography [acetonitrile/water (0.05% formic acid) = 0% to 100%] to obtain compound **411** (7 mg, yield: 28%) as a white solid. MS (ESI, m/z): 534.3 $[M+H]^+$.

$^1H$ NMR (400 MHz, $CDCl_3$) δ 7.61 (d, *J* = 8.0 Hz, 1H), 7.32 (s, 1H), 7.26 - 7.20 (m, 1H), 7.11 - 7.07 (m, 1H), 6.65 (d, *J* = 8.0 Hz, 1H), 6.61 - 6.54 (m, 1H), 6.47 (d, *J* = 8.0 Hz, 1H), 4.72 - 4.64 (m, 1H), 3.87 - 3.82 (m, 5H), 3.13 - 2.96 (m, 3H), 2.91 -

2.82 (m, 4H), 2.54 - 2.46 (m, 1H), 2.28 - 2.16 (m, 2H), 1.97 - 1.84 (m, 2H), 1.58 (d, *J*= 6.4 Hz, 3H), 1.15 - 1.07 (m, 1H), 0.92 (s, 9H), 0.69 - 0.59 (m, 1H), 0.52 - 0.42 (m, 1H), 0.41 - 0.31 (m, 1H), 0.27 - 0.18 (m, 1H).

**Example 412:**

Synthetic Route:

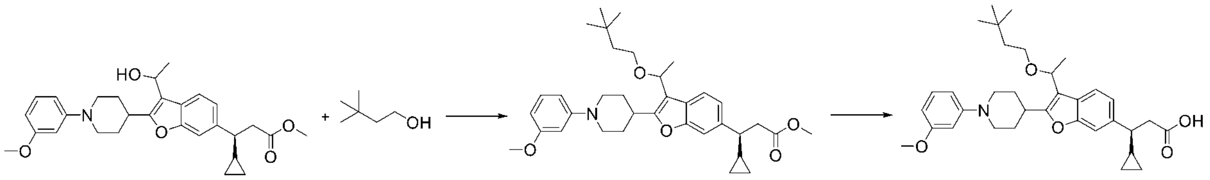

406-1 412-1 412-2 412

Referring to the synthetic route of compound **406,** compound **406-2** was replaced with compound **412-1** to carry out the synthesis. The resulting crude product was purified by reverse-phase column chromatography [acetonitrile/water (0.05% formic acid) = 0% to 100%] to obtain compound **412** (34 mg, yield: 65%) as a white solid. MS (ESI, m/z): 548.3 $[M+H]^+$.

$^1H$ NMR (400 MHz, $CDCl_3$) δ 7.62 (d, *J* = 8.0 Hz, 1H), 7.33 (s, 1H), 7.27-7.20 (m, 1H), 7.11 (dd, *J* = 8.0, 1.2 Hz, 1H), 6.66 (d, *J* = 7.6 Hz, 1H), 6.58 (s, 1H), 6.48 (d, *J* = 8.0 Hz, 1H), 4.74 (q, *J* = 6.4 Hz, 1H), 3.90 - 3.81 (m, 5H), 3.48 - 3.36 (m, 2H), 3.15 - 3.02 (m, 1H), 2.95 - 2.83 (m, 4H), 2.58 - 2.46 (m, 1H), 2.31 - 2.13 (m, 2H), 1.96 - 1.77 (m, 2H), 1.66 - 1.48 (m, 5H), 1.16 - 1.06 (m, 1H), 0.89 (s, 9H), 0.68 - 0.60 (m, 1H), 0.53 - 0.42 (m, 1H), 0.41 - 0.34 (m, 1H), 0.27 - 0.18 (m, 1H).

**Example 413:**

Synthetic Route:

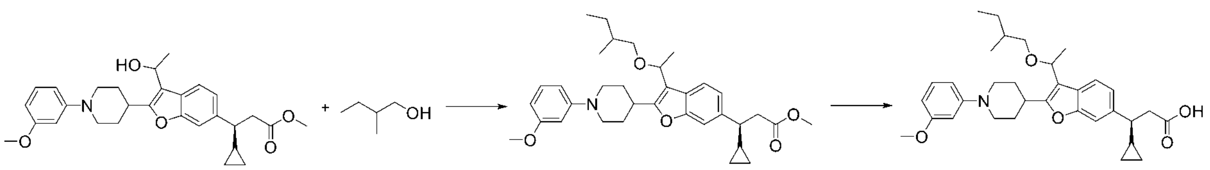

406-1 413-1 413-2 413

Referring to the synthetic route of compound **406,** compound **406-2** was replaced with compound **413-1** to carry out the synthesis. The resulting crude product was purified by reverse-phase column chromatography [acetonitrile/water (0.05% formic acid) = 0% to 100%] to obtain compound **413** (28 mg, yield: 66%) as a white solid. MS (ESI, m/z): 534.3 $[M+H]^+$.

$^1H$ NMR (400 MHz, $CDCl_3$) δ 7.66 - 7.60 (m, 1H), 7.33 (s, 1H), 7.27 - 7.20 (m, 1H), 7.11 (d, *J* = 8.0 Hz, 1H), 6.65 (d, *J* = 7.6 Hz, 1H), 6.57 (s, 1H), 6.47 (d, *J* = 7.6 Hz, 1H), 4.71 (q, *J* = 6.4 Hz, 1H), 3.92 - 3.80 (m, 5H), 3.29 - 3.19 (m, 1H), 3.17 - 3.11 (m, 1H), 3.09 - 3.00 (m, 1H), 2.95 - 2.79 (m, 4H), 2.58 - 2.46 (m, 1H), 2.30 - 2.15 (m, 2H), 1.99 - 1.83 (m, 2H), 1.70 - 1.63 (m, 1H), 1.60 (d, *J* = 6.4 Hz, 3H), 1.55 - 1.44 (m, 1H), 1.18 - 1.06 (m, 2H), 0.94 - 0.82 (m, 6H), 0.68 - 0.59 (m, 1H), 0.54 - 0.41 (m, 1H), 0.40 - 0.32 (m, 1H), 0.29 - 0.17 (m, 1H).

**Example 414:**

Synthetic Route:

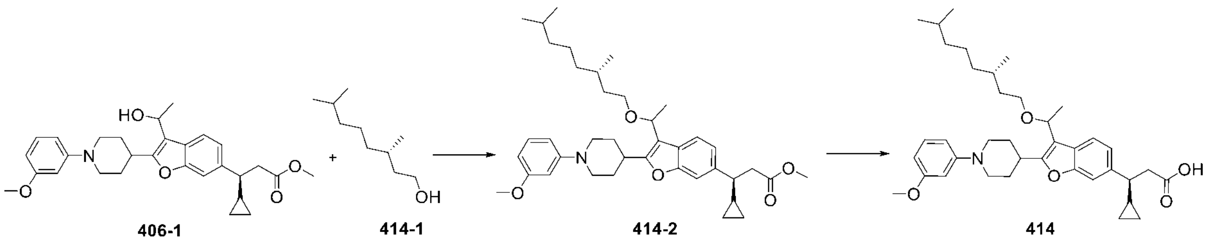

406-1 414-1 414-2 414

Referring to the synthetic route of compound **406,** compound **406-2** was replaced with compound **414-1** to carry out the synthesis. The resulting crude product was purified by reverse-phase column chromatography [acetonitrile/water (0.05% formic acid) = 0% to 100%] to obtain compound **414** (18 mg, yield: 50%) as a white solid. MS (ESI, m/z): 604.3 $[M+H]^+$.

$^1$H NMR (400 MHz, $CDCl_3$) δ 7.63 (dd, *J* = 8.0, 1.6 Hz, 1H), 7.33 (s, 1H), 7.27 - 7.19 (m, 1H), 7.11 (d, *J* = 8.0 Hz, 1H), 6.65 (d, *J* = 8.0 Hz, 1H), 6.57 (s, 1H), 6.48 (d, *J* = 7.6 Hz, 1H), 4.73 (q, *J* = 6.4 Hz, 1H), 3.91 - 3.81 (m, 5H), 3.48 - 3.32 (m, 2H), 3.11 - 3.01 (m, 1H), 2.94 - 2.81 (m, 4H), 2.57 - 2.47 (m, 1H), 2.31 - 2.12 (m, 2H), 1.98 - 1.80 (m, 2H), 1.65 - 1.58 (m, 4H), 1.58 - 1.50 (m, 2H), 1.43 - 1.35 (m, 1H), 1.31 - 1.21 (m, 3H), 1.17 - 1.03 (m, 4H), 0.90 - 0.77 (m, 9H), 0.70 - 0.60 (m, 1H), 0.53 - 0.42 (m, 1H), 0.41 - 0.32 (m, 1H), 0.27 - 0.19 (m, 1H).

**Example 415:**

Synthetic Route:

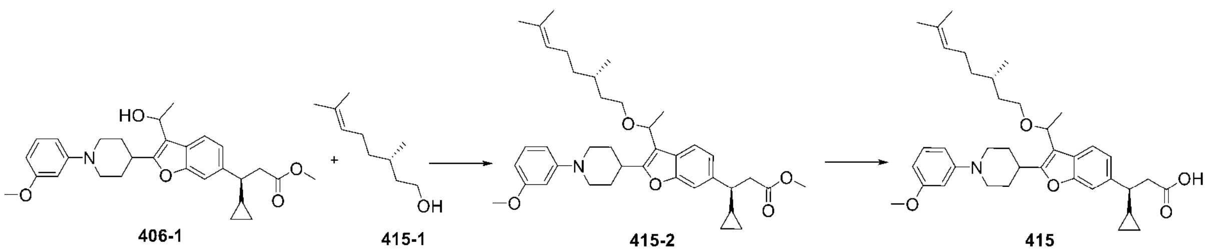

406-1 415-1 415-2 415

Referring to the synthetic route of compound **406,** compound **406-2** was replaced with compound **415-1** to carry out the synthesis. The resulting crude product was purified by reverse-phase column chromatography [acetonitrile/water (0.05% formic acid) = 0% to 100%] to obtain compound **415** (28 mg, yield: 54%) as a white solid. MS (ESI, m/z): 602.3 $[M+H]^+$.

$^1$H NMR (400 MHz, $CDCl_3$) δ 7.64 (d, *J* = 8.0 Hz, 1H), 7.33 (s, 1H), 7.27 - 7.20 (m, 1H), 7.11 (d, *J* = 8.0 Hz, 1H), 6.66 (d, *J* = 8.0 Hz, 1H), 6.58 (s, 1H), 6.48 (d, *J* = 8.0 Hz, 1H), 5.17 - 5.04 (m, 1H), 4.78 - 4.69 (m, 1H), 3.91 - 3.82 (m, 5H), 3.47 - 3.33 (m, 2H), 3.11 - 3.01 (m, 1H), 2.98 - 2.82 (m, 4H), 2.56 - 2.48 (m, 1H), 2.34 - 2.14 (m, 2H), 2.07 - 1.82 (m, 4H), 1.71 (d, *J* = 5.6 Hz, 3H), 1.65 - 1.59 (m, 7H), 1.58 - 1.50 (m, 1H), 1.47 - 1.36 (m, 1H), 1.35 - 1.25 (m, 1H), 1.18 - 1.06 (m, 2H), 0.91 - 0.77 (m, 3H), 0.70 - 0.57 (m, 1H), 0.53 - 0.43 (m, 1H), 0.40 - 0.33 (m, 1H), 0.27 - 0.18 (m, 1H).

**Example 416:**

Synthetic Route:

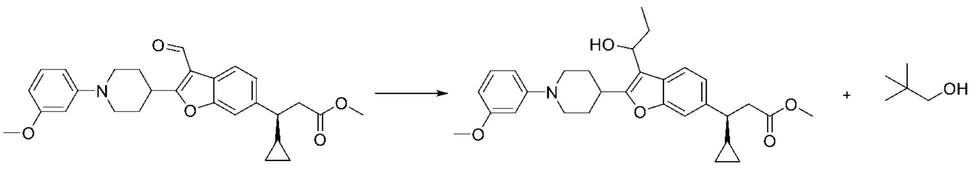

49-5 416-1 401-1

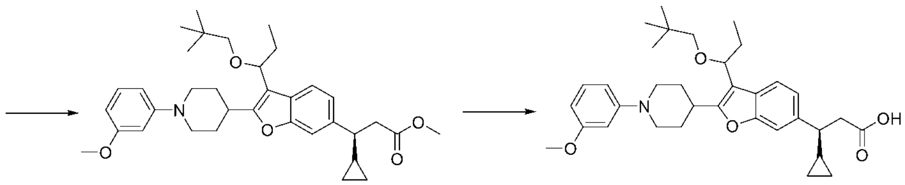

416-2 416

Referring to the synthetic route of compound **401,** methyl Grignard reagent was replaced with ethyl Grignard reagent to carry out the synthesis. The resulting crude product was purified by reverse-phase column chromatography [acetonitrile/water (0.05% formic acid) = 0% to 100%] to obtain compound **416** (31 mg, yield: 35%) as a white solid. MS (ESI, m/z): 548.3 $[M+H]^+$.

$^1H$ NMR (400 MHz, $CDCl_3$) δ 7.58 (d, *J* = 8.0 Hz, 1H), 7.31 (s, 1H), 7.26 - 7.19 (m, 1H), 7.07 (dd, *J* = 8.0, 1.6 Hz, 1H), 6.64 (dd, *J* = 8.0, 2.4 Hz, 1H), 6.59 - 6.55 (m, 1H), 6.47 (dd, *J* = 8.0, 2.4 Hz, 1H), 4.38 (t, *J* = 6.8 Hz, 1H), 3.84 (s, 5H), 3.07 - 3.01 (m, 2H), 2.95 - 2.82 (m, 5H), 2.54 - 2.46 (m, 1H), 2.27 - 2.18 (m, 2H), 2.09 - 2.02 (m, 1H), 1.95 - 1.76 (m, 3H), 1.13 - 1.09 (m, 1H), 0.99 (t, *J* = 7.6 Hz, 3H), 0.92 (s, 9H), 0.67 - 0.60 (m, 1H), 0.50 - 0.44 (m, 1H), 0.39 - 0.33 (m, 1H), 0.25 - 0.20 (m, 1H).

**Example 417:**

Synthetic Route:

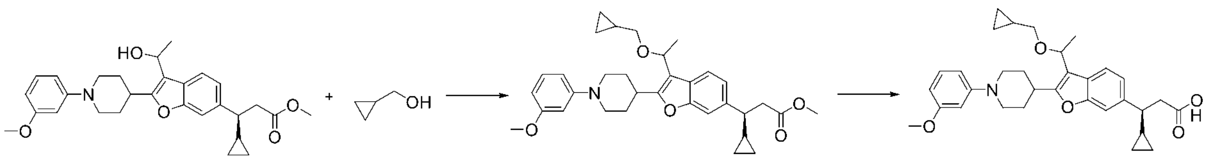

406-1 417-1 417-2 417

Referring to the synthetic route of compound **406,** compound **406-2** was replaced with compound **417-1** to carry out the synthesis. The resulting crude product was purified by reverse-phase column chromatography [acetonitrile/water (0.05% formic acid) = 0% to 100%] to obtain compound **417** (20 mg, yield: 48%) as a white solid. MS (ESI, m/z): 518.3 $[M+H]^+$.

$^1H$ NMR (400 MHz, $CDCl_3$) δ 7.51 (d, *J* = 8.0 Hz, 1H), 7.16 (s, 1H), 7.10 - 7.03 (m, 1H), 6.95 (dd, *J* = 8.0, 1.2 Hz, 1H), 6.49 (d, *J* = 8.0 Hz, 1H), 6.41 (s, 1H), 6.32 (d, *J* = 7.6 Hz, 1H), 4.63 (q, *J* = 6.4 Hz, 1H), 3.76 - 3.62 (m, 5H), 3.14 - 2.96 (m, 2H), 2.93 - 2.84 (m, 1H), 2.77 - 2.68 (m, 4H), 2.39 - 2.27 (m, 1H), 2.14 - 1.97 (m, 2H), 1.80 - 1.65 (m, 2H), 1.47 (d, *J* = 6.4 Hz, 3H), 1.01 - 0.87 (m, 2H), 0.52 - 0.44 (m, 1H), 0.39 - 0.26 (m, 3H), 0.24 - 0.15 (m, 1H), 0.12 - 0.05 (m, 3H).

**Example 418:**

Synthetic Route:

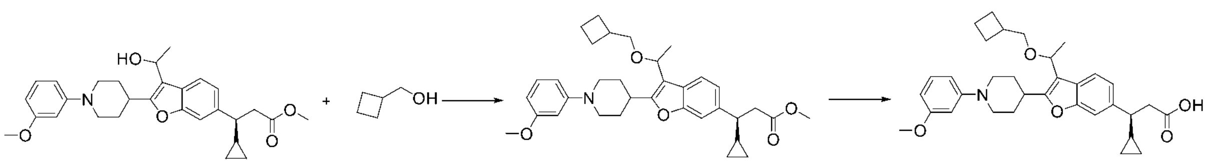

406-1 418-1 418-2 418

Referring to the synthetic route of compound **406,** compound **406-2** was replaced with compound **418-1** to carry out the synthesis. The resulting crude product was purified by reverse-phase column chromatography [acetonitrile/water (0.05% formic acid) = 0% to 100%] to obtain compound **418** (19 mg, yield: 51%) as a white solid. MS (ESI, m/z): 532.4 $[M+H]^+$.

$^1$H NMR (400 MHz, $CDCl_3$) δ 7.62 - 7.55 (m, 1H), 7.29 (s, 1H), 7.22 - 7.16 (m, 1H), 7.09 - 7.04 (m, 1H), 6.61 (d, *J* = 8.0 Hz, 1H), 6.53 (s, 1H), 6.43 (d, *J* = 6.4 Hz, 1H), 4.69 (q, *J* = 6.4 Hz, 1H), 3.87 - 3.76 (m, 5H), 3.36 - 3.24 (m, 2H), 3.06 - 2.95 (m, 1H), 2.90 - 2.77 (m, 4H), 2.59 - 2.43 (m, 2H), 2.26 - 2.11 (m, 2H), 2.07 - 1.95 (m, 2H), 1.94 - 1.77 (m, 4H), 1.72 - 1.61 (m, 2H), 1.56 (d, *J* = 6.4 Hz, 3H), 1.14 - 1.02 (m, 1H), 0.65 - 0.56 (m, 1H), 0.47 - 0.39 (m, 1H), 0.36 - 0.28 (m, 1H), 0.23 - 0.14 (m, 1H).

**Example 419:**

Synthetic Route:

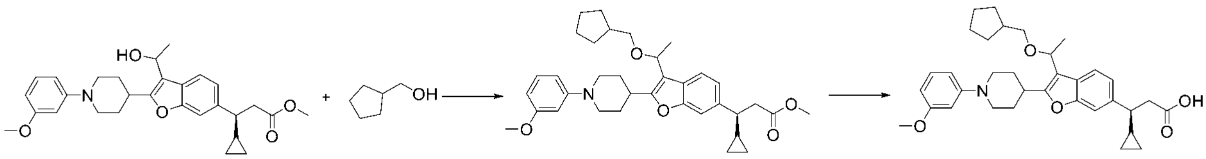

406-1 419-1 419-2 419

Referring to the synthetic route of compound **406,** compound **406-2** was replaced with compound **419-1** to carry out the synthesis. The resulting crude product was purified by reverse-phase column chromatography [acetonitrile/water (0.05% formic acid) = 0% to 100%] to obtain compound **419** (19 mg, yield: 32%) as a white solid. MS (ESI, m/z): 546.3 $[M+H]^+$.

$^1$H NMR (400 MHz, $CDCl_3$) δ 7.60 (d, *J* = 8.0 Hz, 1H), 7.29 (s, 1H), 7.23 - 7.15 (m, 1H), 7.07 (d, *J* = 8.0 Hz, 1H), 6.61 (d, *J* = 8.0 Hz, 1H), 6.54 (s, 1H), 6.44 (d, *J* = 8.0 Hz, 1H), 4.71 - 4.62 (m, 1H), 3.85 - 3.72 (m, 6H), 3.20 (d, *J* = 6.8 Hz, 2H), 3.05 - 2.92 (m, 1H), 2.90 - 2.77 (m, 4H), 2.51 - 2.38 (m, 1H), 2.24 - 2.04 (m, 3H), 1.90 - 1.78 (m, 2H), 1.73 - 1.65 (m, 2H), 1.57 - 1.45 (m, 8H), 1.20 - 1.11 (m, 1H), 0.62 - 0.53 (m, 1H), 0.45 - 0.41 (m, 1H), 0.34 - 0.30 (m, 1H), 0.23 - 0.12 (m, 1H).

**Example 420:**

Synthetic Route:

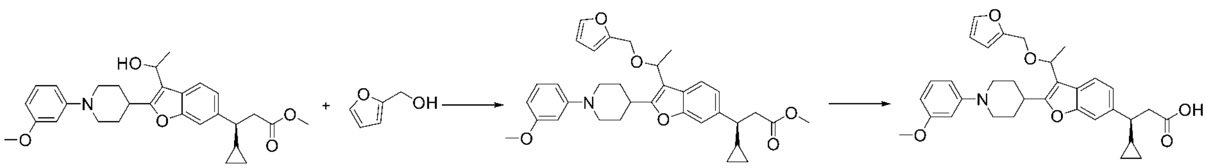

406-1 420-1 420-2 420

Referring to the synthetic route of compound **406,** compound **406-2** was replaced with compound **420-1** to carry out the synthesis. The resulting crude product was purified by reverse-phase column chromatography [acetonitrile/water (0.05% formic acid) = 0% to 100%] to obtain compound **420** (16 mg, yield: 27%) as a white solid. MS (ESI, m/z): 544.3 $[M+H]^+$.

$^1$H NMR (400 MHz, $CDCl_3$) δ 7.63 (d, *J* = 8.0 Hz, 1H), 7.42 - 7.36 (m, 1H), 7.31 (s, 1H), 7.24 - 7.16 (m, 1H), 7.13 - 7.06 (m, 1H), 6.61 (d, *J* = 8.0 Hz, 1H), 6.57 - 6.51 (m, 1H), 6.47 - 6.41 (m, 1H), 6.35 - 6.30 (m, 1H), 6.25 - 6.20 (m, 1H), 4.81 (q, *J* = 6.4 Hz, 1H), 4.43 (d, *J* = 12.8 Hz, 1H), 4.27 (d, *J* = 12.8 Hz, 1H), 3.84 - 3.78 (m, 5H), 3.04 - 2.89 (m, 1H), 2.89 - 2.80 (m, 4H), 2.53 - 2.43 (m, 1H), 2.27 -2.14 (m, 2H), 1.97 - 1.77 (m, 1H), 1.59 (d, *J*= 6.4 Hz, 3H), 1.10 - 1.05 (m, 1H), 0.64 - 0.59 (m, 1H), 0.47 - 0.41 (m, 1H), 0.35 - 0.31 (m, 1H), 0.22 - 0.17 (m, 1H).

**Example 421:**

Synthetic Route:

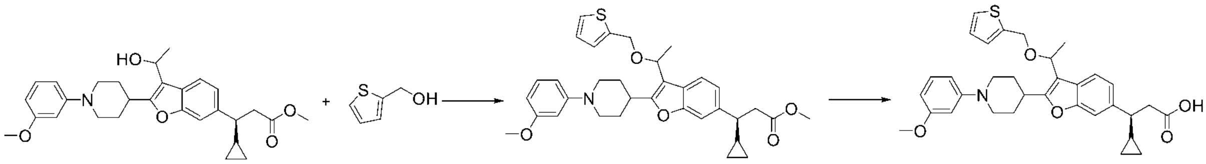

406-1 421-1 421-2 421

Referring to the synthetic route of compound **406,** compound **406-2** was replaced with compound **421-1** to carry out the synthesis. The resulting crude product was purified by reverse-phase column chromatography [acetonitrile/water (0.05% formic acid) = 0% to 100%] to obtain compound **421** (18 mg, yield: 42%) as a white solid. MS (ESI, m/z): 560.3 $[M+H]^+$.

$^1$H NMR (400 MHz, $CDCl_3$) δ 7.65 (d, *J* = 8.0 Hz, 1H), 7.31 (s, 1H), 7.29 - 7.25 (m, 1H), 7.23 - 7.17 (m, 1H), 7.12 - 7.07 (m, 1H), 6.98 - 6.89 (m, 2H), 6.60 (dd, *J* = 8.0, 4.4 Hz, 1H), 6.54 - 6.50 (m, 1H), 6.43 (dd, *J* = 8.0, 4.4 Hz, 1H), 4.88 - 4.78 (m, 1H), 4.65 (d, *J* = 12.8, 1H), 4.48 (d, *J* = 12.8 Hz, 1H), 3.86 - 3.72 (m, 5H), 2.96 - 2.75 (m, 5H), 2.54 - 2.42 (m, 1H), 2.29 - 2.07 (m, 2H), 1.97 - 1.72 (m, 1H), 1.60 (d, *J* = 6.4 Hz, 3H), 1.14 - 1.02 (m, 1H), 0.66 - 0.56 (m, 1H), 0.49 - 0.40 (m, 1H), 0.38 - 0.28 (m, 1H), 0.24 - 0.14 (m, 1H).

**Example 422:**

Synthetic Route:

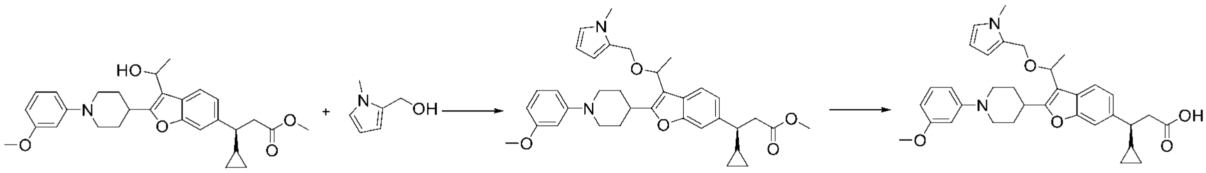

406-1 422-1 422-2 422

Referring to the synthetic route of compound **406,** compound **406-2** was replaced with compound **422-1** to carry out the synthesis. The resulting crude product was purified by reverse-phase column chromatography [acetonitrile/water (0.05% formic acid) = 0% to 100%] to obtain compound **422** (16 mg, yield: 27%) as a white solid. MS (ESI, m/z): 571.3 $[M+H]^+$.

$^1$H NMR (400 MHz, $CDCl_3$) δ 7.83 (d, *J* = 8.0 Hz, 1H), 7.38 (s, 1H), 7.27 (d, *J* = 5.6 Hz, 1H), 7.19 (t, *J* = 8.0 Hz, 1H), 7.02 (d, *J* = 6.8 Hz, 1H), 6.88 - 6.84 (m, 1H), 6.76 - 6.64 (m, 1H), 6.56 (dd, *J* = 8.0, 3.2 Hz, 1H), 6.43 (t, *J* = 8.0 Hz, 1H), 5.73 (d, *J* = 6.8 Hz, 1H), 4.73 - 4.64 (m, 1H), 3.81 (s, 3H), 3.59 (s, 3H), 2.91 - 2.75 (m, 5H), 2.56 - 2.41 (m, 1H), 2.32 - 2.17 (m, 2H), 1.93 (d, *J* = 12.8 Hz, 1H), 1.75 (d, *J* = 12.8 Hz, 1H), 1.62 (d, *J* = 6.4 Hz, 4H), 1.49 (s, 3H), 1.12 - 1.02 (m, 1H), 0.66 - 0.57 (m, 1H), 0.48 - 0.40 (m, 1H), 0.38 - 0.29 (m, 1H), 0.26 - 0.14 (m, 1H).

**Example 423:**

Synthetic Route:

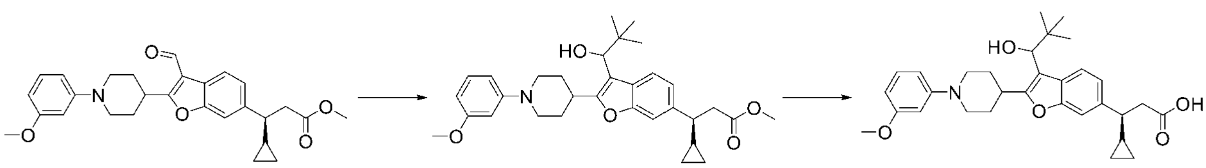

49-5 423-1 423

Referring to the synthetic route of compound **401,** methyl Grignard reagent was replaced with *tert*-butyl Grignard

reagent to obtain compound **423-1** (31 mg, yield: 35%) as a white solid. MS (ESI, m/z): 520.3 [M+H]$^+$.

Then, referring to the synthetic route of compound **401,** compound **401-2** was replaced with compound **423-1** to carry out the synthesis. The resulting crude product was purified by reverse-phase column chromatography [acetonitrile/-water (0.05% formic acid) = 0% to 100%] to obtain compound **423** (30 mg, yield: 48%) as a white solid. MS (ESI, m/z): 506.3 [M+H]$^+$.

$^1$H NMR (400 MHz, $CDCl_3$) δ 7.63 (d, *J* = 8.0 Hz, 1H), 7.31 (s, 1H), 7.25 - 7.19 (m, 1H), 7.09 (d, *J* = 8.0 Hz, 1H), 6.67 - 6.61 (m, 1H), 6.59 - 6.55 (m, 1H), 6.49 - 6.44 (m, 1H), 4.71 (s, 1H), 3.89 - 3.78 (m, 5H), 3.08 - 2.98 (m, 1H), 2.92 - 2.78 (m, 4H), 2.54 - 2.44 (m, 1H), 2.31 - 2.15 (m, 2H), 1.91 - 1.82 (m, 2H), 1.15 - 1.04 (m, 10H), 0.68 - 0.59 (m, 1H), 0.51 - 0.41 (m, 1H), 0.39 - 0.31 (m, 1H), 0.25 - 0.17 (m, 1H).

**Example 424:**

Synthetic Route:

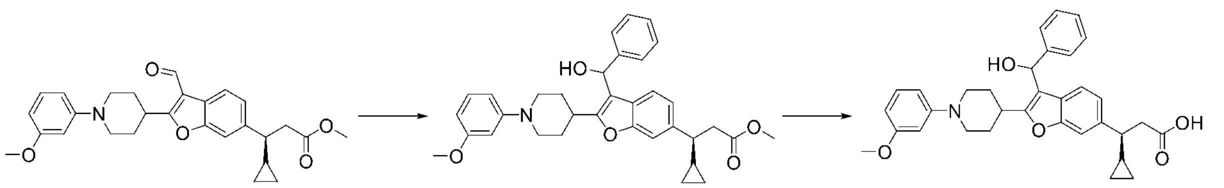

49-5 424-1 424

Referring to the synthetic route of compound **423,** methyl Grignard reagent was replaced with phenyl Grignard reagent to carry out the synthesis. The resulting crude product was purified by reverse-phase column chromatography [acetonitrile/water (0.05% formic acid) = 0% to 100%] to obtain compound **424** (14 mg, yield: 35%) as a white solid. MS (ESI, m/z): 526.3 [M+H]$^+$.

$^1$H NMR (400 MHz, $CDCl_3$) δ 7.54 - 7.49 (m, 2H), 7.42 - 7.35 (m, 2H), 7.34 - 7.31 (m, 3H), 7.25 - 7.19 (m, 1H), 7.05 - 6.99 (m, 1H), 6.66 - 6.61 (m, 1H), 6.57 - 6.54 (m, 1H), 6.49 - 6.45 (m, 1H), 6.18 (s, 1H), 3.87 - 3.77 (m, 5H), 3.09 - 2.98 (m, 1H), 2.90 - 2.74 (m, 4H), 2.50 - 2.42 (m, 1H), 2.27 - 2.16 (m, 2H), 1.94 - 1.83 (m, 2H), 1.13 - 1.02 (m, 1H), 0.68 - 0.56 (m, 1H), 0.49 - 0.39 (m, 1H), 0.37 - 0.28 (m, 1H), 0.22 - 0.13 (m, 1H).

**Example 425:**

Synthetic Route:

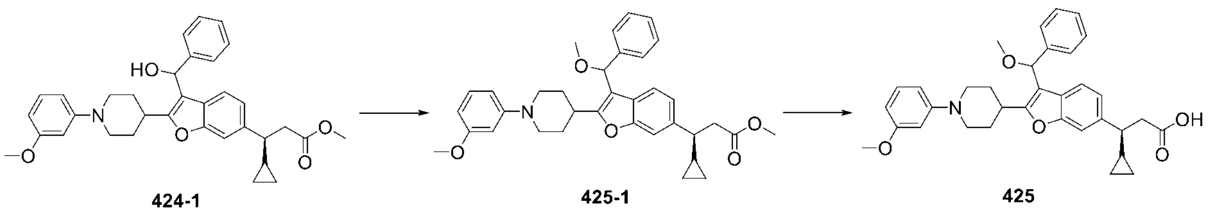

424-1 425-1 425

Compound **424-1** (54 mg, 0.1 mmol) was dissolved in *N*,*N*-dimethylformamide (5 mL). Sodium hydride (6 mg, 0.15 mmol) was added at 0°C, and the reaction was carried out at 25°C for 1 hour. Iodomethane (43 mg, 0.30 mmol) was then added, followed by quenching with saturated sodium bicarbonate solution (1 mL). The reaction mixture was concentrated and purified by normal-phase column chromatography (ethyl acetate: petroleum ether = 0% to 100%) to obtain compound **425-1** (32 mg, yield: 61%) as a yellow oil. MS (ESI, m/z): 554.3 [M+H]$^+$.

Compound **425-1** (32 mg, 0.06 mmol) was added to methanol (1 mL), water (1 mL), and tetrahydrofuran (1 mL) and stirred. Lithium hydroxide (4 mg, 0.14 mmol) was added, and the reaction mixture was stirred at 25°C for 4 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound **425** (3 mg, yield: 23%) as a white solid. MS (ESI, m/z): 540.2 [M+H]$^+$.

$^1$H NMR (400 MHz, $CDCl_3$) δ 7.49 - 7.41 (m, 3H), 7.36 (t, *J* = 7.6 Hz, 2H), 7.32 -7.26 (m, 2H), 7.25 - 7.18 (m, 1H),

7.04 (d, $J$ = 8.4 Hz, 1H), 6.65 - 6.59 (m, 1H), 6.55 - 6.52 (m, 1H), 6.48 - 6.43 (m, 1H), 5.58 (s, 1H), 3.88 - 3.78 (m, 5H), 3.46 (s, 3H), 3.08 - 2.96 (m, 1H), 2.91 - 2.77 (m, 4H), 2.53 - 2.44 (m, 1H), 2.26 - 2.16 (m, 2H), 1.91-1.81 (m, 2H), 1.14 - 1.03 (m, 1H), 0.67 - 0.59 (m, 1H), 0.50 - 0.41 (m, 1H), 0.39 - 0.31 (m, 1H), 0.25 - 0.16 (m, 1H).

**Example 426:**

Synthetic Route:

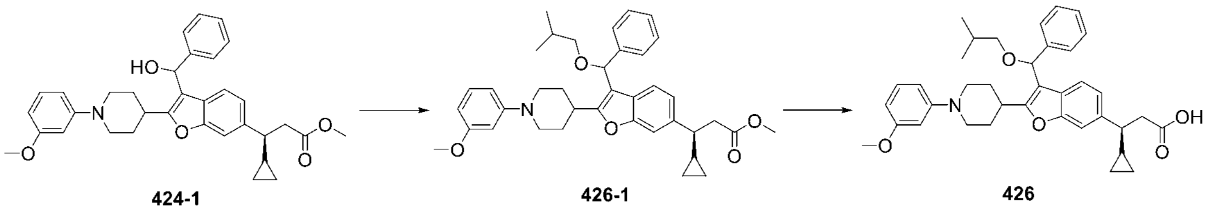

424-1 426-1 426

Referring to the synthetic route of compound **425,** iodomethane was replaced with isobutyl iodide to carry out the synthesis. The resulting crude product was purified by reverse-phase column chromatography [acetonitrile/water (0.05% formic acid) = 0% to 100%] to obtain compound **426** (12 mg, yield: 24%) as a white solid. MS (ESI, m/z): 582.3 [M+H]$^+$.

$^1$H NMR (400 MHz, $CDCl_3$) δ 7.47 (d, $J$ = 7.6 Hz, 2H), 7.42 (d, $J$ = 8.0 Hz, 1H), 7.38 - 7.22 (m, 5H), 7.03 (d, $J$ = 8.0 Hz, 1H), 6.67 - 6.59 (m, 1H), 6.59 - 6.53 (m, 1H), 6.50 - 6.42 (m, 1H), 5.65 (s, 1H), 3.85 - 3.80 (m, 5H), 3.34 - 3.25 (m, 2H), 3.13 - 3.02 (m, 1H), 2.90 - 2.76 (m, 4H), 2.53 - 2.43 (m, 1H), 2.28 - 2.14 (m, 2H), 2.02 - 1.95 (m, 1H), 1.88 - 1.82 (m, 2H), 1.13 - 0.94 (m, 7H), 0.67 - 0.57 (m, 1H), 0.50 - 0.40 (m, 1H), 0.39 - 0.30 (m, 1H), 0.26 - 0.15 (m, 1H).

**Example 427:**

Synthetic Route:

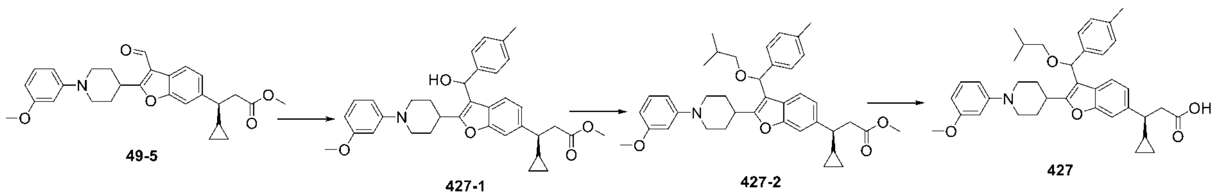

49-5 427-1 427-2 427

Referring to the synthetic route of compound **426,** phenyl Grignard reagent was replaced with 4-methyl-phenyl Grignard reagent to carry out the synthesis. The resulting crude product was purified by reverse-phase column chromatography [acetonitrile/water (0.05% formic acid) = 0% to 100%] to obtain compound **427** (27 mg, yield: 34%) as a white solid. MS (ESI, m/z): 596.3 [M+H]$^+$.

$^1$H NMR (400 MHz, $CDCl_3$) δ 7.44 (d, $J$ = 8.0 Hz, 1H), 7.37 (d, $J$ = 8.0 Hz, 2H), 7.31 - 7.21 (m, 2H), 7.17 (d, $J$ = 8.0 Hz, 2H), 7.03 (d, $J$ = 8.0 Hz, 1H), 6.68 - 6.42 (m, 3H), 5.63 (s, 1H), 3.85 - 3.81 (m, 5H), 3.35 - 3.22 (m, 2H), 3.13 - 3.07 (m, 1H), 2.91 - 2.75 (m, 4H), 2.51 - 2.45 (m, 1H), 2.37 (s, 3H), 2.29 - 2.15 (m, 2H), 2.02 - 1.96 (m, 1H), 1.93-1.81 (m, 2H), 1.15 - 0.93 (m, 7H), 0.65 - 0.58 (m, 1H), 0.48 - 0.41 (m, 1H), 0.37 - 0.31 (m, 1H), 0.22 - 0.16 (m, 1H).

**Example 428:**

Synthetic Route:

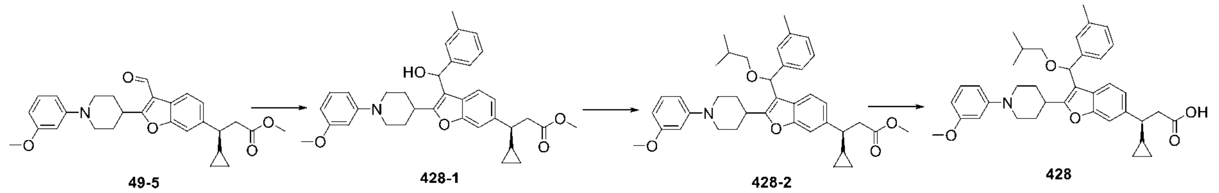

49-5 428-1 428-2 428

Referring to the synthetic route of compound **426,** phenyl Grignard reagent was replaced with 3-methyl-phenyl Grignard reagent to carry out the synthesis. The resulting crude product was purified by reverse-phase column chromatography [acetonitrile/water (0.05% formic acid) = 0% to 100%] to obtain compound 428 (31 mg, yield: 45%) as a white solid. MS (ESI, m/z): 596.3 $[M+H]^+$.

$^1$H NMR (400 MHz, $CDCl_3$) δ 7.44 (d, *J* = 8.0 Hz, 1H), 7.35 - 7.15 (m, 5H), 7.10 (d, *J* = 7.2 Hz, 1H), 7.04 (d, *J* = 8.0 Hz, 1H), 6.67-6.42 (m, 3H), 5.62 (s, 1H), 3.84 - 3.81 (m, 5H), 3.34 - 3.16 (m, 2H), 3.09 - 3.03 (m, 1H), 2.91 - 2.72 (m, 4H), 2.53 - 2.42 (m, 1H), 2.37 (s, 3H), 2.30 - 2.14 (m, 2H), 2.04 - 1.94 (m, 1H), 1.88 - 1.82 (m, 2H), 1.11 - 0.94 (m, 7H), 0.67 - 0.57 (m, 1H), 0.48 - 0.41 (m, 1H), 0.37 - 0.31 (m, 1H), 0.22 - 0.16 (m, 1H).

**Example 429:**

Synthetic Route:

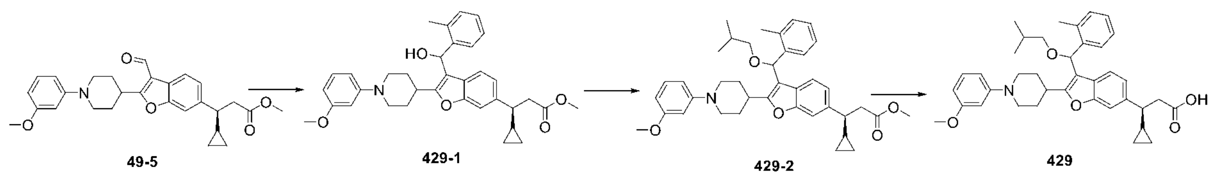

49-5 429-1 429-2 429

Referring to the synthetic route of compound **426,** phenyl Grignard reagent was replaced with 2-methyl-phenyl Grignard reagent to carry out the synthesis. The resulting crude product was purified by reverse-phase column chromatography [acetonitrile/water (0.05% formic acid) = 0% to 100%] to obtain compound **429** (37 mg, yield: 48%) as a white solid. MS (ESI, m/z): 596.3 $[M+H]^+$.

$^1$H NMR (400 MHz, $CDCl_3$) δ 7.42 - 7.27 (m, 1H), 7.13 - 6.94 (m, 5H), 6.86 - 6.69 (m, 1H), 6.60 - 6.31 (m, 3H), 6.27 - 6.22 (m, 1H), 5.54 (s, 1H), 3.68 - 3.45 (m, 5H), 3.17 - 2.97 (m, 2H), 2.68 - 2.44 (m, 5H), 2.34 - 2.16 (m, 1H), 2.20 - 1.35 (m, 8H), 0.97 - 0.82 (m, 1H), 0.82 - 0.73 (m, 6H), 0.48 - 0.37 (m, 1H), 0.31 -0.24 (m, 1H), 0.17 - 0.09 (m, 1H), 0.06 - -0.02 (m, 1H).

**Example 430:**

Synthetic Route:

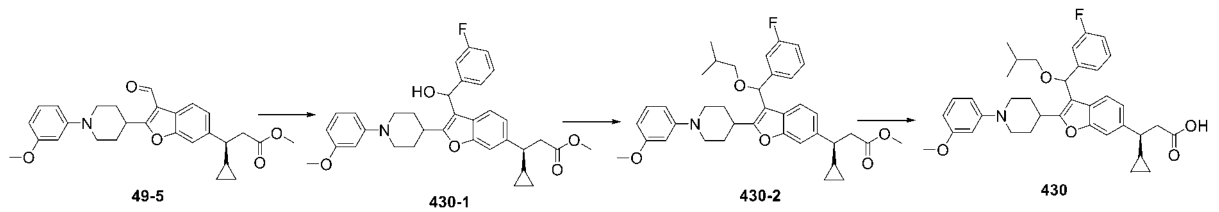

49-5 430-1 430-2 430

Referring to the synthetic route of compound **426,** phenyl Grignard reagent was replaced with 3-fluoro-phenyl Grignard reagent to carry out the synthesis. The resulting crude product was purified by reverse-phase column chromatography [acetonitrile/water (0.05% formic acid) = 0% to 100%] to obtain compound **430** (31 mg, yield: 52%) as a white solid. MS (ESI, m/z): 600.3 $[M+H]^+$.

$^1$H NMR (400 MHz, $CDCl_3$) δ 7.42 - 7.25 (m, 2H), 7.24 - 7.11 (m, 3H), 7.04 - 6.81 (m, 2H), 6.78 - 6.34 (m, 4H), 5.60 (s, 1H), 3.86 - 3.70 (m, 5H), 3.34 - 3.12 (m, 2H), 3.13 - 2.91 (m, 1H), 2.89 - 2.60 (m, 4H), 2.49 - 2.27 (m, 1H), 2.27 - 2.08 (m, 2H), 2.01 - 1.75 (m, 3H), 1.08 - 0.85 (m, 7H), 0.63 - 0.52 (m, 1H), 0.51 - 0.36 (m, 1H), 0.36 - 0.23 (m, 1H), 0.21 - 0.11 (m, 1H).

**Example 431:**

Synthetic Route:

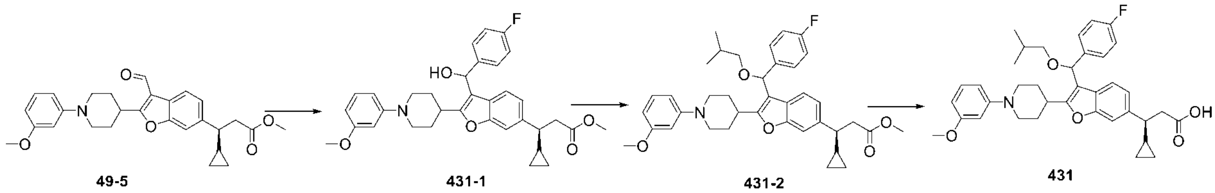

49-5 431-1 431-2 431

Referring to the synthetic route of compound **426,** phenyl Grignard reagent was replaced with 4-fluoro-phenyl Grignard reagent to carry out the synthesis. The resulting crude product was purified by reverse-phase column chromatography [acetonitrile/water (0.05% formic acid) = 0% to 100%] to obtain compound **431** (34 mg, yield: 61%) as a white solid. MS (ESI, m/z): 600.3 $[M+H]^+$.

$^1H$ NMR (400 MHz, $CDCl_3$) δ 7.46 - 7.38 (m, 2H), 7.36 - 6.95 (m, 5H), 6.80 - 6.36 (m, 4H), 5.59 (s, 1H), 3.87 - 3.74 (m, 5H), 3.33 - 3.12 (m, 2H), 3.12 - 2.91 (m, 1H), 2.93 - 2.29 (m, 5H), 2.29 - 2.07 (m, 2H), 1.98 - 1.79 (m, 3H), 1.10 - 0.89 (m, 7H), 0.65 - 0.51 (m, 1H), 0.48 - 0.38 (m, 1H), 0.37 - 0.23 (m, 1H), 0.20 -0.10 (m, 1H).

**Example 432:**

Synthetic Route:

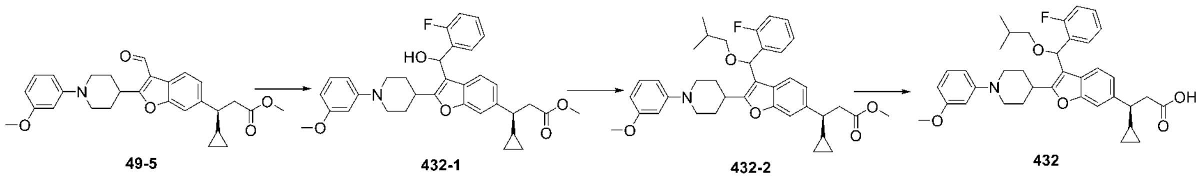

49-5 432-1 432-2 432

Referring to the synthetic route of compound **426,** phenyl Grignard reagent was replaced with 2-fluoro-phenyl Grignard reagent to carry out the synthesis. The resulting crude product was purified by reverse-phase column chromatography [acetonitrile/water (0.05% formic acid) = 0% to 100%] to obtain compound **432** (43 mg, yield: 51%) as a white solid. MS (ESI, m/z): 600.3 $[M+H]^+$.

$^1H$ NMR (400 MHz, $CDCl_3$) δ 7.78 - 7.28 (m, 2H), 7.25 - 6.92 (m, 5H), 6.78 - 5.86 (m, 5H), 3.89 - 3.56 (m, 6H), 3.29 - 3.13 (m, 2H), 2.92 - 2.59 (m, 4H), 2.38 - 2.25 (m, 1H), 1.97 - 1.79 (m, 3H), 1.78 - 1.50 (m, 2H), 1.03 - 0.88 (m, 7H), 0.64 - 0.53 (m, 1H), 0.48 - 0.37 (m, 1H), 0.34 - 0.25 (m, 1H), 0.20 - 0.10 (m, 1H).

**Example 433:**

Synthetic Route:

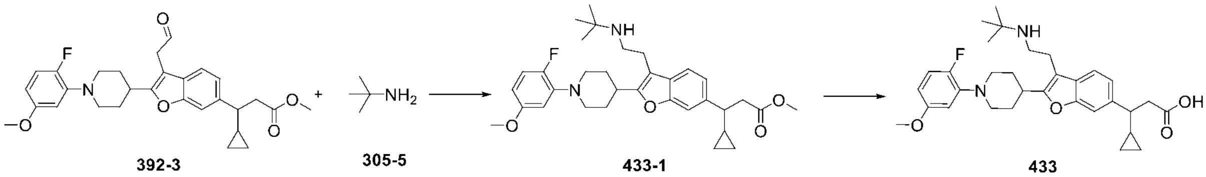

392-3 305-5 433-1 433

Referring to the synthetic route of compound **405,** compound **126-2** was replaced with compound **392-3,** and compound **200-1** was replaced with compound **305-5.** The synthesis was carried out, and the resulting crude product was purified by reverse-phase column chromatography [acetonitrile/water (0.05% formic acid) = 0% to 100%] to obtain

compound **433** (5 mg, yield: 17%) as a white solid. MS (ESI, m/z): 537.3 [M+H]+.

$^1$H NMR (400 MHz, MeOD) δ 7.39 (d, *J* = 8.0 Hz, 1H), 7.34 (s, 1H), 7.15 (dd, *J* = 8.0 Hz, 1.4, 1H), 6.95 (dd, *J* = 12.4, 8.8 Hz, 1H), 6.61 (dd, *J* = 7.2, 3.2 Hz, 1H), 6.52 - 6.46 (m, 1H), 3.76 (s, 3H), 3.58 - 3.51 (m, 2H), 3.08 - 2.98 (m, 1H), 2.87 - 2.78 (m, 6H), 2.70 - 2.54 (m, 2H), 2.52 - 2.44 (m, 1H), 2.24 - 2.14 (m, 2H), 1.90 - 1.84 (m, 2H), 1.10 (s, 9H), 1.07 - 1.02 (m, 1H), 0.60 - 0.51 (m, 1H), 0.40 - 0.33 (m, 2H), 0.14 - 0.08 (m, 1H).

**Example 434:**

Synthetic Route:

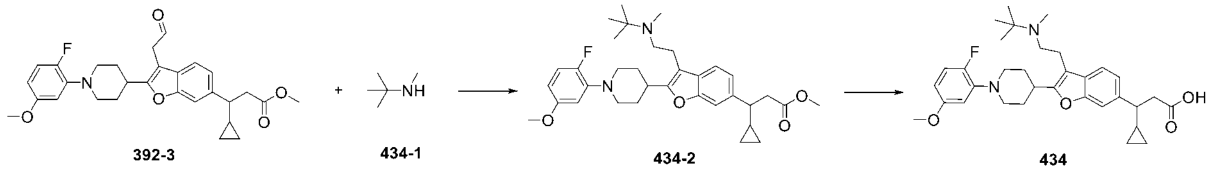

392-3 434-1 434-2 434

Referring to the synthetic route of compound **433,** compound **305-5** was replaced with compound **434-1** to carry out the synthesis. The resulting crude product was purified by reverse-phase column chromatography [acetonitrile/water (0.05% formic acid) = 0% to 100%] to obtain compound **434** (2 mg, yield: 14%) as a white solid. MS (ESI, m/z): 551.3 [M+H]+.

$^1$H NMR (400 MHz, DMSO-*d*$_6$) δ 8.38 (s, 1H), 7.41 - 7.36 (m, 2H), 7.11 (d, *J* = 7.6 Hz, 1H), 7.05 (dd, *J* = 12.4, 8.8 Hz, 1H), 6.60 - 6.55 (m, 1H), 6.52 - 6.46 (m, 1H), 3.73 (s, 3H), 3.52 - 3.49 (m, 2H), 3.08 - 2.99 (m, 1H), 2.86 - 2.75 (m, 2H), 2.72 - 2.66 (m, 4H), 2.55 - 2.48 (m, 2H), 2.37 - 2.32 (m, 1H), 2.26 (s, 3H), 2.07 - 1.98 (m, 2H), 1.89 - 1.82 (m, 2H), 1.09 - 1.02 (m, 1H), 0.95 (s, 9H), 0.55 - 0.45 (m, 1H), 0.32 - 0.21 (m, 2H), 0.17 - 0.11 (m, 1H).

**Example 435:**

Synthetic Route:

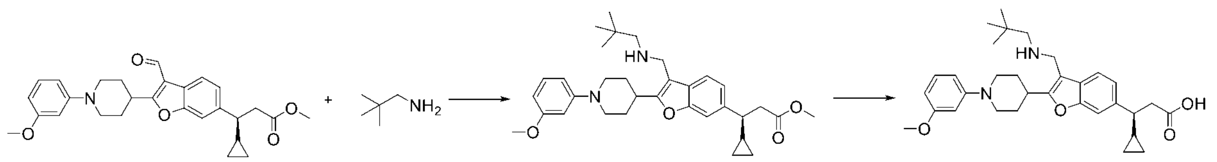

49-5 435-1 435-2 435

Referring to the synthetic route of compound **405,** compound **126-2** was replaced with compound **49-5,** and compound **200-1** was replaced with compound **435-1.** The synthesis was carried out, and the resulting crude product was purified by reverse-phase column chromatography [acetonitrile/water (0.05% formic acid) = 0% to 100%] to obtain compound **435** (38 mg, yield: 53%) as a white solid. MS (ESI, m/z): 519.3 [M+H]+.

$^1$H NMR (400 MHz, $CDCl_3$) δ 7.39 (d, *J* = 7.6 Hz, 1H), 7.32 (s, 1H), 7.22 - 7.15 (m, 2H), 6.60-6.55 (m, 1H), 6.53-6.49 (m, 1H), 6.45-6.39 (m, 1H), 3.85 - 3.72 (m, 5H), 3.55 (s, 2H), 2.86 - 2.73 (m, 6H), 2.54 - 2.45 (m, 1H), 2.36 (s, 2H), 2.15 - 2.01 (m, 2H), 1.76 - 1.66 (m, 2H), 1.09 - 1.00 (m, 1H), 0.82 (s, 9H), 0.62 - 0.55 (m, 1H), 0.45 - 0.37 (m, 1H), 0.36 - 0.27 (m, 1H), 0.25 - 0.17 (m, 1H).

**Example 436:**

Synthetic Route:

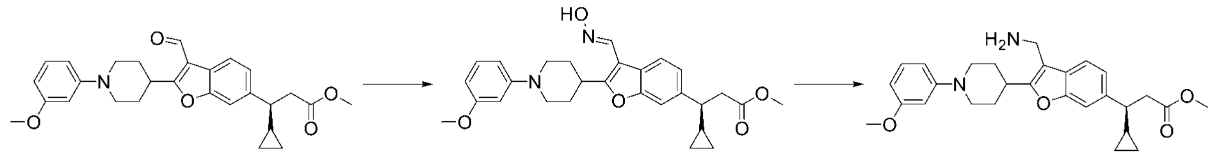

49-5 436-1 436-2

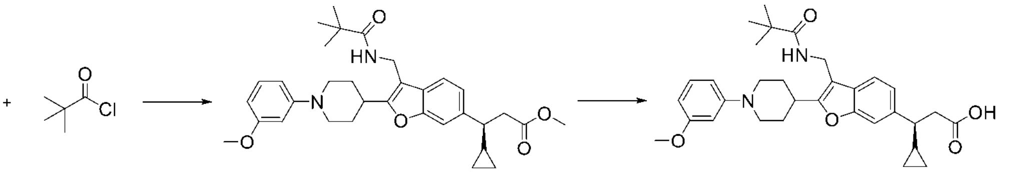

436-3 436-4 436

Compound **49-5** (330 mg, 0.72 mmol) was dissolved in ethanol (5 mL), and 50% hydroxylamine hydrochloride aqueous solution (2 mL) was added. The reaction was carried out at 80°C for 2 hours. After concentration, the resulting crude product was purified by normal-phase column chromatography (ethyl acetate: petroleum ether = 0% to 100%) to obtain compound **436-1** (330 mg, yield: 97%) as a white solid. MS (ESI, m/z): 477.3 $[M+H]^+$.

Compound **436-1** (330 mg, 0.69 mmol) and 5% Pd/C (100 mg) were added to concentrated hydrochloric acid (2 mL) and methanol (15 mL), and the reaction was stirred under hydrogen at 60°C overnight. After concentration, the resulting crude product was purified by normal-phase column chromatography (ethyl acetate: petroleum ether = 0% to 100%) to obtain compound **436-2** (150 mg, yield: 47%) as a yellow oil. MS (ESI, m/z): 463.3 $[M+H]^+$.

Compound **436-2** (150 mg, 0.33 mmol) was added to triethylamine (0.1 mL) and dichloromethane (5 mL). At 0°C, compound **436-3** (0.1 mL) was added, and the reaction mixture was stirred at 25°C for 4 hours. After the reaction was completed, the reaction mixture was concentrated and purified by normal-phase column chromatography (ethyl acetate: petroleum ether = 0% to 100%) to obtain compound **436-4** (116 mg, yield: 64%) as a yellow oil. MS (ESI, m/z): 547.3 $[M+H]^+$.

Compound **436-4** (116 mg, 0.21 mmol) was added to methanol (2 mL), water (2 mL), and tetrahydrofuran (2 mL) and stirred. Lithium hydroxide (8 mg, 0.32 mmol) was added, and the reaction mixture was stirred at 60°C for 4 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound **436** (31 mg, yield: 27%) as a white solid. MS (ESI, m/z): 533.2 $[M+H]^+$.

$^1$H NMR (400 MHz, $CDCl_3$) δ 7.42 (d, *J* = 8.0 Hz, 1H), 7.36 (s, 1H), 7.25 - 7.18 (m, 1H), 7.14 (d, *J* = 8.0 Hz, 1H), 6.63 (dd, *J* = 8.0, 2.0 Hz, 1H), 6.58 - 6.55 (m, 1H), 6.47 (dd, *J* = 8.0, 2.0 Hz, 1H), 5.80 - 5.74 (m, 1H), 4.52 (d, *J* = 4.8 Hz, 2H), 3.87 - 3.78 (m, 5H), 3.13 - 3.01 (m, 1H), 2.95 - 2.77 (m, 4H), 2.55 - 2.45 (m, 1H), 2.26 - 2.13 (m, 2H), 1.93 - 1.84 (m, 2H), 1.23 (s, 9H), 1.15 - 1.05 (m, 1H), 0.69 - 0.60 (m, 1H), 0.51 - 0.42 (m, 1H), 0.40 - 0.32 (m, 1H), 0.25 - 0.17 (m, 1H).

**Example 437:**

Synthetic Route:

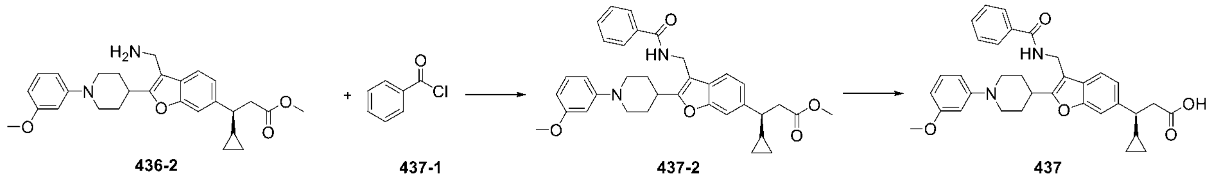

436-2 437-1 437-2 437

Referring to the synthetic route of compound **434,** compound **436-3** was replaced with compound **437-1** to carry out the synthesis. The resulting crude product was purified by reverse-phase column chromatography [acetonitrile/water (0.05% formic acid) = 0% to 100%] to obtain compound **437** (16 mg, yield: 29%) as a white solid. MS (ESI, m/z): 553.3 $[M+H]^+$.

$^1$H NMR (400 MHz, $CDCl_3$) δ 7.74 (d, *J* = 7.6 Hz, 2H), 7.51 -7.44 (m, 2H), 7.43 - 7.36 (m, 2H), 7.32 (s, 1H), 7.26 - 7.19 (m, 1H), 7.10 (d, *J* = 8.0 Hz, 1H), 6.64 - 6.57 (m, 1H), 6.55 - 6.50 (m, 1H), 6.46 - 6.41 (m, 1H), 6.34 - 6.26 (m, 1H), 4.70 (d, *J* = 4.8 Hz, 2H), 3.82 - 3.76 (m, 5H), 3.18 - 3.03 (m, 1H), 2.94 - 2.71 (m, 4H), 2.52 - 2.40 (m, 1H), 2.26 - 2.09 (m, 2H), 1.99 - 1.86 (m, 2H), 1.14 - 1.01 (m, 1H), 0.68 - 0.55 (m, 1H), 0.49 - 0.36 (m, 1H), 0.36 - 0.25 (m, 1H), 0.21 - 0.12 (m, 1H).

**Example 438:**

Synthetic Route:

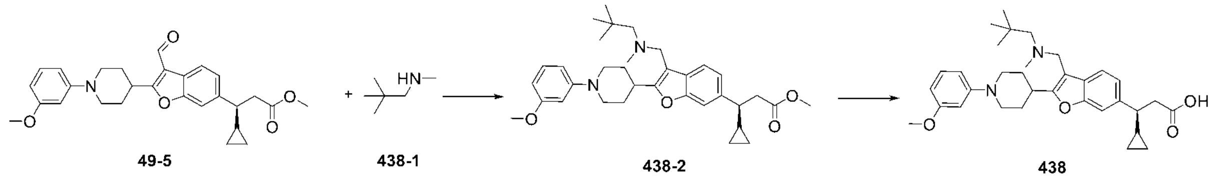

49-5 438-1 438-2 438

Referring to the synthetic route of compound **405,** compound **126-2** was replaced with compound **49-5,** and compound **200-1** was replaced with compound **438-1.** The synthesis was carried out, and the resulting crude product was purified by reverse-phase column chromatography [acetonitrile/water (0.05% formic acid) = 0% to 100%] to obtain compound **438** (36 mg, yield: 62%) as a white solid. MS (ESI, m/z): 533.3 $[M+H]^+$.

$^1H$ NMR (400 MHz, $CDCl_3$) δ 7.59 (d, $J$ = 8.4 Hz, 1H), 7.29 (s, 1H), 7.21 - 7.16 (m, 1H), 7.10 (d, $J$ = 8.0 Hz, 1H), 6.61 (d, $J$ = 8.4 Hz, 1H), 6.51 - 6.55 (m, 1H), 6.43 (d, $J$ = 8.0 Hz, 1H), 3.85 - 3.77 (m, 5H), 3.71 - 3.52 (m, 2H), 3.22 - 3.04 (m, 1H), 2.90 - 2.76 (m, 4H), 2.50 - 2.45 (m, 1H), 2.28 - 2.15 (m, 7H), 1.90 - 1.81 (m, 2H), 1.13 - 1.04 (m, 1H), 0.95 (s, 9H), 0.65 - 0.55 (m, 1H), 0.47 - 0.39 (m, 1H), 0.38 - 0.28 (m, 1H), 0.25 - 0.13 (m, 1H).

**Example 439:**

Synthetic Route:

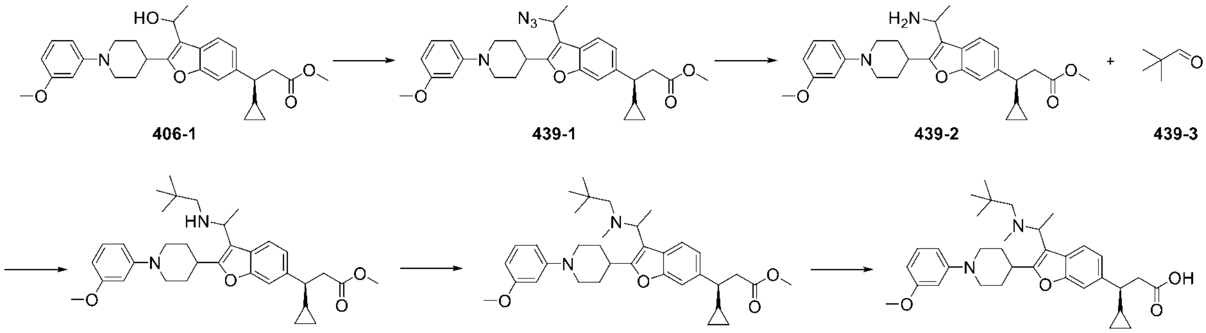

406-1 439-1 439-2 439-3

439-4 439-5 439

Compound **406-1** (114 mg, 0.239 mmol), diphenylphosphoryl azide (131 mg, 0.478 mmol), and 1,8-diazabicyclo [5.4.0]undec-7-ene (73 mg, 0.478 mmol) were dissolved in dichloromethane (5 mL). The reaction was carried out at 25°C for 24 hours. After concentration, the resulting crude product was purified by normal-phase column chromatography (ethyl acetate: petroleum ether = 0% to 100%) to obtain compound **439-1** (67 mg, yield: 56%) as a white solid. MS (ESI, m/z): 503.3 $[M+H]^+$.

Compound **439-1** (67 mg, 0.133 mmol) and triphenylphosphine (70 mg, 0.267 mmol) were added to a mixture of tetrahydrofuran (5 mL) and water (5 mL), and the reaction was stirred at 60°C overnight. After concentration, the resulting crude product was purified by normal-phase column chromatography (ethyl acetate: petroleum ether = 0% to 100%) to obtain compound **439-2** (51 mg, yield: 83%) as a yellow oil. MS (ESI, m/z): 477.3 $[M+H]^+$.

Compound **439-2** (51 mg, 0.107 mmol) and compound **439-3** (13 mg, 0.161 mmol) were added to a mixture of acetic acid (0.05 mL) and dichloromethane (5 mL). Sodium triacetoxyborohydride (32 mg, 0.15 mmol) was added at 0°C, and the reaction mixture was stirred at 25°C for 24 hours. After the reaction was completed, the reaction mixture was concentrated and purified by normal-phase column chromatography (ethyl acetate: petroleum ether = 0% to 100%) to obtain compound **439-4** (55 mg, yield: 94%) as a yellow oil. MS (ESI, m/z): 547.3 $[M+H]^+$.

Compound **439-4** (55 mg, 0.1 mmol) and 40% formaldehyde (0.1 mL) were added to a mixture of acetic acid (0.05 mL) and dichloromethane (5 mL). Sodium triacetoxyborohydride (32 mg, 0.15 mmol) was added at 0°C, and the reaction mixture was stirred at 25°C for 24 hours. After the reaction was completed, the reaction mixture was concentrated and purified by normal-phase column chromatography (ethyl acetate: petroleum ether = 0% to 100%) to obtain compound

**439-5** (38 mg, yield: 68%) as a yellow oil. MS (ESI, m/z): 561.3 [M+H]+.

Compound **439-5** (36 mg, 0.0643 mmol) was added to methanol (1 mL), water (1 mL), and tetrahydrofuran (1 mL) and stirred. Lithium hydroxide (31 mg, 1.29 mmol) was added, and the reaction mixture was stirred at 60°C for 4 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound 439 (5 mg, yield: 14%) as a white solid. MS (ESI, m/z): 547.3 [M+H]+.

$^1$H NMR (400 MHz, $CDCl_3$) δ 7.64 - 7.55 (m, 1H), 7.32 - 7.28 (m, 1H), 7.22 - 7.15 (m, 1H), 7.11 - 7.05 (m, 1H), 6.60 (d, *J*= 8.4 Hz, 1H), 6.55 - 6.51 (m, 1H), 6.43 (dd, *J* = 8.0, 2.0 Hz, 1H), 4.14 - 3.94 (m, 1H), 3.86 - 3.73 (m, 5H), 3.17 - 3.04 (m, 1H), 2.92 - 2.73 (m, 4H), 2.53 - 1.98 (m, 8H), 1.90 - 1.65 (m, 2H), 1.59 - 1.42 (m, 3H), 1.13 - 1.04 (m, 1H), 0.96 (s, 9H), 0.66 - 0.55 (m, 1H), 0.49 - 0.39 (m, 1H), 0.37 - 0.28 (m, 1H), 0.24 - 0.14 (m, 1H).

**Example 440:**

Synthetic Route:

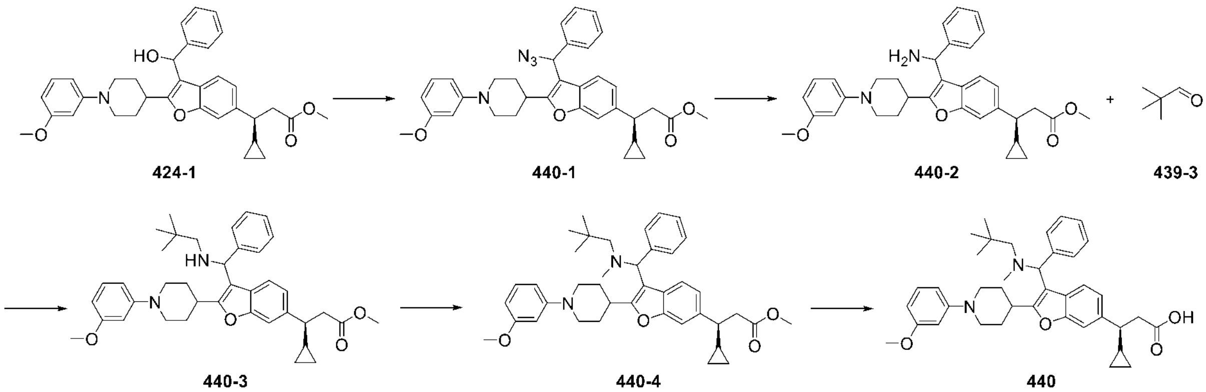

424-1 440-1 440-2 439-3

440-3 440-4 440

Referring to the synthetic route of compound **439,** compound **406-2** was replaced with compound **424-1** to carry out the synthesis. The resulting crude product was purified by reverse-phase column chromatography [acetonitrile/water (0.05% formic acid) = 0% to 100%] to obtain compound **440** (7 mg, yield: 15%) as a white solid. MS (ESI, m/z): 613.3 [M+H]+.

$^1$H NMR (400 MHz, $CDCl_3$) δ 7.53 (d, *J* = 7.2 Hz, 2H), 7.42 (d, *J* = 8.0 Hz, 1H), 7.35 - 7.30 (m, 3H), 7.28 - 7.18 (m, 2H), 7.01 (d, *J* = 8.0 Hz, 1H), 6.68 - 6.60 (m, 1H), 6.58 - 6.54 (m, 1H), 6.50 - 6.41 (m, 1H), 4.92 (s, 1H), 3.93 - 3.75 (m, 5H), 3.12 - 3.01 (m, 1H), 2.93 - 2.73 (m, 4H), 2.57 - 2.03 (m, 8H), 1.97 -1.69 (m, 2H), 1.13 - 1.07 (m, 1H), 0.97 (s, 9H), 0.69 - 0.58 (m, 1H), 0.54 - 0.42 (m, 1H), 0.41 - 0.31 (m, 1H), 0.28 - 0.15 (m, 1H).

**Example 441:**

Synthetic Route:

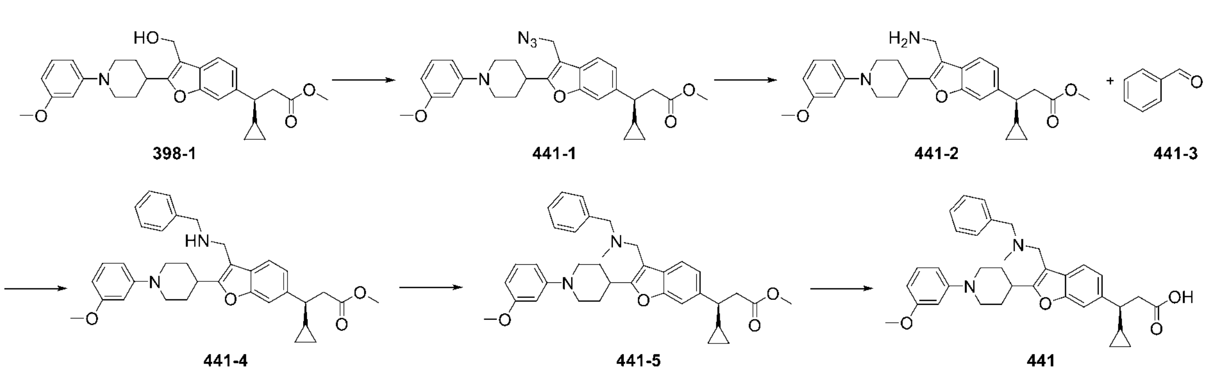

398-1 441-1 441-2 441-3

441-4 441-5 441

Referring to the synthetic route of compound **439,** compound **406-2** was replaced with compound **398-1,** and compound **439-3** was replaced with compound **441-3.** The synthesis was carried out, and the resulting crude product was purified by reverse-phase column chromatography [acetonitrile/water (0.05% formic acid) = 0% to 100%] to obtain compound **441** (7 mg, yield: 15%) as a white solid. MS (ESI, m/z): 613.3 $[M+H]^+$.

$^1$H NMR (400 MHz, $CDCl_3$) δ 7.43 (d, *J* = 7.6 Hz, 1H), 7.37 - 7.28 (m, 6H), 7.26 - 7.19 (m, 1H), 7.16 (d, *J* = 8.0 Hz, 1H), 6.64 (d, *J* = 8.4 Hz, 1H), 6.59 - 6.55 (m, 1H), 6.48 (dd, *J*= 8.0 Hz, 2.0 Hz, 1H), 3.87 - 3.64 (m, 9H), 3.04 - 2.95 (m, 1H), 2.93 - 2.77 (m, 4H), 2.56 - 2.47 (m, 1H), 2.28 - 2.09 (m, 5H), 1.85 - 1.75 (m, 2H), 1.14 - 1.04 (m, 1H), 0.68 - 0.57 (m, 1H), 0.48 - 0.31 (m, 2H), 0.25 - 0.17 (m, 1H).

**Example 442:**

Synthetic Route:

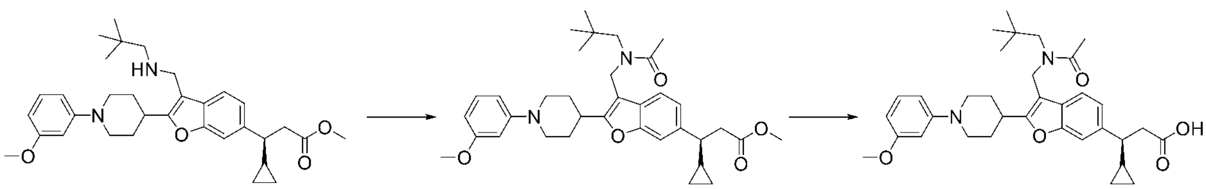

435-2 442-1 442

Compound **435-2** (75 mg, 0.14 mmol), diisopropylethylamine (54 mg, 0.42 mmol), and acetic anhydride (54 mg, 0.42 mmol) were added to dichloromethane (2 mL) and stirred at 25°C for 24 hours. After the reaction was completed, the reaction mixture was concentrated and purified by normal-phase column chromatography (ethyl acetate: petroleum ether = 0% to 100%) to obtain compound **442-1** (68 mg, yield: 84%) as a yellow oil. MS (ESI, m/z): 575.3 $[M+H]^+$.

Referring to the synthetic route of compound **439,** compound **439-5** was replaced with compound **442-1** to carry out the synthesis. The resulting crude product was purified by reverse-phase column chromatography [acetonitrile/water (0.05% formic acid) = 0% to 100%] to obtain compound **435** (56 mg, yield: 83%) as a white solid. MS (ESI, m/z): 561.3 $[M+H]^+$.

$^1$H NMR (400 MHz, $CDCl_3$) δ 7.45 - 7.28 (m, 2H), 7.24 - 7.17 (m, 1H), 7.11 - 7.05 (m, 1H), 6.74 - 6.36 (m, 3H), 4.86 - 4.66 (m, 2H), 3.88 - 3.75 (m, 5H), 3.33 - 2.98 (m, 3H), 2.95 - 2.74 (m, 4H), 2.55 - 2.46 (m, 1H), 2.31 - 2.10 (m, 5H), 1.90 - 1.79 (m, 2H), 1.12 - 0.95 (m, 10H), 0.66 - 0.57 (m, 1H), 0.47 - 0.39 (m, 1H), 0.36 - 0.28 (m, 1H), 0.21 - 0.14 (m, 1H).

**Example 443:**

Synthetic Route:

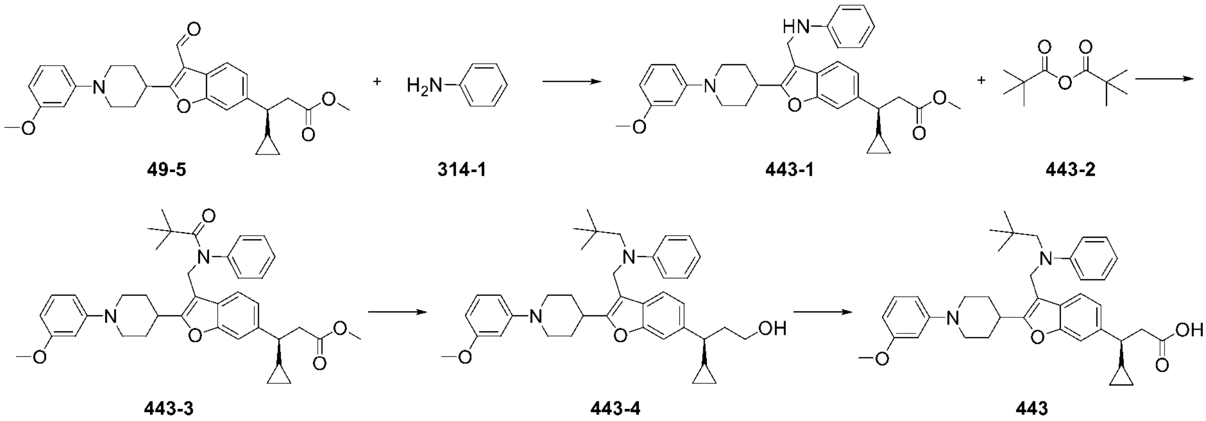

49-5 314-1 443-1 443-2

443-3 443-4 443

Compound **49-5** (78 mg, 0.17 mmol), compound **314-1** (24 mg, 0.26 mmol), and acetic acid (0.5 mL) were added to dichloromethane (15 mL) and stirred. The reaction was carried out at room temperature for 3 hours, followed by the addition of sodium triacetoxyborohydride (110 mg, 0.52 mmol). The reaction was then carried out at room temperature for

16 hours. After the reaction was completed, the reaction mixture was concentrated to obtain a crude product. The resulting crude product was purified by normal-phase column chromatography (petroleum ether: ethyl acetate = 8:2) to obtain compound **443-1** (90 mg, yield: 98%) as a yellow oil. MS (ESI, m/z): 539.3 $[M+H]^+$.

Compound **443-1** (90 mg, 0.167 mmol) and trimethylacetic anhydride **443-2** (2 mL) were added to pyridine (1 mL) and stirred. The reaction was carried out at 120°C for 48 hours. After the reaction was completed, the reaction mixture was concentrated to obtain a crude product. The resulting crude product was purified by normal-phase column chromatography (petroleum ether: ethyl acetate = 7:3) to obtain compound 443-3 (100 mg, yield: 96%) as a yellow oil. MS (ESI, m/z): 623.3 $[M+H]^+$.

Compound **443-3** (100 mg, 0.16 mmol) was added to tetrahydrofuran (10 mL) and stirred. Borane dimethyl sulfide complex (2.0 M, 1.0 mL, 2.0 mmol) was added, and the reaction was carried out at 60°C for 16 hours. After the reaction was completed, the reaction mixture was slowly quenched with methanol (1 mL) at 0°C and concentrated to obtain a crude product. The resulting crude product was then purified by reverse-phase column chromatography (water (0.1% formic acid): acetonitrile = 2:8) to obtain compound **443-4** (45 mg, yield: 48%) as a white solid. MS (ESI, m/z): 581.3 $[M+H]^+$.

Compound **443-4** (45 mg, 0.078 mmol), 1-hydroxycyclohexyl phenyl ketone (160 mg, 0.78 mmol), and sodium hydroxide (31 mg, 0.78 mmol) were added to ethylene glycol dimethyl ether (10 mL) and stirred. The reaction was carried out at 110°C under a nitrogen atmosphere for 16 hours. After the reaction was completed, the reaction mixture was concentrated to obtain a crude product. The resulting crude product was then purified by reverse-phase column chromatography (water (0.1% formic acid): acetonitrile = 1:9) to obtain compound **443** (4 mg, yield: 9%) as an off-white solid. MS (ESI, m/z): 595.3 $[M+H]^+$.

$^1$H NMR (400 MHz, $CDCl_3$) δ 7.54 - 7.32 (m, 2H), 7.25 (t, *J* = 8.4 Hz, 2H), 7.18 -7.12 (m, 1H), 7.09 (d, *J* = 8.4 Hz, 1H), 6.83 (s, 1H), 6.76 (dd, *J* = 8.4, 2.8 Hz, 1H), 6.65 (dd, *J* = 17.2, 8.0 Hz, 2H), 6.58 (s, 1H), 6.49 (d, *J* = 8.4 Hz, 1H), 4.72 (s, 2H), 3.83 (s, 3H), 3.77 (d, *J* = 12.8 Hz, 2H), 3.31 (s, 2H), 2.92 -2.78 (m, 4H), 2.74 - 2.66 (m, 1H), 2.55 - 2.43 (m, 1H), 2.17 (t, *J* = 13.2 Hz, 2H), 1.69 (d, *J* = 13.2 Hz, 2H), 1.14 - 1.05 (m, 10H), 0.71 - 0.59 (m, 1H), 0.52 - 0.44 (m, 1H), 0.39 - 0.32 (m, 1H), 0.23 - 0.19 (m, 1H).

**Example 444:**

Synthetic Route:

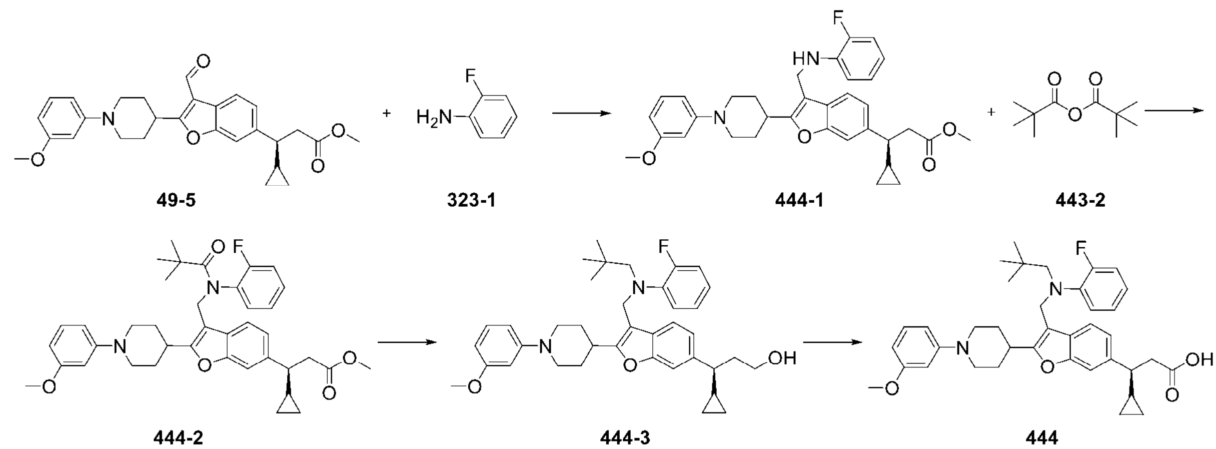

49-5 323-1 444-1 443-2

444-2 444-3 444

Referring to the synthetic route of compound **443,** compound **314-1** was replaced with compound **323-1** to carry out the synthesis. The resulting crude product was purified by reverse-phase column chromatography [acetonitrile/water (0.05% formic acid) = 0% to 100%] to obtain compound **444** (56 mg, yield: 83%) as a white solid. MS (ESI, m/z): 613.3 $[M+H]^+$.

**Example 445:**

Synthetic Route:

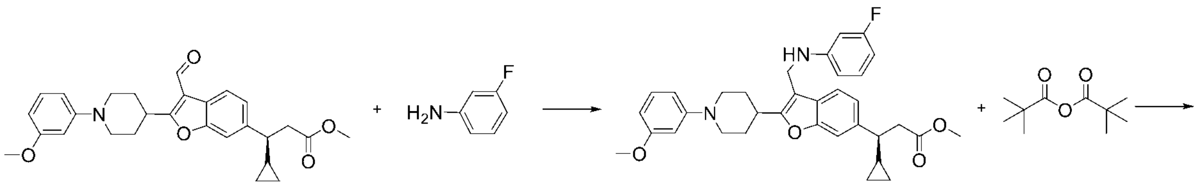

49-5 318-1 445-1 443-2

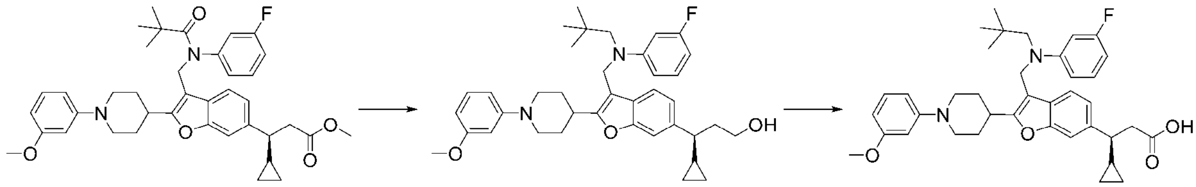

445-2 445-3 445

Referring to the synthetic route of compound **443,** compound **314-1** was replaced with compound **318-1** to carry out the synthesis. The resulting crude product was purified by reverse-phase column chromatography [acetonitrile/water (0.05% formic acid) = 0% to 100%] to obtain compound **445** (56 mg, yield: 83%) as a white solid. MS (ESI, m/z): 613.3 [M+H]+.

**Example 446:**

Synthetic Route:

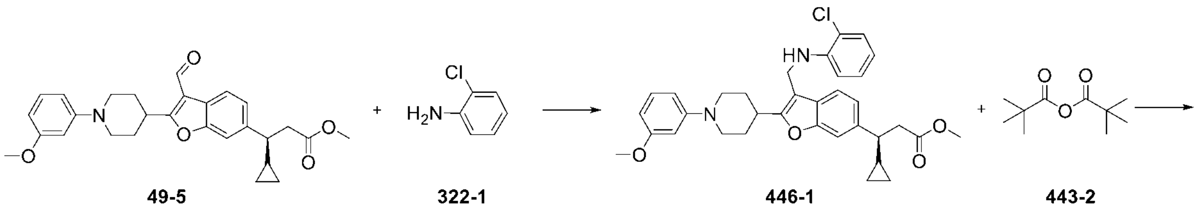

49-5 322-1 446-1 443-2

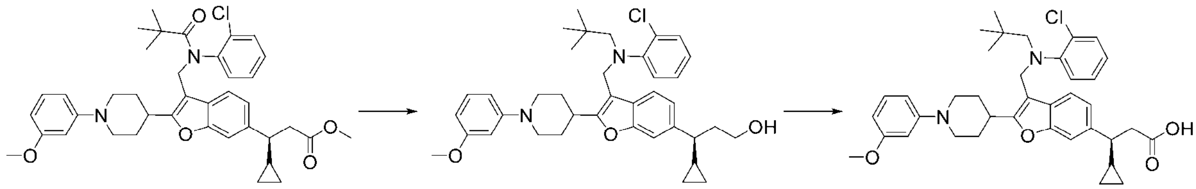

446-2 446-3 446

Referring to the synthetic route of compound **443,** compound **314-1** was replaced with compound **323-1** to carry out the synthesis. The resulting crude product was purified by reverse-phase column chromatography [acetonitrile/water (0.05% formic acid) = 0% to 100%] to obtain compound **444** (56 mg, yield: 83%) as a white solid. MS (ESI, m/z): 629.3 [M+H]+.

**Example 447:**

Synthetic Route:

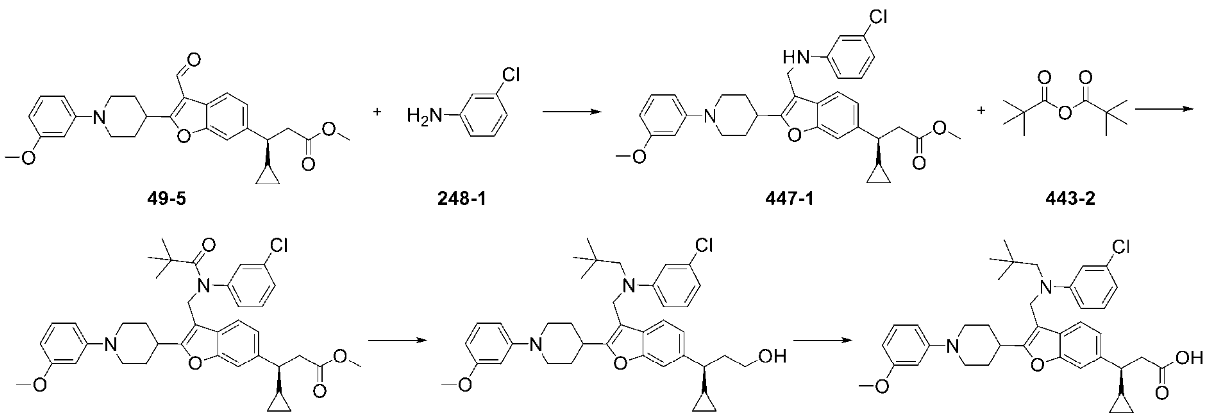

49-5 248-1 447-1 443-2

447-2 447-3 447

Referring to the synthetic route of compound **443,** compound **314-1** was replaced with compound **248-1** to carry out the synthesis. The resulting crude product was purified by reverse-phase column chromatography [acetonitrile/water (0.05% formic acid) = 0% to 100%] to obtain compound **447** (4 mg, yield: 9%) as a white solid. MS (ESI, m/z): 629.3 $[M+H]^+$.

$^1$H NMR (400 MHz, $CDCl_3$) δ 7.17 - 7.29 (m, 3H), 7.11 - 7.04 (m, 2H), 6.85 (s, 1H), 6.75 (dd, *J* = 8.4, 2.8 Hz, 1H), 6.69 - 6.58 (m, 2H), 6.58 - 6.51 (m, 1H), 6.46 (d, *J* = 8.4 Hz, 1H), 4.70 (s, 2H), 3.85 (s, 3H), 3.84 - 3.74 (m, 2H), 3.32 (s, 2H), 2.91 -2.79 (m, 3H), 2.76 - 2.67 (m, 2H), 2.54 - 2.45 (m, 1H), 2.21 - 2.08 (m, 2H), 1.72 - 1.63 (m, 2H), 1.13 - 1.05 (m, 10H), 0.70 - 0.59 (m, 1H), 0.50 - 0.43 (m, 1H), 0.39 - 0.31 (m, 1H), 0.22 - 0.19 (m, 1H).

**Example 448:**

Synthetic Route:

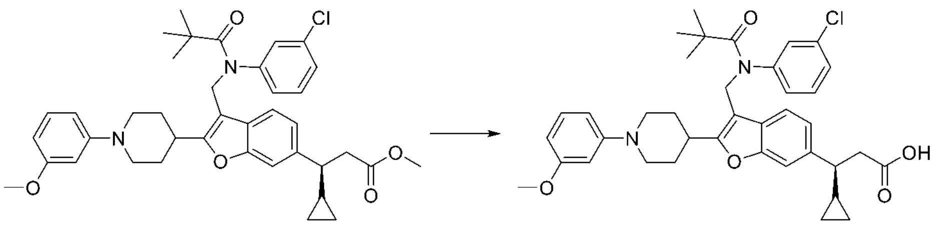

447-2 448

Referring to the synthetic route of compound **443,** compound **443-4** was replaced with compound **447-2** to carry out the synthesis. The resulting crude product was purified by reverse-phase column chromatography [acetonitrile/water (0.05% formic acid) = 0% to 100%] to obtain compound **448** (3 mg, yield: 8%) as a white solid. MS (ESI, m/z): 643.3 $[M+H]^+$.

$^1$H NMR (400 MHz, $CDCl_3$) δ 7.48 (d, *J* = 8.0 Hz, 1H), 7.34 - 7.29 (m, 2H), 7.25 - 7.16 (m, 2H), 7.10 (dd, *J* = 8.0, 1.6 Hz, 1H), 7.07 - 7.04 (m, 1H), 6.88 - 6.81 (m, 1H), 6.58 (dd, *J* = 8.4, 1.1 Hz, 1H), 6.52 - 6.48 (m, 1H), 6.44 (dd, *J* = 8.4, 2.4 Hz, 1H), 5.00 (s, 2H), 3.83 (s, 3H), 3.74 - 3.64 (m, 2H), 2.93 - 2.81 (m, 2H), 2.67 - 2.57 (m, 2H), 2.55 - 2.47 (m, 1H), 2.44 - 2.34 (m, 1H), 2.03 - 1.89 (m, 2H), 1.37 - 1.31 (m, 2H), 1.13 - 1.10 (m, 1H), 1.07 (s, 9H), 0.68 - 0.59 (m, 1H), 0.51 - 0.44 (m, 1H), 0.39 - 0.33 (m, 1H), 0.25 - 0.19 (m, 1H).

**Example 449:**

Synthetic Route:

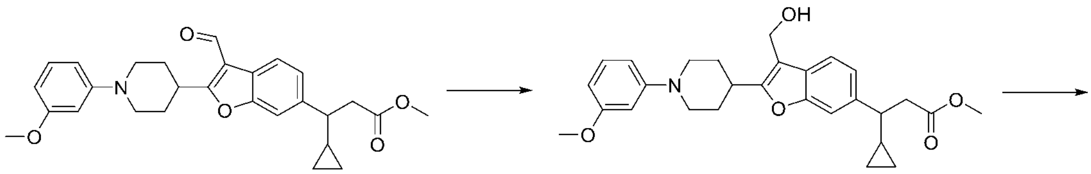

126-2 449-1

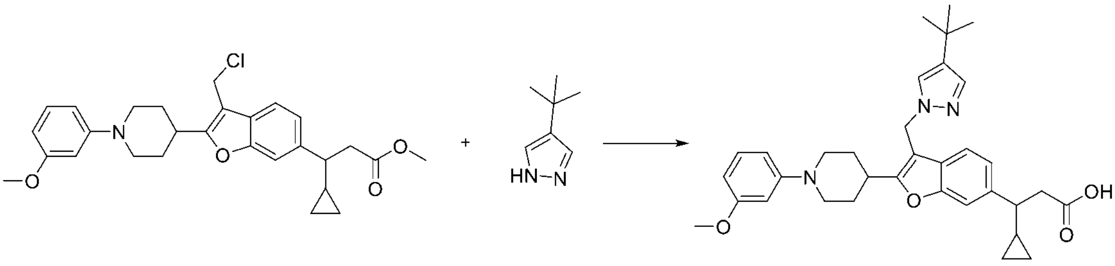

449-2 449-3 449

Referring to the synthetic route of compound **398,** compound **49-5** was replaced with compound **126-2** to synthesize compound **449-1** (20 mg, yield: 98%). MS (ESI, m/z): 464.3 $[M+H]^+$.

Compound **449-1** (20 mg, 0.04 mmol) and triethylamine (13.08 mg, 0.13 mmol) were added to dichloromethane (10 mL). Methanesulfonyl chloride (7.4 mg, 0.06 mmol) was added at 0°C, and the reaction mixture was stirred at 25°C for 4 hours. After the reaction was completed, the reaction mixture was concentrated and purified by normal-phase column chromatography (ethyl acetate: petroleum ether = 0% to 100%) to obtain compound **449-2** (20 mg, yield: 96%) as a yellow oil. MS (ESI, m/z): 482.3 $[M+H]^+$.

Compound **449-2** (20 mg, 0.04 mmol) was added to N,N-dimethylformamide (5 mL), followed by the addition of sodium hydride (1.2 mg, 0.05 mmol) at 0°C. The reaction mixture was stirred at 0°C for 0.5 hours, and then compound **449-3** (6.2 mg, 0.05 mmol) was added. The reaction mixture was stirred at 80°C for 4 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound **449** (2 mg, yield: 7%) as a white solid. MS (ESI, m/z): 556.3 $[M+H]^+$.

$^1$H NMR (400 MHz, MeOD) δ 7.49 (s, 1H), 7.39 (s, 1H), 7.33 (s, 1H), 7.27 (d, *J* = 8.0 Hz, 1H), 7.18 - 7.12 (m, 1H), 7.09 (dd, *J*= 8.1, 1.1 Hz, 1H), 6.65 - 6.60 (m, 1H), 6.56 - 6.53 (m, 1H), 6.44 (dd, *J* = 8.0, 2.1 Hz, 1H), 5.40 (s, 2H), 3.81 - 3.74 (m, 5H), 3.06 - 2.95 (m, 1H), 2.87 - 2.68 (m, 4H), 2.47 - 2.36 (m, 1H), 2.18 - 2.07 (m, 2H), 1.91 - 1.80 (m, 2H), 1.23 (s, 9H), 1.13 - 1.05 (m, 1H), 0.64 - 0.55 (m, 1H), 0.42 - 0.26 (m, 2H), 0.18 - 0.10 (m, 1H).

**Example 450:**

Synthetic Route:

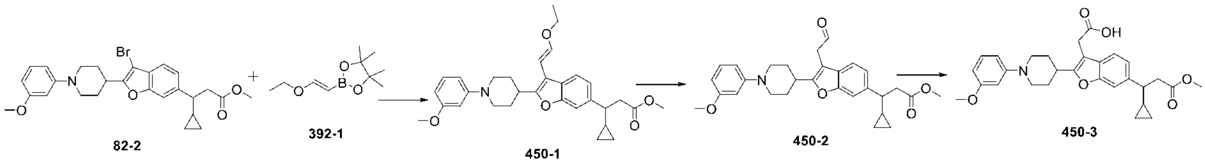

82-2 392-1 450-1 450-2 450-3

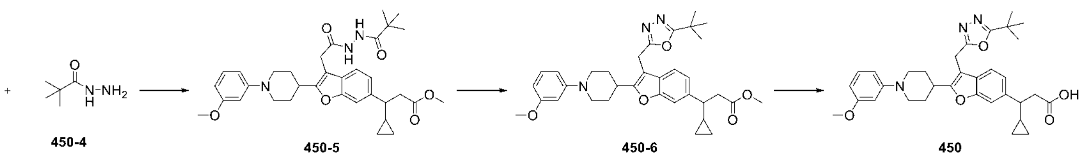

450-4 450-5 450-6 450

Referring to the synthetic route of compound **397,** compound **98-1** was replaced with compound **82-2** to synthesize compound **450-3** (550 mg, yield: 37%). MS (ESI, m/z): 492.3 $[M+H]^+$.

Compound **450-3** (492 mg, 1 mmol), 2,2-dimethylpropionic acid hydrazide **450-4** (128 mg, 1.1 mmol), 2-(7-azabenzotriazol-1-yl)-*N,N,N',N'*-tetramethyluronium hexafluorophosphate (418 mg, 1.1 mmol), and triethylamine (303 mg, 3 mmol) were added to dichloromethane (6 mL), and the reaction mixture was stirred at room temperature for 1 hour.

After the reaction was completed, the reaction mixture was concentrated and purified by normal-phase column chromatography (ethyl acetate: petroleum ether = 0% to 100%) to obtain compound **450-5** (367 mg, yield: 62%) as a yellow solid. MS (ESI, m/z): 590.3 [M+H]$^+$.

To a mixture of compound **450-5** (367 mg, 0.62 mmol), p-toluenesulfonyl chloride (288 mg, 1.51 mmol), and dichloromethane (4 mL), triethylamine (382 mg, 3.78 mmol) was added dropwise at 0°C. The reaction mixture was stirred at room temperature for 3 hours. After the reaction was completed, the reaction mixture was concentrated and purified by normal-phase column chromatography (ethyl acetate: petroleum ether = 0% to 100%) to obtain compound **450-6** (290 mg, yield: 82%) as a yellow solid. MS (ESI, m/z): 572.3 [M+H]$^+$.

Compound **450-6** (290 mg, 0.51 mmol) was added to methanol (2 mL), water (2 mL), and tetrahydrofuran (2 mL) and stirred. Lithium hydroxide (41 mg, 1.7 mmol) was added, and the reaction mixture was stirred at 60°C for 2 hours. After the reaction was completed, the reaction mixture was concentrated to obtain a crude product. The resulting crude product was purified by reverse-phase column chromatography [acetonitrile/water (0.05% formic acid) = 0% to 100%] to obtain compound **450** (120 mg, yield: 42%) as a white solid. MS (ESI, m/z): 558.3 [M+H]$^+$.

$^1$H NMR (400 MHz, $CDCl_3$) δ 7.47 (d, *J* = 8.0 Hz, 1H), 7.32 (s, 1H), 7.24 - 7.17 (m, 1H), 7.12 (d, *J* = 8.0 Hz, 1H), 6.66 - 6.58 (m, 1H), 6.58 - 6.50 (m, 1H), 6.49 - 6.39 (m, 1H), 4.20 (s, 2H), 3.87 - 3.74 (m, 5H), 3.11 - 2.98 (m, 1H), 2.95 - 2.71 (m, 4H), 2.55 - 2.38 (m, 1H), 2.31 - 2.12 (m, 2H), 1.97 - 1.87 (m, 2H), 1.37 (s, 9H), 1.15 - 0.98 (m, 1H), 0.69 - 0.53 (m, 1H), 0.48 - 0.38 (m, 1H), 0.39 - 0.25 (m, 1H), 0.23 - 0.04 (m, 1H).

**Example 451:**

Synthetic Route:

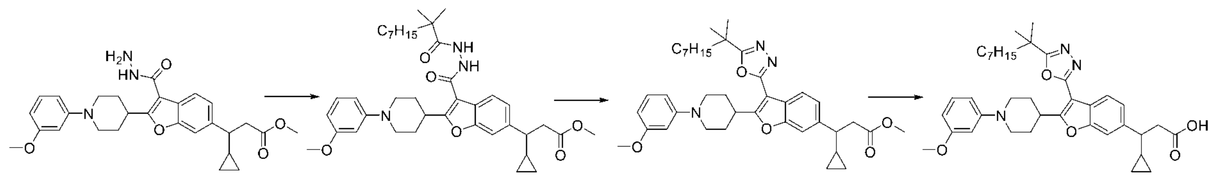

126-5 451-1 451-2 451

Referring to the synthetic route of compound **126,** 3,3,3-trifluoro-2,2-dimethylpropionic acid was replaced with 2,2-dimethylnonanoic acid to carry out the synthesis. The resulting crude product was purified by reverse-phase column chromatography [acetonitrile/water (0.05% formic acid) = 0% to 100%] to obtain compound **451** (20 mg, yield: 25%) as a white solid. MS (ESI, m/z): 628.3 [M+H]$^+$. $^1$H NMR (400 MHz, $CDCl_3$) δ 7.93 (d, *J* = 8.0 Hz, 1H), 7.41 (s, 1H), 7.27 - 7.16 (m, 2H), 6.71 - 6.42 (m, 3H), 3.91 - 3.79 (m, 6H), 3.05 - 2.76 (m, 4H), 2.56 - 2.46 (m, 1H), 2.35 - 2.11 (m, 2H), 2.12 - 2.02 (m, 2H), 1.86 - 1.73 (m, 2H), 1.50 (s, 6H), 1.36 - 1.16 (m, 10H), 1.11 - 1.06 (m, 1H), 0.89 - 0.82 (m, 3H), 0.66 - 0.59 (m, 1H), 0.49 - 0.42 (m, 1H), 0.36 - 0.32 (m, 1H), 0.21 - 0.16 (m, 1H).

**Example 452:**

Synthetic Route:

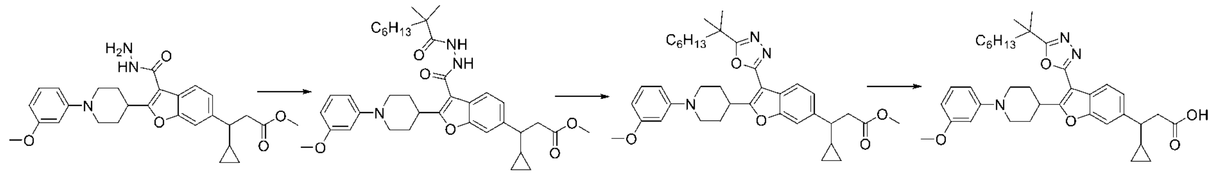

126-5 452-1 452-2 452

Referring to the synthetic route of compound **126,** 3,3,3-trifluoro-2,2-dimethylpropionic acid was replaced with 2,2-dimethyloctanoic acid to carry out the synthesis. The resulting crude product was purified by reverse-phase column chromatography [acetonitrile/water (0.05% formic acid) = 0% to 100%] to obtain compound **452** (15 mg, yield: 36%) as a white solid. MS (ESI, m/z): 614.3 [M+H]$^+$. $^1$H NMR (400 MHz, $CDCl_3$) δ 7.96 (d, *J* = 8.0 Hz, 1H), 7.43 (s, 1H), 7.31 - 7.26 (m,

1H), 7.26 - 7.19 (m, 1H), 6.65 (dd, *J* = 8.0, 2.4 Hz, 1H), 6.59 - 6.56 (m, 1H), 6.47 (dd, *J* = 8.0, 2.4 Hz, 1H), 3.89 - 3.79 (m, 6H), 3.02 - 2.82 (m, 4H), 2.60 - 2.50 (m, 1H), 2.28 - 2.19 (m, 2H), 2.15 - 2.02 (m, 2H), 1.86 - 1.77 (m, 2H), 1.53 (s, 6H), 1.35 - 1.25 (m, 8H), 1.17 - 1.07 (m, 1H), 0.88 (t, *J* = 6.8 Hz, 1H 3H), 0.69 - 0.63 (m, 1H), 0.51 - 0.44 (m, 1H), 0.39 - 0.34 (m, 1H), 0.24 - 0.18 (m, 1H).

**Example 453:**

Synthetic Route:

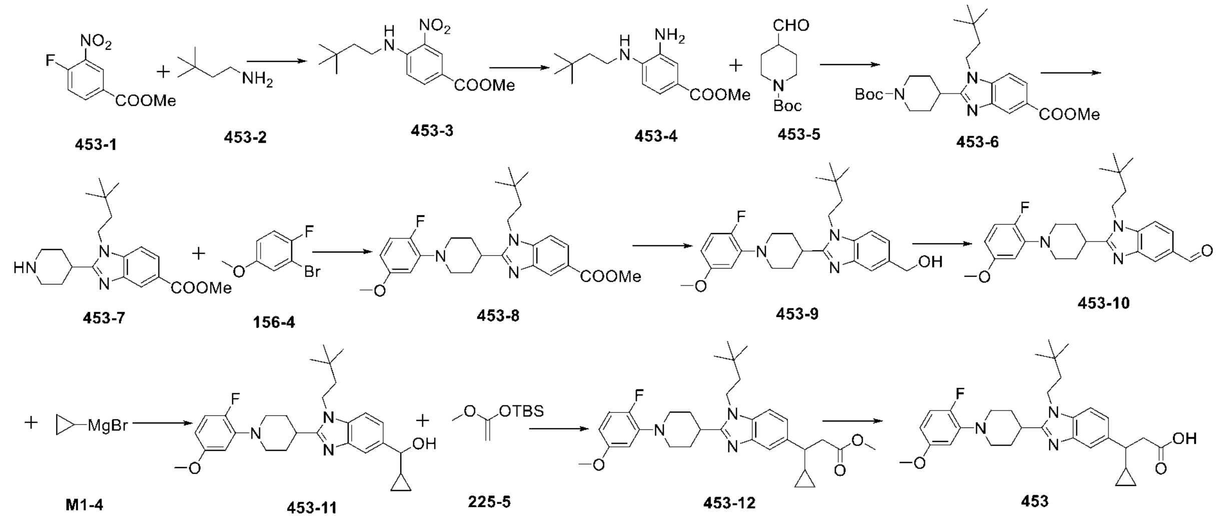

453-1 453-2 453-3 453-4 453-5 453-6

453-7 156-4 453-8 453-9 453-10

M1-4 453-11 225-5 453-12 453

Compound **453-1** (900 mg, 4.5 mmol), compound **453-2** (450 mg, 4.5 mmol), and anhydrous potassium carbonate (621 mg, 4.5 mmol) were added to tetrahydrofuran (20 mL) and stirred at room temperature for 16 hours. After the reaction was completed, the reaction mixture was concentrated and purified by normal-phase column chromatography (ethyl acetate: petroleum ether = 0% to 100%) to obtain compound **453-3** (983 mg, yield: 78%) as a brick-red solid. MS (ESI, m/z): 281.3 $[M+H]^+$.

Compound **453-3** (983 mg, 3.5 mmol) and 5% platinum on carbon (200 mg) were added to tetrahydrofuran (5 mL) and methanol (5 mL). The reaction mixture was stirred under a hydrogen atmosphere at room temperature for 16 hours. After the reaction was completed, the reaction mixture was concentrated and purified by normal-phase column chromatography (ethyl acetate: petroleum ether = 0% to 100%) to obtain compound **453-4** (812 mg, yield: 92%) as a white solid. MS (ESI, m/z): 251.3 $[M+H]^+$.

Compound **453-4** (812 mg, 3.25 mmol) and compound **453-5** (692 mg, 3.25 mmol) were added to *N,N*-dimethylformamide (10 mL) and water (10 mL), and stirred at 110°C for 48 hours. After the reaction was completed, the reaction mixture was concentrated and purified by normal-phase column chromatography (ethyl acetate: petroleum ether = 0% to 100%) to obtain compound **453-6** (1170 mg, yield: 81%) as a white solid. MS (ESI, m/z): 444.3 $[M+H]^+$.

Compound **453-6** (1.17 g, 2.6 mmol) and trifluoroacetic acid (3 mL) were added to dichloromethane (10 mL) and stirred at room temperature for 16 hours. After the reaction was completed, saturated sodium bicarbonate solution was added to adjust the pH to 8. The organic phase was dried over anhydrous sodium sulfate, filtered, and concentrated. The resulting crude product was purified by normal-phase column chromatography (ethyl acetate: petroleum ether = 0% to 100%) to obtain compound **453-7** (724 mg, yield: 63%) as a white solid. MS (ESI, m/z): 344.3 $[M+H]^+$.

To a sealed tube, compound **453-7** (724 mg, 2.1 mmol), compound **156-4** (1.3 g, 6.3 mmol), tris(dibenzylideneacetone)dipalladium (92 mg, 0.1 mmol), 4,5-bis(diphenylphosphino)-9,9-dimethylxanthene (116 mg, 0.2 mmol), cesium carbonate (1.31 g, 4.2 mmol), and dioxane (8 mL) were added under a nitrogen atmosphere. The reaction mixture was stirred at 120°C for 48 hours. After the reaction was completed, water (50 mL) was added, and the reaction mixture was extracted with ethyl acetate (30 mL × 3). The organic phase was dried over anhydrous sodium sulfate, filtered, and concentrated. The resulting crude product was purified by normal-phase column chromatography (ethyl acetate: petroleum ether = 0% to 100%) to obtain compound **453-8** (822 mg, yield: 84%) as a white solid. MS (ESI, m/z): 468.3 $[M+H]^+$.

Compound **453-8** (822 mg, 1.76 mmol) was added to tetrahydrofuran (10 mL), followed by the addition of 1.5 M diisobutylaluminum hydride (2.6 mL, 3.9 mmol) at 0°C. The reaction mixture was stirred at 25°C for 24 hours. After the

reaction was completed, 1 N hydrochloric acid (5 mL) was added to quench the reaction. The reaction mixture was extracted with ethyl acetate (30 mL × 3), and the organic phase was dried over anhydrous sodium sulfate, filtered, and concentrated. The resulting crude product was purified by normal-phase column chromatography (ethyl acetate: petroleum ether = 0% to 100%) to obtain compound **453-9** (731 mg, yield: 92%) as a white solid. MS (ESI, m/z): 440.3 $[M+H]^+$.

Dess-Martin periodinane (1.06 g, 2.5 mmol) and sodium bicarbonate (630 mg, 7.5 mmol) were added to dichloromethane (20 mL). A solution of compound **453-9** (731 mg, 1.66 mmol) in dichloromethane (5 mL) was added at 0°C, and the reaction mixture was stirred at 25°C for 4 hours. After the reaction was completed, the reaction mixture was concentrated to obtain a crude product. The resulting crude product was purified by normal-phase column chromatography (ethyl acetate: petroleum ether = 0% to 100%) to obtain compound **453-10** (362 mg, yield: 50%) as a white solid. MS (ESI, m/z): 438.3 $[M+H]^+$.

Compound **453-10** (362 mg, 0.83 mmol) was added to tetrahydrofuran (10 mL). At 0°C, 1.0 M cyclopropylmagnesium bromide (1.25 mL, 1.25 mmol) was added, and the reaction mixture was stirred at 0°C for 2 hours. After the reaction was completed, the reaction mixture was quenched with 1 N hydrochloric acid (5 mL) and extracted with ethyl acetate (10 mL × 3). The organic phase was dried over anhydrous sodium sulfate, filtered, and concentrated. The resulting crude product was purified by normal-phase column chromatography (ethyl acetate: petroleum ether = 0% to 100%) to obtain compound **453-11** (327 mg, yield: 82%) as a white solid. MS (ESI, m/z): 480.3 $[M+H]^+$.

To a sealed tube, compound **453-11** (160 mg, 0.34 mmol), compound **225-5** (94 mg, 0.5 mmol), rhenium pentacarbonyl bromide (14 mg, 0.034 mmol), and toluene (2 mL) were added under a nitrogen atmosphere. The reaction mixture was reacted at 120°C for 8 hours, cooled to room temperature, added with water (10 mL), and extracted with ethyl acetate (10 mL × 3). The organic phase was dried over anhydrous sodium sulfate, filtered, and concentrated. The resulting crude product was purified by normal-phase column chromatography (ethyl acetate: petroleum ether = 0% to 100%) to obtain compound **453-12** (34 mg, yield: 29%) as a white solid. MS (ESI, m/z): 536.3 $[M+H]^+$.

Compound **453-12** (34 mg, 0.058 mmol) was added to methanol (2 mL), followed by the dropwise addition of 0.5 mL of 1.0 M sodium hydroxide aqueous solution to the reaction system. The reaction was carried out at 50°C overnight. After cooling to room temperature, water (10 mL) was added, and the pH was adjusted to 3 with dilute hydrochloric acid. The mixture was extracted with ethyl acetate (10 mL × 3), and the organic phase was dried over anhydrous sodium sulfate, filtered, and concentrated. The resulting crude product was purified by reverse-phase column chromatography [acetonitrile/water (0.05% formic acid) = 0% to 100%] to obtain compound **453** (18 mg, yield: 55%) as a white solid. MS (ESI, m/z): 522.3 $[M+H]^+$. $^1H$ NMR (400 MHz, $CDCl_3$) δ 7.69 (s, 1H), 7.25 - 7.21 (m, 2H), 7.00 - 6.86 (m, 1H), 6.58 - 6.48 (m, 1H), 6.46-6.36 (m, 1H), 4.11 (t, *J* = 8.5 Hz, 2H), 3.77 (s, 3H), 3.68 - 3.57 (m, 2H), 2.98 - 2.74 (m, 5H), 2.0 - 2.49 (m, 1H), 2.41 - 2.26 (m, 2H), 2.06 - 1.94 (m, 2H), 1.68 (t, *J* = 8.0 Hz, 2H), 1.15 - 0.99 (m, 10H), 0.66 - 0.56 (m, 1H), 0.46 - 0.31 (m, 2H), 0.27 - 0.16 (m, 1H).

## Comparative Example 1

Synthetic Route:

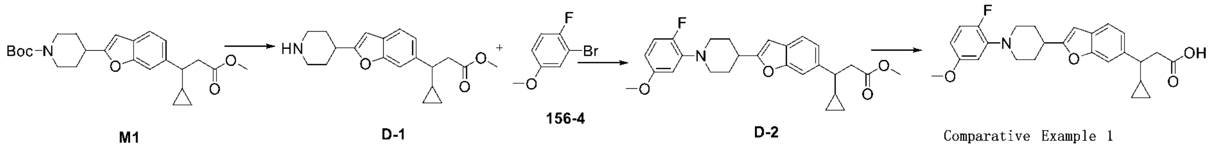

M1 D-1 156-4 D-2 Comparative Example 1

Compound **M1** (100 mg, 0.23 mmol) was added to dichloromethane (5 mL), and then trifluoroacetic acid (0.1 mL) was slowly added to the reaction mixture and stirred at room temperature for 1 hour. After the reaction was completed, the reaction mixture was directly concentrated and purified by normal-phase column chromatography (ethyl acetate: petroleum ether = 0% to 100%) to obtain compound **D-1** (13 mg, yield: 17%) as a yellow oil. MS (ESI, m/z): 328.3 $[M+H]^+$.

Under a nitrogen atmosphere, compound **D-1** (13 mg, 0.048 mmol), compound **156-4** (10 mg, 0.048 mmol), tris(dibenzylideneacetone)dipalladium (3.6 mg, 0.0048 mmol), 2-dicyclohexylphosphino-2',6'-diisopropoxybiphenyl (4.5 mg, 0.008 mmol), and cesium carbonate (26 mg, 0.08 mmol) were added to 1,4-dioxane (3 mL). The reaction mixture was heated to 100°C and stirred overnight. After the reaction was completed, the reaction mixture was directly concentrated and purified by normal-phase column chromatography (ethyl acetate: petroleum ether = 0% to 100%) to obtain compound **D-2** (10 mg, yield: 46%) as a yellow oil. MS (ESI, m/z): 452.3 $[M+H]^+$.

Compound **D-2** (10 mg, 0.022 mmol) was added to tetrahydrofuran (2 mL) and methanol (2 mL). Then, a solution of lithium hydroxide (11 mg, 0.44 mmol) in water (1 mL) was added to the reaction mixture, which was heated to 60°C and

stirred for 2 hours. After the reaction was completed, the reaction mixture was cooled to room temperature, and the pH was adjusted to 3 with 1 N hydrochloric acid. The reaction mixture was extracted with ethyl acetate (10 mL), and the organic phase was concentrated and purified by reverse-phase column chromatography [acetonitrile/water (0.05% formic acid) = 0% to 100%] to obtain **comparative example 1** (2 mg, yield: 22%) as a white solid. MS (ESI, m/z): 438.3 $[M+H]^+$.

$^1H$ NMR (400 MHz, $CDCl_3$) δ 7.43 (d, *J* = 8.4 Hz, 1H), 7.33 (s, 1H), 7.09 (d, *J* = 8.0 Hz, 1H), 6.98 - 6.93 (m, 1H), 6.58 - 6.53 (m, 1H), 6.46 - 6.41 (m, 1H), 6.39 (s, 1H), 3.79 (s, 3H), 3.56 (d, *J* = 11.6 Hz, 2H), 2.88 - 2.80 (m, 4H), 2.51 - 2.45 (m, 1H), 2.25 - 2.13 (m, 2H), 2.06 - 1.96 (m, 3H), 1.13 - 1.05 (m, 1H), 0.66 - 0.57 (m, 1H), 0.47 - 0.40 (m, 1H), 0.37 - 0.29 (m, 1H), 0.22 - 0.16 (m, 1H).

## I. *In Vitro* Activity Assay Experimental Section:

### Efficacy Test Example 1:

### Detection of GPR40 Agonistic Activity of Compounds Using NFAT-RE-Based Reporter Gene Activity Assay

### 1. Method

#### 1.1 Construction and preparation of plasmid pcDNA3.0-flag-FFAR1 (GPR40)

The pcDNA3.0-flag-FFAR1 (GPR40) plasmid was constructed using conventional molecular cloning methods. The main steps were as follows: The full-length cDNA sequence of human FFAR1 (GPR40) (NM_005303.3) was inserted into the *HindIII* and *Xba*I restriction sites of the pcDNA3.0 vector via PCR technology, resulting in the pcDNA3.0-flag-FFAR1 (GPR40) plasmid; pGL4.30 [luc2P NFAT-RE] (# E8481) and pRL-TK (#E2241) plasmids were both purchased from Promega; the plasmids were transformed into DH5α *E. coli* using the $CaCl_2$ method, further cultured and amplified, and then purified using a plasmid extraction kit (TIANGEN, # DP117) to obtain the corresponding plasmid DNA.

#### 1.2 **Co-transfection of HEK293T cells with plasmids and compound treatment**

HEK293T cells were seeded in a 96-well plate at a density of $1 \times 10^4$/well one day prior to plasmid transfection. Cell transfection was performed according to the instructions of the transfection reagent FuGENE® HD (Promega, # E2311). The main steps were as follows: Taking one well as an example, plasmids pcDNA3.0-flag-FFAR1 (GPR40), pGL4.30 [luc2P NFAT-RE], and pRL-TK were added at ratios of 50 ng, 50 ng, and 5 ng, respectively, into 10 μL of Opti-MEM™ I medium (Gibco, #11058021) and mixed thoroughly; then, 0.2 μL of FuGENE® HD was added, mixed thoroughly, and allowed to stand at room temperature for 5 minutes; finally, this 10 μL mixture was added to the cell well containing 100 μL of culture medium. 24 hours after co-transfection, the compounds were serially diluted at half-logarithmic intervals with 1 μM as the highest concentration, resulting in 10 concentration gradients, which were added to the cell culture medium for a 4-hour treatment. Two replicate wells were set up for each condition, with compounds TAK875 (previously entered Phase III clinical trials) and SCO267 (previously entered Phase I clinical trials) serving as positive controls.

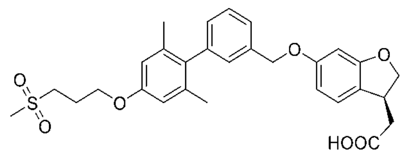

TAK-875

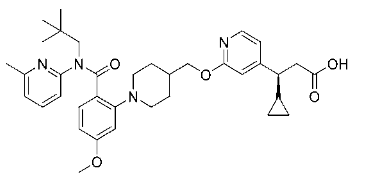

SCO-267

#### 1.3 Dual-Glo Luciferase assay

After 4 hours of compound treatment, the cells were assayed according to the instructions of the Dual-Glo® Luciferase Assay System (Promega, # E2940). The main steps were as follows: 50 μL of culture medium was aspirated from each well and discarded, followed by the addition of 50 μL of Dual-Glo® Luciferase Reagent, and the mixture was shaken at room temperature for 10 minutes. Then, 80 μL of the lysate reaction solution was transferred to a white opaque optiPlate-96 well plate, and the luminescence signal of Firefly luciferase (Firefly-Luc) was measured using an MD i3x multifunctional microplate reader. Subsequently, 40 μL of Dual-Glo® Stop & Glo® Reagent was added, and the mixture was shaken at room temperature for 10 minutes. Finally, the luminescence signal of Renilla luciferase (Renilla-Luc) was measured using the MD i3x multifunctional microplate reader. The ratio of Firefly-Luc/Renilla-Luc was used as the compound's agonistic activity toward GPR40, and normalization was performed using the ratio of the solvent DMSO

group. A dose-response curve was fitted using a four-parameter model with GraphPad Prism 8.0 software to calculate the EC50 value.

**2. Results**

The experimental data are shown in Table 1 below. Table 1

| Cpd ID | Nuclear Factor of Activated T-Cells Reporter Gene Activity Assay (NFAT reporter assay) | |
|---|---|---|
| | EC50 (μM) | Efficacy (%) |
| TAK875 | * | 65 |
| SCO-267 | *** | 100 |
| Comparative Example **1** | NA | NA |
| Compound **1** | NA | † |
| Compound **2** | * | †† |
| Compound **3** | ** | †† |
| Compound **4** | ** | † |
| Compound **5** | ** | ††† |
| Compound **6** | * | †† |
| Compound **7** | ** | †† |
| Compound **8** | * | †† |
| Compound **9** | ** | †† |
| Compound **10** | ** | †† |
| Compound **11** | *** | ††† |
| Compound **12** | *** | †† |
| Compound **13** | ** | ††† |
| Compound **14** | *** | ††† |
| Compound **15** | ** | ††† |
| Compound **20** | ** | †† |
| Compound **21** | ** | †† |
| Compound **22** | ** | †† |
| Compound **23** | ** | †† |
| Compound **24** | ** | † |
| Compound **26** | ** | †† |
| Compound **27** | ** | † |
| Compound **28** | ** | ††† |
| Compound **29** | ** | †† |
| Compound **30** | ** | †† |
| Compound **31** | ** | †† |
| Compound **32** | ** | †† |
| Compound **33** | *** | †† |
| Compound **34** | *** | †† |
| Compound **35** | ** | †† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **36** | ** | †† |
| Compound **37** | ** | †† |
| Compound **38** | *** | ††† |
| Compound **39** | ** | ††† |
| Compound **41** | ** | †† |
| Compound **43** | ** | †† |
| Compound **44** | ** | †† |
| Compound **45** | ** | †† |
| Compound **47** | ** | †† |
| Compound **48** | ** | †† |
| Compound **49** | **** | †† |
| Compound **52** | ** | † |
| Compound **53** | ** | †† |
| Compound **54** | *** | †† |
| Compound **55** | **** | †† |
| Compound **56** | ** | † |
| Compound **60** | ** | †† |
| Compound **61** | ** | †† |
| Compound **65** | ** | †† |
| Compound **66** | ** | †† |
| Compound **70** | * | †† |
| Compound **71** | ** | †† |
| Compound **79** | ** | †† |
| Compound **81** | ** | † |
| Compound **82** | ** | † |
| Compound **83** | ** | † |
| Compound **85** | ** | †† |
| Compound **86** | *** | ††† |
| Compound **87** | *** | †† |
| Compound **88** | ** | †† |
| Compound **89** | ** | †† |
| Compound **90** | ** | † |
| Compound **91** | ** | † |
| Compound **92** | ** | ††† |
| Compound **93** | ** | ††† |
| Compound **94** | ** | † |
| Compound **95** | ** | †† |
| Compound **96** | ** | ††† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **97** | *** | †† |
| Compound **99** | ** | †† |
| Compound **101** | ** | †† |
| Compound **102** | *** | †† |
| Compound **103** | *** | †† |
| Compound **104** | * | †† |
| Compound **105** | *** | †† |
| Compound **106** | *** | †† |
| Compound **107** | ** | ††† |
| Compound **108** | *** | ††† |
| Compound **109** | ** | †† |
| Compound **110** | *** | †† |
| Compound **111** | *** | ††† |
| Compound **112** | *** | †† |
| Compound **113** | **** | †† |
| Compound **114** | *** | †† |
| Compound **115** | **** | †† |
| Compound **116** | **** | †† |
| Compound **117** | *** | †† |
| Compound **118** | *** | †† |
| Compound **120** | * | †† |
| Compound **121** | * | †† |
| Compound **122** | *** | †† |
| Compound **123** | ** | †† |
| Compound **124** | *** | †† |
| Compound **125** | *** | †† |
| Compound **126** | *** | †† |
| Compound **127** | ** | ††† |
| Compound **128** | *** | †† |
| Compound **129** | *** | †† |
| Compound **130** | *** | †† |
| Compound **131** | *** | †† |
| Compound **132** | ** | ††† |
| Compound **133** | ** | † |
| Compound **134** | ** | † |
| Compound **135** | *** | †† |
| Compound **137** | *** | ††† |
| Compound **138** | ** | †† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **139** | ** | †† |
| Compound **140** | ** | ††† |
| Compound **141** | **** | ††† |
| Compound **142** | ** | †† |
| Compound **143** | *** | †† |
| Compound **144** | * | †† |
| Compound **145** | *** | †† |
| Compound **146** | ** | ††† |
| Compound **147** | *** | ††† |
| Compound **148** | ** | †† |
| Compound **149** | ** | †† |
| Compound **150** | ** | †† |
| Compound **151** | ** | †† |
| Compound **152** | ** | †† |
| Compound **153** | ** | ††† |
| Compound **154** | ** | ††† |
| Compound **155** | *** | ††† |
| Compound **156** | ** | †† |
| Compound **157** | ** | ††† |
| Compound **158** | ** | †† |
| Compound **159** | ** | †† |
| Compound **160** | ** | †† |
| Compound **161** | ** | † |
| Compound **162** | ** | ††† |
| Compound **163** | ** | †† |
| Compound **164** | ** | †† |
| Compound **165** | ** | ††† |
| Compound **166** | *** | †† |
| Compound **167** | ** | †† |
| Compound **168** | ** | ††† |
| Compound **169** | ** | ††† |
| Compound **170** | ** | †† |
| Compound **171** | ** | †† |
| Compound **172** | ** | † |
| Compound **173** | ** | ††† |
| Compound **174** | ** | †† |
| Compound **175** | * | †† |
| Compound **176** | ** | †† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **177** | ** | †† |
| Compound **178** | ** | †† |
| Compound **179** | ** | ††† |
| Compound **180** | ** | †† |
| Compound **181** | ** | †† |
| Compound **183** | ** | †† |
| Compound **184** | ** | † |
| Compound **185** | ** | †† |
| Compound **186** | ** | † |
| Compound **187** | ** | †† |
| Compound **188** | ** | †† |
| Compound **190** | ** | †† |
| Compound **191** | ** | †† |
| Compound **192** | ** | †† |
| Compound **193** | ** | †† |
| Compound **198** | ** | †† |
| Compound **200** | ** | †† |
| Compound **202** | ** | †† |
| Compound **203** | ** | † |
| Compound **205** | ** | † |
| Compound **206** | ** | †† |
| Compound **208** | ** | ††† |
| Compound **209** | ** | †† |
| Compound **210** | ** | †† |
| Compound **211** | ** | ††† |
| Compound **212** | ** | †† |
| Compound **213** | *** | †† |
| Compound **214** | ** | † |
| Compound **215** | ** | †† |
| Compound **217** | *** | †† |
| Compound **218** | ** | ††† |
| Compound **219** | *** | † |
| Compound **220** | ** | †††† |
| Compound **222** | ** | ††† |
| Compound **223** | ** | † |
| Compound **231** | ** | † |
| Compound **232** | ** | † |
| Compound **233** | ** | †† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **234** | **** | †† |
| Compound **235** | **** | †† |
| Compound **236** | *** | ††† |
| Compound **237** | *** | †† |
| Compound **238** | *** | ††† |
| Compound **239** | *** | ††† |
| Compound **240** | ** | †† |
| Compound **241** | ** | †† |
| Compound **242** | ** | †† |
| Compound **243** | * | †† |
| Compound **244** | * | †† |
| Compound **245** | ** | †† |
| Compound **246** | ** | †† |
| Compound **247** | ** | ††† |
| Compound **248** | ** | †† |
| Compound **249** | ** | †† |
| Compound **250** | ** | †† |
| Compound **251** | ** | †† |
| Compound **252** | ** | †† |
| Compound **253** | **** | ††† |
| Compound **254** | ** | ††† |
| Compound **255** | ** | †† |
| Compound **256** | ** | ††† |
| Compound **257** | **** | ††† |
| Compound **258** | ** | ††† |
| Compound **259** | * | †† |
| Compound **260** | *** | ††† |
| Compound **261** | **** | ††† |
| Compound **262** | ***** | †† |
| Compound **263** | ** | †† |
| Compound **264** | ** | †† |
| Compound **265** | **** | ††† |
| Compound **266** | ***** | †††† |
| Compound **267** | **** | ††† |
| Compound **268** | **** | †† |
| Compound **269** | *** | ††† |
| Compound **270** | *** | †††† |
| Compound **271** | **** | ††† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **272** | *** | ††† |
| Compound **273** | **** | †† |
| Compound **274** | ** | †† |
| Compound **275** | *** | ††† |
| Compound **276** | *** | †† |
| Compound **277** | * | †††† |
| Compound **278** | ***** | †††† |
| Compound **279** | * | †† |
| Compound **280** | ** | †† |
| Compound **281** | ** | †† |
| Compound **282** | *** | †† |
| Compound **283** | *** | ††† |
| Compound **284** | **** | †† |
| Compound **285** | **** | †† |
| Compound **286** | ** | †† |
| Compound **288** | ** | †† |
| Compound **290** | ** | †† |
| Compound **293** | ** | †† |
| Compound **296** | ** | ††† |
| Compound **297** | ** | †† |
| Compound **298** | ** | †† |
| Compound **299** | ** | †† |
| Compound **300** | ** | †† |
| Compound **301** | ** | †† |
| Compound **302** | ** | † |
| Compound **304** | ** | ††† |
| Compound **305** | ** | †† |
| Compound **306** | ** | †† |
| Compound **307** | ** | †† |
| Compound **308** | * | †† |
| Compound **309** | ** | †† |
| Compound **310** | ** | †† |
| Compound **312** | ** | † |
| Compound **314** | ** | †† |
| Compound **315** | ** | †† |
| Compound **316** | ** | †† |
| Compound **317** | ** | † |
| Compound **319** | ** | †† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **320** | *** | †† |
| Compound **321** | ** | †† |
| Compound **322** | **** | †† |
| Compound **323** | **** | †† |
| Compound **324** | **** | †† |
| Compound **325** | ** | †† |
| Compound **326** | ** | †† |
| Compound **327** | ** | †† |
| Compound **328** | * | †† |
| Compound **330** | *** | †† |
| Compound **331** | ** | †† |
| Compound **332** | **** | †† |
| Compound **333** | **** | †† |
| Compound **334** | **** | †† |
| Compound **335** | **** | †† |
| Compound **336** | **** | †† |
| Compound **337** | **** | †† |
| Compound **338** | *** | †† |
| Compound **339** | **** | †† |
| Compound **340** | ***** | ††† |
| Compound **341** | **** | †† |
| Compound **342** | **** | †† |
| Compound **343** | **** | ††† |
| Compound **344** | ** | †† |
| Compound **345** | ** | †† |
| Compound **346** | ** | †† |
| Compound **347** | *** | †† |
| Compound **348** | ** | †† |
| Compound **349** | ** | †† |
| Compound **351** | *** | †† |
| Compound **352** | *** | †† |
| Compound **353** | ** | ††† |
| Compound **354** | ** | †† |
| Compound **355** | ** | †† |
| Compound **358** | ** | † |
| Compound **359** | ** | † |
| Compound **360** | * | ††† |
| Compound **361** | *** | †† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **362** | ** | †† |
| Compound **363** | ** | †† |
| Compound **364** | ** | †† |
| Compound **365** | *** | ††† |
| Compound **366** | *** | †† |
| Compound **367** | **** | †† |
| Compound **368** | ** | ††† |
| Compound **369** | ** | † |
| Compound **370** | ** | †† |
| Compound **371** | ** | †† |
| Compound **372** | ** | †† |
| Compound **373** | *** | †† |
| Compound **374** | *** | †† |
| Compound **375** | ** | †† |
| Compound **376** | *** | †† |
| Compound **377** | *** | †† |
| Compound **379** | ** | †† |
| Compound **380** | * | †† |
| Compound **383** | ** | ††† |
| Compound **384** | ** | †† |
| Compound **385** | *** | †† |
| Compound **386** | *** | †† |
| Compound **387** | *** | †† |
| Compound **388** | ** | †† |
| Compound **389** | ** | †† |
| Compound **390** | ** | †† |
| Compound **391** | ** | †† |
| Compound **393** | ** | †† |
| Compound **394** | * | †† |
| Compound **395** | ** | † |
| Compound **398** | ** | ††† |
| Compound **399** | ** | †† |
| Compound **400** | ** | † |
| Compound **401** | ** | ††† |
| Compound **402** | ** | † |
| Compound **404** | ** | †† |
| Compound **405** | ** | † |
| Compound **406** | ** | ††† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **407** | ** | ††† |
| Compound **408** | ** | ††† |
| Compound **409** | *** | ††† |
| Compound **410** | ** | ††† |
| Compound **411** | *** | †† |
| Compound **412** | ** | ††† |
| Compound **413** | ** | ††† |
| Compound **414** | * | ††† |
| Compound **415** | * | ††† |
| Compound **416** | *** | †† |
| Compound **417** | ** | ††† |
| Compound **418** | *** | ††† |
| Compound **419** | *** | †††† |
| Compound **420** | ** | †† |
| Compound **421** | ** | ††† |
| Compound **423** | ** | † |
| Compound **424** | * | †† |
| Compound **425** | ** | †† |
| Compound **426** | *** | ††† |
| Compound **427** | ** | ††† |
| Compound **428** | ** | †† |
| Compound **429** | ** | ††† |
| Compound **430** | ** | ††† |
| Compound **431** | ** | †† |
| Compound **432** | *** | ††† |
| Compound **435** | *** | †† |
| Compound **436** | * | †† |
| Compound **438** | *** | †† |
| Compound **439** | ** | †† |
| Compound **440** | *** | ††† |
| Compound **441** | ** | †† |
| Compound **442** | ** | †† |
| Compound **447** | ** | †† |
| Compound **448** | ** | †† |
| Compound **450** | ** | † |
| Compound **451** | *** | †† |

(continued)

| Cpd ID | Nuclear Factor of Activated T-Cells Reporter Gene Activity Assay (NFAT reporter assay) | |
|---|---|---|
| | EC50 (μM) | Efficacy (%) |
| Compound **452** | *** | †† |
| *: EC50 > 0.5 μM; **: 0.5 μM ≥ EC50 > 0.1 μM; ***: 0.1 μM ≥ EC50 > 0.01 μM; ****: 0.01 μM ≥ EC50; NA indicates no activity.<br>†: 100 ≥ Efficacy (%) > 50; ††: 150 ≥ Efficacy (%) > 100; ††† : Efficacy (%) > 150.<br>Conclusion: The compounds of the present disclosure generally have good GPR40 activity. | | |

**II. *In Vivo* Pharmacodynamic Assay Section:**

**Efficacy Test Example 1: Effect of Compounds of the Present Disclosure on Oral Glucose Tolerance Test (OGTT) in Normal C57BL6/J Mice**

1. Experimental method

Eighteen normal male C57BL6/J mice (8-9 weeks old) were selected and randomly divided into 3 groups based on body weight and blood glucose levels, with 6 mice in each group. On the day of the OGTT, fasting was initiated at 8:30 AM. At 11:30 AM, drug interventions were administered via gavage (vehicle control, compound **14,** 1 mg/kg, and compound **169,** 1 mg/kg). At 1:30 PM, glucose (2 g/kg) was administered via gavage, while the blank control group received the vehicle control orally (2% DMSO + 15% solutol + 83% DDW solution) at a dosing volume of 10 mL/kg. Blood glucose measurements during the OGTT were taken at the following time points: 0, 15, 30, 60, 90, and 120 minutes after glucose administration.

2. Observation indicators and calculations

2.1 Blood glucose measurement

Blood glucose levels were measured at 0, 15, 30, 60, and 120 minutes after glucose administration. The area under the curve (AUC) for blood glucose over 120 minutes was calculated.

AUC (mmol/L·h) = (BG0 + BG15) × 0.25/2 + (BG15 + BG30) × 0.25/2 + (BG30 + BG60) × 0.5/2 + (BG60 + BG90) × 0.5/2 + (BG90 + BG120) × 0.5/2

Note: BG0, BG15, BG30, BG60, BG90, and BG120 represent the blood glucose levels at 0, 15, 30, 60, 90, and 120 minutes after glucose administration, respectively.

3. Data processing and statistical analysis

Data were expressed as mean ± standard deviation. One-way ANOVA was employed for statistical analysis, with $p < 0.05$ considered statistically significant.

4. The experimental results are shown in Table 2.

Table 2: Blood Glucose Levels at Each Time Point and $AUC_{0-120}$ minutes

| Group | | | Blank Control Group | Compound **14** | Compound **169** |
|---|---|---|---|---|---|
| Blood Glucose (mmol/L) | 0 | 7.58±0.59* | 8.92±1.32 | 7.58±0.59* | 7.88±0.62 |
| | 15 | 11.08±2.04**** | 21.03±2.44 | 11.08±2.04**** | 12.78±1.65**** |
| | 30 | 9.17±1.37**** | 14.33±1.54 | 9.17±1.37**** | 9.28±1.32**** |
| | 60 | 7.6±1.34**** | 15.17±1.4 | 7.6±1.34**** | 9.28±1.32**** |
| | 90 | 6.52±2* | 10.08±2.53 | 6.52±2* | 7.67±2.31 |
| | 120 | 5.87±1.13** | 10.67±2.9 | 5.87±1.13** | 7.13±1.25* |
| AUC | 0-120 minutes | 941±62.00**** | 1622±84.00 | 941±62.00**** | 1075±66.01 **** |
| Note: One-way ANOVA, *$p$ < 0.05, **$p$ < 0.01, ***$p$ < 0.001, ****$p$ < 0.0001. | | | | | |

The effect of a single administration of the compound **14** and compound **169** of the present disclosure on oral glucose tolerance in normal C57BL6/J mice was investigated. As shown in Table 2, compound **14** and compound **169** significantly reduced the $AUC_{0-120}$ minutes after a single administration at the dose of 1 mg/kg. Compared with the blank control, compound **14** and compound **169** at the dose of 1 mg/kg were observed to lower blood glucose levels and $AUC_{0-120}$ minutes at all time points.

In summary, compound **14** and compound **169** of the present disclosure can significantly reduce oral glucose tolerance in normal C57BL6/J mice after a single administration at the dose of 1 mg/kg, demonstrating good hypoglycemic effects.

**Efficacy Test Example 2: Effect of Compounds of the Present Disclosure on Oral Glucose Tolerance Test (OGTT) in High-Fat Diet-Induced DIO Mice**

1. Experimental method

Normal male C57BL6/J mice (8-9 weeks old) were housed 3-4 per cage and fed a high-fat, high-cholesterol, high-fructose diet (D09100310, Research Diets) for 13 weeks with unrestricted access to water. After 13 weeks of high-fat, high-cholesterol, high-fructose diet feeding, the mice were randomly divided into 5 groups based on body weight and blood glucose levels, with age-matched mice fed a normal diet serving as the normal blank control group, with 6 mice in each group. Under fasting conditions (16 hours), the test compound **169** was administered via oral gavage at different concentrations of 0.03, 0.1, 0.6, and 3 mg/kg, while the normal blank control and model control groups were given the vehicle (15% solutol + 85% DDW solution), with a dosing volume of 10 mL/kg. On the day of administration, blood glucose levels in mice were measured. 2 hours after oral gavage administration, each group was given 2 g/kg of glucose orally. Blood glucose levels were measured at 0, 15, 30, 60, 90, 120, and 150 minutes after glucose administration.

2. Observation indicators and calculations

2.1 Blood glucose measurement

Blood glucose levels were measured at 0, 15, 30, 60, and 120 minutes after glucose administration. The area under the curve (AUC) for blood glucose over 120 minutes was calculated.

AUC (mmol/L·h) = (BG0 + BG15) × 0.25/2 + (BG15 + BG30) × 0.25/2 + (BG30 + BG60) × 0.5/2 + (BG60 + BG90) × 0.5/2 + (BG90 + BG120) × 0.5/2

Note: BG0, BG15, BG30, BG60, BG90, and BG120 represent the blood glucose levels at 0, 15, 30, 60, 90, and 120 minutes after glucose administration, respectively.

3. Data processing and statistical analysis

Data were expressed as mean ± standard deviation. One-way ANOVA was employed for statistical analysis, with p < 0.05 considered statistically significant.

4. The experimental results are shown in Table 3.

Table 3: Blood Glucose Levels at Each Time Point and $AUC_{0-150}$ minutes

| Group | | Normal Blank Control Group | Model Control Group | 0.03 mg/kg | 0.1 mg/kg | 0.6 mg/kg | 3 mg/kg |
|---|---|---|---|---|---|---|---|
| Blood Glucose (mmol/L) | 0 minutes | 8.68±1.15 | 10.2±1.23 | 10.78±0.81 | 9.28±2.89 | 9.57±2.06 | 13.02±2.46 |
| | 15 minutes | 21.9±2.22 | 22.2±2.39 | 20.08±2.94 | 17.65±2.09 ** | 16.48±2.15** * | 17.22±2.02 ** |
| | 30 minutes | 17.77±1.78 | 16.63±2.38 | 16.63±3.66 | 18.67±4.11 | 12.97±3.71 | 14±3.49 |
| | 60 minutes | 12.23±0.78 | 12.97±2.76 | 14.62±3.62 | 13.53±3.03 | 11.98±2.95 | 10.52±2.23 |
| | 90 minutes | 10.42±1.22 * | 13.92±3.41 | 14.92±2.34 | 10.27±1.68 * | 11.27±2.66 | 9.88±0.85* |
| | 120 minutes | 8.38±0.49 | 11.181±3.48 | 13±3.0 5 | 10.05±1.47 | 9.12±2.14 | 8.3±1.63 |
| | 150 minutes | 8.08±0.82* | 10.67±2.26 | 10.75±1.1 | 10.17±1.68 | 9.52±1.62 | 7.37±1.44* * |
| $AUC_{0-150\ min}$ (mmol*-min) | | 1846±52.00 ** | 2094±133.0 0 | 2194±132.70 | 1922±113.0 0* | 1725±119.90 **** | 1642±91.83 **** |
| One-way ANOVA, compared with the model control group, *$p$ < 0.05, **$p$ < 0.01, ***$p$ < 0.001, ****$p$ < 0.0001. | | | | | | | |

The effect of a single administration of the compound **169** of the present disclosure at doses of 0.03, 0.1, 0.6, and 3 mg/kg on oral glucose tolerance in a high-fat diet-induced DIO mouse model was investigated. As shown in Table 3, compound **169** significantly reduced the $AUC_{0-150}$ minutes at doses of 0.1, 0.6, and 3 mg/kg, exhibiting a dose-dependent effect.

In summary, a single administration of compound **169** of the present disclosure can significantly reduce the oral glucose tolerance of DIO mice, showing a good hypoglycemic effect.

**Efficacy Test Example 3: *In Vivo* Hypoglycemia Risk Assay**

1. Experimental method

Twenty-four normal male C57BL6/J mice (8-9 weeks old) were selected and randomly divided into 4 groups based on body weight and blood glucose levels, with 6 mice in each group. Under fasting conditions (16 hours), compound **169** was orally administered at doses of 3 and 10 mg/kg, while the positive control drug Glibenclamide was given at 10 mg/kg. The blank control group was orally administered with the vehicle control (2% DMO + 15% solutol + 83% DDW solution), with a dosing volume of 10 mL/kg. Blood glucose levels were measured at 0, 30, 60, 120, and 180 minutes after administration on the day of dosing.

2. Observation indicators and calculations

2.1 Blood glucose measurement

Blood glucose levels were measured at 0, 30, 60, 120, and 180 minutes after administration. The area under the blood glucose curve (AUC) over 180 minutes was calculated.

$$\text{AUC (mmol/L·h)} = (BG0 + BG30) \times 0.5/2 + (BG30 + BG60) \times 0.5/2 + (BG60 + BG120) \times 1/2 + (BG120 + BG180) \times 1/2$$

Note: BG0, BG30, BG60, BG120, and BG180 represent blood glucose levels at 0, 30, 60, 120, and 180 minutes after administration, respectively.

3. Data processing and statistical analysis

Data were expressed as mean ± standard deviation. One-way ANOVA was employed for statistical analysis, with $p < 0.05$ considered statistically significant.

4. The experimental results are shown in Table 4.

Table 4: Blood Glucose Levels at Each Time Point and $AUC_{0-120}$ minutes

| Group | Blood Glucose (mmol/L) | | | | $AUC_{0-120}$ minutes (mmol*min) |
|---|---|---|---|---|---|
| | 0 minutes | 30 minutes | 60 minutes | 120 minutes | |
| Blank Control Group | 4.181±1.13 | 5.48±0.77 | 5.13±0.97 | 4.22±0.88 | 589±48.00 |
| Glibenclamide, 10 mg/kg | 4.47±0.85 | 4.57±0.92 | 4.18±0.94 | 3.32±1.05 | 492±50.30** |
| Compound 169, 3 mg/kg | 4.3±0.59 | 5.42±0.56 | 4.73±0.82 | 4.1±0.51 | 563±34.91 |
| Compound **169,** 10 mg/kg | 4.65±0.75 | 6.1±1.01 | 4.92±1.01 | 4±0.87 | 594±49.00 |

Note: One-way ANOVA, $^*p < 0.05$, $^{**}p < 0.01$, $^{***}p < 0.001$, $^{****}p < 0.0001$.

As shown in Table 4, compared to the blank control, the $AUC_{0-120\,min}$ data of Glibenclamide at 10 mg/kg indicated a risk of hypoglycemia; the compound of the present disclosure **169** at both 3 mg/kg and 10 mg/kg did not exhibit hypoglycemia, demonstrating no risk of hypoglycemia.

**Efficacy Test Example 4: *In Vivo* Pharmacodynamic Assay for Non-Alcoholic Fatty Liver Disease**

1. Experimental method

Normal male C57BL6/J mice (7-5 weeks old) were fed a high-fat, high-cholesterol, high-fructose diet (D09100310, Research Diets) for 20 weeks. Based on body weight and biochemical data, the mice were randomly divided into groups, with 6 mice per group. The compound **169** (0.03, 0.1, and 0.6 mg/kg) was administered via oral gavage. The positive control was MGL3196 (1 mg/kg), and the blank control group consisted of mice of the same strain and age fed a normal diet for the same duration. The blank control and model control groups were given the vehicle (15% solutol + 85% DDW), totaling 6 groups. Administration was performed once daily for 28 days. After overnight fasting, on day 29 at the experimental endpoint, blood was collected from the heart after CO2 euthanasia. Serum ALT and AST biochemical indicators were measured, and liver tissue was harvested for hepatic hydroxyproline content determination.

2. Measurement of blood biochemical ALT, AST, and hepatic hydroxyproline content

Blood was collected using anticoagulant-free tubes, allowed to stand at room temperature for 30 minutes, and then centrifuged at 4000 rpm for 10 minutes to separate serum, which was tested by Dian Diagnostics. Approximately 50 mg of liver tissue was taken from each mouse and tested according to the hydroxyproline assay kit method (Nanjing Jiancheng, Cat. No.: A030-2-1).

3. Data processing and statistical analysis

Data were expressed as mean ± standard deviation. One-way ANOVA was employed for statistical analysis, with $p < 0.05$ considered statistically significant.

4. The experimental results are shown in Table 5.

Table 5: Blood Biochemistry and Hepatic Hydroxyproline Content

| Group | ALT (U/L) | AST (U/L) | Hepatic Hydroxyproline (μg/g) | Total Liver Hydroxyproline (μg/liver) |
|---|---|---|---|---|
| Blank Control | 34±19.32 | 84±32.42 | 91.95±27.41 | 101.97±28.92 |
| Model Control | 259.5 ±137.71 | 308±170.34 | 236.37±87.34 | 559.7±260.05 |
| MGL3196, 1 mpk | 140±63.06 | 242±67.42 | 173.63±32.06 | 313.58±98.24* |
| Compound **169,** 0.03 mpk | 148±81.95 | 204.5 ±79.09 | 128.06±55.18** | 308.56±121.44* |
| Compound **169,** 0.1 mpk | 110±37.12* | 156±30.59* | 119.16±39.65** | 266.4±80.67** |
| Compound **169,** 0.6 mpk | 103.8±35.53* | 163.2 ±42.98 | 85.24±47.63*** | 187.94±110.56*** |
| Note: One-way ANOVA, $*p < 0.05$, $**p < 0.01$. | | | | |

As shown in Table 5, compared with the model control, compound **169** at doses of 0.03, 0.1, and 0.6 mg/kg reduced ALT, AST, and liver hydroxyproline levels, indicating that compound **169** has liver protection and liver fibrosis inhibition effects.

**III. Pharmacokinetic Evaluation**

The bioavailability and pharmacokinetic behavior of the compounds were evaluated in mice. 6 male ICR mice with similar body weights were selected, among which 3 mice were administered a single gavage dose of 10 mg/kg, and the other 3 mice were intravenously administered a single dose of 5 mg/kg. Blood samples were collected at 5 minutes (intravenous administration), 15 minutes, 30 minutes, 1 hour, 2 hours, 4 hours, and 7 hours after administration. The plasma samples were analyzed for concentration by LCMS/MS, and the pharmacokinetic parameters of the compounds were analyzed using the PKSolver free tool and non-compartmental analysis (NCA) software.

**Experimental protocol:**

**Experimental animals:** Each compound test group included 6 healthy male ICR mice, weighing 18-25 g, purchased from Charles River, which were randomly divided into 2 groups with 3 mice each.

**Preparation of formulations:** A certain amount of the compound was weighed and added to 2% DMSO + 15% Solutol + 83% physiological saline to form a clear solution.

**Dosage:** ICR mice were fasted overnight and administered the compound at a dose of 10 mg/kg via gavage or 5 mg/kg via intravenous injection. The dosing volumes for gavage and intravenous administration were 10 mL/kg and 5 mL/kg, respectively. Uniform feeding was conducted 2 hours after administration.

**Sample collection:** Approximately 30 μL of blood was collected from the great saphenous vein at 5 minutes (intravenous administration), 15 minutes, 30 minutes, 1 hour, 2 hours, 4 hours, and 7 hours after administration. The blood was placed into commercially available tubes containing $K_2$-EDTA. The blood samples were then centrifuged at 4°C and 4600 rpm for 5 minutes to obtain plasma samples. All plasma samples were rapidly frozen on dry ice and maintained at -70°C until LCMS/MS analysis was performed.

**Sample preparation:** 10 μL of plasma sample was aspirated and precipitated with a methanol solution containing 50 nmol/L of α-naphthoflavone (internal standard). The mixture was thoroughly mixed and centrifuged at 4°C and 14000 rpm for 5 minutes. Then, 75 μL of the supernatant was mixed with 75 μL of methanol for LCMS/MS analysis.

The pharmacokinetic parameter results are shown in Table 6.

Table 6

| Pharmacokinetic Parameters of Mice | | | | | | | |
|---|---|---|---|---|---|---|---|
| | | Intravenous Administration (5 mg/kg) | | | Gavage Administration (10 mg/kg) | | |
| Compound | F (%) | $T_{1/2}$ (h) | $CL_{_obs}$ (mL/min/kg) | $Vss_{_obs}$ (mL/kg) | $T_{max}$ (h) | $C_{max}$ (ng/mL ) | $AUC_{0\text{-}7\ h}$ (h*ng/mL ) |
| Compound **12** | 44.6 | 5.02 | 1.9 | 520 | 2 | 6575 | 38020 |
| Compound **38** | 77.4 | 2.41 | 2.3 | 405 | 2 | 8596 | 55433 |
| Compound **89** | 73.7 | 5.46 | 1.3 | 546 | 2 | 11059 | 92234 |
| Compound **166** | 48.0 | 6.7 | 1.6 | 668 | 4 | 4948 | 48126 |
| Compound **168** | 24.0 | 3.9 | 2.1 | 494 | 3 | 3470 | 15359 |

Conclusion: These compounds are well absorbed in mice, with slow elimination, high exposure, and relatively high bioavailability, which can be used for further study.

**IV. Tissue Distribution Evaluation**

**Experimental protocol:** The tissue distribution characteristics of the compounds were evaluated in mice. 2 male ICR mice with similar body weights were selected, and the compound was administered via oral gavage at a dose of 5 mg/kg. The animals were sacrificed at 1 hour, and blood, liver, and heart were collected. After sample processing, the concentration of the compounds in each matrix was analyzed by LCMS/MS, and the average values of each group were calculated to evaluate the tissue/plasma ratio of the compounds.

**Experimental animals:** 2 healthy male ICR mice were purchased from Charles River.

**Preparation of formulations:** A certain amount of the compound was weighed and added to 2% DMSO + 15% Solutol + 83% distilled water to form a clear solution.

**Dosage:** ICR mice were fasted overnight and administered the compound via oral gavage at a dose of 5 mg/kg. The volume of oral gavage administration was 10 mL/kg. Uniform feeding was conducted 2 hours after administration.

**Sample collection:** Animals were sacrificed 1 hour after administration, and blood, liver, and heart tissues were collected. The blood was placed into commercially available tubes containing $K_2$-EDTA. The blood samples were then centrifuged at 4°C and 4600 rpm for 5 minutes to obtain plasma samples. All plasma and tissue samples were rapidly frozen on dry ice and maintained at -70°C until LCMS/MS analysis was performed.

**Sample preparation:** 50 μL of the plasma sample was aspirated, precipitated with methanol, thoroughly mixed, and centrifuged at 4°C and 14000 rpm for 5 minutes. Then, 75 μL of the supernatant was mixed with 75 μL of methanol for LCMS/MS analysis. Tissue samples were homogenized using methanol. After homogenization, the mixture was centrifuged at 14,000 rpm for 5 minutes at 4°C. Subsequently, 75 μL of the supernatant was mixed with 75 μL of methanol for LCMS/MS analysis.

The tissue distribution results are shown in Table 7.

Table 7

| Compound | Plasma Concentration (ng/mL) or Tissue Concentration (ng/g) | | | |
|---|---|---|---|---|
| | Plasma | Blood | Liver | Heart |
| Compound **165** | 1290 | 842 | 1548 | 418 |
| Compound **168** | 1550 | 936 | 1923 | 485 |

Conclusion: These compounds are not enriched in liver tissue, and it is speculated that the likelihood of the compounds causing hepatotoxicity is low, which can be used for further study.

## Claims

**1.** A compound of formula I or a pharmaceutically acceptable salt thereof:

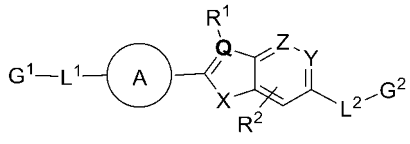

I

wherein X is O or $NR^3$, and $R^3$ is H or $C_1$-$C_6$ alkyl; Q is C or N;

Z and Y are independently C or N;

$G^1$ is H, $C_1$-$C_6$ alkyl, $C_1$-$C_6$ alkyl substituted by one or more $G^{1-1}$, $C_6$-$C_{14}$ aryl, $C_6$-$C_{14}$ aryl substituted by one or more $G^{1-2}$, 5- to 10-membered heteroaryl, 5- to 10-membered heteroaryl substituted by one or more $G^{1-3}$, $C_1$-$C_6$ alkoxy, $C_1$-$C_6$ alkoxy substituted by one or more $G^{1-4}$, $C_2$-$C_6$ alkenyl, $C_2$-$C_6$ alkenyl substituted by one or more $G^{1-5}$, $C_2$-$C_6$ alkynyl, $C_2$-$C_6$ alkynyl substituted by one or more $G^{1-6}$, $C_3$-$C_8$ cycloalkyl, $C_3$-$C_8$ cycloalkyl substituted by one or more $G^{1-7}$, $C_3$-$C_8$ cycloalkenyl, $C_3$-$C_8$ cycloalkenyl substituted by one or more $G^{1-8}$, 3- to 8-membered heterocycloalkyl, 3- to 8-membered heterocycloalkyl substituted by one or more $G^{1-9}$, 3- to 8-membered heterocycloalkenyl, or 3- to 8-membered heterocycloalkenyl substituted by one or more $G^{1-10}$;

the 5- to 10-membered heteroaryl, the 5- to 10-membered heteroaryl substituted by one or more $G^{1-3}$, the 3- to 8-membered heterocycloalkyl, the 3- to 8-membered heterocycloalkyl substituted by one or more $G^{1-9}$, the 3- to 8-membered heterocycloalkenyl, and the 3- to 8-membered heterocycloalkenyl substituted by one or more $G^{1-10}$ have 1, 2, 3, or 4 heteroatoms selected from one or more types of N, S, and O;

each $G^{1-1}$ , each $G^{1-2}$ , each $G^{1-3}$, each $G^{1-4}$, each $G^{1-5}$, each $G^{1-6}$, each $G^{1-7}$, each $G^{1-8}$, each $G^{1-9}$, and each $G^{1-10}$ is independently deuterium, halogen, cyano, -$NG^{1-1-1}G^{1-1-2}$, -NC(=O)$G^{1-1-3}G^{1-1-4}$, hydroxyl, -$S(=O)_2$-$C_1$-$C_6$ alkyl, $C_1$-$C_6$ alkyl, $C_1$-$C_6$ alkyl substituted by one or more $G^{1-1-5}$, $C_1$-$C_6$ alkoxy, $C_1$-$C_6$ alkoxy substituted by one or more $G^{1-1-6}$, -S-$C_1$-$C_6$ alkyl, -S-$C_1$-$C_6$ alkyl substituted by one or more $G^{1-1-7}$, $C_3$-$C_8$ cycloalkyl, $C_3$-$C_8$ cycloalkyl substituted by one or more $G^{1-1-8}$, -O-$C_3$-$C_8$ cycloalkyl, -O-$C_3$-$C_8$ cycloalkyl substituted by one or more $G^{1-1-9}$, or - C(=O)$NG^{1-1-11}G^{1-1-12}$;

alternatively, any two adjacent $G^{1-2}$, together with the carbon atom to which they are attached, form a 3- to 8-membered heterocycloalkyl, a 3- to 8-membered heterocycloalkyl substituted by one or more $G^{1-1-9}$, a $C_3$-$C_8$ cycloalkyl, or a $C_3$-$C_8$ cycloalkyl substituted by one or more $G^{1-1-10}$;

$G^{1-1-1}$, $G^{1-1-2}$, $G^{1-1-3}$, $G^{1-1-4}$, $G^{1-1-11}$, and $G^{1-1-12}$ are independently H, $C_1$-$C_6$ alkyl, $C_3$-$C_8$ cycloalkyl, 5- to 10-membered heteroaryl, 5- to 10-membered heteroaryl substituted by one or more $G^{1-1-10-1}$, $C_6$-$C_{14}$ aryl, or $C_6$-$C_{14}$ aryl substituted by one or more $G^{1-1-10-2}$;

each $G^{1-1-10-1}$ and each $G^{1-1-10-2}$ is independently $C_1$-$C_6$ alkyl;

each $G^{1-1-5}$, each $G^{1-1-6}$, each $G^{1-1-7}$, each $G^{1-1-8}$, each $G^{1-1-9}$, and each $G^{1-1-10}$ is independently halogen, oxo, $C_1$-$C_6$ alkyl, $C_3$-$C_8$ cycloalkyl, 5- to 10-membered heteroaryl, or 5- to 10-membered heteroaryl substituted by one or more $C_1$-$C_6$ alkyl groups;

the 3- to 8-membered heterocycloalkyl, the 3- to 8-membered heterocycloalkyl substituted by one or more $G^{1-1-9}$, the 5- to 10-membered heteroaryl, the 5- to 10-membered heteroaryl substituted by one or more $G^{1-1-10-1}$, and the 5- to 10-membered heteroaryl substituted by one or more $C_1$-$C_6$ alkyl groups have 1, 2, 3, or 4 heteroatoms independently selected from one or more types of N, S, and O;

$L^1$ is a bond or $C_1$-$C_6$ alkylene;

ring A is $C_4$-$C_6$ cycloalkyl, $C_4$-$C_6$ cycloalkyl substituted by one or more $A^1$, $C_4$-$C_6$ cycloalkenyl, $C_4$-$C_6$ cycloalkenyl substituted by one or more $A^2$, 4- to 8-membered heterocycloalkyl, 4- to 8-membered heterocycloalkyl substituted by one or more $A^3$, 4- to 6-membered heterocycloalkenyl, or 4- to 6-membered heterocycloalkenyl substituted by one or more $A^4$;

the 4- to 8-membered heterocycloalkyl, the 4- to 8-membered heterocycloalkyl substituted by one or more $A^3$, the 4- to 6-membered heterocycloalkenyl, and the 4- to 6-membered heterocycloalkenyl substituted by one or more $A^4$ have 1 or 2 heteroatoms independently selected from one or more types of N, S, and O;

each $A^1$, each $A^2$, each $A^3$, and each $A^4$ is independently deuterium, halogen, cyano, -$NA^{1-1}A^{1-2}$, -NC(=O) $A^{1-3}A^{1-4}$, hydroxyl, $C_1$-$C_6$ alkyl, $C_1$-$C_6$ alkyl substituted by one or more $A^{1-5}$, $C_1$-$C_6$ alkoxy, or $C_1$-$C_6$ alkoxy substituted by one or more $A^{1-6}$.

$A^{1-1}$, $A^{1-2}$, $A^{1-3}$ , and $A^{1-4}$ are independently deuterium, halogen, cyano, $C_1$-$C_6$ alkyl, or $C_1$-$C_6$ alkoxy;

each $A^{1-5}$ and each $A^{1-6}$ is independently hydrogen, deuterium, halogen, cyano, $C_1$-$C_6$ alkyl, or $C_1$-$C_6$ alkoxy;

$R^1$ is -C(=O)$NR^{1-1}R^{1-2}$, $C_1$-$C_6$ alkyl, $C_1$-$C_6$ alkyl substituted by one or more $R^{1-3}$, $C_2$-$C_6$ alkenyl, $C_2$-$C_6$ alkenyl substituted by one or more $R^{1-10}$, -C(=O)$R^{1-11}$, or ring B;

$R^{1-1}$, $R^{1-2}$, and $R^{1-11}$ are independently H, -$S(=O)_2C_1$-$C_{12}$ alkyl, $C_1$-$C_{12}$ alkyl, $C_1$-$C_{12}$ alkyl substituted by one or more $R^{1-1-1}$, $C_3$-$C_{12}$ cycloalkyl, $C_3$-$C_{12}$ cycloalkyl substituted by one or more $R^{1-1-2}$, $C_6$-$C_{14}$ aryl, $C_6$-$C_{14}$ aryl substituted by one or more $R^{1-1-3}$, 5- to 14-membered heteroaryl, or 5- to 14-membered heteroaryl substituted by

one or more $R^{1-1-4}$,

alternatively, $R^{1-1}$ and $R^{1-2}$, together with the N atom to which they are attached, form a 3-to 14-membered heterocycloalkyl or a 3- to 14-membered heterocycloalkyl substituted by one or more $R^{1-1-5}$;

each $R^{1-1-1}$, each $R^{1-1-2}$, each $R^{1-1-3}$, $R^{1-1-4}$, and each $R^{1-1-5}$ is independently halogen, cyano, nitro, hydroxyl, amino, -NH($C_1$-$C_{12}$ alkyl), -N($C_1$-$C_{12}$ alkyl)$_2$, -C(=O)-$C_1$-$C_{12}$ alkyl, - NHC(=O)-$C_1$-$C_{12}$ alkyl, $C_1$-$C_{12}$ alkyl, $C_1$-$C_{12}$ alkyl substituted by one or more $R^{1-1-1-1}$, $C_1$-$C_{12}$ alkoxy, $C_1$-$C_{12}$ alkoxy substituted by one or more $R^{1-1-1-2}$, $C_3$-$C_{12}$ cycloalkyl, $C_3$-$C_{12}$ cycloalkyl substituted by one or more $R^{1-1-1-3}$, $C_6$-$C_{14}$ aryl, 3- to 12-membered heterocycloalkyl, or 5- to 14-membered heteroaryl;

each $R^{1-1-1-1}$, each $R^{1-1-1-2}$, and each $R^{1-1-1-3}$ is independently halogen, $C_1$-$C_{12}$ alkyl, $C_3$-$C_{12}$ cycloalkyl, or $C_1$-$C_{12}$ alkoxy;

each $R^{1-3}$ and each $R^{1-10}$ is independently deuterium, halogen, cyano, hydroxyl, -$NR^{1-3-1}R^{1-3-2}$, -C(-O) $NR^{1-3-3}R^{1-3-4}$, -C(=O)$R^{1-3-5}$, -S(=O)$_2$-$C_1$-$C_{12}$ alkyl, -S-$C_1$-$C_{12}$ alkyl, $C_1$-$C_{12}$ alkyl, $C_1$-$C_{12}$ alkyl substituted by one or more $R^{1-3-6}$, $C_1$-$C_{12}$ alkoxy, $C_1$-$C_{12}$ alkoxy substituted by one or more $R^{1-3-7}$, 5- to 14-membered heteroaryl, 5- to 14-membered heteroaryl substituted by one or more $R^{1-3-8}$, $C_3$-$C_{12}$ cycloalkyl, $C_3$-$C_{12}$ cycloalkyl substituted by one or more $R^{1-3-9}$, $C_2$-$C_6$ alkenyl, $C_2$-$C_6$ alkenyl substituted by one or more $R^{1-3-10}$, $C_6$-$C_{14}$ aryl, $C_6$-$C_{14}$ aryl substituted by one or more $R^{1-3-11}$, -O-$C_6$-$C_{14}$ aryl, -O-C(=O)$C_6$-$C_{14}$ aryl, -O-5- to 14-membered heteroaryl, $C_3$-$C_8$ cycloalkenyl, $C_3$-$C_8$ cycloalkenyl substituted by one or more $R^{1-3-12}$, 3- to 12-membered heterocycloalkyl, or 3- to 12-membered heterocycloalkyl substituted by one or more $R^{1 -3-13}$;

$R^{1-3-1}$, $R^{1-3-2}$, $R^{1-3-3}$, and $R^{1-3-4}$ are independently H, hydroxyl, $C_1$-$C_6$ alkyl, -$C_1$-$C_6$ alkyl-$C_6$-$C_{14}$ aryl, $C_1$-$C_6$ alkoxy, -C(=O)$R^{1-3-1-1}$, $C_3$-$C_8$ cycloalkyl, $C_6$-$C_{14}$ aryl, or $C_6$-$C_{14}$ aryl substituted by one or more $R^{1-3-1-4}$,

alternatively, $R^{1-3-1}$ and $R^{1-3-2}$, together with the N atom to which they are attached, form a 3- to 8-membered heterocycloalkyl or a 3- to 8-membered heterocycloalkyl substituted by one or more $R^{1-3-1-2}$,

alternatively, $R^{1-3-3}$ and $R^{1-3-4}$, together with the N atom to which they are attached, form a 3- to 8-membered heterocycloalkyl or a 3- to 8-membered heterocycloalkyl substituted by one or more $R^{1-3-3-1}$,

each $R^{1-3-1-1}$, each $R^{1-3-1-2}$, each $R^{1-3-1-4}$, and each $R^{1-3-3-1}$ is independently halogen or $C_1$-$C_6$ alkyl;

$R^{1-3-5}$ is independently H, hydroxyl, $C_1$-$C_6$ alkyl, $C_1$-$C_6$ alkoxy, or $C_3$-$C_8$ cycloalkyl; each $R^{1-3-6}$, each $R^{1-3-7}$, each $R^{1-3-8}$, each $R^{1-3-9}$, each $R^{1-3-10}$, each $R^{1-3-11}$, each $R^{1-3-12}$, and each $R^{1-3-13}$ is independently halogen, hydroxyl, carboxyl, cyano, -C(=O)-O-$C_1$-$C_6$ alkyl, - C(=O)-N($C_1$-$C_6$ alkyl)$_2$, -C(=O)-N($C_1$-$C_6$ alkyl)$_2$, $C_1$-$C_6$ alkyl, $C_1$-$C_6$ alkoxy, $C_3$-$C_8$ cycloalkyl, 3- to 8-membered heterocycloalkyl, $C_2$-$C_6$ alkenyl, $C_6$-$C_{14}$ aryl, $C_6$-$C_{14}$ aryl substituted by one or more $C_1$-$C_6$ alkyl groups, 5- to 10-membered heteroaryl, or 5- to 10-membered heteroaryl substituted by one or more $C_1$-$C_6$ alkyl groups;

each $R^{1-3-8}$ and each $R^{1-3-11}$ is independently halogen, hydroxyl, carboxyl, cyano, -C(=O)-O-$C_1$-$C_6$ alkyl, -C(=O)-N($C_1$-$C_6$ alkyl)$_2$, -C(=O)-N($C_1$-$C_6$ alkyl)$_2$, $C_1$-$C_6$ alkyl, $C_1$-$C_6$ alkoxy, $C_3$-$C_8$ cycloalkyl, 3- to 8-membered heterocycloalkyl, or $C_2$-$C_6$ alkenyl;

the 5- to 14-membered heteroaryl, the 5- to 14-membered heteroaryl substituted by one or more $R^{1-1-4}$, the 3- to 14-membered heterocycloalkyl, the 3- to 14-membered heterocycloalkyl substituted by one or more $R^{1-1-5}$, the 3- to 12-membered heterocycloalkyl, the 3- to 8-membered heterocycloalkyl, the 3- to 8-membered heterocycloalkyl substituted by one or more $R^{1-3-1-2}$, the 3- to 8-membered heterocycloalkyl substituted by one or more $R^{1-3-3-1}$, the 3- to 8-membered heterocycloalkyl substituted by one or more $R^{1-3-3-1}$, and the 5- to 10-membered heteroaryl substituted by one or more $C_1$-$C_6$ alkyl groups have 1, 2, 3, or 4 heteroatoms independently selected from one or more types of N, S, and O;

ring B is $C_3$-$C_{12}$ cycloalkyl, $C_3$-$C_{12}$ cycloalkyl substituted by one or more $R^{1-4}$, $C_3$-$C_{12}$ cycloalkenyl, $C_3$-$C_{12}$ cycloalkenyl substituted by one or more $R^{1-5}$, 3- to 12-membered heterocycloalkyl, 3- to 12-membered heterocycloalkyl substituted by one or more $R^{1-6}$, 3- to 12-membered heterocycloalkenyl, 3- to 12-membered heterocycloalkenyl substituted by one or more $R^{1-7}$, $C_6$-$C_{14}$ aryl, $C_6$-$C_{14}$ aryl substituted by one or more $R^{1-8}$, 5- to 14-membered heteroaryl, or 5- to 14-membered heteroaryl substituted by one or more $R^{1-9}$;

each $R^{1-4}$, each $R^{1-5}$, each $R^{1-6}$, each $R^{1-7}$, each $R^{1-8}$, and each $R^{1-9}$ is independently deuterium, halogen, cyano, hydroxyl, -$NR^{1-3-1a}R^{1-3-2a}$, -C(=o)$NR^{1-3-3a}R^{1-3-4a}$, -C(-O)$R^{1-3-5a}$, - S(=O)$_2$-$C_1$-$C_{12}$ alkyl, -S-$C_1$-$C_{12}$ alkyl, $C_1$-$C_{12}$ alkyl, $C_1$-$C_{12}$ alkyl substituted by one or more $R^{1-3-6a}$, $C_1$-$C_{12}$ alkoxy, $C_1$-$C_{12}$ alkoxy substituted by one or more $R^{1-3-7a}$, 5- to 14-membered heteroaryl, 5- to 14-membered heteroaryl substituted by one or more $R^{1-3-8a}$, $C_3$-$C_{12}$ cycloalkyl, $C_3$-$C_{12}$ cycloalkyl substituted by one or more $R^{1-3-9a}$, $C_2$-$C_6$ alkenyl, $C_2$-$C_6$ alkenyl substituted by one or more $R^{1-3-10a}$, $C_6$-$C_{14}$ aryl, $C_6$-$C_{14}$ aryl substituted by one or more $R^{1-3-11a}$, -O-$C_6$-$C_{14}$ aryl, -O-C(=O)$C_6$-$C_{14}$ aryl, -O-5- to 10-membered heteroaryl, $C_3$-$C_8$ cycloalkenyl, $C_3$-$C_8$ cycloalkenyl substituted by one or more $R^{1-3-12a}$, 3- to 12-membered heterocycloalkyl, or 3- to 12-membered heterocycloalkyl substituted by one or more $R^{1-3-13a}$;

alternatively, any two adjacent $R^{1-8}$, together with the carbon atom to which they are attached, form a 3- to 8-membered heterocycloalkyl, a 3- to 8-membered heterocycloalkyl substituted by one or more $R^{1-8-1}$, or a $C_3$-$C_{14}$ cycloalkyl;

$R^{1-3-1a}$, $R^{1-3-2a}$, $R^{1-3-3a}$, and $R^{1-3-4a}$ are independently H, hydroxyl, $C_1$-$C_6$ alkyl, $C_1$-$C_6$ alkoxy, -C(=O)$R^{1-3-1-1a}$, or $C_3$-$C_8$ cycloalkyl,
alternatively, $R^{1-3-1a}$ and $R^{1-3-2a}$, together with the N atom to which they are attached, form a 3- to 8-membered heterocycloalkyl or a 3- to 8-membered heterocycloalkyl substituted by one or more $R^{1-3-1-2a}$,
alternatively, $R^{1-3-3a}$ and $R^{1-3-4a}$, together with the N atom to which they are attached, form a 3- to 8-membered heterocycloalkyl or a 3- to 8-membered heterocycloalkyl substituted by one or more $R^{1-3-3-1a}$,
each $R^{1-3-1-1a}$, each $R^{1-3-1-2a}$, and each $R^{1-3-3-1a}$ is independently halogen or $C_1$-$C_6$ alkyl;
$R^{1-3-5a}$ is independently H, hydroxyl, $C_1$-$C_6$ alkyl, $C_1$-$C_6$ alkoxy, $C_3$-$C_8$ cycloalkyl, or 3- to 8-membered heterocycloalkyl;
each $R^{1-3-6a}$ , each $R^{1-3-7a}$, each $R^{1-3-8a}$, each $R^{1-3-9a}$, each $R^{1-3-10a}$, each $R^{1-3-11a}$ , each $R^{1-3-12a}$, each $R^{1-3-13a}$, and each $R^{1-8-1}$ is independently halogen, hydroxyl, carboxyl, cyano, -C(=O)-O-$C_1$-$C_6$ alkyl, -C(=O)-NH-$C_1$-$C_6$ alkyl, -C(=O)-N($C_1$-$C_6$ alkyl)$_2$, $C_1$-$C_6$ alkyl, $C_1$-$C_6$ alkoxy, $C_3$-$C_8$ cycloalkyl, 3- to 12-membered heterocycloalkyl, $C_2$-$C_6$ alkenyl, or -O-C(=O)-$C_1$-$C_6$ alkyl-3- to 12-membered heterocycloalkyl;
the 3- to 8-membered heterocycloalkyl, the 3- to 12-membered heterocycloalkyl, the 3- to 12-membered heterocycloalkenyl, the 5- to 10-membered heteroaryl, the 5- to 14-membered heteroaryl, the 3- to 12-membered heterocycloalkyl substituted by one or more $R^{1-6}$, the 3- to 12-membered heterocycloalkenyl substituted by one or more $R^{1-7}$, the 5- to 14-membered heteroaryl substituted by one or more $R^{1-9}$, the 5- to 14-membered heteroaryl substituted by one or more $R^{1-3-8a}$, the 3- to 12-membered heterocycloalkyl substituted by one or more $R^{1-3-13a}$, the 3- to 8-membered heterocycloalkyl substituted by one or more $R^{1-8-1}$, the 3- to 8-membered heterocycloalkyl substituted by one or more $R^{1-3-1-2a}$, the 3- to 8-membered heterocycloalkyl substituted by one or more $R^{1-3-3-1a}$, the 3- to 8-membered heterocycloalkyl substituted by one or more $R^{1-3-3-1a}$, the -O-5- to 10-membered heteroaryl, and the -O-C(=O)-$C_1$-$C_6$ alkyl-3- to 12-membered heterocycloalkyl have 1, 2, 3, or 4 heteroatoms independently selected from one or more types of N, S, and O;
$R^2$ is hydrogen, deuterium, halogen, cyano, $C_1$-$C_6$ alkyl, or $C_1$-$C_6$ alkoxy;
$L^2$ is a bond, $C_1$-$C_6$ alkylene, $C_1$-$C_6$ alkylene substituted by one or more $L^{2-1}$, $C_3$-$C_8$ cycloalkylene, $C_3$-$C_8$ cycloalkylene substituted by one or more $L^{2-2}$, -O-$C_1$-$C_6$ alkylene, -NH-$C_1$-$C_6$ alkylene, or -N($C_1$-$C_6$ alkyl)-$C_1$-$C_6$ alkylene;
each $L^{2-1}$ and each $L^{2-2}$ is independently halogen, $C_1$-$C_6$ alkyl, $C_1$-$C_6$ alkyl substituted by one or more $L^{2-1-1}$, $C_1$-$C_6$ alkoxy, $C_1$-$C_6$ alkoxy substituted by one or more $L^{2-1-2}$, $C_3$-$C_8$ cycloalkyl, $C_3$-$C_8$ cycloalkyl substituted by one or more $L^{2-1-3}$, $C_2$-$C_6$ alkynyl, or $C_2$-$C_6$ alkynyl substituted by one or more $L^{2-1-4}$;
each $L^{2-1-1}$, each $L^{2-1-2}$, each $L^{2-1-3}$, and each $L^{2-1-4}$ is independently $C_3$-$C_8$ cycloalkyl or $C_3$-$C_8$ cycloalkyl substituted by one or more $L^{2-1-1-1}$;
each $L^{2-1-1-1}$ is independently halogen or $C_1$-$C_6$ alkyl;
$G^2$ is H, -C(=O)$G^{2-1}$, -C(=O)N$G^{2-2}G^{2-3}$, 5- to 10-membered heteroaryl, 5- to 10-membered heteroaryl substituted by one or more $G^{2-4}$, -S(=O)$_2$-OH, -P(=O)-(OH)$_2$, -P(=O)-(O$C_1$-$C_6$ alkyl)(OH), 3- to 8-membered heterocycloalkenyl, or 3- to 8-membered heterocycloalkenyl substituted by one or more $G^{2-5}$;
$G^{2-1}$ is hydroxyl, $C_1$-$C_6$ alkyl, or -O-$NH_2$;
$G^{2-2}$ and $G^{2-3}$ are independently H, -S(=O)$_2$-$C_1$-$C_6$ alkyl, $C_3$-$C_8$ cycloalkyl, $C_1$-$C_6$ alkyl, $C_1$-$C_6$ alkyl substituted by one or more $G^{2-2-1}$, or -NH(=O)-5- to 10-membered heteroaryl;
each $G^{2-2-1}$ is independently carboxyl or -S(=O)$_2$OH;
each $G^{2-4}$ and each $G^{2-5}$ is independently hydroxyl or oxo;
the 5- to 10-membered heteroaryl, the 5- to 10-membered heteroaryl substituted by one or more $G^{2-4}$, the 3- to 8-membered heterocycloalkenyl, the 3- to 8-membered heterocycloalkenyl substituted by one or more $G^{2-5}$, and the -NH(=O)-5- to 10-membered heteroaryl have 1, 2, 3, or 4 heteroatoms independently selected from one or more types of N, S, and O.

2. The compound of formula I or the pharmaceutically acceptable salt thereof according to claim 1, wherein the compound of formula I is a compound of formula Ia

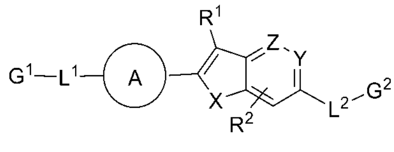

Ia

wherein X is O or $NR^3$, and $R^3$ is H or $C_1$-$C_6$ alkyl;
Z and Y are independently C or N;

$G^1$ is H, $C_1$-$C_6$ alkyl, $C_1$-$C_6$ alkyl substituted by one or more $G^{1-1}$, $C_6$-$C_{14}$ aryl, $C_6$-$C_{14}$ aryl substituted by one or more $G^{1-2}$, 5- to 10-membered heteroaryl, 5- to 10-membered heteroaryl substituted by one or more $G^{1-3}$, $C_1$-$C_6$ alkoxy, $C_1$-$C_6$ alkoxy substituted by one or more $G^{1-4}$, $C_2$-$C_6$ alkenyl, $C_2$-$C_6$ alkenyl substituted by one or more $G^{1-5}$, $C_2$-$C_6$ alkynyl, $C_2$-$C_6$ alkynyl substituted by one or more $G^{1-6}$, $C_3$-$C_8$ cycloalkyl, $C_3$-$C_8$ cycloalkyl substituted by one or more $G^{1-7}$, $C_3$-$C_8$ cycloalkenyl, $C_3$-$C_8$ cycloalkenyl substituted by one or more $G^{1-8}$, 3- to 8-membered heterocycloalkyl, 3- to 8-membered heterocycloalkyl substituted by one or more $G^{1-9}$, 3- to 8-membered heterocycloalkenyl, or 3- to 8-membered heterocycloalkenyl substituted by one or more $G^{1-10}$;

each $G^{1-1}$, each $G^{1-2}$, each $G^{1-3}$, each $G^{1-4}$, each $G^{1-5}$, each $G^{1-6}$ , each $G^{1-7}$, each $G^{1-8}$, each $G^{1-9}$, and each $G^{1-10}$ is independently deuterium, halogen, cyano, -$NG^{1-1-1}G^{1-1-2}$, -NC(=O)$G^{1-1-3}G^{1-1-4}$, hydroxyl, -S(=O)$_2$-$C_1$-$C_6$ alkyl, $C_1$-$C_6$ alkyl, $C_1$-$C_6$ alkyl substituted by one or more $G^{1-1-5}$, $C_1$-$C_6$ alkoxy, $C_1$-$C_6$ alkoxy substituted by one or more $G^{1-1-6}$, -S-$C_1$-$C_6$ alkyl, -S-$C_1$-$C_6$ alkyl substituted by one or more $G^{1-1-7}$, $C_3$-$C_8$ cycloalkyl, $C_3$-$C_8$ cycloalkyl substituted by one or more $G^{1-1-8}$, -O-$C_3$-$C_8$ cycloalkyl, -O-$C_3$-$C_8$ cycloalkyl substituted by one or more $G^{1-1-9}$, or - C(=O)$NG^{1-1-11}G^{1-1-12}$;

alternatively, any two adjacent $G^{1-2}$, together with the carbon atom to which they are attached, form a 3- to 8-membered heterocycloalkyl, a 3- to 8-membered heterocycloalkyl substituted by one or more $G^{1-1-9}$, a $C_3$-$C_8$ cycloalkyl, or a $C_3$-$C_8$ cycloalkyl substituted by one or more $G^{1-1-10}$;

$G^{1-1-1}$, $G^{1-1-2}$, $G^{1-1-3}$, $G^{1-1-4}$, $G^{1-1-11}$, and $G^{1-1-12}$ are independently H, $C_1$-$C_6$ alkyl, $C_3$-$C_8$ cycloalkyl, 5- to 10-membered heteroaryl, or 5- to 10-membered heteroaryl substituted by one or more $G^{1-1-10-1}$;

each $G^{1-1-10-1}$ is independently $C_1$-$C_6$ alkyl;

each $G^{1-1-5}$, each $G^{1-1-6}$, each $G^{1-1-7}$, each $G^{1-1-8}$, each $G^{1-1-9}$, and each $G^{1-1-10}$ is independently halogen, oxo, $C_1$-$C_6$ alkyl, or $C_3$-$C_8$ cycloalkyl;

$L^1$ is a bond or $C_1$-$C_6$ alkylene;

ring A is $C_4$-$C_6$ cycloalkyl, $C_4$-$C_6$ cycloalkyl substituted by one or more $A^1$, $C_4$-$C_6$ cycloalkenyl, $C_4$-$C_6$ cycloalkenyl substituted by one or more $A^2$, 4- to 6-membered heterocycloalkyl, 4- to 6-membered heterocycloalkyl substituted by one or more $A^1$, 4- to 6-membered heterocycloalkenyl, or 4- to 6-membered heterocycloalkenyl substituted by one or more $A^4$; the 4- to 6-membered heterocycloalkyl, the 4- to 6-membered heterocycloalkyl substituted by one or more $A^1$, the 4- to 6-membered heterocycloalkenyl, and the 4- to 6-membered heterocycloalkenyl substituted by one or more $A^4$ have 1 or 2 heteroatoms independently selected from one or more types of N, S, and O;

each $A^1$, each $A^2$, each $A^3$, and each $A^4$ is independently deuterium, halogen, cyano, -$NA^{1-1}A^{1-2}$, -NC(=O) $A^{1-3}A^{1-4}$, hydroxyl, $C_1$-$C_6$ alkyl, $C_1$-$C_6$ alkyl substituted by one or more $A^{1-5}$, $C_1$-$C_6$ alkoxy, or $C_1$-$C_6$ alkoxy substituted by one or more $A^{1-6}$;

$A^{1-1}$, $A^{1-2}$, $A^{1-3}$, and $A^{1-4}$ are independently deuterium, halogen, cyano, $C_1$-$C_6$ alkyl, or $C_1$-$C_6$ alkoxy;

each $A^{1-5}$ and each $A^{1-6}$ is independently hydrogen, deuterium, halogen, cyano, $C_1$-$C_6$ alkyl, or $C_1$-$C_6$ alkoxy;

$R^1$ is -C(=O)$NR^{1-1}R^{1-2}$, $C_1$-$C_6$ alkyl substituted by one or more $R^{1-3}$, or ring B;

$R^{1-1}$ and $R^{1-2}$ are independently H, $C_1$-$C_6$ alkyl, $C_1$-$C_6$ alkyl substituted by one or more $R^{1-1-1}$, $C_3$-$C_{10}$ cycloalkyl, $C_3$-$C_{10}$ cycloalkyl substituted by one or more $R^{1-1-2}$, $C_6$-$C_{14}$ aryl, $C_6$-$C_{14}$ aryl substituted by one or more $R^{1-1-3}$, 5- to 10-membered heteroaryl, or 5- to 10-membered heteroaryl substituted by one or more $R^{1-1-4}$;

each $R^{1-1-1}$, each $R^{1-1-2}$, each $R^{1-1-3}$, and $R^{1-1-4}$ is independently halogen, $C_1$-$C_6$ alkyl, or $C_3$-$C_8$ cycloalkyl;

ring B is $C_3$-$C_8$ cycloalkyl, $C_3$-$C_8$ cycloalkyl substituted by one or more $R^{1-4}$, $C_3$-$C_8$ cycloalkenyl, $C_3$-$C_8$ cycloalkenyl substituted by one or more $R^{1-5}$, 3- to 8-membered heterocycloalkyl, 3- to 8-membered heterocycloalkyl substituted by one or more $R^{1-6}$, 3- to 8-membered heterocycloalkenyl, 3- to 8-membered heterocycloalkenyl substituted by one or more $R^{1-7}$, $C_6$-$C_{14}$ aryl, $C_6$-$C_{14}$ aryl substituted by one or more $R^{1-8}$, 5- to 10-membered heteroaryl, or 5- to 10-membered heteroaryl substituted by one or more $R^{1-9}$;

each $R^{1-3}$, each $R^{1-4}$, each $R^{1-5}$, each $R^{1-6}$ , each $R^{1-7}$, each $R^{1-8}$, and each $R^{1-9}$ is independently deuterium, halogen, cyano, hydroxyl, -$NR^{1-3-1}R^{1-3-2}$, -C(-O)$NR^{1-3-3}R^{1-3-4}$, - C(=O)$R^{1-3-5}$, -S(=O)$_2$-$C_1$-$C_6$ alkyl, -S-$C_1$-$C_6$ alkyl, $C_1$-$C_6$ alkyl, $C_1$-$C_6$ alkyl substituted by one or more $R^{1-3-6}$, $C_1$-$C_6$ alkoxy, $C_1$-$C_6$ alkoxy substituted by one or more $R^{1-3-7}$, 5- to 10-membered heteroaryl, 5- to 10-membered heteroaryl substituted by one or more $R^{1-3-8}$, $C_3$-$C_8$ cycloalkyl, $C_3$-$C_8$ cycloalkyl substituted by one or more $R^{1-3-9}$, $C_2$-$C_6$ alkenyl, or $C_2$-$C_6$ alkenyl substituted by one or more $R^{1-3-10}$,

alternatively, any two adjacent $R^{1-8}$, together with the carbon atom to which they are attached, form a 3- to 8-membered heterocycloalkyl or a 3- to 8-membered heterocycloalkyl substituted by one or more $R^{1-8-1}$;

$R^{1-3-1}$, $R^{1-3-2}$, $R^{1-3-3}$, and $R^{1-3-4}$ are independently H, hydroxyl, $C_1$-$C_6$ alkyl, $C_1$-$C_6$ alkoxy, - C(=O)$R^{1-3-1-1}$, or $C_3$-$C_8$ cycloalkyl,

alternatively, $R^{1-3-1}$ and $R^{1-3-2}$, together with the N atom to which they are attached, form a 3- to 8-membered heterocycloalkyl or a 3- to 8-membered heterocycloalkyl substituted by one or more $R^{1-3-1-2}$,

alternatively, $R^{1-3-3}$ and $R^{1-3-4}$, together with the N atom to which they are attached, form a 3- to 8-membered heterocycloalkyl or a 3- to 8-membered heterocycloalkyl substituted by one or more $R^{1-3-3-1}$,

$R^{1-3-1-1}$ and each $R^{1-3-1-1}$ is independently $C_1$-$C_6$ alkyl or 5- to 10-membered heteroaryl; $R^{1-3-5}$ is independently H, hydroxyl, $C_1$-$C_6$ alkyl, $C_1$-$C_6$ alkoxy, or $C_3$-$C_8$ cycloalkyl;

each $R^{1-3-6}$, each $R^{1-3-7}$, each $R^{1-3-8}$, each $R^{1-3-9}$, and each $R^{1-3-10}$ is independently halogen, hydroxyl, carboxyl, -C(=O)-O-$C_1$-$C_6$ alkyl, -C(=O)-NH-$C_1$-$C_6$ alkyl, $C_1$-$C_6$ alkyl, $C_1$-$C_6$ alkoxy, $C_3$-$C_8$ cycloalkyl, or 3- to 8-membered heterocycloalkyl;

$R^2$ is hydrogen, deuterium, halogen, cyano, $C_1$-$C_6$ alkyl, or $C_1$-$C_6$ alkoxy;

$L^2$ is $C_1$-$C_6$ alkylene, $C_1$-$C_6$ alkylene substituted by one or more $L^{2-1}$, $C_3$-$C_8$ cycloalkylene, or $C_3$-$C_8$ cycloalkylene substituted by one or more $L^{2-2}$;

each $L^{2-1}$ and each $L^{2-2}$ is independently halogen, $C_1$-$C_6$ alkyl, $C_1$-$C_6$ alkyl substituted by one or more $L^{2-1-1}$, $C_1$-$C_6$ alkoxy, $C_1$-$C_6$ alkoxy substituted by one or more $L^{2-1-2}$, $C_3$-$C_8$ cycloalkyl, $C_3$-$C_8$ cycloalkyl substituted by one or more $L^{2-1-3}$, $C_2$-$C_6$ alkynyl, or $C_2$-$C_6$ alkynyl substituted by one or more $L^{2-1-4}$;

each $L^{2-1-1}$, each $L^{2-1-2}$, each $L^{2-1-3}$, and each $L^{2-1-4}$ is independently $C_3$-$C_8$ cycloalkyl or $C_3$-$C_8$ cycloalkyl substituted by one or more $L^{2-1-1-1}$;

$G^2$ is -C(=O)$G^{2-1}$, -C(=O)N$G^{2-2}G^{2-3}$, 5- to 10-membered heteroaryl, or 5- to 10-membered heteroaryl substituted by one or more $G^{2-4}$;

$G^{2-1}$ is hydroxyl, $C_1$-$C_6$ alkyl, or -O-$NH_2$;

$G^{2-2}$ and $G^{2-3}$ are independently H, -S(=O)$_2$-$C_1$-$C_6$ alkyl, $C_3$-$C_8$ cycloalkyl, $C_1$-$C_6$ alkyl, or $C_1$-$C_6$ alkyl substituted by one or more $G^{2-2-1}$;

each $G^{2-2-1}$ is independently carboxyl or -S(=O)$_2$OH;

each 5- to 10-membered heteroaryl, each 3- to 8-membered heterocycloalkenyl, and each 3- to 8-membered heterocycloalkyl has 1, 2, 3, or 4 heteroatoms independently selected from one or more types of N, S, and O.

**3.** The compound of formula I or the pharmaceutically acceptable salt thereof according to claim 1, wherein the compound of formula I or the pharmaceutically acceptable salt thereof satisfies one or more of the following conditions:

(1) in $R^3$ and $G^1$, the "$C_1$-$C_6$ alkyl" in the $C_1$-$C_6$ alkyl and the $C_1$-$C_6$ alkyl substituted by one or more $G^{1-1}$ is independently methyl, ethyl, n-propyl, isopropyl, n-butyl, isobutyl, *sec*-butyl, or tert-butyl, such as methyl;

(2) in $G^1$, the "$C_6$-$C_{14}$ aryl" in the $C_6$-$C_{14}$ aryl and the $C_6$-$C_{14}$ aryl substituted by one or more $G^{1-2}$ is independently phenyl or naphthyl;

(3) in $G^1$, the "5- to 10-membered heteroaryl" in the 5- to 10-membered heteroaryl and the 5- to 10-membered heteroaryl substituted by one or more $G^{1-3}$ is independently 5-, 6-, 9-, or 10-membered monocyclic or bicyclic heteroaryl with 1 or 2 heteroatoms independently selected from one or more types of N, S, and O, and may further be pyridyl, thiazolyl, furanophenyl, or oxazolophenyl, such as

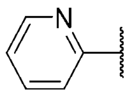, 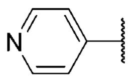, 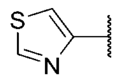, 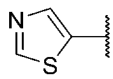,

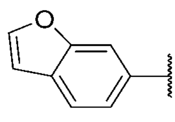, or 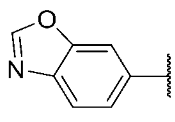;

(4) in each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, each $G^{1-10}$, and each $G^{1-11}$, the halogen is independently fluorine, chlorine, or bromine, such as fluorine or chlorine;

(5) in each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$, the "$C_1$-$C_6$ alkyl" in the -S(=O)$_2$-$C_1$-$C_6$ alkyl, the $C_1$-$C_6$ alkyl, the $C_1$-$C_6$ alkyl substituted by one or more $G^{1-1-5}$, the -S-$C_1$-$C_6$ alkyl, and the -S-$C_1$-$C_6$ alkyl substituted by one or more $G^{1-1-7}$ may independently be methyl, ethyl, n-propyl, isopropyl, n-butyl, isobutyl, sec-butyl, or *tert-butyl,* such as methyl or ethyl;

(6) in each $G^{1-1}$, each $G^2$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$, the "$C_1$-$C_6$ alkoxy" in the $C_1$-$C_6$ alkoxy and the $C_1$-$C_6$ alkoxy substituted by one or more $G^{1-1-6}$ is independently methoxy, ethoxy, n-propoxy, isopropoxy, n-butoxy, isobutoxy, sec-butoxy, or tert-butoxy, such as methoxy or ethoxy;

(7) in each $G^{1-1}$, each $G^{1-2}$, each $G^{1-3}$, each $G^{1-4}$, each $G^{1-5}$, each $G^{1-6}$, each $G^{1-7}$, each $G^{1-8}$, each $G^{1-9}$, and each $G^{1-10}$, the "$C_3$-$C_8$ cycloalkyl" in the $C_3$-$C_8$ cycloalkyl, the $C_3$-$C_8$ cycloalkyl substituted by one or more $G^{1-1-8}$, the -O-$C_3$-$C_8$ cycloalkyl, and the -O-$C_3$-$C_8$ cycloalkyl substituted by one or more $G^{1-1-9}$ is independently cyclopropyl,

cyclobutyl, cyclopentyl, or cyclohexyl, such as cyclopropyl;

(8) the "3- to 8-membered heterocycloalkyl" formed by any two adjacent $G^{1-2}$ together with the carbon atom to which they are attached and the "3- to 8-membered heterocycloalkyl" in the 3- to 8-membered heterocycloalkyl substituted by one or more $G^{1-1-9}$ are independently 5- to 6-membered heterocycloalkyl with 1 or 2 heteroatoms independently being N and/or O, such as

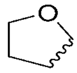, 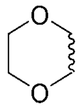, or 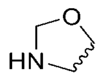;

(9) the "$C_3$-$C_8$ cycloalkyl" formed by any two adjacent $G^{1-2}$ together with the carbon atom to which they are attached and the "$C_3$-$C_8$ cycloalkyl" in the $C_3$-$C_8$ cycloalkyl substituted by one or more $G^{1-1-10}$ are independently $C_3$-$C_6$ cycloalkyl, such as

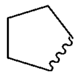;

(10) in $G^{1-1-1}$, $G^{1-1-2}$, $G^{1-1-3}$, $G^{1-1-4}$, $G^{1-1-11}$, and $G^{1-1-12}$, the $C_1$-$C_6$ alkyl is independently methyl, ethyl, *n*-propyl, isopropyl, *n*-butyl, isobutyl, *sec*-butyl, or *tert*-butyl, such as methyl;

(11) in $G^{1-1-1}$, $G^{1-1-2}$, $G^{1-1-3}$, $G^{1-1-4}$, $G^{1-1-11}$, and $G^{1-1-12}$, the $C_1$-$C_6$ alkyl may independently be methyl, ethyl, *n*-propyl, isopropyl, *n*-butyl, isobutyl, *sec*-butyl, *tert*-butyl, or *n*-hexyl, such as methyl, *tert*-butyl, or *n*-hexyl;

(12) in $G^{1-1-1}$, $G^{1-1-2}$, $G^{1-1-3}$ , $G^{1-1-4}$, $G^{1-1-11}$, and $G^{1-1-12}$ , the "5- to 10-membered heteroaryl" in the 5- to 10-membered heteroaryl and the 5- to 10-membered heteroaryl substituted by one or more $G^{1-1-10-1}$ is independently 5- to 6-membered heteroaryl with 1 or 2 heteroatoms being N, such as pyridyl;

(13) in each $G^{1-1-10-1}$ and each $G^{1-1-10-2}$, the "$C_1$-$C_6$ alkyl" in the $C_1$-$C_6$ alkyl is independently methyl, ethyl, *n*-propyl, isopropyl, *n*-butyl, isobutyl, *sec*-butyl, or *tert*-butyl, such as methyl;

(14) in each $G^{1-1-5}$, each $G^1$, each $G^{1-1-7}$, each $G^{1-1-8}$, each $G^{1-1-9}$, and each $G^{1-1-10}$, the halogen is independently fluorine, chlorine, or bromine, such as fluorine;

(15) in each $G^{1-1-5}$, each $G^{1-1-6}$, each $G^{1-1-7}$, each $G^{1-1-8}$, each $G^{1-1-9}$, and each $G^{1-1-10}$, the $C_3$-$C_8$ cycloalkyl is independently cyclopropyl, cyclobutyl, cyclopentyl, or cyclohexyl, such as cyclopropyl;

(16) in each $G^{1-1-5}$, each $G^1$, each $G^{1-1-7}$, each $G^{1-1-8}$, each $G^{1-1-9}$, and each $G^{1-1-10}$, the "5- to 10-membered heteroaryl" in the 5- to 10-membered heteroaryl and the 5- to 10-membered heteroaryl substituted by one or more $C_1$-$C_6$ alkyl groups may be 5-, 6-, 9-, or 10-membered monocyclic or bicyclic heteroaryl with 1, 2, or 3 heteroatoms being N, and may be triazolyl, such as

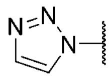;

(17) in $L^1$, the $C_1$-$C_6$ alkylene is methylene, ethylene, or propylene, such as methylene,

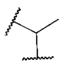 or 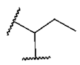;

(18) in ring A, the "$C_4$-$C_6$ cycloalkyl" in the $C_4$-$C_6$ cycloalkyl and the $C_4$-$C_6$ cycloalkyl substituted by one or more $A^1$ is independently cyclopropyl, cyclobutyl, cyclopentyl, or cyclohexyl, such as cyclohexyl; preferably, the cyclohexyl is

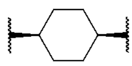 or 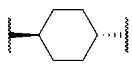;

(19) in ring A, the "$C_4$-$C_6$ cycloalkenyl" in the $C_4$-$C_6$ cycloalkenyl and the $C_4$-$C_6$ cycloalkenyl substituted by one or more $A^2$ is cyclohexenyl containing one double bond, such as

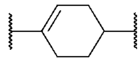;

(20) in ring A, the "4- to 8-membered heterocycloalkyl" in the 4- to 8-membered heterocycloalkyl and the 4- to 8-membered heterocycloalkyl substituted by one or more $A^3$ may independently be 4- to 6-membered heterocycloalkyl with 1 or 2 heteroatoms being N or 7- to 8-membered bridged heterocycloalkyl with 1 or 2 heteroatoms being N;

the 4- to 6-membered heterocycloalkyl may be azetidinyl, pyrrolidinyl, or piperidinyl, such as

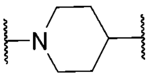, 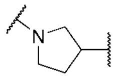, or 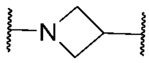;

the 7- to 8-membered bridged heterocycloalkyl may be azabicyclo[3.2.1]octanyl, such as

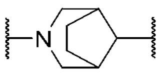;

ring A is connected to L via N at the left end and to

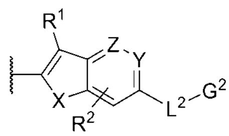

via C at the right end;

(21) in ring A, the "4- to 6-membered heterocycloalkenyl" in the 4- to 6-membered heterocycloalkenyl and the 4- to 6-membered heterocycloalkenyl substituted by one or more $A^4$ is independently 6-membered heterocycloalkenyl with 1 heteroatom being N, containing 1 double bond;

(22) in each $A^1$, each $A^2$, each $A^3$, and each $A^4$, the halogen is independently fluorine, chlorine, or bromine, such as fluorine;

(23) in each $A^1$, each $A^2$, each $A^3$, and each $A^4$, the "$C_1$-$C_6$ alkyl" in the $C_1$-$C_6$ alkyl and the $C_1$-$C_6$ alkyl substituted by one or more $A^{1-5}$ is independently methyl, ethyl, *n*-propyl, isopropyl, *n*-butyl, isobutyl, *sec*-butyl, or *tert*-butyl, such as methyl;

(24) in $R^1$, the "$C_1$-$C_6$ alkyl" in the $C_1$-$C_6$ alkyl and the $C_1$-$C_6$ alkyl substituted by one or more $R^{1-3}$ may be methyl, ethyl, *n*-propyl, isopropyl, *n*-butyl, isobutyl, *sec*-butyl, *tert*-butyl, *n*-pentyl,

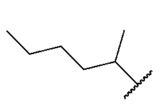, 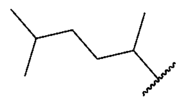, or 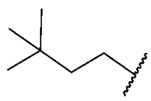;

(25) in $R^1$, the "$C_2$-$C_6$ alkenyl" in the $C_2$-$C_6$ alkenyl and the $C_2$-$C_6$ alkenyl substituted by one or more $R^{1-10}$ may be vinyl, propenyl, pentenyl, or hexenyl, such as

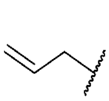, 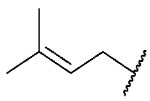,

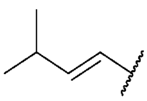, 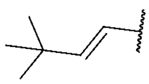, or 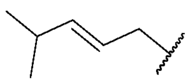;

(26) in $R^{1-1}$, $R^{1-2}$, and $R^{1-11}$, the "$C_1$-$C_{12}$ alkyl" in the $C_1$-$C_{12}$ alkyl, the $C_1$-$C_{12}$ alkyl substituted by one or more $R^{1-1-1}$, and the -$S(=O)_2C_1$-$C_{12}$ alkyl may independently be $C_1$-$C_6$ alkyl or $C_7$-$C_{12}$ alkyl; the "$C_1$-$C_6$ alkyl" in the $C_1$-$C_6$ alkyl, the $C_1$-$C_6$ alkyl substituted by one or more $R^{1-1-1}$, and the -$S(=O)_2C_1$-$C_6$ alkyl is independently methyl, ethyl, *n*-propyl, isopropyl, *n*-butyl, isobutyl, *sec*-butyl, *tert*-butyl, or

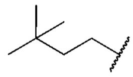,

such as methyl, ethyl, or isopropyl;

(27) in $R^{1-1}$, $R^{1-2}$, and $R^{1-11}$, the "$C_3$-$C_{12}$ cycloalkyl" in the $C_3$-$C_{12}$ cycloalkyl and the $C_3$-$C_{12}$ cycloalkyl substituted by one or more $R^{1-1-2}$ may independently be $C_3$-$C_{10}$ cycloalkyl or $C_{11}$-$C_{12}$ cycloalkyl; the "$C_3$-$C_{10}$ cycloalkyl" in the $C_3$-$C_{10}$ cycloalkyl and the $C_3$-$C_{10}$ cycloalkyl substituted by one or more $R^{1-1-2}$ may independently be $C_3$-$C_6$ monocyclic cycloalkyl, $C_5$-$C_7$ bridged cycloalkyl, or adamantyl, and may further be cyclopropyl, cyclobutyl, cyclopentyl, cyclohexyl, bicyclo[1.1.1]pentyl, bicyclo[2.2.1]heptyl, or adamantyl;

(28) $R^{1-1}$ and $R^{1-2}$, together with the N atom to which they are attached, form a 3- to 14-membered heterocycloalkyl, wherein the 3- to 14-membered heterocycloalkyl may be 4- to 6-membered monocyclic heterocycloalkyl with 1 or 2 heteroatoms being N or 6- to 14-membered bicyclic spirocycloalkyl with 1 or 2 heteroatoms being N, and may further be pyrrolidinyl or 2-azaspiro[3.3]heptyl;

(29) in $R^{1-1}$, $R^{1-2}$, and $R^{1-11}$, the "$C_6$-$C_{14}$ aryl" in the $C_6$-$C_{14}$ aryl and the $C_6$-$C_{14}$ aryl substituted by one or more $R^{1-1-3}$ is independently phenyl or naphthyl;

(30) in $R^{1-1}$, $R^{1-2}$, and $R^{1-11}$, the "5- to 14-membered heteroaryl" in the 5- to 14-membered heteroaryl and the 5- to 14-membered heteroaryl substituted by one or more $G^{1-1-10-1}$ may independently be 5- to 10-membered heteroaryl or 11- to 14-membered heteroaryl, wherein the "5- to 10-membered heteroaryl" in the 5- to 10-membered heteroaryl and the 5- to 10-membered heteroaryl substituted by one or more $G^{1-1-10-1}$ may independently be 5- to 6-membered heteroaryl or 8- to 10-membered bicyclic heteroaryl, and may further be thiazolyl, oxazolyl, imidazolyl, pyrazolyl, thiadiazolyl, triazolyl, tetrazolyl, pyridyl, benzo[*d*]isoxazolyl, or benzo[d]thiazolyl, such as

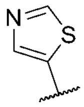, 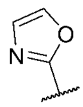, 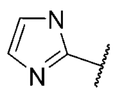, 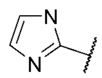, 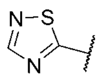, 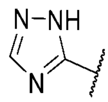,

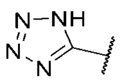, 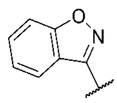, or 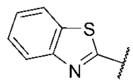;

(31) in each $R^{1-1-1}$, each $R^{1-1-2}$, each $R^{1-1-3}$, $R^{1-1-4}$, and each $R^{1-1-5}$, the halogen is independently fluorine, chlorine, or bromine, such as fluorine;

(32) in each $R^{1-1-1}$, each $R^{1-1-2}$, each $R^{1-1-3}$, $R^{1-1-4}$, and each $R^{1-1-5}$, the "$C_1$-$C_{12}$ alkyl" in the -NH($C_1$-$C_{12}$ alkyl), the -N($C_1$-$C_{12}$ alkyl)$_2$, the -C(=O)-$C_1$-$C_{12}$ alkyl, the -NHC(=O)-$C_1$-$C_{12}$ alkyl, the $C_1$-$C_{12}$ alkyl, and the $C_1$-$C_{12}$ alkyl substituted by one or more $R^{1-1-1-1}$ may independently be $C_1$-$C_6$ alkyl or $C_7$-$C_{12}$ alkyl; the $C_1$-$C_6$ alkyl is methyl, ethyl, n-propyl, isopropyl, n-butyl, isobutyl, sec-butyl, or *tert-butyl,* such as methyl;

(33) in each $R^{1-1-1}$, each $R^{1-1-2}$, each $R^{1-1-3}$, $R^{1-1-4}$, and each $R^{1-1-5}$, the "$C_3$-$C_{12}$ cycloalkyl" in the $C_3$-$C_{12}$ cycloalkyl and the $C_3$-$C_{12}$ cycloalkyl substituted by one or more $R^{1-1-1-3}$ is independently $C_3$-$C_8$ cycloalkyl or $C_9$-$C_{10}$ cycloalkyl; the $C_3$-$C_8$ cycloalkyl is cyclopropyl, cyclobutyl, cyclopentyl, or cyclohexyl, such as cyclopropyl;

(34) in each $R^{1-1-1}$, each $R^{1-1-2}$, each $R^{1-1-3}$, $R^{1-1-4}$, and each $R^{1-1-5}$, the $C_6$-$C_{14}$ aryl is phenyl or naphthyl;

(35) in each $R^{1-1-1}$, each $R^{1-1-2}$, each $R^{1-1-3}$, $R^{1-1-4}$, and each $R^{1-1-5}$, the 3- to 12-membered heterocycloalkyl may be 3- to 6-membered heterocycloalkyl with 1 or 2 heteroatoms being N and/or O, and may be piperidinyl or morpholinyl;

(36) in each $R^{1-3}$ and each $R^{1-10}$, the halogen is independently fluorine, chlorine, or bromine, such as fluorine or chlorine;

(37) in each $R^{1-3}$ and each $R^{1-10}$, the "$C_1$-$C_{12}$ alkyl" in the -$S(=O)_2$-$C_1$-$C_{12}$ alkyl, the -S-$C_1$-$C_{12}$ alkyl, the $C_1$-$C_{12}$ alkyl, and the $C_1$-$C_{12}$ alkyl substituted by one or more $R^{1-3-6}$ is independently $C_1$-$C_6$ alkyl or $C_7$-$C_{12}$ alkyl; the "$C_1$-$C_6$ alkyl" in the -$S(=O)_2$-$C_1$-$C_6$ alkyl, the -S-$C_1$-$C_6$ alkyl, the $C_1$-$C_6$ alkyl, and the $C_1$-$C_6$ alkyl substituted by one or more $R^{1-3-6}$ may independently be methyl, ethyl, *n*-propyl, isopropyl, *n*-butyl, isobutyl, *sec*-butyl, or *tert*-

butyl, such as isopropyl; the $C_7$-$C_{12}$ alkyl may be pentyl, hexyl, or heptyl;

(38) in each $R^{1-3}$ and each $R^{1-10}$, the "$C_1$-$C_{12}$ alkoxy" in the $C_1$-$C_{12}$ alkoxy and the $C_1$-$C_{12}$ alkoxy substituted by one or more $R^{1-3-7}$ is independently $C_1$-$C_6$ alkoxy; the "$C_1$-$C_6$ alkoxy" in the $C_1$-$C_6$ alkoxy and the $C_1$-$C_6$ alkoxy substituted by one or more $R^{1-3-7}$ may independently be methoxy, ethoxy, *n*-propoxy, isopropoxy, *n*-butoxy, isobutoxy, *sec*-butoxy, or *tert*-butoxy;

(39) in each $R^{1-3}$ and each $R^{1-10}$, the "5- to 14-membered heteroaryl" in the 5- to 14-membered heteroaryl, the 5- to 14-membered heteroaryl substituted by one or more $R^{1-3-8}$, and the -O-5- to 14-membered heteroaryl is independently 5- to 10-membered heteroaryl; the "5-to 10-membered heteroaryl" in the 5- to 10-membered heteroaryl and the 5- to 10-membered heteroaryl substituted by one or more $R^{1-3-8}$ may independently be 5- to 6-membered monocyclic heteroaryl with 1, 2, or 3 heteroatoms independently selected from one or two types of N, S, and O, and may further be 1*H*-pyrazolyl, pyridyl, or oxadiazolyl;

(40) in each $R^{1-3}$ and each $R^{1-10}$, the "$C_2$-$C_6$ alkenyl" in the $C_2$-$C_6$ alkenyl and the $C_2$-$C_6$ alkenyl substituted by one or more $R^{1-3-10}$ is independently vinyl or propenyl, such as

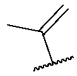;

(41) in each $R^{1-3}$ and each $R^{1-10}$, the "$C_6$-$C_{14}$ aryl" in the $C_6$-$C_{14}$ aryl, the $C_6$-$C_{14}$ aryl substituted by one or more $R^{1-3-11}$, the -O-$C_6$-$C_{14}$ aryl, and the -O-C(=O)$C_6$-$C_{14}$ aryl is independently phenyl;

(42) in each $R^{1-3}$ and each $R^{1-10}$, the "3- to 12-membered heterocycloalkyl" in the 3- to 12-membered heterocycloalkyl and the 3- to 12-membered heterocycloalkyl substituted by one or more $R^{1-3-13}$ is 3- to 6-membered monocyclic heterocycloalkyl with 1 or 2 heteroatoms being O, such as

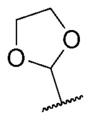;

(43) in $R^{1-3-1}$, $R^{1-3-2}$, $R^{1-3-3}$, $R^{1-3-4}$, $R^{1-3-1a}$, $R^{1-3-2a}$, $R^{1-3-3a}$, and $R^{1-3-4a}$, the $C_1$-$C_6$ alkyl is methyl, ethyl, *n*-propyl, isopropyl, *n*-butyl, isobutyl, *sec*-butyl, or *tert*-butyl;

(44) in each $R^{1-3}$ and each $R^{1-10}$, the "$C_6$-$C_{14}$ aryl" in the $C_6$-$C_{14}$ aryl and the $C_6$-$C_{14}$ aryl substituted by one or more $R^{1-3-1-4}$ is independently phenyl;

(45) in each $R^{1-3-1-1}$, each $R^{1-3-1-2}$, each $R^{1-3-1-4}$, and each $R^{1-3-3-1}$, the $C_1$-$C_6$ alkyl is methyl, ethyl, *n*-propyl, isopropyl, *n*-butyl, isobutyl, *sec*-butyl, or *tert*-butyl;

(46) in each $R^{1-3-6}$ , each $R^{1-3-7}$ , each $R^{1-3-8}$, each $R^{1-3-9}$, each $R^{1-3-10}$, each $R^{1-3-11}$, each $R^{1-3-12}$, and each $R^{1-3-13}$, the "$C_1$-$C_6$ alkyl" in the -C(=O)-O-$C_1$-$C_6$ alkyl, the -C(=O)-N($C_1$-$C_6$ alkyl)$_2$, the -C(=O)-N($C_1$-$C_6$ alkyl)$_2$, and the $C_1$-$C_6$ alkyl is independently methyl, ethyl, *n*-propyl, isopropyl, *n*-butyl, isobutyl, *sec*-butyl, or *tert*-butyl;

(47) in each $R^{1-3-6}$, $R^{1-3-7}$, $R^{1-3-8}$, $R^{1-3-9}$, $R^{1-3-10}$, $R^{1-3-11}$, $R^{1-3-12}$, and $R^{1-3-13}$, the $C_3$-$C_8$ cycloalkyl is cyclopropyl, cyclobutyl, cyclopentyl, or cyclohexyl;

(48) in each $R^{1-3-6}$, each $R^{1-3-7}$, each $R^{1-3-9}$, each $R^{1-3-10}$, each $R^{1-3-12}$, and each $R^{1-3-13}$, the "5- to 10-membered heteroaryl" in the 5- to 10-membered heteroaryl and the 5- to 10-membered heteroaryl substituted by one or more $C_1$-$C_6$ alkyl groups is 5- to 6-membered heteroaryl with 1 or 2 heteroatoms selected from one or two types of N, S, and O, and may further be furanyl or thienyl;

(49) in ring B, the "$C_3$-$C_{12}$ cycloalkyl" in the $C_3$-$C_{12}$ cycloalkyl and the $C_3$-$C_{12}$ cycloalkyl substituted by one or more $R^{1-4}$ is independently $C_3$-$C_8$ cycloalkyl or $C_9$-$C_{12}$ cycloalkyl; the "$C_3$-$C_8$ cycloalkyl" in the $C_3$-$C_8$ cycloalkyl and the $C_3$-$C_8$ cycloalkyl substituted by one or more $R^{1-4}$ is independently cyclopropyl, cyclobutyl, cyclopentyl, or cyclohexyl, such as cyclohexyl;

(50) in ring B, the "$C_3$-$C_{12}$ cycloalkenyl" in the $C_3$-$C_{12}$ cycloalkenyl and the $C_3$-$C_{12}$ cycloalkenyl substituted by one or more $R^{1-5}$ is independently $C_3$-$C_8$ cycloalkenyl or $C_9$-$C_{12}$ cycloalkenyl; the "$C_3$-$C_8$ cycloalkenyl" in the $C_3$-$C_8$ cycloalkenyl and the $C_3$-$C_8$ cycloalkenyl substituted by one or more $R^{1-5}$ is cyclopropenyl containing one double bond, cyclobutenyl containing one double bond, cyclopentenyl containing one double bond, or cyclohexenyl containing one double bond, such as cyclopentenyl or cyclohexenyl;

(51) in ring B, the "3- to 12-membered heterocycloalkyl" in the 3- to 12-membered heterocycloalkyl and the 3- to 12-membered heterocycloalkyl substituted by one or more $R^{1-6}$ is independently 3- to 8-membered heterocycloalkyl or 9- to 12-membered heterocycloalkyl; the "3- to 8-membered heterocycloalkyl" in the 3- to 8-membered heterocycloalkyl and the 3-to 8-membered heterocycloalkyl substituted by one or more $R^{1-6}$ is independently 5- to 6-membered heterocycloalkyl with 1 or 2 heteroatoms independently being O and/or N, and

the number is 1 or 2, such as piperidinyl, dioxolanyl, or dioxanyl;

(52) in ring B, the "3- to 12-membered heterocycloalkenyl" in the 3- to 12-membered heterocycloalkenyl and the 3- to 12-membered heterocycloalkenyl substituted by one or more $R^{1-7}$ is independently 3- to 8-membered heterocycloalkenyl or 9- to 12-membered heterocycloalkenyl; the "3- to 8-membered heterocycloalkenyl" in the 3- to 8-membered heterocycloalkenyl and the 3- to 8-membered heterocycloalkenyl substituted by one or more $R^{1-7}$ is independently 5- to 6-membered heterocycloalkenyl with 1 or 2 heteroatoms independently being N and containing one double bond, such as 1,2,3,6-tetrahydropyridyl;

(53) in ring B, the "$C_6$-$C_{14}$ aryl" in the $C_6$-$C_{14}$ aryl and the $C_6$-$C_{14}$ aryl substituted by one or more $R^{1-8}$ is independently phenyl or naphthyl; preferably, when the "$C_6$-$C_{14}$ aryl" in the $C_6$-$C_{14}$ aryl substituted by one or more $R^{1-8}$ is phenyl, the number of is 1, and the substitution position is at the ortho, meta, or para position of the phenyl, such as the para position;

(54) in ring B, the "5- to 14-membered heteroaryl" in the the 5- to 14-membered heteroaryl and the 5- to 14-membered heteroaryl substituted by one or more $R^{1-9}$ is independently 5- to 10-membered heteroaryl or 11- to 14-membered heteroaryl; the "5- to 10-membered heteroaryl" in the the 5- to 10-membered heteroaryl and the 5- to 10-membered heteroaryl substituted by one or more $R^{1-9}$ may independently be 5-, 6-, or 9-membered monocyclic or bicyclic heteroaryl with 1, 2, 3, or 4 heteroatoms selected from one or more types of N, S, and O, and may further be pyrrolyl, imidazolyl, 1*H*-pyrazolyl, oxazolyl, isoxazolyl, thiazolyl, 1,3,4-oxadiazolyl, 1,3,4-oxadiazolyl, 1,2,4-oxadiazolyl, thienyl, thiazolyl, pyridyl, pyrimidinyl, indolyl, indazolyl, thiazolophenyl, or triazolopyridyl; or may further be 1*H*-pyrazolyl, 1,3,4-oxadiazolyl, thienyl, thiazolyl, pyridyl, pyrimidinyl, indolyl, indazolyl, thiazolophenyl, or triazolopyridyl;

(55) in each $R^{1-4}$, each $R^{1-5}$, each $R^{1-6}$, each $R^{1-7}$, each $R^{1-8}$, and each $R^{1-9}$, the halogen is independently fluorine, chlorine, or bromine, such as fluorine or chlorine;

(56) in each $R^{1-4}$, each $R^{1-5}$, each $R^{1-6}$, each $R^{1-7}$, each $R^{1-8}$, and each $R^{1-9}$, the "$C_1$-$C_{12}$ alkyl" in the $-S(=O)_2$-$C_1$-$C_{12}$ alkyl, the -S-$C_1$-$C_{12}$ alkyl, the $C_1$-$C_{12}$ alkyl, and the $C_1$-$C_{12}$ alkyl substituted by one or more $R^{1-3-6a}$ is independently $C_1$-$C_6$ alkyl or $C_7$-$C_{12}$ alkyl; the "$C_1$-$C_6$ alkyl" in the $-S(=O)_2$-$C_1$-$C_6$ alkyl, the -S-$C_1$-$C_6$ alkyl, the $C_1$-$C_6$ alkyl, and the $C_1$-$C_6$ alkyl substituted by one or more $R^{1-3-6a}$ may independently be methyl, ethyl, *n*-propyl, isopropyl, *n*-butyl, isobutyl, *sec*-butyl, or *tert*-butyl, such as isopropyl; the $C_7$-$C_{12}$ alkyl may be pentyl, hexyl, or heptyl;

(57) in each $R^{1-4}$, each $R^{1-5}$, each $R^{1-6}$, each $R^{1-7}$, each $R^{1-8}$, and each $R^{1-9}$, the "$C_1$-$C_{12}$ alkoxy" in the $C_1$-$C_{12}$ alkoxy and the $C_1$-$C_{12}$ alkoxy substituted by one or more $R^{1-3-7a}$ is independently $C_1$-$C_6$ alkoxy; the "$C_1$-$C_6$ alkoxy" in the $C_1$-$C_6$ alkoxy and the $C_1$-$C_6$ alkoxy substituted by one or more $R^{1-3-7a}$ may independently be methoxy, ethoxy, n-propoxy, isopropoxy, *n*-butoxy, isobutoxy, *sec*-butoxy, or *tert*-butoxy;

(58) in each $R^{1-4}$, each $R^{1-5}$, each $R^{1-6}$, each $R^{1-7}$, each $R^{1-8}$, and each $R^{1-9}$, the "5- to 14-membered heteroaryl" in the 5- to 14-membered heteroaryl and the 5- to 14-membered heteroaryl substituted by one or more $R^{1-3-8a}$ is independently 5- to 10-membered heteroaryl; the "5- to 10-membered heteroaryl" in the 5- to 10-membered heteroaryl and the 5- to 10-membered heteroaryl substituted by one or more $R^{1-3-8a}$ may independently be 5- to 6-membered monocyclic heteroaryl with 1 or 2 heteroatoms independently being N, and may further be 1*H*-pyrazolyl or pyridyl;

(59) in each $R^{1-4}$, each $R^{1-5}$, each $R^{1-6}$, each $R^{1-7}$, each $R^{1-8}$, and each $R^{1-9}$, the "$C_3$-$C_{12}$ cycloalkyl" in the $C_3$-$C_{12}$ cycloalkyl and the $C_3$-$C_{12}$ cycloalkyl substituted by one or more $R^{1-3-9a}$ is independently $C_3$-$C_8$ cycloalkyl or $C_9$-$C_{12}$ cycloalkyl; the "$C_3$-$C_8$ cycloalkyl" in the $C_3$-$C_8$ cycloalkyl and the $C_3$-$C_8$ cycloalkyl substituted by one or more $R^{1-3-9a}$ may independently be cyclopropyl, cyclobutyl, cyclopentyl, or cyclohexyl, such as cyclopropyl or cyclobutyl; the $C_9$-$C_{12}$ cycloalkyl may be adamantyl;

(60) in each $R^{1-4}$, each $R^{1-5}$, each $R^{1-6}$, each $R^{1-7}$, each $R^{1-8}$, and each $R^{1-9}$, the "$C_2$-$C_6$ alkenyl" in the $C_2$-$C_6$ alkenyl and the $C_2$-$C_6$ alkenyl substituted by one or more $R^{1-3-10a}$ may independently be vinyl or propenyl, such as

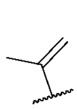;

(61) the "3- to 8-membered heterocycloalkyl" formed by any two adjacent $R^{1-8}$ together with the carbon atom to which they are attached and the "3- to 8-membered heterocycloalkyl" in the 3- to 8-membered heterocycloalkyl substituted by one or more $R^{1-8-1}$ are independently 5- to 6-membered heterocycloalkyl with 1 or 2 heteroatoms independently being N and/or O, such as

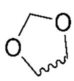 or 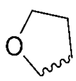;

(62) any two adjacent $R^{1-8}$, together with the carbon atom to which they are attached, form a $C_1$-$C_{14}$ cycloalkyl, and the $C_1$-$C_{14}$ cycloalkyl is $C_{11}$-$C_{14}$ tricyclic cycloalkyl, such as

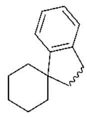
;

(63) in $R^{1-3-1a}$, $R^{1-3-2a}$, $R^{1-3-3a}$, $R^{1-3-4a}$, and $R^{1-3-5a}$, the $C_1$-$C_6$ alkyl may independently be methyl, ethyl, *n*-propyl, isopropyl, *n*-butyl, isobutyl, *sec*-butyl, *tert*-butyl,

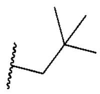
,

or

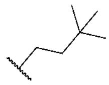
;

(64) in $R^{1-3-1a}$, $R^{1-3-2a}$, $R^{1-3-3a}$, $R^{1-3-4a}$, and $R^{1-3-5a}$, the $C_1$-$C_6$ alkoxy may independently be methoxy, ethoxy, n-propoxy, isopropoxy, *n*-butoxy, isobutoxy, *sec*-butoxy, or *tert*-butoxy;
(65) in $R^{1-3-1a}$, $R^{1-3-2a}$, $R^{1-3-3a}$, $R^{1-3-4a}$, and $R^{1-3-5a}$, the $C_3$-$C_8$ cycloalkyl is independently cyclopropyl, cyclobutyl, cyclopentyl, or cyclohexyl, such as cyclopropyl or cyclopentyl;
(66) the "3- to 8-membered heterocycloalkyl" formed by $R^{1-3-1a}$ and $R^{1-3-2a}$ together with the N atom to which they are attached and the "3- to 8-membered heterocycloalkyl" in the 3-to 8-membered heterocycloalkyl substituted by one or more $R^{1-3-1-2a}$ are independently 5- to 6-membered heterocycloalkyl with 1 or 2 heteroatoms independently being N, and may further be pyrrolidinyl;
(67) the "3- to 8-membered heterocycloalkyl" formed by $R^{1-3-3a}$ and $R^{1-3-4a}$ together with the N atom to which they are attached and the "3- to 8-membered heterocycloalkyl" in the 3-to 8-membered heterocycloalkyl substituted by one or more $R^{1-3-3-1a}$ may independently be 5-to 6-membered heterocycloalkyl with 1 or 2 heteroatoms independently being N or 6- to 7-membered dispiro heterocycloalkyl with 1 or 2 heteroatoms independently being N, and may further be pyrrolidinyl or 2-azaspiro[3.3]heptyl;
(68) in each $R^{1-3-1-1a}$, each $R^{1-3-1-2a}$, and each $R^{1-3-3-1a}$, the $C_1$-$C_6$ alkyl is independently methyl, ethyl, *n*-propyl, isopropyl, *n*-butyl, isobutyl, *sec*-butyl, or *tert*-butyl, such as methyl;
(69) in each $R^{1-3-6a}$, each $R^{1-3-7a}$, each $R^{1-3-8a}$, each $R^{1-3-8a}$, each $R^{1-3-10a}$, each $R^{1-3-11a}$, each $R^{1-3-12a}$, and each $R^{1-8-1}$, the "$C_1$-$C_6$ alkyl" in the $C_1$-$C_6$ alkyl, the -C(=O)-O-$C_1$-$C_6$ alkyl, the - C(=O)-NH-$C_1$-$C_6$ alkyl, and the -C(=O)-N($C_1$-$C_6$ alkyl)$_2$ may independently be methyl, ethyl, *n*-propyl, isopropyl, *n*-butyl, isobutyl, *sec*-butyl, or *tert*-butyl, such as methyl or *tert*-butyl;
(70) in each $R^{1-3-6a}$, each $R^{1-3-7a}$, each $R^{1-3-8a}$, each $R^{1-3-8a}$, each $R^{1-3-10a}$, each $R^{1-3-11a}$, each $R^{1-3-12a}$, and each $R^{1-8-1}$, the "3- to 12-membered heterocycloalkyl" in the 3- to 12-membered heterocycloalkyl and the -O-C(=O)-$C_1$-$C_6$ alkyl-3- to 12-membered heterocycloalkyl is independently 5- to 6-membered monocyclic heterocycloalkyl with 1 or 2 heteroatoms being N, such as pyrrolidinyl;
(71) in $L^2$, the "$C_1$-$C_6$ alkylene" in the $C_1$-$C_6$ alkylene, the $C_1$-$C_6$ alkylene substituted by one or more $L^{2-1}$, the -O-$C_1$-$C_6$ alkylene, and the -N-$C_1$-$C_6$ alkylene may independently be methylene, ethylene, *n*-propylene, isopropylene, *n*-butylene, isobutylene, *sec*-butylene, or *tert*-butylene, such as methyl, ethyl, *n*-propyl, or isopropyl, for example, methylene or ethylene; preferably, when $L^2$ is $C_1$-$C_6$ alkylene, then the C atom in the $C_1$-$C_6$ alkylene connected to

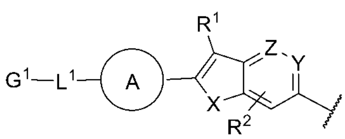

may be a non-chiral C, an *S*-configuration C, or an *R*-configuration C, and may further be an *S*-configuration C;
(72) in $L^2$, the "$C_3$-$C_8$ cycloalkylene" in the $C_3$-$C_8$ cycloalkylene and the $C_3$-$C_8$ cycloalkylene substituted by one or

more $L^{2-2}$ is independently cyclopropylene, cyclobutylene, cyclopentylene, or cyclohexylene, such as cyclopropylene;

(73) in each $L^{2-1}$, each $L^{2-2}$, and each $L^{2-1-1-1}$, the halogen is independently fluorine, chlorine, or bromine, such as fluorine;

(74) in each $L^{2-1}$, each $L^{2-2}$, and each $L^{2-1-1-1}$, the "$C_1$-$C_6$ alkyl" in the $C_1$-$C_6$ alkyl and the $C_1$-$C_6$ alkyl substituted by one or more $L^{2-1-1}$ is independently methyl, ethyl, *n*-propyl, isopropyl, *n*-butyl, isobutyl, *sec*-butyl, or *tert*-butyl, such as methyl, ethyl, *n*-propyl, or isopropyl;

(75) in each $L^{2-1}$ and each $L^{2-2}$, the "$C_1$-$C_6$ alkoxy" in the $C_1$-$C_6$ alkoxy and the $C_1$-$C_6$ alkoxy substituted by one or more $L^{2-1-2}$ is independently methoxy, ethoxy, *n*-propoxy, isopropoxy, *n*-butoxy, isobutoxy, *sec*-butoxy, or *tert*-butoxy, such as methoxy or ethoxy;

(76) in each $L^{2-1}$ and each $L^{2-2}$, the "$C_3$-$C_8$ cycloalkyl" in the $C_3$-$C_8$ cycloalkyl and the $C_3$-$C_8$ cycloalkyl substituted by one or more $L^{2-1-3}$ is independently cyclopropyl, cyclobutyl, cyclopentyl, or cyclohexyl, such as cyclopropyl or cyclobutyl;

(77) in each $L^{2-1}$ and each $L^{2-2}$, the "$C_2$-$C_6$ alkynyl" in the $C_2$-$C_6$ alkynyl and the $C_2$-$C_6$ alkynyl substituted by one or more $L^{2-1-4}$ is independently ethynyl;

(78) in $G^2$, the "5- to 10-membered heteroaryl" in the 5- to 10-membered heteroaryl and the 5- to 10-membered heteroaryl substituted by one or more $G^{2-4}$ is independently 5- to 6-membered heteroaryl with 2, 3, or 4 heteroatoms selected from one or more types of N, O, and S, and may further be 5- to 6-membered heteroaryl with 3 or 4 heteroatoms being N and/or O, such as tetrazolyl,

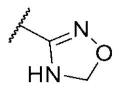,

oxazolyl, or

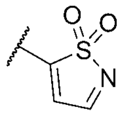;

(79) in $G^2$, the "3- to 8-membered heterocycloalkenyl" in the 3- to 8-membered heterocycloalkenyl and the 3- to 8-membered heterocycloalkenyl substituted by one or more $G^{2-5}$ is independently 3- to 5-membered heterocycloalkenyl with 2 or 3 heteroatoms being N and/or S, such as

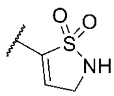;

(80) in $G^{2-1}$, $G^{2-2}$, and $G^{2-3}$, the "$C_1$-$C_6$ alkyl" in the -S(=O)$_2$-$C_1$-$C_6$ alkyl, $C_3$-$C_8$ cycloalkyl, the $C_1$-$C_6$ alkyl, and the $C_1$-$C_6$ alkyl substituted by one or more $G^{2-2-1}$ is independently methyl, ethyl, *n*-propyl, isopropyl, *n*-butyl, isobutyl, sec-butyl, or *tert*-butyl, such as methyl or ethyl;

(81) in $G^{2-1}$, $G^{2-2}$, and $G^{2-3}$, the $C_3$-$C_8$ cycloalkyl is independently cyclopropyl, cyclobutyl, cyclopentyl, or cyclohexyl, such as cyclopropyl;

(82) in $G^{2-2}$ and $G^{2-3}$, the "5- to 10-membered heteroaryl" in the -NH(=O)-5- to 10-membered heteroaryl is 5- to 6-membered heteroaryl with 1 or 2 heteroatoms being N, such as pyridyl.

**4.** The compound of formula I or the pharmaceutically acceptable salt thereof according to claim 3, wherein the compound of formula I or the pharmaceutically acceptable salt thereof satisfies one or more of the following conditions:

(1) in $G^1$, the $C_1$-$C_6$ alkyl substituted by one or more $G^{1-1}$ is

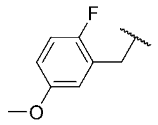, 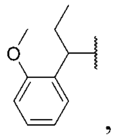,

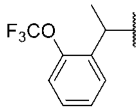, or 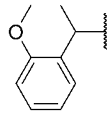;

(2) in $G^1$, the $C_6$-$C_{14}$ aryl substituted by one or more $G^{1-2}$ is phenyl substituted by 1 or 2 $G^{1-2}$, and may further be any one of the following groups:

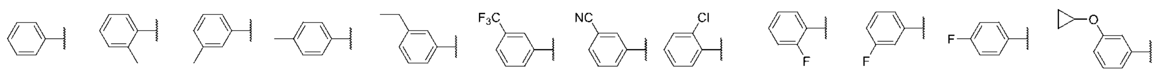

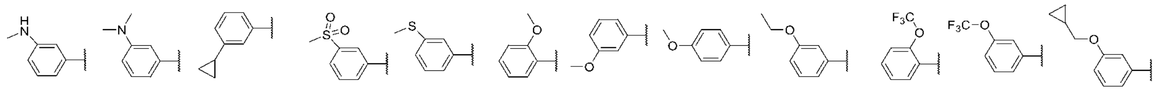

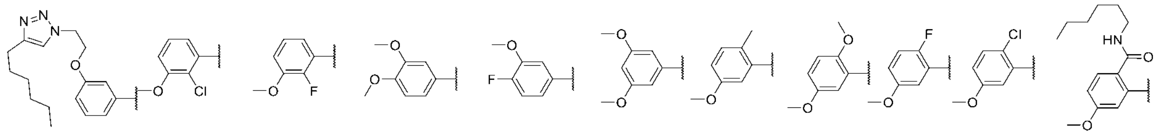

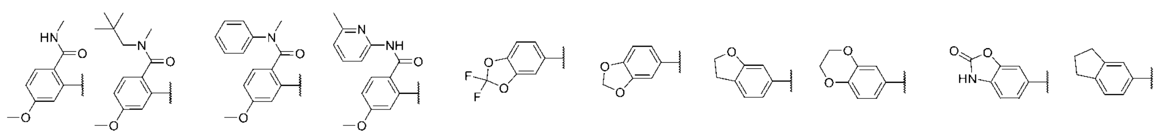

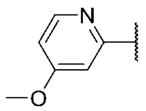 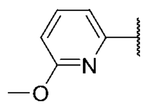 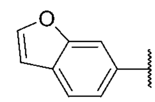 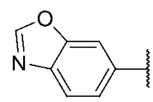;

such as

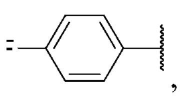, 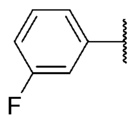, 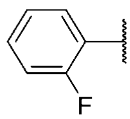, 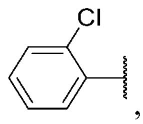, 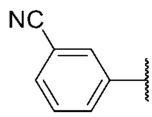, 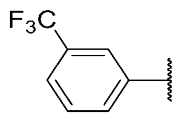,

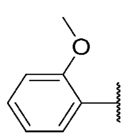, 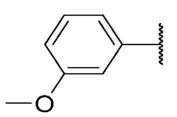, 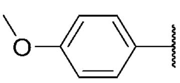, 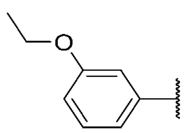, 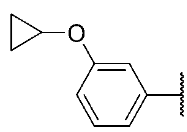,

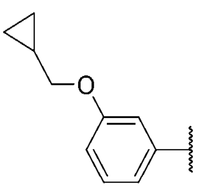, 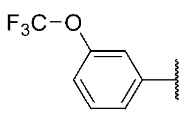, 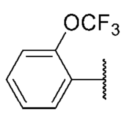, 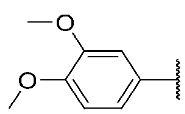, 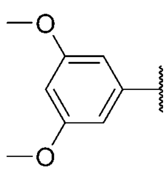, 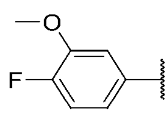,

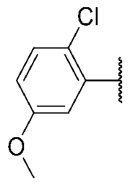, 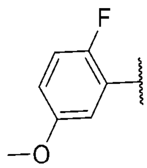, 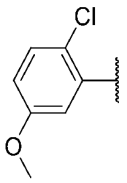, 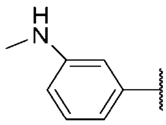, 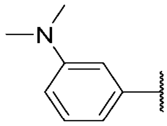, 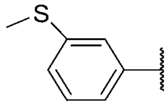,

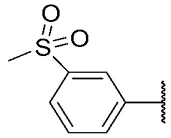, 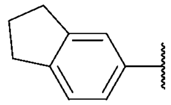, 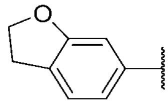, 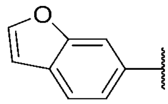, 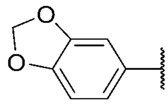, 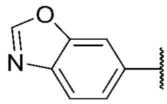,

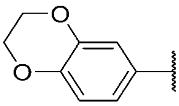, 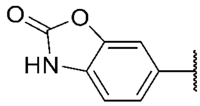, 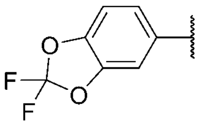, 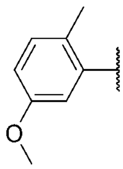, 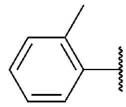, 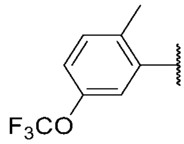,

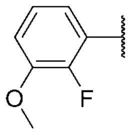, 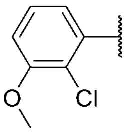, 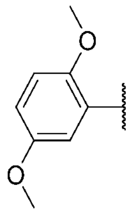, 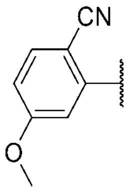, 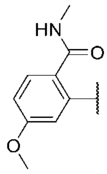, or 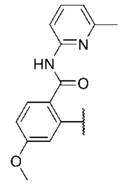;

(3) in $G^1$, the 5- to 10-membered heteroaryl substituted by one or more $G^{1-3}$ is 5- to 6-membered monocyclic heterocycloalkyl substituted by 1 or 2 $G^{1-3}$, and may further be

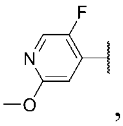, 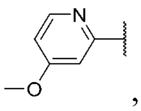, 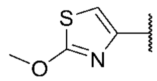, 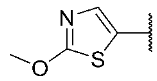, 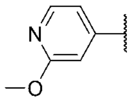, 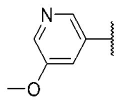, or 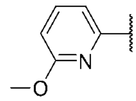;

(4) in $R^{1-1}$, $R^{1-2}$, and $R^{1-11}$, the $C_1$-$C_{12}$ alkyl substituted by one or more $R^{1-1-1}$ is $C_1$-$C_6$ alkyl substituted by one or more $R^{1-1-1}$, and may further be

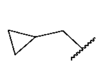 or 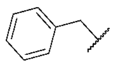,

such as

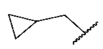;

(5) in $R^{1-1}$, $R^{1-2}$, and $R^{1-11}$, the $C_6$-$C_{14}$ aryl substituted by one or more $R^{1-1-3}$ is phenyl substituted by 1, 2, or 3 $R^{1-1-3}$, and may further be

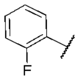, 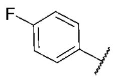, 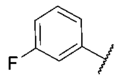, 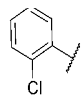,

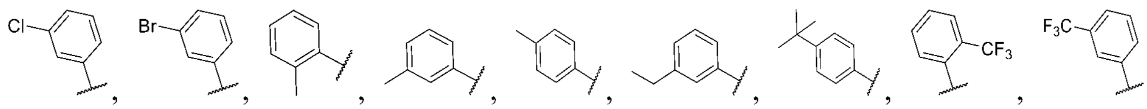

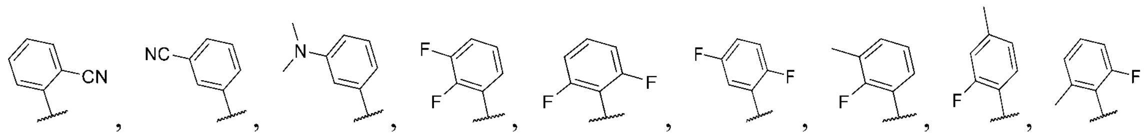

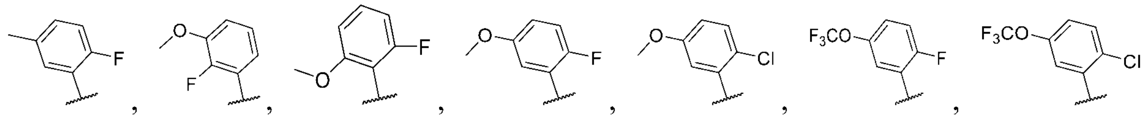

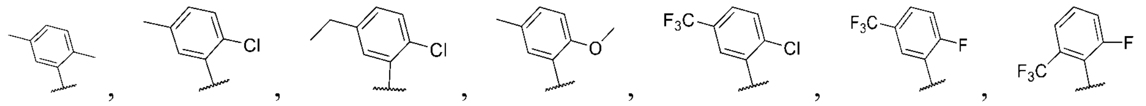

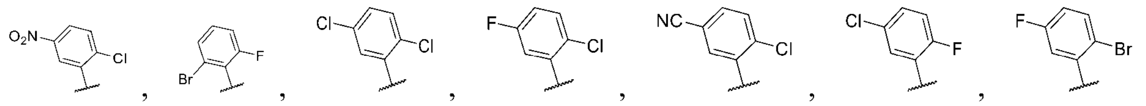

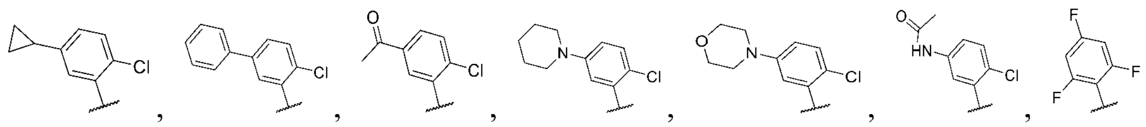

or

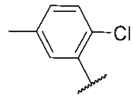,

such as

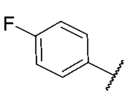, 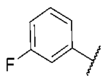, 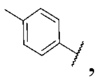, or 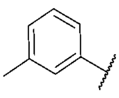;

(6) in $R^{1-1}$, $R^{1-2}$, and $R^{1-11}$, the 5- to 14-membered heteroaryl substituted by one or more $R^{1-1-4}$ is 5- to 6-membered monocyclic heteroaryl substituted by 1 or 2 $R^{1-1-3}$ or 9- to 10-membered fused heteroaryl substituted by 1 or 2 $R^{1-1-3}$, and may further be

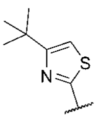, 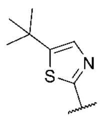,

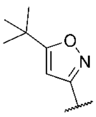, 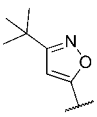, 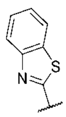, or 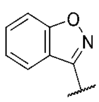;

(7) in ring A, the 4- to 8-membered heterocycloalkyl substituted by one or more $A^3$ is 4-to 6-membered

heterocycloalkyl substituted by one or more $A^1$ or 7- to 8-membered bridged heterocycloalkyl substituted by one or more $A^1$; the 4- to 6-membered heterocycloalkyl substituted by one or more $A^1$ may be

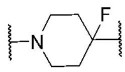 or 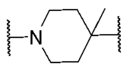;

the 7- to 8-membered bridged heterocycloalkyl substituted by one or more $A^1$ or

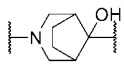;

**5.** The compound of formula I or the pharmaceutically acceptable salt thereof according to claim 1, wherein the compound of formula I or the pharmaceutically acceptable salt thereof satisfies one or more of the following conditions:

(1) ring A is

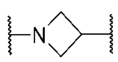, 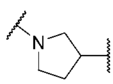, 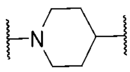, 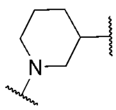, 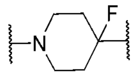, 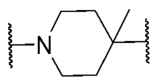,

or

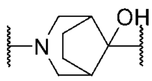;

(2) in $R^1$, in -C(=O)$NR^{1-1}R^{1-2}$, one of $R^{1-1}$ and $R^{1-2}$ may be H or $C_1$-$C_{12}$ alkyl, and the other may be -S(=O)$_2C_1$-$C_{12}$ alkyl, $C_1$-$C_{12}$ alkyl, $C_1$-$C_{12}$ alkyl substituted by one or more $R^{1-1-1}$, $C_3$-$C_{12}$ cycloalkyl, $C_3$-$C_{12}$ cycloalkyl substituted by one or more $R^{1-1-2}$, $C_6$-$C_{14}$ aryl, $C_6$-$C_{14}$ aryl substituted by one or more $R^{1-1-3}$, 5- to 14-membered heteroaryl, or 5- to 14-membered heteroaryl substituted by one or more $R^{1-1-4}$;
-C(=O)$NR^{1-1}R^{1-2}$ may further be the following group:

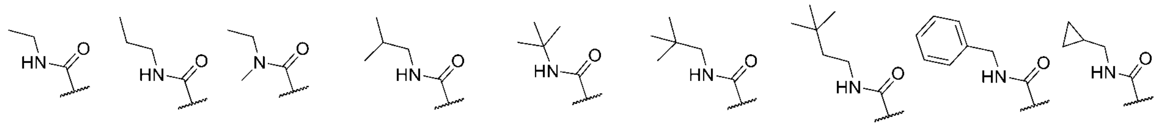

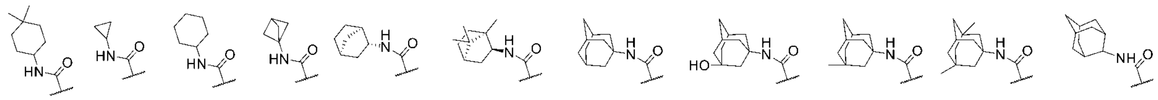

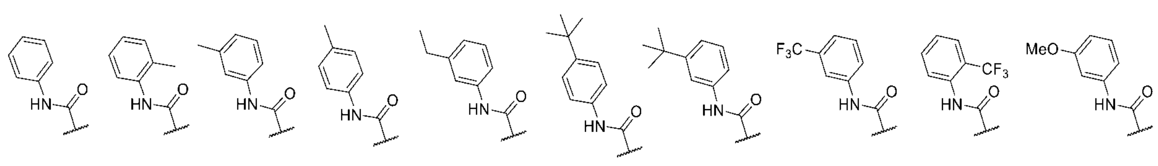

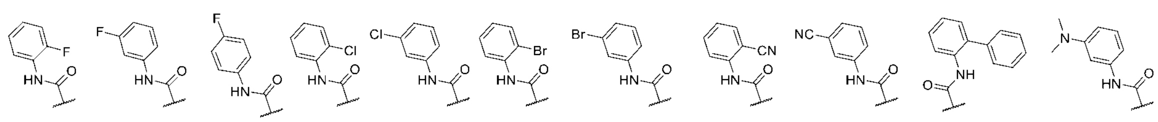

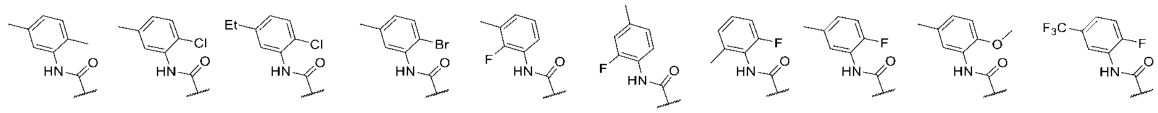

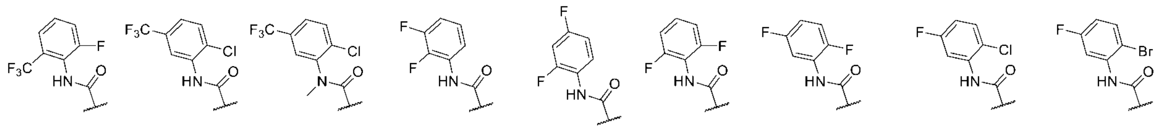

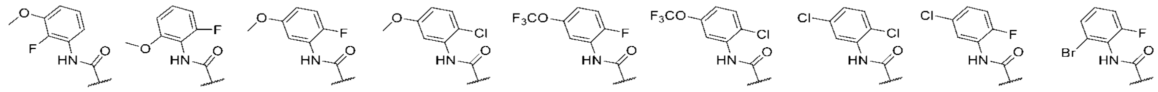

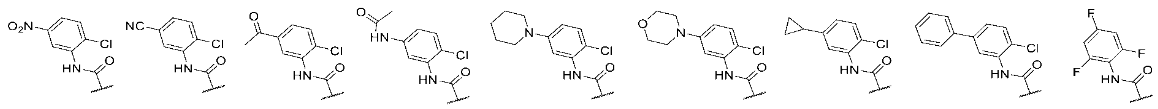

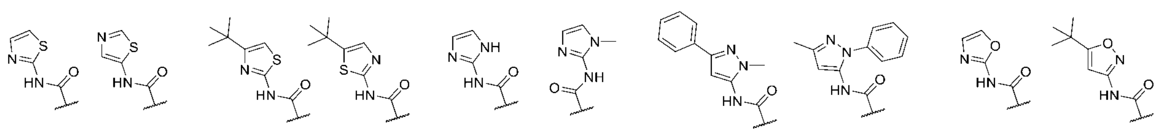

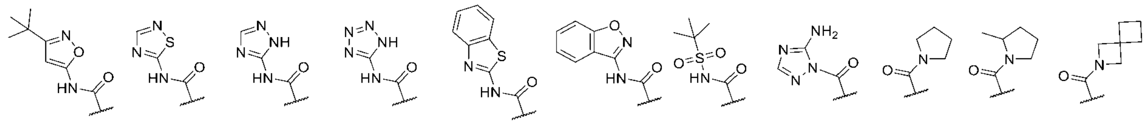
;

such as

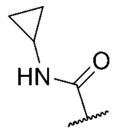, 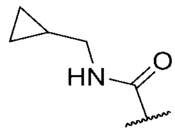, 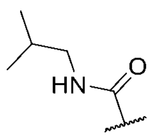, 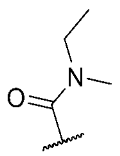, 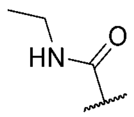, 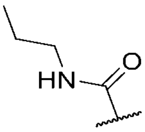,

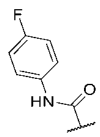, 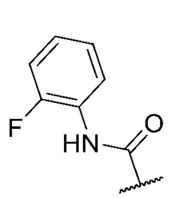, 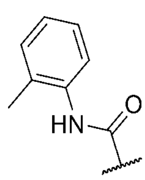, 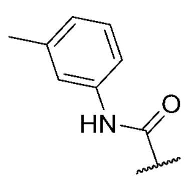, 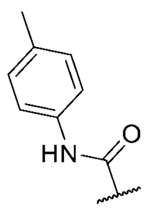, 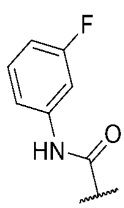, 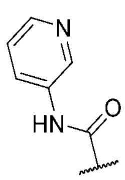,

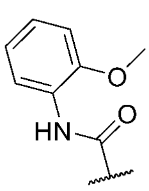, 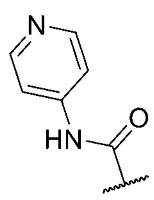, 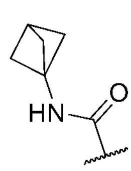 or 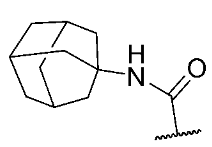;

(3) in $R^1$, the $C_1$-$C_6$ alkyl substituted by one or more $R^{1-3}$ is any one of the following groups:

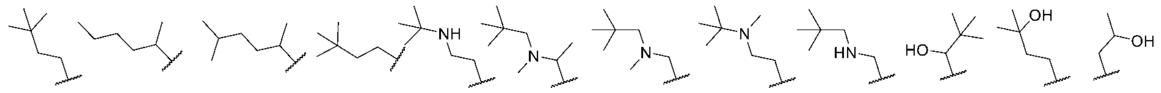

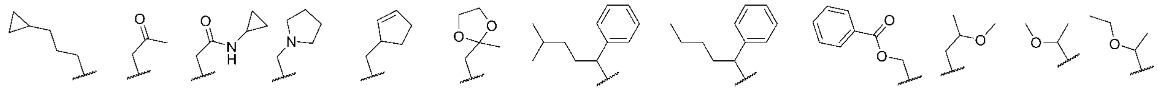

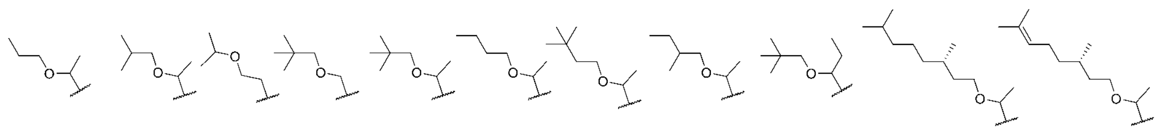

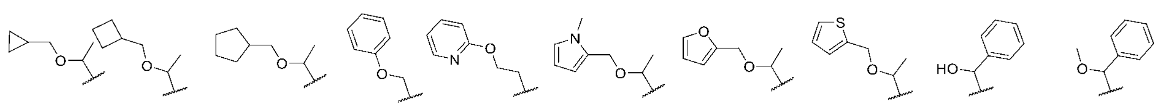

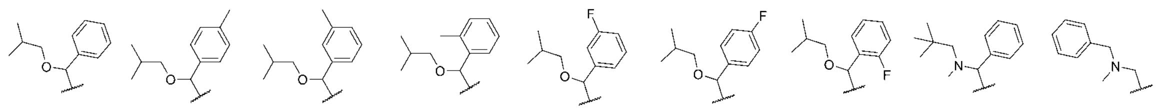

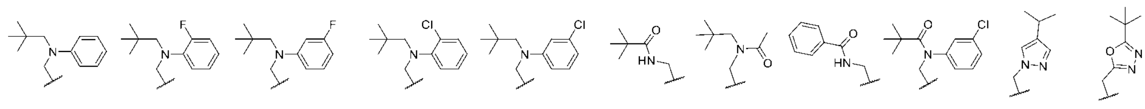;

(4) in $R^1$, the $C_2$-$C_6$ alkenyl substituted by one or more $R^{1-10}$ is

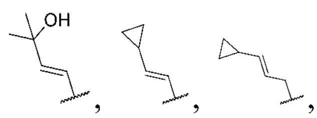,  ,  ,

or

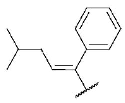;

(5) in $R^1$,-C(=O)$R^{1-11}$ is

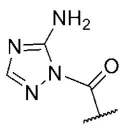;

(7) in ring B, the $C_3$-$C_{12}$ cycloalkyl substituted by one or more $R^{1-4}$ is $C_3$-$C_8$ cycloalkyl substituted by one or more $R^{1-4}$, and may further be

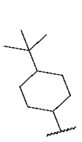;

(8) in ring B, the $C_3$-$C_{12}$ cycloalkenyl substituted by one or more $R^{1-5}$ is $C_3$-$C_8$ cycloalkenyl substituted by one or

more $R^{1-5}$, and may be

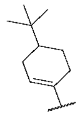;

(9) in ring B, the 3- to 12-membered heterocycloalkyl substituted by one or more $R^{1-6}$ is 3- to 8-membered heterocycloalkyl substituted by one or more $R^{1-6}$, and may be

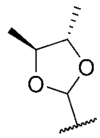,

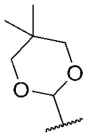, 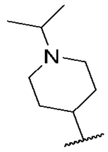, or 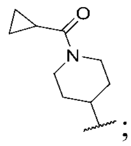;

(10) in ring B, the $C_3$-$C_{12}$ cycloalkenyl substituted by one or more $R^{1-5}$ is 3- to 8-membered heterocycloalkenyl substituted by one or more $R^{1-7}$, and may be

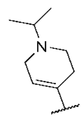;

(11) in ring B, the $C_6$-$C_{14}$ aryl substituted by one or more $R^{1-8}$ is any one of the following groups:

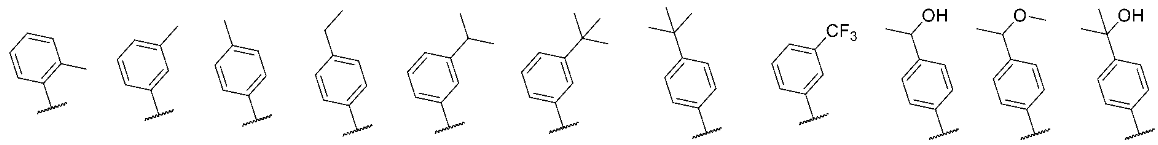

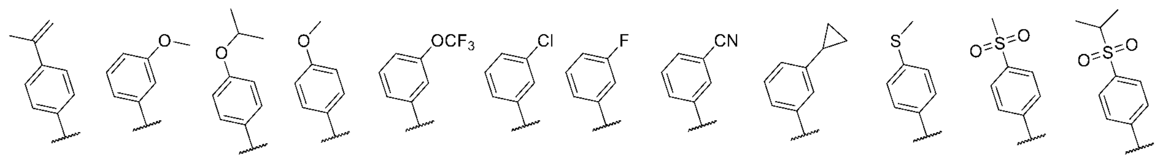

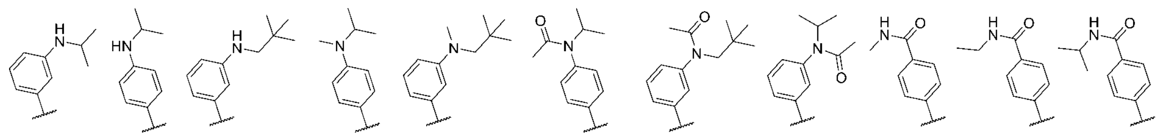

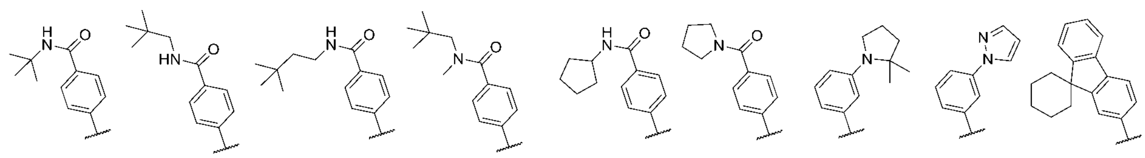,

such as

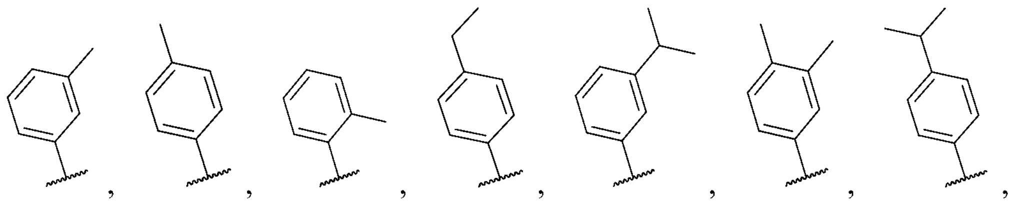

, , , , , , ,

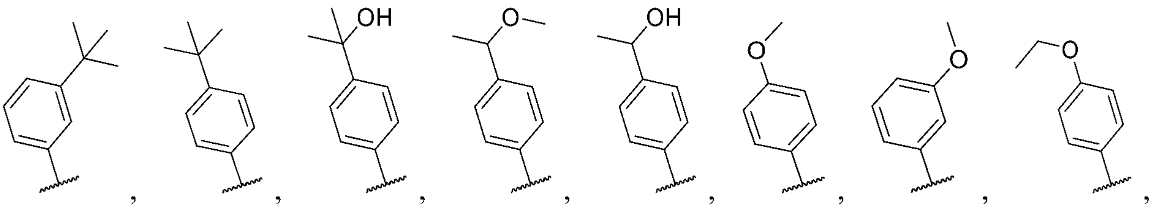

, , , , , , , ,

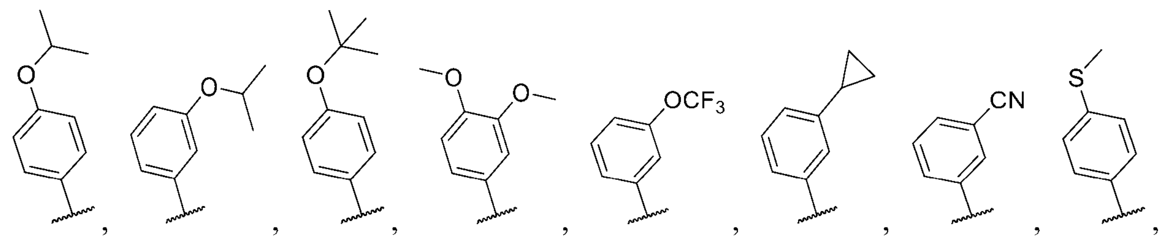

, , , , , , , ,

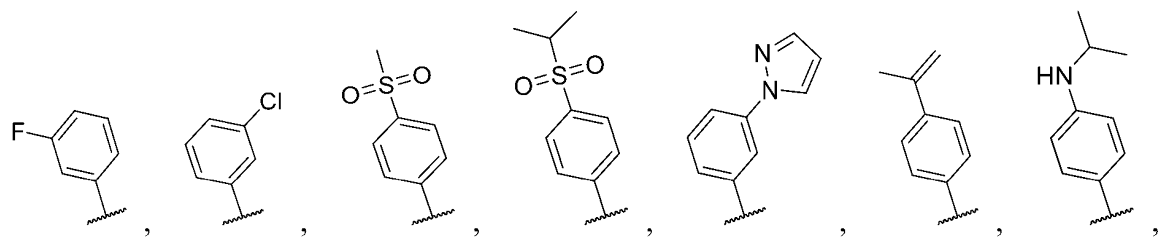

, , , , , , ,

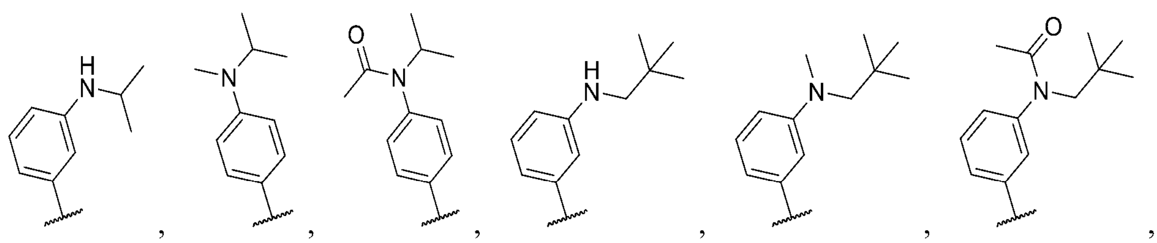

, , , , , ,

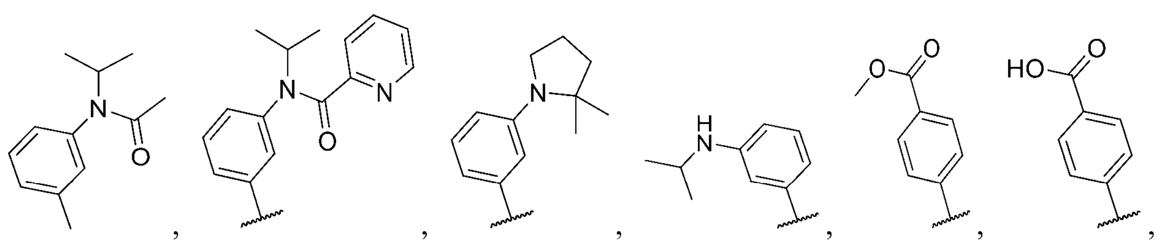

, , , , , ,

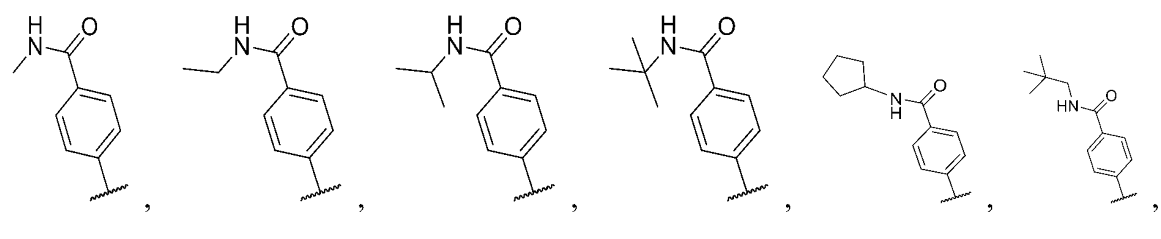

, , , , , ,

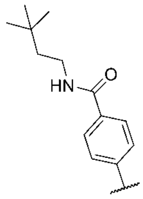, 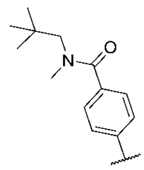, 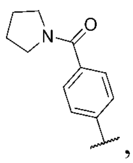, 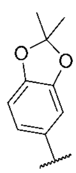, or 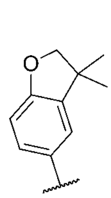;

(12) in ring B, the 5- to 10-membered heteroaryl substituted by one or more $R^{1-9}$ is any one of the following groups:

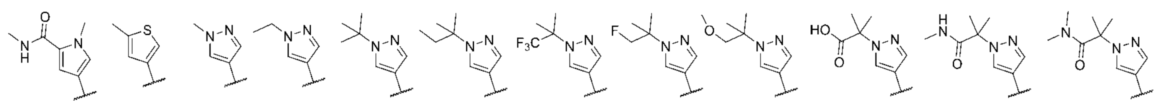

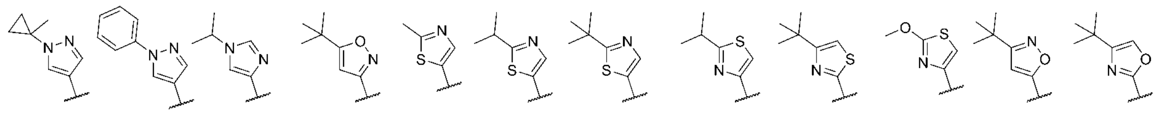

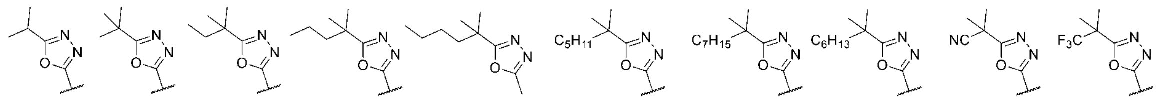

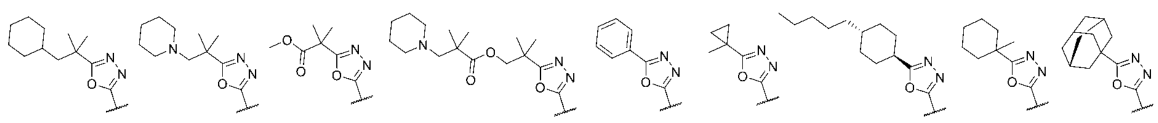

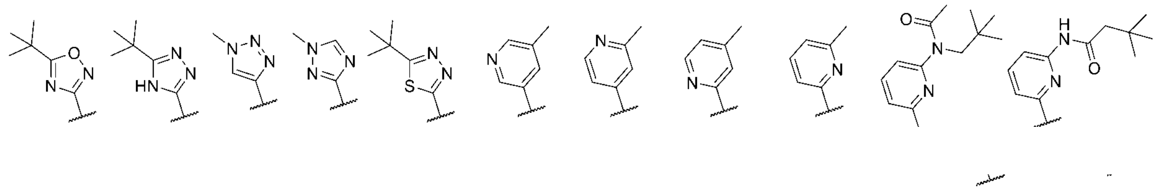

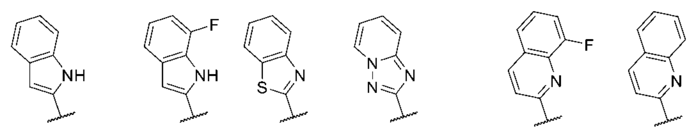,

such as

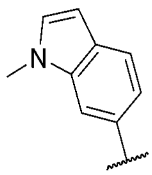, 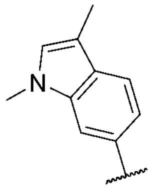, 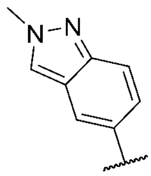, 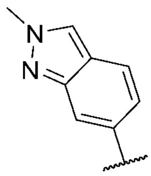, 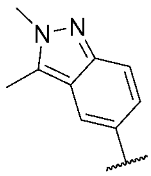, 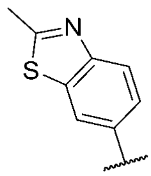,

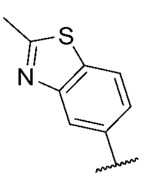, 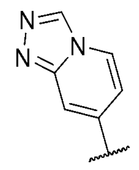, 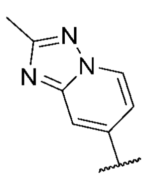, 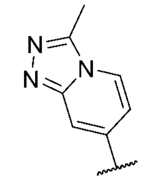, 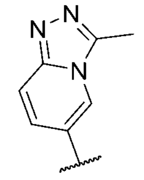, 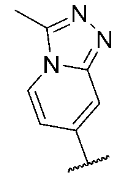, 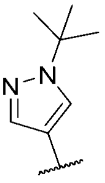,

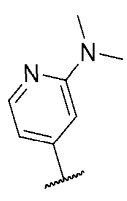, 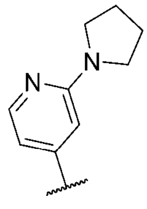, 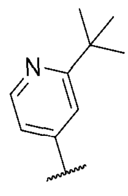, 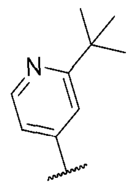, 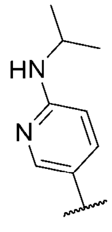, 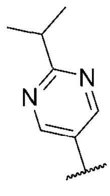, 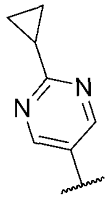, 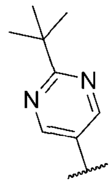,

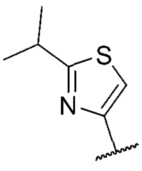, 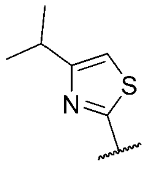, 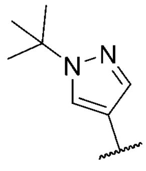, 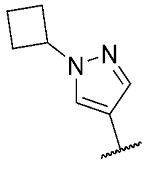, 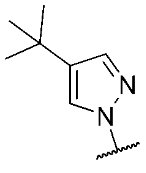, 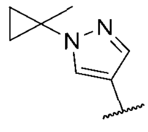, 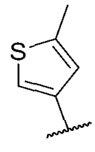,

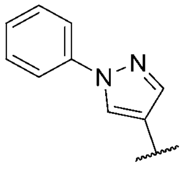, 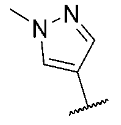, 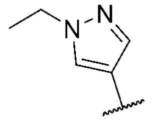, 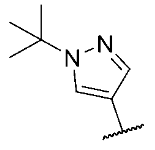, 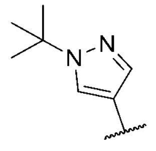, 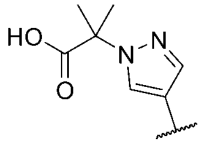,

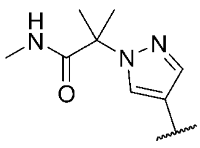, 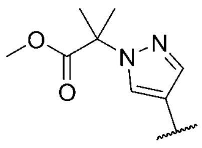, 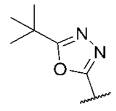, 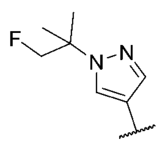, 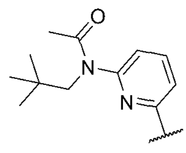, 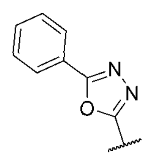,

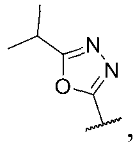, or 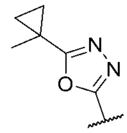;

(13)

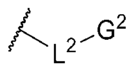

is any one of the following groups:

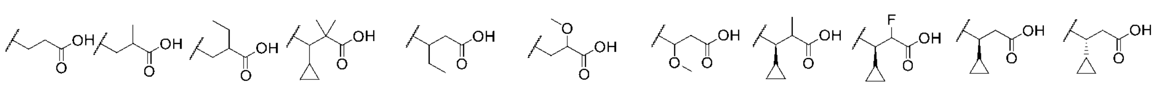

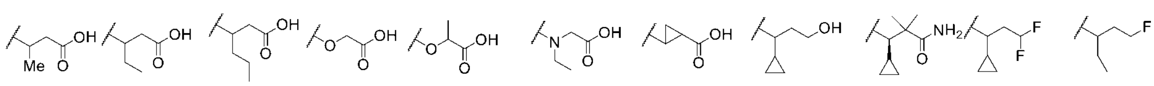

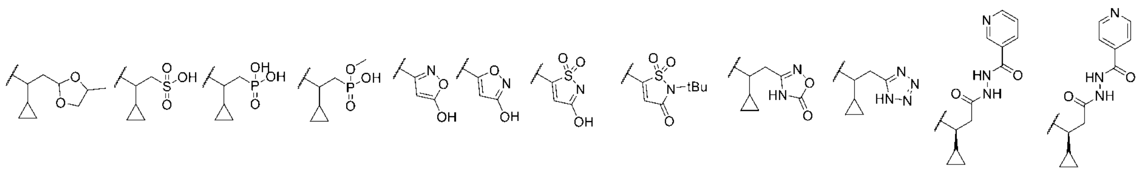,

such as

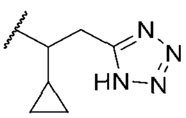, 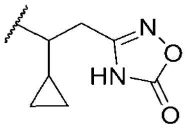, 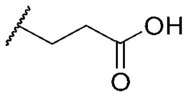, 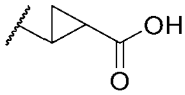, 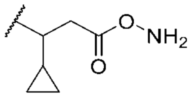,

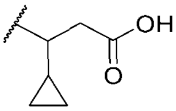, 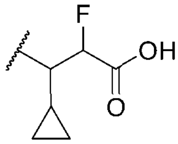, 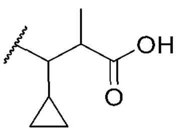, 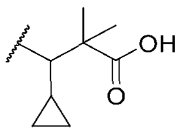, 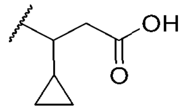,

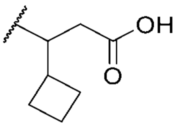, 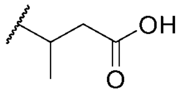, 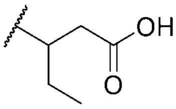, 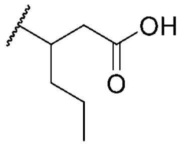, 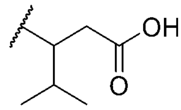,

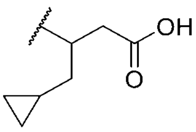, 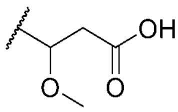, 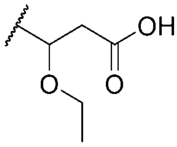, 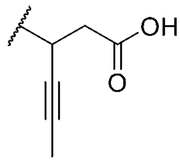, 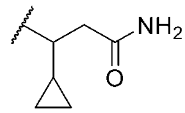,

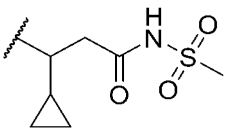, 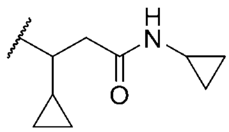, 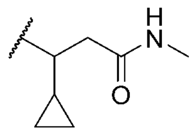, 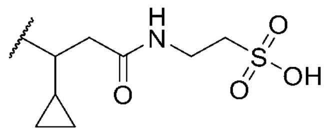,

or

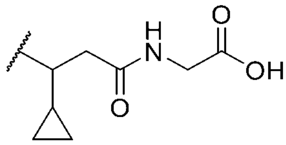;

preferably,

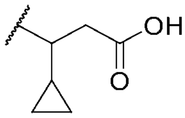

is

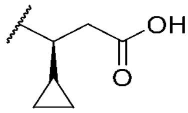.

**6.** The compound of formula I or the pharmaceutically acceptable salt thereof according to claim 1, wherein the compound of formula I or the pharmaceutically acceptable salt thereof satisfies one or more of the following conditions:

(1) X is O;

(2) Z and Y are C;
(3) $R^2$ is hydrogen;
(4) $G^1$ is $C_1$-$C_6$ alkyl, $C_6$-$C_{14}$ aryl, $C_6$-$C_{14}$ aryl substituted by one or more $G^{1-2}$ 5- to 10-membered heteroaryl, or 5- to 10-membered heteroaryl substituted by one or more $G^{1-3}$;
(5) each $G^{1-2}$ is independently halogen, cyano, $-NG^{1-1-1}G^{1-1-2}$, $-S(=O)_2$-$C_1$-$C_6$ alkyl, $C_1$-$C_6$ alkyl, $C_1$-$C_6$ alkyl substituted by one or more $G^{1-1-5}$ $C_1$-$C_6$ alkoxy, $C_1$-$C_6$ alkoxy substituted by one or more $G^{1-1-6}$, -S-$C_1$-$C_6$ alkyl, $C_3$-$C_8$ cycloalkyl, $C_3$-$C_8$ cycloalkyl substituted by one or more $G^{1-1-8}$, or -O-$C_3$-$C_8$ cycloalkyl;
(6) each $G^{1-3}$ is independently halogen or $C_1$-$C_6$ alkoxy;
(7) ring A is 4- to 6-membered heterocycloalkyl or 4- to 6-membered heterocycloalkyl substituted by one or more $A^1$; the 4- to 6-membered heterocycloalkyl and the 4- to 6-membered heterocycloalkyl substituted by one or more $A^1$ have 1 heteroatom being N; preferably, when ring A is 4- to 6-membered heterocycloalkyl or 4- to 6-membered heterocycloalkyl substituted by one or more $A^3$, then $L^1$ is a bond, and $G^1$ is connected to ring A via a heteroatom;
(8) each $A^1$ is independently halogen or $C_1$-$C_6$ alkyl;
(9)

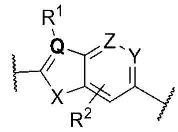

is

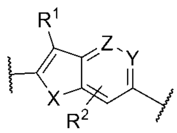 or 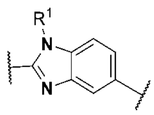, and 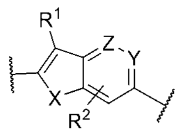

may be

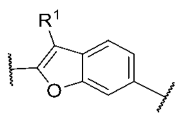 or 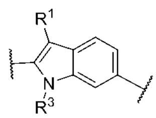.

**7.** The compound of formula I or the pharmaceutically acceptable salt thereof according to claim 1, wherein the compound of formula I is selected from the following general formulas I-1 to I-14:

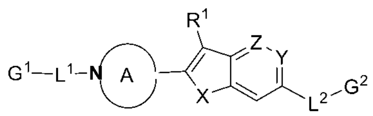

I-1

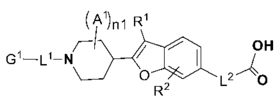

I-3

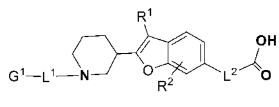

I-4

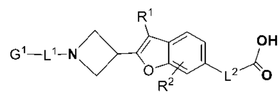

I-5

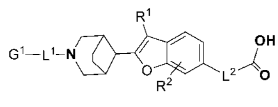

I-6

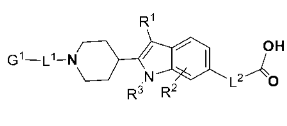

I-7

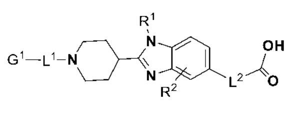

I-8

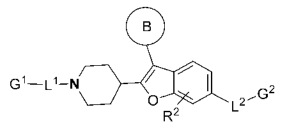

I-9

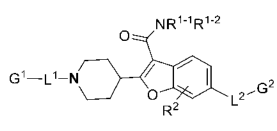

I-10

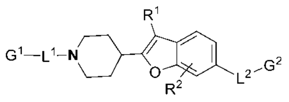
I-11
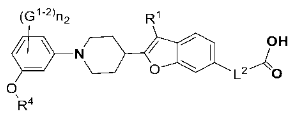
I-12
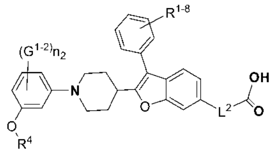
I-13
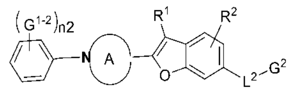
I-14
;

in formula I-3, n1 is 0, 1, or 2;
in formula I-11, $R^1$ is $C_1$-$C_6$ alkyl, $C_1$-$C_6$ alkyl substituted by one or more $R^{1-3}$, $C_2$-$C_6$ alkenyl, or $C_2$-$C_6$ alkenyl substituted by one or more $R^{1-10}$;
in formula I-12, n2 is 0, 1, or 2; $R^4$ is $C_1$-$C_6$ alkyl, or $R^4$ and $G^{1-2}$ together form $-(CH_2)n_3-$, wherein $n_3$ is 1, 2, or 3, and 1 or 2 of the $-(CH_2)n_3-$ in $-(CH_2)n_3-$ are optionally replaced by a group selected from: $-CHR^{4a}-$, $-CR^{4b}R^{4c}-$, -NH-, -O-, and -C(=O)-; $R^{4a}$, $R^{4b}$, and $R^{4c}$ are independently $C_1$-$C_6$ alkyl or halogen;
in formula I-13, n2 is 0, 1, or 2;
in formula I-14, n2 is 0, 1, or 2.

**8.** The compound of formula I or the pharmaceutically acceptable salt thereof according to claim 7, wherein

in formula I-1,
ring A is azetidinyl, pyrrolidinyl, or piperidinyl;
X is O; Z and Y are C;
more preferably, $L^2$ is $C_1$-$C_6$ alkylene substituted by one or more $L^{2-1}$, at least one $L^{2-1}$ is $C_3$-$C_8$ cycloalkyl, and $L^{2-1}$ is substituted at the terminal group of $L^2$;
in formula I-9,
ring B is $C_3$-$C_{12}$ cycloalkyl, $C_3$-$C_{12}$ cycloalkyl substituted by one or more $R^{1-4}$, $C_3$-$C_{12}$ cycloalkenyl, $C_3$-$C_{12}$ cycloalkenyl substituted by one or more $R^{1-5}$, 3- to 12-membered heterocycloalkyl, 3- to 12-membered heterocycloalkyl substituted by one or more $R^{1-6}$, 3- to 12-membered heterocycloalkenyl, 3- to 12-membered heterocycloalkenyl substituted by one or more $R^{1-7}$, $C_6$-$C_{14}$ aryl, $C_6$-$C_{14}$ aryl substituted by one or more $R^{1-8}$, 5- to 14-membered heteroaryl, or 5- to 14-membered heteroaryl substituted by one or more $R^{1-9}$;
each $R^{1-4}$, each $R^{1-5}$, each $R^{1-6}$, each $R^{1-7}$, each $R^{1-8}$, and each $R^{1-9}$ is independently halogen, cyano, hydroxyl, $-NR^{1-3-1a}R^{1-3-2a}$, $-C(=O)NR^{1-3-3a}R^{1-3-4a}$, $-C(=O)R^{1-3-5a}$, $-S(=O)_2$-$C_1$-$C_{12}$ alkyl, - S-$C_1$-$C_{12}$ alkyl, $C_1$-$C_{12}$ alkyl, $C_1$-$C_{12}$ alkyl substituted by one or more $R^{1-3-6a}$, $C_1$-$C_{12}$ alkoxy, $C_1$-$C_{12}$ alkoxy substituted by one or more $R^{1-3-7a}$, 5- to 14-membered heteroaryl, 5- to 14-membered heteroaryl substituted by one or more $R^{1-3-8a}$, $C_3$-$C_{12}$ cycloalkyl, $C_3$-$C_{12}$ cycloalkyl substituted by one or more $R^{1-3-9a}$, $C_2$-$C_6$ alkenyl, $C_2$-$C_6$ alkenyl substituted by one or more $R^{1-3-10a}$, $C_6$-$C_{14}$ aryl, $C_6$-$C_{14}$ aryl substituted by one or more $R^{1-3-11a}$, -O-$C_6$-$C_{14}$ aryl, -O-C(=O) $C_6$-$C_{14}$ aryl, -O-5- to 10-membered heteroaryl, 3- to 12-membered heterocycloalkyl, or 3- to 12-membered heterocycloalkyl substituted by one or more $R^{1-3-13a}$; alternatively, any two adjacent $R^{1-8}$, together with the carbon atom to which they are attached, form a $C_3$-$C_{14}$ cycloalkyl;
$R^{1-3-1a}$, $R^{1-3-2a}$, $R^{1-3-3a}$, and $R^{1-3-4a}$ are independently H, $C_1$-$C_6$ alkyl, $-C(=O)R^{1-3-1-1a}$ or $C_3$-$C_8$ cycloalkyl;
$R^{1-3-1-1a}$ is $C_1$-$C_6$ alkyl;
$R^{1-3-5a}$ is $C_3$-$C_8$ cycloalkyl;
each $R^{1-3-6a}$ and each $R^{1-3-7a}$ is independently halogen, hydroxyl, carboxyl, cyano, $C_1$-$C_6$ alkoxy, $C_3$-$C_8$ cycloalkyl, 3- to 12-membered heterocycloalkyl, or -O-C(=O)-$C_1$-$C_6$ alkyl-3- to 12-membered heterocycloalkyl;
each $R^{1-3-8a}$, each $R^{1-3-9a}$, each $R^{1-3-10a}$, each $R^{1-3-11a}$, and each $R^{1-3-12a}$ is independently $C_1$-$C_6$ alkyl;
in formula I-10, $R^{1-1}$ and $R^{1-2}$ are independently H, $-S(=O)_2C_1$-$C_{12}$ alkyl, $C_1$-$C_{12}$ alkyl, $C_1$-$C_{12}$ alkyl substituted by one or more $R^{1-1-1}$, $C_3$-$C_{12}$ cycloalkyl, $C_3$-$C_{12}$ cycloalkyl substituted by one or more $R^{1-1-2}$, $C_6$-$C_{14}$ aryl, $C_6$-$C_{14}$ aryl substituted by one or more $R^{1-1-3}$, 5- to 14-membered heteroaryl, or 5- to 14-membered heteroaryl substituted by one or more $R^{1-1-4}$,
alternatively, $R^{1-1}$ and $R^{1-2}$, together with the N atom to which they are attached, form a 3-to 14-membered heterocycloalkyl or a 3- to 14-membered heterocycloalkyl substituted by one or more $R^{1-1-5}$;
each $R^{1-1-1}$ and each $R^{1-1-2}$ is independently halogen, cyano, nitro, hydroxyl, amino, - NH($C_1$-$C_{12}$ alkyl), -N($C_1$-$C_{12}$ alkyl)$_2$, $C_3$-$C_{12}$ cycloalkyl, $C_3$-$C_{12}$ cycloalkyl substituted by one or more $R^{1-1-1-3}$, $C_6$-$C_{14}$ aryl, 3- to 12-membered heterocycloalkyl, or 5- to 14-membered heteroaryl;
each $R^{1-1-3}$, $R^{1-1-4}$, and each $R^{1-1-5}$ is independently halogen, cyano, nitro, hydroxyl, amino, -NH($C_1$-$C_{12}$ alkyl), -N($C_1$-$C_{12}$ alkyl)$_2$, -C(=O)-$C_1$-$C_{12}$ alkyl, -NHC(=O)-$C_1$-$C_{12}$ alkyl, $C_1$-$C_{12}$ alkyl, $C_1$-$C_{12}$ alkyl substituted by one or more $R^{1-1-1-1}$, $C_1$-$C_{12}$ alkoxy, $C_1$-$C_{12}$ alkoxy substituted by one or more $R^{1-1-1-2}$, $C_3$-$C_{12}$ cycloalkyl, $C_3$-$C_{12}$ cycloalkyl substituted by one or more $R^{1-1-1-3}$, $C_6$-$C_{14}$ aryl, 3- to 12-membered heterocycloalkyl, or 5- to 14-membered heteroaryl;

each $R^{1-1-1-1}$, each $R^{1-1-1-2}$, and each $R^{1-1-1-3}$ is independently halogen, $C_1$-$C_{12}$ alkyl, or $C_3$-$C_{12}$ cycloalkyl;

in formula I-11,

each $R^{1-3}$ and each $R^{1-10}$ is independently deuterium, halogen, cyano, hydroxyl, -$NR^{1-3-1}R^{1-3-2}$ -C(=O)$NR^{1-3-3}R^{1-3-4}$, -C(=O)$R^{1-3-5}$, -S(=O)$_2$-$C_1$-$C_{12}$ alkyl, -S-$C_1$-$C_{12}$ alkyl, $C_1$-$C_{12}$ alkyl, $C_1$-$C_{12}$ alkyl substituted by one or more $R^{1-3-6}$, $C_1$-$C_{12}$ alkoxy, $C_1$-$C_{12}$ alkoxy substituted by one or more $R^{1-3-7}$, 5- to 14-membered heteroaryl, 5- to 14-membered heteroaryl substituted by one or more $R^{1-3-8}$, $C_3$-$C_{12}$ cycloalkyl, $C_3$-$C_{12}$ cycloalkyl substituted by one or more $R^{1-3-9}$, $C_2$-$C_6$ alkenyl, $C_6$-$C_{14}$ aryl, $C_6$-$C_{14}$ aryl substituted by one or more $R^{1-3-11}$, -O-$C_6$-$C_{14}$ aryl, -O-C(=O)$C_6$-$C_{14}$ aryl, -O-5- to 14-membered heteroaryl, $C_3$-$C_8$ cycloalkenyl, $C_3$-$C_8$ cycloalkenyl substituted by one or more $R^{1-3-12}$, 3- to 12-membered heterocycloalkyl, or 3- to 12-membered heterocycloalkyl substituted by one or more $R^{1-3-13}$;

$R^{1-3-1}$, $R^{1-3-2}$, $R^{1-3-3}$, and $R^{1-3-4}$ are independently H, $C_1$-$C_6$ alkyl, -$C_1$-$C_6$ alkyl-$C_6$-$C_{14}$ aryl, -C(=O)$R^{1-3-1-1}$, $C_6$-$C_{14}$ aryl, or $C_6$-$C_{14}$ aryl substituted by one or more $R^{1-3-1-4}$

each $R^{1-3-6}$ and each $R^{1-3-7}$ is independently $C_3$-$C_8$ cycloalkyl, 3- to 8-membered heterocycloalkyl, $C_2$-$C_6$ alkenyl, 5- to 10-membered heteroaryl, or 5- to 10-membered heteroaryl substituted by one or more $C_1$-$C_6$ alkyl groups;

each $R^{1-3-8}$, each $R^{1-3-9}$, each $R^{1-3-10}$, each $R^{1-3-11}$, each $R^{1-3-12}$, and each $R^{1-3-13}$ is independently halogen, hydroxyl, carboxyl, cyano, $C_1$-$C_6$ alkyl, or $C_1$-$C_6$ alkoxy;

in formula I-13, $R^4$ is $C_1$-$C_6$ alkyl; $G^{1-2}$ is halogen; $R^4$ is $C_1$-$C_6$ alkyl;

in formula I-14, $R^2$ is hydrogen or halogen.

**9.** The compound of formula I or the pharmaceutically acceptable salt thereof according to claim 1, wherein the compound of formula I is any one of the following compounds:

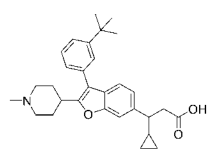 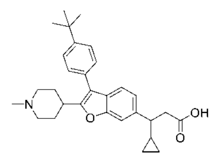 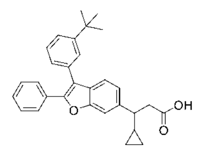 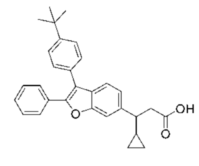

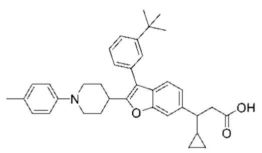 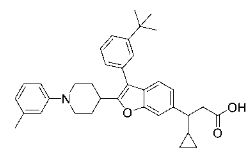 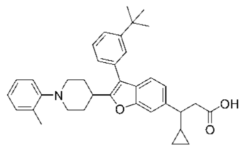 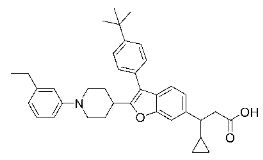

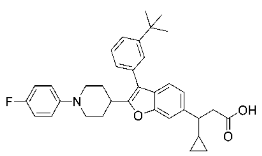 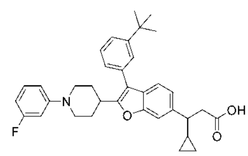 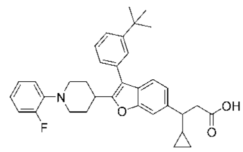 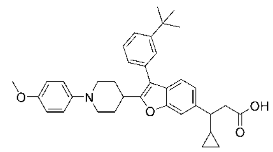

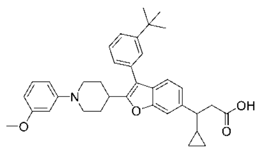 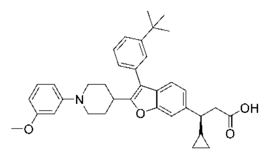 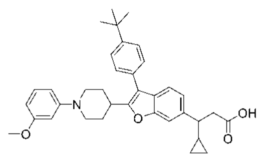 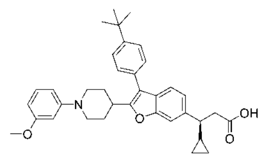

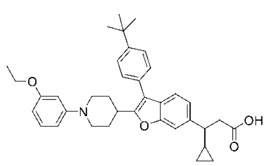 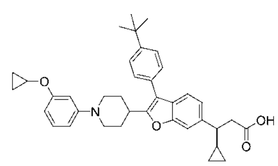 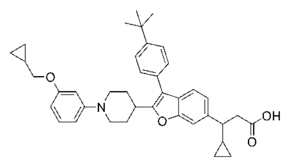 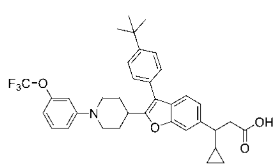

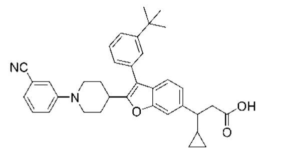
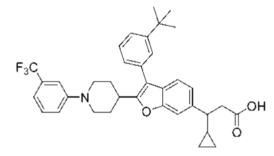
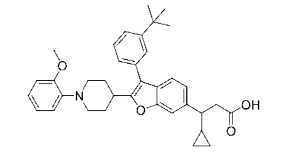
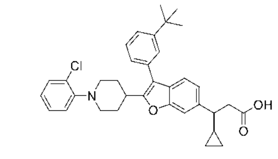

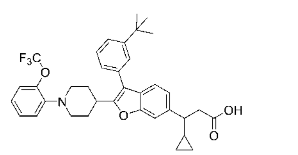
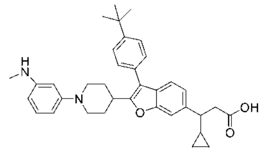
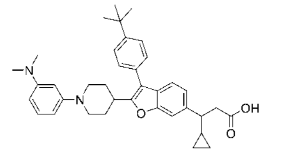
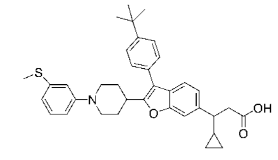

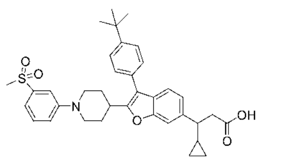
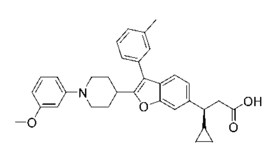
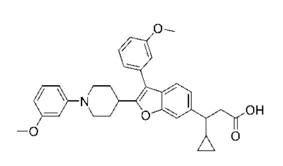
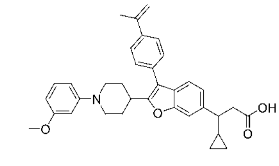

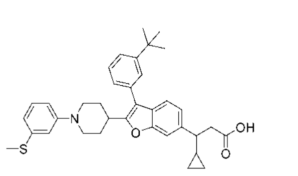
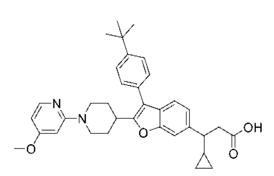
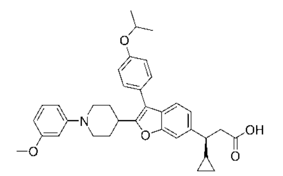
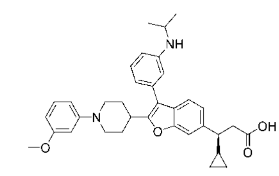

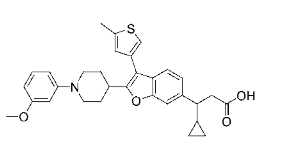
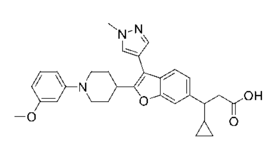
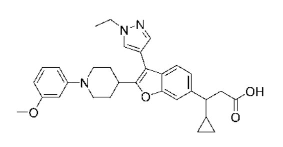
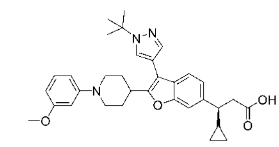

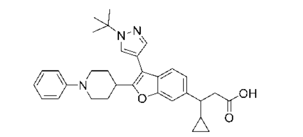
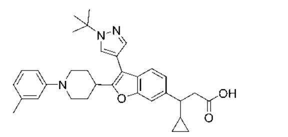
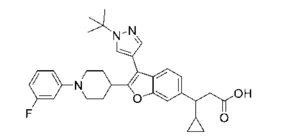
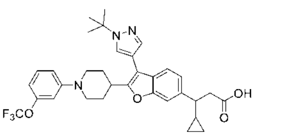

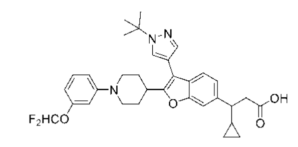
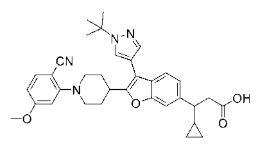
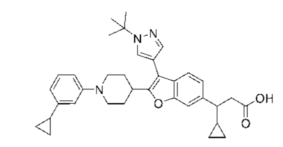
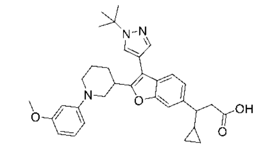

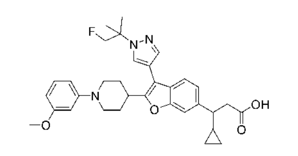
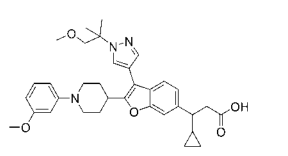
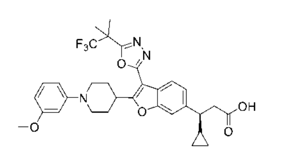
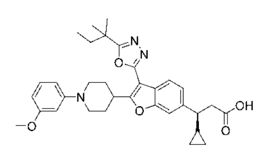

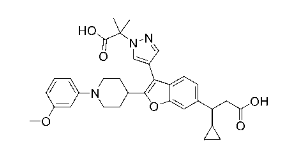
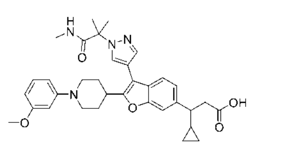
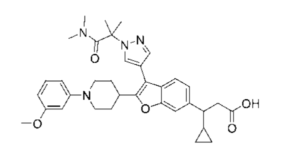
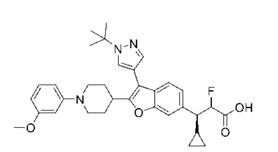

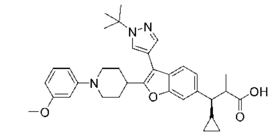
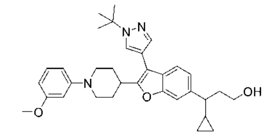
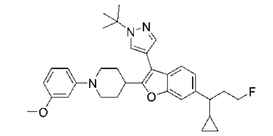
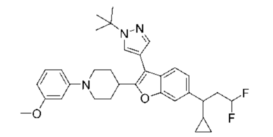
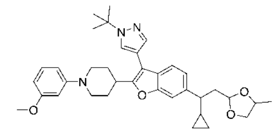
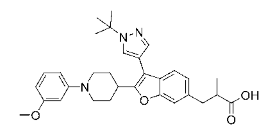
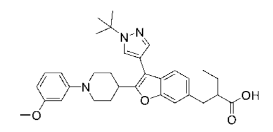
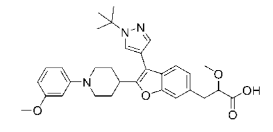
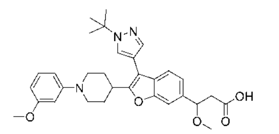
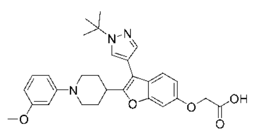
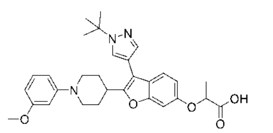
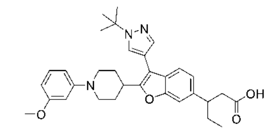
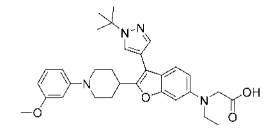
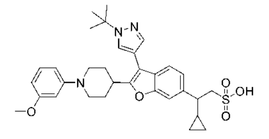
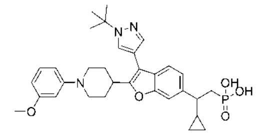
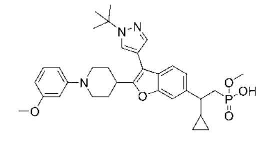
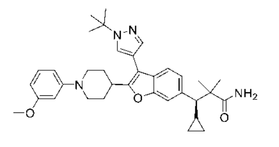
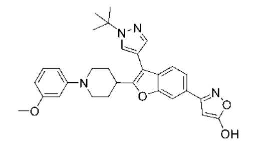
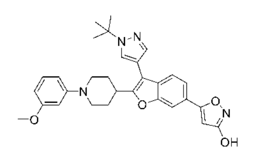
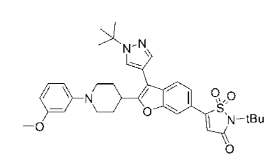
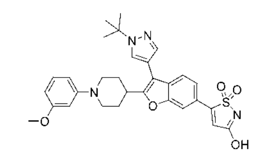
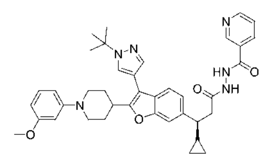
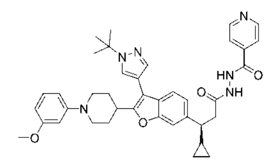
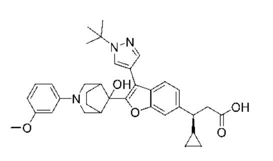
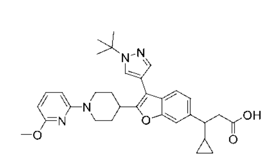
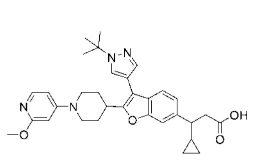
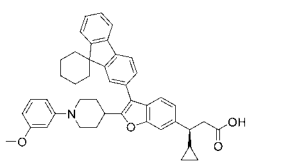
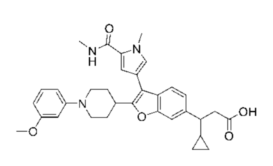
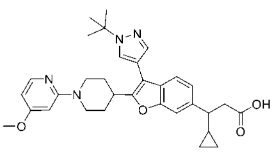
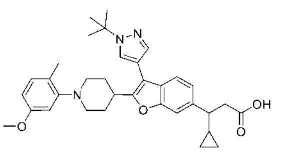
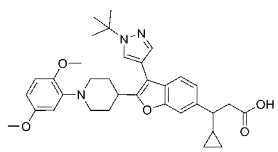
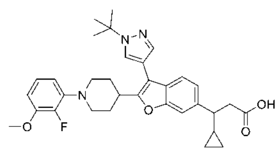
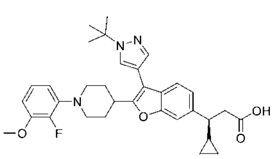
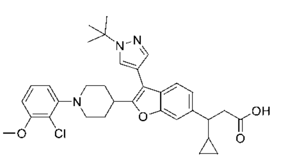
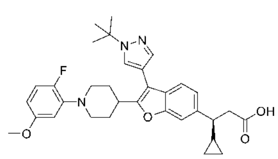
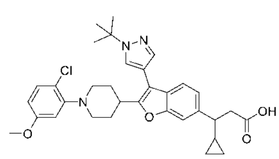

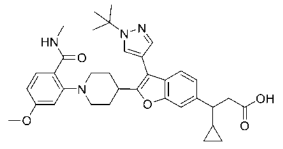
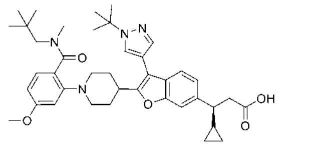
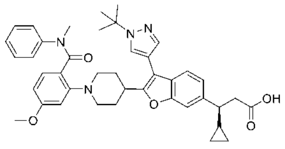
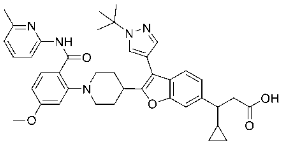
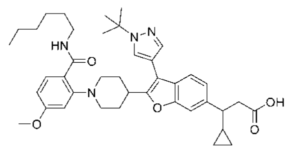
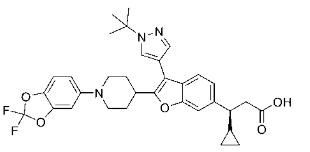
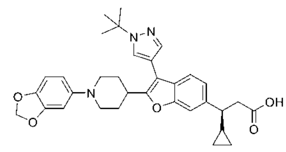
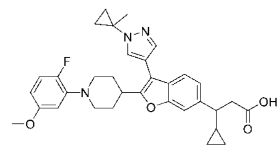
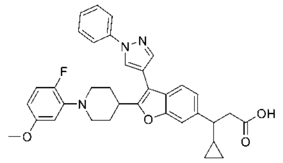
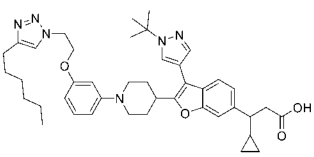
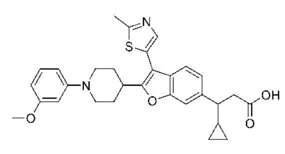
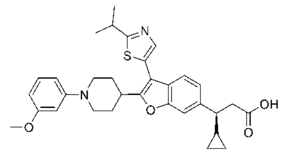
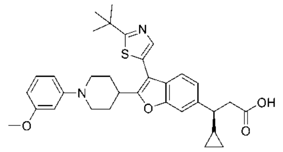
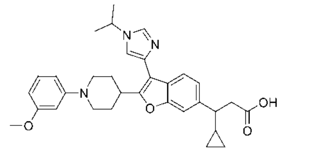
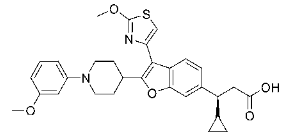
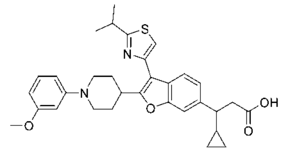
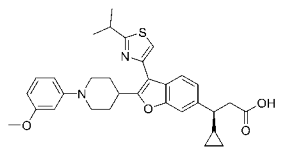
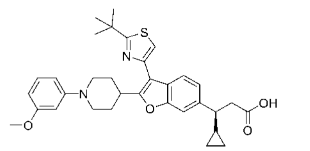
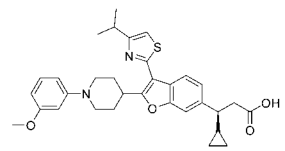
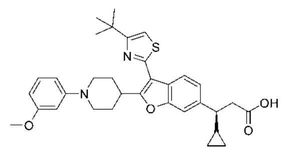
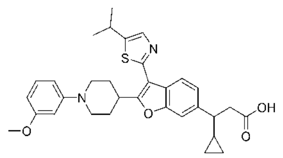
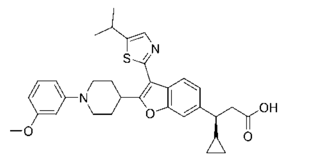
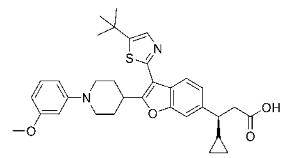
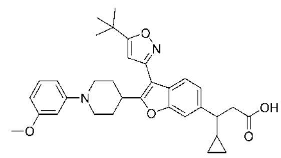
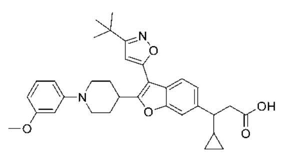
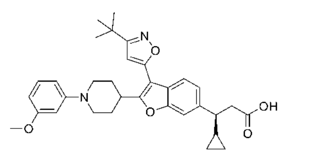
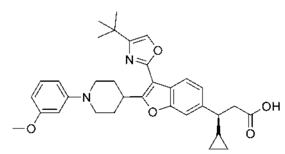
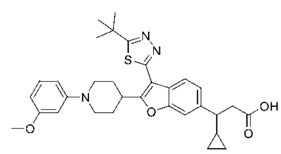
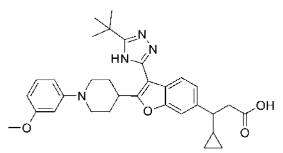
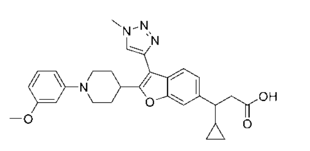
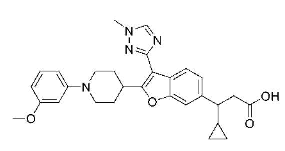
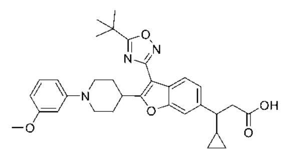
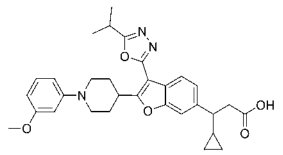
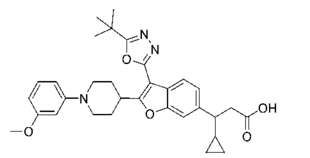
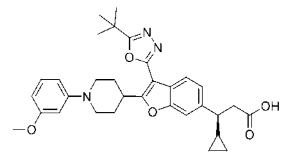
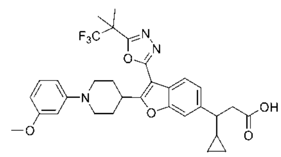

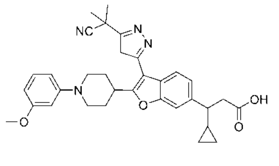 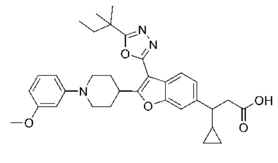 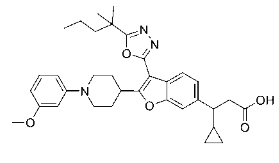 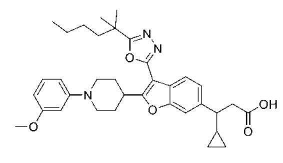

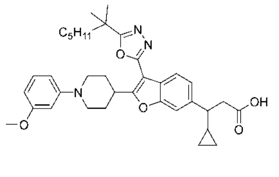 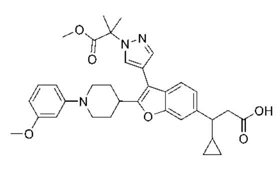 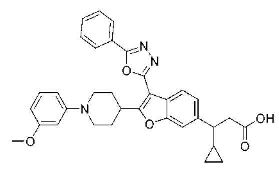 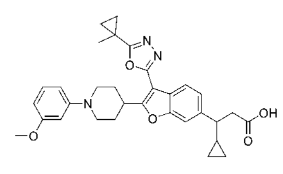

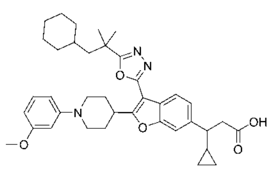 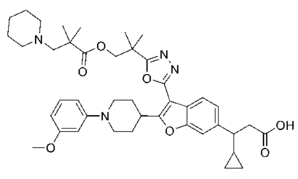 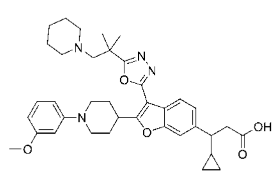 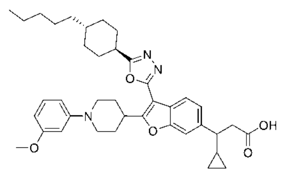

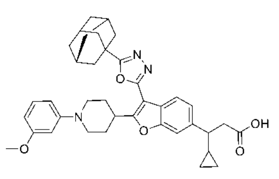 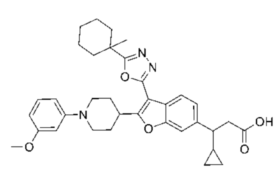 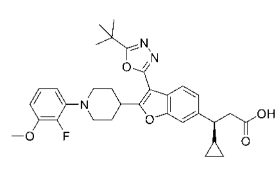 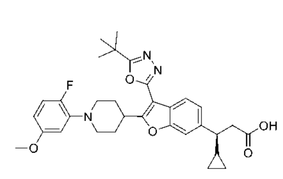

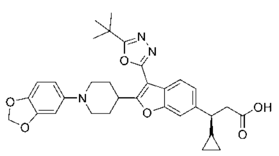 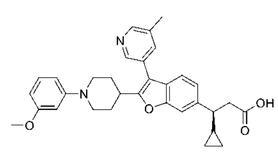 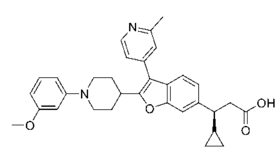 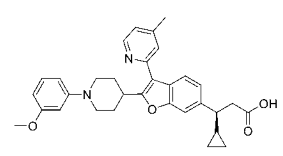

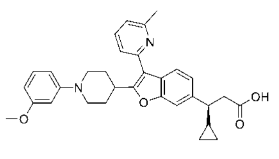 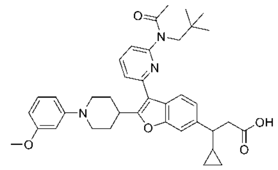 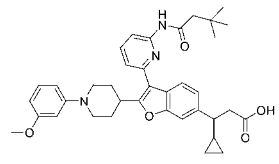 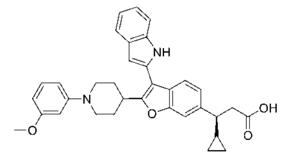

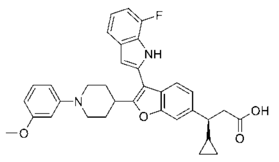 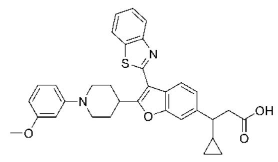 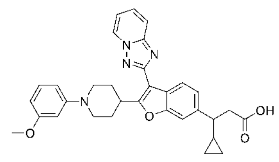 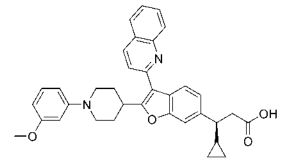

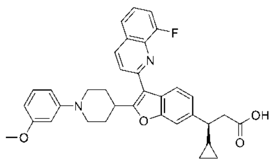 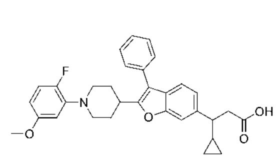 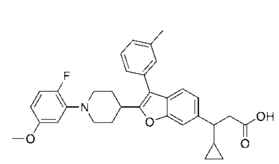 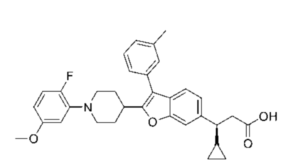

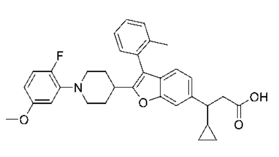 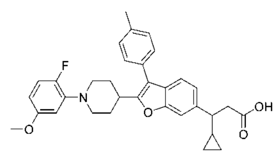 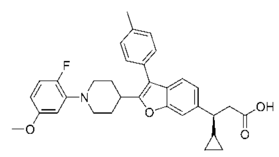 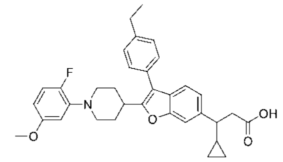

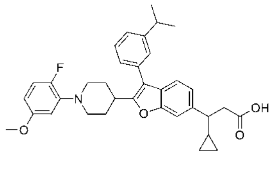 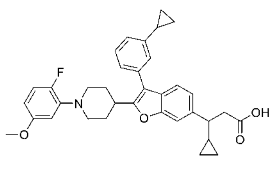 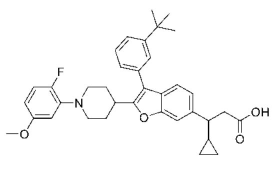 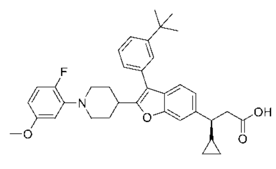

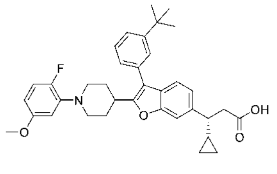 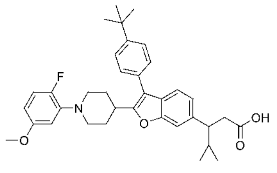 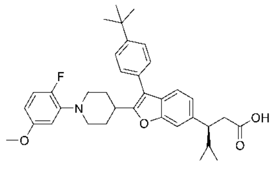 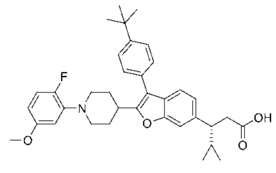

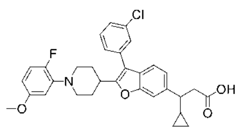 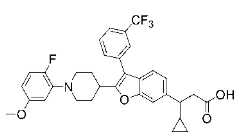 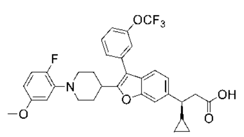 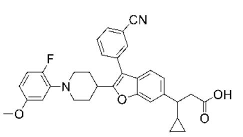

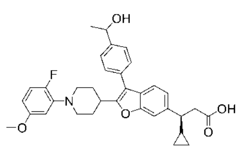 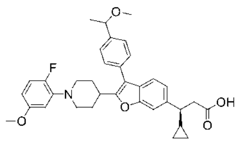 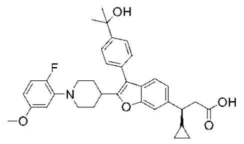 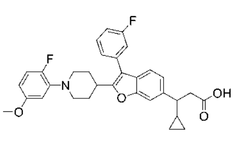

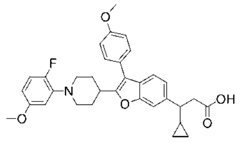 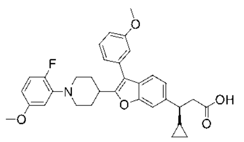 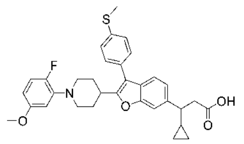 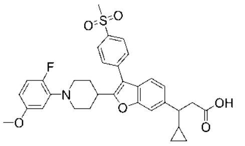

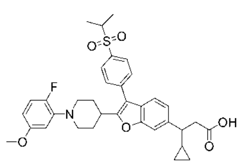 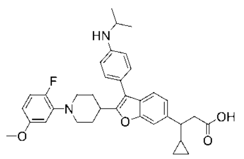 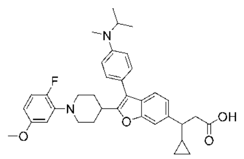 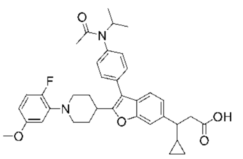

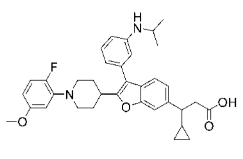 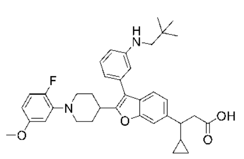 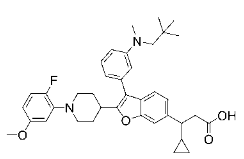 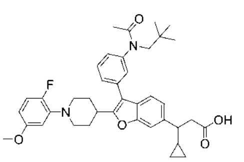

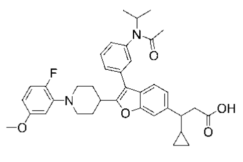 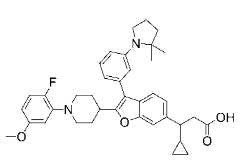 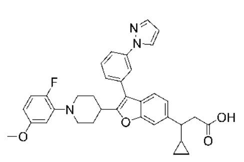 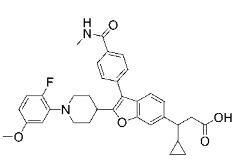

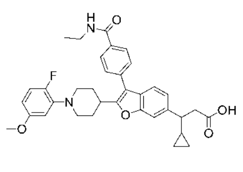 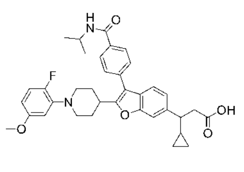 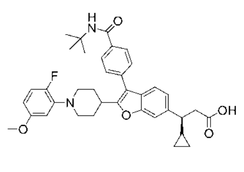 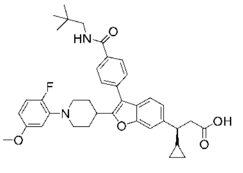

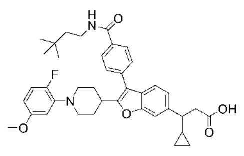
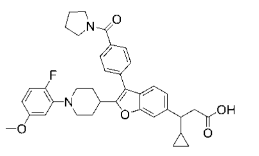
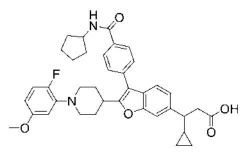
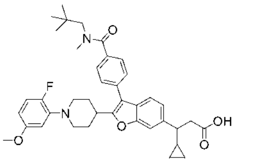
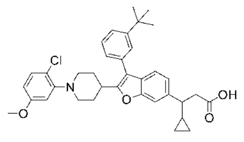
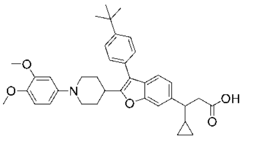
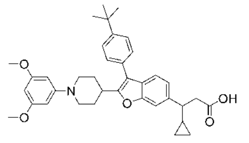
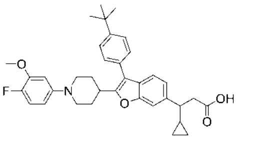
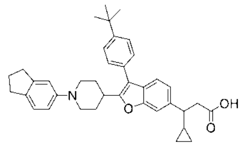
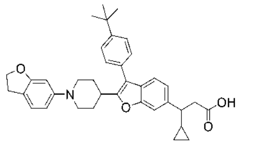
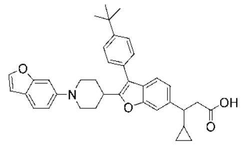
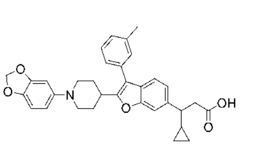
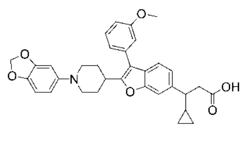
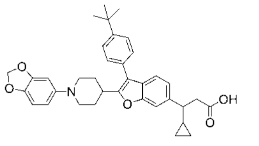
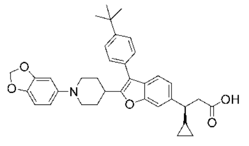
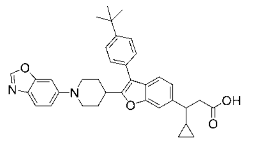
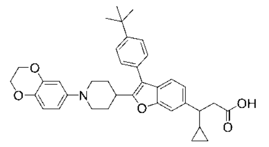
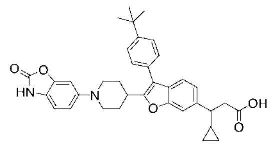
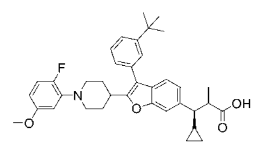
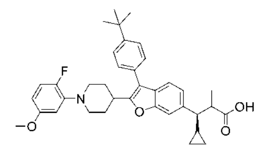
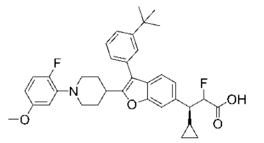
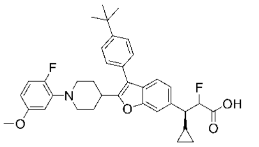
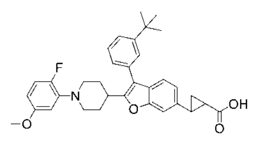
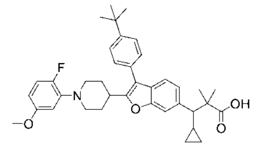
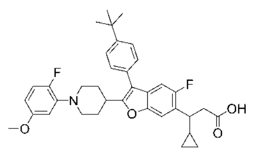
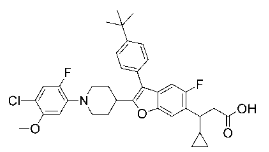
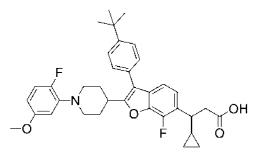
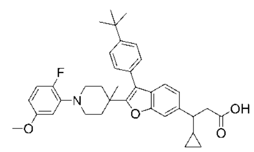
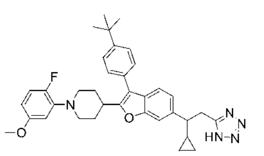
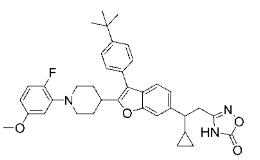
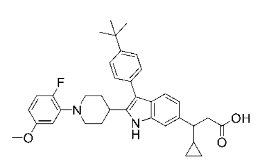
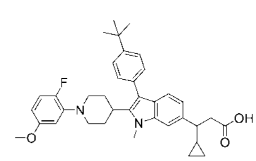
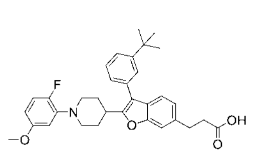
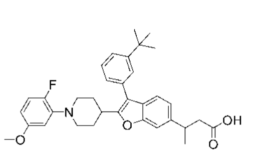
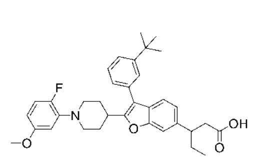
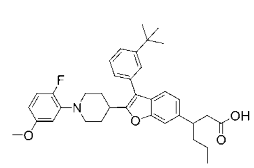

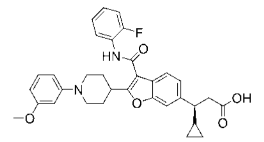
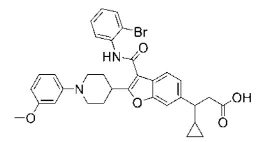
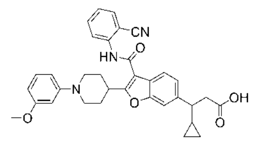
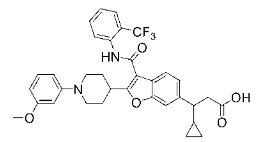
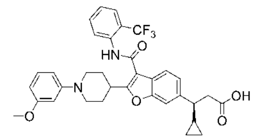
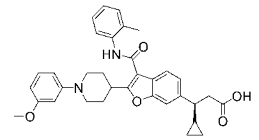
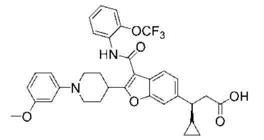
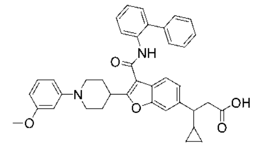
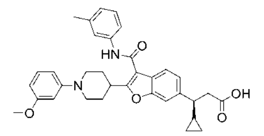
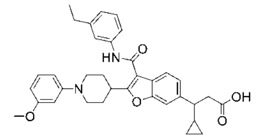
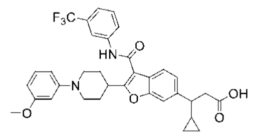
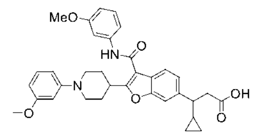
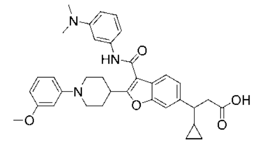
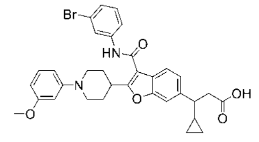
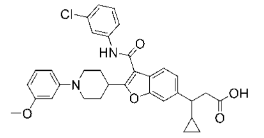
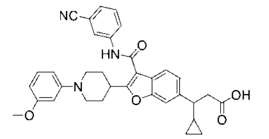
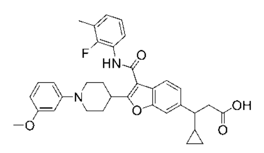
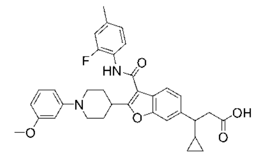
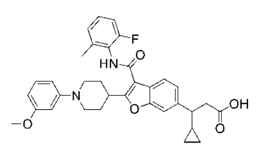
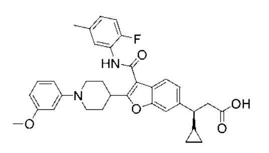
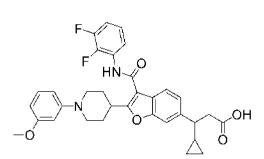
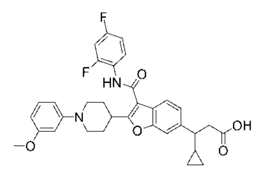
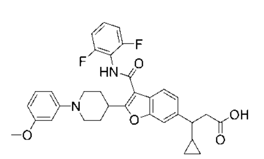
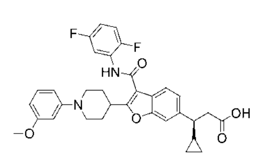
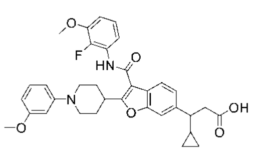
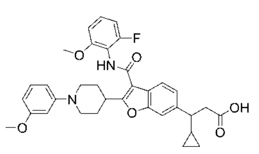
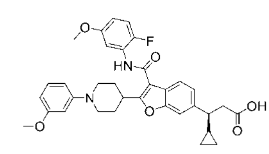
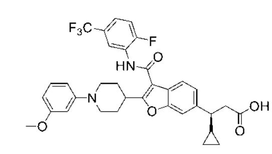
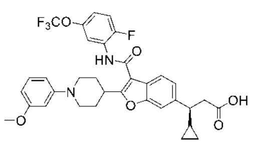
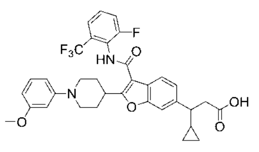
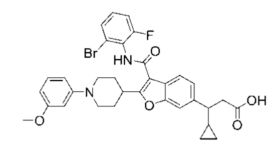
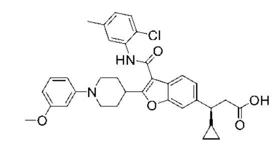
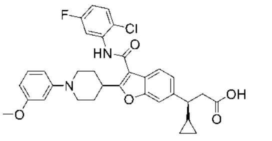
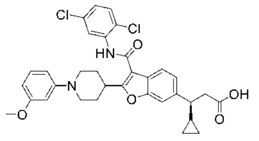
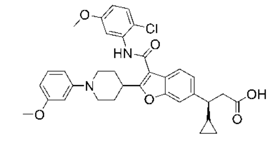
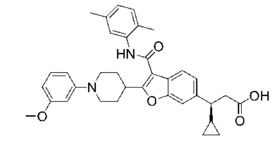

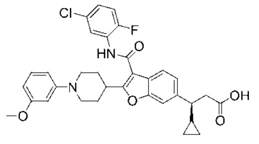
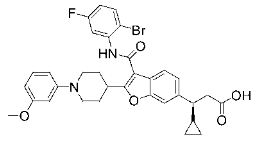
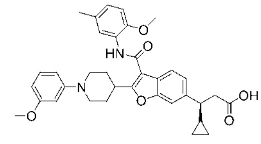
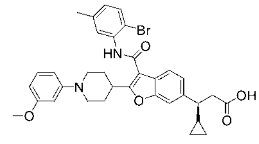
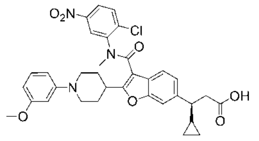
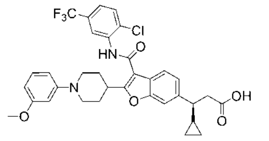
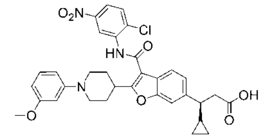
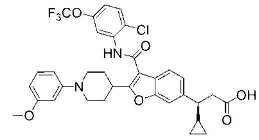
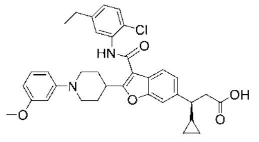
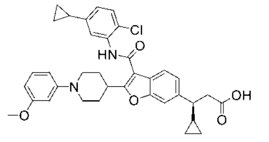
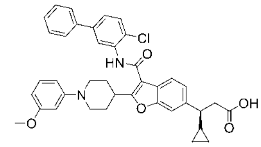
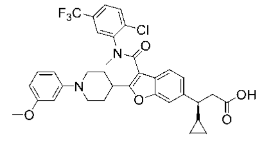
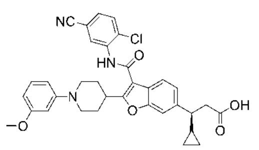
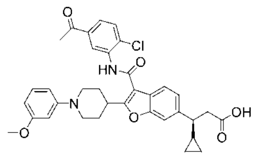
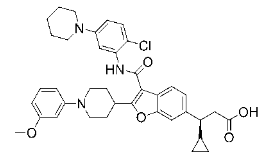
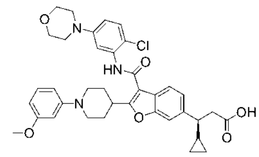
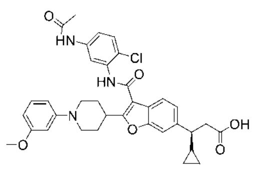
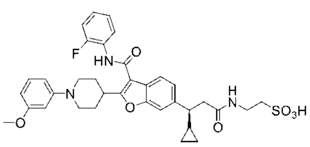
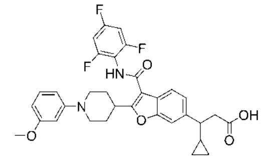
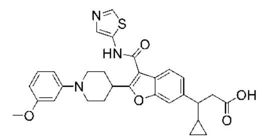
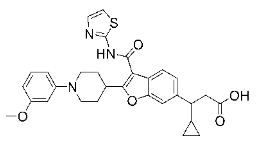
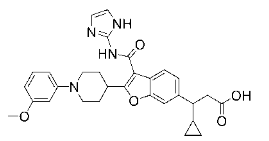
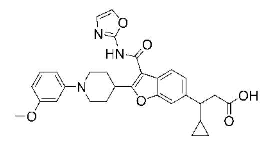
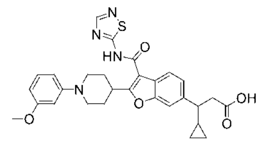
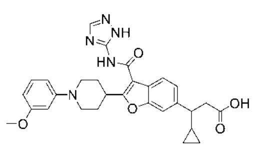
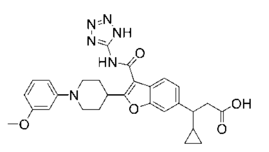
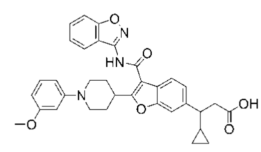
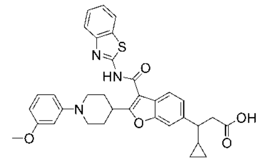
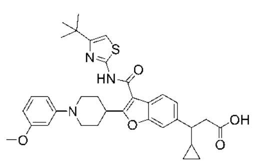
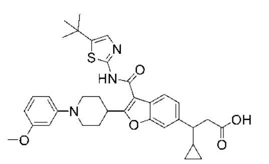
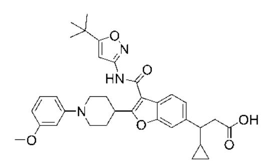
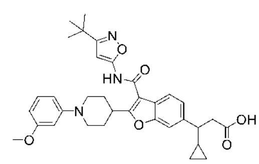
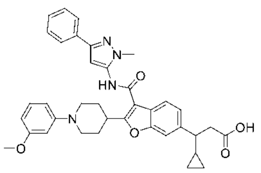
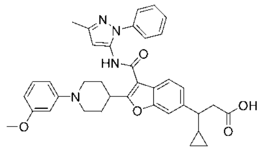
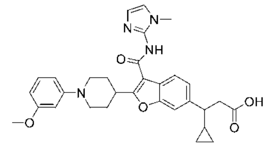
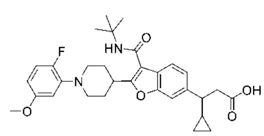

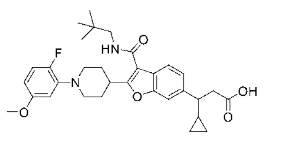
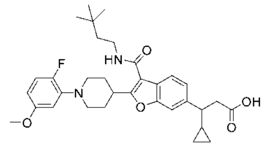
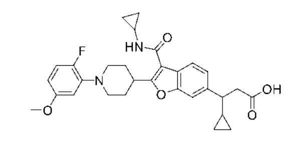
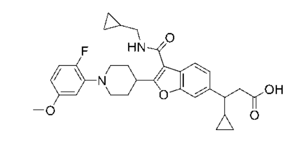
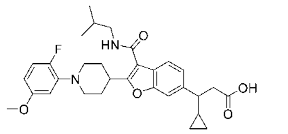
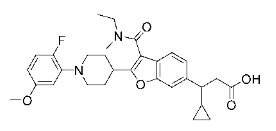
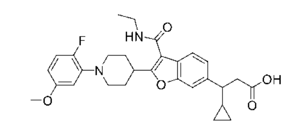
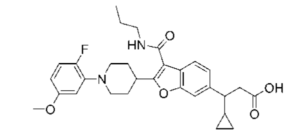
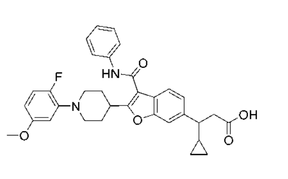
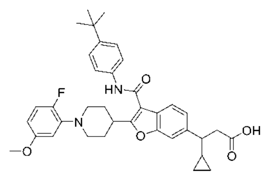
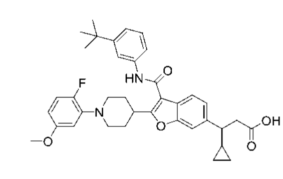
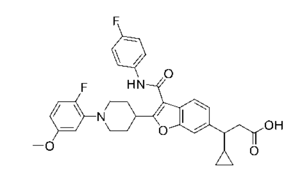
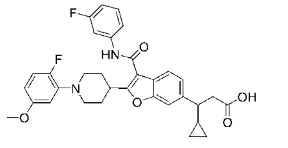
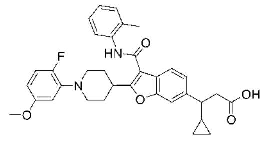
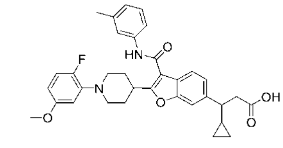
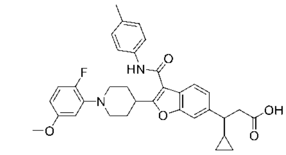
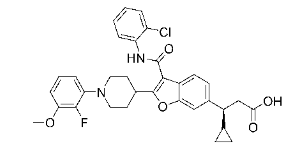
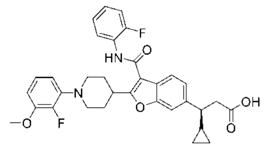
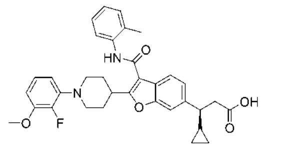
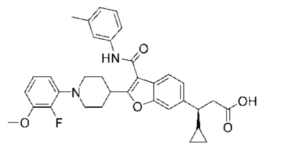
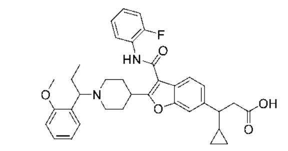
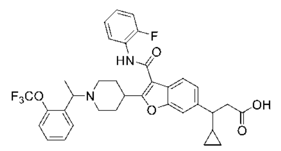
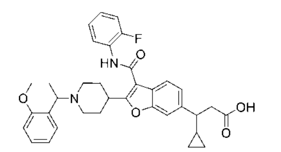
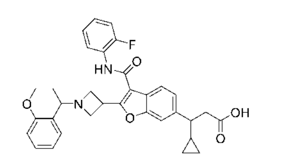
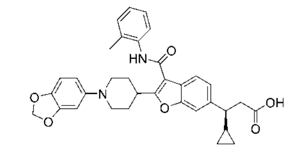
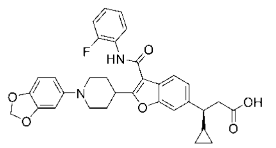
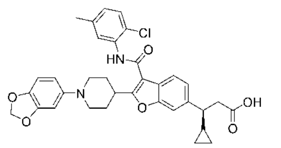
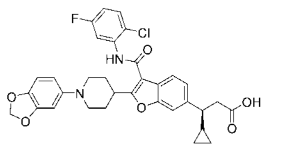
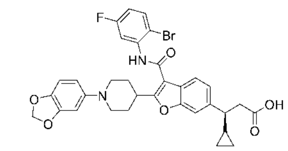
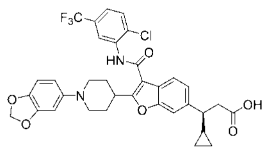
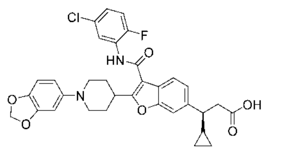
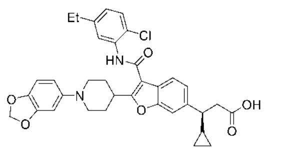
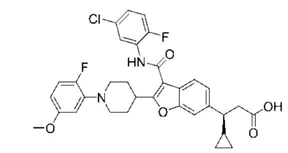
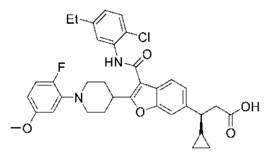
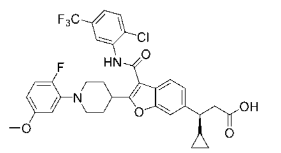
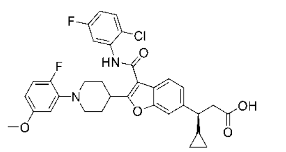

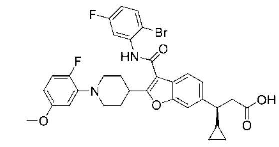
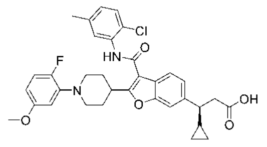
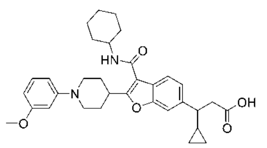
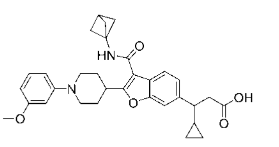
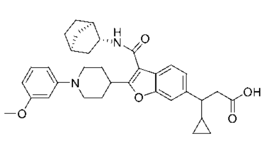
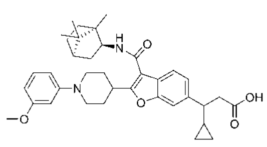
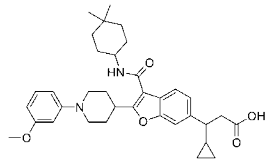
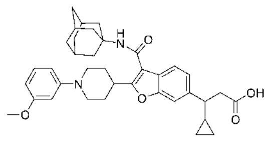
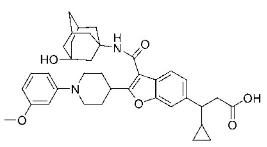
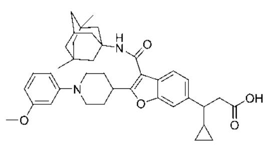
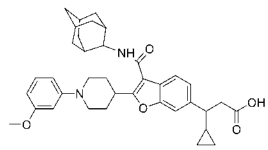
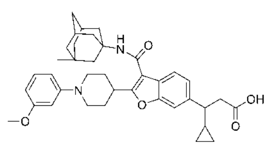
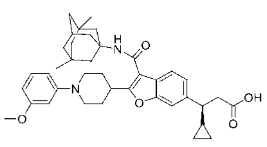
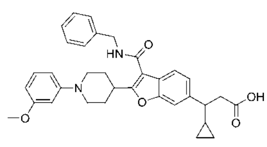
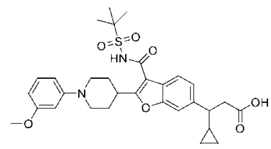
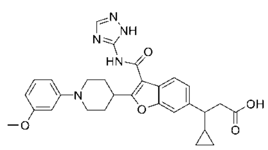
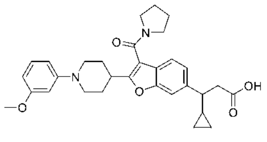
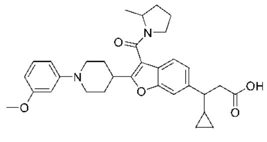
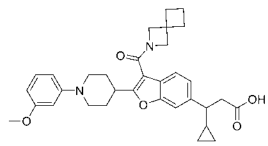
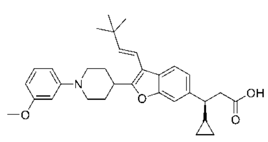
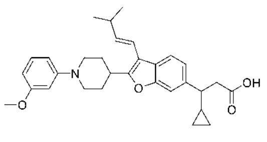
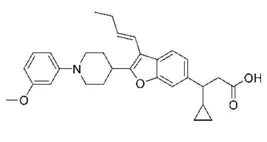
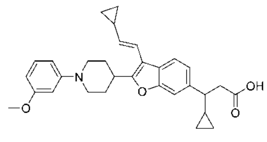
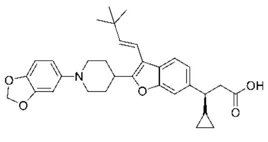
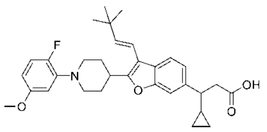
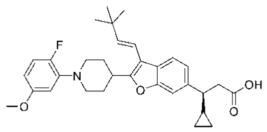
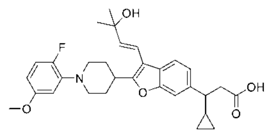
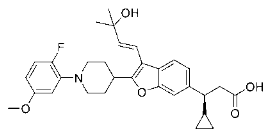
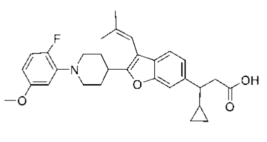
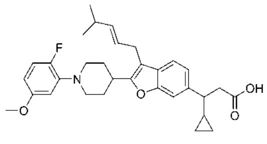
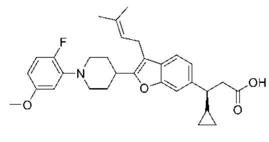
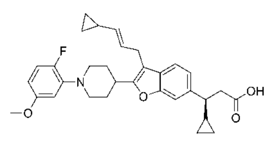
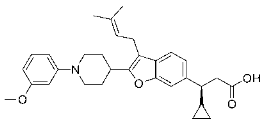
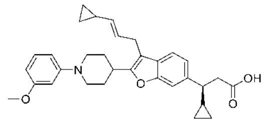
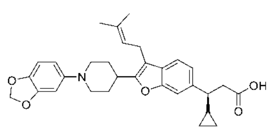
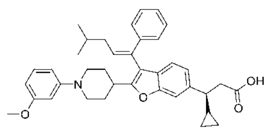

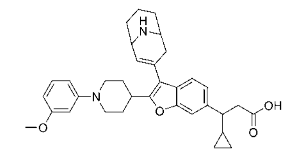 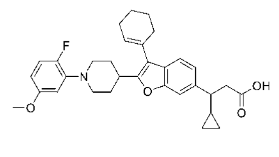 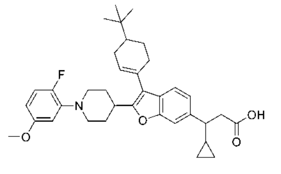 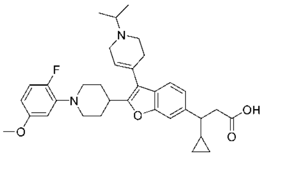

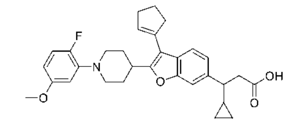 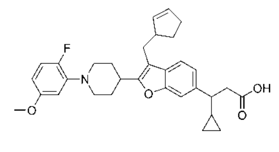 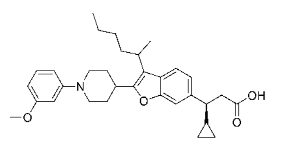 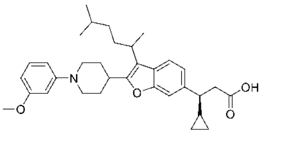

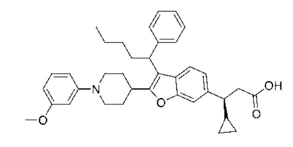 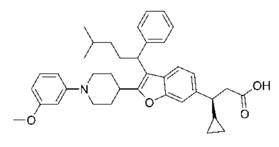 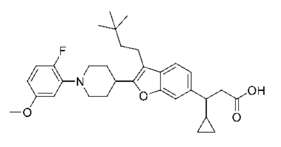 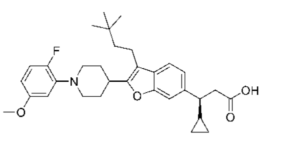

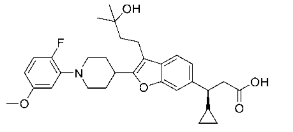 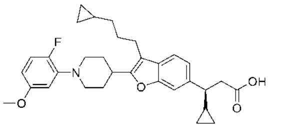 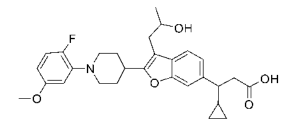 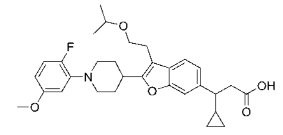

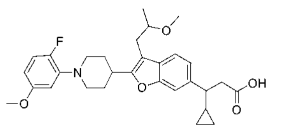 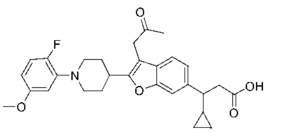 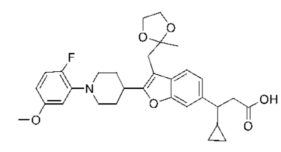 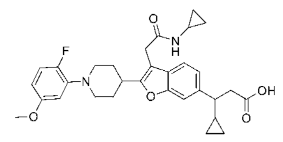

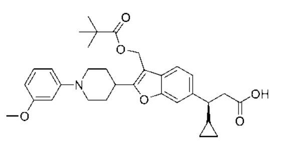 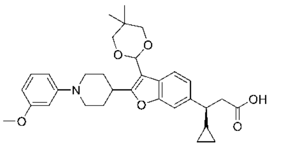 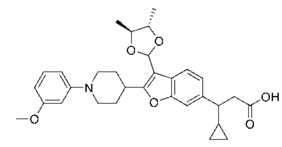 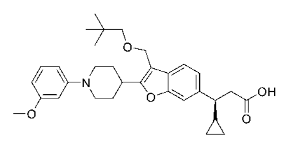

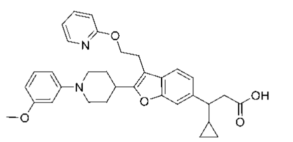 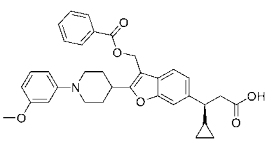 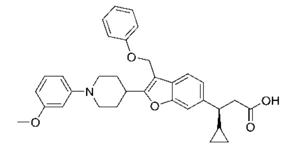 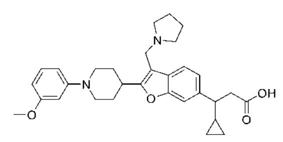

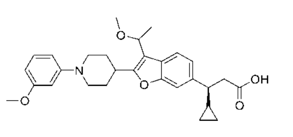 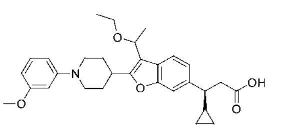 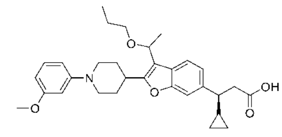 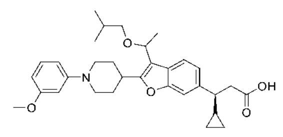

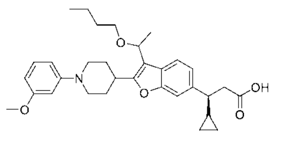 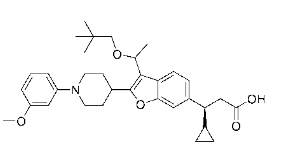 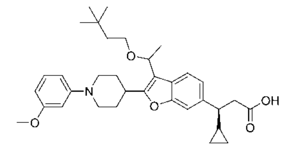 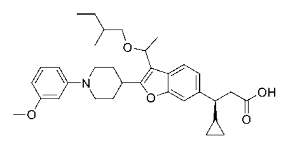

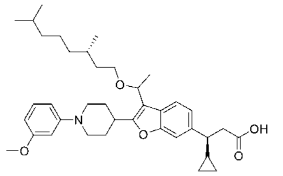
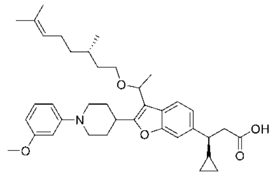
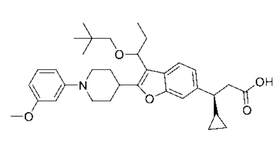
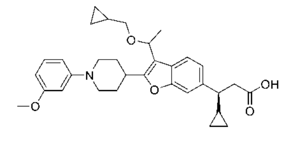
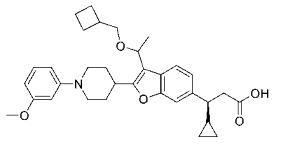
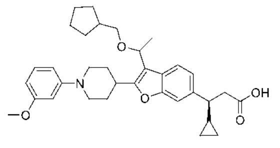
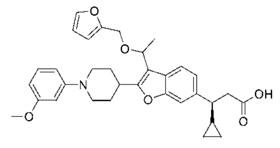
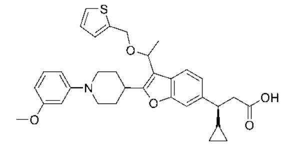
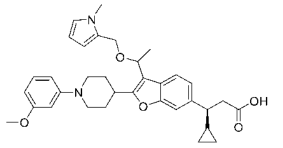
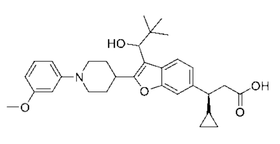
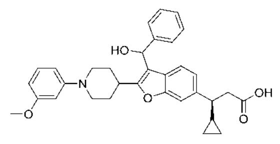
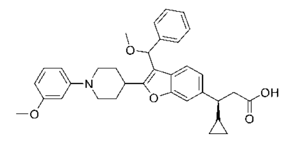
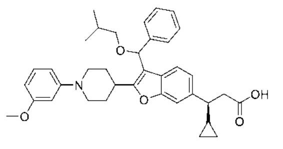
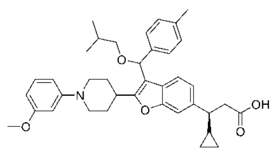
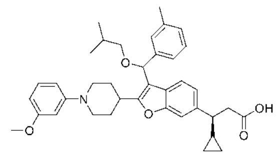
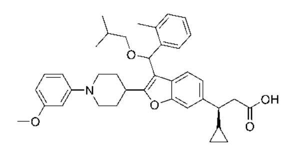
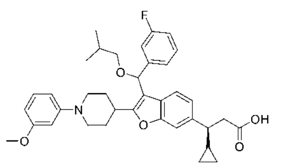
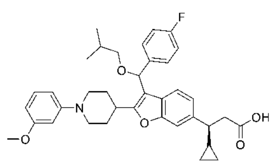
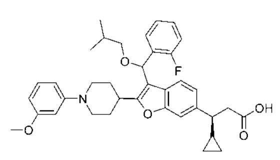
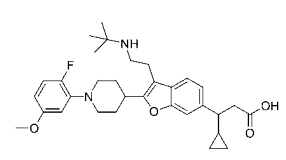
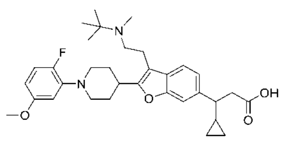
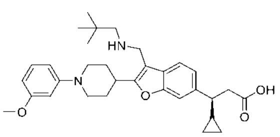
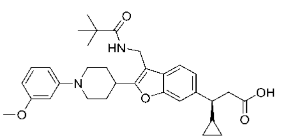
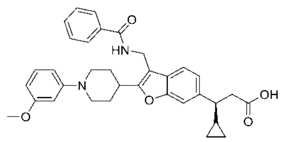
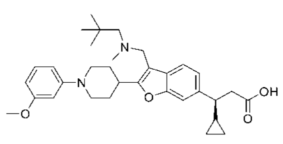
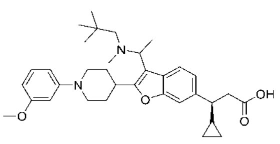
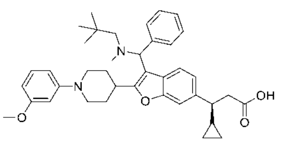
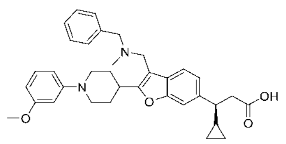
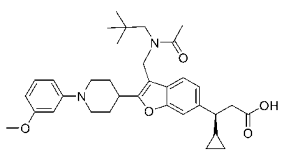
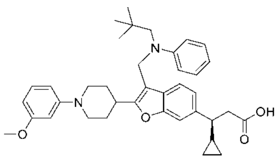
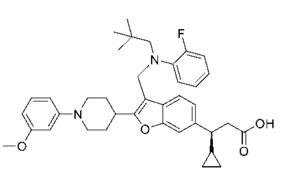
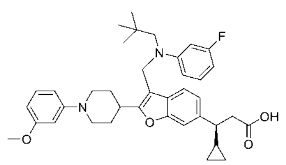
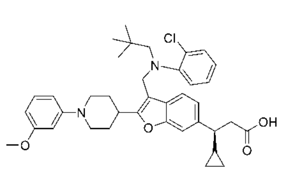
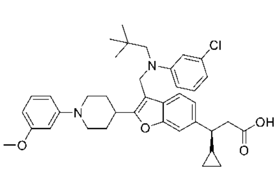
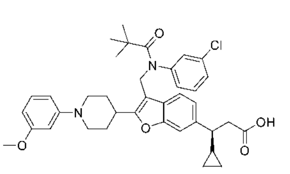
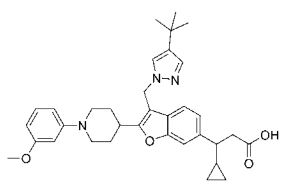

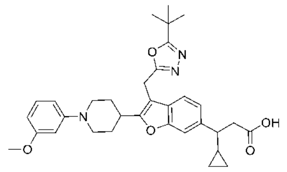 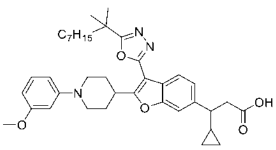 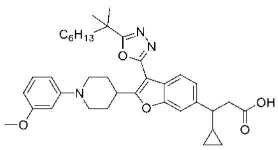 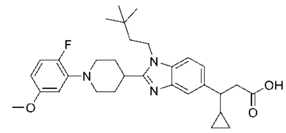

.

**10.** A preparation method for the compound of formula I according to any one of claims 1 to 9, wherein the method is method 1 or 2:

when $G^2$ is -C(=O)OH, the method is method 1; method 1 comprises the following step: subjecting compound -1 to a hydrolysis reaction in a solvent in the presence of a base to obtain the compound of formula I;

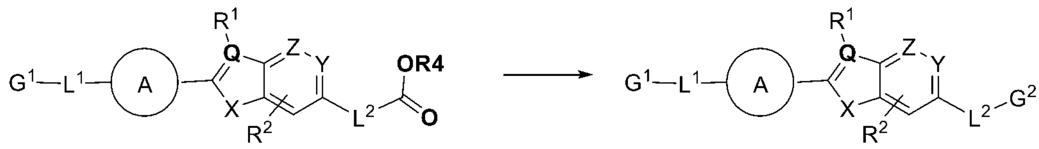

II-1 I

;

wherein $R^4$ is $C_1$-$C_6$ alkyl; Q, X, Y, Z, $R^1$, $R^2$, L, $G^1$, $G^2$, and ring A are as defined in any one of claims 1 to 6;
when $G^2$ is 5- to 10-membered heteroaryl, the method is method 2; method 2 comprises the following step: subjecting compound II-2 and trimethylsilyl azide to a cyclization reaction in a solvent in the presence of a catalyst to obtain the compound of formula I;
alternatively, subjecting compound II-2 and *N,N'*-carbonyldiimidazole to a cyclization reaction in a solvent in the presence of a catalyst to obtain the compound of formula I;

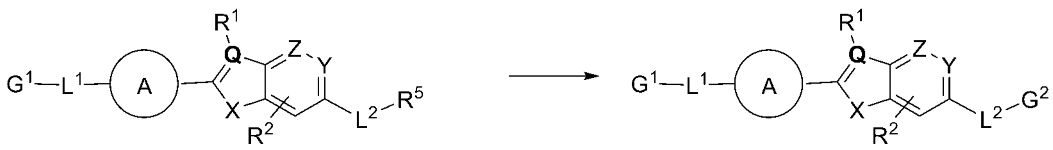

II-2 I

;

wherein $R^5$ is

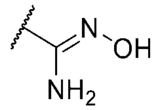

or cyano; Q, X, Y, Z, $R^1$, $R^2$, L, $G^1$, $G^2$, and ring A are as defined in any one of claims 1 to 8.

**11.** A compound II-1, II-2, II-1a, or II-2a:

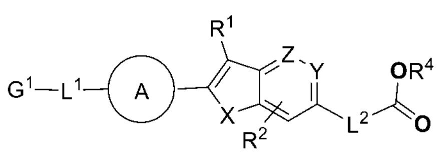

II-1a

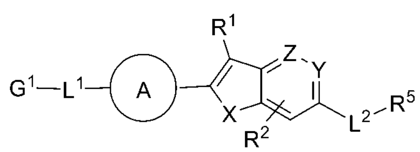

II-2a

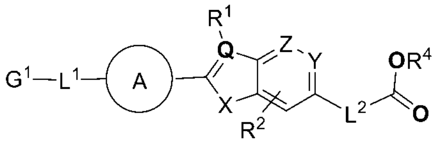

II-1

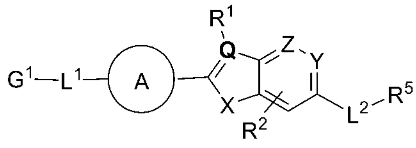

II-2

wherein Q, X, Y, Z, $R^1$, $R^2$, $R^3$, $R^4$, $R^5$, $L^1$, $L^2$, $G^1$, and ring A are as defined in any one of claims 1 to 9; the compound II-1a or II-2a is preferably any one of the following compounds:

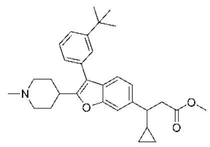 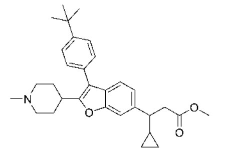 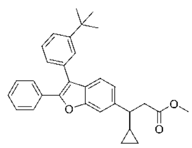 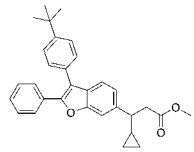

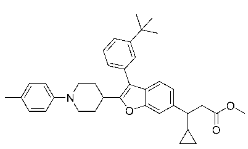 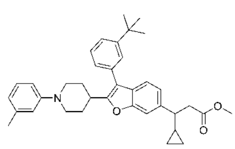 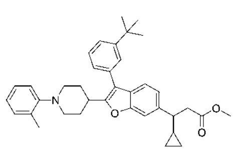 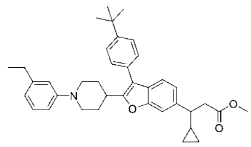

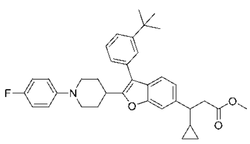 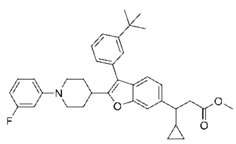 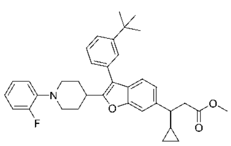 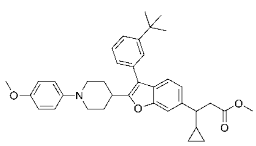

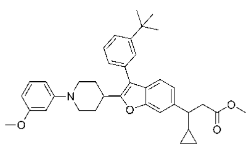 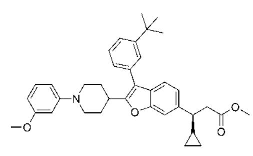 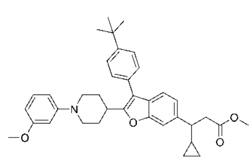 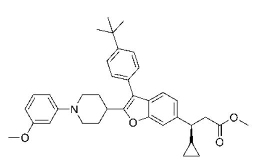

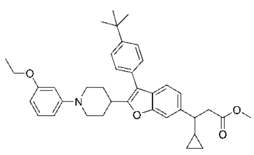 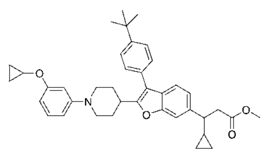 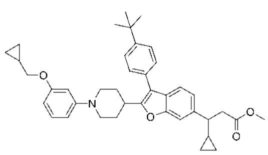 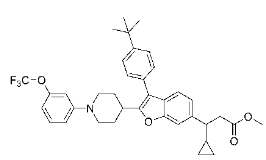

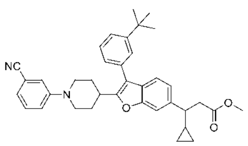 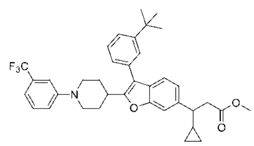 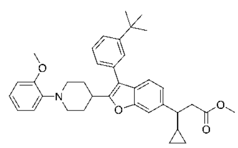 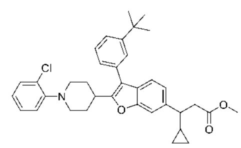

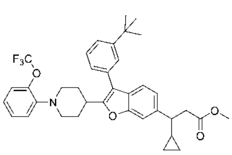 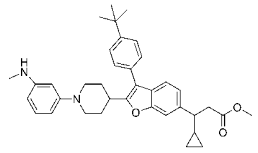 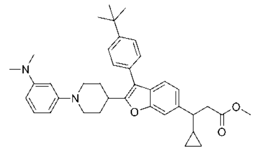 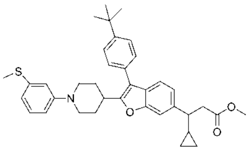

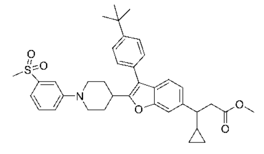
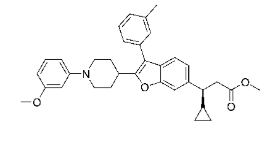
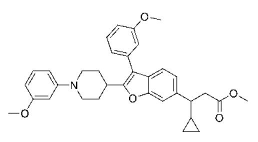
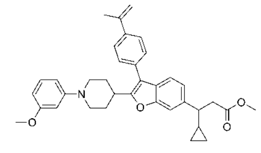
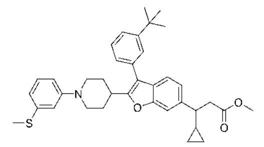
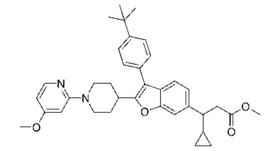
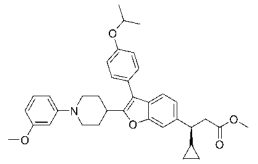
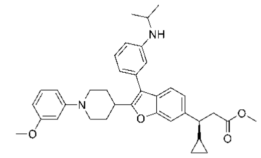
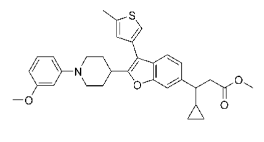
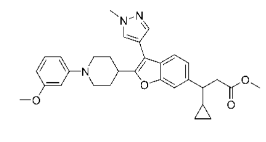
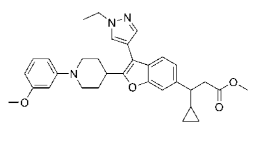
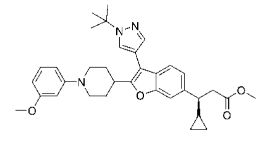
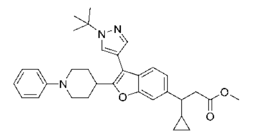
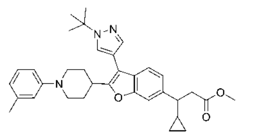
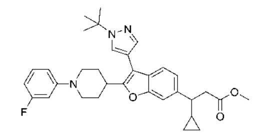
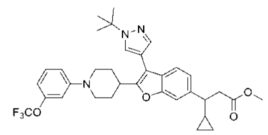
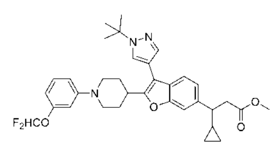
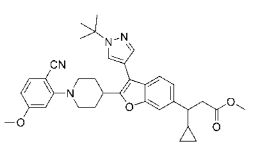
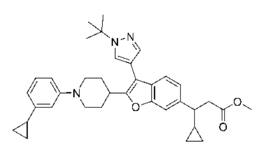
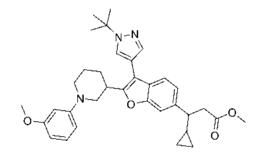
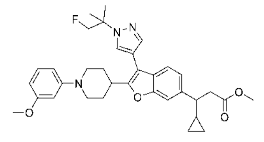
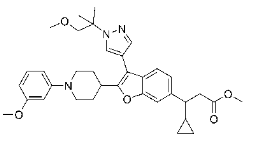
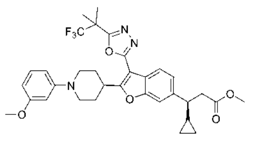
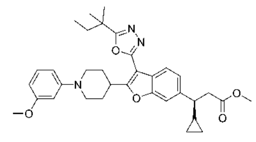
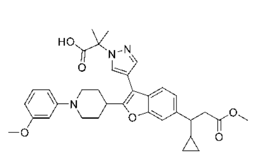
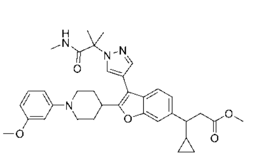
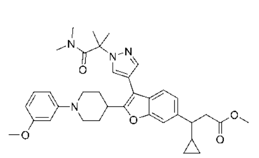
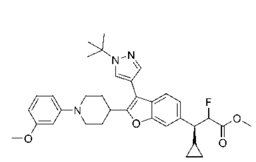
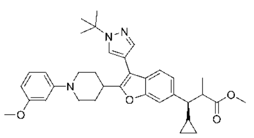
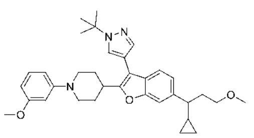
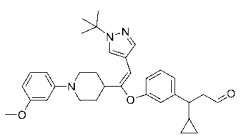
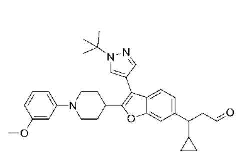
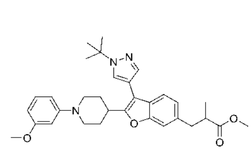
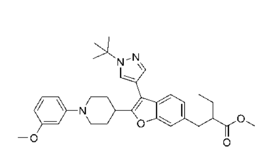
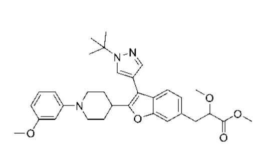
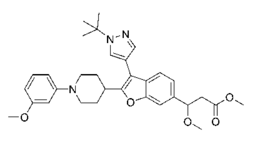
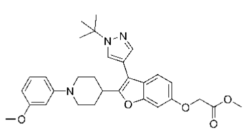
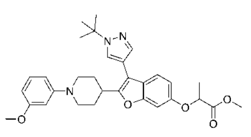
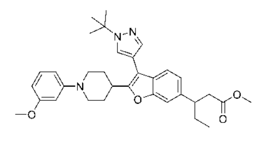

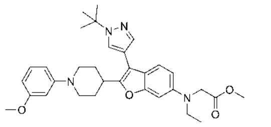
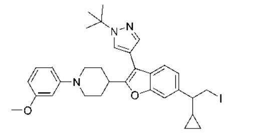
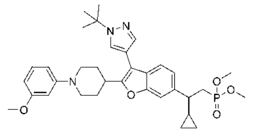
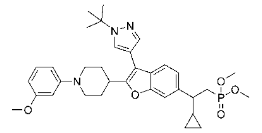
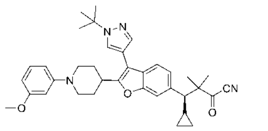
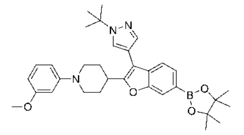
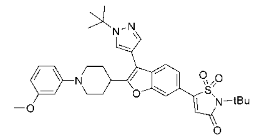
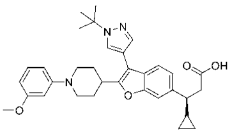
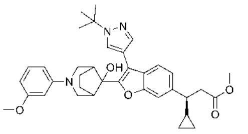
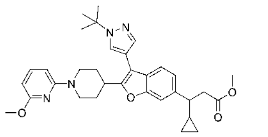
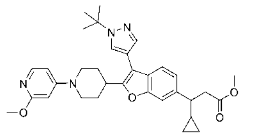
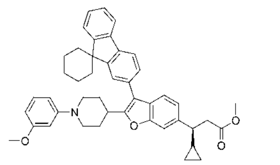
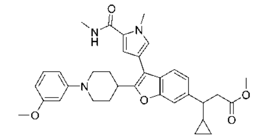
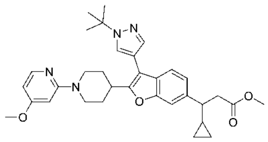
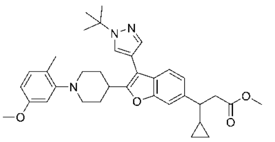
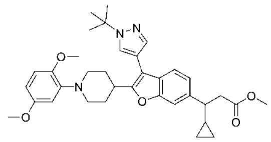
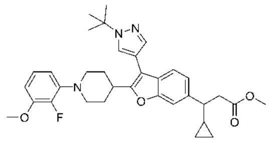
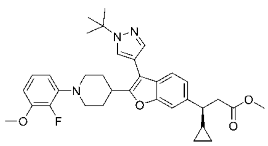
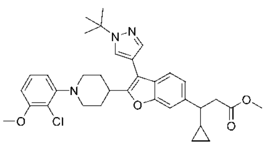
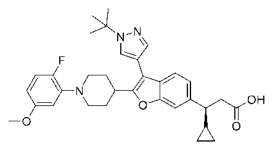
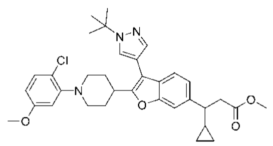
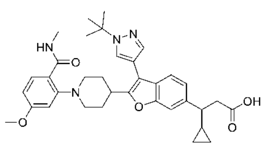
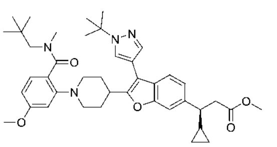
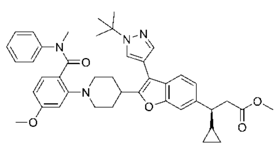
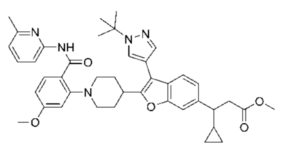
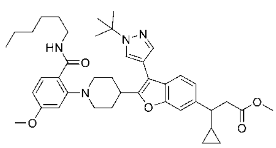
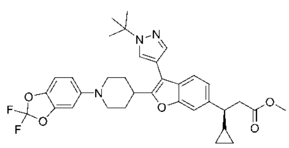
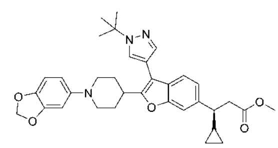
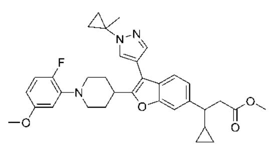
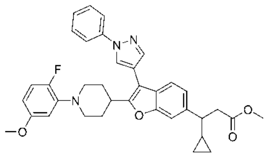
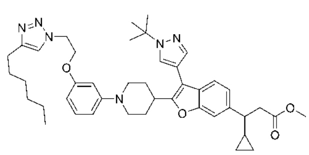
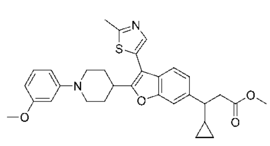
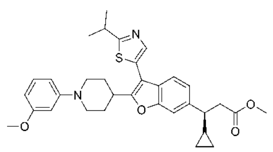

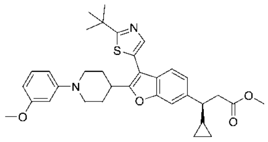
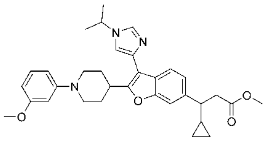
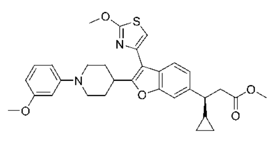
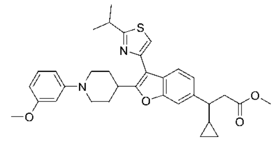
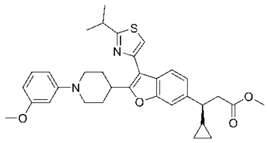
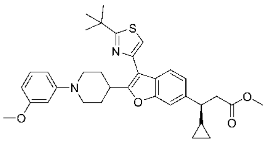
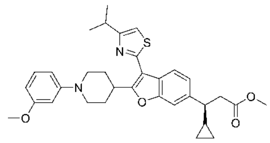
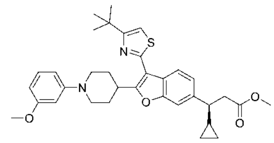
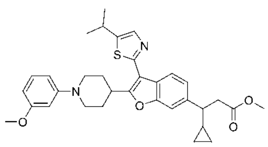
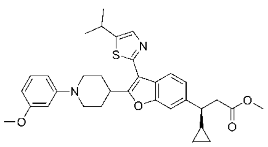
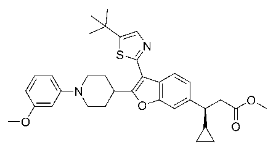
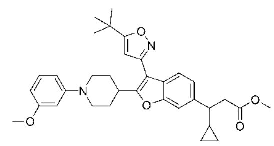
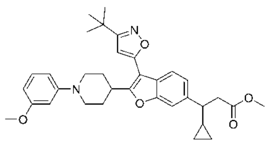
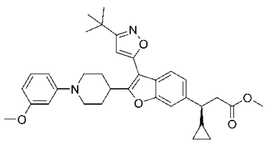
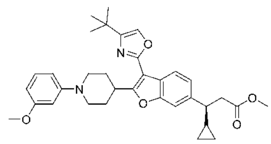
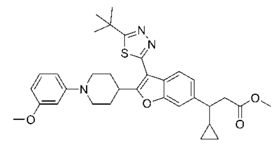
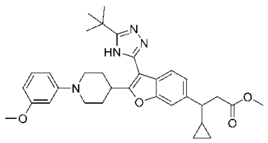
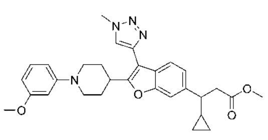
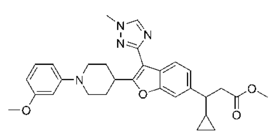
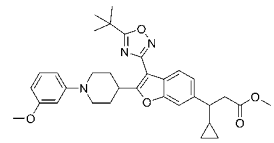
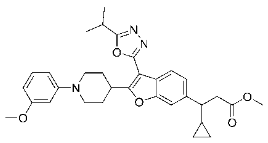
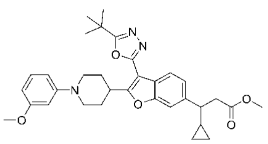
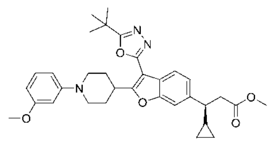
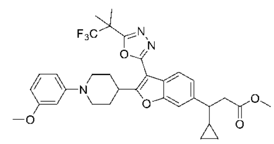
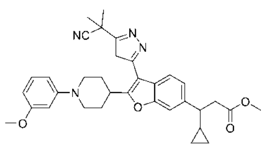
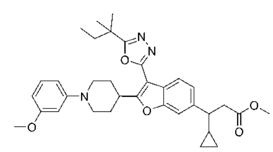
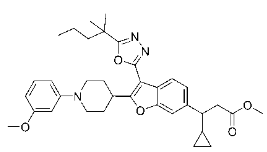
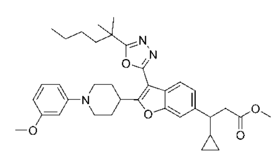
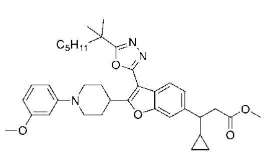
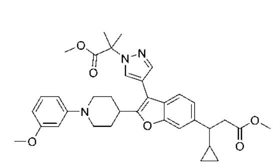
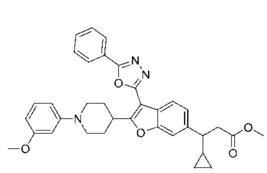
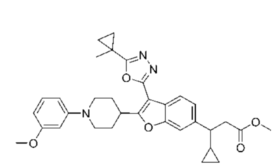
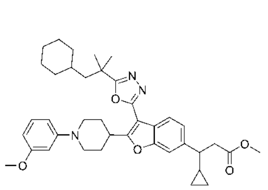
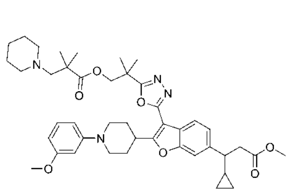
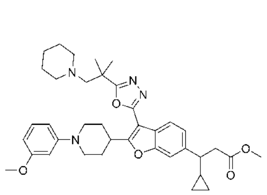
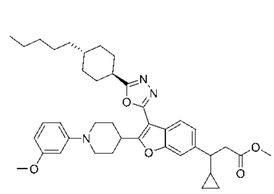

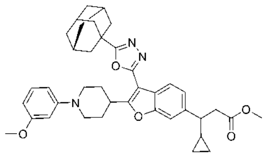 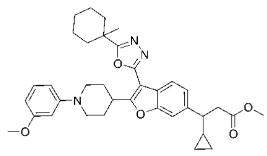 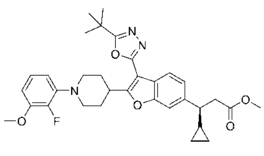 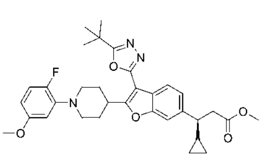

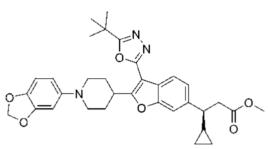 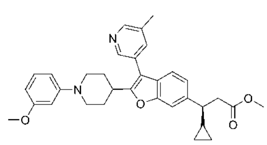 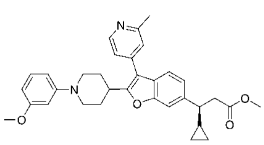 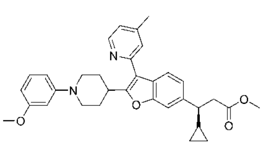

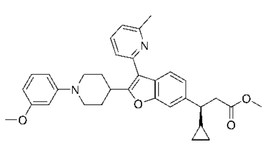 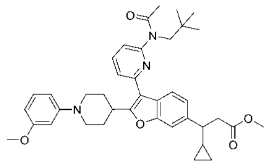 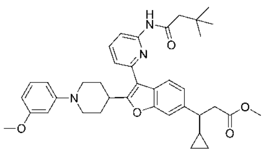 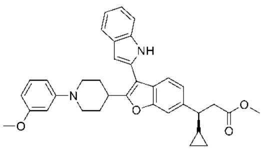

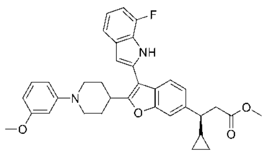 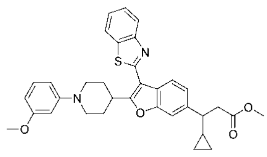 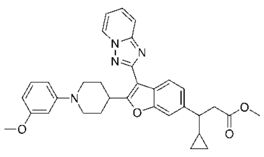 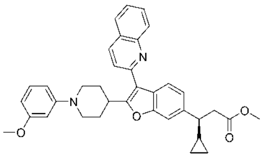

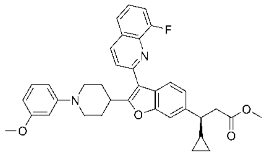 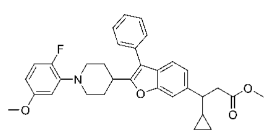 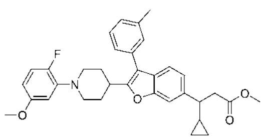 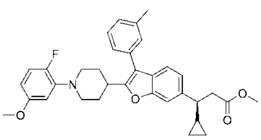

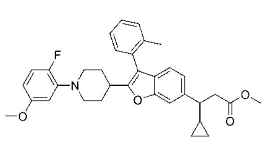 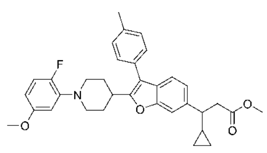 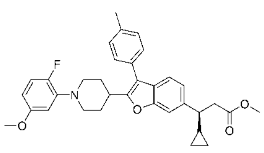 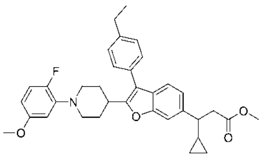

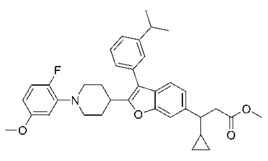 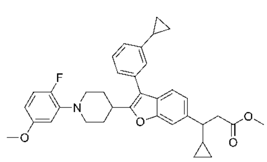 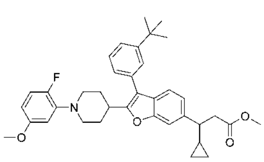 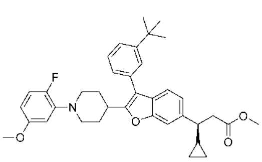

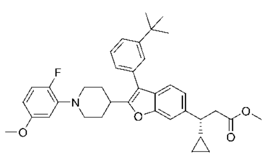 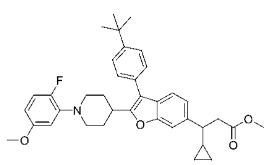 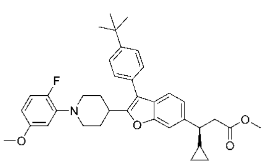 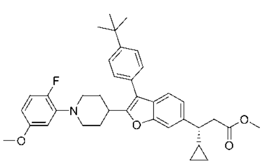

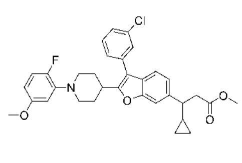 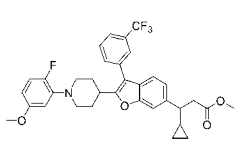 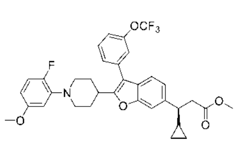 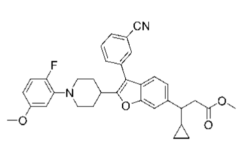

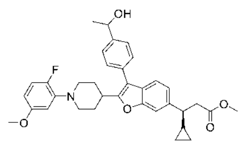
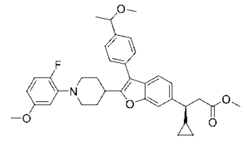
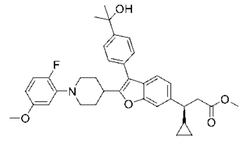
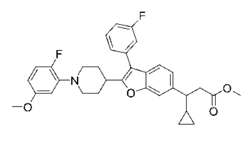

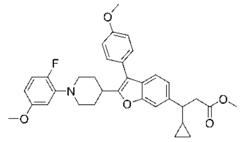
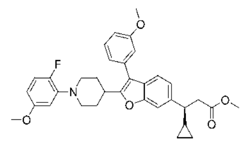
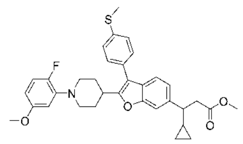
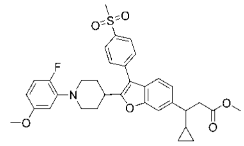

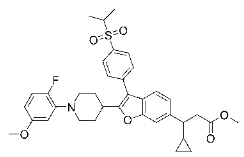
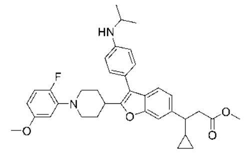
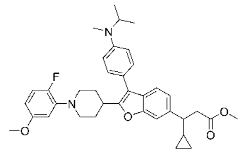
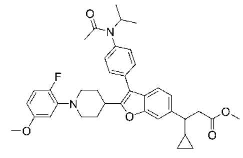

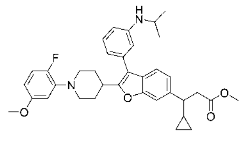
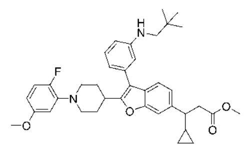
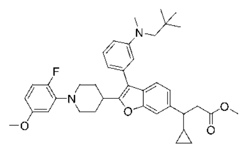
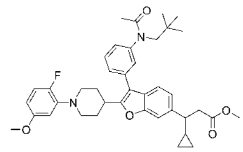

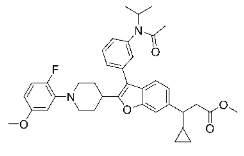
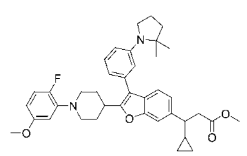
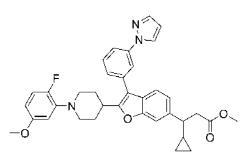
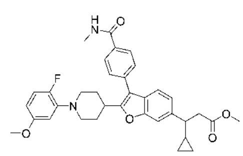

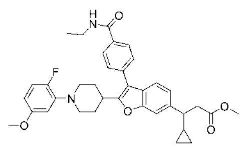
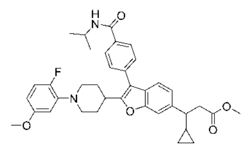
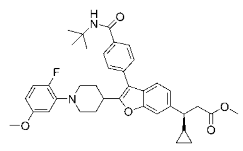
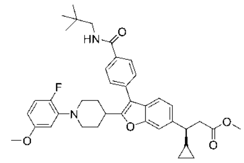

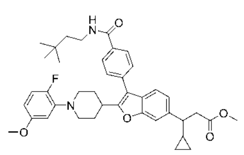
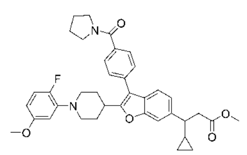
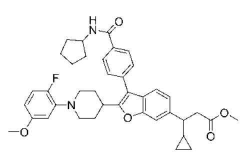
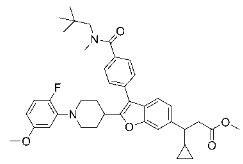

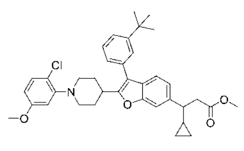
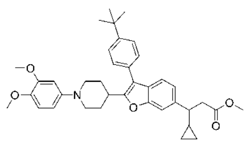
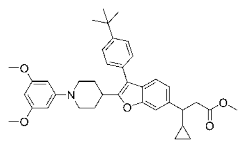
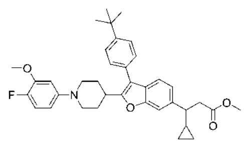

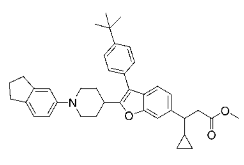
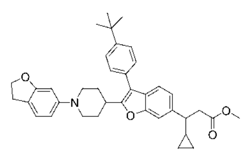
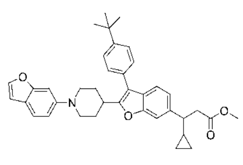
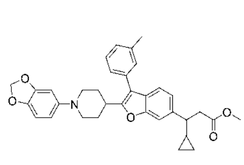

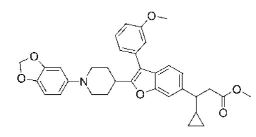
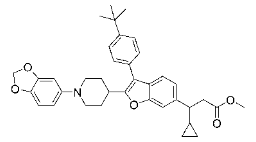
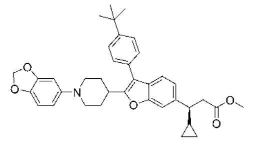
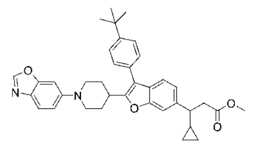

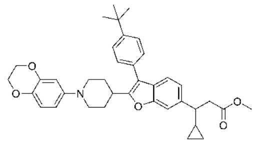
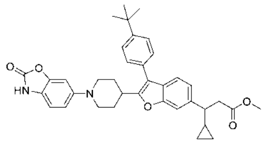
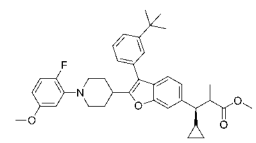
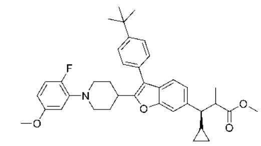

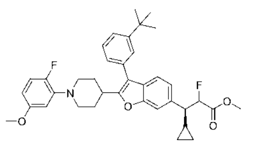
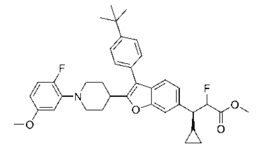
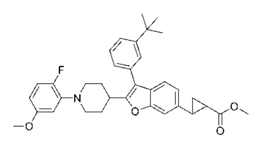
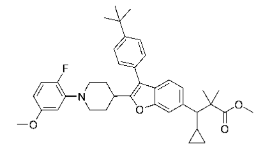

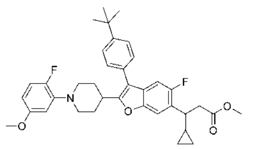
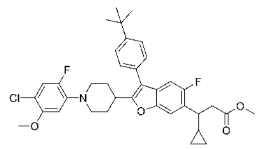
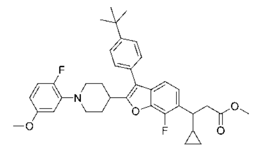
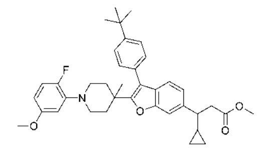

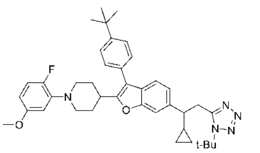
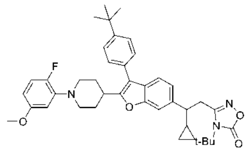
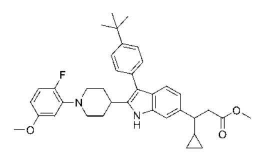
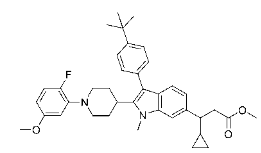

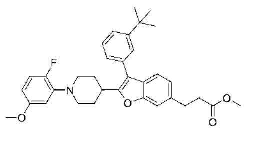
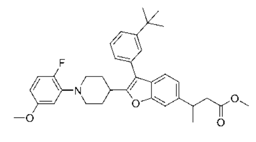
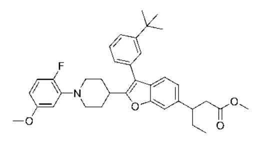
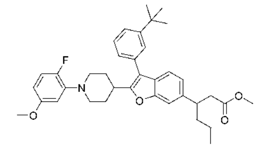

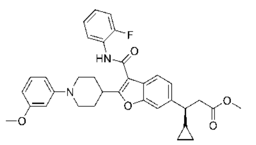
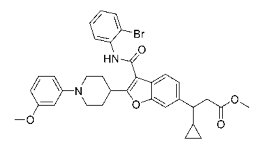
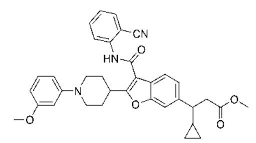
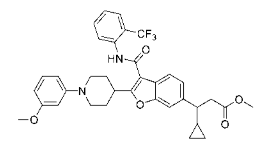

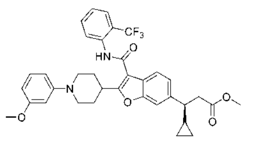
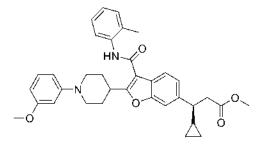
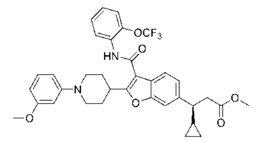
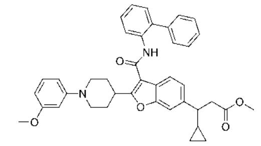

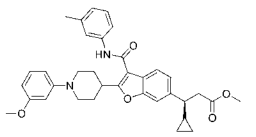
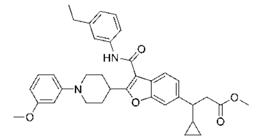
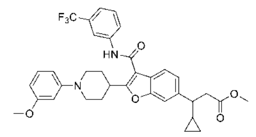
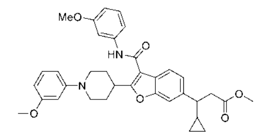

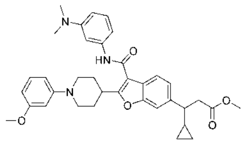
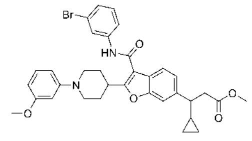
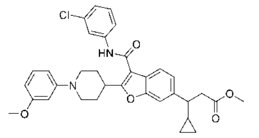
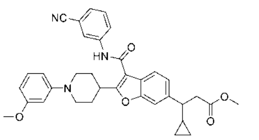
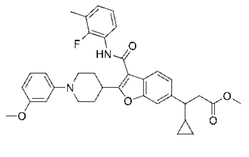
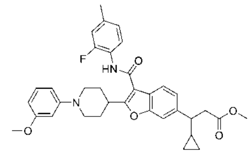
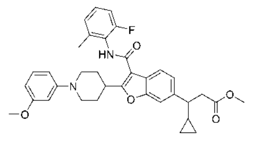
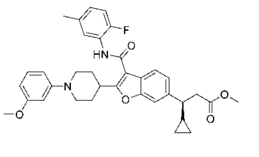
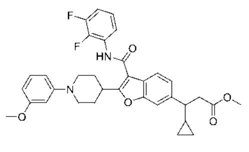
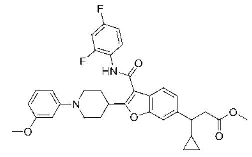
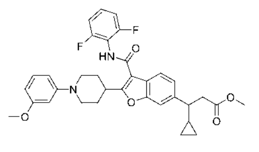
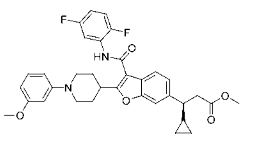
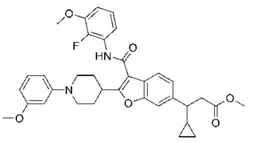
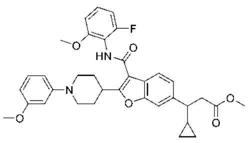
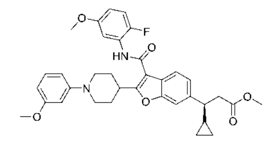
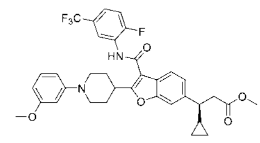
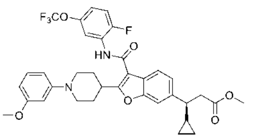
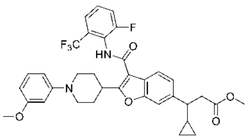
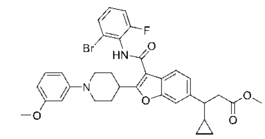
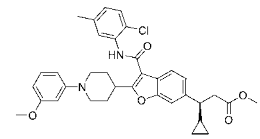
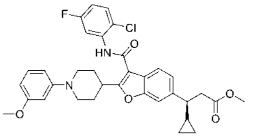
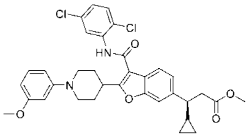
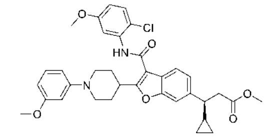
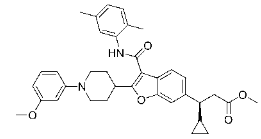
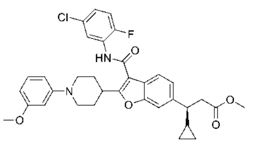
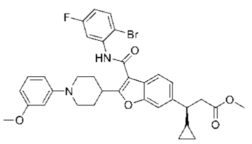
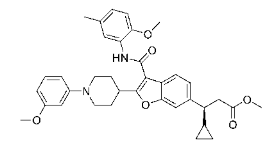
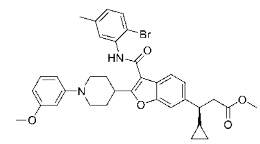
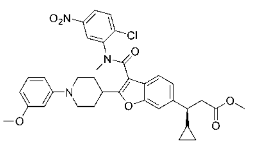
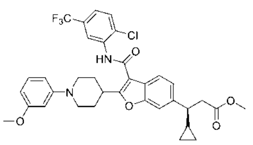
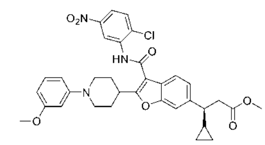
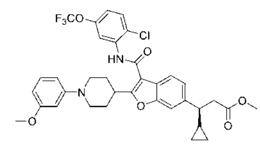
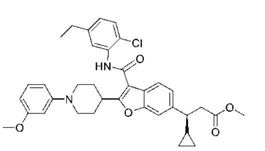
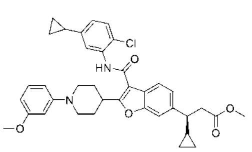
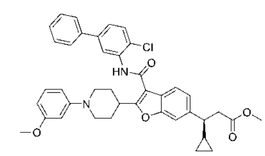
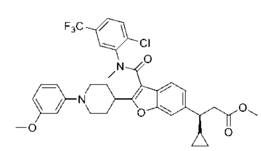

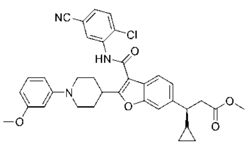
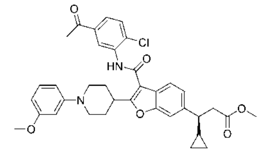
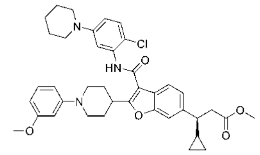
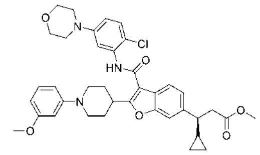
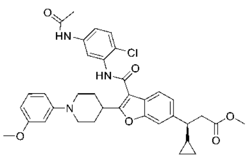
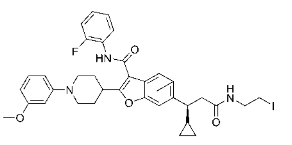
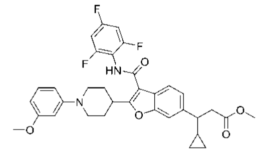
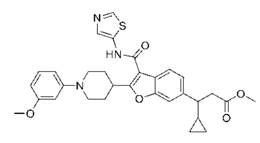
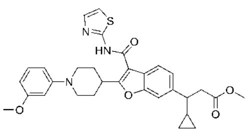
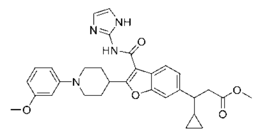
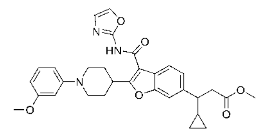
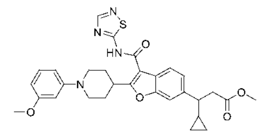
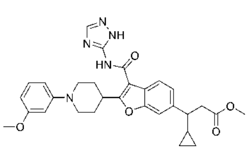
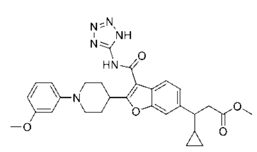
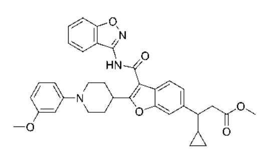
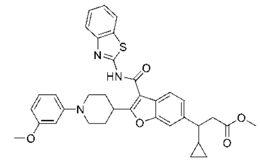
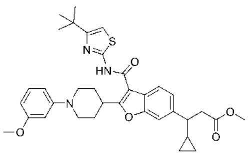
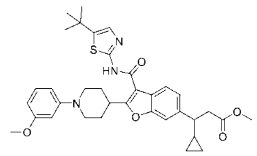
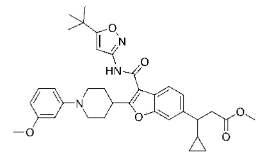
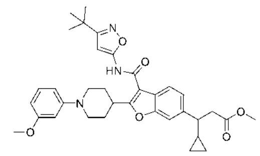
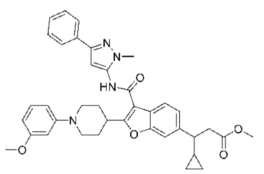
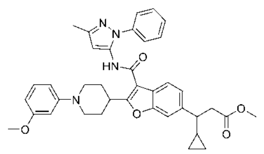
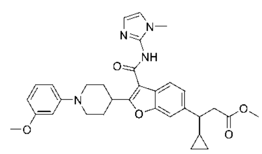
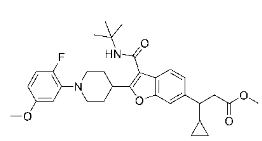
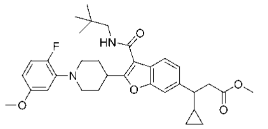
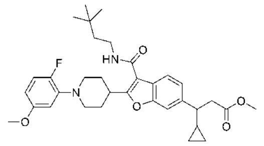
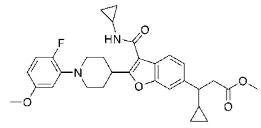
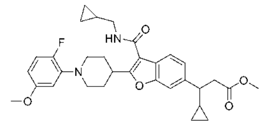
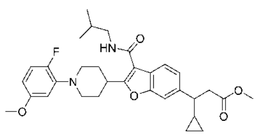
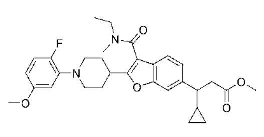
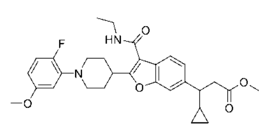
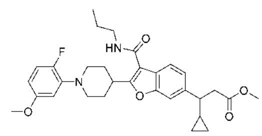
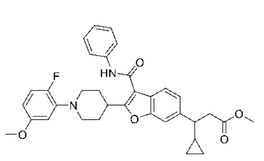
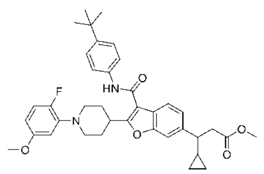
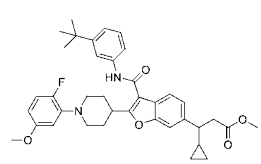
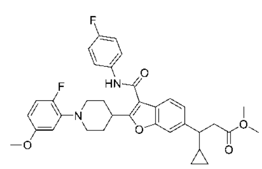

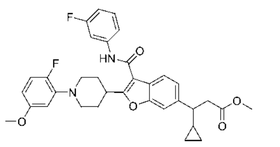
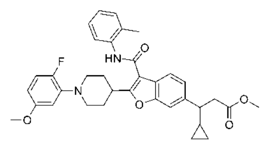
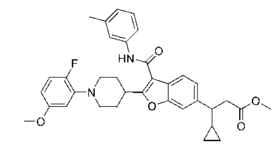
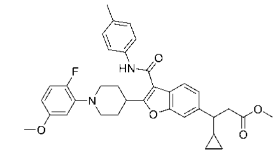

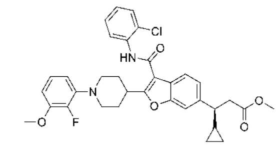
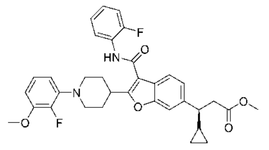
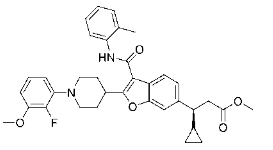
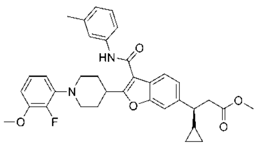

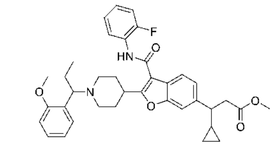
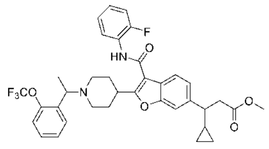
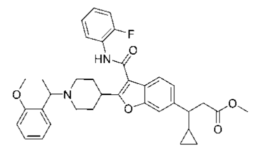
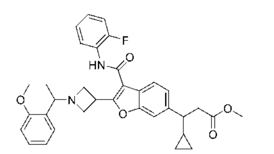

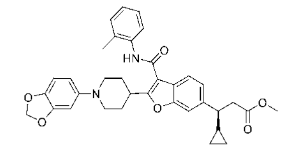
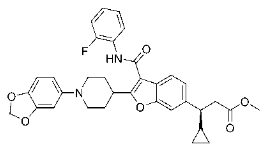
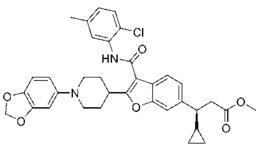
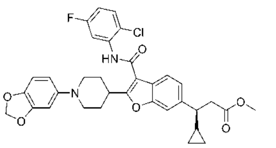

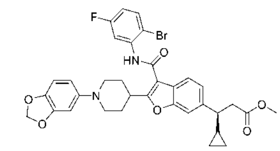
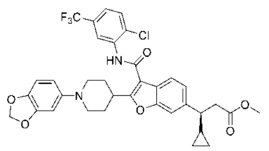
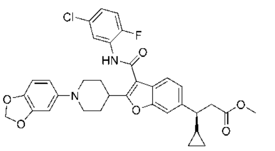
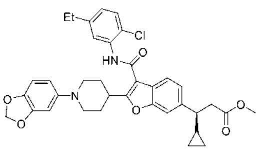

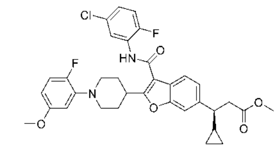
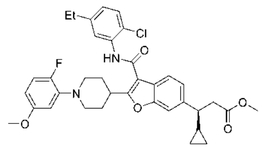
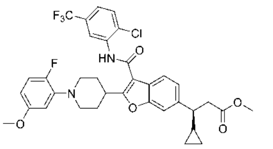
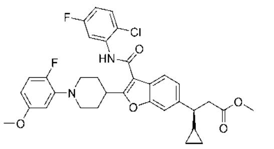

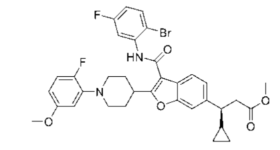
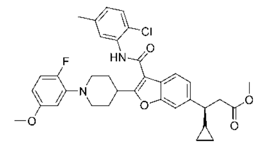
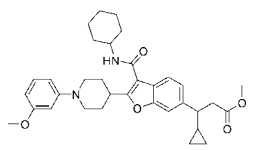
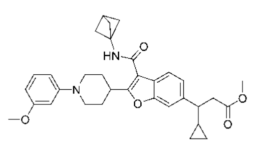

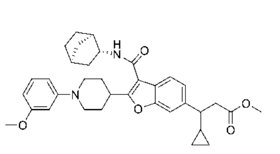
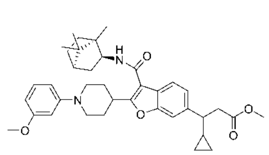
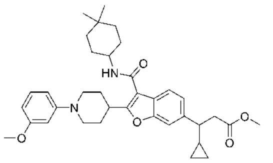
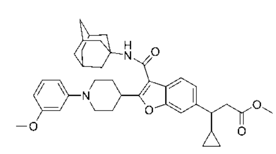

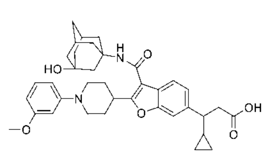
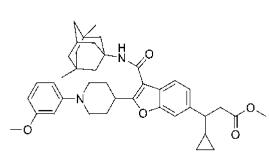
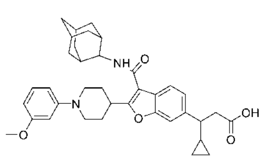
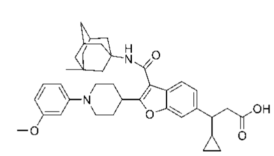

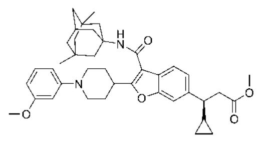
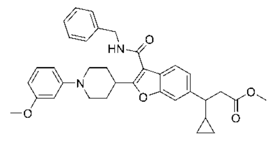
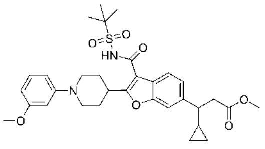
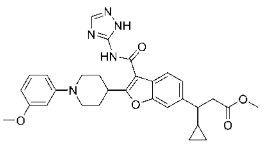
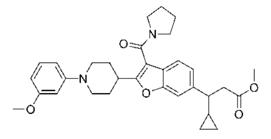
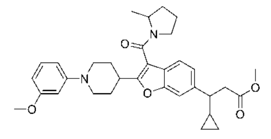
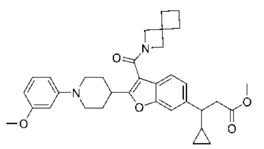
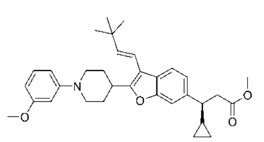
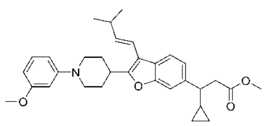
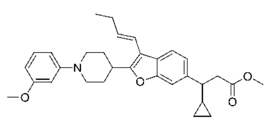
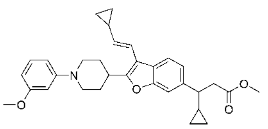
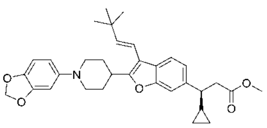
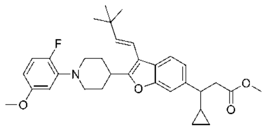
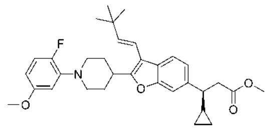
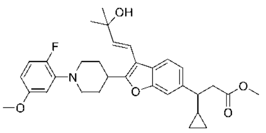
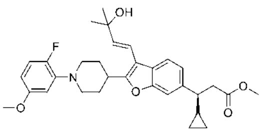
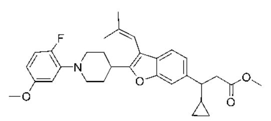
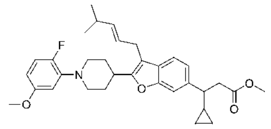
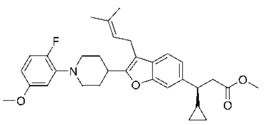
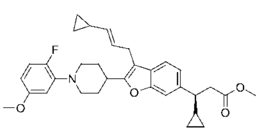
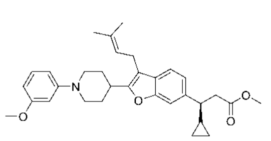
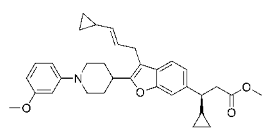
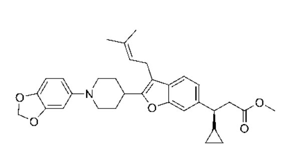
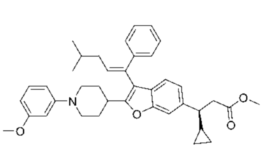
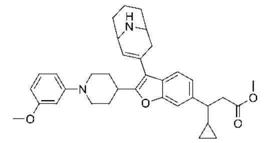
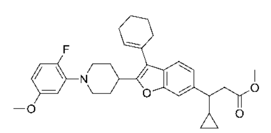
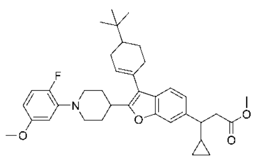
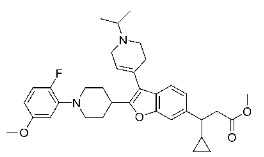
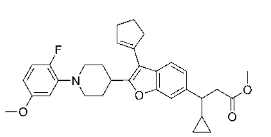
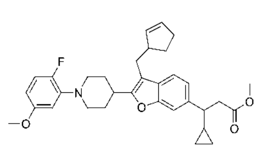
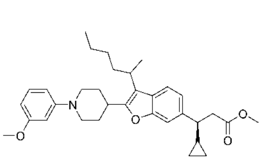
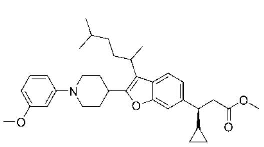
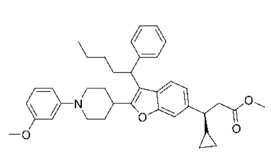
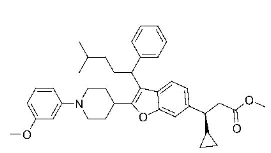
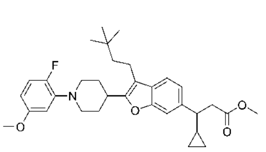
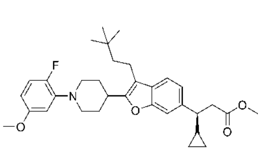
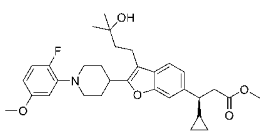
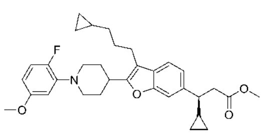
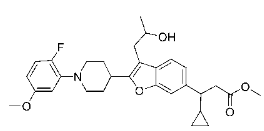
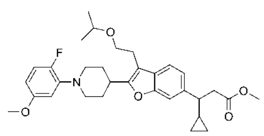

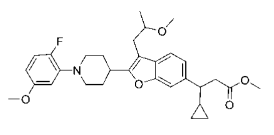
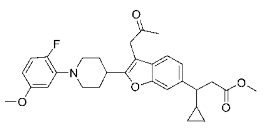
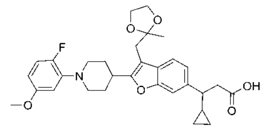
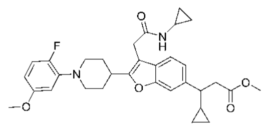
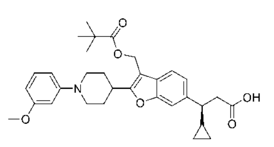
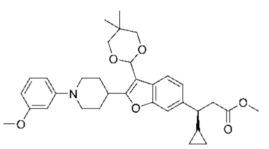
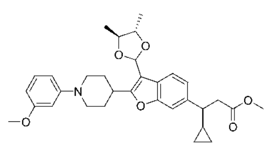
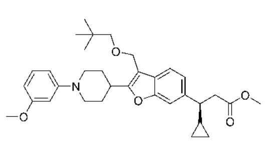
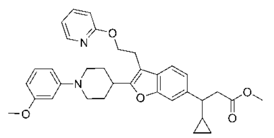
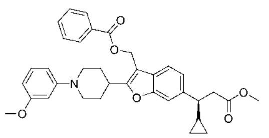
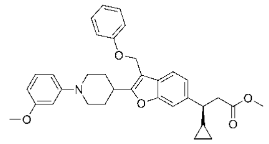
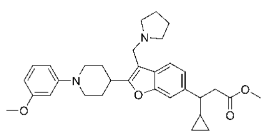
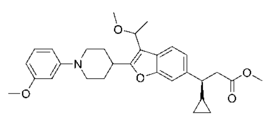
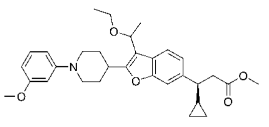
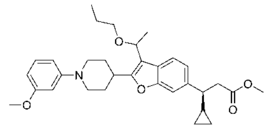
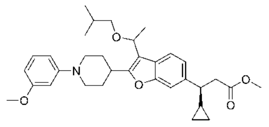
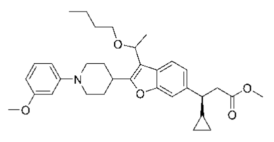
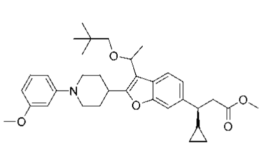
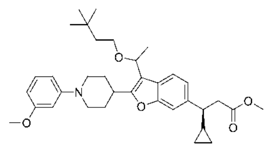
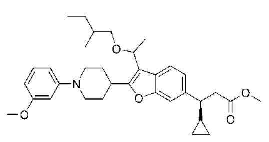
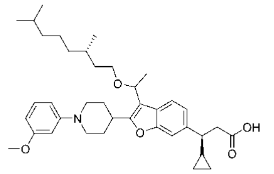
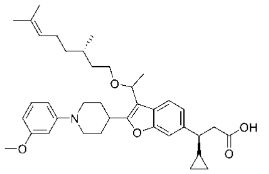
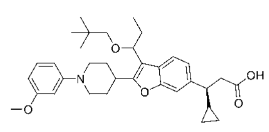
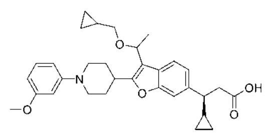
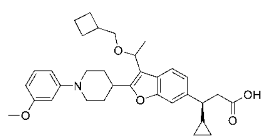
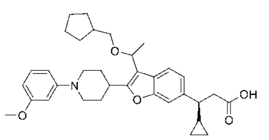
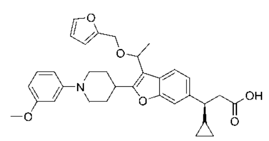
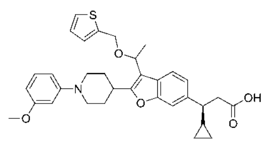
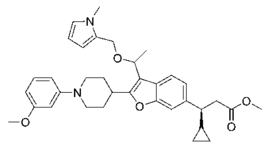
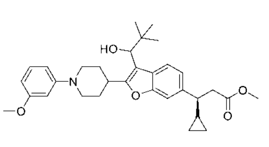
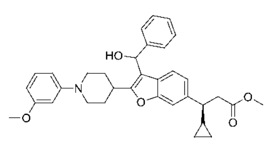
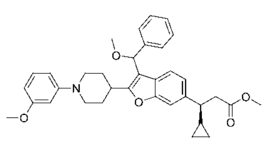
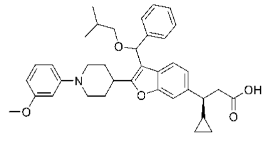
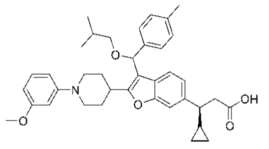
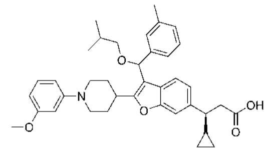
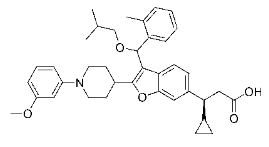

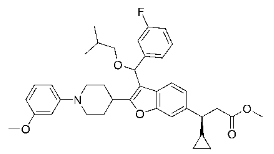
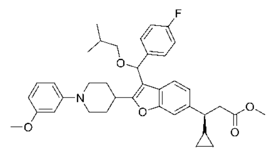
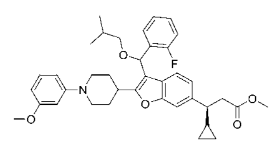
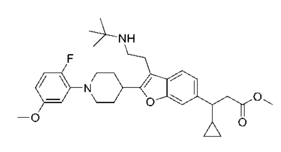
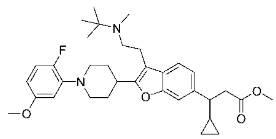
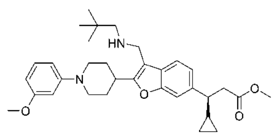
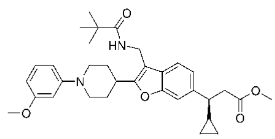
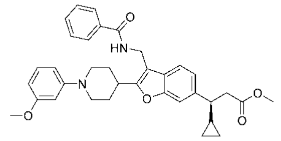
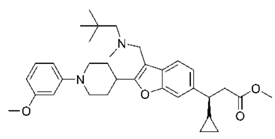
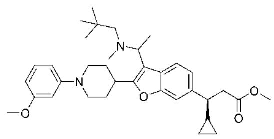
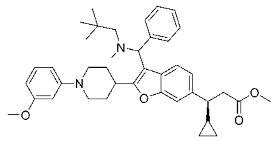
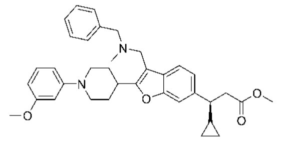
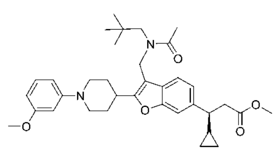
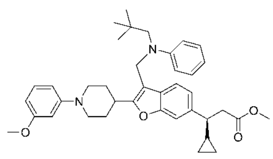
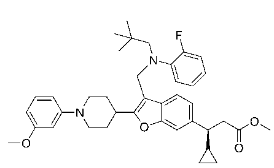
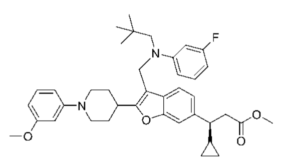
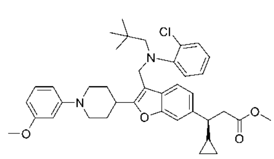
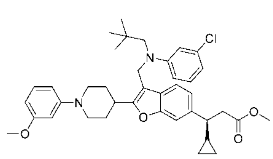
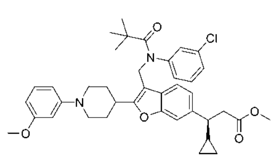
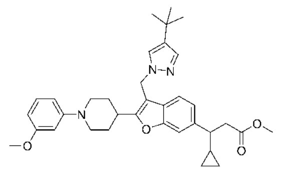
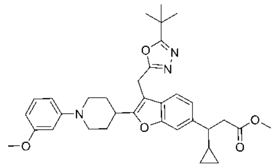
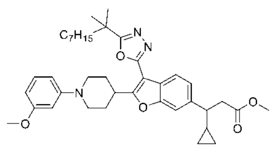
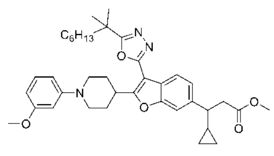
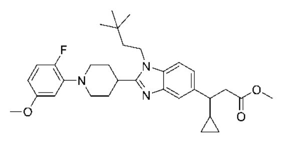

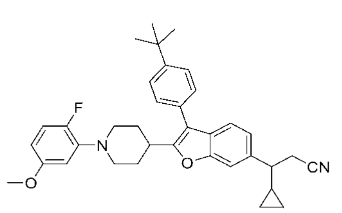 and 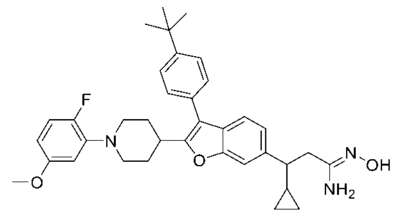 .

**12.** A pharmaceutical composition comprising the compound of formula I or the pharmaceutically acceptable salt thereof according to any one of claims 1 to 9, and a pharmaceutically acceptable excipient.

**13.** A use of the compound of formula I or the pharmaceutically acceptable salt thereof according to any one of claims 1 to 9 in the manufacture of a GPR40 agonist or in the manufacture of a medicament;

the medicament is used for treating or preventing diabetes or a GPR40-related disease;
the GPR40-related disease is preferably diabetes.

**14.** A method for treating or preventing a disease, comprising administering to a patient an effective amount of the compound of formula I or the pharmaceutically acceptable salt thereof according to any one of claims 1 to 9;

wherein the disease is diabetes or a GPR40-related disease;

the GPR40-related disease is preferably diabetes.

**INTERNATIONAL SEARCH REPORT**

International application No.
**PCT/CN2024/081713**

**A. CLASSIFICATION OF SUBJECT MATTER**

C07D405/04(2006.01)i； C07D405/14(2006.01)i； A61K31/343(2006.01)i； A61K31/4184(2006.01)i； A61K31/404(2006.01)i； A61P3/10(2006.01)i

According to International Patent Classification (IPC) or to both national classification and IPC

**B. FIELDS SEARCHED**

Minimum documentation searched (classification system followed by classification symbols)

IPC： C07D405/04， C07D405/14， A61K31/343， A61K31/4184， A61K31/404， A61P3/10

Documentation searched other than minimum documentation to the extent that such documents are included in the fields searched

Electronic data base consulted during the international search (name of data base and, where practicable, search terms used)

CNTXT, ENTXTC, VEN, CNKI, REGISTRY, CAPLUS, MARPAT: 苯并呋喃, 吲哚, 苯并咪唑, GPR40, 糖尿病, G蛋白偶联受体, GPR40, ffa1, gpcr, benzofuran+, benzimidazol+, benzofuran+, indol+, +diabetes, 根据式I, II-1, II-2, II-1a 和II-2a进行的化合物子结构检索, search for substructures of compounds according to formulae I, II-1, II-2, II-1a and II-2a

**C. DOCUMENTS CONSIDERED TO BE RELEVANT**

| Category* | Citation of document, with indication, where appropriate, of the relevant passages | Relevant to claim No. |
|---|---|---|
| A | CN 104788405 A (SHANDONG XUANZHU PHARMA CO., LTD.) 22 July 2015 (2015-07-22)<br>entire document | 1-14 |
| A | EP 3031799 A1 (TAKEDA PHARMACEUTICAL COMPANY LIMITED) 15 June 2016 (2016-06-15)<br>entire document, in particular abstract, claims, and description, embodiment 4, and page 157 | 1-14 |
| A | CN 102083783 A (AMGEN INC.) 01 June 2011 (2011-06-01)<br>entire document | 1-14 |
| A | US 2019367495 A1 (MERCK SHARP & DOHME LIMITED) 05 December 2019 (2019-12-05)<br>entire document | 1-14 |
| PA | WO 2023134712 A1 (REZUBIO PHARMACEUTICALS CO., LTD.) 20 July 2023 (2023-07-20)<br>abstract, claims, and description, paragraph [0186], formula III-B-3, and embodiment 177 | 1-14 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 July 2024** | **09 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/081713**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:
Although claim 14 relates to a treatment method implemented on a human or animal body (PCT Rule 39.1(iv)), a search is still carried out on the basis of the pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/081713**

| Patent document cited in search report | | | Publication date (day/month/year) | Patent family member(s) | | | Publication date (day/month/year) |
|---|---|---|---|---|---|---|---|
| CN | 104788405 | A | 22 July 2015 | CN | 104788405 | B | 19 April 2017 |
| EP | 3031799 | A1 | 15 June 2016 | WO | 2015020184 | A1 | 12 February 2015 |
| | | | | PL | 3031799 | T3 | 28 September 2018 |
| | | | | CY | 1120855 | T1 | 11 December 2019 |
| | | | | US | 2016115128 | A1 | 28 April 2016 |
| | | | | US | 9776962 | B2 | 03 October 2017 |
| | | | | RS | 57359 | B1 | 31 August 2018 |
| | | | | DK | 3031799 | T3 | 28 May 2018 |
| | | | | PT | 3031799 | T | 04 July 2018 |
| | | | | HRP | 20181011 | T1 | 24 August 2018 |
| | | | | LT | 3031799 | T | 25 June 2018 |
| | | | | HUE | 039259 | T2 | 28 December 2018 |
| | | | | SI | 3031799 | T1 | 31 July 2018 |
| | | | | ES | 2672992 | T3 | 19 June 2018 |
| | | | | JPWO | 2015020184 | A1 | 02 March 2017 |
| | | | | JP | 6397410 | B2 | 26 September 2018 |
| | | | | EP | 3031799 | A4 | 11 January 2017 |
| | | | | EP | 3031799 | B1 | 04 April 2018 |
| CN | 102083783 | A | 01 June 2011 | IL | 204635 | A0 | 30 November 2010 |
| | | | | JP | 2011500562 | A | 06 January 2011 |
| | | | | JP | 5591706 | B2 | 17 September 2014 |
| | | | | TN | 2010000125 | A1 | 26 September 2011 |
| | | | | MA | 31841 | B1 | 01 November 2010 |
| | | | | BRPI | 0818253 | A2 | 07 April 2015 |
| | | | | TW | 200927091 | A | 01 July 2009 |
| | | | | PE | 20091222 | A1 | 14 September 2009 |
| | | | | CO | 6270312 | A2 | 20 April 2011 |
| | | | | KR | 20100090249 | A | 13 August 2010 |
| | | | | MX | 2010003876 | A | 02 December 2010 |
| | | | | US | 2009137561 | A1 | 28 May 2009 |
| | | | | US | 8030354 | B2 | 04 October 2011 |
| | | | | AR | 068840 | A1 | 09 December 2009 |
| | | | | CA | 2702047 | A1 | 16 April 2009 |
| | | | | CA | 2702047 | C | 17 April 2012 |
| | | | | AU | 2008311355 | A1 | 16 April 2009 |
| | | | | AU | 2008311355 | B2 | 19 January 2012 |
| | | | | CR | 11420 | A | 12 August 2010 |
| | | | | WO | 2009048527 | A1 | 16 April 2009 |
| | | | | CL | 2008002997 | A1 | 09 October 2009 |
| | | | | EP | 2205548 | A1 | 14 July 2010 |
| US | 2019367495 | A1 | 05 December 2019 | EP | 3551176 | A1 | 16 October 2019 |
| | | | | EP | 3551176 | A4 | 24 June 2020 |
| | | | | US | 11072602 | B2 | 27 July 2021 |
| | | | | WO | 2018106518 | A1 | 14 June 2018 |
| WO | 2023134712 | A1 | 20 July 2023 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023102432757
- CN 2024102637408